# EUROPEAN PATENT APPLICATION

(11) **EP 4 226 938 A2**
(43) Date of publication of application: **16.08.2023**
(21) Application number: 22210373.1
(22) Date of filing: 29.11.2022
(51) Int. Cl.: A61K 39/12, A61P 31/14

(54) **CORONAVIRUS VACCINE**

(30) Priority: 29.11.2021 US 202163283976 P; 08.12.2021 US 202163287486 P; 17.12.2021 US 202163291347 P; 25.01.2022 US 202263302997 P; 28.03.2022 US 202263324586 P; 16.05.2022 US 202263342614 P; 25.06.2022 US 202263355597 P; 26.06.2022 US 202263355648 P; 30.06.2022 US 202263357628 P; 05.07.2022 US 202263358522 P; 02.08.2022 US 202263394571 P; 30.08.2022 US 202263402444 P; 19.10.2022 US 202263417680 P; 03.11.2022 US 202263422404 P; 14.11.2022 US 202263425290 P
(71) Applicant: BioNTech SE, 55131 Mainz (DE)
(72) Inventor: MUIK, Alexander, 55131 Mainz (DE); PORAN, Asaf, Cambridge, 02139 (US); SAHIN, Ugur, 55131 Mainz (DE); SWANSON, Kena Anne, Pearl River, 10965 (US); YANG, Qi, Pearl River, 10965 (US); CAI, Hui, Pearl River, 10965 (US)
(74) Representative: Thomann, William John

(57) **Abstract**

This disclosure relates to the field of RNA to prevent or treat coronavirus infection. In particular, the present disclosure relates to methods and agents for vaccination against coronavirus infection and inducing effective coronavirus antigen-specific immune responses such as antibody and/or T cell responses. Specifically, in one embodiment, the present disclosure relates to methods comprising administering to a subject RNA encoding a peptide or protein comprising an epitope of SARS-CoV-2 spike protein (S protein) for inducing an immune response against coronavirus S protein, in particular S protein of SARS-CoV-2, in the subject, i.e., vaccine RNA encoding vaccine antigen.

## Description

This disclosure relates to the field of RNA to prevent or treat coronavirus infection. In particular, the present disclosure relates to methods and agents for vaccination against coronavirus infection and inducing effective coronavirus antigen-specific immune responses such as antibody and/or T cell responses. These methods and agents are, in particular, useful for the prevention or treatment of coronavirus infection. Administration of RNA disclosed herein to a subject can protect the subject against coronavirus infection. Specifically, in one embodiment, the present disclosure relates to methods comprising administering to a subject RNA encoding a peptide or protein comprising an epitope of SARS-CoV-2 spike protein (S protein) for inducing an immune response against coronavirus S protein, in particular S protein of SARS-CoV-2, in the subject, i.e., vaccine RNA encoding vaccine antigen. Administering to the subject RNA encoding vaccine antigen may provide (following expression of the RNA by appropriate target cells) vaccine antigen for inducing an immune response against vaccine antigen (and disease-associated antigen) in the subject.

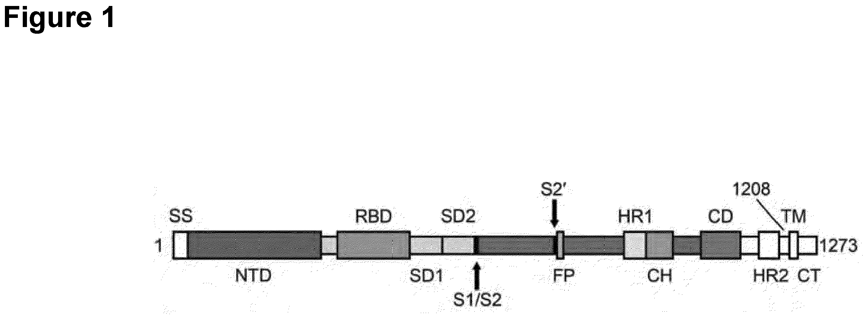

Coronaviruses are positive-sense, single-stranded RNA ((+)ssRNA) enveloped viruses that encode for a total of four structural proteins, spike protein (S), envelope protein (E), membrane protein (M) and nucleocapsid protein (N). The spike protein (S protein) is responsible for receptor-recognition, attachment to the cell, infection via the endosomal pathway, and the genomic release driven by fusion of viral and endosomal membranes. Though sequences between the different family members vary, there are conserved regions and motifs within the S protein making it possible to divide the S protein into two subdomains: S1 and S2. While the S2, with its transmembrane domain, is responsible for membrane fusion, the S1 domain recognizes the virus-specific receptor and binds to the target host cell. Within several coronavirus isolates, the receptor binding domain (RBD) was identified and a general structure of the S protein defined (Figure 1).

In December 2019, a pneumonia outbreak of unknown cause occurred in Wuhan, China and it became clear that a novel coronavirus (severe acute respiratory syndrome coronavirus 2; SARS-CoV-2) was the underlying cause. The genetic sequence of SARS-CoV-2 became available to the WHO and public (MN908947.3) and the virus was categorized into the betacoronavirus subfamily. By sequence analysis, the phylogenetic tree revealed a closer relationship to severe acute respiratory syndrome (SARS) virus isolates than to another coronavirus infecting humans, namely the Middle East respiratory syndrome (MERS) virus.

SARS-CoV-2 infections and the resulting disease COVID-19 have spread globally, affecting a growing number of countries. On 11 March 2020 the WHO characterized the COVID-19 outbreak as a pandemic. As of 01 December 2020, there have been >63 million globally confirmed COVID-19 cases and >1.4 million deaths, with 191 countries/regions affected. The ongoing pandemic remains a significant challenge to public health and economic stability worldwide.

Every individual is at risk of infection as there is no pre-existing immunity to SARS-CoV-2. Following infection some but not all individuals develop protective immunity in terms of neutralising antibody responses and cell mediated immunity. However, it is currently unknown to what extent and for how long this protection lasts. According to WHO 80% of infected individuals recover without need for hospital care, while 15% develop more severe disease and 5% need intensive care. Increasing age and underlying medical conditions are considered risk factors for developing severe disease.

The presentation of COVID-19 is generally with cough and fever, with chest radiography showing ground-glass opacities or patchy shadowing. However, many patients present without fever or radiographic changes, and infections may be asymptomatic which is relevant to controlling transmission. For symptomatic subjects, progression of disease may lead to acute respiratory distress syndrome requiring ventilation and subsequent multi-organ failure and death. Common symptoms in hospitalized patients (in order of highest to lowest frequency) include fever, dry cough, shortness of breath, fatigue, myalgias, nausea/vomiting or diarrhoea, headache, weakness, and rhinorrhoea. Anosmia (loss of smell) or ageusia (loss of taste) may be the sole presenting symptom in approximately 3% of individuals who have COVID-19.

All ages may present with the disease, but notably case fatality rates (CFR) are elevated in persons >60 years of age. Comorbidities are also associated with increased CFR, including cardiovascular disease, diabetes, hypertension, and chronic respiratory disease. Healthcare workers are overrepresented among COVID-19 patients due to occupational exposure to infected patients.

In most situations, a molecular test is used to detect SARS-CoV-2 and confirm infection. The reverse transcription polymerase chain reaction (RT-PCR) test methods targeting SARS-CoV-2 viral RNA are the gold standard in vitro methods for diagnosing suspected cases of COVID-19. Samples to be tested are collected from the nose and/or throat with a swab.

Among other things, the present disclosure provides insights into immune responses elicited by exposure to (e.g., by vaccination and/or infection) different SARS-CoV-2 variants or immunogenic polypeptides (e.g., S protein), or immunogenic fragments thereof. For example, in some embodiments, administering RNA encoding an S protein of a BA.2 and/or BA.4/5 Omicron SARS-CoV-2 variant, or an immunogenic fragment thereof, can result in an improved immune response, which includes, e.g., improved neutralization of Omicron BA.4 and/or Omicron BA.5 SARS-CoV-2 variants and/or broader cross-neutralization of Omicron variants of concern (e.g., higher neutralization titers against a larger number of Omicron variants of concern). In some embodiments, the present disclosure provides an insight that a bivalent coronavirus vaccine (e.g., a bivalent BA.4/5 vaccine comprising a first RNA encoding a SARS-CoV-2 S protein of a Wuhan strain or an immunogenic fragment thereof, and a second RNA encoding a SARS-CoV-2 S protein comprising one or more mutations that are characteristic of a BA.4/5 Omicron variant or an immunogenic fragment thereof) can provide broader cross-neutralization against SARS-CoV-2 Wuhan strain and certain variants thereof (e.g., in some embodiments variants that are prevalent and/or rapidly spreading in a relevant jurisdiction, e.g., certain Omicron variants) in certain subjects as compared to a monovalent coronavirus vaccine (e.g., a vaccine comprising RNA encoding a SARS-CoV-2 S protein of a coronavirus strain or variant thereof). In some embodiments, such broader cross-neutralization can be observed in vaccine-naïve subjects. In some embodiments, such broader cross-neutralization can be observed in subjects without a coronavirus infection (e.g., a SARS-CoV-2 infection). In some embodiments, such broader cross-neutralization can be observed in subjects who previously received a SARS-CoV-2 vaccine (e.g., in some embodiments an RNA vaccine encoding a SARS-CoV-2 S protein, e.g., in some embodiments of a Wuhan strain). In some embodiments, such broader cross-neutralization can be observed in in young pediatric subjects (e.g., subjects aged 6 months to less than 2 years, and/or 2 years to less than 5 years). In some embodiments, the present disclosure provides an insight that exposure to at least two certain SARS-CoV-2 variants or immunogenic polypeptides (e.g., S protein), or immunogenic fragments thereof can result in a synergistic improvement in immune response (e.g., higher neutralization titers, broader cross-neutralization, and/or an immune response that is less susceptible to immune escape) as compared to exposure to one SARS-CoV-2 strain and/or other combination of SARS-CoV-2 variants. In some embodiments, the present disclosure provides an insight that exposure to a SARS-CoV-2 S protein from a Wuhan strain or an immunogenic fragment thereof (e.g., by vaccination and/or infection), and exposure to a SARS-CoV-2 S protein of an Omicron BA.1 variant or an immunogenic fragment thereof (e.g., by vaccination and/or infection) can result in a synergistic improvement in immune response (e.g., higher neutralization titers, broader cross-neutralization, and/or an immune response that is less susceptible to immune escape) as compared to exposure to one SARS-CoV-2 strain and/or other combinations of SARS-CoV-2 variants. In some embodiments, the present disclosure provides an insight that exposure to a SARS-CoV-2 S protein from a Wuhan strain or an immunogenic fragment thereof (e.g., by vaccination and/or infection), and exposure to a SARS-CoV-2 S protein of an Omicron BA.4 or BA.5 variant or an immunogenic fragment thereof (e.g., by vaccination and/or infection) can result in an synergistic improvement in immune response (e.g., higher neutralization titers, broader cross-neutralization, and/or an immune response that is less susceptible to immune escape) as compared to exposure to one SARS-CoV-2 strain and/or other combinations of SARS-CoV-2 variants. In some embodiments, the present disclosure provides an insight that (i) exposure to a SARS-CoV-2 S protein from a strain/variant selected from the group consisting of Wuhan strain, an alpha variant, beta variant, delta variant, Omicron BA.1, and sublineages derived from any of the aforementioned strains/variants, or immunogenic fragments thereof (e.g., by vaccination and/or infection), combined with (ii) exposure to a SARS-CoV-2 S protein from a strain/variant selected from the group consisting of Omicron BA.2, Omicron BA.4, Omicron BA.5, and sublineages derived from any of the aforementioned strains/variants, or immunogenic fragments thereof (e.g., by vaccination and/or infection) can result in a synergistic improvement in immune response (e.g., higher neutralization titers, broader cross-neutralization, and/or an immune response that is less susceptible to immune escape) as compared to exposure to one SARS-CoV-2 strain and/or other combinations of SARS-CoV-2 variants).

The present disclosure also provides significant insights into how an immune response develops in subjects following exposures to (e.g., vaccinations and/or infections) multiple, different SARS-CoV-2 strains. Among other things, disclosed herein is a finding that different combinations of SARS-CoV-2 variants elicit different immune responses. Specifically, the present disclosure provides an insight that exposure to certain combinations of SARS-CoV-2 variants can elicit an improved immune response (e.g., higher neutralization titers, broader cross-neutralization, and/or an immune response that is less susceptible to immune escape). In some embodiments, an improved immune response can be produced when subjects are delivered two or more antigens (e.g., as polypeptides or RNAs encoding such polypeptides), each having few shared epitopes. In some embodiments, an improved immune response can be produced when subjects are delivered a combination of SARS-CoV-2 S proteins (e.g., as polypeptides or RNAs encoding such polypeptides) sharing no more than 50% (e.g., no more than 40%, no more than 30%, no more 20% or more) of epitopes (including, e.g., amino acid mutations) that can be bound by neutralization antibodies. In some embodiments, an improved immune response can be produced by delivering, as polypeptides or RNAs encoding such polypeptides, (a) a SARS-CoV-2 S protein from a Wuhan strain, an Alpha variant, Beta variant, or a Delta variant of SARS-CoV-2 or an immunogenic fragment thereof, and (b) an S protein from a SARS-CoV-2 Omicron variant or an immunogenic fragment thereof. In some embodiments, an improved immune response can be produced by delivering, as polypeptides or RNAs encoding such polypeptides, (a) a SARS-CoV-2 S protein from a Wuhan strain, an Alpha variant, a Beta variant, or a Delta variant of SARS-CoV-2 or an immunogenic fragment thereof, and (b) an S protein of a SARS-CoV-2 Omicron variant that is not a BA.1 Omicron variant or an immunogenic fragment thereof. In some embodiments, an improved immune response can be produced by delivering, as polypeptides or RNAs encoding such polypeptides, (a) an S protein from a Wuhan strain, an Alpha variant, a Beta Variant, a Delta SARS-CoV-2 variant, or a BA.1 Omicron variant or an immunogenic fragment thereof and (b) an S protein of a SARS-CoV-2 Omicron variant that is not a BA.1 Omicron variant or an immunogenic fragment thereof. In some embodiments, an improved immune response can be produced by delivering, as polypeptides or RNAs encoding such polypeptides, (a) a SARS-CoV-2 S protein from a Wuhan strain, an Alpha variant, a Beta variant, or a Delta variant, or an immunogenic fragment thereof and (b) an S protein of a BA.2 or a BA.4 or BA.5 SARS-CoV-2 Omicron variant or an immunogenic fragment thereof.

In some embodiments, the present disclosure also provides an insight that administration of multiple doses (e.g., at least 2, at least 3, at least 4, or more doses) of a coronavirus vaccine described herein (e.g., a bivalent vaccine described herein such as a bivalent BA.4/5 vaccine) may provide certain beneficial effect(s) on affinity of antibodies against one or more SARS-CoV-2 strain or variants thereof. In some embodiments, such beneficial effect(s) on affinity of antibodies may be observed with respect to antibodies against certain Omicron variants. By way of example only, in some embodiments, such beneficial effect(s) on affinity of antibodies may be observed with respect to antibodies against certain Omicron variants that share at least one or more common epitopes, for example, with a Wuhan strain.

Also disclosed herein are compositions that can produce an improved immune response (e.g., an immune response having broader cross-neutralization activity, stronger neutralization, and/or which is less susceptible to immune escape). In some embodiments, a composition described herein comprises two or more antigens or nucleic acids (e.g., RNA) that encodes such antigens that have few shared epitopes. In some embodiments, a composition described herein delivers, as polypeptides or nucleic acids encoding such polypeptides, a combination of SARS-CoV-2 S proteins or immunogenic fragments thereof sharing no more than 50% (e.g., no more than 40%, no more than 30%, no more than 20% or more) of epitopes (including, e.g., amino acid mutations) that can be bound by neutralization antibodies. In some embodiments, a composition described herein comprises (a) RNA encoding a SARS-CoV-2 S protein from a Wuhan strain, an Alpha variant, a Beta variant, or a Delta variant or an immunogenic fragment thereof and (b) RNA encoding an S protein from an Omicron variant of SARS-CoV-2 (e.g., in some embodiments an S protein from a BA.1, BA.2, or BA.4/5 Omicron variant) or an immunogenic fragment thereof. In some embodiments, a composition described herein comprises (a) RNA encoding a SARS-CoV-2 S protein from a Wuhan strain, an Alpha variant, a Beta variant, or a Delta variant or an immunogenic fragment thereof and (b) RNA encoding an S protein of an Omicron variant of SARS-CoV-2 that is not a BA.1 Omicron variant or an immunogenic fragment thereof. In some embodiments, a composition described herein comprises (a) RNA encoding a SARS-CoV-2 S protein of a Wuhan strain, an Alpha variant, a Beta variant, or a Delta variant or a BA.1 Omicron variant or an immunogenic fragment thereof and (b) RNA encoding an S protein of a Omicron variant that is not a BA.1 Omicron variant or an immunogenic fragment thereof. In some embodiments, a composition described herein comprises (a) RNA encoding a SARS-CoV-2 S protein of a Wuhan strain, an Alpha variant, a Beta variant or a Delta variant of SARS-CoV-2 and (b) RNA encoding an S protein from a BA.2 or a BA.4 or BA.5 Omicron variant of SARS-CoV-2 or an immunogenic fragment thereof. In some embodiments, a composition described herein comprises RNA encoding an S protein from a BA.2 Omicron variant of SARS-CoV-2 or an immunogenic fragment thereof. In some embodiments, a composition comprises RNA encoding an S protein from a BA.4 or BA.5 Omicron variant of SARS-CoV-2 or an immunogenic fragment thereof.

SARS-CoV-2 is an RNA virus with four structural proteins. One of them, the spike protein is a surface protein which binds the angiotensin-converting enzyme 2 (ACE-2) present on host cells. Therefore, the spike protein is considered a relevant antigen for vaccine development. BNT162b2 (SEQ ID NO: 20) is an mRNA vaccine for prevention of COVID-19 and demonstrated an efficacy of 95% or more at preventing COVID-19. The vaccine is made of a 5'capped mRNA encoding for the full-length SARS-CoV-2 spike glycoprotein (S) encapsulated in lipid nanoparticles (LNPs). The finished product is presented as a concentrate for dispersion for injection containing BNT162b2 as active substance. Other ingredients are: ALC-0315 (4-hydroxybutyl)azanediyl)bis(hexane-6,1-diyl)bis(2-hexyldecanoate), ALC-0159 (2-[(polyethylene glycol)-2000]-N,N-ditetradecylacetamide), 1,2-Distearoyl-sn-glycero-3-phosphocholine (DSPC), cholesterol, potassium chloride, potassium dihydrogen phosphate, sodium chloride, disodium phosphate dihydrate, sucrose and water for injection.

In some embodiments, a different buffer may be used in lieu of PBS. In some embodiments, the buffer is formulated in a Tris-buffered solution. In some embodiments, the formulation comprises ALC-0315 (4-hydroxybutyl)azanediyl)bis(hexane-6,1-diyl)bis(2-hexyldecanoate), ALC-0159 (2-[(polyethylene glycol)-2000]-N,N-ditetradecylacetamide), DSPC (1,2-distearoyl-sn-glycero-3-phosphocholine), cholesterol, sucrose, trometamol (Tris), trometamol hydrochloride and water.

In some embodiments, the concentration of RNA in a pharmaceutical RNA preparation is about 0.1 mg/ml. In some embodiments about 30 ug of RNA is administered by administering about 200 uL of RNA preparation. In some embodiments, RNA in a pharmaceutical RNA preparation is diluted prior to administration (e.g., diluted to a concentration of about 0.05 mg/ml). In some embodiments, administration volumes are between about 200 µl and about 300 µl. In some embodiments, RNA in a pharmaceutical RNA preparation is formulated in about 10 mM Tris buffer, and about 10% sucrose.

In some embodiments, the concentration of RNA in a pharmaceutical RNA preparation is about 0.1 mg/ml, and is formulated in about 10 mM Tris buffer, about 10% sucrose and a dose of about 10 µg or RNA is administered by diluting a pharmaceutical RNA preparation about 1:1 and administering about 200 µl of diluted pharmaceutical RNA preparation. In some embodiments, the concentration of RNA in a pharmaceutical RNA preparation is about 0.1 mg/ml, and is formulated in about 10 mM Tris buffer, about 10% sucrose and a dose of RNA of about 10 µg is administered by diluting a pharmaceutical RNA preparation about 1:5.75 and administering about 200 µl of diluted pharmaceutical RNA preparation.

The amino acid sequence of the S protein encoded by BNT162b2 was chosen based on the sequence for the "SARS-CoV-2 isolate Wuhan-Hu -1": GenBank: MN908947.3 (complete genome) and GenBank: QHD43416.1 (spike surface glycoprotein). The BNT162b2 active substance consists of a single-stranded, 5'-capped codon-optimized mRNA that is translated into the spike antigen of SARS-CoV-2. The spike antigen protein sequence encoded by BNT162b2 contains two proline mutations, which stabilizes an antigenically improved prefusion confirmation (P2 S). BNT162b2 does not contain any uridines; instead of uridine the modified N1-methylpseudouridine is used in RNA synthesis. BNT162b2 mRNA is translated into a SARS-CoV-2 S protein in host cells. The S protein is then expressed on the cell surface where it induces an adaptive immune response. The S protein encoded by BNT162b2 is identified as a target for neutralising antibodies against the virus and is considered a relevant vaccine component. For adult vaccine naïve subjects (i.e., subjects 16 years and older who have not previously been administered a SARS-CoV-2 vaccine), the dosing regimen of BNT162b2 approved by the FDA comprises administering intramuscularly (IM) two 30 µg doses of the diluted vaccine solution approximately 21 days apart.

The recent emergence of novel circulating variants of SARS-CoV-2 has raised significant concerns about geographic and temporal efficacy of vaccine interventions. One of the earliest variants that emerged and rapidly became globally dominant was D614G.

The alpha variant (also known as B.1.1.7, VOC202012/01, 501Y.V1 or GRY) was initially detected in the United Kingdom. The alpha variant has a large number of mutations, including several mutations in the S gene. It has been shown to be inherently more transmissible, with a growth rate that has been estimated to be 40-70% higher than other SARS-CoV-2 lineages in multiple countries (Volz et al., 2021, Nature, https://doi.org/10.1038/s41586-021-03470-x; Washington et al., 2021, Cell https://doi.org/10.1016/j.cell.2021.03.052).

The beta variant (also known as B.1.351 or GH/501Y.V2) was first detected in South Africa. The beta variant carries several mutations in the S gene. Three of these mutations are at sites in the RBD that are associated with immune evasion: N501Y (shared with alpha) and E484K and K417N.

The gamma variant (also known as P.1 or GR/501Y.V3) was first detected in Brazil. The gamma variant carries several mutations that affect the spike protein, including two shared with beta (N501Y and E484K), as well as a different mutation at position 417 (K417T).

The delta variant (also known as B.1.617.2 or G/478K.V1) was first documented in India. The delta variant has several point mutations that affect the spike protein, including P681R (a mutation position shared with alpha and adjacent to the furin cleavage site), and L452R, which is in the RBD and has been linked with increased binding to ACE2 and neutralizing antibody resistance. There is also a deletion in the spike protein at position 156/157.

These four VOCs have circulated globally and became dominant variants in the geographic regions where they were first identified.

On 24 November 2021, the Omicron (B.1.1.529) variant was first reported to WHO from South Africa. SARS-CoV-2 Omicron and its sublineages have had a major impact on the epidemiological landscape of the COVID-19 pandemic since initial emergence in November 2021 (WHO Technical Advisory Group on SARS-CoV-2 Virus Evolution (TAG-VE): Classification of Omicron (B.1.1.259): SARS-CoV-2 Variant of Concern (2021); WHO Headquarters (HQ), WHO Health Emergencies Programme, Enhancing Response to Omicron SARS-CoV-2 variant: Technical brief and priority actions for Member States (2022)). Significant alterations in the spike (S) glycoprotein of the first Omicron variant BA.1 leading to the loss of many neutralizing antibody epitopes (M. Hoffmann et al., "The Omicron variant is highly resistant against antibody mediated neutralization: Implications for control of the COVID-19 pandemic", Cell 185, 447-456.e11 (2022)) rendered BA.1 capable of partially escaping previously established SARS-CoV-2 wild-type strain (Wuhan-Hu-1)-based immunity (V. Servellita, et al., "Neutralizing immunity in vaccine breakthrough infections from the SARS-CoV-2 Omicron and Delta variants", Cell 185, 1539-1548.e5 (2022); Y. Cao et al., "Omicron escapes the majority of existing SARS-CoV-2 neutralizing antibodies", Nature 602, 657-663 (2022)). Hence, breakthrough infection of vaccinated individuals with Omicron are more common than with previous Variants of Concern (VOCs). While Omicron BA.1 was displaced by the BA.2 variant in many countries around the globe, other variants such as BA.1.1 and BA.3 temporarily and/or locally gained momentum but did not become globally dominant (S. Xia et al., "Origin, virological features, immune evasion and intervention of SARS-CoV-2 Omicron sublineages. Signal Transduct. Target. Ther. 7, 241 (2022); H. Gruell et al., "SARS-CoV-2 Omicron sublineages exhibit distinct antibody escape patterns, Cell Host Microbe 7, 241 (2022).).

Omicron BA.2.12.1 subsequently displaced BA.2 to become dominant in the United States, whereas BA.4 and BA.5 displaced BA.2 in Europe, parts of Africa, and Asia/Pacific (H. Gruell et al., "SARS-CoV-2 Omicron sublineages exhibit distinct antibody escape patterns," Cell Host Microbe 7, 241 (2022); European Centre for Disease Prevention and Control, Weekly COVID-19 country overview -Country overview report: Week 31 2022 (2022); J. Hadfield et al., "Nextstrain: Real-time tracking of pathogen evolution," Bioinformatics 34, 4121-4123 (2018)). Currently, Omicron BA.5 is dominant globally, including in the United States (Centers for Disease Control and Prevention. COVID Data Tracker. Atlanta, GA: US Department of Health and Human Services, CDC; 2022, August 12. https://covid.cdc.gov/coviddata-tracker (2022)). Omicron has acquired numerous alterations (amino acid exchanges, insertions, or deletions) in the S glycoprotein, among which some are shared between all Omicron VOCs while others are specific to one or more Omicron sublineages. Antigenically, BA.2.12.1 exhibits high similarity with BA.2 but not BA.1, whereas BA.4 and BA.5 differ considerably from their ancestor BA.2 and even more so from BA.1, in line with their genealogy (A. Z. Mykytyn et al., "Antigenic cartography of SARS-CoV-2 reveals that Omicron BA.1 and BA.2 are antigenically distinct," Sci. Immunol. 7, eabq4450 (2022).). Major differences of BA.1 from the remaining Omicron VOCs include Δ143-145, L212I, or ins214EPE in the S glycoprotein N-terminal domain and G446S or G496S in the receptor binding domain (RBD). Amino acid changesT376A, D405N, and R408S in the RBD are in turn common to BA.2 and its descendants but not found in BA.1. In addition, some alterations are specific for individual BA.2-descendant VOCs, including L452Q for BA.2.12.1 or L452R and F486V for BA.4 and BA.5 (BA.4 and BA.5 encode for the same S sequence). Most of these shared and VOC-specific alterations were shown to play an important role in immune escape from monoclonal antibodies and polyclonal sera raised against the wild-type S glycoprotein. In particular, the BA.4/BA.5-specific alterations are strongly implicated in immune escape of these VOCs (P. Wang et al., "Antibody resistance of SARS-CoV-2 variants B.1.351 and B.1.1.7. Nature 593, 130-135 (2021); Q. Wang et al., "Antibody evasion by SARS-CoV-2 Omicron subvariants BA.2.12.1, BA.4, & BA.5. Nature 608, 603-608 (2022)).

### Summary

The present disclosure generally embraces immunotherapeutic treatment of a subject comprising administration of RNA, e.g., vaccine RNA, encoding an amino acid sequence, e.g., a vaccine antigen, comprising a SARS-CoV-2 S protein, an immunogenic variant thereof, or an immunogenic fragment of the SARS-CoV-2 S protein or the immunogenic variant thereof, i.e., an antigenic peptide or protein. Thus, vaccine antigen comprises an epitope of SARS-CoV-2 S protein for inducing an immune response against coronavirus S protein, in particular SARS-CoV-2 S protein, in the subject. RNA encoding vaccine antigen is administered to provide (following expression of the polynucleotide by appropriate target cells) antigen for induction, i.e., stimulation, priming and/or expansion, of an immune response, e.g., antibodies and/or immune effector cells, which is targeted to target antigen (coronavirus S protein, in particular SARS-CoV-2 S protein) or a procession product thereof. In one embodiment, the immune response which is to be induced according to the present disclosure is a B cell-mediated immune response, i.e., an antibody-mediated immune response. Additionally or alternatively, in one embodiment, the immune response which is to be induced according to the present disclosure is a T cell-mediated immune response. In one embodiment, the immune response is an anti-coronavirus, in particular anti-SARS-CoV-2 immune response.

Vaccines described herein comprise as an active principle single-stranded RNA that may be translated into protein upon entering cells of a recipient. In addition to wildtype or codon-optimized sequences encoding an antigen sequence, RNA may contain one or more structural elements optimized for maximal efficacy with respect to stability and translational efficiency (e.g., 5' cap, 5' UTR, 3' UTR, poly(A)-tail, and combinations thereof). In one embodiment, RNA contains all of these elements. In one embodiment, a cap1 structure may be utilized as specific capping structure at the 5'-end of an RNA drug substance. In one embodiment, beta-S-ARCA(D1) (m₂^{7,2-O}GppSpG) or m₂^{7,3'-O}Gppp(m₁^{2'-O})ApG may be utilized as specific capping structure at the 5'-end of an RNA drug substance. As 5'-UTR sequence, the 5'-UTR sequence of the human alpha-globin mRNA, optionally with an optimized 'Kozak sequence' to increase translational efficiency (e.g., SEQ ID NO: 12) may be used. As 3'-UTR sequence, a combination of two sequence elements (FI element) derived from the "amino terminal enhancer of split" (AES) mRNA (called F) and the mitochondrial encoded 12S ribosomal RNA (called I) (e.g., SEQ ID NO: 13) placed between the coding sequence and the poly(A)-tail to assure higher maximum protein levels and prolonged persistence of the mRNA may be used. These features were identified by an ex *vivo* selection process for sequences that confer RNA stability and augment total protein expression (see WO 2017/060314, herein incorporated by reference). Alternatively, the 3'-UTR may be two re-iterated 3'-UTRs of the human beta-globin mRNA.

Additionally or alternatively, in some embodiments, a poly(A)-tail may comprise a length of at least 100 adenosine residues (including, e.g., at least 110 adenosine residues, at least 120 adenosine residues, 130 adenosine residues, or longer). In some embodiments, a poly(A)-tail may comprise a length of about 100 to about 150 adenosine residues. In some embodiments a poly(A)-tail may comprise an interrupted poly(A)-tail. For example, in some such embodiments, a poly(A)-tail measuring 110 nucleotides in length, consisting of a stretch of 30 adenosine residues, followed by a 10 nucleotide linker sequence (of random nucleotides) and another 70 adenosine residues (e.g., SEQ ID NO: 14) may be used. This poly(A)-tail sequence was designed to enhance RNA stability and translational efficiency.

Furthermore, in some embodiments, a nucleotide sequence encoding a secretory signal peptide (sec) may be fused to antigen-encoding regions of an RNA, preferably in some embodiments in a way that the sec is translated as an N terminal tag. In one embodiment, sec corresponds to the secretory signal peptide of a SARS-CoV-2 S protein (e.g., of a Wuhan strain). In some embodiments, sequences coding for short linker peptides predominantly consisting of the amino acids glycine (G) and serine (S), as commonly used for fusion proteins, may be used as GS/Linkers between sec and an antigen.

Vaccine RNA described herein may be complexed with proteins and/or lipids, preferably lipids, to generate RNA-particles for administration. If a combination of different RNAs is used, RNAs may be complexed together or complexed separately with proteins and/or lipids to generate RNA-particles for administration.

In one aspect, the present disclosure relates to a composition or medical preparation comprising RNA encoding an amino acid sequence comprising a SARS-CoV-2 S protein, an immunogenic variant thereof, or an immunogenic fragment of the SARS-CoV-2 S protein or the immunogenic variant thereof.

In one embodiment, an immunogenic fragment of the SARS-CoV-2 S protein comprises the S1 subunit of the SARS-CoV-2 S protein, or the receptor binding domain (RBD) of the S1 subunit of the SARS-CoV-2 S protein.

In one embodiment, an amino acid sequence comprising a SARS-CoV-2 S protein, an immunogenic variant thereof, or an immunogenic fragment of the SARS-CoV-2 S protein or the immunogenic variant thereof is able to form a multimeric complex, in particular a trimeric complex. To this end, an amino acid sequence comprising a SARS-CoV-2 S protein, an immunogenic variant thereof, or an immunogenic fragment of the SARS-CoV-2 S protein or the immunogenic variant thereof may comprise a domain allowing the formation of a multimeric complex, in particular a trimeric complex of the amino acid sequence comprising a SARS-CoV-2 S protein, an immunogenic variant thereof, or an immunogenic fragment of the SARS-CoV-2 S protein or the immunogenic variant thereof. In one embodiment, the domain allowing formation of a multimeric complex comprises a trimerization domain, for example, a trimerization domain as described herein, e.g., SARS-CoV-2 S protein trimerization domain. In one embodiment, trimerization is achieved by addition of a trimerization domain, e.g., a T4-fibritin-derived "foldon" trimerization domain (e.g., SEQ ID NO: 10), in particular if the amino acid sequence comprising a SARS-CoV-2 S protein, an immunogenic variant thereof, or an immunogenic fragment of the SARS-CoV-2 S protein or the immunogenic variant thereof corresponds to a portion of a SARS-CoV-2 S protein that does not comprise the SARS-CoV-2 S protein trimerization domain.

In one embodiment, the amino acid sequence comprising a SARS-CoV-2 S protein, an immunogenic variant thereof, or an immunogenic fragment of the SARS-CoV-2 S protein or the immunogenic variant thereof is encoded by a coding sequence which is codon-optimized and/or the G/C content of which is increased compared to wild type coding sequence, wherein the codon-optimization and/or the increase in the G/C content preferably does not change the sequence of the encoded amino acid sequence. Those skilled in the art will appreciate that codon optimization involves choosing between or among alternative codons encoding the same amino acid residue. Codon optimization typically includes consideration of codon(s) preferred by a particular host in which a sequence is to be expressed. In accordance with the present disclosure, in many embodiments, a preferred host is a human. In some embodiments, a preferred host may be a domestic animal. Alternatively or additionally, in some embodiments, selection between or among possible codons encoding the same amino acid may consider one or more other features such as, for example, overall G/C content (as noted above) and/or similarity to a particular reference. For example, in some embodiments of the present disclosure, a provided coding sequence that encodes a SARS-CoV-2 S protein or immunogenic variant thereof that differs in amino acid sequence from that encoded by a BNT162b2 construct described herein utilizes a codon, in at least one position of such difference, that preserves greater similarity to the BNT162b2 construct sequence relative to at least one alternative codon encoding the same amino acid at such position of difference. In one embodiment,
(i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 979 to 1584 of SEQ ID NO: 2, 8 or 9, a nucleotide sequence having at least 99%, 98%, 97%, 96%, 95%, 90%, 85%, or 80% identity to the nucleotide sequence of nucleotides 979 to 1584 of SEQ ID NO: 2, 8 or 9, or a fragment of the nucleotide sequence of nucleotides 979 to 1584 of SEQ ID NO: 2, 8 or 9, or the nucleotide sequence having at least 99%, 98%, 97%, 96%, 95%, 90%, 85%, or 80% identity to the nucleotide sequence of nucleotides 979 to 1584 of SEQ ID NO: 2, 8 or 9; and/or
(ii) a SARS-CoV-2 S protein, an immunogenic variant thereof, or an immunogenic fragment of the SARS-CoV-2 S protein or the immunogenic variant thereof comprises the amino acid sequence of amino acids 327 to 528 of SEQ ID NO: 1, an amino acid sequence having at least 99%, 98%, 97%, 96%, 95%, 90%, 85%, or 80% identity to the amino acid sequence of amino acids 327 to 528 of SEQ ID NO: 1, or an immunogenic fragment of the amino acid sequence of amino acids 327 to 528 of SEQ ID NO: 1, or the amino acid sequence having at least 99%, 98%, 97%, 96%, 95%, 90%, 85%, or 80% identity to the amino acid sequence of amino acids 327 to 528 of SEQ ID NO: 1.

In one embodiment,
(i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 111 to 986 of SEQ ID NO: 30, a nucleotide sequence having at least 99%, 98%, 97%, 96%, 95%, 90%, 85%, or 80% identity to the nucleotide sequence of nucleotides 111 to 986 of SEQ ID NO: 30, or a fragment of the nucleotide sequence of nucleotides 111 to 986 of SEQ ID NO: 30, or the nucleotide sequence having at least 99%, 98%, 97%, 96%, 95%, 90%, 85%, or 80% identity to the nucleotide sequence of nucleotides 111 to 986 of SEQ ID NO: 30; and/or
(ii) a SARS-CoV-2 S protein, an immunogenic variant thereof, or an immunogenic fragment of the SARS-CoV-2 S protein or the immunogenic variant thereof comprises the amino acid sequence of amino acids 20 to 311 of SEQ ID NO: 29, an amino acid sequence having at least 99%, 98%, 97%, 96%, 95%, 90%, 85%, or 80% identity to the amino acid sequence of amino acids 20 to 311 of SEQ ID NO: 29, or an immunogenic fragment of the amino acid sequence of amino acids 20 to 311 of SEQ ID NO: 29, or the amino acid sequence having at least 99%, 98%, 97%, 96%, 95%, 90%, 85%, or 80% identity to the amino acid sequence of amino acids 20 to 311 of SEQ ID NO: 29.

In one embodiment,
(i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 49 to 2055 of SEQ ID NO: 2, 8 or 9, a nucleotide sequence having at least 99%, 98%, 97%, 96%, 95%, 90%, 85%, or 80% identity to the nucleotide sequence of nucleotides 49 to 2055 of SEQ ID NO: 2, 8 or 9, or a fragment of the nucleotide sequence of nucleotides 49 to 2055 of SEQ ID NO: 2, 8 or 9, or the nucleotide sequence having at least 99%, 98%, 97%, 96%, 95%, 90%, 85%, or 80% identity to the nucleotide sequence of nucleotides 49 to 2055 of SEQ ID NO: 2, 8 or 9; and/or
(ii) a SARS-CoV-2 S protein, an immunogenic variant thereof, or an immunogenic fragment of the SARS-CoV-2 S protein or the immunogenic variant thereof comprises the amino acid sequence of amino acids 17 to 685 of SEQ ID NO: 1, an amino acid sequence having at least 99%, 98%, 97%, 96%, 95%, 90%, 85%, or 80% identity to the amino acid sequence of amino acids 17 to 685 of SEQ ID NO: 1, or an immunogenic fragment of the amino acid sequence of amino acids 17 to 685 of SEQ ID NO: 1, or the amino acid sequence having at least 99%, 98%, 97%, 96%, 95%, 90%, 85%, or 80% identity to the amino acid sequence of amino acids 17 to 685 of SEQ ID NO: 1.

In one embodiment,
(i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 49 to 3819 of SEQ ID NO: 2, 8 or 9, a nucleotide sequence having at least 99%, 98%, 97%, 96%, 95%, 90%, 85%, or 80% identity to the nucleotide sequence of nucleotides 49 to 3819 of SEQ ID NO: 2, 8 or 9, or a fragment of the nucleotide sequence of nucleotides 49 to 3819 of SEQ ID NO: 2, 8 or 9, or the nucleotide sequence having at least 99%, 98%, 97%, 96%, 95%, 90%, 85%, or 80% identity to the nucleotide sequence of nucleotides 49 to 3819 of SEQ ID NO: 2, 8 or 9; and/or
(ii) a SARS-CoV-2 S protein, an immunogenic variant thereof, or an immunogenic fragment of the SARS-CoV-2 S protein or the immunogenic variant thereof comprises the amino acid sequence of amino acids 17 to 1273 of SEQ ID NO: 1 or 7, an amino acid sequence having at least 99%, 98%, 97%, 96%, 95%, 90%, 85%, or 80% identity to the amino acid sequence of amino acids 17 to 1273 of SEQ ID NO: 1 or 7, or an immunogenic fragment of the amino acid sequence of amino acids 17 to 1273 of SEQ ID NO: 1 or 7, or the amino acid sequence having at least 99%, 98%, 97%, 96%, 95%, 90%, 85%, or 80% identity to the amino acid sequence of amino acids 17 to 1273 of SEQ ID NO: 1 or 7.

In one embodiment, the amino acid sequence comprising a SARS-CoV-2 S protein, an immunogenic variant thereof, or an immunogenic fragment of the SARS-CoV-2 S protein or the immunogenic variant thereof comprises a secretory signal peptide.

In one embodiment, the secretory signal peptide is fused, preferably N-terminally, to a SARS-CoV-2 S protein, an immunogenic variant thereof, or an immunogenic fragment of the SARS-CoV-2 S protein or the immunogenic variant thereof.

In one embodiment,
(i) the RNA encoding the secretory signal peptide comprises the nucleotide sequence of nucleotides 1 to 48 of SEQ ID NO: 2, 8 or 9, a nucleotide sequence having at least 99%, 98%, 97%, 96%, 95%, 90%, 85%, or 80% identity to the nucleotide sequence of nucleotides 1 to 48 of SEQ ID NO: 2, 8 or 9, or a fragment of the nucleotide sequence of nucleotides 1 to 48 of SEQ ID NO: 2, 8 or 9, or the nucleotide sequence having at least 99%, 98%, 97%, 96%, 95%, 90%, 85%, or 80% identity to the nucleotide sequence of nucleotides 1 to 48 of SEQ ID NO: 2, 8 or 9; and/or
(ii) the secretory signal peptide comprises the amino acid sequence of amino acids 1 to 16 of SEQ ID NO: 1, an amino acid sequence having at least 99%, 98%, 97%, 96%, 95%, 90%, 85%, or 80% identity to the amino acid sequence of amino acids 1 to 16 of SEQ ID NO: 1, or a functional fragment of the amino acid sequence of amino acids 1 to 16 of SEQ ID NO: 1, or the amino acid sequence having at least 99%, 98%, 97%, 96%, 95%, 90%, 85%, or 80% identity to the amino acid sequence of amino acids 1 to 16 of SEQ ID NO: 1.

In one embodiment,
(i) the RNA encoding a SARS-CoV-2 S protein, an immunogenic variant thereof, or an immunogenic fragment of the SARS-CoV-2 S protein or the immunogenic variant thereof comprises the nucleotide sequence of SEQ ID NO: 6, a nucleotide sequence having at least 99%, 98%, 97%, 96%, 95%, 90%, 85%, or 80% identity to the nucleotide sequence of SEQ ID NO: 6, or a fragment of the nucleotide sequence of SEQ ID NO: 6, or the nucleotide sequence having at least 99%, 98%, 97%, 96%, 95%, 90%, 85%, or 80% identity to the nucleotide sequence of SEQ ID NO: 6; and/or
(ii) a SARS-CoV-2 S protein, an immunogenic variant thereof, or an immunogenic fragment of the SARS-CoV-2 S protein or the immunogenic variant thereof comprises the amino acid sequence of SEQ ID NO: 5, an amino acid sequence having at least 99%, 98%, 97%, 96%, 95%, 90%, 85%, or 80% identity to the amino acid sequence of SEQ ID NO: 5, or an immunogenic fragment of the amino acid sequence of SEQ ID NO: 5, or the amino acid sequence having at least 99%, 98%, 97%, 96%, 95%, 90%, 85%, or 80% identity to the amino acid sequence of SEQ ID NO: 5.

In one embodiment,
(i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 54 to 986 of SEQ ID NO: 30, a nucleotide sequence having at least 99%, 98%, 97%, 96%, 95%, 90%, 85%, or 80% identity to the nucleotide sequence of nucleotides 54 to 986 of SEQ ID NO: 30, or a fragment of the nucleotide sequence of nucleotides 54 to 986 of SEQ ID NO: 30, or the nucleotide sequence having at least 99%, 98%, 97%, 96%, 95%, 90%, 85%, or 80% identity to the nucleotide sequence of nucleotides 54 to 986 of SEQ ID NO: 30; and/or
(ii) a SARS-CoV-2 S protein, an immunogenic variant thereof, or an immunogenic fragment of the SARS-CoV-2 S protein or the immunogenic variant thereof comprises the amino acid sequence of amino acids 1 to 311 of SEQ ID NO: 29, an amino acid sequence having at least 99%, 98%, 97%, 96%, 95%, 90%, 85%, or 80% identity to the amino acid sequence of amino acids 1 to 311 of SEQ ID NO: 29, or an immunogenic fragment of the amino acid sequence of amino acids 1 to 311 of SEQ ID NO: 29, or the amino acid sequence having at least 99%, 98%, 97%, 96%, 95%, 90%, 85%, or 80% identity to the amino acid sequence of amino acids 1 to 311 of SEQ ID NO: 29.

In one embodiment, the RNA is a modified RNA, in particular a stabilized mRNA. In one embodiment, the RNA comprises a modified nucleoside in place of at least one uridine. In one embodiment, the RNA comprises a modified nucleoside in place of each uridine. In one embodiment, the modified nucleoside is independently selected from pseudouridine (ψ), N1-methyl-pseudouridine (m1ψ), and 5-methyl-uridine (m5U).

In one embodiment, RNA comprises a modified nucleoside in place of uridine.

In one embodiment, the modified nucleoside is selected from pseudouridine (ψ), N1-methyl-pseudouridine (m1ψ), and 5-methyl-uridine (m5U).

In one embodiment, RNA comprises a 5' cap.

In one embodiment, RNA encoding an amino acid sequence comprising a SARS-CoV-2 S protein, an immunogenic variant thereof, or an immunogenic fragment of the SARS-CoV-2 S protein or the immunogenic variant thereof comprises a 5' UTR comprising the nucleotide sequence of SEQ ID NO: 12, or a nucleotide sequence having at least 99%, 98%, 97%, 96%, 95%, 90%, 85%, or 80% identity to the nucleotide sequence of SEQ ID NO: 12.

In one embodiment, RNA encoding an amino acid sequence comprising a SARS-CoV-2 S protein, an immunogenic variant thereof, or an immunogenic fragment of the SARS-CoV-2 S protein or the immunogenic variant thereof comprises a 3' UTR comprising the nucleotide sequence of SEQ ID NO: 13, or a nucleotide sequence having at least 99%, 98%, 97%, 96%, 95%, 90%, 85%, or 80% identity to the nucleotide sequence of SEQ ID NO: 13.

In one embodiment, RNA encoding an amino acid sequence comprising a SARS-CoV-2 S protein, an immunogenic variant thereof, or an immunogenic fragment of the SARS-CoV-2 S protein or the immunogenic variant thereof comprises a poly-A sequence.

In one embodiment, the poly-A sequence comprises at least 100 nucleotides.

In one embodiment, the poly-A sequence comprises or consists of the nucleotide sequence of SEQ ID NO: 14.

In one embodiment, RNA is formulated or is to be formulated as a liquid, a solid, or a combination thereof.

In one embodiment, RNA is formulated or is to be formulated for injection.

In one embodiment, RNA is formulated or is to be formulated for intramuscular administration.

In one embodiment, RNA is formulated or is to be formulated as particles.

In one embodiment, particles are lipid nanoparticles (LNP) or lipoplex (LPX) particles.

In one embodiment, LNPs comprise ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoyl-sn-glycero-3-phosphocholine, and cholesterol.

In one embodiment, RNA lipoplex particles are obtainable by mixing RNA with liposomes. In one embodiment, RNA lipoplex particles are obtainable by mixing RNA with lipids.

In one embodiment, RNA is formulated or is to be formulated as colloid. In one embodiment, RNA is formulated or is to be formulated as particles, forming the dispersed phase of a colloid. In one embodiment, 50% or more, 75% or more, or 85% or more of RNA is present in the dispersed phase. In one embodiment, RNA is formulated or is to be formulated as particles comprising RNA and lipids. In one embodiment, particles are formed by exposing RNA, dissolved in an aqueous phase, with lipids, dissolved in an organic phase. In one embodiment, the organic phase comprises ethanol. In one embodiment, particles are formed by exposing RNA, dissolved in an aqueous phase, with lipids, dispersed in an aqueous phase. In one embodiment, the lipids dispersed in an aqueous phase form liposomes.

In one embodiment, RNA is mRNA or saRNA.

In one embodiment, a composition or medical preparation is a pharmaceutical composition. In one embodiment, a composition or medical preparation is a vaccine.

In one embodiment, a pharmaceutical composition further comprises one or more pharmaceutically acceptable carriers, diluents and/or excipients.

In one embodiment, a composition or medical preparation is a kit.

In one embodiment, RNA and optionally the particle forming components are in separate vials. In one embodiment, a kit further comprises instructions for use of a composition or medical preparation for inducing an immune response against coronavirus in a subject.

In one aspect, the present disclosure relates to a composition or medical preparation described herein for pharmaceutical use.

In one embodiment, a pharmaceutical use comprises inducing an immune response against coronavirus in a subject.

In one embodiment, a pharmaceutical use comprises a therapeutic or prophylactic treatment of a coronavirus infection.

In one embodiment, a composition or medical preparation described herein is for administration to a human.

In one embodiment, the coronavirus is a betacoronavirus.

In one embodiment, the coronavirus is a sarbecovirus.

In one embodiment, the coronavirus is SARS-CoV-2.

In one aspect, the present disclosure relates to a method of inducing an immune response against coronavirus in a subject comprising administering to the subject a composition comprising RNA encoding an amino acid sequence comprising a SARS-CoV-2 S protein, an immunogenic variant thereof, or an immunogenic fragment of the SARS-CoV-2 S protein or the immunogenic variant thereof.

In one embodiment, an immunogenic fragment of the SARS-CoV-2 S protein comprises the S1 subunit of the SARS-CoV-2 S protein, or the receptor binding domain (RBD) of the S1 subunit of the SARS-CoV-2 S protein.

In one embodiment, an amino acid sequence comprising a SARS-CoV-2 S protein, an immunogenic variant thereof, or an immunogenic fragment of the SARS-CoV-2 S protein or the immunogenic variant thereof is able to form a multimeric complex, in particular a trimeric complex. To this end, an amino acid sequence comprising a SARS-CoV-2 S protein, an immunogenic variant thereof, or an immunogenic fragment of the SARS-CoV-2 S protein or the immunogenic variant thereof may comprise a domain allowing the formation of a multimeric complex, in particular a trimeric complex of the amino acid sequence comprising a SARS-CoV-2 S protein, an immunogenic variant thereof, or an immunogenic fragment of the SARS-CoV-2 S protein or the immunogenic variant thereof. In one embodiment, the domain allowing the formation of a multimeric complex comprises a trimerization domain, for example, a trimerization domain as described herein.

In one embodiment, an amino acid sequence comprising a SARS-CoV-2 S protein, an immunogenic variant thereof, or an immunogenic fragment of the SARS-CoV-2 S protein or the immunogenic variant thereof is encoded by a coding sequence which is codon-optimized and/or the G/C content of which is increased compared to wild type coding sequence, wherein the codon-optimization and/or the increase in the G/C content preferably does not change the sequence of the encoded amino acid sequence.

In one embodiment,
(i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 979 to 1584 of SEQ ID NO: 2, 8 or 9, a nucleotide sequence having at least 99%, 98%, 97%, 96%, 95%, 90%, 85%, or 80% identity to the nucleotide sequence of nucleotides 979 to 1584 of SEQ ID NO: 2, 8 or 9, or a fragment of the nucleotide sequence of nucleotides 979 to 1584 of SEQ ID NO: 2, 8 or 9, or the nucleotide sequence having at least 99%, 98%, 97%, 96%, 95%, 90%, 85%, or 80% identity to the nucleotide sequence of nucleotides 979 to 1584 of SEQ ID NO: 2, 8 or 9; and/or
(ii) a SARS-CoV-2 S protein, an immunogenic variant thereof, or an immunogenic fragment of the SARS-CoV-2 S protein or the immunogenic variant thereof comprises the amino acid sequence of amino acids 327 to 528 of SEQ ID NO: 1, an amino acid sequence having at least 99%, 98%, 97%, 96%, 95%, 90%, 85%, or 80% identity to the amino acid sequence of amino acids 327 to 528 of SEQ ID NO: 1, or an immunogenic fragment of the amino acid sequence of amino acids 327 to 528 of SEQ ID NO: 1, or the amino acid sequence having at least 99%, 98%, 97%, 96%, 95%, 90%, 85%, or 80% identity to the amino acid sequence of amino acids 327 to 528 of SEQ ID NO: 1.

In one embodiment,
(i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 111 to 986 of SEQ ID NO: 30, a nucleotide sequence having at least 99%, 98%, 97%, 96%, 95%, 90%, 85%, or 80% identity to the nucleotide sequence of nucleotides 111 to 986 of SEQ ID NO: 30, or a fragment of the nucleotide sequence of nucleotides 111 to 986 of SEQ ID NO: 30, or the nucleotide sequence having at least 99%, 98%, 97%, 96%, 95%, 90%, 85%, or 80% identity to the nucleotide sequence of nucleotides 111 to 986 of SEQ ID NO: 30; and/or
(ii) a SARS-CoV-2 S protein, an immunogenic variant thereof, or an immunogenic fragment of the SARS-CoV-2 S protein or the immunogenic variant thereof comprises the amino acid sequence of amino acids 20 to 311 of SEQ ID NO: 29, an amino acid sequence having at least 99%, 98%, 97%, 96%, 95%, 90%, 85%, or 80% identity to the amino acid sequence of amino acids 20 to 311 of SEQ ID NO: 29, or an immunogenic fragment of the amino acid sequence of amino acids 20 to 311 of SEQ ID NO: 29, or the amino acid sequence having at least 99%, 98%, 97%, 96%, 95%, 90%, 85%, or 80% identity to the amino acid sequence of amino acids 20 to 311 of SEQ ID NO: 29.

In one embodiment,
(i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 49 to 2055 of SEQ ID NO: 2, 8 or 9, a nucleotide sequence having at least 99%, 98%, 97%, 96%, 95%, 90%, 85%, or 80% identity to the nucleotide sequence of nucleotides 49 to 2055 of SEQ ID NO: 2, 8 or 9, or a fragment of the nucleotide sequence of nucleotides 49 to 2055 of SEQ ID NO: 2, 8 or 9, or the nucleotide sequence having at least 99%, 98%, 97%, 96%, 95%, 90%, 85%, or 80% identity to the nucleotide sequence of nucleotides 49 to 2055 of SEQ ID NO: 2, 8 or 9; and/or
(ii) a SARS-CoV-2 S protein, an immunogenic variant thereof, or an immunogenic fragment of the SARS-CoV-2 S protein or the immunogenic variant thereof comprises the amino acid sequence of amino acids 17 to 685 of SEQ ID NO: 1, an amino acid sequence having at least 99%, 98%, 97%, 96%, 95%, 90%, 85%, or 80% identity to the amino acid sequence of amino acids 17 to 685 of SEQ ID NO: 1, or an immunogenic fragment of the amino acid sequence of amino acids 17 to 685 of SEQ ID NO: 1, or the amino acid sequence having at least 99%, 98%, 97%, 96%, 95%, 90%, 85%, or 80% identity to the amino acid sequence of amino acids 17 to 685 of SEQ ID NO: 1.

In one embodiment,
(i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 49 to 3819 of SEQ ID NO: 2, 8 or 9, a nucleotide sequence having at least 99%, 98%, 97%, 96%, 95%, 90%, 85%, or 80% identity to the nucleotide sequence of nucleotides 49 to 3819 of SEQ ID NO: 2, 8 or 9, or a fragment of the nucleotide sequence of nucleotides 49 to 3819 of SEQ ID NO: 2, 8 or 9, or the nucleotide sequence having at least 99%, 98%, 97%, 96%, 95%, 90%, 85%, or 80% identity to the nucleotide sequence of nucleotides 49 to 3819 of SEQ ID NO: 2, 8 or 9; and/or
(ii) a SARS-CoV-2 S protein, an immunogenic variant thereof, or an immunogenic fragment of the SARS-CoV-2 S protein or the immunogenic variant thereof comprises the amino acid sequence of amino acids 17 to 1273 of SEQ ID NO: 1 or 7, an amino acid sequence having at least 99%, 98%, 97%, 96%, 95%, 90%, 85%, or 80% identity to the amino acid sequence of amino acids 17 to 1273 of SEQ ID NO: 1 or 7, or an immunogenic fragment of the amino acid sequence of amino acids 17 to 1273 of SEQ ID NO: 1 or 7, or the amino acid sequence having at least 99%, 98%, 97%, 96%, 95%, 90%, 85%, or 80% identity to the amino acid sequence of amino acids 17 to 1273 of SEQ ID NO: 1 or 7.

In one embodiment, an amino acid sequence comprising a SARS-CoV-2 S protein, an immunogenic variant thereof, or an immunogenic fragment of the SARS-CoV-2 S protein or the immunogenic variant thereof comprises a secretory signal peptide.

In one embodiment, a secretory signal peptide is fused, preferably N-terminally, to a SARS-CoV-2 S protein, an immunogenic variant thereof, or an immunogenic fragment of the SARS-CoV-2 S protein or the immunogenic variant thereof.

In one embodiment,
(i) the RNA encoding the secretory signal peptide comprises the nucleotide sequence of nucleotides 1 to 48 of SEQ ID NO: 2, 8 or 9, a nucleotide sequence having at least 99%, 98%, 97%, 96%, 95%, 90%, 85%, or 80% identity to the nucleotide sequence of nucleotides 1 to 48 of SEQ ID NO: 2, 8 or 9, or a fragment of the nucleotide sequence of nucleotides 1 to 48 of SEQ ID NO: 2, 8 or 9, or the nucleotide sequence having at least 99%, 98%, 97%, 96%, 95%, 90%, 85%, or 80% identity to the nucleotide sequence of nucleotides 1 to 48 of SEQ ID NO: 2, 8 or 9; and/or
(ii) the secretory signal peptide comprises the amino acid sequence of amino acids 1 to 16 of SEQ ID NO: 1, an amino acid sequence having at least 99%, 98%, 97%, 96%, 95%, 90%, 85%, or 80% identity to the amino acid sequence of amino acids 1 to 16 of SEQ ID NO: 1, or a functional fragment of the amino acid sequence of amino acids 1 to 16 of SEQ ID NO: 1, or the amino acid sequence having at least 99%, 98%, 97%, 96%, 95%, 90%, 85%, or 80% identity to the amino acid sequence of amino acids 1 to 16 of SEQ ID NO: 1.

In one embodiment,
(i) the RNA encoding a SARS-CoV-2 S protein, an immunogenic variant thereof, or an immunogenic fragment of the SARS-CoV-2 S protein or the immunogenic variant thereof comprises the nucleotide sequence of SEQ ID NO: 6, a nucleotide sequence having at least 99%, 98%, 97%, 96%, 95%, 90%, 85%, or 80% identity to the nucleotide sequence of SEQ ID NO: 6, or a fragment of the nucleotide sequence of SEQ ID NO: 6, or the nucleotide sequence having at least 99%, 98%, 97%, 96%, 95%, 90%, 85%, or 80% identity to the nucleotide sequence of SEQ ID NO: 6; and/or
(ii) a SARS-CoV-2 S protein, an immunogenic variant thereof, or an immunogenic fragment of the SARS-CoV-2 S protein or the immunogenic variant thereof comprises the amino acid sequence of SEQ ID NO: 5, an amino acid sequence having at least 99%, 98%, 97%, 96%, 95%, 90%, 85%, or 80% identity to the amino acid sequence of SEQ ID NO: 5, or an immunogenic fragment of the amino acid sequence of SEQ ID NO: 5, or the amino acid sequence having at least 99%, 98%, 97%, 96%, 95%, 90%, 85%, or 80% identity to the amino acid sequence of SEQ ID NO: 5.

In one embodiment,
(i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 54 to 986 of SEQ ID NO: 30, a nucleotide sequence having at least 99%, 98%, 97%, 96%, 95%, 90%, 85%, or 80% identity to the nucleotide sequence of nucleotides 54 to 986 of SEQ ID NO: 30, or a fragment of the nucleotide sequence of nucleotides 54 to 986 of SEQ ID NO: 30, or the nucleotide sequence having at least 99%, 98%, 97%, 96%, 95%, 90%, 85%, or 80% identity to the nucleotide sequence of nucleotides 54 to 986 of SEQ ID NO: 30; and/or
(ii) a SARS-CoV-2 S protein, an immunogenic variant thereof, or an immunogenic fragment of the SARS-CoV-2 S protein or the immunogenic variant thereof comprises the amino acid sequence of amino acids 1 to 311 of SEQ ID NO: 29, an amino acid sequence having at least 99%, 98%, 97%, 96%, 95%, 90%, 85%, or 80% identity to the amino acid sequence of amino acids 1 to 311 of SEQ ID NO: 29, or an immunogenic fragment of the amino acid sequence of amino acids 1 to 311 of SEQ ID NO: 29, or the amino acid sequence having at least 99%, 98%, 97%, 96%, 95%, 90%, 85%, or 80% identity to the amino acid sequence of amino acids 1 to 311 of SEQ ID NO: 29.

In one embodiment, RNA is modified RNA, in particular stabilized mRNA. In one embodiment, RNA comprises a modified nucleoside in place of at least one uridine. In one embodiment, RNA comprises a modified nucleoside in place of each uridine. In one embodiment, a modified nucleoside is independently selected from pseudouridine (ψ), N1-methyl-pseudouridine (m1ψ), and 5-methyl-uridine (m5U).

In one embodiment, RNA comprises a modified nucleoside in place of uridine.

In one embodiment, the modified nucleoside is selected from pseudouridine (ψ), N1-methyl-pseudouridine (m1ψ), and 5-methyl-uridine (m5U).

In one embodiment, RNA comprises a cap.

In one embodiment, RNA encoding an amino acid sequence comprising a SARS-CoV-2 S protein, an immunogenic variant thereof, or an immunogenic fragment of the SARS-CoV-2 S protein or the immunogenic variant thereof comprises a 5' UTR comprising the nucleotide sequence of SEQ ID NO: 12, or a nucleotide sequence having at least 99%, 98%, 97%, 96%, 95%, 90%, 85%, or 80% identity to the nucleotide sequence of SEQ ID NO: 12.

In one embodiment, RNA encoding an amino acid sequence comprising a SARS-CoV-2 S protein, an immunogenic variant thereof, or an immunogenic fragment of the SARS-CoV-2 S protein or the immunogenic variant thereof comprises a 3' UTR comprising the nucleotide sequence of SEQ ID NO: 13, or a nucleotide sequence having at least 99%, 98%, 97%, 96%, 95%, 90%, 85%, or 80% identity to the nucleotide sequence of SEQ ID NO: 13.

In one embodiment, RNA encoding an amino acid sequence comprising a SARS-CoV-2 S protein, an immunogenic variant thereof, or an immunogenic fragment of the SARS-CoV-2 S protein or the immunogenic variant thereof comprises a poly-A sequence.

In one embodiment, a poly-A sequence comprises at least 100 nucleotides.

In one embodiment, a poly-A sequence comprises or consists of the nucleotide sequence of SEQ ID NO: 14.

In one embodiment, RNA is formulated as a liquid, a solid, or a combination thereof.

In one embodiment, RNA is administered by injection.

In one embodiment, RNA is administered by intramuscular administration.

In one embodiment, RNA is formulated as particles.

In one embodiment, the particles are lipid nanoparticles (LNP) or lipoplex (LPX) particles.

In one embodiment, LNPs comprise ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoyl-sn-glycero-3-phosphocholine, and cholesterol.

In one embodiment, RNA lipoplex particles are obtainable by mixing RNA with liposomes. In one embodiment, RNA lipoplex particles are obtainable by mixing RNA with lipids.

In one embodiment, RNA is formulated as colloid. In one embodiment, RNA is formulated as particles, forming the dispersed phase of a colloid. In one embodiment, 50% or more, 75% or more, or 85% or more of RNA are present in the dispersed phase. In one embodiment, RNA is formulated as particles comprising RNA and lipids. In one embodiment, particles are formed by exposing RNA, dissolved in an aqueous phase, with lipids, dissolved in an organic phase. In one embodiment, the organic phase comprises ethanol. In one embodiment, particles are formed by exposing RNA, dissolved in an aqueous phase, with lipids, dispersed in an aqueous phase. In one embodiment, the lipids dispersed in an aqueous phase form liposomes.

In one embodiment, RNA is mRNA or saRNA.

In one embodiment, a method disclosed herein is a method for vaccination against coronavirus.

In one embodiment, a method disclosed herein is a method for therapeutic or prophylactic treatment of a coronavirus infection.

In one embodiment, a subject is a human.

In one embodiment, the coronavirus is a betacoronavirus.

In one embodiment, the coronavirus is a sarbecovirus.

In one embodiment, the coronavirus is SARS-CoV-2.

In one embodiment of methods described herein, a composition described herein is administered to a subject.

In one aspect, the present disclosure relates to a composition or medical preparation described herein for use in a method described herein.

Among other things, the present disclosure teaches that a composition comprising a lipid nanoparticle encapsulated RNA encoding at least a portion (e.g., that is or comprises an epitope) of a SARS-CoV-2-encoded polypeptide (e.g., of a SARS-CoV-2-encoded S protein) can achieve detectable antibody titer against an epitope in serum within 7 days after administration to a population of adult human subjects according to a regimen that includes administration of at least one dose of the composition. Moreover, the present disclosure teaches persistence of such antibody titer. In some embodiments, such antibody titer is increased when a modified mRNA is used, as compared with titer achieved with a corresponding unmodified mRNA.

In some embodiments, a provided regimen includes at least one dose. In some embodiments, a provided regimen includes a first dose and at least one subsequent dose. In some embodiments, the first dose is the same amount as at least one subsequent dose. In some embodiments, the first dose is the same amount as all subsequent doses. In some embodiments, the first dose is a different amount as at least one subsequent dose. In some embodiments, the first dose is a different amount than all subsequent doses. In some embodiments, a provided regimen comprises two doses. In some embodiments, a provided regimen consists of two doses.

In particular embodiments, an immunogenic composition is formulated as a single-dose in a container, e.g., a vial. In some embodiments, an immunogenic composition is formulated as a multi-dose formulation in a vial. In some embodiments, the multi-dose formulation includes at least 2 doses per vial. In some embodiments, the multi-dose formulation includes a total of 2-20 doses per vial, such as, for example, 2, 3, 4, 5, 6, 7, 8, 9, 10, 11, or 12 doses per vial. In some embodiments, each dose in the vial is equal in volume. In some embodiments, a first dose is a different volume than a subsequent dose.

A "stable" multi-dose formulation exhibits no unacceptable levels of microbial growth, and substantially no or no breakdown or degradation of the active biological molecule component(s). As used herein, a "stable" immunogenic composition includes a formulation that remains capable of eliciting a desired immunologic response when administered to a subject.

In some embodiments, a multi-dose formulation remains stable for a specified time with multiple or repeated inoculations/insertions into the multi-dose container. For example, in some embodiments a multi-dose formulation may be stable for at least three days with up to ten usages, when contained within a multi-dose container. In some embodiments, multi-dose formulations remain stable with 2-20 inoculations/insertions.

In some embodiments, administration of a composition comprising a lipid nanoparticle encapsulated RNA (e.g., in some embodiments mRNA) encoding at least a portion (e.g., that is or comprises an epitope) of a SARS-CoV-2-encoded polypeptide (e.g., of a SARS-CoV-2-encoded S protein), e.g., according to a regimen as described herein, may result in lymphopenia in some subjects (e.g., in all subjects, in most subjects, in about 50% or fewer, in about 40% or fewer, in about 40% or fewer, in about 25% or fewer, in about 20% or fewer, in about 15% or fewer, in about 10% or fewer, in about 5% or fewer, etc). Among other things, the present disclosure teaches that such lymphopenia can resolve over time. For example, in some embodiments, lymphopenia resolves within about 14, about 10, about 9, about 8, about 7 days or less. In some embodiments, lymphopenia is Grade 3, Grade 2, or less.

Thus, among other things, the present disclosure provides compositions comprising a lipid nanoparticle encapsulated RNA (e.g., in some embodiments mRNA) encoding at least a portion (e.g., that is or comprises an epitope) of a SARS-CoV-2-encoded polypeptide (e.g., of a SARS-CoV-2-encoded S protein) that are characterized, when administered to a relevant population of adults, to display certain characteristics (e.g., achieve certain effects) as described herein. In some embodiments, provided compositions may have been prepared, stored, transported, characterized, and/or used under conditions where temperature does not exceed a particular threshold. Alternatively or additionally, in some embodiments, provided compositions may have been protected from light (e.g., from certain wavelengths) during some or all of their preparation, storage, transport, characterization, and/or use. In some embodiments, one or more features of provided compositions (e.g., RNA stability, as may be assessed, for example, by one or more of size, presence of particular moiety or modification, etc; lipid nanoparticle stability or aggregation, pH, etc) may be or have been assessed at one or more points during preparation, storage, transport, and/or use prior to administration.

Among other things, the present disclosure documents that certain provided compositions in which nucleotides within an RNA (e.g., in some embodiments mRNA) are not modified (e.g., are naturally-occurring A, U, C, G), and/or provided methods relating to such compositions, are characterized (e.g., when administered to a relevant population, which may in some embodiments be or comprise an adult population), by an intrinsic adjuvant effect. In some embodiments, such a composition and/or method can induce an antibody and/or a T cell response. In some embodiments, such a composition and/or method can induce a higher T cell response, as compared to conventional vaccines (e.g., non-RNA vaccines such as protein vaccines).

Alternatively or additionally, the present disclosure documents that provided compositions (e.g., compositions comprising a lipid nanoparticle encapsulated RNA encoding at least a portion (e.g., that is or comprises an epitope) of a SARS-CoV-2-encoded polypeptide (e.g., of a SARS-CoV-2-encoded S protein)) in which nucleotides within an RNA are modified, and/or provided methods relating to such compositions, are characterized (e.g., when administered to a relevant population, which may in some embodiments be or comprise an adult population), by absence of an intrinsic adjuvant effect, or by a reduced intrinsic adjuvant effect as compared with an otherwise comparable composition (or method) with unmodified results. Alternatively or additionally, in some embodiments, such compositions (or methods) are characterized in that they (e.g., when administered to a relevant population, which may in some embodiments be or comprise an adult population) induce an antibody response and/or a CD4+ T cell response. Still further alternatively or additionally, in some embodiments, such compositions (or methods) are characterized in that they (e.g., when administered to a relevant population, which may in some embodiments be or comprise an adult population) induce a higher CD4+ T cell response than that observed with an alternative vaccine format (e.g., a peptide vaccine). In some embodiments involving modified nucleotides, such modified nucleotides may be present, for example, in a 3' UTR sequence, an antigen-encoding sequence, and/or a 5'UTR sequence. In some embodiments, modified nucleotides are or include one or more modified uracil residues and/or one or more modified cytosine residues. Among other things, the present disclosure documents that provided (e.g., compositions comprising a lipid nanoparticle encapsulated RNA encoding at least a portion (e.g., that is or comprises an epitope) of a SARS-CoV-2-encoded polypeptide (e.g., of a SARS-CoV-2-encoded S protein)) and/or methods are characterized by (e.g., when administered to a relevant population, which may in some embodiments be or comprise an adult population) sustained expression of an encoded polypeptide (e.g., of a SARS-CoV-2-encoded protein [such as an S protein] or portion thereof, which portion, in some embodiments, may be or comprise an epitope thereof). For example, in some embodiments, such compositions and/or methods are characterized in that, when administered to a human, they achieve detectable polypeptide expression in a biological sample (e.g., serum) from such human and, in some embodiments, such expression persists for a period of time that is at least at least 36 hours or longer, including, e.g., at least 48 hours, at least 60 hours, at least 72 hours, at least 96 hours, at least 120 hours, at least 148 hours, or longer.

Those skilled in the art, reading the present disclosure, will appreciate that it describes various RNA constructs (e.g., in some embodiments mRNA constructs) encoding at least a portion (e.g., that is or comprises an epitope) of a SARS-CoV-2-encoded polypeptide (e.g., of a SARS-CoV-2-encoded S protein)). Such person of ordinary skill, reading the present disclosure, will particularly appreciate that it describes various RNA constructs (e.g., in some embodiments mRNA constructs) encoding at least a portion of a SARS-CoV-2 S protein, for example at least an RBD portion of a SARS-CoV-2 S protein. Still further, such a person of ordinary skill, reading the present disclosure, will appreciate that it describes particular characteristics and/or advantages of RNA constructs (e.g., in some embodiments mRNA constructs) encoding at least a portion (e.g., that is or comprises an epitope) of a SARS-CoV-2-encoded polypeptide (e.g., of a SARS-CoV-2-encoded S protein). In some embodiments, an RNA construct (e.g., in some embodiments, an mRNA construct) may encode at least one domain of a SARS-CoV-2 encoded polypeptide (e.g., one or more domains of a SARS-CoV-2 encoded polypeptide as described in WO 2021/159040, including, e.g., an N-terminal domain (NTD) of a SARS-CoV-2 Spike protein, a receptor binding domain (RBD) of a SARS- CoV-2 Spike protein, Heptapeptide repeat sequence 1 (HR1) of a SARS-CoV-2 Spike protein, Heptapeptide repeat sequence 2 (HR1) of a SARS-CoV-2 Spike protein, and/or combinations thereof). Among other things, the present disclosure particularly documents surprising and useful characteristics and/or advantages of certain RNA constructs (e.g., in some embodiments mRNA constructs) encoding a SARS-CoV-2 RBD portion and, in some embodiments, not encoding a full length SARS-CoV-2 S protein. Without wishing to be bound by any particular theory, the present disclosure suggests that provided RNA constructs (e.g., in some embodiments mRNA constructs) that encode less than a full-length SARS-CoV-2 S protein, and particularly those that encode at least an RBD portion of such SARS-CoV-2 S protein may be particularly useful and/or effective for use as or in an immunogenic composition (e.g., a vaccine), and/or for achieving immunological effects as described herein (e.g., generation of SARS-CoV-2 neutralizing antibodies, and/or T cell responses (e.g., CD4+ and/or CD8+ T cell responses)).

In some embodiments, the present disclosure provides an RNA (e.g., mRNA) comprising an open reading frame encoding a polypeptide that comprises a receptor-binding portion of a SARS-CoV-2 S protein, which RNA is suitable for intracellular expression of the polypeptide. In some embodiments, such an encoded polypeptide does not comprise the complete S protein. In some embodiments, an encoded polypeptide comprises the receptor binding domain (RBD), for example, as shown in SEQ ID NO: 5. In some embodiments, the encoded polypeptide comprises the peptide according to SEQ ID NO: 29 or 31. In some embodiments, such an RNA (e.g., mRNA) may be complexed by a (poly)cationic polymer, polyplex(es), protein(s) or peptide(s). In some embodiments, such an RNA may be formulated in a lipid nanoparticle (e.g., ones described herein). In some embodiments, such an RNA (e.g., mRNA) may be particularly useful and/or effective for use as or in an immunogenic composition (e.g., a vaccine), and/or for achieving immunological effects as described herein (e.g., generation of SARS-CoV-2 neutralizing antibodies, and/or T cell responses (e.g., CD4+ and/or CD8+ T cell responses)). In some embodiments, such an RNA (e.g., mRNA) may be useful for vaccinating humans (including, e.g., humans known to have been exposed and/or infected by SARS-CoV-2, and/or humans not known to have been exposed to SARS-CoV-2).

Those skilled in the art, reading the present disclosure, will further appreciate that it describes various mRNA constructs comprising a nucleic acid sequence that encodes a full-length SARS-CoV-2 Spike protein (e.g., including embodiments in which such encoded SARS-CoV-2 Spike protein may comprise at least one or more amino acid substitutions, e.g., proline substitutions as described herein, and/or embodiments in which the mRNA sequence is codon-optimized e.g., for mammalian, e.g., human, subjects). In some embodiments, such a full-length SARS-CoV-2 Spike protein may have an amino acid sequence that is or comprises that set forth in SEQ ID NO: 7. Still further, such a person of ordinary skill, reading the present disclosure, will appreciate, among other things, that it describes particular characteristics and/or advantages of certain mRNA constructs comprising a nucleic acid sequence that encodes a full-length SARS-CoV-2 Spike protein. Without wishing to be bound by any particular theory, the present disclosure suggests that provided mRNA constructs that encode a full-length SARS-CoV-2 S protein may be particularly useful and/or effective for use as or in an immunogenic composition (e.g., a vaccine) in particular subject population (e.g., particular age populations). For example, in some embodiments, such an mRNA composition may be particularly useful in younger (e.g., less than 25 years old, 20 years old, 18 years old, 15 years, 10 years old, or lower) subjects; alternatively or additionally, in some embodiments, such an mRNA composition may be particularly useful in elderly subjects (e.g., over 55 years old, 60 years old, 65 years old, 70 years old, 75 years old, 80 years old, 85 years old, or higher). In particular embodiments, an immunogenic composition comprising such an mRNA construct provided herein exhibits a minimal to modest increase (e.g., no more than 30% increase, no more than 20% increase, or no more than 10% increase, or lower) in dose level and/or dose number-dependent systemic reactogenicity (e.g., fever, fatigue, headache, chills, diarrhea, muscle pain, and/or joint pain, etc.) and/or local tolerability (e.g., pain, redness, and/orswelling, etc.), at least in some subjects (e.g., in some subject age groups); in some embodiments, such reactogenicity and/or local tolerability is observed particularly, in in younger age group (e.g., less than 25 years old, 20 years old, 18 years old or lower) subjects, and/or in older (e.g., elderly) age group (e.g., 65-85 years old). In some embodiments, provided mRNA constructs that encode a full-length SARS-CoV-2 S protein may be particularly useful and/or effective for use as or in an immunogenic composition (e.g., a vaccine) for inducing SARS-CoV-2 neutralizing antibody response level in a population of subjects that are at high risk for severe diseases associated with SARS-CoV-2 infection (e.g., an elderly population, for example, 65-85 year-old group). In some embodiments, a person of ordinary skill, reading the present disclosure, will appreciate, among other things, that provided mRNA constructs that encode a full-length SARS-CoV-2 S protein, which exhibit a favorable reactogenicity profile (e.g., as described herein) in younger and elderly age populations, may be particularly useful and/or effective for use as or in an immunogenic composition (e.g., a vaccine) for achieving immunological effects as described herein (e.g., generation of SARS-CoV-2 neutralizing antibodies, and/or T cell responses (e.g., CD4+ and/or CD8+ T cell responses)). In some embodiments, the present disclosure also suggests that provided mRNA constructs that encode a full-length SARS-CoV-2 S protein may be particularly effective to protect against SARS-CoV-2 infection, as characterized by earlier clearance of SARS-CoV-2 viral RNA in non-human mammalian subjects (e.g., rhesus macaques) that were immunized with immunogenic compositions comprising such mRNA constructs and subsequently challenged by SARS-CoV-2 strain. In some embodiments, such earlier clearance of SARS-CoV-2 viral RNA may be observed in the nose of non-human mammalian subjects (e.g., rhesus macaques) that were immunized with immunogenic compositions comprising such mRNA constructs and subsequently challenged by SARS-CoV-2 strain.

In some embodiments, the present disclosure provides an RNA (e.g., mRNA) comprising an open reading frame encoding a full-length SARS-CoV-2 S protein (e.g., a full-length SARS-CoV-2 S protein with one or more amino acid substitutions), which RNA is suitable for intracellular expression of the polypeptide. In some embodiments, the encoded polypeptide comprises the amino acid sequence of SEQ ID NO:7. In some embodiments, such an RNA (e.g., mRNA) may be complexed by a (poly)cationic polymer, polyplex(es), protein(s) or peptide(s). In some embodiments, such an RNA may be formulated in a lipid nanoparticle (e.g., ones described herein).

In some embodiments, an immunogenic composition provided herein may comprise a plurality of (e.g., at least two or more, including, e.g., at least three, at least four, at least five, at least six, at least seven, at least eight, at least nine, at least ten, etc.) immunoreactive epitopes of a SARS-CoV-2 polypeptide or variants thereof. In some such embodiments, such a plurality of immunoreactive epitopes may be encoded by a plurality of RNAs (e.g., mRNAs). In some such embodiments, such a plurality of immunoreactive epitopes may be encoded by a single RNA (e.g., mRNA). In some embodiments, nucleic acid sequences encoding a plurality of immunoreactive epitopes may be separated from each other in a single RNA (e.g., mRNA) by a linker (e.g., a peptide linker in some embodiments). Without wishing to be bound by any particular theory, in some embodiments, provided polyepitope immunogenic compositions (including, e.g., those that encode a full-length SARS-CoV-2 spike protein) may be particularly useful, when considering the genetic diversity of SARS-CoV-2 variants, to provide protection against numerous viral variants and/or may offer a greater opportunity for development of a diverse and/or otherwise robust (e.g., persistent, e.g., detectable about 5, 10, 15, 20, 25, 30, 35, 40, 45, 50, 55, 60 or more days after administration of one or more doses) neutralizing antibody and/or T cell response, and in particular a particularly robust T_{H}1-type T cell (e.g., CD4+ and/or CD8+ T cell) response.

In some embodiments, the present disclosure documents that provided compositions and/or methods are characterized by (e.g., when administered to a relevant population, which may in some embodiments be or comprise an adult population) in that they achieve one or more particular therapeutic outcomes (e.g., effective immune responses as described herein and/or detectable expression of encoded SARS-CoV-2 S protein or an immunogenic fragment thereof) with a single administration; in some such embodiments, an outcome may be assessed, for example, as compared to that observed in absence of RNA vaccines (e.g., mRNA vaccines) described herein. In some embodiments, a particular outcome may be achieved at a lower dose than required for one or more alternative strategies.

In some embodiments, the present disclosure provides an immunogenic composition comprising an isolated messenger ribonucleic acid (mRNA) polynucleotide, wherein the isolated mRNA polynucleotide comprises an open reading frame encoding a polypeptide that comprises a receptor-binding portion of a SARs-CoV-2 S protein, and wherein the isolated mRNA polynucleotide is formulated in at least one lipid nanoparticle. For example, in some embodiments, such a lipid nanoparticle may comprise a molar ratio of 20-60% ionizable cationic lipid, 5-25% non-cationic lipid (e.g., neutral lipid), 25-55% sterol or steroid, and 0.5-15% polymer-conjugated lipid (e.g., PEG-modified lipid). In some embodiments, a sterol or steroid included in a lipid nanoparticle may be orcomprise cholesterol. In some embodiments, a neutral lipid may be or comprise 1,2-distearoyl-sn-glycero-3-phosphocholine (DSPC). In some embodiments, a polymer-conjugated lipid may be or comprise PEG2000 DMG. In some embodiments, such an immunogenic composition may comprise a total lipid content of about 1 mg to 10 mg, or 3 mg to 8 mg, or 4 mg to 6 mg. In some embodiments, such an immunogenic composition may comprise a total lipid content of about 5 mg/mL -15 mg/mL or 7.5 mg/mL-12.5 mg/mL or 9-11 mg/mL. In some embodiments, such an isolated mRNA polynucleotide is provided in an effective amount to induce an immune response in a subject administered at least one dose of the immunogenic composition. In some embodiments, a polypeptide encoded by a provided isolated mRNA polynucleotide does not comprise the complete S protein. In some embodiments, such an isolated mRNA polynucleotide provided in an immunogenic composition is not self-replicating RNA.

In some embodiments, an immune response may comprise generation of a binding antibody titer against SARS-CoV-2 protein (including, e.g., a stabilized prefusion spike trimer in some embodiments) or a fragment thereof. In some embodiments, an immune response may comprise generation of a binding antibody titer against the receptor binding domain (RBD) of the SARS-CoV-2 spike protein. In some embodiments, a provided immunogenic composition has been established to achieve a detectable binding antibody titer after administration of a first dose, with seroconversion in at least 70% (including, e.g., at least 80%, at least 90%, at least 95% and up to 100°/) of a population of subjects receiving such a provided immunogenic composition, for example, by about 2 weeks.

In some embodiments, an immune response may comprise generation of a neutralizing antibody titer against SARS-CoV-2 protein (including, e.g., a stabilized prefusion spike trimer in some embodiments) or a fragment thereof. In some embodiments, an immune response may comprise generation of a neutralizing antibody titer against the receptor binding domain (RBD) of the SARS-CoV-2 spike protein. In some embodiments, a provided immunogenic composition has been established to achieve a neutralizing antibody titer in an appropriate system (e.g., in a human infected with SARS-CoV-2 and/or a population thereof, and/or in a model system therefor). For example, in some embodiments, such neutralizing antibody titer may have been demonstrated in one or more of a population of humans, a non-human primate model (e.g., rhesus macaques), and/or a mouse model.

In some embodiments, a neutralizing antibody titer is a titer that is (e.g., that has been established to be) sufficient to reduce viral infection of B cells relative to that observed for an appropriate control (e.g., an unvaccinated control subject, or a subject vaccinated with a live attenuated viral vaccine, an inactivated viral vaccine, or a protein subunit viral vaccine, or a combination thereof). In some such embodiments, such reduction is of at least 30%, 35%, 40%, 45%, 50%, 55%, 60%, 65%, 70%, 75%, 80%, 85%, 90%, 95%, or more.

In some embodiments, a neutralizing antibody titer is a titer that is (e.g., that has been established to be) sufficient to reduce the rate of asymptomatic viral infection relative to that observed for an appropriate control (e.g., an unvaccinated control subject, or a subject vaccinated with a live attenuated viral vaccine, an inactivated viral vaccine, or a protein subunit viral vaccine, or a combination thereof). In some such embodiments, such reduction is of at least 30%, 35%, 40%, 45%, 50%, 55%, 60%, 65%, 70%, 75%, 80%, 85%, 90%, 95%, or more. In some embodiments, such reduction can be characterized by assessment of SARS-CoV-2 N protein serology. Significant protection against asymptomatic infection was also confirmed by real life observations (see also: Dagan N. et al., N Engl J Med. 2021, doi: 10.1056/NEJMoa2101765. Epub ahead of print. PMID: 33626250)

In some embodiments, a neutralizing antibody titer is a titer that is (e.g., that has been established to be) sufficient to reduce or block fusion of virus with epithelial cells and/or B cells of a vaccinated subject relative to that observed for an appropriate control (e.g., an unvaccinated control subject, or a subject vaccinated with a live attenuated viral vaccine, an inactivated viral vaccine, or a protein subunit viral vaccine, or a combination thereof). In some such embodiments, such reduction is of at least 30%, 35%, 40%, 45%, 50%, 55%, 60%, 65%, 70%, 75%, 80%, 85%, 90%, 95%, or more.

In some embodiments, induction of a neutralizing antibody titer may be characterized by an elevation in the number of B cells, which in some embodiments may include plasma cells, class-switched IgG1- and IgG2-positive B cells, and/or germinal center B cells. In some embodiments, a provided immunogenic composition has been established to achieve such an elevation in the number of B cells in an appropriate system (e.g., in a human infected with SARS-CoV-2 and/or a population thereof, and/or in a model system therefor). For example, in some embodiments, such an elevation in the number of B cells may have been demonstrated in one or more of a population of humans, a non-human primate model (e.g., rhesus macaques), and/or a mouse model. In some embodiments, such an elevation in the number of B cells may have been demonstrated in draining lymph nodes and/or spleen of a mouse model after (e.g., at least 7 days, at least 8 days, at least 9 days, at least 10 days, at least 11 days, at least 12 days, at least 13 days, at least 14 days, after) immunization of such a mouse model with a provided immunogenic composition.

In some embodiments, induction of a neutralizing antibody titer may be characterized by a reduction in the number of circulating B cells in blood. In some embodiments, a provided immunogenic composition has been established to achieve such a reduction in the number of circulating B cells in blood of an appropriate system (e.g., in a human infected with SARS-CoV-2 and/or a population thereof, and/or in a model system therefor). For example, in some embodiments, such a reduction in the number of circulating B cells in blood may have been demonstrated in one or more of a population of humans, a non-human primate model (e.g., rhesus macaques), and/or a mouse model. In some embodiments, such a reduction in the number of circulating B cells in blood may have been demonstrated in a mouse model after (e.g., at least 4 days, at least 5 days, at least 6 days, at least 7 days, at least 8 days, at least 9 days, at least 10 days, after) immunization of such a mouse model with a provided immunogenic composition. Without wishing to be bound by theory, a reduction in circulating B cells in blood may be due to B cell homing to lymphoid compartments.

In some embodiments, an immune response induced by a provided immunogenic composition may comprise an elevation in the number of T cells. In some embodiments, such an elevation in the number of T cells may include an elevation in the number of T follicular helper (T_{FH}) cells, which in some embodiments may comprise one or more subsets with ICOS upregulation. One of skilled in the art will understand that proliferation of T_{FH} in germinal centres is integral for generation of an adaptive B-cell response, and also that in humans, T_{FH} occurring in the circulation after vaccination is typically correlated with a high frequency of antigen-specific antibodies. In some embodiments, a provided immunogenic composition has been established to achieve such an elevation in the number of T cells (e.g., T_{FH} cells) in an appropriate system (e.g., in a human infected with SARS-CoV-2 and/or a population thereof, and/or in a model system therefor). For example, in some embodiments, such an elevation in the number of T cells (e.g., T_{FH} cells) may have been demonstrated in one or more of a population of humans, a non-human primate model (e.g., rhesus macaques), and/or a mouse model. In some embodiments, such an elevation in the number of T cells (e.g., e.g., T_{FH} cells) may have been demonstrated in draining lymph nodes, spleen, and/or blood of a mouse model after (e.g., at least 4 days, at least 5 days, at least 6 days, at least 7 days, at least 8 days, at least 9 days, at least 10 days, at least 11 days, at least 12 days, at least 13 days, at least 14 days, after) immunization of such a mouse model with a provided immunogenic composition. In some embodiments, a protective response against SARS-CoV-2 induced by a provided immunogenic composition has been established in an appropriate model system for SARS-CoV-2. For example, in some embodiments, such a protective response may have been demonstrated in an animal model, e.g., a non-human primate model (e.g., rhesus macaques) and/or a mouse model. In some embodiments, a non-human primate (e.g., rhesus macaque) or a population thereof that has/have received at least one immunization with a provided immunogenic composition is/are challenged with SARS-CoV-2, e.g., through intranasal and/or intratracheal route. In some embodiments, such a challenge may be performed several weeks (e.g., 5-10 weeks) after at least one immunization (including, e.g., at least two immunizations) with a provided immunogenic composition. In some embodiments, such a challenge may be performed when a detectable level of a SARS-CoV-2 neutralizing titer (e.g., antibody response to SARS-CoV-2 spike protein and/or a fragment thereof, including, e.g., but not limited to a stabilized prefusion spike trimer, S-2P, and/or antibody response to receptor-binding portion of SARS-CoV-2) is achieved in non-human primate(s) (e.g., rhesus macaque(s)) that has received at least one immunization (including, e.g., at least two immunizations) with a provided immunogenic composition. In some embodiments, a protective response is characterized by absence of or reduction in detectable viral RNA in bronchoalveolar lavage (BAL) and/or nasal swabs of challenged non-human primate(s) (e.g., rhesus macaque(s)). In some embodiments, immunogenic compositions described herein may have been characterized in that a larger percent of challenged animals, for example, non-human primates in a population (e.g., rhesus macaques), that have received at least one immunization (including, e.g., at least two immunizations) with a provided immunogenic composition display absence of detectable RNA in their BAL and/or nasal swab, as compared to a population of non-immunized animals, for example, non-human primates (e.g., rhesus macaques). In some embodiments, immunogenic compositions described herein may have been characterized in that challenged animals, for example, non-human in a population (e.g., rhesus macaques), that have received at least one immunization (including, e.g., at least two immunizations) with a provided immunogenic composition may show clearance of viral RNA in nasal swab no later than 10 days, including, e.g., no later than 8 days, no later than 6 days, no later than 4 days, etc., as compared to a population of non-immunized animals, for example, non-human primates (e.g., rhesus macaques).

In some embodiments, immunogenic compositions described herein when administered to subjects in need thereof do not substantially increase the risk of vaccine-associated enhanced respiratory disease. In some embodiments, such vaccine-associated enhanced respiratory disease may be associated with antibody-dependent enhancement of replication and/or with vaccine antigens that induced antibodies with poor neutralizing activity and Th2-biased responses. In some embodiments, immunogenic compositions described herein when administered to subjects in need thereof do not substantially increase the risk of antibody-dependent enhancement of replication.

In some embodiments, a single dose of an RNA composition (e.g., mRNA formulated in lipid nanoparticles) can induce a therapeutic antibody response in less than 10 days of vaccination. In some embodiments, such a therapeutic antibody response may be characterized in that when such an RNA vaccine can induce production of about 10-100 ug/mL IgG measured at 10 days after vaccination at a dose of 0.1 to 10 ug or 0.2- 5 ug in an animal model. In some embodiments, such a therapeutic antibody response may be characterized in that such an RNA vaccine induces about 100-1000 ug/mL IgG measured at 20 days of vaccination at a dose of 0.1 to 10 ug or 0.2- 5 ug in an animal model. In some embodiments, a single dose may induce a pseudovirus-neutralization titer, as measured in an animal model, of 10-200 pVN50 titer 15 days after vaccination. In some embodiments, a single dose may induce a pseudovirus-neutralization titer, as measured in an animal model, of 50-500 pVN50 titer 15 days after vaccination.

In some embodiments, a single dose of an RNA composition (e.g., mRNA composition) can expand antigen-specific CD8 and/or CD4 T cell response by at least at 50% or more (including, e.g., at least 60%, at least 70%, at least 80%, at least 90%, at least 95%, or more), as compared to that observed in absence of such an RNA construct encoding a SARS-COV2 immunogenic protein or fragment thereof (e.g., spike protein and/or receptor binding domain). In some embodiments, a single dose of an RNA composition can expand antigen-specific CD8 and/or CD4 T cell response by at least at 1.5-fold or more (including, e.g., at least 2-fold, at least 3-fold, at least 5-fold, at least 10-fold, at least 50-fold, at least 100-fold, at least 500-fold, at least 1000-fold, or more), as compared to that observed in absence of such an RNA construct encoding a SARS-COV2 immunogenic protein or fragment thereof (e.g., spike protein and/or receptor binding domain).

In some embodiments, a regimen (e.g., a single dose of an mRNA composition) can expand T cells that exhibit a Th1 phenotype (e.g., as characterized by expression of IFN-gamma, IL-2, IL-4, and/or IL-5) by at least at 50% or more (including, e.g., at least 60%, at least 70%, at least 80%, at least 90%, at least 95%, or more), as compared to that observed in absence of such an mRNA construct encoding a SARS-COV2 immunogenic protein or fragment thereof (e.g., spike protein and/or receptor binding domain). In some embodiments, a regimen (e.g., a single dose of an mRNA composition) can expand T cells that exhibit a Th1 phenotype (e.g., as characterized by expression of IFN-gamma, IL-2, IL-4, and/or IL-5), for example by at least at 1.5-fold or more (including, e.g., at least 2-fold, at least 3-fold, at least 5-fold, at least 10-fold, at least 50-fold, at least 100-fold, at least 500-fold, at least 1000-fold, or more), as compared to that observed in absence of such an mRNA construct encoding a SARS-COV2 immunogenic protein or fragment thereof (e.g., spike protein and/or receptor binding domain). In some embodiments, a T-cell phenotype may be or comprise a Th1-dominant cytokine profile (e.g., as characterized by INF-gamma positive and/or IL-2 positive), and/or no by or biologically insignificant IL-4 secretion.

In some embodiments, a regimen as described herein (e.g., one or more doses of an mRNA composition) induces and/or achieves production of RBD-specific CD4+ T cells. Among other things, the present disclosure documents that mRNA compositions encoding an RBD-containing portion of a SARS-CoV-2 spike protein (e.g., and not encoding a full-length SARS-CoV-2 spike protein) may be particularly useful and/or effective in such induction and/or production of RBD-specific CD4+ T cells. In some embodiments, RBD-specific CD4+ T-cells induced by an mRNA composition described herein (e.g., by an mRNA composition that encodings an RBD-containing-portion of a SARS-CoV-2 spike protein and, in some embodiments not encoding a full-length SARS-CoV-2 spike protein) demonstrate a Th1-dominant cytokine profile (e.g., as characterized by INF-gamma positive and/or IL-2 positive), and/or by no or biologically insignificant IL-4 secretion.

In some embodiments, characterization of CD4+ and/or CD8+ T cell responses (e.g., described herein) in subjects receiving RNA compositions (e.g., as described herein) may be performed using ex vivo assays using PBMCs collected from the subjects.

In some embodiments, immunogenicity of RNA (e.g., mRNA) compositions described herein may be assessed by one of or more of the following serological immunongenicity assays: detection of IgG, IgM, and/or IgA to SARS-CoV-2 S protein present in blood samples of a subject receiving a provided RNA composition, and/or neutralization assays using SARS-CoV-2 pseudovirus and/or a wild-type SARS-CoV-2 virus.

In some embodiments, an RNA composition (e.g., as described herein) provide a relatively low adverse effect (e.g., Grade 1-Grade 2 pain, redness and/or swelling) within 7 days after vaccinations at a dose of 10 ug - 100 ug or 1 ug-50 ug. In some embodiments, RNA compositions (e.g., as described herein) provide a relatively low observation of systemic events (e.g., Grade 1-Grade 2 fever, fatigue, headache, chills, vomiting, diarrhea, muscle pain, joint pain, medication, and combinations thereof ) within 7 days after vaccinations at a dose of 10 ug - 100 ug.

In some embodiments, RNA (e.g., mRNA) compositions are characterized in that when administered to subjects at 10-100 ug dose or 1 ug-50 ug, IgG directed to a SARS-CoV2 immunogenic protein or fragment thereof (e.g., spike protein and/or receptor binding domain) may be produced at a level of 100-100,000 U/mL or 500-50,000 U/mL 21 days after vaccination.

In some embodiments, an RNA (e.g., mRNA) encodes a natively-folded trimeric receptor binding protein of SARS-CoV-2. In some embodiments, an RNA (e.g., mRNA) encodes a variant of such receptor binding protein such that the encoded variant binds to ACE2 at a Kd of 10 pM or lower, including, e.g., at a Kd of 9 pM, 8 pM, 7 pM, 6 pM, 5 pM, 4 pM, or lower. In some embodiments, an RNA (e.g., mRNA) encodes a variant of such receptor binding protein such that the encoded variant binds to ACE2 at a Kd of 5 pM. In some embodiments, an RNA (e.g., mRNA) encodes a trimeric receptor binding portion of SARS-CoV-2 that comprises an ACE2 receptor binding site. In some embodiments, an RNA (e.g., mRNA) comprises a coding sequence for a receptor-binding portion of SARS-CoV-2 and a trimerization domain (e.g., a natural trimerization domain (foldon) of T4 fibritin) such that the coding sequence directs expression of a trimeric protein that has an ACE2 receptor binding site and binds ACE2. In some embodiments, an RNA (e.g., mRNA) encodes a trimeric receptor binding portion of SARS-CoV-2 or a variant thereof such that its Kd is smaller than that for a monomeric receptor-binding domain (RBD) of SARS-CoV-2. For example, in some embodiments, an RNA (e.g., mRNA) encodes a trimeric receptor binding portion of SARS-CoV-2 or a variant thereof such that its Kd is at least 10-fold (including, e.g., at least 50-fold, at least 100-fold, at least 500-fold, at least 1000-fold, etc.) smaller than that for a RBD of SARS-CoV-2.

In some embodiments, a trimer receptor binding portion of SARS-CoV-2 encoded by an RNA (e.g., as described herein) may be determined to have a size of about 3-4 angstroms when it is complexed with ACE2 and B⁰AT1 neutral amino acid transporter in a closed conformation, as characterized by electron cryomicroscopy (cryoEM). In some embodiments, geometric mean SARS-CoV-2 neutralizing titer that characterizes and/or is achieved by an RNA composition or method as described herein can reach at least 1.5-fold, including, at least 2-fold, at least 2.5-fold, at least 3-fold, or higher, that of a COVID-19 convalescent human panel (e.g., a panel of sera from COVID-19 convalescing humans obtained 20-40 days after the onset of symptoms and at least 14 days after the start of asymptomatic convalescence.

In some embodiments, RNA compositions as provided herein may be characterized in that subjects who have been treated with such compositions (e.g., with at least one dose, at least two doses, etc) may show reduced and/or more transient presence of viral RNA in relevant site(s) (e.g., nose and/or lungs, etc, and/or any other tissue susceptible to infection) as compared with an appropriate control (e.g., an established expected level for a comparable subject or population not having been so treated and having been exposed to virus under reasonably comparable exposure conditions)

In some embodiments, the RBD antigen expressed by an mRNA construct (e.g., as described herein) can be modified by addition of a T4-fibritin-derived "foldon" trimerization domain, for example, to increase its immunogenicity.

In some embodiments, RNA (e.g., mRNA) compositions and/or methods described herein are characterized in that certain local reactions (e.g., pain, redness, and/or swelling, etc.) and/or systemic events (e.g., fever, fatigue, headache, etc.) may appear and/or peak at Day 2 after vaccination. In some embodiments, RNA (e.g., mRNA) compositions described herein are characterized in that certain local reactions (e.g., pain, redness, and/or swelling, etc.) and/or systemic events (e.g., fever, fatigue, headache, etc.) may resolve by Day 7 after vaccination. In some embodiments, RNA compositions (e.g., mRNA) and/or methods described herein are characterized in that no Grade 1 or greater change in routine clinical laboratory values or laboratory abnormalities are observed in subjects receiving RNA compositions (e.g., as described herein). Examples of such clinical laboratory assays may include lymphocyte count, hematological changes, etc.

In some embodiments, RNA (e.g., mRNA) compositions and/or methods described herein are characterized in that by 21 days after a first dose (e.g., 10-100 ug inclusive or 1 ug-50 ug inclusive), geometric mean concentrations (GMCs) of IgG directed to a SARS-CoV-2 S polypeptide or an immunogenic fragment thereof (e.g., RBD) may reach 200-3000 units/mL or 500-3000 units/mL or 500-2000 units/mL, compared to 602 units/mL for a panel of COVID-19 convalescent human sera. In some embodiments, RNA (e.g., mRNA) compositions described herein are characterized in that by 7 days after a second dose (e.g., 10-30 ug inclusive; or 1 ug-50 ug inclusive), geometric mean concentrations (GMCs) of IgG directed to a SARS-CoV-2 spike polypeptide or an immunogenic fragment thereof (e.g., RBD) may increase by at least 8-fold or higher, including, e.g., at least 9-fold, at least 10-fold, at least 15-fold, at least 20-fold, at least 25-fold, at least 30-fold, at least 35-fold, at least 40-fold, or higher. In some embodiments, RNA (e.g., mRNA) compositions described herein are characterized in that by 7 days after a second dose (e.g., 10-30 ug inclusive; or 1 ug-50 ug inclusive), geometric mean concentrations (GMCs) of IgG directed to a SARS-CoV-2 S polypeptide or an immunogenic fragment thereof (e.g., RBD) may increase to 1500 units/mL to 40,000 units/mL or 4000 units/mL to 40,000 units/mL. In some embodiments, antibody concentrations described herein can persist to at least 20 days or longer, including, e.g., at least 25 days, at least 30 days, at least 35 days, at least 40 days, at least 45 days, at least 50 days, after a first dose, or at least 10 days or longer, including, e.g., at least 15 days, at least 20 days, at least 25 days, or longer, after a second dose. In some embodiments, antibody concentrations can persist to 35 days after a first dose, or at least 14 days after a second dose.

In some embodiments, RNA (e.g., mRNA) compositions described herein are characterized in that when measured at 7 days after a second dose (e.g., 1-50 ug inclusive), GMC of IgG directed to a SARS-CoV-2 S polypeptide or an immunogenic fragment thereof (e.g., RBD) is at least 30% higher (including, e.g., at least 40% higher, at least 50% higher, at least 60%, higher, at least 70% higher, at least 80% higher, at least 90% higher, at least 95 % higher, as compared to antibody concentrations observed in a panel of COVID-19 convalescent human serum. In many embodiments, geometric mean concentration (GMC) of IgG described herein is GMCs of RBD-binding IgG.

In some embodiments, RNA (e.g., mRNA) compositions described herein are characterized in that when measured at 7 days after a second dose (e.g., 10-50 ug inclusive), GMC of IgG directed to a SARS-CoV-2 S polypeptide or an immunogenic fragment thereof (e.g., RBD) is at least 1.1-fold higher (including, e.g., at least 1.5-fold, at least 2-fold, at least 3-fold, at least 4-fold, at least 5-fold, at least 6-fold higher, at least 7-fold higher, at least 8-fold higher, at least 9-fold higher, at least 10-fold higher, at least 15-fold higher, at least 20-fold higher, at least 25-fold higher, at least 30-fold higher), as compared to antibody concentrations observed in a panel of COVID-19 convalescent human serum, In many embodiments, geometric mean concentration (GMC) of IgG described herein is GMCs of RBD-binding IgG.

In some embodiments, RNA (e.g., mRNA) compositions described herein are characterized in that when measured at 21 days after a second dose, GMC of IgG directed to a SARS-CoV-2 S polypeptide or an immunogenic fragment thereof (e.g., RBD) is at least 5-fold higher (including, e.g., at least 6-fold higher, at least 7-fold higher, at least 8-fold higher, at least 9-fold higher, at least 10-fold higher, at least 15-fold higher, at least 20-fold higher, at least 25-fold higher, at least 30-fold higher), as compared to antibody concentrations observed in a panel of COVID-19 convalescent human serum, In many embodiments, geometric mean concentration (GMC) of IgG described herein is GMCs of RBD-binding IgG.

In some embodiments, RNA (e.g., mRNA) compositions and/or methods described herein are characterized in that an increase (e.g., at least 30%, at least 40%, at least 50%, or more) in SARS-CoV-2 neutralizing geometric mean titers (GMTs) is observed 21 days after a first dose. In some embodiments, RNA (e.g., mRNA) compositions described herein are characterized in that a substantially greater serum neutralizing GMTs are achieved 7 days after subjects receive a second dose (e.g., 10 µg-30 µg inclusive), reaching 150-300, compared to 94 for a COVID-19 convalescent serum panel.

In some embodiments, RNA (e.g., mRNA) compositions and/or methods described herein are characterized in that 7 days after administration of the second dose, the protective efficacy is at least 60%, e.g., at least 70%, at least 80%, at least 90, or at least 95%. In one embodiment, RNA (e.g., mRNA) compositions and/or methods described herein are characterized in that 7 days after administration of the second dose, the protective efficacy is at least 70%. In one embodiment, RNA (e.g., mRNA) compositions and/or methods described herein are characterized in that 7 days after administration of the second dose, the protective efficacy is at least 80%. In one embodiment, RNA (e.g., mRNA) compositions and/or methods described herein are characterized in that 7 days after administration of the second dose, the protective efficacy is at least 90%. In one embodiment, RNA (e.g., mRNA) compositions and/or methods described herein are characterized in that 7 days after administration of the second dose, the protective efficacy is at least 95%.

In some embodiments, an RNA composition provided herein is characterized in that it induces an immune response against SARS-CoV-2 after at least 7 days after a dose (e.g., after a second dose). In some embodiments, an RNA composition provided herein is characterized in that it induces an immune response against SARS-CoV-2 in less than 14 days after a dose (e.g., after a second dose). In some embodiments, an RNA composition provided herein is characterized in that it induces an immune response against SARS-CoV-2 after at least 7 days after a vaccination regimen. In some embodiments, a vaccination regimen comprises a first dose and a second dose. In some embodiments, a first dose and a second dose are administered by at least 21 days apart. In some such embodiments, an immune response against SARS-CoV-2 is induced at least after 28 days after a first dose.

In some embodiments, RNA (e.g., mRNA) compositions and/or methods described herein are characterized in that geometric mean concentration (GMCs) of antibodies directed to a SARS-CoV-2 spike polypeptide or an immunogenic fragment thereof (e.g., RBD), as measured in serum from subjects receiving RNA (e.g., mRNA) compositions of the present disclosure (e.g., at a dose of 10-30 ug inclusive), is substantially higher than in a convalescent serum panel (e.g., as described herein). In some embodiments where a subject may receive a second dose (e.g., 21 days after 1 first dose), geometric mean concentration (GMCs) of antibodies directed to a SARS-CoV-2 spike polypeptide or an immunogenic fragment thereof (e.g., RBD), as measured in serum from the subject, may be 8.0-fold to 50-fold higher than a convalescent serum panel GMC. In some embodiments where a subject may receive a second dose (e.g., 21 days after 1 first dose), geometric mean concentration (GMCs) of antibodies directed to a SARS-CoV-2 spike polypeptide or an immunogenic fragment thereof (e.g., RBD), as measured in serum from the subject, may be at least 8.0-fold or higher, including, e.g., at least 10-fold, at least 20-fold, at least 30-fold, at least 40-fold, at least 50-fold, at least 60-fold or higher, as compared to a convalescent serum panel GMC.

In some embodiments, RNA (e.g., mRNA) compositions and/or methods described herein are characterized in that the SARS-CoV-2 neutralizing geometric mean titer, as measured at 28 days after a first dose or 7 days after a second dose, may be at least 1.5-fold or higher (including, e.g., at least 2-fold, at least 2.5-fold, at least 3-fold, at least 3.5-fold or higher), as compared to a neutralizing GMT of a convalescent serum panel.

In some embodiments, a regimen administered to a subject may be or comprise a single dose. In some embodiments, a regimen administered to a subject may comprise a plurality of doses (e.g., at least two doses, at least three doses, or more). In some embodiments, a regimen administered to a subject may comprise a first dose and a second dose, which are given at least 2 weeks apart, at least 3 weeks apart, at least 4 weeks apart, or more. In some embodiments, such doses may be at least 1 month, at least 2 months, at least 3 months, at least 4 months, at least 5 months, at least 6 months, at least 7 months, at least 8 months, at least 9 months, at least 10 months, at least 11 months, at least 12 months, or more apart. In some embodiments, doses may be administered days apart, such as 1, 2, 3, 4, 5, 6, 7, 8, 9 ,10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or more days apart. In some embodiments, doses may be administered about 1 to about 3 weeks apart, or about 1 to about 4 weeks apart, or about 1 to about 5 weeks apart, or about 1 to about 6 weeks apart, or about 1 to more than 6 weeks apart. In some embodiments, doses may be separated by a period of about 7 to about 60 days, such as for example about 14 to about 48 days, etc. In some embodiments, a minimum number of days between doses may be about 1, 2, 3, 4, 5, 6, 7, 8, 9, 10, 11, 12, 13, 14, 15, 16, 17, 18, 19, 20, 21 or more. In some embodiments, a maximum number of days between doses may be about 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, or fewer. In some embodiments, doses may be about 21 to about 28 days apart. In some embodiments, doses may be about 19 to about 42 days apart. In some embodiments, doses may be about 7 to about 28 days apart. In some embodiments, doses may be about 14 to about 24 days. In some embodiments, doses may be about 21 to about 42 days.

In some embodiments, particularly for compositions established to achieve elevated antibody and/or T-cell titres for a period of time longer than about 3 weeks - e.g., in some embodiments, a provided composition is established to achieve elevated antibody and/or T-cell titres (e.g., specific for a relevant portion of a SARS-CoV-2 spike protein) for a period of time longer than about 3 weeks; in some such embodiments, a dosing regimen may involve only a single dose, or may involve two or more doses, which may, in some embodiments, be separated from one another by a period of time that is longer than about 21 days or three weeks. For example, in some such embodiments, such period of time may be about 4 weeks, 5 weeks, 6 weeks 7 weeks, 8 weeks, 9 weeks, 10 weeks, 11 weeks, 12 weeks, 13 weeks, 14 weeks, 15 wees, 16 weeks, 17 weeks, 18 weeks, 19 weeks, 20 weeks or more, or about 1 month, 2 months, 3 months, 4 months, 5 months, 6 months, 7 months, 8 months, 9 months, 10, months, 11 months, 12 months or more, or in some embodiments about a year or more. In some embodiments, a first dose and a second dose (and/or other subsequent dose) may be administered by intramuscular injection. In some embodiments, a first dose and a second dose may be administered in the deltoid muscle. In some embodiments, a first dose and a second dose may be administered in the same arm. In some embodiments, an RNA (e.g., mRNA) composition described herein is administered (e.g., by intramuscular injection) as a series of two doses (e.g., 0.3 mL each) 21 days part. In some embodiments, each dose is about 30 ug. In some embodiments, each dose may be higher than 30 ug, e.g., about 40 ug, about 50 ug, about 60 ug. In some embodiments, each dose may be lower than 30 ug, e.g., about 20 ug, about 10 ug, about 5 ug, etc. In some embodiments, each dose is about 3 ug or lower, e.g., about 1 ug. In some such embodiments, an RNA (e.g., mRNA) composition described herein is administered to subjects of age 16 or older (including, e.g., 16-85 years). In some such embodiments, an RNA composition (e.g., mRNA) described herein is administered to subjects of age 18-55. In some such embodiments, an RNA composition (e.g., mRNA) described herein is administered to subjects of age 56-85. In some embodiments, an RNA (e.g., mRNA) composition described herein is administered (e.g., by intramuscular injection) as a single dose.

In some embodiments, RNA (e.g., mRNA) compositions and/or methods described herein are characterized in that RBD-specific IgG (e.g., polyclonal response) induced by such RNA compositions and/or methods exhibit a higher binding affinity to RBD, as compared to a reference human monoclonal antibody with SARS-CoV-2 RBD-binding affinity (e.g., CR3022 as described in J. ter Meulen et al., PLOS Med. 3, e237 (2006).)

In some embodiments, RNA (e.g., mRNA) compositions and/or methods described herein are characterized in that sera of vaccinated subjects display neutralizing activity across a panel (e.g., at least 10, at least 15, or more) of SARs-CoV-2 spike variants (e.g., across a panel of variants described herein). In some embodiments, such SARs-CoV-2 spike variants include mutations in RBD (e.g., but not limited to Q321L, V341I, A348T, N354D, S359N, V367F, K378R, R408I, Q409E, A435S, N439K, K458R, I472V, G476S, S477N, V483A, Y508H, H519P, etc., as compared to SEQ ID NO: 1), and/or mutations in spike protein (e.g., but not limited to D614G, etc., as compared to SEQ ID NO: 1). Those skilled in the art are aware of various spike variants, and/or resources that document them (e.g., the Table of mutating sites in Spike maintained by the COVID-19 Viral Genome Analysis Pipeline and found at https://cov.lanl.gov/components/sequence/COV/int_sites tbls.comp) (last accessed 24 Aug 2020), and, reading the present specification, will appreciate that RNA (e..g, mRNA) compositions and/or methods described herein can be characterized for their ability to induce sera in vaccinated subject that display neutralizing activity with respect to any or all of such variants and/or combinations thereof.

In particular embodiments, RNA (e.g., mRNA) compositions encoding RBD of a SARS-CoV-2 spike protein are characterized in that sera of vaccinated subjects display neutralizing activity across a panel (e.g., at least 10, at least 15, or more) of SARs-CoV-2 spike variants including RBD variants (e.g., but not limited to Q321L, V341I, A348T, N354D, S359N, V367F, K378R, R408I, Q409E, A435S, N439K, K458R, I472V, G476S, S477N, V483A, Y508H, H519P, etc., as compared to SEQ ID NO: 1) and spike protein variants (e.g., but not limited to D614G, as compared to SEQ ID NO: 1).

In particular embodiments, RNA (e.g., mRNA) compositions encoding a SARS-CoV-2 spike protein variant that includes two consecutive proline substitutions at amino acid positions 986 and 987, at the top of the central helix in the S2 subunit, are characterized in that sera of vaccinated subjects display neutralizing activity across a panel (e.g., at least 10, at least 15, or more) of SARs-CoV-2 spike variants including RBD variants (e.g., but not limited to Q321L, V341I, A348T, N354D, S359N, V367F, K378R, R408I, Q409E, A435S, N439K, K458R, I472V, G476S, S477N, V483A, Y508H, H519P, etc., as compared to SEQ ID NO: 1) and spike protein variants (e.g., but not limited to D614G, as compared to SEQ ID NO: 1). For example, in some embodiments, an RNA (e.g., mRNA) composition encoding SEQ ID NO: 7 (S P2) elicits an immune response against any one of a SARs-CoV-2 spike variant including RBD variants (e.g., but not limited to Q321L, V341I, A348T, N354D, S359N, V367F, K378R, R408I, Q409E, A435S, N439K, K458R, I472V, G476S, S477N, V483A, Y508H, H519P, etc., as compared to SEQ ID NO: 1) and spike protein variants (e.g., but not limited to D614G, as compared to SEQ ID NO: 1). In some embodiments, RNA (e.g., mRNA) compositions and/or methods described herein are characterized in that sera of vaccinated subjects display neutralizing activity against one or more SARs-CoV-2 spike variants including a mutation at position 501 in spike protein as compared to SEQ ID NO: 1. In some embodiments, RNA (e.g., mRNA) compositions and/or methods described herein are characterized in that sera of vaccinated subjects display neutralizing activity against one or more SARs-CoV-2 spike variants including a N501Y mutation in spike protein as compared to SEQ ID NO: 1.

Said one or more SARs-CoV-2 spike variants including a mutation at position 501 in spike protein as compared to SEQ ID NO: 1 or said one or more SARs-CoV-2 spike variants including a N501Y mutation in spike protein as compared to SEQ ID NO: 1 may include one or more further mutations as compared to SEQ ID NO: 1 (e.g., but not limited to H69/V70 deletion, Y144 deletion, A570D, D614G, P681H, T716I, S982A, D1118H, D80A, D215G, E484K, A701V, L18F, R246I, K417N, L242/A243/L244 deletion etc., as compared to SEQ ID NO: 1).

In particular embodiments, RNA (e.g., mRNA) compositions and/or methods described herein are characterized in that sera of vaccinated subjects display neutralizing activity against SARs-CoV-2 spike variant "Variant of Concern 202012/01" (VOC-202012/01; also known as lineage B.1.1.7). The variant had previously been named the first Variant Under Investigation in December 2020 (VUI - 202012/01) by Public Health England, but was reclassified to a Variant of Concern (VOC-202012/01). VOC-202012/01 is a variant of SARS-CoV-2 which was first detected in October 2020 during the COVID-19 pandemic in the United Kingdom from a sample taken the previous month, and it quickly began to spread by mid-December. It is correlated with a significant increase in the rate of COVID-19 infection in United Kingdom; this increase is thought to be at least partly because of change N501Y inside the spike glycoprotein's receptor-binding domain, which is needed for binding to ACE2 in human cells. The VOC-202012/01 variant is defined by 23 mutations: 13 non-synonymous mutations, 4 deletions, and 6 synonymous mutations (i.e., there are 17 mutations that change proteins and six that do not). The spike protein changes in VOC 202012/01 include deletion 69-70, deletion 144, N501Y, A570D, D614G, P681H, T716I, S982A, and D1118H. One of the most important changes in VOC-202012/01 seems to be N501Y, a change from asparagine (N) to tyrosine (Y) at amino-acid site 501. This mutation alone or in combination with the deletion at positions 69/70 in the N terminal domain (NTD) may enhance the transmissibility of the virus.

In particular embodiments, RNA (e.g., mRNA) compositions and/or methods described herein are characterized in that sera of vaccinated subjects display neutralizing activity against SARs-CoV-2 spike variant including the following mutations: deletion 69-70, deletion 144, N501Y, A570D, D614G, P681H, T716I, S982A, and D1118H as compared to SEQ ID NO: 1.

In particular embodiments, RNA (e.g., mRNA) compositions and/or methods described herein are characterized in that sera of vaccinated subjects display neutralizing activity against SARs-CoV-2 spike variant "501.V2". This variant was first observed in samples from October 2020, and since then more than 300 cases with the 501.V2 variant have been confirmed by whole genome sequencing (WGS) in South Africa, where in December 2020 it was the dominant form of the virus. Preliminary results indicate that this variant may have an increased transmissibility. The 501.V2 variant is defined by multiple spike protein changes including: D80A, D215G, E484K, N501Y and A701V, and more recently collected viruses have additional changes: L18F, R246I, K417N, and deletion 242-244.

In particular embodiments, RNA (e.g., mRNA) compositions and/or methods described herein are characterized in that sera of vaccinated subjects display neutralizing activity against SARs-CoV-2 spike variant including the following mutations: D80A, D215G, E484K, N501Y and A701V as compared to SEQ ID NO: 1, and optionally: L18F, R246I, K417N, and deletion 242-244 as compared to SEQ ID NO: 1. Said SARs-CoV-2 spike variant may also include a D614G mutation as compared to SEQ ID NO: 1.

In some embodiments, RNA (e.g., mRNA) compositions and/or methods described herein are characterized in that sera of vaccinated subjects display neutralizing activity against one or more SARs-CoV-2 spike variants including a H69/V70 deletion in spike protein as compared to SEQ ID NO: 1.

In some embodiments, one or more SARs-CoV-2 spike variants including a H69/V70 deletion in spike protein as compared to SEQ ID NO: 1 may include one or more further mutations as compared to SEQ ID NO: 1 (e.g., but not limited to Y144 deletion, N501Y, A570D, D614G, P681H, T716I, S982A, D1118H, D80A, D215G, E484K, A701V, L18F, R246I, K417N, L242/A243/L244 deletion, Y453F, I692V, S1147L, M1229I etc., as compared to SEQ ID NO: 1), In particular embodiments, RNA (e.g., mRNA) compositions and/or methods described herein are characterized in that sera of vaccinated subjects display neutralizing activity against SARs-CoV-2 spike variant "Variant of Concern 202012/01" (VOC-202012/01; also known as lineage B.1.1.7).

In particular embodiments, RNA (e.g., mRNA) compositions and/or methods described herein are characterized in that sera of vaccinated subjects display neutralizing activity against SARs-CoV-2 spike variant including the following mutations: deletion 69-70, deletion 144, N501Y, A570D, D614G, P681H, T716I, S982A, and D1118H as compared to SEQ ID NO: 1.

In particular embodiments, RNA (e.g., mRNA) compositions and/or methods described herein are characterized in that sera of vaccinated subjects display neutralizing activity against SARs-CoV-2 spike variant "Cluster 5", also referred to as ΔFVI-spike by the Danish State Serum Institute (SSI). It was discovered in North Jutland, Denmark, and is believed to have been spread from minks to humans via mink farms. In cluster 5, several different mutations in the spike protein of the virus have been confirmed. The specific mutations include 69-70deltaHV (a deletion of the histidine and valine residues at the 69th and 70th position in the protein), Y453F (a change from tyrosine to phenylalanine at position 453), I692V (isoleucine to valine at position 692), M1229I (methionine to isoleucine at position 1229), and optionally S1147L (serine to leucine at position 1147).

In particular embodiments, RNA (e.g., mRNA) compositions and/or methods described herein are characterized in that sera of vaccinated subjects display neutralizing activity against SARs-CoV-2 spike variant including the following mutations: deletion 69-70, Y453F, I692V, M1229I, and optionally S1147L, as compared to SEQ ID NO: 1.

In some embodiments, RNA (e.g., mRNA) compositions and/or methods described herein are characterized in that sera of vaccinated subjects display neutralizing activity against one or more SARs-CoV-2 spike variants including a mutation at position 614 in spike protein as compared to SEQ ID NO: 1. In some embodiments, RNA (e.g., mRNA) compositions and/or methods described herein are characterized in that sera of vaccinated subjects display neutralizing activity against one or more SARs-CoV-2 spike variants including a D614G mutation in spike protein as compared to SEQ ID NO: 1.

In some embodiments, one or more SARs-CoV-2 spike variants including a mutation at position 614 in spike protein as compared to SEQ ID NO: 1 or said one or more SARs-CoV-2 spike variants including a D614G mutation in spike protein as compared to SEQ ID NO: 1 may include one or more further mutations as compared to SEQ ID NO: 1 (e.g., but not limited to H69/V70 deletion, Y144 deletion, N501Y, A570D, P681H, T716I, S982A, D1118H, D80A, D215G, E484K, A701V, L18F, R246I, K417N, L242/A243/L244 deletion, Y453F, I692V, S1147L, M1229I etc., as compared to SEQ ID NO: 1).

In particular embodiments, RNA (e.g., mRNA) compositions and/or methods described herein are characterized in that sera of vaccinated subjects display neutralizing activity against SARs-CoV-2 spike variant "Variant of Concern 202012/01" (VOC-202012/01; also known as lineage B.1.1.7).

In particular embodiments, RNA (e.g., mRNA) compositions and/or methods described herein are characterized in that sera of vaccinated subjects display neutralizing activity against SARs-CoV-2 spike variant including the following mutations: deletion 69-70, deletion 144, N501Y, A570D, D614G, P681H, T716I, S982A, and D1118H as compared to SEQ ID NO: 1.

In particular embodiments, RNA (e.g., mRNA) compositions and/or methods described herein are characterized in that sera of vaccinated subjects display neutralizing activity against SARs-CoV-2 spike variant including the following mutations: D80A, D215G, E484K, N501Y, A701V, and D614G as compared to SEQ ID NO: 1, and optionally: L18F, R246I, K417N, and deletion 242-244 as compared to SEQ ID NO: 1.

In some embodiments, RNA (e.g., mRNA) compositions and/or methods described herein are characterized in that sera of vaccinated subjects display neutralizing activity against one or more SARs-CoV-2 spike variants including a mutation at positions 501 and 614 in spike protein as compared to SEQ ID NO: 1. In some embodiments, RNA (e.g., mRNA) compositions and/or methods described herein are characterized in that sera of vaccinated subjects display neutralizing activity against one or more SARs-CoV-2 spike variants including a N501Y mutation and a D614G mutation in spike protein as compared to SEQ ID NO: 1.

In some embodiments, one or more SARS-CoV-2 spike variants including a mutation at positions 501 and 614 in spike protein as compared to SEQ ID NO: 1 or said one or more SARs-CoV-2 spike variants including a N501Y mutation and a D614G mutation in spike protein as compared to SEQ ID NO: 1 may include one or more further mutations as compared to SEQ ID NO: 1 (e.g., but not limited to H69/V70 deletion, Y144 deletion, A570D, P681H, T716I, S982A, D1118H, D80A, D215G, E484K, A701V, L18F, R246I, K417N, L242/A243/L244 deletion, Y453F, 1692V, S1147L, M1229I etc., as compared to SEQ ID NO: 1).

In particular embodiments, RNA (e.g., mRNA) compositions and/or methods described herein are characterized in that sera of vaccinated subjects display neutralizing activity against SARs-CoV-2 spike variant "Variant of Concern 202012/01" (VOC-202012/01; also known as lineage B.1.1.7).

In particular embodiments, RNA (e.g., mRNA) compositions and/or methods described herein are characterized in that sera of vaccinated subjects display neutralizing activity against SARs-CoV-2 spike variant including the following mutations: deletion 69-70, deletion 144, N501Y, A570D, D614G, P681H, T716I, S982A, and D1118H as compared to SEQ ID NO: 1.

In particular embodiments, RNA (e.g., mRNA) compositions and/or methods described herein are characterized in that sera of vaccinated subjects display neutralizing activity against SARs-CoV-2 spike variant including the following mutations: D80A, D215G, E484K, N501Y, A701V, and D614G as compared to SEQ ID NO: 1, and optionally: L18F, R246I, K417N, and deletion 242-244 as compared to SEQ ID NO: 1.

In some embodiments, RNA (e.g., mRNA) compositions and/or methods described herein are characterized in that sera of vaccinated subjects display neutralizing activity against one or more SARs-CoV-2 spike variants including a mutation at position 484 in spike protein as compared to SEQ ID NO: 1. In some embodiments, RNA (e.g., mRNA) compositions and/or methods described herein are characterized in that sera of vaccinated subjects display neutralizing activity against one or more SARs-CoV-2 spike variants including a E484K mutation in spike protein as compared to SEQ ID NO: 1.

In some embodiments, one or more SARs-CoV-2 spike variants including a mutation at position 484 in spike protein as compared to SEQ ID NO: 1 or said one or more SARs-CoV-2 spike variants including a E484K mutation in spike protein as compared to SEQ ID NO: 1 may include one or more further mutations as compared to SEQ ID NO: 1 (e.g., but not limited to H69/V70 deletion, Y144 deletion, N501Y, A570D, D614G, P681H, T7161, S982A, D1118H, D80A, D215G, A701V, L18F, R246I, K417N, L242/A243/L244 deletion, Y453F, I692V, S1147L, M1229I, T20N, P26S, D138Y, R190S, K417T, H655Y, T1027I, V1176F etc., as compared to SEQ ID NO: 1).

In particular embodiments, RNA (e.g., mRNA) compositions and/or methods described herein are characterized in that sera of vaccinated subjects display neutralizing activity against SARs-CoV-2 spike variant "501.V2".

In particular embodiments, RNA (e.g., mRNA) compositions and/or methods described herein are characterized in that sera of vaccinated subjects display neutralizing activity against SARs-CoV-2 spike variant including the following mutations: D80A, D215G, E484K, N501Y, and A701V, as compared to SEQ ID NO: 1, and optionally: L18F, R246I, K417N, and deletion 242-244 as compared to SEQ ID NO: 1. Said SARs-CoV-2 spike variant may also include a D614G mutation as compared to SEQ ID NO: 1.

Lineage B.1.1.248, known as the Brazil(ian) variant, is one of the variants of SARS-CoV-2 which has been named P.1 lineage and has 17 unique amino acid changes, 10 of which in its spike protein, including N501Y and E484K. B.1.1.248 originated from B.1.1.28. E484K is present in both B.1.1.28 and B.1.1.248. B.1.1.248 has a number of S-protein polymorphisms [L18F, T20N, P26S, D138Y, R190S, K417T, E484K, N501Y, H655Y, T1027I, V1176F] and is similar in certain key RBD positions (K417, E484, N501) to variant described from South Africa.

In particular embodiments, RNA (e.g., mRNA) compositions and/or methods described herein are characterized in that sera of vaccinated subjects display neutralizing activity against SARs-CoV-2 spike variant "B.1.1.28".

In particular embodiments, RNA (e.g., mRNA) compositions and/or methods described herein are characterized in that sera of vaccinated subjects display neutralizing activity against SARs-CoV-2 spike variant "B.1.1.248".

In particular embodiments, RNA (e.g., mRNA) compositions and/or methods described herein are characterized in that sera of vaccinated subjects display neutralizing activity against SARs-CoV-2 spike variant including the following mutations: L18F, T20N, P26S, D138Y, R190S, K417T, E484K, N501Y, H655Y, T1027I, and V1176F as compared to SEQ ID NO: 1.

In some embodiments, RNA (e.g., mRNA) compositions and/or methods described herein are characterized in that sera of vaccinated subjects display neutralizing activity against one or more SARs-CoV-2 spike variants including a mutation at positions 501 and 484 in spike protein as compared to SEQ ID NO: 1. In some embodiments, RNA (e.g., mRNA) compositions and/or methods described herein are characterized in that sera of vaccinated subjects display neutralizing activity against one or more SARs-CoV-2 spike variants including a N501Y mutation and a E484K mutation in spike protein as compared to SEQ ID NO: 1.

In some embodiments, one or more SARs-CoV-2 spike variants including a mutation at positions 501 and 484 in spike protein as compared to SEQ ID NO: 1 or said one or more SARs-CoV-2 spike variants including a N501Y mutation and a E484K mutation in spike protein as compared to SEQ ID NO: 1 may include one or more further mutations as compared to SEQ ID NO: 1 (e.g., but not limited to H69/V70 deletion, Y144 deletion, A570D, D614G, P681H, T716I, S982A, D1118H, D80A, D215G, A701V, L18F, R246I, K417N, L242/A243/L244 deletion, Y453F, I692V, S1147L, M1229I, T20N, P26S, D138Y, R190S, K417T, H655Y, T1027I, V1176F etc., as compared to SEQ ID NO: 1).

In particular embodiments, RNA (e.g., mRNA) compositions and/or methods described herein are characterized in that sera of vaccinated subjects display neutralizing activity against SARs-CoV-2 spike variant "501.V2".

In particular embodiments, RNA (e.g., mRNA) compositions and/or methods described herein are characterized in that sera of vaccinated subjects display neutralizing activity against SARs-CoV-2 spike variant including the following mutations: D80A, D215G, E484K, N501Y and A701V as compared to SEQ ID NO: 1, and optionally: L18F, R246I, K417N, and deletion 242-244 as compared to SEQ ID NO: 1. Said SARs-CoV-2 spike variant may also include a D614G mutation as compared to SEQ ID NO: 1.

In particular embodiments, RNA (e.g., mRNA) compositions and/or methods described herein are characterized in that sera of vaccinated subjects display neutralizing activity against SARs-CoV-2 spike variant "B.1.1.248".

In particular embodiments, RNA (e.g., mRNA) compositions and/or methods described herein are characterized in that sera of vaccinated subjects display neutralizing activity against SARs-CoV-2 spike variant including the following mutations: L18F, T20N, P26S, D138Y, R190S, K417T, E484K, N501Y, H655Y, T1027I, and V1176F as compared to SEQ ID NO: 1.

In some embodiments, RNA (e.g., mRNA) compositions and/or methods described herein are characterized in that sera of vaccinated subjects display neutralizing activity against one or more SARs-CoV-2 spike variants including a mutation at positions 501, 484 and 614 in spike protein as compared to SEQ ID NO: 1. In some embodiments, RNA (e.g., mRNA) compositions and/or methods described herein are characterized in that sera of vaccinated subjects display neutralizing activity against one or more SARs-CoV-2 spike variants including a N501Y mutation, a E484K mutation and a D614G mutation in spike protein as compared to SEQ ID NO: 1.

In some embodiments, one or more SARs-CoV-2 spike variants including a mutation at positions 501, 484 and 614 in spike protein as compared to SEQ ID NO: 1 or said one or more SARs-CoV-2 spike variants including a N501Y mutation, a E484K mutation and a D614G mutation in spike protein as compared to SEQ ID NO: 1 may include one or more further mutations as compared to SEQ ID NO: 1 (e.g., but not limited to H69/V70 deletion, Y144 deletion, A570D, P681H, T716I, S982A, D1118H, D80A, D215G, A701V, L18F, R246I, K417N, L242/A243/L244 deletion, Y453F, I692V, S1147L, M1229I, T20N, P26S, D138Y, R190S, K417T, H655Y, T1027I, V1176F etc., as compared to SEQ ID NO: 1).

In particular embodiments, RNA (e.g., mRNA) compositions and/or methods described herein are characterized in that sera of vaccinated subjects display neutralizing activity against SARs-CoV-2 spike variant including the following mutations: D80A, D215G, E484K, N501Y, A701V, and D614G as compared to SEQ ID NO: 1, and optionally: L18F, R246I, K417N, and deletion 242-244 as compared to SEQ ID NO: 1.

In some embodiments, RNA (e.g., mRNA) compositions and/or methods described herein are characterized in that sera of vaccinated subjects display neutralizing activity against one or more SARs-CoV-2 spike variants including a L242/A243/L244 deletion in spike protein as compared to SEQ ID NO: 1.

In some embodiments, one or more SARs-CoV-2 spike variants including a L242/A243/L244 deletion in spike protein as compared to SEQ ID NO: 1 may include one or more further mutations as compared to SEQ ID NO: 1 (e.g., but not limited to H69/V70 deletion, Y144 deletion, N501Y, A570D, D614G, P681H, T716I,S982A, D1118H, D80A, D215G, E484K, A701V, L18F, R246I, K417N, Y453F, I692V, S1147L, M1229I, T20N, P26S, D138Y, R190S, K417T, H655Y, T1027I, V1176F etc., as compared to SEQ ID NO: 1).

In particular embodiments, RNA (e.g., mRNA) compositions and/or methods described herein are characterized in that sera of vaccinated subjects display neutralizing activity against SARs-CoV-2 spike variant "501.V2".

In particular embodiments, RNA (e.g., mRNA) compositions and/or methods described herein are characterized in that sera of vaccinated subjects display neutralizing activity against SARs-CoV-2 spike variant including the following mutations: D80A, D215G, E484K, N501Y, A701V and deletion 242-244 as compared to SEQ ID NO: 1, and optionally: L18F, R246I, and K417N, as compared to SEQ ID NO: 1. Said SARs-CoV-2 spike variant may also include a D614G mutation as compared to SEQ ID NO: 1.

In some embodiments, RNA (e.g., mRNA) compositions and/or methods described herein are characterized in that sera of vaccinated subjects display neutralizing activity against one or more SARs-CoV-2 spike variants including a mutation at position 417 in spike protein as compared to SEQ ID NO: 1. In some embodiments, RNA (e.g., mRNA) compositions and/or methods described herein are characterized in that sera of vaccinated subjects display neutralizing activity against one or more SARs-CoV-2 spike variants including a K417N or K417T mutation in spike protein as compared to SEQ ID NO: 1.

In some embodiments, one or more SARs-CoV-2 spike variants including a mutation at position 417 in spike protein as compared to SEQ ID NO: 1 or said one or more SARs-CoV-2 spike variants including a K417N or K417T mutation in spike protein as compared to SEQ ID NO: 1 may include one or more further mutations as compared to SEQ ID NO: 1 (e.g., but not limited to H69/V70 deletion, Y144 deletion, N501Y, A570D, D614G, P681H, T716I, S982A, D1118H, D80A, D215G, E484K, A701V, L18F, R246I, L242/A243/L244 deletion, Y453F, I692V, S1147L, M1229I, T20N, P26S, D138Y, R190S, H655Y, T1027I, V1176F etc., as compared to SEQ ID NO: 1).

In particular embodiments, RNA (e.g., mRNA) compositions and/or methods described herein are characterized in that sera of vaccinated subjects display neutralizing activity against SARs-CoV-2 spike variant "501.V2".

In particular embodiments, RNA (e.g., mRNA) compositions and/or methods described herein are characterized in that sera of vaccinated subjects display neutralizing activity against SARs-CoV-2 spike variant including the following mutations: D80A, D215G, E484K, N501Y, A701V and K417N,, as compared to SEQ ID NO: 1, and optionally: L18F, R246I, and deletion 242-244 as compared to SEQ ID NO: 1. Said SARs-CoV-2 spike variant may also include a D614G mutation as compared to SEQ ID NO: 1.

In particular embodiments, RNA (e.g., mRNA) compositions and/or methods described herein are characterized in that sera of vaccinated subjects display neutralizing activity against SARs-CoV-2 spike variant "B.1.1.248".

In particular embodiments, RNA (e.g., mRNA) compositions and/or methods described herein are characterized in that sera of vaccinated subjects display neutralizing activity against SARs-CoV-2 spike variant including the following mutations: L18F, T20N, P26S, D138Y, R190S, K417T, E484K, N501Y, H655Y, T1027I, and V1176F as compared to SEQ ID NO: 1.

In some embodiments, RNA (e.g., mRNA) compositions and/or methods described herein are characterized in that sera of vaccinated subjects display neutralizing activity against one or more SARs-CoV-2 spike variants including a mutation at positions 417 and 484 and/or 501 in spike protein as compared to SEQ ID NO: 1. In some embodiments, RNA (e.g., mRNA) compositions and/or methods described herein are characterized in that sera of vaccinated subjects display neutralizing activity against one or more SARs-CoV-2 spike variants including a K417N or K417T mutation and a E484K and/or N501Y mutation in spike protein as compared to SEQ ID NO: 1.

In some embodiments, one or more SARs-CoV-2 spike variants including a mutation at positions 417 and 484 and/or 501 in spike protein as compared to SEQ ID NO: 1 or said one or more SARs-CoV-2 spike variants including a K417N or K417T mutation and a E484K and/or N501Y mutation in spike protein as compared to SEQ ID NO: 1 may include one or more further mutations as compared to SEQ ID NO: 1 (e.g., but not limited to H69/V70 deletion, Y144 deletion, A570D, D614G, P681H, T716I, S982A, D1118H, D80A, D215G, A701V, L18F, R246I, L242/A243/L244 deletion, Y453F, I692V, S1147L, M1229I, T20N, P26S, D138Y, R190S, H655Y, T1027I, V1176F etc., as compared to SEQ ID NO: 1).

In particular embodiments, RNA (e.g., mRNA) compositions and/or methods described herein are characterized in that sera of vaccinated subjects display neutralizing activity against SARs-CoV-2 spike variant "501.V2".

In particular embodiments, RNA (e.g., mRNA) compositions and/or methods described herein are characterized in that sera of vaccinated subjects display neutralizing activity against SARs-CoV-2 spike variant including the following mutations: D80A, D215G, E484K, N501Y, A701V and K417N, as compared to SEQ ID NO: 1, and optionally: L18F, R246I, and deletion 242-244 as compared to SEQ ID NO: 1. Said SARs-CoV-2 spike variant may also include a D614G mutation as compared to SEQ ID NO: 1.

In particular embodiments, RNA (e.g., mRNA) compositions and/or methods described herein are characterized in that sera of vaccinated subjects display neutralizing activity against SARs-CoV-2 spike variant "B.1.1.248".

In particular embodiments, RNA (e.g., mRNA) compositions and/or methods described herein are characterized in that sera of vaccinated subjects display neutralizing activity against SARs-CoV-2 spike variant including the following mutations: L18F, T20N, P26S, D138Y, R190S, K417T, E484K, N501Y, H655Y, T1027I, and V1176F as compared to SEQ ID NO: 1.

In some embodiments, RNA (e.g., mRNA) compositions and/or methods described herein are characterized in that sera of vaccinated subjects display neutralizing activity against SARs-CoV-2 spike variant of the Omicron (B.1.1.529) variant. Omicron (B.1.1.529) variant is a variant of SARS-CoV-2 which was detected in South Africa. Multiple Omicron variants or sublineages have arisen, including e.g., the BA.1, BA.2, BA.2.12.1, BA.3, BA.4, BA.5, and BA.2.75 sublineages. As used herein, unless otherwise specified, "Omicron variant" refers to the first disclosed Omicron variant (BA.1) or any variant thereof that has since arisen (e.g., Omicron variants described herein). In some embodiments, the spike protein changes in Omicron (B.1.1.529) BA.1 variant include A67V, Δ69-70, T95I, G142D, Δ143-145, Δ211, L212I, ins214EPE (insertion of EPE following amino acid 214), G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, and L981F. In some embodiments, the spike protein changes in Omicron (B.1.1.529) variant include A67V, Δ69-70, T95I, G142D, Δ143-145, Δ211, L212I, ins214EPE (insertion of EPE following amino acid 214), G339D, S371L, S373P, S375F, S477N, T478K, E484A, Q493R, G496S, Q498R, N501Y, Y505H, T547K, D614G, H655Y, N679K, P681H, N764K, D796Y, N856K, Q954H, N969K, and L981F. In some embodiments, the spike changes in Omicron BA.2 variant include T19I, Δ24-26, A27S, G142D, V213G, G339D, S371F, S373P, S375F, T376A, D405N, R408S, K417N, N440K, S477N, T478K, E484A, Q493R, Q498R, N501Y, Y505H, D614G, H655Y, N679K, P681H, N764K, D796Y, Q954H, N969K. In some embodiments BA.4 and BA.5 have the same Spike protein amino acid sequence, in which case "BA.4/5" is used to either Omicron variant. In some embodiments, the spike changes in Omicron BA.4/5 include: T19I, Δ24-26, A27S, Δ69/70, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, and N969K. In some embodiments, the spike changes in Omicron BA.2.75 include T19I, Δ24-26, A27S, G142D, K147E, W152R, F157L, I210V, V213G, G257S, G339H, N354D, S371F, S373P, S375F, T376A, D405N, R408S, K417N, N440K, G446S, N460K, S477N, T478K, E484A, Q498R, N501Y,, Y505H D614G, H655Y, N679K, P681H, N764K, D796Y, Q954H, and N969K.

In some embodiments, RNA (e.g., mRNA) compositions and/or methods described herein are characterized in that sera of vaccinated subjects display neutralizing activity against SARs-CoV-2 spike variant including at least 10, at least 15, at least 20, at least 21, at least 22, at least 23, at least 24, at least 25, at least 26, at least 27, at least 28, at least 29, at least 30, at least 31, at least 32, at least 33, at least 34, at least 35, at least 36, or at least 37 of the following mutations: T547K, H655Y, D614G, N679K, P681H, N969K, S373P, S371L, N440K, G339D, G446S, N856K, N764K, K417N, D796Y, Q954H, T95I, A67V, L981F, S477N, G496S, T478K, Q498R, Q493R, E484A, N501Y, S375F, Y505H, V143del, H69del, V70del, N211del, L212I, ins214EPE, G142D, Y144del, Y145del, L141del, Y144F, Y145D, G142del, as compared to SEQ ID NO: 1.

In some embodiments, RNA (e.g., mRNA) compositions and/or methods described herein are characterized in that sera of vaccinated subjects display neutralizing activity against SARs-CoV-2 spike variant including at least 10, at least 15, at least 20, at least 21, at least 22, at least 23, at least 24, or all of the following mutations: T547K, H655Y, D614G, N679K, P681H, N969K, S373P, S371L, N440K, G339D, G446S, N856K, N764K, K417N, D796Y, Q954H, T95I, A67V, L981F, S477N, G496S, T478K, Q498R, Q493R, E484A, as compared to SEQ ID NO: 1. Said SARs-CoV-2 spike variant may include at least 1, at least 2, at least 3, at least 4, at least 5, or all of the following mutations: N501Y, S375F, Y505H, V143del, H69del, V70del, as compared to SEQ ID NO: 1, and/or may include at least 1, at least 2, at least 3, at least 4, at least 5, or all of the following mutations: N211del, L212I, ins214EPE, G142D, Y144del, Y145del, as compared to SEQ ID NO: 1. In some embodiments, said SARs-CoV-2 spike variant may include at least 1, at least 2, at least 3, or all of the following mutations: L141del, Y144F, Y145D, G142del, as compared to SEQ ID NO: 1.

In some embodiments, RNA (e.g., mRNA) compositions and/or methods described herein are characterized in that sera of vaccinated subjects display neutralizing activity against a SARs-CoV-2 spike variant including at least 10, at least 15, at least 20, at least 21, at least 22, at least 23, at least 24, at least 25, at least 26, at least 27, at least 28, at least 29, at least 30, at least 31, at least 32, at least 33 of the following mutations: A67V, Δ69-70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, and L981F, as compared to SEQ ID NO: 1.

In some embodiments, RNA (e.g., mRNA) compositions and/or methods described herein are characterized in that sera of vaccinated subjects display neutralizing activity against a SARS-CoV-2 spike variant including at least 10, at least 15, at least 20, at least 21, at least 22, at least 23, at least 24, at least 25, at least 26, at least 27, at least 28, at least 29, at least 30, or at least 31, of the following mutations: T19I, Δ24-26, A27S, G142D, V213G, G339D, S371F, S373P, S375F, T376A, D405N, R408S, K417N, N440K, S477N, T478K, E484A, Q493R, Q498R, N501Y, Y505H, D614G, H655Y, N679K, P681H, N764K, D796Y, Q954H, N969K, as compared to SEQ ID NO: 1.

In some embodiments, RNA (e.g., mRNA) compositions and/or methods described herein are characterized in that sera of vaccinated subjects display neutralizing activity against a SARS-CoV-2 spike variant including at least 10, at least 15, at least 20, at least 21, at least 22, at least 23, at least 24, at least 25, at least 26, at least 27, at least 28, at least 29, at least 30, at least 31, at least 32, at least 33, or at least 34 of the following mutations: T19I, Δ24-26, A27S, Δ69/70, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, and N969K, as compared to SEQ ID NO: 1.

In some embodiments, RNA (e.g., mRNA) compositions and/or methods described herein are characterized in that sera of vaccinated subjects display neutralizing activity against a SARs-CoV-2 spike variant including the following mutations: A67V, Δ69-70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, and L981F, as compared to SEQ ID NO: 1.

In some emdbodiments, RNA (e.g., mRNA) compositions and/or methods described herein are characterized in that sera of vaccinated subjects display neutralizating against a SARS-CoV-2 spike variant including the following mutations: T19I, Δ24-26, A27S, G142D, V213G, G339D, S371F, S373P, S375F, T376A, D405N, R408S, K417N, N440K, S477N, T478K, E484A, Q493R, Q498R, N501Y, Y505H, D614G, H655Y, N679K, P681H, N764K, D796Y, Q954H, N969K. In some emdbodiments, RNA (e.g., mRNA) compositions and/or methods described herein are charcterized in that sera of vaccinated subjects display neutralizating against SARS-CoV2 spike variant including the following mutations: T19I, Δ24-26, A27S, Δ69/70, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, and N969K, as compared to SEQ ID NO: 1.

In some embodiments, RNA (e.g., mRNA) compositions and/or methods described herein are characterized in that sera of vaccinated subjects display neutralizing activity against SARs-CoV-2 spike variant including the following mutations: A67V, Δ69-70, T95I, G142D, Δ143-145, Δ211, L212I, ins214EPE, G339D, S371L, S373P, S375F, S477N, T478K, E484A, Q493R, G496S, Q498R, N501Y, Y505H, T547K, D614G, H655Y, N679K, P681H, N764K, D796Y, N856K, Q954H, N969K, and L981F, as compared to SEQ ID NO: 1.

In some embodiments, RNA (e.g., mRNA) compositions and/or methods described herein are characterized in that sera of vaccinated subjects display neutralizing activity against SARs-CoV-2 spike variant including the following mutations: T19I, Δ24-26, A27S, G142D, K147E, W152R, F157L, I210V, V213G, G257S, G339H, N354D, S371F, S373P, S375F, T376A, D405N, R408S, K417N, N440K, G446S, N460K, S477N, T478K, E484A, Q498R, N501Y, Y505H D614G, H655Y, N679K, P681H, N764K, D796Y, Q954H, and N969K, as compared to SEQ ID NO: 1. SARs-CoV-2 spike proteins encoded by RNA described herein may or may not include a D614G mutation as compared to SEQ ID NO: 1.

In some embodiments, SARS-CoV-2 spike proteins encoded by RNA described herein comprise a mutation in a furin cleavage site (e.g., in some embodiments residues 682-685 of SEQ ID NO: 1). In some embodiments, SARS-CoV-2 spike proteins encoded by RNA described herein comprise a mutation in the furin cleavage site that prevents cleavage by a furin protease (e.g., a human furin protease). In some embodiments, a SARS-CoV-2 protein described herein comprises a furin mutation disclosed in WO2021163365 or WO2021243122 (e.g., a GSAS mutation), the contents of both of which are incorporated by reference herein in their entirety.

In some embodiments, RNA (e.g., mRNA) compositions and/or methods described herein can provide protection against SARS-CoV-2 and/or decrease severity of SARS-CoV-2 infection in at least 50% of subjects receiving such RNA compositions and/or methods.

In some embodiments, populations to be treated with RNA (e.g., mRNA) compositions described herein include subjects of age 18-55. In some embodiments, populations to be treated with RNA (e.g., mRNA) compositions described herein include subjects of age 56-85. In some embodiments, populations to be treated with RNA (e.g., mRNA) compositions described herein include older subjects (e.g., over age 60, 65, 70, 75, 80, 85, etc, for example subjects of age 65-85). In some embodiments, populations to be treated with RNA (e.g., mRNA) compositions described herein include subjects of age 18-85. In some embodiments, populations to be treated with RNA (e.g., mRNA) compositions described herein include subjects of age 18 or younger. In some embodiments, populations to be treated with RNA (e.g., mRNA) compositions described herein include subjects of age 12 or younger. In some embodiments, populations to be treated with RNA (e.g., mRNA) compositions described herein include subjects of age 10 or younger. In some embodiments, populations to be treated with RNA (e.g., mRNA) compositions described herein may include adolescent populations (e.g., individuals approximately 12 to approximately 17 years of age). In some embodiments, populations to be treated with RNA (e.g., mRNA) compositions described herein may include pediatric populations (e.g., as described herein). In some embodiments, populations to be treated with RNA (e.g., mRNA) compositions described herein include infants (e.g., less than 1 year old). In some embodiments, populations to be treated with RNA (e.g., mRNA) compositions described herein do not include infants (e.g., less than 1 year) whose mothers have received such RNA (e.g., mRNA) compositions described herein during pregnancy. Without wishing to be bound by any particular theory, a rat study has suggested that a SARS-CoV-2 neutralizing antibody response induced in female rats given such RNA compositions during pregnancy can pass onto fetuses. In some embodiments, populations to be treated with RNA (e.g., mRNA) compositions described herein include infants (e.g., less than 1 year) whose mothers did not receive such RNA compositions described herein during pregnancy. In some embodiments, populations to be treated with RNA (e.g., mRNA) compositions described herein may include pregnant women; in some embodiments, infants whose mothers were vaccinated during pregnancy (e.g., who received at least one dose, or alternatively only who received both doses), are not vaccinated during the first weeks, months, or even years (e.g., 1, 2, 3, 4, 5, 6, 7, 8 weeks or more, or 1, 2, 3, 4, 5, 6, 7, 8, 9, 10, 11, 12, 13, 14, 15, 16, 17, 18, 19, 20, 21, 22, 23, 24 months or more, or 1, 2, 3, 4, 5 years or more) post-birth. Alternatively or additionally, in some embodiments, infants whose mothers were vaccinated during pregnancy (e.g., who received at least one dose, or alternatively only who received both doses), receive reduced vaccination (e.g., lower doses and/or smaller numbers of administrations - e.g., boosters - and/or lower total exposure over a given period of time) after birth, for example during the first weeks, months, or even years (e.g., 1, 2, 3, 4, 5, 6, 7, 8 weeks or more, or 1, 2, 3, 4, 5, 6, 7, 8, 9, 10, 11, 12, 13, 14, 15, 16, 17, 18, 19, 20, 21, 22, 23, 24 months or more, or 1, 2, 3, 4, 5 years or more) post-birth or may need reduced vaccination (e.g., lower doses and/or smaller numbers of administrations - e.g., boosters - over a given period of time), In some embodiments, compositions as provided herein are administered to populations that do not include pregnant women.

In some particular embodiments, compositions as provided herein are administered to pregnant women according to a regimen that includes a first dose administered after about 24 weeks of gestation (e.g., after about 22, 23, 24, 25, 26, 27, 28 or more weeks of gestation); in some embodiments, compositions as provided herein are administered to pregnant women according to a regimen that includes a first dose administered before about 34 weeks of gestation (e.g., before about 30, 31, 32, 33, 34, 35, 36, 37, 38 weeks of gestation). In some embodiments, compositions as provided herein are administered to pregnant women according to a regimen that includes a first dose administered after about 24 weeks (e.g., after about 27 weeks of gestation, e.g., between about 24 weeks and 34 weeks, or between about 27 weeks and 34 weeks) of gestation and a second dose administered about 21 days later; in some embodiments both doses are administered prior to delivery. Without wishing to be bound by any particular theory, it is proposed that such a regimen (e.g., involving administration of a first dose after about 24 weeks, or 27 weeks of gestation and optionally before about 34 weeks of gestation), and optionally a second dose within about 21 days, ideally before delivery, may have certain advantages in terms of safety (e.g., reduced risk of premature delivery or of fetal morbidity or mortality) and/or efficacy (e.g., carryover vaccination imparted to the infant) relative to alternative dosing regimens (e.g., dosing at any time during pregnancy, refraining from dosing during pregnancy, and/or dosing later in pregnancy for example so that only one dose is administered during gestation. In some embodiments, infants born of mothers vaccinated during pregnancy, e.g., according to a particular regimen as described herein, may not need further vaccination, or may need reduced vaccination (e.g., lower doses and/or smaller numbers of administrations - e.g., boosters -, and/or lower overall exposure over a given period of time), for a period of time (e.g., as noted herein) after birth.

In some embodiments, compositions as provided herein are administered to populations in which women are advised against becoming pregnant for a period of time after receipt of the vaccine (e.g., after receipt of a first dose of the vaccine, after receipt of a final dose of the vaccine, etc.); in some such embodiments, the period of time may be at least 1 week, at least 2 weeks, at least 3 weeks, at least 4 weeks, at least 5 weeks, at least 6 weeks, at least 7 weeks, at least 8 weeks, at least 9 weeks, at least 10 weeks or more, or may be at least 1 month, at least 2 months, at least 3 months, at least 4 months, at least 5 months, at least 6 months, or more.

In some embodiments, populations to be treated with RNA (e.g., mRNA) compositions described herein may include one or more populations with one or more particularly high risk conditions or history, e.g., as noted herein. For example, in some embodiments, populations to be treated with RNA compositions described herein may include subjects whose profession and/or environmental exposure may dramatically increase their risk of getting SARS-CoV-2 infection (including, e.g., but not limited to mass transportation, prisoners, grocery store workers, residents in long-term care facilities, butchers or other meat processing workers, healthcare workers, and/or first responders, e.g., emergency responders). In particular embodiments, populations to be treated with RNA (e.g., mRNA) compositions described herein may include healthcare workers and/or first responders, e.g., emergency responders. In some embodiments, populations to be treated with RNA (e.g., mRNA) compositions described herein may include those with a history of smoking or vaping (e.g., within 6 months, 12 months or more, including a history of chronic smoking or vaping). In some embodiments, populations to be treated with RNA (e.g., mRNA) compositions described herein may include certain ethnic groups that have been determined to be more susceptible to SARS-CoV-2 infection.

In some embodiments, populations to be treated with RNA (e.g., mRNA) compositions described herein may include certain populations with a blood type that may have been determined to more susceptible to SARS-CoV-2 infection. In some embodiments, populations to be treated with RNA (e.g., mRNA) compositions described herein may include immunocompromised subjects (e.g., those with HIV/AIDS; cancer patients (e.g., receiving antitumor treatment); patients who are taking certain immunosuppressive drugs (e.g., transplant patients, cancer patients, etc.); autoimmune diseases or other physiological conditions expected to warrant immunosuppressive therapy (e.g., within 3 months, within 6 months, or more); and those with inherited diseases that affect the immune system (e.g., congenital agammaglobulinemia, congenital IgA deficiency)). In some embodiments, populations to be treated with RNA (e.g., mRNA) compositions described herein may include those with an infectious disease. For example, in some embodiments, populations to be treated with RNA (e.g., mRNA) compositions described herein may include those infected with human immunodeficiency virus (HIV) and/or a hepatitis virus (e.g., HBV, HCV). In some embodiments, populations to be treated with RNA (e.g., mRNA) compositions described herein may include those with underlying medical conditions. Examples of such underlying medical conditions may include, but are not limited to hypertension, cardiovascular disease, diabetes, chronic respiratory disease, e.g., chronic pulmonary disease, asthma, etc., cancer, and other chronic diseases such as, e.g., lupus, rheumatoid arthritis, chronic liver diseases, chronic kidney diseases (e.g., Stage 3 or worse such as in some embodiments as characterized by a glomerular filtration rate (GFR) of less than 60 mL/min/1.73m²). In some embodiments, populations to be treated with RNA (e.g., mRNA) compositions described herein may include overweight or obese subjects, e.g., specifically including those with a body mass index (BMI) above about 30 kg/m². In some embodiments, populations to be treated with RNA (e.g., mRNA) compositions described herein may include subjects who have prior diagnosis of COVID-19 or evidence of current or prior SARS-CoV-2 infection, e.g., based on serology or nasal swab. In some embodiments, populations to be treated include white and/or non-Hispanic/non-Latino.

In some embodiments, certain RNA (e.g., mRNA) compositions described herein may be selected for administration to Asian populations (e.g., Chinese populations), or in particular embodiments to older Asian populations (e.g., 60 years old or over, e.g., 60-85 or 65-85 years old).

In some embodiments, an RNA (e.g., mRNA) composition as provided herein is administered to and/or assessed in subject(s) who have been determined not to show evidence of prior infection, and/or of present infection, before administration; in some embodiments, evidence of prior infection and/or of present infection, may be or include evidence of intact virus, or any viral nucleic acid, protein, lipid etc. present in the subject (e.g., in a biological sample thereof, such as blood, cells, mucus, and/or tissue), and/or evidence of a subject's immune response to the same. In some embodiments, an RNA (e.g., mRNA) composition as provided herein is administered to and/or assessed in subject(s) who have been determined to show evidence of prior infection, and/or of present infection, before administration; in some embodiments, evidence of prior infection and/or of present infection, may be or include evidence of intact virus, or any viral nucleic acid, protein, lipid etc. present in the subject (e.g., in a biological sample thereof, such as blood, cells, mucus, and/or tissue), and/or evidence of a subject's immune response to the same. In some embodiments, a subject is considered to have a prior infection based on having a positive N-binding antibody test result or positive nucleic acid amplification test (NAAT) result on the day of Dose 1.

In some embodiments, an RNA (e.g., mRNA) composition as provided herein is administered to a subject who has been informed of a risk of side effects that may include one or more of, for example: chills, fever, headache, injection site pain, muscle pain, tiredness; in some embodiments, an RNA (e.g., mRNA) composition is administered to a subject who has been invited to notify a healthcare provider if one or more such side effects occurs, is experienced as more than mild or moderate, persists for a period of more than a day or a few days, or if any serious or unexpected event is experienced that the subject reasonably considers may be associated with receipt of the composition. In some embodiments, an RNA (e.g., mRNA) composition as provided herein is administered to a subject who has been invited to notify a healthcare provider of particular medical conditions which may include, for example, one or more of allergies, bleeding disorder or taking a blood thinner medication, breastfeeding, fever, immunocompromised state or taking medication that affects the immune system, pregnancy or plan to become pregnant, etc. In some embodiments, an RNA (e.g., mRNA) composition as provided herein is administered to a subject who has been invited to notify a healthcare provider of having received another COVID-19 vaccine. In some embodiments, an RNA (e.g., mRNA) composition as provided herein is administered to a subject not having one of the following medical conditions: experiencing febrile illness, receiving immunosuppressant therapy, receiving anticoagulant therapy, suffering from a bleeding disorder (e.g., one that would contraindicate intramuscular injection), or pregnancy and/or breastfeeding/lactation. In some embodiments, an RNA (e.g., mRNA) composition as provided herein is administered to a subject not having received another COVID-19 vaccine. In some embodiments, an RNA (e.g., mRNA) composition as provided herein is administered to a subject who has not had an allergic reaction to any component of the RNA (e.g., mRNA) composition. Examples of such allergic reaction may include, but are not limited to difficulty breathing, swelling of fact and/or throat, fast heartbeat, rash, dizziness and/or weakness. In some embodiments, an RNA (e.g., mRNA) composition as provided herein is administered to a subject who received a first dose and did not have an allergic reaction (e.g., as described herein) to the first dose. In some embodiments where allergic reaction occurs in subject(s) after receiving a dose of an RNA (e.g., mRNA) composition as provided herein, such subject(s) may be administered one or more interventions such as treatment to manage and/or reduce symptom(s) of such allergic reactions, for example, fever-reducing and/or anti-inflammatory agents.

In some embodiments, a subject who has received at least one dose of an RNA (e.g., mRNA) composition as provided herein is informed of avoiding being exposed to a coronavirus (e.g., SARS-CoV-2) unless and until several days (e.g., at least 7 days, at least 8 days, 9 days, at least 10 days, at least 11 days, at least 12 days, at least 13 days, at least 14 days, etc.) have passed since administration of a second dose. For example, a subject who has received at least one dose of an RNA (e.g., mRNA) composition as provided herein is informed of taking precautionary measures against SARS-CoV-2 infection (e.g., remaining socially distant, wearing masks, frequent hand-washing, etc.) unless and until several days (e.g., at least 7 days, at least 8 days, 9 days, at least 10 days, at least 11 days, at least 12 days, at least 13 days, at least 14 days, etc.) have passed since administration of a second dose. Accordingly, in some embodiments, methods of administering an RNA (e.g., mRNA) composition as provided herein comprise administering a second dose of such an RNA (e.g., mRNA) composition as provided herein to a subject who received a first dose and took precautionary measures to avoid being exposed to a coronavirus (e.g., SARS-CoV-2).

In some embodiments, RNA (e.g., mRNA) compositions described herein may be delivered to a draining lymph node of a subject in need thereof, for example, for vaccine priming. In some embodiments, such delivery may be performed by intramuscular administration of a provided RNA (e.g., mRNA) composition.

In some embodiments, different particular RNA (e.g., mRNA) compositions may be administered to different subject population(s); alternatively or additionally, in some embodiments, different dosing regimens may be administered to different subject populations. For example, in some embodiments, RNA (e.g., mRNA) compositions administered to particular subject population(s) may be characterized by one or more particular effects (e.g., incidence and/or degree of effect) in those subject populations. In some embodiments, such effect(s) may be or comprise, for example titer and/or persistence of neutralizing antibodies and/orT cells (e.g., T_{H}1-type T cells such as CD4⁺ and/or CD8⁺ T cells), protection against challenge (e.g., via injection and/or nasal exposure, etc), incidence, severity, and/or persistence of side effects (e.g., reactogenicity), etc.

In some embodiments, one or more RNA (e.g., mRNA) compositions described herein may be administered according to a regimen established to reduce COVID-19 incidence per 1000 person-years, e.g., based on a laboratory test such as nucleic acid amplification test (NAAT). In some embodiments, one or more RNA (e.g., mRNA) compositions described herein may be administered according to a regimen established to reduce COVID-19 incidence per 1000 person-years based on a laboratory test such as nucleic acid amplification test (NAAT) in subjects receiving at least one dose of a provided RNA (e.g., mRNA) composition with no serological or virological evidence (e.g., up to 7 days after receipt of the last dose) of past SARS-CoV-2 infection. In some embodiments, one or more RNA (e.g., mRNA) compositions described herein may be administered according to a regimen established to reduce confirmed severe COVID-19 incidence per 1000 person-years. In some embodiments, one or more RNA (e.g., mRNA) compositions described herein may be administered according to a regimen established to reduce confirmed severe COVID-19 incidence per 1000 person-years in subjects receiving at least one dose of a provided RNA (e.g., mRNA) composition with no serological or virological evidence of past SARS-CoV-2 infection.

In some embodiments, one or more RNA (e.g., mRNA) compositions described herein may be administered according to a regimen established to produce neutralizing antibodies directed to a SARS-CoV-2 spike polypeptide and/or an immunogenic fragment thereof (e.g., RBD) as measured in serum from a subject that achieves or exceeds a reference level (e.g., a reference level determined based on human SARS-CoV-2 infection/COVID-19 convalescent sera) for a period of time and/or induction of cell-mediated immune response (e.g., a T cell response against SARS-CoV-2), including, e.g., in some embodiments induction of T cells that recognize at least one or more MHC-restricted (e.g., MHC class I-restricted) epitopes within a SARS-CoV-2 spike polypeptide and/or an immunogenic fragment thereof (e.g., RBD) for a period of time. In some such embodiments, the period of time may be at least 2 months, 3 months, at least 4 months, at least 5 months, at least 6 months, at least 7 months, at least 8 months, at least 9 months, at least 10 months, at least 11 months, at least 12 months or longer. In some embodiments, one or more epitopes recognized by vaccine-induced T cells (e.g., CD8+ T cells) may be presented on a MHC class I allele that is present in at least 50% of subjects in a population, including, e.g., at least 60%, at least 70%, at least 80%, at least 90%, or more; in some such embodiments, the MHC class I allele may be HLA-B^{∗}0702, HLA-A^{∗}2402, HLA-B*3501, HLA-B*4401, or HLA-A*0201. In some embodiments, an epitope may comprise HLA-A*0201 YLQPRTFLL; HLA-A*0201 RLQSLQTYV; HLA-A^{∗}2402 QYIKWPWYI; HLA-A^{∗}2402 NYNYLYRLF; HLA-A^{∗}2402 KWPWYIWLGF; HLA-B*3501 QPTESIVRF; HLA-B*3501 IPFAMQMAY; or HLA-B*3501 LPFNDGVYF.

In some embodiments, efficacy is assessed as COVID-19 incidence per 1000 person-years in individuals without serological or virological evidence of past SARS-CoV-2 infection before and during vaccination regimen; alternatively or additionally, in some embodiments, efficacy is assessed as COVID-19 incidence per 1000 person-years in subjects with and without evidence of past SARS-CoV-2 infection before and during vaccination regimen. In some such embodiments, such incidence is of COVID-19 cases confirmed within a specific time period after the final vaccination dose (e.g., a first dose in a single-dose regimen; a second dose in a two-dose regimen, etc); in some embodiments, such time period may be within (i.e., up to and including 7 days) a particular number of days (e.g., 3, 4, 5, 6, 7, 8, 9, 10, 11, 12, 13, 14, 15, 16, 17, 18, 19, 20, 21, 22, 23, 24, 25, 26, 27, 28, 29, 30 days or more). In some embodiments, such time period may be within 7 days or within 14 days or within 21 days or within 28 days. In some embodiments, such time period may be within 7 days. In some embodiments, such time period may be within 14 days.

In some embodiments (e.g., in some embodiments of assessing efficacy), a subject is determined to have experienced COVID-19 infection if one or more of the following is established: detection of SARS-CoV-2 nucleic acid in a sample from the subject, detection of antibodies that specifically recognize SARS-CoV-2 (e.g., a SARS-Co-V-2 spike protein), one or more symptoms of COVID-19 infection, and combinations thereof. In some such embodiments, detection of SARS-CoV-2 nucleic acid may involve, for example, NAAT testing on a mid-turbinatae swap sample. In some such embodiments, detection of relevant antibodies may involve serological testing of a blood sample or portion thereof. In some such embodiments, symptoms of COVID-19 infection may be or include: fever, new or increased cough, new or increased shortness of breath, chills, new or increased muscle pain, new loss of taste or smell, sore throat, diarrhea, vomiting and combinations thereof. In some such embodiments, symptoms of COVID-19 infection may be or include: fever, new or increased cough, new or increased shortness of breath, chills, new or increased muscle pain, new loss of taste or smell, sore throat, diarrhea, vomiting, fatigue, headache, nasal congestion or runny nose, nausea, and combinations thereof. In some such embodiments, a subject is determined to have experienced COVID-19 infection if such subject both has experienced one such symptom and also has received a positive test for SARS-CoV-2 nucleic acid or antibodies, or both. In some such embodiments, a subject is determined to have experienced COVID-19 infection if such subject both has experienced one such symptom and also has received a positive test for SARS-CoV-2 nucleic acid. In some such embodiments, a subject is determined to have experienced COVID-19 infection if such subject both has experienced one such symptom and also has received a positive test for SARS-CoV-2 antibodies.

In some embodiments (e.g., in some embodiments of assessing efficacy), a subject is determined to have experienced severe COVID-19 infection if such subject has experienced one or more of: clinical signs at rest indicative or severe systemic illness (e.g., one or more of respiratory rate at greater than or equal to 30 breaths per minute, heart rate at or above 125 beats per minute, SpO₂ less than or equal to 93% on room air at sea level or a PaO₂/FiO₂ below 300 m Hg), respiratory failure (e.g., one or more of needing high-flow oxygen, noninvasive ventilation, mechanical ventilation, ECMO), evidence of shock (systolic blood pressure below 90 mm Hg, diastolic blood pressure below 60mm Hg, requiring vasopressors), significant acute renal, hepatic, or neurologic dysfunction, admission to an intensive care unit, death, and combinations thereof.

In some embodiments, one or more RNA (e.g., mRNA) compositions described herein may be administered according to a regimen established to reduce the percentage of subjects reporting at least one of the following: (i) one or more local reactions (e.g., as described herein) for up to 7 days following each dose; (ii) one or more systemic events for up to 7 days following each dose; (iii) adverse events (e.g., as described herein) from a first dose to 1 month after the last dose; and/or (iv) serious adverse events (e.g., as described herein) from a first dose to 6 months after the last dose.

In some embodiments, one or more subjects who have received an RNA (e.g., mRNA) composition as described herein may be monitored (e.g., for a period of at least 1, 2, 3, 4, 5, 6, 7, 8, 9, 10 days or more, including, for example, 1, 2, 3, 4, 5, 6, 7, 8, 9, 10, 11, 12 weeks or more, including for example 1, 2, 3, 4, 5, 6, 7, 8, 9 10, 11, 12, 13, 14, 15, 16, 17, 18, 19, 20, 21, 22, 23, 24 months or more, including for example 1, 2, 3, 4, 5, 6, 7, 8, 9, 10 years or more) to assess, for example, presence of an immune response to component(s) of the administered composition, evidence of exposure to and/or immune response to SARS-CoV-2 or another coronavirus, evidence of any adverse event, etc. In some embodiments, monitoring may be via tele-visit. Alternatively or additionally, in some embodiments, monitoring may be in-person.

In some embodiments, a treatment effect conferred by one or more RNA (e.g., mRNA) compositions described herein may be characterized by (i) a SARS-CoV-2 anti-S1 binding antibody level above a pre-determined threshold; (ii) a SARS-CoV-2 anti-RBD binding antibody level above a pre-determined threshold; and/or (iii) a SARS-CoV-2 serum neutralizing titer above a threshold level, e.g., at baseline, 1 month, 3 months, 6 months, 9 months, 12 months, 18 months, and/or 24 months after completion of vaccination. In some embodiments, anti-S1 binding antibody and/or anti-RBD binding antibody levels and/or serum neutralizing titers may be characterized by geometric mean concentration (GMC), geometric mean titer (GMT), or geometric mean fold-rise (GMFR).

In some embodiments, a treatment effect conferred by one or more RNA (e.g., mRNA) compositions described herein may be characterized in that percentage of treated subjects showing a SARS-CoV-2 serum neutralizing titer above a pre-determined threshold, e.g., at baseline, 1 month, 3 months, 6 months, 9 months, 12 months, 18 months, and/or 24 months after completion of vaccination, is higher than the percentage of non-treated subjects showing a SARS-CoV-2 serum neutralizing titer above such a pre-determined threshold (e.g., as described herein). In some embodiments, a serum neutralizing titer may be characterized by geometric mean concentration (GMC), geometric mean titer (GMT), or geometric mean fold-rise (GMFR).

In some embodiments, a treatment effect conferred by one or more RNA (e.g., mRNA) compositions described herein may be characterized by detection of SARS-CoV-2 NVA-specific binding antibody.

In some embodiments, a treatment effect conferred by one or more RNA (e.g., mRNA) compositions described herein may be characterized by SARS-CoV-2 detection by nucleic acid amplification test.

In some embodiments, a treatment effect conferred by one or more RNA (e.g., mRNA) compositions described herein may be characterized by induction of cell-mediated immune response (e.g., a T cell response against SARS-CoV-2), including, e.g., in some embodiments induction of T cells that recognize at least one or more MHC-restricted (e.g., MHC class I-restricted) epitopes within a SARS-CoV-2 spike polypeptide and/or an immunogenic fragment thereof (e.g., RBD). In some embodiments, one or more epitopes recognized by vaccine-induced T cells (e.g., CD8+ T cells) may be presented on a MHC class I allele that is present in at least 50% of subjects in a population, including, e.g., at least 60%, at least 70%, at least 80%, at least 90%, or more; in some such embodiments, the MHC class I allele may be HLA-B^{∗}0702, HLA-A^{∗}2402, HLA-B*3501, HLA-B*4401, or HLA-A*0201. In some embodiments, an epitope may comprise HLA-A*0201 YLQPRTFLL; HLA-A*0201 RLQSLQTYV; HLA-A^{∗}2402 QYIKWPWYI; HLA-A^{∗}2402 NYNYLYRLF; HLA-A^{∗}2402 KWPWYIWLGF; HLA-B*3501 QPTESIVRF; HLA-B*3501 IPFAMQMAY; or HLA-B*3501 LPFNDGVYF.

In some embodiments, primary vaccine efficacy (VE) of one or more RNA (e.g., mRNA) compositions described herein may be established when there is sufficient evidence (posterior probability) that either primary VE1 or both primary VE1 and primary VE2 are >30% or higher (including, e.g., greater than 40%, greater than 50%, greater than 60%, greater than 70%, greater than 80%, greater than 90%, greater than 95%, greater than 96%, greater than 97%, greater than 98%, or higher), wherein primary VE is defined as primary VE = 100 × (1 - IRR); and IRR is calculated as the ratio of COVID-19 illness rate in the vaccine group to the corresponding illness rate in the placebo group. Primary VE1 represents VE for prophylactic RNA (e.g., mRNA) compositions described herein against confirmed COVID-19 in participants without evidence of infection before vaccination, and primary VE2 represents VE for prophylactic RNA (e.g., mRNA) compositions described herein against confirmed COVID-19 in all participants after vaccination. In some embodiments, primary VE1 and VE2 can be evaluated sequentially to control the overall type I error of 2.5% (hierarchical testing). In some embodiments where one or more RNA (e.g., mRNA) compositions described herein are demonstrated to achieve primary VE endponts as discussed above, secondary VE endpoints (e.g., confirmed severe COVID-19 in participants without evidence of infection before vaccination and confirmed severe COVID-19 in all participants) can be evaluated sequentially, e.g., by the same method used for the primary VE endpoint evaluation (hierarchical testing) as discussed above. In some embodiments, evaluation of primary and/or secondary VE endpoints may be based on at least 20,000 or more subjects (e.g., at least 25,000 or more subjects) randomized in a 1:1 ratio to the vaccine or placebo group, e.g., based on the following assumptions: (i) 1.0% illness rate per year in the placebo group, and (ii) 20% of the participants being non-evaluable or having serological evidence of prior infection with SARS-CoV-2, potentially making them immune to further infection.

In some embodiments, one or more RNA (e.g., mRNA) compositions described herein may be administered according to a regimen established to achieve maintenance and/or continued enhancement of an immune response. For example, in some embodiments, an administration regimen may include a first dose optionally followed by one or more subsequent doses; in some embodiments, need for, timing of, and/or magnitude of any such subsequent dose(s) may be selected to maintain, enhance, and/or modify one or more immune responses or features thereof. In some embodiments, number, timing, and/or amount(s) of dose(s) have been established to be effective when administered to a relevant population. In some embodiments, number, timing and/or amount(s) of dose(s) may be adjusted for an individual subject; for example, in some embodiments, one or more features of an immune response in an individual subject may be assessed at least once (and optionally more than once, for example multiple times, typically spaced apart, often at pre-selected intervals) after receipt of a first dose. For example, presence of antibodies, B cells, and/or T cells (e.g., CD4⁺ and/or CD8⁺ T cells), and/or of cytokines secreted thereby and/or identity of and/or extent of responses to particular antigen(s) and/or epitope(s) may be assessed. In some embodiments, need for, timing of, and/or amount of a subsequent dose may be determined in light of such assessments.

As noted hereinabove, in some embodiments, one or more subjects who have received an RNA (e.g., mRNA) composition as described herein may be monitored (e.g., for a period of at least 1, 2, 3, 4, 5, 6, 7, 8, 9, 10 days or more, including, for example, 1, 2, 3, 4, 5, 6, 7, 8, 9, 10, 11, 12 weeks or more, including for example 1, 2, 3, 4, 5, 6, 7, 8, 9 ,10, 11, 12, 13, 14, 15, 16, 17, 18, 19, 20, 21, 22, 23, 24 months or more, including for example 1, 2, 3, 4, 5, 6, 7, 8, 9, 10 years or more) from receipt of any particular dose to assess, for example, presence of an immune response to component(s) of the administered composition, evidence of exposure to and/or immune response to SARS-CoV-2 or another coronavirus, evidence of any adverse event, etc, including to perform assessment of one or more of presence of antibodies, B cells, and/or T cells (e.g., CD4⁺ and/or CD8⁺ T cells), and/or of cytokines secreted thereby and/or identity of and/or extent of responses to particular antigen(s) and/or epitope(s) may be assessed. Administration of a composition as described herein may be in accordance with a regimen that includes one or more such monitoring steps.

For example, in some embodiments, need for, timing of, and/or amount of a second dose relative to a first dose (and/or of a subsequent dose relative to a prior dose) is assessed, determined, and/or selected such that administration of such second (or subsequent) dose achieves amplification or modification of an immune response (e.g., as described herein) observed after the first (or other prior) dose. In some embodiments, such amplification of an immune response (e.g., ones described herein) may be at least 30%, at least 40%, at least 50%, at least 60%, at least 70%, at least 80%, at least 90%, at least 95%, or higher, as compared to the level of an immune response observed after the first dose. In some embodiments, such amplification of an immune response may be at least 1.5 fold, at least 2-fold, at least 3-fold, at least 4-fold, at least 5-fold, at least 6-fold, at least 7-fold, at least 8-fold, at least 9-fold, at least 10-fold, at least 20-fold, at least 30-fold, or higher, as compared to the level of an immune response observed after the first dose.

In some embodiments, need for, timing of, and/or amount of a second (or subsequent) dose relative to a first (or other prior) dose is assessed, determined, and/or selected such that administration of the later dose extends the durability of an immune response (e.g., as described herein) observed after the earlier dose; in some such embodiments, the durability may be extended by at least 1 week, at least 2 weeks, at least 3 weeks, at least 1 month, at least 2 months, at least 3 months, at least 4 months, at least 5 months, at least 6 months, at least 7 months, at least 8 months, at least 9 months, or longer. In some embodiments, an immune response observed after the first dose may be characterized by production of neutralizing antibodies directed to a SARS-CoV-2 spike polypeptide and/or an immunogenic fragment thereof (e.g., RBD) as measured in serum from a subject and/or induction of cell-mediated immune response (e.g., a T cell response against SARS-CoV-2), including, e.g., in some embodiments induction of T cells that recognize at least one or more MHC-restricted (e.g., MHC class I-restricted) epitopes within a SARS-CoV-2 spike polypeptide and/or an immunogenic fragment thereof (e.g., RBD). In some embodiments, one or more epitopes recognized by vaccine-induced T cells (e.g., CD8+ T cells) may be presented on a MHC class I allele that is present in at least 50% of subjects in a population, including, e.g., at least 60%, at least 70%, at least 80%, at least 90%, or more; in some such embodiments, the MHC class I allele may be HLA-B^{∗}0702, HLA-A^{∗}2402, HLA-B*3501, HLA-B*4401, or HLA-A*0201. In some embodiments, an epitope may comprise HLA-A*0201 YLQPRTFLL; HLA-A*0201 RLQSLQTYV; HLA-A^{∗}2402 QYIKWPWYI; HLA-A^{∗}2402 NYNYLYRLF; HLA-A^{∗}2402 KWPWYIWLGF; HLA-B*3501 QPTESIVRF; HLA-B*3501 IPFAMQMAY; or HLA-B*3501 LPFNDGVYF.

In some embodiments, need for, timing of, and/or amount of a second dose relative to a first dose (or other subsequent dose relative to a prior dose) is assessed, determined and/or selected such that administration of such second (or subsequent) dose maintains or exceeds a reference level of an immune response; in some such embodiments, the reference level is determined based on human SARS-CoV-2 infection/COVID-19 convalescent sera and/or PBMC samples drawn from subjects (e.g., at least a period of time such as at least 14 days or longer, including, e.g., 15 days, 16 days, 17 days, 18 days, 19 days, 20 days, 25 days, 30 days, 35 days, 40 days, 45 days, 50 days, 55 days, 60 days, or longer, after PCR-confirmed diagnosis when the subjects were asymptomatic. In some embodiments, an immune response may be characterized by production of neutralizing antibodies directed to a SARS-CoV-2 spike polypeptide and/or an immunogenic fragment thereof (e.g., RBD) as measured in serum from a subject and/or induction of cell-mediated immune response (e.g., a T cell response against SARS-CoV-2), including, e.g., in some embodiments induction of T cells that recognize at least one or more MHC-restricted (e.g., MHC class I-restricted) epitopes within a SARS-CoV-2 spike polypeptide and/or an immunogenic fragment thereof (e.g., RBD). In some embodiments, one or more epitopes recognized by vaccine-induced T cells (e.g., CD8+ T cells) may be presented on a MHC class I allele that is present in at least 50% of subjects in a population, including, e.g., at least 60%, at least 70%, at least 80%, at least 90%, or more; in some such embodiments, the MHC class I allele may be HLA-B^{∗}0702, HLA-A^{∗}2402, HLA-B*3501, HLA-B*4401, or HLA-A*0201. In some embodiments, an epitope may comprise HLA-A*0201 YLQPRTFLL; HLA-A*0201 RLQSLQTYV; HLA-A^{∗}2402 QYIKWPWYI; HLA-A^{∗}2402 NYNYLYRLF; HLA-A^{∗}2402 KWPWYIWLGF; HLA-B*3501 QPTESIVRF; HLA-B*3501 IPFAMQMAY; or HLA-B*3501 LPFNDGVYF.

In some embodiments, determination of need for, timing of, and/or amount of a second (or subsequent) dose may include one or more steps of assessing, after (e.g., 7, 8, 9, 10, 11, 12, 13, 14, 15, 16, 17, 18, 19, 20, 21 days or longer after) a first (or other prior) dose, presence and/or expression levels of neutralizing antibodies directed to a SARS-CoV-2 spike polypeptide and/or an immunogenic fragment thereof (e.g., RBD) as measured in serum from a subject and/or induction of cell-mediated immune response (e.g., a T cell response against SARS-CoV-2), including, e.g., in some embodiments induction of T cells that recognize at least one or more MHC-restricted (e.g., MHC class I-restricted) epitopes within a SARS-CoV-2 spike polypeptide and/or an immunogenic fragment thereof (e.g., RBD). In some embodiments, one or more epitopes recognized by vaccine-induced T cells (e.g., CD8+ T cells) may be presented on a MHC class I allele that is present in at least 50% of subjects in a population, including, e.g., at least 60%, at least 70%, at least 80%, at least 90%, or more; in some such embodiments, the MHC class I allele may be HLA-B^{∗}0702, HLA-A^{∗}2402, HLA-B*3501, HLA-B*4401, or HLA-A*0201. In some embodiments, an epitope may comprise HLA-A*0201 YLQPRTFLL; HLA-A*0201 RLQSLQTYV; HLA-A^{∗}2402 QYIKWPWYI; HLA-A^{∗}2402 NYNYLYRLF; HLA-A^{∗}2402 KWPWYIWLGF; HLA-B*3501 QPTESIVRF; HLA-B*3501 IPFAMQMAY; or HLA-B*3501 LPFNDGVYF.

In some embodiments, a kit as provided herein may comprise a real-time monitoring logging device, which, for example in some embodiments, is capable of providing shipment temperatures, shipment time and/or location.

In some embodiments, an RNA (e.g., mRNA) composition as described herein may be shipped, stored, and/or utilized, in a container (such as a vial or syringe), e.g., a glass container (such as a glass vial or syringe), which, in some embodiments, may be a single-dose container or a multi-dose container (e.g., may be arranged and constructed to hold, and/or in some embodiments may hold, a single dose, or multiple doses of a product for administration). In some embodiments, a multi-dose container (such as a multi-dose vial or syringe) may be arranged and constructed to hold, and/or may hold 2, 3, 4, 5, 6, 7, 8, 9, 10 or more doses; in some particular embodiments, it may be designed to hold and/or may hold 5 doses. In some embodiments, a single-dose or multi-dose container (such as a single-dose or multi-dose vial or syringe) may be arranged and constructed to hold and/or may hold a volume or amount greater than the indicated number of doses, e.g., in order to permit some loss in transfer and/or administration. In some embodiments, an RNA (e.g., mRNA) composition as described herein may be shipped, stored, and/or utilized, in a preservative-free glass container (e.g., a preservative-free glass vial or syringe, e.g., a single-dose or multi-dose preservative-free glass vial or syringe). In some embodiments, an RNA (e.g., mRNA) composition as described herein may be shipped, stored, and/or utilized, in a preservative-free glass container (e.g., a preservative-free glass vial or syringe, e.g., a single-dose or multi-dose preservative-free glass vial or syringe) that contains a frozen liquid, e.g., in some embodiments 0.45 ml of frozen liquid (e.g., including 5 doses). In some embodiments, an RNA (e.g., mRNA) composition as described herein and/or a container (e.g., a vial or syringe) in which it is disposed, is shipped, stored, and/or utilized may be maintained at a temperature below room temperature, at or below 4 °C, at or below 0 °C, at or below -20 °C, at or below -60 °C, at or below -70 °C, at or below -80 °C , at or below -90 °C, etc. In some embodiments, an RNA (e.g., mRNA) composition as described herein and/or a container (e.g., a viral or syringe) in which it is disposed, is shipped, stored, and/or utilized may be maintained at a temperature between -80°C and -60°C and in some embodiments protected from light. In some embodiments, an RNA (e.g., mRNA) composition as described herein and/or a container (e.g., a viral or syringe) in which it is disposed, is shipped, stored, and/or utilized may be maintained at a temperature below about 25°C, and in some embodiments protected from light. In some embodiments, an RNA (e.g., mRNA) composition as described herein and/or a container (e.g., a viral or syringe) in which it is disposed, is shipped, stored, and/or utilized may be maintained at a temperature below about 5°C (e.g., below about 4°C), and in some embodiments protected from light. In some embodiments, an RNA (e.g., mRNA) composition as described herein and/or a container (e.g., a viral or syringe) in which it is disposed, is shipped, stored, and/or utilized may be maintained at a temperature below about -20°C, and in some embodiments protected from light. In some embodiments, an RNA (e.g., mRNA) composition as described herein and/or a container (e.g., a viral or syringe) in which it is disposed, is shipped, stored, and/or utilized may be maintained at a temperature above about -60°C (e.g., in some embodiments at or above about -20°C, and in some embodiments at or above about 4-5°C, in either case optionally below about 25°C), and in some embodiments protected from light, or otherwise without affirmative steps (e.g., cooling measures) taken to achieve a storage temperature materially below about -20°C.

In some embodiments, an RNA (e.g., mRNA) composition as described herein and/or a container (e.g., a vial or syringe) in which it is disposed is shipped, stored, and/or utilized together with and/or in the context of a thermally protective material or container and/or of a temperature adjusting material. For example, in some embodiments, an RNA (e.g., mRNA) composition as described herein and/or a container (e.g., a vial or syringe) in which it is disposed is shipped, stored, and/or utilized together with ice and/or dry ice and/or with an insulating material. In some particular embodiments, a container (e.g., a vial or syringe) in which an RNA (e.g., mRNA) composition is disposed is positioned in a tray or other retaining device and is further contacted with (or otherwise in the presence of) temperature adjusting (e.g., ice and/or dry ice) material and/or insulating material. In some embodiments, multiple containers (e.g., multiple vials or syringes such as single use or multi-use vials or syringes as described herein) in which a provided RNA (e.g., mRNA) composition is disposed are co-localized (e.g., in a common tray, rack, box, etc.) and packaged with (or otherwise in the presence of) temperature adjusting (e.g., ice and/or dry ice) material and/or insulating material. To give but one example, in some embodiments, multiple containers (e.g., multiple vials or syringes such as single use or multi-use vials or syringes as described herein) in which an RNA (e.g., mRNA) composition is disposed are positioned in a common tray or rack, and multiple such trays or racks are stacked in a carton that is surrounded by a temperature adjusting material (e.g., dry ice) in a thermal (e.g., insulated) shipper. In some embodiments, temperature adjusting material is replenished periodically (e.g., within 24 hours of arrival at a site, and/or every 2 hours, 4 hours, 6 hours, 8 hours, 10 hours, 12 hours, 14 hours, 16 hours, 18 hours, 20 hours, 22 hours, 1 day, 2 days, 3 days, 4 days, 5 days, 6 days, 7 days, 8 days, 9 days, 10 days, etc.). Preferably, re-entry into a thermal shipper should be infrequent, and desirably should not occur more than twice a day. In some embodiments, a thermal shipper is re-closed within 5, 4, 3, 2, or 1 minute, or less, of having been opened. In some embodiments, a provided RNA (e.g., mRNA) composition that has been stored within a thermal shipper for a period of time, optionally within a particular temperature range remains useful. For example, in some embodiments, if a thermal shipper as described herein containing a provided RNA (e.g., mRNA) composition is or has been maintained (e.g., stored) at a temperature within a range of about 15 °C to about 25 °C, the RNA (e.g., mRNA) composition may be used for up to 10 days; that is, in some embodiments, a provided RNA (e.g., mRNA) composition that has been maintained within a thermal shipper, which thermal shipper is at a temperature within a range of about 15 °C to about 25 °C, for a period of not more than 10 days is administered to a subject. Alternatively or additionally, in some embodiments, if a provided RNA (e.g., mRNA) composition is or has been maintained (e.g., stored) within a thermal shipper, which thermal shipper has been maintained (e.g., stored) at a temperature within a range of about 15 °C to about 25 °C, it may be used for up to 10 days; that is, in some embodiments, a provided RNA (e.g., mRNA) composition that has been maintained within a thermal shipper, which thermal shipper has been maintained at a temperature within a range of about 15 °C to about 25 °C for a period of not more than 10 days is administered to a subject.

In some embodiments, a provided RNA (e.g., mRNA) composition is shipped and/or stored in a frozen state. In some embodiments, a provided RNA (e.g., mRNA composition is shipped and/or stored as a frozen suspension, which in some embodiments does not contain preservative. In some embodiments, a frozen RNA (e.g., mRNA) composition is thawed. In some embodiments, a thawed RNA (e.g., mRNA) composition (e.g., a suspension) may contain white to off-white opaque amorphous particles. In some embodiments, a thawed RNA (e.g., mRNA) composition may be used for up to a small number (e.g., 1, 2, 3, 4, 5, or 6) of days after thawing if maintained (e.g., stored) at a temperature at or below room temperature (e.g., below about 30 °C, 25 °C, 20 °C, 15 °C, 10 °C, 8 °C, 4 °C, etc). In some embodiments, a thawed RNA (e.g., mRNA) composition may be used after being stored (e.g., for such small number of days) at a temperature between about 2 °C and about 8 °C; alternatively or additionally, a thawed RNA (e.g., mRNA) composition may be used within a small number (e.g., 1, 2, 3, 4, 5, 6) of hours after thawing at room temperature. Thus, in some embodiments, a provided RNA (e.g., mRNA) composition that has been thawed and maintained at a temperature at or below room temperature, and in some embodiments between about 2 °C and about 8 °C, for not more than 6, 5, 4, 3, 2, or 1 days is administered to a subject. Alternatively or additionally, in some embodiments, a provided RNA (e.g., mRNA) composition that has been thawed and maintained at room temperature for not more than 6, 5, 4, 3, 2, or 1 hours is administered to a subject. In some embodiments, a provided RNA (e.g., mRNA) composition is shipped and/or stored in a concentrated state. In some embodiments, such a concentrated composition is diluted prior to administration. In some embodiments, a diluted composition is administered within a period of about 10, 9, 8, 7, 6, 5, 4, 3, 2, or 1 hour(s) post-dilution; in some embodiments, such administration is within 6 hours post-dilution. Thus, in some embodiments, diluted preparation of a provided RNA (e.g., mRNA) composition is administered to a subject within 6 hours post-dilution (e.g., as described herein after having been maintained at an appropriate temperature, e.g., at a temperature below room temperature, at or below 4 °C, at or below 0 °C, at or below -20 °C, at or below -60 °C, at or below -70 °C, at or below - 80 °C, etc, and typically at or above about 2 °C, for example between about 2 °C and about 8 °C or between about 2 °C and about 25 °C). In some embodiments, unused composition is discarded within several hours (e.g., about 10, about 9, about 8, about 7, about 6, about 5 or fewer hours) after dilution; in some embodiments, unused composition is discarded within 6 hours of dilution.

In some embodiments, an RNA (e.g., mRNA) composition that is stored, shipped or utilized (e.g., a frozen composition, a liquid concentrated composition, a diluted liquid composition, etc.) may have been maintained at a temperature materially above -60°C for a period of time of at least 1, 2, 3, 4, 5, 6, 7 days or more, or at least 1, 2, 3, 4, 5, 6, 7, 8, 9, 10 weeks or more, or at least 1, 2, 3, 4, 5, 6, 7, 8, 9, 10, 11, 12 months or more; in some such embodiments, such composition may have been maintained at a temperature at or above about -20°C for such period of time, and/or at a temperature up to or about 4-5°C for such period of time, and/or may have been maintained at a temperature above about 4-5°C, and optionally about 25°C for a period of time up that is less than two (2) months and/or optionally up to about one (1) month. In some embodiments, such composition may not have been stored, shipped or utilized (or otherwise exposed to) a temperature materially above about 4-5°C, and in particular not at or near a temperature of about 25°C for a period of time as long as about 2 weeks, or in some embodiments 1 week. In some embodiments, such composition may not have been stored, shipped or utilized (or otherwise exposed to) a temperature materially above about -20°C, and in particular not at or near a temperature of about 4-5°C for a period of time as long as about 12 months, 11 months, 10 months, 9 months, 8 months, 7 months, 6 months, 5 months, 4 months, 3 months, 2 months, or, in some embodiments, for a period of time as long as about 8 weeks or 6 weeks or materially more than about 2 months or, in some embodiments, 3 months or, in some embodiments 4 months.

In some embodiments, an RNA (e.g., mRNA) composition that is stored, shipped or utilized (e.g., a frozen composition, a liquid concentrated composition, a diluted liquid composition, etc.) may be protected from light. In some embodiments, one or more steps may be taken to reduce or minimize exposure to light for such compositions (e.g., which may be disposed within a container such as a vial or a syringe). In some embodiments, exposure to direct sunlight and/or to ultraviolent light is avoided. In some embodiments, a diluted solution may be handled and/or utilized under normal room light conditions (e.g., without particular steps taken to minimize or reduce exposure to room light). It should be understood that strict adherence to aseptic techniques is desirable during handling (e.g., diluting and/or administration) of an RNA (e.g., mRNA) composition as described herein. In some embodiments, an RNA (e.g., mRNA) composition as described herein is not administered (e.g., is not injected) intravenously. In some embodiments, an RNA (e.g., mRNA) composition as described herein is not administered (e.g., is not injected) intradermally. In some embodiments, an RNA (e.g., mRNA) composition as described herein is not administered (e.g., is not injected) subcutaneously. In some embodiments, an RNA (e.g., mRNA) composition as described herein is not administered (e.g., is not injected) any of intravenously, intradermally, or subcutaneously. In some embodiments, an RNA (e.g., mRNA) composition as described herein is not administered to a subject with a known hypersensitivity to any ingredient thereof. In some embodiments, a subject to whom an RNA (e.g., mRNA) composition has been administered is monitored for one or more signs of anaphylaxis. In some embodiments, a subject to whom an RNA (e.g., mRNA) composition is administered had previously received at least one dose of a different vaccine for SARS-CoV-2; in some embodiments, a subject to whom an RNA (e.g., mRNA) composition is administered had not previously received a different vaccine for SARS-CoV-2. In some embodiments, a subject's temperature is taken promptly prior to administration of an RNA (e.g., mRNA) composition (e.g., shortly before or after thawing, dilution, and/or administration of such composition); in some embodiments, if such subject is determined to be febrile, administration is delayed or canceled. In some embodiments, an RNA (e.g., mRNA) composition as described herein is not administered to a subject who is receiving anticoagulant therapy or is suffering from or susceptible to a bleeding disorder or condition that would contraindicate intramuscular injection. In some embodiments, an RNA (e.g., mRNA) composition as described herein is administered by a healthcare professional who has communicated with the subject receiving the composition information relating to side effects and risks. In some embodiments, an RNA (e.g., mRNA) composition as described herein is administered by a healthcare professional who has agreed to submit an adverse event report for any serious adverse events, which may include for example one or more of death, development of a disability or congenital anomaly/birth defect (e.g., in a child of the subject), in-patient hospitalization (including prolongation of an existing hospitalization), a life-threatening event, a medical or surgical intervention to prevent death, a persistent or significant or substantial disruption of the ability to conduct normal life functions; or another important medical event that may jeopardize the individual and may require medical or surgical intervention (treatment) to prevent one of the other outcomes. In some embodiments, provided RNA compositions are administered to a population of individuals under 18 years of age, or under 17 years of age, or under 16 years of age, or under 15 years of age, or under 14 years of age, or under 13 years of age, for example according to a regimen established to have a rate of incidence for one or more of the local reaction events indicated below that does not exceed the rate of incidence indicated below:
- pain at the injection site (75% after a first dose and/or a second dose, and/or a lower incidence after a second dose, e.g., 65% after a second dose);
- redness at the injection site (less than 5% after a first dose and/or a second dose); and/or
- swelling at the injection site (less than 5% after a first dose and/or a second dose).

In some embodiments, provided RNA compositions are administered to a population of individuals under 18 years of age, or under 17 years of age, or under 16 years of age, or under 15 years of age, or under 14 years of age, or under 13 years of age, for example according to a regimen established to have a rate of incidence for one or more of the systemic reaction events indicated below that does not exceed the rate of incidence indicated below:
- fatigue (55% after a first dose and/or a second dose);
- headache (50% after a first dose and/or a second dose);
- muscle pain (40% after a first dose and/or a second dose);
- chills (40% after a first dose and/or a second dose);
- joint pain (20% after a first dose and/or a second dose);
- fever (25% after a first dose and/or a second dose);
- vomiting (10% after a first dose and/or a second dose); and/or
- diarrhea (10% after a first dose and/or a second dose).

In some embodiments, medication that alleviates one or more symptoms of one or more local reaction and/or systemic reaction events (e.g., described herein) are administered to individuals under 18 years of age, or under 17 years of age, or under 16 years of age, or under 15 years of age, or under 14 years of age, or under 13 years of age who have been administered with provided RNA compositions and have experienced one or more of the local and/or systemic reaction events (e.g., described herein). In some embodiments, antipyretic and/or pain medication can be administered to such individuals.

### Brief description of the drawings

**Figure 1****. Schematic overview of the S protein organization of the SARS-CoV-2 S protein.** The sequence within the S1 subunit consists of the signal sequence (SS) and the receptor binding domain (RBD) which is the key subunit within the S protein which is relevant for binding to the human cellular receptor ACE2. The S2 subunit contains the S2 protease cleavage site (S2') followed by a fusion peptide (FP) for membrane fusion, heptad repeats (HR1 and HR2) with a central helix (CH) domain, the transmembrane domain (TM) and a cytoplasmic tail (CT).

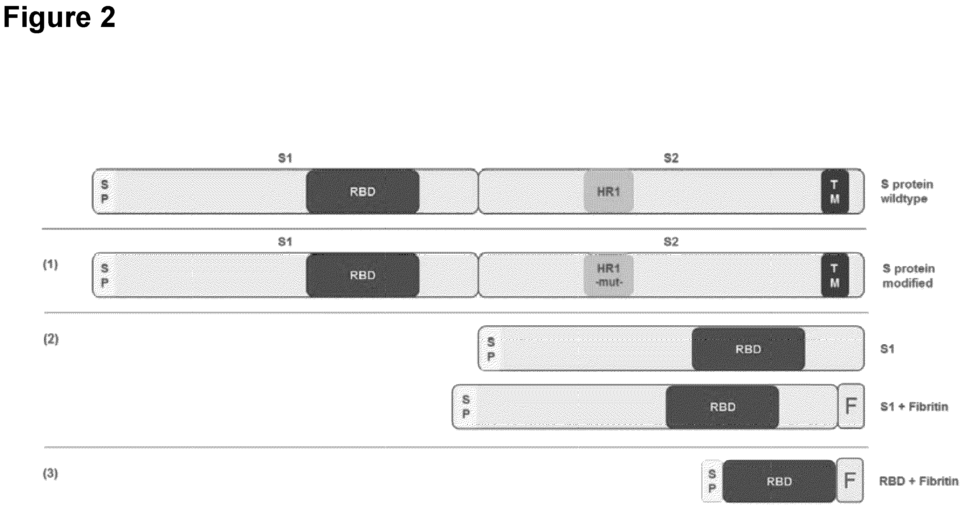

**Figure 2****. Exemplary SARS-CoV-2 vaccine constructs.**
   Based on the full and wildtype S protein, we have designed different constructs encoding the (1) full protein with mutations in close distance to the first heptad repeat (HRP1) that include stabilizing mutations preserving neutralisation sensitive sites, the (2) S1 domain or the (3) RB domain (RBD) only. Furthermore, to stabilize the protein fragments a fibritin domain (F) was fused to the C-terminus. All constructs start with the signal peptide (SP) to ensure Golgi transport to the cell membrane.

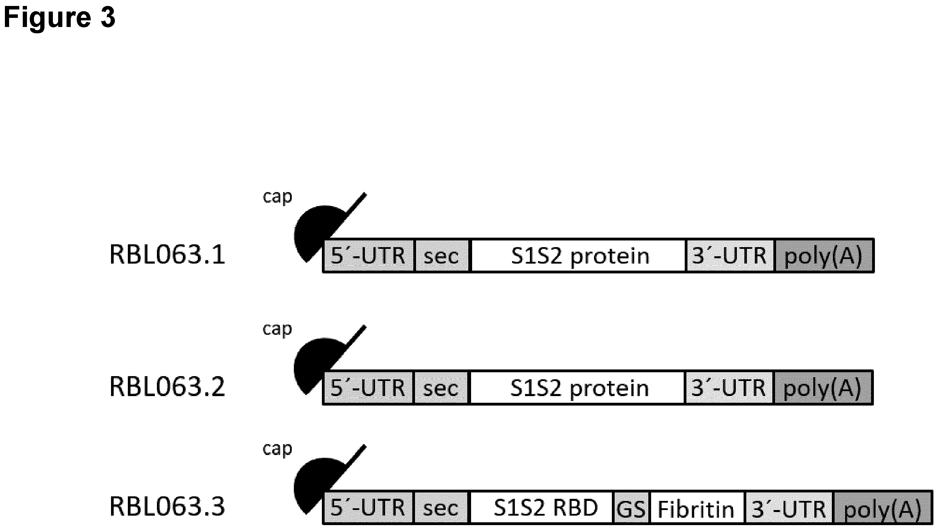

**Figure 3****. General structure of the RNA.**
   Schematic illustration of the general structure of the RNA vaccines with 5'-cap, 5'- and 3'-untranslated regions, coding sequences with intrinsic secretory signal peptide as well as GS-linker, and poly(A)-tail. Please note that the individual elements are not drawn exactly true to scale compared to their respective sequence lengths.
   UTR = Untranslated region; sec = Secretory signal peptide; RBD = Receptor Binding Domain; GS = Glycine-serine linker.

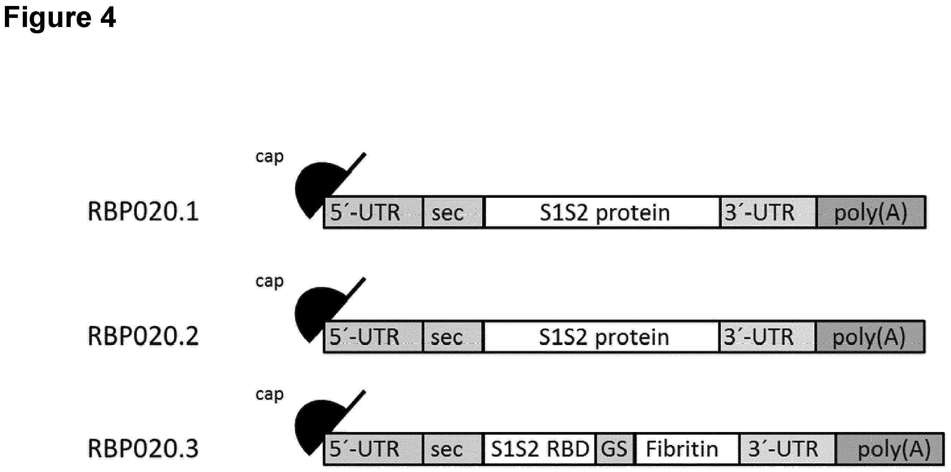

**Figure 4****. General structure of the RNA.**
   Schematic illustration of the general structure of RNA drug substances with 5'-cap, 5'- and 3'-untranslated regions, coding sequences with intrinsic secretory signal peptide as well as GS-linker, and poly(A)-tail. Please note that the individual elements are not drawn exactly true to scale compared to their respective sequence lengths.
   GS = Glycine-serine linker; UTR = Untranslated region; Sec = Secretory signal peptide; RBD = Receptor Binding Domain.

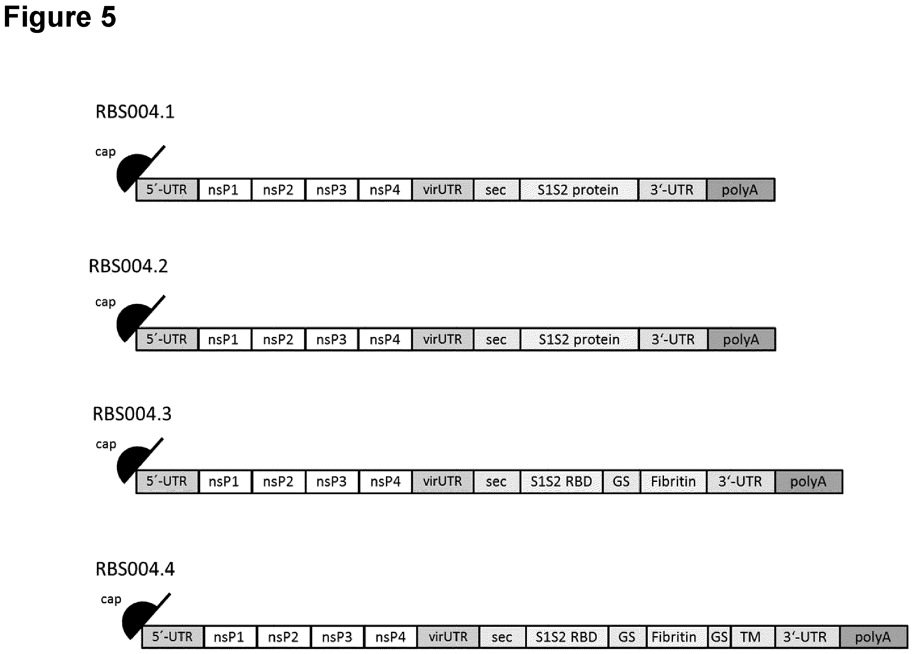

**Figure 5****. General structure of the RNA.**
   Schematic illustration of the general structure of RNA vaccines with 5'-cap, 5'- and 3'-untranslated regions, coding sequences of the Venezuelan equine encephalitis virus (VEEV) RNA-dependent RNA polymerase replicase and the SARS-CoV-2 antigen with intrinsic secretory signal peptide as well as GS-linker, and poly(A)-tail. Please note that the individual elements are not drawn exactly true to scale compared to their respective sequence lengths. UTR = Untranslated region; Sec = Secretory signal peptide; RBD = Receptor Binding Domain; GS = Glycine-serine linker.

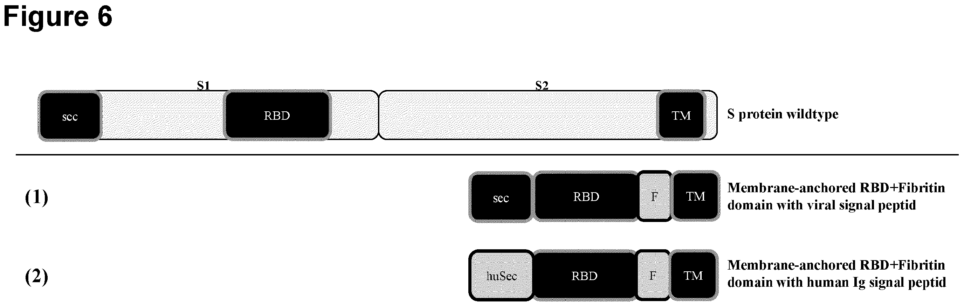

**Figure 6****. Schematic overview of the S protein organization of the SARS-CoV-2 S protein and constructs for the development of a SARS-CoV-2 vaccine.**
   Based on the wildtype S protein, we have designed two different transmembrane-anchored RBD-based vaccine constructs encoding the RBD fragment fused to the T4 fibritin trimerization domain (F) and the autochthonus transmembrane domain (TM). Construct (1) starts with the SARS-CoV-2-S signal peptide (SP; AA 1-19 of the S protein) whereas construct (2) starts with the human Ig heavy chain signal peptide (huSec) to ensure Golgi transport to the cell membrane.

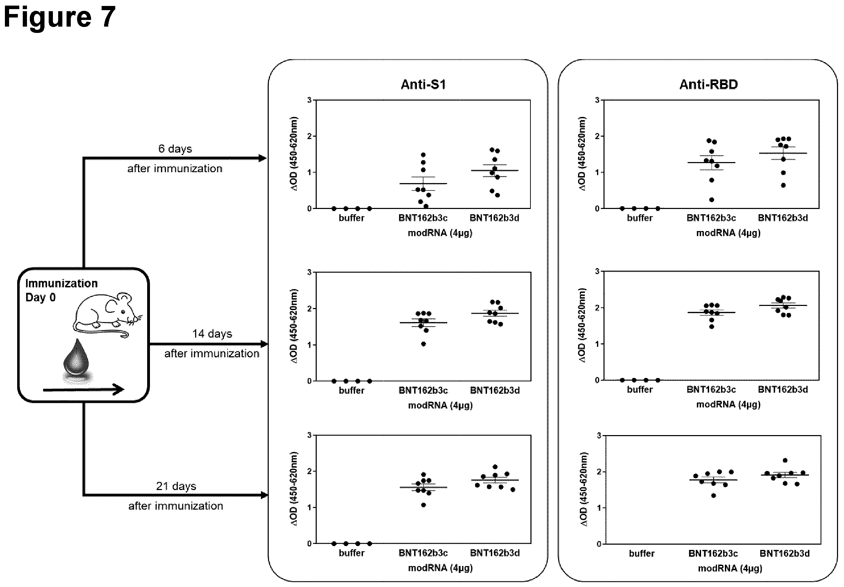

**Figure 7****. Anti-S protein IgG response 6, 14 and 21 d after immunization with LNP-C12 formulated modRNA coding for transmembrane-anchored RBD-based vaccine constructs.** BALB/c mice were immunized IM once with 4 µg of LNP-C12-formulated transmembrane-anchored RBD-based vaccine constructs (surrogate to BNT162b3c/BNT162b3d). On day 6, 14 and 21 after immunization, animals were bled and the serum samples were analyzed for total amount of anti-S1 (left) and anti-RBD (right) antigen specific immunoglobulin G (IgG) measured via ELISA. For day 6 (1:50), day 14 (1:300) and day 21 (1:900) different serum dilution were included in the graph. One point in the graph stands for one mouse, every mouse sample was measured in duplicates (group size n=8; mean + SEM is included for the groups).

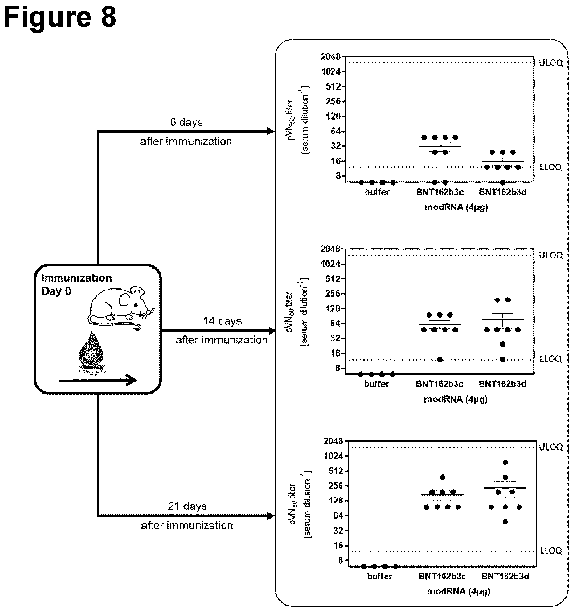

**Figure 8****. Neutralization of SARS-CoV-2 pseudovirus 6, 14 and 21 d after immunization with LNP-C12 formulated modRNA coding for transmembrane-anchored RBD-based vaccine constructs.**
   BALB/c mice were immunized IM once with 4 µg of LNP-C12-formulated transmembrane-anchored RBD-based vaccine constructs (surrogate to BNT162b3c/BNT162b3d). On day 6, 14 and 21 after immunization, animals were bled and the sera were tested for SARS CoV-2 pseudovirus neutralization. Graphs depict pVNso serum dilutions (50% reduction of infectious events, compared to positive controls without serum). One point in the graphs stands for one mouse. Every mouse sample was measured in duplicate. Group size n=8. Mean + SEM is shown by horizontal bars with whiskers for each group. LLOQ, lower limit of quantification. ULOQ, upper limit of quantification.

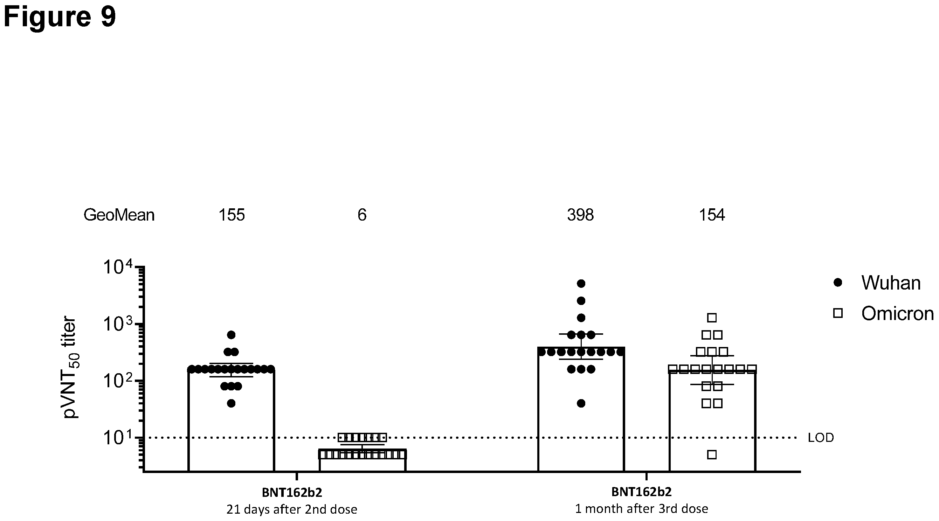

**Fig. 9**. 50% pseudovirus neutralization titers (pVNT**₅₀) of sera collected 21 days after the second dose and 1 month after the third dose of BNT162b2 against VSV-SARS-CoV-2-S** pseudovirus bearing the Wuhan Hu-1 reference or Omicron lineage spike protein. N=19-20 sera from immunized subjects collected either 21 days after the second BNT162b2 dose or 1 months after the third BNT162b2 dose were tested. For values below the limit of detection (LOD; 10), LOD/2 values are plotted. Group GMTs (values above bars) with 95% confidence intervals are shown.

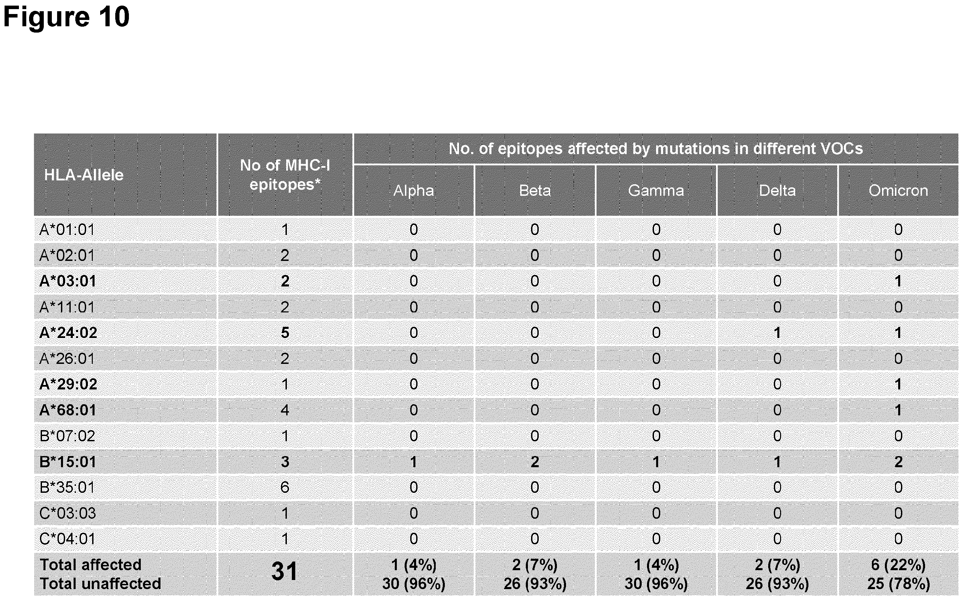

**Fig. 10****. CD8+ T cell epitopes in BNT162b2 vaccine remain largely unaffected by Omicron BA.1 variant mutations.** Shown is the number of previously identified MHC-I epitopes affected in various variants of concern (VOCs). Approximately 80% of previously identified CD8+ epitopes are not affected by the mutations in the BA.1 Omicron variant, suggesting that two doses of BNT162b2 may still induce protection against severe disease.

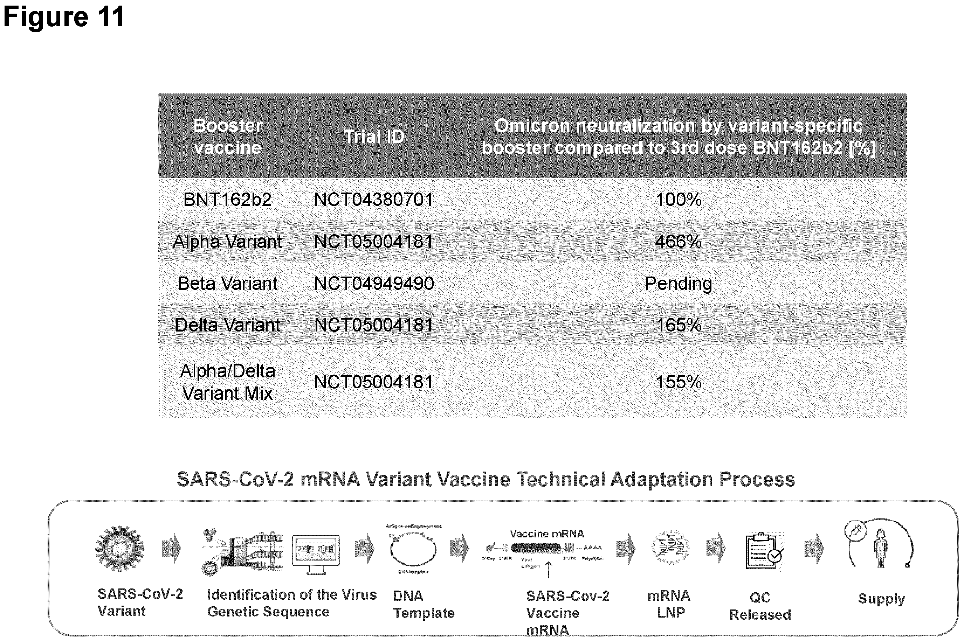

**Fig. 11****. Neutralization of Omicron BA.1 after two doses of BNT162b2 and variant specific booster.** Shown is neutralization of the Omicron BA.1 variant from sera of patients administered two doses of BNT162b2 and (i) a third booster dose of BNT162b2, or (ii) a third booster dose of an RNA encoding a Spike protein with alpha or delta variant mutations, or a third booster dose of both a Spike protein comprising alpha mutations and a Spike protein comprising delta mutations. The values are derived from separate neutralization GMTs from the pseudovirus testing. Also shown is a schematic depicting a process for developing new SARS-CoV-2 variant specific vaccines.

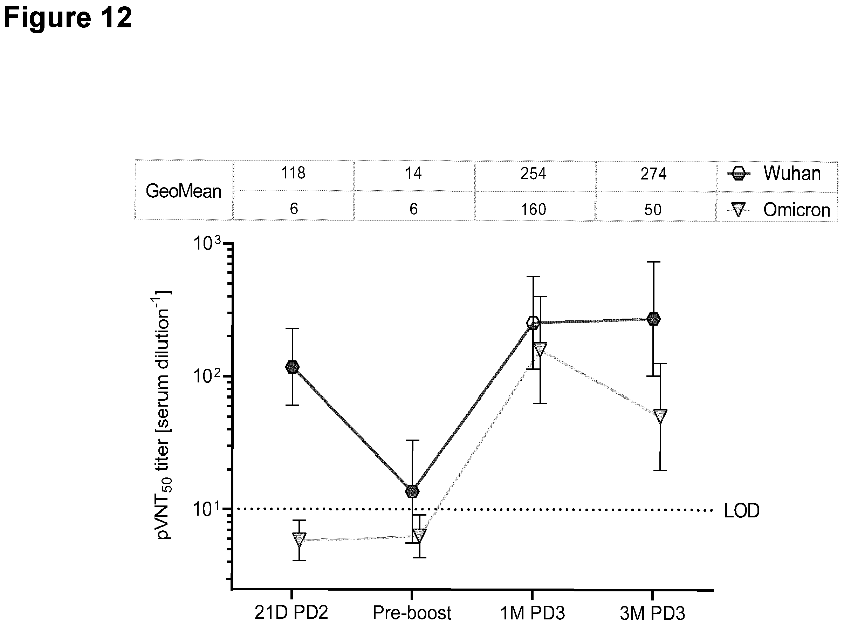

**Fig. 12****. Longitudinal analysis of neutralizing antibody responses against VSV-SARS-CoV-2-S pseudovirus bearing the Wuhan or Omicron BA.1 variant spike protein in a subset of study participants.** Sera from n=9 participants drawn at 21 days after dose 2, prior to dose 3, 1 month after dose 3 and 3 months after dose 3 were tested. Each serum was tested in duplicate and individual geometric mean 50% pseudovirus neutralizing titers (GMTs) were calculated. For values below the limit of detection (LOD), LOD/2 values were assigned. Group GMTs (values in table) and 95% confidence intervals per timepoint are indicated.

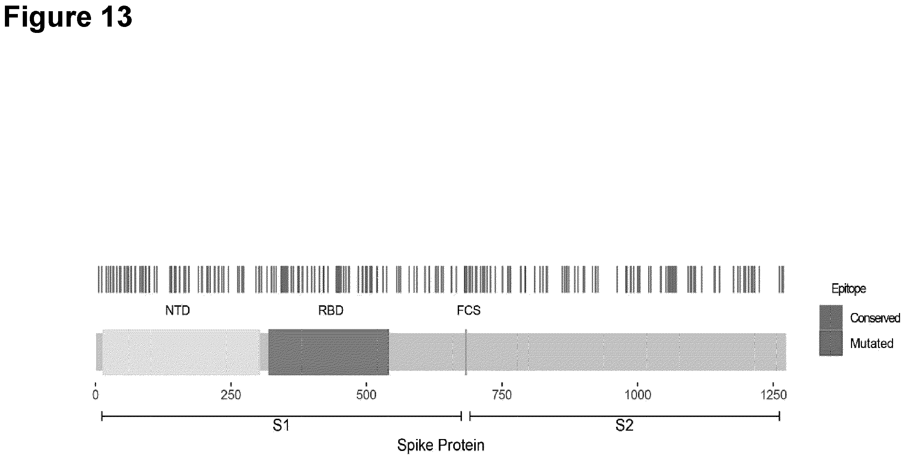

**Fig. 13****. Analysis of HLA class I T cell epitopes conservation between the Wuhan and Omicron BA.1 variants.** HLA class I restricted Spike protein epitopes with T cell reactivity identified based on their recognition by CD8+ T cells and reported in IEDB (n=244) are plotted by their position (top row) along the Spike protein (bottom row). Epitope indications are positioned by the amino acid position of the center of the epitope; epitopes conserved in both variants are marked in light grey (n=208); while epitopes spanning an Omicron BA.1 mutation site are marked dark grey (n=36). NTD=N-terminal domain; RBD=Receptor-binding domain; FCS=Furin cleavage site. The S1 and S2 regions of the Spike protein are indicated.

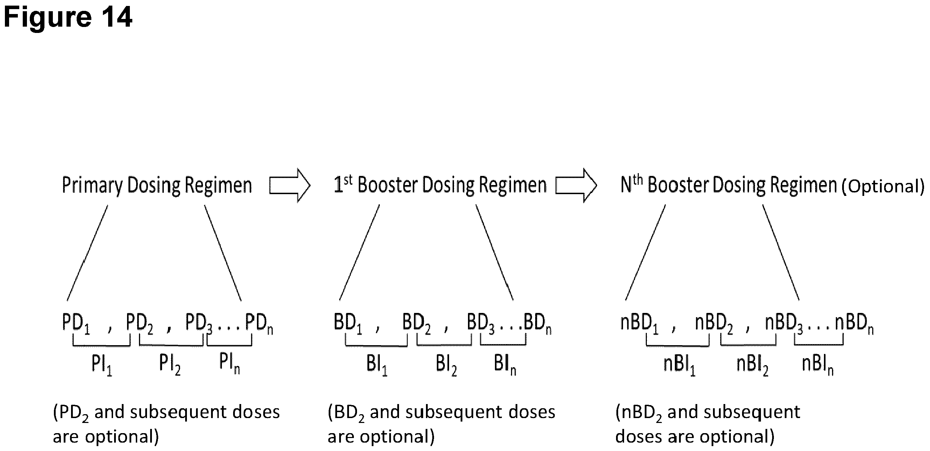

**Fig. 14****. Schematics of an exemplary vaccination regimen.**

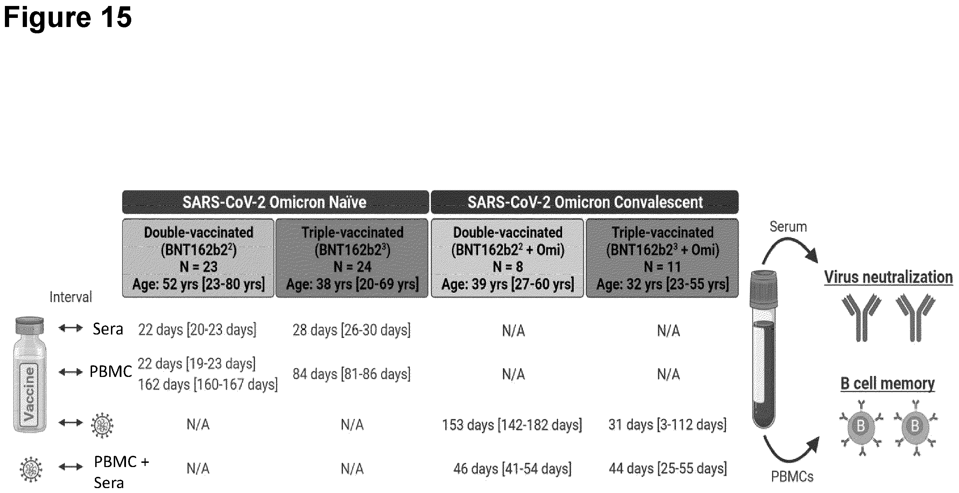

**Fig. 15****. Cohorts, sampling and experimental setup for characterization of immune response in Omicron breakthrough cases.** Blood samples were drawn from four cohorts: Omicron-naïve individuals double- or triple-vaccinated with BNT162b2, and individuals double- or triple-vaccinated with BNT162b2 that subsequently had a breakthrough infection with Omicron BA.1. PBMCs and sera were isolated in the Omicron-naïve cohorts at the time-points indicated following their most recent vaccination; for convalescent cohorts, the time from their most recent vaccination to Omicron BA.1 infection, and infection to PBMC and serum isolation are indicated (all values specified as median-range). Serum neutralizing capacity was assessed using a pseudovirus and live virus neutralization test; SARS-CoV-2 spike-specific B_{MEM} cells were assessed via a flow cytometry-based B cell phenotyping assay using bulk PBMCs. N/A, not applicable.

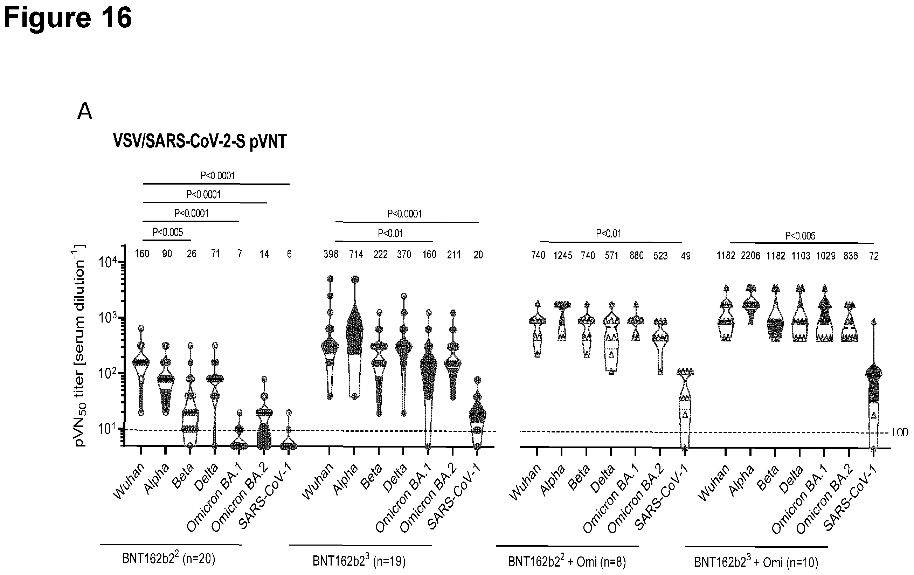

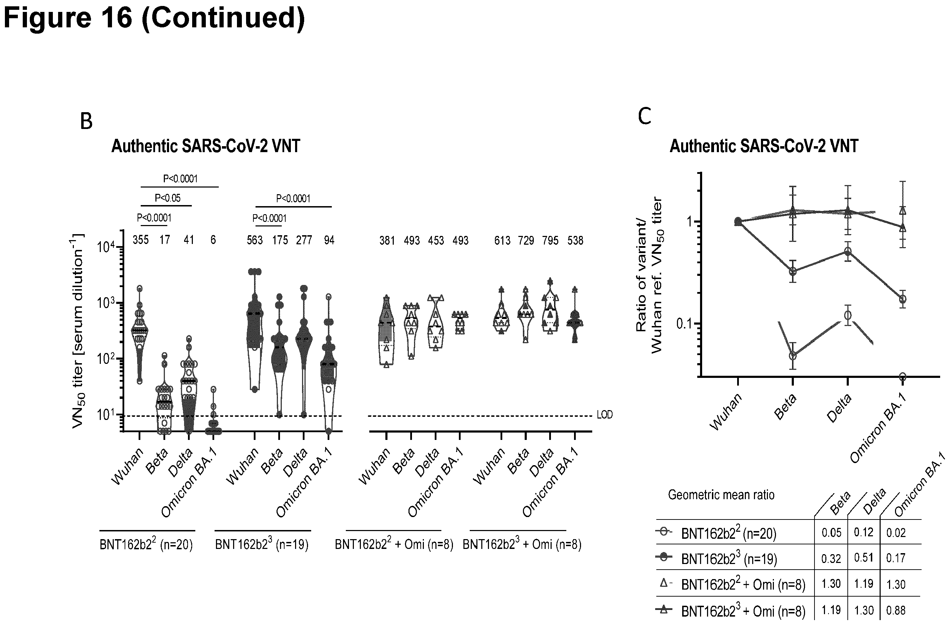

**Fig. 16****. Omicron breakthrough infection in BNT162b2 double- and triple-vaccinated individuals induces broad neutralization of Omicron BA.1, BA.2 and other VOCs.**
   Serum was drawn from double-vaccinated individuals (BNT162b2²) at 22 days after the second dose (open circles), from triple-vaccinated individuals (BNT162b2³) at 28 days after the third dose (closed circles), from double-vaccinated individuals with an Omicron breakthrough infection (BNT162b2² + Omi) at 46 days post-infection (open triangles), and from triple-vaccinated individuals and Omicron breakthrough infection (BNT162b2³ + Omi) at 44 days post-infection (closed triangles). Serum was tested in duplicate; (A) shows 50% pseudovirus neutralization (pVN₅₀) geometric mean titers (GMTs), (B) shows 50% virus neutralization (VN₅₀) GMTs, and (C) shows the geometric mean ratio of SARS-CoV-2 variant of concern (VOC) and Wuhan VN₅₀ GMTs. For titer values below the limit of detection (LOD), LOD/2 values were plotted. Values above violin plots represent group GMTs. The non-parametric Friedman test with Dunn's multiple comparisons correction was used to compare Wuhan neutralizing group GMTs with titers against the indicated variants and SARS-CoV-1. Multiplicity-adjusted p values are shown. (A) pVNso GMTs against Wuhan, VOC and SARS-CoV-1 pseudovirus. (B) VN₅₀ GMTs against live SARS-CoV-2 Wuhan, Beta, Delta and Omicron BA.1. (C) Group geometric mean ratios with 95% confidence intervals for all cohorts shown in (B).

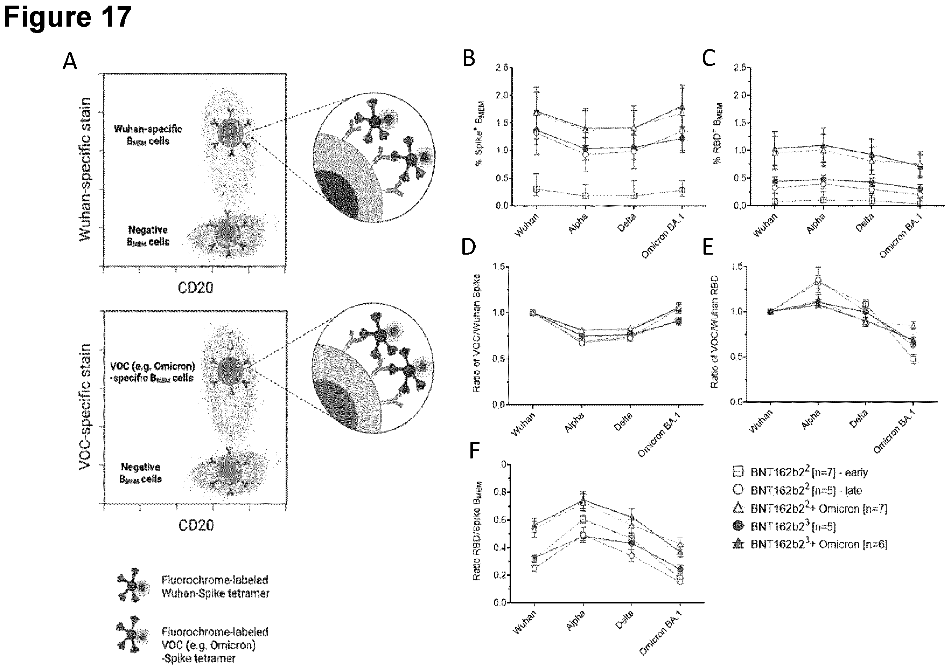

**Fig. 17****. B_{MEM} cells of individuals double- and triple-vaccinated with BNT162b2 broadly recognize VOCs and are further boosted by Omicron breakthrough infection.** PBMC samples from double-vaccinated individuals (BNT162b2²) at 22 days after the second dose (open squares) and 5 months after the second dose (open circles), from triple-vaccinated individuals (BNT162b2³) at 84 days after the third dose (closed circles), from double-vaccinated individuals with Omicron breakthrough infection (BNT162b2² + Omi) at 46 days post-infection (open triangles), and from triple-vaccinated individuals with Omicron breakthrough infection (BNT162b23 + Omi) at 44 days post-infection (closed triangles) were analyzed via flow cytometry for SARS-CoV-2-specific B_{MEM} cell (B_{MEM} - CD3-CD19+CD20+IgD-CD38^{int/low}) frequencies via B cell bait staining. (A) Schematic of one-dimensional staining of B_{MEM} cells with fluorochrome-labeled SARS-CoV-2 S protein tetramer bait allowing discrimination of variant recognition. Frequencies of Wuhan or VOC full-length S protein- (B) and RBD- (C) specific B_{MEM} cells for the four groups of individuals were analyzed. Variant-specific B_{MEM} cell frequencies were normalized to Wuhan frequencies for S protein (D) and RBD- (E) binding. (F) Depicts the frequency ratios of RBD protein specific B_{MEM} cells over full-length S protein-specific BMEM cells.

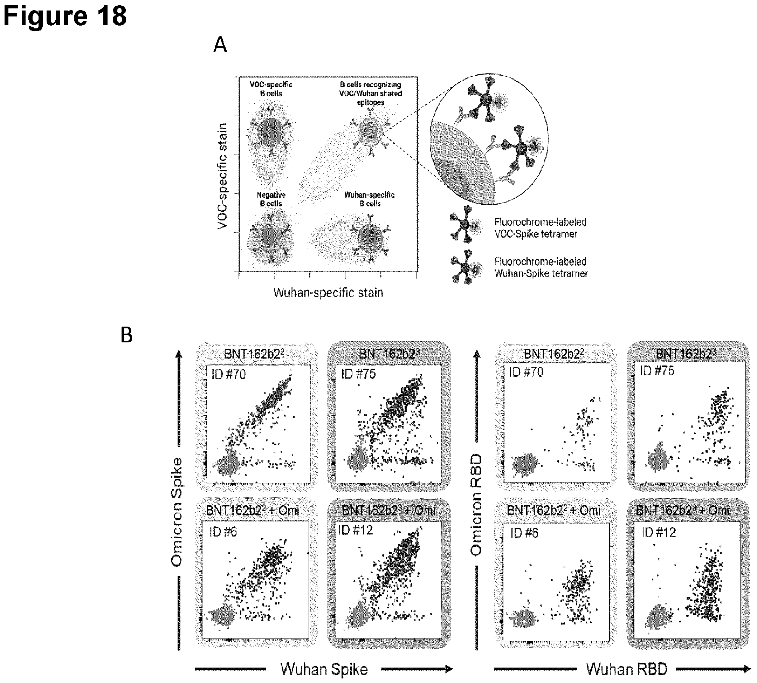

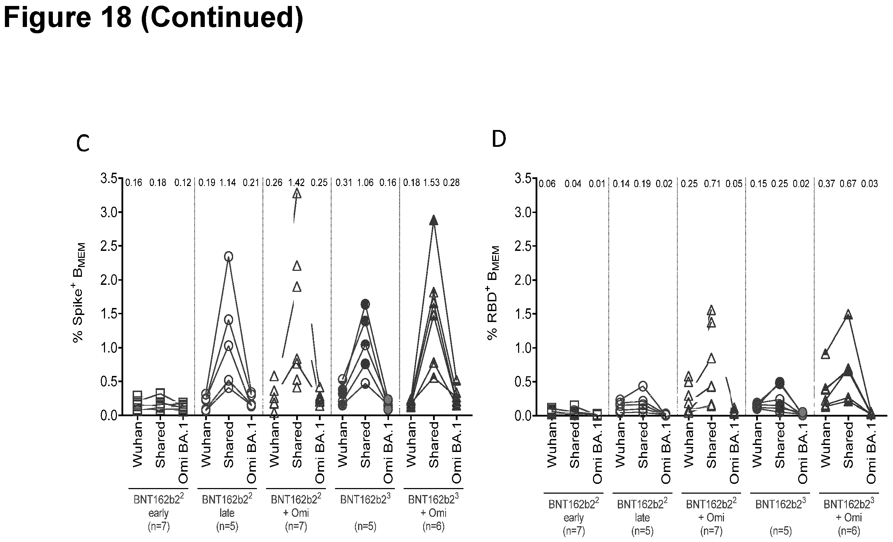

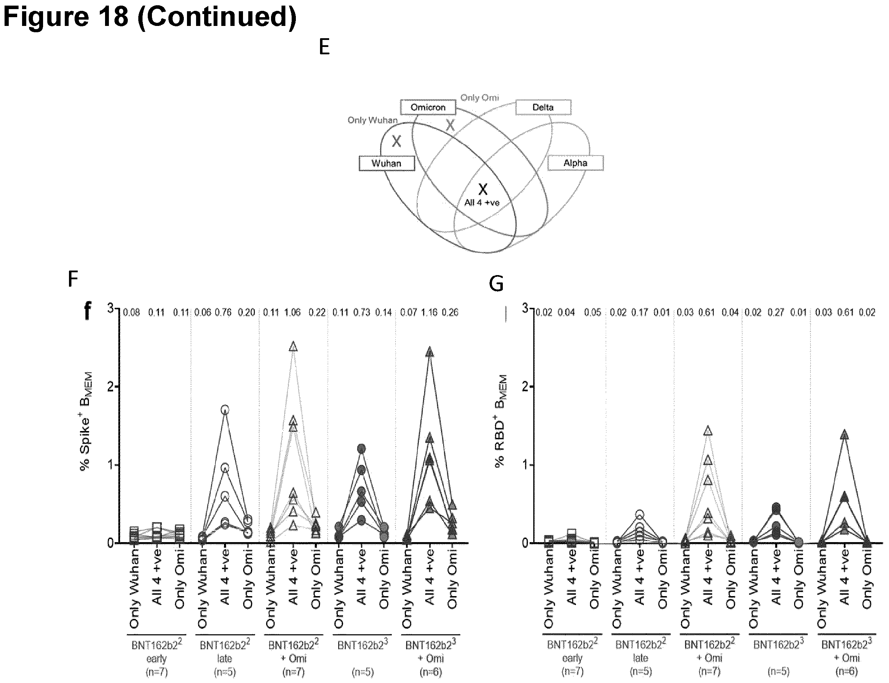

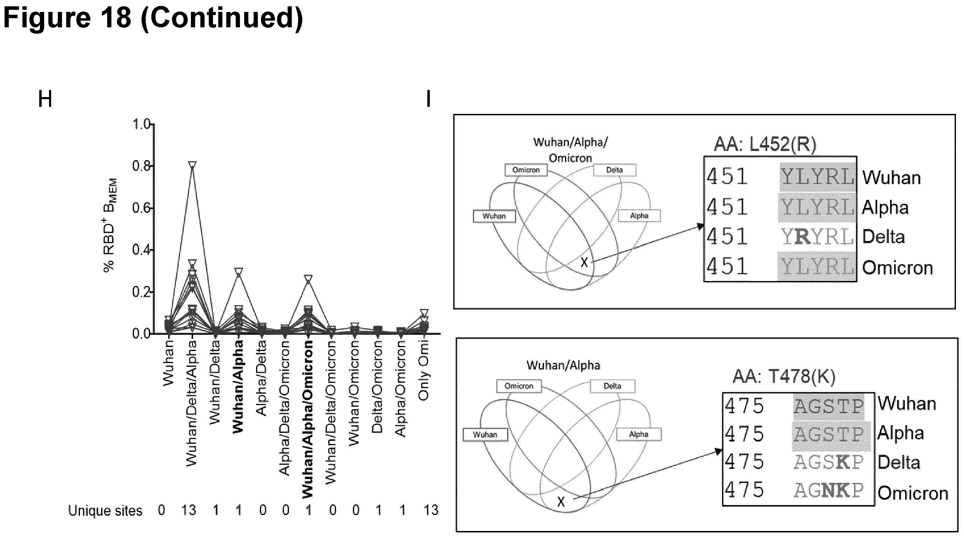

**Fig. 18****. Omicron breakthrough infection of BNT162b2 double- and triple-vaccinated individuals primarily boosts B_{MEM} against conserved epitopes shared broadly between S proteins of Wuhan and other VOCs rather than strictly Omicron S-specific epitopes.** PBMC samples from double-vaccinated individuals (BNT162b2²) at 22 days after the second dose (open squares) and 5 months after the second dose (open circles), from triple-vaccinated individuals (BNT162b2³) at 84 days after the third dose (closed circles), from double-vaccinated individuals with Omicron breakthrough infection (BNT162b2² + Omi) at 46 days post-infection (open triangles), and from triple-vaccinated individuals with Omicron breakthrough infection (BNT162b2³ + Omi) at 44 days post-infection (closed triangle) were analyzed via flow cytometry for SARS-CoV-2-specific memory B cell (B_{MEM} - CD3-CD19+CD20+IgD-CD38^{int/low}) frequencies via B cell bait staining (schematic shown in (A)). (B) shows representative flow plots of Omicron and Wuhan S protein- and RBD-binding for each of the four groups of individuals investigated. Frequencies of B_{MEM} binding Omicron, Wuhan, or both (shared) shown for full-length S protein in (C) and RBD shown in (D) for Omicron-experienced and naïve BNT162b2 double and triple vaccinees. (E) Venn diagrams visualizing the combinatorial (Boolean) gating strategy to identify cross-reactive B_{MEM} recognizing all four variants simultaneously (All 4 +ve) and B_{MEM} recognizing only Omicron (only Omi) or only Wuhan (only Wuhan) S proteins. Frequencies for these three B_{MEM} sub-groups were compared for full-length S protein (F) and RBD (G) in the four different groups of individuals investigated. RBD variant recognition pattern by B_{MEM} was assessed through Boolean flow cytometric gating strategy and frequencies recognizing combinations of Wuhan and/or variant RBDs are displayed in (H), for all Omicron convalescent subjects (double and triple vaccinees pooled, n=13). (I) Conserved site within the RBD domain recognized by RBD-specific B_{MEM} after Omicron break-through infection. Mean values are indicated in C, D, F, and G. n = number of individuals per group.

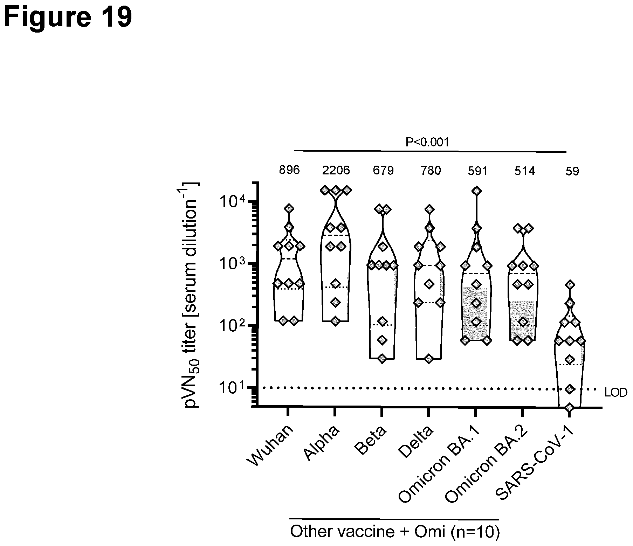

**Fig. 19****. Omicron breakthrough infection of individuals vaccinated with other approved COVID-19 vaccines or mixed regimens results in immune sera that broadly neutralize Omicron BA.1, BA.2 and other VOCs plus SARS-CoV-1.** Serum was drawn from 10 individuals vaccinated with other approved COVID-19 vaccines or mixed regimens at a median of 43 days after infection (diamonds). Serum was tested in duplicate; individual 50% pseudovirus neutralization (pVN₅₀) geometric mean titers (GMTs) against SARS-CoV-2 Wuhan, Alpha, Beta, Delta and Omicron BA.1 and BA.2 variants, plus SARS-CoV-1 were plotted. For titer values below the limit of detection (LOD), LOD/2 values were plotted. Values above violin plots represent group GMTs. The non-parametric Friedman test with Dunn's multiple comparisons correction was used to compare Wuhan neutralizing group GMTs with titers against the indicated variants and SARS-CoV-1. Multiplicity-adjusted p values are shown. Approved vaccines included AZD1222, BNT162b2 (in some embodiments as part of a 4-dose series), Ad26.COV2.S, mRNA-1273 (administered as a two-dose or three-dose series), and combinations thereof.

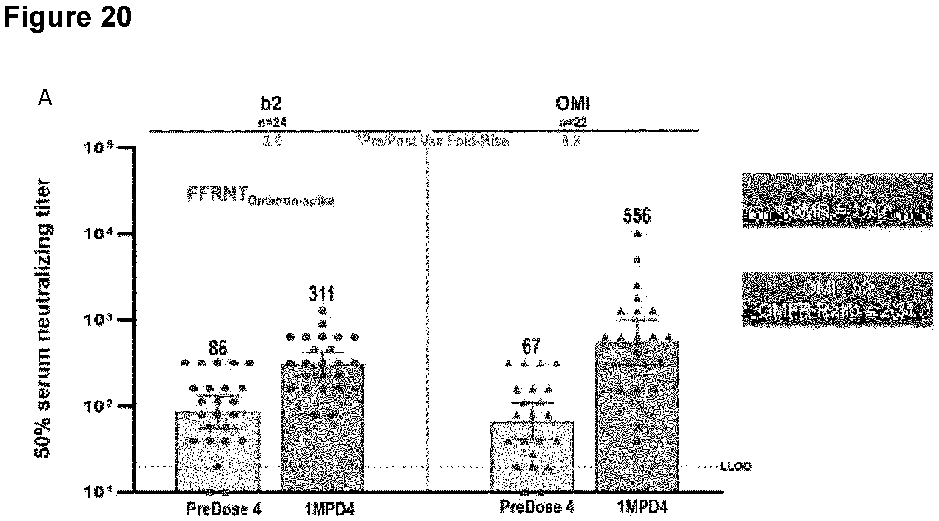

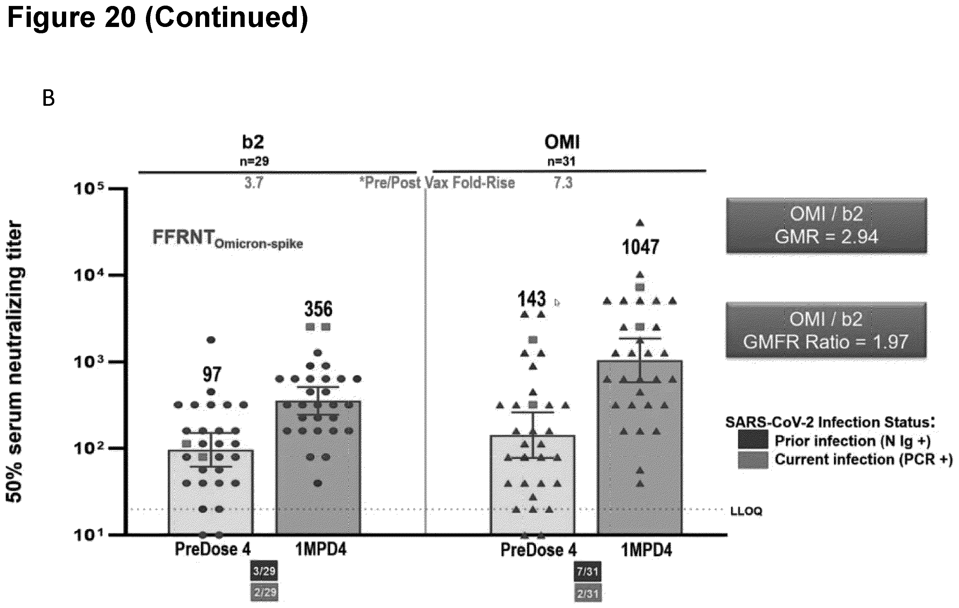

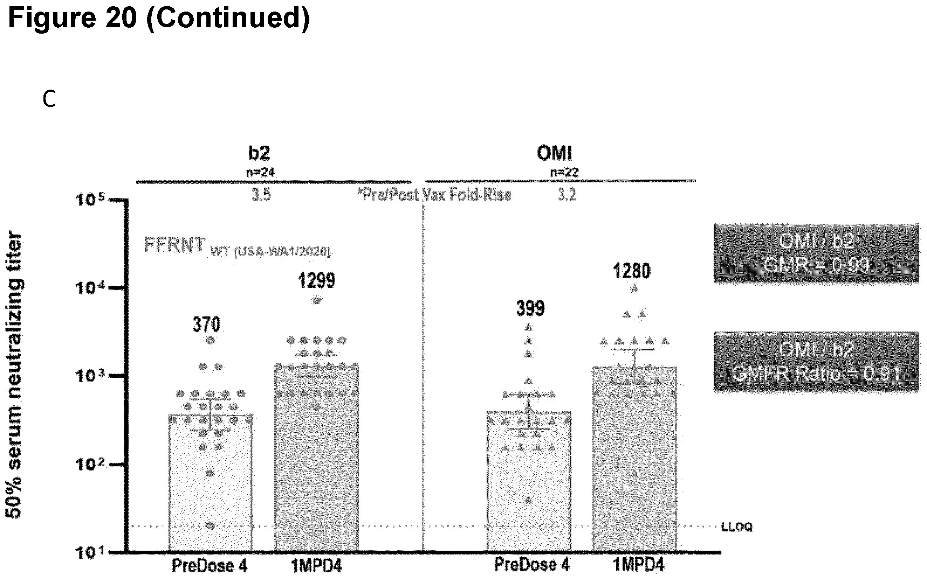

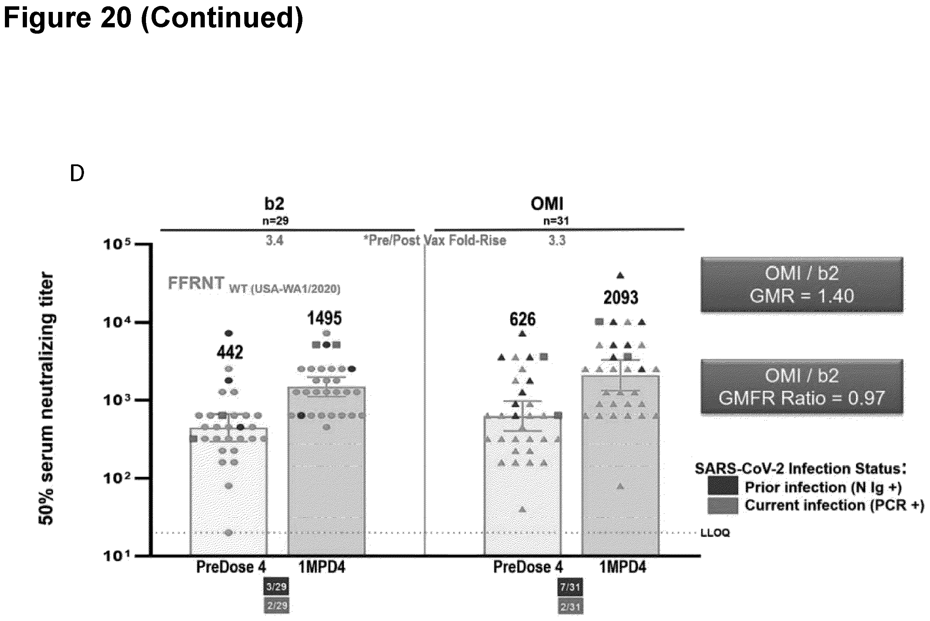

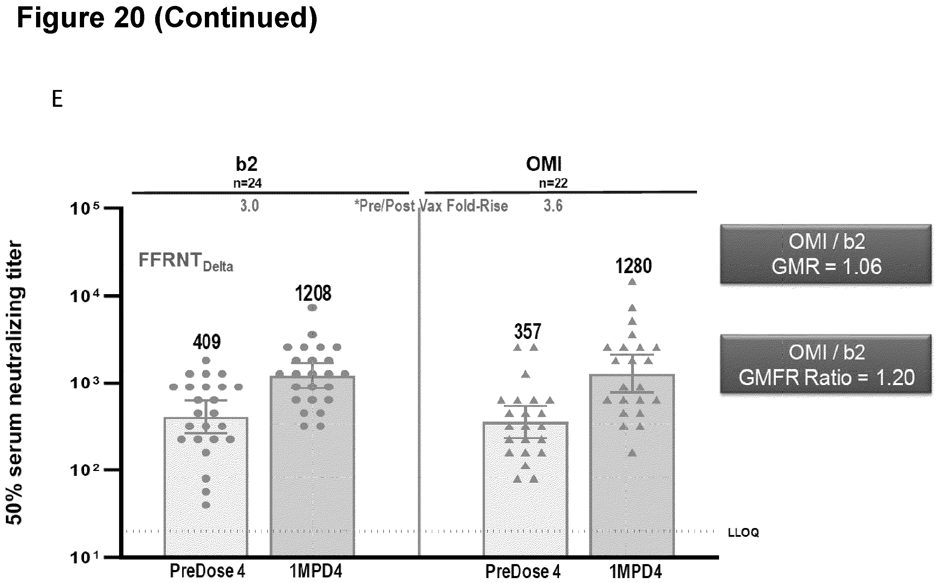

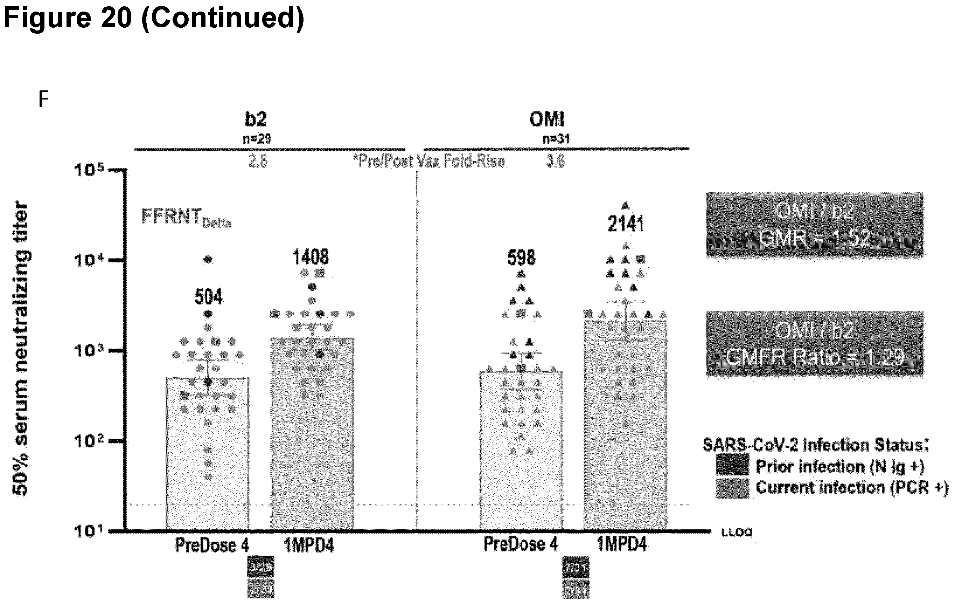

**Fig. 20**. 50% neutralization titers of sera collected 1 month after a fourth dose of BNT162b2 **or an Omicron BA.1-specific booster.** Subjects who were previously administered two doses of BNT162b2, and a third (booster) dose of BNT162b2 (30 ug) received a dose (30 ug) of (i) an RNA encoding a SARS-CoV-2 S protein from an Omicron BA.1 variant (e.g., as described herein (referred to herein as "Omicron BA.1-specific RNA vaccine"), or (ii) BNT162b2, as a fourth (boster) dose. Serum from the subjects were collected one month after administration of the 4th (booster) dose. Group GMTs (values above bars) with 95% confidence intervals are shown. "b2" refers to sera from subjects administered Wuhan-specific RNA vaccine as the 4^{th} (booster) dose of BNT162b2. "OMI" refers to sera from subjects administered an Omicron BA.1-specific 4^{th} (booster) dose. Also shown is the fold-change in titer from before administration of the 4^{th} dose to after administration of the 4^{th} dose (Pre/Post Vax Fold-Rise), and the ratio of geometric mean ratio (GMR) and geometric mean fold rise (GMFR) observed in subjects administered a 4^{th} dose of an Omicron BA.1-specific RNA vaccine as the 4^{th} dose, as compared to subjects administered BNT162b2 as the 4^{th} dose. "FFRNT" refers to fluorescent focus reduction neutralization test. Neutralization data was obtained using an FFRNT assay, with a viral particle containing a SARS-CoV-2 S protein from the variant indicated in the figures. (A) Comparison of titers of neutralizing antibodies against a SARS-CoV-2-S pseudovirus comprising a SARS-CoV-2 S protein having mutations characteristics of an Omicron BA.1 variant. Sera from subjects previously or currently infected with SARS-CoV-2 excluded. (B) Comparison of titers of neutralizing antibodies against a SARS-CoV-2 pseudovirus comprising a SARS-CoV-2 S protein having mutations characteristics of an Omicron BA.1 variant in sera from a population that includes subjects previously or currently infected with SARS-CoV-2 (as determined by an antigen assay or a PCR assay respectively). (C) Comparison of titers of neutralizing antibodies against a SARS-CoV-2 pseudovirus comprising a SARS-CoV-2 S protein from a Wuhan strain. Sera from subjects previously or currently infected with SARS-CoV-2 excluded. (D) Comparison of titers of neutralizing antibodies against a SARS-CoV-2 pseudovirus comprising a SARS-CoV-2 S protein from a Wuhan strain, in sera from a population comprising individuals previously or currently infected with SARS-CoV-2 (as determined by an antigen assay or a PCR assay, respectively. (E) Comparison of titers of neutralizing antibodies against a SARS-CoV-2 pseudovirus comprising a SARS-CoV-2 S protein having mutations characteristics of a delta variant. Sera from subjects previously or currently infected with SARS-CoV-2 excluded. (F) Comparison of titers of neutralization antibodies against a SARS-CoV-2 pseudovirus comprising a SARS-CoV-2 protein having mutations characteristic of a delta variant, in sera from a population including subjects previously or currently infected with SARS-CoV-2 (as determined by an antigen assay or a PCR assay, respectively).

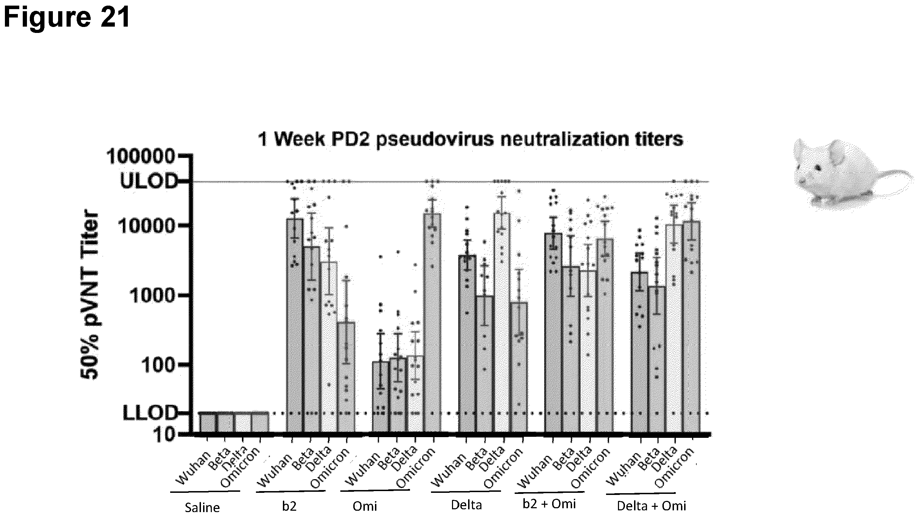

**Fig. 21****. Neutralization of SARS-CoV-2 pseudovirus 7 days after immunization with modRNA coding for variant specific S proteins.** Mice were immunized twice with LNP-formulated vaccine comprising (i) BNT162b2 (encoding a SARS-CoV-2 S protein from a Wuhan strain), (ii) RNA encoding a SARS-CoV-2 S protein having mutations characteristic of an Omicron BA.1 variant (Omi), (iii) RNA encoding an S protein having mutations characteristic of a delta variant, (iv) a combination of BNT162b2 and an RNA encoding an protein having mutations characteristic of an Omicron BA.1 variant (B2+Omi), or (v) RNA encoding a SARS-CoV-2 S protein having mutations characteristic of a delta variant and RNA encoding a SARS-CoV-2 S protein having mutations characteristic of an Omicron BA.1 variant (Delta + Omi). 7 days after the second immunization, animals were bled and sera was tested for neutralization of a SARS-CoV-2-S pseudovirus comprising a SARS-CoV-2 S protein from a Wuhan strain, or a SARS-CoV-2 S protein having mutations characteristic of a beta, delta, or Omicron BA.1 variant. Graphs depict pVN₅₀ serum dilutions (50% reduction of infectious events, compared to positive controls without serum). One point in the graphs stands for one mouse. Every mouse sample was measured in duplicate. Mean + SEM is shown by horizontal bars with whiskers for each group. LLOD, lower limit of detection. ULOD, upper limit of detection.

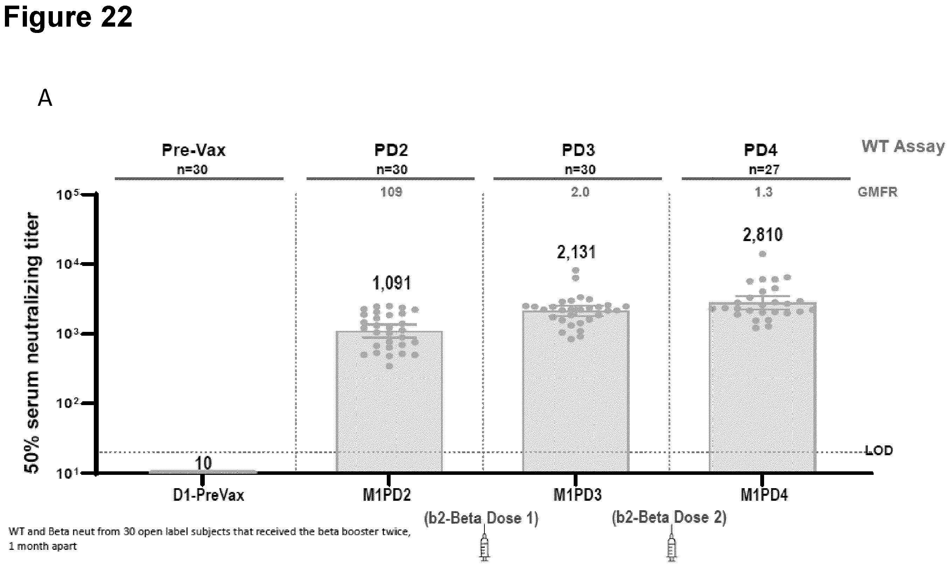

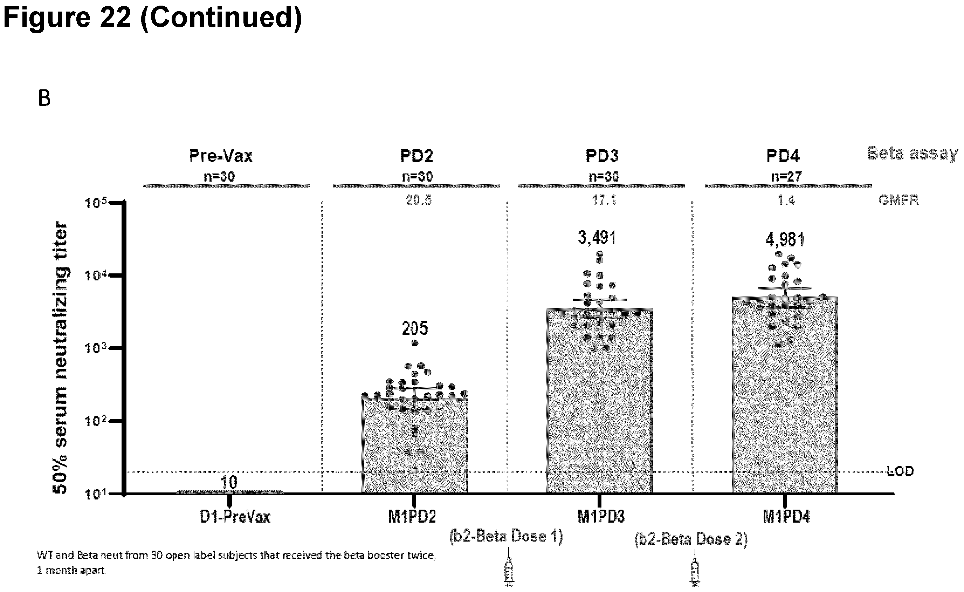

**Fig. 22****. RNA encoding a SARS-CoV-2 S protein having mutations characteristic of a Beta variant increases neutralization antibody titers against SARS-CoV-2 when administered to patients previously administered two doses of a vaccine encoding a SARS-CoV-2 S protein of a Wuhan strain.** Subjects previously administered two doses of an RNA vaccine encoding a SARS-CoV-2 S protein of a Wuhan strain were administered a third and a fourth dose of an RNA vaccine encoding a SARS-CoV-2 S protein having mutations characteristic of a Beta variant. Neutralization antibody titers were measured before administration of an RNA vaccine encoding a SARS-CoV-2 S protein of a Wuhan strain (D1-PreVax), one month after administration of a second dose of an RNA vaccine encoding a SARS-CoV-2 S protein of a Wuhan strain (M1PD2), one-month after administration of a third dose of an RNA vaccine encoding a SARS-CoV-2 S protein having mutations characteristic of a SARS-CoV-2 Beta variant, and one month after administration of a fourth dose of an RNA vaccine encoding a SARS-CoV-2 S protein having mutations characteristic of a SARS-CoV-2 Beta variant. The third and fourth doses were administered 1 month apart from one another. GMFR refers to the geometric mean fold rise, and is a measure of the increase in neutralization antibody titers since the previous vaccine dose (e.g., the GMFR for Post-Dose2 (PD2) is a measure of the increase in neutralization antibody titers relative to before administration of any vaccine (pre-vax)). (A) Neutralization antibody titers measured in a viral neutralization assay that uses a viral particle comprising a SARS-CoV-2 S protein of a Wuhan strain. (B) Neutralization antibody titers measured in a viral neutralization assay that uses a viral particle comprising a SARS-CoV-2 S protein having mutations characteristic of a Beta variant.

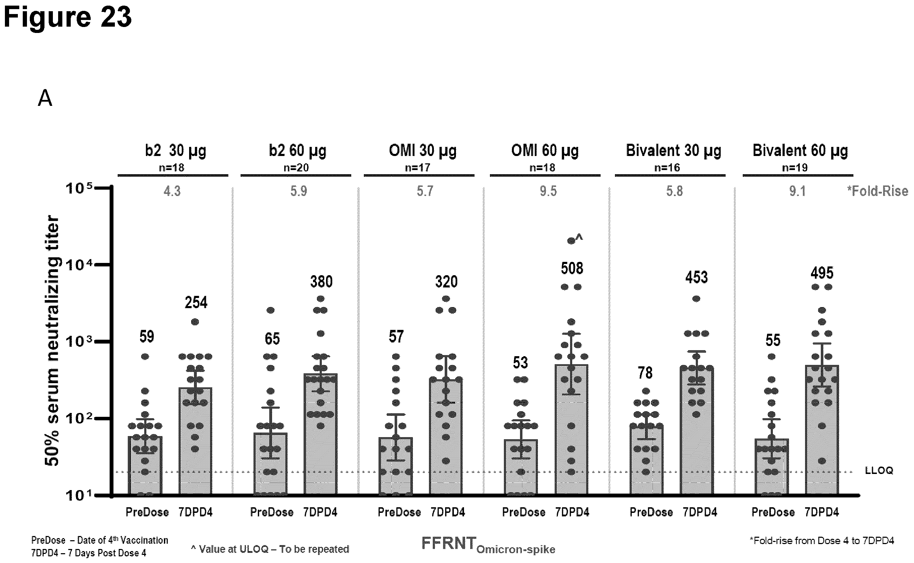

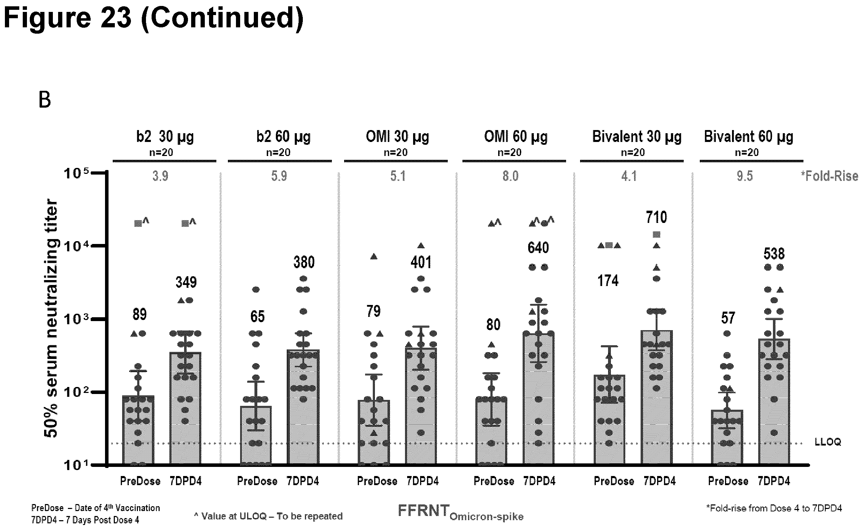

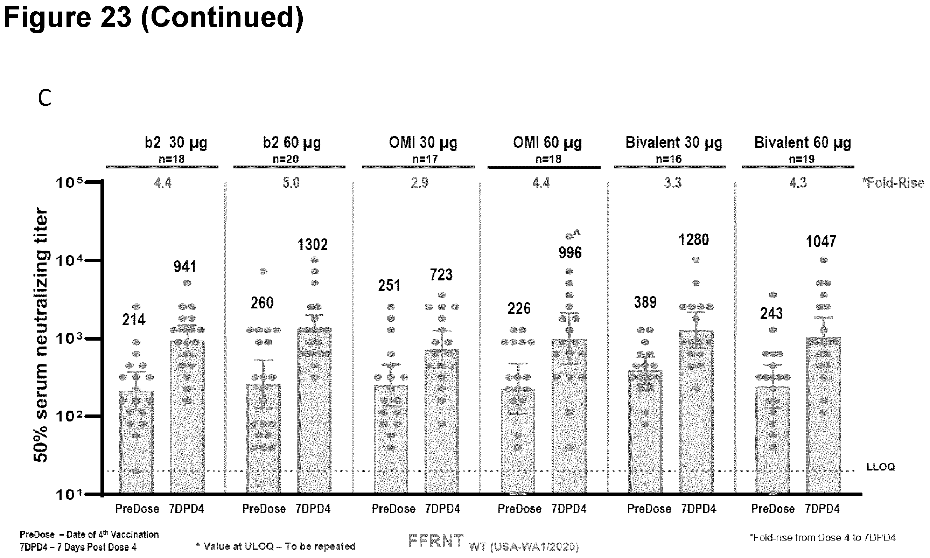

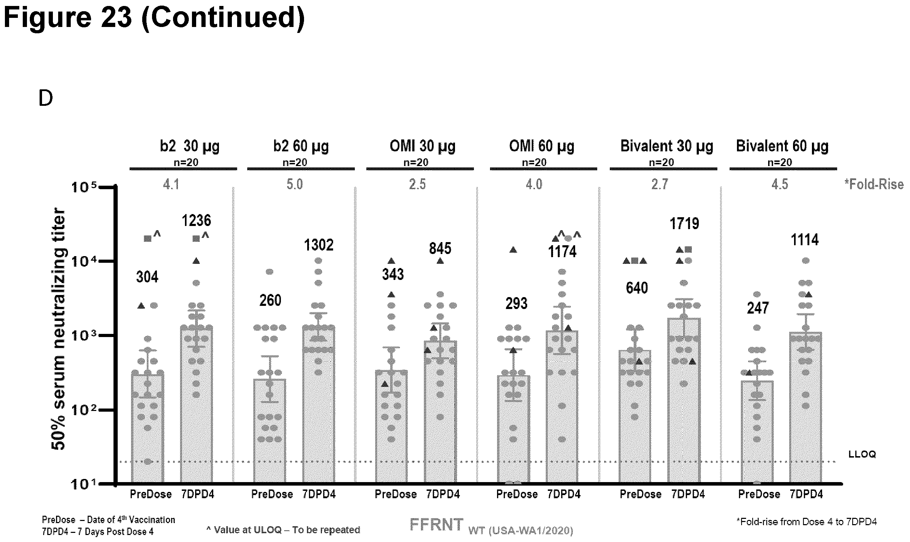

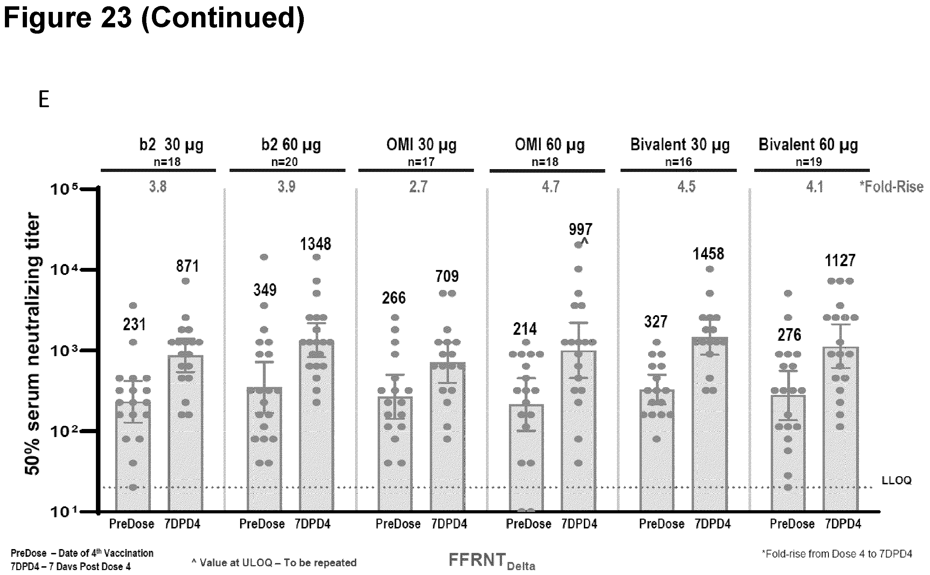

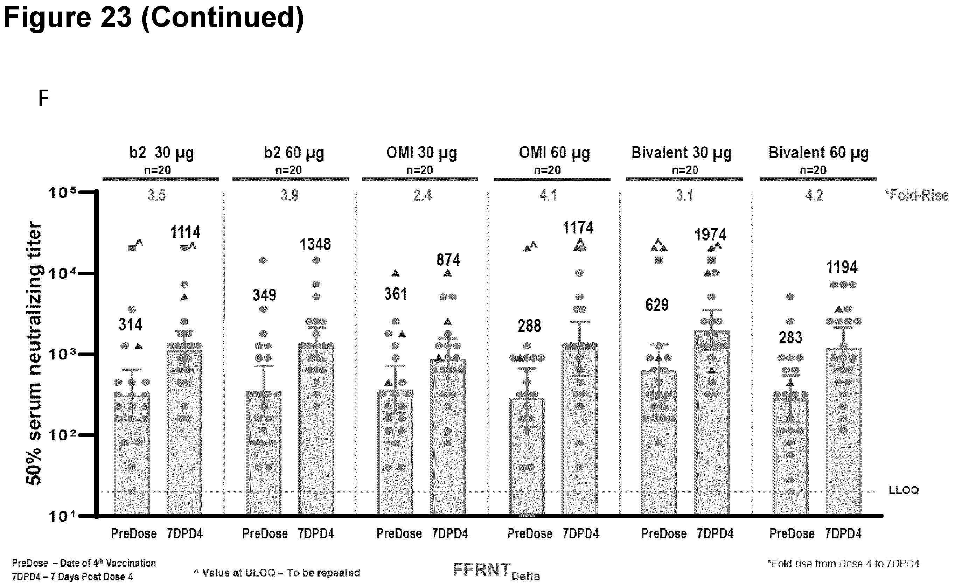

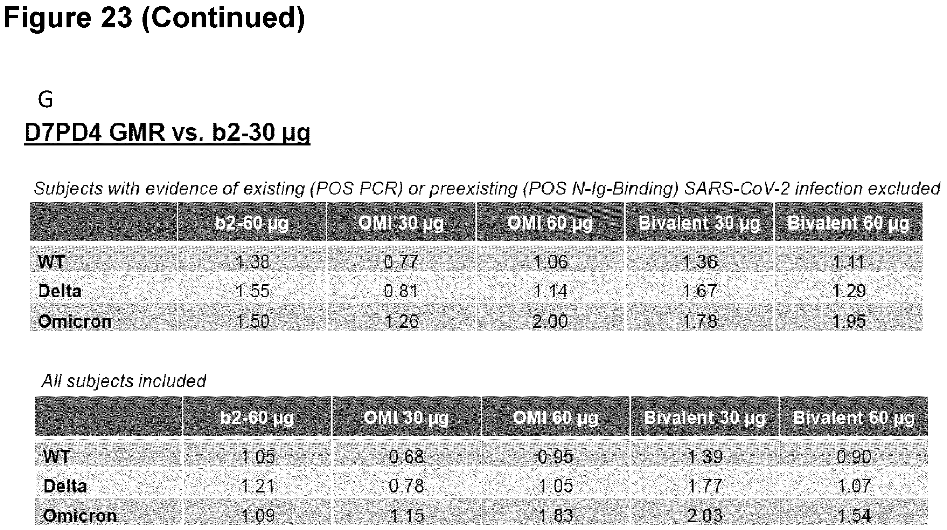

**Fig. 23**. 50% neutralization titers of sera collected 7 days after a fourth dose of BNT162b2, **an Omicron BA.1-specific booster, or a bivalent vaccine.** Subjects who were previously administered two doses of BNT162b2 (30 ug), and a third (booster) dose of BNT162b2 (30 ug) received (i) a 30 ug dose of BNT162b2 (encoding a SARS-CoV-2 S protein from a Wuhan strain), (ii) a 60 ug dose of BNT162b2, (iii) a 30 ug dose of RNA encoding a SARS-CoV-2 S protein having mutations characteristic of an Omicron BA.1 variant (e.g., as described herein (referred to herein as "Omicron BA.1-specific RNA vaccine")), (iii) a 60 ug dose of RNA encoding a SARS-CoV-2 S protein having mutations characteristic of an Omicron BA.1 variant, (iv) a 30 ug dose of a bivalent vaccine, comprising 15 ug of BNT162b2 and 15 ug of RNA encoding a SARS-CoV-2 S protein comprising mutations characteristic of an Omicron BA.1 variant, or (v) a 60 ug dose of a bivalent vaccine, comprising 30 ug of BNT162b2 and 30 ug of RNA encoding a SARS-CoV-2 S protein comprising mutations characteristic of an Omicron BA.1 variant. Geometric mean ratio (GMR) of titers in serum from subjects were collected 7 days after administration of a 4th dose. "b2" refers to sera from subjects administered a Wuhan-specific RNA vaccine as a 4^{th} dose of BNT162b2. "OMI" refers to sera from subjects administered an Omicron BA.1-specific 4^{th} dose. "Bivalent" refers to sera from subjects administered a composition comprising BNT162b2 and an RNA encoding a SARS-CoV-2 S protein comprising mutations that are characteristic of an Omicron BA.1 variant as a 4^{th} dose. Also shown is the fold-rise in titer from before administration of a 4^{th} dose to 7 days after administration of a 4^{th} dose (*Fold-Rise). "FFRNT" refers to fluorescent focus reduction neutralization test. Neutralization data was obtained using an FFRNT assay, with a viral particle containing a SARS-CoV-2 S protein having mutations characteristic of the variant indicated in the figures. LLOQ refers to Lower Limit of Quantification and ULOQ refers to Upper Limit of Quantification. (A) Comparison of titers of neutralizing antibodies against a SARS-CoV-2 pseudovirus comprising a SARS-CoV-2 S protein having mutations characteristics of an Omicron BA.1 variant. Sera from subjects previously or currently infected with SARS-CoV-2 excluded. (B) Comparison of titers of neutralizing antibodies against a SARS-CoV-2 pseudovirus comprising a SARS-CoV-2 S protein having mutations characteristics of an Omicron BA.1 variant in sera from a population that includes subjects previously or currently infected with SARS-CoV-2 (e.g., as determined by an antibody test or a PCR assay respectively). (C) Comparison of titers of neutralizing antibodies against a SARS-CoV-2 pseudovirus comprising a SARS-CoV-2 S protein of a Wuhan strain. Sera from subjects previously or currently infected with SARS-CoV-2 excluded. (D) Comparison of titers of neutralizing antibodies against a SARS-CoV-2 pseudovirus comprising a SARS-CoV-2 S protein of a Wuhan strain, in sera from a population that includes individuals previously or currently infected with SARS-CoV-2. (E) Comparison of titers of neutralizing antibodies against a SARS-CoV-2 pseudovirus comprising a SARS-CoV-2 S protein having mutations characteristics of a Delta variant. Sera from subjects previously or currently infected with SARS-CoV-2 excluded. (F) Comparison of titers of neutralization antibodies against a SARS-CoV-2 pseudovirus comprising a SARS-CoV-2 S protein having mutations characteristic of a Delta variant, in sera from a population including subjects previously or currently infected with SARS-CoV-2. (G) Geometric mean rise (GMR) of neutralization antibodies observed in subjects administered 60 ug of BNT162b2, 30 ug of RNA encoding a SARS-CoV-2 S protein having mutations characteristic of an Omicron BA.1 variant (OMI 30 ug), 60 ug of RNA encoding a SARS-CoV-2 S protein having mutations characteristic of an Omicron BA.1 variant (OMI 60 ug), 30 ug of a bivalent vaccine comprising 15 ug of BNT162b2 and 15 ug of RNA encoding a SARS-CoV-2 S protein having mutations characteristic of an Omicron BA.1 variant (Bivalent 30 ug), or 60 ug of a bivalent vaccine comprising 30 ug of BNT162b2 and 30 ug of RNA encoding a SARS-CoV-2 S protein having mutations characteristic of an Omicron BA.1 variant (Bivalent 60 ug), as compared to subjects administered 30 ug of BNT162b2 as a 4^{th} dose. Results are shown both for a population pool that excludes subjects previously or currently infected with SARS-CoV-2 and a population pool that includes these subjects.

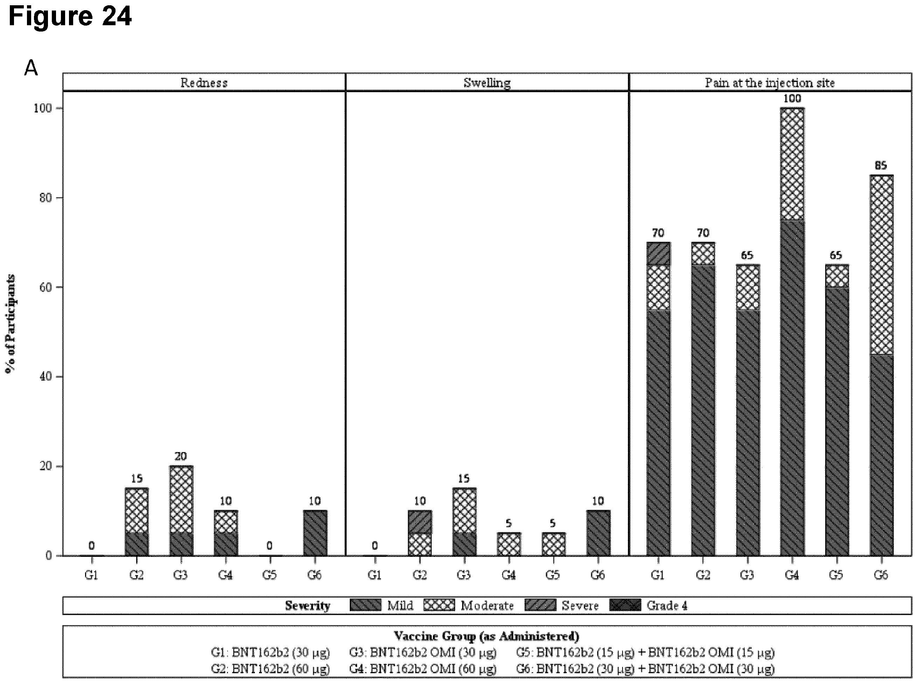

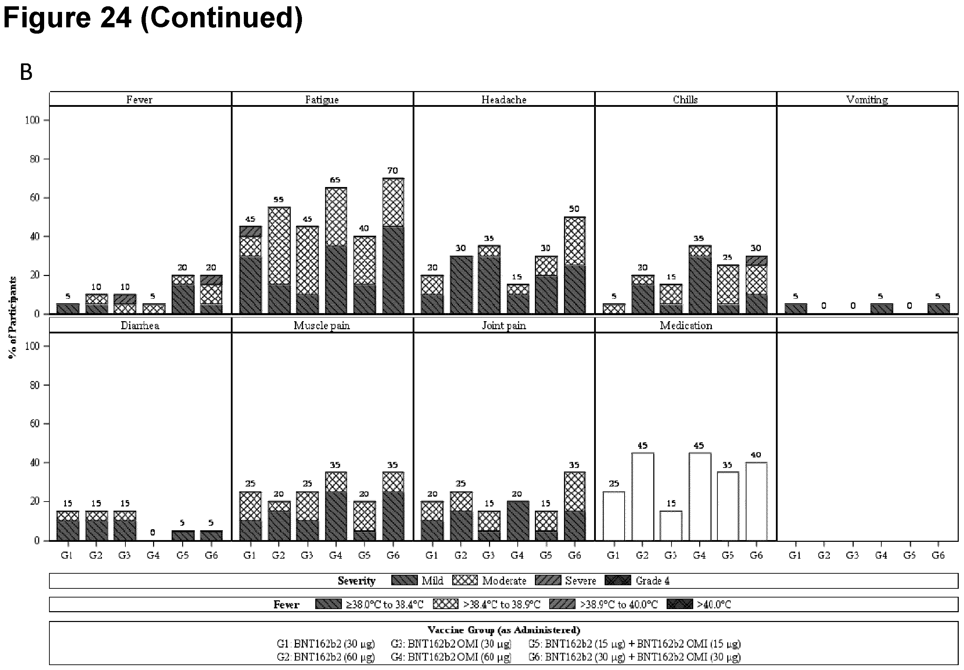

**Fig. 24****. Reactogenicity of certain exemplary RNA (formulated in LNP) at a given dose: subjects administered a 60 ug dose of RNA encoding a SARS-CoV-2 S protein are more likely to exhibit a higher injection site pain and exhibit similar systemic reactions as subjects administered a 30 ug dose of RNA.** Subjects were administered 30 ug or 60 ug of RNA encoding a SARS-CoV-2 S protein from a Wuhan strain (BNT162b2, corresponding to groups G1 and G2, respectively), 30 ug or 60 ug of RNA encoding a SARS-CoV-2 S protein having mutations characteristic of an Omicron BA.1 variant (BNT162b2 OMI, corresponding to groups G3 and G4, respectively), 30 ug of a bivalent vaccine comprising 15 ug of RNA encoding a SARS-CoV-2 S protein from a Wuhan strain and 15 ug of RNA encoding a SARS-CoV-2 S protein having mutations characteristic of an Omicron BA.1 variant (BNT162b2 (15 ug) + BNT162b2 OMI (15 ug), corresponding to group G5), or 60 ug of a bivalent vaccine comprising 30 ug of RNA encoding a SARS-CoV-2 S protein from a Wuhan strain and 30 ug of RNA encoding a SARS-CoV-2 S protein having mutations characteristic of an Omicron BA.1 variant (BNT162b2 (30 ug) + BNT162b2 OMI (30 ug), corresponding to group G6). (A) Local reactions, including redness, swelling, and pain at the injection site, observed within 7 days of injection. Injection site pain was found to be increased in subjects administered 60 ug of RNA encoding a SARS-CoV-2 S protein comprising mutations characteristic an Omicron BA.1 variant or a bivalent vaccine, as compared to other doses tested. (B) Systemic reactions, including fever, fatigue, headache, chills, vomiting, diarrhea, muscle pain, joint pain, and use of medication, observed within 7 days of injection. Systemic reactions through 7 days were observed to be broadly similar across different groups. Fatigue was found to trend higher after administration of 60 ug doses, as compared to 30 ug doses.

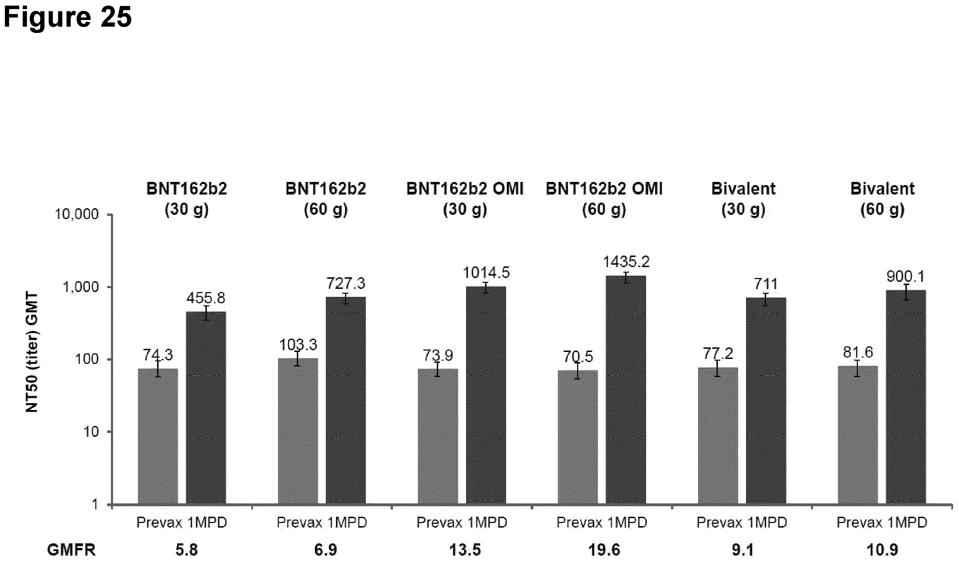

**Fig. 25**. 50% neutralization titers of sera collected 1 month after a fourth dose of BNT162b2, **an Omicron BA.1-specific booster, or a bivalent vaccine against an Omicron BA.1 variant.** Subjects who were previously administered two doses of BNT162b2 (30 ug), and a third (booster) dose of BNT162b2 (30 ug) were administered (i) a 30 ug dose of BNT162b2 (encoding a SARS-CoV-2 S protein from a Wuhan strain), (ii) a 60 ug dose of BNT162b2, (iii) a 30 ug dose of RNA encoding a SARS-CoV-2 S protein having mutations characteristic of an Omicron BA.1 variant ("BNT162b2 OMI"), (iii) a 60 ug dose of BNT162b2 OMI, (iv) a 30 ug dose of a bivalent vaccine, comprising 15 ug of BNT162b2 and 15 ug of BNT162b2 OMI ("Bivalent"), or (v) a 60 ug dose of a bivalent vaccine, comprising 30 ug of BNT162b2 and 30 ug of BNT162b2 OMI. Geometric mean titer (GMT) of neutralization antibodies were measured in serum from subjects collected 1 month after administration of a 4th dose (GMT indicated above each bar). Shown below the x-axis is the geometric fold rise (GMFR) in titer from before administration of a 4^{th} dose (pre-vax) to 1 month after administration of a 4^{th} dose (1MPD) for each patient group. Neutralization data was obtained using a neutralization assay, with a viral particle containing a SARS-CoV-2 S protein having mutations characteristic of a Omicron BA.1 variant.

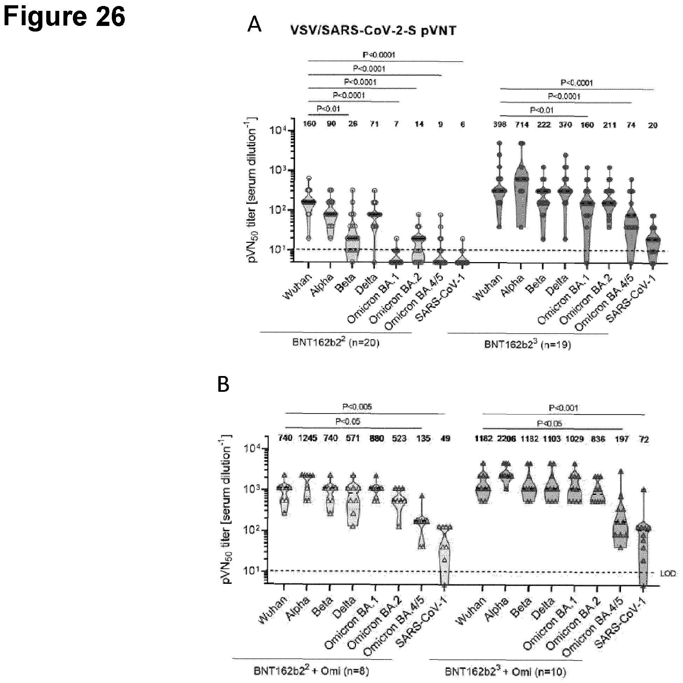

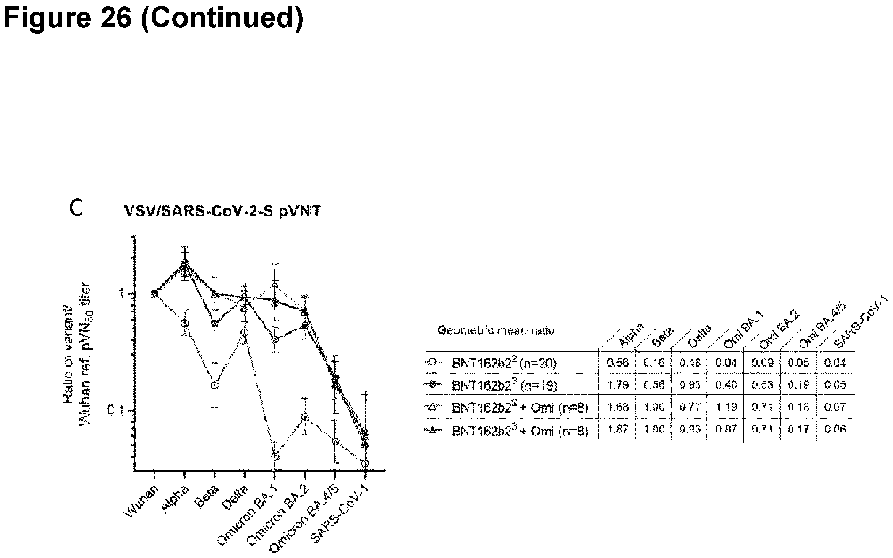

**Fig. 26****. Omicron BA.1 breakthrough infection of BNT162b2 double- and triple-vaccinated individuals induces broad neutralization of Omicron BA.1, BA.2 and other VOCs, but to a lesser extent against BA.4 and BA.5.** This figure is an extension of Fig. 16, including data neutralizing activity against Omicron BA.4 and BA.5. As described in Fig. 16, serum was tested in duplicate; 50% pseudovirus neutralization (pVN₅₀) geometric mean titers (GMTs) (in A and B), and the geometric mean ratio of SARS-CoV-2 variants of concern (VOCs) and SARS-CoV-1 pVN₅₀ GMTs normalized against Wuhan pVN₅₀ GMTs (in C) were plotted. For titer values below the limit of detection (LOD), LOD/2 values were plotted. Values above violin plots represent the group GMTs. The nonparametric Friedman test with Dunn's multiple comparisons correction was used to compare Wuhan neutralizing group GMTs with titers against the indicated variants and SARS-CoV-1. Multiplicity adjusted p values are shown. (A) pVN₅₀ GMTs against Wuhan, VOC and SARS-CoV-1 pseudovirus in patients who received two doses or three doses of BNT162b2. (B) pVNso GMTs against Wuhan, VOC and SARS-CoV-1 pseudovirus in patients who received two doses or three doses of BNT162b2 and who have been previously infected with an Omicron BA.1 variant of SARS-CoV-2. (C) Group geometric mean ratios with 95% confidence intervals for all cohorts shown in (A) and (B).

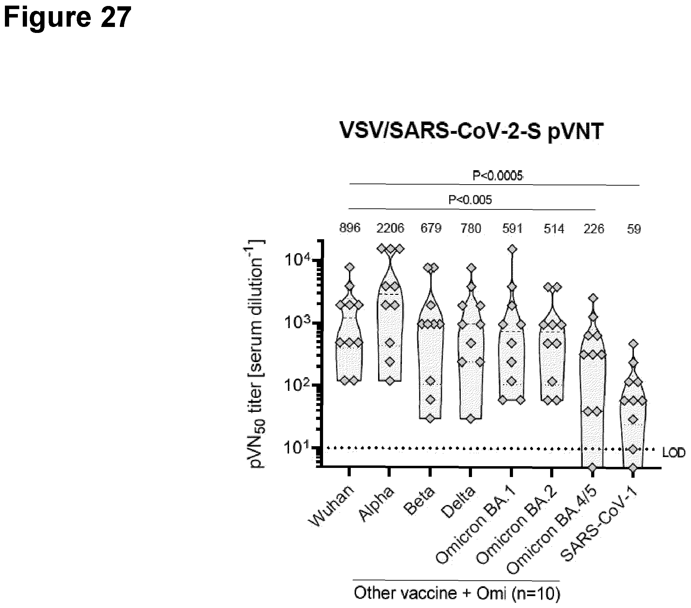

**Fig. 27****. Omicron BA.1 breakthrough infection of individuals vaccinated with other approved COVID-19 vaccines or mixed regimens results in immune sera that broadly neutralize Omicron BA.1, BA.2 and other VOCs, but to a lesser extent against BA.4 and BA.5.** This figure is an extension of Fig. 19, including data neutralizing activity against Omicron BA.4 and BA.5. As described in Fig. 19, serum was tested in duplicate; individual 50% pseudovirus neutralization (pVN₅₀) geometric mean titers (GMTs) against SARS-CoV-2 Wuhan, Alpha, Beta, Delta and Omicron BA.1, BA.2 and BA.4/5 variants, plus SARS-CoV-1 were plotted. For titer values below the limit of detection (LOD), LOD/2 values were plotted. Values above violin plots represent group GMTs. The nonparametric Friedman test with Dunn's multiple comparisons correction was used to compare Wuhan neutralizing group GMTs with titers against the indicated variants and SARS-CoV-1. Multiplicity-adjusted p values are shown.

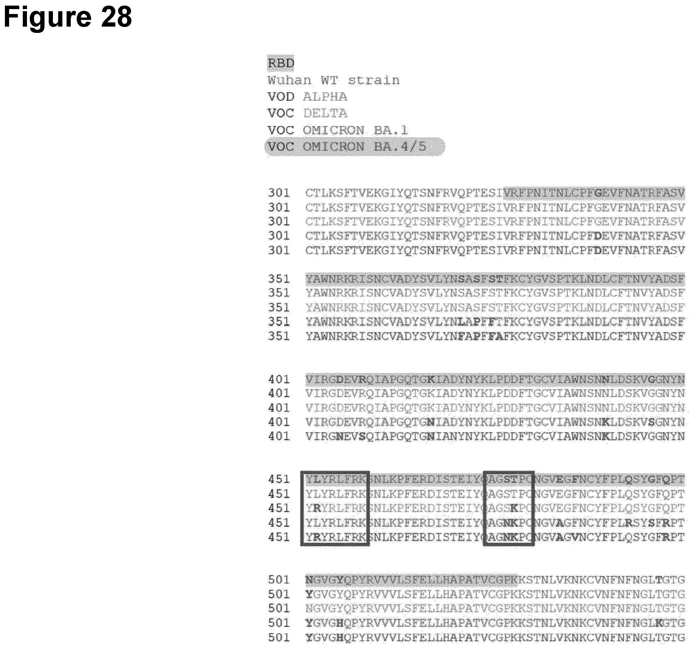

**Fig. 28****. Sequences of RBDs of SARS-COV-2 Wuhan strain and variants thereof.** Variant-specific amino acid alterations are indicated in bold red font, with the original Wuhan amino acid highlighted in bold blue font.

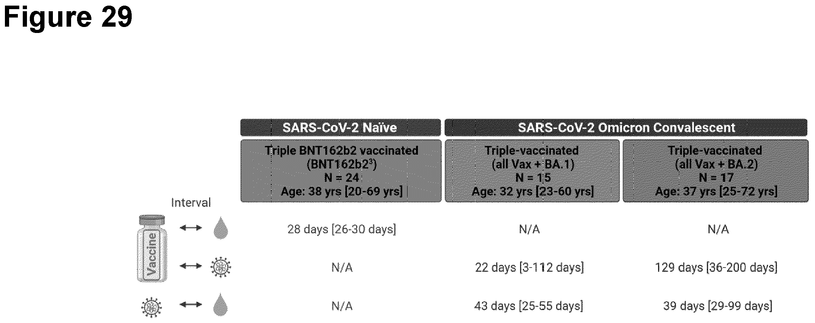

**Fig. 29****. Cohorts and sampling for the study described in Example 14.** A schematic is shown for testing immune responses in triple-vaccinated patients who are (i) Omicron naïve, (ii) have been infected with an Omicron BA.1 variant, or (iii) have been infected with an Omicron BA.2 variant. Blood samples were drawn from three cohorts: Omicron-naïve individuals triple-vaccinated with BNT162b2 (BNT162b2³, green), and individuals vaccinated with homologous or heterologous three doses regimens that subsequently had either a breakthrough infection with Omicron at a time of BA.1 dominance (November 2021 to January 2022; all Vax + BA.1, purple) or at a time of BA.2 dominance (March to May 2022; all Vax + BA.2, blue) in Germany. Sera (droplet) were isolated in the Omicron-naïve cohort at the time-point indicated following their most recent vaccination; for convalescent cohorts, the time from their most recent vaccination to Omicron infection, and infection to serum isolation are indicated. All values specified as median-range. Serum neutralizing capacity was assessed using a pseudovirus neutralization test.

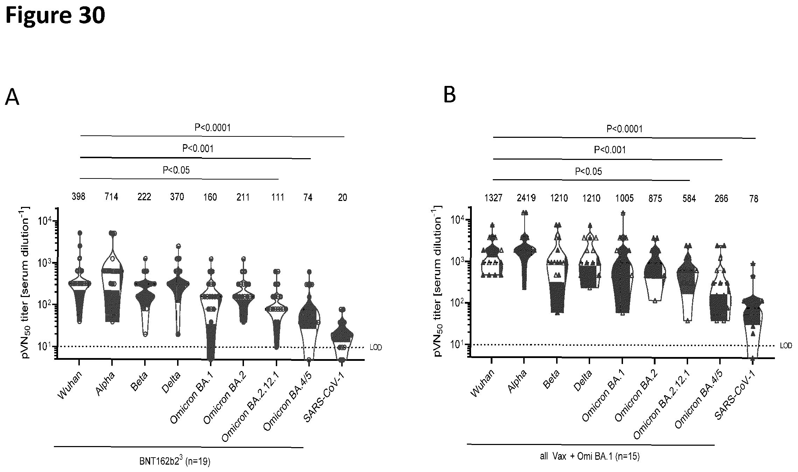

**Fig. 30**. 50% pseudovirus neutralization (pVN**₅₀) geometric mean titers (GMTs) from the BNT162b2³ and All Vax + Omi BA.1 breakthrough infection cohorts.** Serum was drawn from Omicron-naïve BNT162b2 triple-vaccinated individuals (BNT162b2³, circles) at 28 days after the third dose, and from vaccinated individuals with subsequent Omicron BA.1 breakthrough infection (all Vax + Omi BA.1, triangles) at a median 43 days post-infection. 50% pseudovirus neutralization (pVN₅₀) geometric mean titers (GMTs) for Omicron-naive individuals are plotted in (A) and for BA.1 breakthrough infected individuals in (B). This data was previously published in Quandt et al. ("Omicron BA.1 breakthrough infection drives cross-variant neutralization and memory B cell formation against conserved epitopes." Science immunology, eabq2427 (2022), doi:10.1126/sciimmunol.abq2427), except for BA.2.12.1 neutralization data. Serum was tested in duplicate. For titer values below the limit of detection (LOD), LOD/2 values were plotted. Values above violin plots represent group GMTs. The non-parametric Friedman test with Dunn's multiple comparisons correction was used to compare Wuhan neutralizing group GMTs with titers against the indicated variants and SARS-CoV-1. Multiplicity-adjusted p values are shown.

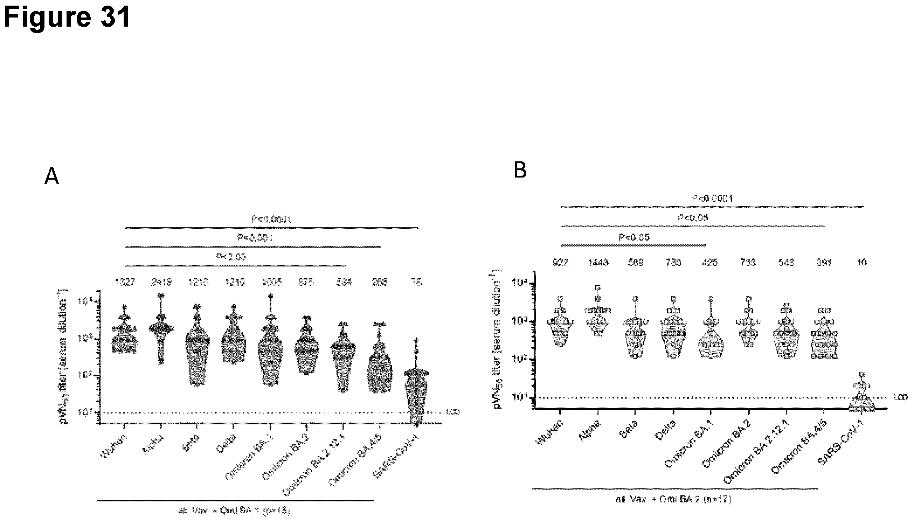

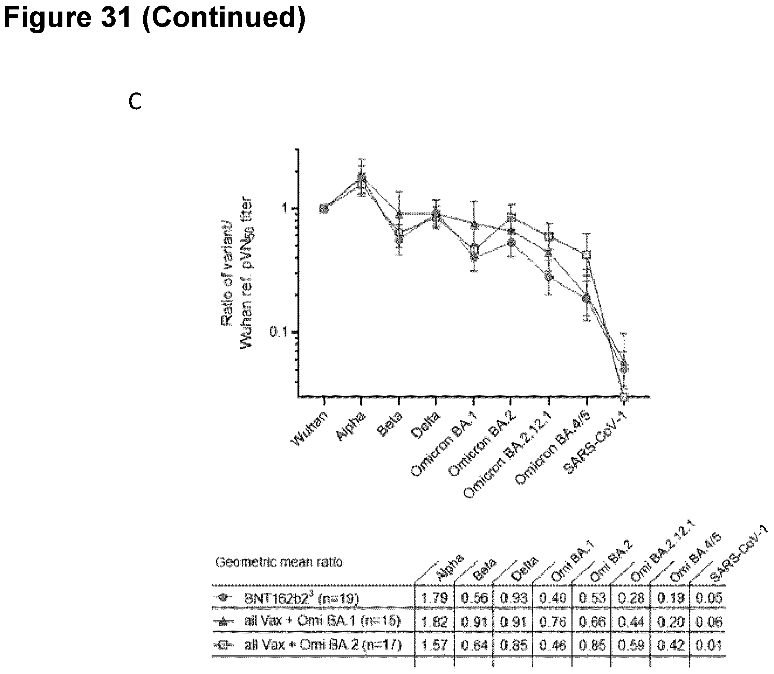

**Fig. 31****. Omicron BA.2 breakthrough infection of previously vaccinated individuals refocuses neutralization against Omicron BA.2 and the BA.2-derived subvariants BA.2.12.1 and BA.4/BA.5.** Serum was drawn from BNT162b2 triple-vaccinated individuals with subsequent Omicron BA.1 breakthrough infection at a median 44 days post-infection (BNT162b2³ + Omi BA.1, triangles), and from BNT162b2 triple-vaccinated individuals with subsequent Omicron BA.2 breakthrough infection at 38 days post-infection (BNT162b2³ + Omi BA.2, squares). 50% pseudovirus neutralization (pVN₅₀) geometric mean titers (GMTs) (in A, B), and the geometric mean ratio of SARS-CoV-2 variants of concern (VOCs) and SARS-CoV-1 pVNso GMTs normalized against Wuhan pVN50 GMTs (in C) were plotted. pVN₅₀ GMT and geometric mean ratio data for Omicron-naïve BNT162b2 triple-vaccinated individuals (BNT162b2³, circles) and BNT162b2 triple-vaccinated individuals with Omicron BA.1 breakthrough infection was previously published in Quandt et al. ("Omicron BA.1 breakthrough infection drives cross-variant neutralization and memory B cell formation against conserved epitopes." Science immunology, eabq2427 (2022), doi:10.1126/sciimmunol.abq2427), except for BA.2.12.1 neutralization data. Serum was tested in duplicate. For titer values below the limit of detection (LOD), LOD/2 values were plotted. Values above violin plots represent group GMTs. The non-parametric Friedman test with Dunn's multiple comparisons correction was used to compare Wuhan neutralizing group GMTs with titers against the indicated variants and SARS-CoV-1. Multiplicity-adjusted p values are shown. (A, B) pVNso GMTs against Wuhan, VOC and SARS-CoV-1 pseudovirus. (C) Group geometric mean ratios with 95% confidence intervals.

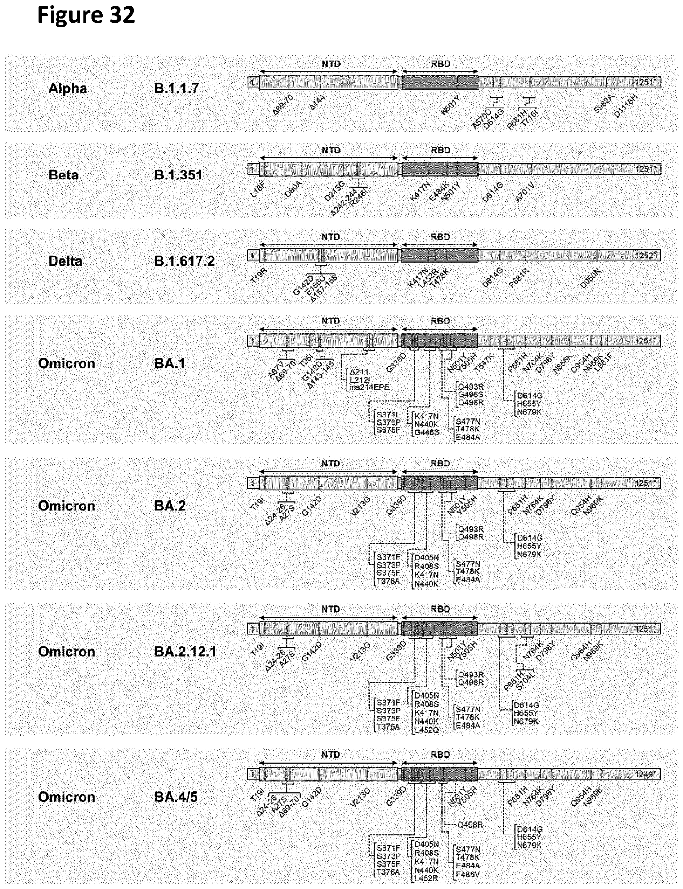

**Fig. 32****. Characteristics of SARS-CoV-2 S glycoproteins used in the VSV-SARS-CoV-2 pseudovirus based neutralization assays.** The sequence of the Wuhan-Hu-1 isolate SARS-CoV-2 S glycoprotein (GenBank: QHD43416.1) was used as reference. Amino acid positions, amino acid descriptions (one letter code) and kind of mutations (substitutions, deletions, insertions) are indicated. NTD, N-terminal domain; RBD, Receptor-binding domain, Δ, deletion; ins, insertion; *, Cytoplasmic domain truncated for the C-terminal 19 amino acids.

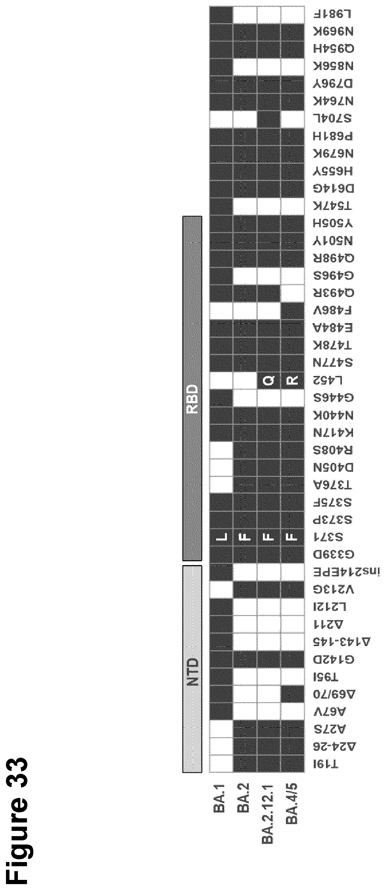

**Fig. 33****. Alterations on the spike glycoprotein amino acid sequence of SARS-CoV-2 Omicron sub-lineages.** Amino acid positions, amino acid descriptions (one letter code) and kind of mutations substitutions, deletions, insertions) are indicated. White letters in boxes indicate the amino acid substitution per sub-lineage; Δ, deletion; ins, insertion; NTD, N-terminal domain; RBD, receptor-binding domain.

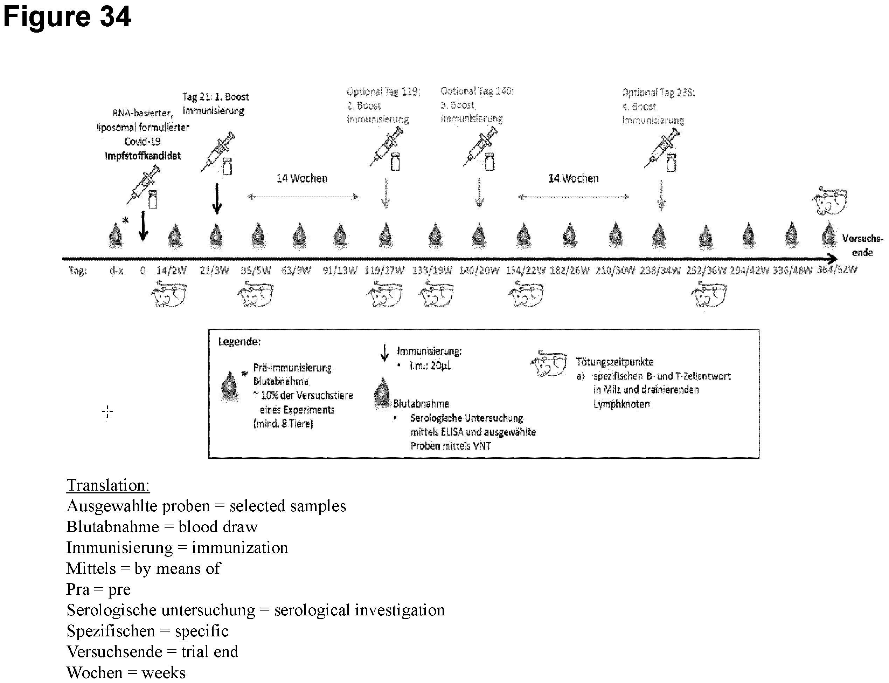

**Fig. 34****. Immunization protocol for studies with VOC boosters.** BALB/c mice were immunized according to the indicated schedule with two doses (1 ug each) of the original BNT162b2 vaccine, followed by at least one dose (1 ug total) of a monovalent, bivalent, or trivalent booster dose of either: (a) the original BNT162b2 ("BNT162b2"); (b) BNT162b2 OMI BA.1 ("OMI BA.1"); (c) BNT162b2 OMI BA.4/5 ("OMI BA.4/5"); or a combination thereof.

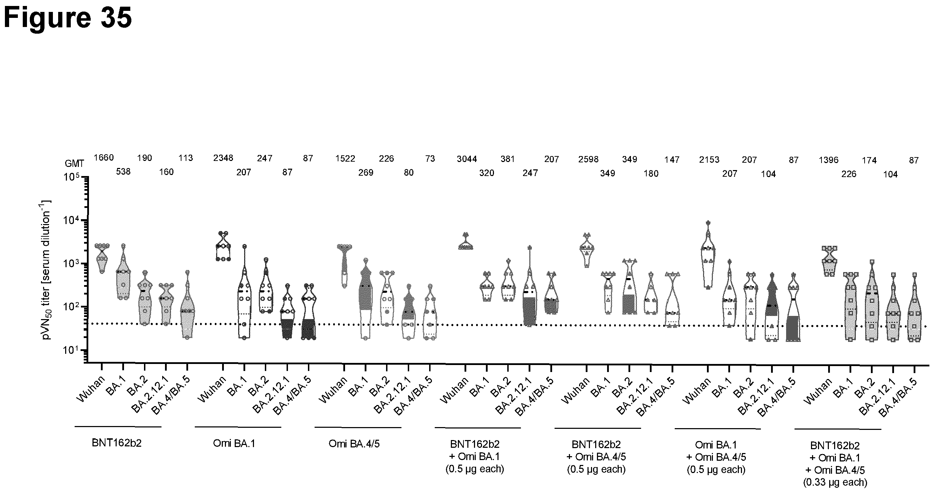

**Fig. 35****. Baseline grouped neutralizing GMTs.** Sera drawn from mice immunized as depicted in Fig. 34 (day 104, pre-boost) were assessed for geometric mean titers of neutralizing antibodies against various strains. Data are presented grouped by cohort.

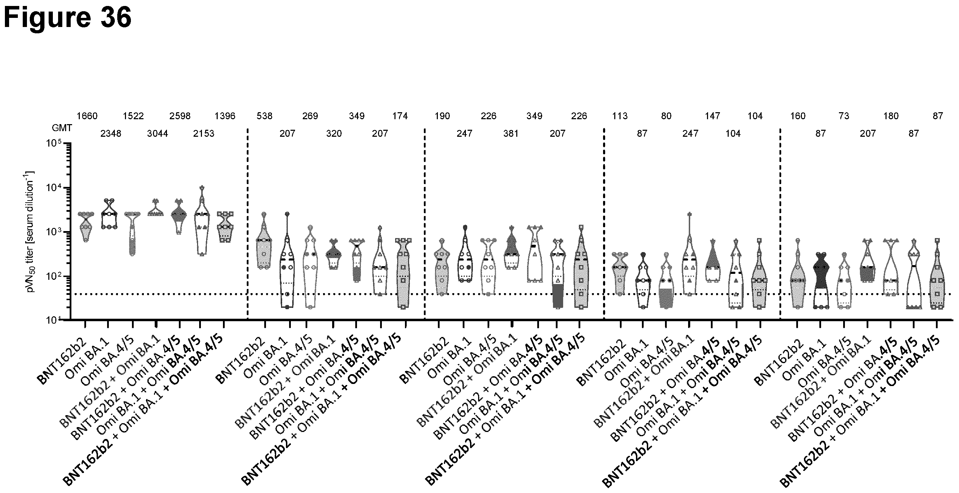

**Fig. 36****. Baseline staggered neutralizing GMTs.** Sera drawn from mice immunized as depicted in Fig. 34 (day 104, pre-boost) were assessed for geometric mean titers of neutralizing antibodies against various strains. Data are presented in staggered format (i.e., by strain against which neutralization was assessed).

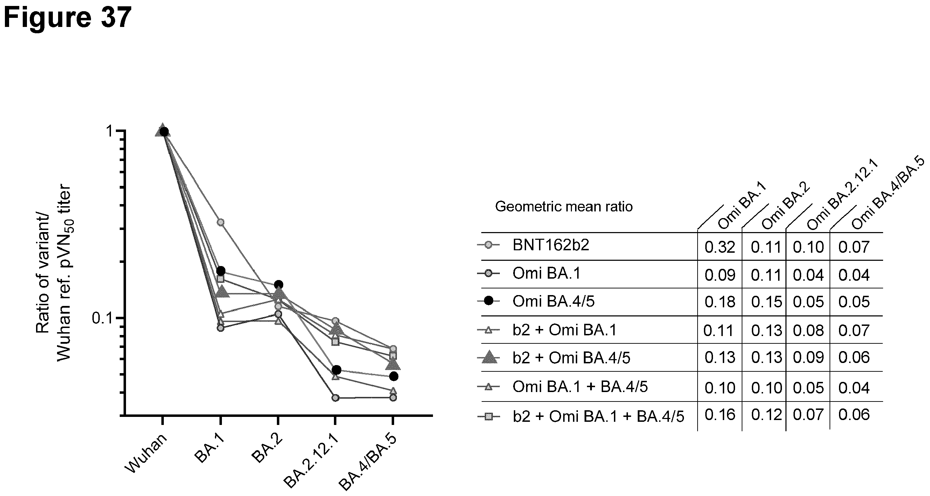

**Fig. 37****. Baseline cross-neutralization.** Sera drawn from mice immunized as depicted in Fig. 34 (day 104, pre-boost) were assessed for geometric mean titers of neutralizing antibodies against various strains. Cross-neutralization results are presented as calculated variant/Wuhan reference GMT Ratios.

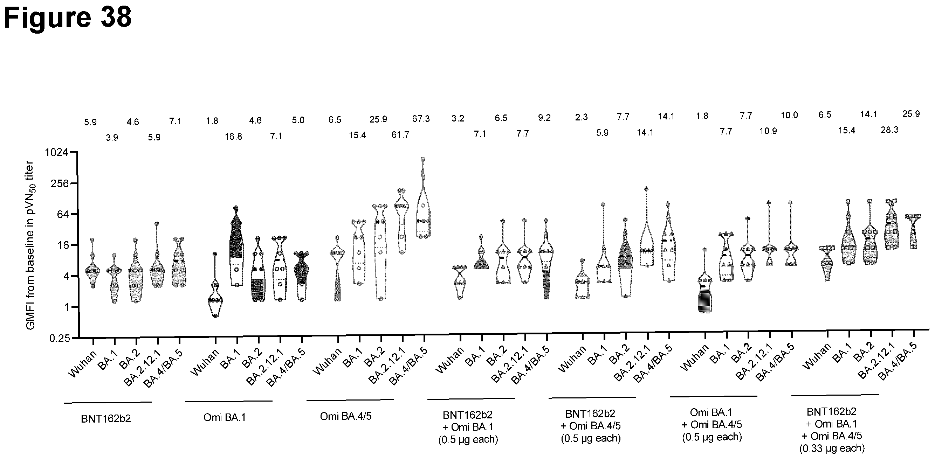

**Fig. 38****. Post-boost geometric mean fold increase in GMTs.** Sera drawn from mice immunized as depicted in Fig. 34 (day 111, 7-days post-boost) were assessed for geometric mean fold increase in GMT of neutralizing antibodies against various strains.

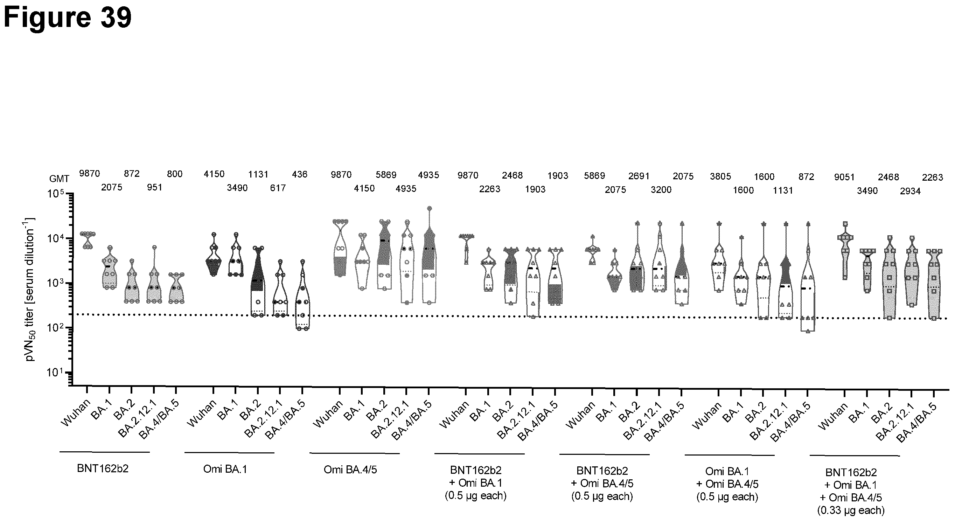

**Fig. 39****. Post-boost grouped neutralizing GMTs.** Sera drawn from mice immunized as depicted in Fig. 34 (day 111, 7-days post-boost) were assessed for geometric mean fold increase in GMT of neutralizing antibodies against various strains. Data are presented grouped by cohort.

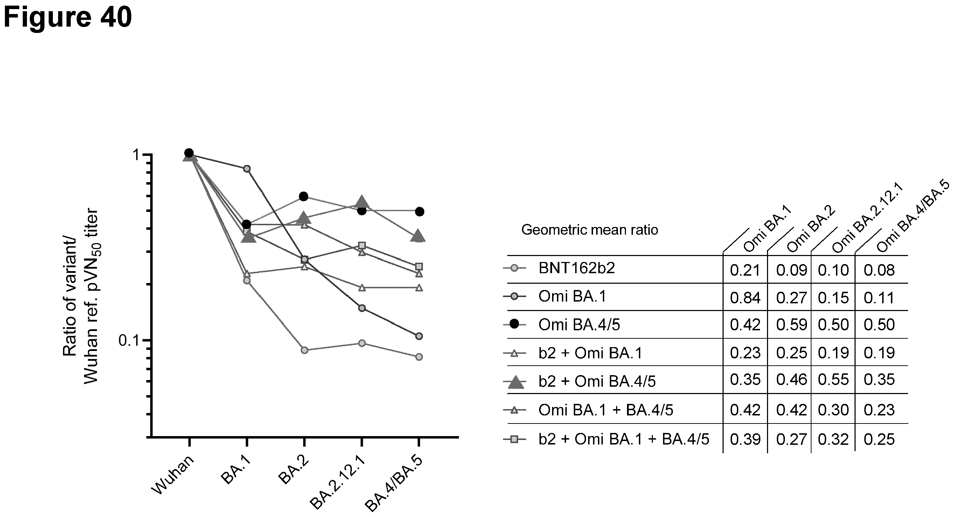

**Fig. 40****. Post-boost cross-neutralization.** Sera drawn from mice immunized as depicted in Fig. 34 (day 111, 7-days post-boost) were assessed for geometric mean fold increase in GMT of neutralizing antibodies against various strains. Cross-neutralization results are presented as calculated variant/Wuhan reference GMT Ratios.

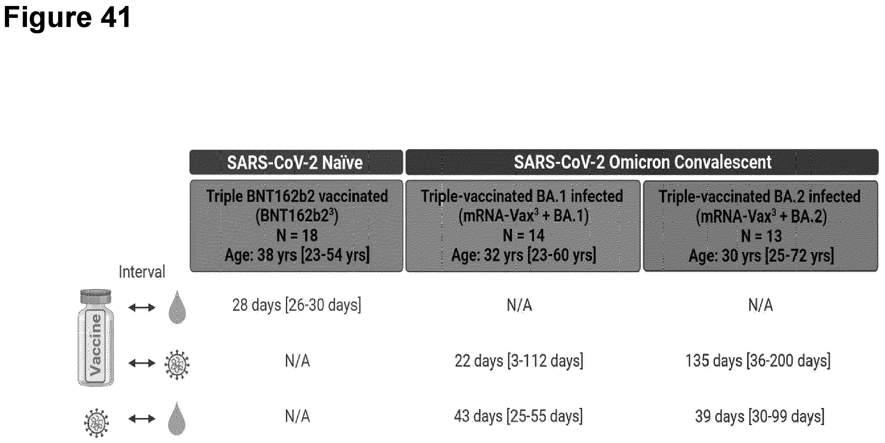

**Fig. 41****. Cohorts and sampling.** Serum samples (droplet) were drawn from three cohorts: individuals triple-vaccinated with BNT162b2 that were SARS-CoV-2-naïve at the time of sampling (BNT162b2³), and from individuals vaccinated with three doses of mRNA COVID-19 vaccine (BNT162b2/mRNA-1273 homologous or heterologous regimens) who subsequently had a breakthrough infection with Omicron either at a time of BA.1 dominance (November 2021 to January 2022; mRNA-Vax³ + BA.1) or at a time of BA.2 dominance (March to May 2022; mRNA-Vax³ + BA.2). For convalescent cohorts, relevant intervals between key events such as the most recent vaccination, SARS-CoV-2 infection, and serum isolation are indicated. All values specified as median-range. N/A, not applicable.

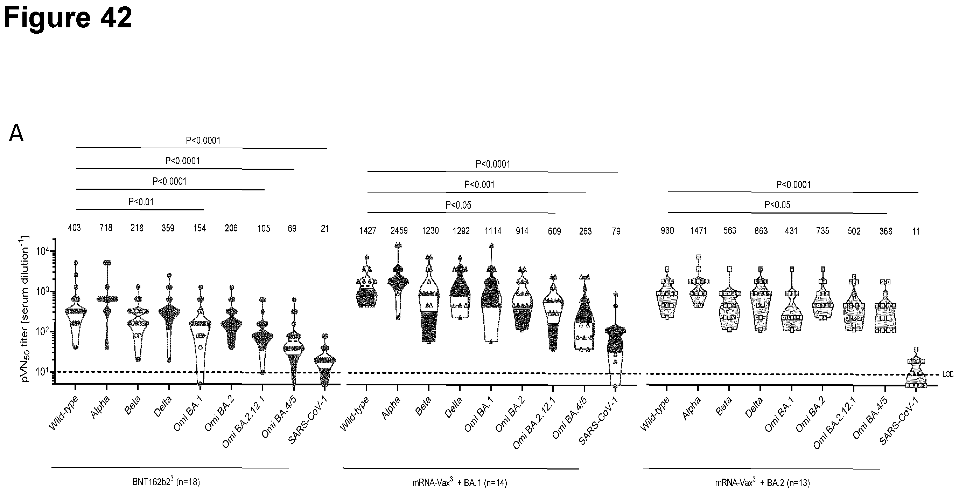

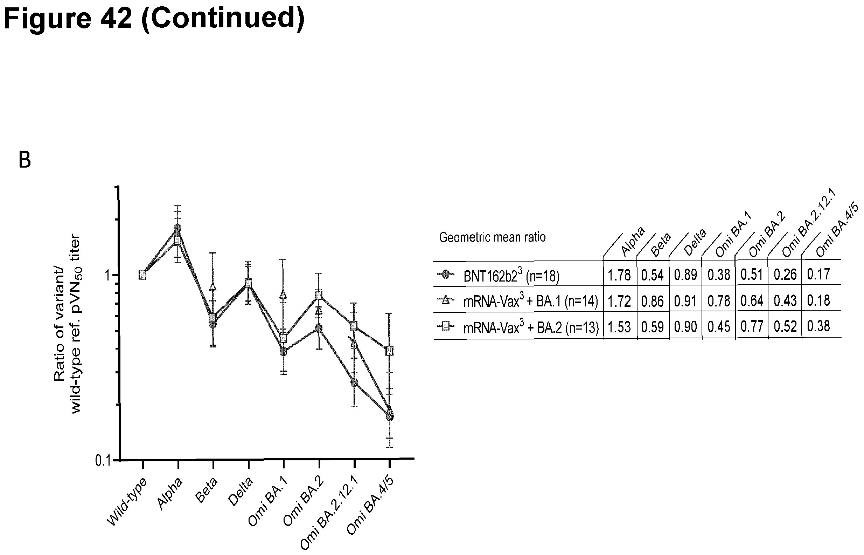

**Fig. 42****. Omicron BA.2 breakthrough infection of triple mRNA vaccinated individuals induces broad neutralization of SARS-CoV-2 variant pseudoviruses including Omicron BA.4/5.** Cohorts and serum sampling as described in Fig. 41. (A) 50% pseudovirus neutralization (pVN₅₀) geometric mean titers (GMTs) against the indicated SARS-CoV-2 variants of concern (VOCs) or SARS-CoV-1 pseudoviruses. Values above violin plots represent group geometric mean titers (GMTs). BNT162b2³ indicates triple vaccinated, SARS-CoV-2- naïve individuals; mRNA-Vax³ + BA.1 indicates triple-vaccinated individuals who subsequently had a breakthrough infection with a Omicron BA.1 variant; and mRNA-Vax³ + BA.2 indicates triple vaccinated indivuals who subsequently had a breakthrough infection with an Omicron BA.2 variant. (B) SARS-CoV-2 variant of concern (VOC) pVNso GMTs normalized against the wild-type strain pVN₅₀ GMT (ratio VOC to wild-type). Group geometric mean ratios with 95% confidence intervals are shown in the graph and listed in the table. Serum was tested in duplicate. For titer values below the limit of detection (LOD), LOD/2 values were plotted. The non-parametric Friedman test with Dunn's multiple comparisons correction was used to compare the wild-type strain neutralizing group GMTs with titers against the indicated variants and SARS-CoV-1. Multiplicity-adjusted p values are shown.

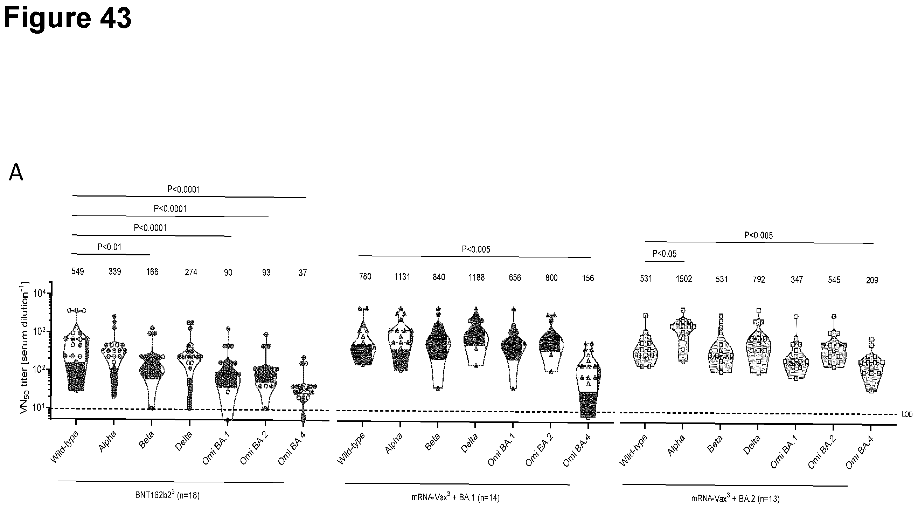

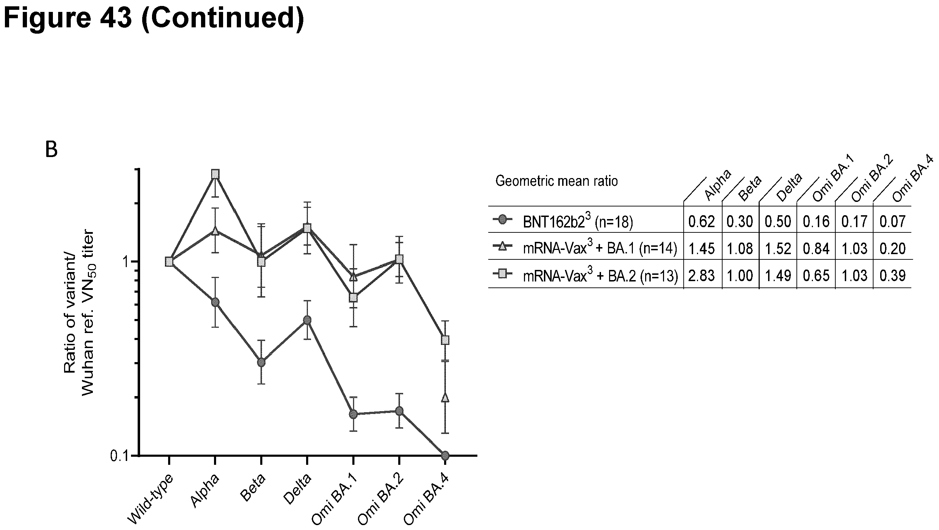

**Fig. 43****. Omicron BA.2 breakthrough infection of previously vaccinated individuals induces broad neutralization of authentic live SARS-CoV-2 variants including Omicron BA.4/5.** Cohorts and serum sampling as described in Fig. 41. (A) 50% virus neutralization (VNso) geometric mean titers (GMTs) against the indicated SARS-CoV-2 variants of concern (VOCs). Values listed above violin plots represent group GMTs. (B) SARS-CoV-2 VOC VN₅₀ GMTs normalized against the wild-type strain VN₅₀ GMT (ratio VOC to wild-type). Group geometric mean ratios with 95% confidence intervals are shown in the graph and listed in the table. Serum was tested in duplicate. For titer values below the limit of detection (LOD), LOD/2 values were plotted. The non-parametric Friedman test with Dunn's multiple comparisons correction was used to compare the wild-type strain neutralizing group GMTs with titers against the indicated variants and SARS-CoV-1. Multiplicity-adjusted p values are shown.

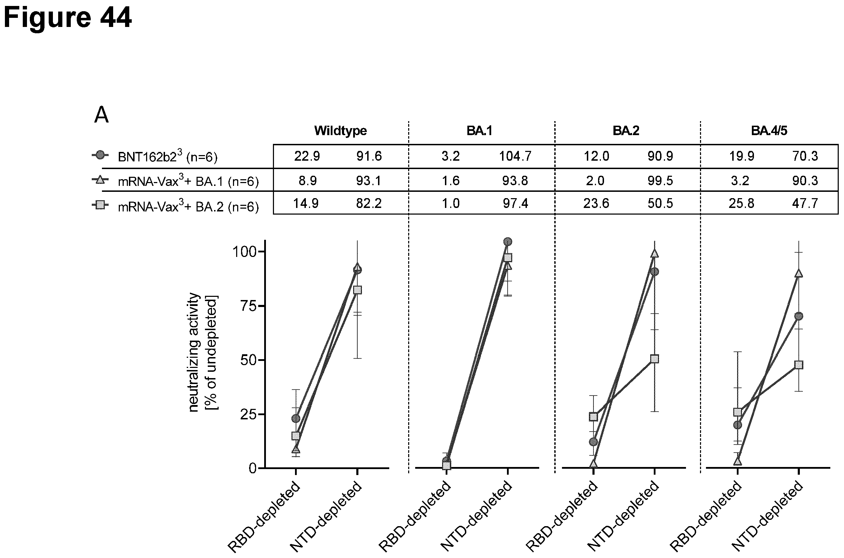

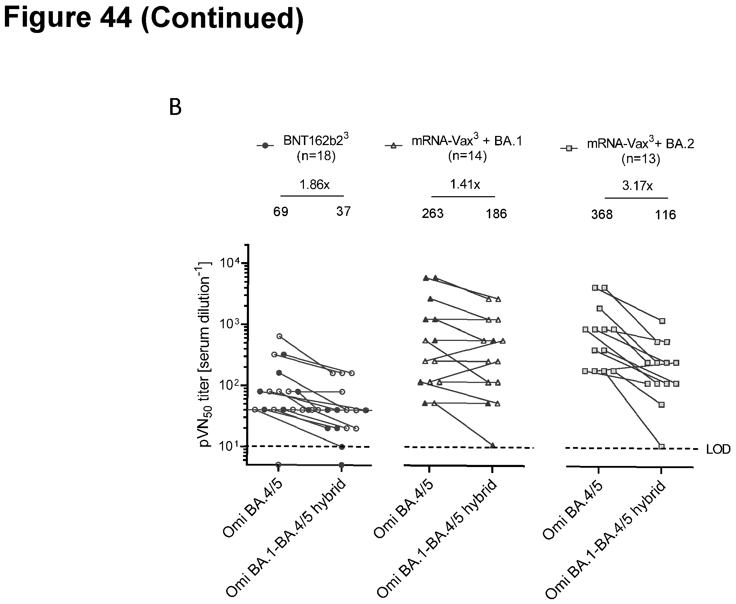

**Fig. 44****. Neutralization of Omicron BA.2 and BA.4/5 by sera of triple mRNA vaccinated BA.2 convalescent individuals is mediated to a large extent by NTD-targeting antibodies.** Cohorts and serum sampling as described in Fig. 41. (A) Serum samples (n=6 per cohort) were depleted of RBD- or NTD-binding antibodies. Relative neutralizing activity of RBD- and NTD-depleted sera (pVN₅₀ titers of undepleted control sera were set to 100%) against the wild-type strain, BA.1, BA.2, and BA.4/5 was calculated and group geometric mean with 95% confidence intervals are shown. (B) 50% pseudovirus neutralization (pVN₅₀) geometric mean titers (GMTs) against Omicron BA.4/5 and Omicron BA.1-BA.4/5 hybrid pseudoviruses. Numbers above plots indicate group geometric mean titers (GMTs) and fold-change in GMTs between BA.4/5 and the hybrid pseudovirus. For titer values below the limit of detection (LOD), LOD/2 values are plotted.

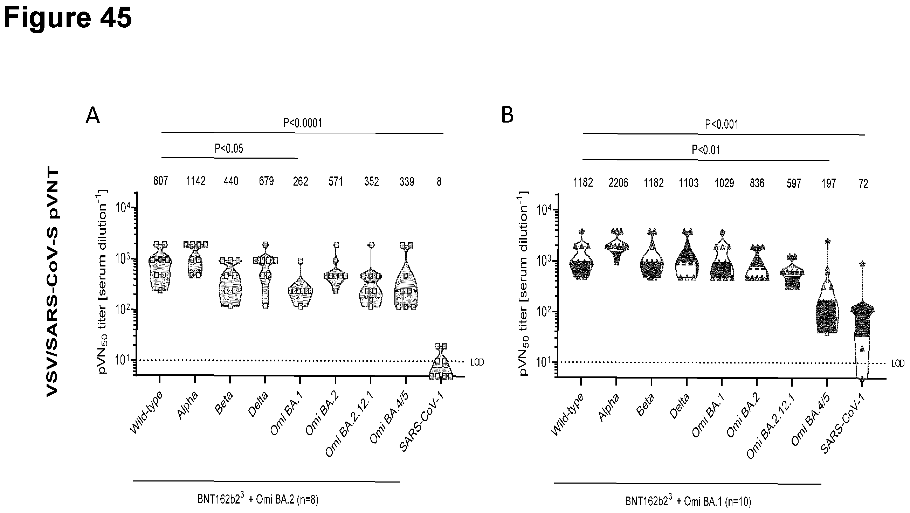

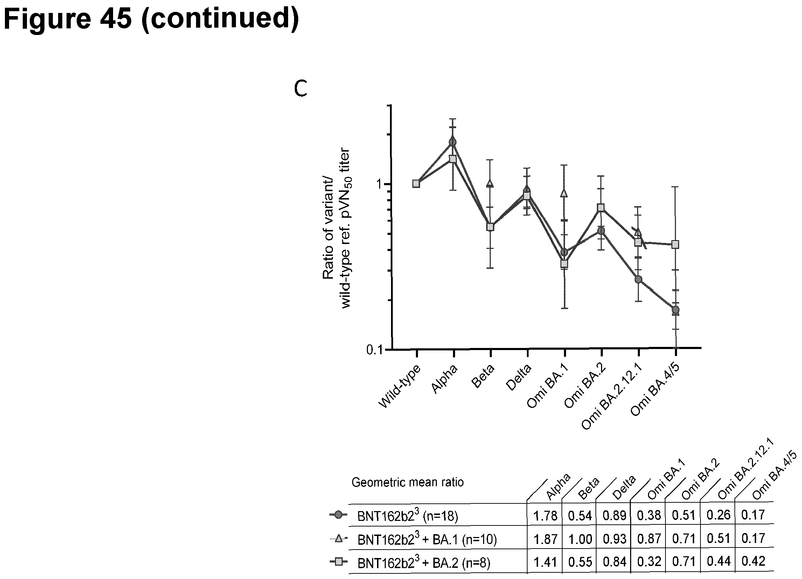

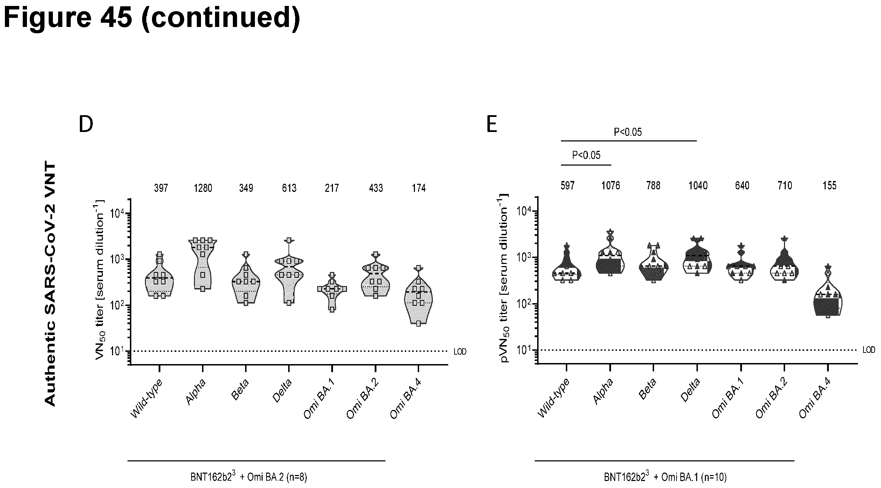

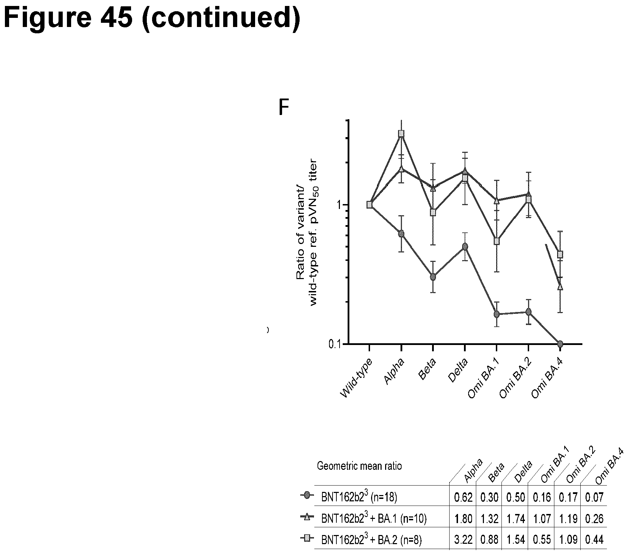

**Fig. 45****. Omicron BA.2 breakthrough infection of BNT162b2 triple-vaccinated individuals induces broad neutralization of VOCs including Omicron BA.4/BA.5.** Cohorts and serum sampling as described in Fig. 41. (A)-(B) 50% pseudovirus neutralization (pVNso) geometric mean titers (GMTs) against the indicated SARS-CoV-2 variants of concern (VOCs) or SARS-CoV-1 pseudoviruses. Values listed above violin plots represent group GMTs. (C) The ratio of SARS-CoV-2 VOC pVN₅₀ GMTs normalized against the wild-type strain pVN₅₀ GMT. Geometric mean ratios for the Omicron BA.2 breakthrough infected cohort were compared to BNT162b2³ and BNT162b2³ + BA.1. Group geometric mean ratios with 95% confidence intervals are shown. (D)-(E) 50% virus neutralization (VN₅₀) GMTs for BNT162b2³ + BA.2 and BNT162b2³ + BA.1. Values listed above violin plots represent group GMTs. (F) The ratio of SARS-CoV-2 VOC GMTs normalized against the wild-type strain VN₅₀ GMT. Serum was tested in duplicate. For titer values below the limit of detection (LOD), LOD/2 values are plotted. The non-parametric Friedman test with Dunn's multiple comparisons correction was used to compare the group GMT against the wild-type strain with group GMTs against the indicated variants and SARS-CoV-1. Multiplicity-adjusted p values are shown.

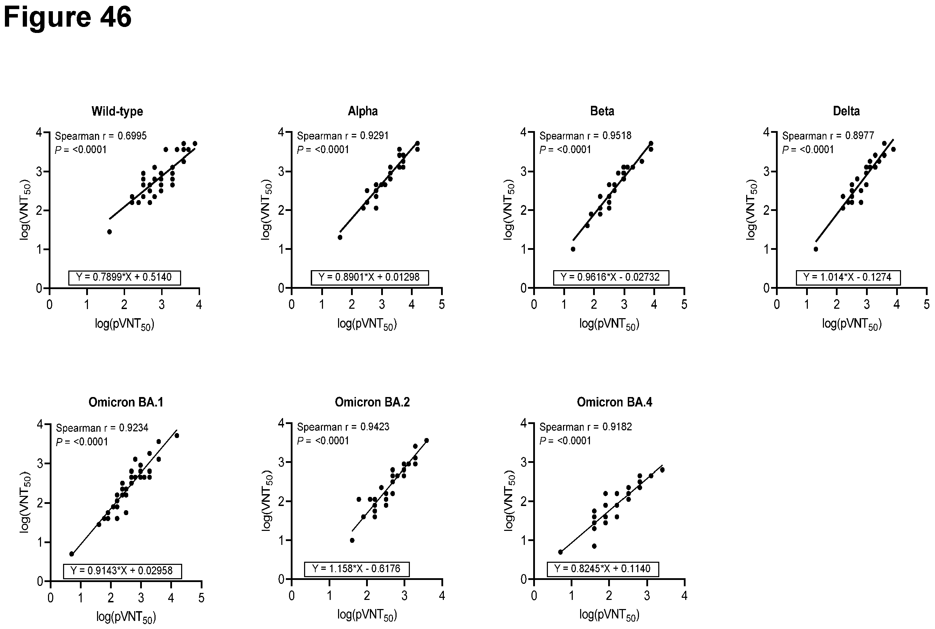

**Fig. 46**. 50% pseudovirus neutralization (pVN**₅₀) correlates with 50% live SARS-CoV-2 neutralization (VN₅₀) titer data.** Nonparametric Spearman correlation of VSV-SARS-CoV-2 pVN₅₀ with live SARS-CoV-2 VN₅₀ titers for n=45 serum samples drawn from SARS-CoV-2-naïve BNT162b2 triple-vaccinated individuals (BNT162b2³; n=18) after the third dose, from triple mRNA vaccinated individuals with subsequent Omicron BA.1 breakthrough infection (mRNA-Vax³ + BA.1; n=14) post-infection, and from triple mRNA vaccinated individuals with subsequent Omicron BA.2 breakthrough infection (mRNA-Vax³ + BA.2; n=13) post-infection. Correlations are plotted per SARS-CoV-2 variant. Correlation coefficient r, two-tailed P values and the linear equation are given.

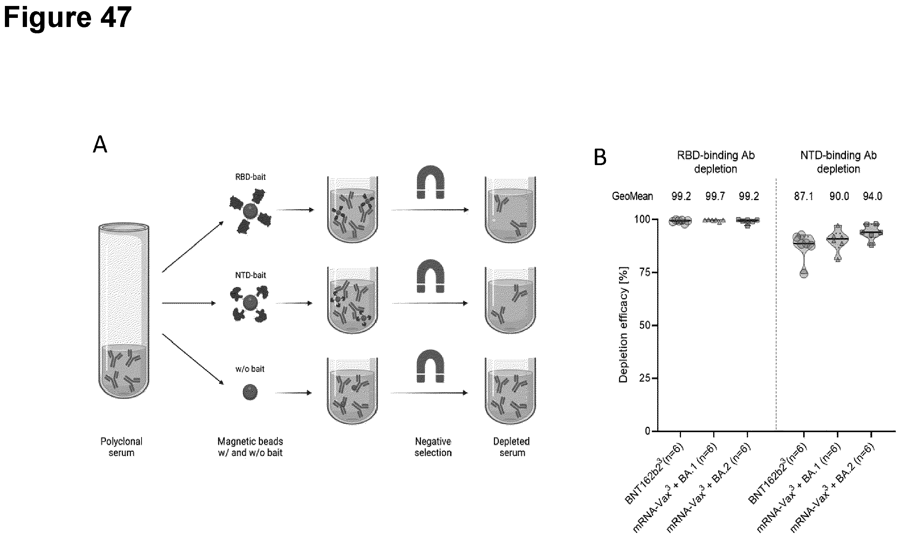

**Fig. 47****. RBD-binding and NTD-binding antibodies can be depleted from human serum.** Serum was drawn from SARS-CoV-2-naïve BNT162b2 triple-vaccinated individuals (BNT162b2³; n=6), and from triple RNA vaccinated individuals with Omicron BA.1 (mRNA-Vax³ + BA.1; n=6) or Omicron BA.2 breakthrough infection (mRNA-Vax³ + BA.2; n=6). Magnetic bead technology was used for depleting serum of RBD- or NTD-binding antibodies, or for mock depleting. (A) Schematic of antibody depletion from serum. (B) The relative concentration of RBD-binding and NTD-binding antibodies was determined by a multiplexed electrochemiluminescence immunoassay. The relative decrease in antibody concentrations in depleted compared to mock-depleted sera are shown. Numbers above graph depict geometric mean reduction within groups.

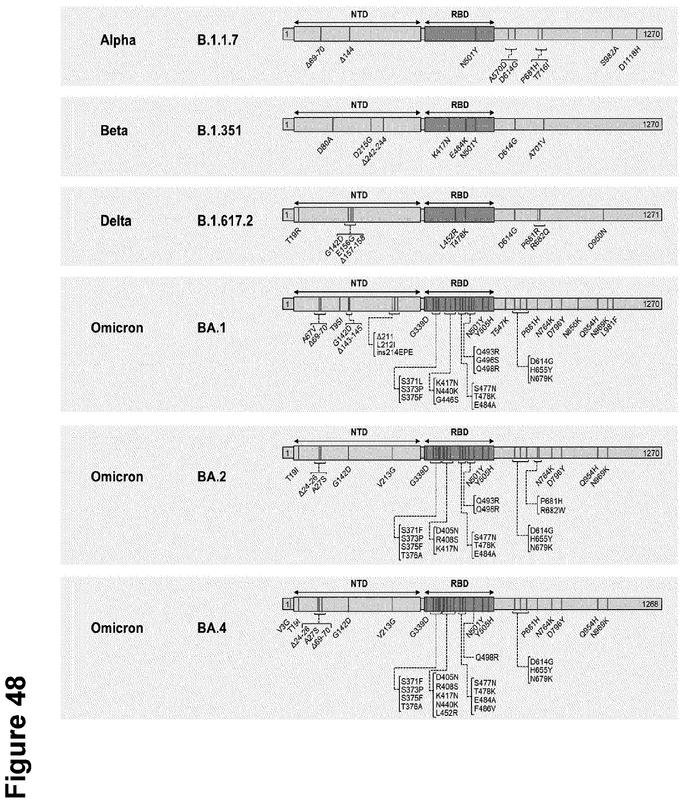

**Fig. 48****. Characterization of SARS-CoV-2 S glycoproteins used in the assays based on live authentic SARS-CoV-2.** The sequence of the Wuhan-Hu-1 isolate SARS-CoV-2 S glycoprotein (GenBank: QHD43416.1) was used as reference. Amino acid positions, amino acid descriptions (one letter code) and kind of alterations (substitutions, deletions, insertions) are indicated. NTD, N-terminal domain; RBD, Receptor-binding domain, Δ, deletion; ins, insertion; *, Cytoplasmic domain truncated for the C-terminal 19 amino acids.

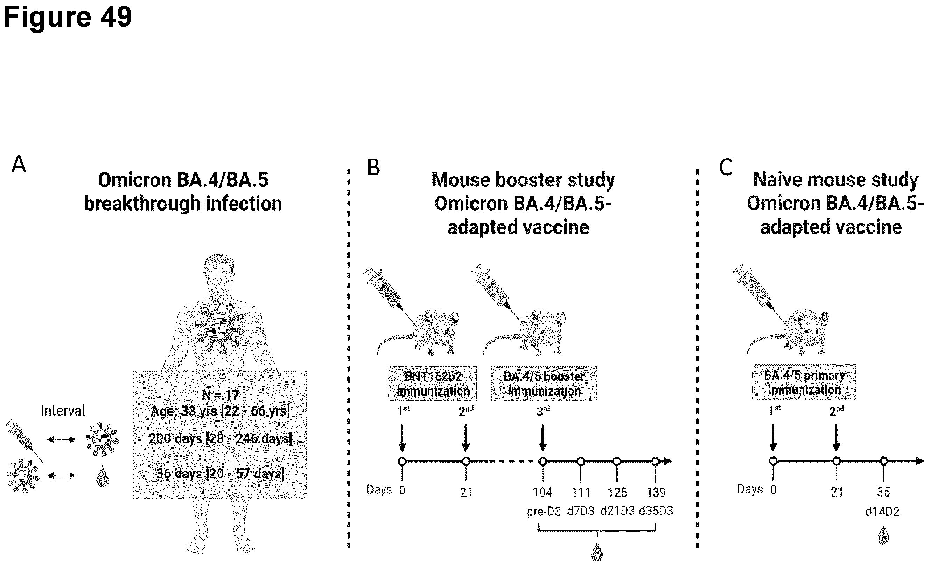

**Fig. 49****. BA.4/5-Breakthrough Infection and BA.4/5-Booster Study Design,** (a) The effect of Omicron BA.4/BA.5 breakthrough infection on serum neutralizing activity was evaluated in individuals vaccinated with three doses of mRNA COVID-19 vaccine (BNT162b2/mRNA-1273 homologous or heterologous regimens) who subsequently experienced an infection with Omicron BA.4 or BA.5. The intervals between vaccination, breakthrough infection and sampling are indicated as median/range. (b) Effects of Omicron BA.4/BA.5-adapted booster vaccines on serum neutralizing activity was investigated in mice vaccinated twice (with the two vaccines administered 21-days apart) with BNT162b2, followed by a booster dose of BA.4/BA.5-adapted vaccines 3.5 months later. Neutralizing activity was assessed before (pre-D3) and 7, 21, and 35 days after the booster dose (d7D3, d21D3, d35D3, respectively). (c) The effects of Omicron BA.4/BA.5-adapted vaccines on serum neutralizing activity were investigated in vaccine-naïve mice vaccinated twice (with the two vaccines administered 21-days apart) with BA.4/BA.5-adapted vaccines. Neutralizing activity was assessed 14 days after administration of the second dose (d14D2).

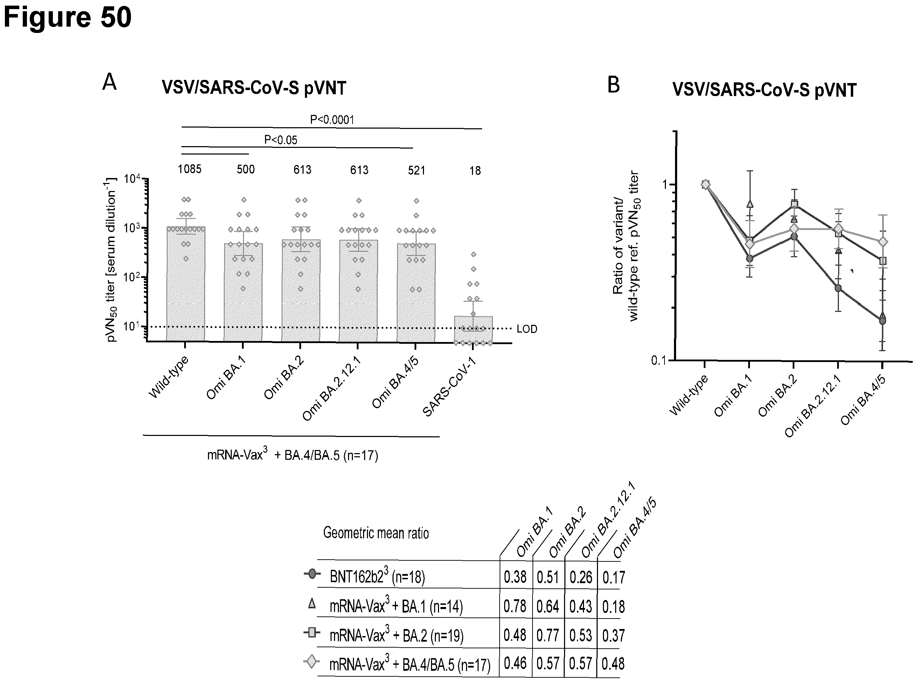

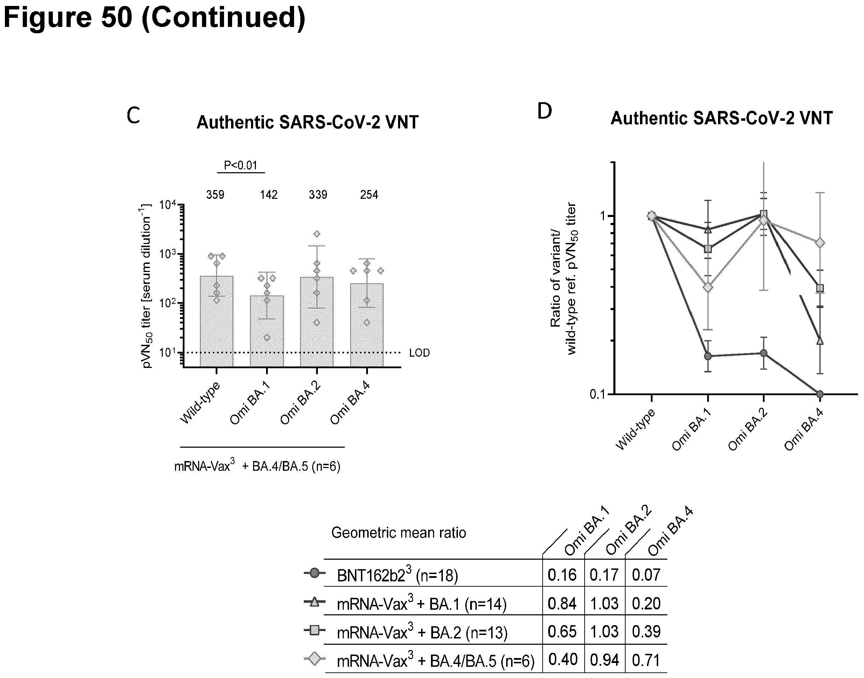

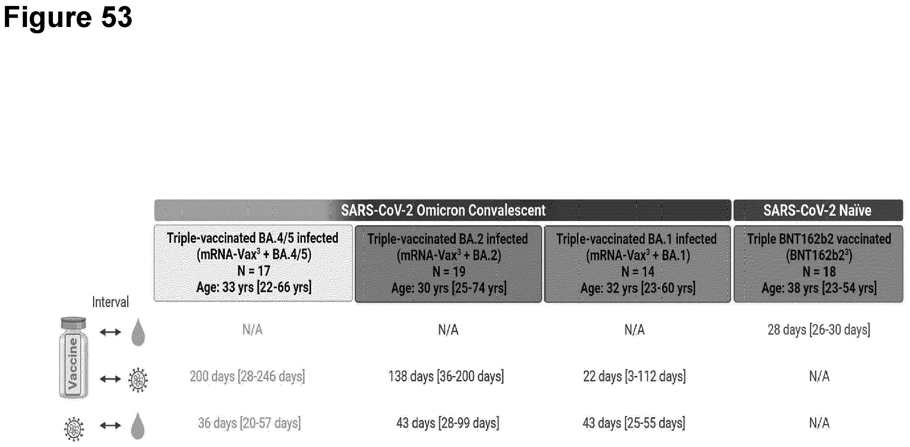

**Fig. 50****. Omicron BA.4/BA.5 breakthrough infection of triple mRNA vaccinated individuals mediates pan-Omicron neutralization.** Cohorts and serum sampling as described in Figure 53. (a) 50% pseudovirus neutralization (pVN₅₀) geometric mean titers (GMTs) in sera of mRNA-Vax³ + BA.4/BA.5 against the indicated SARS-CoV-2 variants of concern (VOCs) or SARS-CoV-1 pseudoviruses. Values above bars represent group GMTs. (b) SARS-CoV-2 VOC pVNso GMTs normalized against the wild-type strain pVN₅₀ GMT (ratio VOC to wild-type) of mRNA-Vax³ + BA.4/BA.5 and the reference cohorts as outlined in Figure 53. Group geometric mean ratios with 95% confidence intervals are shown. (c) 50% virus neutralization (VN₅₀) GMTs in sera of mRNA-Vax³ + BA.4/BA.5 against the indicated SARS-CoV-2 VOCs. (d) SARS-CoV-2 VOC VN₅₀ GMTs normalized against the wild-type strain VN₅₀ GMT (ratio VOC to wild-type). Serum was tested in duplicate. For titer values below the limit of detection (LOD), LOD/2 values were plotted. The non-parametric Friedman test with Dunn's multiple comparisons correction was used to compare the wild-type strain neutralizing group GMTs with titers against the indicated variants and SARS-CoV-1. Multiplicity-adjusted p values are shown.

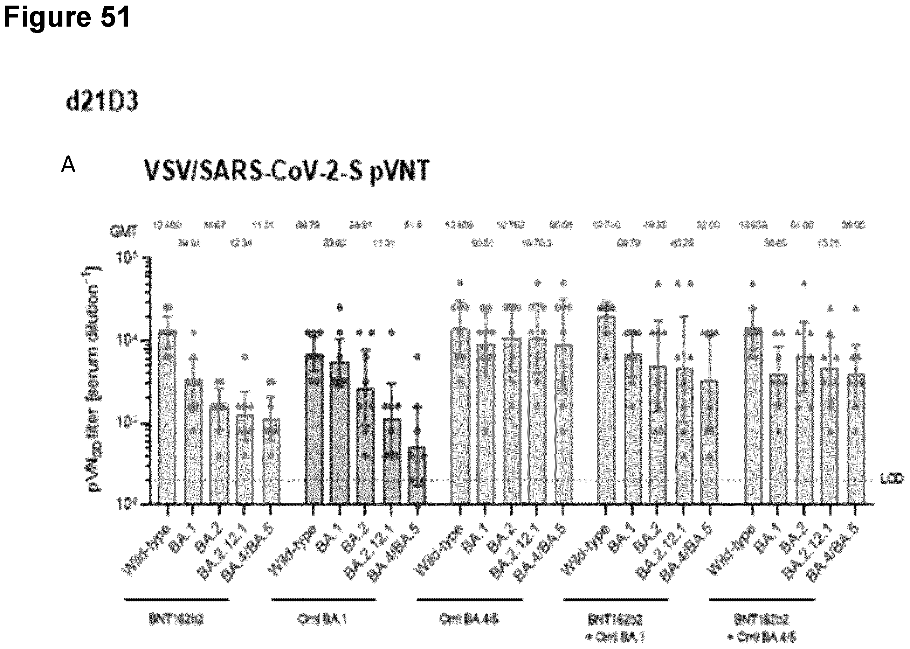

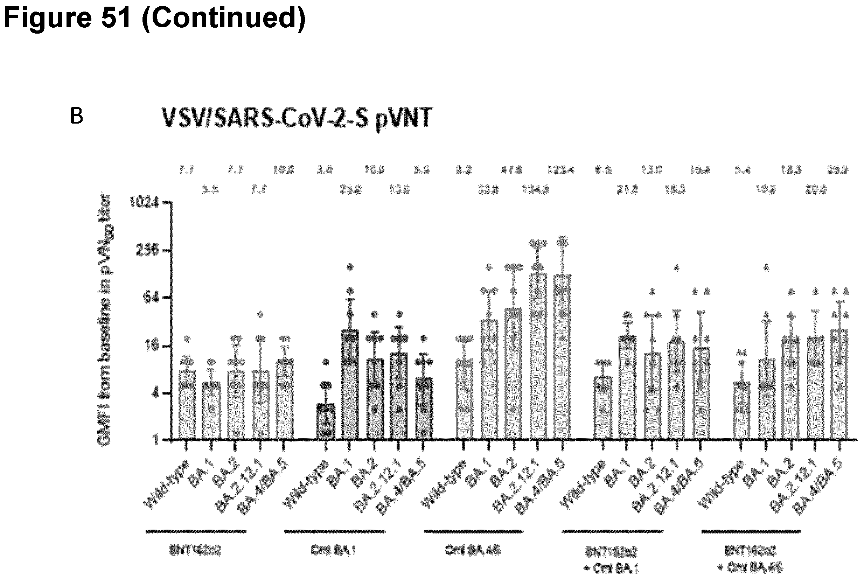

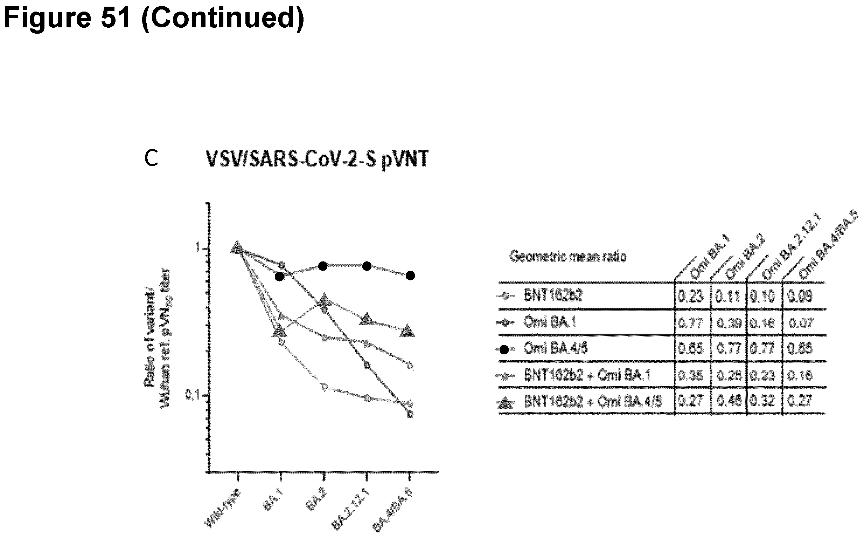

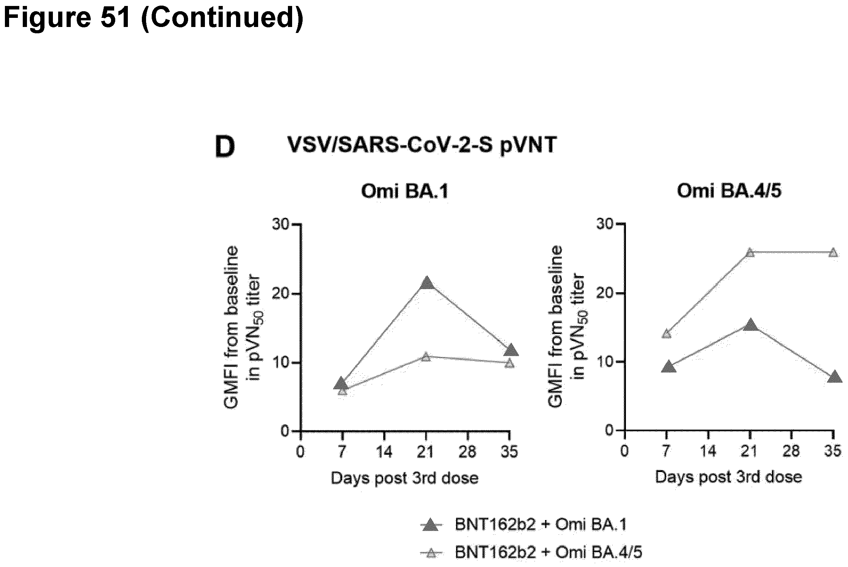

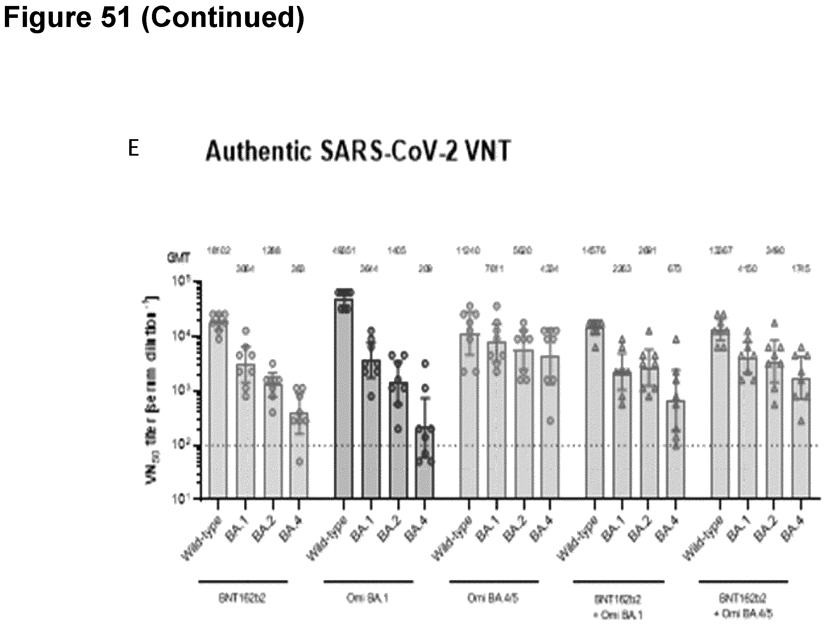

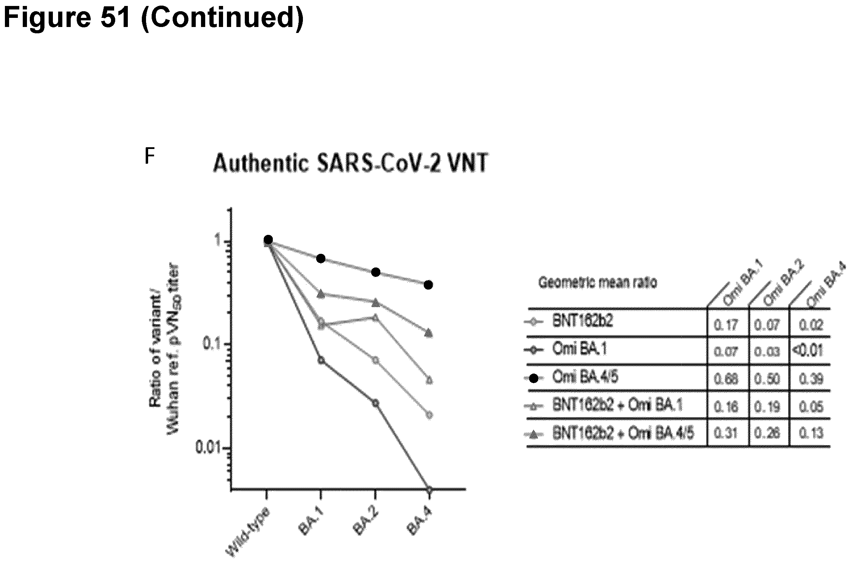

**Fig. 51****. Booster immunization with an Omicron BA.4/BA.5 S glycoprotein adapted RNA-vaccine mediates pan-Omicron neutralization in double-vaccinated mice.** BALB/c mice (n=8) were injected intramuscularly with two doses of 1 µg BNT162b2 21 days apart, and a third dose of either BNT162b2 (1 µg) or the indicated monovalent (1 µg) or bivalent (0.5 µg each) Omicron BA.1 or BA.4/5-adapted vaccines 104 days after the first vaccination. (a) 50% pseudovirus neutralization (pVN₅₀) geometric mean titers (GMTs) against the indicated SARS-CoV-2 variants of concern (VOCs) in sera collected 21 days after the third vaccination (d21D3). Values above bars represent group GMTs. (b) Geometric mean fold-increase (GMFI) of pVNso titers on d21D3 relative to baseline titers before the third vaccination. Values above bars represent group GMFIs. (c) SARS-CoV-2 VOC pVN₅₀ GMTs normalized against the wild-type strain pVN₅₀ GMT (ratio VOC to wild-type). Group geometric mean ratios are shown. (d) GMFI of pVN₅₀ titers against BA.1 and BA.4/5 over time relative to baseline titers before the booster vaccination with BNT162b2/BA.1 or BNT162b2/BA.4/5 bivalent vaccines. For titer values below the limit of detection (LOD), LOD/2 values were plotted. Error bars represent 95% confidence intervals (e) 50% virus neutralization (VN₅₀) GMTs against the indicated SARS-CoV-2 VOCs on d21D3. Values above bars represent group GMTs. Error bars represent 95% confidence interval. (f) SARS-CoV-2 VOC VN₅₀ GMTs normalized against the wild-type strain VN₅₀ GMT (ratio VOC to wild-type). Group geometric mean ratios are shown. Serum was tested in duplicate. For titer values below the limit of detection (LOD), LOD/2 values were plotted. Error bars represent 95% confidence intervals.

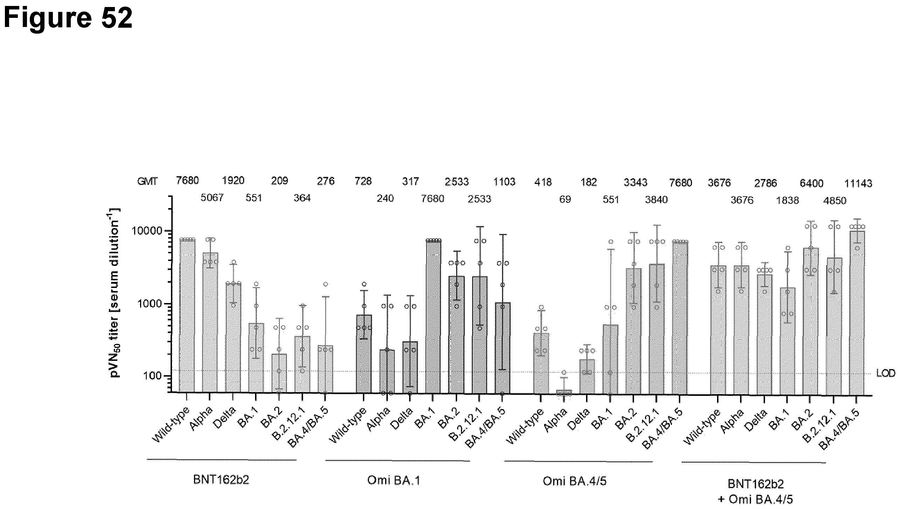

**Fig. 52****. Immunization with an Omicron BA.4/BA.5 S glycoprotein supplemented BNT162b2 mRNA vaccine drives pan-Omicron neutralization in previously unvaccinated mice.** Vaccine-naïve BALB/c mice (n=5) were injected intramuscularly with two doses of either BNT162b2 (1 µg) or the indicated monovalent (1 µg) or bivalent (0.5 µg each) Omicron BA.1 or BA.4/5-adapted vaccines, 21 days apart. (a) 50% pseudovirus neutralization (pVN₅₀) geometric mean titers (GMTs) against the indicated SARS-CoV-2 variants of concern (VOCs) in sera collected 14 days after the second vaccination (d14D2). Values above bars represent group GMTs. Error bars represent 95% confidence interval. Serum was tested in duplicate. For titer values below the limit of detection (LOD), LOD/2 values were plotted. "Wild-type" refers to neutralization titers against a pseudovirus comprising a SARS-CoV-2 S protein of the original Wuhan variant.
**Fig. 53****. Cohorts and sampling.** Serum samples (droplet) were drawn from four cohorts: individuals vaccinated with three doses of mRNA COVID-19 vaccine (BNT162b2/mRNA-1273 homologous or heterologous regimens) who subsequently had a breakthrough infection with Omicron BA.4/BA.5 (mRNA-Vax³ + BA.4/BA.5). The breakthrough infections occurred at a time of BA.4/BA.5 dominance and/or were variant-confirmed by genome sequencing. Three cohorts were included in the study as references: triple-mRNA vaccinated individuals who experienced breakthrough infection at a time of either BA.2 dominance (March to May 2022; mRNA-Vax³ + BA.2), or BA.1 dominance (November 2021 to January 2022; mRNA-Vax³ + BA.1), or individuals triple-vaccinated with BNT162b2 that were SARS-CoV-2-naïve at the time of sampling (BNT162b2³). For convalescent cohorts, relevant intervals between key events such as the most recent vaccination, SARS-CoV-2 infection, and serum isolation are indicated. All values specified as median-range. N/A, not applicable.

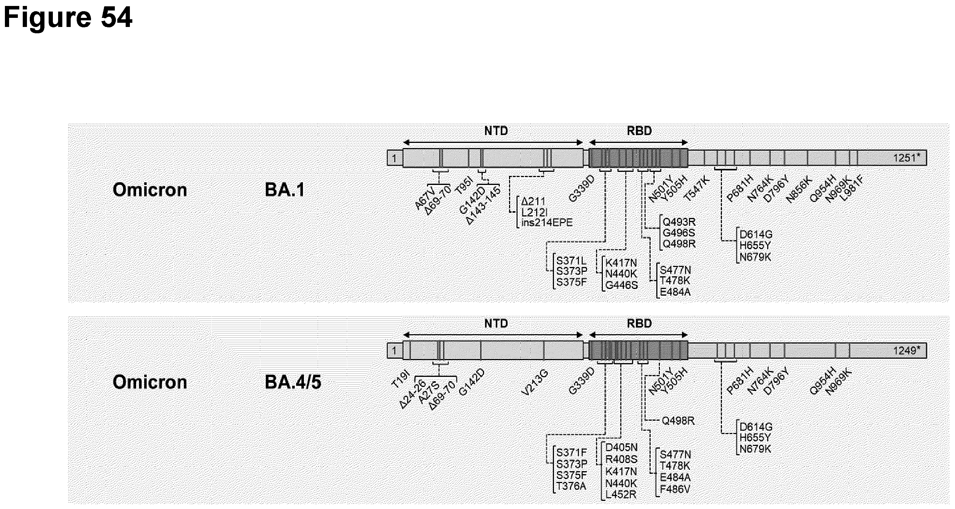

**Fig. 54****. Design of Omicron-adapted vaccines.** Characterization of SARS-CoV-2 S glycoproteins encoded by variant-specific RNA vaccines (e.g., in some embodiments mRNA vaccines). The sequence of the Wuhan-Hu-1 isolate SARS-CoV-2 S glycoprotein (GenBank: QHD43416.1) was used as reference. Amino acid positions, amino acid descriptions (one letter code) and type of alterations (substitutions, deletions, insertions) are indicated. NTD, N-terminal domain; RBD, Receptor-binding domain, Δ, deletion; ins, insertion. In some embodiments, variant specific vaccines further comprise mutations that stabalize a pre-fusion conformation (e.g., proline mutations at positions corresponding to residues 986 and 987 of SEQ ID NO: 1) and/or do not comprise a C-terminal truncation.

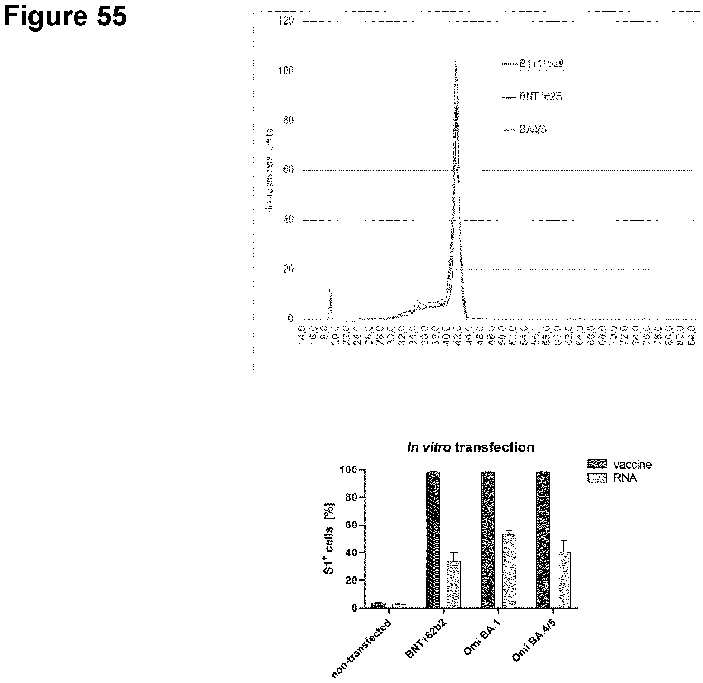

**Fig. 55****. Omicron-specific vaccines exhibit comparable RNA purity and integrity, and *in vitro* expression of antigens.** (a) Liquid capillary electropherograms of *in vitro* transcribed samples. (b) Surface expression of BNT162b2 and Omicron-adapted vaccines in HEK293T cells measured using mFc-tagged human ACE-2 as a detection reagent with subsequent analysis in flow cytometry. HEK293T cells were transfected with BNT162b2 or Omicron-adapted vaccines formulated as lipid nanoparticles or vaccine RNAs mixed with a commercial transfection reagent, or no vaccine/RNA (non-transfected). Heights of bars indicate the means of technical replicates.

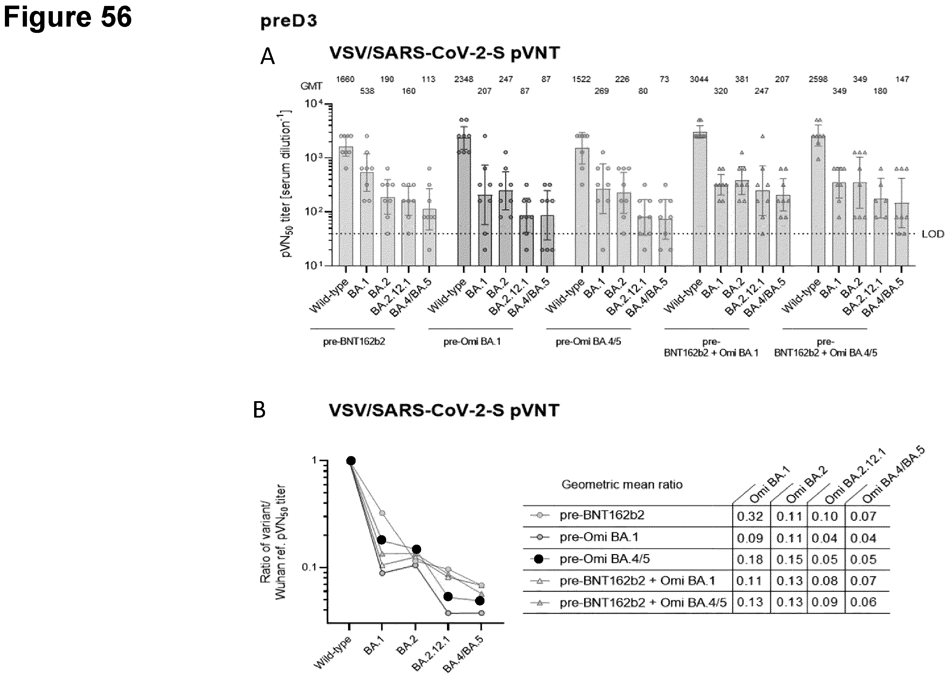

**Fig. 56****. Breadth and magnitude of neutralizing activity against SARS-CoV-2 variants are comparable in BNT162b2-vaccinated mice prior to booster vaccination.** BALB/c mice were injected intramuscularly with two doses of 1 µg BNT162b2, administered 21 days apart. Mice were allocated to groups (n=8) prior to administration with a booster dose of the indicated vaccines. Serum was collected from mice on day 104 after the first vaccination, before booster vaccines were injected. (a) 50% pseudovirus neutralization (pVN₅₀) geometric mean titers (GMTs) against the indicated SARS-CoV-2 variants of concern (VOCs). Values above bars represent group GMTs. Error bars represent 95% confidence intervals. (b) SARS-CoV-2 VOC pVN₅₀ GMTs normalized against the wild-type strain pVN₅₀ GMT (ratio VOC to wild-type). Group geometric mean ratios are shown. Serum was tested in duplicate. For titervalues below the limit of detection (LOD), LOD/2 values were plotted.

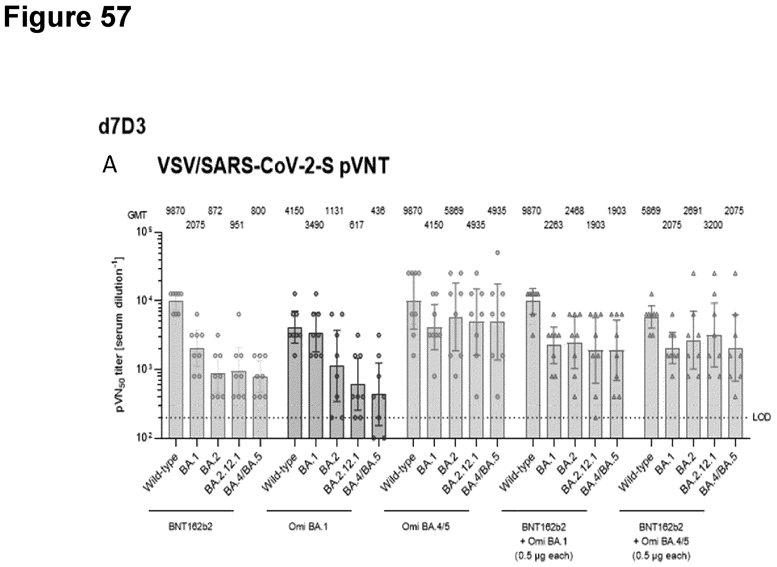

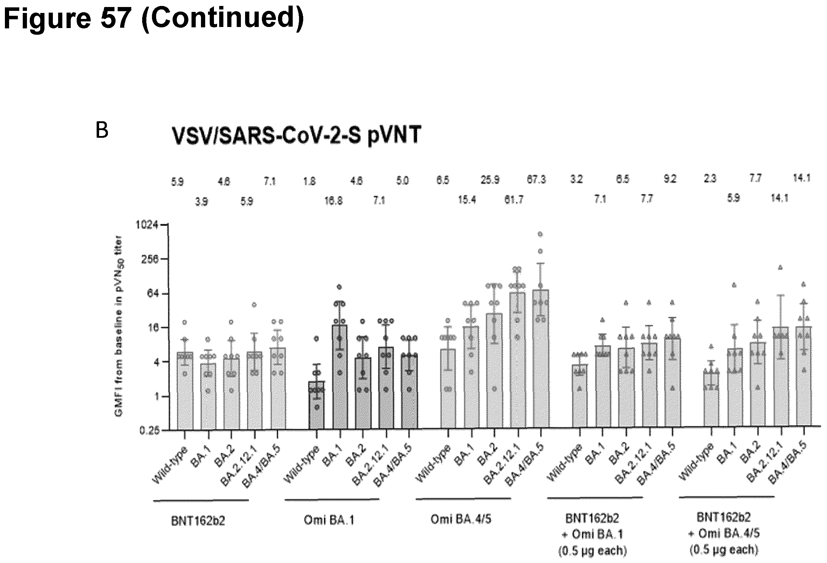

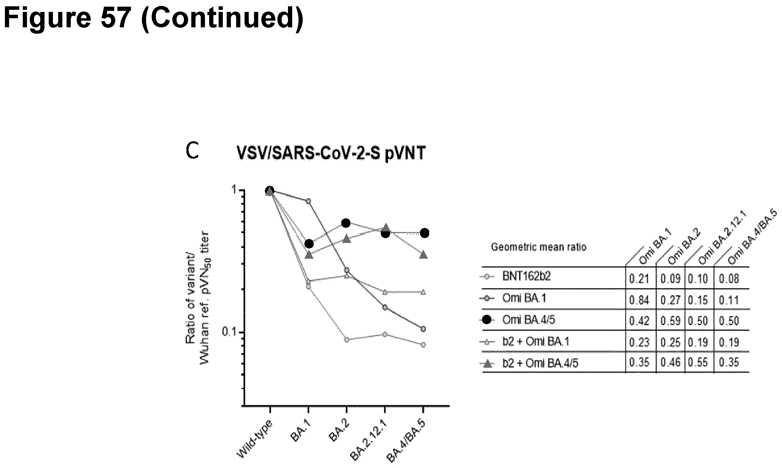

**Fig. 57****. Booster immunization with an Omicron BA.4/BA.5 S glycoprotein adapted mRNA vaccine mediates pan-Omicron neutralization in mice 7 days after administering the booster.** BALB/c mice (n=8) were injected intramuscularly with two doses of 1 µg BNT162b2 (with a 21-day interval between the two doses), and a third dose of either BNT162b2 (1 µg) or the indicated monovalent (1 µg) or bivalent (0.5 µg each) Omicron BA.1 or BA.4/5-adapted vaccines 104 days after the first vaccination. (a) 50% pseudovirus neutralization (pVN₅₀) geometric mean titers (GMTs) against the indicated SARS-CoV-2 variants of concern (VOCs) in sera collected 7 days after the third vaccination (d7D3). Values above bars represent group GMTs. (b) Geometric mean fold-increase (GMFI) of pVNso titers on d7D3 relative to baseline titers before the third vaccination. Values above bars represent group GMFIs. (c) SARS-CoV-2 VOC pVN₅₀ GMTs normalized against the wild-type strain pVN₅₀ GMT (ratio VOC to wild-type). Group geometric mean ratios are shown. Serum was tested in duplicate. For titer values below the limit of detection (LOD), LOD/2 values were plotted. Error bars represent 95% confidence intervals.

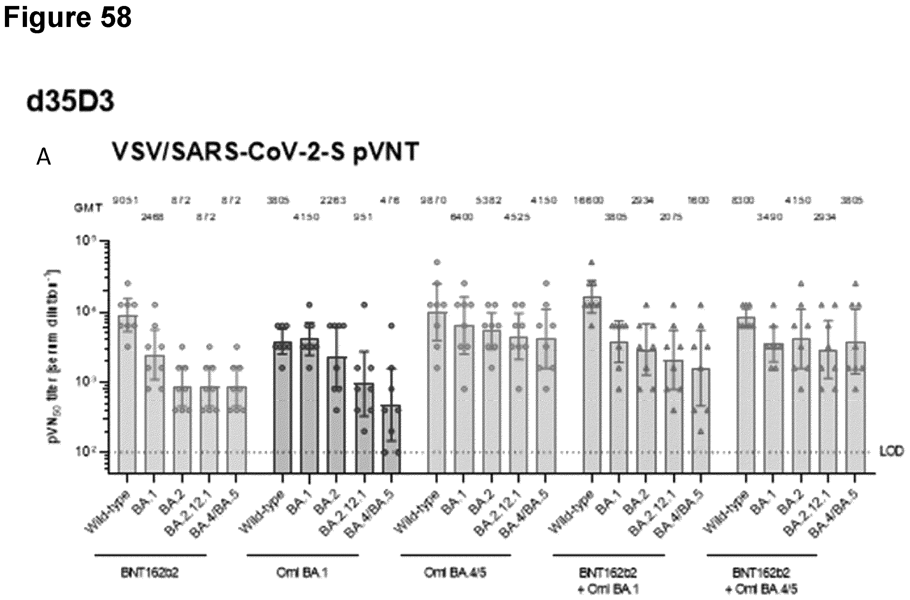

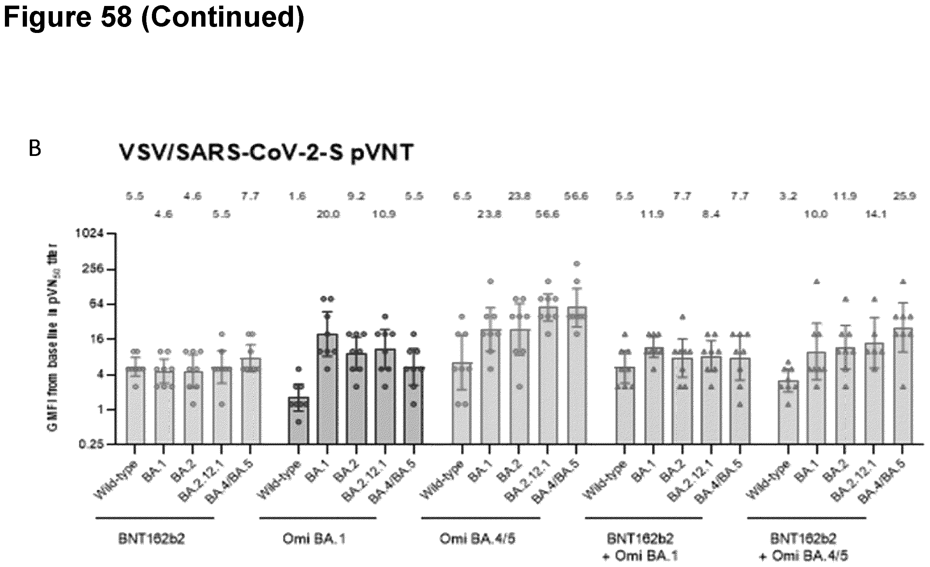

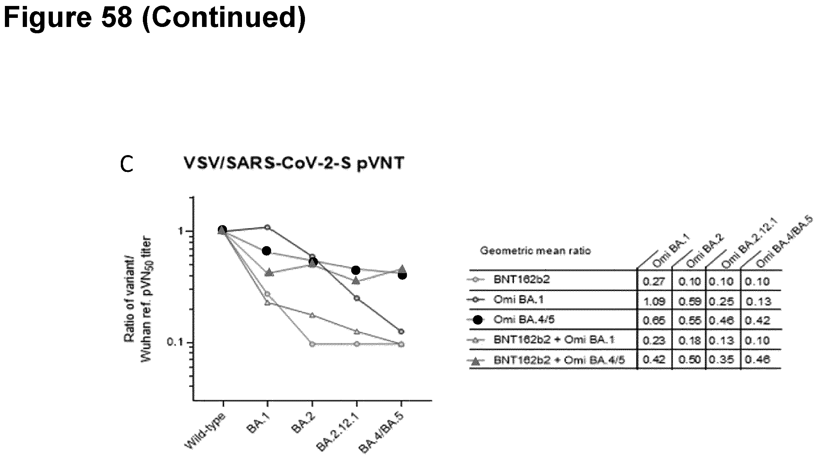

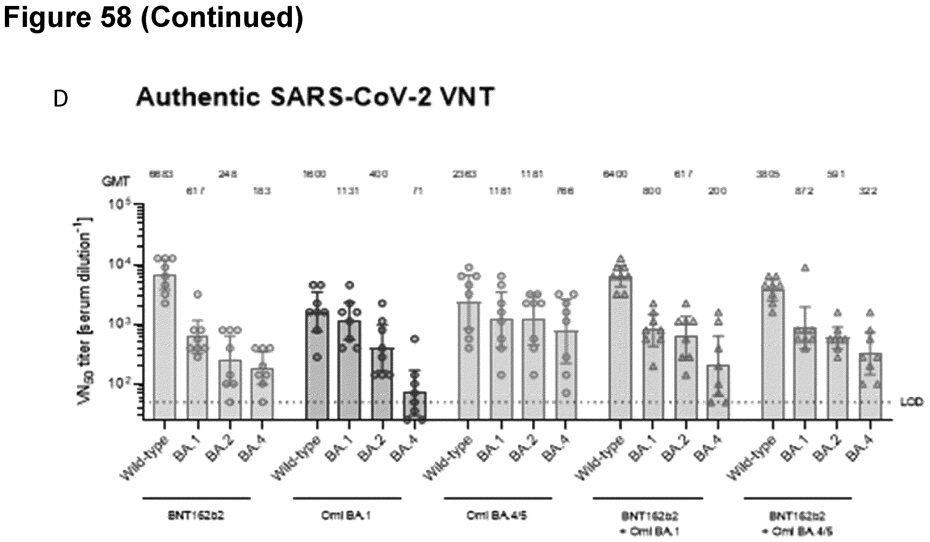

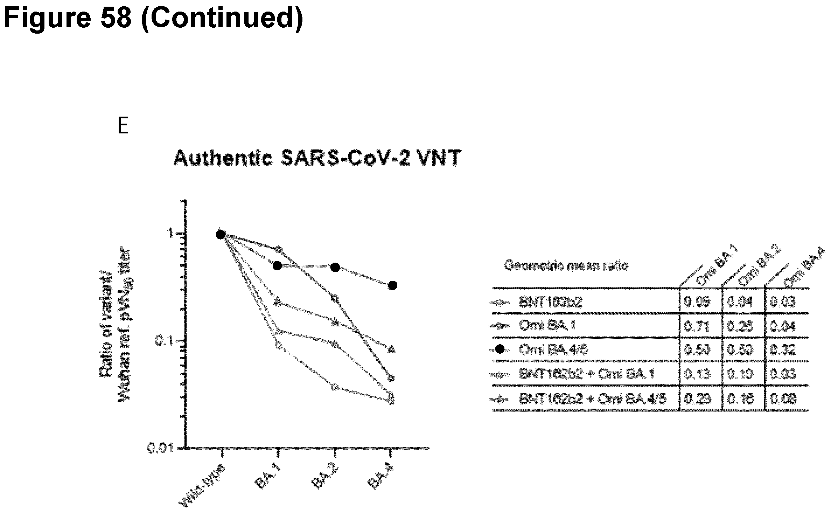

**Fig. 58****. Booster immunization with an Omicron BA.4/BA.5 S glycoprotein adapted vaccine mediates pan-Omicron neutralization in mice 35 days after the booster.** BALB/c mice (n=8) were injected intramuscularly with two doses of 1 µg BNT162b2 (with a 21-day interval between the two doses), and a third dose of either BNT162b2 (1 µg) or the indicated monovalent (1 µg) or bivalent (0.5 µg each) Omicron BA.1 or BA.4/5-adapted vaccines 104 days after the first vaccination. (a) 50% pseudovirus neutralization (pVN₅₀) geometric mean titers (GMTs) against the indicated SARS-CoV-2 variants of concern (VOCs) in sera collected 35 days after the third vaccination (d35D3). Values above bars represent group GMTs. (b) Geometric mean fold-increase (GMFI) of pVN₅₀ titers on d35D3 relative to baseline titers before the third vaccination. Values above bars represent group GMFIs. (c) SARS-CoV-2 VOC pVN₅₀ GMTs normalized against the wild-type strain pVN₅₀ GMT (ratio VOC to wild-type). Group geometric mean ratios are shown. (d) 50% virus neutralization (VN₅₀) GMTs against the indicated SARS-CoV-2 VOCs on d35D3. Values above bars represent group GMTs. Error bars represent 95% confidence interval. (e) SARS-CoV-2 VOC VN₅₀ GMTs normalized against the wild-type strain VN₅₀ GMT (ratio VOC to wild-type). Group geometric mean ratios are shown. Serum was tested in duplicate. For titer values below the limit of detection (LOD), LOD/2 values were plotted. Error bars represent 95% confidence intervals.

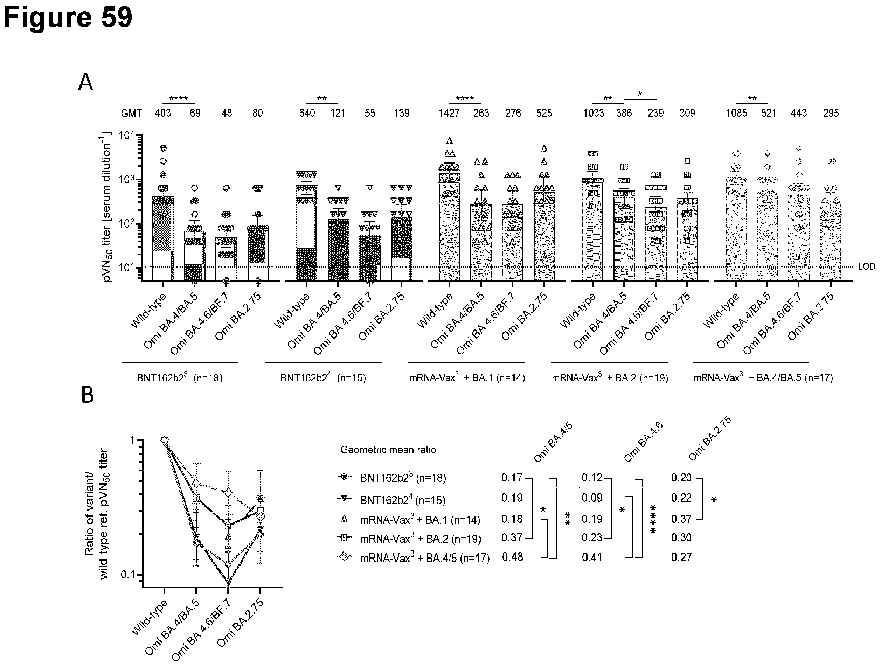

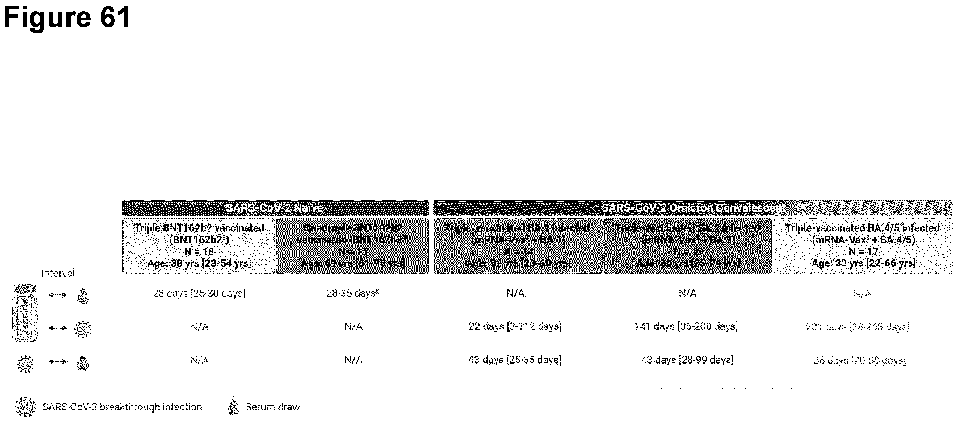

**Fig. 59****. Omicron BA.4/BA.5 breakthrough infection of triple mRNA vaccinated individuals induces cross-neutralization of Omicron BA.4.6 and BA.2.75.** Cohorts and serum sampling as described in Fig. 61. (a) 50% pseudovirus neutralization (pVN₅₀) geometric mean titers (GMTs) against the indicated SARS-CoV-2 wild-type strain or Omicron variants of concern (VOCs). Values above bar graphs represent group GMTs. For titer values below the limit of detection (LOD), LOD/2 values were plotted. The non-parametric Friedman test with Dunn's multiple comparisons correction was used to compare neutralizing titers against the Omicron BA.4/BA.5 pseudovirus (which represents currently dominating BA.5) with titers against the other pseudoviruses. Multiplicity-adjusted p values are shown. (b) SARS-CoV-2 VOC pVN₅₀ GMTs normalized against the wild-type strain pVN₅₀ GMT (ratio VOC to wild-type). Group geometric mean ratios with 95% confidence intervals are shown. The non-parametric Kruskal-Wallis test with Dunn's multiple comparisons correction was used to compare the VOC GMT ratios between cohorts. ****, P<0.0001; **, P<0.01; *, P<0.05. Serum was tested in duplicate.

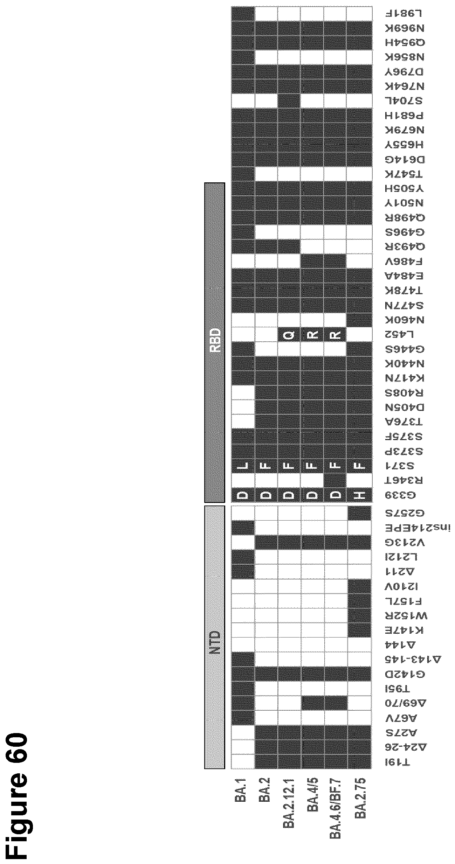

**Fig. 60****. Alterations of the spike glycoprotein amino acid sequence of SARS-CoV-2 Omicron sub-lineages.** White letters in boxes indicate the amino acid substitution per sub-lineage; Δ, deletion; ins, insertion; NTD, N-terminal domain; RBD, receptor-binding domain.
**Fig. 61****. Cohorts and sampling.** Serum samples were drawn from five cohorts: SARS-CoV-2-naïve individuals triple-vaccinated with BNT162b2 (BNT162b2³) or quadruple-vaccinated with BNT162b2 (BNT162b2⁴), and individuals with three doses of mRNA COVID-19 vaccine (BNT162b2/mRNA-1273 homologous or heterologous regimens) who subsequently had a breakthrough infection with Omicron BA.1 (mRNA-Vax³ + BA.1), with BA.2 (mRNA-Vax³ + BA.2) or with BA.4/BA.5 (mRNA-Vax³ + BA.4/5). Breakthrough infections occurred at a time of respective VOC dominance (BA.1: November 2021 to January 2021, BA.2: March to May 2022, BA.4/5: mid-June to mid-July 2022) and/or were variant confirmed by genome sequencing. For convalescent cohorts, relevant intervals between key events such as the most recent vaccination, SARS-CoV-2 infection, and serum isolation are indicated. All values specified as median-range. N/A, not applicable; ^{§}, Serum draw was performed between 28 to 35 Days after vaccination as per protocol.

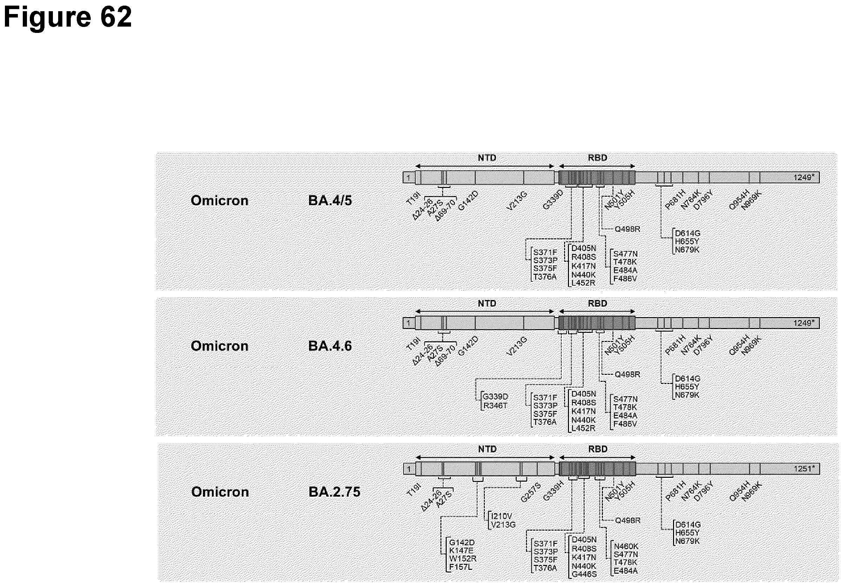

**Fig. 62****. Characterization of SARS-CoV-2 S glycoproteins used in VSV-SARS-CoV-2 variant pseudovirus neutralization assays.** Mutation positions shown in reference to the sequence of the Wuhan-Hu-1 isolate SARS-CoV-2 S glycoprotein (GenBank: QHD43416.1). Amino acid positions, amino acid descriptions (one letter code) and kind of alterations (substitutions, deletions, insertions) are indicated. NTD, N-terminal domain; RBD, Receptor-binding domain, Δ, deletion; ins, insertion; *, Cytoplasmic domain truncated for the C-terminal 19 amino acids.

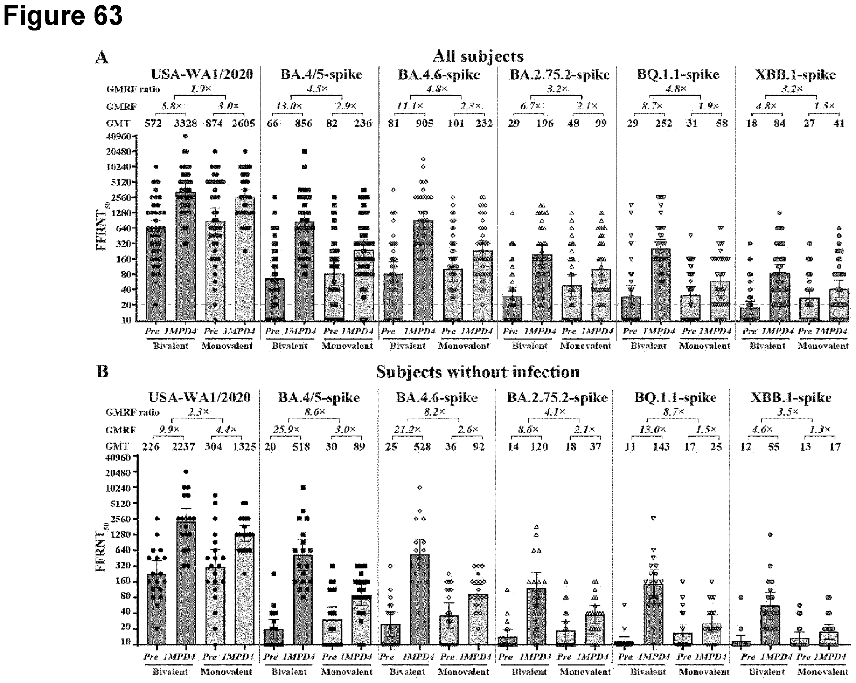

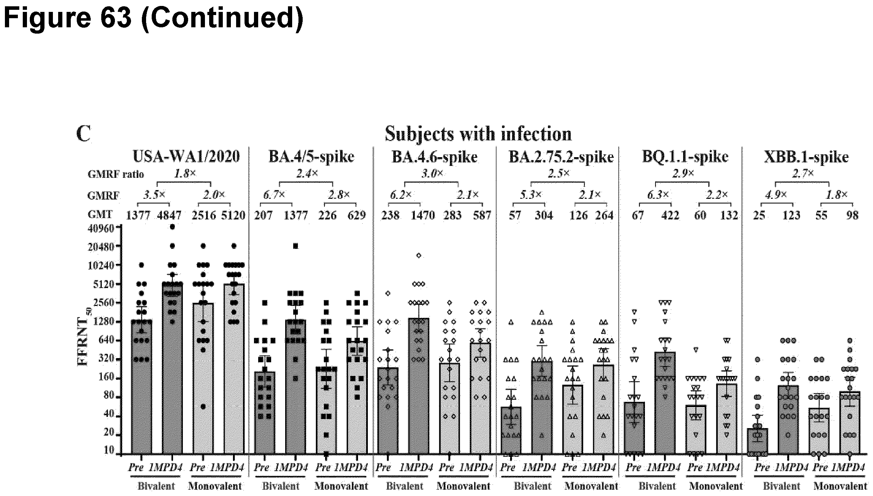

**Fig. 63****. Omicron BA.4/5, BA.4.6, BA.2.75.2, BQ.1.1, and XBB.1 neutralizing response with a bivalent (BA.4/5-adapted RNA** + **BNT162b2) or BNT16b2 monovalent booster.** Bar heights and numbers immediately above bars indicate geometric means of neutralization titers (GMTs). Whiskers indicate 95% Cl. Bars labeled as "Bivalent" indicate neutralization titers from subjects administered as a 4th dose a bivalent vaccine (comprising RNA encoding a SARS-CoV-2 S protein of a Wuhan strain and RNA encoding a SARS-CoV-2 S protein comprising one or more mutations characteristic of a BA.4/5 Omicron variant); bars labeled as "Monovalent" indicate neutralization titers from subjects administered as a 4th dose a monovalent vaccine (comprising RNA encoding a SARS-CoV-2 S protein of a Wuhan strain). FFRNT₅₀s against USA-WA1/2020 spike, BA.4/5-spike, BA.4.6-spike, BA.2.75.2-spike, BQ.1.1-spike, XBB.1-spike are shown for the bivalent or monovalent booster. "Pre" samples correspond to serum samples collected on the day of booster administration; "1MPD4" samples correspond to serum samples collected one month post dose 4 (i.e., one month post booster administration). GMFR corresponds to GMT fold rise, and was calculated as a ratio of 1MPD4 GMTs to Pre GMTs. Numbers above GMFRs indicate ratios between GMFRs of bivalent and GMFRs of monovalent. (A) FFRNT₅₀s of all subjects regardless of infection status. p values (two-tailed, Wilcoxon matched-pairs signed-rank test) for GMFR of bivalent or monovalent booster against USA-WA1/2020, BA.4/5-spike, BA.4.6-spike, BA.2.75.2-spike, BQ.1.1-spike, XBB.1-spike: all <0.0001. p values (two-tailed, Mann-Whitney test) for GMFR ratios of bivalent to monovalent booster against USA-WA1/2020, BA.4/5-spike, BA.4.6-spike, BA.2.75.2-spike, BQ.1.1-spike, XBB.1-spike: 0.0061, <0.0001, <0.0001, <0.0001, <0.0001, <0.0001. (B) FFRNTsos of all subjects without evidence of prior SARS-CoV-2 infection, p values (two-tailed, Wilcoxon matched-pairs signed-rank test) for GMFR of bivalent booster against USA-WA1/2020, BA.4/5-spike, BA.4.6-spike, BA.2.75.2-spike, BQ.1.1-spike, XBB.1-spike: all <0.0001. p values (two-tailed, Wilcoxon matched-pairs signed-rank test) for GMFR of monovalent booster against USA-WA1/2020, BA.4/5-spike, BA.4.6-spike, BA.2.75.2-spike, BQ.1.1-spike, XBB.1-spike: 0.013, 0.0006, <0.0001, <0.0001, 0.013, 0.016. p values (two-tailed, Mann-Whitney test) for GMFR ratios of bivalent to monovalent booster against USA-WA1/2020, BA.4/5-spike, BA.4.6-spike, BA.2.75.2-spike, BQ.1.1-spike, XBB.1-spike: 0.035, <0.0001, <0.0001, <0.0001, <0.0001, <0.0001. (C) FFRNT₅₀s of all subjects with evidence of prior SARS-CoV-2 infection, p values (two-tailed, Wilcoxon matched-pairs signed-rank test) for GMFR of bivalent booster against USA-WA1/2020, BA.4/5-spike, BA.4.6-spike, BA.2.75.2-spike, BQ.1.1-spike, XBB.1-spike: all <0.0001. p values (two-tailed, Wilcoxon matched-pairs signed-rank test) for GMFR of monovalent booster against USA-WA1/2020, BA.4/5-spike, BA.4.6-spike, BA.2.75.2-spike, BQ.1.1-spike, XBB.1-spike: 0.0016, 0.0003, 0.0026, 0.0011, <0.0001, <0.0001. p values (two-tailed, Mann-Whitney test) for GMFR ratios of bivalent to monovalent booster against USA-WA1/2020, BA.4/5-spike, BA.4.6-spike, BA.2.75.2-spike, BQ.1.1-spike, XBB.1-spike: 0.065, 0.025, 0.0032, 0.0086, 0.0064, 0.0006.

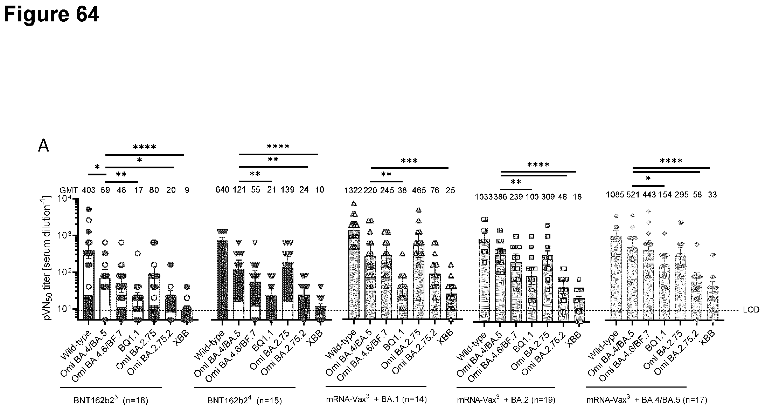

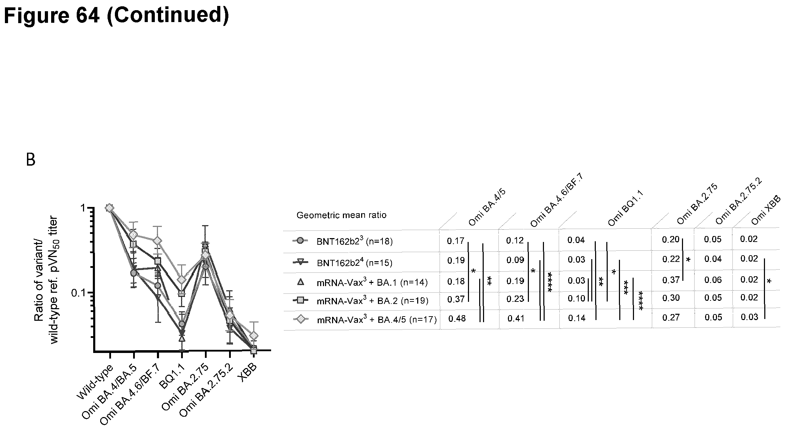

**Fig. 64****.** Distinct cross-neutralization of Omicron sublineages by vaccine-elicited and convalescent immune sera. (A) 50% pseudovirus neutralization (pVN₅₀) geometric mean titers (GMTs) against the indicated SARS-CoV-2 wild-type strain or Omicron variants of concern (VOCs) in individuals (i) triple- or (ii) quadruple-vaccinated with BNT162b2 that were SARS-CoV-2-naïve at the time of sampling, and from individuals vaccinated with three doses of mRNA COVID-19 vaccine (BNT162b2/mRNA-1273 homologous or heterologous regimens) who subsequently had a breakthrough infection with Omicron at a time of (iii) BA.1 dominance, (iv) BA.2 dominance or (v) BA.4/5 dominance. Values above bar graphs represent group GMTs. For titer values below the limit of detection (LOD), LOD/2 values were plotted. The non-parametric Friedman test with Dunn's multiple comparisons correction was used to compare neutralizing titers against the Omicron BA.4/BA.5 pseudovirus (which represents currently dominating BA.5) with titers against the other pseudoviruses. Multiplicity-adjusted p values are shown. (B) SARS-CoV-2 VOC pVNso GMTs normalized against the wild-type strain pVN₅₀ GMT (ratio VOC to wild-type). Group geometric mean ratios with 95% confidence intervals are shown. The non-parametric Kruskal-Wallis test with Dunn's multiple comparisons correction was used to compare the VOC GMT ratios between cohorts. ****, P<0.0001; ***, p<0.001; **, P<0.01; *, P<0.05. Serum was tested in duplicate.

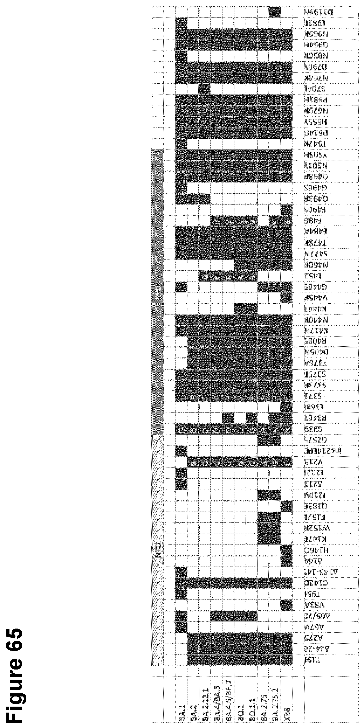

**Fig. 65****.** Alterations on the spike glycoprotein amino acid sequence of SARS-CoV-2 Omicron sub-lineages. Amino acid positions, amino acid descriptions (one letter code) and types of mutations (substitutions, deletions, insertions) are indicated. White letters in boxes indicate the amino acid substitution per sub-lineage; Δ, deletion; ins, insertion; NTD, N-terminal domain; RBD, receptor-binding domain

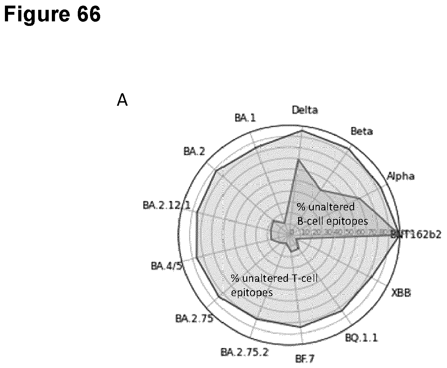

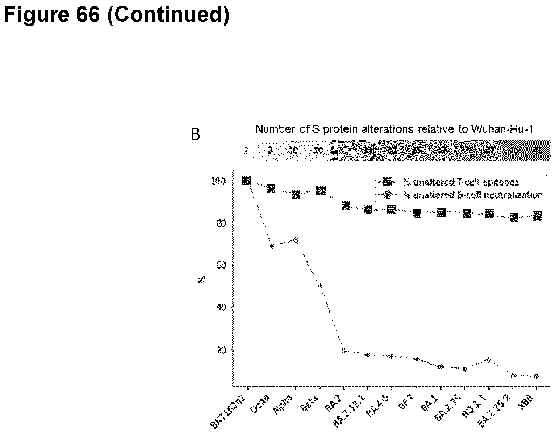

**Fig. 66****.** T cell epitope and B cell epitope retention in the S protein of certain SARS-CoV-2 variants of concern. All potential T cell epitopes were retrieved from the Immune Epitope Database (IEDB) on November 11, 2022, and the percentages of unaltered S protein linear T cell epitopes (as compared to epitopes present in BNT162b2 - 100%) present in each variant strain were calculated. Percentages of unaltered B cell neutralization epitopes (NTD and RBD) present in each variant strain compared to BNT162b2 were estimated by an automated Early Warning System (described in Beguir, Karim, et al., "Early computational detection of potential high risk SARS-CoV-2 variants," bioRxiv (2021)). (A) Radar chart and (B) line plot analysis of T-cell and neutralizing B cell epitope conservedness in the S protein across SARS-CoV-2 variants of concern. Percent unaltered T cell epitopes andpercent unaltered B cell neutralization epitopes are shown. In (B) numbers on top corresponding to each variant represent number of mutations in spike protein comparing to Wuhan strain. Variants are ranked in ascending order in terms of mutation number.

### Detailed description

Although the present disclosure is described in detail below, it is to be understood that this disclosure is not limited to the particular methodologies, protocols and reagents described herein as these may vary. It is also to be understood that the terminology used herein is for the purpose of describing particular embodiments only, and is not intended to limit the scope of the present disclosure which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

The practice of the present disclosure will employ, unless otherwise indicated, conventional methods of chemistry, biochemistry, cell biology, immunology, and recombinant DNA techniques which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

In the following, the elements of the present disclosure will be described. These elements are listed with specific embodiments, however, it should be understood that they may be combined in any manner and in any number to create additional embodiments. The variously described examples and embodiments should not be construed to limit the present disclosure to only the explicitly described embodiments. This description should be understood to disclose and encompass embodiments which combine the explicitly described embodiments with any number of the disclosed elements. Furthermore, any permutations and combinations of all described elements should be considered disclosed by this description unless the context indicates otherwise.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the present disclosure was not entitled to antedate such disclosure.

### Definitions

In the following, definitions will be provided which apply to all aspects of the present disclosure. The following terms have the following meanings unless otherwise indicated. Any undefined terms have their art recognized meanings.

The term "about" means approximately or nearly, and in the context of a numerical value or range set forth herein in one embodiment means ± 20%, ± 10%, ± 5%, or ± 3% of the numerical value or range recited or claimed.

The terms "a" and "an" and "the" and similar reference used in the context of describing the present disclosure (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it was individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the disclosure and does not pose a limitation on the scope of the claims. No language in the specification should be construed as indicating any non-claimed element essential to the practice of the disclosure.

Unless expressly specified otherwise, the term "comprising" is used in the context of the present document to indicate that further members may optionally be present in addition to the members of the list introduced by "comprising". It is, however, contemplated as a specific embodiment of the present disclosure that the term "comprising" encompasses the possibility of no further members being present, i.e., for the purpose of this embodiment "comprising" is to be understood as having the meaning of "consisting of" or "consisting essentially of". Terms such as "reduce", "decrease", "inhibit" or "impair" as used herein relate to an overall reduction or the ability to cause an overall reduction, preferably of at least 5%, at least 10%, at least 20%, at least 50%, at least 75% or even more, in the level. These terms include a complete or essentially complete inhibition, i.e., a reduction to zero or essentially to zero. Terms such as "increase", "enhance" or "exceed" preferably relate to an increase or enhancement by at least 10%, at least 20%, at least 30%, at least 40%, at least 50%, at least 80%, at least 100%, at least 200%, at least 500%, or even more.

According to the present disclosure, the term "peptide" comprises oligo- and polypeptides and refers to substances which comprise about two or more, about 3 or more, about 4 or more, about 6 or more, about 8 or more, about 10 or more, about 13 or more, about 16 or more, about 20 or more, and up to about 50, about 100 or about 150, consecutive amino acids linked to one another via peptide bonds. The term "protein" or "polypeptide" refers to large peptides, in particular peptides having at least about 150 amino acids, but the terms "peptide", "protein" and "polypeptide" are used herein usually as synonyms.

A "therapeutic protein" has a positive or advantageous effect on a condition or disease state of a subject when provided to the subject in a therapeutically effective amount. In one embodiment, a therapeutic protein has curative or palliative properties and may be administered to ameliorate, relieve, alleviate, reverse, delay onset of or lessen the severity of one or more symptoms of a disease or disorder. A therapeutic protein may have prophylactic properties and may be used to delay the onset of a disease or to lessen the severity of such disease or pathological condition. The term "therapeutic protein" includes entire proteins or peptides, and can also refer to therapeutically active fragments thereof. It can also include therapeutically active variants of a protein. Examples of therapeutically active proteins include, but are not limited to, antigens for vaccination and immunostimulants such as cytokines.

"Fragment", with reference to an amino acid sequence (peptide or protein), relates to a part of an amino acid sequence, i.e. a sequence which represents the amino acid sequence shortened at the N-terminus and/or C-terminus. A fragment shortened at the C-terminus (N-terminal fragment) is obtainable e.g. by translation of a truncated open reading frame that lacks the 3'-end of the open reading frame. A fragment shortened at the N-terminus (C-terminal fragment) is obtainable e.g. by translation of a truncated open reading frame that lacks the 5'-end of the open reading frame, as long as the truncated open reading frame comprises a start codon that serves to initiate translation. A fragment of an amino acid sequence comprises e.g. at least 50 %, at least 60 %, at least 70 %, at least 80%, at least 90% of the amino acid residues from an amino acid sequence. A fragment of an amino acid sequence preferably comprises at least 6, in particular at least 8, at least 12, at least 15, at least 20, at least 30, at least 50, or at least 100 consecutive amino acids from an amino acid sequence.

By "variant" herein is meant an amino acid sequence that differs from a parent amino acid sequence by virtue of at least one amino acid modification. The parent amino acid sequence may be a naturally occurring or wild type (WT) amino acid sequence, or may be a modified version of a wild type amino acid sequence. Preferably, the variant amino acid sequence has at least one amino acid modification compared to the parent amino acid sequence, e.g., from 1 to about 20 amino acid modifications, and preferably from 1 to about 10 or from 1 to about 5 amino acid modifications compared to the parent.

By "wild type" or "WT" or "native" herein is meant an amino acid sequence that is found in nature, including allelic variations. A wild type amino acid sequence, peptide or protein has an amino acid sequence that has not been intentionally modified.

In some embodiments, the present disclosure refers to a SARS-CoV-2 variant that is prevalent and/or rapidly spreading in a relevant jurisdiction. In some embodiments, such variants may be identified based on publicly available data (e.g., data provided in the GISAID Initiative database: https://www.gisaid.org, and/or data provided by the World Health Organization WHO (e.g., as provided at https://www.who.int/activities/tracking-SARS-CoV-2-variants). In some embodiments, such a variant refers to a variant disclosed herein.

For the purposes of the present disclosure, "variants" of an amino acid sequence (peptide, protein or polypeptide) comprise amino acid insertion variants, amino acid addition variants, amino acid deletion variants and/or amino acid substitution variants. The term "variant" includes all mutants, splice variants, posttranslationally modified variants, conformations, isoforms, allelic variants, species variants, and species homologs, in particular those which are naturally occurring. The term "variant" includes, in particular, fragments of an amino acid sequence.

Amino acid insertion variants comprise insertions of single or two or more amino acids in a particular amino acid sequence. In the case of amino acid sequence variants having an insertion, one or more amino acid residues are inserted into a particular site in an amino acid sequence, although random insertion with appropriate screening of the resulting product is also possible. Amino acid addition variants comprise amino- and/or carboxy-terminal fusions of one or more amino acids, such as 1, 2, 3, 5, 10, 20, 30, 50, or more amino acids. Amino acid deletion variants are characterized by the removal of one or more amino acids from the sequence, such as by removal of 1, 2, 3, 5, 10, 20, 30, 50, or more amino acids. The deletions may be in any position of the protein. Amino acid deletion variants that comprise the deletion at the N-terminal and/or C-terminal end of the protein are also called N-terminal and/or C-terminal truncation variants. Amino acid substitution variants are characterized by at least one residue in the sequence being removed and another residue being inserted in its place. Preference is given to the modifications being in positions in the amino acid sequence which are not conserved between homologous proteins or peptides and/or to replacing amino acids with other ones having similar properties. Preferably, amino acid changes in peptide and protein variants are conservative amino acid changes, i.e., substitutions of similarly charged or uncharged amino acids. A conservative amino acid change involves substitution of one of a family of amino acids which are related in their side chains. Naturally occurring amino acids are generally divided into four families: acidic (aspartate, glutamate), basic (lysine, arginine, histidine), non-polar (alanine, valine, leucine, isoleucine, proline, phenylalanine, methionine, tryptophan), and uncharged polar (glycine, asparagine, glutamine, cysteine, serine, threonine, tyrosine) amino acids. Phenylalanine, tryptophan, and tyrosine are sometimes classified jointly as aromatic amino acids. In one embodiment, conservative amino acid substitutions include substitutions within the following groups:
glycine, alanine;
valine, isoleucine, leucine;
aspartic acid, glutamic acid;
asparagine, glutamine;
serine, threonine;
lysine, arginine; and
phenylalanine, tyrosine.

Preferably the degree of similarity, preferably identity between a given amino acid sequence and an amino acid sequence which is a variant of said given amino acid sequence will be at least about 60%, 70%, 80%, 81%, 82%, 83%, 84%, 85%, 86%, 87%, 88%, 89%, 90%, 91%, 92%, 93%, 94%, 95%, 96%, 97%, 98%, or 99%. The degree of similarity or identity is given preferably for an amino acid region which is at least about 10%, at least about 20%, at least about 30%, at least about 40%, at least about 50%, at least about 60%, at least about 70%, at least about 80%, at least about 90% or about 100% of the entire length of the reference amino acid sequence. For example, if the reference amino acid sequence consists of 200 amino acids, the degree of similarity or identity is given preferably for at least about 20, at least about 40, at least about 60, at least about 80, at least about 100, at least about 120, at least about 140, at least about 160, at least about 180, or about 200 amino acids, in some embodiments continuous amino acids. In some embodiments, the degree of similarity or identity is given for the entire length of the reference amino acid sequence. The alignment for determining sequence similarity, preferably sequence identity can be done with art known tools, preferably using the best sequence alignment, for example, using Align, using standard settings, preferably EMBOSS::needle, Matrix: Blosum62, Gap Open 10.0, Gap Extend 0.5.

"Sequence similarity" indicates the percentage of amino acids that either are identical or that represent conservative amino acid substitutions. "Sequence identity" between two amino acid sequences indicates the percentage of amino acids that are identical between the sequences. "Sequence identity" between two nucleic acid sequences indicates the percentage of nucleotides that are identical between the sequences.

The terms "% identical", "% identity" or similar terms are intended to refer, in particular, to the percentage of nucleotides or amino acids which are identical in an optimal alignment between the sequences to be compared. Said percentage is purely statistical, and the differences between the two sequences may be but are not necessarily randomly distributed over the entire length of the sequences to be compared. Comparisons of two sequences are usually carried out by comparing the sequences, after optimal alignment, with respect to a segment or "window of comparison", in order to identify local regions of corresponding sequences. The optimal alignment for a comparison may be carried out manually or with the aid of the local homology algorithm by Smith and Waterman, 1981, Ads App. Math. 2, 482, with the aid of the local homology algorithm by Neddleman and Wunsch, 1970, J. Mol. Biol. 48, 443, with the aid of the similarity search algorithm by Pearson and Lipman, 1988, Proc. Natl Acad. Sci. USA 88, 2444, or with the aid of computer programs using said algorithms (GAP, BESTFIT, FASTA, BLAST P, BLAST N and TFASTA in Wisconsin Genetics Software Package, Genetics Computer Group, 575 Science Drive, Madison, Wis.). In some embodiments, percent identity of two sequences is determined using the BLASTN or BLASTP algorithm, as available on the United States National Center for Biotechnology Information (NCBI) website (e.g., at blast.ncbi.nlm.nih.gov/Blast.cgi?PAGE_TYPE=BlastSearch&BLAST_SPEC=blast2seq&LINK_LOC =align2seq). In some embodiments, the algorithm parameters used for BLASTN algorithm on the NCBI website include: (i) Expect Threshold set to 10; (ii) Word Size set to 28; (iii) Max matches in a query range set to 0; (iv) Match/Mismatch Scores set to 1, -2; (v) Gap Costs set to Linear; and (vi) the filter for low complexity regions being used. In some embodiments, the algorithm parameters used for BLASTP algorithm on the NCBI website include: (i) Expect Threshold set to 10; (ii) Word Size set to 3; (iii) Max matches in a query range set to 0; (iv) Matrix set to BLOSUM62; (v) Gap Costs set to Existence: 11 Extension: 1; and (vi) conditional compositional score matrix adjustment.

Percentage identity is obtained by determining the number of identical positions at which the sequences to be compared correspond, dividing this number by the number of positions compared (e.g., the number of positions in the reference sequence) and multiplying this result by 100.

In some embodiments, the degree of similarity or identity is given for a region which is at least about 50%, at least about 60%, at least about 70%, at least about 80%, at least about 90% or about 100% of the entire length of the reference sequence. For example, if the reference nucleic acid sequence consists of 200 nucleotides, the degree of identity is given for at least about 100, at least about 120, at least about 140, at least about 160, at least about 180, or about 200 nucleotides, in some embodiments continuous nucleotides. In some embodiments, the degree of similarity or identity is given for the entire length of the reference sequence. Homologous amino acid sequences exhibit according to the present disclosure at least 40%, in particular at least 50%, at least 60%, at least 70%, at least 80%, at least 90% and preferably at least 95%, at least 98 or at least 99% identity of the amino acid residues.

The amino acid sequence variants described herein may readily be prepared by the skilled person, for example, by recombinant DNA manipulation. The manipulation of DNA sequences for preparing peptides or proteins having substitutions, additions, insertions or deletions, is described in detail in Sambrook et al. (1989), for example. Furthermore, the peptides and amino acid variants described herein may be readily prepared with the aid of known peptide synthesis techniques such as, for example, by solid phase synthesis and similar methods.

In one embodiment, a fragment or variant of an amino acid sequence (peptide or protein) is preferably a "functional fragment" or "functional variant". The term "functional fragment" or "functional variant" of an amino acid sequence relates to any fragment or variant exhibiting one or more functional properties identical or similar to those of the amino acid sequence from which it is derived, i.e., it is functionally equivalent. With respect to antigens or antigenic sequences, one particular function is one or more immunogenic activities displayed by the amino acid sequence from which the fragment or variant is derived. The term "functional fragment" or "functional variant", as used herein, in particular refers to a variant molecule or sequence that comprises an amino acid sequence that is altered by one or more amino acids compared to the amino acid sequence of the parent molecule or sequence and that is still capable of fulfilling one or more of the functions of the parent molecule or sequence, e.g., inducing an immune response. In one embodiment, the modifications in the amino acid sequence of the parent molecule or sequence do not significantly affect or alter the characteristics of the molecule or sequence. In different embodiments, the function of the functional fragment or functional variant may be reduced but still significantly present, e.g., immunogenicity of the functional variant may be at least 50%, at least 60%, at least 70%, at least 80%, or at least 90% of the parent molecule or sequence. However, in other embodiments, immunogenicity of the functional fragment or functional variant may be enhanced compared to the parent molecule or sequence.

An amino acid sequence (peptide, protein or polypeptide) "derived from" a designated amino acid sequence (peptide, protein or polypeptide) refers to the origin of the first amino acid sequence. Preferably, the amino acid sequence which is derived from a particular amino acid sequence has an amino acid sequence that is identical, essentially identical or homologous to that particular sequence or a fragment thereof. Amino acid sequences derived from a particular amino acid sequence may be variants of that particular sequence or a fragment thereof. For example, it will be understood by one of ordinary skill in the art that the antigens suitable for use herein may be altered such that they vary in sequence from the naturally occurring or native sequences from which they were derived, while retaining the desirable activity of the native sequences.

As used herein, an "instructional material" or "instructions" includes a publication, a recording, a diagram, or any other medium of expression which can be used to communicate the usefulness of the compositions and methods of the present disclosure. The instructional material of the kit of the present disclosure may, for example, be affixed to a container which contains the compositions of the present disclosure or be shipped together with a container which contains the compositions. Alternatively, the instructional material may be shipped separately from the container with the intention that the instructional material and the compositions be used cooperatively by the recipient.

"Isolated" means altered or removed from the natural state. For example, a nucleic acid or a peptide naturally present in a living animal is not "isolated", but the same nucleic acid or peptide partially or completely separated from the coexisting materials of its natural state is "isolated". An isolated nucleic acid or protein can exist in substantially purified form, or can exist in a non-native environment such as, for example, a host cell.

The term "recombinant" in the context of the present disclosure means "made through genetic engineering". Preferably, a "recombinant object" such as a recombinant nucleic acid in the context of the present disclosure is not occurring naturally.

The term "naturally occurring" as used herein refers to the fact that an object can be found in nature. For example, a peptide or nucleic acid that is present in an organism (including viruses) and can be isolated from a source in nature and which has not been intentionally modified by man in the laboratory is naturally occurring.

"Physiological pH" as used herein refers to a pH of about 7.5.

The term "genetic modification" or simply "modification" includes the transfection of cells with nucleic acid. The term "transfection" relates to the introduction of nucleic acids, in particular RNA, into a cell. For purposes of the present disclosure, the term "transfection" also includes the introduction of a nucleic acid into a cell or the uptake of a nucleic acid by such cell, wherein the cell may be present in a subject, e.g., a patient. Thus, according to the present disclosure, a cell for transfection of a nucleic acid described herein can be present *in vitro* or *in vivo,* e.g. the cell can form part of an organ, a tissue and/or an organism of a patient. According to the disclosure, transfection can be transient or stable. For some applications of transfection, it is sufficient if the transfected genetic material is only transiently expressed. RNA can be transfected into cells to transiently express its coded protein. Since the nucleic acid introduced in the transfection process is usually not integrated into the nuclear genome, the foreign nucleic acid will be diluted through mitosis or degraded. Cells allowing episomal amplification of nucleic acids greatly reduce the rate of dilution. If it is desired that the transfected nucleic acid actually remains in the genome of the cell and its daughter cells, a stable transfection must occur. Such stable transfection can be achieved by using virus-based systems or transposon-based systems for transfection. Generally, nucleic acid encoding antigen is transiently transfected into cells. RNA can be transfected into cells to transiently express its coded protein.

The term "seroconversion" includes a ≥4-fold rise from before vaccination to 1-month post Dose 2.

### Coronavirus

Coronaviruses are enveloped, positive-sense, single-stranded RNA ((+) ssRNA) viruses. They have the largest genomes (26-32 kb) among known RNA viruses and are phylogenetically divided into four genera (α, β, γ, and δ), with betacoronaviruses further subdivided into four lineages (A, B, C, and D). Coronaviruses infect a wide range of avian and mammalian species, including humans. Some human coronaviruses generally cause mild respiratory diseases, although severity can be greater in infants, the elderly, and the immunocompromised. Middle East respiratory syndrome coronavirus (MERS-CoV) and severe acute respiratory syndrome coronavirus (SARS-CoV), belonging to betacoronavirus lineages C and B, respectively, are highly pathogenic. Both viruses emerged into the human population from animal reservoirs within the last 15 years and caused outbreaks with high case-fatality rates. The outbreak of severe acute respiratory syndrome coronavirus-2 (SARS-CoV-2) that causes atypical pneumonia (coronavirus disease 2019; COVID-19) has raged in China since mid-December 2019, and has developed to be a public health emergency of international concern. SARS-CoV-2 (MN908947.3) belongs to betacoronavirus lineage B. It has at least 70% sequence similarity to SARS-CoV.

In general, coronaviruses have four structural proteins, namely, envelope (E), membrane (M), nucleocapsid (N), and spike (S). The E and M proteins have important functions in the viral assembly, and the N protein is necessary for viral RNA synthesis. The critical glycoprotein S is responsible for virus binding and entry into target cells. The S protein is synthesized as a single-chain inactive precursor that is cleaved by furin-like host proteases in the producing cell into two noncovalently associated subunits, S1 and S2. The S1 subunit contains the receptor-binding domain (RBD), which recognizes the host-cell receptor. The S2 subunit contains the fusion peptide, two heptad repeats, and a transmembrane domain, all of which are required to mediate fusion of the viral and host-cell membranes by undergoing a large conformational rearrangement. The S1 and S2 subunits trimerize to form a large prefusion spike.

The S precursor protein of SARS-CoV-2 can be proteolytically cleaved into S1 (685 aa) and S2 (588 aa) subunits. The S1 subunit comprises the receptor-binding domain (RBD), which mediates virus entry into sensitive cells through the host angiotensin-converting enzyme 2 (ACE2) receptor.

### Antigen

The present disclosure comprises the use of RNA encoding an amino acid sequence comprising SARS-CoV-2 S protein, an immunogenic variant thereof, or an immunogenic fragment of the SARS-CoV-2 S protein or the immunogenic variant thereof. Thus, the RNA encodes a peptide or protein comprising at least an epitope SARS-CoV-2 S protein or an immunogenic variant thereof for inducing an immune response against coronavirus S protein, in particular SARS-CoV-2 S protein in a subject. The amino acid sequence comprising SARS-CoV-2 S protein, an immunogenic variant thereof, or an immunogenic fragment of the SARS-CoV-2 S protein or the immunogenic variant thereof (i.e., the antigenic peptide or protein) is also designated herein as "vaccine antigen", "peptide and protein antigen", "antigen molecule" or simply "antigen". The SARS-CoV-2 S protein, an immunogenic variant thereof, or an immunogenic fragment of the SARS-CoV-2 S protein or the immunogenic variant thereof is also designated herein as "antigenic peptide or protein" or "antigenic sequence".

The SARS-CoV-2 coronavirus full length spike (S) protein from the first detected SARS-CoV-2 strain (referred to as the Wuhan strain herein) consists of 1273 amino acids and has the amino acid sequence according to SEQ ID NO: 1:



















(SEQ ID NO: 1)

For purposes of the present disclosure, the above sequence is considered the wildtype or Wuhan SARS-CoV-2 S protein amino acid sequence. Unless otherwise indicated, position numberings in a SARS-CoV-2 S protein given herein are in relation to the amino acid sequence according to SEQ ID NO: 1. One of skill in the art reading the present disclosure will understand the corresponding positions in SARS-CoV-2 S protein variants based on the position numbering relative to the amino acid sequence of SEQ ID NO: 1.

In specific embodiments, full length spike (S) protein encoded by an RNA described herein can be modified in such a way that the prototypical prefusion conformation is stabilized. Certain mutations that stabilize a prefusion confirmation are known in the art, e.g., as disclosed in WO 2021243122 A2 and Hsieh, Ching-Lin, et al. ("Structure-based design of prefusion-stabilized SARS-CoV-2 spikes," Science 369.6510 (2020): 1501-1505), the contents of each which are incorporated by reference herein in their entirety. In some embodiments, a SARS-CoV-2 S protein may be stabilized by introducing one or more proline mutations. In some embodiments, a SARS-CoV-2 S protein comprises a proline substitution at positions corresponding to residues 986 and/or 987 of SEQ ID NO: 1. In some embodiments, a SARS-CoV-2 S protein comprises a proline substitution at one or more positions corresponding to residues 817, 892, 899, and 942 of SEQ ID NO: 1. In some embodiments, a SARS-CoV-2 S protein comprises a proline substitution at positions corresponding to each of residues 817, 892, 899, and 942 of SEQ ID NO: 1. In some embodiments, a SARS-CoV-2 S protein comprises a proline substitution at positions corresponding to each of residues 817, 892, 899, 942, 986, and 987 of SEQ ID NO: 1.

In some embodiments, stabilization of the prefusion conformation may be obtained by introducing two consecutive proline substitutions at residues 986 and 987 in the full length spike protein. Specifically, spike (S) protein stabilized protein variants are obtained in a way that the amino acid residue at position 986 is exchanged to proline and the amino acid residue at position 987 is also exchanged to proline. In one embodiment, a SARS-CoV-2 S protein variant wherein the prototypical prefusion conformation is stabilized comprises the amino acid sequence shown in SEQ ID NO: 7:

MFVFLVLLPLVSSQCVNLTTRTQLPPAYTNSFTRGVYYPDKVFRSSVLHSTQDLFLPFFSNVTWFHAIHVSGTNG
TKRFDNPVLPFNDGVYFASTEKSNIIRGWIFGTTLDSKTQSLLIVNNATNVVIKVCEFQFCNDPFLGVYYHKNNK
SWMESEFRVYSSANNCTFEYVSQPFLMDLEGKQGNFKNLREFVFKNIDGYFKIYSKHTPINLVRDLPQGFSALEP
LVDLPIGINITRFQTLLALHRSYLTPGDSSSGWTAGAAAYYVGYLQPRTFLLKYNENGTITDAVDCALDPLSETK
CTLKSFTVEKGIYQTSNFRVQPTESIVRFPNITNLCPFGEVFNATRFASVYAWNRKRISNCVADYSVLYNSASFS
TFKCYGVSPTKLNDLCFTNVYADSFVIRGDEVRQIAPGQTGKIADYNYKLPDDFTGCVIAWNSNNLDSKVGGNYN
YLYRLFRKSNLKPFERDISTEIYQAGSTPCNGVEGFNCYFPLQSYGFQPTNGVGYQPYRVVVLSFELLHAPATVC
GPKKSTNLVKNKCVNFNFNGLTGTGVLTESNKKFLPFQQFGRDIADTTDAVRDPQTLEILDITPCSFGGVSVITP
GTNTSNQVAVLYQDVNCTEVPVAIHADQLTPTWRVYSTGSNVFQTRAGCLIGAEHVNNSYECDIPIGAGICASYQ
TQTNSPRRARSVASQSIIAYTMSLGAENSVAYSNNSIAIPTNFTISVTTEILPVSMTKTSVDCTMYICGDSTECS
NLLLQYGSFCTQLNRALTGIAVEQDKNTQEVFAQVKQIYKTPPIKDFGGFNFSQILPDPSKPSKRSFIEDLLFNK
VTLADAGFIKQYGDCLGDIAARDLICAQKFNGLTVLPPLLTDEMIAQYTSALLAGTITSGWTFGAGAALQIPFAM
QMAYRFNGIGVTQNVLYENQKLIANQFNSAIGKIQDSLSSTASALGKLQDVVNQNAQALNTLVKQLSSNFGAISS
VLNDILSRLDPPEAEVQIDRLITGRLQSLQTYVTQQLIRAAEIRASANLAATKMSECVLGQSKRVDFCGKGYHLM
SFPQSAPHGVVFLHVTYVPAQEKNFTTAPAICHDGKAHFPREGVFVSNGTHWFVTQRNFYEPQIITTDNTFVSGN
CDVVIGIVNNTVYDPLQPELDSFKEELDKYFKNHTSPDVDLGDISGINASVVNIQKEIDRLNEVAKNLNESLIDL
QELGKYEQYIKWPWYIWLGFIAGLIAIVMVTIMLCCMTSCCSCLKGCCSCGSCCKFDEDDSEPVLKGVKLHYT
(SEQ ID NO: 7)

Those skilled in the art are aware of various spike variants, and/or resources that document them. For example, the following strains, their SARS-CoV-2 S protein amino acid sequences and, in particular, modifications thereof compared to wildtype SARS-CoV-2 S protein amino acid sequence, e.g., as compared to SEQ ID NO: 1, are useful herein.

### B.1.1.7 ("Variant of Concern 202012/01" (VOC-202012/01)

B.1.1.7 (the "alpha variant") is a variant of SARS-CoV-2 which was first detected in October 2020 during the COVID-19 pandemic in the United Kingdom from a sample taken the previous month, and it quickly began to spread by mid-December. It is correlated with a significant increase in the rate of COVID-19 infection in United Kingdom; this increase is thought to be at least partly because of change N501Y inside the spike glycoprotein's receptor-binding domain, which is needed for binding to ACE2 in human cells. The B.1.1.7 variant is defined by 23 mutations: 13 non-synonymous mutations, 4 deletions, and 6 synonymous mutations (i.e., there are 17 mutations that change proteins and six that do not). The spike protein changes in B.1.1.7 include deletion 69-70, deletion 144, N501Y, A570D, D614G, P681H, T716I, S982A, and D1118H.

### B.1.351 (501.V2)

B.1.351 lineage (the "Beta variant") and colloquially known as South African COVID-19 variant, is a variant of SARS-CoV-2. Preliminary results indicate that this variant may have an increased transmissibility. The B.1.351 variant is defined by multiple spike protein changes including: L18F, D80A, D215G, deletion 242-244, R246I, K417N, E484K, N501Y, D614G and A701V. There are three mutations of particular interest in the spike region of the B.1.351 genome: K417N, E484K, N501Y.

### B.1.1.298 (Cluster 5)

B.1.1.298 was discovered in North Jutland, Denmark, and is believed to have been spread from minks to humans via mink farms. Several different mutations in the spike protein of the virus have been confirmed. The specific mutations include deletion 69-70, Y453F, D614G, I692V, M1229I, and optionally S1147L.

### P.1 (B.1.1.248)

Lineage B.1.1.248 (the "gamma variant"), known as the Brazil(ian) variant, is one of the variants of SARS-CoV-2 which has been named P.1 lineage. P.1 has a number of S-protein modifications [L18F, T20N, P26S, D138Y, R190S, K417T, E484K, N501Y, D614G, H655Y, T1027I, V1176F] and is similar in certain key RBD positions (K417, E484, N501) to variant B.1.351 from South Africa.

### B.1.427/B.1.429 (CAL.20C)

Lineage B.1.427/B.1.429 (the "epsilon variant"), also known as CAL.20C, is defined by the following modifications in the S-protein: S13I, W152C, L452R, and D614G of which the L452R modification is of particular concern. CDC has listed B.1.427/B.1.429 as "variant of concern".

### B.1.525

B.1.525 (the "eta variant") carries the same E484K modification as found in the P.1, and B.1.351 variants, and also carries the same ΔH69/ΔV70 deletion as found in B.1.1.7, and B.1.1.298. It also carries the modifications D614G, Q677H and F888L.

### B.1.526

B.1.526 (the "iota variant") was detected as an emerging lineage of viral isolates in the New York region that shares mutations with previously reported variants. The most common sets of spike mutations in this lineage are L5F, T95I, D253G, E484K, D614G, and A701V.

The following table shows an overview of circulating SARS-CoV-2 strains which are VOI/VOC.

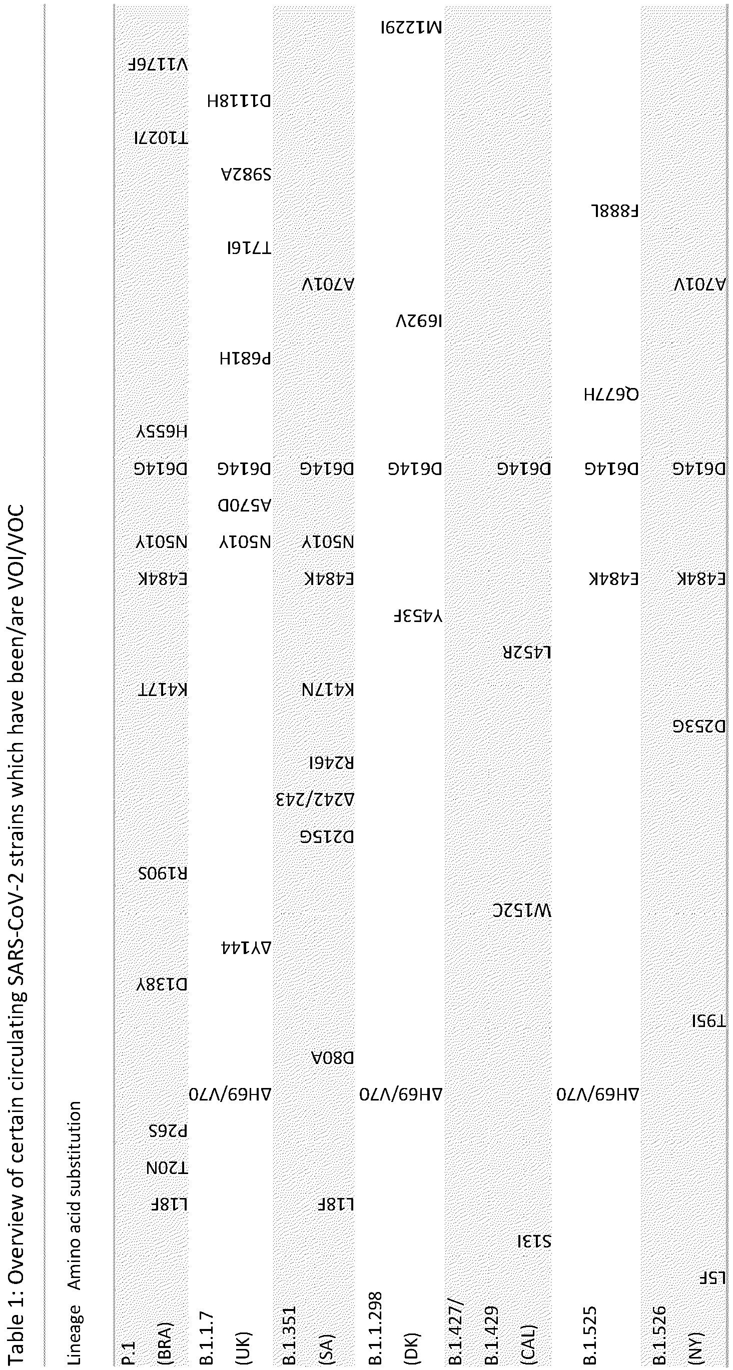

### B.1.1.529

B.1.529 ("Omicron variant") was first detected in South Africa in November 2021. Omicron multiplies around 70 times fasterthan Delta variants, and quickly became the dominant strain of SARS-CoV-2 worldwide. Since its initial detection, a number of Omicron sublineages have arisen. Listed below are the current Omicron variants of concern, along with certain characteristic mutations associated with the S protein of each. The S protein of BA.4 and BA.5 have the same set of characteristic mutations, which is why the below table has a single row for "BA.4 or BA.5", and why the present disclosure refers to a "BA.4/5" S protein in some embodiments. Similarly, the S proteins of the BA.4.6 and BF.7 Omicron variants have the same set of characteristic mutations, which is why the below table has a single row for "BA.4.6 or BF.7").

**Table 2: Omicron Variants of Concern and Characteristic mutations**

| Subvariant | Common mutations |
|---|---|
| BA.1 | A67V, Δ69-70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F |
| BA.2 | T19I, Δ24-26, A27S, G142D, V213G, G339D, S371F, S373P, S375F, T376A, D405N, R408S, K417N, N440K, S477N, T478K, E484A, Q493R, Q498R, N501Y, Y505H, D614G, H655Y, N679K, P681H, N764K, D796Y, Q954H, N969K |
| BA.2.12.1 | T19I, Δ24-26, A27S, G142D, V213G, G339D, S371F, S373P, S375F, T376A, D405N, R408S, K417N, N440K, L452Q, S477N, T478K, E484A, Q493R, Q498R, N501Y, Y505H, D614G, H655Y, N679K, P681H, S704L, N764K, D796Y, Q954H, N969K |
| BA.4 or BA.5 | T19I, Δ24-26, A27S, Δ69/70, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, N969K |
| BA.2.75 | T19I, Δ24-26, A27S, G142D, K147E, W152R, F157L, I210V, V213G, G257S, G339H, N354D, S371F, S373P, S375F, T376A, D405N, R408S, K417N, N440K, G446S, N460K, S477N, T478K, E484A, Q498R, N501Y, Y505H D614G, H655Y, N679K, P681H, N764K, D796Y, Q954H, and N969K |
| BA.2.75.2 | T19I, Δ24-26, A27S, G142D, K147E, W152R, F157L, I210V, V213G, G257S, G339H, R346T, N354D, S371F, S373P, S375F, T376A, D405N, R408S, K417N, N440K, G446S, N460K, S477N, T478K, E484A, F486S, Q498R, N501Y, Y505H, D614G, H655Y, N679K, P681H, N764K, D796Y, Q954H, N969K, and D1199N |
| BJ. 1 | T19I, Δ24-26, A27S, V83A, G142D, Δ144, H146Q, Q183E, V213E, G339H, R346T, L368I, S371F, S373P, S375F, T376A, D405N, R408S, K417N, N440K, V445P, G446S, S477N, T478K, V483A, E484A, F490V, Q493R, Q498R, N501Y, Y505H, D614G, H655Y, N679K, P681H, N764K, D796Y, G798D, Q954H, N969K, and S1003I |
| BA.4.6 or BF.7 | T19I, Δ24-26, A27S, Δ69/70, G142D, V213G, G339D, R346T, S371F, S373P, S375F, T376A, D405N, R408S, K417N, N440K, L452R, S477N, T478K, E484A, F486V, Q498R, N501Y, Y505H, D614G, H655Y, N658S, N679K, P681H, N764K, D796Y, Q954H, and N969K |
| XBB | T19I, Δ24-26, A27S, V83A, G142D, Δ144, H146Q, Q183E, V213E, G339H, R346T, L368I, S371F, S373P, S375F, T376A, D405N, R408S, K417N, N440K, V445P, G446S, N460K, S477N, T478K, E484A, F486S, F490S, Q493R, Q498R, N501Y, Y505H, D614G, H655Y, N679K, P681H, N764K, D796Y, Q954H, and N969K |
| XBB.1 | T19I, Δ24-26, A27S, V83A, G142D, Δ144, H146Q, Q183E, V213E, G252V, G339H, R346T, L368I, S371F, S373P, S375F, T376A, D405N, R408S, K417N, N440K, V445P, G446S, N460K, S477N, T478K, E484A, F486S, F490S, Q493R, Q498R, N501Y, Y505H, D614G, H655Y, N679K, P681H, N764K, D796Y, Q954H, and N969K |
| XBB.2 | T19I, Δ24-26, A27S, V83A, G142D, Δ144, H146Q, Q183E, V213E, D253G, G339H, R346T, L368I, S371F, S373P, S375F, T376A, D405N, R408S, K417N, N440K, V445P, G446S, N460K, S477N, T478K, E484A, F486S, F490S, Q493R, Q498R, N501Y, Y505H, D614G, H655Y, N679K, P681H, N764K, D796Y, Q954H, and N969K |
| XBB.1.3 | T19I, Δ24-26, A27S, V83A, G142D, Δ144, H146Q, Q183E, V213E, G252V, G339H, R346T, L368I, S371F, S373P, S375F, T376A, D405N, R408S, K417N, N440K, V445P, G446S, N460K, S477N, T478K, A484T, F486S, F490S, Q493R, Q498R, N501Y, Y505H, D614G, H655Y, N679K, P681H, N764K, D796Y, Q954H, and N969K |
| BA.2.3.20 | T19I, Δ24-26, A27S, G142D, M153T, N164K, V213G, H245N, G257D, G339D, S371F, S373P, S375F, T376A, D405N, R408S, K417N, N440K, K444R, E484R N450D, L452M, N460K, S477N, T478K, E484A, Q493R, Q498R, N501Y, Y505H, D614G, H655Y, N679K, P681H, N764K, D796Y, Q954H, and N969K |
| BQ.1.1 | T19I, Δ24-26, A27S, Δ69/70, G142D, V213G, G339D, R346T, S371F, S373P, S375F, T376A, D405N, R408S, K417N, N440K, K444T, L452R, N460K, S477N, T478K, E484A, F486V, Q498R, N501Y, Y505H, D614G, H655Y, N679K, P681H, N764K, D796Y, Q954H, N969K |

In some embodiments, RNA described herein comprises a nucleotide sequence encoding a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of an Omicron variant (e.g., one or more mutations of an Omicron variant listed in Table 2). In some embodiments, such RNA further comprises one or more mutations that stabilize the S protein in a pre-fusion confirmation (e.g., in some embodiments, such RNA further comprises proline residues at positions corresponding to residues 986 and 987 of SEQ ID NO: 1). In some embodiments, an RNA comprises a nucleotide sequence encoding a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) listed in Table 2. In some such embodiments, one or more mutations may come from two or more variants as listed in Table 2. In some embodiments, an RNA comprises a nucleotide sequence encoding a SARS-CoV-2 S protein comprising each of the mutations identified in Table 2 as being characteristic of a certain Omicron variant (e.g., in some embodiments, an RNA comprises a nucleotide sequence encoding a SARS-CoV-2 S protein comprising each of the mutations listed in Table 2 as being characteristic of an Omicron BA.1, BA.2, BA.2.12.1, BA.4/5, BA.2.75, BA.2.75.1, BA.4.6, BQ.1.1, XBB, XBB.1, XBB.2, or XBB.1.3 variant).

In some embodiments, an RNA disclosed herein comprises a nucleotide sequence that encodes an immunogenic fragment of the SARS-Cov-2 S protein (e.g., the RBD) and which comprises one or more mutations that are characteristic of a SARS-CoV-2 variant (e.g., an Omicron variant described herein). For example, in some embodiments, an RNA comprises a nucleotide sequence encoding the RBD of an S protein of a SARS-CoV-2 variant (e.g., a region of the S protein corresponding to amino acids 327 to 528 of SEQ ID NO: 1, and comprising one or more mutations that are characteristic of a variant of concern that lie within this region of the S protein).

In some embodiments, an RNA encodes a SARS-CoV-2 S protein comprising a subset of the mutations listed in Table 2. In some embodiments, an RNA encodes a SARS-CoV-2 S protein comprising the mutations listed in Table 2 that are most prevalent in a certain variant (e.g., mutations that have been detected in at least 30%, at least 40%, at least 50%, at least 60%, at least 70%, at least 80%, at least 90%, at least 95%, at least 96%, at least 97%, at least 98%, at least 99%, or 100% of sequences collected to date for a given variant sequenced). Mutation prevalence can be determined, e.g., based on published sequences (e.g., sequences that are collected and made available to the public by GISAID).

In some embodiments, RNA described herein encodes a SARS-CoV-2 S protein comprising one or more mutations that are characteristic of a BA.4/5 variant. In some embodiments, the one or more mutations characteristic of a BA.4/5 variant include T19I, Δ24-26, A27S, Δ69/70, G142D, V213G, G339D, S371F, S373P, S375F, T376A, D405N, K417N, N440K, L452R, S477N, T478K, E484A, F486V, Q498R, N501Y, Y505H, D614G, H655Y, N679K, P681H, N764K, D796Y, Q954H, and N969K. In some embodiments, RNA described herein encodes a SARS-CoV-2 S protein comprising one or more mutations that are characteristic of a BA.4/5 variant and excludes R408S. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of a BA.4/5 variant and excludes R408S.

In some embodiments, RNA described herein encodes a SARS-CoV-2 S protein comprising one or more (including, e.g., 2, 3, 4, 5, 6, 7, 8, 9, 10, 11, 12, 13, 14, 15, 16, 17, 18, 19, 20, or more) mutations characteristic of a BA.2.75 variant. In some embodiments, the one or more mutations characteristic of a BA.2.75 variant include T19I, Δ24-26, A27S, G142D, K147E, W152R, F157L, I210V, V213G, G257S, G339H, S371F, S373P, S375F, T376A, D405N, R408S, K417N, N440K, G446S, N460K, S477N, T478K, E484A, Q498R, N501Y, Y505H D614G, H655Y, N679K, P681H, N764K, Q954H, and N969K. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of a BA.2.75 variant, and which excludes N354D. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of a BA.2.75 variant, and which excludes D796Y. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of a BA.2.75 variant, and which excludes D796Y and N354D.

In some embodiments, RNA described herein encodes a SARS-CoV-2 S protein comprising one or more mutations characteristic of a BA.2.75.2 variant. In some embodiments, the one or more mutations characteristic of a BA.2.75.2 variant include T19I, Δ24-26, A27S, G142D, K147E, W152R, F157L, I210V, V213G, G257S, G339H, R346T, N354D, S371F, S373P, S375F, T376A, D405N, R408S, K417N, N440K, G446S, N460K, S477N, T478K, E484A, F486S, Q498R, N501Y, Y505H, D614G, H655Y, N679K, P681H, N764K, D796Y, Q954H, N969K, and D1199N. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of a BA.2.75.2 variant, and which excludes R346T.

In some embodiments, RNA described herein encodes a SARS-CoV-2 S protein comprising one or more mutations characteristic of a BA.4.6 or BF.7 variant. In some embodiments, the one or more mutations characteristic of a BA.4.6 or BF.7 variant include T19I, Δ24-26, A27S, Δ69/70, G142D, V213G, G339D, R346T, S371F, S373P, S375F, T376A, D405N, K417N, N440K, L452R, S477N, T478K, E484A, F486V, Q498R, N501Y, Y505H, D614G, H655Y, N679K, P681H, N764K, D796Y, Q954H, and N969K. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of a BA.4.6 or BF.7 variant, and which exclude R408S. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of a BA.4.6 or BF.7 variant, and which exclude N658S. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of a BA.4.6 or BF.7 variant, and which exclude N658S and R408S.

In some embodiments, RNA described herein encodes a SARS-CoV-2 S protein comprising one or more mutations characteristic of an Omicron XBB variant. In some embodiments, the one or more mutations characteristic of an Omicron XBB variant include T19I, Δ24-26, A27S, V83A, G142D, Δ144, H146Q, Q183E, V213E, G339H, R346T, L368I, S371F, S373P, S375F, T376A, D405N, R408S, K417N, N440K, V445P, G446S, N460K, S477N, T478K, E484A, F486S, F490S, Q498R, N501Y, Y505H, D614G, H655Y, N679K, P681H, N764K, D796Y, Q954H, and N969K.

In some embodiments, RNA described herein encodes a SARS-CoV-2 S protein comprising one or more mutations characteristic of an Omicron XBB.1 variant. In some embodiments, the one or more mutations characteristic of an Omicron XBB.1 variant include G252V. In some embodiments, the one or more mutations characteristic of an Omicron XBB.1 variant include T19I, Δ24-26, A27S, V83A, G142D, Δ144, H146Q, Q183E, V213E, G252V, G339H, R346T, L368I, S371F, S373P, S375F, T376A, D405N, R408S, K417N, N440K, V445P, G446S, N460K, S477N, T478K, E484A, F486S, F490S, Q498R, N501Y, Y505H, D614G, H655Y, N679K, P681H, N764K, D796Y, Q954H, and N969K. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of an Omicron XBB.1 variant and which exclude Q493R. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of an Omicron XBB variant and which exclude Q493R and G252V.

In some embodiments, RNA described herein encodes a SARS-CoV-2 S protein comprising one or more mutations characteristic of an Omicron XBB.2 variant. In some embodiments, the one or more mutations characteristic of an Omicron XBB.2 variant include D253G. In some embodiments, the one or more mutations characteristic of an Omicron-XBB.2 variant include T19I, Δ24-26, A27S, V83A, G142D, Δ144, H146Q, Q183E, V213E, D253G, G339H, R346T, L368I, S371F, S373P, S375F, T376A, D405N, R408S, K417N, N440K, V445P, G446S, N460K, S477N, T478K, E484A, F486S, F490S, Q493R, Q498R, N501Y, Y505H, D614G, H655Y, N679K, P681H, N764K, D796Y, Q954H, and N969K.

In some embodiments, RNA described herein encodes a SARS-CoV-2 S protein comprising one or more mutations characteristic of an Omicron XBB.1.3 variant. In some embodiments, the one or more mutations characteristic of an Omicron XBB.1.3 variant include G252V and A484T. In some embodiments, the one or more mutations characteristic of an Omicron XBB.1.3 variant include T19I, Δ24-26, A27S, V83A, G142D, Δ144, H146Q, Q183E, V213E, G252V, G339H, R346T, L368I, S371F, S373P, S375F, T376A, D405N, R408S, K417N, N440K, V445P, G446S, N460K, S477N, T478K, A484T, F486S, F490S, Q493R, Q498R, N501Y, Y505H, D614G, H655Y, N679K, P681H, N764K, D796Y, Q954H, and N969K.

In some embodiments, RNA described herein encodes a SARS-CoV-2 S protein comprising one or more mutations that are characteristic of a BQ.1.1 variant. In some embodiments, the one or more mutations characteristic of a BQ.1.1 variant include T19I, Δ24-26, A27S, Δ69/70, G142D, V213G, G339D, S371F, S373P, S375F, T376A, D405N, R408S, K417N, N440K, K444T, L452R, N463K, S477N, T478K, E484A, F486V, Q498R, N501Y, Y505H, D614G, H655Y, N679K, P681H, N764K, D796Y, Q954H, and N969K. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of a BQ.1.1 variant.

In one embodiment, the vaccine antigen described herein comprises, consists essentially of or consists of a spike protein (S) of SARS-CoV-2, a variant thereof, or an immunogenic fragment thereof (e.g., but not limited to RBD).

In one embodiment, a vaccine antigen comprises the amino acid sequence of amino acids 17 to 1273 of SEQ ID NO: 1 or 7, an amino acid sequence having at least 99%, 98%, 97%, 96%, 95%, 90%, 85%, or 80% identity to the amino acid sequence of amino acids 17 to 1273 of SEQ ID NO: 1 or 7, or an immunogenic fragment of the amino acid sequence of amino acids 17 to 1273 of SEQ ID NO: 1 or 7, or the amino acid sequence having at least 99%, 98%, 97%, 96%, 95%, 90%, 85%, or 80% identity to the amino acid sequence of amino acids 17 to 1273 of SEQ ID NO: 1 or 7. In one embodiment, a vaccine antigen comprises the amino acid sequence of amino acids 17 to 1273 of SEQ ID NO: 1 or 7.

In one embodiment, RNA encoding a vaccine antigen (i) comprises the nucleotide sequence of nucleotides 49 to 3819 of SEQ ID NO: 2, 8 or 9, a nucleotide sequence having at least 99%, 98%, 97%, 96%, 95%, 90%, 85%, or 80% identity to the nucleotide sequence of nucleotides 49 to 3819 of SEQ ID NO: 2, 8 or 9, or a fragment of the nucleotide sequence of nucleotides 49 to 3819 of SEQ ID NO: 2, 8 or 9, or the nucleotide sequence having at least 99%, 98%, 97%, 96%, 95%, 90%, 85%, or 80% identity to the nucleotide sequence of nucleotides 49 to 3819 of SEQ ID NO: 2, 8 or 9; and/or (ii) encodes an amino acid sequence comprising the amino acid sequence of amino acids 17 to 1273 of SEQ ID NO: 1 or 7, an amino acid sequence having at least 99%, 98%, 97%, 96%, 95%, 90%, 85%, or 80% identity to the amino acid sequence of amino acids 17 to 1273 of SEQ ID NO: 1 or 7, or an immunogenic fragment of the amino acid sequence of amino acids 17 to 1273 of SEQ ID NO: 1 or 7, or the amino acid sequence having at least 99%, 98%, 97%, 96%, 95%, 90%, 85%, or 80% identity to the amino acid sequence of amino acids 17 to 1273 of SEQ ID NO: 1 or 7. In one embodiment, RNA encoding a vaccine antigen (i) comprises the nucleotide sequence of nucleotides 49 to 3819 of SEQ ID NO: 2, 8 or 9; and/or (ii) encodes an amino acid sequence comprising the amino acid sequence of amino acids 17 to 1273 of SEQ ID NO: 1 or 7.

In one embodiment, the vaccine antigen comprises, consists essentially of or consists of SARS-CoV-2 spike S1 fragment (S1) (the S1 subunit of a spike protein (S) of SARS-CoV-2), a variant thereof, or a fragment thereof.

In one embodiment, a vaccine antigen comprises the amino acid sequence of amino acids 17 to 683 of SEQ ID NO: 1, an amino acid sequence having at least 99%, 98%, 97%, 96%, 95%, 90%, 85%, or 80% identity to the amino acid sequence of amino acids 17 to 683 of SEQ ID NO: 1, or an immunogenic fragment of the amino acid sequence of amino acids 17 to 683 of SEQ ID NO: 1, or the amino acid sequence having at least 99%, 98%, 97%, 96%, 95%, 90%, 85%, or 80% identity to the amino acid sequence of amino acids 17 to 683 of SEQ ID NO: 1. In one embodiment, a vaccine antigen comprises the amino acid sequence of amino acids 17 to 683 of SEQ ID NO: 1.

In one embodiment, RNA encoding a vaccine antigen (i) comprises the nucleotide sequence of nucleotides 49 to 2049 of SEQ ID NO: 2, 8 or 9, a nucleotide sequence having at least 99%, 98%, 97%, 96%, 95%, 90%, 85%, or 80% identity to the nucleotide sequence of nucleotides 49 to 2049 of SEQ ID NO: 2, 8 or 9, or a fragment of the nucleotide sequence of nucleotides 49 to 2049 of SEQ ID NO: 2, 8 or 9, or the nucleotide sequence having at least 99%, 98%, 97%, 96%, 95%, 90%, 85%, or 80% identity to the nucleotide sequence of nucleotides 49 to 2049 of SEQ ID NO: 2, 8 or 9; and/or (ii) encodes an amino acid sequence comprising the amino acid sequence of amino acids 17 to 683 of SEQ ID NO: 1, an amino acid sequence having at least 99%, 98%, 97%, 96%, 95%, 90%, 85%, or 80% identity to the amino acid sequence of amino acids 17 to 683 of SEQ ID NO: 1, or an immunogenic fragment of the amino acid sequence of amino acids 17 to 683 of SEQ ID NO: 1, or the amino acid sequence having at least 99%, 98%, 97%, 96%, 95%, 90%, 85%, or 80% identity to the amino acid sequence of amino acids 17 to 683 of SEQ ID NO: 1. In one embodiment, RNA encoding a vaccine antigen (i) comprises the nucleotide sequence of nucleotides 49 to 2049 of SEQ ID NO: 2, 8 or 9; and/or (ii) encodes an amino acid sequence comprising the amino acid sequence of amino acids 17 to 683 of SEQ ID NO: 1.

In one embodiment, a vaccine antigen comprises the amino acid sequence of amino acids 17 to 685 of SEQ ID NO: 1, an amino acid sequence having at least 99%, 98%, 97%, 96%, 95%, 90%, 85%, or 80% identity to the amino acid sequence of amino acids 17 to 685 of SEQ ID NO: 1, or an immunogenic fragment of the amino acid sequence of amino acids 17 to 685 of SEQ ID NO: 1, or the amino acid sequence having at least 99%, 98%, 97%, 96%, 95%, 90%, 85%, or 80% identity to the amino acid sequence of amino acids 17 to 685 of SEQ ID NO: 1. In one embodiment, a vaccine antigen comprises the amino acid sequence of amino acids 17 to 685 of SEQ ID NO: 1.

In one embodiment, RNA encoding a vaccine antigen (i) comprises the nucleotide sequence of nucleotides 49 to 2055 of SEQ ID NO: 2, 8 or 9, a nucleotide sequence having at least 99%, 98%, 97%, 96%, 95%, 90%, 85%, or 80% identity to the nucleotide sequence of nucleotides 49 to 2055 of SEQ ID NO: 2, 8 or 9, or a fragment of the nucleotide sequence of nucleotides 49 to 2055 of SEQ ID NO: 2, 8 or 9, or the nucleotide sequence having at least 99%, 98%, 97%, 96%, 95%, 90%, 85%, or 80% identity to the nucleotide sequence of nucleotides 49 to 2055 of SEQ ID NO: 2, 8 or 9; and/or (ii) encodes an amino acid sequence comprising the amino acid sequence of amino acids 17 to 685 of SEQ ID NO: 1, an amino acid sequence having at least 99%, 98%, 97%, 96%, 95%, 90%, 85%, or 80% identity to the amino acid sequence of amino acids 17 to 685 of SEQ ID NO: 1, or an immunogenic fragment of the amino acid sequence of amino acids 17 to 685 of SEQ ID NO: 1, or the amino acid sequence having at least 99%, 98%, 97%, 96%, 95%, 90%, 85%, or 80% identity to the amino acid sequence of amino acids 17 to 685 of SEQ ID NO: 1. In one embodiment, RNA encoding a vaccine antigen (i) comprises the nucleotide sequence of nucleotides 49 to 2055 of SEQ ID NO: 2, 8 or 9; and/or (ii) encodes an amino acid sequence comprising the amino acid sequence of amino acids 17 to 685 of SEQ ID NO: 1.

In one embodiment, a vaccine antigen comprises, consists essentially of or consists of the receptor binding domain (RBD) of the S1 subunit of a spike protein (S) of SARS-CoV-2, a variant thereof, or a fragment thereof. The amino acid sequence of amino acids 327 to 528 of SEQ ID NO: 1, a variant thereof, or a fragment thereof is also referred to herein as "RBD" or "RBD domain".

In one embodiment, a vaccine antigen comprises the amino acid sequence of amino acids 327 to 528 of SEQ ID NO: 1, an amino acid sequence having at least 99%, 98%, 97%, 96%, 95%, 90%, 85%, or 80% identity to the amino acid sequence of amino acids 327 to 528 of SEQ ID NO: 1, or an immunogenic fragment of the amino acid sequence of amino acids 327 to 528 of SEQ ID NO: 1, or the amino acid sequence having at least 99%, 98%, 97%, 96%, 95%, 90%, 85%, or 80% identity to the amino acid sequence of amino acids 327 to 528 of SEQ ID NO: 1. In one embodiment, a vaccine antigen comprises the amino acid sequence of amino acids 327 to 528 of SEQ ID NO: 1.

In one embodiment, RNA encoding a vaccine antigen (i) comprises the nucleotide sequence of nucleotides 979 to 1584 of SEQ ID NO: 2, 8 or 9, a nucleotide sequence having at least 99%, 98%, 97%, 96%, 95%, 90%, 85%, or 80% identity to the nucleotide sequence of nucleotides 979 to 1584 of SEQ ID NO: 2, 8 or 9, or a fragment of the nucleotide sequence of nucleotides 979 to 1584 of SEQ ID NO: 2, 8 or 9, or the nucleotide sequence having at least 99%, 98%, 97%, 96%, 95%, 90%, 85%, or 80% identity to the nucleotide sequence of nucleotides 979 to 1584 of SEQ ID NO: 2, 8 or 9; and/or (ii) encodes an amino acid sequence comprising the amino acid sequence of amino acids 327 to 528 of SEQ ID NO: 1, an amino acid sequence having at least 99%, 98%, 97%, 96%, 95%, 90%, 85%, or 80% identity to the amino acid sequence of amino acids 327 to 528 of SEQ ID NO: 1, or an immunogenic fragment of the amino acid sequence of amino acids 327 to 528 of SEQ ID NO: 1, or the amino acid sequence having at least 99%, 98%, 97%, 96%, 95%, 90%, 85%, or 80% identity to the amino acid sequence of amino acids 327 to 528 of SEQ ID NO: 1. In one embodiment, RNA encoding a vaccine antigen (i) comprises the nucleotide sequence of nucleotides 979 to 1584 of SEQ ID NO: 2, 8 or 9; and/or (ii) encodes an amino acid sequence comprising the amino acid sequence of amino acids 327 to 528 of SEQ ID NO: 1.

According to certain embodiments, a signal peptide is fused, either directly or through a linker, to a SARS-CoV-2 S protein, a variant thereof, or a fragment thereof, i.e., the antigenic peptide or protein. Accordingly, in one embodiment, a signal peptide is fused to the above described amino acid sequences derived from SARS-CoV-2 S protein or immunogenic fragments thereof (antigenic peptides or proteins) comprised by the vaccine antigens described above.

Such signal peptides are sequences, which typically exhibit a length of about 15 to 30 amino acids and are preferably located at the N-terminus of the antigenic peptide or protein, without being limited thereto. Signal peptides as defined herein preferably allow the transport of the antigenic peptide or protein as encoded by the RNA into a defined cellular compartment, preferably the cell surface, the endoplasmic reticulum (ER) or the endosomal-lysosomal compartment. In one embodiment, the signal peptide sequence as defined herein includes, without being limited thereto, the signal peptide sequence of SARS-CoV-2 S protein, in particular a sequence comprising the amino acid sequence of amino acids 1 to 16 or 1 to 19 of SEQ ID NO: 1 or a functional variant thereof.

In one embodiment, a signal sequence comprises the amino acid sequence of amino acids 1 to 16 of SEQ ID NO: 1, an amino acid sequence having at least 99%, 98%, 97%, 96%, 95%, 90%, 85%, or 80% identity to the amino acid sequence of amino acids 1 to 16 of SEQ ID NO: 1, or a functional fragment of the amino acid sequence of amino acids 1 to 16 of SEQ ID NO: 1, or the amino acid sequence having at least 99%, 98%, 97%, 96%, 95%, 90%, 85%, or 80% identity to the amino acid sequence of amino acids 1 to 16 of SEQ ID NO: 1. In one embodiment, a signal sequence comprises the amino acid sequence of amino acids 1 to 16 of SEQ ID NO: 1.

In one embodiment, RNA encoding a signal sequence (i) comprises the nucleotide sequence of nucleotides 1 to 48 of SEQ ID NO: 2, 8 or 9, a nucleotide sequence having at least 99%, 98%, 97%, 96%, 95%, 90%, 85%, or 80% identity to the nucleotide sequence of nucleotides 1 to 48 of SEQ ID NO: 2, 8 or 9, or a fragment of the nucleotide sequence of nucleotides 1 to 48 of SEQ ID NO: 2, 8 or 9, or the nucleotide sequence having at least 99%, 98%, 97%, 96%, 95%, 90%, 85%, or 80% identity to the nucleotide sequence of nucleotides 1 to 48 of SEQ ID NO: 2, 8 or 9; and/or (ii) encodes an amino acid sequence comprising the amino acid sequence of amino acids 1 to 16 of SEQ ID NO: 1, an amino acid sequence having at least 99%, 98%, 97%, 96%, 95%, 90%, 85%, or 80% identity to the amino acid sequence of amino acids 1 to 16 of SEQ ID NO: 1, or a functional fragment of the amino acid sequence of amino acids 1 to 16 of SEQ ID NO: 1, or the amino acid sequence having at least 99%, 98%, 97%, 96%, 95%, 90%, 85%, or 80% identity to the amino acid sequence of amino acids 1 to 16 of SEQ ID NO: 1. In one embodiment, RNA encoding a signal sequence (i) comprises the nucleotide sequence of nucleotides 1 to 48 of SEQ ID NO: 2, 8 or 9; and/or (ii) encodes an amino acid sequence comprising the amino acid sequence of amino acids 1 to 16 of SEQ ID NO: 1.

In one embodiment, a signal sequence comprises the amino acid sequence of amino acids 1 to 19 of SEQ ID NO: 1, an amino acid sequence having at least 99%, 98%, 97%, 96%, 95%, 90%, 85%, or 80% identity to the amino acid sequence of amino acids 1 to 19 of SEQ ID NO: 1, or a functional fragment of the amino acid sequence of amino acids 1 to 19 of SEQ ID NO: 1, or the amino acid sequence having at least 99%, 98%, 97%, 96%, 95%, 90%, 85%, or 80% identity to the amino acid sequence of amino acids 1 to 19 of SEQ ID NO: 1. In one embodiment, a signal sequence comprises the amino acid sequence of amino acids 1 to 19 of SEQ ID NO: 1.

In one embodiment, RNA encoding a signal sequence (i) comprises the nucleotide sequence of nucleotides 1 to 57 of SEQ ID NO: 2, 8 or 9, a nucleotide sequence having at least 99%, 98%, 97%, 96%, 95%, 90%, 85%, or 80% identity to the nucleotide sequence of nucleotides 1 to 57 of SEQ ID NO: 2, 8 or 9, or a fragment of the nucleotide sequence of nucleotides 1 to 57 of SEQ ID NO: 2, 8 or 9, or the nucleotide sequence having at least 99%, 98%, 97%, 96%, 95%, 90%, 85%, or 80% identity to the nucleotide sequence of nucleotides 1 to 57 of SEQ ID NO: 2, 8 or 9; and/or (ii) encodes an amino acid sequence comprising the amino acid sequence of amino acids 1 to 19 of SEQ ID NO: 1, an amino acid sequence having at least 99%, 98%, 97%, 96%, 95%, 90%, 85%, or 80% identity to the amino acid sequence of amino acids 1 to 19 of SEQ ID NO: 1, or a functional fragment of the amino acid sequence of amino acids 1 to 19 of SEQ ID NO: 1, or the amino acid sequence having at least 99%, 98%, 97%, 96%, 95%, 90%, 85%, or 80% identity to the amino acid sequence of amino acids 1 to 19 of SEQ ID NO: 1. In one embodiment, RNA encoding a signal sequence (i) comprises the nucleotide sequence of nucleotides 1 to 57 of SEQ ID NO: 2, 8 or 9; and/or (ii) encodes an amino acid sequence comprising the amino acid sequence of amino acids 1 to 19 of SEQ ID NO: 1.

In some embodiments, a signal peptide sequence as defined herein includes, without being limited thereto, the signal peptide sequence of an immunoglobulin, e.g., the signal peptide sequence of an immunoglobulin heavy chain variable region, wherein the immunoglobulin may be human immunoglobulin. In particular, in some embodiments the signal peptide sequence as defined herein includes a sequence comprising the amino acid sequence of amino acids 1 to 22 of SEQ ID NO: 31 or a functional variant thereof.

In some embodiments, a signal peptide sequence is functional in mammalian cells.

In some embodiments, a utilized signal sequence is "intrinsic" in that it is , in nature, it is associated with (e.g., linked to) the encoded polypeptide.

In some embodiments, a utilized signal sequence is heterologous to the encoded polypeptide - e.g., is not naturally part of a polypeptide (e.g., protein) whose sequences are included in the encoded polypeptide.

In some embodiments, signal peptides are sequences, which are typically characterized by a length of about 15 to 30 amino acids.

In many embodiments, signal peptides are positioned at the N-terminus of an encoded polypeptide as described herein, without being limited thereto. In some embodiments, signal peptides preferably allow the transport of the polypeptide encoded by RNAs of the present disclosure with which they are associated into a defined cellular compartment, preferably the cell surface, the endoplasmic reticulum (ER) or the endosomal-lysosomal compartment.

In some embodiments, a signal sequence is selected from an S1S2 signal peptide (aa 1-19), an immunoglobulin secretory signal peptide (aa 1-22), an HSV-1 gD signal peptide (MGGAAARLGAVILFVVIVGLHGVRSKY), an HSV-2 gD signal peptide (MGRLTSGVGTAALLVVAVGLRVVCA); a human SPARC signal peptide, a human insulin isoform 1 signal peptide, a human albumin signal peptide, etc. Those skilled in the art will be aware of other secretory signal peptides such as, for example, as disclosed in WO2017/081082 (e.g., SEQ ID NOs: 1-1115 and 1728, or fragments variants thereof) and WO2019008001.

In some embodiments, an RNA sequence encodes an epitope that may comprise or otherwise be linked to a signal sequence (e.g., secretory sequence), such as those listed in Table A, or at least a sequence having 1, 2, 3, 4, or 5 amino acid differences relative thereto. In some embodiments, a signal sequence such as MFVFLVLLPLVSSQCVNLT, or at least a sequence having 1, 2, 3, 4, or at the most 5 amino acid differences relative thereto is utilized. In some embodiments, a sequence such as MFVFLVLLPLVSSQCVNLT, or a sequence having 1, 2, 3, 4, or at the most 5 amino acid differences relative thereto, is utilized.

In some embodiments, a signal sequence is selected from those included in the Table A below and/or those encoded by the sequences in Table B below:

**Table A: Exemplary signal sequences**

| **SEQ ID NO:** | **Signal** | **Sequence (Amino Acid)** |
|---|---|---|
| 105 | HSV-1 gD SP | MGGAAARLGAVILFVVIVGLHGVRSKY |
| 106 | HSV-2 gD SP | MGRLTSGVGTAALLVVAVGLRVVCA |
| 107 | HSV-2 | MGRLTSGVGTAALLVVAVGLRVVCAKYA |
| 108 | SARS-CoV-2-S | MFVFLVLLPLVSSQCVNLT |
| 109 | human Ig heavy chain signal peptide (huSec) | MDWIWRILFLVGAATGAHSQM |
| 110 | HuIgGk signal peptide | M ETPAQLLFLLLLW LPDTTG |
| 111 | IgE heavy chain epsilon-1signal peptide | MDWTWILFLVAAATRVHS |
| 112 | Japanese encephalitis PRM signal sequence | MLGSNSGQRVVFTILLLLVAPAYS |
| 113 | VSVg protein signal sequence | MKCLLYLAFLFIGVNCA |
| 114 | | MDWTWILFLVAAATRVHS |
| 115 | | ETPAQLLFLLLLWLPDTTG |
| 116 | | MLGSNSGQRVVFTILLLLVAPAYS |
| 117 | | MKCLLYLAFLFIGVNCA |
| 118 | | MWLVSLAIVTACAGA |
| 119 | | MFVFLVLLPLVSSQC |

**Table B: Exemplary nucleotide sequences encoding signal sequences**

| **SEQ ID NO:** | **Signal** | **Sequence (Nucleotide)** |
|---|---|---|
| 120 | HSV-1 gD SP wild-type | ATGGGGGGGGCTGCCGCCAGGTTGGGGGCCGTGATTTTGTTTGTCGTC ATAGTGGGCCTCCATGGGGTCCGCAGCAAATAT |
| 121 | HSV-1 gD SP optimized nucleotide sequence | ATGggaggagccGCCGCCagactgggaGCCGTGatcctgttcgtggtgatcGTGgga ctgCATggagtgagaAGCaagtac |
| 122 | SARS-CoV-2-S | ATGTTTGTGTTTCTTGTGCTGCTGCCTCTTGTGTCTTCTCAGTGTGTGAAT TTGACA |
| 123 | human Ig heavy chain signal peptide (huSec) | ATGGATTGGATTTGGAGAATCCTGTTCCTCGTGGGAGCCGCTACAGGA GCCCACTCCCAGATG |

In some embodiments, an RNA utilized as described herein encodes a multimerization element (e.g., a heterologous multimerization element). In some embodiments, a heterologous multimerization element comprises a dimerization, trimerization or tetramerization element.

In some embodiments, a multimerization element is one described in WO2017/081082 (e.g., SEQ ID NOs: 1116-1167, or fragments or variants thereof).

Exemplary trimerization and tetramerization elements include, but are not limited to, engineered leucine zippers, fibritin foldon domain from enterobacteria phage T4, GCN4pll, GCN4-pll, and p53.

In some embodiments, a provided encoded polypeptide(s) is able to form a trimeric complex. For example, a utilized encoded polypeptide(s) may comprise a domain allowing formation of a multimeric complex, such as for example particular a trimeric complex of an amino acid sequence comprising an encoded polypeptide(s) as described herein. In some embodiments, a domain allowing formation of a multimeric complex comprises a trimerization domain, for example, a trimerization domain as described herein.

In some embodiments, an encoded polypeptide(s) can be modified by addition of a T4-fibritin-derived "foldon" trimerization domain, for example, to increase its immunogenicity.

In some embodiments, an RNAs described herein encodes a membrane association element (e.g., a heterologous membrane association element), such as a transmembrane domain.

A transmembrane domain can be N-terminal, C-terminal, or internal to an encoded polypeptide. A coding sequence of a transmembrane element is typically placed in frame (i.e., in the same reading frame), 5', 3', or internal to coding sequences of sequences (e.g., sequences encoding polypeptide(s)) with which it is to be linked.

In some embodiments, a transmembrane domain comprises or is a transmembrane domain of Hemagglutinin (HA) of Influenza virus, Env of HIV-1, equine infectious anaemia virus (EIAV), murine leukaemia virus (MLV), mouse mammary tumor virus, G protein of vesicular stomatitis virus (VSV), Rabies virus, or a seven transmembrane domain receptor.

In one embodiment, a signal sequence comprises the amino acid sequence of amino acids 1 to 22 of SEQ ID NO: 31, an amino acid sequence having at least 99%, 98%, 97%, 96%, 95%, 90%, 85%, or 80% identity to the amino acid sequence of amino acids 1 to 22 of SEQ ID NO: 31, or a functional fragment of the amino acid sequence of amino acids 1 to 22 of SEQ ID NO: 31, or the amino acid sequence having at least 99%, 98%, 97%, 96%, 95%, 90%, 85%, or 80% identity to the amino acid sequence of amino acids 1 to 22 of SEQ ID NO: 31. In one embodiment, a signal sequence comprises the amino acid sequence of amino acids 1 to 22 of SEQ ID NO: 31.

In one embodiment, RNA encoding a signal sequence (i) comprises the nucleotide sequence of nucleotides 54 to 119 of SEQ ID NO: 32, a nucleotide sequence having at least 99%, 98%, 97%, 96%, 95%, 90%, 85%, or 80% identity to the nucleotide sequence of nucleotides 54 to 119 of SEQ ID NO: 32, or a fragment of the nucleotide sequence of nucleotides 54 to 119 of SEQ ID NO: 32, or the nucleotide sequence having at least 99%, 98%, 97%, 96%, 95%, 90%, 85%, or 80% identity to the nucleotide sequence of nucleotides 54 to 119 of SEQ ID NO: 32; and/or (ii) encodes an amino acid sequence comprising the amino acid sequence of amino acids 1 to 22 of SEQ ID NO: 31, an amino acid sequence having at least 99%, 98%, 97%, 96%, 95%, 90%, 85%, or 80% identity to the amino acid sequence of amino acids 1 to 22 of SEQ ID NO: 31, or a functional fragment of the amino acid sequence of amino acids 1 to 22 of SEQ ID NO: 31, or the amino acid sequence having at least 99%, 98%, 97%, 96%, 95%, 90%, 85%, or 80% identity to the amino acid sequence of amino acids 1 to 22 of SEQ ID NO: 31. In one embodiment, RNA encoding a signal sequence (i) comprises the nucleotide sequence of nucleotides 54 to 119 of SEQ ID NO: 32; and/or (ii) encodes an amino acid sequence comprising the amino acid sequence of amino acids 1 to 22 of SEQ ID NO: 31.

Such signal peptides are preferably used in order to promote secretion of the encoded antigenic peptide or protein. More preferably, a signal peptide as defined herein is fused to an encoded antigenic peptide or protein as defined herein.

Accordingly, in particularly preferred embodiments, the RNA described herein comprises at least one coding region encoding an antigenic peptide or protein and a signal peptide, said signal peptide preferably being fused to the antigenic peptide or protein, more preferably to the N-terminus of the antigenic peptide or protein as described herein.

In one embodiment, a vaccine antigen comprises the amino acid sequence of SEQ ID NO: 1 or 7, an amino acid sequence having at least 99%, 98%, 97%, 96%, 95%, 90%, 85%, or 80% identity to the amino acid sequence of SEQ ID NO: 1 or 7, or an immunogenic fragment of the amino acid sequence of SEQ ID NO: 1 or 7, or the amino acid sequence having at least 99%, 98%, 97%, 96%, 95%, 90%, 85%, or 80% identity to the amino acid sequence of SEQ ID NO: 1 or 7. In one embodiment, a vaccine antigen comprises the amino acid sequence of SEQ ID NO: 1 or 7.

In one embodiment, RNA encoding a vaccine antigen (i) comprises the nucleotide sequence of SEQ ID NO: 2, 8 or 9, a nucleotide sequence having at least 99%, 98%, 97%, 96%, 95%, 90%, 85%, or 80% identity to the nucleotide sequence of SEQ ID NO: 2, 8 or 9, or a fragment of the nucleotide sequence of SEQ ID NO: 2, 8 or 9, or the nucleotide sequence having at least 99%, 98%, 97%, 96%, 95%, 90%, 85%, or 80% identity to the nucleotide sequence of SEQ ID NO: 2, 8 or 9; and/or (ii) encodes an amino acid sequence comprising the amino acid sequence of SEQ ID NO: 1 or 7, an amino acid sequence having at least 99%, 98%, 97%, 96%, 95%, 90%, 85%, or 80% identity to the amino acid sequence of SEQ ID NO: 1 or 7, or an immunogenic fragment of the amino acid sequence of SEQ ID NO: 1 or 7, or the amino acid sequence having at least 99%, 98%, 97%, 96%, 95%, 90%, 85%, or 80% identity to the amino acid sequence of SEQ ID NO: 1 or 7. In one embodiment, RNA encoding a vaccine antigen (i) comprises the nucleotide sequence of SEQ ID NO: 2, 8 or 9; and/or (ii) encodes an amino acid sequence comprising the amino acid sequence of SEQ ID NO: 1 or 7.

In one embodiment, a vaccine antigen comprises the amino acid sequence of SEQ ID NO: 7, an amino acid sequence having at least 99%, 98%, 97%, 96%, 95%, 90%, 85%, or 80% identity to the amino acid sequence of SEQ ID NO: 7, or an immunogenic fragment of the amino acid sequence of SEQ ID NO: 7, or the amino acid sequence having at least 99%, 98%, 97%, 96%, 95%, 90%, 85%, or 80% identity to the amino acid sequence of SEQ ID NO: 7. In one embodiment, a vaccine antigen comprises the amino acid sequence of SEQ ID NO: 7.

In one embodiment, RNA encoding a vaccine antigen (i) comprises the nucleotide sequence of SEQ ID NO: 15, 16, 19, 20, 24, or 25, a nucleotide sequence having at least 99%, 98%, 97%, 96%, 95%, 90%, 85%, or 80% identity to the nucleotide sequence of SEQ ID NO: 15, 16, 19, 20, 24, or 25, or a fragment of the nucleotide sequence of SEQ ID NO: 15, 16, 19, 20, 24, or 25, or the nucleotide sequence having at least 99%, 98%, 97%, 96%, 95%, 90%, 85%, or 80% identity to the nucleotide sequence of SEQ ID NO: 15, 16, 19, 20, 24, or 25; and/or (ii) encodes an amino acid sequence comprising the amino acid sequence of SEQ ID NO: 7, an amino acid sequence having at least 99%, 98%, 97%, 96%, 95%, 90%, 85%, or 80% identity to the amino acid sequence of SEQ ID NO: 7, or an immunogenic fragment of the amino acid sequence of SEQ ID NO: 7, or the amino acid sequence having at least 99%, 98%, 97%, 96%, 95%, 90%, 85%, or 80% identity to the amino acid sequence of SEQ ID NO: 7. In one embodiment, RNA encoding a vaccine antigen (i) comprises the nucleotide sequence of SEQ ID NO: 15, 16, 19, 20, 24, or 25; and/or (ii) encodes an amino acid sequence comprising the amino acid sequence of SEQ ID NO: 7.

In one embodiment, a vaccine antigen comprises the amino acid sequence of amino acids 1 to 683 of SEQ ID NO: 1, an amino acid sequence having at least 99%, 98%, 97%, 96%, 95%, 90%, 85%, or 80% identity to the amino acid sequence of amino acids 1 to 683 of SEQ ID NO: 1, or an immunogenic fragment of the amino acid sequence of amino acids 1 to 683 of SEQ ID NO: 1, or the amino acid sequence having at least 99%, 98%, 97%, 96%, 95%, 90%, 85%, or 80% identity to the amino acid sequence of amino acids 1 to 683 of SEQ ID NO: 1. In one embodiment, a vaccine antigen comprises the amino acid sequence of amino acids 1 to 683 of SEQ ID NO: 1.

In one embodiment, RNA encoding a vaccine antigen (i) comprises the nucleotide sequence of nucleotides 1 to 2049 of SEQ ID NO: 2, 8 or 9, a nucleotide sequence having at least 99%, 98%, 97%, 96%, 95%, 90%, 85%, or 80% identity to the nucleotide sequence of nucleotides 1 to 2049 of SEQ ID NO: 2, 8 or 9, or a fragment of the nucleotide sequence of nucleotides 1 to 2049 of SEQ ID NO: 2, 8 or 9, or the nucleotide sequence having at least 99%, 98%, 97%, 96%, 95%, 90%, 85%, or 80% identity to the nucleotide sequence of nucleotides 1 to 2049 of SEQ ID NO: 2, 8 or 9; and/or (ii) encodes an amino acid sequence comprising the amino acid sequence of amino acids 1 to 683 of SEQ ID NO: 1, an amino acid sequence having at least 99%, 98%, 97%, 96%, 95%, 90%, 85%, or 80% identity to the amino acid sequence of amino acids 1 to 683 of SEQ ID NO: 1, or an immunogenic fragment of the amino acid sequence of amino acids 1 to 683 of SEQ ID NO: 1, or the amino acid sequence having at least 99%, 98%, 97%, 96%, 95%, 90%, 85%, or 80% identity to the amino acid sequence of amino acids 1 to 683 of SEQ ID NO: 1. In one embodiment, RNA encoding a vaccine antigen (i) comprises the nucleotide sequence of nucleotides 1 to 2049 of SEQ ID NO: 2, 8 or 9; and/or (ii) encodes an amino acid sequence comprising the amino acid sequence of amino acids 1 to 683 of SEQ ID NO: 1.

In one embodiment, a vaccine antigen comprises the amino acid sequence of amino acids 1 to 685 of SEQ ID NO: 1, an amino acid sequence having at least 99%, 98%, 97%, 96%, 95%, 90%, 85%, or 80% identity to the amino acid sequence of amino acids 1 to 685 of SEQ ID NO: 1, or an immunogenic fragment of the amino acid sequence of amino acids 1 to 685 of SEQ ID NO: 1, or the amino acid sequence having at least 99%, 98%, 97%, 96%, 95%, 90%, 85%, or 80% identity to the amino acid sequence of amino acids 1 to 685 of SEQ ID NO: 1. In one embodiment, a vaccine antigen comprises the amino acid sequence of amino acids 1 to 685 of SEQ ID NO: 1.

In one embodiment, RNA encoding a vaccine antigen (i) comprises the nucleotide sequence of nucleotides 1 to 2055 of SEQ ID NO: 2, 8 or 9, a nucleotide sequence having at least 99%, 98%, 97%, 96%, 95%, 90%, 85%, or 80% identity to the nucleotide sequence of nucleotides 1 to 2055 of SEQ ID NO: 2, 8 or 9, or a fragment of the nucleotide sequence of nucleotides 1 to 2055 of SEQ ID NO: 2, 8 or 9, or the nucleotide sequence having at least 99%, 98%, 97%, 96%, 95%, 90%, 85%, or 80% identity to the nucleotide sequence of nucleotides 1 to 2055 of SEQ ID NO: 2, 8 or 9; and/or (ii) encodes an amino acid sequence comprising the amino acid sequence of amino acids 1 to 685 of SEQ ID NO: 1, an amino acid sequence having at least 99%, 98%, 97%, 96%, 95%, 90%, 85%, or 80% identity to the amino acid sequence of amino acids 1 to 685 of SEQ ID NO: 1, or an immunogenic fragment of the amino acid sequence of amino acids 1 to 685 of SEQ ID NO: 1, or the amino acid sequence having at least 99%, 98%, 97%, 96%, 95%, 90%, 85%, or 80% identity to the amino acid sequence of amino acids 1 to 685 of SEQ ID NO: 1. In one embodiment, RNA encoding a vaccine antigen (i) comprises the nucleotide sequence of nucleotides 1 to 2055 of SEQ ID NO: 2, 8 or 9; and/or (ii) encodes an amino acid sequence comprising the amino acid sequence of amino acids 1 to 685 of SEQ ID NO: 1.

In one embodiment, a vaccine antigen comprises the amino acid sequence of SEQ ID NO: 3, an amino acid sequence having at least 99%, 98%, 97%, 96%, 95%, 90%, 85%, or 80% identity to the amino acid sequence of SEQ ID NO: 3, or an immunogenic fragment of the amino acid sequence of SEQ ID NO: 3, or the amino acid sequence having at least 99%, 98%, 97%, 96%, 95%, 90%, 85%, or 80% identity to the amino acid sequence of SEQ ID NO: 3. In one embodiment, a vaccine antigen comprises the amino acid sequence of SEQ ID NO: 3.

In one embodiment, RNA encoding a vaccine antigen (i) comprises the nucleotide sequence of SEQ ID NO: 4, a nucleotide sequence having at least 99%, 98%, 97%, 96%, 95%, 90%, 85%, or 80% identity to the nucleotide sequence of SEQ ID NO: 4, or a fragment of the nucleotide sequence of SEQ ID NO: 4, or the nucleotide sequence having at least 99%, 98%, 97%, 96%, 95%, 90%, 85%, or 80% identity to the nucleotide sequence of SEQ ID NO: 4; and/or (ii) encodes an amino acid sequence comprising the amino acid sequence of SEQ ID NO: 3, an amino acid sequence having at least 99%, 98%, 97%, 96%, 95%, 90%, 85%, or 80% identity to the amino acid sequence of SEQ ID NO: 3, or an immunogenic fragment of the amino acid sequence of SEQ ID NO: 3, or the amino acid sequence having at least 99%, 98%, 97%, 96%, 95%, 90%, 85%, or 80% identity to the amino acid sequence of SEQ ID NO: 3. In one embodiment, RNA encoding a vaccine antigen (i) comprises the nucleotide sequence of SEQ ID NO: 4; and/or (ii) encodes an amino acid sequence comprising the amino acid sequence of SEQ ID NO: 3.

In one embodiment, a vaccine antigen comprises the amino acid sequence of amino acids 1 to 221 of SEQ ID NO: 29, an amino acid sequence having at least 99%, 98%, 97%, 96%, 95%, 90%, 85%, or 80% identity to the amino acid sequence of amino acids 1 to 221 of SEQ ID NO: 29, or an immunogenic fragment of the amino acid sequence of amino acids 1 to 221 of SEQ ID NO: 29, or the amino acid sequence having at least 99%, 98%, 97%, 96%, 95%, 90%, 85%, or 80% identity to the amino acid sequence of amino acids 1 to 221 of SEQ ID NO: 29. In one embodiment, a vaccine antigen comprises the amino acid sequence of amino acids 1 to 221 of SEQ ID NO: 29.

In one embodiment, RNA encoding a vaccine antigen (i) comprises the nucleotide sequence of nucleotides 54 to 716 of SEQ ID NO: 30, a nucleotide sequence having at least 99%, 98%, 97%, 96%, 95%, 90%, 85%, or 80% identity to the nucleotide sequence of nucleotides 54 to 716 of SEQ ID NO: 30, or a fragment of the nucleotide sequence of nucleotides 54 to 716 of SEQ ID NO: 30, or the nucleotide sequence having at least 99%, 98%, 97%, 96%, 95%, 90%, 85%, or 80% identity to the nucleotide sequence of nucleotides 54 to 716 of SEQ ID NO: 30; and/or (ii) encodes an amino acid sequence comprising the amino acid sequence of amino acids 1 to 221 of SEQ ID NO: 29, an amino acid sequence having at least 99%, 98%, 97%, 96%, 95%, 90%, 85%, or 80% identity to the amino acid sequence of amino acids 1 to 221 of SEQ ID NO: 29, or an immunogenic fragment of the amino acid sequence of amino acids 1 to 221 of SEQ ID NO: 29, or the amino acid sequence having at least 99%, 98%, 97%, 96%, 95%, 90%, 85%, or 80% identity to the amino acid sequence of amino acids 1 to 221 of SEQ ID NO: 29. In one embodiment, RNA encoding a vaccine antigen (i) comprises the nucleotide sequence of nucleotides 54 to 716 of SEQ ID NO: 30; and/or (ii) encodes an amino acid sequence comprising the amino acid sequence of amino acids 1 to 221 of SEQ ID NO: 29.

In one embodiment, a vaccine antigen comprises the amino acid sequence of amino acids 1 to 224 of SEQ ID NO: 31, an amino acid sequence having at least 99%, 98%, 97%, 96%, 95%, 90%, 85%, or 80% identity to the amino acid sequence of amino acids 1 to 224 of SEQ ID NO: 31, or an immunogenic fragment of the amino acid sequence of amino acids 1 to 224 of SEQ ID NO: 31, or the amino acid sequence having at least 99%, 98%, 97%, 96%, 95%, 90%, 85%, or 80% identity to the amino acid sequence of amino acids 1 to 224 of SEQ ID NO: 31. In one embodiment, a vaccine antigen comprises the amino acid sequence of amino acids 1 to 224 of SEQ ID NO: 31.

In one embodiment, RNA encoding a vaccine antigen (i) comprises the nucleotide sequence of nucleotides 54 to 725 of SEQ ID NO: 32, a nucleotide sequence having at least 99%, 98%, 97%, 96%, 95%, 90%, 85%, or 80% identity to the nucleotide sequence of nucleotides 54 to 725 of SEQ ID NO: 32, or a fragment of the nucleotide sequence of nucleotides 54 to 725 of SEQ ID NO: 32, or the nucleotide sequence having at least 99%, 98%, 97%, 96%, 95%, 90%, 85%, or 80% identity to the nucleotide sequence of nucleotides 54 to 725 of SEQ ID NO: 32; and/or (ii) encodes an amino acid sequence comprising the amino acid sequence of amino acids 1 to 224 of SEQ ID NO: 31, an amino acid sequence having at least 99%, 98%, 97%, 96%, 95%, 90%, 85%, or 80% identity to the amino acid sequence of amino acids 1 to 224 of SEQ ID NO: 31, or an immunogenic fragment of the amino acid sequence of amino acids 1 to 224 of SEQ ID NO: 31, or the amino acid sequence having at least 99%, 98%, 97%, 96%, 95%, 90%, 85%, or 80% identity to the amino acid sequence of amino acids 1 to 224 of SEQ ID NO: 31. In one embodiment, RNA encoding a vaccine antigen (i) comprises the nucleotide sequence of nucleotides 54 to 725 of SEQ ID NO: 32; and/or (ii) encodes an amino acid sequence comprising the amino acid sequence of amino acids 1 to 224 of SEQ ID NO: 31.

According to certain embodiments, a trimerization domain is fused, either directly or through a linker, e.g., a glycine/serine linker, to a SARS-CoV-2 S protein, a variant thereof, or a fragment thereof, i.e., the antigenic peptide or protein. Accordingly, in one embodiment, a trimerization domain is fused to the above described amino acid sequences derived from SARS-CoV-2 S protein or immunogenic fragments thereof (antigenic peptides or proteins) comprised by the vaccine antigens described above (which may optionally be fused to a signal peptide as described above).

Such trimerization domains are preferably located at the C-terminus of the antigenic peptide or protein, without being limited thereto. Trimerization domains as defined herein preferably allow the trimerization of the antigenic peptide or protein as encoded by the RNA. Examples of trimerization domains as defined herein include, without being limited thereto, foldon, the natural trimerization domain of T4 fibritin. The C-terminal domain of T4 fibritin (foldon) is obligatory for the formation of the fibritin trimer structure and can be used as an artificial trimerization domain. In one embodiment, the trimerization domain as defined herein includes, without being limited thereto, a sequence comprising the amino acid sequence of amino acids 3 to 29 of SEQ ID NO: 10 or a functional variant thereof. In one embodiment, the trimerization domain as defined herein includes, without being limited thereto, a sequence comprising the amino acid sequence of SEQ ID NO: 10 or a functional variant thereof.

In one embodiment, a trimerization domain comprises the amino acid sequence of amino acids 3 to 29 of SEQ ID NO: 10, an amino acid sequence having at least 99%, 98%, 97%, 96%, 95%, 90%, 85%, or 80% identity to the amino acid sequence of amino acids 3 to 29 of SEQ ID NO: 10, or a functional fragment of the amino acid sequence of amino acids 3 to 29 of SEQ ID NO: 10, or the amino acid sequence having at least 99%, 98%, 97%, 96%, 95%, 90%, 85%, or 80% identity to the amino acid sequence of amino acids 3 to 29 of SEQ ID NO: 10. In one embodiment, a trimerization domain comprises the amino acid sequence of amino acids 3 to 29 of SEQ ID NO: 10.

In one embodiment, RNA encoding a trimerization domain (i) comprises the nucleotide sequence of nucleotides 7 to 87 of SEQ ID NO: 11, a nucleotide sequence having at least 99%, 98%, 97%, 96%, 95%, 90%, 85%, or 80% identity to the nucleotide sequence of nucleotides 7 to 87 of SEQ ID NO: 11, or a fragment of the nucleotide sequence of nucleotides 7 to 87 of SEQ ID NO: 11, or the nucleotide sequence having at least 99%, 98%, 97%, 96%, 95%, 90%, 85%, or 80% identity to the nucleotide sequence of nucleotides 7 to 87 of SEQ ID NO: 11; and/or (ii) encodes an amino acid sequence comprising the amino acid sequence of amino acids 3 to 29 of SEQ ID NO: 10, an amino acid sequence having at least 99%, 98%, 97%, 96%, 95%, 90%, 85%, or 80% identity to the amino acid sequence of amino acids 3 to 29 of SEQ ID NO: 10, or a functional fragment of the amino acid sequence of amino acids 3 to 29 of SEQ ID NO: 10, or the amino acid sequence having at least 99%, 98%, 97%, 96%, 95%, 90%, 85%, or 80% identity to the amino acid sequence of amino acids 3 to 29 of SEQ ID NO: 10. In one embodiment, RNA encoding a trimerization domain (i) comprises the nucleotide sequence of nucleotides 7 to 87 of SEQ ID NO: 11; and/or (ii) encodes an amino acid sequence comprising the amino acid sequence of amino acids 3 to 29 of SEQ ID NO: 10.

In one embodiment, a trimerization domain comprises the amino acid sequence SEQ ID NO: 10, an amino acid sequence having at least 99%, 98%, 97%, 96%, 95%, 90%, 85%, or 80% identity to the amino acid sequence of SEQ ID NO: 10, or a functional fragment of the amino acid sequence of SEQ ID NO: 10, or the amino acid sequence having at least 99%, 98%, 97%, 96%, 95%, 90%, 85%, or 80% identity to the amino acid sequence of SEQ ID NO: 10. In one embodiment, a trimerization domain comprises the amino acid sequence of SEQ ID NO: 10.

In one embodiment, RNA encoding a trimerization domain (i) comprises the nucleotide sequence of SEQ ID NO: 11, a nucleotide sequence having at least 99%, 98%, 97%, 96%, 95%, 90%, 85%, or 80% identity to the nucleotide sequence of SEQ ID NO: 11, or a fragment of the nucleotide sequence of SEQ ID NO: 11, or the nucleotide sequence having at least 99%, 98%, 97%, 96%, 95%, 90%, 85%, or 80% identity to the nucleotide sequence of SEQ ID NO: 11; and/or (ii) encodes an amino acid sequence comprising the amino acid sequence of SEQ ID NO: 10, an amino acid sequence having at least 99%, 98%, 97%, 96%, 95%, 90%, 85%, or 80% identity to the amino acid sequence of SEQ ID NO: 10, or a functional fragment of the amino acid sequence of SEQ ID NO: 10, or the amino acid sequence having at least 99%, 98%, 97%, 96%, 95%, 90%, 85%, or 80% identity to the amino acid sequence of SEQ ID NO: 10. In one embodiment, RNA encoding a trimerization domain (i) comprises the nucleotide sequence of SEQ ID NO: 11; and/or (ii) encodes an amino acid sequence comprising the amino acid sequence of SEQ ID NO: 10.

Such trimerization domains are preferably used in order to promote trimerization of the encoded antigenic peptide or protein. More preferably, a trimerization domain as defined herein is fused to an antigenic peptide or protein as defined herein.

Accordingly, in particularly preferred embodiments, the RNA described herein comprises at least one coding region encoding an antigenic peptide or protein and a trimerization domain as defined herein, said trimerization domain preferably being fused to the antigenic peptide or protein, more preferably to the C-terminus of the antigenic peptide or protein.

In one embodiment, a vaccine antigen comprises the amino acid sequence of SEQ ID NO: 5, an amino acid sequence having at least 99%, 98%, 97%, 96%, 95%, 90%, 85%, or 80% identity to the amino acid sequence of SEQ ID NO: 5, or an immunogenic fragment of the amino acid sequence of SEQ ID NO: 5, or the amino acid sequence having at least 99%, 98%, 97%, 96%, 95%, 90%, 85%, or 80% identity to the amino acid sequence of SEQ ID NO: 5. In one embodiment, a vaccine antigen comprises the amino acid sequence of SEQ ID NO: 5.

In one embodiment, RNA encoding a vaccine antigen (i) comprises the nucleotide sequence of SEQ ID NO: 6, a nucleotide sequence having at least 99%, 98%, 97%, 96%, 95%, 90%, 85%, or 80% identity to the nucleotide sequence of SEQ ID NO: 6, or a fragment of the nucleotide sequence of SEQ ID NO: 6, or the nucleotide sequence having at least 99%, 98%, 97%, 96%, 95%, 90%, 85%, or 80% identity to the nucleotide sequence of SEQ ID NO: 6; and/or (ii) encodes an amino acid sequence comprising the amino acid sequence of SEQ ID NO: 5, an amino acid sequence having at least 99%, 98%, 97%, 96%, 95%, 90%, 85%, or 80% identity to the amino acid sequence of SEQ ID NO: 5, or an immunogenic fragment of the amino acid sequence of SEQ ID NO: 5, or the amino acid sequence having at least 99%, 98%, 97%, 96%, 95%, 90%, 85%, or 80% identity to the amino acid sequence of SEQ ID NO: 5. In one embodiment, RNA encoding a vaccine antigen (i) comprises the nucleotide sequence of SEQ ID NO: 6; and/or (ii) encodes an amino acid sequence comprising the amino acid sequence of SEQ ID NO: 5.

In one embodiment, RNA encoding a vaccine antigen (i) comprises the nucleotide sequence of SEQ ID NO: 17, 21, or 26, a nucleotide sequence having at least 99%, 98%, 97%, 96%, 95%, 90%, 85%, or 80% identity to the nucleotide sequence of SEQ ID NO: 17, 21, or 26, or a fragment of the nucleotide sequence of SEQ ID NO: 17, 21, or 26, or the nucleotide sequence having at least 99%, 98%, 97%, 96%, 95%, 90%, 85%, or 80% identity to the nucleotide sequence of SEQ ID NO: 17, 21, or 26; and/or (ii) encodes an amino acid sequence comprising the amino acid sequence of SEQ ID NO: 5, an amino acid sequence having at least 99%, 98%, 97%, 96%, 95%, 90%, 85%, or 80% identity to the amino acid sequence of SEQ ID NO: 5, or an immunogenic fragment of the amino acid sequence of SEQ ID NO: 5, or the amino acid sequence having at least 99%, 98%, 97%, 96%, 95%, 90%, 85%, or 80% identity to the amino acid sequence of SEQ ID NO: 5. In one embodiment, RNA encoding a vaccine antigen (i) comprises the nucleotide sequence of SEQ ID NO: 17, 21, or 26; and/or (ii) encodes an amino acid sequence comprising the amino acid sequence of SEQ ID NO: 5.

In one embodiment, a vaccine antigen comprises the amino acid sequence of SEQ ID NO: 18, an amino acid sequence having at least 99%, 98%, 97%, 96%, 95%, 90%, 85%, or 80% identity to the amino acid sequence of SEQ ID NO: 18, or an immunogenic fragment of the amino acid sequence of SEQ ID NO: 18, or the amino acid sequence having at least 99%, 98%, 97%, 96%, 95%, 90%, 85%, or 80% identity to the amino acid sequence of SEQ ID NO: 18. In one embodiment, a vaccine antigen comprises the amino acid sequence of SEQ ID NO: 18.

In one embodiment, a vaccine antigen comprises the amino acid sequence of amino acids 1 to 257 of SEQ ID NO: 29, an amino acid sequence having at least 99%, 98%, 97%, 96%, 95%, 90%, 85%, or 80% identity to the amino acid sequence of amino acids 1 to 257 of SEQ ID NO: 29, or an immunogenic fragment of the amino acid sequence of amino acids 1 to 257 of SEQ ID NO: 29, or the amino acid sequence having at least 99%, 98%, 97%, 96%, 95%, 90%, 85%, or 80% identity to the amino acid sequence of amino acids 1 to 257 of SEQ ID NO: 29. In one embodiment, a vaccine antigen comprises the amino acid sequence of amino acids 1 to 257 of SEQ ID NO: 29.

In one embodiment, RNA encoding a vaccine antigen (i) comprises the nucleotide sequence of nucleotides 54 to 824 of SEQ ID NO: 30, a nucleotide sequence having at least 99%, 98%, 97%, 96%, 95%, 90%, 85%, or 80% identity to the nucleotide sequence of nucleotides 54 to 824 of SEQ ID NO: 30, or a fragment of the nucleotide sequence of nucleotides 54 to 824 of SEQ ID NO: 30, or the nucleotide sequence having at least 99%, 98%, 97%, 96%, 95%, 90%, 85%, or 80% identity to the nucleotide sequence of nucleotides 54 to 824 of SEQ ID NO: 30; and/or (ii) encodes an amino acid sequence comprising the amino acid sequence of amino acids 1 to 257 of SEQ ID NO: 29, an amino acid sequence having at least 99%, 98%, 97%, 96%, 95%, 90%, 85%, or 80% identity to the amino acid sequence of amino acids 1 to 257 of SEQ ID NO: 29, or an immunogenic fragment of the amino acid sequence of amino acids 1 to 257 of SEQ ID NO: 29, or the amino acid sequence having at least 99%, 98%, 97%, 96%, 95%, 90%, 85%, or 80% identity to the amino acid sequence of amino acids 1 to 257 of SEQ ID NO: 29. In one embodiment, RNA encoding a vaccine antigen (i) comprises the nucleotide sequence of nucleotides 54 to 824 of SEQ ID NO: 30; and/or (ii) encodes an amino acid sequence comprising the amino acid sequence of amino acids 1 to 257 of SEQ ID NO: 29.

In one embodiment, a vaccine antigen comprises the amino acid sequence of amino acids 1 to 260 of SEQ ID NO: 31, an amino acid sequence having at least 99%, 98%, 97%, 96%, 95%, 90%, 85%, or 80% identity to the amino acid sequence of amino acids 1 to 260 of SEQ ID NO: 31, or an immunogenic fragment of the amino acid sequence of amino acids 1 to 260 of SEQ ID NO: 31, or the amino acid sequence having at least 99%, 98%, 97%, 96%, 95%, 90%, 85%, or 80% identity to the amino acid sequence of amino acids 1 to 260 of SEQ ID NO: 31. In one embodiment, a vaccine antigen comprises the amino acid sequence of amino acids 1 to 260 of SEQ ID NO: 31.

In one embodiment, RNA encoding a vaccine antigen (i) comprises the nucleotide sequence of nucleotides 54 to 833 of SEQ ID NO: 32, a nucleotide sequence having at least 99%, 98%, 97%, 96%, 95%, 90%, 85%, or 80% identity to the nucleotide sequence of nucleotides 54 to 833 of SEQ ID NO: 32, or a fragment of the nucleotide sequence of nucleotides 54 to 833 of SEQ ID NO: 32, or the nucleotide sequence having at least 99%, 98%, 97%, 96%, 95%, 90%, 85%, or 80% identity to the nucleotide sequence of nucleotides 54 to 833 of SEQ ID NO: 32; and/or (ii) encodes an amino acid sequence comprising the amino acid sequence of amino acids 1 to 260 of SEQ ID NO: 31, an amino acid sequence having at least 99%, 98%, 97%, 96%, 95%, 90%, 85%, or 80% identity to the amino acid sequence of amino acids 1 to 260 of SEQ ID NO: 31, or an immunogenic fragment of the amino acid sequence of amino acids 1 to 260 of SEQ ID NO: 31, or the amino acid sequence having at least 99%, 98%, 97%, 96%, 95%, 90%, 85%, or 80% identity to the amino acid sequence of amino acids 1 to 260 of SEQ ID NO: 31. In one embodiment, RNA encoding a vaccine antigen (i) comprises the nucleotide sequence of nucleotides 54 to 833 of SEQ ID NO: 32; and/or (ii) encodes an amino acid sequence comprising the amino acid sequence of amino acids 1 to 260 of SEQ ID NO: 31.

In one embodiment, a vaccine antigen comprises the amino acid sequence of amino acids 20 to 257 of SEQ ID NO: 29, an amino acid sequence having at least 99%, 98%, 97%, 96%, 95%, 90%, 85%, or 80% identity to the amino acid sequence of amino acids 20 to 257 of SEQ ID NO: 29, or an immunogenic fragment of the amino acid sequence of amino acids 20 to 257 of SEQ ID NO: 29, or the amino acid sequence having at least 99%, 98%, 97%, 96%, 95%, 90%, 85%, or 80% identity to the amino acid sequence of amino acids 20 to 257 of SEQ ID NO: 29. In one embodiment, a vaccine antigen comprises the amino acid sequence of amino acids 20 to 257 of SEQ ID NO: 29.

In one embodiment, RNA encoding a vaccine antigen (i) comprises the nucleotide sequence of nucleotides 111 to 824 of SEQ ID NO: 30, a nucleotide sequence having at least 99%, 98%, 97%, 96%, 95%, 90%, 85%, or 80% identity to the nucleotide sequence of nucleotides 111 to 824 of SEQ ID NO: 30, or a fragment of the nucleotide sequence of nucleotides 111 to 824 of SEQ ID NO: 30, or the nucleotide sequence having at least 99%, 98%, 97%, 96%, 95%, 90%, 85%, or 80% identity to the nucleotide sequence of nucleotides 111 to 824 of SEQ ID NO: 30; and/or (ii) encodes an amino acid sequence comprising the amino acid sequence of amino acids 20 to 257 of SEQ ID NO: 29, an amino acid sequence having at least 99%, 98%, 97%, 96%, 95%, 90%, 85%, or 80% identity to the amino acid sequence of amino acids 20 to 257 of SEQ ID NO: 29, or an immunogenic fragment of the amino acid sequence of amino acids 20 to 257 of SEQ ID NO: 29, or the amino acid sequence having at least 99%, 98%, 97%, 96%, 95%, 90%, 85%, or 80% identity to the amino acid sequence of amino acids 20 to 257 of SEQ ID NO: 29. In one embodiment, RNA encoding a vaccine antigen (i) comprises the nucleotide sequence of nucleotides 111 to 824 of SEQ ID NO: 30; and/or (ii) encodes an amino acid sequence comprising the amino acid sequence of amino acids 20 to 257 of SEQ ID NO: 29.

In one embodiment, a vaccine antigen comprises the amino acid sequence of amino acids 23 to 260 of SEQ ID NO: 31, an amino acid sequence having at least 99%, 98%, 97%, 96%, 95%, 90%, 85%, or 80% identity to the amino acid sequence of amino acids 23 to 260 of SEQ ID NO: 31, or an immunogenic fragment of the amino acid sequence of amino acids 23 to 260 of SEQ ID NO: 31, or the amino acid sequence having at least 99%, 98%, 97%, 96%, 95%, 90%, 85%, or 80% identity to the amino acid sequence of amino acids 23 to 260 of SEQ ID NO: 31. In one embodiment, a vaccine antigen comprises the amino acid sequence of amino acids 23 to 260 of SEQ ID NO: 31.

In one embodiment, RNA encoding a vaccine antigen (i) comprises the nucleotide sequence of nucleotides 120 to 833 of SEQ ID NO: 32, a nucleotide sequence having at least 99%, 98%, 97%, 96%, 95%, 90%, 85%, or 80% identity to the nucleotide sequence of nucleotides 120 to 833 of SEQ ID NO: 32, or a fragment of the nucleotide sequence of nucleotides 120 to 833 of SEQ ID NO: 32, or the nucleotide sequence having at least 99%, 98%, 97%, 96%, 95%, 90%, 85%, or 80% identity to the nucleotide sequence of nucleotides 120 to 833 of SEQ ID NO: 32; and/or (ii) encodes an amino acid sequence comprising the amino acid sequence of amino acids 23 to 260 of SEQ ID NO: 31, an amino acid sequence having at least 99%, 98%, 97%, 96%, 95%, 90%, 85%, or 80% identity to the amino acid sequence of amino acids 23 to 260 of SEQ ID NO: 31, or an immunogenic fragment of the amino acid sequence of amino acids 23 to 260 of SEQ ID NO: 31, or the amino acid sequence having at least 99%, 98%, 97%, 96%, 95%, 90%, 85%, or 80% identity to the amino acid sequence of amino acids 23 to 260 of SEQ ID NO: 31. In one embodiment, RNA encoding a vaccine antigen (i) comprises the nucleotide sequence of nucleotides 120 to 833 of SEQ ID NO: 32; and/or (ii) encodes an amino acid sequence comprising the amino acid sequence of amino acids 23 to 260 of SEQ ID NO: 31.

According to certain embodiments, a transmembrane domain is fused, either directly or through a linker, e.g., a glycine/serine linker, to a SARS-CoV-2 S protein, a variant thereof, or a fragment thereof, i.e., the antigenic peptide or protein. Accordingly, in one embodiment, a transmembrane domain is fused to the above described amino acid sequences derived from SARS-CoV-2 S protein or immunogenic fragments thereof (antigenic peptides or proteins) comprised by the vaccine antigens described above (which may optionally be fused to a signal peptide and/or trimerization domain as described above).

Such transmembrane domains are preferably located at the C-terminus of the antigenic peptide or protein, without being limited thereto. Preferably, such transmembrane domains are located at the C-terminus of the trimerization domain, if present, without being limited thereto. In one embodiment, a trimerization domain is present between the SARS-CoV-2 S protein, a variant thereof, or a fragment thereof, i.e., the antigenic peptide or protein, and the transmembrane domain.

Transmembrane domains as defined herein preferably allow the anchoring into a cellular membrane of the antigenic peptide or protein as encoded by the RNA.

In one embodiment, the transmembrane domain sequence as defined herein includes, without being limited thereto, the transmembrane domain sequence of SARS-CoV-2 S protein, in particular a sequence comprising the amino acid sequence of amino acids 1207 to 1254 of SEQ ID NO: 1 or a functional variant thereof.

In one embodiment, a transmembrane domain sequence comprises the amino acid sequence of amino acids 1207 to 1254 of SEQ ID NO: 1, an amino acid sequence having at least 99%, 98%, 97%, 96%, 95%, 90%, 85%, or 80% identity to the amino acid sequence of amino acids 1207 to 1254 of SEQ ID NO: 1, or a functional fragment of the amino acid sequence of amino acids 1207 to 1254 of SEQ ID NO: 1, or the amino acid sequence having at least 99%, 98%, 97%, 96%, 95%, 90%, 85%, or 80% identity to the amino acid sequence of amino acids 1207 to 1254 of SEQ ID NO: 1. In one embodiment, a transmembrane domain sequence comprises the amino acid sequence of amino acids 1207 to 1254 of SEQ ID NO: 1.

In one embodiment, RNA encoding a transmembrane domain sequence (i) comprises the nucleotide sequence of nucleotides 3619 to 3762 of SEQ ID NO: 2, 8 or 9, a nucleotide sequence having at least 99%, 98%, 97%, 96%, 95%, 90%, 85%, or 80% identity to the nucleotide sequence of nucleotides 3619 to 3762 of SEQ ID NO: 2, 8 or 9, or a fragment of the nucleotide sequence of nucleotides 3619 to 3762 of SEQ ID NO: 2, 8 or 9, or the nucleotide sequence having at least 99%, 98%, 97%, 96%, 95%, 90%, 85%, or 80% identity to the nucleotide sequence of nucleotides 3619 to 3762 of SEQ ID NO: 2, 8 or 9; and/or (ii) encodes an amino acid sequence comprising the amino acid sequence of amino acids 1207 to 1254 of SEQ ID NO: 1, an amino acid sequence having at least 99%, 98%, 97%, 96%, 95%, 90%, 85%, or 80% identity to the amino acid sequence of amino acids 1207 to 1254 of SEQ ID NO: 1, or a functional fragment of the amino acid sequence of amino acids 1207 to 1254 of SEQ ID NO: 1, or the amino acid sequence having at least 99%, 98%, 97%, 96%, 95%, 90%, 85%, or 80% identity to the amino acid sequence of amino acids 1207 to 1254 of SEQ ID NO: 1. In one embodiment, RNA encoding a transmembrane domain sequence (i) comprises the nucleotide sequence of nucleotides 3619 to 3762 of SEQ ID NO: 2, 8 or 9; and/or (ii) encodes an amino acid sequence comprising the amino acid sequence of amino acids 1207 to 1254 of SEQ ID NO: 1.

In one embodiment, a vaccine antigen comprises the amino acid sequence of amino acids 1 to 311 of SEQ ID NO: 29, an amino acid sequence having at least 99%, 98%, 97%, 96%, 95%, 90%, 85%, or 80% identity to the amino acid sequence of amino acids 1 to 311 of SEQ ID NO: 29, or an immunogenic fragment of the amino acid sequence of amino acids 1 to 311 of SEQ ID NO: 29, or the amino acid sequence having at least 99%, 98%, 97%, 96%, 95%, 90%, 85%, or 80% identity to the amino acid sequence of amino acids 1 to 311 of SEQ ID NO: 29. In one embodiment, a vaccine antigen comprises the amino acid sequence of amino acids 1 to 311 of SEQ ID NO: 29.

In one embodiment, RNA encoding a vaccine antigen (i) comprises the nucleotide sequence of nucleotides 54 to 986 of SEQ ID NO: 30, a nucleotide sequence having at least 99%, 98%, 97%, 96%, 95%, 90%, 85%, or 80% identity to the nucleotide sequence of nucleotides 54 to 986 of SEQ ID NO: 30, or a fragment of the nucleotide sequence of nucleotides 54 to 986 of SEQ ID NO: 30, or the nucleotide sequence having at least 99%, 98%, 97%, 96%, 95%, 90%, 85%, or 80% identity to the nucleotide sequence of nucleotides 54 to 986 of SEQ ID NO: 30; and/or (ii) encodes an amino acid sequence comprising the amino acid sequence of amino acids 1 to 311 of SEQ ID NO: 29, an amino acid sequence having at least 99%, 98%, 97%, 96%, 95%, 90%, 85%, or 80% identity to the amino acid sequence of amino acids 1 to 311 of SEQ ID NO: 29, or an immunogenic fragment of the amino acid sequence of amino acids 1 to 311 of SEQ ID NO: 29, or the amino acid sequence having at least 99%, 98%, 97%, 96%, 95%, 90%, 85%, or 80% identity to the amino acid sequence of amino acids 1 to 311 of SEQ ID NO: 29. In one embodiment, RNA encoding a vaccine antigen (i) comprises the nucleotide sequence of nucleotides 54 to 986 of SEQ ID NO: 30; and/or (ii) encodes an amino acid sequence comprising the amino acid sequence of amino acids 1 to 311 of SEQ ID NO: 29.

In one embodiment, a vaccine antigen comprises the amino acid sequence of amino acids 1 to 314 of SEQ ID NO: 31, an amino acid sequence having at least 99%, 98%, 97%, 96%, 95%, 90%, 85%, or 80% identity to the amino acid sequence of amino acids 1 to 314 of SEQ ID NO: 31, or an immunogenic fragment of the amino acid sequence of amino acids 1 to 314 of SEQ ID NO: 31, or the amino acid sequence having at least 99%, 98%, 97%, 96%, 95%, 90%, 85%, or 80% identity to the amino acid sequence of amino acids 1 to 314 of SEQ ID NO: 31. In one embodiment, a vaccine antigen comprises the amino acid sequence of amino acids 1 to 314 of SEQ ID NO: 31.

In one embodiment, RNA encoding a vaccine antigen (i) comprises the nucleotide sequence of nucleotides 54 to 995 of SEQ ID NO: 32, a nucleotide sequence having at least 99%, 98%, 97%, 96%, 95%, 90%, 85%, or 80% identity to the nucleotide sequence of nucleotides 54 to 995 of SEQ ID NO: 32, or a fragment of the nucleotide sequence of nucleotides 54 to 995 of SEQ ID NO: 32, or the nucleotide sequence having at least 99%, 98%, 97%, 96%, 95%, 90%, 85%, or 80% identity to the nucleotide sequence of nucleotides 54 to 995 of SEQ ID NO: 32; and/or (ii) encodes an amino acid sequence comprising the amino acid sequence of amino acids 1 to 314 of SEQ ID NO: 31, an amino acid sequence having at least 99%, 98%, 97%, 96%, 95%, 90%, 85%, or 80% identity to the amino acid sequence of amino acids 1 to 314 of SEQ ID NO: 31, or an immunogenic fragment of the amino acid sequence of amino acids 1 to 314 of SEQ ID NO: 31, or the amino acid sequence having at least 99%, 98%, 97%, 96%, 95%, 90%, 85%, or 80% identity to the amino acid sequence of amino acids 1 to 314 of SEQ ID NO: 31. In one embodiment, RNA encoding a vaccine antigen (i) comprises the nucleotide sequence of nucleotides 54 to 995 of SEQ ID NO: 32; and/or (ii) encodes an amino acid sequence comprising the amino acid sequence of amino acids 1 to 314 of SEQ ID NO: 31.

In one embodiment, a vaccine antigen comprises the amino acid sequence of amino acids 20 to 311 of SEQ ID NO: 29, an amino acid sequence having at least 99%, 98%, 97%, 96%, 95%, 90%, 85%, or 80% identity to the amino acid sequence of amino acids 20 to 311 of SEQ ID NO: 29, or an immunogenic fragment of the amino acid sequence of amino acids 20 to 311 of SEQ ID NO: 29, or the amino acid sequence having at least 99%, 98%, 97%, 96%, 95%, 90%, 85%, or 80% identity to the amino acid sequence of amino acids 20 to 311 of SEQ ID NO: 29. In one embodiment, a vaccine antigen comprises the amino acid sequence of amino acids 20 to 311 of SEQ ID NO: 29.

In one embodiment, RNA encoding a vaccine antigen (i) comprises the nucleotide sequence of nucleotides 111 to 986 of SEQ ID NO: 30, a nucleotide sequence having at least 99%, 98%, 97%, 96%, 95%, 90%, 85%, or 80% identity to the nucleotide sequence of nucleotides 111 to 986 of SEQ ID NO: 30, or a fragment of the nucleotide sequence of nucleotides 111 to 986 of SEQ ID NO: 30, or the nucleotide sequence having at least 99%, 98%, 97%, 96%, 95%, 90%, 85%, or 80% identity to the nucleotide sequence of nucleotides 111 to 986 of SEQ ID NO: 30; and/or (ii) encodes an amino acid sequence comprising the amino acid sequence of amino acids 20 to 311 of SEQ ID NO: 29, an amino acid sequence having at least 99%, 98%, 97%, 96%, 95%, 90%, 85%, or 80% identity to the amino acid sequence of amino acids 20 to 311 of SEQ ID NO: 29, or an immunogenic fragment of the amino acid sequence of amino acids 20 to 311 of SEQ ID NO: 29, or the amino acid sequence having at least 99%, 98%, 97%, 96%, 95%, 90%, 85%, or 80% identity to the amino acid sequence of amino acids 20 to 311 of SEQ ID NO: 29. In one embodiment, RNA encoding a vaccine antigen (i) comprises the nucleotide sequence of nucleotides 111 to 986 of SEQ ID NO: 30; and/or (ii) encodes an amino acid sequence comprising the amino acid sequence of amino acids 20 to 311 of SEQ ID NO: 29.

In one embodiment, a vaccine antigen comprises the amino acid sequence of amino acids 23 to 314 of SEQ ID NO: 31, an amino acid sequence having at least 99%, 98%, 97%, 96%, 95%, 90%, 85%, or 80% identity to the amino acid sequence of amino acids 23 to 314 of SEQ ID NO: 31, or an immunogenic fragment of the amino acid sequence of amino acids 23 to 314 of SEQ ID NO: 31, or the amino acid sequence having at least 99%, 98%, 97%, 96%, 95%, 90%, 85%, or 80% identity to the amino acid sequence of amino acids 23 to 314 of SEQ ID NO: 31. In one embodiment, a vaccine antigen comprises the amino acid sequence of amino acids 23 to 314 of SEQ ID NO: 31.

In one embodiment, RNA encoding a vaccine antigen (i) comprises the nucleotide sequence of nucleotides 120 to 995 of SEQ ID NO: 32, a nucleotide sequence having at least 99%, 98%, 97%, 96%, 95%, 90%, 85%, or 80% identity to the nucleotide sequence of nucleotides 120 to 995 of SEQ ID NO: 32, or a fragment of the nucleotide sequence of nucleotides 120 to 995 of SEQ ID NO: 32, or the nucleotide sequence having at least 99%, 98%, 97%, 96%, 95%, 90%, 85%, or 80% identity to the nucleotide sequence of nucleotides 120 to 995 of SEQ ID NO: 32; and/or (ii) encodes an amino acid sequence comprising the amino acid sequence of amino acids 23 to 314 of SEQ ID NO: 31, an amino acid sequence having at least 99%, 98%, 97%, 96%, 95%, 90%, 85%, or 80% identity to the amino acid sequence of amino acids 23 to 314 of SEQ ID NO: 31, or an immunogenic fragment of the amino acid sequence of amino acids 23 to 314 of SEQ ID NO: 31, or the amino acid sequence having at least 99%, 98%, 97%, 96%, 95%, 90%, 85%, or 80% identity to the amino acid sequence of amino acids 23 to 314 of SEQ ID NO: 31. In one embodiment, RNA encoding a vaccine antigen (i) comprises the nucleotide sequence of nucleotides 120 to 995 of SEQ ID NO: 32; and/or (ii) encodes an amino acid sequence comprising the amino acid sequence of amino acids 23 to 314 of SEQ ID NO: 31.

In one embodiment, RNA encoding a vaccine antigen (i) comprises the nucleotide sequence of SEQ ID NO: 30, a nucleotide sequence having at least 99%, 98%, 97%, 96%, 95%, 90%, 85%, or 80% identity to the nucleotide sequence of SEQ ID NO: 30, or a fragment of the nucleotide sequence of SEQ ID NO: 30, or the nucleotide sequence having at least 99%, 98%, 97%, 96%, 95%, 90%, 85%, or 80% identity to the nucleotide sequence of SEQ ID NO: 30; and/or (ii) encodes an amino acid sequence comprising the amino acid sequence of SEQ ID NO: 29, an amino acid sequence having at least 99%, 98%, 97%, 96%, 95%, 90%, 85%, or 80% identity to the amino acid sequence of SEQ ID NO: 29, or an immunogenic fragment of the amino acid sequence of SEQ ID NO: 29, or the amino acid sequence having at least 99%, 98%, 97%, 96%, 95%, 90%, 85%, or 80% identity to the amino acid sequence of SEQ ID NO: 29. In one embodiment, RNA encoding a vaccine antigen (i) comprises the nucleotide sequence of SEQ ID NO: 30; and/or (ii) encodes an amino acid sequence comprising the amino acid sequence of SEQ ID NO: 29.

In one embodiment, RNA encoding a vaccine antigen (i) comprises the nucleotide sequence of SEQ ID NO: 32, a nucleotide sequence having at least 99%, 98%, 97%, 96%, 95%, 90%, 85%, or 80% identity to the nucleotide sequence of SEQ ID NO: 32, or a fragment of the nucleotide sequence of SEQ ID NO: 32, or the nucleotide sequence having at least 99%, 98%, 97%, 96%, 95%, 90%, 85%, or 80% identity to the nucleotide sequence of SEQ ID NO: 32; and/or (ii) encodes an amino acid sequence comprising the amino acid sequence of SEQ ID NO: 31, an amino acid sequence having at least 99%, 98%, 97%, 96%, 95%, 90%, 85%, or 80% identity to the amino acid sequence of SEQ ID NO: 31, or an immunogenic fragment of the amino acid sequence of SEQ ID NO: 31, or the amino acid sequence having at least 99%, 98%, 97%, 96%, 95%, 90%, 85%, or 80% identity to the amino acid sequence of SEQ ID NO: 31. In one embodiment, RNA encoding a vaccine antigen (i) comprises the nucleotide sequence of SEQ ID NO: 32; and/or (ii) encodes an amino acid sequence comprising the amino acid sequence of SEQ ID NO: 31.

In one embodiment, a vaccine antigen comprises the amino acid sequence of SEQ ID NO: 28, an amino acid sequence having at least 99%, 98%, 97%, 96%, 95%, 90%, 85%, or 80% identity to the amino acid sequence of SEQ ID NO: 28, or an immunogenic fragment of the amino acid sequence of SEQ ID NO: 28, or the amino acid sequence having at least 99%, 98%, 97%, 96%, 95%, 90%, 85%, or 80% identity to the amino acid sequence of SEQ ID NO: 28. In one embodiment, a vaccine antigen comprises the amino acid sequence of SEQ ID NO: 28.

In one embodiment, RNA encoding a vaccine antigen (i) comprises the nucleotide sequence of SEQ ID NO: 27, a nucleotide sequence having at least 99%, 98%, 97%, 96%, 95%, 90%, 85%, or 80% identity to the nucleotide sequence of SEQ ID NO: 27, or a fragment of the nucleotide sequence of SEQ ID NO: 27, or the nucleotide sequence having at least 99%, 98%, 97%, 96%, 95%, 90%, 85%, or 80% identity to the nucleotide sequence of SEQ ID NO: 27; and/or (ii) encodes an amino acid sequence comprising the amino acid sequence of SEQ ID NO: 28, an amino acid sequence having at least 99%, 98%, 97%, 96%, 95%, 90%, 85%, or 80% identity to the amino acid sequence of SEQ ID NO: 28, or an immunogenic fragment of the amino acid sequence of SEQ ID NO: 28, or the amino acid sequence having at least 99%, 98%, 97%, 96%, 95%, 90%, 85%, or 80% identity to the amino acid sequence of SEQ ID NO: 28. In one embodiment, RNA encoding a vaccine antigen (i) comprises the nucleotide sequence of SEQ ID NO: 27; and/or (ii) encodes an amino acid sequence comprising the amino acid sequence of SEQ ID NO: 28.

In one embodiment, a vaccine antigen comprises the amino acid sequence of SEQ ID NO: 49, an amino acid sequence having at least 99.5%, 99%, 98.5%, 98%, 98.5% or 97% identity to the amino acid sequence of SEQ ID NO: 49, or an immunogenic fragment of the amino acid sequence of SEQ ID NO: 49, or the amino acid sequence having at least 99.5%, 99%, 98.5%, 98%, 98.5% or 97% identity to the amino acid sequence of SEQ ID NO: 49. In one embodiment, a vaccine antigen comprises the amino acid sequence of SEQ ID NO: 49. The amino acid sequence of SEQ ID NO: 49 corresponds to the amino acid sequence of the full-length S protein from Omicron BA.1, which includes proline residues at positions corresponding to residues 986 and 987 of SEQ ID NO: 1 (residues 983 and 984 of SEQ ID NO: 49).

In one embodiment, RNA encoding a vaccine antigen (i) comprises the nucleotide sequence of SEQ ID NO: 50, a nucleotide sequence having at least 99.5%, 99%, 98.5%, 98%, 98.5% or 97% identity to the nucleotide sequence of SEQ ID NO: 50, or a fragment of the nucleotide sequence of SEQ ID NO: 50, or the nucleotide sequence having at least 99.5%, 99%, 98.5%, 98%, 98.5% or 97% identity to the nucleotide sequence of SEQ ID NO: 50; and/or (ii) encodes an amino acid sequence comprising the amino acid sequence of SEQ ID NO: 49, an amino acid sequence having at least 99.5%, 99%, 98.5%, 98%, 98.5% or 97% identity to the amino acid sequence of SEQ ID NO: 49, or an immunogenic fragment of the amino acid sequence of SEQ ID NO: 49, or the amino acid sequence having at least 99.5%, 99%, 98.5%, 98%, 98.5% or 97% identity to the amino acid sequence of SEQ ID NO: 49. In one embodiment, RNA encoding a vaccine antigen (i) comprises the nucleotide sequence of SEQ ID NO: 50; and/or (ii) encodes an amino acid sequence comprising the amino acid sequence of SEQ ID NO: 49. The nucleotide sequence of SEQ ID NO: 50 is a nucleotide sequence designed to encode the amino acid sequence of the full-length S protein from Omicron BA.1 with proline residues at positions corresponding to residues 986 and 987 of SEQ ID NO: 1 (residues 983 and 984 of SEQ ID NO: 49).

In one embodiment, RNA encoding a vaccine antigen (i) comprises the nucleotide sequence of SEQ ID NO: 51, a nucleotide sequence having at least 99.5%, 99%, 98.5%, 98%, 98.5% or 97% identity to the nucleotide sequence of SEQ ID NO: 51, or a fragment of the nucleotide sequence of SEQ ID NO: 51, or the nucleotide sequence having at least 99.5%, 99%, 98.5%, 98%, 98.5% or 97% identity to the nucleotide sequence of SEQ ID NO: 51; and/or (ii) encodes an amino acid sequence comprising the amino acid sequence of SEQ ID NO: 49, an amino acid sequence having at least 99.5%, 99%, 98.5%, 98%, 98.5% or 97% identity to the amino acid sequence of SEQ ID NO: 49, or an immunogenic fragment of the amino acid sequence of SEQ ID NO: 49, or the amino acid sequence having at least 99.5%, 99%, 98.5%, 98%, 98.5% or 97% identity to the amino acid sequence of SEQ ID NO: 49. In one embodiment, RNA encoding a vaccine antigen (i) comprises the nucleotide sequence of SEQ ID NO: 51; and/or (ii) encodes an amino acid sequence comprising the amino acid sequence of SEQ ID NO: 49. The nucleotide sequence of SEQ ID NO: 51 corresponds to an RNA construct (e.g., comprising a 5' UTR, a S-protein-encoding sequence, a 3' UTR, and a poly-A tail), which encodes the amino acid sequence of the full-length S protein from Omicron BA.1 with proline residues at positions corresponding to residues 986 and 987 of SEQ ID NO: 1 (corresponding to residues 983 and 984 of SEQ ID NO: 49).

In one embodiment, a vaccine antigen comprises the amino acid sequence of SEQ ID NO: 55, an amino acid sequence having at least 99.5%, 99%, 98.5%, 98%, 98.5% or 97% identity to the amino acid sequence of SEQ ID NO: 55, or an immunogenic fragment of the amino acid sequence of SEQ ID NO: 55, or the amino acid sequence having at least 99.5%, 99%, 98.5%, 98%, 98.5% or 97% identity to the amino acid sequence of SEQ ID NO: 55. In one embodiment, a vaccine antigen comprises the amino acid sequence of SEQ ID NO: 55.

In one embodiment, RNA encoding a vaccine antigen (i) comprises the nucleotide sequence of SEQ ID NO: 56, a nucleotide sequence having at least 99.5%, 99%, 98.5%, 98%, 98.5% or 97% identity to the nucleotide sequence of SEQ ID NO: 56, or a fragment of the nucleotide sequence of SEQ ID NO: 56, or the nucleotide sequence having at least 99.5%, 99%, 98.5%, 98%, 98.5% or 97% identity to the nucleotide sequence of SEQ ID NO: 56; and/or (ii) encodes an amino acid sequence comprising the amino acid sequence of SEQ ID NO: 55, an amino acid sequence having at least 99.5%, 99%, 98.5%, 98%, 98.5% or 97% identity to the amino acid sequence of SEQ ID NO: 55, or an immunogenic fragment of the amino acid sequence of SEQ ID NO: 55, or the amino acid sequence having at least 99.5%, 99%, 98.5%, 98%, 98.5% or 97% identity to the amino acid sequence of SEQ ID NO: 55. In one embodiment, RNA encoding a vaccine antigen (i) comprises the nucleotide sequence of SEQ ID NO: 56; and/or (ii) encodes an amino acid sequence comprising the amino acid sequence of SEQ ID NO: 55.

In one embodiment, RNA encoding a vaccine antigen (i) comprises the nucleotide sequence of SEQ ID NO: 57, a nucleotide sequence having at least 99.5%, 99%, 98.5%, 98%, 98.5% or 97% identity to the nucleotide sequence of SEQ ID NO: 57, or a fragment of the nucleotide sequence of SEQ ID NO: 57, or the nucleotide sequence having at least 99.5%, 99%, 98.5%, 98%, 98.5% or 97% identity to the nucleotide sequence of SEQ ID NO: 57; and/or (ii) encodes an amino acid sequence comprising the amino acid sequence of SEQ ID NO: 55, an amino acid sequence having at least 99.5%, 99%, 98.5%, 98%, 98.5% or 97% identity to the amino acid sequence of SEQ ID NO: 55, or an immunogenic fragment of the amino acid sequence of SEQ ID NO: 55, or the amino acid sequence having at least 99.5%, 99%, 98.5%, 98%, 98.5% or 97% identity to the amino acid sequence of SEQ ID NO: 55. In one embodiment, RNA encoding a vaccine antigen (i) comprises the nucleotide sequence of SEQ ID NO: 57; and/or (ii) encodes an amino acid sequence comprising the amino acid sequence of SEQ ID NO: 55.

In one embodiment, a vaccine antigen comprises the amino acid sequence of SEQ ID NO: 58, an amino acid sequence having at least 99.5%, 99%, 98.5%, 98%, 98.5% or 97% identity to the amino acid sequence of SEQ ID NO: 58, or an immunogenic fragment of the amino acid sequence of SEQ ID NO: 58, or the amino acid sequence having at least 99.5%, 99%, 98.5%, 98%, 98.5% or 97% identity to the amino acid sequence of SEQ ID NO: 58. In one embodiment, a vaccine antigen comprises the amino acid sequence of SEQ ID NO: 58.

In one embodiment, RNA encoding a vaccine antigen (i) comprises the nucleotide sequence of SEQ ID NO: 59, a nucleotide sequence having at least 99.5%, 99%, 98.5%, 98%, 98.5% or 97% identity to the nucleotide sequence of SEQ ID NO: 59, or a fragment of the nucleotide sequence of SEQ ID NO: 59, or the nucleotide sequence having at least 99.5%, 99%, 98.5%, 98%, 98.5% or 97% identity to the nucleotide sequence of SEQ ID NO: 59; and/or (ii) encodes an amino acid sequence comprising the amino acid sequence of SEQ ID NO: 58, an amino acid sequence having at least 99.5%, 99%, 98.5%, 98%, 98.5% or 97% identity to the amino acid sequence of SEQ ID NO: 58, or an immunogenic fragment of the amino acid sequence of SEQ ID NO: 58, or the amino acid sequence having at least 99.5%, 99%, 98.5%, 98%, 98.5% or 97% identity to the amino acid sequence of SEQ ID NO: 58. In one embodiment, RNA encoding a vaccine antigen (i) comprises the nucleotide sequence of SEQ ID NO: 59; and/or (ii) encodes an amino acid sequence comprising the amino acid sequence of SEQ ID NO: 58.

In one embodiment, RNA encoding a vaccine antigen (i) comprises the nucleotide sequence of SEQ ID NO: 60, a nucleotide sequence having at least 99.5%, 99%, 98.5%, 98%, 98.5% or 97% identity to the nucleotide sequence of SEQ ID NO: 60, or a fragment of the nucleotide sequence of SEQ ID NO: 60, or the nucleotide sequence having at least 99.5%, 99%, 98.5%, 98%, 98.5% or 97% identity to the nucleotide sequence of SEQ ID NO: 60; and/or (ii) encodes an amino acid sequence comprising the amino acid sequence of SEQ ID NO: 58, an amino acid sequence having at least 99.5%, 99%, 98.5%, 98%, 98.5% or 97% identity to the amino acid sequence of SEQ ID NO: 58, or an immunogenic fragment of the amino acid sequence of SEQ ID NO: 58, or the amino acid sequence having at least 99.5%, 99%, 98.5%, 98%, 98.5% or 97% identity to the amino acid sequence of SEQ ID NO: 58. In one embodiment, RNA encoding a vaccine antigen (i) comprises the nucleotide sequence of SEQ ID NO: 60; and/or (ii) encodes an amino acid sequence comprising the amino acid sequence of SEQ ID NO: 58.

In one embodiment, a vaccine antigen comprises the amino acid sequence of SEQ ID NO: 61, an amino acid sequence having at least 99.5%, 99%, 98.5%, 98%, 98.5% or 97% identity to the amino acid sequence of SEQ ID NO: 61, or an immunogenic fragment of the amino acid sequence of SEQ ID NO: 61, or the amino acid sequence having at least 99.5%, 99%, 98.5%, 98%, 98.5% or 97% identity to the amino acid sequence of SEQ ID NO: 61. In one embodiment, a vaccine antigen comprises the amino acid sequence of SEQ ID NO: 61.

In one embodiment, RNA encoding a vaccine antigen (i) comprises the nucleotide sequence of SEQ ID NO: 62a, a nucleotide sequence having at least 99.5%, 99%, 98.5%, 98%, 98.5% or 97% identity to the nucleotide sequence of SEQ ID NO: 62a, or a fragment of the nucleotide sequence of SEQ ID NO: 62a, or the nucleotide sequence having at least 99.5%, 99%, 98.5%, 98%, 98.5% or 97% identity to the nucleotide sequence of SEQ ID NO: 62a; and/or (ii) encodes an amino acid sequence comprising the amino acid sequence of SEQ ID NO: 61, an amino acid sequence having at least 99.5%, 99%, 98.5%, 98%, 98.5% or 97% identity to the amino acid sequence of SEQ ID NO: 61, or an immunogenic fragment of the amino acid sequence of SEQ ID NO: 61, or the amino acid sequence having at least 99.5%, 99%, 98.5%, 98%, 98.5% or 97% identity to the amino acid sequence of SEQ ID NO: 61. In one embodiment, RNA encoding a vaccine antigen (i) comprises the nucleotide sequence of SEQ ID NO: 62a; and/or (ii) encodes an amino acid sequence comprising the amino acid sequence of SEQ ID NO: 61.

In one embodiment, RNA encoding a vaccine antigen (i) comprises the nucleotide sequence of SEQ ID NO: 63a, a nucleotide sequence having at least 99.5%, 99%, 98.5%, 98%, 98.5% or 97% identity to the nucleotide sequence of SEQ ID NO: 63a, or a fragment of the nucleotide sequence of SEQ ID NO: 63a, or the nucleotide sequence having at least 99.5%, 99%, 98.5%, 98%, 98.5% or 97% identity to the nucleotide sequence of SEQ ID NO: 63a; and/or (ii) encodes an amino acid sequence comprising the amino acid sequence of SEQ ID NO: 61, an amino acid sequence having at least 99.5%, 99%, 98.5%, 98%, 98.5% or 97% identity to the amino acid sequence of SEQ ID NO: 61, or an immunogenic fragment of the amino acid sequence of SEQ ID NO: 61, or the amino acid sequence having at least 99.5%, 99%, 98.5%, 98%, 98.5% or 97% identity to the amino acid sequence of SEQ ID NO: 61. In one embodiment, RNA encoding a vaccine antigen (i) comprises the nucleotide sequence of SEQ ID NO: 63a; and/or (ii) encodes an amino acid sequence comprising the amino acid sequence of SEQ ID NO: 61.

In one embodiment, a vaccine antigen comprises the amino acid sequence of SEQ ID NO: 52, an amino acid sequence having at least 99.5%, 99%, 98.5%, 98%, 98.5% or 97% identity to the amino acid sequence of SEQ ID NO: 52, or an immunogenic fragment of the amino acid sequence of SEQ ID NO: 52, or the amino acid sequence having at least 99.5%, 99%, 98.5%, 98%, 98.5% or 97% identity to the amino acid sequence of SEQ ID NO: 52. In one embodiment, a vaccine antigen comprises the amino acid sequence of SEQ ID NO: 52.

In one embodiment, RNA encoding a vaccine antigen (i) comprises the nucleotide sequence of SEQ ID NO: 53, a nucleotide sequence having at least 99.5%, 99%, 98.5%, 98%, 98.5% or 97% identity to the nucleotide sequence of SEQ ID NO: 53, or a fragment of the nucleotide sequence of SEQ ID NO: 53, or the nucleotide sequence having at least 99.5%, 99%, 98.5%, 98%, 98.5% or 97% identity to the nucleotide sequence of SEQ ID NO: 53; and/or (ii) encodes an amino acid sequence comprising the amino acid sequence of SEQ ID NO: 52, an amino acid sequence having at least 99.5%, 99%, 98.5%, 98%, 98.5% or 97% identity to the amino acid sequence of SEQ ID NO: 52, or an immunogenic fragment of the amino acid sequence of SEQ ID NO: 52, or the amino acid sequence having at least 99.5%, 99%, 98.5%, 98%, 98.5% or 97% identity to the amino acid sequence of SEQ ID NO: 52. In one embodiment, RNA encoding a vaccine antigen (i) comprises the nucleotide sequence of SEQ ID NO: 53; and/or (ii) encodes an amino acid sequence comprising the amino acid sequence of SEQ ID NO: 52.

In one embodiment, RNA encoding a vaccine antigen (i) comprises the nucleotide sequence of SEQ ID NO: 54, a nucleotide sequence having at least 99.5%, 99%, 98.5%, 98%, 98.5% or 97% identity to the nucleotide sequence of SEQ ID NO: 54, or a fragment of the nucleotide sequence of SEQ ID NO: 54, or the nucleotide sequence having at least 99.5%, 99%, 98.5%, 98%, 98.5% or 97% identity to the nucleotide sequence of SEQ ID NO: 54; and/or (ii) encodes an amino acid sequence comprising the amino acid sequence of SEQ ID NO: 52, an amino acid sequence having at least 99.5%, 99%, 98.5%, 98%, 98.5% or 97% identity to the amino acid sequence of SEQ ID NO: 52, or an immunogenic fragment of the amino acid sequence of SEQ ID NO: 52, or the amino acid sequence having at least 99.5%, 99%, 98.5%, 98%, 98.5% or 97% identity to the amino acid sequence of SEQ ID NO: 52. In one embodiment, RNA encoding a vaccine antigen (i) comprises the nucleotide sequence of SEQ ID NO: 54; and/or (ii) encodes an amino acid sequence comprising the amino acid sequence of SEQ ID NO: 52.

In one embodiment, the vaccine antigens described above comprise a contiguous sequence of SARS-CoV-2 coronavirus spike (S) protein that consists of or essentially consists of the above described amino acid sequences derived from SARS-CoV-2 S protein or immunogenic fragments thereof (antigenic peptides or proteins) comprised by the vaccine antigens described above. In one embodiment, the vaccine antigens described above comprise a contiguous sequence of SARS-CoV-2 coronavirus spike (S) protein of no more than 220 amino acids, 215 amino acids, 210 amino acids, or 205 amino acids.

In one embodiment, RNA encoding a vaccine antigen is nucleoside modified messenger RNA (modRNA) described herein as BNT162b1 (RBP020.3), BNT162b2 (RBP020.1 or RBP020.2), or BNT162b3 (e.g., BNT162b3c). In one embodiment, RNA encoding a vaccine antigen is nucleoside modified messenger RNA (modRNA) described herein as RBP020.2. In one embodiment, RNA encoding a vaccine antigen is nucleoside modified messenger RNA (modRNA) described herein as BNT162b3 (e.g., BNT162b3c).

In one embodiment, RNA encoding a vaccine antigen is nucleoside modified messenger RNA (modRNA) and (i) comprises the nucleotide sequence of SEQ ID NO: 21, a nucleotide sequence having at least 99%, 98%, 97%, 96%, 95%, 90%, 85%, or 80% identity to the nucleotide sequence of SEQ ID NO: 21, and/or (ii) encodes an amino acid sequence comprising the amino acid sequence of SEQ ID NO: 5, or an amino acid sequence having at least 99%, 98%, 97%, 96%, 95%, 90%, 85%, or 80% identity to the amino acid sequence of SEQ ID NO: 5. In one embodiment, RNA encoding a vaccine antigen is nucleoside modified messenger RNA (modRNA) and (i) comprises the nucleotide sequence of SEQ ID NO: 21; and/or (ii) encodes an amino acid sequence comprising the amino acid sequence of SEQ ID NO: 5.

In one embodiment, RNA encoding a vaccine antigen is nucleoside modified messenger RNA (modRNA) and (i) comprises the nucleotide sequence of SEQ ID NO: 19, or 20, a nucleotide sequence having at least 99%, 98%, 97%, 96%, 95%, 90%, 85%, or 80% identity to the nucleotide sequence of SEQ ID NO: 19, or 20, and/or (ii) encodes an amino acid sequence comprising the amino acid sequence of SEQ ID NO: 7, or an amino acid sequence having at least 99%, 98%, 97%, 96%, 95%, 90%, 85%, or 80% identity to the amino acid sequence of SEQ ID NO: 7. In one embodiment, RNA encoding a vaccine antigen is nucleoside modified messenger RNA (modRNA) and (i) comprises the nucleotide sequence of SEQ ID NO: 19, or 20; and/or (ii) encodes an amino acid sequence comprising the amino acid sequence of SEQ ID NO: 7.

In one embodiment, RNA encoding a vaccine antigen is nucleoside modified messenger RNA (modRNA) and (i) comprises the nucleotide sequence of SEQ ID NO: 20, a nucleotide sequence having at least 99%, 98%, 97%, 96%, 95%, 90%, 85%, or 80% identity to the nucleotide sequence of SEQ ID NO: 20, and/or (ii) encodes an amino acid sequence comprising the amino acid sequence of SEQ ID NO: 7, or an amino acid sequence having at least 99%, 98%, 97%, 96%, 95%, 90%, 85%, or 80% identity to the amino acid sequence of SEQ ID NO: 7. In one embodiment, RNA encoding a vaccine antigen is nucleoside modified messenger RNA (modRNA) and (i) comprises the nucleotide sequence of SEQ ID NO: 20; and/or (ii) encodes an amino acid sequence comprising the amino acid sequence of SEQ ID NO: 7.

In one embodiment, RNA encoding a vaccine antigen is nucleoside modified messenger RNA (modRNA) and (i) comprises the nucleotide sequence of SEQ ID NO: 30, a nucleotide sequence having at least 99%, 98%, 97%, 96%, 95%, 90%, 85%, or 80% identity to the nucleotide sequence of SEQ ID NO: 30, and/or (ii) encodes an amino acid sequence comprising the amino acid sequence of SEQ ID NO: 29, or an amino acid sequence having at least 99%, 98%, 97%, 96%, 95%, 90%, 85%, or 80% identity to the amino acid sequence of SEQ ID NO: 29. In one embodiment, RNA encoding a vaccine antigen is nucleoside modified messenger RNA (modRNA) and (i) comprises the nucleotide sequence of SEQ ID NO: 30; and/or (ii) encodes an amino acid sequence comprising the amino acid sequence of SEQ ID NO: 29.

In one embodiment, RNA encoding a vaccine antigen is nucleoside modified messenger RNA (modRNA) and (i) comprises the nucleotide sequence of SEQ ID NO: 50, a nucleotide sequence having at least 99.5%, 99%, 98.5%, 98%, 98.5% or 97% identity to the nucleotide sequence of SEQ ID NO: 50, and/or (ii) encodes an amino acid sequence comprising the amino acid sequence of SEQ ID NO: 49, or an amino acid sequence having at least 99.5%, 99%, 98.5%, 98%, 98.5% or 97% identity to the amino acid sequence of SEQ ID NO: 49. In one embodiment, RNA encoding a vaccine antigen is nucleoside modified messenger RNA (modRNA) and (i) comprises the nucleotide sequence of SEQ ID NO: 50; and/or (ii) encodes an amino acid sequence comprising the amino acid sequence of SEQ ID NO: 49.

In one embodiment, RNA encoding a vaccine antigen is nucleoside modified messenger RNA (modRNA) and (i) comprises the nucleotide sequence of SEQ ID NO: 51, a nucleotide sequence having at least 99.5%, 99%, 98.5%, 98%, 98.5% or 97% identity to the nucleotide sequence of SEQ ID NO: 51, and/or (ii) encodes an amino acid sequence comprising the amino acid sequence of SEQ ID NO: 49, or an amino acid sequence having at least 99.5%, 99%, 98.5%, 98%, 98.5% or 97% identity to the amino acid sequence of SEQ ID NO: 49. In one embodiment, RNA encoding a vaccine antigen is nucleoside modified messenger RNA (modRNA) and (i) comprises the nucleotide sequence of SEQ ID NO: 51; and/or (ii) encodes an amino acid sequence comprising the amino acid sequence of SEQ ID NO: 49.

In one embodiment, RNA encoding a vaccine antigen is nucleoside modified messenger RNA (modRNA) and (i) comprises the nucleotide sequence of SEQ ID NO: 57, a nucleotide sequence having at least 99.5%, 99%, 98.5%, 98%, 98.5% or 97% identity to the nucleotide sequence of SEQ ID NO: 57, and/or (ii) encodes an amino acid sequence comprising the amino acid sequence of SEQ ID NO: 55, or an amino acid sequence having at least 99.5%, 99%, 98.5%, 98%, 98.5% or 97% identity to the amino acid sequence of SEQ ID NO: 55. In one embodiment, RNA encoding a vaccine antigen is nucleoside modified messenger RNA (modRNA) and (i) comprises the nucleotide sequence of SEQ ID NO: 57; and/or (ii) encodes an amino acid sequence comprising the amino acid sequence of SEQ ID NO: 55.

In one embodiment, RNA encoding a vaccine antigen is nucleoside modified messenger RNA (modRNA) and (i) comprises the nucleotide sequence of SEQ ID NO: 60, a nucleotide sequence having at least 99.5%, 99%, 98.5%, 98%, 98.5% or 97% identity to the nucleotide sequence of SEQ ID NO: 60, and/or (ii) encodes an amino acid sequence comprising the amino acid sequence of SEQ ID NO: 58, or an amino acid sequence having at least 99.5%, 99%, 98.5%, 98%, 98.5% or 97% identity to the amino acid sequence of SEQ ID NO: 58. In one embodiment, RNA encoding a vaccine antigen is nucleoside modified messenger RNA (modRNA) and (i) comprises the nucleotide sequence of SEQ ID NO: 60; and/or (ii) encodes an amino acid sequence comprising the amino acid sequence of SEQ ID NO: 58.

In one embodiment, RNA encoding a vaccine antigen is nucleoside modified messenger RNA (modRNA) and (i) comprises the nucleotide sequence of SEQ ID NO: 63a, a nucleotide sequence having at least 99.5%, 99%, 98.5%, 98%, 98.5% or 97% identity to the nucleotide sequence of SEQ ID NO: 63a, and/or (ii) encodes an amino acid sequence comprising the amino acid sequence of SEQ ID NO: 61, or an amino acid sequence having at least 99.5%, 99%, 98.5%, 98%, 98.5% or 97% identity to the amino acid sequence of SEQ ID NO: 61. In one embodiment, RNA encoding a vaccine antigen is nucleoside modified messenger RNA (modRNA) and (i) comprises the nucleotide sequence of SEQ ID NO: 63a; and/or (ii) encodes an amino acid sequence comprising the amino acid sequence of SEQ ID NO: 61.

In one embodiment, RNA encoding a vaccine antigen is nucleoside modified messenger RNA (modRNA) and (i) comprises the nucleotide sequence of SEQ ID NO: 53, a nucleotide sequence having at least 99.5%, 99%, 98.5%, 98%, 98.5% or 97% identity to the nucleotide sequence of SEQ ID NO: 53, and/or (ii) encodes an amino acid sequence comprising the amino acid sequence of SEQ ID NO: 52, or an amino acid sequence having at least 99.5%, 99%, 98.5%, 98%, 98.5% or 97% identity to the amino acid sequence of SEQ ID NO: 52. In one embodiment, RNA encoding a vaccine antigen is nucleoside modified messenger RNA (modRNA) and (i) comprises the nucleotide sequence of SEQ ID NO: 53; and/or (ii) encodes an amino acid sequence comprising the amino acid sequence of SEQ ID NO: 52.

In one embodiment, RNA encoding a vaccine antigen is nucleoside modified messenger RNA (modRNA) and (i) comprises the nucleotide sequence of SEQ ID NO: 54, a nucleotide sequence having at least 99.5%, 99%, 98.5%, 98%, 98.5% or 97% identity to the nucleotide sequence of SEQ ID NO: 54, and/or (ii) encodes an amino acid sequence comprising the amino acid sequence of SEQ ID NO: 52, or an amino acid sequence having at least 99.5%, 99%, 98.5%, 98%, 98.5% or 97% identity to the amino acid sequence of SEQ ID NO: 52. In one embodiment, RNA encoding a vaccine antigen is nucleoside modified messenger RNA (modRNA) and (i) comprises the nucleotide sequence of SEQ ID NO: 54; and/or (ii) encodes an amino acid sequence comprising the amino acid sequence of SEQ ID NO: 52.

As used herein, the term "vaccine" refers to a composition that induces an immune response upon inoculation into a subject. In some embodiments, the induced immune response provides protective immunity.

In one embodiment, the RNA encoding the antigen molecule is expressed in cells of the subject to provide the antigen molecule. In one embodiment, expression of the antigen molecule is at the cell surface or into the extracellular space. In one embodiment, the antigen molecule is presented in the context of MHC. In one embodiment, the RNA encoding the antigen molecule is transiently expressed in cells of the subject. In one embodiment, after administration of the RNA encoding the antigen molecule, in particular after intramuscular administration of the RNA encoding the antigen molecule, expression of the RNA encoding the antigen molecule in muscle occurs. In one embodiment, after administration of the RNA encoding the antigen molecule, expression of the RNA encoding the antigen molecule in spleen occurs. In one embodiment, after administration of the RNA encoding the antigen molecule, expression of the RNA encoding the antigen molecule in antigen presenting cells, preferably professional antigen presenting cells occurs. In one embodiment, the antigen presenting cells are selected from the group consisting of dendritic cells, macrophages and B cells. In one embodiment, after administration of the RNA encoding the antigen molecule, no or essentially no expression of the RNA encoding the antigen molecule in lung and/or liver occurs. In one embodiment, after administration of the RNA encoding the antigen molecule, expression of the RNA encoding the antigen molecule in spleen is at least 5-fold the amount of expression in lung. In some embodiments, the methods and agents, e.g., RNA compositions, described herein following administration, in particular following intramuscular administration, to a subject result in delivery of the RNA encoding a vaccine antigen to lymph nodes and/or spleen. In some embodiments, RNA encoding a vaccine antigen is detectable in lymph nodes and/or spleen 6 hours or later following administration and preferably up to 6 days or longer.

In some embodiments, the methods and agents, e.g., mRNA compositions, described herein following administration, in particular following intramuscular administration, to a subject result in delivery of the RNA encoding a vaccine antigen to B cell follicles, subcapsular sinus, and/or T cell zone, in particular B cell follicles and/or subcapsular sinus of lymph nodes.

In some embodiments, the methods and agents, e.g., mRNA compositions, described herein following administration, in particular following intramuscular administration, to a subject result in delivery of the RNA encoding a vaccine antigen to B cells (CD19+), subcapsular sinus macrophages (CD169+) and/or dendritic cells (CD11c+) in the T cell zone and intermediary sinus of lymph nodes, in particular to B cells (CD19+) and/or subcapsular sinus macrophages (CD169+) of lymph nodes.

In some embodiments, the methods and agents, e.g., mRNA compositions, described herein following administration, in particular following intramuscular administration, to a subject result in delivery of the RNA encoding a vaccine antigen to white pulp of spleen.

In some embodiments, the methods and agents, e.g., mRNA compositions, described herein following administration, in particular following intramuscular administration, to a subject result in delivery of the RNA encoding a vaccine antigen to B cells, DCs (CD11c+), in particular those surrounding the B cells, and/or macrophages of spleen, in particular to B cells and/or DCs (CD11c+).

In one embodiment, the vaccine antigen is expressed in lymph node and/or spleen, in particular in the cells of lymph node and/or spleen described above.

The peptide and protein antigens suitable for use according to the present disclosure typically include a peptide or protein comprising an epitope of SARS-CoV-2 S protein or a functional variant thereof for inducing an immune response. The peptide or protein or epitope may be derived from a target antigen, i.e. the antigen against which an immune response is to be elicited. For example, the peptide or protein antigen or the epitope contained within the peptide or protein antigen may be a target antigen or a fragment or variant of a target antigen. The target antigen may be a coronavirus S protein, in particular SARS-CoV-2 S protein.

The antigen molecule or a procession product thereof, e.g., a fragment thereof, may bind to an antigen receptor such as a BCR or TCR carried by immune effector cells, or to antibodies.

A peptide and protein antigen which is provided to a subject according to the present disclosure by administering RNA encoding the peptide and protein antigen, i.e., a vaccine antigen, preferably results in the induction of an immune response, e.g., a humoral and/or cellular immune response in the subject being provided the peptide or protein antigen. Said immune response is preferably directed against a target antigen, in particular coronavirus S protein, in particular SARS-CoV-2 S protein. Thus, a vaccine antigen may comprise the target antigen, a variant thereof, or a fragment thereof. In one embodiment, such fragment or variant is immunologically equivalent to the target antigen. In the context of the present disclosure, the term "fragment of an antigen" or "variant of an antigen" means an agent which results in the induction of an immune response which immune response targets the antigen, i.e. a target antigen. Thus, the vaccine antigen may correspond to or may comprise the target antigen, may correspond to or may comprise a fragment of the target antigen or may correspond to or may comprise an antigen which is homologous to the target antigen or a fragment thereof. Thus, according to the present disclosure, a vaccine antigen may comprise an immunogenic fragment of a target antigen or an amino acid sequence being homologous to an immunogenic fragment of a target antigen. An "immunogenic fragment of an antigen" according to the present disclosure preferably relates to a fragment of an antigen which is capable of inducing an immune response against the target antigen. The vaccine antigen may be a recombinant antigen.

The term "immunologically equivalent" means that the immunologically equivalent molecule such as the immunologically equivalent amino acid sequence exhibits the same or essentially the same immunological properties and/or exerts the same or essentially the same immunological effects, e.g., with respect to the type of the immunological effect. In the context of the present disclosure, the term "immunologically equivalent" is preferably used with respect to the immunological effects or properties of antigens or antigen variants used for immunization. For example, an amino acid sequence is immunologically equivalent to a reference amino acid sequence if said amino acid sequence when exposed to the immune system of a subject induces an immune reaction having a specificity of reacting with the reference amino acid sequence.

"Activation" or "stimulation", as used herein, refers to the state of an immune effector cell such as T cell that has been sufficiently stimulated to induce detectable cellular proliferation. Activation can also be associated with initiation of signaling pathways, induced cytokine production, and detectable effector functions. The term "activated immune effector cells" refers to, among other things, immune effector cells that are undergoing cell division.

The term "priming" refers to a process wherein an immune effector cell such as a T cell has its first contact with its specific antigen and causes differentiation into effector cells such as effector T cells.

The term "clonal expansion" or "expansion" refers to a process wherein a specific entity is multiplied. In the context of the present disclosure, the term is preferably used in the context of an immunological response in which immune effector cells are stimulated by an antigen, proliferate, and the specific immune effector cell recognizing said antigen is amplified. Preferably, clonal expansion leads to differentiation of the immune effector cells.

The term "antigen" relates to an agent comprising an epitope against which an immune response can be generated. The term "antigen" includes, in particular, proteins and peptides. In one embodiment, an antigen is presented by cells of the immune system such as antigen presenting cells like dendritic cells or macrophages. An antigen or a procession product thereof such as a T-cell epitope is in one embodiment bound by a T- or B-cell receptor, or by an immunoglobulin molecule such as an antibody. Accordingly, an antigen or a procession product thereof may react specifically with antibodies or T lymphocytes (T cells). In one embodiment, an antigen is a viral antigen, such as a coronavirus S protein, e.g., SARS-CoV-2 S protein, and an epitope is derived from such antigen.

The term "viral antigen" refers to any viral component having antigenic properties, i.e. being able to provoke an immune response in an individual. The viral antigen may be coronavirus S protein, e.g., SARS-CoV-2 S protein.

The term "expressed on the cell surface" or "associated with the cell surface" means that a molecule such as an antigen is associated with and located at the plasma membrane of a cell, wherein at least a part of the molecule faces the extracellular space of said cell and is accessible from the outside of said cell, e.g., by antibodies located outside the cell. In this context, a part is preferably at least 4, preferably at least 8, preferably at least 12, more preferably at least 20 amino acids. The association may be direct or indirect. For example, the association may be by one or more transmembrane domains, one or more lipid anchors, or by the interaction with any other protein, lipid, saccharide, or other structure that can be found on the outer leaflet of the plasma membrane of a cell. For example, a molecule associated with the surface of a cell may be a transmembrane protein having an extracellular portion or may be a protein associated with the surface of a cell by interacting with another protein that is a transmembrane protein.

"Cell surface" or "surface of a cell" is used in accordance with its normal meaning in the art, and thus includes the outside of the cell which is accessible to binding by proteins and other molecules. An antigen is expressed on the surface of cells if it is located at the surface of said cells and is accessible to binding by e.g. antigen-specific antibodies added to the cells.

The term "extracellular portion" or "exodomain" in the context of the present disclosure refers to a part of a molecule such as a protein that is facing the extracellular space of a cell and preferably is accessible from the outside of said cell, e.g., by binding molecules such as antibodies located outside the cell. Preferably, the term refers to one or more extracellular loops or domains or a fragment thereof.

The term "epitope" refers to a part or fragment of a molecule such as an antigen that is recognized by the immune system. For example, the epitope may be recognized by T cells, B cells or antibodies. An epitope of an antigen may include a continuous or discontinuous portion of the antigen and may be between about 5 and about 100, such as between about 5 and about 50, more preferably between about 8 and about 30, most preferably between about 8 and about 25 amino acids in length, for example, the epitope may be preferably 9, 10, 11, 12, 13, 14, 15, 16, 17, 18, 19, 20, 21, 22, 23, 24, or 25 amino acids in length. In one embodiment, an epitope is between about 10 and about 25 amino acids in length. The term "epitope" includes T cell epitopes.

The term "T cell epitope" refers to a part or fragment of a protein that is recognized by a T cell when presented in the context of MHC molecules. The term "major histocompatibility complex" and the abbreviation "MHC" includes MHC class I and MHC class II molecules and relates to a complex of genes which is present in all vertebrates. MHC proteins or molecules are important for signaling between lymphocytes and antigen presenting cells or diseased cells in immune reactions, wherein the MHC proteins or molecules bind peptide epitopes and present them for recognition by T cell receptors on T cells. The proteins encoded by the MHC are expressed on the surface of cells, and display both self-antigens (peptide fragments from the cell itself) and non-self-antigens (e.g., fragments of invading microorganisms) to a T cell. In the case of class I MHC/peptide complexes, the binding peptides are typically about 8 to about 10 amino acids long although longer or shorter peptides may be effective. In the case of class II MHC/peptide complexes, the binding peptides are typically about 10 to about 25 amino acids long and are in particular about 13 to about 18 amino acids long, whereas longer and shorter peptides may be effective.

The peptide and protein antigen can be 2-100 amino acids, including for example, 5 amino acids, 10 amino acids, 15 amino acids, 20 amino acids, 25 amino acids, 30 amino acids, 35 amino acids, 40 amino acids, 45 amino acids, or 50 amino acids in length. In some embodiments, a peptide can be greater than 50 amino acids. In some embodiments, the peptide can be greater than 100 amino acids.

The peptide or protein antigen can be any peptide or protein that can induce or increase the ability of the immune system to develop antibodies and T cell responses to the peptide or protein.

In one embodiment, vaccine antigen is recognized by an immune effector cell. Preferably, the vaccine antigen if recognized by an immune effector cell is able to induce in the presence of appropriate co-stimulatory signals, stimulation, priming and/or expansion of the immune effector cell carrying an antigen receptor recognizing the vaccine antigen. In the context of the embodiments of the present disclosure, the vaccine antigen is preferably presented or present on the surface of a cell, preferably an antigen presenting cell. In one embodiment, an antigen is presented by a diseased cell such as a virus-infected cell. In one embodiment, an antigen receptor is a TCR which binds to an epitope of an antigen presented in the context of MHC. In one embodiment, binding of a TCR when expressed by T cells and/or present on T cells to an antigen presented by cells such as antigen presenting cells results in stimulation, priming and/or expansion of said T cells. In one embodiment, binding of a TCR when expressed by T cells and/or present on T cells to an antigen presented on diseased cells results in cytolysis and/or apoptosis of the diseased cells, wherein said T cells preferably release cytotoxic factors, e.g. perforins and granzymes.

In one embodiment, an antigen receptor is an antibody or B cell receptor which binds to an epitope in an antigen. In one embodiment, an antibody or B cell receptor binds to native epitopes of an antigen.

### Nucleic acids

The term "polynucleotide" or "nucleic acid", as used herein, is intended to include DNA and RNA such as genomic DNA, cDNA, mRNA, recombinantly produced and chemically synthesized molecules. A nucleic acid may be single-stranded or double-stranded. RNA includes *in vitro* transcribed RNA (IVT RNA) or synthetic RNA. According to the present disclosure, a polynucleotide is preferably isolated.

Nucleic acids may be comprised in a vector. The term "vector" as used herein includes any vectors known to the skilled person including plasmid vectors, cosmid vectors, phage vectors such as lambda phage, viral vectors such as retroviral, adenoviral or baculoviral vectors, or artificial chromosome vectors such as bacterial artificial chromosomes (BAC), yeast artificial chromosomes (YAC), or P1 artificial chromosomes (PAC). Said vectors include expression as well as cloning vectors. Expression vectors comprise plasmids as well as viral vectors and generally contain a desired coding sequence and appropriate DNA sequences necessary for the expression of the operably linked coding sequence in a particular host organism (e.g., bacteria, yeast, plant, insect, or mammal) or in *in vitro* expression systems. Cloning vectors are generally used to engineer and amplify a certain desired DNA fragment and may lack functional sequences needed for expression of the desired DNA fragments.

In one embodiment of all aspects of the present disclosure, the RNA encoding the vaccine antigen is expressed in cells such as antigen presenting cells of the subject treated to provide the vaccine antigen.

The nucleic acids described herein may be recombinant and/or isolated molecules.

In the present disclosure, the term "RNA" relates to a nucleic acid molecule which includes ribonucleotide residues. In preferred embodiments, the RNA contains all or a majority of ribonucleotide residues. As used herein, "ribonucleotide" refers to a nucleotide with a hydroxyl group at the 2'-position of a β-D-ribofuranosyl group. RNA encompasses without limitation, double stranded RNA, single stranded RNA, isolated RNA such as partially purified RNA, essentially pure RNA, synthetic RNA, recombinantly produced RNA, as well as modified RNA that differs from naturally occurring RNA by the addition, deletion, substitution and/or alteration of one or more nucleotides. Such alterations may refer to addition of non-nucleotide material to internal RNA nucleotides or to the end(s) of RNA. It is also contemplated herein that nucleotides in RNA may be non-standard nucleotides, such as chemically synthesized nucleotides or deoxynucleotides. For the present disclosure, these altered RNAs are considered analogs of naturally-occurring RNA.

In certain embodiments of the present disclosure, the RNA is messenger RNA (mRNA) that relates to a RNA transcript which encodes a peptide or protein. As established in the art, mRNA generally contains a 5' untranslated region (5'-UTR), a peptide coding region and a 3' untranslated region (3'-UTR). In some embodiments, the RNA is produced by *in vitro* transcription or chemical synthesis. In one embodiment, the mRNA is produced by *in vitro* transcription using a DNA template where DNA refers to a nucleic acid that contains deoxyribonucleotides.

In one embodiment, RNA is *in vitro* transcribed RNA (IVT-RNA) and may be obtained by *in vitro* transcription of an appropriate DNA template. The promoter for controlling transcription can be any promoter for any RNA polymerase. A DNA template for *in vitro* transcription may be obtained by cloning of a nucleic acid, in particular cDNA, and introducing it into an appropriate vector for *in vitro* transcription. The cDNA may be obtained by reverse transcription of RNA. In certain embodiments of the present disclosure, the RNA is "replicon RNA" or simply a "replicon", in particular "self-replicating RNA" or "self-amplifying RNA". In one particularly preferred embodiment, the replicon or self-replicating RNA is derived from or comprises elements derived from a ssRNA virus, in particular a positive-stranded ssRNA virus such as an alphavirus. Alphaviruses are typical representatives of positive-stranded RNA viruses. Alphaviruses replicate in the cytoplasm of infected cells (for review of the alphaviral life cycle see José et al., Future Microbiol., 2009, vol. 4, pp. 837-856). The total genome length of many alphaviruses typically ranges between 11,000 and 12,000 nucleotides, and the genomic RNA typically has a 5'-cap, and a 3' poly(A) tail. The genome of alphaviruses encodes non-structural proteins (involved in transcription, modification and replication of viral RNA and in protein modification) and structural proteins (forming the virus particle). There are typically two open reading frames (ORFs) in the genome. The four non-structural proteins (nsP1-nsP4) are typically encoded together by a first ORF beginning near the 5' terminus of the genome, while alphavirus structural proteins are encoded together by a second ORF which is found downstream of the first ORF and extends near the 3' terminus of the genome. Typically, the first ORF is larger than the second ORF, the ratio being roughly 2:1. In cells infected by an alphavirus, only the nucleic acid sequence encoding non-structural proteins is translated from the genomic RNA, while the genetic information encoding structural proteins is translatable from a subgenomic transcript, which is an RNA molecule that resembles eukaryotic messenger RNA (mRNA; Gould et al., 2010, Antiviral Res., vol. 87 pp. 111-124). Following infection, i.e. at early stages of the viral life cycle, the (+) stranded genomic RNA directly acts like a messenger RNA for the translation of the open reading frame encoding the non-structural poly-protein (nsP1234). Alphavirus-derived vectors have been proposed for delivery of foreign genetic information into target cells or target organisms. In simple approaches, the open reading frame encoding alphaviral structural proteins is replaced by an open reading frame encoding a protein of interest. Alphavirus-based trans-replication systems rely on alphavirus nucleotide sequence elements on two separate nucleic acid molecules: one nucleic acid molecule encodes a viral replicase, and the other nucleic acid molecule is capable of being replicated by said replicase in trans (hence the designation trans-replication system). Trans-replication requires the presence of both these nucleic acid molecules in a given host cell. The nucleic acid molecule capable of being replicated by the replicase in trans must comprise certain alphaviral sequence elements to allow recognition and RNA synthesis by the alphaviral replicase.

In one embodiment, the RNA described herein may have modified nucleosides. In some embodiments, the RNA comprises a modified nucleoside in place of at least one (e.g., every) uridine.

The term "uracil," as used herein, describes one of the nucleobases that can occur in the nucleic acid of RNA. The structure of uracil is:

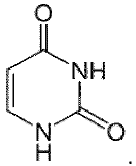

The term "uridine," as used herein, describes one of the nucleosides that can occur in RNA. The structure of uridine is:

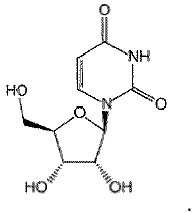

UTP (uridine 5'-triphosphate) has the following structure:

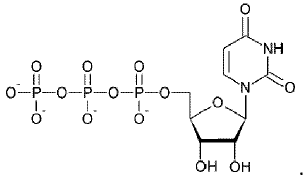

Pseudo-UTP (pseudouridine 5'-triphosphate) has the following structure:

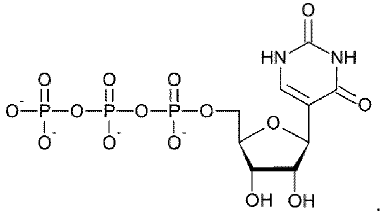

"Pseudouridine" is one example of a modified nucleoside that is an isomer of uridine, where the uracil is attached to the pentose ring via a carbon-carbon bond instead of a nitrogen-carbon glycosidic bond.

Another exemplary modified nucleoside is N1-methyl-pseudouridine (m1Ψ), which has the structure:

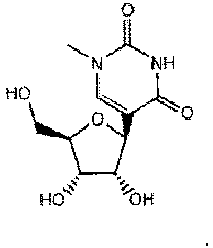

N1-methyl-pseudo-UTP has the following structure:

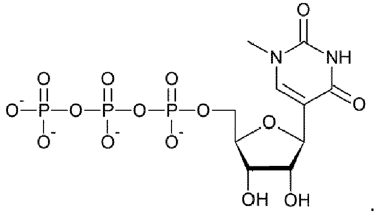

Another exemplary modified nucleoside is 5-methyl-uridine (m5U), which has the structure:

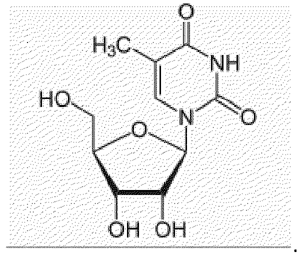

In some embodiments, one or more uridine in the RNA described herein is replaced by a modified nucleoside. In some embodiments, the modified nucleoside is a modified uridine. In some embodiments, RNA comprises a modified nucleoside in place of at least one uridine. In some embodiments, RNA comprises a modified nucleoside in place of each uridine.

In some embodiments, the modified nucleoside is independently selected from pseudouridine (ψ), N1-methyl-pseudouridine (m1ψ), and 5-methyl-uridine (m5U). In some embodiments, the modified nucleoside comprises pseudouridine (ψ). In some embodiments, the modified nucleoside comprises N1-methyl-pseudouridine (m1ψ). In some embodiments, the modified nucleoside comprises 5-methyl-uridine (m5U). In some embodiments, RNA may comprise more than one type of modified nucleoside, and the modified nucleosides are independently selected from pseudouridine (ψ), N1-methyl-pseudouridine (m1ψ), and 5-methyl-uridine (m5U). In some embodiments, the modified nucleosides comprise pseudouridine (ψ) and N1-methyl-pseudouridine (m1ψ). In some embodiments, the modified nucleosides comprise pseudouridine (ψ) and 5-methyl-uridine (m5U). In some embodiments, the modified nucleosides comprise N1-methyl-pseudouridine (m1ψ) and 5-methyl-uridine (m5U). In some embodiments, the modified nucleosides comprise pseudouridine (ψ), N1-methyl-pseudouridine (m1ψ), and 5-methyl-uridine (m5U).

In some embodiments, the modified nucleoside replacing one or more, e.g., all, uridine in the RNA may be any one or more of 3-methyl-uridine (m³U), 5-methoxy-uridine (mo⁵U), 5-aza-uridine, 6-aza-uridine, 2-thio-5-aza-uridine, 2-thio-uridine (s²U), 4-thio-uridine (s⁴U), 4-thio-pseudouridine, 2-thio-pseudouridine, 5-hydroxy-uridine (ho^{S}U), 5-aminoallyl-uridine, 5-halo-uridine (e.g., 5-iodo-uridine or 5-bromo-uridine), uridine 5-oxyacetic acid (cmo⁵U), uridine 5-oxyacetic acid methyl ester (mcmo⁵U), 5-carboxymethyl-uridine (cm⁵U), 1-carboxymethyl-pseudouridine, 5-carboxyhydroxymethyl-uridine (chm⁵U), 5-carboxyhydroxymethyl-uridine methyl ester (mchm⁵U), 5-methoxycarbonylmethyl-uridine (mcm⁵U), 5-methoxycarbonylmethyl-2-thio-uridine (mcm⁵s²U), 5-aminomethyl-2-thio-uridine (nm⁵s²U), 5-methylaminomethyl-uridine (mnm⁵U), 1-ethyl-pseudouridine, 5-methylaminomethyl-2-thio-uridine (mnm⁵s²U), 5-methylaminomethyl-2-seleno-uridine (mnm⁵se²U), 5-carbamoylmethyl-uridine (ncm⁵U), 5-carboxymethylaminomethyl-uridine (cmnm⁵U), 5-carboxymethylaminomethyl-2-thio-uridine (cmnm⁵s²U), 5-propynyl-uridine, 1-propynyl-pseudouridine, 5-taurinomethyl-uridine (τm⁵U), 1-taurinomethyl-pseudouridine, 5-taurinomethyl-2-thio-uridine(τm5s2U), 1-taurinomethyl-4-thio-pseudouridine), 5-methyl-2-thio-uridine (m⁵s²U), 1-methyl-4-thio-pseudouridine (m¹s⁴ψ), 4-thio-1-methyl-pseudouridine, 3-methyl-pseudouridine (m³ψ), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine (m⁵D), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine (acp³U), 1-methyl-3-(3-amino-3-carboxypropyl)pseudouridine (acp³ ψ), 5-(isopentenylaminomethyl)uridine (inm⁵U), 5-(isopentenylaminomethyl)-2-thio-uridine (inm⁵s²U), α-thio-uridine, 2'-O-methyl-uridine (Um), 5,2'-O-dimethyl-uridine (m⁵Um), 2'-O-methyl-pseudouridine (ψm), 2-thio-2'-O-methyl-uridine (s²Um), 5-methoxycarbonylmethyl-2'-O-methyl-uridine (mcm⁵Um), 5-carbamoylmethyl-2'-O-methyl-uridine (ncm⁵Um), 5-carboxymethylaminomethyl-2'-O-methyl-uridine (cmnm⁵Um), 3,2'-O-dimethyl-uridine (m³Um), 5-(isopentenylaminomethyl)-2'-O-methyl-uridine (inm⁵Um), 1-thio-uridine, deoxythymidine, 2'-F-ara-uridine, 2'-F-uridine, 2'-OH-ara-uridine, 5-(2-carbomethoxyvinyl) uridine, 5-[3-(1-E-propenylamino)uridine, or any other modified uridine known in the art.

In one embodiment, the RNA comprises other modified nucleosides or comprises further modified nucleosides, e.g., modified cytidine. For example, in one embodiment, in the RNA 5-methylcytidine is substituted partially or completely, preferably completely, for cytidine. In one embodiment, the RNA comprises 5-methylcytidine and one or more selected from pseudouridine (ψ), N1-methyl-pseudouridine (m1ψ), and 5-methyl-uridine (m5U). In one embodiment, the RNA comprises 5-methylcytidine and N1-methyl-pseudouridine (m1ψ). In some embodiments, the RNA comprises 5-methylcytidine in place of each cytidine and N1-methyl-pseudouridine (m1ψ) in place of each uridine.

In some embodiments, the RNA according to the present disclosure comprises a 5'-cap. In one embodiment, the RNA of the present disclosure does not have uncapped 5'-triphosphates. In one embodiment, the RNA may be modified by a 5'- cap analog. The term "5'-cap" refers to a structure found on the 5'-end of an mRNA molecule and generally consists of a guanosine nucleotide connected to the mRNA via a 5'- to 5'-triphosphate linkage. In one embodiment, this guanosine is methylated at the 7-position. Providing an RNA with a 5'-cap or 5'-cap analog may be achieved by *in vitro* transcription, in which the 5'-cap is co-transcriptionally expressed into the RNA strand, or may be attached to RNA post-transcriptionally using capping enzymes. In some embodiments, the RNA (e.g., mRNA) comprises a cap0, cap1, or cap2, preferably cap1 or cap2, more preferably cap1. According to the present disclosure, the term "cap0" comprises the structure "m⁷GpppN", wherein N is any nucleoside bearing an OH moiety at position 2'. According to the present disclosure, the term "cap1" comprises the structure "m⁷GpppNm", wherein Nm is any nucleoside bearing an OCH₃ moiety at position 2'. According to the present disclosure, the term "cap2" comprises the structure "m⁷GpppNmNm", wherein each Nm is independently any nucleoside bearing an OCH₃ moiety at position 2'.

In some embodiments, the building block cap for RNA is m₂^{7,3'O}Gppp(m₁^{2'-O})ApG (also sometimes referred to as m₂^{7,3'O}G(5')ppp(5')m^{2'-O}ApG), which has the following structure:

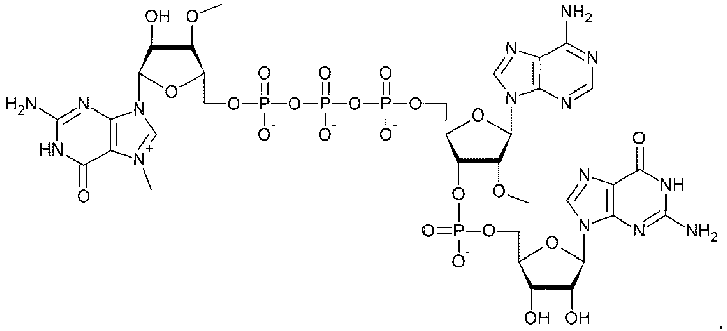

Below is an exemplary Cap1 RNA, which comprises RNA and m₂^{7,3'0}G(5')ppp(5')m^{2'-O}ApG:

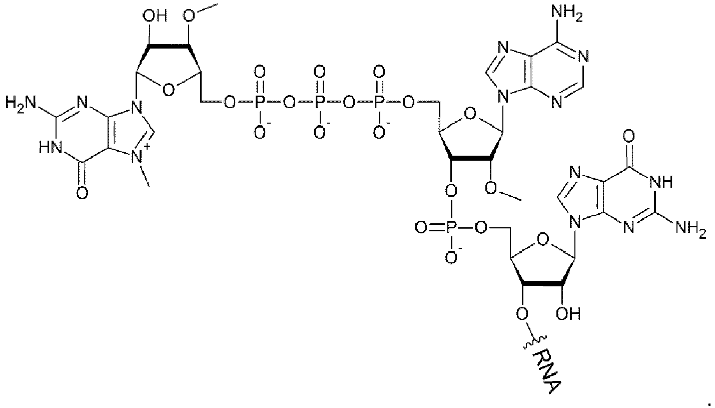

Below is another exemplary Cap1 RNA (no cap analog):

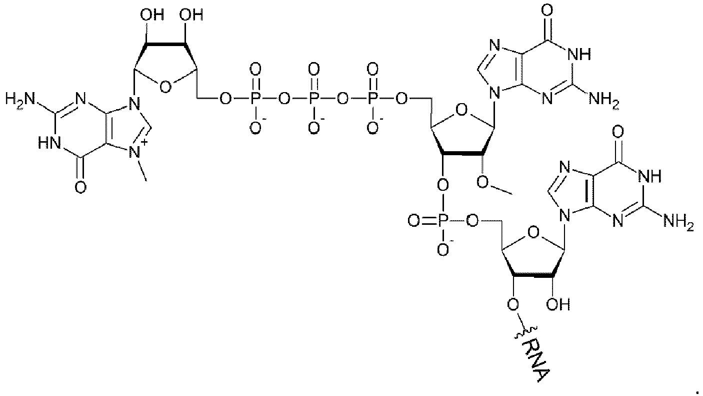

In some embodiments, the RNA is modified with "CapO" structures using, in one embodiment, the cap analog anti-reverse cap (ARCA Cap (m₂^{7,3'O}G(5')ppp(5')G)) with the structure:

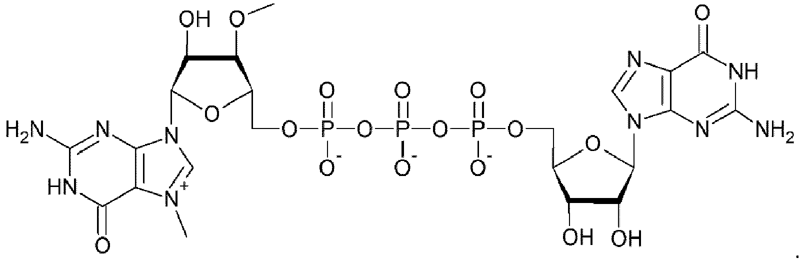

Below is an exemplary Cap0 RNA comprising RNA and m₂^{7,3'O}G(5')ppp(5')G:

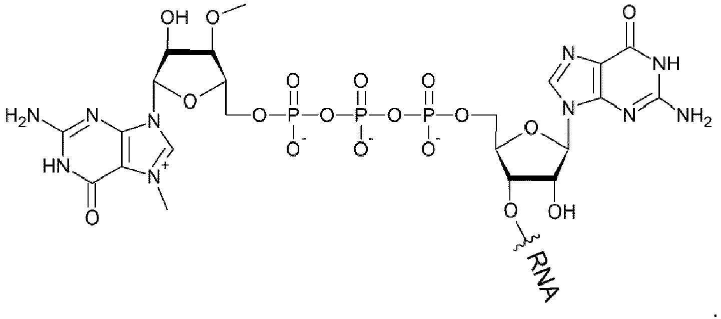

In some embodiments, the "CapO" structures are generated using the cap analog Beta-S-ARCA (m₂^{7,2'O}G(5')ppSp(5')G) with the structure:

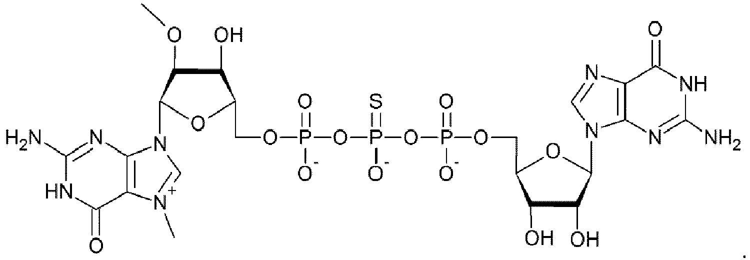

Below is an exemplary Cap0 RNA comprising Beta-S-ARCA (m₂^{7,2'O}G(5')ppSp(5')G) and RNA:

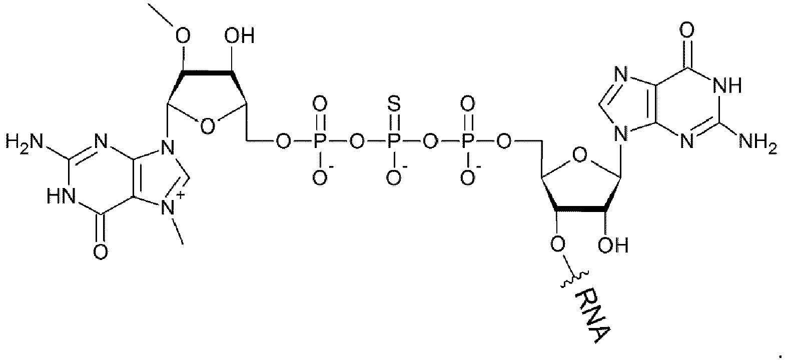

The "D1" diastereomer of beta-S-ARCA or "beta-S-ARCA(D1)" is the diastereomer of beta-S-ARCA which elutes first on an HPLC column compared to the D2 diastereomer of beta-S-ARCA (beta-S-ARCA(D2)) and thus exhibits a shorter retention time (cf., WO 2011/015347, herein incorporated by reference).

A particularly preferred cap is beta-S-ARCA(D1) (m₂^{7,2'-O}GppSpG) or m₂^{7,3'-O}Gppp(m₁^{2'-O})ApG.

In some embodiments, RNA according to the present disclosure comprises a 5'-UTR and/or a 3'-UTR. The term "untranslated region" or "UTR" relates to a region in a DNA molecule which is transcribed but is not translated into an amino acid sequence, or to the corresponding region in an RNA molecule, such as an mRNA molecule. An untranslated region (UTR) can be present 5' (upstream) of an open reading frame (5'-UTR) and/or 3' (downstream) of an open reading frame (3'-UTR). A 5'-UTR, if present, is located at the 5' end, upstream of the start codon of a protein-encoding region. A 5'-UTR is downstream of the 5'-cap (if present), e.g. directly adjacent to the 5'-cap. A 3'-UTR, if present, is located at the 3' end, downstream of the termination codon of a protein-encoding region, but the term "3'-UTR" does preferably not include the poly(A) sequence. Thus, the 3'-UTR is upstream of the poly(A) sequence (if present), e.g. directly adjacent to the poly(A) sequence.

In some embodiments, RNA comprises a 5'-UTR comprising the nucleotide sequence of SEQ ID NO: 12, or a nucleotide sequence having at least 99%, 98%, 97%, 96%, 95%, 90%, 85%, or 80% identity to the nucleotide sequence of SEQ ID NO: 12.

In some embodiments, RNA comprises a 3'-UTR comprising the nucleotide sequence of SEQ ID NO: 13, or a nucleotide sequence having at least 99%, 98%, 97%, 96%, 95%, 90%, 85%, or 80% identity to the nucleotide sequence of SEQ ID NO: 13.

A particularly preferred 5'-UTR comprises the nucleotide sequence of SEQ ID NO: 12. A particularly preferred 3'-UTR comprises the nucleotide sequence of SEQ ID NO: 13.

In some embodiments, the RNA according to the present disclosure comprises a 3'-poly(A) sequence.

As used herein, the term "poly(A) sequence" or "poly-A tail" refers to an uninterrupted or interrupted sequence of adenylate residues which is typically located at the 3'-end of an RNA molecule. Poly(A) sequences are known to those of skill in the art and may follow the 3'-UTR in the RNAs described herein. An uninterrupted poly(A) sequence is characterized by consecutive adenylate residues. In nature, an uninterrupted poly(A) sequence is typical. RNAs disclosed herein can have a poly(A) sequence attached to the free 3'-end of the RNA by a template-independent RNA polymerase after transcription or a poly(A) sequence encoded by DNA and transcribed by a template-dependent RNA polymerase.

It has been demonstrated that a poly(A) sequence of about 120 A nucleotides has a beneficial influence on the levels of RNA in transfected eukaryotic cells, as well as on the levels of protein that is translated from an open reading frame that is present upstream (5') of the poly(A) sequence (Holtkamp et al., 2006, Blood, vol. 108, pp. 4009-4017).

The poly(A) sequence may be of any length. In some embodiments, a poly(A) sequence comprises, essentially consists of, or consists of at least 20, at least 30, at least 40, at least 80, or at least 100 and up to 500, up to 400, up to 300, up to 200, or up to 150 A nucleotides, and, in particular, about 120 A nucleotides. In this context, "essentially consists of" means that most nucleotides in the poly(A) sequence, typically at least 75%, at least 80%, at least 85%, at least 90%, at least 95%, at least 96%, at least 97%, at least 98%, or at least 99% by number of nucleotides in the poly(A) sequence are A nucleotides, but permits that remaining nucleotides are nucleotides other than A nucleotides, such as U nucleotides (uridylate), G nucleotides (guanylate), or C nucleotides (cytidylate). In this context, "consists of" means that all nucleotides in the poly(A) sequence, i.e., 100% by number of nucleotides in the poly(A) sequence, are A nucleotides. The term "A nucleotide" or "A" refers to adenylate.

In some embodiments, a poly(A) sequence is attached during RNA transcription, e.g., during preparation of *in vitro* transcribed RNA, based on a DNA template comprising repeated dT nucleotides (deoxythymidylate) in the strand complementary to the coding strand. The DNA sequence encoding a poly(A) sequence (coding strand) is referred to as poly(A) cassette.

In some embodiments, the poly(A) cassette present in the coding strand of DNA essentially consists of dA nucleotides, but is interrupted by a random sequence of the four nucleotides (dA, dC, dG, and dT). Such random sequence may be 5 to 50, 10 to 30, or 10 to 20 nucleotides in length. Such a cassette is disclosed in WO 2016/005324 A1, hereby incorporated by reference. Any poly(A) cassette disclosed in WO 2016/005324 A1 may be used in the present disclosure. A poly(A) cassette that essentially consists of dA nucleotides, but is interrupted by a random sequence having an equal distribution of the four nucleotides (dA, dC, dG, dT) and having a length of e.g., 5 to 50 nucleotides shows, on DNA level, constant propagation of plasmid DNA in *E. coli* and is still associated, on RNA level, with the beneficial properties with respect to supporting RNA stability and translational efficiency is encompassed. Consequently, in some embodiments, the poly(A) sequence contained in an RNA molecule described herein essentially consists of A nucleotides, but is interrupted by a random sequence of the four nucleotides (A, C, G, U). Such random sequence may be 5 to 50, 10 to 30, or 10 to 20 nucleotides in length.

In some embodiments, no nucleotides other than A nucleotides flank a poly(A) sequence at its 3'-end, i.e., the poly(A) sequence is not masked or followed at its 3'-end by a nucleotide other than A.

In some embodiments, the poly(A) sequence may comprise at least 20, at least 30, at least 40, at least 80, or at least 100 and up to 500, up to 400, up to 300, up to 200, or up to 150 nucleotides. In some embodiments, the poly(A) sequence may essentially consist of at least 20, at least 30, at least 40, at least 80, or at least 100 and up to 500, up to 400, up to 300, up to 200, or up to 150 nucleotides. In some embodiments, the poly(A) sequence may consist of at least 20, at least 30, at least 40, at least 80, or at least 100 and up to 500, up to 400, up to 300, up to 200, or up to 150 nucleotides. In some embodiments, the poly(A) sequence comprises at least 100 nucleotides. In some embodiments, the poly(A) sequence comprises about 150 nucleotides. In some embodiments, the poly(A) sequence comprises about 120 nucleotides.

In some embodiments, a poly(A) sequence included in an RNA described herein is a interrupted poly(A) sequence, e.g., as described in WO2016/005324, the entire content of which is incorporated herein by reference for purposes described herein. In some embodiments, a poly(A) sequence comprises a stretch of at least 20 adenosine residues (including, e.g., at least 30, at least 40, at least 50, at least 60, at least 70, or more adenosine residues), followed by a linker sequence (e.g., in some embodiments comprising non-A nucleotides) and another stretch of at least 20 adenosine residues (including, e.g., at least 30, at least 40, at least 50, at least 60, at least 70, or more adenosine residues). In some embodiments, such a linker sequence may be 3-50 nucleotides in length, or 5-25 nucleotides in length, or 10-15 nucleotides in length. In some embodiments, a poly(A) sequence comprises a stretch of about 30 adenosine residues, followed by a linker sequence having a length of about 5-15 nucleoties (e.g., in some embodiments comprising non-A nucleotides) and another stretch of about 70 adenosine residues.

In some embodiments, RNA comprises a poly(A) sequence comprising the nucleotide sequence of SEQ ID NO: 14, or a nucleotide sequence having at least 99%, 98%, 97%, 96%, 95%, 90%, 85%, or 80% identity to the nucleotide sequence of SEQ ID NO: 14.

A particularly preferred poly(A) sequence comprises the nucleotide sequence of SEQ ID NO: 14.

According to the present disclosure, vaccine antigen is preferably administered as single-stranded, 5'-capped RNA (e.g., mRNA) that is translated into the respective protein upon entering cells of a subject being administered the RNA. Preferably, the RNA contains structural elements optimized for maximal efficacy of the RNA with respect to stability and translational efficiency (5'-cap, 5'-UTR, 3'-UTR, poly(A) sequence).

In one embodiment, beta-S-ARCA(D1) is utilized as specific capping structure at the 5'-end of the RNA. In one embodiment, m₂^{7,3'-O}Gppp(m₁^{2'-O})ApG is utilized as specific capping structure at the 5'-end of the RNA. In one embodiment, the 5'-UTR sequence is derived from the human alpha-globin mRNA and optionally has an optimized 'Kozak sequence' to increase translational efficiency. In one embodiment, a combination of two sequence elements (FI element) derived from the "amino terminal enhancer of split" (AES) mRNA (called F) and the mitochondrial encoded 12S ribosomal RNA (called I) are placed between the coding sequence and the poly(A) sequence to assure higher maximum protein levels and prolonged persistence of the RNA (e.g., mRNA). In one embodiment, two re-iterated 3'-UTRs derived from the human beta-globin mRNA are placed between the coding sequence and the poly(A) sequence to assure higher maximum protein levels and prolonged persistence of the RNA (e.g., mRNA). In one embodiment, a poly(A) sequence measuring 110 nucleotides in length, consisting of a stretch of 30 adenosine residues, followed by a linker sequence (e.g., 10 nucleotide linker sequence) and another 70 adenosine residues is used. This poly(A) sequence was designed to enhance RNA stability and translational efficiency.

In one embodiment of all aspects of the present disclosure, RNA encoding a vaccine antigen is expressed in cells of the subject treated to provide the vaccine antigen. In one embodiment of all aspects of the present disclosure, the RNA is transiently expressed in cells of the subject. In one embodiment of all aspects of the present disclosure, the RNA is *in vitro* transcribed RNA. In one embodiment of all aspects of the present disclosure, expression of the vaccine antigen is at the cell surface. In one embodiment of all aspects of the present disclosure, the vaccine antigen is expressed and presented in the context of MHC. In one embodiment of all aspects of the present disclosure, expression of the vaccine antigen is into the extracellular space, i.e., the vaccine antigen is secreted.

In the context of the present disclosure, the term "transcription" relates to a process, wherein the genetic code in a DNA sequence is transcribed into RNA. Subsequently, the RNA may be translated into peptide or protein.

According to the present disclosure, the term "transcription" comprises "*in vitro* transcription", wherein the term "*in vitro* transcription" relates to a process wherein RNA, in particular mRNA, is *in vitro* synthesized in a cell-free system, preferably using appropriate cell extracts. Preferably, cloning vectors are applied for the generation of transcripts. These cloning vectors are generally designated as transcription vectors and are according to the present disclosure encompassed by the term "vector". According to the present disclosure, the RNA used in the present disclosure preferably is *in vitro* transcribed RNA (IVT-RNA) and may be obtained by *in vitro* transcription of an appropriate DNA template. The promoter for controlling transcription can be any promoter for any RNA polymerase. Particular examples of RNA polymerases are the T7, T3, and SP6 RNA polymerases. Preferably, the *in vitro* transcription according to the disclosure is controlled by a T7 or SP6 promoter. A DNA template for *in vitro* transcription may be obtained by cloning of a nucleic acid, in particular cDNA, and introducing it into an appropriate vector for *in vitro* transcription. The cDNA may be obtained by reverse transcription of RNA.

With respect to RNA, the term "expression" or "translation" relates to the process in the ribosomes of a cell by which a strand of mRNA directs the assembly of a sequence of amino acids to make a peptide or protein.

In one embodiment, after administration of the RNA described herein, e.g., formulated as RNA lipid particles, at least a portion of the RNA is delivered to a target cell. In one embodiment, at least a portion of the RNA is delivered to the cytosol of the target cell. In one embodiment, the RNA is translated by the target cell to produce the peptide or protein it encodes. In one embodiment, the target cell is a spleen cell. In one embodiment, the target cell is an antigen presenting cell such as a professional antigen presenting cell in the spleen. In one embodiment, the target cell is a dendritic cell or macrophage. RNA particles such as RNA lipid particles described herein may be used for delivering RNA to such target cell. Accordingly, the present disclosure also relates to a method for delivering RNA to a target cell in a subject comprising the administration of the RNA particles described herein to the subject. In one embodiment, the RNA is delivered to the cytosol of the target cell. In one embodiment, the RNA is translated by the target cell to produce the peptide or protein encoded by the RNA.

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

In one embodiment, the RNA encoding vaccine antigen to be administered according to the present disclosure is non-immunogenic. RNA encoding immunostimulant may be administered according to the present disclosure to provide an adjuvant effect. The RNA encoding immunostimulant may be standard RNA or non-immunogenic RNA.

The term "non-immunogenic RNA" as used herein refers to RNA that does not induce a response by the immune system upon administration, e.g., to a mammal, or induces a weaker response than would have been induced by the same RNA that differs only in that it has not been subjected to the modifications and treatments that render the non-immunogenic RNA non-immunogenic, i.e., than would have been induced by standard RNA (stdRNA). In one preferred embodiment, non-immunogenic RNA, which is also termed modified RNA (modRNA) herein, is rendered non-immunogenic by incorporating modified nucleosides suppressing RNA-mediated activation of innate immune receptors into the RNA and removing double-stranded RNA (dsRNA).

For rendering the non-immunogenic RNA non-immunogenic by the incorporation of modified nucleosides, any modified nucleoside may be used as long as it lowers or suppresses immunogenicity of the RNA. Particularly preferred are modified nucleosides that suppress RNA-mediated activation of innate immune receptors. In one embodiment, the modified nucleosides comprises a replacement of one or more uridines with a nucleoside comprising a modified nucleobase. In one embodiment, the modified nucleobase is a modified uracil. In one embodiment, the nucleoside comprising a modified nucleobase is selected from the group consisting of 3-methyl-uridine (m³U), 5-methoxy-uridine (mo⁵U), 5-aza-uridine, 6-aza-uridine, 2-thio-5-aza-uridine, 2-thio-uridine (s²U), 4-thio-uridine (s⁴U), 4-thio-pseudouridine, 2-thio-pseudouridine, 5-hydroxy-uridine (ho⁵U), 5-aminoallyl-uridine, 5-halo-uridine (e.g., 5-iodo-uridine or 5-bromo-uridine), uridine 5-oxyacetic acid (cmo⁵U), uridine 5-oxyacetic acid methyl ester (mcmo⁵U), 5-carboxymethyl-uridine (cm⁵U), 1-carboxymethyl-pseudouridine, 5-carboxyhydroxymethyl-uridine (chm⁵U), 5-carboxyhydroxymethyl-uridine methyl ester (mchm⁵U), 5-methoxycarbonylmethyl-uridine (mcm⁵U), 5-methoxycarbonylmethyl-2-thio-uridine (mcm⁵s²U), 5-aminomethyl-2-thio-uridine (nm⁵s²U), 5-methylaminomethyl-uridine (mnm⁵U), 1-ethyl-pseudouridine, 5-methylaminomethyl-2-thio-uridine (mnm⁵s²U), 5-methylaminomethyl-2-seleno-uridine (mnm⁵se²U), 5-carbamoylmethyl-uridine (ncm⁵U), 5-carboxymethylaminomethyl-uridine (cmnm⁵U), 5-carboxymethylaminomethyl-2-thio-uridine (cmnm⁵s²U), 5-propynyl-uridine, 1-propynyl-pseudouridine, 5-taurinomethyl-uridine (τm⁵U), 1-taurinomethyl-pseudouridine, 5-taurinomethyl-2-thio-uridine(τm5s2U), 1-taurinomethyl-4-thio-pseudouridine), 5-methyl-2-thio-uridine (m⁵s²U), 1-methyl-4-thio-pseudouridine (m¹s⁴ψ), 4-thio-1-methyl-pseudouridine, 3-methyl-pseudouridine (m³ψ), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine (m⁵D), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine (acp³U), 1-methyl-3-(3-amino-3-carboxypropyl)pseudouridine (acp³ ψ), 5-(isopentenylaminomethyl)uridine (inm⁵U), 5-(isopentenylaminomethyl)-2-thio-uridine (inm⁵s²U), α-thio-uridine, 2'-O-methyl-uridine (Um), 5,2'-O-dimethyl-uridine (m⁵Um), 2'-O-methyl-pseudouridine (ψm), 2-thio-2'-O-methyl-uridine (s²Um), 5-methoxycarbonylmethyl-2'-O-methyl-uridine (mcm⁵Um), 5-carbamoylmethyl-2'-O-methyl-uridine (ncm⁵Um), 5-carboxymethylaminomethyl-2'-O-methyl-uridine (cmnm⁵Um), 3,2'-O-dimethyl-uridine (m³Um), 5-(isopentenylaminomethyl)-2'-O-methyl-uridine (inm⁵Um), 1-thio-uridine, deoxythymidine, 2'-F-ara-uridine, 2'-F-uridine, 2'-OH-ara-uridine, 5-(2-carbomethoxyvinyl) uridine, and 5-[3-(1-E-propenylamino)uridine. In one particularly preferred embodiment, the nucleoside comprising a modified nucleobase is pseudouridine (ψ), N1-methyl-pseudouridine (m1ψ) or 5-methyl-uridine (m5U), in particular N1-methyl-pseudouridine.

In one embodiment, the replacement of one or more uridines with a nucleoside comprising a modified nucleobase comprises a replacement of at least 1%, at least 2%, at least 3%, at least 4%, at least 5%, at least 10%, at least 25%, at least 50%, at least 75%, at least 90%, at least 95%, at least 96%, at least 97%, at least 98%, at least 99% or 100% of the uridines.

During synthesis of RNA (e.g., mRNA) by *in vitro* transcription (IVT) using T7 RNA polymerase significant amounts of aberrant products, including double-stranded RNA (dsRNA) are produced due to unconventional activity of the enzyme. dsRNA induces inflammatory cytokines and activates effector enzymes leading to protein synthesis inhibition. dsRNA can be removed from RNA such as IVT RNA, for example, by ion-pair reversed phase HPLC using a non-porous or porous C-18 polystyrene-divinylbenzene (PS-DVB) matrix. Alternatively, an enzymatic based method using E. coli RNaselll that specifically hydrolyzes dsRNA but not ssRNA, thereby eliminating dsRNA contaminants from IVT RNA preparations can be used. Furthermore, dsRNA can be separated from ssRNA by using a cellulose material. In one embodiment, an RNA preparation is contacted with a cellulose material and the ssRNA is separated from the cellulose material under conditions which allow binding of dsRNA to the cellulose material and do not allow binding of ssRNA to the cellulose material.

As the term is used herein, "remove" or "removal" refers to the characteristic of a population of first substances, such as non-immunogenic RNA, being separated from the proximity of a population of second substances, such as dsRNA, wherein the population of first substances is not necessarily devoid of the second substance, and the population of second substances is not necessarily devoid of the first substance. However, a population of first substances characterized by the removal of a population of second substances has a measurably lower content of second substances as compared to the non-separated mixture of first and second substances.

In one embodiment, the removal of dsRNA from non-immunogenic RNA comprises a removal of dsRNA such that less than 10%, less than 5%, less than 4%, less than 3%, less than 2%, less than 1%, less than 0.5%, less than 0.3%, or less than 0.1% of the RNA in the non-immunogenic RNA composition is dsRNA. In one embodiment, the non-immunogenic RNA is free or essentially free of dsRNA. In some embodiments, the non-immunogenic RNA composition comprises a purified preparation of single-stranded nucleoside modified RNA. For example, in some embodiments, the purified preparation of single-stranded nucleoside modified RNA is substantially free of double stranded RNA (dsRNA). In some embodiments, the purified preparation is at least 90%, at least 91%, at least 92%, at least 93 %, at least 94%, at least 95%, at least 96%, at least 97%, at least 98%, at least 99%, at least 99.5%, or at least 99.9% single stranded nucleoside modified RNA, relative to all other nucleic acid molecules (DNA, dsRNA, etc.).

In one embodiment, the non-immunogenic RNA is translated in a cell more efficiently than standard RNA with the same sequence. In one embodiment, translation is enhanced by a factor of 2-fold relative to its unmodified counterpart. In one embodiment, translation is enhanced by a 3-fold factor. In one embodiment, translation is enhanced by a 4-fold factor. In one embodiment, translation is enhanced by a 5-fold factor. In one embodiment, translation is enhanced by a 6-fold factor. In one embodiment, translation is enhanced by a 7-fold factor. In one embodiment, translation is enhanced by an 8-fold factor. In one embodiment, translation is enhanced by a 9-fold factor. In one embodiment, translation is enhanced by a 10-fold factor. In one embodiment, translation is enhanced by a 15-fold factor. In one embodiment, translation is enhanced by a 20-fold factor. In one embodiment, translation is enhanced by a 50-fold factor. In one embodiment, translation is enhanced by a 100-fold factor. In one embodiment, translation is enhanced by a 200-fold factor. In one embodiment, translation is enhanced by a 500-fold factor. In one embodiment, translation is enhanced by a 1000-fold factor. In one embodiment, translation is enhanced by a 2000-fold factor. In one embodiment, the factor is 10-1000-fold. In one embodiment, the factor is 10-100-fold. In one embodiment, the factor is 10-200-fold. In one embodiment, the factor is 10-300-fold. In one embodiment, the factor is 10-500-fold. In one embodiment, the factor is 20-1000-fold. In one embodiment, the factor is 30-1000-fold. In one embodiment, the factor is 50-1000-fold. In one embodiment, the factor is 100-1000-fold. In one embodiment, the factor is 200-1000-fold. In one embodiment, translation is enhanced by any other significant amount or range of amounts.

In one embodiment, the non-immunogenic RNA exhibits significantly less innate immunogenicity than standard RNA with the same sequence. In one embodiment, the non-immunogenic RNA exhibits an innate immune response that is 2-fold less than its unmodified counterpart. In one embodiment, innate immunogenicity is reduced by a 3-fold factor. In one embodiment, innate immunogenicity is reduced by a 4-fold factor. In one embodiment, innate immunogenicity is reduced by a 5-fold factor. In one embodiment, innate immunogenicity is reduced by a 6-fold factor. In one embodiment, innate immunogenicity is reduced by a 7-fold factor. In one embodiment, innate immunogenicity is reduced by a 8-fold factor. In one embodiment, innate immunogenicity is reduced by a 9-fold factor. In one embodiment, innate immunogenicity is reduced by a 10-fold factor. In one embodiment, innate immunogenicity is reduced by a 15-fold factor. In one embodiment, innate immunogenicity is reduced by a 20-fold factor. In one embodiment, innate immunogenicity is reduced by a 50-fold factor. In one embodiment, innate immunogenicity is reduced by a 100-fold factor. In one embodiment, innate immunogenicity is reduced by a 200-fold factor. In one embodiment, innate immunogenicity is reduced by a 500-fold factor. In one embodiment, innate immunogenicity is reduced by a 1000-fold factor. In one embodiment, innate immunogenicity is reduced by a 2000-fold factor.

The term "exhibits significantly less innate immunogenicity" refers to a detectable decrease in innate immunogenicity. In one embodiment, the term refers to a decrease such that an effective amount of the non-immunogenic RNA can be administered without triggering a detectable innate immune response. In one embodiment, the term refers to a decrease such that the non-immunogenic RNA can be repeatedly administered without eliciting an innate immune response sufficient to detectably reduce production of the protein encoded by the non-immunogenic RNA. In one embodiment, the decrease is such that the non-immunogenic RNA can be repeatedly administered without eliciting an innate immune response sufficient to eliminate detectable production of the protein encoded by the non-immunogenic RNA.

"Immunogenicity" is the ability of a foreign substance, such as RNA, to provoke an immune response in the body of a human or other animal. The innate immune system is the component of the immune system that is relatively unspecific and immediate. It is one of two main components of the vertebrate immune system, along with the adaptive immune system.

As used herein "endogenous" refers to any material from or produced inside an organism, cell, tissue or system.

As used herein, the term "exogenous" refers to any material introduced from or produced outside an organism, cell, tissue or system.

The term "expression" as used herein is defined as the transcription and/or translation of a particular nucleotide sequence.

As used herein, the terms "linked," "fused", or "fusion" are used interchangeably. These terms refer to the joining together of two or more elements or components or domains.

### Codon-optimization / Increase in G/C content

In some embodiment, the amino acid sequence comprising a SARS-CoV-2 S protein, an immunogenic variant thereof, or an immunogenic fragment of the SARS-CoV-2 S protein or the immunogenic variant thereof described herein is encoded by a coding sequence which is codon-optimized and/or the G/C content of which is increased compared to wild type coding sequence. This also includes embodiments, wherein one or more sequence regions of the coding sequence are codon-optimized and/or increased in the G/C content compared to the corresponding sequence regions of the wild type coding sequence. In one embodiment, the codon-optimization and/or the increase in the G/C content preferably does not change the sequence of the encoded amino acid sequence.

The term "codon-optimized" refers to the alteration of codons in the coding region of a nucleic acid molecule to reflect the typical codon usage of a host organism without preferably altering the amino acid sequence encoded by the nucleic acid molecule. Within the context of the present disclosure, coding regions are preferably codon-optimized for optimal expression in a subject to be treated using the RNA molecules described herein. Codon-optimization is based on the finding that the translation efficiency is also determined by a different frequency in the occurrence of tRNAs in cells. Thus, the sequence of RNA may be modified such that codons for which frequently occurring tRNAs are available are inserted in place of "rare codons".

In some embodiments of the present disclosure, the guanosine/cytosine (G/C) content of the coding region of the RNA described herein is increased compared to the G/C content of the corresponding coding sequence of the wild type RNA, wherein the amino acid sequence encoded by the RNA is preferably not modified compared to the amino acid sequence encoded by the wild type RNA. This modification of the RNA sequence is based on the fact that the sequence of any RNA region to be translated is important for efficient translation of that mRNA. Sequences having an increased G (guanosine)/C (cytosine) content are more stable than sequences having an increased A (adenosine)/U (uracil) content. In respect to the fact that several codons code for one and the same amino acid (so-called degeneration of the genetic code), the most favourable codons for the stability can be determined (so-called alternative codon usage). Depending on the amino acid to be encoded by the RNA, there are various possibilities for modification of the RNA sequence, compared to its wild type sequence. In particular, codons which contain A and/or U nucleotides can be modified by substituting these codons by other codons, which code for the same amino acids but contain no A and/or U or contain a lower content of A and/or U nucleotides.

In various embodiments, the G/C content of the coding region of the RNA described herein is increased by at least 10%, at least 20%, at least 30%, at least 40%, at least 50%, at least 55%, or even more compared to the G/C content of the coding region of the wild type RNA. In some embodiments, G/C content of a coding region is increased by about 10% to about 60% (e.g., by about 20% to about 60%, about 30% to about 60%, about 40% to about 60%, about 50% to about 60%, or by about 51%, about 52%, about 53%, about 54%, about 55%, about 56%, about 57%, about 58%, about 59%, or about 60%) compared to the G/C content of the coding region of the wild type RNA.

In some embodiments, RNA disclosed herein comprises a sequence disclosed herein (e.g., SEQ ID NO: 9), that has been modified to encode one or more mutations characteristic of a SARS-CoV-2 variant (e.g., ones described herein including but not limited to a BA.2 or a BA.4/5 Omicron variant). In some embodiments, RNA can be modified to encode one or more mutations characteristic of a SARS-CoV-2 variant by making as few nucleotide changes as possible. In some embodiments, RNA can be modified to encode one or more mutations that are characteristic of a SARS-CoV-2 variant by introducing mutations that result in high codon-optimization and/or increased G/C content.

In some embodiments, one or more mutations characteristic of a SARS-CoV-2 variant are introduced onto a full-length S protein (e.g., an S protein comprising SEQ ID NO: 1). In some embodiments one or more mutations characteristic of a SARS-CoV-2 variant are introduced onto a full-length S protein having one or more proline mutations that increase stability of a prefusion confirmation. For example, in some embodiments, proline substitutions are made at positions corresponding to positions 986 and 987 of SEQ ID NO: 1. In some embodiments, at least 4 proline substitutions are made. In some embodiments, at least four of such proline mutations include mutations at positions corresponding to residues 817, 892, 899, and 942 of SEQ ID NO: 1, e.g., as described in WO 2021243122 A2, the entire contents of which are incorporated herein by reference in its entirety. In some embodiments, such a SARS-CoV-2 S protein comprising proline substitutions at positions corresponding to residues 817, 892, 899, and 942 of SEQ ID NO: 1, may further comprise proline substitutions at positions corresponding to residues 986 and 987 of SEQ ID NO: 1. In some embodiments, one or more mutations characteristic of a SARS-CoV-2 variant are introduced onto an immunogenic fragment of an S protein (e.g., the RBD of SEQ ID NO: 1).

### Embodiments of administered RNAs

In some embodiments, the present disclosure provides an RNA (e.g., mRNA) comprising an open reading frame encoding a polypeptide that comprises at least a portion of a SARS-CoV-2 S protein. The RNA is suitable for intracellular expression of the polypeptide. In some embodiments, such an encoded polypeptide comprises a sequence corresponding to the complete S protein. In some embodiments, such an encoded polypeptide does not comprise a sequence corresponding to the complete S protein. In some embodiments, the encoded polypeptide comprises a sequence that corresponds to the receptor binding domain (RBD). In some embodiments, the encoded polypeptide comprises a sequence that corresponds to the RBD, and further comprises a trimerization domain (e.g., a trimerization domain as disclosed herein, such as a fibritin domain). In some embodiments an RBD comprises a signaling domain (e.g., a signaling domain as disclosed herein). In some embodiments an RBD comprises a transmembrane domain (e.g., a transmembrane domain as disclosed herein). In some embodiments, an RBD comprises a signaling domain and a trimerization domain. In some embodiments, an RBD comprises a signaling domain, a trimerization domain, and transmembrane domain.

In some embodiments, the encoded polypeptide comprises a sequence that corresponds to two receptor binding domains. In some embodiments, the encoded polypeptide comprises a sequence that corresponds to two receptor binding domains in tandem in an amino acid chain, e.g., as disclosed in Dai, Lianpan, et al. "A universal design of betacoronavirus vaccines against COVID-19, MERS, and SARS," Cell 182.3 (2020): 722-733, the contents of which are incorporated by reference herein in their entirety.

In some embodiments, a SARS-CoV-2 S protein, or an immunogenic fragment thereof comprises one or more mutations to alter or remove a glycosylation site, e.g., as described in WO2022221835A2, US20220323574A1, or WO2022195351A1.

In some embodiments, compositions or medical preparations described herein comprise RNA encoding an amino acid sequence comprising SARS-CoV-2 S protein, an immunogenic variant thereof, or an immunogenic fragment of the SARS-CoV-2 S protein or the immunogenic variant thereof. Likewise, methods described herein comprise administration of such RNA.

The active platform for use herein is based on an antigen-coding RNA vaccine to induce robust neutralising antibodies and accompanying/concomitant T cell response to achieve protective immunization with preferably minimal vaccine doses. The RNA administered is preferably in-*vitro* transcribed RNA.

Three different RNA platforms are particularly preferred, namely non-modified uridine containing mRNA (uRNA), nucleoside modified mRNA (modRNA) and self-amplifying RNA (saRNA). In one particularly preferred embodiment, the RNA is in vitro transcribed RNA. In some embodiments, uRNA is mRNA. In some embodiments, modRNA is mRNA.

In the following, embodiments of these three different RNA platforms are described, wherein certain terms used when describing elements thereof have the following meanings:
**5152 protein/SlS2 RBD:** Sequences encoding the respective antigen of SARS-CoV-2.
**nsP1, nsP2, nsP3, and nsP4:** Wildtype sequences encoding the Venezuelan equine encephalitis virus (VEEV) RNA-dependent RNA polymerase replicase and a subgenomic promotor plus conserved sequence elements supporting replication and translation.
**virUTR:** Viral untranslated region encoding parts of the subgenomic promotor as well as replication and translation supporting sequence elements.
**hAg-Kozak:** 5'-UTR sequence of the human alpha-globin mRNA with an optimized 'Kozak sequence' to increase translational efficiency.
**Sec:** Sec corresponds to a secretory signal peptide (sec), which guides translocation of the nascent polypeptide chain into the endoplasmatic reticulum. In some embodiments, such a secretory signal peptide includes the intrinsic S1S2 secretory signal peptide. In some embodiments, such a secretory signal peptide is a secretory signal peptide from a non-S1S2 protein. For example, an immunoglobulin secretory signal peptide (aa 1-22), an HSV-1 gD signal peptide (MGGAAARLGAVILFVVIVGLHGVRSKY), an HSV-2 gD signal peptide (MGRLTSGVGTAALLVVAVGLRVVCA); a human SPARC signal peptide, a human insulin isoform 1 signal peptide, a human albumin signal peptide, or any other signal peptide described herein.
**Glycine-serine linker (GS):** Sequences coding for short linker peptides predominantly consisting of the amino acids glycine (G) and serine (S), as commonly used for fusion proteins.
**Fibritin:** Partial sequence of T4 fibritin (foldon), used as artificial trimerization domain.
**TM:** TM sequence corresponds to the transmembrane part of a protein. A transmembrane domain can be N-terminal, C-terminal, or internal to an encoded polypeptide. A coding sequence of a transmembrane element is typically placed in frame (i.e., in the same reading frame), 5', 3', or internal to coding sequences of sequences (e.g., sequences encoding polypeptide(s)) with which it is to be linked. In some embodiments, a transmembrane domain comprises or is a transmembrane domain of Hemagglutinin (HA) of Influenza virus, Env of HIV-1, equine infectious anaemia virus (EIAV), murine leukaemia virus (MLV), mouse mammary tumor virus, G protein of vesicular stomatitis virus (VSV), Rabies virus, or a seven transmembrane domain receptor. In some embodiments, the transmembrane part of a protein is from the S1S2 protein.
**FI element:** The 3'-UTR is a combination of two sequence elements derived from the "amino terminal enhancer of split" (AES) mRNA (called F) and the mitochondrial encoded 12S ribosomal RNA (called I). These were identified by an *ex vivo* selection process for sequences that confer RNA stability and augment total protein expression.
**A30L70:** A poly(A)-tail measuring 110 nucleotides in length, consisting of a stretch of 30 adenosine residues, followed by a 10 nucleotide linker sequence and another 70 adenosine residues designed to enhance RNA stability and translational efficiency in dendritic cells.

In some embodiments, vaccine RNA described herein may comprise, from 5' to 3', one of the following structures:
Cap-5'-UTR-Vaccine Antigen-Encoding Sequence-3'-UTR-Poly(A)
   or
Cap- hAg-Kozak-Vaccine Antigen-Encoding Sequence-FI-A30L70.

In some embodiments, a vaccine antigen described herein may comprise a full-length S protein or an immunogenic fragment thereof (e.g., RBD). In some embodiments where a vaccine antigen comprises a full-length S protein, its secretory signal peptide and/or transmembrane domain may be replaced by a heterologous secretory signal peptide (e.g., as described herein) and/or a heterologous transmembrane domain (e.g., as described herein).

In some embodiments, a vaccine antigen described herein may comprise, from N-terminus to C-terminus, one of the following structures:
Signal Sequence-RBD-Trimerization Domain
   or
Signal Sequence-RBD-Trimerization Domain-Transmembrane Domain.

RBD and Trimerization Domain may be separated by a linker, in particular a GS linker such as a linker having the amino acid sequence GSPGSGSGS. Trimerization Domain and Transmembrane Domain may be separated by a linker, in particular a GS linker such as a linker having the amino acid sequence GSGSGS.

Signal Sequence may be a signal sequence as described herein. RBD may be a RBD domain as described herein. Trimerization Domain may be a trimerization domain as described herein. Transmembrane Domain may be a transmembrane domain as described herein.

In one embodiment,
Signal sequence comprises the amino acid sequence of amino acids 1 to 16 or 1 to 19 of SEQ ID NO: 1 or the amino acid sequence of amino acids 1 to 22 of SEQ ID NO: 31, or an amino acid sequence having at least 99%, 98%, 97%, 96%, 95%, 90%, 85%, or 80% identity to this amino acid sequence,
RBD comprises the amino acid sequence of amino acids 327 to 528 of SEQ ID NO: 1, or an amino acid sequence having at least 99%, 98%, 97%, 96%, 95%, 90%, 85%, or 80% identity to this amino acid sequence,
Trimerization Domain comprises the amino acid sequence of amino acids 3 to 29 of SEQ ID NO: 10 or the amino acid sequence of SEQ ID NO: 10, or an amino acid sequence having at least 99%, 98%, 97%, 96%, 95%, 90%, 85%, or 80% identity to this amino acid sequence; and
Transmembrane Domain comprises the amino acid sequence of amino acids 1207 to 1254 of SEQ ID NO: 1, or an amino acid sequence having at least 99%, 98%, 97%, 96%, 95%, 90%, 85%, or 80% identity to this amino acid sequence.

In one embodiment,
Signal sequence comprises the amino acid sequence of amino acids 1 to 16 or 1 to 19 of SEQ ID NO: 1 or the amino acid sequence of amino acids 1 to 22 of SEQ ID NO: 31,
RBD comprises the amino acid sequence of amino acids 327 to 528 of SEQ ID NO: 1,
Trimerization Domain comprises the amino acid sequence of amino acids 3 to 29 of SEQ ID NO: 10 or the amino acid sequence of SEQ ID NO: 10; and
Transmembrane Domain comprises the amino acid sequence of amino acids 1207 to 1254 of SEQ ID NO: 1.

In some embodiments, an RNA polynucleotide comprising a sequence encoding a vaccine antigen (e.g., a SARS-CoV-2 S protein, an immunogenic variant thereof, or an immunogenic fragment of the SARS-CoV-2 S protein or the immunogenic variant thereof) or comprising an open reading frame encoding a vaccine antigen (e.g., a SARS-CoV-2 S protein, an immunogenic variant thereof, or an immunogenic fragment of the SARS-CoV-2 S protein or the immunogenic variant thereof) such as the nucleotide sequence of SEQ ID NO: 50 or the nucleotide sequence of SEQ ID NO: 53, a variant or fragment thereof, further comprises a 5' cap, e.g., a 5' cap comprising a Cap1 structure, a 5' UTR sequence, e.g., a 5' UTR sequence comprising the nucleotide sequence of SEQ ID NO: 12, a 3' UTR sequence, e.g., a 3' UTR sequence comprising the nucleotide sequence of SEQ ID NO: 13, and polyA sequence, e.g., a polyA sequence comprising the nucleotide sequence of SEQ ID NO: 14. In some embodiments, RNA is formulated in a composition comprising ((4-hydroxybutyl)azanediyl)bis(hexane-6,1-diyl)bis(2-hexyldecanoate), cholesterol, distearoylphosphatidylcholine, and (2-[(polyethylene glycol)-2000]-N,N-ditetradecylacetamide).

RNA described herein or RNA encoding the vaccine antigen described herein may be non-modified uridine containing RNA (uRNA), nucleoside modified RNA (modRNA) or self-amplifying RNA (saRNA). In some embodiments, uRNA is mRNA. In some embodiments, modRNA is mRNA. In one embodiment, RNA described herein or RNA encoding the vaccine antigen described herein is nucleoside modified RNA (modRNA).

### Variant Specific Vaccines

In some embodiments, RNA disclosed herein encodes an S protein comprising one or more mutations that are characteristic of a SARS-CoV-2 variant. In some embodiments, RNA encodes a SARS-CoV-2 S protein comprising one or more mutations characteristic of an Alpha variant. In some embodiments, RNA encodes a SARS-CoV-2 S protein comprising one or more mutations characteristic of a Beta variant. In some embodiments, RNA encodes a SARS-CoV-2 S protein comprising one or more mutations characteristic of a Delta variant. In some embodiments, RNA encodes a SARS-CoV-2 S protein comprising one or more mutations characteristic of an Omicron variant (e.g., an S protein comprising one or more mutations characteristic of a BA.1, BA.2, or BA.4/5 Omicron variant). In some embodiments, RNA encodes a SARS-CoV-2 S protein comprising one or more mutations characteristic of an BA.1 Omicron variant. In some embodiments, RNA encodes a SARS-CoV-2 S protein comprising one or more mutations characteristic of an BA.2 Omicron variant. In some embodiments, RNA encodes a SARS-CoV-2 S protein comprising one or more mutations characteristic of an BA.2.12.1 Omicron variant. In some embodiments, RNA encodes a SARS-CoV-2 S protein comprising one or more mutations characteristic of a BA.3 Omicron variant. In some embodiments, RNA encodes a SARS-CoV-2 S protein comprising one or more mutations characteristic of a BA.4 or BA.5 Omicron variant.

### Non-modified uridine RNA (uRNA)

In some embodiments, a non-modified uridine RNA is a messenger RNA. In some embodiments, the active principle of non-modified messenger RNA drug substance is a single-stranded mRNA that is translated upon entering a cell. In addition to the sequence encoding the coronavirus vaccine antigen (i.e. open reading frame), each uRNA preferably contains common structural elements optimized for maximal efficacy of the RNA with respect to stability and translational efficiency (including, e.g., 5'-cap, 5'-UTR, 3'-UTR, poly(A)-tail as described herein). The preferred 5' cap structure is beta-S-ARCA(D1) (m₂^{7,2'-O}GppSpG). The preferred 5'-UTR and 3'-UTR comprise the nucleotide sequence of SEQ ID NO: 12 and the nucleotide sequence of SEQ ID NO: 13, respectively. The preferred poly(A)-tail comprises the sequence of SEQ ID NO: 14.

Different embodiments of this platform are as follows:
**RBL063.1 (SEQ ID NO: 15; SEQ ID NO: 7)**

| | |
|---|---|
| Structure | beta-S-ARCA(D1)-hAg-Kozak-S1S2-PP-FI-A30U0 |
| Encoded | antigen Viral spike protein (S1S2 protein) of the SARS-CoV-2 (S1S2 full-length protein, sequence variant) |

**RBL063.2 (SEQ ID NO: 16; SEQ ID NO: 7)**

| | |
|---|---|
| Structure | beta-S-ARCA(D1)-hAg-Kozak-S1S2-PP-FI-A30L70 |
| Encoded | antigen Viral spike protein (S1S2 protein) of the SARS-CoV-2 (S1S2 full-length protein, sequence variant) |

**BNT162a1; RBL063.3 (SEQ ID NO: 17; SEQ ID NO: 5)**

| | |
|---|---|
| Structu re | beta-S-ARCA(D1)-hAg-Kozak-RBD-GS-Fibritin-FI-A30L70 |
| Encoded | antigen Viral spike protein (S protein) of the SARS-CoV-2 (partial sequence, Receptor Binding Domain (RBD) of S1S2 protein) |

Figure 3 schematizes the general structure of the antigen-encoding RNAs.

### Nucleoside modified RNA (modRNA)

In some embodiments, nucleoside modified RNA is mRNA. In some embodiments, the active principle of nucleoside modified RNA (modRNA) drug substance is a single-stranded RNA (e.g., mRNA) that can be translated upon entering a cell. In addition to a sequence encoding a coronavirus vaccine antigen (i.e., open reading frame), each modRNA contains common structural elements optimized for maximal efficacy of the RNA as the uRNA (5'-cap, 5'-UTR, 3'-UTR, poly(A)-tail). Compared to uRNA, modRNA comprises at least one nucleotide modification (e.g., as described herein). In some embodiments, modRNA contains 1-methyl-pseudouridine instead of uridine. The preferred 5' cap structure is m₂^{7,3'O}Gppp(m₁^{2'-O})ApG. The preferred 5'-UTR and 3'-UTR comprise the nucleotide sequence of SEQ ID NO: 12 and the nucleotide sequence of SEQ ID NO: 13, respectively. The preferred poly(A)-tail comprises the sequence of SEQ ID NO: 14. An additional purification step is applied for modRNA to reduce dsRNA contaminants generated during the in vitro transcription reaction.

Different embodiment of this platform are as follows:
**BNT162b2; RBP020.1 (SEQ ID NO: 19; SEQ ID NO: 7)**

| | |
|---|---|
| Structure | m₂^{7,3'-O}Gppp(m₁^{2'-O})ApG)-hAg-Kozak-S1S2-PP-FI-A30L70 |
| Encoded | antigen Viral spike protein (S1S2 protein) of the SARS-CoV-2 (S1S2 full-length protein, sequence variant) |

**BNT162b2; RBP020.2 (SEQ ID NO: 20; SEQ ID NO: 7)**

| | |
|---|---|
| Structure | m₂^{7,3'-O}Gppp(m₁^{2'-O})ApG)-hAg-Kozak-S1S2-PP-FI-A30L70 |
| Encoded | antigen Viral spike protein (S1S2 protein) of the SARS-CoV-2 (S1S2 full-length protein, sequence variant) |

**BNT162b1; RBP020.3 (SEQ ID NO: 21; SEQ ID NO: 5)**

| | |
|---|---|
| Structu re | m₂^{7,3'-O}Gppp(m₁^{2'-O})ApG)-hAg-Kozak-RBD-GS-Fibritin-FI-A30L70 |
| Encoded | antigen Viral spike protein (S1S2 protein) of the SARS-CoV-2 (partial sequence, |

Receptor Binding Domain (RBD) of S1S2 protein fused to fibritin)
Figure 4 schematizes the general structure of the antigen-encoding RNAs.
**BNT162b3c (SEQ ID NO: 29; SEQ ID NO: 30)**

| | |
|---|---|
| Structu re | m₂^{7'3-O}Gppp(m₁^{2'-O})ApG-hAg-Kozak-RBD-GS-Fibritin-GS-TM-FI-A30L70 |
| Encoded | antigen Viral spike protein (S1S2 protein) of the SARS-CoV-2 (partial sequence, |

Receptor Binding Domain (RBD) of S1S2 protein fused to Fibritin fused to Transmembrane Domain (TM) of S1S2 protein); intrinsic S1S2 protein secretory signal peptide (aa 1-19) at the N-terminus of the antigen sequence
**BNT162b3d (SEQ ID NO: 31; SEQ ID NO: 32)**

| | |
|---|---|
| Structu re | m₂^{7,3'-O}Gppp(m₁^{2'-O})ApG-hAg-Kozak-RBD-G5-Fibritin-GS-TM-FI-A30L70 |
| Encoded | antigen Viral spike protein (S1S2 protein) of the SARS-CoV-2 (partial sequence, |

Receptor Binding Domain (RBD) of S1S2 protein fused to Fibritin fused to Transmembrane Domain (TM) of S1S2 protein); immunoglobulin secretory signal peptide (aa 1-22) at the N-terminus of the antigen sequence.
**BNT162b2** - **Beta variant; RBP020.11 (SEQ ID NO: 57; SEQ ID NO: 55)**

| | |
|---|---|
| Structure | m₂^{7,3'-O}Gppp(m₁^{2'-O})ApG)-hAg-Kozak-S1S2-PP-FI-A30L70 |
| Encoded | antigen Viral spike protein (S1S2 protein) of the SARS-CoV-2 (S1S2 full-length protein, sequence variant), comprising mutations characteristic of the Beta variant of SARS-CoV-2 |

**BNT162b2 - Alpha variant; RBP020.14 (SEQ ID NO: 60; SEQ ID NO: 58)**

| | |
|---|---|
| Structure | m₂^{7,3'-O}Gppp(m₁^{2'-O})ApG)-hAg-Kozak-S1S2-PP-FI-A30L70 |
| Encoded | antigen Viral spike protein (S1S2 protein) of the SARS-CoV-2 (S1S2 full-length protein, sequence variant), comprising mutations characteristic of the Alpha variant of SARS-CoV-2 |

**BNT162b2 - Delta variant; RBP020.16 (SEQ ID NO: 63a; SEQ ID NO: 61)**

| | |
|---|---|
| Structure | m₂^{7,3'-O}Gppp(m₁^{2'-O})ApG)-hAg-Kozak-S1S2-PP-FI-A30L70 |
| Encoded | antigen Viral spike protein (S1S2 protein) of the SARS-CoV-2 (S1S2 full-length protein, sequence variant), comprising mutations characteristic of the Delta variant of SARS-CoV-2 |

### Nucleotide Sequence of RBP020.11 (Beta-specific vaccine)

Nucleotide sequence is shown with individual sequence elements as indicated in bold letters. In addition, the sequence of the translated protein is shown in italic letters below the coding nucleotide sequence (* = stop codon). Red text indicates point mutations in the nucleotide and amino acid sequences.

AGAATAAACT AGTATTCTTC TGGTCCCCAC AGACTCAGAG AGAACCCGCC ACC
hAg-Kozak

63 73 83 93 103 113
ATGTTCGTGT TCCTGGTGCT GCTGCCTCTG GTGTCCAGCC AGTGTGTGAA CTTCACCACC
M F V F L V L L P L V S S Q C V N F T T
S Protein mut

123 133 143 153 163 173
AGAACACAGC TGCCTCCAGC CTACACCAAC AGCTTTACCA GAGGCGTGTA CTACCCCGAC
R T Q L P P A Y T N S F T R G V Y Y P D
S Protein mut

183 193 203 213 223 233
AAGGTGTTCA GATCCAGCGT GCTGCACTCT ACCCAGGACC TGTTCCTGCC TTTCTTCAGC
K V F R S S V L H S T Q D L F L P F F S
S Protein mut

243 253 263 273 283 293
AACGTGACCT GGTTCCACGC CATCCACGTG TCCGGCACCA ATGGCACCAA GAGATTCGCC
N V T W F H A I H V S G T N G T K R F A
S Protein mut

303 313 323 333 343 353
AACCCCGTGC TGCCCTTCAA CGACGGGGTG TACTTTGCCA GCACCGAGAA GTCCAACATC
N P V L P F N D G V Y F A S T E K S N I
S Protein mut

363 373 383 393 403 413
ATCAGAGGCT GGATCTTCGG CACCACACTG GACAGCAAGA CCCAGAGCCT GCTGATCGTG
I R G W I F G T T L D S K T Q S L L I V
S Protein mut

423 433 443 453 463 473
AACAACGCCA CCAACGTGGT CATCAAAGTG TGCGAGTTCC AGTTCTGCAA CGACCCCTTC
N N A T N V V I K V C E F Q F C N D P F
S Protein mut

483 493 503 513 523 533
CTGGGCGTCT ACTACCACAA GAACAACAAG AGCTGGATGG AAAGCGAGTT CCGGGTGTAC
L G V Y Y H K N N K S W M E S E F R V Y
S Protein mut

543 553 563 573 583 593
AGCAGCGCCA ACAACTGCAC CTTCGAGTAC GTGTCCCAGC CTTTCCTGAT GGACCTGGAA
S S A N N C T F E Y V S Q P F L M D L E
S Protein mut

603 613 623 633 643 653
GGCAAGCAGG GCAACTTCAA GAACCTGCGC GAGTTCGTGT TTAAGAACAT CGACGGCTAC
G K Q G N F K N L R E F V F K N I D G Y
S Protein mut

663 673 683 693 703 713
TTCAAGATCT ACAGCAAGCA CACCCCTATC AACCTCGTGC GCGGCCTGCC TCAGGGCTTC
F K I Y S K H T P I N L V R G L P Q G F
S Protein mut

723 733 743 753 763 773
TCTGCTCTGG AACCCCTGGT GGATCTGCCC ATCGGCATCA ACATCACCCG GTTTCAGACA
S A L E P L V D L P I G I N I T R F Q T
S Protein mut

783 793 803 813 823 833
CTGCACATCA GCTACCTGAC ACCTGGCGAT AGCAGCAGCG GATGGACAGC TGGTGCCGCC
L H I S Y L T P G D S S S G W T A G A A
S Protein mut

843 853 863 873 883 893
GCTTACTATG TGGGCTACCT GCAGCCTAGA ACCTTCCTGC TGAAGTACAA CGAGAACGGC
A Y Y V G Y L Q P R T F L L K Y N E N G
S Protein mut

903 913 923 933 943 953
ACCATCACCG ACGCCGTGGA TTGTGCTCTG GATCCTCTGA GCGAGACAAA GTGCACCCTG
T I T D A V D C A L D P L S E T K C T L
S Protein mut

963 973 983 993 1003 1013
AAGTCCTTCA CCGTGGAAAA GGGCATCTAC CAGACCAGCA ACTTCCGGGT GCAGCCCACC
K S F T V E K G I Y Q T S N F R V Q P T
S Protein mut

1023 1033 1043 1053 1063 1073
GAATCCATCG TGCGGTTCCC CAATATCACC AATCTGTGCC CCTTCGGCGA GGTGTTCAAT
E S I V R F P N I T N L C P F G E V F N
S Protein mut

1083 1093 1103 1113 1123 1133
GCCACCAGAT TCGCCTCTGT GTACGCCTGG AACCGGAAGC GGATCAGCAA TTGCGTGGCC
A T R F A S V Y A W N R K R I S N C V A
S Protein mut

1143 1153 1163 1173 1183 1193
GACTACTCCG TGCTGTACAA CTCCGCCAGC TTCAGCACCT TCAAGTGCTA CGGCGTGTCC
D Y S V L Y N S A S F S T F K C Y G V S
S Protein mut

1203 1213 1223 1233 1243 1253
CCTACCAAGC TGAACGACCT GTGCTTCACA AACGTGTACG CCGACAGCTT CGTGATCCGG
P T K L N D L C F T N V Y A D S F V I R
S Protein mut

1263 1273 1283 1293 1303 1313
GGAGATGAAG TGCGGCAGAT TGCCCCTGGA CAGACAGGCA ACATCGCCGA CTACAACTAC
G D E V R Q I A P G Q T G N I A D Y N Y
S Protein mut

1323 1333 1343 1353 1363 1373
AAGCTGCCCG ACGACTTCAC CGGCTGTGTG ATTGCCTGGA ACAGCAACAA CCTGGACTCC
K L P D D F T G C V I A W N S N N L D S
S Protein mut

1383       1393       1403       1413       1423       1433
AAAGTCGGCG GCAACTACAA TTACCTGTAC CGGCTGTTCC GGAAGTCCAA TCTGAAGCCC
K  V  G   G  N  Y   N  Y  L  Y   R  L  F   R  K  S   N  L  K  P
S Protein mut

1443       1453       1463       1473       1483       1493
TTCGAGCGGG ACATCTCCAC CGAGATCTAT CAGGCCGGCA GCACCCCTTG TAACGGCGTG
F  E  R   D  I  S   T  E  I  Y   Q  A  G   S  T  P   C  N  G  V
S Protein mut

1503       1513       1523       1533       1543       1553
AAGGGCTTCA ACTGCTACTT CCCACTGCAG TCCTACGGCT TTCAGCCCAC ATACGGCGTG
K  G  F   N  C  Y   F  P  L  Q   S  Y  G   F  Q  P   T  Y  G  V
S Protein mut

1563       1573       1583       1593       1603       1613
GGCTATCAGC CCTACAGAGT GGTGGTGCTG AGCTTCGAAC TGCTGCATGC CCCTGCCACA
G  Y  Q   P  Y  R   V  V  V  L   S  F  E   L  L  H   A  P  A  T
S Protein mut

1623       1633       1643       1653       1663       1673
GTGTGCGGCC CTAAGAAAAG CACCAATCTC GTGAAGAACA AATGCGTGAA CTTCAACTTC
V  C  G   P  K  K   S  T  N  L   V  K  N   K  C  V   N  F  N  F
S Protein mut

1683       1693       1703       1713       1723       1733
AACGGCCTGA CCGGCACCGG CGTGCTGACA GAGAGCAACA AGAAGTTCCT GCCATTCCAG
N  G  L   T  G  T   G  V  L  T   E  S  N   K  K  F   L  P  F  Q
S Protein mut

1743       1753       1763       1773       1783       1793
CAGTTTGGCC GGGATATCGC CGATACCACA GACGCCGTTA GAGATCCCCA GACACTGGAA
Q  F  G   R  D  I   A  D  T  T   D  A  V   R  D  P   Q  T  L  E
S Protein mut

1803       1813       1823       1833       1843       1853
ATCCTGGACA TCACCCCTTG CAGCTTCGGC GGAGTGTCTG TGATCACCCC TGGCACCAAC
I  L  D   I  T  P   C  S  F  G   G  V  S   V  I  T   P  G  T  N
S Protein mut

1863       1873       1883       1893       1903       1913
ACCAGCAATC AGGTGGCAGT GCTGTACCAG GGCGTGAACT GTACCGAAGT GCCCGTGGCC
T  S  N   Q  V  A   V  L  Y  Q   G  V  N   C  T  E   V  P  V  A
S Protein mut

1923       1933       1943       1953       1963       1973
ATTCACGCCG ATCAGCTGAC ACCTACATGG CGGGTGTACT CCACCGGCAG CAATGTGTTT
I  H  A   D  Q  L   T  P  T  W   R  V  Y   S  T  G   S  N  V  F
S Protein mut

1983       1993       2003       2013       2023       2033
CAGACCAGAG CCGGCTGTCT GATCGGAGCC GAGCACGTGA ACAATAGCTA CGAGTGCGAC
Q  T  R   A  G  C   L  I  G  A   E  H  V   N  N  S   Y  E  C  D
S Protein mut

2043 2053 2063 2073 2083 2093
ATCCCCATCG GCGCTGGAAT CTGCGCCAGC TACCAGACAC AGACAAACAG CCCTCGGAGA
I P I G A G I C A S Y Q T Q T N S P R R
S Protein mut

2103 2113 2123 2133 2143 2153
GCCAGAAGCG TGGCCAGCCA GAGCATCATT GCCTACACAA TGTCTCTGGG CGTCGAGAAC
A R S V A S Q S I I A Y T M S L G V E N
S Protein mut

2163 2173 2183 2193 2203 2213
AGCGTGGCCT ACTCCAACAA CTCTATCGCT ATCCCCACCA ACTTCACCAT CAGCGTGACC
S V A Y S N N S I A I P T N F T I S V T
S Protein mut

2223 2233 2243 2253 2263 2273
ACAGAGATCC TGCCTGTGTC CATGACCAAG ACCAGCGTGG ACTGCACCAT GTACATCTGC
T E I L P V S M T K T S V D C T M Y I C
S Protein mut

2283 2293 2303 2313 2323 2333
GGCGATTCCA CCGAGTGCTC CAACCTGCTG CTGCAGTACG GCAGCTTCTG CACCCAGCTG
G D S T E C S N L L L Q Y G S F C T Q L
S Protein mut

2343 2353 2363 2373 2383 2393
AATAGAGCCC TGACAGGGAT CGCCGTGGAA CAGGACAAGA ACACCCAAGA GGTGTTCGCC
N R A L T G I A V E Q D K N T Q E V F A
S Protein mut

2403 2413 2423 2433 2443 2453
CAAGTGAAGC AGATCTACAA GACCCCTCCT ATCAAGGACT TCGGCGGCTT CAATTTCAGC
Q V K Q I Y K T P P I K D F G G F N F S
S Protein mut

2463 2473 2483 2493 2503 2513
CAGATTCTGC CCGATCCTAG CAAGCCCAGC AAGCGGAGCT TCATCGAGGA CCTGCTGTTC
Q I L P D P S K P S K R S F I E D L L F
S Protein mut

2523 2533 2543 2553 2563 2573
AACAAAGTGA CACTGGCCGA CGCCGGCTTC ATCAAGCAGT ATGGCGATTG TCTGGGCGAC
N K V T L A D A G F I K Q Y G D C L G D
S Protein mut

2583 2593 2603 2613 2623 2633
ATTGCCGCCA GGGATCTGAT TTGCGCCCAG AAGTTTAACG GACTGACAGT GCTGCCTCCT
I A A R D L I C A Q K F N G L T V L P P
S Protein mut

2643 2653 2663 2673 2683 2693
CTGCTGACCG ATGAGATGAT CGCCCAGTAC ACATCTGCCC TGCTGGCCGG CACAATCACA
L L T D E M I A Q Y T S A L L A G T I T
S Protein mut

      2703       2713       2723       2733       2743       2753
AGCGGCTGGA CATTTGGAGC AGGCGCCGCT CTGCAGATCC CCTTTGCTAT GCAGATGGCC
 S  G  W   T  F  G   A  G  A  A   L  Q  I   P  F  A   M  Q  M  A
                          S Protein mut

      2763       2773       2783       2793       2803       2813
TACCGGTTCA ACGGCATCGG AGTGACCCAG AATGTGCTGT ACGAGAACCA GAAGCTGATC
 Y  R  F   N  G  I   G  V  T  Q   N  V  L   Y  E  N   Q  K  L  I
                          S Protein mut

      2823       2833       2843       2853       2863       2873
GCCAACCAGT TCAACAGCGC CATCGGCAAG ATCCAGGACA GCCTGAGCAG CACAGCAAGC
 A  N  Q   F  N  S   A  I  G  K   I  Q  D   S  L  S   S  T  A  S
                          S Protein mut

      2883       2893       2903       2913       2923       2933
GCCCTGGGAA AGCTGCAGGA CGTGGTCAAC CAGAATGCCC AGGCACTGAA CACCCTGGTC
 A  L  G   K  L  Q   D  V  V  N   Q  N  A   Q  A  L   N  T  L  V
                          S Protein mut

      2943       2953       2963       2973       2983       2993
AAGCAGCTGT CCTCCAACTT CGGCGCCATC AGCTCTGTGC TGAACGATAT CCTGAGCAGA
 K  Q  L   S  S  N   F  G  A  I   S  S  V   L  N  D   I  L  S  R
                          S Protein mut

      3003       3013       3023       3033       3043       3053
CTGGACCCTC CTGAGGCCGA GGTGCAGATC GACAGACTGA TCACAGGCAG ACTGCAGAGC
 L  D  P   P  E  A   E  V  Q  I   D  R  L   I  T  G   R  L  Q  S
                          S Protein mut

      3063       3073       3083       3093       3103       3113
CTCCAGACAT ACGTGACCCA GCAGCTGATC AGAGCCGCCG AGATTAGAGC CTCTGCCAAT
 L  Q  T   Y  V  T   Q  Q  L  I   R  A  A   E  I  R   A  S  A  N
                          S Protein mut

      3123       3133       3143       3153       3163       3173
CTGGCCGCCA CCAAGATGTC TGAGTGTGTG CTGGGCCAGA GCAAGAGAGT GGACTTTTGC
 L  A  A   T  K  M   S  E  C  V   L  G  Q   S  K  R   V  D  F  C
                          S Protein mut

      3183       3193       3203       3213       3223       3233
GGCAAGGGCT ACCACCTGAT GAGCTTCCCT CAGTCTGCCC CTCACGGCGT GGTGTTTCTG
 G  K  G   Y  H  L   M  S  F  P   Q  S  A   P  H  G   V  V  F  L
                          S Protein mut

      3243       3253       3263       3273       3283       3293
CACGTGACAT ATGTGCCCGC TCAAGAGAAG AATTTCACCA CCGCTCCAGC CATCTGCCAC
 H  V  T   Y  V  P   A  Q  E  K   N  F  T   T  A  P   A  I  C  H
                          S Protein mut

      3303       3313       3323       3333       3343       3353
GACGGCAAAG CCCACTTTCC TAGAGAAGGC GTGTTCGTGT CCAACGGCAC CCATTGGTTC
 D  G  K   A  H  F   P  R  E  G   V  F  V   S  N  G   T  H  W  F
                          S Protein mut

3363 3373 3383 3393 3403 3413
GTGACACAGC GGAACTTCTA CGAGCCCCAG ATCATCACCA CCGACAACAC CTTCGTGTCT
V T Q R N F Y E P Q I I T T D N T F V S
S Protein mut

3423 3433 3443 3453 3463 3473
GGCAACTGCG ACGTCGTGAT CGGCATTGTG AACAATACCG TGTACGACCC TCTGCAGCCC
G N C D V V I G I V N N T V Y D P L Q P
S Protein mut

3483 3493 3503 3513 3523 3533
GAGCTGGACA GCTTCAAAGA GGAACTGGAC AAGTACTTTA AGAACCACAC AAGCCCCGAC
E L D S F K E E L D K Y F K N H T S P D
S Protein mut

3543 3553 3563 3573 3583 3593
GTGGACCTGG GCGATATCAG CGGAATCAAT GCCAGCGTCG TGAACATCCA GAAAGAGATC
V D L G D I S G I N A S V V N I Q K E I
S Protein mut

3603 3613 3623 3633 3643 3653
GACCGGCTGA ACGAGGTGGC CAAGAATCTG AACGAGAGCC TGATCGACCT GCAAGAACTG
D R L N E V A K N L N E S L I D L Q E L
S Protein mut

3663 3673 3683 3693 3703 3713
GGGAAGTACG AGCAGTACAT CAAGTGGCCC TGGTACATCT GGCTGGGCTT TATCGCCGGA
G K Y E Q Y I K W P W Y I W L G F I A G
S Protein mut

3723 3733 3743 3753 3763 3773
CTGATTGCCA TCGTGATGGT CACAATCATG CTGTGTTGCA TGACCAGCTG CTGTAGCTGC
L I A I V M V T I M L C C M T S C C S C
S Protein mut

3783 3793 3803 3813 3823 3833
CTGAAGGGCT GTTGTAGCTG TGGCAGCTGC TGCAAGTTCG ACGAGGACGA TTCTGAGCCC
L K G C C S C G S C C K F D E D D S E P
S Protein mut

3843 3853 3863 3870
GTGCTGAAGG GCGTGAAACT GCACTACACA TGATGAC
V L K G V K L H Y T * *
S Protein mut

3880 3890 3900 3910 3920 3930
TCGAGCTGGT ACTGCATGCA CGCAATGCTA GCTGCCCCTT TCCCGTCCTG GGTACCCCGA
FI Element

3940 3950 3960 3970 3980 3990
GTCTCCCCCG ACCTCGGGTC CCAGGTATGC TCCCACCTCC ACCTGCCCCA CTCACCACCT
FI Element

4000 4010 4020 4030 4040 4050
CTGCTAGTTC CAGACACCTC CCAAGCACGC AGCAATGCAG CTCAAAACGC TTAGCCTAGC
FI Element

4060 4070 4080 4090 4100 4110
CACACCCCCA CGGGAAACAG CAGTGATTAA CCTTTAGCAA TAAACGAAAG TTTAACTAAG
FI Element

4120 4130 4140 4150 4160 4164
CTATACTAAC CCCAGGGTTG GTCAATTTCG TGCCAGCCAC ACCCTGGAGC TAGC
FI Element

4174 4184 4194 4204 4214 4224
AAAAAAAAAA AAAAAAAAAA AAAAAAAAAA GCATATGACT AAAAAAAAAA AAAAAAAAAA
A30L70

4234 4244 4254 4264 4274
AAAAAAAAAA AAAAAAAAAA AAAAAAAAAA AAAAAAAAAA AAAAAAAAAA
A30L70

Sequences of RBP020.11 are also shown in Table 3.

**Table 3: Sequences of RBP020.11 (a Beta-specific RNA vaccine)**

| SEQ ID NO. | Brief Description | Sequence |
|---|---|---|
| 55 | Amino acid sequence of RNA-encoded SARS-CoV-2 S protein from a Beta variant (with PRO mutations at positions corresponding to K986P and V987P of SEQ ID NO: 1; i.e., PRO mutations at positions 983 and 984 of SEQ ID NO: 55) | MFVFLVLLPLVSSQCVNFTTRTQLPPAYTNSFTRGVYYPDKVFRSSVLHSTQDLFLPFFSNVTWFH AIHVSGTNGTKRFANPVLPFNDGVYFASTEKSNIIRGWIFGTTLDSKTQSLLIVNNATNVVIKVCE FQFCNDPFLGVYYHKNNKSWMESEFRVYSSANNCTFEYVSQPFLMDLEGKQGNFKNLREFVFKNID GYFKIYSKHTPINLVRGLPQGFSALEPLVDLPIGINITRFQTLHISYLTPGDSSSGWTAGAAAYYV GYLQPRTFLLKYNENGTITDAVDCALDPLSETKCTLKSFTVEKGIYQTSNFRVQPTESIVRFPNIT NLCPFGEVFNATRFASVYAWNRKRISNCVADYSVLYNSASFSTFKCYGVSPTKLNDLCFTNVYADS FVIRGDEVRQIAPGQTGNIADYNYKLPDDFTGCVIAWNSNNLDSKVGGNYNYLYRLFRKSNLKPFE RDISTEIYQAGSTPCNGVKGFNCYFPLQSYGFQPTYGVGYQPYRVVVLSFELLHAPATVCGPKKST NLVKNKCVNFNFNGLTGTGVLTESNKKFLPFQQFGRDIADTTDAVRDPQTLEILDITPCSFGGVSV ITPGTNTSNQVAVLYQGVNCTEVPVAIHADQLTPTWRVYSTGSNVFQTRAGCLIGAEHVNNSYECD IPIGAGICASYQTQTNSPRRARSVASQSIIAYTMSLGVENSVAYSNNSIAIPTNFTISVTTEILPV SMTKTSVDCTMYICGDSTECSNLLLQYGSFCTQLNRALTGIAVEQDKNTQEVFAQVKQIYKTPPIK DFGGFNFSQILPDPSKPSKRSFIEDLLFNKVTLADAGFIKQYGDCLGDIAARDLICAQKFNGLTVL PPLLTDEMIAQYTSALLAGTITSGWTFGAGAALQIPFAMQMAYRFNGIGVTQNVLYENQKLIANQF NSAIGKIQDSLSSTASALGKLQDVVNQNAQALNTLVKQLSSNFGAISSVLNDILSRLDPPEAEVQI DRLITGRLQSLQTYVTQQLIRAAEIRASANLAATKMSECVLGQSKRVDFCGKGYHLMSFPQSAPHG VVFLHVTYVPAQEKNFTTAPAICHDGKAHFPREGVFVSNGTHWFVTQRNFYEPQIITTDNTFVSGN CDVVIGIVNNTVYDPLQPELDSFKEELDKYFKNHTSPDVDLGDISGINASVVNIQKEIDRLNEVAK NLNESLIDLQELGKYEQYIKWPWYIWLGFIAGLIAIVMVTIMLCCMTSCCSCLKGCCSCGSCCKFD EDDSEPVLKGVKLHYT** |
| 56 | RNA sequence encoding a SARS-CoV-2 S protein from a Beta variant | auguucgugu uccuggugcu gcugccucug guguccagcc agugugugaa cuucaccacc agaacacagc ugccuccagc cuacaccaac agcuuuacca gaggcgugua cuaccccgac aagguguuca gauccagcgu gcugcacucu acccaggacc uguuccugcc uuucuucagc aacgugaccu gguuccacgc cauccacgug uccggcacca auggcaccaa gagauucgcc aaccccgugc ugcccuucaa cgacggggug uacuuugcca gcaccgagaa guccaacauc aucagaggcu ggaucuucgg caccacacug gacagcaaga cccagagccu gcugaucgug aacaacgcca ccaacguggu caucaaagug ugcgaguucc aguucugcaa cgaccccuuc cugggcgucu acuaccacaa gaacaacaag agcuggaugg aaagcgaguu ccggguguac agcagcgcca acaacugcac cuucgaguac gugucccagc cuuuccugau ggaccuggaa ggcaagcagg gcaacuucaa gaaccugcgc gaguucgugu uuaagaacau cgacggcuac uucaagaucu acagcaagca caccccuauc aaccucgugc ggggccugcc ucagggcuuc ucugcucugg aaccccuggu ggaucugccc aucggcauca acaucacccg guuucagaca cugcacauca gcuaccugac accuggcgau agcagcagcg gauggacagc uggugccgcc gcuuacuaug ugggcuaccu gcagccuaga accuuccugc ugaaguacaa cgagaacggc accaucaccg acgccgugga uugugcucug gauccucuga gcgagacaaa gugcacccug aaguccuuca ccguggaaaa gggcaucuac cagaccagca acuuccgggu gcagcccacc gaauccaucg ugcgguuccc caauaucacc aaucugugcc ccuucggcga gguguucaau gccaccagau ucgccucugu guacgccugg aaccggaagc ggaucagcaa uugcguggcc gacuacuccg ugcuguacaa cuccgccagc uucagcaccu ucaagugcua cggcgugucc ccuaccaagc ugaacgaccu gugcuucaca aacguguacg ccgacagcuu cgugauccgg ggagaugaag ugcggcagau ugccccugga cagacaggca acaucgccga cuacaacuac aagcugcccg acgacuucac cggcugugug auugccugga acagcaacaa ccuggacucc aaagucggcg gcaacuacaa uuaccuguac cggcuguucc ggaaguccaa ucugaagccc uucgagcggg acaucuccac cgagaucuau caggccggca gcaccccuug uaacggcgug aagggcuuca acugcuacuu cccacugcag uccuacggcu uucagcccac auacggcgug ggcuaucagc ccuacagagu gguggugcug agcuucgaac ugcugcaugc cccugccaca gugugcggcc cuaagaaaag caccaaucuc gugaagaaca aaugcgugaa cuucaacuuc aacggccuga ccggcaccgg cgugcugaca gagagcaaca agaaguuccu gccauuccag caguuuggcc gggauaucgc cgauaccaca gacgccguua gagaucccca gacacuggaa auccuggaca ucaccccuug cagcuucggc ggagugucug ugaucacccc uggcaccaac accagcaauc agguggcagu gcuguaccag ggcgugaacu guaccgaagu gcccguggcc auucacgccg aucagcugac accuacaugg cggguguacu ccaccggcag caauguguuu cagaccagag ccggcugucu gaucggagcc gagcacguga acaauagcua cgagugcgac auccccaucg gcgcuggaau cugcgccagc uaccagacac agacaaacag cccucggaga gccagaagcg uggccagcca gagcaucauu gccuacacaa ugucucuggg cgucgagaac agcguggccu acuccaacaa cucuaucgcu auccccacca acuucaccau cagcgugacc acagagaucc ugccuguguc caugaccaag accagcgugg acugcaccau guacaucugc ggcgauucca ccgagugcuc caaccugcug cugcaguacg gcagcuucug cacccagcug aauagagccc ugacagggau cgccguggaa caggacaaga acacccaaga gguguucgcc |
| | | caagugaagc agaucuacaa gaccccuccu aucaaggacu ucggcggcuu caauuucagc cagauucugc ccgauccuag caagcccagc aagcggagcu ucaucgagga ccugcuguuc aacaaaguga cacuggccga cgccggcuuc aucaagcagu auggcgauug ucugggcgac auugccgcca gggaucugau uugcgcccag aaguuuaacg gacugacagu gcugccuccu cugcugaccg augagaugau cgcccaguac acaucugccc ugcuggccgg cacaaucaca agcggcugga cauuuggagc aggcgccgcu cugcagaucc ccuuugcuau gcagauggcc uaccgguuca acggcaucgg agugacccag aaugugcugu acgagaacca gaagcugauc gccaaccagu ucaacagcgc caucggcaag auccaggaca gccugagcag cacagcaagc gcccugggaa agcugcagga cguggucaac cagaaugccc aggcacugaa cacccugguc aagcagcugu ccuccaacuu cggcgccauc agcucugugc ugaacgauau ccugagcaga cuggacccuc cugaggccga ggugcagauc gacagacuga ucacaggcag acugcagagc cuccagacau acgugaccca gcagcugauc agagccgccg agauuagagc cucugccaau cuggccgcca ccaagauguc ugagugugug cugggccaga gcaagagagu ggacuuuugc ggcaagggcu accaccugau gagcuucccu cagucugccc cucacggcgu gguguuucug cacgugacau augugcccgc ucaagagaag aauuucacca ccgcuccagc caucugccac gacggcaaag cccacuuucc uagagaaggc guguucgugu ccaacggcac ccauugguuc gugacacagc ggaacuucua cgagccccag aucaucacca ccgacaacac cuucgugucu ggcaacugcg acgucgugau cggcauugug aacaauaccg uguacgaccc ucugcagccc gagcuggaca gcuucaaaga ggaacuggac aaguacuuua agaaccacac aagccccgac guggaccugg gcgauaucag cggaaucaau gccagcgucg ugaacaucca gaaagagauc gaccggcuga acgagguggc caagaaucug aacgagagcc ugaucgaccu gcaagaacug gggaaguacg agcaguacau caaguggccc ugguacaucu ggcugggcuu uaucgccgga cugauugcca ucgugauggu cacaaucaug cuguguugca ugaccagcug cuguagcugc cugaagggcu guuguagcug uggcagcugc ugcaaguucg acgaggacga uucugagccc gugcugaagg gcgugaaacu gcacuacaca ugaugac |
| 56a | DNA sequence encoding a SARS-CoV-2 S protein from a Beta variant | atgttcgtgt tcctggtgct gctgcctctg gtgtccagcc agtgtgtgaa cttcaccacc agaacacagc tgcctccagc ctacaccaac agctttacca gaggcgtgta ctaccccgac aaggtgttca gatccagcgt gctgcactct acccaggacc tgttcctgcc tttcttcagc aacgtgacct ggttccacgc catccacgtg tccggcacca atggcaccaa gagattcgcc aaccccgtgc tgcccttcaa cgacggggtg tactttgcca gcaccgagaa gtccaacatc atcagaggct ggatcttcgg caccacactg gacagcaaga cccagagcct gctgatcgtg aacaacgcca ccaacgtggt catcaaagtg tgcgagttcc agttctgcaa cgaccccttc ctgggcgtct actaccacaa gaacaacaag agctggatgg aaagcgagtt ccgggtgtac agcagcgcca acaactgcac cttcgagtac gtgtcccagc ctttcctgat ggacctggaa ggcaagcagg gcaacttcaa gaacctgcgc gagttcgtgt ttaagaacat cgacggctac ttcaagatct acagcaagca cacccctatc aacctcgtgc ggggcctgcc tcagggcttc tctgctctgg aacccctggt ggatctgccc atcggcatca acatcacccg gtttcagaca ctgcacatca gctacctgac acctggcgat agcagcagcg gatggacagc tggtgccgcc |
| | | gcttactatg tggcgtacct gcagcctaga accttcctgc tgaagtacaa cgagaacggc accatcaccg acgccgtgga ttgtgctctg gatcctctga gcgagacaaa gtgcaccctg aagtccttca ccgtggaaaa gggcatctac cagaccagca acttccgggt gcagcccacc gaatccatcg tgcggttccc caatatcacc aatctgtgcc ccttcggcga ggtgttcaat gccaccagat tcgcctctgt gtacgcctgg aaccggaagc ggatcagcaa ttgcgtggcc gactactccg tgctgtacaa ctccgccagc ttcagcacct tcaagtgcta cggcgtgtcc cctaccaagc tgaacgacct gtgcttcaca aacgtgtacg ccgacagctt cgtgatccgg ggagatgaag tgcggcagat tgcccctgga cagacaggca acatcgccga ctacaactac aagctgcccg acgacttcac cggctgtgtg attgcctgga acagcaacaa cctggactcc aaagtcggcg gcaactacaa ttacctgtac cggctgttcc ggaagtccaa tctgaagccc ttcgagcggg acatctccac cgagatctat caggccggca gcaccccttg taacggcgtg aagggcttca actgctactt cccactgcag tcctacggct ttcagcccac atacggcgtg ggctatcagc cctacagagt ggtggtgctg agcttcgaac tgctgcatgc ccctgccaca gtgtgcggcc ctaagaaaag caccaatctc gtgaagaaca aatgcgtgaa cttcaacttc aacggcctga ccggcaccgg cgtgctgaca gagagcaaca agaagttcct gccattccag cagtttggcc gggatatcgc cgataccaca gacgccgtta gagatcccca gacactggaa atcctggaca tcaccccttg cagcttcggc ggagtgtctg tgatcacccc tggcaccaac accagcaatc aggtggcagt gctgtaccag ggcgtgaact gtaccgaagt gcccgtggcc attcacgccg atcagctgac acctacatgg cgggtgtact ccaccggcag caatgtgttt cagaccagag ccggctgtct gatcggagcc gagcacgtga acaatagcta cgagtgcgac atccccatcg gcgctggaat ctgcgccagc taccagacac agacaaacag ccctcggaga gccagaagcg tggccagcca gagcatcatt gcctacacaa tgtctctggg cgtcgagaac agcgtggcct actccaacaa ctctatcgct atccccacca acttcaccat cagcgtgacc acagagatcc tgcctgtgtc catgaccaag accagcgtgg actgcaccat gtacatctgc ggcgattcca ccgagtgctc caacctgctg ctgcagtacg gcagcttctg cacccagctg aatagagccc tgacagggat cgccgtggaa caggacaaga acacccaaga ggtgttcgcc caagtgaagc agatctacaa gacccctcct atcaaggact tcggcggctt caatttcagc cagattctgc ccgatcctag caagcccagc aagcggagct tcatcgagga cctgctgttc aacaaagtga cactggccga cgccggcttc atcaagcagt atggcgattg tctgggcgac attgccgcca gggatctgat ttgcgcccag aagtttaacg gactgacagt gctgcctcct ctgctgaccg atgagatgat cgcccagtac acatctgccc tgctggccgg cacaatcaca agcggctgga cattcggagc aggcgccgct ctgcagatcc cctttgctat gcagatggcc taccggttca acggcatcgg agtgacccag aatgtgctgt acgagaacca gaagctgatc gccaaccagt tcaacagcgc catcggcaag atccaggaca gcctgagcag cacagcaagc gccctgggaa agctgcagga cgtggtcaac cagaatgccc aggcactgaa caccctggtc aagcagctgt cctccaactt cggcgccatc agctctgtgc tgaacgatat cctgagcaga ctggaccctc ctgaggccga ggtgcagatc gacagactga tcacaggcag actgcagagc ctccagacat acgtgaccca gcagctgatc agagccgccg agattagagc ctctgccaat ctggccgcca ccaagatgtc tgagtgtgtg ctgggccaga gcaagagagt ggacttttgc ggcaagggct accacctgat gagcttccct cagtctgccc ctcacggcgt ggtgtttctg |
| | | cacgtgacat atgtgcccgc tcaagagaag aatttcacca ccgctccagc catctgccac gacggcaaag cccactttcc tagagaaggc gtgttcgtgt ccaacggcac ccattggttc gtgatacagc ggaacttcta cgagccccag atcatcacca ccgacaacac cttcgtgtct ggcaactgcg acgtcgtgat cggcattgtg aacaataccg tgtacgaccc tctgcagccc gagctggaca gcttcaaaga ggaactggac aagtacttta agaaccacac aagccccgac gtggacctgg gcgatatcag cggaatcaat gccagcgtcg tgaacatcca gaaagagatc gaccggctga acgaggtggc caagaatctg aacgagagcc tgatcgacct gcaagaactg gggaagtacg agcagtacat caagtggccc tggtacatct ggctgggctt tatcgccgga ctgattgcca tcgtgatggt cacaatcatg ctgtgttgca tgaccagctg ctgtagctgc ctgaagggct gttgtagctg tggcagctgc tgcaagttcg acgaggacga ttctgagccc gtgctgaagg gcgtgaaact gcactacaca tgatgac |
| 57 | Full length RNA sequence of RBP020.11 | agaauaaacu aguauucuuc ugguccccac agacucagag agaacccgcc acc auguucgugu uccuggugcu gcugccucug guguccagcc agugugugaa cuucaccacc agaacacagc ugccuccagc cuacaccaac agcuuuacca gaggcgugua cuaccccgac aagguguuca gauccagcgu gcugcacucu acccaggacc uguuccugcc uuucuucagc aacgugaccu gguuccacgc cauccacgug uccggcacca auggcaccaa gagauucgcc aaccccgugc ugcccuucaa cgacggggug uacuuugcca gcaccgagaa guccaacauc aucagaggcu ggaucuucgg caccacacug gacagcaaga cccagagccu gcugaucgug aacaacgcca ccaacguggu caucaaagug ugcgaguucc aguucugcaa cgaccccuuc cugggcgucu acuaccacaa gaacaacaag agcuggaugg aaagcgaguu ccggguguac agcagcgcca acaacugcac cuucgaguac gugucccagc cuuuccugau ggaccuggaa ggcaagcagg gcaacuucaa gaaccugcgc gaguucgugu uuaagaacau cgacggcuac uucaagaucu acagcaagca caccccuauc aaccucgugc ggggccugcc ucagggcuuc ucugcucugg aaccccuggu ggaucugccc aucggcauca acaucacccg guuucagaca cugcacauca gcuaccugac accuggcgau agcagcagcg gauggacagc uggugccgcc gcuuacuaug ugggcuaccu gcagccuaga accuuccugc ugaaguacaa cgagaacggc accaucaccg acgccgugga uugugcucug gauccucuga gcgagacaaa gugcacccug aaguccuuca ccguggaaaa gggcaucuac cagaccagca acuuccgggu gcagcccacc gaauccaucg ugcgguuccc caauaucacc aaucugugcc ccuucggcga gguguucaau gccaccagau ucgccucugu guacgccugg aaccggaagc ggaucagcaa uugcguggcc gacuacuccg ugcuguacaa cuccgccagc uucagcaccu ucaagugcua cggcgugucc ccuaccaagc ugaacgaccu gugcuucaca aacguguacg ccgacagcuu cgugauccgg ggagaugaag ugcggcagau ugccccugga cagacaggca acaucgccga cuacaacuac aagcugcccg acgacuucac cggcugugug auugccugga acagcaacaa ccuggacucc aaagucggcg gcaacuacaa uuaccuguac cggcuguucc ggaaguccaa ucugaagccc uucgagcggg acaucuccac cgagaucuau caggccggca gcaccccuug uaacggcgug aagggcuuca acugcuacuu cccacugcag uccuacggcu uucagcccac auacggcgug |
| | | ggcuaucagc ccuacagagu gguggugcug agcuucgaac ugcugcaugc cccugccaca gugugcggcc cuaagaaaag caccaaucuc gugaagaaca aaugcgugaa cuucaacuuc aacggccuga ccggcaccgg cgugcugaca gagagcaaca agaaguuccu gccauuccag caguuuggcc gggauaucgc cgauaccaca gacgccguua gagaucccca gacacuggaa auccuggaca ucaccccuug cagcuucggc ggagugucug ugaucacccc uggcaccaac accagcaauc agguggcagu gcuguaccag ggcgugaacu guaccgaagu gcccguggcc auucacgccg aucagcugac accuacaugg cggguguacu ccaccggcag caauguguuu cagaccagag ccggcugucu gaucggagcc gagcacguga acaauagcua cgagugcgac auccccaucg gcgcuggaau cugcgccagc uaccagacac agacaaacag cccucggaga gccagaagcg uggccagcca gagcaucauu gccuacacaa ugucucuggg cgucgagaac agcguggccu acuccaacaa cucuaucgcu auccccacca acuucaccau cagcgugacc acagagaucc ugccuguguc caugaccaag accagcgugg acugcaccau guacaucugc ggcgauucca ccgagugcuc caaccugcug cugcaguacg gcagcuucug cacccagcug aauagagccc ugacagggau cgccguggaa caggacaaga acacccaaga gguguucgcc caagugaagc agaucuacaa gaccccuccu aucaaggacu ucggcggcuu caauuucagc cagauucugc ccgauccuag caagcccagc aagcggagcu ucaucgagga ccugcuguuc aacaaaguga cacuggccga cgccggcuuc aucaagcagu auggcgauug ucugggcgac auugccgcca gggaucugau uugcgcccag aaguuuaacg gacugacagu gcugccuccu cugcugaccg augagaugau cgcccaguac acaucugccc ugcuggccgg cacaaucaca agcggcugga cauuuggagc aggcgccgcu cugcagaucc ccuuugcuau gcagauggcc uaccgguuca acggcaucgg agugacccag aaugugcugu acgagaacca gaagcugauc gccaaccagu ucaacagcgc caucggcaag auccaggaca gccugagcag cacagcaagc gcccugggaa agcugcagga cguggucaac cagaaugccc aggcacugaa cacccugguc aagcagcugu ccuccaacuu cggcgccauc agcucugugc ugaacgauau ccugagcaga cuggacccuc cugaggccga ggugcagauc gacagacuga ucacaggcag acugcagagc cuccagacau acgugaccca gcagcugauc agagccgccg agauuagagc cucugccaau cuggccgcca ccaagaugu c ugagugugug cugggccaga gcaagagagu ggacuuuugc ggcaagggcu accaccugau gagcuucccu cagucugccc cucacggcgu gguguuucug cacgugacau augugcccgc ucaagagaag aauuucacca ccgcuccagc caucugccac gacggcaaag cccacuuucc uagagaaggc guguucgugu ccaacggcac ccauugguuc gugacacagc ggaacuucua cgagccccag aucaucacca ccgacaacac cuucgugucu ggcaacugcg acgucgugau cggcauugug aacaauaccg uguacgaccc ucugcagccc gagcuggaca gcuucaaaga ggaacuggac aaguacuuua agaaccacac aagccccgac guggaccugg gcgauaucag cggaaucaau gccagcgucg ugaacaucca gaaagagauc gaccggcuga acgagguggc caagaaucug aacgagagcc ugaucgaccu gcaagaacug gggaaguacg agcaguacau caaguggccc ugguacaucu ggcugggcuu uaucgccgga cugauugcca ucgugauggu cacaaucaug cuguguugca ugaccagcug cuguagcugc cugaagggcu guuguagcug uggcagcugc ugcaaguucg acgaggacga uucugagccc gugcugaagg gcgugaaacu gcacuacaca ugaugac |
| | | ucgagcuggu acugcaugca cgcaaugcua gcugccccuu ucccguccug gguaccccga gucucccccg accucggguc ccagguaugc ucccaccucc accugcccca cucaccaccu cugcuaguuc cagacaccuc ccaagcacgc agcaaugcag cucaaaacgc uuagccuagc cacaccccca cgggaaacag cagugauuaa ccuuuagcaa uaaacgaaag uuuaacuaag cuauacuaac cccaggguug gucaauuucg ugccagccac acccuggagc uagc aaaaaaaaaa aaaaaaaaaa aaaaaaaaaa gcauaugacu aaaaaaaaaa aaaaaaaaaa aaaaaaaaaa aaaaaaaaaa aaaaaaaaaa aaaaaaaaaa aaaaaaaaaa |
| 57a | Full length DNA sequence of RBP020.11 | agaataaact agtattcttc tggtccccac agactcagag agaacccgcc acc atgttcgtgt tcctggtgct gctgcctctg gtgtccagcc agtgtgtgaa cttcaccacc agaacacagc tgcctccagc ctacaccaac agctttacca gaggcgtgta ctaccccgac aaggtgttca gatccagcgt gctgcactct acccaggacc tgttcctgcc tttcttcagc aacgtgacct ggttccacgc catccacgtg tccggcacca atggcaccaa gagattcgcc aaccccgtgc tgcccttcaa cgacggggtg tactttgcca gcaccgagaa gtccaacatc atcagaggct ggatcttcgg caccacactg gacagcaaga cccagagcct gctgatcgtg aacaacgcca ccaacgtggt catcaaagtg tgcgagttcc agttctgcaa cgaccccttc ctgggcgtct actaccacaa gaacaacaag agctggatgg aaagcgagtt ccgggtgtac agcagcgcca acaactgcac cttcgagtac gtgtcccagc ctttcctgat ggacctggaa ggcaagcagg gcaacttcaa gaacctgcgc gagttcgtgt ttaagaacat cgacggctac ttcaagatct acagcaagca cacccctatc aacctcgtgc ggggcctgcc tcagggcttc tctgctctgg aacccctggt ggatctgccc atcggcatca acatcacccg gtttcagaca ctgcacatca gctacctgac acctggcgat agcagcagcg gatggacagc tggtgccgcc gcttactatg tgggctacct gcagcctaga accttcctgc tgaagtacaa cgagaacggc accatcaccg acgccgtgga ttgtgctctg gatcctctga gcgagacaaa gtgcaccctg aagtccttca ccgtggaaaa gggcatctac cagaccagca acttccgggt gcagcccacc gaatccatcg tgcggttccc caatatcacc aatctgtgcc ccttcggcga ggtgttcaat gccaccagat tcgcctctgt gtacgcctgg aaccggaagc ggatcagcaa ttgcgtggcc gactactccg tgctgtacaa ctccgccagc ttcagcacct tcaagtgcta cggcgtgtcc cctaccaagc tgaacgacct gtgcttcaca aacgtgtacg ccgacagctt cgtgatccgg ggagatgaag tgcggcagat tgcccctgga cagacaggca acatcgccga ctacaactac aagctgcccg acgacttcac cggctgtgtg attgcctgga acagcaacaa cctggactcc aaagtcggcg gcaactacaa ttacctgtac cggctgttcc ggaagtccaa tctgaagccc ttcgagcggg acatctccac cgagatctat caggccggca gcaccccttg taacggcgtg aagggcttca actgctactt cccactgcag tcctacggct ttcagcccac atacggcgtg ggctatcagc cctacagagt ggtggtgctg agcttcgaac tgctgcatgc ccctgccaca gtgtgcggcc ctaagaaaag caccaatctc gtgaagaaca aatgcgtgaa cttcaacttc aacggcctga ccggcaccgg cgtgctgaca gagagcaaca agaagttcct gccattccag cagtttggcc gggatatcgc cgataccaca gacgccgtta gagatcccca gacactggaa |
| | | atcctggaca tcaccccttg cagcttcggc ggagtgtctg tgatcacccc tggcaccaac accagcaatc aggtggcagt gctgtaccag ggcgtgaact gtaccgaagt gcccgtggcc attcacgccg atcagctgac acctacatgg cgggtgtact ccaccggcag caatgtgttt cagaccagag ccggctgtct gatcggagcc gagcacgtga acaatagcta cgagtgcgac atccccatcg gcgctggaat ctgcgccagc taccagacac agacaaacag ccctcggaga gccagaagcg tggccagcca gagcatcatt gcctacacaa tgtctctggg cgtcgagaac agcgtggcct actccaacaa ctctatcgct atccccacca acttcaccat cagcgtgacc acagagatcc tgcctgtgtc catgaccaag accagcgtgg actgcaccat gtacatctgc ggcgattcca ccgagtgctc caacctgctg ctgcagtacg gcagcttctg cacccagctg aatagagccc tgacagggat cgccgtggaa caggacaaga acacccaaga ggtgttcgcc caagtgaagc agatctacaa gacccctcct atcaaggact tcggcggctt caatttcagc cagattctgc ccgatcctag caagcccagc aagcggagct tcatcgagga cctgctgttc aacaaagtga cactggccga cgccggcttc atcaagcagt atggcgattg tctgggcgac attgccgcca gggatctgat ttgcgcccag aagtttaacg gactgacagt gctgcctcct ctgctgaccg atgagatgat cgcccagtac acatctgccc tgctggccgg cacaatcaca agcggctgga catttggagc aggcgccgct ctgcagatcc cctttgctat gcagatggcc taccggttca acggcatcgg agtgacccag aatgtgctgt acgagaacca gaagctgatc gccaaccagt tcaacagcgc catcggcaag atccaggaca gcctgagcag cacagcaagc gccctgggaa agctgcagga cgtggtcaac cagaatgccc aggcactgaa caccctggtc aagcagctgt cctccaactt cggcgccatc agctctgtgc tgaacgatat cctgagcaga ctggaccctc ctgaggccga ggtgcagatc gacagactga tcacaggcag actgcagagc ctccagacat acgtgaccca gcagctgatc agagccgccg agattagagc ctctgccaat ctggccgcca ccaagatgtc tgagtgtgtg ctgggccaga gcaagagagt ggacttttgc ggcaagggct accacctgat gagcttccct cagtctgccc ctcacggcgt ggtgtttctg cacgtgacat atgtgcccgc tcaagagaag aatttcacca ccgctccagc catctgccac gacggcaaag cccactttcc tagagaaggc gtgttcgtgt ccaacggcac ccattggttc gtgacacagc ggaacttcta cgagccccag atcatcacca ccgacaacac cttcgtgtct ggcaactgcg acgtcgtgat cggcattgtg aacaataccg tgtacgaccc tctgcagccc gagctggaca gcttcaaaga ggaactggac aagtacttta agaaccacac aagccccgac gtggacctgg gcgatatcag cggaatcaat gccagcgtcg tgaacatcca gaaagagatc gaccggctga acgaggtggc caagaatctg aacgagagcc tgatcgacct gcaagaactg gggaagtacg agcagtacat caagtggccc tggtacatct ggctgggctt tatcgccgga ctgattgcca tcgtgatggt cacaatcatg ctgtgttgca tgaccagctg ctgtagctgc ctgaagggct gttgtagctg tggcagctgc tgcaagttcg acgaggacga ttctgagccc gtgctgaagg gcgtgaaact gcactacaca tgatgac tcgagctggt actgcatgca cgcaatgcta gctgcccctt tcccgtcctg ggtaccccga gtctcccccg acctcgggtc ccaggtatgc tcccacctcc acctgcccca ctcaccacct ctgctagttc cagacacctc ccaagcacgc agcaatgcag ctcaaaacgc ttagcctagc cacaccccca cgggaaacag cagtgattaa cctttagcaa taaacgaaag tttaactaag |
| | | ctatactaac cccagggttg gtcaa[?]ttcg tgccagccac accctggagc tagc aaaaaaaaaa aaaaaaaaaa aaaaaaaaaa gcatatgact aaaaaaaaaa aaaaaaaaaa aaaaaaaaaa aaaaaaaaaa aaaaaaaaaa aaaaaaaaaa aaaaaaaaaa |

### Nucleotide Sequence of RBP020.14 (Alpha-specific vaccine)

Nucleotide sequence is shown with individual sequence elements as indicated in bold letters. In addition, the sequence of the translated protein is shown in italic letters below the coding nucleotide sequence (* = stop codon). Red text indicates point mutations in both the nucleotide and amino acid sequences.

10 20 30 40 50 53
AGAAUAAACU AGUAUUCUUC UGGUCCCCAC AGACUCAGAG AGAACCCGCC ACC
hAg-Kozak

63 73 83 93 103 113
AUGUUCGUGU UCCUGGUGCU GCUGCCUCUG GUGUCCAGCC AGUGUGUGAA CCUGACCACC
M F V F L V L L P L V S S Q C V N L T T
S protein mut4

123 133 143 153 163 173
AGAACACAGC UGCCUCCAGC CUACACCAAC AGCUUUACCA GAGGCGUGUA CUACCCCGAC
R T Q L P P A Y T N S F T R G V Y Y P D
S protein mut3

183 193 203 213 223 233
AAGGUGUUCA GAUCCAGCGU GCUGCACUCU ACCCAGGACC UGUUCCUGCC UUUCUUCAGC
K V F R S S V L H S T Q D L F L P F F S
S protein mut3

243 253 263 273 283 293
AACGUGACCU GGUUCCACGC CAUCUCCGGC ACCAAUGGCA CCAAGAGAUU CGACAACCCC
N V T W F H A I S G T N G T K R F D N P
S protein mut3

303 313 323 333 343 353
GUGCUGCCCU UCAACGACGG GGUGUACUUU GCCAGCACCG AGAAGUCCAA CAUCAUCAGA
V L P F N D G V Y F A S T E K S N I I R
S protein mut3

363 373 383 393 403 413
GGCUGGAUCU UCGGCACCAC ACUGGACAGC AAGACCCAGA GCCUGCUGAU CGUGAACAAC
G W I F G T T L D S K T Q S L L I V N N
S protein mut3

423 433 443 453 463 473
GCCACCAACG UGGUCAUCAA AGUGUGCGAG UUCCAGUUCU GCAACGACCC CUUCCUGGGC
A T N V V I K V C E F Q F C N D P F L G
S protein mut3

483 493 503 513 523 533
GUCUACCACA AGAACAACAA GAGCUGGAUG GAAAGCGAGU UCCGGGUGUA CAGCAGCGCC
V Y H K N N K S W M E S E F R V Y S S A
S protein mut3

543 553 563 573 583 593
AACAACUGCA CCUUCGAGUA CGUGUCCCAG CCUUUCCUGA UGGACCUGGA AGGCAAGCAG
N N C T F E Y V S Q P F L M D L E G K Q
S protein mut3

603 613 623 633 643 653
GGCAACUUCA AGAACCUGCG CGAGUUCGUG UUUAAGAACA UCGACGGCUA CUUCAAGAUC
G N F K N L R E F V F K N I D G Y F K I
S protein mut3

663 673 683 693 703 713
UACAGCAAGC ACACCCCUAU CAACCUCGUG CGGGAUCUGC CUCAGGGCUU CUCUGCUCUG
Y S K H T P I N L V R D L P Q G F S A L
S protein mut3

723 733 743 753 763 773
GAACCCCUGG UGGAUCUGCC CAUCGGCAUC AACAUCACCC GGUUUCAGAC ACUGCUGGCC
E P L V D L P I G I N I T R F Q T L L A
S protein mut3

783 793 803 813 823 833
CUGCACAGAA GCUACCUGAC ACCUGGCGAU AGCAGCAGCG GAUGGACAGC UGGUGCCGCC
L H R S Y L T P G D S S S G W T A G A A
S protein mut3

843 853 863 873 883 893
GCUUACUAUG UGGGCUACCU GCAGCCUAGA ACCUUCCUGC UGAAGUACAA CGAGAACGGC
A Y Y V G Y L Q P R T F L L K Y N E N G
S protein mut3

903 913 923 933 943 953
ACCAUCACCG ACGCCGUGGA UUGUGCUCUG GAUCCUCUGA GCGAGACAAA GUGCACCCUG
T I T D A V D C A L D P L S E T K C T L
S protein mut3

963 973 983 993 1003 1013
AAGUCCUUCA CCGUGGAAAA GGGCAUCUAC CAGACCAGCA ACUUCCGGGU GCAGCCCACC
K S F T V E K G I Y Q T S N F R V Q P T
S protein mut3

1023 1033 1043 1053 1063 1073
GAAUCCAUCG UGCGGUUCCC CAAUAUCACC AAUCUGUGCC CCUUCGGCGA GGUGUUCAAU
E S I V R F P N I T N L C P F G E V F N
S protein mut3

1083 1093 1103 1113 1123 1133
GCCACCAGAU UCGCCUCUGU GUACGCCUGG AACCGGAAGC GGAUCAGCAA UUGCGUGGCC
A T R F A S V Y A W N R K R I S N C V A
S protein mut3

1143 1153 1163 1173 1183 1193
GACUACUCCG UGCUGUACAA CUCCGCCAGC UUCAGCACCU UCAAGUGCUA CGGCGUGUCC
D Y S V L Y N S A S F S T F K C Y G V S
S protein mut3

1203 1213 1223 1233 1243 1253
CCUACCAAGC UGAACGACCU GUGCUUCACA AACGUGUACG CCGACAGCUU CGUGAUCCGG
P T K L N D L C F T N V Y A D S F V I R
S protein mut3

1263 1273 1283 1293 1303 1313
GGAGAUGAAG UGCGGCAGAU UGCCCCUGGA CAGACAGGCA AGAUCGCCGA CUACAACUAC
G D E V R Q I A P G Q T G K I A D Y N Y
S protein mut3

1323 1333 1343 1353 1363 1373
AAGCUGCCCG ACGACUUCAC CGGCUGUGUG AUUGCCUGGA ACAGCAACAA CCUGGACUCC
K L P D D F T G C V I A W N S N N L D S
S protein mut3

1383 1393 1403 1413 1423 1433
AAAGUCGGCG GCAACUACAA UUACCUGUAC CGGCUGUUCC GGAAGUCCAA UCUGAAGCCC
K V G G N Y N Y L Y R L F R K S N L K P
S protein mut3

1443 1453 1463 1473 1483 1493
UUCGAGCGGG ACAUCUCCAC CGAGAUCUAU CAGGCCGGCA GCACCCCUUG UAACGGCGUG
F E R D I S T E I Y Q A G S T P C N G V
S protein mut3

1503 1513 1523 1533 1543 1553
GAAGGCUUCA ACUGCUACUU CCCACUGCAG UCCUACGGCU UUCAGCCCAC AUACGGCGUG
E G F N C Y F P L Q S Y G F Q P T Y G V
S protein mut3

1563 1573 1583 1593 1603 1613
GGCUAUCAGC CCUACAGAGU GGUGGUGCUG AGCUUCGAAC UGCUGCAUGC CCCUGCCACA
G Y Q P Y R V V V L S F E L L H A P A T
S protein mut3

1623 1633 1643 1653 1663 1673
GUGUGCGGCC CUAAGAAAAG CACCAAUCUC GUGAAGAACA AAUGCGUGAA CUUCAACUUC
V C G P K K S T N L V K N K C V N F N F
S protein mut3

1683 1693 1703 1713 1723 1733
AACGGCCUGA CCGGCACCGG CGUGCUGACA GAGAGCAACA AGAAGUUCCU GCCAUUCCAG
N G L T G T G V L T E S N K K F L P F Q
S protein mut3

1743 1753 1763 1773 1783 1793
CAGUUUGGCC GGGAUAUCGA CGAUACCACA GACGCCGUUA GAGAUCCCCA GACACUGGAA
Q F G R D I D D T T D A V R D P Q T L E
S protein mut3

1803 1813 1823 1833 1843 1853
AUCCUGGACA UCACCCCUUG CAGCUUCGGC GGAGUGUCUG UGAUCACCCC UGGCACCAAC
I L D I T P C S F G G V S V I T P G T N
S protein mut3

1863 1873 1883 1893 1903 1913
ACCAGCAAUC AGGUGGCAGU GCUGUACCAG GGCGUGAACU GUACCGAAGU GCCCGUGGCC
T S N Q V A V L Y Q G V N C T E V P V A
S protein mut3

1923 1933 1943 1953 1963 1973
AUUCACGCCG AUCAGCUGAC ACCUACAUGG CGGGUGUACU CCACCGGCAG CAAUGUGUUU
I H A D Q L T P T W R V Y S T G S N V F
S protein mut3

1983 1993 2003 2013 2023 2033
CAGACCAGAG CCGGCUGUCU GAUCGGAGCC GAGCACGUGA ACAAUAGCUA CGAGUGCGAC
Q T R A G C L I G A E H V N N S Y E C D
S protein mut3

2043 2053 2063 2073 2083 2093
AUCCCCAUCG GCGCUGGAAU CUGCGCCAGC UACCAGACAC AGACAAACAG CCACCGGAGA
I P I G A G I C A S Y Q T Q T N S H R R
S protein mut3

2103 2113 2123 2133 2143 2153
GCCAGAAGCG UGGCCAGCCA GAGCAUCAUU GCCUACACAA UGUCUCUGGG CGCCGAGAAC
A R S V A S Q S I I A Y T M S L G A E N
S protein mut3

2163 2173 2183 2193 2203 2213
AGCGUGGCCU ACUCCAACAA CUCUAUCGCU AUCCCCAUCA ACUUCACCAU CAGCGUGACC
S V A Y S N N S I A I P I N F T I S V T
S protein mut3

2223 2233 2243 2253 2263 2273
ACAGAGAUCC UGCCUGUGUC CAUGACCAAG ACCAGCGUGG ACUGCACCAU GUACAUCUGC
T E I L P V S M T K T S V D C T M Y I C
S protein mut3

2283 2293 2303 2313 2323 2333
GGCGAUUCCA CCGAGUGCUC CAACCUGCUG CUGCAGUACG GCAGCUUCUG CACCCAGCUG
G D S T E C S N L L L Q Y G S F C T Q L
S protein mut3

2343 2353 2363 2373 2383 2393
AAUAGAGCCC UGACAGGGAU CGCCGUGGAA CAGGACAAGA ACACCCAAGA GGUGUUCGCC
N R A L T G I A V E Q D K N T Q E V F A
S protein mut3

2403 2413 2423 2433 2443 2453
CAAGUGAAGC AGAUCUACAA GACCCCUCCU AUCAAGGACU UCGGCGGCUU CAAUUUCAGC
Q V K Q I Y K T P P I K D F G G F N F S
S protein mut3

2463 2473 2483 2493 2503 2513
CAGAUUCUGC CCGAUCCUAG CAAGCCCAGC AAGCGGAGCU UCAUCGAGGA CCUGCUGUUC
Q I L P D P S K P S K R S F I E D L L F
S protein mut3

2523 2533 2543 2553 2563 2573
AACAAAGUGA CACUGGCCGA CGCCGGCUUC AUCAAGCAGU AUGGCGAUUG UCUGGGCGAC
N K V T L A D A G F I K Q Y G D C L G D
S protein mut3

2583 2593 2603 2613 2623 2633
AUUGCCGCCA GGGAUCUGAU UUGCGCCCAG AAGUUUAACG GACUGACAGU GCUGCCUCCU
I A A R D L I C A Q K F N G L T V L P P
S protein mut3

2643 2653 2663 2673 2683 2693
CUGCUGACCG AUGAGAUGAU CGCCCAGUAC ACAUCUGCCC UGCUGGCCGG CACAAUCACA
L L T D E M I A Q Y T S A L L A G T I T
S protein mut3

2703 2713 2723 2733 2743 2753
AGCGGCUGGA CAUUUGGAGC AGGCGCCGCU CUGCAGAUCC CCUUUGCUAU GCAGAUGGCC
S G W T F G A G A A L Q I P F A M Q M A
S protein mut3

2763 2773 2783 2793 2803 2813
UACCGGUUCA ACGGCAUCGG AGUGACCCAG AAUGUGCUGU ACGAGAACCA GAAGCUGAUC
Y R F N G I G V T Q N V L Y E N Q K L I
S protein mut3

2823 2833 2843 2853 2863 2873
GCCAACCAGU UCAACAGCGC CAUCGGCAAG AUCCAGGACA GCCUGAGCAG CACAGCAAGC
A N Q F N S A I G K I Q D S L S S T A S
S protein mut3

2883 2893 2903 2913 2923 2933
GCCCUGGGAA AGCUGCAGGA CGUGGUCAAC CAGAAUGCCC AGGCACUGAA CACCCUGGUC
A L G K L Q D V V N Q N A Q A L N T L V
S protein mut3

2943 2953 2963 2973 2983 2993
AAGCAGCUGU CCUCCAACUU CGGCGCCAUC AGCUCUGUGC UGAACGAUAU CCUGGCCAGA
K Q L S S N F G A I S S V L N D I L A R
S protein mut3

3003 3013 3023 3033 3043 3053
CUGGACCCUC CUGAGGCCGA GGUGCAGAUC GACAGACUGA UCACAGGCAG ACUGCAGAGC
L D P P E A E V Q I D R L I T G R L Q S
S protein mut3

3063 3073 3083 3093 3103 3113
CUCCAGACAU ACGUGACCCA GCAGCUGAUC AGAGCCGCCG AGAUUAGAGC CUCUGCCAAU
L Q T Y V T Q Q L I R A A E I R A S A N
S protein mut3

3123 3133 3143 3153 3163 3173
CUGGCCGCCA CCAAGAUGUC UGAGUGUGUG CUGGGCCAGA GCAAGAGAGU GGACUUUUGC
L A A T K M S E C V L G Q S K R V D F C
S protein mut3

3183 3193 3203 3213 3223 3233
GGCAAGGGCU ACCACCUGAU GAGCUUCCCU CAGUCUGCCC CUCACGGCGU GGUGUUUCUG
G K G Y H L M S F P Q S A P H G V V F L
S protein mut3

3243 3253 3263 3273 3283 3293
CACGUGACAU AUGUGCCCGC UCAAGAGAAG AAUUUCACCA CCGCUCCAGC CAUCUGCCAC
H V T Y V P A Q E K N F T T A P A I C H
S protein mut3

3303 3313 3323 3333 3343 3353
GACGGCAAAG CCCACUUUCC UAGAGAAGGC GUGUUCGUGU CCAACGGCAC CCAUUGGUUC
D G K A H F P R E G V F V S N G T H W F
S protein mut3

3363 3373 3383 3393 3403 3413
GUGACACAGC GGAACUUCUA CGAGCCCCAG AUCAUCACCA CCCACAACAC CUUCGUGUCU
V T Q R N F Y E P Q I I T T H N T F V S
S protein mut3

3423 3433 3443 3453 3463 3473
GGCAACUGCG ACGUCGUGAU CGGCAUUGUG AACAAUACCG UGUACGACCC UCUGCAGCCC
G N C D V V I G I V N N T V Y D P L Q P
S protein mut3

3483 3493 3503 3513 3523 3533
GAGCUGGACA GCUUCAAAGA GGAACUGGAC AAGUACUUUA AGAACCACAC AAGCCCCGAC
E L D S F K E E L D K Y F K N H T S P D
S protein mut3

3543 3553 3563 3573 3583 3593
GUGGACCUGG GCGAUAUCAG CGGAAUCAAU GCCAGCGUCG UGAACAUCCA GAAAGAGAUC
V D L G D I S G I N A S V V N I Q K E I
S protein mut3

3603 3613 3623 3633 3643 3653
GACCGGCUGA ACGAGGUGGC CAAGAAUCUG AACGAGAGCC UGAUCGACCU GCAAGAACUG
D R L N E V A K N L N E S L I D L Q E L
S protein mut3

3663 3673 3683 3693 3703 3713
GGGAAGUACG AGCAGUACAU CAAGUGGCCC UGGUACAUCU GGCUGGGCUU UAUCGCCGGA
G K Y E Q Y I K W P W Y I W L G F I A G
S protein mut3

3723 3733 3743 3753 3763 3773
CUGAUUGCCA UCGUGAUGGU CACAAUCAUG CUGUGUUGCA UGACCAGCUG CUGUAGCUGC
L I A I V M V T I M L C C M T S C C S C
S protein mut3

3783 3793 3803 3813 3823 3833
CUGAAGGGCU GUUGUAGCUG UGGCAGCUGC UGCAAGUUCG ACGAGGACGA UUCUGAGCCC
L K G C C S C G S C C K F D E D D S E P
S protein mut3

3843 3853 3863 3869
GUGCUGAAGG GCGUGAAACU GCACUACACA UGAUGA
V L K G V K L H Y T * *
S protein mut3

3879 3889 3899 3909 3919 3929
GAUCUGCUGG UACUGCAUGC ACGCAAUGCU AGCUGCCCCU UUCCCGUCCU GGGUACCCCG
FI element

3939 3949 3959 3969 3979 3989
AGUCUCCCCC GACCUCGGGU CCCAGGUAUG CUCCCACCUC CACCUGCCCC ACUCACCACC
FI element

3999 4009 4019 4029 4039 4049
UCUGCUAGUU CCAGACACCU CCCAAGCACG CAGCAAUGCA GCUCAAAACG CUUAGCCUAG
FI element

4059 4069 4079 4089 4099 4109
CCACACCCCC ACGGGAAACA GCAGUGAUUA ACCUUUAGCA AUAAACGAAA GUUUAACUAA
FI element

4119 4129 4139 4149 4159 4164
GCUAUACUAA CCCCAGGGUU GGUCAAUUUC GUGCCAGCCA CACCCUGGAG CUAGC
FI element

4174 4184 4194 4204 4214 4224
AAAAAAAAAA AAAAAAAAAA AAAAAAAAAA GCAUAUGACU AAAAAAAAAA AAAAAAAAAA
Poly(A)

4234 4244 4254 4264 4274
AAAAAAAAAA AAAAAAAAAA AAAAAAAAAA AAAAAAAAAA AAAAAAAAAA
Poly(A)

Sequences of RBP020.14 are also shown in Table 4.

**Table 4: Sequences of RBP020.14 (Alpha-specific RNA vaccine)**

| SEQ ID NO. | Brief Description | Sequence |
|---|---|---|
| 58 | Amino acid sequence of RNA-encoded SARS-CoV-2 S protein from an Alpha variant (with PRO mutations at positions corresponding to K986P and V987P of SEQ ID NO: 1; i.e., PRO mutations at positions 983 and 984 of SEQ ID NO: 58) | MFVFLVLLPLVSSQCVNLTTRTQLPPAYTNSFTRGVYYPDKVFRSSVLHSTQDLFLPFFSNVTWF HAISGTNGTKRFDNPVLPFNDGVYFASTEKSNIIRGWIFGTTLDSKTQSLLIVNNATNVVIKVCE FQFCNDPFLGVYHKNNKSWMESEFRVYSSANNCTFEYVSQPFLMDLEGKQGNFKNLREFVFKNID GYFKIYSKHTPINLVRDLPQGFSALEPLVDLPIGINITRFQTLLALHRSYLTPGDSSSGWTAGAA AYYVGYLQPRTFLLKYNENGTITDAVDCALDPLSETKCTLKSFTVEKGIYQTSNFRVQPTESIVR FPNITNLCPFGEVFNATRFASVYAWNRKRISNCVADYSVLYNSASFSTFKCYGVSPTKLNDLCFT NVYADSFVIRGDEVRQIAPGQTGKIADYNYKLPDDFTGCVIAWNSNNLDSKVGGNYNYLYRLFRK SNLKPFERDISTEIYQAGSTPCNGVEGFNCYFPLQSYGFQPTYGVGYQPYRVVVLSFELLHAPAT VCGPKKSTNLVKNKCVNFNFNGLTGTGVLTESNKKFLPFQQFGRDIDDTTDAVRDPQTLEILDIT PCSFGGVSVITPGTNTSNQVAVLYQGVNCTEVPVAIHADQLTPTWRVYSTGSNVFQTRAGCLIGA EHVNNSYECDIPIGAGICASYQTQTNSHRRARSVASQSIIAYTMSLGAENSVAYSNNSIAIPINF TISVTTEILPVSMTKTSVDCTMYICGDSTECSNLLLQYGSFCTQLNRALTGIAVEQDKNTQEVFA QVKQIYKTPPIKDFGGFNFSQILPDPSKPSKRSFIEDLLFNKVTLADAGFIKQYGDCLGDIAARD LICAQKFNGLTVLPPLLTDEMIAQYTSALLAGTITSGWTFGAGAALQIPFAMQMAYRFNGIGVTQ NVLYENQKLIANQFNSAIGKIQDSLSSTASALGKLQDVVNQNAQALNTLVKQLSSNFGAISSVLN DILARLDPPEAEVQIDRLITGRLQSLQTYVTQQLIRAAEIRASANLAATKMSECVLGQSKRVDFC GKGYHLMSFPQSAPHGVVFLHVTYVPAQEKNFTTAPAICHDGKAHFPREGVFVSNGTHWFVTQRN FYEPQIITTHNTFVSGNCDVVIGIVNNTVYDPLQPELDSFKEELDKYFKNHTSPDVDLGDISGIN ASVVNIQKEIDRLNEVAKNLNESLIDLQELGKYEQYIKWPWYIWLGFIAGLIAIVMVTIMLCCMT SCCSCLKGCCSCGSCCKFDEDDSEPVLKGVKLHYT** |
| 59 | RNA sequence encoding a SARS-CoV-2 S protein from a Alpha variant | AUGUUCGUGU UCCUGGUGCU GCUGCCUCUG GUGUCCAGCC AGUGUGUGAA CCUGACCACC AGAACACAGC UGCCUCCAGC CUACACCAAC AGCUUUACCA GAGGCGUGUA CUACCCCGAC AAGGUGUUCA GAUCCAGCGU GCUGCACUCU ACCCAGGACC UGUUCCUGCC UUUCUUCAGC AACGUGACCU GGUUCCACGC CAUCUCCGGC ACCAAUGGCA CCAAGAGAUU CGACAACCCC GUGCUGCCCU UCAACGACGG GGUGUACUUU GCCAGCACCG AGAAGUCCAA CAUCAUCAGA GGCUGGAUCU UCGGCACCAC ACUGGACAGC AAGACCCAGA GCCUGCUGAU CGUGAACAAC |
| | | GCCACCAACG UGGUCAUCAA AGUGUGCGAG UUCCAGUUCU GCAACGACCC CUUCCUGGGC GUCUACCACA AGAACAACAA GAGCUGGAUG GAAAGCGAGU UCCGGGUGUA CAGCAGCGCC AACAACUGCA CCUUCGAGUA CGUGUCCCAG CCUUUCCUGA UGGACCUGGA AGGCAAGCAG GGCAACUUCA AGAACCUGCG CGAGUUCGUG UUUAAGAACA UCGACGGCUA CUUCAAGAUC UACAGCAAGC ACACCCCUAU CAACCUCGUG CGGGAUCUGC CUCAGGGCUU CUCUGCUCUG GAACCCCUGG UGGAUCUGCC CAUCGGCAUC AACAUCACCC GGUUUCAGAC ACUGCUGGCC CUGCACAGAA GCUACCUGAC ACCUGGCGAU AGCAGCAGCG GAUGGACAGC UGGUGCCGCC GCUUACUAUG UGGGCUACCU GCAGCCUAGA ACCUUCCUGC UGAAGUACAA CGAGAACGGC ACCAUCACCG ACGCCGUGGA UUGUGCUCUG GAUCCUCUGA GCGAGACAAA GUGCACCCUG AAGUCCUUCA CCGUGGAAAA GGGCAUCUAC CAGACCAGCA ACUUCCGGGU GCAGCCCACC GAAUCCAUCG UGCGGUUCCC CAAUAUCACC AAUCUGUGCC CCUUCGGCGA GGUGUUCAAU GCCACCAGAU UCGCCUCUGU GUACGCCUGG AACCGGAAGC GGAUCAGCAA UUGCGUGGCC GACUACUCCG UGCUGUACAA CUCCGCCAGC UUCAGCACCU UCAAGUGCUA CGGCGUGUCC CCUACCAAGC UGAACGACCU GUGCUUCACA AACGUGUACG CCGACAGCUU CGUGAUCCGG GGAGAUGAAG UGCGGCAGAU UGCCCCUGGA CAGACAGGCA AGAUCGCCGA CUACAACUAC AAGCUGCCCG ACGACUUCAC CGGCUGUGUG AUUGCCUGGA ACAGCAACAA CCUGGACUCC AAAGUCGGCG GCAACUACAA UUACCUGUAC CGGCUGUUCC GGAAGUCCAA UCUGAAGCCC UUCGAGCGGG ACAUCUCCAC CGAGAUCUAU CAGGCCGGCA GCACCCCUUG UAACGGCGUG GAAGGCUUCA ACUGCUACUU CCCACUGCAG UCCUACGGCU UUCAGCCCAC AUACGGCGUG GGCUAUCAGC CCUACAGAGU GGUGGUGCUG AGCUUCGAAC UGCUGCAUGC CCCUGCCACA GUGUGCGGCC CUAAGAAAAG CACCAAUCUC GUGAAGAACA AAUGCGUGAA CUUCAACUUC AACGGCCUGA CCGGCACCGG CGUGCUGACA GAGAGCAACA AGAAGUUCCU GCCAUUCCAG CAGUUUGGCC GGGAUAUCGA CGAUACCACA GACGCCGUUA GAGAUCCCCA GACACUGGAA AUCCUGGACA UCACCCCUUG CAGCUUCGGC GGAGUGUCUG UGAUCACCCC UGGCACCAAC ACCAGCAAUC AGGUGGCAGU GCUGUACCAG GGCGUGAACU GUACCGAAGU GCCCGUGGCC AUUCACGCCG AUCAGCUGAC ACCUACAUGG CGGGUGUACU CCACCGGCAG CAAUGUGUUU CAGACCAGAG CCGGCUGUCU GAUCGGAGCC GAGCACGUGA ACAAUAGCUA CGAGUGCGAC AUCCCCAUCG GCGCUGGAAU CUGCGCCAGC UACCAGACAC AGACAAACAG CCACCGGAGA GCCAGAAGCG UGGCCAGCCA GAGCAUCAUU GCCUACACAA UGUCUCUGGG CGCCGAGAAC AGCGUGGCCU ACUCCAACAA CUCUAUCGCU AUCCCCAUCA ACUUCACCAU CAGCGUGACC ACAGAGAUCC UGCCUGUGUC CAUGACCAAG ACCAGCGUGG ACUGCACCAU GUACAUCUGC GGCGAUUCCA CCGAGUGCUC CAACCUGCUG CUGCAGUACG GCAGCUUCUG CACCCAGCUG AAUAGAGCCC UGACAGGGAU CGCCGUGGAA CAGGACAAGA ACACCCAAGA GGUGUUCGCC CAAGUGAAGC AGAUCUACAA GACCCCUCCU AUCAAGGACU UCGGCGGCUU CAAUUUCAGC CAGAUUCUGC CCGAUCCUAG CAAGCCCAGC AAGCGGAGCU UCAUCGAGGA CCUGCUGUUC AACAAAGUGA CACUGGCCGA CGCCGGCUUC AUCAAGCAGU AUGGCGAUUG UCUGGGCGAC AUUGCCGCCA GGGAUCUGAU UUGCGCCCAG AAGUUUAACG GACUGACAGU GCUGCCUCCU CUGCUGACCG AUGAGAUGAU CGCCCAGUAC ACAUCUGCCC UGCUGGCCGG CACAAUCACA |
| | | AGCGGCUGGA CAUUUGGAGC AGGCGCCGCU CUGCAGAUCC CCUUUGCUAU GCAGAUGGCC UACCGGUUCA ACCGCAUCCC ACUCACCCAC AAUCUGCUGU ACGACAACCA CAACCUCAUC GCCAACCAGU UCAACAGCGC CAUCGGCAAG AUCCAGGACA GCCUGAGCAG CACAGCAAGC GCCCUGGGAA AGCUGCAGGA CGUGGUCAAC CAGAAUGCCC AGGCACUGAA CACCCUGGUC AAGCAGCUGU CCUCCAACUU CGGCGCCAUC AGCUCUGUGC UGAACGAUAU CCUGGCCAGA CUGGACCCUC CUGAGGCCGA GGUGCAGAUC GACAGACUGA UCACAGGCAG ACUGCAGAGC CUCCAGACAU ACGUGACCCA GCAGCUGAUC AGAGCCGCCG AGAUUAGAGC CUCUGCCAAU CUGGCCGCCA CCAAGAUGUC UGAGUGUGUG CUGGGCCAGA GCAAGAGAGU GGACUUUUGC GGCAAGGGCU ACCACCUGAU GAGCUUCCCU CAGUCUGCCC CUCACGGCGU GGUGUUUCUG CACGUGACAU AUGUGCCCGC UCAAGAGAAG AAUUUCACCA CCGCUCCAGC CAUCUGCCAC GACGGCAAAG CCCACUUUCC UAGAGAAGGC GUGUUCGUGU CCAACGGCAC CCAUUGGUUC GUGACACAGC GGAACUUCUA CGAGCCCCAG AUCAUCACCA CCCACAACAC CUUCGUGUCU GGCAACUGCG ACGUCGUGAU CGGCAUUGUG AACAAUACCG UGUACGACCC UCUGCAGCCC GAGCUGGACA GCUUCAAAGA GGAACUGGAC AAGUACUUUA AGAACCACAC AAGCCCCGAC GUGGACCUGG GCGAUAUCAG CGGAAUCAAU GCCAGCGUCG UGAACAUCCA GAAAGAGAUC GACCGGCUGA ACGAGGUGGC CAAGAAUCUG AACGAGAGCC UGAUCGACCU GCAAGAACUG GGGAAGUACG AGCAGUACAU CAAGUGGCCC UGGUACAUCU GGCUGGGCUU UAUCGCCGGA CUCAUUGCCA UCGUCAUGGU CACAAUCAUG CUCUGUUGCA UGACCAGCUG CUGUAGCUGC CUGAAGGGCU GUUGUAGCUG UGGCAGCUGC UGCAAGUUCG ACGAGGACGA UUCUGAGCCC GUCCUGAAGG GCGUGAAACU GCACUACACA UGAUGA |
| 59a | Sequence encoding a SARS-CoV-2 S protein from a Alpha variant | ATGTTCGTGT TCCTGGTGCT GCTGCCTCTG GTGTCCAGCC AGTGTGTGAA CCTGACCACC AGAACACAGC TGCCTCCAGC CTACACCAAC AGCTTTACCA GAGGCGTGTA CTACCCCGAC AAGGTGTTCA GATCCAGCGT GCTGCACTCT ACCCAGGACC TGTTCCTGCC TTTCTTCAGC AACGTGACCT GGTTCCACGC CATCTCCGGC ACCAATGGCA CCAAGAGATT CGACAACCCC GTGCTGCCCT TCAACGACGG GGTGTACTTT GCCAGCACCG AGAAGTCCAA CATCATCAGA GGCTGGATCT TCGGCACCAC ACTGGACAGC AAGACCCAGA GCCTGCTGAT CGTGAACAAC GCCACCAACG TGGTCATCAA AGTGTGCGAG TTCCAGTTCT GCAACGACCC CTTCCTGGGC GTCTACCACA AGAACAACAA GAGCTGGATG GAAAGCGAGT TCCGGGTGTA CAGCAGCGCC AACAACTGCA CCTTCGAGTA CGTGTCCCAG CCTTTCCTGA TGGACCTGGA AGGCAAGCAG GGCAACTTCA AGAACCTGCG CGAGTTCGTG TTTAAGAACA TCGACGGCTA CTTCAAGATC TACAGCAAGC ACACCCCTAT CAACCTCGTG CGGGATCTGC CTCAGGGCTT CTCTGCTCTG GAACCCCTGG TGGATCTGCC CATCGGCATC AACATCACCC GGTTTCAGAC ACTGCTGGCC CTGCACAGAA GCTACCTGAC ACCTGGCGAT AGCAGCAGCG GATGGACAGC TGGTGCCGCC GCTTACTATG TGGGCTACCT GCAGCCTAGA ACCTTCCTGC TGAAGTACAA CGAGAACGGC ACCATCACCG ACGCCGTGGA TTGTGCTCTG GATCCTCTGA GCGAGACAAA GTGCACCCTG AAGTCCTTCA CCGTGGAAAA GGGCATCTAC CAGACCAGCA ACTTCCGGGT GCAGCCCACC CAATCCATCG TGCGGTTCCC CAATATCACC AATCTGTGCC CCTTCGGCGA GGTGTTCAAT |
| | | GCCACCAGAT TCGCCTCTGT GTACGCCTGG AACCGGAAGC GGATCAGCAA TTGCGTGGCC GACTACTCCG TGCTGTACAA CTCCGCCAGC TTCAGCACCT TCAAGTGCTA CGGCGTGTCC CCTACCAAGC TGAACGACCT GTGCTTCACA AACGTGTACG CCGACAGCTT CGTGATCCGG GGAGATGAAG TGCGGCAGAT TGCCCCTGGA CAGACAGGCA AGATCGCCGA CTACAACTAC AAGCTGCCCG ACGACTTCAC CGGCTGTGTG ATTGCCTGGA ACAGCAACAA CCTGGACTCC AAAGTCGGCG GCAACTACAA TTACCTGTAC CGGCTGTTCC GGAAGTCCAA TCTGAAGCCC TTCGAGCGGG ACATCTCCAC CGAGATCTAT CAGGCCGGCA GCACCCCTTG TAACGGCGTG GAAGGCTTCA ACTGCTACTT CCCACTGCAG TCCTACGGCT TTCAGCCCAC ATACGGCGTG GGCTATCAGC CCTACAGAGT GGTGGTGCTG AGCTTCGAAC TGCTGCATGC CCCTGCCACA GTGTGCGGCC CTAAGAAAAG CACCAATCTC GTGAAGAACA AATGCGTGAA CTTCAACTTC AACGGCCTGA CCGGCACCGG CGTGCTGACA GAGAGCAACA AGAAGTTCCT GCCATTCCAG CAGTTTGGCC GGGATATCGA CGATACCACA GACGCCGTTA GAGATCCCCA GACACTGGAA ATCCTGGACA TCACCCCTTG CAGCTTCGGC GGAGTGTCTG TGATCACCCC TGGCACCAAC ACCAGCAATC AGGTGGCAGT GCTGTACCAG GGCGTGAACT GTACCGAAGT GCCCGTGGCC ATTCACGCCG ATCAGCTGAC ACCTACATGG CGGGTGTACT CCACCGGCAG CAATGTGTTT CAGACCAGAG CCGGCTGTCT GATCGGAGCC GAGCACGTGA ACAATAGCTA CGAGTGCGAC ATCCCCATCG GCGCTGGAAT CTGCGCCAGC TACCAGACAC AGACAAACAG CCACCGGAGA GCCAGAAGCG TGGCCAGCCA GAGCATCATT GCCTACACAA TGTCTCTGGG CGCCGAGAAC AGCGTGGCCT ACTCCAACAA CTCTATCGCT ATCCCCATCA ACTTCACCAT CAGCGTGACC ACAGAGATCC TGCCTGTGTC CATGACCAAG ACCAGCGTGG ACTGCACCAT GTACATCTGC GGCGATTCCA CCGAGTGCTC CAACCTGCTG CTGCAGTACG GCAGCTTCTG CACCCAGCTG AATAGAGCCC TGACAGGGAT CGCCGTGGAA CAGGACAAGA ACACCCAAGA GGTGTTCGCC CAAGTGAAGC AGATCTACAA GACCCCTCCT ATCAAGGACT TCGGCGGCTT CAATTTCAGC CAGATTCTGC CCGATCCTAG CAAGCCCAGC AAGCGGAGCT TCATCGAGGA CCTGCTGTTC AACAAAGTGA CACTGGCCGA CGCCGGCTTC ATCAAGCAGT ATGGCGATTG TCTGGGCGAC ATTGCCGCCA GGGATCTGAT TTGCGCCCAG AAGTTTAACG GACTGACAGT GCTGCCTCCT CTGCTGACCG ATGAGATGAT CGCCCAGTAC ACATCTGCCC TGCTGGCCGG CACAATCACA AGCGGCTGGA CATTTGGAGC AGGCGCCGCT CTGCAGATCC CCTTTGCTAT GCAGATGGCC TACCGGTTCA ACGGCATCGG AGTGACCCAG AATGTGCTGT ACGAGAACCA GAAGCTGATC GCCAACCAGT TCAACAGCGC CATCGGCAAG ATCCAGGACA GCCTGAGCAG CACAGCAAGC GCCCTGGGAA AGCTGCAGGA CGTGGTCAAC CAGAATGCCC AGGCACTGAA CACCCTGGTC AAGCAGCTGT CCTCCAACTT CGGCGCCATC AGCTCTGTGC TGAACGATAT CCTGGCCAGA CTGGACCCTC CTGAGGCCGA GGTGCAGATC GACAGACTGA TCACAGGCAG ACTGCAGAGC CTCCAGACAT ACGTGACCCA GCAGCTGATC AGAGCCGCCG AGATTAGAGC CTCTGCCAAT CTGGCCGCCA CCAAGATGTC TGAGTGTGTG CTGGGCCAGA GCAAGAGAGT GGACTTTTGC GGCAAGGGCT ACCACCTGAT GAGCTTCCCT CAGTCTGCCC CTCACGGCGT GGTGTTTCTG CACGTGACAT ATGTGCCCGC TCAAGAGAAG AATTTCACCA CCGCTCCAGC CATCTGCCAC GACGGCAAAG CCCACTTTCC TAGAGAAGGC GTGTTCGTGT CCAACGGCAC CCATTGGTTC |
| | | GTGACACAGC GGAACTTCTA CGAGCCCCAG ATCATCACCA CCCACAACAC CTTCGTGTCT GGCAACTGCG ACGTCGTGAT CGGCATTGTG AACAATACCG TGTACGACCC TCTGCAGCCC GAGCTGGACA GCTTCAAAGA GGAACTGGAC AAGTACTTTA AGAACCACAC AAGCCCCGAC GTGGACCTGG GCGATATCAG CGGAATCAAT GCCAGCGTCG TGAACATCCA GAAAGAGATC GACCGGCTGA ACGAGGTGGC CAAGAATCTG AACGAGAGCC TGATCGACCT GCAAGAACTG GGGAAGTACG AGCAGTACAT CAAGTGGCCC TGGTACATCT GGCTGGGCTT TATCGCCGGA CTGATTGCCA TCGTGATGGT CACAATCATG CTGTGTTGCA TGACCAGCTG CTGTAGCTGC CTGAAGGGCT GTTGTAGCTG TGGCAGCTGC TGCAAGTTCG ACGAGGACGA TTCTGAGCCC GTGCTGAAGG GCGTGAAACT GCACTACACA TGATGA |
| 60 | Full length sequence of RBP020.14 (RNA) | AGAAUAAACU AGUAUUCUUC UGGUCCCCAC AGACUCAGAG AGAACCCGCC ACC AUGUUCGUGU UCCUGGUGCU GCUGCCUCUG GUGUCCAGCC AGUGUGUGAA CCUGACCACC AGAACACAGC UGCCUCCAGC CUACACCAAC AGCUUUACCA GAGGCGUGUA CUACCCCGAC AAGGUGUUCA GAUCCAGCGU GCUGCACUCU ACCCAGGACC UGUUCCUGCC UUUCUUCAGC AACGUGACCU GGUUCCACGC CAUCUCCGGC ACCAAUGGCA CCAAGAGAUU CGACAACCCC GUGCUGCCCU UCAACGACGG GGUGUACUUU GCCAGCACCG AGAAGUCCAA CAUCAUCAGA GGCUGGAUCU UCGGCACCAC ACUGGACAGC AAGACCCAGA GCCUGCUGAU CGUGAACAAC GCCACCAACG UGGUCAUCAA AGUGUGCGAG UUCCAGUUCU GCAACGACCC CUUCCUGGGC GUCUACCACA AGAACAACAA GAGCUGGAUG GAAAGCGAGU UCCGGGUGUA CAGCAGCGCC AACAACUGCA CCUUCGAGUA CGUGUCCCAG CCUUUCCUGA UGGACCUGGA AGGCAAGCAG GGCAACUUCA AGAACCUGCG CGAGUUCGUG UUUAAGAACA UCGACGGCUA CUUCAAGAUC UACAGCAAGC ACACCCCUAU CAACCUCGUG CGGGAUCUGC CUCAGGGCUU CUCUGCUCUG GAACCCCUGG UGGAUCUGCC CAUCGGCAUC AACAUCACCC GGUUUCAGAC ACUGCUGGCC CUGCACAGAA GCUACCUGAC ACCUGGCGAU AGCAGCAGCG GAUGGACAGC UGGUGCCGCC GCUUACUAUG UGGGCUACCU GCAGCCUAGA ACCUUCCUGC UGAAGUACAA CGAGAACGGC ACCAUCACCG ACGCCGUGGA UUGUGCUCUG GAUCCUCUGA GCGAGACAAA GUGCACCCUG AAGUCCUUCA CCGUGGAAAA GGGCAUCUAC CAGACCAGCA ACUUCCGGGU GCAGCCCACC GAAUCCAUCG UGCGGUUCCC CAAUAUCACC AAUCUGUGCC CCUUCGGCGA GGUGUUCAAU GCCACCAGAU UCGCCUCUGU GUACGCCUGG AACCGGAAGC GGAUCAGCAA UUGCGUGGCC GACUACUCCG UGCUGUACAA CUCCGCCAGC UUCAGCACCU UCAAGUGCUA CGGCGUGUCC CCUACCAAGC UGAACGACCU GUGCUUCACA AACGUGUACG CCGACAGCUU CGUGAUCCGG GGAGAUGAAG UGCGGCAGAU UGCCCCUGGA CAGACAGGCA AGAUCGCCGA CUACAACUAC AAGCUGCCCG ACGACUUCAC CGGCUGUGUG AUUGCCUGGA ACAGCAACAA CCUGGACUCC AAAGUCGGCG GCAACUACAA UUACCUGUAC CGGCUGUUCC GGAAGUCCAA UCUGAAGCCC UUCGAGCGGG ACAUCUCCAC CGAGAUCUAU CAGGCCGGCA GCACCCCUUG UAACGGCGUG GAAGGCUUCA ACUGCUACUU CCCACUGCAG UCCUACGGCU UUCAGCCCAC AUACGGCGUG GGCUAUCAGC CCUACAGAGU GGUGGUGCUG AGCUUCGAAC UGCUGCAUGC CCCUGCCACA GUGUGCGGCC CUAAGAAAAG CACCAAUCUC GUGAAGAACA AAUGCGUGAA CUUCAACUUC |
| | | AACGGCCUGA CCGGCACCGG CGUGCUGACA GAGAGCAACA AGAAGUUCCU GCCAUUCCAG CAGUUUGGCC GGGAUAUCGA CGAUACCACA GACGCCGUUA GAGAUCCCCA GACACUGGAA AUCCUGGACA UCACCCCUUG CAGCUUCGGC GGAGUGUCUG UGAUCACCCC UGGCACCAAC ACCAGCAAUC AGGUGGCAGU GCUGUACCAG GGCGUGAACU GUACCGAAGU GCCCGUGGCC AUUCACGCCG AUCAGCUGAC ACCUACAUGG CGGGUGUACU CCACCGGCAG CAAUGUGUUU CAGACCAGAG CCGGCUGUCU GAUCGGAGCC GAGCACGUGA ACAAUAGCUA CGAGUGCGAC AUCCCCAUCG GCGCUGGAAU CUGCGCCAGC UACCAGACAC AGACAAACAG CCACCGGAGA GCCAGAAGCG UGGCCAGCCA GAGCAUCAUU GCCUACACAA UGUCUCUGGG CGCCGAGAAC AGCGUGGCCU ACUCCAACAA CUCUAUCGCU AUCCCCAUCA ACUUCACCAU CAGCGUGACC ACAGAGAUCC UGCCUGUGUC CAUGACCAAG ACCAGCGUGG ACUGCACCAU GUACAUCUGC GGCGAUUCCA CCGAGUGCUC CAACCUGCUG CUGCAGUACG GCAGCUUCUG CACCCAGCUG AAUAGAGCCC UGACAGGGAU CGCCGUGGAA CAGGACAAGA ACACCCAAGA GGUGUUCGCC CAAGUGAAGC AGAUCUACAA GACCCCUCCU AUCAAGGACU UCGGCGGCUU CAAUUUCAGC CAGAUUCUGC CCGAUCCUAG CAAGCCCAGC AAGCGGAGCU UCAUCGAGGA CCUGCUGUUC AACAAAGUGA CACUGGCCGA CGCCGGCUUC AUCAAGCAGU AUGGCGAUUG UCUGGGCGAC AUUGCCGCCA GGGAUCUGAU UUGCGCCCAG AAGUUUAACG GACUGACAGU GCUGCCUCCU CUGCUGACCG AUGAGAUGAU CGCCCAGUAC ACAUCUGCCC UGCUGGCCGG CACAAUCACA AGCGGCUGGA CAUUUGGAGC AGGCGCCGCU CUGCAGAUCC CCUUUGCUAU GCAGAUGGCC UACCGGUUCA ACGGCAUCGG AGUGACCCAG AAUGUGCUGU ACGAGAACCA GAAGCUGAUC GCCAACCAGU UCAACAGCGC CAUCGGCAAG AUCCAGGACA GCCUGAGCAG CACAGCAAGC GCCCUGGGAA AGCUGCAGGA CGUGGUCAAC CAGAAUGCCC AGGCACUGAA CACCCUGGUC AAGCAGCUGU CCUCCAACUU CGGCGCCAUC AGCUCUGUGC UGAACGAUAU CCUGGCCAGA CUGGACCCUC CUGAGGCCGA GGUGCAGAUC GACAGACUGA UCACAGGCAG ACUGCAGAGC CUCCAGACAU ACGUGACCCA GCAGCUGAUC AGAGCCGCCG AGAUUAGAGC CUCUGCCAAU CUGGCCGCCA CCAAGAUGUC UGAGUGUGUG CUGGGCCAGA GCAAGAGAGU GGACUUUUGC GGCAAGGGCU ACCACCUGAU GAGCUUCCCU CAGUCUGCCC CUCACGGCGU GGUGUUUCUG CACGUGACAU AUGUGCCCGC UCAAGAGAAG AAUUUCACCA CCGCUCCAGC CAUCUGCCAC GACGGCAAAG CCCACUUUCC UAGAGAAGGC GUGUUCGUGU CCAACGGCAC CCAUUGGUUC GUGACACAGC GGAACUUCUA CGAGCCCCAG AUCAUCACCA CCCACAACAC CUUCGUGUCU GGCAACUGCG ACGUCGUGAU CGGCAUUGUG AACAAUACCG UGUACGACCC UCUGCAGCCC GAGCUGGACA GCUUCAAAGA GGAACUGGAC AAGUACUUUA AGAACCACAC AAGCCCCGAC GUGGACCUGG GCGAUAUCAG CGGAAUCAAU GCCAGCGUCG UGAACAUCCA GAAAGAGAUC GACCGGCUGA ACGAGGUGGC CAAGAAUCUG AACGAGAGCC UGAUCGACCU GCAAGAACUG GGGAAGUACG AGCAGUACAU CAAGUGGCCC UGGUACAUCU GGCUGGGCUU UAUCGCCGGA CUGAUUGCCA UCGUGAUGGU CACAAUCAUG CUGUGUUGCA UGACCAGCUG CUGUAGCUGC CUGAAGGGCU GUUGUAGCUG UGGCAGCUGC UGCAAGUUCG ACGAGGACGA UUCUGAGCCC GUGCUGAAGG GCGUGAAACU GCACUACACA UGAUGA GAUCUGCUGG UACUGCAUGC ACGCAAUGCU AGCUGCCCCU UUCCCGUCCU GGGUACCCCG |
| | | AGUCUCCCCC GACCUCGGGU CCCAGGUAUG CUCCCACCUC CACCUGCCCC ACUCACCACC UCUGCUAGUU CCAGACACCU CCCAAGCACG CAGCAAUGCA GCUCAAAACG CUUAGCCUAG CCACACCCCC ACGGGAAACA GCAGUGAUUA ACCUUUAGCA AUAAACGAAA GUUUAACUAA GCUAUACUAA CCCCAGGGUU GGUCAAUUUC GUGCCAGCCA CACCCUGGAG CUAGC AAAAAAAAAA AAAAAAAAAA AAAAAAAAAA GCAUAUGACU AAAAAAAAAA AAAAAAAAAA AAAAAAAAAA AAAAAAAAAA AAAAAAAAAA AAAAAAAAAA AAAAAAAAAA |
| 60a | Full length sequence of RBP020.14 (DNA) | AGAATAAACT AGTATTCTTC TGGTCCCCAC AGACTCAGAG AGAACCCGCC ACC ATGTTCGTGT TCCTGGTGCT GCTGCCTCTG GTGTCCAGCC AGTGTGTGAA CCTGACCACC AGAACACAGC TGCCTCCAGC CTACACCAAC AGCTTTACCA GAGGCGTGTA CTACCCCGAC AAGGTGTTCA GATCCAGCGT GCTGCACTCT ACCCAGGACC TGTTCCTGCC TTTCTTCAGC AACGTGACCT GGTTCCACGC CATCTCCGGC ACCAATGGCA CCAAGAGATT CGACAACCCC GTGCTGCCCT TCAACGACGG GGTGTACTTT GCCAGCACCG AGAAGTCCAA CATCATCAGA GGCTGGATCT TCGGCACCAC ACTGGACAGC AAGACCCAGA GCCTGCTGAT CGTGAACAAC GCCACCAACG TGGTCATCAA AGTGTGCGAG TTCCAGTTCT GCAACGACCC CTTCCTGGGC GTCTACCACA AGAACAACAA GAGCTGGATG GAAAGCGAGT TCCGGGTGTA CAGCAGCGCC AACAACTGCA CCTTCGAGTA CGTGTCCCAG CCTTTCCTGA TGGACCTGGA AGGCAAGCAG GGCAACTTCA AGAACCTGCG CGAGTTCGTG TTTAAGAACA TCGACGGCTA CTTCAAGATC TACAGCAAGC ACACCCCTAT CAACCTCGTG CGGGATCTGC CTCAGGGCTT CTCTGCTCTG GAACCCCTGG TGGATCTGCC CATCGGCATC AACATCACCC GGTTTCAGAC ACTGCTGGCC CTGCACAGAA GCTACCTGAC ACCTGGCGAT AGCAGCAGCG GATGGACAGC TGGTGCCGCC GCTTACTATG TGGGCTACCT GCAGCCTAGA ACCTTCCTGC TGAAGTACAA CGAGAACGGC ACCATCACCG ACGCCGTGGA TTGTGCTCTG GATCCTCTGA GCGAGACAAA GTGCACCCTG AAGTCCTTCA CCGTGGAAAA GGGCATCTAC CAGACCAGCA ACTTCCGGGT GCAGCCCACC GAATCCATCG TGCGGTTCCC CAATATCACC AATCTGTGCC CCTTCGGCGA GGTGTTCAAT GCCACCAGAT TCGCCTCTGT GTACGCCTGG AACCGGAAGC GGATCAGCAA TTGCGTGGCC GACTACTCCG TGCTGTACAA CTCCGCCAGC TTCAGCACCT TCAAGTGCTA CGGCGTGTCC CCTACCAAGC TGAACGACCT GTGCTTCACA AACGTGTACG CCGACAGCTT CGTGATCCGG GGAGATGAAG TGCGGCAGAT TGCCCCTGGA CAGACAGGCA AGATCGCCGA CTACAACTAC AAGCTGCCCG ACGACTTCAC CGGCTGTGTG ATTGCCTGGA ACAGCAACAA CCTGGACTCC AAAGTCGGCG GCAACTACAA TTACCTGTAC CGGCTGTTCC GGAAGTCCAA TCTGAAGCCC TTCGAGCGGG ACATCTCCAC CGAGATCTAT CAGGCCGGCA GCACCCCTTG TAACGGCGTG GAAGCCTTCA ACTGCTACTT CCCACTCCAG TCCTACGGCT TTCAGCCCAC ATACGGCGTC GGCTATCAGC CCTACAGAGT GGTGGTGCTG AGCTTCGAAC TGCTGCATGC CCCTGCCACA GTGTGCGGCC CTAAGAAAAG CACCAATCTC GTGAAGAACA AATGCGTGAA CTTCAACTTC AACGGCCTCA CCGGCACCGG CGTCCTCACA GAGAGCAACA AGAAGTTCCT GCCATTCCAG CAGTTTGGCC GGGATATCGA CGATACCACA GACGCCGTTA GAGATCCCCA GACACTGGAA ATCCTGGACA TCACCCCTTG CAGCTTCGGC GGAGTGTCTG TGATCACCCC TGGCACCAAC ACCAGCAATC AGGTGGCAGT GCTGTACCAG GGCGTGAACT GTACCGAAGT GCCCGTGGCC |
| | | ATTCACGCCG ATCAGCTGAC ACCTACATGG CGGGTGTACT CCACCGGCAG CAATGTGTTT CAGACCAGAG CCGGCTGTCT GATCGGAGCC GAGCACGTGA ACAATAGCTA CGAGTGCGAC ATCCCCATCG GCGCTGGAAT CTGCGCCAGC TACCAGACAC AGACAAACAG CCACCGGAGA GCCAGAAGCG TGGCCAGCCA GAGCATCATT GCCTACACAA TGTCTCTGGG CGCCGAGAAC AGCGTGGCCT ACTCCAACAA CTCTATCGCT ATCCCCATCA ACTTCACCAT CAGCGTGACC ACAGAGATCC TGCCTGTGTC CATGACCAAG ACCAGCGTGG ACTGCACCAT GTACATCTGC GGCGATTCCA CCGAGTGCTC CAACCTGCTG CTGCAGTACG GCAGCTTCTG CACCCAGCTG AATAGAGCCC TGACAGGGAT CGCCGTGGAA CAGGACAAGA ACACCCAAGA GGTGTTCGCC CAAGTGAAGC AGATCTACAA GACCCCTCCT ATCAAGGACT TCGGCGGCTT CAATTTCAGC CAGATTCTGC CCGATCCTAG CAAGCCCAGC AAGCGGAGCT TCATCGAGGA CCTGCTGTTC AACAAAGTGA CACTGGCCGA CGCCGGCTTC ATCAAGCAGT ATGGCGATTG TCTGGGCGAC ATTGCCGCCA GGGATCTGAT TTGCGCCCAG AAGTTTAACG GACTGACAGT GCTGCCTCCT CTGCTGACCG ATGAGATGAT CGCCCAGTAC ACATCTGCCC TGCTGGCCGG CACAATCACA AGCGGCTGGA CATTTGGAGC AGGCGCCGCT CTGCAGATCC CCTTTGCTAT GCAGATGGCC TACCGGTTCA ACGGCATCGG AGTGACCCAG AATGTGCTGT ACGAGAACCA GAAGCTGATC GCCAACCAGT TCAACAGCGC CATCGGCAAG ATCCAGGACA GCCTGAGCAG CACAGCAAGC GCCCTGGGAA AGCTGCAGGA CGTGGTCAAC CAGAATGCCC AGGCACTGAA CACCCTGGTC AAGCAGCTGT CCTCCAACTT CGGCGCCATC AGCTCTGTGC TGAACGATAT CCTGGCCAGA CTGGACCCTC CTGAGGCCGA GGTGCAGATC GACAGACTGA TCACAGGCAG ACTGCAGAGC CTCCAGACAT ACGTGACCCA GCAGCTGATC AGAGCCGCCG AGATTAGAGC CTCTGCCAAT CTGGCCGCCA CCAAGATGTC TGAGTGTGTG CTGGGCCAGA GCAAGAGAGT GGACTTTTGC GGCAAGGGCT ACCACCTGAT GAGCTTCCCT CAGTCTGCCC CTCACGGCGT GGTGTTTCTG CACGTGACAT ATGTGCCCGC TCAAGAGAAG AATTTCACCA CCGCTCCAGC CATCTGCCAC GACGGCAAAG CCCACTTTCC TAGAGAAGGC GTGTTCGTGT CCAACGGCAC CCATTGGTTC GTGACACAGC GGAACTTCTA CGAGCCCCAG ATCATCACCA CCCACAACAC CTTCGTGTCT GGCAACTGCG ACGTCGTGAT CGGCATTGTG AACAATACCG TGTACGACCC TCTGCAGCCC GAGCTGGACA GCTTCAAAGA GGAACTGGAC AAGTACTTTA AGAACCACAC AAGCCCCGAC GTGGACCTGG GCGATATCAG CGGAATCAAT GCCAGCGTCG TGAACATCCA GAAAGAGATC GACCGGCTGA ACGAGGTGGC CAAGAATCTG AACGAGAGCC TGATCGACCT GCAAGAACTG GGGAAGTACG AGCAGTACAT CAAGTGGCCC TGGTACATCT GGCTGGGCTT TATCGCCGGA CTGATTGCCA TCGTGATGGT CACAATCATG CTGTGTTGCA TGACCAGCTG CTGTAGCTGC CTGAAGGGCT GTTGTAGCTG TGGCAGCTGC TGCAAGTTCG ACGAGGACGA TTCTGAGCCC GTGCTGAAGG GCGTGAAACT GCACTACACA TGATGA GATCTGCTGG TACTGCATGC ACGCAATGCT AGCTGCCCCT TTCCCGTCCT GGGTACCCCG AGTCTCCCCC GACCTCGGGT CCCAGGTATG CTCCCACCTC CACCTGCCCC ACTCACCACC TCTGCTAGTT CCAGACACCT CCCAAGCACG CAGCAATGCA GCTCAAAACG CTTAGCCTAG CCACACCCCC ACGGGAAACA GCAGTGATTA ACCTTTAGCA ATAAACGAAA GTTTAACTAA GCTATACTAA CCCCAGGGTT GGTCAATTTC GTGCCAGCCA CACCCTGGAG CTAGC |
| | | AAAAAAAAAA AAAAAAAAAA AAAAAAAAAA GCATATGACT AAAAAAAAAA AAAAAAAAAA AAAAAAAAAA AAAAAAAAAA AAAAAAAAAA AAAAAAAAAA AAAAAAAAAA |

### Nucleotide Sequence of RBP020.16 (Delta-specific vaccine)

Nucleotide sequence is shown with individual sequence elements as indicated in bold letters. In addition, the sequence of the translated protein is shown in italic letters below the coding nucleotide sequence (* = stop codon). Red text indicates point mutations in both the nucleotide and amino acid sequences.

10         20         30         40         50 53
AGAATAAACT AGTATTCTTC TGGTCCCCAC AGACTCAGAG AGAACCCGCC ACC
hAg-Kozak

63         73         83         93         103        113
ATGTTCGTGT TCCTGGTGCT GCTGCCTCTG GTGTCCAGCC AGTGTGTGAA CCTGAGAACC
M  F  V   F  L  V   L  L  P  L   V  S  S   Q  C  V   N  L  R  T
S protein mut4

123        133        143        153        163        173
AGAACACAGC TGCCTCCAGC CTACACCAAC AGCTTTACCA GAGGCGTGTA CTACCCCGAC
R  T  Q   L  P  P   A  Y  T  N   S  F  T   R  G  V   Y  Y  P  D
S protein mut4

183        193        203        213        223        233
AAGGTGTTCA GATCCAGCGT GCTGCACTCT ACCCAGGACC TGTTCCTGCC TTTCTTCAGC
K  V  F   R  S  S   V  L  H  S   T  Q  D   L  F  L   P  F  F  S
S protein mut4

243        253        263        273        283        293
AACGTGACCT GGTTCCACGC CATCCACGTG TCCGGCACCA ATGGCACCAA GAGATTCGAC
N  V  T   W  F  H   A  I  H  V   S  G  T   N  G  T   K  R  F  D
S protein mut4

303        313        323        333        343        353
AACCCCGTGC TGCCCTTCAA CGACGGGGTG TACTTTGCCA GCACCGAGAA GTCCAACATC
N  P  V   L  P  F   N  D  G  V   Y  F  A   S  T  E   K  S  N  I
S protein mut4

363        373        383        393        403        413
ATCAGAGGCT GGATCTTCGG CACCACACTG GACAGCAAGA CCCAGAGCCT GCTGATCGTG
I  R  G   W  I  F   G  T  T  L   D  S  K   T  Q  S   L  L  I  V
S protein mut4

423        433        443        453        463        473
AACAACGCCA CCAACGTGGT CATCAAAGTG TGCGAGTTCC AGTTCTGCAA CGACCCCTTC
N  N  A   T  N  V   V  I  K  V   C  E  F   Q  F  C   N  D  P  F
S protein mut4

483        493        503        513        523        533
CTGGACGTCT ACTACCACAA GAACAACAAG AGCTGGATGG AAAGCGGCGT GTACAGCAGC
L  D  V   Y  Y  H   K  N  N  K   S  W  M   E  S  G   V  Y  S  S
S protein mut4

543 553 563 573 583 593
GCCAACAACT GCACCTTCGA GTACGTGTCC CAGCCTTTCC TGATGGACCT GGAAGGCAAG
A N N C T F E Y V S Q P F L M D L E G K
S protein mut4

603 613 623 633 643 653
CAGGGCAACT TCAAGAACCT GCGCGAGTTC GTGTTTAAGA ACATCGACGG CTACTTCAAG
Q G N F K N L R E F V F K N I D G Y F K
S protein mut4

663 673 683 693 703 713
ATCTACAGCA AGCACACCCC TATCAACCTC GTGCGGGATC TGCCTCAGGG CTTCTCTGCT
I Y S K H T P I N L V R D L P Q G F S A
S protein mut4

723 733 743 753 763 773
CTGGAACCCC TGGTGGATCT GCCCATCGGC ATCAACATCA CCCGGTTTCA GACACTGCTG
L E P L V D L P I G I N I T R F Q T L L
S protein mut4

783 793 803 813 823 833
GCCCTGCACA GAAGCTACCT GACACCTGGC GATAGCAGCA GCGGATGGAC AGCTGGTGCC
A L H R S Y L T P G D S S S G W T A G A
S protein mut4

843 853 863 873 883 893
GCCGCTTACT ATGTGGGCTA CCTGCAGCCT AGAACCTTCC TGCTGAAGTA CAACGAGAAC
A A Y Y V G Y L Q P R T F L L K Y N E N
S protein mut4

903 913 923 933 943 953
GGCACCATCA CCGACGCCGT GGATTGTGCT CTGGATCCTC TGAGCGAGAC AAAGTGCACC
G T I T D A V D C A L D P L S E T K C T
S protein mut4

963 973 983 993 1003 1013
CTGAAGTCCT TCACCGTGGA AAAGGGCATC TACCAGACCA GCAACTTCCG GGTGCAGCCC
L K S F T V E K G I Y Q T S N F R V Q P
S protein mut4

1023 1033 1043 1053 1063 1073
ACCGAATCCA TCGTGCGGTT CCCCAATATC ACCAATCTGT GCCCCTTCGG CGAGGTGTTC
T E S I V R F P N I T N L C P F G E V F
S protein mut4

1083 1093 1103 1113 1123 1133
AATGCCACCA GATTCGCCTC TGTGTACGCC TGGAACCGGA AGCGGATCAG CAATTGCGTG
N A T R F A S V Y A W N R K R I S N C V
S protein mut4

1143 1153 1163 1173 1183 1193
GCCGACTACT CCGTGCTGTA CAACTCCGCC AGCTTCAGCA CCTTCAAGTG CTACGGCGTG
A D Y S V L Y N S A S F S T F K C Y G V
S protein mut4

1203 1213 1223 1233 1243 1253
TCCCCTACCA AGCTGAACGA CCTGTGCTTC ACAAACGTGT ACGCCGACAG CTTCGTGATC
S P T K L N D L C F T N V Y A D S F V I
S protein mut4

1263 1273 1283 1293 1303 1313
CGGGGAGATG AAGTGCGGCA GATTGCCCCT GGACAGACAG GCAAGATCGC CGACTACAAC
R G D E V R Q I A P G Q T G K I A D Y N
S protein mut4

1323 1333 1343 1353 1363 1373
TACAAGCTGC CCGACGACTT CACCGGCTGT GTGATTGCCT GGAACAGCAA CAACCTGGAC
Y K L P D D F T G C V I A W N S N N L D
S protein mut4

1383 1393 1403 1413 1423 1433
TCCAAAGTCG GCGGCAACTA CAATTACAGG TACCGGCTGT TCCGGAAGTC CAATCTGAAG
S K V G G N Y N Y R Y R L F R K S N L K
S protein mut4

1443 1453 1463 1473 1483 1493
CCCTTCGAGC GGGACATCTC CACCGAGATC TATCAGGCCG GCAGCAAGCC TTGTAACGGC
P F E R D I S T E I Y Q A G S K P C N G
S protein mut4

1503 1513 1523 1533 1543 1553
GTGGAAGGCT TCAACTGCTA CTTCCCACTG CAGTCCTACG GCTTTCAGCC CACAAATGGC
V E G F N C Y F P L Q S Y G F Q P T N G
S protein mut4

1563 1573 1583 1593 1603 1613
GTGGGCTATC AGCCCTACAG AGTGGTGGTG CTGAGCTTCG AACTGCTGCA TGCCCCTGCC
V G Y Q P Y R V V V L S F E L L H A P A
S protein mut4

1623 1633 1643 1653 1663 1673
ACAGTGTGCG GCCCTAAGAA AAGCACCAAT CTCGTGAAGA ACAAATGCGT GAACTTCAAC
T V C G P K K S T N L V K N K C V N F N
S protein mut4

1683 1693 1703 1713 1723 1733
TTCAACGGCC TGACCGGCAC CGGCGTGCTG ACAGAGAGCA ACAAGAAGTT CCTGCCATTC
F N G L T G T G V L T E S N K K F L P F
S protein mut4

1743 1753 1763 1773 1783 1793
CAGCAGTTTG GCCGGGATAT CGCCGATACC ACAGACGCCG TTAGAGATCC CCAGACACTG
Q Q F G R D I A D T T D A V R D P Q T L
S protein mut4

1803 1813 1823 1833 1843 1853
GAAATCCTGG ACATCACCCC TTGCAGCTTC GGCGGAGTGT CTGTGATCAC CCCTGGCACC
E I L D I T P C S F G G V S V I T P G T
S protein mut4

1863 1873 1883 1893 1903 1913
AACACCAGCA ATCAGGTGGC AGTGCTGTAC CAGGGCGTGA ACTGTACCGA AGTGCCCGTG
N T S N Q V A V L Y Q G V N C T E V P V
S protein mut4

1923 1933 1943 1953 1963 1973
GCCATTCACG CCGATCAGCT GACACCTACA TGGCGGGTGT ACTCCACCGG CAGCAATGTG
A I H A D Q L T P T W R V Y S T G S N V
S protein mut4

1983 1993 2003 2013 2023 2033
TTTCAGACCA GAGCCGGCTG TCTGATCGGA GCCGAGCACG TGAACAATAG CTACGAGTGC
F Q T R A G C L I G A E H V N N S Y E C
S protein mut4

2043 2053 2063 2073 2083 2093
GACATCCCCA TCGGCGCTGG AATCTGCGCC AGCTACCAGA CACAGACAAA CAGCAGGCGG
D I P I G A G I C A S Y Q T Q T N S R R
S protein mut4

2103 2113 2123 2133 2143 2153
AGAGCCAGAA GCGTGGCCAG CCAGAGCATC ATTGCCTACA CAATGTCTCT GGGCGCCGAG
R A R S V A S Q S I I A Y T M S L G A E
S protein mut4

2163 2173 2183 2193 2203 2213
AACAGCGTGG CCTACTCCAA CAACTCTATC GCTATCCCCA CCAACTTCAC CATCAGCGTG
N S V A Y S N N S I A I P T N F T I S V
S protein mut4

2223 2233 2243 2253 2263 2273
ACCACAGAGA TCCTGCCTGT GTCCATGACC AAGACCAGCG TGGACTGCAC CATGTACATC
T T E I L P V S M T K T S V D C T M Y I
S protein mut4

2283 2293 2303 2313 2323 2333
TGCGGCGATT CCACCGAGTG CTCCAACCTG CTGCTGCAGT ACGGCAGCTT CTGCACCCAG
C G D S T E C S N L L L Q Y G S F C T Q
S protein mut4

2343 2353 2363 2373 2383 2393
CTGAATAGAG CCCTGACAGG GATCGCCGTG GAACAGGACA AGAACACCCA AGAGGTGTTC
L N R A L T G I A V E Q D K N T Q E V F
S protein mut4

2403 2413 2423 2433 2443 2453
GCCCAAGTGA AGCAGATCTA CAAGACCCCT CCTATCAAGG ACTTCGGCGG CTTCAATTTC
A Q V K Q I Y K T P P I K D F G G F N F
S protein mut4

2463 2473 2483 2493 2503 2513
AGCCAGATTC TGCCCGATCC TAGCAAGCCC AGCAAGCGGA GCTTCATCGA GGACCTGCTG
S Q I L P D P S K P S K R S F I E D L L
S protein mut4

2523 2533 2543 2553 2563 2573
TTCAACAAAG TGACACTGGC CGACGCCGGC TTCATCAAGC AGTATGGCGA TTGTCTGGGC
F N K V T L A D A G F I K Q Y G D C L G
S protein mut4

2583 2593 2603 2613 2623 2633
GACATTGCCG CCAGGGATCT GATTTGCGCC CAGAAGTTTA ACGGACTGAC AGTGCTGCCT
D I A A R D L I C A Q K F N G L T V L P
S protein mut4

2643 2653 2663 2673 2683 2693
CCTCTGCTGA CCGATGAGAT GATCGCCCAG TACACATCTG CCCTGCTGGC CGGCACAATC
P L L T D E M I A Q Y T S A L L A G T I
S protein mut4

2703 2713 2723 2733 2743 2753
ACAAGCGGCT GGACATTTGG AGCAGGCGCC GCTCTGCAGA TCCCCTTTGC TATGCAGATG
T S G W T F G A G A A L Q I P F A M Q M
S protein mut4

2763 2773 2783 2793 2803 2813
GCCTACCGGT TCAACGGCAT CGGAGTGACC CAGAATGTGC TGTACGAGAA CCAGAAGCTG
A Y R F N G I G V T Q N V L Y E N Q K L
S protein mut4

2823 2833 2843 2853 2863 2873
ATCGCCAACC AGTTCAACAG CGCCATCGGC AAGATCCAGG ACAGCCTGAG CAGCACAGCA
I A N Q F N S A I G K I Q D S L S S T A
S protein mut4

2883 2893 2903 2913 2923 2933
AGCGCCCTGG GAAAGCTGCA GAACGTGGTC AACCAGAATG CCCAGGCACT GAACACCCTG
S A L G K L Q N V V N Q N A Q A L N T L
S protein mut4

2943 2953 2963 2973 2983 2993
GTCAAGCAGC TGTCCTCCAA CTTCGGCGCC ATCAGCTCTG TGCTGAACGA TATCCTGAGC
V K Q L S S N F G A I S S V L N D I L S
S protein mut4

3003 3013 3023 3033 3043 3053
AGACTGGACC CTCCTGAGGC CGAGGTGCAG ATCGACAGAC TGATCACAGG CAGACTGCAG
R L D P P E A E V Q I D R L I T G R L Q
S protein mut4

3063 3073 3083 3093 3103 3113
AGCCTCCAGA CATACGTGAC CCAGCAGCTG ATCAGAGCCG CCGAGATTAG AGCCTCTGCC
S L Q T Y V T Q Q L I R A A E I R A S A
S protein mut4

3123 3133 3143 3153 3163 3173
AATCTGGCCG CCACCAAGAT GTCTGAGTGT GTGCTGGGCC AGAGCAAGAG AGTGGACTTT
N L A A T K M S E C V L G Q S K R V D F
S protein mut4

3183 3193 3203 3213 3223 3233
TGCGGCAAGG GCTACCACCT GATGAGCTTC CCTCAGTCTG CCCCTCACGG CGTGGTGTTT
C G K G Y H L M S F P Q S A P H G V V F
S protein mut4

3243 3253 3263 3273 3283 3293
CTGCACGTGA CATATGTGCC CGCTCAAGAG AAGAATTTCA CCACCGCTCC AGCCATCTGC
L H V T Y V P A Q E K N F T T A P A I C
S protein mut4

3303 3313 3323 3333 3343 3353
CACGACGGCA AAGCCCACTT TCCTAGAGAA GGCGTGTTCG TGTCCAACGG CACCCATTGG
H D G K A H F P R E G V F V S N G T H W
S protein mut4

3363 3373 3383 3393 3403 3413
TTCGTGACAC AGCGGAACTT CTACGAGCCC CAGATCATCA CCACCGACAA CACCTTCGTG
F V T Q R N F Y E P Q I I T T D N T F V
S protein mut4

3423 3433 3443 3453 3463 3473
TCTGGCAACT GCGACGTCGT GATCGGCATT GTGAACAATA CCGTGTACGA CCCTCTGCAG
S G N C D V V I G I V N N T V Y D P L Q
S protein mut4

3483 3493 3503 3513 3523 3533
CCCGAGCTGG ACAGCTTCAA AGAGGAACTG GACAAGTACT TTAAGAACCA CACAAGCCCC
P E L D S F K E E L D K Y F K N H T S P
S protein mut4

3543 3553 3563 3573 3583 3593
GACGTGGACC TGGGCGATAT CAGCGGAATC AATGCCAGCG TCGTGAACAT CCAGAAAGAG
D V D L G D I S G I N A S V V N I Q K E
S protein mut4

3603 3613 3623 3633 3643 3653
ATCGACCGGC TGAACGAGGT GGCCAAGAAT CTGAACGAGA GCCTGATCGA CCTGCAAGAA
I D R L N E V A K N L N E S L I D L Q E
S protein mut4

3663 3673 3683 3693 3703 3713
CTGGGGAAGT ACGAGCAGTA CATCAAGTGG CCCTGGTACA TCTGGCTGGG CTTTATCGCC
L G K Y E Q Y I K W P W Y I W L G F I A
S protein mut4

3723 3733 3743 3753 3763 3773
GGACTGATTG CCATCGTGAT GGTCACAATC ATGCTGTGTT GCATGACCAG CTGCTGTAGC
G L I A I V M V T I M L C C M T S C C S
S protein mut4

3783 3793 3803 3813 3823 3833
TGCCTGAAGG GCTGTTGTAG CTGTGGCAGC TGCTGCAAGT TCGACGAGGA CGATTCTGAG
C L K G C C S C G S C C K F D E D D S E
S protein mut4

3843 3853 3863 3872
CCCGTGCTGA AGGGCGTGAA ACTGCACTAC ACATGATGA
P V L K G V K L H Y T * *
S protein mut4

3882 3892 3902 3912 3922 3932
TTTCACCTGG TACTGCATGC ACGCAATGCT AGCTGCCCCT TTCCCGTCCT GGGTACCCCG
FI element

3942 3952 3962 3972 3982 3992
AGTCTCCCCC GACCTCGGGT CCCAGGTATG CTCCCACCTC CACCTGCCCC ACTCACCACC
FI element

4002 4012 4022 4032 4042 4052
TCTGCTAGTT CCAGACACCT CCCAAGCACG CAGCAATGCA GCTCAAAACG CTTAGCCTAG
FI element

4062 4072 4082 4092 4102 4112
CCACACCCCC ACGGGAAACA GCAGTGATTA ACCTTTAGCA ATAAACGAAA GTTTAACTAA
FI element

4122 4132 4142 4152 4162 4167
GCTATACTAA CCCCAGGGTT GGTCAATTTC GTGCCAGCCA CACCCTGGAG CTAGC
FI element

4177 4187 4197 4207 4217 4227
AAAAAAAAAA AAAAAAAAAA AAAAAAAAAA GCATATGACT AAAAAAAAAA AAAAAAAAAA
Poly(A)

4237 4247 4257 4267 4277
AAAAAAAAAA AAAAAAAAAA AAAAAAAAAA AAAAAAAAAA AAAAAAAAAA
Poly(A)

Sequences of RBP020.14 are also shown in Table 5.

**Table 5: Sequences of RBP020.16 (Delta-specific RNA vaccine)**

| SEQ ID NO. | Brief Description | Sequence |
|---|---|---|
| 61 | Amino acid sequence of RNA-encoded SARS-CoV-2 S protein from a Delta variant (with PRO mutations at positions corresponding to K986P and V987P of SEQ ID NO: 1; i.e., PRO mutations at positions 984 and 985 of SEQ ID NO: 61) | MFVFLVLLPLVSSQCVNLRTRTQLPPAYTNSFTRGVYYPDKVFRSSVLHSTQDLFLPFFSNVTWF HAIHVSGTNGTKRFDNPVLPFNDGVYFASTEKSNIIRGWIFGTTLDSKTQSLLIVNNATNVVIKV CEFQFCNDPFLDVYYHKNNKSWMESGVYSSANNCTFEYVSQPFLMDLEGKQGNFKNLREFVFKNI DGYFKIYSKHTPINLVRDLPQGFSALEPLVDLPIGINITRFQTLLALHRSYLTPGDSSSGWTAGA AAYYVGYLQPRTFLLKYNENGTITDAVDCALDPLSETKCTLKSFTVEKGIYQTSNFRVQPTESIV RFPNITNLCPFGEVFNATRFASVYAWNRKRISNCVADYSVLYNSASFSTFKCYGVSPTKLNDLCF TNVYADSFVIRGDEVRQIAPGQTGKIADYNYKLPDDFTGCVIAWNSNNLDSKVGGNYNYRYRLFR KSNLKPFERDISTEIYQAGSKPCNGVEGFNCYFPLQSYGFQPTNGVGYQPYRVVVLSFELLHAPA TVCGPKKSTNLVKNKCVNFNFNGLTGTGVLTESNKKFLPFQQFGRDIADTTDAVRDPQTLEILDI TPCSFGGVSVITPGTNTSNQVAVLYQGVNCTEVPVAIHADQLTPTWRVYSTGSNVFQTRAGCLIG AEHVNNSYECDIPIGAGICASYQTQTNSRRRARSVASQSIIAYTMSLGAENSVAYSNNSIAIPTN FTISVTTEILPVSMTKTSVDCTMYICGDSTECSNLLLQYGSFCTQLNRALTGIAVEQDKNTQEVF AQVKQIYKTPPIKDFGGFNFSQILPDPSKPSKRSFIEDLLFNKVTLADAGFIKQYGDCLGDIAAR DLICAQKFNGLTVLPPLLTDEMIAQYTSALLAGTITSGWTFGAGAALQIPFAMQMAYRFNGIGVT QNVLYENQKLIANQFNSAIGKIQDSLSSTASALGKLQNVVNQNAQALNTLVKQLSSNFGAISSVL NDILSRLDPPEAEVQIDRLITGRLQSLQTYVTQQLIRAAEIRASANLAATKMSECVLGQSKRVDF CGKGYHLMSFPQSAPHGVVFLHVTYVPAQEKNFTTAPAICHDGKAHFPREGVFVSNGTHWFVTQR NFYEPQIITTDNTFVSGNCDVVIGIVNNTVYDPLQPELDSFKEELDKYFKNHTSPDVDLGDISGI NASVVNIQKEIDRLNEVAKNLNESLIDLQELGKYEQYIKWPWYIWLGFIAGLIAIVMVTIMLCCM TSCCSCLKGCCSCGSCCKFDEDDSEPVLKGVKLHYT-- |
| 62 | Sequence encoding a SARS-CoV-2 S protein from a Delta variant | ATGTTCGTGT TCCTGGTGCT GCTGCCTCTG GTGTCCAGCC AGTGTGTGAA CCTGAGAACC AGAACACAGC TGCCTCCAGC CTACACCAAC AGCTTTACCA GAGGCGTGTA CTACCCCGAC AAGGTGTTCA GATCCAGCGT GCTGCACTCT ACCCAGGACC TGTTCCTGCC TTTCTTCAGC AACGTGACCT GGTTCCACGC CATCCACGTG TCCGGCACCA ATGGCACCAA GAGATTCGAC AACCCCGTGC TGCCCTTCAA CGACGGGGTG TACTTTGCCA GCACCGAGAA GTCCAACATC ATCAGAGGCT GGATCTTCGG CACCACACTG GACAGCAAGA CCCAGAGCCT GCTGATCGTG |
| | | AACAACGCCA CCAACGTGGT CATCAAAGTG TGCGAGTTCC AGTTCTGCAA CGACCCCTTC CTGGACGTCT ACTACCACAA GAACAACAAG AGCTGGATGG AAAGCGGCGT GTACAGCAGC GCCAACAACT GCACCTTCGA GTACGTGTCC CAGCCTTTCC TGATGGACCT GGAAGGCAAG CAGGGCAACT TCAAGAACCT GCGCGAGTTC GTGTTTAAGA ACATCGACGG CTACTTCAAG ATCTACAGCA AGCACACCCC TATCAACCTC GTGCGGGATC TGCCTCAGGG CTTCTCTGCT CTGGAACCCC TGGTGGATCT GCCCATCGGC ATCAACATCA CCCGGTTTCA GACACTGCTG GCCCTGCACA GAAGCTACCT GACACCTGGC GATAGCAGCA GCGGATGGAC AGCTGGTGCC GCCGCTTACT ATGTGGGCTA CCTGCAGCCT AGAACCTTCC TGCTGAAGTA CAACGAGAAC GGCACCATCA CCGACGCCGT GGATTGTGCT CTGGATCCTC TGAGCGAGAC AAAGTGCACC CTGAAGTCCT TCACCGTGGA AAAGGGCATC TACCAGACCA GCAACTTCCG GGTGCAGCCC ACCGAATCCA TCGTGCGGTT CCCCAATATC ACCAATCTGT GCCCCTTCGG CGAGGTGTTC AATGCCACCA GATTCGCCTC TGTGTACGCC TGGAACCGGA AGCGGATCAG CAATTGCGTG GCCGACTACT CCGTGCTGTA CAACTCCGCC AGCTTCAGCA CCTTCAAGTG CTACGGCGTG TCCCCTACCA AGCTGAACGA CCTGTGCTTC ACAAACGTGT ACGCCGACAG CTTCGTGATC CGGGGAGATG AAGTGCGGCA GATTGCCCCT GGACAGACAG GCAAGATCGC CGACTACAAC TACAAGCTGC CCGACGACTT CACCGGCTGT GTGATTGCCT GGAACAGCAA CAACCTGGAC TCCAAAGTCG GCGGCAACTA CAATTACAGG TACCGGCTGT TCCGGAAGTC CAATCTGAAG CCCTTCGAGC GGGACATCTC CACCGAGATC TATCAGGCCG GCAGCAAGCC TTGTAACGGC GTGGAAGGCT TCAACTGCTA CTTCCCACTG CAGTCCTACG GCTTTCAGCC CACAAATGGC GTGGGCTATC AGCCCTACAG AGTGGTGGTG CTGAGCTTCG AACTGCTGCA TGCCCCTGCC ACAGTGTGCG GCCCTAAGAA AAGCACCAAT CTCGTGAAGA ACAAATGCGT GAACTTCAAC TTCAACGGCC TGACCGGCAC CGGCGTGCTG ACAGAGAGCA ACAAGAAGTT CCTGCCATTC CAGCAGTTTG GCCGGGATAT CGCCGATACC ACAGACGCCG TTAGAGATCC CCAGACACTG GAAATCCTGG ACATCACCCC TTGCAGCTTC GGCGGAGTGT CTGTGATCAC CCCTGGCACC AACACCAGCA ATCAGGTGGC AGTGCTGTAC CAGGGCGTGA ACTGTACCGA AGTGCCCGTG GCCATTCACG CCGATCAGCT GACACCTACA TGGCGGGTGT ACTCCACCGG CAGCAATGTG TTTCAGACCA GAGCCGGCTG TCTGATCGGA GCCGAGCACG TGAACAATAG CTACGAGTGC GACATCCCCA TCGGCGCTGG AATCTGCGCC AGCTACCAGA CACAGACAAA CAGCAGGCGG AGAGCCAGAA GCGTGGCCAG CCAGAGCATC ATTGCCTACA CAATGTCTCT GGGCGCCGAG AACAGCGTGG CCTACTCCAA CAACTCTATC GCTATCCCCA CCAACTTCAC CATCAGCGTG ACCACAGAGA TCCTGCCTGT GTCCATGACC AAGACCAGCG TGGACTGCAC CATGTACATC TGCGGCGATT CCACCGAGTG CTCCAACCTG CTGCTGCAGT ACGGCAGCTT CTGCACCCAG CTGAATAGAG CCCTGACAGG GATCGCCGTG GAACAGGACA AGAACACCCA AGAGGTGTTC GCCCAAGTGA AGCAGATCTA CAAGACCCCT CCTATCAAGG ACTTCGGCGG CTTCAATTTC AGCCAGATTC TGCCCGATCC TAGCAAGCCC AGCAAGCGGA GCTTCATCGA GGACCTGCTG TTCAACAAAG TGACACTGGC CGACGCCGGC TTCATCAAGC AGTATGGCGA TTGTCTGGGC GACATTGCCG CCAGGGATCT GATTTGCGCC CAGAAGTTTA ACGGACTGAC AGTGCTGCCT CCTCTGCTGA CCGATGAGAT GATCGCCCAG TACACATCTG CCCTGCTGGC CGGCACAATC ACAAGCGGCT GGACATTTGG AGCAGGCGCC GCTCTGCAGA TCCCCTTTGC TATGCAGATG GCCTACCGGT TCAACGGCAT CGGAGTGACC CAGAATGTGC TGTACGAGAA CCAGAAGCTG |
| | | ATCGCCAACC AGTTCAACAG CGCCATCGGC AAGATCCAGG ACAGCCTGAG CAGCACAGCA AGCGCCCTGG GAAAGCTGCA GAACGTGGTC AACCAGAATG CCCAGGCACT GAACACCCTG GTCAAGCAGC TGTCCTCCAA CTTCGGCGCC ATCAGCTCTG TGCTGAACGA TATCCTGAGC AGACTGGACC CTCCTGAGGC CGAGGTGCAG ATCGACAGAC TGATCACAGG CAGACTGCAG AGCCTCCAGA CATACGTGAC CCAGCAGCTG ATCAGAGCCG CCGAGATTAG AGCCTCTGCC AATCTGGCCG CCACCAAGAT GTCTGAGTGT GTGCTGGGCC AGAGCAAGAG AGTGGACTTT TGCGGCAAGG GCTACCACCT GATGAGCTTC CCTCAGTCTG CCCCTCACGG CGTGGTGTTT CTGCACGTGA CATATGTGCC CGCTCAAGAG AAGAATTTCA CCACCGCTCC AGCCATCTGC CACGACGGCA AAGCCCACTT TCCTAGAGAA GGCGTGTTCG TGTCCAACGG CACCCATTGG TTCGTGACAC AGCGGAACTT CTACGAGCCC CAGATCATCA CCACCGACAA CACCTTCGTG TCTGGCAACT GCGACGTCGT GATCGGCATT GTGAACAATA CCGTGTACGA CCCTCTGCAG CCCGAGCTGG ACAGCTTCAA AGAGGAACTG GACAAGTACT TTAAGAACCA CACAAGCCCC GACGTGGACC TGGGCGATAT CAGCGGAATC AATGCCAGCG TCGTGAACAT CCAGAAAGAG ATCGACCGGC TGAACGAGGT GGCCAAGAAT CTGAACGAGA GCCTGATCGA CCTGCAAGAA CTGGGGAAGT ACGAGCAGTA CATCAAGTGG CCCTGGTACA TCTGGCTGGG CTTTATCGCC GGACTGATTG CCATCGTGAT GGTCACAATC ATGCTGTGTT GCATGACCAG CTGCTGTAGC TGCCTGAAGG GCTGTTGTAG CTGTGGCAGC TGCTGCAAGT TCGACGAGGA CGATTCTGAG CCCGTGCTGA AGGGCGTGAA ACTGCACTAC ACATGATGA |
| 62a | RNA sequence encoding a SARS-CoV-2 S protein from a Delta variant | AUGUUCGUGU UCCUGGUGCU GCUGCCUCUG GUGUCCAGCC AGUGUGUGAA CCUGAGAACC AGAACACAGC UGCCUCCAGC CUACACCAAC AGCUUUACCA GAGGCGUGUA CUACCCCGAC AAGGUGUUCA GAUCCAGCGU GCUGCACUCU ACCCAGGACC UGUUCCUGCC UUUCUUCAGC AACGUGACCU GGUUCCACGC CAUCCACGUG UCCGGCACCA AUGGCACCAA GAGAUUCGAC AACCCCGUGC UGCCCUUCAA CGACGGGGUG UACUUUGCCA GCACCGAGAA GUCCAACAUC AUCAGAGGCU GGAUCUUCGG CACCACACUG GACAGCAAGA CCCAGAGCCU GCUGAUCGUG AACAACGCCA CCAACGUGGU CAUCAAAGUG UGCGAGUUCC AGUUCUGCAA CGACCCCUUC CUGGACGUCU ACUACCACAA GAACAACAAG AGCUGGAUGG AAAGCGGCGU GUACAGCAGC GCCAACAACU GCACCUUCGA GUACGUGUCC CAGCCUUUCC UGAUGGACCU GGAAGGCAAG CAGGGCAACU UCAAGAACCU GCGCGAGUUC GUGUUUAAGA ACAUCGACGG CUACUUCAAG AUCUACAGCA AGCACACCCC UAUCAACCUC GUGCGGGAUC UGCCUCAGGG CUUCUCUGCU CUGGAACCCC UGGUGGAUCU GCCCAUCGGC AUCAACAUCA CCCGGUUUCA GACACUGCUG GCCCUGCACA GAAGCUACCU GACACCUGGC GAUAGCAGCA GCGGAUGGAC AGCUGGUGCC GCCGCUUACU AUGUGGGCUA CCUGCAGCCU AGAACCUUCC UGCUGAAGUA CAACGAGAAC GGCACCAUCA CCGACGCCGU GGAUUGUGCU CUGGAUCCUC UGAGCGAGAC AAAGUGCACC CUGAAGUCCU UCACCGUGGA AAAGGGCAUC UACCAGACCA GCAACUUCCG GGUGCAGCCC ACCGAAUCCA UCGUGCGGUU CCCCAAUAUC ACCAAUCUGU GCCCCUUCGG CGAGGUGUUC AAUGCCACCA GAUUCGCCUC UGUGUACGCC UGGAACCGGA AGCGGAUCAG CAAUUGCGUG GCCGACUACU CCGUGCUGUA CAACUCCGCC AGCUUCAGCA CCUUCAAGUG CUACGGCGUG UCCCCUACCA AGCUGAACGA CCUGUGCUUC ACAAACGUGU ACGCCGACAG CUUCGUGAUC CGGGGAGAUG AAGUGCGGCA GAUUGCCCCU GGACAGACAG GCAAGAUCGC CGACUACAAC |
| | | UACAAGCUGC CCGACGACUU CACCGGCUGU GUGAUUGCCU GGAACAGCAA CAACCUGGAC UCCAAAGUCG GCGGCAACUA CAAUUACAGG UACCGGCUGU UCCGGAAGUC CAAUCUGAAG CCCUUCGAGC GGGACAUCUC CACCGAGAUC UAUCAGGCCG GCAGCAAGCC UUGUAACGGC GUGGAAGGCU UCAACUGCUA CUUCCCACUG CAGUCCUACG GCUUUCAGCC CACAAAUGGC GUGGGCUAUC AGCCCUACAG AGUGGUGGUG CUGAGCUUCG AACUGCUGCA UGCCCCUGCC ACAGUGUGCG GCCCUAAGAA AAGCACCAAU CUCGUGAAGA ACAAAUGCGU GAACUUCAAC UUCAACGGCC UGACCGGCAC CGGCGUGCUG ACAGAGAGCA ACAAGAAGUU CCUGCCAUUC CAGCAGUUUG GCCGGGAUAU CGCCGAUACC ACAGACGCCG UUAGAGAUCC CCAGACACUG GAAAUCCUGG ACAUCACCCC UUGCAGCUUC GGCGGAGUGU CUGUGAUCAC CCCUGGCACC AACACCAGCA AUCAGGUGGC AGUGCUGUAC CAGGGCGUGA ACUGUACCGA AGUGCCCGUG GCCAUUCACG CCGAUCAGCU GACACCUACA UGGCGGGUGU ACUCCACCGG CAGCAAUGUG UUUCAGACCA GAGCCGGCUG UCUGAUCGGA GCCGAGCACG UGAACAAUAG CUACGAGUGC GACAUCCCCA UCGGCGCUGG AAUCUGCGCC AGCUACCAGA CACAGACAAA CAGCAGGCGG AGAGCCAGAA GCGUGGCCAG CCAGAGCAUC AUUGCCUACA CAAUGUCUCU GGGCGCCGAG AACAGCGUGG CCUACUCCAA CAACUCUAUC GCUAUCCCCA CCAACUUCAC CAUCAGCGUG ACCACAGAGA UCCUGCCUGU GUCCAUGACC AAGACCAGCG UGGACUGCAC CAUGUACAUC UGCGGCGAUU CCACCGAGUG CUCCAACCUG CUGCUGCAGU ACGGCAGCUU CUGCACCCAG CUGAAUAGAG CCCUGACAGG GAUCGCCGUG GAACAGGACA AGAACACCCA AGAGGUGUUC GCCCAAGUGA AGCAGAUCUA CAAGACCCCU CCUAUCAAGG ACUUCGGCGG CUUCAAUUUC AGCCAGAUUC UGCCCGAUCC UAGCAAGCCC AGCAAGCGGA GCUUCAUCGA GGACCUGCUG UUCAACAAAG UGACACUGGC CGACGCCGGC UUCAUCAAGC AGUAUGGCGA UUGUCUGGGC GACAUUGCCG CCAGGGAUCU GAUUUGCGCC CAGAAGUUUA ACGGACUGAC AGUGCUGCCU CCUCUGCUGA CCGAUGAGAU GAUCGCCCAG UACACAUCUG CCCUGCUGGC CGGCACAAUC ACAAGCGGCU GGACAUUUGG AGCAGGCGCC GCUCUGCAGA UCCCCUUUGC UAUGCAGAUG GCCUACCGGU UCAACGGCAU CGGAGUGACC CAGAAUGUGC UGUACGAGAA CCAGAAGCUG AUCGCCAACC AGUUCAACAG CGCCAUCGGC AAGAUCCAGG ACAGCCUGAG CAGCACAGCA AGCGCCCUGG GAAAGCUGCA GAACGUGGUC AACCAGAAUG CCCAGGCACU GAACACCCUG GUCAAGCAGC UGUCCUCCAA CUUCGGCGCC AUCAGCUCUG UGCUGAACGA UAUCCUGAGC AGACUGGACC CUCCUGAGGC CGAGGUGCAG AUCGACAGAC UGAUCACAGG CAGACUGCAG AGCCUCCAGA CAUACGUGAC CCAGCAGCUG AUCAGAGCCG CCGAGAUUAG AGCCUCUGCC AAUCUGGCCG CCACCAAGAU GUCUGAGUGU GUGCUGGGCC AGAGCAAGAG AGUGGACUUU UGCGGCAAGG GCUACCACCU GAUGAGCUUC CCUCAGUCUG CCCCUCACGG CGUGGUGUUU CUGCACGUGA CAUAUGUGCC CGCUCAAGAG AAGAAUUUCA CCACCGCUCC AGCCAUCUGC CACGACGGCA AAGCCCACUU UCCUAGAGAA GGCGUGUUCG UGUCCAACGG CACCCAUUGG UUCGUGACAC AGCGGAACUU CUACGAGCCC CAGAUCAUCA CCACCGACAA CACCUUCGUG UCUGGCAACU GCGACGUCGU GAUCGGCAUU GUGAACAAUA CCGUGUACGA CCCUCUGCAG CCCGAGCUGG ACAGCUUCAA AGAGGAACUG GACAAGUACU UUAAGAACCA CACAAGCCCC GACGUGGACC UGGGCGAUAU CAGCGGAAUC AAUGCCAGCG UCGUGAACAU CCAGAAAGAG AUCGACCGGC UGAACGAGGU GGCCAAGAAU CUGAACGAGA GCCUGAUCGA CCUGCAAGAA CUGGGGAAGU ACGAGCAGUA CAUCAAGUGG CCCUGGUACA UCUGGCUGGG CUUUAUCGCC |
| | | GGACUGAUUG CCAUCGUGAU GGUCACAAUC AUGCUGUGUU GCAUGACCAG CUGCUGUAGC UGCCUGAAGG GCUGUUGUAG CUGUGGCAGC UGCUGCAAGU UCGACGAGGA CGAUUCUGAG CCCGUGCUGA AGGGCGUGAA ACUGCACUAC ACAUGAUGA |
| 63 | Full length sequence of RBP020.14 | AGAATAAACT AGTATTCTTC TGGTCCCCAC AGACTCAGAG AGAACCCGCC ACC ATGTTCGTGT TCCTGGTGCT GCTGCCTCTG GTGTCCAGCC AGTGTGTGAA CCTGAGAACC AGAACACAGC TGCCTCCAGC CTACACCAAC AGCTTTACCA GAGGCGTGTA CTACCCCGAC AAGGTGTTCA GATCCAGCGT GCTGCACTCT ACCCAGGACC TGTTCCTGCC TTTCTTCAGC AACGTGACCT GGTTCCACGC CATCCACGTG TCCGGCACCA ATGGCACCAA GAGATTCGAC AACCCCGTGC TGCCCTTCAA CGACGGGGTG TACTTTGCCA GCACCGAGAA GTCCAACATC ATCAGAGGCT GGATCTTCGG CACCACACTG GACAGCAAGA CCCAGAGCCT GCTGATCGTG AACAACGCCA CCAACGTGGT CATCAAAGTG TGCGAGTTCC AGTTCTGCAA CGACCCCTTC CTGGACGTCT ACTACCACAA GAACAACAAG AGCTGGATGG AAAGCGGCGT GTACAGCAGC GCCAACAACT GCACCTTCGA GTACGTGTCC CAGCCTTTCC TGATGGACCT GGAAGGCAAG CAGGGCAACT TCAAGAACCT GCGCGAGTTC GTGTTTAAGA ACATCGACGG CTACTTCAAG ATCTACAGCA AGCACACCCC TATCAACCTC GTGCGGGATC TGCCTCAGGG CTTCTCTGCT CTGGAACCCC TGGTGGATCT GCCCATCGGC ATCAACATCA CCCGGTTTCA GACACTGCTG GCCCTGCACA GAAGCTACCT GACACCTGGC GATAGCAGCA GCGGATGGAC AGCTGGTGCC GCCGCTTACT ATGTGGGCTA CCTGCAGCCT AGAACCTTCC TGCTGAAGTA CAACGAGAAC GGCACCATCA CCGACGCCGT GGATTGTGCT CTGGATCCTC TGAGCGAGAC AAAGTGCACC CTGAAGTCCT TCACCGTGGA AAAGGGCATC TACCAGACCA GCAACTTCCG GGTGCAGCCC ACCGAATCCA TCGTGCGGTT CCCCAATATC ACCAATCTGT GCCCCTTCGG CGAGGTGTTC AATGCCACCA GATTCGCCTC TGTGTACGCC TGGAACCGGA AGCGGATCAG CAATTGCGTG GCCGACTACT CCGTGCTGTA CAACTCCGCC AGCTTCAGCA CCTTCAAGTG CTACGGCGTG TCCCCTACCA AGCTGAACGA CCTGTGCTTC ACAAACGTGT ACGCCGACAG CTTCGTGATC CGGGGAGATG AAGTGCGGCA GATTGCCCCT GGACAGACAG GCAAGATCGC CGACTACAAC TACAAGCTGC CCGACGACTT CACCGGCTGT GTGATTGCCT GGAACAGCAA CAACCTGGAC TCCAAAGTCG GCGGCAACTA CAATTACAGG TACCGGCTGT TCCGGAAGTC CAATCTGAAG CCCTTCGAGC GGGACATCTC CACCGAGATC TATCAGGCCG GCAGCAAGCC TTGTAACGGC GTGGAAGGCT TCAACTGCTA CTTCCCACTG CAGTCCTACG GCTTTCAGCC CACAAATGGC GTGGGCTATC AGCCCTACAG AGTGGTGGTG CTGAGCTTCG AACTGCTGCA TGCCCCTGCC ACAGTGTGCG GCCCTAAGAA AAGCACCAAT CTCGTGAAGA ACAAATGCGT GAACTTCAAC TTCAACGGCC TGACCGGCAC CGGCGTGCTG ACAGAGAGCA ACAAGAAGTT CCTGCCATTC CAGCAGTTTG GCCGGGATAT CGCCGATACC ACAGACGCCG TTAGAGATCC CCAGACACTG GAAATCCTGG ACATCACCCC TTGCAGCTTC GGCGGAGTGT CTGTGATCAC CCCTGGCACC AACACCAGCA ATCAGGTGGC AGTGCTGTAC CAGGGCGTGA ACTGTACCGA AGTGCCCGTG GCCATTCACG CCGATCAGCT GACACCTACA TGGCGGGTGT ACTCCACCGG CAGCAATGTG TTTCAGACCA GAGCCGGCTG TCTGATCGGA GCCGAGCACG TGAACAATAG CTACGAGTGC GACATCCCCA TCGGCGCTGG AATCTGCGCC AGCTACCAGA CACAGACAAA CAGCAGGCGG |
| | | AGAGCCAGAA GCGTGGCCAG CCAGAGCATC ATTGCCTACA CAATGTCTCT GGGCGCCGAG AACAGCGTGG CCTACTCCAA CAACTCTATC GCTATCCCCA CCAACTTCAC CATCAGCGTG ACCACAGAGA TCCTGCCTGT GTCCATGACC AAGACCAGCG TGGACTGCAC CATGTACATC TGCGGCGATT CCACCGAGTG CTCCAACCTG CTGCTGCAGT ACGGCAGCTT CTGCACCCAG CTGAATAGAG CCCTGACAGG GATCGCCGTG GAACAGGACA AGAACACCCA AGAGGTGTTC GCCCAAGTGA AGCAGATCTA CAAGACCCCT CCTATCAAGG ACTTCGGCGG CTTCAATTTC AGCCAGATTC TGCCCGATCC TAGCAAGCCC AGCAAGCGGA GCTTCATCGA GGACCTGCTG TTCAACAAAG TGACACTGGC CGACGCCGGC TTCATCAAGC AGTATGGCGA TTGTCTGGGC GACATTGCCG CCAGGGATCT GATTTGCGCC CAGAAGTTTA ACGGACTGAC AGTGCTGCCT CCTCTGCTGA CCGATGAGAT GATCGCCCAG TACACATCTG CCCTGCTGGC CGGCACAATC ACAAGCGGCT GGACATTTGG AGCAGGCGCC GCTCTGCAGA TCCCCTTTGC TATGCAGATG GCCTACCGGT TCAACGGCAT CGGAGTGACC CAGAATGTGC TGTACGAGAA CCAGAAGCTG ATCGCCAACC AGTTCAACAG CGCCATCGGC AAGATCCAGG ACAGCCTGAG CAGCACAGCA AGCGCCCTGG GAAAGCTGCA GAACGTGGTC AACCAGAATG CCCAGGCACT GAACACCCTG GTCAAGCAGC TGTCCTCCAA CTTCGGCGCC ATCAGCTCTG TGCTGAACGA TATCCTGAGC AGACTGGACC CTCCTGAGGC CGAGGTGCAG ATCGACAGAC TGATCACAGG CAGACTGCAG AGCCTCCAGA CATACGTGAC CCAGCAGCTG ATCAGAGCCG CCGAGATTAG AGCCTCTGCC AATCTGGCCG CCACCAAGAT GTCTGAGTGT GTGCTGGGCC AGAGCAAGAG AGTGGACTTT TGCGGCAAGG GCTACCACCT GATGAGCTTC CCTCAGTCTG CCCCTCACGG CGTGGTGTTT CTGCACGTGA CATATGTGCC CGCTCAAGAG AAGAATTTCA CCACCGCTCC AGCCATCTGC CACGACGGCA AAGCCCACTT TCCTAGAGAA GGCGTGTTCG TGTCCAACGG CACCCATTGG TTCGTGACAC AGCGGAACTT CTACGAGCCC CAGATCATCA CCACCGACAA CACCTTCGTG TCTGGCAACT GCGACGTCGT GATCGGCATT GTGAACAATA CCGTGTACGA CCCTCTGCAG CCCGAGCTGG ACAGCTTCAA AGAGGAACTG GACAAGTACT TTAAGAACCA CACAAGCCCC GACGTGGACC TGGGCGATAT CAGCGGAATC AATGCCAGCG TCGTGAACAT CCAGAAAGAG ATCGACCGGC TGAACGAGGT GGCCAAGAAT CTGAACGAGA GCCTGATCGA CCTGCAAGAA CTGGGGAAGT ACGAGCAGTA CATCAAGTGG CCCTGGTACA TCTGGCTGGG CTTTATCGCC GGACTGATTG CCATCGTGAT GGTCACAATC ATGCTGTGTT GCATGACCAG CTGCTGTAGC TGCCTGAAGG GCTGTTGTAG CTGTGGCAGC TGCTGCAAGT TCGACGAGGA CGATTCTGAG CCCGTGCTGA AGGGCGTGAA ACTGCACTAC ACATGATGA TTTCACCTGG TACTGCATGC ACGCAATGCT AGCTGCCCCT TTCCCGTCCT GGGTACCCCG AGTCTCCCCC GACCTCGGGT CCCAGGTATG CTCCCACCTC CACCTGCCCC ACTCACCACC TCTGCTAGTT CCAGACACCT CCCAAGCACG CAGCAATGCA GCTCAAAACG CTTAGCCTAG CCACACCCCC ACGGGAAACA GCAGTGATTA ACCTTTAGCA ATAAACGAAA GTTTAACTAA GCTATACTAA CCCCAGGGTT GGTCAATTTC GTGCCAGCCA CACCCTGGAG CTAGC AAAAAAAAAA AAAAAAAAAA AAAAAAAAAA GCATATGACT AAAAAAAAAA AAAAAAAAAA AAAAAAAAAA AAAAAAAAAA AAAAAAAAAA AAAAAAAAAA AAAAAAAAAA |
| 63a | Full length sequence of RBP020.14 (RNA) | AGAAUAAACU AGUAUUCUUC UGGUCCCCAC AGACUCAGAG AGAACCCGCC ACC AUGUUCGUGU UCCUGGUGCU GCUGCCUCUG GUGUCCAGCC AGUGUGUGAA CCUGAGAACC AGAACACAGC UGCCUCCAGC CUACACCAAC AGCUUUACCA GAGGCGUGUA CUACCCCGAC AAGGUGUUCA GAUCCAGCGU GCUGCACUCU ACCCAGGACC UGUUCCUGCC UUUCUUCAGC AACGUGACCU GGUUCCACGC CAUCCACGUG UCCGGCACCA AUGGCACCAA GAGAUUCGAC AACCCCGUGC UGCCCUUCAA CGACGGGGUG UACUUUGCCA GCACCGAGAA GUCCAACAUC AUCAGAGGCU GGAUCUUCGG CACCACACUG GACAGCAAGA CCCAGAGCCU GCUGAUCGUG AACAACGCCA CCAACGUGGU CAUCAAAGUG UGCGAGUUCC AGUUCUGCAA CGACCCCUUC CUGGACGUCU ACUACCACAA GAACAACAAG AGCUGGAUGG AAAGCGGCGU GUACAGCAGC GCCAACAACU GCACCUUCGA GUACGUGUCC CAGCCUUUCC UGAUGGACCU GGAAGGCAAG CAGGGCAACU UCAAGAACCU GCGCGAGUUC GUGUUUAAGA ACAUCGACGG CUACUUCAAG AUCUACAGCA AGCACACCCC UAUCAACCUC GUGCGGGAUC UGCCUCAGGG CUUCUCUGCU CUGGAACCCC UGGUGGAUCU GCCCAUCGGC AUCAACAUCA CCCGGUUUCA GACACUGCUG GCCCUGCACA GAAGCUACCU GACACCUGGC GAUAGCAGCA GCGGAUGGAC AGCUGGUGCC GCCGCUUACU AUGUGGGCUA CCUGCAGCCU AGAACCUUCC UGCUGAAGUA CAACGAGAAC GGCACCAUCA CCGACGCCGU GGAUUGUGCU CUGGAUCCUC UGAGCGAGAC AAAGUGCACC CUGAAGUCCU UCACCGUGGA AAAGGGCAUC UACCAGACCA GCAACUUCCG GGUGCAGCCC ACCGAAUCCA UCGUGCGGUU CCCCAAUAUC ACCAAUCUGU GCCCCUUCGG CGAGGUGUUC AAUGCCACCA GAUUCGCCUC UGUGUACGCC UGGAACCGGA AGCGGAUCAG CAAUUGCGUG GCCGACUACU CCGUGCUGUA CAACUCCGCC AGCUUCAGCA CCUUCAAGUG CUACGGCGUG UCCCCUACCA AGCUGAACGA CCUGUGCUUC ACAAACGUGU ACGCCGACAG CUUCGUGAUC CGGGGAGAUG AAGUGCGGCA GAUUGCCCCU GGACAGACAG GCAAGAUCGC CGACUACAAC UACAAGCUGC CCGACGACUU CACCGGCUGU GUGAUUGCCU GGAACAGCAA CAACCUGGAC UCCAAAGUCG GCGGCAACUA CAAUUACAGG UACCGGCUGU UCCGGAAGUC CAAUCUGAAG CCCUUCGAGC GGGACAUCUC CACCGAGAUC UAUCAGGCCG GCAGCAAGCC UUGUAACGGC GUGGAAGGCU UCAACUGCUA CUUCCCACUG CAGUCCUACG GCUUUCAGCC CACAAAUGGC GUGGGCUAUC AGCCCUACAG AGUGGUGGUG CUGAGCUUCG AACUGCUGCA UGCCCCUGCC ACAGUGUGCG GCCCUAAGAA AAGCACCAAU CUCGUGAAGA ACAAAUGCGU GAACUUCAAC UUCAACGGCC UGACCGGCAC CGGCGUGCUG ACAGAGAGCA ACAAGAAGUU CCUGCCAUUC CAGCAGUUUG GCCGGGAUAU CGCCGAUACC ACAGACGCCG UUAGAGAUCC CCAGACACUG GAAAUCCUGG ACAUCACCCC UUGCAGCUUC GGCGGAGUGU CUGUGAUCAC CCCUGGCACC AACACCAGCA AUCAGGUGGC AGUGCUGUAC CAGGGCGUGA ACUGUACCGA AGUGCCCGUG GCCAUUCACG CCGAUCAGCU GACACCUACA UGGCGGGUGU ACUCCACCGG CAGCAAUGUG UUUCAGACCA GAGCCGGCUG UCUGAUCGGA GCCGAGCACG UGAACAAUAG CUACGAGUGC GACAUCCCCA UCGGCGCUGG AAUCUGCGCC AGCUACCAGA CACAGACAAA CAGCAGGCGG AGAGCCAGAA GCGUGGCCAG CCAGAGCAUC AUUGCCUACA CAAUGUCUCU GGGCGCCGAG AACAGCGUGG CCUACUCCAA CAACUCUAUC GCUAUCCCCA CCAACUUCAC CAUCAGCGUG ACCACAGAGA UCCUGCCUGU GUCCAUGACC AAGACCAGCG UGGACUGCAC CAUGUACAUC UGCGGCGAUU CCACCGAGUG CUCCAACCUG CUGCUGCAGU ACGGCAGCUU CUGCACCCAG CUGAAUAGAG CCCUGACAGG GAUCGCCGUG GAACAGGACA AGAACACCCA AGAGGUGUUC |
| | | GCCCAAGUGA AGCAGAUCUA CAAGACCCCU CCUAUCAAGG ACUUCGGCGG CUUCAAUUUC AGCCAGAUUC UGCCCGAUCC UAGCAAGCCC AGCAAGCGGA GCUUCAUCGA GGACCUGCUG UUCAACAAAG UGACACUGGC CGACGCCGGC UUCAUCAAGC AGUAUGGCGA UUGUCUGGGC GACAUUGCCG CCAGGGAUCU GAUUUGCGCC CAGAAGUUUA ACGGACUGAC AGUGCUGCCU CCUCUGCUGA CCGAUGAGAU GAUCGCCCAG UACACAUCUG CCCUGCUGGC CGGCACAAUC ACAAGCGGCU GGACAUUUGG AGCAGGCGCC GCUCUGCAGA UCCCCUUUGC UAUGCAGAUG GCCUACCGGU UCAACGGCAU CGGAGUGACC CAGAAUGUGC UGUACGAGAA CCAGAAGCUG AUCGCCAACC AGUUCAACAG CGCCAUCGGC AAGAUCCAGG ACAGCCUGAG CAGCACAGCA AGCGCCCUGG GAAAGCUGCA GAACGUGGUC AACCAGAAUG CCCAGGCACU GAACACCCUG GUCAAGCAGC UGUCCUCCAA CUUCGGCGCC AUCAGCUCUG UGCUGAACGA UAUCCUGAGC AGACUGGACC CUCCUGAGGC CGAGGUGCAG AUCGACAGAC UGAUCACAGG CAGACUGCAG AGCCUCCAGA CAUACGUGAC CCAGCAGCUG AUCAGAGCCG CCGAGAUUAG AGCCUCUGCC AAUCUGGCCG CCACCAAGAU GUCUGAGUGU GUGCUGGGCC AGAGCAAGAG AGUGGACUUU UGCGGCAAGG GCUACCACCU GAUGAGCUUC CCUCAGUCUG CCCCUCACGG CGUGGUGUUU CUGCACGUGA CAUAUGUGCC CGCUCAAGAG AAGAAUUUCA CCACCGCUCC AGCCAUCUGC CACGACGGCA AAGCCCACUU UCCUAGAGAA GGCGUGUUCG UGUCCAACGG CACCCAUUGG UUCGUGACAC AGCGGAACUU CUACGAGCCC CAGAUCAUCA CCACCGACAA CACCUUCGUG UCUGGCAACU GCGACGUCGU GAUCGGCAUU GUGAACAAUA CCGUGUACGA CCCUCUGCAG CCCGAGCUGG ACAGCUUCAA AGAGGAACUG GACAAGUACU UUAAGAACCA CACAAGCCCC GACGUGGACC UGGGCGAUAU CAGCGGAAUC AAUGCCAGCG UCGUGAACAU CCAGAAAGAG AUCGACCGGC UGAACGAGGU GGCCAAGAAU CUGAACGAGA GCCUGAUCGA CCUGCAAGAA CUGGGGAAGU ACGAGCAGUA CAUCAAGUGG CCCUGGUACA UCUGGCUGGG CUUUAUCGCC GGACUGAUUG CCAUCGUGAU GGUCACAAUC AUGCUGUGUU GCAUGACCAG CUGCUGUAGC UGCCUGAAGG GCUGUUGUAG CUGUGGCAGC UGCUGCAAGU UCGACGAGGA CGAUUCUGAG CCCGUGCUGA AGGGCGUGAA ACUGCACUAC ACAUGAUGA UUUCACCUGG UACUGCAUGC ACGCAAUGCU AGCUGCCCCU UUCCCGUCCU GGGUACCCCG AGUCUCCCCC GACCUCGGGU CCCAGGUAUG CUCCCACCUC CACCUGCCCC ACUCACCACC UCUGCUAGUU CCAGACACCU CCCAAGCACG CAGCAAUGCA GCUCAAAACG CUUAGCCUAG CCACACCCCC ACGGGAAACA GCAGUGAUUA ACCUUUAGCA AUAAACGAAA GUUUAACUAA GCUAUACUAA CCCCAGGGUU GGUCAAUUUC GUGCCAGCCA CACCCUGGAG CUAGC AAAAAAAAAA AAAAAAAAAA AAAAAAAAAA GCAUAUGACU AAAAAAAAAA AAAAAAAAAA AAAAAAAAAA AAAAAAAAAA AAAAAAAAAA AAAAAAAAAA AAAAAAAAAA |

**Table 6: Sequences of RBP020.18 (Omicron BA.2-specific RNA vaccine)**

| SEQ ID NO. | Brief Description | Sequence |
|---|---|---|
| 64 | Amino acid sequence of RNA-encoded SARS-CoV-2 S protein from an Omicron BA.2 variant (with PRO mutations at positions corresponding to K986P and V987P of SEQ ID NO: 1; i.e., PRO mutations at positions 983 and 984 of SEQ ID NO: 64) | MFVFLVLLPLVSSQCVNLITRTQSYTNSFTRGVYYPDKVFRSSVLHSTQDLFLPFFSNVTWFHAIH VSGTNGTKRFDNPVLPFNDGVYFASTEKSNIIRGWIFGTTLDSKTQSLLIVNNATNVVIKVCEFQF CNDPFLDVYYHKNNKSWMESEFRVYSSANNCTFEYVSQPFLMDLEGKQGNFKNLREFVFKNIDGYF KIYSKHTPINLGRDLPQGFSALEPLVDLPIGINITRFQTLLALHRSYLTPGDSSSGWTAGAAAYYV GYLQPRTFLLKYNENGTITDAVDCALDPLSETKCTLKSFTVEKGIYQTSNFRVQPTESIVRFPNIT NLCPFDEVFNATRFASVYAWNRKRISNCVADYSVLYNFAPFFAFKCYGVSPTKLNDLCFTNVYADS FVIRGNEVSQIAPGQTGNIADYNYKLPDDFTGCVIAWNSNKLDSKVGGNYNYLYRLFRKSNLKPFE RDISTEIYQAGNKPCNGVAGFNCYFPLRSYGFRPTYGVGHQPYRVVVLSFELLHAPATVCGPKKST NLVKNKCVNFNFNGLTGTGVLTESNKKFLPFQQFGRDIADTTDAVRDPQTLEILDITPCSFGGVSV ITPGTNTSNQVAVLYQGVNCTEVPVAIHADQLTPTWRVYSTGSNVFQTRAGCLIGAEYVNNSYECD IPIGAGICASYQTQTKSHRRARSVASQSIIAYTMSLGAENSVAYSNNSIAIPTNFTISVTTEILPV SMTKTSVDCTMYICGDSTECSNLLLQYGSFCTQLKRALTGIAVEQDKNTQEVFAQVKQIYKTPPIK YFGGFNFSQILPDPSKPSKRSFIEDLLFNKVTLADAGFIKQYGDCLGDIAARDLICAQKFNGLTVL PPLLTDEMIAQYTSALLAGTITSGWTFGAGAALQIPFAMQMAYRFNGIGVTQNVLYENQKLIANQF NSAIGKIQDSLSSTASALGKLQDVVNHNAQALNTLVKQLSSKFGAISSVLNDILSRLDPPEAEVQI DRLITGRLQSLQTYVTQQLIRAAEIRASANLAATKMSECVLGQSKRVDFCGKGYHLMSFPQSAPHG VVFLHVTYVPAQEKNFTTAPAICHDGKAHFPREGVFVSNGTHWFVTQRNFYEPQIITTDNTFVSGN CDVVIGIVNNTVYDPLQPELDSFKEELDKYFKNHTSPDVDLGDISGINASVVNIQKEIDRLNEVAK NLNESLIDLQELGKYEQYIKWPWYIWLGFIAGLIAIVMVTIMLCCMTSCCSCLKGCCSCGSCCKFD EDDSEPVLKGVKLHYT** |
| 65 | RNA sequence encoding a SARS-CoV-2 S protein from an Omicron BA.2 variant | AUGUUCGUGUUCCUGGUGCUGCUGCCUCUGGUGUCCAGCCAGUGUGUGAACCUGAUCACCAGAACA CAGUCAUACACCAACAGCUUUACCAGAGGCGUGUACUACCCCGACAAGGUGUUCAGAUCCAGCGUG CUGCACUCUACCCAGGACCUGUUCCUGCCUUUCUUCAGCAACGUGACCUGGUUCCACGCCAUCCAC GUGUCCGGCACCAAUGGCACCAAGAGAUUCGACAACCCCGUGCUGCCCUUCAACGACGGGGUGUAC UUUGCCAGCACCGAGAAGUCCAACAUCAUCAGAGGCUGGAUCUUCGGCACCACACUGGACAGCAAG ACCCAGAGCCUGCUGAUCGUGAACAACGCCACCAACGUGGUCAUCAAAGUGUGCGAGUUCCAGUUC UGCAACGACCCCUUCCUGGACGUCUACUACCACAAGAACAACAAGAGCUGGAUGGAAAGCGAGUUC CGGGUGUACAGCAGCGCCAACAACUGCACCUUCGAGUACGUGUCCCAGCCUUUCCUGAUGGACCUG GAAGGCAAGCAGGGCAACUUCAAGAACCUGCGCGAGUUCGUGUUUAAGAACAUCGACGGCUACUUC AAGAUCUACAGCAAGCACACCCCUAUCAACCUCGGCCGGGAUCUGCCUCAGGGCUUCUCUGCUCUG GAACCCCUGGUGGAUCUGCCCAUCGGCAUCAACAUCACCCGGUUUCAGACACUGCUGGCCCUGCAC AGAAGCUACCUGACACCUGGCGAUAGCAGCAGCGGAUGGACAGCUGGUGCCGCCGCUUACUAUGUG GGCUACCUGCAGCCUAGAACCUUCCUGCUGAAGUACAACGAGAACGGCACCAUCACCGACGCCGUG GAUUGUGCUCUGGAUCCUCUGAGCGAGACAAAGUGCACCCUGAAGUCCUUCACCGUGGAAAAGGGC |
| | | AUCUACCAGACCAGCAACUUCCGGGUGCAGCCCACCGAAUCCAUCGUGCGGUUCCCCAAUAUCACC AAUCUGUGCCCCUUCGACGAGGUGUUCAAUGCCACCAGAUUCGCCUCUGUGUACGCCUGGAACCGG AAGCGGAUCAGCAAUUGCGUGGCCGACUACUCCGUGCUGUACAACUUCGCCCCCUUCUUCGCAUUC AAGUGCUACGGCGUGUCCCCUACCAAGCUGAACGACCUGUGCUUCACAAACGUGUACGCCGACAGC UUCGUGAUCCGGGGAAACGAAGUGUCACAGAUUGCCCCUGGACAGACAGGCAACAUCGCCGACUAC AACUACAAGCUGCCCGACGACUUCACCGGCUGUGUGAUUGCCUGGAACAGCAACAAGCUGGACUCC AAAGUCGGCGGCAACUACAAUUACCUGUACCGGCUGUUCCGGAAGUCCAAUCUGAAGCCCUUCGAG CGGGACAUCUCCACCGAGAUCUAUCAGGCCGGCAACAAGCCUUGUAACGGCGUGGCAGGCUUCAAC UGCUACUUCCCACUGAGGUCCUACGGCUUUAGGCCCACAUACGGCGUGGGCCACCAGCCCUACAGA GUGGUGGUGCUGAGCUUCGAACUGCUGCAUGCCCCUGCCACAGUGUGCGGCCCUAAGAAAAGCACC AAUCUCGUGAAGAACAAAUGCGUGAACUUCAACUUCAACGGCCUGACCGGCACCGGCGUGCUGACA GAGAGCAACAAGAAGUUCCUGCCAUUCCAGCAGUUUGGCCGGGAUAUCGCCGAUACCACAGACGCC GUUAGAGAUCCCCAGACACUGGAAAUCCUGGACAUCACCCCUUGCAGCUUCGGCGGAGUGUCUGUG AUCACCCCUGGCACCAACACCAGCAAUCAGGUGGCAGUGCUGUACCAGGGCGUGAACUGUACCGAA GUGCCCGUGGCCAUUCACGCCGAUCAGCUGACACCUACAUGGCGGGUGUACUCCACCGGCAGCAAU GUGUUUCAGACCAGAGCCGGCUGUCUGAUCGGAGCCGAGUACGUGAACAAUAGCUACGAGUGCGAC AUCCCCAUCGGCGCUGGAAUCUGCGCCAGCUACCAGACACAGACAAAGAGCCACCGGAGAGCCAGA AGCGUGGCCAGCCAGAGCAUCAUUGCCUACACAAUGUCUCUGGGCGCCGAGAACAGCGUGGCCUAC UCCAACAACUCUAUCGCUAUCCCCACCAACUUCACCAUCAGCGUGACCACAGAGAUCCUGCCUGUG UCCAUGACCAAGACCAGCGUGGACUGCACCAUGUACAUCUGCGGCGAUUCCACCGAGUGCUCCAAC CUGCUGCUGCAGUACGGCAGCUUCUGCACCCAGCUGAAAAGAGCCCUGACAGGGAUCGCCGUGGAA CAGGACAAGAACACCCAAGAGGUGUUCGCCCAAGUGAAGCAGAUCUACAAGACCCCUCCUAUCAAG UACUUCGGCGGCUUCAAUUUCAGCCAGAUUCUGCCCGAUCCUAGCAAGCCCAGCAAGCGGAGCUUC AUCGAGGACCUGCUGUUCAACAAAGUGACACUGGCCGACGCCGGCUUCAUCAAGCAGUAUGGCGAU UGUCUGGGCGACAUUGCCGCCAGGGAUCUGAUUUGCGCCCAGAAGUUUAACGGACUGACAGUGCUG CCUCCUCUGCUGACCGAUGAGAUGAUCGCCCAGUACACAUCUGCCCUGCUGGCCGGCACAAUCACA AGCGGCUGGACAUUUGGAGCAGGCGCCGCUCUGCAGAUCCCCUUUGCUAUGCAGAUGGCCUACCGG UUCAACGGCAUCGGAGUGACCCAGAAUGUGCUGUACGAGAACCAGAAGCUGAUCGCCAACCAGUUC AACAGCGCCAUCGGCAAGAUCCAGGACAGCCUGAGCAGCACAGCAAGCGCCCUGGGAAAGCUGCAG GACGUGGUCAACCACAAUGCCCAGGCACUGAACACCCUGGUCAAGCAGCUGUCCUCCAAGUUCGGC GCCAUCAGCUCUGUGCUGAACGAUAUCCUGAGCAGACUGGACCCUCCUGAGGCCGAGGUGCAGAUC GACAGACUGAUCACAGGCAGACUGCAGAGCCUCCAGACAUACGUGACCCAGCAGCUGAUCAGAGCC GCCGAGAUUAGAGCCUCUGCCAAUCUGGCCGCCACCAAGAUGUCUGAGUGUGUGCUGGGCCAGAGC AAGAGAGUGGACUUUUGCGGCAAGGGCUACCACCUGAUGAGCUUCCCUCAGUCUGCCCCUCACGGC GUGGUGUUUCUGCACGUGACAUAUGUGCCCGCUCAAGAGAAGAAUUUCACCACCGCUCCAGCCAUC UGCCACGACGGCAAAGCCCACUUUCCUAGAGAAGGCGUGUUCGUGUCCAACGGCACCCAUUGGUUC GUGACACAGCGGAACUUCUACGAGCCCCAGAUCAUCACCACCGACAACACCUUCGUGUCUGGCAAC UGCGACGUCGUGAUCGGCAUUGUGAACAAUACCGUGUACGACCCUCUGCAGCCCGAGCUGGACAGC UUCAAAGAGGAACUGGACAAGUACUUUAAGAACCACACAAGCCCCGACGUGGACCUGGGCGAUAUC AGCGGAAUCAAUGCCAGCGUCGUGAACAUCCAGAAAGAGAUCGACCGGCUGAACGAGGUGGCCAAG |
| | | AAUCUGAACGAGAGCCUGAUCGACCUGCAAGAACUGGGGAAGUACGAGCAGUACAUCAAGUGGCCC UGGUACAUCUGGCUGGGCUUUAUCGCCGGACUGAUUGCCAUCGUGAUGGUCACAAUCAUGCUGUGU UGCAUGACCAGCUGCUGUAGCUGCCUGAAGGGCUGUUGUAGCUGUGGCAGCUGCUGCAAGUUCGAC GAGGACGAUUCUGAGCCCGUGCUGAAGGGCGUGAAACUGCACUACACAUGAUGA |
| 66 | DNA sequence encoding a SARS-CoV-2 S protein from an Omicron BA.2 variant | ATGTTCGTGTTCCTGGTGCTGCTGCCTCTGGTGTCCAGCCAGTGTGTGAACCTGATCACCAGAACA CAGTCATACACCAACAGCTTTACCAGAGGCGTGTACTACCCCGACAAGGTGTTCAGATCCAGCGTG CTGCACTCTACCCAGGACCTGTTCCTGCCTTTCTTCAGCAACGTGACCTGGTTCCACGCCATCCAC GTGTCCGGCACCAATGGCACCAAGAGATTCGACAACCCCGTGCTGCCCTTCAACGACGGGGTGTAC TTTGCCAGCACCGAGAAGTCCAACATCATCAGAGGCTGGATCTTCGGCACCACACTGGACAGCAAG ACCCAGAGCCTGCTGATCGTGAACAACGCCACCAACGTGGTCATCAAAGTGTGCGAGTTCCAGTTC TGCAACGACCCCTTCCTGGACGTCTACTACCACAAGAACAACAAGAGCTGGATGGAAAGCGAGTTC CGGGTGTACAGCAGCGCCAACAACTGCACCTTCGAGTACGTGTCCCAGCCTTTCCTGATGGACCTG GAAGGCAAGCAGGGCAACTTCAAGAACCTGCGCGAGTTCGTGTTTAAGAACATCGACGGCTACTTC AAGATCTACAGCAAGCACACCCCTATCAACCTCGGCCGGGATCTGCCTCAGGGCTTCTCTGCTCTG GAACCCCTGGTGGATCTGCCCATCGGCATCAACATCACCCGGTTTCAGACACTGCTGGCCCTGCAC AGAAGCTACCTGACACCTGGCGATAGCAGCAGCGGATGGACAGCTGGTGCCGCCGCTTACTATGTG GGCTACCTGCAGCCTAGAACCTTCCTGCTGAAGTACAACGAGAACGGCACCATCACCGACGCCGTG GATTGTGCTCTGGATCCTCTGAGCGAGACAAAGTGCACCCTGAAGTCCTTCACCGTGGAAAAGGGC ATCTACCAGACCAGCAACTTCCGGGTGCAGCCCACCGAATCCATCGTGCGGTTCCCCAATATCACC AATCTGTGCCCCTTCGACGAGGTGTTCAATGCCACCAGATTCGCCTCTGTGTACGCCTGGAACCGG AAGCGGATCAGCAATTGCGTGGCCGACTACTCCGTGCTGTACAACTTCGCCCCCTTCTTCGCATTC AAGTGCTACGGCGTGTCCCCTACCAAGCTGAACGACCTGTGCTTCACAAACGTGTACGCCGACAGC TTCGTGATCCGGGGAAACGAAGTGTCACAGATTGCCCCTGGACAGACAGGCAACATCGCCGACTAC AACTACAAGCTGCCCGACGACTTCACCGGCTGTGTGATTGCCTGGAACAGCAACAAGCTGGACTCC AAAGTCGGCGGCAACTACAATTACCTGTACCGGCTGTTCCGGAAGTCCAATCTGAAGCCCTTCGAG CGGGACATCTCCACCGAGATCTATCAGGCCGGCAACAAGCCTTGTAACGGCGTGGCAGGCTTCAAC TGCTACTTCCCACTGAGGTCCTACGGCTTTAGGCCCACATACGGCGTGGGCCACCAGCCCTACAGA GTGGTGGTGCTGAGCTTCGAACTGCTGCATGCCCCTGCCACAGTGTGCGGCCCTAAGAAAAGCACC AATCTCGTGAAGAACAAATGCGTGAACTTCAACTTCAACGGCCTGACCGGCACCGGCGTGCTGACA GAGAGCAACAAGAAGTTCCTGCCATTCCAGCAGTTTGGCCGGGATATCGCCGATACCACAGACGCC GTTAGAGATCCCCAGACACTGGAAATCCTGGACATCACCCCTTGCAGCTTCGGCGGAGTGTCTGTG ATCACCCCTGGCACCAACACCAGCAATCAGGTGGCAGTGCTGTACCAGGGCGTGAACTGTACCGAA GTGCCCGTGGCCATTCACGCCGATCAGCTGACACCTACATGGCGGGTGTACTCCACCGGCAGCAAT GTGTTTCAGACCAGAGCCGGCTGTCTGATCGGAGCCGAGTACGTGAACAATAGCTACGAGTGCGAC ATCCCCATCGGCGCTGGAATCTGCGCCAGCTACCAGACACAGACAAAGAGCCACCGGAGAGCCAGA AGCGTGGCCAGCCAGAGCATCATTGCCTACACAATGTCTCTGGGCGCCGAGAACAGCGTGGCCTAC TCCAACAACTCTATCGCTATCCCCACCAACTTCACCATCAGCGTGACCACAGAGATCCTGCCTGTG TCCATGACCAAGACCAGCGTGGACTGCACCATGTACATCTGCGGCGATTCCACCGAGTGCTCCAAC CTGCTGCTGCAGTACGGCAGCTTCTGCACCCAGCTGAAAAGAGCCCTGACAGGGATCGCCGTGGAA |
| | | CAGGACAAGAACACCCAAGAGGTGTTCGCCCAAGTGAAGCAGATCTACAAGACCCCTCCTATCAAG TACTTCGGCGGCTTCAATTTCAGCCAGATTCTGCCCGATCCTAGCAAGCCCAGCAAGCGGAGCTTC ATCGAGGACCTGCTGTTCAACAAAGTGACACTGGCCGACGCCGGCTTCATCAAGCAGTATGGCGAT TGTCTGGGCGACATTGCCGCCAGGGATCTGATTTGCGCCCAGAAGTTTAACGGACTGACAGTGCTG CCTCCTCTGCTGACCGATGAGATGATCGCCCAGTACACATCTGCCCTGCTGGCCGGCACAATCACA AGCGGCTGGACATTTGGAGCAGGCGCCGCTCTGCAGATCCCCTTTGCTATGCAGATGGCCTACCGG TTCAACGGCATCGGAGTGACCCAGAATGTGCTGTACGAGAACCAGAAGCTGATCGCCAACCAGTTC AACAGCGCCATCGGCAAGATCCAGGACAGCCTGAGCAGCACAGCAAGCGCCCTGGGAAAGCTGCAG GACGTGGTCAACCACAATGCCCAGGCACTGAACACCCTGGTCAAGCAGCTGTCCTCCAAGTTCGGC GCCATCAGCTCTGTGCTGAACGATATCCTGAGCAGACTGGACCCTCCTGAGGCCGAGGTGCAGATC GACAGACTGATCACAGGCAGACTGCAGAGCCTCCAGACATACGTGACCCAGCAGCTGATCAGAGCC GCCGAGATTAGAGCCTCTGCCAATCTGGCCGCCACCAAGATGTCTGAGTGTGTGCTGGGCCAGAGC AAGAGAGTGGACTTTTGCGGCAAGGGCTACCACCTGATGAGCTTCCCTCAGTCTGCCCCTCACGGC GTGGTGTTTCTGCACGTGACATATGTGCCCGCTCAAGAGAAGAATTTCACCACCGCTCCAGCCATC TGCCACGACGGCAAAGCCCACTTTCCTAGAGAAGGCGTGTTCGTGTCCAACGGCACCCATTGGTTC GTGACACAGCGGAACTTCTACGAGCCCCAGATCATCACCACCGACAACACCTTCGTGTCTGGCAAC TGCGACGTCGTGATCGGCATTGTGAACAATACCGTGTACGACCCTCTGCAGCCCGAGCTGGACAGC TTCAAAGAGGAACTGGACAAGTACTTTAAGAACCACACAAGCCCCGACGTGGACCTGGGCGATATC AGCGGAATCAATGCCAGCGTCGTGAACATCCAGAAAGAGATCGACCGGCTGAACGAGGTGGCCAAG AATCTGAACGAGAGCCTGATCGACCTGCAAGAACTGGGGAAGTACGAGCAGTACATCAAGTGGCCC TGGTACATCTGGCTGGGCTTTATCGCCGGACTGATTGCCATCGTGATGGTCACAATCATGCTGTGT TGCATGACCAGCTGCTGTAGCTGCCTGAAGGGCTGTTGTAGCTGTGGCAGCTGCTGCAAGTTCGAC GAGGACGATTCTGAGCCCGTGCTGAAGGGCGTGAAACTGCACTACACATGATGA |
| 67 | Full length RNA construct sequence of RBP020.18 | AGAAUAAACUAGUAUUCUUCUGGUCCCCACAGACUCAGAGAGAACCCGCCACCAUGUUCGUGUUCC UGGUGCUGCUGCCUCUGGUGUCCAGCCAGUGUGUGAACCUGAUCACCAGAACACAGUCAUACACCA ACAGCUUUACCAGAGGCGUGUACUACCCCGACAAGGUGUUCAGAUCCAGCGUGCUGCACUCUACCC AGGACCUGUUCCUGCCUUUCUUCAGCAACGUGACCUGGUUCCACGCCAUCCACGUGUCCGGCACCA AUGGCACCAAGAGAUUCGACAACCCCGUGCUGCCCUUCAACGACGGGGUGUACUUUGCCAGCACCG AGAAGUCCAACAUCAUCAGAGGCUGGAUCUUCGGCACCACACUGGACAGCAAGACCCAGAGCCUGC UGAUCGUGAACAACGCCACCAACGUGGUCAUCAAAGUGUGCGAGUUCCAGUUCUGCAACGACCCCU UCCUGGACGUCUACUACCACAAGAACAACAAGAGCUGGAUGGAAAGCGAGUUCCGGGUGUACAGCA GCGCCAACAACUGCACCUUCGAGUACGUGUCCCAGCCUUUCCUGAUGGACCUGGAAGGCAAGCAGG GCAACUUCAAGAACCUGCGCGAGUUCGUGUUUAAGAACAUCGACGGCUACUUCAAGAUCUACAGCA AGCACACCCCUAUCAACCUCGGCCGGGAUCUGCCUCAGGGCUUCUCUGCUCUGGAACCCCUGGUGG AUCUGCCCAUCGGCAUCAACAUCACCCGGUUUCAGACACUGCUGGCCCUGCACAGAAGCUACCUGA CACCUGGCGAUAGCAGCAGCGGAUGGACAGCUGGUGCCGCCGCUUACUAUGUGGGCUACCUGCAGC CUAGAACCUUCCUGCUGAAGUACAACGAGAACGGCACCAUCACCGACGCCGUGGAUUGUGCUCUGG AUCCUCUGAGCGAGACAAAGUGCACCCUGAAGUCCUUCACCGUGGAAAAGGGCAUCUACCAGACCA GCAACUUCCGGGUGCAGCCCACCGAAUCCAUCGUGCGGUUCCCCAAUAUCACCAAUCUGUGCCCCU |
| | | UCGACGAGGUGUUCAAUGCCACCAGAUUCGCCUCUGUGUACGCCUGGAACCGGAAGCGGAUCAGCA AUUGCGUGGCCGACUACUCCGUGCUGUACAACUUCGCCCCCUUCUUCGCAUUCAAGUGCUACGGCG UGUCCCCUACCAAGCUGAACGACCUGUGCUUCACAAACGUGUACGCCGACAGCUUCGUGAUCCGGG GAAACGAAGUGUCACAGAUUGCCCCUGGACAGACAGGCAACAUCGCCGACUACAACUACAAGCUGC CCGACGACUUCACCGGCUGUGUGAUUGCCUGGAACAGCAACAAGCUGGACUCCAAAGUCGGCGGCA ACUACAAUUACCUGUACCGGCUGUUCCGGAAGUCCAAUCUGAAGCCCUUCGAGCGGGACAUCUCCA CCGAGAUCUAUCAGGCCGGCAACAAGCCUUGUAACGGCGUGGCAGGCUUCAACUGCUACUUCCCAC UGAGGUCCUACGGCUUUAGGCCCACAUACGGCGUGGGCCACCAGCCCUACAGAGUGGUGGUGCUGA GCUUCGAACUGCUGCAUGCCCCUGCCACAGUGUGCGGCCCUAAGAAAAGCACCAAUCUCGUGAAGA ACAAAUGCGUGAACUUCAACUUCAACGGCCUGACCGGCACCGGCGUGCUGACAGAGAGCAACAAGA AGUUCCUGCCAUUCCAGCAGUUUGGCCGGGAUAUCGCCGAUACCACAGACGCCGUUAGAGAUCCCC AGACACUGGAAAUCCUGGACAUCACCCCUUGCAGCUUCGGCGGAGUGUCUGUGAUCACCCCUGGCA CCAACACCAGCAAUCAGGUGGCAGUGCUGUACCAGGGCGUGAACUGUACCGAAGUGCCCGUGGCCA UUCACGCCGAUCAGCUGACACCUACAUGGCGGGUGUACUCCACCGGCAGCAAUGUGUUUCAGACCA GAGCCGGCUGUCUGAUCGGAGCCGAGUACGUGAACAAUAGCUACGAGUGCGACAUCCCCAUCGGCG CUGGAAUCUGCGCCAGCUACCAGACACAGACAAAGAGCCACCGGAGAGCCAGAAGCGUGGCCAGCC AGAGCAUCAUUGCCUACACAAUGUCUCUGGGCGCCGAGAACAGCGUGGCCUACUCCAACAACUCUA UCGCUAUCCCCACCAACUUCACCAUCAGCGUGACCACAGAGAUCCUGCCUGUGUCCAUGACCAAGA CCAGCGUGGACUGCACCAUGUACAUCUGCGGCGAUUCCACCGAGUGCUCCAACCUGCUGCUGCAGU ACGGCAGCUUCUGCACCCAGCUGAAAAGAGCCCUGACAGGGAUCGCCGUGGAACAGGACAAGAACA CCCAAGAGGUGUUCGCCCAAGUGAAGCAGAUCUACAAGACCCCUCCUAUCAAGUACUUCGGCGGCU UCAAUUUCAGCCAGAUUCUGCCCGAUCCUAGCAAGCCCAGCAAGCGGAGCUUCAUCGAGGACCUGC UGUUCAACAAAGUGACACUGGCCGACGCCGGCUUCAUCAAGCAGUAUGGCGAUUGUCUGGGCGACA UUGCCGCCAGGGAUCUGAUUUGCGCCCAGAAGUUUAACGGACUGACAGUGCUGCCUCCUCUGCUGA CCGAUGAGAUGAUCGCCCAGUACACAUCUGCCCUGCUGGCCGGCACAAUCACAAGCGGCUGGACAU UUGGAGCAGGCGCCGCUCUGCAGAUCCCCUUUGCUAUGCAGAUGGCCUACCGGUUCAACGGCAUCG GAGUGACCCAGAAUGUGCUGUACGAGAACCAGAAGCUGAUCGCCAACCAGUUCAACAGCGCCAUCG GCAAGAUCCAGGACAGCCUGAGCAGCACAGCAAGCGCCCUGGGAAAGCUGCAGGACGUGGUCAACC ACAAUGCCCAGGCACUGAACACCCUGGUCAAGCAGCUGUCCUCCAAGUUCGGCGCCAUCAGCUCUG UGCUGAACGAUAUCCUGAGCAGACUGGACCCUCCUGAGGCCGAGGUGCAGAUCGACAGACUGAUCA CAGGCAGACUGCAGAGCCUCCAGACAUACGUGACCCAGCAGCUGAUCAGAGCCGCCGAGAUUAGAG CCUCUGCCAAUCUGGCCGCCACCAAGAUGUCUGAGUGUGUGCUGGGCCAGAGCAAGAGAGUGGACU UUUGCGGCAAGGGCUACCACCUGAUGAGCUUCCCUCAGUCUGCCCCUCACGGCGUGGUGUUUCUGC ACGUGACAUAUGUGCCCGCUCAAGAGAAGAAUUUCACCACCGCUCCAGCCAUCUGCCACGACGGCA AAGCCCACUUUCCUAGAGAAGGCGUGUUCGUGUCCAACGGCACCCAUUGGUUCGUGACACAGCGGA ACUUCUACGAGCCCCAGAUCAUCACCACCGACAACACCUUCGUGUCUGGCAACUGCGACGUCGUGA UCGGCAUUGUGAACAAUACCGUGUACGACCCUCUGCAGCCCGAGCUGGACAGCUUCAAAGAGGAAC UGGACAAGUACUUUAAGAACCACACAAGCCCCGACGUGGACCUGGGCGAUAUCAGCGGAAUCAAUG CCAGCGUCGUGAACAUCCAGAAAGAGAUCGACCGGCUGAACGAGGUGGCCAAGAAUCUGAACGAGA GCCUGAUCGACCUGCAAGAACUGGGGAAGUACGAGCAGUACAUCAAGUGGCCCUGGUACAUCUGGC |
| | | UGGGCUUUAUCGCCGGACUGAUUGCCAUCGUGAUGGUCACAAUCAUGCUGUGUUGCAUGACCAGCU GCUGUAGCUGCCUGAAGGGCUGUUGUAGCUGUGGCAGCUGCUGCAAGUUCGACGAGGACGAUUCUG AGCCCGUGCUGAAGGGCGUGAAACUGCACUACACAUGAUGACUCGAGCUGGUACUGCAUGCACGCA AUGCUAGCUGCCCCUUUCCCGUCCUGGGUACCCCGAGUCUCCCCCGACCUCGGGUCCCAGGUAUGC UCCCACCUCCACCUGCCCCACUCACCACCUCUGCUAGUUCCAGACACCUCCCAAGCACGCAGCAAU GCAGCUCAAAACGCUUAGCCUAGCCACACCCCCACGGGAAACAGCAGUGAUUAACCUUUAGCAAUA AACGAAAGUUUAACUAAGCUAUACUAACCCCAGGGUUGGUCAAUUUCGUGCCAGCCACACCCUGGA GCUAGCAAAAAAAAAAAAAAAAAAAAAAAAAAAAAAGCAUAUGACUAAAAAAAAAAAAAAAAAAAA AAAAAAAAAAAAAAAAAAAAAAAAAAAAAAAAAAAAAAAAAAAAAAAAAA |
| 68 | Full length DNA construct sequence of RBP020.18 | AGAATAAACTAGTATTCTTCTGGTCCCCACAGACTCAGAGAGAACCCGCCACCATGTTCGTGTTCC TGGTGCTGCTGCCTCTGGTGTCCAGCCAGTGTGTGAACCTGATCACCAGAACACAGTCATACACCA ACAGCTTTACCAGAGGCGTGTACTACCCCGACAAGGTGTTCAGATCCAGCGTGCTGCACTCTACCC AGGACCTGTTCCTGCCTTTCTTCAGCAACGTGACCTGGTTCCACGCCATCCACGTGTCCGGCACCA ATGGCACCAAGAGATTCGACAACCCCGTGCTGCCCTTCAACGACGGGGTGTACTTTGCCAGCACCG AGAAGTCCAACATCATCAGAGGCTGGATCTTCGGCACCACACTGGACAGCAAGACCCAGAGCCTGC TGATCGTGAACAACGCCACCAACGTGGTCATCAAAGTGTGCGAGTTCCAGTTCTGCAACGACCCCT TCCTGGACGTCTACTACCACAAGAACAACAAGAGCTGGATGGAAAGCGAGTTCCGGGTGTACAGCA GCGCCAACAACTGCACCTTCGAGTACGTGTCCCAGCCTTTCCTGATGGACCTGGAAGGCAAGCAGG GCAACTTCAAGAACCTGCGCGAGTTCGTGTTTAAGAACATCGACGGCTACTTCAAGATCTACAGCA AGCACACCCCTATCAACCTCGGCCGGGATCTGCCTCAGGGCTTCTCTGCTCTGGAACCCCTGGTGG ATCTGCCCATCGGCATCAACATCACCCGGTTTCAGACACTGCTGGCCCTGCACAGAAGCTACCTGA CACCTGGCGATAGCAGCAGCGGATGGACAGCTGGTGCCGCCGCTTACTATGTGGGCTACCTGCAGC CTAGAACCTTCCTGCTGAAGTACAACGAGAACGGCACCATCACCGACGCCGTGGATTGTGCTCTGG ATCCTCTGAGCGAGACAAAGTGCACCCTGAAGTCCTTCACCGTGGAAAAGGGCATCTACCAGACCA GCAACTTCCGGGTGCAGCCCACCGAATCCATCGTGCGGTTCCCCAATATCACCAATCTGTGCCCCT TCGACGAGGTGTTCAATGCCACCAGATTCGCCTCTGTGTACGCCTGGAACCGGAAGCGGATCAGCA ATTGCGTGGCCGACTACTCCGTGCTGTACAACTTCGCCCCCTTCTTCGCATTCAAGTGCTACGGCG TGTCCCCTACCAAGCTGAACGACCTGTGCTTCACAAACGTGTACGCCGACAGCTTCGTGATCCGGG GAAACGAAGTGTCACAGATTGCCCCTGGACAGACAGGCAACATCGCCGACTACAACTACAAGCTGC CCGACGACTTCACCGGCTGTGTGATTGCCTGGAACAGCAACAAGCTGGACTCCAAAGTCGGCGGCA ACTACAATTACCTGTACCGGCTGTTCCGGAAGTCCAATCTGAAGCCCTTCGAGCGGGACATCTCCA CCGAGATCTATCAGGCCGGCAACAAGCCTTGTAACGGCGTGGCAGGCTTCAACTGCTACTTCCCAC TGAGGTCCTACGGCTTTAGGCCCACATACGGCGTGGGCCACCAGCCCTACAGAGTGGTGGTGCTGA GCTTCGAACTGCTGCATGCCCCTGCCACAGTGTGCGGCCCTAAGAAAAGCACCAATCTCGTGAAGA ACAAATGCGTGAACTTCAACTTCAACGGCCTGACCGGCACCGGCGTGCTGACAGAGAGCAACAAGA AGTTCCTGCCATTCCAGCAGTTTGGCCGGGATATCGCCGATACCACAGACGCCGTTAGAGATCCCC AGACACTGGAAATCCTGGACATCACCCCTTGCAGCTTCGGCGGAGTGTCTGTGATCACCCCTGGCA CCAACACCAGCAATCAGGTGGCAGTGCTGTACCAGGGCGTGAACTGTACCGAAGTGCCCGTGGCCA TTCACGCCGATCAGCTGACACCTACATGGCGGGTGTACTCCACCGGCAGCAATGTGTTTCAGACCA |
| | | GAGCCGGCTGTCTGATCGGAGCCGAGTACGTGAACAATAGCTACGAGTGCGACATCCCCATCGGCG CTGGAATCTGCGCCAGCTACCAGACACAGACAAAGAGCCACCGGAGAGCCAGAAGCGTGGCCAGCC AGAGCATCATTGCCTACACAATGTCTCTGGGCGCCGAGAACAGCGTGGCCTACTCCAACAACTCTA TCGCTATCCCCACCAACTTCACCATCAGCGTGACCACAGAGATCCTGCCTGTGTCCATGACCAAGA CCAGCGTGGACTGCACCATGTACATCTGCGGCGATTCCACCGAGTGCTCCAACCTGCTGCTGCAGT ACGGCAGCTTCTGCACCCAGCTGAAAAGAGCCCTGACAGGGATCGCCGTGGAACAGGACAAGAACA CCCAAGAGGTGTTCGCCCAAGTGAAGCAGATCTACAAGACCCCTCCTATCAAGTACTTCGGCGGCT TCAATTTCAGCCAGATTCTGCCCGATCCTAGCAAGCCCAGCAAGCGGAGCTTCATCGAGGACCTGC TGTTCAACAAAGTGACACTGGCCGACGCCGGCTTCATCAAGCAGTATGGCGATTGTCTGGGCGACA TTGCCGCCAGGGATCTGATTTGCGCCCAGAAGTTTAACGGACTGACAGTGCTGCCTCCTCTGCTGA CCGATGAGATGATCGCCCAGTACACATCTGCCCTGCTGGCCGGCACAATCACAAGCGGCTGGACAT TTGGAGCAGGCGCCGCTCTGCAGATCCCCTTTGCTATGCAGATGGCCTACCGGTTCAACGGCATCG GAGTGACCCAGAATGTGCTGTACGAGAACCAGAAGCTGATCGCCAACCAGTTCAACAGCGCCATCG GCAAGATCCAGGACAGCCTGAGCAGCACAGCAAGCGCCCTGGGAAAGCTGCAGGACGTGGTCAACC ACAATGCCCAGGCACTGAACACCCTGGTCAAGCAGCTGTCCTCCAAGTTCGGCGCCATCAGCTCTG TGCTGAACGATATCCTGAGCAGACTGGACCCTCCTGAGGCCGAGGTGCAGATCGACAGACTGATCA CAGGCAGACTGCAGAGCCTCCAGACATACGTGACCCAGCAGCTGATCAGAGCCGCCGAGATTAGAG CCTCTGCCAATCTGGCCGCCACCAAGATGTCTGAGTGTGTGCTGGGCCAGAGCAAGAGAGTGGACT TTTGCGGCAAGGGCTACCACCTGATGAGCTTCCCTCAGTCTGCCCCTCACGGCGTGGTGTTTCTGC ACGTGACATATGTGCCCGCTCAAGAGAAGAATTTCACCACCGCTCCAGCCATCTGCCACGACGGCA AAGCCCACTTTCCTAGAGAAGGCGTGTTCGTGTCCAACGGCACCCATTGGTTCGTGACACAGCGGA ACTTCTACGAGCCCCAGATCATCACCACCGACAACACCTTCGTGTCTGGCAACTGCGACGTCGTGA TCGGCATTGTGAACAATACCGTGTACGACCCTCTGCAGCCCGAGCTGGACAGCTTCAAAGAGGAAC TGGACAAGTACTTTAAGAACCACACAAGCCCCGACGTGGACCTGGGCGATATCAGCGGAATCAATG CCAGCGTCGTGAACATCCAGAAAGAGATCGACCGGCTGAACGAGGTGGCCAAGAATCTGAACGAGA GCCTGATCGACCTGCAAGAACTGGGGAAGTACGAGCAGTACATCAAGTGGCCCTGGTACATCTGGC TGGGCTTTATCGCCGGACTGATTGCCATCGTGATGGTCACAATCATGCTGTGTTGCATGACCAGCT GCTGTAGCTGCCTGAAGGGCTGTTGTAGCTGTGGCAGCTGCTGCAAGTTCGACGAGGACGATTCTG AGCCCGTGCTGAAGGGCGTGAAACTGCACTACACATGATGACTCGAGCTGGTACTGCATGCACGCA ATGCTAGCTGCCCCTTTCCCGTCCTGGGTACCCCGAGTCTCCCCCGACCTCGGGTCCCAGGTATGC TCCCACCTCCACCTGCCCCACTCACCACCTCTGCTAGTTCCAGACACCTCCCAAGCACGCAGCAAT GCAGCTCAAAACGCTTAGCCTAGCCACACCCCCACGGGAAACAGCAGTGATTAACCTTTAGCAATA AACGAAAGTTTAACTAAGCTATACTAACCCCAGGGTTGGTCAATTTCGTGCCAGCCACACCCTGGA GCTAGCAAAAAAAAAAAAAAAAAAAAAAAAAAAAAAGCATATGACTAAAAAAAAAAAAAAAAAAAA AAAAAAAAAAAAAAAAAAAAAAAAAAAAAAAAAAAAAAAAAAAAAA |

**Table 7: Description of sequences of RBP020.18 (Omicron BA.2-specific RNA vaccine) as described in Table 6 above (amino acid mutation positioning shown relative to SEQ ID NO: 1)**

| **Construct** | **Omicron BA.2 P2** | |
|---|---|---|
| **Antigen** | P2-mutated full spike protein | |
| **Changes** | Amino Acid^{a} | mRNA Nucleotides (location) |
| **Furin site** | RRAR | CGGAGAGCCAGA (2088-2099) |
| **Proline** | K986P | CCU (3000-3002) |
| | V987P | CCU (3003-3005) |
| **Lineage** | T19I | AUC (108-110) |
| | L24del | / |
| | P25del | / |
| | P26del | / |
| | A27S | UCA (123-125) |
| | G142D | GAC (468-470) |
| | V213G | GGC (681-683) |
| | G339D | GAC (1059-1061) |
| | S371F | UUC (1155-1157) |
| | S373P | CCC (1161-1163) |
| | S375F | UUC (1167-1169) |
| | T376A | GCA (1170-1172) |
| | D405N | AAC (1257-1259) |
| | R408S | UCA (1266-1268) |
| | K417N | AAC (1293-1295) |
| | N440K | AAG (1362-1364) |
| | S477N | AAC (1473-1475) |
| | T478K | AAG (1476-1478) |
| | E484A | GCA (1494-1496) |
| | Q493R | AGG (1521-1523) |
| | Q498R | AGG (1536-1538) |
| | N501Y | UAC (1545-1547) |
| | Y505H | CAC (1557-1559) |
| | D614G | GGC (1884-1886) |
| | H655Y | UAC (2007-2009) |
| | N679K | AAG (2079-2081) |
| | P681H | CAC (2085-2087) |
| | N764K | AAA (2334-2336) |
| | D796Y | UAC (2430-2432) |
| | Q954H | CAC (2904-2906) |
| | N969K | AAG (2949-2951) |

**Table 8: Sequences of RBP020.22 (Omicron BA.4/BA.5-specific RNA vaccine)**

| SEQ ID NO. | Brief Description | Sequence |
|---|---|---|
| 69 | Amino acid sequence of RNA-encoded SARS-CoV-2 S protein from an Omicron BA.4/BA.5 variant (with PRO mutations at positions corresponding to positions K986P and V987P of SEQ ID NO: 7, i.e., at residues 981 and 982 of SEQ ID NO: 69) | MFVFLVLLPLVSSQCVNLITRTQSYTNSFTRGVYYPDKVFRSSVLHSTQDLFLPFFSNVTWFHAIS GTNGTKRFDNPVLPFNDGVYFASTEKSNIIRGWIFGTTLDSKTQSLLIVNNATNVVIKVCEFQFCN DPFLDVYYHKNNKSWMESEFRVYSSANNCTFEYVSQPFLMDLEGKQGNFKNLREFVFKNIDGYFKI YSKHTPINLGRDLPQGFSALEPLVDLPIGINITRFQTLLALHRSYLTPGDSSSGWTAGAAAYYVGY LQPRTFLLKYNENGTITDAVDCALDPLSETKCTLKSFTVEKGIYQTSNFRVQPTESIVRFPNITNL CPFDEVFNATRFASVYAWNRKRISNCVADYSVLYNFAPFFAFKCYGVSPTKLNDLCFTNVYADSFV IRGNEVRQIAPGQTGNIADYNYKLPDDFTGCVIAWNSNKLDSKVGGNYNYRYRLFRKSNLKPFERD ISTEIYQAGNKPCNGVAGVNCYFPLQSYGFRPTYGVGHQPYRVVVLSFELLHAPATVCGPKKSTNL VKNKCVNFNFNGLTGTGVLTESNKKFLPFQQFGRDIADTTDAVRDPQTLEILDITPCSFGGVSVIT PGTNTSNQVAVLYQGVNCTEVPVAIHADQLTPTWRVYSTGSNVFQTRAGCLIGAEYVNNSYECDIP IGAGICASYQTQTKSHRRARSVASQSIIAYTMSLGAENSVAYSNNSIAIPTNFTISVTTEILPVSM TKTSVDCTMYICGDSTECSNLLLQYGSFCTQLKRALTGIAVEQDKNTQEVFAQVKQIYKTPPIKYF GGFNFSQILPDPSKPSKRSFIEDLLFNKVTLADAGFIKQYGDCLGDIAARDLICAQKFNGLTVLPP LLTDEMIAQYTSALLAGTITSGWTFGAGAALQIPFAMQMAYRFNGIGVTQNVLYENQKLIANQFNS AIGKIQDSLSSTASALGKLQDVVNHNAQALNTLVKQLSSKFGAISSVLNDILSRLDPPEAEVQIDR LITGRLQSLQTYVTQQLIRAAEIRASANLAATKMSECVLGQSKRVDFCGKGYHLMSFPQSAPHGVV FLHVTYVPAQEKNFTTAPAICHDGKAHFPREGVFVSNGTHWFVTQRNFYEPQIITTDNTFVSGNCD VVIGIVNNTVYDPLQPELDSFKEELDKYFKNHTSPDVDLGDISGINASVVNIQKEIDRLNEVAKNL NESLIDLQELGKYEQYIKWPWYIWLGFIAGLIAIVMVTIMLCCMTSCCSCLKGCCSCGSCCKFDED DSEPVLKGVKLHYT** |
| 70 | RNA sequence encoding a SARS-CoV-2 S protein from an Omicron BA.4/BA.5 variant | AUGUUCGUGUUCCUGGUGCUGCUGCCUCUGGUGUCCAGCCAGUGUGUGAACCUGAUCACCAGAACA CAGUCAUACACCAACAGCUUUACCAGAGGCGUGUACUACCCCGACAAGGUGUUCAGAUCCAGCGUG CUGCACUCUACCCAGGACCUGUUCCUGCCUUUCUUCAGCAACGUGACCUGGUUCCACGCCAUCUCC GGCACCAAUGGCACCAAGAGAUUCGACAACCCCGUGCUGCCCUUCAACGACGGGGUGUACUUUGCC AGCACCGAGAAGUCCAACAUCAUCAGAGGCUGGAUCUUCGGCACCACACUGGACAGCAAGACCCAG AGCCUGCUGAUCGUGAACAACGCCACCAACGUGGUCAUCAAAGUGUGCGAGUUCCAGUUCUGCAAC GACCCCUUCCUGGACGUCUACUACCACAAGAACAACAAGAGCUGGAUGGAAAGCGAGUUCCGGGUG UACAGCAGCGCCAACAACUGCACCUUCGAGUACGUGUCCCAGCCUUUCCUGAUGGACCUGGAAGGC AAGCAGGGCAACUUCAAGAACCUGCGCGAGUUCGUGUUUAAGAACAUCGACGGCUACUUCAAGAUC UACAGCAAGCACACCCCUAUCAACCUCGGCCGGGAUCUGCCUCAGGGCUUCUCUGCUCUGGAACCC CUGGUGGAUCUGCCCAUCGGCAUCAACAUCACCCGGUUUCAGACACUGCUGGCCCUGCACAGAAGC UACCUGACACCUGGCGAUAGCAGCAGCGGAUGGACAGCUGGUGCCGCCGCUUACUAUGUGGGCUAC |
| | | CUGCAGCCUAGAACCUUCCUGCUGAAGUACAACGAGAACGGCACCAUCACCGACGCCGUGGAUUGU GCUCUGGAUCCUCUGAGCGAGACAAAGUGCACCCUGAAGUCCUUCACCGUGGAAAAGGGCAUCUAC CAGACCAGCAACUUCCGGGUGCAGCCCACCGAAUCCAUCGUGCGGUUCCCCAAUAUCACCAAUCUG UGCCCCUUCGACGAGGUGUUCAAUGCCACCAGAUUCGCCUCUGUGUACGCCUGGAACCGGAAGCGG AUCAGCAAUUGCGUGGCCGACUACUCCGUGCUGUACAACUUCGCCCCCUUCUUCGCAUUCAAGUGC UACGGCGUGUCCCCUACCAAGCUGAACGACCUGUGCUUCACAAACGUGUACGCCGACAGCUUCGUG AUCCGGGGAAACGAAGUGCGGCAGAUUGCCCCUGGACAGACAGGCAACAUCGCCGACUACAACUAC AAGCUGCCCGACGACUUCACCGGCUGUGUGAUUGCCUGGAACAGCAACAAGCUGGACUCCAAAGUC GGCGGCAACUACAAUUACAGGUACCGGCUGUUCCGGAAGUCCAAUCUGAAGCCCUUCGAGCGGGAC AUCUCCACCGAGAUCUAUCAGGCCGGCAACAAGCCUUGUAACGGCGUGGCAGGCGUGAACUGCUAC UUCCCACUGCAGUCCUACGGCUUUAGGCCCACAUACGGCGUGGGCCACCAGCCCUACAGAGUGGUG GUGCUGAGCUUCGAACUGCUGCAUGCCCCUGCCACAGUGUGCGGCCCUAAGAAAAGCACCAAUCUC GUGAAGAACAAAUGCGUGAACUUCAACUUCAACGGCCUGACCGGCACCGGCGUGCUGACAGAGAGC AACAAGAAGUUCCUGCCAUUCCAGCAGUUUGGCCGGGAUAUCGCCGAUACCACAGACGCCGUUAGA GAUCCCCAGACACUGGAAAUCCUGGACAUCACCCCUUGCAGCUUCGGCGGAGUGUCUGUGAUCACC CCUGGCACCAACACCAGCAAUCAGGUGGCAGUGCUGUACCAGGGCGUGAACUGUACCGAAGUGCCC GUGGCCAUUCACGCCGAUCAGCUGACACCUACAUGGCGGGUGUACUCCACCGGCAGCAAUGUGUUU CAGACCAGAGCCGGCUGUCUGAUCGGAGCCGAGUACGUGAACAAUAGCUACGAGUGCGACAUCCCC AUCGGCGCUGGAAUCUGCGCCAGCUACCAGACACAGACAAAGAGCCACCGGAGAGCCAGAAGCGUG GCCAGCCAGAGCAUCAUUGCCUACACAAUGUCUCUGGGCGCCGAGAACAGCGUGGCCUACUCCAAC AACUCUAUCGCUAUCCCCACCAACUUCACCAUCAGCGUGACCACAGAGAUCCUGCCUGUGUCCAUG ACCAAGACCAGCGUGGACUGCACCAUGUACAUCUGCGGCGAUUCCACCGAGUGCUCCAACCUGCUG CUGCAGUACGGCAGCUUCUGCACCCAGCUGAAAAGAGCCCUGACAGGGAUCGCCGUGGAACAGGAC AAGAACACCCAAGAGGUGUUCGCCCAAGUGAAGCAGAUCUACAAGACCCCUCCUAUCAAGUACUUC GGCGGCUUCAAUUUCAGCCAGAUUCUGCCCGAUCCUAGCAAGCCCAGCAAGCGGAGCUUCAUCGAG GACCUGCUGUUCAACAAAGUGACACUGGCCGACGCCGGCUUCAUCAAGCAGUAUGGCGAUUGUCUG GGCGACAUUGCCGCCAGGGAUCUGAUUUGCGCCCAGAAGUUUAACGGACUGACAGUGCUGCCUCCU CUGCUGACCGAUGAGAUGAUCGCCCAGUACACAUCUGCCCUGCUGGCCGGCACAAUCACAAGCGGC UGGACAUUUGGAGCAGGCGCCGCUCUGCAGAUCCCCUUUGCUAUGCAGAUGGCCUACCGGUUCAAC GGCAUCGGAGUGACCCAGAAUGUGCUGUACGAGAACCAGAAGCUGAUCGCCAACCAGUUCAACAGC GCCAUCGGCAAGAUCCAGGACAGCCUGAGCAGCACAGCAAGCGCCCUGGGAAAGCUGCAGGACGUG GUCAACCACAAUGCCCAGGCACUGAACACCCUGGUCAAGCAGCUGUCCUCCAAGUUCGGCGCCAUC AGCUCUGUGCUGAACGAUAUCCUGAGCAGACUGGACCCUCCUGAGGCCGAGGUGCAGAUCGACAGA CUGAUCACAGGCAGACUGCAGAGCCUCCAGACAUACGUGACCCAGCAGCUGAUCAGAGCCGCCGAG AUUAGAGCCUCUGCCAAUCUGGCCGCCACCAAGAUGUCUGAGUGUGUGCUGGGCCAGAGCAAGAGA GUGGACUUUUGCGGCAAGGGCUACCACCUGAUGAGCUUCCCUCAGUCUGCCCCUCACGGCGUGGUG UUUCUGCACGUGACAUAUGUGCCCGCUCAAGAGAAGAAUUUCACCACCGCUCCAGCCAUCUGCCAC GACGGCAAAGCCCACUUUCCUAGAGAAGGCGUGUUCGUGUCCAACGGCACCCAUUGGUUCGUGACA CAGCGGAACUUCUACGAGCCCCAGAUCAUCACCACCGACAACACCUUCGUGUCUGGCAACUGCGAC GUCGUGAUCGGCAUUGUGAACAAUACCGUGUACGACCCUCUGCAGCCCGAGCUGGACAGCUUCAAA |
| | | GAGGAACUGGACAAGUACUUUAAGAACCACACAAGCCCCGACGUGGACCUGGGCGAUAUCAGCGGA AUCAAUGCCAGCGUCGUGAACAUCCAGAAAGAGAUCGACCGGCUGAACGAGGUGGCCAAGAAUCUG AACGAGAGCCUGAUCGACCUGCAAGAACUGGGGAAGUACGAGCAGUACAUCAAGUGGCCCUGGUAC AUCUGGCUGGGCUUUAUCGCCGGACUGAUUGCCAUCGUGAUGGUCACAAUCAUGCUGUGUUGCAUG ACCAGCUGCUGUAGCUGCCUGAAGGGCUGUUGUAGCUGUGGCAGCUGCUGCAAGUUCGACGAGGAC GAUUCUGAGCCCGUGCUGAAGGGCGUGAAACUGCACUACACAUGAUGA |
| 71 | DNA sequence encoding a SARS-CoV-2 S protein from an Omicron BA.4/BA.5 variant | ATGTTCGTGTTCCTGGTGCTGCTGCCTCTGGTGTCCAGCCAGTGTGTGAACCTGATCACCAGAACA CAGTCATACACCAACAGCTTTACCAGAGGCGTGTACTACCCCGACAAGGTGTTCAGATCCAGCGTG CTGCACTCTACCCAGGACCTGTTCCTGCCTTTCTTCAGCAACGTGACCTGGTTCCACGCCATCTCC GGCACCAATGGCACCAAGAGATTCGACAACCCCGTGCTGCCCTTCAACGACGGGGTGTACTTTGCC AGCACCGAGAAGTCCAACATCATCAGAGGCTGGATCTTCGGCACCACACTGGACAGCAAGACCCAG AGCCTGCTGATCGTGAACAACGCCACCAACGTGGTCATCAAAGTGTGCGAGTTCCAGTTCTGCAAC GACCCCTTCCTGGACGTCTACTACCACAAGAACAACAAGAGCTGGATGGAAAGCGAGTTCCGGGTG TACAGCAGCGCCAACAACTGCACCTTCGAGTACGTGTCCCAGCCTTTCCTGATGGACCTGGAAGGC AAGCAGGGCAACTTCAAGAACCTGCGCGAGTTCGTGTTTAAGAACATCGACGGCTACTTCAAGATC TACAGCAAGCACACCCCTATCAACCTCGGCCGGGATCTGCCTCAGGGCTTCTCTGCTCTGGAACCC CTGGTGGATCTGCCCATCGGCATCAACATCACCCGGTTTCAGACACTGCTGGCCCTGCACAGAAGC TACCTGACACCTGGCGATAGCAGCAGCGGATGGACAGCTGGTGCCGCCGCTTACTATGTGGGCTAC CTGCAGCCTAGAACCTTCCTGCTGAAGTACAACGAGAACGGCACCATCACCGACGCCGTGGATTGT GCTCTGGATCCTCTGAGCGAGACAAAGTGCACCCTGAAGTCCTTCACCGTGGAAAAGGGCATCTAC CAGACCAGCAACTTCCGGGTGCAGCCCACCGAATCCATCGTGCGGTTCCCCAATATCACCAATCTG TGCCCCTTCGACGAGGTGTTCAATGCCACCAGATTCGCCTCTGTGTACGCCTGGAACCGGAAGCGG ATCAGCAATTGCGTGGCCGACTACTCCGTGCTGTACAACTTCGCCCCCTTCTTCGCATTCAAGTGC TACGGCGTGTCCCCTACCAAGCTGAACGACCTGTGCTTCACAAACGTGTACGCCGACAGCTTCGTG ATCCGGGGAAACGAAGTGCGGCAGATTGCCCCTGGACAGACAGGCAACATCGCCGACTACAACTAC AAGCTGCCCGACGACTTCACCGGCTGTGTGATTGCCTGGAACAGCAACAAGCTGGACTCCAAAGTC GGCGGCAACTACAATTACAGGTACCGGCTGTTCCGGAAGTCCAATCTGAAGCCCTTCGAGCGGGAC ATCTCCACCGAGATCTATCAGGCCGGCAACAAGCCTTGTAACGGCGTGGCAGGCGTGAACTGCTAC TTCCCACTGCAGTCCTACGGCTTTAGGCCCACATACGGCGTGGGCCACCAGCCCTACAGAGTGGTG GTGCTGAGCTTCGAACTGCTGCATGCCCCTGCCACAGTGTGCGGCCCTAAGAAAAGCACCAATCTC GTGAAGAACAAATGCGTGAACTTCAACTTCAACGGCCTGACCGGCACCGGCGTGCTGACAGAGAGC AACAAGAAGTTCCTGCCATTCCAGCAGTTTGGCCGGGATATCGCCGATACCACAGACGCCGTTAGA GATCCCCAGACACTGGAAATCCTGGACATCACCCCTTGCAGCTTCGGCGGAGTGTCTGTGATCACC CCTGGCACCAACACCAGCAATCAGGTGGCAGTGCTGTACCAGGGCGTGAACTGTACCGAAGTGCCC GTGGCCATTCACGCCGATCAGCTGACACCTACATGGCGGGTGTACTCCACCGGCAGCAATGTGTTT CAGACCAGAGCCGGCTGTCTGATCGGAGCCGAGTACGTGAACAATAGCTACGAGTGCGACATCCCC ATCGGCGCTGGAATCTGCGCCAGCTACCAGACACAGACAAAGAGCCACCGGAGAGCCAGAAGCGTG GCCAGCCAGAGCATCATTGCCTACACAATGTCTCTGGGCGCCGAGAACAGCGTGGCCTACTCCAAC AACTCTATCGCTATCCCCACCAACTTCACCATCAGCGTGACCACAGAGATCCTGCCTGTGTCCATG |
| | | ACCAAGACCAGCGTGGACTGCACCATGTACATCTGCGGCGATTCCACCGAGTGCTCCAACCTGCTG CTGCAGTACGGCAGCTTCTGCACCCAGCTGAAAAGAGCCCTGACAGGGATCGCCGTGGAACAGGAC AAGAACACCCAAGAGGTGTTCGCCCAAGTGAAGCAGATCTACAAGACCCCTCCTATCAAGTACTTC GGCGGCTTCAATTTCAGCCAGATTCTGCCCGATCCTAGCAAGCCCAGCAAGCGGAGCTTCATCGAG GACCTGCTGTTCAACAAAGTGACACTGGCCGACGCCGGCTTCATCAAGCAGTATGGCGATTGTCTG GGCGACATTGCCGCCAGGGATCTGATTTGCGCCCAGAAGTTTAACGGACTGACAGTGCTGCCTCCT CTGCTGACCGATGAGATGATCGCCCAGTACACATCTGCCCTGCTGGCCGGCACAATCACAAGCGGC TGGACATTTGGAGCAGGCGCCGCTCTGCAGATCCCCTTTGCTATGCAGATGGCCTACCGGTTCAAC GGCATCGGAGTGACCCAGAATGTGCTGTACGAGAACCAGAAGCTGATCGCCAACCAGTTCAACAGC GCCATCGGCAAGATCCAGGACAGCCTGAGCAGCACAGCAAGCGCCCTGGGAAAGCTGCAGGACGTG GTCAACCACAATGCCCAGGCACTGAACACCCTGGTCAAGCAGCTGTCCTCCAAGTTCGGCGCCATC AGCTCTGTGCTGAACGATATCCTGAGCAGACTGGACCCTCCTGAGGCCGAGGTGCAGATCGACAGA CTGATCACAGGCAGACTGCAGAGCCTCCAGACATACGTGACCCAGCAGCTGATCAGAGCCGCCGAG ATTAGAGCCTCTGCCAATCTGGCCGCCACCAAGATGTCTGAGTGTGTGCTGGGCCAGAGCAAGAGA GTGGACTTTTGCGGCAAGGGCTACCACCTGATGAGCTTCCCTCAGTCTGCCCCTCACGGCGTGGTG TTTCTGCACGTGACATATGTGCCCGCTCAAGAGAAGAATTTCACCACCGCTCCAGCCATCTGCCAC GACGGCAAAGCCCACTTTCCTAGAGAAGGCGTGTTCGTGTCCAACGGCACCCATTGGTTCGTGACA CAGCGGAACTTCTACGAGCCCCAGATCATCACCACCGACAACACCTTCGTGTCTGGCAACTGCGAC GTCGTGATCGGCATTGTGAACAATACCGTGTACGACCCTCTGCAGCCCGAGCTGGACAGCTTCAAA GAGGAACTGGACAAGTACTTTAAGAACCACACAAGCCCCGACGTGGACCTGGGCGATATCAGCGGA ATCAATGCCAGCGTCGTGAACATCCAGAAAGAGATCGACCGGCTGAACGAGGTGGCCAAGAATCTG AACGAGAGCCTGATCGACCTGCAAGAACTGGGGAAGTACGAGCAGTACATCAAGTGGCCCTGGTAC ATCTGGCTGGGCTTTATCGCCGGACTGATTGCCATCGTGATGGTCACAATCATGCTGTGTTGCATG ACCAGCTGCTGTAGCTGCCTGAAGGGCTGTTGTAGCTGTGGCAGCTGCTGCAAGTTCGACGAGGAC GATTCTGAGCCCGTGCTGAAGGGCGTGAAACTGCACTACACATGATGA |
| 72 | Full length RNA construct sequence of RBP020.22 | AGAAUAAACUAGUAUUCUUCUGGUCCCCACAGACUCAGAGAGAACCCGCCACCAUGUUCGUGUUCC UGGUGCUGCUGCCUCUGGUGUCCAGCCAGUGUGUGAACCUGAUCACCAGAACACAGUCAUACACCA ACAGCUUUACCAGAGGCGUGUACUACCCCGACAAGGUGUUCAGAUCCAGCGUGCUGCACUCUACCC AGGACCUGUUCCUGCCUUUCUUCAGCAACGUGACCUGGUUCCACGCCAUCUCCGGCACCAAUGGCA CCAAGAGAUUCGACAACCCCGUGCUGCCCUUCAACGACGGGGUGUACUUUGCCAGCACCGAGAAGU CCAACAUCAUCAGAGGCUGGAUCUUCGGCACCACACUGGACAGCAAGACCCAGAGCCUGCUGAUCG UGAACAACGCCACCAACGUGGUCAUCAAAGUGUGCGAGUUCCAGUUCUGCAACGACCCCUUCCUGG ACGUCUACUACCACAAGAACAACAAGAGCUGGAUGGAAAGCGAGUUCCGGGUGUACAGCAGCGCCA ACAACUGCACCUUCGAGUACGUGUCCCAGCCUUUCCUGAUGGACCUGGAAGGCAAGCAGGGCAACU UCAAGAACCUGCGCGAGUUCGUGUUUAAGAACAUCGACGGCUACUUCAAGAUCUACAGCAAGCACA CCCCUAUCAACCUCGGCCGGGAUCUGCCUCAGGGCUUCUCUGCUCUGGAACCCCUGGUGGAUCUGC CCAUCGGCAUCAACAUCACCCGGUUUCAGACACUGCUGGCCCUGCACAGAAGCUACCUGACACCUG GCGAUAGCAGCAGCGGAUGGACAGCUGGUGCCGCCGCUUACUAUGUGGGCUACCUGCAGCCUAGAA CCUUCCUGCUGAAGUACAACGAGAACGGCACCAUCACCGACGCCGUGGAUUGUGCUCUGGAUCCUC |
| | | UGAGCGAGACAAAGUGCACCCUGAAGUCCUUCACCGUGGAAAAGGGCAUCUACCAGACCAGCAACU UCCGGGUGCAGCCCACCGAAUCCAUCGUGCGGUUCCCCAAUAUCACCAAUCUGUGCCCCUUCGACG AGGUGUUCAAUGCCACCAGAUUCGCCUCUGUGUACGCCUGGAACCGGAAGCGGAUCAGCAAUUGCG UGGCCGACUACUCCGUGCUGUACAACUUCGCCCCCUUCUUCGCAUUCAAGUGCUACGGCGUGUCCC CUACCAAGCUGAACGACCUGUGCUUCACAAACGUGUACGCCGACAGCUUCGUGAUCCGGGGAAACG AAGUGCGGCAGAUUGCCCCUGGACAGACAGGCAACAUCGCCGACUACAACUACAAGCUGCCCGACG ACUUCACCGGCUGUGUGAUUGCCUGGAACAGCAACAAGCUGGACUCCAAAGUCGGCGGCAACUACA AUUACAGGUACCGGCUGUUCCGGAAGUCCAAUCUGAAGCCCUUCGAGCGGGACAUCUCCACCGAGA UCUAUCAGGCCGGCAACAAGCCUUGUAACGGCGUGGCAGGCGUGAACUGCUACUUCCCACUGCAGU CCUACGGCUUUAGGCCCACAUACGGCGUGGGCCACCAGCCCUACAGAGUGGUGGUGCUGAGCUUCG AACUGCUGCAUGCCCCUGCCACAGUGUGCGGCCCUAAGAAAAGCACCAAUCUCGUGAAGAACAAAU GCGUGAACUUCAACUUCAACGGCCUGACCGGCACCGGCGUGCUGACAGAGAGCAACAAGAAGUUCC UGCCAUUCCAGCAGUUUGGCCGGGAUAUCGCCGAUACCACAGACGCCGUUAGAGAUCCCCAGACAC UGGAAAUCCUGGACAUCACCCCUUGCAGCUUCGGCGGAGUGUCUGUGAUCACCCCUGGCACCAACA CCAGCAAUCAGGUGGCAGUGCUGUACCAGGGCGUGAACUGUACCGAAGUGCCCGUGGCCAUUCACG CCGAUCAGCUGACACCUACAUGGCGGGUGUACUCCACCGGCAGCAAUGUGUUUCAGACCAGAGCCG GCUGUCUGAUCGGAGCCGAGUACGUGAACAAUAGCUACGAGUGCGACAUCCCCAUCGGCGCUGGAA UCUGCGCCAGCUACCAGACACAGACAAAGAGCCACCGGAGAGCCAGAAGCGUGGCCAGCCAGAGCA UCAUUGCCUACACAAUGUCUCUGGGCGCCGAGAACAGCGUGGCCUACUCCAACAACUCUAUCGCUA UCCCCACCAACUUCACCAUCAGCGUGACCACAGAGAUCCUGCCUGUGUCCAUGACCAAGACCAGCG UGGACUGCACCAUGUACAUCUGCGGCGAUUCCACCGAGUGCUCCAACCUGCUGCUGCAGUACGGCA GCUUCUGCACCCAGCUGAAAAGAGCCCUGACAGGGAUCGCCGUGGAACAGGACAAGAACACCCAAG AGGUGUUCGCCCAAGUGAAGCAGAUCUACAAGACCCCUCCUAUCAAGUACUUCGGCGGCUUCAAUU UCAGCCAGAUUCUGCCCGAUCCUAGCAAGCCCAGCAAGCGGAGCUUCAUCGAGGACCUGCUGUUCA ACAAAGUGACACUGGCCGACGCCGGCUUCAUCAAGCAGUAUGGCGAUUGUCUGGGCGACAUUGCCG CCAGGGAUCUGAUUUGCGCCCAGAAGUUUAACGGACUGACAGUGCUGCCUCCUCUGCUGACCGAUG AGAUGAUCGCCCAGUACACAUCUGCCCUGCUGGCCGGCACAAUCACAAGCGGCUGGACAUUUGGAG CAGGCGCCGCUCUGCAGAUCCCCUUUGCUAUGCAGAUGGCCUACCGGUUCAACGGCAUCGGAGUGA CCCAGAAUGUGCUGUACGAGAACCAGAAGCUGAUCGCCAACCAGUUCAACAGCGCCAUCGGCAAGA UCCAGGACAGCCUGAGCAGCACAGCAAGCGCCCUGGGAAAGCUGCAGGACGUGGUCAACCACAAUG CCCAGGCACUGAACACCCUGGUCAAGCAGCUGUCCUCCAAGUUCGGCGCCAUCAGCUCUGUGCUGA ACGAUAUCCUGAGCAGACUGGACCCUCCUGAGGCCGAGGUGCAGAUCGACAGACUGAUCACAGGCA GACUGCAGAGCCUCCAGACAUACGUGACCCAGCAGCUGAUCAGAGCCGCCGAGAUUAGAGCCUCUG CCAAUCUGGCCGCCACCAAGAUGUCUGAGUGUGUGCUGGGCCAGAGCAAGAGAGUGGACUUUUGCG GCAAGGGCUACCACCUGAUGAGCUUCCCUCAGUCUGCCCCUCACGGCGUGGUGUUUCUGCACGUGA CAUAUGUGCCCGCUCAAGAGAAGAAUUUCACCACCGCUCCAGCCAUCUGCCACGACGGCAAAGCCC ACUUUCCUAGAGAAGGCGUGUUCGUGUCCAACGGCACCCAUUGGUUCGUGACACAGCGGAACUUCU ACGAGCCCCAGAUCAUCACCACCGACAACACCUUCGUGUCUGGCAACUGCGACGUCGUGAUCGGCA UUGUGAACAAUACCGUGUACGACCCUCUGCAGCCCGAGCUGGACAGCUUCAAAGAGGAACUGGACA AGUACUUUAAGAACCACACAAGCCCCGACGUGGACCUGGGCGAUAUCAGCGGAAUCAAUGCCAGCG |
| | | UCGUGAACAUCCAGAAAGAGAUCGACCGGCUGAACGAGGUGGCCAAGAAUCUGAACGAGAGCCUGA UCGACCUGCAAGAACUGGGGAAGUACGAGCAGUACAUCAAGUGGCCCUGGUACAUCUGGCUGGGCU UUAUCGCCGGACUGAUUGCCAUCGUGAUGGUCACAAUCAUGCUGUGUUGCAUGACCAGCUGCUGUA GCUGCCUGAAGGGCUGUUGUAGCUGUGGCAGCUGCUGCAAGUUCGACGAGGACGAUUCUGAGCCCG UGCUGAAGGGCGUGAAACUGCACUACACAUGAUGACUCGAGCUGGUACUGCAUGCACGCAAUGCUA GCUGCCCCUUUCCCGUCCUGGGUACCCCGAGUCUCCCCCGACCUCGGGUCCCAGGUAUGCUCCCAC CUCCACCUGCCCCACUCACCACCUCUGCUAGUUCCAGACACCUCCCAAGCACGCAGCAAUGCAGCU CAAAACGCUUAGCCUAGCCACACCCCCACGGAAACAGCAGUGAUUAACCUUUAGCAAUAAACGAA AGUUUAACUAAGCUAUACUAACCCCAGGGUUGGUCAAUUUCGUGCCAGCCACACCCUGGAGCUAGC AAAAAAAAAAAAAAAAAAAAAAAAAAAAAAGCAUAUGACUAAAAAAAAAAAAAAAAAAAAAAAAAAAA AAAAAAAAAAAAAAAAAAAAAAAAAAAAAAAAAAAAAAAA |
| 73 | Full length DNA construct sequence of RBP020.22 | AGAATAAACTAGTATTCTTCTGGTCCCCACAGACTCAGAGAGAACCCGCCACCATGTTCGTGTTCC TGGTGCTGCTGCCTCTGGTGTCCAGCCAGTGTGTGAACCTGATCACCAGAACACAGTCATACACCA ACAGCTTTACCAGAGGCGTGTACTACCCCGACAAGGTGTTCAGATCCAGCGTGCTGCACTCTACCC AGGACCTGTTCCTGCCTTTCTTCAGCAACGTGACCTGGTTCCACGCCATCTCCGGCACCAATGGCA CCAAGAGATTCGACAACCCCGTGCTGCCCTTCAACGACGGGGTGTACTTTGCCAGCACCGAGAAGT CCAACATCATCAGAGGCTGGATCTTCGGCACCACACTGGACAGCAAGACCCAGAGCCTGCTGATCG TGAACAACGCCACCAACGTGGTCATCAAAGTGTGCGAGTTCCAGTTCTGCAACGACCCCTTCCTGG ACGTCTACTACCACAAGAACAACAAGAGCTGGATGGAAAGCGAGTTCCGGGTGTACAGCAGCGCCA ACAACTGCACCTTCGAGTACGTGTCCCAGCCTTTCCTGATGGACCTGGAAGGCAAGCAGGGCAACT TCAAGAACCTGCGCGAGTTCGTGTTTAAGAACATCGACGGCTACTTCAAGATCTACAGCAAGCACA CCCCTATCAACCTCGGCCGGGATCTGCCTCAGGGCTTCTCTGCTCTGGAACCCCTGGTGGATCTGC CCATCGGCATCAACATCACCCGGTTTCAGACACTGCTGGCCCTGCACAGAAGCTACCTGACACCTG GCGATAGCAGCAGCGGATGGACAGCTGGTGCCGCCGCTTACTATGTGGGCTACCTGCAGCCTAGAA CCTTCCTGCTGAAGTACAACGAGAACGGCACCATCACCGACGCCGTGGATTGTGCTCTGGATCCTC TGAGCGAGACAAAGTGCACCCTGAAGTCCTTCACCGTGGAAAAGGGCATCTACCAGACCAGCAACT TCCGGGTGCAGCCCACCGAATCCATCGTGCGGTTCCCCAATATCACCAATCTGTGCCCCTTCGACG AGGTGTTCAATGCCACCAGATTCGCCTCTGTGTACGCCTGGAACCGGAAGCGGATCAGCAATTGCG TGGCCGACTACTCCGTGCTGTACAACTTCGCCCCCTTCTTCGCATTCAAGTGCTACGGCGTGTCCC CTACCAAGCTGAACGACCTGTGCTTCACAAACGTGTACGCCGACAGCTTCGTGATCCGGGGAAACG AAGTGCGGCAGATTGCCCCTGGACAGACAGGCAACATCGCCGACTACAACTACAAGCTGCCCGACG ACTTCACCGGCTGTGTGATTGCCTGGAACAGCAACAAGCTGGACTCCAAAGTCGGCGGCAACTACA ATTACAGGTACCGGCTGTTCCGGAAGTCCAATCTGAAGCCCTTCGAGCGGGACATCTCCACCGAGA TCTATCAGGCCGGCAACAAGCCTTGTAACGGCGTGGCAGGCGTGAACTGCTACTTCCCACTGCAGT CCTACGGCTTTAGGCCCACATACGGCGTGGGCCACCAGCCCTACAGAGTGGTGGTGCTGAGCTTCG AACTGCTGCATGCCCCTGCCACAGTGTGCGGCCCTAAGAAAAGCACCAATCTCGTGAAGAACAAAT GCGTGAACTTCAACTTCAACGGCCTGACCGGCACCGGCGTGCTGACAGAGAGCAACAAGAAGTTCC TGCCATTCCAGCAGTTTGGCCGGGATATCGCCGATACCACAGACGCCGTTAGAGATCCCCAGACAC TGGAAATCCTGGACATCACCCCTTGCAGCTTCGGCGGAGTGTCTGTGATCACCCCTGGCACCAACA |
| | | CCAGCAATCAGGTGGCAGTGCTGTACCAGGGCGTGAACTGTACCGAAGTGCCCGTGGCCATTCACG CCGATCAGCTGACACCTACATGGCGGGTGTACTCCACCGGCAGCAATGTGTTTCAGACCAGAGCCG GCTGTCTGATCGGAGCCGAGTACGTGAACAATAGCTACGAGTGCGACATCCCCATCGGCGCTGGAA TCTGCGCCAGCTACCAGACACAGACAAAGAGCCACCGGAGAGCCAGAAGCGTGGCCAGCCAGAGCA TCATTGCCTACACAATGTCTCTGGGCGCCGAGAACAGCGTGGCCTACTCCAACAACTCTATCGCTA TCCCCACCAACTTCACCATCAGCGTGACCACAGAGATCCTGCCTGTGTCCATGACCAAGACCAGCG TGGACTGCACCATGTACATCTGCGGCGATTCCACCGAGTGCTCCAACCTGCTGCTGCAGTACGGCA GCTTCTGCACCCAGCTGAAAAGAGCCCTGACAGGGATCGCCGTGGAACAGGACAAGAACACCCAAG AGGTGTTCGCCCAAGTGAAGCAGATCTACAAGACCCCTCCTATCAAGTACTTCGGCGGCTTCAATT TCAGCCAGATTCTGCCCGATCCTAGCAAGCCCAGCAAGCGGAGCTTCATCGAGGACCTGCTGTTCA ACAAAGTGACACTGGCCGACGCCGGCTTCATCAAGCAGTATGGCGATTGTCTGGGCGACATTGCCG CCAGGGATCTGATTTGCGCCCAGAAGTTTAACGGACTGACAGTGCTGCCTCCTCTGCTGACCGATG AGATGATCGCCCAGTACACATCTGCCCTGCTGGCCGGCACAATCACAAGCGGCTGGACATTTGGAG CAGGCGCCGCTCTGCAGATCCCCTTTGCTATGCAGATGGCCTACCGGTTCAACGGCATCGGAGTGA CCCAGAATGTGCTGTACGAGAACCAGAAGCTGATCGCCAACCAGTTCAACAGCGCCATCGGCAAGA TCCAGGACAGCCTGAGCAGCACAGCAAGCGCCCTGGGAAAGCTGCAGGACGTGGTCAACCACAATG CCCAGGCACTGAACACCCTGGTCAAGCAGCTGTCCTCCAAGTTCGGCGCCATCAGCTCTGTGCTGA ACGATATCCTGAGCAGACTGGACCCTCCTGAGGCCGAGGTGCAGATCGACAGACTGATCACAGGCA GACTGCAGAGCCTCCAGACATACGTGACCCAGCAGCTGATCAGAGCCGCCGAGATTAGAGCCTCTG CCAATCTGGCCGCCACCAAGATGTCTGAGTGTGTGCTGGGCCAGAGCAAGAGAGTGGACTTTTGCG GCAAGGGCTACCACCTGATGAGCTTCCCTCAGTCTGCCCCTCACGGCGTGGTGTTTCTGCACGTGA CATATGTGCCCGCTCAAGAGAAGAATTTCACCACCGCTCCAGCCATCTGCCACGACGGCAAAGCCC ACTTTCCTAGAGAAGGCGTGTTCGTGTCCAACGGCACCCATTGGTTCGTGACACAGCGGAACTTCT ACGAGCCCCAGATCATCACCACCGACAACACCTTCGTGTCTGGCAACTGCGACGTCGTGATCGGCA TTGTGAACAATACCGTGTACGACCCTCTGCAGCCCGAGCTGGACAGCTTCAAAGAGGAACTGGACA AGTACTTTAAGAACCACACAAGCCCCGACGTGGACCTGGGCGATATCAGCGGAATCAATGCCAGCG TCGTGAACATCCAGAAAGAGATCGACCGGCTGAACGAGGTGGCCAAGAATCTGAACGAGAGCCTGA TCGACCTGCAAGAACTGGGGAAGTACGAGCAGTACATCAAGTGGCCCTGGTACATCTGGCTGGGCT TTATCGCCGGACTGATTGCCATCGTGATGGTCACAATCATGCTGTGTTGCATGACCAGCTGCTGTA GCTGCCTGAAGGGCTGTTGTAGCTGTGGCAGCTGCTGCAAGTTCGACGAGGACGATTCTGAGCCCG TGCTGAAGGGCGTGAAACTGCACTACACATGATGACTCGAGCTGGTACTGCATGCACGCAATGCTA GCTGCCCCTTTCCCGTCCTGGGTACCCCGAGTCTCCCCCGACCTCGGGTCCCAGGTATGCTCCCAC CTCCACCTGCCCCACTCACCACCTCTGCTAGTTCCAGACACCTCCCAAGCACGCAGCAATGCAGCT CAAAACGCTTAGCCTAGCCACACCCCCACGGGAAACAGCAGTGATTAACCTTTAGCAATAAACGAA AGTTTAACTAAGCTATACTAACCCCAGGGTTGGTCAATTTCGTGCCAGCCACACCCTGGAGCTAGC AAAAAAAAAAAAAAAAAAAAAAAAAAAAAAGCATATGACTAAAAAAAAAAAAAAAAAAAAAAAAAA AAAAAAAAAAAAAAAAAAAAAAAAAAAAAAAAAAAAAAAAAA |

**Table 9: Description of RBP020.22 (Omicron BA.4/BA.5-specific RNA vaccine) as shown in Table 8 above (positioning of amino acid mutations shown relative to SEQ ID NO: 1)**

| **Construct** | **Omicron BA.4/BA.5 P2** | |
|---|---|---|
| **Antigen** | P2-mutated full spike protein | |
| **Changes** | Amino Acid^{a} | mRNA Nucleotides (location) |
| **Furin site** | RRAR | CGGAGAGCCAGA (2082-2093) |
| **Proline** | K986P | CCU (3000-3002) |
| | V987P | CCU (3003-3005) |
| **Lineage** | T19I | AUC (108-110) |
| | L24del | / |
| | P25del | / |
| | P26del | / |
| | A27S | UCA (123-125) |
| | H69del | / |
| | V70del | / |
| | G142D | GAC (462-464) |
| | V213G | GGC (675-677) |
| | G339D | GAC (1053-1055) |
| | S371F | UUC (1149-1151) |
| | S373P | CCC (1155-1157) |
| | S375F | UUC (1161-1163) |
| | T376A | GCA (1164-1166) |
| | D405N | AAC (1251-1253) |
| | K417N | AAC (1287-1289) |
| | N440K | AAG (1356-1358) |
| | L452R | AGG (1392-1394) |
| | S477N | AAC (1467-1469) |
| | T478K | AAG (1470-1472) |
| | E484A | GCA (1488-1490) |
| | F486V | GUG (1494-1496) |
| | Q498R | AGG (1530-1532) |
| | N501Y | UAC (1539-1541) |
| | Y505H | CAC (1551-1553) |
| | D614G | GGC (1878-1880) |
| | H655Y | UAC (2001-2003) |
| | N679K | AAG (2073-2075) |
| | P681H | CAC (2079-2081) |
| | N764K | AAA (2328-2330) |
| | D796Y | UAC (2424-2426) |
| | Q954H | CAC (2898-2900) |
| | N969K | AAG (2943-2945) |

**Table 10: Sequence of one embodiment of Omicron BA.4/BA.5-specific RNA vaccine**

| SEQ ID NO. | Brief Description | Sequence |
|---|---|---|
| 74 | Amino acid sequence of RNA-encoded SARS-CoV-2 S protein from an Omicron BA.4/BA.5 variant (with PRO mutations at positions corresponding to K986P and V987P of SEQ ID NO: 1; i.e., PRO mutations at positions 981 and 982 of SEQ ID NO: 74) | MFVFLVLLPLVSSQCVNLITRTQSYTNSFTRGVYYPDKVFRSSVLHSTQDLFLPFFSNVTWFHAIS GTNGTKRFDNPVLPFNDGVYFASTEKSNIIRGWIFGTTLDSKTQSLLIVNNATNVVIKVCEFQFCN DPFLDVYYHKNNKSWMESEFRVYSSANNCTFEYVSQPFLMDLEGKQGNFKNLREFVFKNIDGYFKI YSKHTPINLGRDLPQGFSALEPLVDLPIGINITRFQTLLALHRSYLTPGDSSSGWTAGAAAYYVGY LQPRTFLLKYNENGTITDAVDCALDPLSETKCTLKSFTVEKGIYQTSNFRVQPTESIVRFPNITNL CPFDEVFNATRFASVYAWNRKRISNCVADYSVLYNFAPFFAFKCYGVSPTKLNDLCFTNVYADSFV IRGNEVSQIAPGQTGNIADYNYKLPDDFTGCVIAWNSNKLDSKVGGNYNYRYRLFRKSNLKPFERD ISTEIYQAGNKPCNGVAGVNCYFPLQSYGFRPTYGVGHQPYRVVVLSFELLHAPATVCGPKKSTNL VKNKCVNFNFNGLTGTGVLTESNKKFLPFQQFGRDIADTTDAVRDPQTLEILDITPCSFGGVSVIT PGTNTSNQVAVLYQGVNCTEVPVAIHADQLTPTWRVYSTGSNVFQTRAGCLIGAEYVNNSYECDIP IGAGICASYQTQTKSHRRARSVASQSIIAYTMSLGAENSVAYSNNSIAIPTNFTISVTTEILPVSM TKTSVDCTMYICGDSTECSNLLLQYGSFCTQLKRALTGIAVEQDKNTQEVFAQVKQIYKTPPIKYF GGFNFSQILPDPSKPSKRSFIEDLLFNKVTLADAGFIKQYGDCLGDIAARDLICAQKFNGLTVLPP LLTDEMIAQYTSALLAGTITSGWTFGAGAALQIPFAMQMAYRFNGIGVTQNVLYENQKLIANQFNS AIGKIQDSLSSTASALGKLQDVVNHNAQALNTLVKQLSSKFGAISSVLNDILSRLDPPEAEVQIDR LITGRLQSLQTYVTQQLIRAAEIRASANLAATKMSECVLGQSKRVDFCGKGYHLMSFPQSAPHGVV FLHVTYVPAQEKNFTTAPAICHDGKAHFPREGVFVSNGTHWFVTQRNFYEPQIITTDNTFVSGNCD VVIGIVNNTVYDPLQPELDSFKEELDKYFKNHTSPDVDLGDISGINASVVNIQKEIDRLNEVAKNL NESLIDLQELGKYEQYIKWPWYIWLGFIAGLIAIVMVTIMLCCMTSCCSCLKGCCSCGSCCKFDED DSEPVLKGVKLHYT |
| 75 | RNA sequence encoding a SARS-CoV-2 S protein from an Omicron BA.4/BA.5 variant (with proline residues at positions corresponding to | AUGUUCGUGUUCCUGGUGCUGCUGCCUCUGGUGUCCAGCCAGUGUGUGAACCUGAUCACCAGAACA CAGAGCUACACCAACAGCUUUACCAGAGGCGUGUACUACCCCGACAAGGUGUUCAGAUCCAGCGUG CUGCACUCUACCCAGGACCUGUUCCUGCCUUUCUUCAGCAACGUGACCUGGUUCCACGCCAUCUCC GGCACCAAUGGCACCAAGAGAUUCGACAACCCCGUGCUGCCCUUCAACGACGGGGUGUACUUUGCC AGCACCGAGAAGUCCAACAUCAUCAGAGGCUGGAUCUUCGGCACCACACUGGACAGCAAGACCCAG AGCCUGCUGAUCGUGAACAACGCCACCAACGUGGUCAUCAAAGUGUGCGAGUUCCAGUUCUGCAAC GACCCCUUCCUGGACGUGUACUACCACAAGAACAACAAGAGCUGGAUGGAAAGCGAGUUCCGGGUG UACAGCAGCGCCAACAACUGCACCUUCGAGUACGUGUCCCAGCCUUUCCUGAUGGACCUGGAAGGC AAGCAGGGCAACUUCAAGAACCUGCGCGAGUUCGUGUUUAAGAACAUCGACGGCUACUUCAAGAUC UACAGCAAGCACACCCCUAUCAACCUCGGCCGGGAUCUGCCUCAGGGCUUCUCUGCUCUGGAACCC CUGGUGGAUCUGCCCAUCGGCAUCAACAUCACCCGGUUUCAGACACUGCUGGCCCUGCACAGAAGC |
| | K986P and V987P of SEQ ID NO: 1) | UACCUGACACCUGGCGAUAGCAGCAGCGGAUGGACAGCUGGUGCCGCCGCUUACUAUGUGGGCUAC CUGCAGCCUAGAACCUUCCUGCUGAAGUACAACGAGAACGGCACCAUCACCGACGCCGUGGAUUGU GCUCUGGAUCCUCUGAGCGAGACAAAGUGCACCCUGAAGUCCUUCACCGUGGAAAAGGGCAUCUAC CAGACCAGCAACUUCCGGGUGCAGCCCACCGAAUCCAUCGUGCGGUUCCCCAAUAUCACCAAUCUG UGCCCCUUCGACGAGGUGUUCAAUGCCACCAGAUUCGCCUCUGUGUACGCCUGGAACCGGAAGCGG AUCAGCAAUUGCGUGGCCGACUACUCCGUGCUGUACAACUUCGCCCCCUUCUUCGCCUUCAAGUGC UACGGCGUGUCCCCUACCAAGCUGAACGACCUGUGCUUCACAAACGUGUACGCCGACAGCUUCGUG AUCCGGGGAAACGAAGUGAGCCAGAUUGCCCCUGGACAGACAGGCAACAUCGCCGACUACAACUAC AAGCUGCCCGACGACUUCACCGGCUGUGUGAUUGCCUGGAACAGCAACAAGCUGGACUCCAAAGUC GGCGGCAACUACAAUUACAGGUACCGGCUGUUCCGGAAGUCCAAUCUGAAGCCCUUCGAGCGGGAC AUCUCCACCGAGAUCUAUCAGGCCGGCAACAAGCCUUGUAACGGCGUGGCCGGCGUGAACUGCUAC UUCCCACUGCAGUCCUACGGCUUUAGGCCCACAUAUGGCGUGGGCCAUCAGCCCUACAGAGUGGUG GUGCUGAGCUUCGAACUGCUGCAUGCCCCUGCCACAGUGUGCGGCCCUAAGAAAAGCACCAAUCUC GUGAAGAACAAAUGCGUGAACUUCAACUUCAACGGCCUGACCGGCACCGGCGUGCUGACAGAGAGC AACAAGAAGUUCCUGCCAUUCCAGCAGUUUGGCCGGGAUAUCGCCGAUACCACAGACGCCGUUAGA GAUCCCCAGACACUGGAAAUCCUGGACAUCACCCCUUGCAGCUUCGGCGGAGUGUCUGUGAUCACC CCUGGCACCAACACCAGCAAUCAGGUGGCAGUGCUGUACCAGGGCGUGAACUGUACCGAAGUGCCC GUGGCCAUUCACGCCGAUCAGCUGACACCUACAUGGCGGGUGUACUCCACCGGCAGCAAUGUGUUU CAGACCAGAGCCGGCUGUCUGAUCGGAGCCGAGUACGUGAACAAUAGCUACGAGUGCGACAUCCCC AUCGGCGCUGGAAUCUGCGCCAGCUACCAGACACAGACAAAGAGCCACCGGAGAGCCAGAAGCGUG GCCAGCCAGAGCAUCAUUGCCUACACAAUGUCUCUGGGCGCCGAGAACAGCGUGGCCUACUCCAAC AACUCUAUCGCUAUCCCCACCAACUUCACCAUCAGCGUGACCACAGAGAUCCUGCCUGUGUCCAUG ACCAAGACCAGCGUGGACUGCACCAUGUACAUCUGCGGCGAUUCCACCGAGUGCUCCAACCUGCUG CUGCAGUACGGCAGCUUCUGCACCCAGCUGAAGAGAGCCCUGACAGGGAUCGCCGUGGAACAGGAC AAGAACACCCAAGAGGUGUUCGCCCAAGUGAAGCAGAUCUACAAGACCCCUCCUAUCAAGUACUUC GGCGGCUUCAAUUUCAGCCAGAUUCUGCCCGAUCCUAGCAAGCCCAGCAAGCGGAGCUUCAUCGAG GACCUGCUGUUCAACAAAGUGACACUGGCCGACGCCGGCUUCAUCAAGCAGUAUGGCGAUUGUCUG GGCGACAUUGCCGCCAGGGAUCUGAUUUGCGCCCAGAAGUUUAACGGACUGACAGUGCUGCCUCCU CUGCUGACCGAUGAGAUGAUCGCCCAGUACACAUCUGCCCUGCUGGCCGGCACAAUCACAAGCGGC UGGACAUUUGGAGCAGGCGCCGCUCUGCAGAUCCCCUUUGCUAUGCAGAUGGCCUACCGGUUCAAC GGCAUCGGAGUGACCCAGAAUGUGCUGUACGAGAACCAGAAGCUGAUCGCCAACCAGUUCAACAGC GCCAUCGGCAAGAUCCAGGACAGCCUGAGCAGCACAGCAAGCGCCCUGGGAAAGCUGCAGGACGUG GUCAACCACAAUGCCCAGGCACUGAACACCCUGGUCAAGCAGCUGUCCUCCAAGUUCGGCGCCAUC AGCUCUGUGCUGAACGAUAUCCUGAGCAGACUGGACCCUCCUGAGGCCGAGGUGCAGAUCGACAGA CUGAUCACAGGCAGACUGCAGAGCCUCCAGACAUACGUGACCCAGCAGCUGAUCAGAGCCGCCGAG AUUAGAGCCUCUGCCAAUCUGGCCGCCACCAAGAUGUCUGAGUGUGUGCUGGGCCAGAGCAAGAGA GUGGACUUUUGCGGCAAGGGCUACCACCUGAUGAGCUUCCCUCAGUCUGCCCCUCACGGCGUGGUG UUUCUGCACGUGACAUAUGUGCCCGCUCAAGAGAAGAAUUUCACCACCGCUCCAGCCAUCUGCCAC GACGGCAAAGCCCACUUUCCUAGAGAAGGCGUGUUCGUGUCCAACGGCACCCAUUGGUUCGUGACA CAGCGGAACUUCUACGAGCCCCAGAUCAUCACCACCGACAACACCUUCGUGUCUGGCAACUGCGAC |
| | | GUCGUGAUCGGCAUUGUGAACAAUACCGUGUACGACCCUCUGCAGCCCGAGCUGGACAGCUUCAAA GAGGAACUGGACAAGUACUUUAAGAACCACACAAGCCCCGACGUGGACCUGGGCGAUAUCAGCGGA AUCAAUGCCAGCGUCGUGAACAUCCAGAAAGAGAUCGACCGGCUGAACGAGGUGGCCAAGAAUCUG AACGAGAGCCUGAUCGACCUGCAAGAACUGGGGAAGUACGAGCAGUACAUCAAGUGGCCCUGGUAC AUCUGGCUGGGCUUUAUCGCCGGACUGAUUGCCAUCGUGAUGGUCACAAUCAUGCUGUGUUGCAUG ACCAGCUGCUGUAGCUGCCUGAAGGGCUGUUGUAGCUGUGGCAGCUGCUGCAAGUUCGACGAGGAC GAUUCUGAGCCCGUGCUGAAGGGCGUGAAACUGCACUACACAUGAUGA |
| 76 | DNA sequence encoding a SARS-CoV-2 S protein from an Omicron BA.4/BA.5 variant (with proline residues at positions corresponding to K986P and V987P of SEQ ID NO: 1) | ATGTTCGTGTTCCTGGTGCTGCTGCCTCTGGTGTCCAGCCAGTGTGTGAACCTGATCACCAGAACA CAGAGCTACACCAACAGCTTTACCAGAGGCGTGTACTACCCCGACAAGGTGTTCAGATCCAGCGTG CTGCACTCTACCCAGGACCTGTTCCTGCCTTTCTTCAGCAACGTGACCTGGTTCCACGCCATCTCC GGCACCAATGGCACCAAGAGATTCGACAACCCCGTGCTGCCCTTCAACGACGGGGTGTACTTTGCC AGCACCGAGAAGTCCAACATCATCAGAGGCTGGATCTTCGGCACCACACTGGACAGCAAGACCCAG AGCCTGCTGATCGTGAACAACGCCACCAACGTGGTCATCAAAGTGTGCGAGTTCCAGTTCTGCAAC GACCCCTTCCTGGACGTGTACTACCACAAGAACAACAAGAGCTGGATGGAAAGCGAGTTCCGGGTG TACAGCAGCGCCAACAACTGCACCTTCGAGTACGTGTCCCAGCCTTTCCTGATGGACCTGGAAGGC AAGCAGGGCAACTTCAAGAACCTGCGCGAGTTCGTGTTTAAGAACATCGACGGCTACTTCAAGATC TACAGCAAGCACACCCCTATCAACCTCGGCCGGGATCTGCCTCAGGGCTTCTCTGCTCTGGAACCC CTGGTGGATCTGCCCATCGGCATCAACATCACCCGGTTTCAGACACTGCTGGCCCTGCACAGAAGC TACCTGACACCTGGCGATAGCAGCAGCGGATGGACAGCTGGTGCCGCCGCTTACTATGTGGGCTAC CTGCAGCCTAGAACCTTCCTGCTGAAGTACAACGAGAACGGCACCATCACCGACGCCGTGGATTGT GCTCTGGATCCTCTGAGCGAGACAAAGTGCACCCTGAAGTCCTTCACCGTGGAAAAGGGCATCTAC CAGACCAGCAACTTCCGGGTGCAGCCCACCGAATCCATCGTGCGGTTCCCCAATATCACCAATCTG TGCCCCTTCGACGAGGTGTTCAATGCCACCAGATTCGCCTCTGTGTACGCCTGGAACCGGAAGCGG ATCAGCAATTGCGTGGCCGACTACTCCGTGCTGTACAACTTCGCCCCCTTCTTCGCCTTCAAGTGC TACGGCGTGTCCCCTACCAAGCTGAACGACCTGTGCTTCACAAACGTGTACGCCGACAGCTTCGTG ATCCGGGGAAACGAAGTGAGCCAGATTGCCCCTGGACAGACAGGCAACATCGCCGACTACAACTAC AAGCTGCCCGACGACTTCACCGGCTGTGTGATTGCCTGGAACAGCAACAAGCTGGACTCCAAAGTC GGCGGCAACTACAATTACAGGTACCGGCTGTTCCGGAAGTCCAATCTGAAGCCCTTCGAGCGGGAC ATCTCCACCGAGATCTATCAGGCCGGCAACAAGCCTTGTAACGGCGTGGCCGGCGTGAACTGCTAC TTCCCACTGCAGTCCTACGGCTTTAGGCCCACATATGGCGTGGGCCATCAGCCCTACAGAGTGGTG GTGCTGAGCTTCGAACTGCTGCATGCCCCTGCCACAGTGTGCGGCCCTAAGAAAAGCACCAATCTC GTGAAGAACAAATGCGTGAACTTCAACTTCAACGGCCTGACCGGCACCGGCGTGCTGACAGAGAGC AACAAGAAGTTCCTGCCATTCCAGCAGTTTGGCCGGGATATCGCCGATACCACAGACGCCGTTAGA GATCCCCAGACACTGGAAATCCTGGACATCACCCCTTGCAGCTTCGGCGGAGTGTCTGTGATCACC CCTGGCACCAACACCAGCAATCAGGTGGCAGTGCTGTACCAGGGCGTGAACTGTACCGAAGTGCCC GTGGCCATTCACGCCGATCAGCTGACACCTACATGGCGGGTGTACTCCACCGGCAGCAATGTGTTT CAGACCAGAGCCGGCTGTCTGATCGGAGCCGAGTACGTGAACAATAGCTACGAGTGCGACATCCCC ATCGGCGCTGGAATCTGCGCCAGCTACCAGACACAGACAAAGAGCCACCGGAGAGCCAGAAGCGTG GCCAGCCAGAGCATCATTGCCTACACAATGTCTCTGGGCGCCGAGAACAGCGTGGCCTACTCCAAC |
| | | AACTCTATCGCTATCCCCACCAACTTCACCATCAGCGTGACCACAGAGATCCTGCCTGTGTCCATG ACCAAGACCAGCGTGGACTGCACCATGTACATCTGCGGCGATTCCACCGAGTGCTCCAACCTGCTG CTGCAGTACGGCAGCTTCTGCACCCAGCTGAAGAGAGCCCTGACAGGGATCGCCGTGGAACAGGAC AAGAACACCCAAGAGGTGTTCGCCCAAGTGAAGCAGATCTACAAGACCCCTCCTATCAAGTACTTC GGCGGCTTCAATTTCAGCCAGATTCTGCCCGATCCTAGCAAGCCCAGCAAGCGGAGCTTCATCGAG GACCTGCTGTTCAACAAAGTGACACTGGCCGACGCCGGCTTCATCAAGCAGTATGGCGATTGTCTG GGCGACATTGCCGCCAGGGATCTGATTTGCGCCCAGAAGTTTAACGGACTGACAGTGCTGCCTCCT CTGCTGACCGATGAGATGATCGCCCAGTACACATCTGCCCTGCTGGCCGGCACAATCACAAGCGGC TGGACATTTGGAGCAGGCGCCGCTCTGCAGATCCCCTTTGCTATGCAGATGGCCTACCGGTTCAAC GGCATCGGAGTGACCCAGAATGTGCTGTACGAGAACCAGAAGCTGATCGCCAACCAGTTCAACAGC GCCATCGGCAAGATCCAGGACAGCCTGAGCAGCACAGCAAGCGCCCTGGGAAAGCTGCAGGACGTG GTCAACCACAATGCCCAGGCACTGAACACCCTGGTCAAGCAGCTGTCCTCCAAGTTCGGCGCCATC AGCTCTGTGCTGAACGATATCCTGAGCAGACTGGACCCTCCTGAGGCCGAGGTGCAGATCGACAGA CTGATCACAGGCAGACTGCAGAGCCTCCAGACATACGTGACCCAGCAGCTGATCAGAGCCGCCGAG ATTAGAGCCTCTGCCAATCTGGCCGCCACCAAGATGTCTGAGTGTGTGCTGGGCCAGAGCAAGAGA GTGGACTTTTGCGGCAAGGGCTACCACCTGATGAGCTTCCCTCAGTCTGCCCCTCACGGCGTGGTG TTTCTGCACGTGACATATGTGCCCGCTCAAGAGAAGAATTTCACCACCGCTCCAGCCATCTGCCAC GACGGCAAAGCCCACTTTCCTAGAGAAGGCGTGTTCGTGTCCAACGGCACCCATTGGTTCGTGACA CAGCGGAACTTCTACGAGCCCCAGATCATCACCACCGACAACACCTTCGTGTCTGGCAACTGCGAC GTCGTGATCGGCATTGTGAACAATACCGTGTACGACCCTCTGCAGCCCGAGCTGGACAGCTTCAAA GAGGAACTGGACAAGTACTTTAAGAACCACACAAGCCCCGACGTGGACCTGGGCGATATCAGCGGA ATCAATGCCAGCGTCGTGAACATCCAGAAAGAGATCGACCGGCTGAACGAGGTGGCCAAGAATCTG AACGAGAGCCTGATCGACCTGCAAGAACTGGGGAAGTACGAGCAGTACATCAAGTGGCCCTGGTAC ATCTGGCTGGGCTTTATCGCCGGACTGATTGCCATCGTGATGGTCACAATCATGCTGTGTTGCATG ACCAGCTGCTGTAGCTGCCTGAAGGGCTGTTGTAGCTGTGGCAGCTGCTGCAAGTTCGACGAGGAC GATTCTGAGCCCGTGCTGAAGGGCGTGAAACTGCACTACACATGATGA |

**Table 11. Sequence of one embodiment of Omicron BA.4/BA.5-specific RNA vaccine**

| SEQ ID NO. | Brief Description | Sequence |
|---|---|---|
| 77 | Amino acid sequence of RNA-encoded SARS-CoV-2 S protein from an Omicron BA.4/BA.5 variant (with PRO mutations at positions corresponding to K986P and V987P of SEQ ID NO: 1; i.e., PRO mutations at positions 981 and 982 of SEQ ID NO: 77) | MFVFLVLLPLVSSQCVNLITRTQSYTNSFTRGVYYPDKVFRSSVLHSTQDLFLPFFSNVTWFHAIS GTNGTKRFDNPVLPFNDGVYFASTEKSNIIRGWIFGTTLDSKTQSLLIVNNATNVVIKVCEFQFCN DPFLDVYYHKNNKSWMESEFRVYSSANNCTFEYVSQPFLMDLEGKQGNFKNLREFVFKNIDGYFKI YSKHTPINLGRDLPQGFSALEPLVDLPIGINITRFQTLLALHRSYLTPGDSSSGWTAGAAAYYVGY LQPRTFLLKYNENGTITDAVDCALDPLSETKCTLKSFTVEKGIYQTSNFRVQPTESIVRFPNITNL CPFDEVFNATRFASVYAWNRKRISNCVADYSVLYNFAPFFAFKCYGVSPTKLNDLCFTNVYADSFV IRGNEVRQIAPGQTGNIADYNYKLPDDFTGCVIAWNSNKLDSKVGGNYNYRYRLFRKSNLKPFERD ISTEIYQAGNKPCNGVAGVNCYFPLQSYGFRPTYGVGHQPYRVVVLSFELLHAPATVCGPKKSTNL VKNKCVNFNFNGLTGTGVLTESNKKFLPFQQFGRDIADTTDAVRDPQTLEILDITPCSFGGVSVIT PGTNTSNQVAVLYQGVNCTEVPVAIHADQLTPTWRVYSTGSNVFQTRAGCLIGAEYVNNSYECDIP IGAGICASYQTQTKSHRRARSVASQSIIAYTMSLGAENSVAYSNNSIAIPTNFTISVTTEILPVSM TKTSVDCTMYICGDSTECSNLLLQYGSFCTQLKRALTGIAVEQDKNTQEVFAQVKQIYKTPPIKYF GGFNFSQILPDPSKPSKRSFIEDLLFNKVTLADAGFIKQYGDCLGDIAARDLICAQKFNGLTVLPP LLTDEMIAQYTSALLAGTITSGWTFGAGAALQIPFAMQMAYRFNGIGVTQNVLYENQKLIANQFNS AIGKIQDSLSSTASALGKLQDVVNHNAQALNTLVKQLSSKFGAISSVLNDILSRLDPPEAEVQIDR LITGRLQSLQTYVTQQLIRAAEIRASANLAATKMSECVLGQSKRVDFCGKGYHLMSFPQSAPHGVV FLHVTYVPAQEKNFTTAPAICHDGKAHFPREGVFVSNGTHWFVTQRNFYEPQIITTDNTFVSGNCD VVIGIVNNTVYDPLQPELDSFKEELDKYFKNHTSPDVDLGDISGINASVVNIQKEIDRLNEVAKNL NESLIDLQELGKYEQYIKWPWYIWLGFIAGLIAIVMVTIMLCCMTSCCSCLKGCCSCGSCCKFDED DSEPVLKGVKLHYT |
| 78 | RNA sequence encoding a SARS-CoV-2 S protein from an Omicron BA.4/BA.5 variant | AUGUUCGUGUUCCUGGUGCUGCUGCCUCUGGUGUCCAGCCAGUGUGUGAACCUGAUCACCAGAACA CAGUCAUACACCAACAGCUUUACCAGAGGCGUGUACUACCCCGACAAGGUGUUCAGAUCCAGCGUG CUGCACUCUACCCAGGACCUGUUCCUGCCUUUCUUCAGCAACGUGACCUGGUUCCACGCCAUCUCC GGCACCAAUGGCACCAAGAGAUUCGACAACCCCGUGCUGCCCUUCAACGACGGGGUGUACUUUGCC AGCACCGAGAAGUCCAACAUCAUCAGAGGCUGGAUCUUCGGCACCACACUGGACAGCAAGACCCAG AGCCUGCUGAUCGUGAACAACGCCACCAACGUGGUCAUCAAAGUGUGCGAGUUCCAGUUCUGCAAC GACCCCUUCCUGGACGUCUACUACCACAAGAACAACAAGAGCUGGAUGGAAAGCGAGUUCCGGGUG UACAGCAGCGCCAACAACUGCACCUUCGAGUACGUGUCCCAGCCUUUCCUGAUGGACCUGGAAGGC AAGCAGGGCAACUUCAAGAACCUGCGCGAGUUCGUGUUUAAGAACAUCGACGGCUACUUCAAGAUC |
| | | UACAGCAAGCACACCCCUAUCAACCUCGGCCGGGAUCUGCCUCAGGGCUUCUCUGCUCUGGAACCC CUGGUGGAUCUGCCCAUCGGCAUCAACAUCACCCGGUUUCAGACACUGCUGGCCCUGCACAGAAGC UACCUGACACCUGGCGAUAGCAGCAGCGGAUGGACAGCUGGUGCCGCCGCUUACUAUGUGGGCUAC CUGCAGCCUAGAACCUUCCUGCUGAAGUACAACGAGAACGGCACCAUCACCGACGCCGUGGAUUGU GCUCUGGAUCCUCUGAGCGAGACAAAGUGCACCCUGAAGUCCUUCACCGUGGAAAAGGGCAUCUAC CAGACCAGCAACUUCCGGGUGCAGCCCACCGAAUCCAUCGUGCGGUUCCCCAAUAUCACCAAUCUG UGCCCCUUCGACGAGGUGUUCAAUGCCACCAGAUUCGCCUCUGUGUACGCCUGGAACCGGAAGCGG AUCAGCAAUUGCGUGGCCGACUACUCCGUGCUGUACAACUUCGCCCCCUUCUUCGCAUUCAAGUGC UACGGCGUGUCCCCUACCAAGCUGAACGACCUGUGCUUCACAAACGUGUACGCCGACAGCUUCGUG AUCCGGGGAAACGAAGUGCGGCAGAUUGCCCCUGGACAGACAGGCAACAUCGCCGACUACAACUAC AAGCUGCCCGACGACUUCACCGGCUGUGUGAUUGCCUGGAACAGCAACAAGCUGGACUCCAAAGUC GGCGGCAACUACAAUUACAGGUACCGGCUGUUCCGGAAGUCCAAUCUGAAGCCCUUCGAGCGGGAC AUCUCCACCGAGAUCUAUCAGGCCGGCAACAAGCCUUGUAACGGCGUGGCAGGCGUGAACUGCUAC UUCCCACUGCAGUCCUACGGCUUUAGGCCCACAUACGGCGUGGGCCACCAGCCCUACAGAGUGGUG GUGCUGAGCUUCGAACUGCUGCAUGCCCCUGCCACAGUGUGCGGCCCUAAGAAAAGCACCAAUCUC GUGAAGAACAAAUGCGUGAACUUCAACUUCAACGGCCUGACCGGCACCGGCGUGCUGACAGAGAGC AACAAGAAGUUCCUGCCAUUCCAGCAGUUUGGCCGGGAUAUCGCCGAUACCACAGACGCCGUUAGA GAUCCCCAGACACUGGAAAUCCUGGACAUCACCCCUUGCAGCUUCGGCGGAGUGUCUGUGAUCACC CCUGGCACCAACACCAGCAAUCAGGUGGCAGUGCUGUACCAGGGCGUGAACUGUACCGAAGUGCCC GUGGCCAUUCACGCCGAUCAGCUGACACCUACAUGGCGGGUGUACUCCACCGGCAGCAAUGUGUUU CAGACCAGAGCCGGCUGUCUGAUCGGAGCCGAGUACGUGAACAAUAGCUACGAGUGCGACAUCCCC AUCGGCGCUGGAAUCUGCGCCAGCUACCAGACACAGACAAAGAGCCACCGGAGAGCCAGAAGCGUG GCCAGCCAGAGCAUCAUUGCCUACACAAUGUCUCUGGGCGCCGAGAACAGCGUGGCCUACUCCAAC AACUCUAUCGCUAUCCCCACCAACUUCACCAUCAGCGUGACCACAGAGAUCCUGCCUGUGUCCAUG ACCAAGACCAGCGUGGACUGCACCAUGUACAUCUGCGGCGAUUCCACCGAGUGCUCCAACCUGCUG CUGCAGUACGGCAGCUUCUGCACCCAGCUGAAAAGAGCCCUGACAGGGAUCGCCGUGGAACAGGAC |
| | | AAGAACACCCAAGAGGUGUUCGCCCAAGUGAAGCAGAUCUACAAGACCCCUCCUAUCAAGUACUUC GGCGGCUUCAAUUUCAGCCAGAUUCUGCCCGAUCCUAGCAAGCCCAGCAAGCGGAGCUUCAUCGAG GACCUGCUGUUCAACAAAGUGACACUGGCCGACGCCGGCUUCAUCAAGCAGUAUGGCGAUUGUCUG GGCGACAUUGCCGCCAGGGAUCUGAUUUGCGCCCAGAAGUUUAACGGACUGACAGUGCUGCCUCCU CUGCUGACCGAUGAGAUGAUCGCCCAGUACACAUCUGCCCUGCUGGCCGGCACAAUCACAAGCGGC UGGACAUUUGGAGCAGGCGCCGCUCUGCAGAUCCCCUUUGCUAUGCAGAUGGCCUACCGGUUCAAC GGCAUCGGAGUGACCCAGAAUGUGCUGUACGAGAACCAGAAGCUGAUCGCCAACCAGUUCAACAGC GCCAUCGGCAAGAUCCAGGACAGCCUGAGCAGCACAGCAAGCGCCCUGGGAAAGCUGCAGGACGUG GUCAACCACAAUGCCCAGGCACUGAACACCCUGGUCAAGCAGCUGUCCUCCAAGUUCGGCGCCAUC AGCUCUGUGCUGAACGAUAUCCUGAGCAGACUGGACCCUCCUGAGGCCGAGGUGCAGAUCGACAGA CUCAUCACAGGCAGACUGCAGAGCCUCCAGACAUACGUGACCCAGCACCUGAUCAGAGCCGCCGAG AUUAGAGCCUCUGCCAAUCUGGCCGCCACCAAGAUGUCUGAGUGUGUGCUGGGCCAGAGCAAGAGA GUGGACUUUUGCGGCAAGGGCUACCACCUGAUGAGCUUCCCUCAGUCUGCCCCUCACGGCGUGGUG UUUCUGCACGUGACAUAUGUGCCCGCUCAAGAGAAGAAUUUCACCACCGCUCCAGCCAUCUGCCAC GACGGCAAAGCCCACUUUCCUAGAGAAGGCGUGUUCGUGUCCAACGGCACCCAUUGGUUCGUGACA CAGCGGAACUUCUACGAGCCCCAGAUCAUCACCACCGACAACACCUUCGUGUCUGGCAACUGCGAC GUCGUGAUCGGCAUUGUGAACAAUACCGUGUACGACCCUCUGCAGCCCGAGCUGGACAGCUUCAAA GAGGAACUGGACAAGUACUUUAAGAACCACACAAGCCCCGACGUGGACCUGGGCGAUAUCAGCGGA AUCAAUGCCAGCGUCGUGAACAUCCAGAAAGAGAUCGACCGGCUGAACGAGGUGGCCAAGAAUCUG AACGAGAGCCUGAUCGACCUGCAAGAACUGGGGAAGUACGAGCAGUACAUCAAGUGGCCCUGGUAC AUCUGGCUGGGCUUUAUCGCCGGACUGAUUGCCAUCGUGAUGGUCACAAUCAUGCUGUGUUGCAUG ACCAGCUGCUGUAGCUGCCUGAAGGGCUGUUGUAGCUGUGGCAGCUGCUGCAAGUUCGACGAGGAC GAUUCUGAGCCCGUGCUGAAGGGCGUGAAACUGCACUACACAUGAUGA |
| 79 | DNA sequence encoding a SARS-CoV-2 S protein from an Omicron BA.4/BA.5 variant | ATGTTCGTGTTCCTGGTGCTGCTGCCTCTGGTGTCCAGCCAGTGTGTGAACCTGATCACCAGAACA CAGTCATACACCAACAGCTTTACCAGAGGCGTGTACTACCCCGACAAGGTGTTCAGATCCAGCGTG CTGCACTCTACCCAGGACCTGTTCCTGCCTTTCTTCAGCAACGTGACCTGGTTCCACGCCATCTCC GGCACCAATGGCACCAAGAGATTCGACAACCCCGTGCTGCCCTTCAACGACGGGGTGTACTTTGCC AGCACCGAGAAGTCCAACATCATCAGAGGCTGGATCTTCGGCACCACACTGGACAGCAAGACCCAG AGCCTGCTGATCGTGAACAACGCCACCAACGTGGTCATCAAAGTGTGCGAGTTCCAGTTCTGCAAC GACCCCTTCCTGGACGTCTACTACCACAAGAACAACAAGAGCTGGATGGAAAGCGAGTTCCGGGTG TACAGCAGCGCCAACAACTGCACCTTCGAGTACGTGTCCCAGCCTTTCCTGATGGACCTGGAAGGC AAGCAGGGCAACTTCAAGAACCTGCGCGAGTTCGTGTTTAAGAACATCGACGGCTACTTCAAGATC TACAGCAAGCACACCCCTATCAACCTCGGCCGGGATCTGCCTCAGGGCTTCTCTGCTCTGGAACCC CTGGTGGATCTGCCCATCGGCATCAACATCACCCGGTTTCAGACACTGCTGGCCCTGCACAGAAGC TACCTGACACCTGGCGATAGCAGCAGCGGATGGACAGCTGGTGCCGCCGCTTACTATGTGGGCTAC CTGCAGCCTAGAACCTTCCTGCTGAAGTACAACGAGAACGGCACCATCACCGACGCCGTGGATTGT GCTCTGGATCCTCTGAGCGAGACAAAGTGCACCCTGAAGTCCTTCACCGTGGAAAAGGGCATCTAC CAGACCAGCAACTTCCGGGTGCAGCCCACCGAATCCATCGTGCGGTTCCCCAATATCACCAATCTG TGCCCCTTCGACGAGGTGTTCAATGCCACCAGATTCGCCTCTGTGTACGCCTGGAACCGGAAGCGG ATCAGCAATTGCGTGGCCGACTACTCCGTGCTGTACAACTTCGCCCCCTTCTTCGCATTCAAGTGC TACGGCGTGTCCCCTACCAAGCTGAACGACCTGTGCTTCACAAACGTGTACGCCGACAGCTTCGTG ATCCGGGGAAACGAAGTGCGGCAGATTGCCCCTGGACAGACAGGCAACATCGCCGACTACAACTAC AAGCTGCCCGACGACTTCACCGGCTGTGTGATTGCCTGGAACAGCAACAAGCTGGACTCCAAAGTC GGCGGCAACTACAATTACAGGTACCGGCTGTTCCGGAAGTCCAATCTGAAGCCCTTCGAGCGGGAC ATCTCCACCGAGATCTATCAGGCCGGCAACAAGCCTTGTAACGGCGTGGCAGGCGTGAACTGCTAC TTCCCACTGCAGTCCTACGGCTTTAGGCCCACATACGGCGTGGGCCACCAGCCCTACAGAGTGGTG GTGCTGAGCTTCGAACTGCTGCATGCCCCTGCCACAGTGTGCGGCCCTAAGAAAAGCACCAATCTC GTGAAGAACAAATGCGTGAACTTCAACTTCAACGGCCTGACCGGCACCGGCGTGCTGACAGAGAGC AACAAGAAGTTCCTGCCATTCCAGCAGTTTGGCCGGGATATCGCCGATACCACAGACGCCGTTAGA |
| | | GATCCCCAGACACTGGAAATCCTGGACATCACCCCTTGCAGCTTCGGCGGAGTGTCTGTGATCACC CCTGGCACCAACACCAGCAATCAGGTGGCAGTGCTGTACCAGGGCGTGAACTGTACCGAAGTGCCC GTGGCCATTCACGCCGATCAGCTGACACCTACATGGCGGGTGTACTCCACCGGCAGCAATGTGTTT CAGACCAGAGCCGGCTGTCTGATCGGAGCCGAGTACGTGAACAATAGCTACGAGTGCGACATCCCC ATCGGCGCTGGAATCTGCGCCAGCTACCAGACACAGACAAAGAGCCACCGGAGAGCCAGAAGCGTG GCCAGCCAGAGCATCATTGCCTACACAATGTCTCTGGGCGCCGAGAACAGCGTGGCCTACTCCAAC AACTCTATCGCTATCCCCACCAACTTCACCATCAGCGTGACCACAGAGATCCTGCCTGTGTCCATG ACCAAGACCAGCGTGGACTGCACCATGTACATCTGCGGCGATTCCACCGAGTGCTCCAACCTGCTG CTGCAGTACGGCAGCTTCTGCACCCAGCTGAAAAGAGCCCTGACAGGGATCGCCGTGGAACAGGAC AAGAACACCCAAGAGGTGTTCGCCCAAGTGAAGCAGATCTACAAGACCCCTCCTATCAAGTACTTC GGCGGCTTCAATTTCAGCCAGATTCTGCCCGATCCTAGCAAGCCCAGCAAGCGGAGCTTCATCGAG GACCTGCTGTTCAACAAAGTGACACTGGCCGACGCCGGCTTCATCAAGCAGTATGGCGATTGTCTG GGCGACATTGCCGCCAGGGATCTGATTTGCGCCCAGAAGTTTAACGGACTGACAGTGCTGCCTCCT CTGCTGACCGATGAGATGATCGCCCAGTACACATCTGCCCTGCTGGCCGGCACAATCACAAGCGGC TGGACATTTGGAGCAGGCGCCGCTCTGCAGATCCCCTTTGCTATGCAGATGGCCTACCGGTTCAAC GGCATCGGAGTGACCCAGAATGTGCTGTACGAGAACCAGAAGCTGATCGCCAACCAGTTCAACAGC GCCATCGGCAAGATCCAGGACAGCCTGAGCAGCACAGCAAGCGCCCTGGGAAAGCTGCAGGACGTG GTCAACCACAATGCCCAGGCACTGAACACCCTGGTCAAGCAGCTGTCCTCCAAGTTCGGCGCCATC AGCTCTGTGCTGAACGATATCCTGAGCAGACTGGACCCTCCTGAGGCCGAGGTGCAGATCGACAGA CTGATCACAGGCAGACTGCAGAGCCTCCAGACATACGTGACCCAGCAGCTGATCAGAGCCGCCGAG ATTAGAGCCTCTGCCAATCTGGCCGCCACCAAGATGTCTGAGTGTGTGCTGGGCCAGAGCAAGAGA GTGGACTTTTGCGGCAAGGGCTACCACCTGATGAGCTTCCCTCAGTCTGCCCCTCACGGCGTGGTG TTTCTGCACGTGACATATGTGCCCGCTCAAGAGAAGAATTTCACCACCGCTCCAGCCATCTGCCAC GACGGCAAAGCCCACTTTCCTAGAGAAGGCGTGTTCGTGTCCAACGGCACCCATTGGTTCGTGACA CAGCGGAACTTCTACGAGCCCCAGATCATCACCACCGACAACACCTTCGTGTCTGGCAACTGCGAC GTCGTGATCGGCATTGTGAACAATACCGTGTACGACCCTCTGCAGCCCGAGCTGGACAGCTTCAAA |
| | | GAGGAACTGGACAAGTACTTTAAGAACCACACAAGCCCCGACGTGGACCTGGGCGATATCAGCGGA ATCAATGCCAGCGTCGTGAACATCCAGAAAGAGATCGACCGGCTGAACGAGGTGGCCAAGAATCTG AACGAGAGCCTGATCGACCTGCAAGAACTGGGGAAGTACGAGCAGTACATCAAGTGGCCCTGGTAC ATCTGGCTGGGCTTTATCGCCGGACTGATTGCCATCGTGATGGTCACAATCATGCTGTGTTGCATG ACCAGCTGCTGTAGCTGCCTGAAGGGCTGTTGTAGCTGTGGCAGCTGCTGCAAGTTCGACGAGGAC GATTCTGAGCCCGTGCTGAAGGGCGTGAAACTGCACTACACATGATGA |

**Table 12. Sequence of one embodiment of an exemplary Omicron BA.2.75-specific RNA vaccine**

| SEQ ID NO. | Brief Description | Sequence |
|---|---|---|
| 80 | Amino acid sequence of RNA-encoded SARS-CoV-2 S protein from an Omicron BA.2.75 variant (with PRO mutations at positions corresponding to positions K986P and V987P of SEQ ID NO: 1; i.e., PRO mutations at positions 983 and 984 of SEQ ID NO: 80) | MFVFLVLLPLVSSQCVNLITRTQSYTNSFTRGVYYPDKVFRSSVLHSTQDLFLPFFSNVTWFHAIH VSGTNGTKRFDNPVLPFNDGVYFASTEKSNIIRGWIFGTTLDSKTQSLLIVNNATNVVIKVCEFQF CNDPFLDVYYHENNKSRMESELRVYSSANNCTFEYVSQPFLMDLEGKQGNFKNLREFVFKNIDGYF KIYSKHTPVNLGRDLPQGFSALEPLVDLPIGINITRFQTLLALHRSYLTPGDSSSSWTAGAAAYYV GYLQPRTFLLKYNENGTITDAVDCALDPLSETKCTLKSFTVEKGIYQTSNFRVQPTESIVRFPNIT NLCPFHEVFNATRFASVYAWNRKRISNCVADYSVLYNFAPFFAFKCYGVSPTKLNDLCFTNVYADS FVIRGNEVSQIAPGQTGNIADYNYKLPDDFTGCVIAWNSNKLDSKVSGNYNYLYRLFRKSKLKPFE RDISTEIYQAGNKPCNGVAGFNCYFPLQSYGFRPTYGVGHQPYRVVVLSFELLHAPATVCGPKKST NLVKNKCVNFNFNGLTGTGVLTESNKKFLPFQQFGRDIADTTDAVRDPQTLEILDITPCSFGGVSV ITPGTNTSNQVAVLYQGVNCTEVPVAIHADQLTPTWRVYSTGSNVFQTRAGCLIGAEYVNNSYECD IPIGAGICASYQTQTKSHRRARSVASQSIIAYTMSLGAENSVAYSNNSIAIPTNFTISVTTEILPV SMTKTSVDCTMYICGDSTECSNLLLQYGSFCTQLKRALTGIAVEQDKNTQEVFAQVKQIYKTPPIK YFGGFNFSQILPDPSKPSKRSFIEDLLFNKVTLADAGFIKQYGDCLGDIAARDLICAQKFNGLTVL PPLLTDEMIAQYTSALLAGTITSGWTFGAGAALQIPFAMQMAYRFNGIGVTQNVLYENQKLIANQF NSAIGKIQDSLSSTASALGKLQDVVNHNAQALNTLVKQLSSKFGAISSVLNDILSRLDPPEAEVQI DRLITGRLQSLQTYVTQQLIRAAEIRASANLAATKMSECVLGQSKRVDFCGKGYHLMSFPQSAPHG VVFLHVTYVPAQEKNFTTAPAICHDGKAHFPREGVFVSNGTHWFVTQRNFYEPQIITTDNTFVSGN CDVVIGIVNNTVYDPLQPELDSFKEELDKYFKNHTSPDVDLGDISGINASVVNIQKEIDRLNEVAK NLNESLIDLQELGKYEQYIKWPWYIWLGFIAGLIAIVMVTIMLCCMTSCCSCLKGCCSCGSCCKFD EDDSEPVLKGVKLHYT |
| 81 | RNA sequence encoding a SARS-CoV-2 S protein from an Omicron BA.2.75 variant | auguucguguuccuggugcugcugccucugguguccagccagugugugaaccugaucaccagaaca cagucauacaccaacagcuuuaccagaggcguguacuaccccgacaagguguucagauccagcgug cugcacucuacccaggaccuguuccugccuuucuucagcaacgugaccugguuccacgccauccac guguccggcaccaauggcaccaagagauucgacaaccccgugcugcccuucaacgacgggguguac uuugccagcaccgagaaguccaacaucaucagaggcuggaucuucggcaccacacuggacagcaag acccagagccugcugaucgugaacaacgccaccaacguggucaucaaagugugcgaguuccaguuc ugcaacgaccccuuccuggacgucuacuaccacgagaacaacaagagcaggauggaaagcgagcuc cggguguacagcagcgccaacaacugcaccuucgaguacguguccagccuuuccugauggaccug gaaggcaagcagggcaacuucaagaaccugcgcgaguucgugguuuaagaacaucgacggcuacuuc aagaucuacagcaagcacaccccugugaaccucggccgggaucugccucagggcuucucugcucug gaaccccugguggaucugcccaucggcaucaacaucacccgguuucagacacugcuggcccugcac agaagcuaccugacaccuggcgauagcagcagcagcuggacagcuggugccgccgcuuacuaugug |
| | | ggcuaccugcagccuagaaccuuccugcugaaguacaacgagaacggcaccaucaccgacgccgug gauugugcucuggauccucugagcgagacaaagugcacccugaaguccuucaccguggaaagggc aucuaccagaccagcaacuuccgggugcagcccaccgaauccaucgugcgguucccaauaucacc aaucugugccccuuccacgagguguucaaugccaccagauucgccucuguguacgccuggaaccgg aagcggaucagcaauugcguggccgacuacuccgugcuguacaacuucgccccuucuucgcauuc aagugcuacggcguguccccuaccaagcugaacgaccugugcuucacaaacguguacgccgacagc uucgugauccggggaaacgaagugucacagauugccccuggacagacaggcaacaucgccgacuac aacuacaagcugcccgacgacuucaccggcugugugauugccuggaacagcaacaagcuggacucc aaagucagcggcaacuacaauuaccuguaccggcuguuccggaaguccaagcugaagcccuucgag cgggacaucuccaccgagaucuaucaggccggcaacaagccuuguaacggcguggcaggcuucaac ugcuacuucccacugcaguccuacggcuuuaggcccacauacggcgugggccaccagcccuacaga gugguggugcugagcuucgaacugcugcaugccccugccacagugugcggcccuaagaaaagcacc aaucucgugaagaacaaaugcgugaacuucaacuucaacggccugaccggcaccggcgugcugaca gagagcaacaagaaguuccugccauuccagcaguuuggccgggauaucgccgauaccacagacgcc guuagagauccccagacacuggaaauccuggacaucaccccuugcagcuucggcggagugucugug aucaccccuggcaccaacaccagcaaucagguggcagugcuguaccagggcgugaacuguaccgaa gugcccguggccauucacgccgaucagcugacaccuacauggcggguguacuccaccggcagcaau guguuucagaccagagccggcugucugaucggagccgaguacgugaacaauagcuacgagugcgac auccccaucggcgcuggaaucugcgccagcuaccagacacagacaaagagccaccggagagccaga agcguggccagccagagcaucauugccuacacaaugucucugggcgccgagaacagcguggccuac uccaacaacucuaucgcuauccccaccaacuucaccaucagcgugaccacagagauccugccugug uccaugaccaagaccagcguggacugcaccauguacaucugcggcgauuccaccgagugcuccaac cugcugcugcaguacggcagcuucugcacccagcugaaaagagcccugacagggaucgccguggaa caggacaagaacacccaagagguguucgcccaagugaagcagaucuacaagaccccuccuaucaag uacuucggcggcuucaauuucagccagauucugcccgauccuagcaagcccagcaagcggagcuuc aucgaggaccugcuguucaacaaagugacacuggccgacgccggcuucaucaagcaguauggcgau ugucugggcgacauugccgccagggaucugauuugcgcccagaaguuuaacggacugacagugcug ccuccucugcugaccgaugagaugaucgcccaguacacaucugcccugcuggccggcacaaucaca agcggcuggacauuuggagcaggcgccgcucugcagauccccuuugcuaugcagauggccuaccgg uucaacggcaucggagugacccagaaugugcuguacgagaaccagaagcugaucgccaaccaguuc aacagcgccaucggcaagauccaggacagccugagcagcacagcaagcgcccugggaaagcugcag gacguggucaaccacaaugcccaggcacugaacacccuggucaagcagcuguccuccaaguucggc gccaucagcucugugcugaacgauauccugagcagacuggacccuccugaggccgaggugcagauc gacagacugaucacaggcagacugcagagccuccagacauacgugacccagcagcugaucagagcc gccgagauuagagccucugccaaucuggccgccaccaagaugucugagugugugcuggccagagc aagagagaguggacuuuugcggcaagggcuaccaccugaugagcuucccucagucugccccucacggc gugguguuucugcacgugacauaugugcccgcucaagagaagaauuucaccaccgcuccagccauc ugccacgacggcaaagcccacuuuccuagagaaggcguguucguguccaacggcacccauugguuc gugacacagcggaacuucuacgagccccagaucaucaccaccgacaacaccuucgugucuggcaac ugcgacgucgugaucggcauugugaacaauaccguguacgacccucugcagcccgagcuggacagc |
| | | uucaaagaggaacuggacaaguacuuuaagaaccacacaagccccgacguggaccuggcgauauc agcggaaucaaugccagcgucgugaacauccagaaagagaucgaccggcugaacgagguggccaag aaucugaacgagagccugaucgaccugcaagaacugggggaaguacgagcaguacaucaaguggccc ugguacaucuggcugggcuuuaucgccggacugauugccaucgugauggucacaaucaugcugugu ugcaugaccagcugcuguagcugccugaagggcuguuguagcuguggcagcugcugcaaguucgac gaggacgauucugagcccgugcugaagggcgugaaacugcacuacacaugauga |
| 82 | DNA sequence encoding a SARS-CoV-2 S protein from an Omicron BA.2.75 variant | atgttcgtgttcctggtgctgctgcctctggtgtccagccagtgtgtgaacctgatcaccagaaca cagtcatacaccaacagctttaccagaggcgtgtactaccccgacaaggtgttcagatccagcgtg ctgcactctacccaggacctgttcctgcctttcttcagcaacgtgacctggttccacgccatccac gtgtccggcaccaatggcaccaagagattcgacaaccccgtgctgcccttcaacgacggggtgtac tttgccagcaccgagaagtccaacatcatcagaggctggatcttcggcaccacactggacagcaag acccagagcctgctgatcgtgaacaacgccaccaacgtggtcatcaaagtgtgcgagttccagttc tgcaacgaccccttcctggacgtctactaccacgagaacaacaagagcaggatggaaagcgagctc cgggtgtacagcagcgccaacaactgcaccttcgagtacgtgtcccagcctttcctgatggacctg gaaggcaagcagggcaacttcaagaacctgcgcgagttcgtgtttaagaacatcgacggctacttc aagatctacagcaagcacacccctgtgaacctcggccgggatctgcctcagggcttctctgctctg gaacccctggtggatctgcccatcggcatcaacatcacccggtttcagacactgctggccctgcac agaagctacctgacacctggcgatagcagcagcagctggacagctggtgccgccgcttactatgtg ggctacctgcagcctagaaccttcctgctgaagtacaacgagaacggcaccatcaccgacgccgtg gattgtgctctggatcctctgagcgagacaaagtgcaccctgaagtccttcaccgtggaaaagggc atctaccagaccagcaacttccgggtgcagcccaccgaatccatcgtgcggttccccaatatcacc aatctgtgccccttccacgaggtgttcaatgccaccagattcgcctctgtgtacgcctggaaccgg aagcggatcagcaattgcgtggccgactactccgtgctgtacaacttcgccccttcttcgcattc aagtgctacggcgtgtcccctaccaagctgaacgacctgtgcttcacaaacgtgtacgccgacagc ttcgtgatccggggaaacgaagtgtcacagattgcccctggacagacaggcaacatcgccgactac aactacaagctgcccgacgacttcaccggctgtgtgattgcctggaacagcaacaagctggactcc aaagtcagcggcaactacaattacctgtaccggctgttccggaagtccaagctgaagcccttcgag cgggacatctccaccgagatctatcaggccggcaacaagccttgtaacggcgtggcaggcttcaac tgctacttcccactgcagtcctacggctttaggcccacatacggcgtgggccaccagccctacaga gtggtggtgctgagcttcgaactgctgcatgcccctgccacagtgtgcggccctaagaaaagcacc aatctcgtgaagaacaaatgcgtgaacttcaacttcaacggcctgaccggcaccggcgtgctgaca gagagcaacaagaagttcctgccattccagcagtttggccgggatatcgccgataccacagacgcc gttagagatccccagacactggaaatcctggacatcaccccttgcagcttcggcggagtgtctgtg atcacccctggcaccaacaccagcaatcaggtggcagtgctgtaccaggcgtgaactgtaccgaa gtgcccgtggccattcacgccgatcagctgacacctacatggcgggtgtactccaccggcagcaat gtgtttcagaccagagccggctgtctgatcggagccgagtacgtgaacaatagctacgagtgcgac atccccatcggcgctggaatctgcgccagctaccagacacagacaaagagccaccggagagccaga agcgtggccagccagagcatcattgcctacacaatgtctctgggcgccgagaacagcgtggcctac tccaacaactctatcgctatccccaccaacttcaccatcagcgtgaccacagagatcctgcctgtg |
| | | tccatgaccaagaccagcgtggactgcaccatgtacatctgcggcgattccaccgagtgctccaac ctgctgctgcagtacggcagcttctgcacccagctgaaaagagccctgacagggatcgccgtggaa caggacaagaacacccaagaggtgttcgcccaagtgaagcagatctacaagacccctcctatcaag tacttcggcggcttcaatttcagccagattctgcccgatcctagcaagcccagcaagcggagcttc atcgaggacctgctgttcaacaaagtgacactggccgacgccggcttcatcaagcagtatggcgat tgtctgggcgacattgccgccagggatctgatttgcgcccagaagtttaacggactgacagtgctg cctcctctgctgaccgatgagatgatcgcccagtacacatctgccctgctggccggcacaatcaca agcggctggacatttggagcaggcgccgctctgcagatcccctttgctatgcagatggcctaccgg ttcaacggcatcggagtgacccagaatgtgctgtacgagaaccagaagctgatcgccaaccagttc aacagcgccatcggcaagatccaggacagcctgagcagcacagcaagcgccctgggaaagctgcag gacgtggtcaaccacaatgcccaggcactgaacaccctggtcaagcagctgtcctccaagttcggc gccatcagctctgtgctgaacgatatcctgagcagactggaccctcctgaggccgaggtgcagatc gacagactgatcacaggcagactgcagagcctccagacatacgtgacccagcagctgatcagagcc gccgagattagagcctctgccaatctggccgccaccaagatgtctgagtgtgtgctgggccagagc aagagagtggacttttgcggcaagggctaccacctgatgagcttccctcagtctgcccctcacggc gtggtgtttctgcacgtgacatatgtgcccgctcaagagaagaatttcaccaccgctccagccatc tgccacgacggcaaagcccactttcctagagaaggcgtgttcgtgtccaacggcacccattggttc gtgacacagcggaacttctacgagccccagatcatcaccaccgacaacaccttcgtgtctggcaac tgcgacgtcgtgatcggcattgtgaacaataccgtgtacgaccctctgcagcccgagctggacagc ttcaaagaggaactggacaagtactttaagaaccacacaagccccgacgtggacctgggcgatatc agcggaatcaatgccagcgtcgtgaacatccagaaagagatcgaccggctgaacgaggtggccaag aatctgaacgagagcctgatcgacctgcaagaactggggaagtacgagcagtacatcaagtggccc tggtacatctggctgggctttatcgccggactgattgccatcgtgatggtcacaatcatgctgtgt tgcatgaccagctgctgtagctgcctgaagggctgttgtagctgtggcagctgctgcaagttcgac gaggacgattctgagcccgtgctgaagggcgtgaaactgcactacacatgatga |
| 83 | Full length RNA construct encoding a SARS-CoV-2 S protein from an Omicron BA.2.75 variant | agaauaaacuaguauucuucuggucccacagacucagagagaacccgccaccauguucguguucc uggugcugcugccucugguguccagccagugugugaaccugaucaccagaacacagucauacacca acagcuuuaccagaggcguguacuaccccgacaagguguucagauccagcgugcugcacucuaccc aggaccuguuccugccuuucuucagcaacgugaccugguuccacgccauccacguguccggcacca auggcaccaagagauucgacaaccccgugcugcccuucaacgacgggguguacuuugccagcaccg agaaguccaacaucaucagaggcuggaucuucggcaccacacuggacagcaagacccagagccugc ugaucgugaacaacgccaccaacguggucaucaaagugugcgaguuccaguucugcaacgaccccu uccuggacgucuacuaccacgagaacaacaagagcaggauggaaagcgagcuccggguguacagca gcgccaacaacugcaccuucgaguacguguccagccuuuccugauggaccuggaaggcaagcagg gcaacuucaagaaccugcgcgaguucguguuuaagaacaucgacggcuacuucaagaucuacagca agcacaccccugugaaccucggccgggaucugccucagggcuucucugcucuggaaccccuggugg aucugcccaucggcaucaacaucacccgguuucagacacugcuggcccugcacagaagcuaccuga caccuggcgauagcagcagcagcuggacagcuggugccgccgcuuacuaugugggcuaccugcagc cuagaaccuuccugcugaaguacaacgagaacggcaccaucaccgacgccguggauugugcucugg |
| | | auccucugagcgagacaaagugcacccugaaguccuucaccguggaaagggcaucuaccagacca gcaacuuccgggugcagcccaccgaauccaucgugcgguuccccaauaucaccaaucugugcccu uccacgagguguucaaugccaccagauucgccucuguguacgccuggaaccggaagcggaucagca auugcguggccgacuacuccgugcuguacaacuucgcccccuucuucgcauucaagugcuacggcg uguccccuaccaagcugaacgaccugugcuucacaaacguguacgccgacagcuucgugauccggg gaaacgaagugucacagauugccccuggacagacaggcaacaucgccgacuacaacuacaagcugc ccgacgacuucaccggcugugugauugccuggaacagcaacaagcuggacuccaaagucagcggca acuacaauuaccuguaccggcuguuccggaaguccaagcugaagcccuucgagcgggacaucucca ccgagaucuaucaggccggcaacaagccuuguaacggcguggcaggcuucaacugcuacuucccac ugcaguccuacggcuuuaggcccacauacggcguggccaccagcccuacagagugguggugcuga gcuucgaacugcugcaugccccugccacagugugcggcccuaagaaaagcaccaaucucgugaaga acaaaugcgugaacuucaacuucaacggccugaccggcaccggcgugcugacagagagcaacaaga aguuccugccauuccagcaguuuggccgggauaucgccgauaccacagacgccguuagagaucccc agacacuggaaauccuggacaucaccccuugcagcuucggcggagugucugugaucaccccuggca ccaacaccagcaaucagguggcagugcuguaccagggcgugaacuguaccgaagugcccguggcca uucacgccgaucagcugacaccuacauggcggguguacuccaccggcagcaauguguuucagacca gagccggcugucugaucggagccgaguacgugaacaauagcuacgagugcgacauccccaucggcg cuggaaucugcgccagcuaccagacacagacaaagagccaccggagagccagaagcguggccagcc agagcaucauugccuacacaaugucucugggcgccgagaacagcguggccuacuccaacaacucua ucgcuauccccaccaacuucaccaucagcgugaccacagagauccugccuguguccaugaccaaga ccagcguggacugcaccauguacaucugcggcgauuccaccgagugcuccaaccugcugcugcagu acggcagcuucugcacccagcugaaaagagcccugacagggaucgccguggaacaggacaagaaca cccaagagguguucgcccaagugaagcagaucuacaagaccccuccuaucaaguacuucggcggcu ucaauuucagccagauucugcccgauccuagcaagcccagcaagcggagcuucaucgaggaccugc uguucaacaaagugacacuggccgacgccggcuucaucaagcaguauggcgauugucugggcgaca uugccgccagggaucugauuugcgcccagaaguuuaacggacugacagugcugccuccucugcuga ccgaugagaugaucgcccaguacacaucugcccugcuggccggcacaaucacaagcggcuggacau uuggagcaggcgccgcucugcagauccccuuugcuaugcagauggccuaccgguucaacggcaucg gagugacccagaaugugcuguacgagaaccagaagcugaucgccaaccaguucaacagcgccaucg gcaagauccaggacagccugagcagcacagcaagcgcccugggaaagcugcaggacguggucaacc acaaugcccaggcacugaacacccuggucaagcagcuguccuccaaguucggcgccaucagcucug ugcugaacgauauccugagcagacuggacccuccugaggccgaggugcagaucgacagacugauca caggcagacugcagagccuccagacauacgugacccagcagcugaucagagccgccgagauuagag ccucugccaaucuggccgccaccaagaugucugagugugugcugggccagagcaagagaguggacu uuugcggcaagggcuaccaccugaugagcuucccucagucugccccucacggcgugguguuucugc acgugacauaugugcccgcucaagagaagaauuucaccaccgcuccagccaucugccacgacggca aagcccacuuuccuagagaaggcguguucguguccaacggcacccauugguucgugacacagcgga acuucuacgagccccagaucaucaccaccgacaacaccuucgugucuggcaacugcgacgucguga ucggcauugugaacaauaccguguacgacccucugcagcccgagcuggacagcuucaaagaggaac uggacaaguacuuuaagaaccacacaagccccgacguggaccugggcgauaucagcggaaucaaug |
| | | ccagcgucgugaacauccagaaagagaucgaccggcugaacgagguggccaagaaucugaacgaga gccugaucgaccugcaagaacuggggaaguacgagcaguacaucaaguggcccugguacaucuggc ugggcuuuaucgccggacugauugccaucgugauggucacaaucaugcuguguugcaugaccagcu gcuguagcugccugaagggcuguuguagcuguggcagcugcugcaaguucgacgaggacgauucug agcccgugcugaagggcgugaaacugcacuacacaugaugacucgagcugguacugcaugcacgca augcuagcugccccuuucccguccuggguaccccgagucucccccgaccucggguccagguaugc ucccaccuccaccugccccacucaccaccucugcuaguuccagacaccucccaagcacgcagcaau gcagcucaaaacgcuuagccuagccacacccccacgggaaacagcagugauuaaccuuuagcaaua aacgaaaguuuaacuaagcuauacuaaccccaggguuggucaauuucgugccagccacacccugga gcuagcaaaaaaaaaaaaaaaaaaaaaaaaaaaaaagcauaugacuaaaaaaaaaaaaaaaaaaaaa aaaaaaaaaaaaaaaaaaaaaaaaaaaaaaaaaaaaaaaaaaaaaa |
| 84 | Full length DNA construct encoding a SARS-CoV-2 S protein from an Omicron BA.2.75 variant | agaataaactagtattcttctggtccccacagactcagagagaacccgccaccatgttcgtgttcc tggtgctgctgcctctggtgtccagccagtgtgtgaacctgatcaccagaacacagtcatacacca acagctttaccagaggcgtgtactaccccgacaaggtgttcagatccagcgtgctgcactctaccc aggacctgttcctgcctttcttcagcaacgtgacctggttccacgccatccacgtgtccggcacca atggcaccaagagattcgacaaccccgtgctgcccttcaacgacggggtgtacttgccagcaccg agaagtccaacatcatcagaggctggatcttcggcaccacactggacagcaagacccagagcctgc tgatcgtgaacaacgccaccaacgtggtcatcaaagtgtgcgagttccagttctgcaacgacccct tcctggacgtctactaccacgagaacaacaagagcaggatggaaagcgagctccgggtgtacagca gcgccaacaactgcaccttcgagtacgtgtcccagcctttcctgatggacctggaaggcaagcagg gcaacttcaagaacctgcgcgagttcgtgtttaagaacatcgacggctacttcaagatctacagca agcacacccctgtgaacctcggccgggatctgcctcagggcttctctgctctggaacccctggtgg atctgcccatcggcatcaacatcacccggtttcagacactgctggccctgcacagaagctacctga cacctggcgatagcagcagcagctggacagctggtgccgccgcttactatgtgggctacctgcagc ctagaaccttcctgctgaagtacaacgagaacggcaccatcaccgacgccgtggattgtgctctgg atcctctgagcgagacaaagtgcaccctgaagtccttcaccgtggaaaagggcatctaccagacca gcaacttccgggtgcagcccaccgaatccatcgtgcggttccccaatatcaccaatctgtgcccct tccacgaggtgttcaatgccaccagattcgcctctgtgtacgcctggaaccggaagcggatcagca attgcgtggccgactactccgtgctgtacaacttcgcccccttcttcgcattcaagtgctacggcg tgtcccctaccaagctgaacgacctgtgcttcacaaacgtgtacgccgacagcttcgtgatccggg gaaacgaagtgtcacagattgcccctggacagacaggcaacatcgccgactacaactacaagctgc ccgacgacttcaccggctgtgtgattgcctggaacagcaacaagctggactccaaagtcagcggca actacaattacctgtaccggctgttccggaagtccaagctgaagcccttcgagcgggacatctcca ccgagatctatcaggccggcaacaagccttgtaacggcgtggcaggcttcaactgctacttcccac tgcagtcctacggctttaggcccacatacggcgtgggccaccagccctacagagtggtggtgctga gcttcgaactgctgcatgcccctgccacagtgtgcggccctaagaaaagcaccaatctcgtgaaga acaaatgcgtgaacttcaacttcaacggcctgaccggcaccggcgtgctgacagagagcaacaaga agttcctgccattccagcagtttggccgggatatcgccgataccacagacgccgttagagatcccc agacactggaaatcctggacatcaccccttgcagcttcggcggagtgtctgtgatcacccctggca |
| | | ccaacaccagcaatcaggtggcagtgctgtaccagggcgtgaactgtaccgaagtgcccgtggcca ttcacgccgatcagctgacacctacatggcgggtgtactccaccggcagcaatgtgtttcagacca gagccggctgtctgatcggagccgagtacgtgaacaatagctacgagtgcgacatccccatcggcg ctggaatctgcgccagctaccagacacagacaaagagccaccggagagccagaagcgtggccagcc agagcatcattgcctacacaatgtctctgggcgccgagaacagcgtggcctactccaacaactcta tcgctatccccaccaacttcaccatcagcgtgaccacagagatcctgcctgtgtccatgaccaaga ccagcgtggactgcaccatgtacatctgcggcgattccaccgagtgctccaacctgctgctgcagt acggcagcttctgcacccagctgaaaagagccctgacagggatcgccgtggaacaggacaagaaca cccaagaggtgttcgcccaagtgaagcagatctacaagacccctcctatcaagtacttcggcggct tcaatttcagccagattctgcccgatcctagcaagcccagcaagcggagcttcatcgaggacctgc tgttcaacaaagtgacactggccgacgccggcttcatcaagcagtatggcgattgtctgggcgaca ttgccgccagggatctgatttgcgcccagaagtttaacggactgacagtgctgcctcctctgctga ccgatgagatgatcgcccagtacacatctgccctgctggccggcacaatcacaagcggctggacat ttggagcaggcgccgctctgcagatccccttttgctatgcagatggcctaccggttcaacggcatcg gagtgacccagaatgtgctgtacgagaaccagaagctgatcgccaaccagttcaacagcgccatcg gcaagatccaggacagcctgagcagcacagcaagcgccctgggaaagctgcaggacgtggtcaacc acaatgcccaggcactgaacaccctggtcaagcagctgtcctccaagttcggcgccatcagctctg tgctgaacgatatcctgagcagactggaccctcctgaggccgaggtgcagatcgacagactgatca caggcagactgcagagcctccagacatacgtgacccagcagctgatcagagccgccgagattagag cctctgccaatctggccgccaccaagatgtctgagtgtgtgctgggccagagcaagagagtggact tttgcggcaagggctaccacctgatgagcttccctcagtctgcccctcacggcgtggtgtttctgc acgtgacatatgtgcccgctcaagagaagaatttcaccaccgctccagccatctgccacgacggca aagcccactttcctagagaaggcgtgttcgtgtccaacggcacccattggttcgtgacacagcgga acttctacgagccccagatcatcaccaccgacaacaccttcgtgtctggcaactgcgacgtcgtga tcggcattgtgaacaataccgtgtacgaccctctgcagcccgagctggacagcttcaaagaggaac tggacaagtactttaagaaccacacaagccccgacgtggacctgggcgatatcagcggaatcaatg ccagcgtcgtgaacatccagaaagagatcgaccggctgaacgaggtggccaagaatctgaacgaga gcctgatcgacctgcaagaactggggaagtacgagcagtacatcaagtggccctggtacatctggc tgggctttatcgccggactgattgccatcgtgatggtcacaatcatgctgtgttgcatgaccagct gctgtagctgcctgaagggctgttgtagctgtggcagctgctgcaagttcgacgaggacgattctg agcccgtgctgaagggcgtgaaactgcactacacatgatgactcgagctggtactgcatgcacgca atgctagctgcccctttcccgtcctgggtaccccgagtctcccccgacctcgggtcccaggtatgc tcccacctccacctgccccactcaccacctctgctagttccagacacctcccaagcacgcagcaat gcagctcaaaacgcttagcctagccacacccccacgggaaacagcagtgattaacctttagcaata aacgaaagtttaactaagctatactaaccccagggttggtcaattcgtgccagccacaccctgga gctagcaaaaaaaaaaaaaaaaaaaaaaaaaaaaaagcatatgactaaaaaaaaaaaaaaaaaaaa aaaaaaaaaaaaaaaaaaaaaaaaaaaaaaaaaaaaaaaaaaaaaa |

**Table 13. Sequence of one embodiment of an exemplary Omicron BA.2.75.2-specific RNA vaccine**

| SEQ ID NO. | Brief Description | Sequence |
|---|---|---|
| | | |
| 85 | Amino acid sequence of RNA-encoded SARS-CoV-2 S protein from an Omicron BA.2.75.2 variant (with PRO mutations at positions corresponding to K986P and V987P of SEQ ID NO: 1; i.e., PRO mutations at positions 983 and 984 of SEQ ID NO: 85) | MFVFLVLLPLVSSQCVNLITRTQSYTNSFTRGVYYPDKVFRSSVLHSTQDLFLPFFSNVTWFHAIH VSGTNGTKRFDNPVLPFNDGVYFASTEKSNIIRGWIFGTTLDSKTQSLLIVNNATNVVIKVCEFQF CNDPFLDVYYHENNKSRMESELRVYSSANNCTFEYVSQPFLMDLEGKQGNFKNLREFVFKNIDGYF KIYSKHTPVNLGRDLPQGFSALEPLVDLPIGINITRFQTLLALHRSYLTPGDSSSSWTAGAAAYYV GYLQPRTFLLKYNENGTITDAVDCALDPLSETKCTLKSFTVEKGIYQTSNFRVQPTESIVRFPNIT NLCPFHEVFNATTFASVYAWNRKRISNCVADYSVLYNFAPFFAFKCYGVSPTKLNDLCFTNVYADS FVIRGNEVSQIAPGQTGNIADYNYKLPDDFTGCVIAWNSNKLDSKVSGNYNYLYRLFRKSKLKPFE RDISTEIYQAGNKPCNGVAGSNCYFPLQSYGFRPTYGVGHQPYRVVVLSFELLHAPATVCGPKKST NLVKNKCVNFNFNGLTGTGVLTESNKKFLPFQQFGRDIADTTDAVRDPQTLEILDITPCSFGGVSV ITPGTNTSNQVAVLYQGVNCTEVPVAIHADQLTPTWRVYSTGSNVFQTRAGCLIGAEYVNNSYECD IPIGAGICASYQTQTKSHRRARSVASQSIIAYTMSLGAENSVAYSNNSIAIPTNFTISVTTEILPV SMTKTSVDCTMYICGDSTECSNLLLQYGSFCTQLKRALTGIAVEQDKNTQEVFAQVKQIYKTPPIK YFGGFNFSQILPDPSKPSKRSFIEDLLFNKVTLADAGFIKQYGDCLGDIAARDLICAQKFNGLTVL PPLLTDEMIAQYTSALLAGTITSGWTFGAGAALQIPFAMQMAYRFNGIGVTQNVLYENQKLIANQF NSAIGKIQDSLSSTASALGKLQDVVNHNAQALNTLVKQLSSKFGAISSVLNDILSRLDPPEAEVQI DRLITGRLQSLQTYVTQQLIRAAEIRASANLAATKMSECVLGQSKRVDFCGKGYHLMSFPQSAPHG VVFLHVTYVPAQEKNFTTAPAICHDGKAHFPREGVFVSNGTHWFVTQRNFYEPQIITTDNTFVSGN CDVVIGIVNNTVYDPLQPELDSFKEELDKYFKNHTSPDVDLGDISGINASVVNIQKEIDRLNEVAK NLNESLINLQELGKYEQYIKWPWYIWLGFIAGLIAIVMVTIMLCCMTSCCSCLKGCCSCGSCCKFD EDDSEPVLKGVKLHYT |
| 86 | RNA sequence encoding a SARS-CoV-2 S protein from an Omicron BA.2.75.2 variant | auguucguguuccuggugcugcugccucugguguccagccagugugugaaccugaucaccagaaca cagucauacaccaacagcuuuaccagaggcguguacuaccccgacaagguguucagauccagcgug cugcacucuacccaggaccuguuccugccuuucuucagcaacgugaccugguuccacgccauccac guguccggcaccaauggcaccaagagauucgacaaccccgugcugcccuucaacgacgggguguac uuugccagcaccgagaaguccaacaucaucagaggcuggaucuucggcaccacacuggacagcaag acccagagccugcugaucgugaacaacgccaccaacguggucaucaaagugugcgaguuccaguuc ugcaacgaccccuuccuggacgucuacuaccacgagaacaacaagagcaggauggaaagcgagcuc cggguguacagcagcgccaacaacugcaccuucgaguacguguccagccuuuccugauggaccug gaaggcaagcagggcaacuucaagaaccugcgcgaguucguguuuaagaacaucgacggcuacuuc aagaucuacagcaagcacaccccugugaaccucggccgggaucugccucagggcuucucugcucug gaaccccugguggaucugcccaucggcaucaacaucacccgguuucagacacugcuggcccugcac agaagcuaccugacaccuggcgauagcagcagcagcuggacagcuggugccgccgcuuacuaugug ggcuaccugcagccuagaaccuuccugcugaaguacaacgagaacggcaccaucaccgacgccgug gauugugcucuggaucccucugagcgagacaaagugcacccugaaguccuucaccguggaaaagggc aucuaccagaccagcaacuuccgggugcagcccaccgaauccaucgugcgguuccccaauaucacc aaucugugccccuuccacgagguguucaaugccaccaccuucgccucuguguacgccuggaaccgg |
| | | aagcggaucagcaauugcguggccgacuacuccgugcuguacaacuucgccccuucuucgcauuc aagugcuacggcguguccccuaccaagcugaacgaccugugcuucacaaacguguacgccgacagc uucgugauccggggaaacgaagugucacagauugccccuggacagacaggcaacaucgccgacuac aacuacaagcugcccgacgacuucaccggcugugugauugccuggaacagcaacaagcuggacucc aaagucagcggcaacuacaauuaccuguaccggcuguuccggaaguccaagcugaagcccuucgag cgggacaucuccaccgagaucuaucaggccggcaacaagccuuguaacggcguggcaggcagcaac ugcuacuucccacugcaguccuacggcuuuaggcccacauacggcgugggccaccagcccuacaga gugguggugcugagcuucgaacugcugcaugccccugccacagugugcggcccuaagaaaagcacc aaucucgugaagaacaaaugcgugaacuucaacuucaacggccugaccggcaccggcgugcugaca gagagcaacaagaaguuccugccauuccagcaguuuggccgggauaucgccgauaccacagacgcc guuagagauccccagacacuggaaauccuggacaucaccccuugcagcuucggcggagugucugug aucaccccuggcaccaacaccagcaaucagguggcagugcuguaccagggcgugaacuguaccgaa gugcccguggccauucacgccgaucagcugacaccuacauggcggguguacuccaccggcagcaau guguuucagaccagagccggcugucugaucggagccgaguacgugaacaauagcuacgagugcgac auccccaucggcgcuggaaucugcgccagcuaccagacacagacaaagagccaccggagagccaga agcguggccagccagagcaucauugccuacacaaugucucugggcgccgagaacagcguggccuac uccaacaacucuaucgcuauccccaccaacuucaccaucagcgugaccacagagauccugccugug uccaugaccaagaccagcguggacugcaccauguacaucugcggcgauuccaccgagugcuccaac cugcugcugcaguacggcagcuucugcacccagcugaaaagagcccugacagggaucgccguggaa caggacaagaacacccaagagguguucgcccaagugaagcagaucuacaagaccccuccuaucaag uacuucggcggcuucaauuucagccagauucugcccgauccuagcaagcccagcaagcggagcuuc aucgaggaccugcuguucaacaaagugacacuggccgacgccggcuucaucaagcaguauggcgau ugucugggcgacauugccgccagggaucugauuugcgcccagaaguuuaacggacugacagugcug ccuccucugcugaccgaugagaugaucgcccaguacacaucugcccugcuggccggcacaaucaca agcggcuggacauuuggagcaggcgccgcucugcagauccccuuugcuaugcagauggccuaccgg uucaacggcaucggagugacccagaaugugcuguacgagaaccagaagcugaucgccaaccaguuc aacagcgccaucggcaagauccaggacagccugagcagcacagcaagcgcccuggaaagcugcag gacguggucaaccacaaugcccaggcacugaacacccuggucaagcagcuguccuccaaguucggc gccaucagcucugugcugaacgauauccugagcagacuggacccuccugaggccgaggugcagauc gacagacugaucacaggcagacugcagagccuccagacauacgugacccagcagcugaucagagcc gccgagauuagagccucugccaaucuggccgccaccaagaugucugagugugugcuggccagagc aagagaguggacuuuugcggcaagggcuaccaccugaugagcuucccucagucugccccucacggc gugguguuucugcacgugacauaugugcccgcucaagagaagaauuucaccaccgcuccagccauc ugccacgacggcaaagcccacuuuccuagagaaggcguguucguguccaacggcacccauugguuc gugacacagcggaacuucuacgagccccagaucaucaccaccgacaacaccuucgugucuggcaac ugcgacgucgugaucggcauugugaacaauaccguguacgacccucugcagcccgagcuggacagc uucaaagaggaacuggacaaguacuuuaagaaccacacaagccccgacguggaccugggcgauauc agcggaaucaaugccagcgucgugaacauccagaaagagaucgaccggcugaacgagguggccaag aaucugaacgagagccugaucaaccugcaagaacugggggaaguacgagcaguacaucaaguggccc ugguacaucuggcugggcuuuaucgccggacugauugccaucgugauggucacaaucaugcugugu |
| | | ugcaugaccagcugcuguagcugccugaagggcuguuguagcuguggcagcugcugcaaguucgac gaggacgauucugagcccgugcugaagggcgugaaacugcacuacacaugauga |
| 87 | DNA sequence encoding a SARS-CoV-2 S protein from an Omicron BA.2.75.2 variant | atgttcgtgttcctggtgctgctgcctctggtgtccagccagtgtgtgaacctgatcaccagaaca cagtcatacaccaacagctttaccagaggcgtgtactaccccgacaaggtgttcagatccagcgtg ctgcactctacccaggacctgttcctgcctttcttcagcaacgtgacctggttccacgccatccac gtgtccggcaccaatggcaccaagagattcgacaaccccgtgctgcccttcaacgacggggtgtac tttgccagcaccgagaagtccaacatcatcagaggctggatcttcggcaccacactggacagcaag acccagagcctgctgatcgtgaacaacgccaccaacgtggtcatcaaagtgtgcgagttccagttc tgcaacgaccccttcctggacgtctactaccacgagaacaacaagagcaggatggaaagcgagctc cgggtgtacagcagcgccaacaactgcaccttcgagtacgtgtcccagcctttcctgatggacctg gaaggcaagcagggcaacttcaagaacctgcgcgagttcgtgtttaagaacatcgacggctacttc aagatctacagcaagcacacccctgtgaacctcggccgggatctgcctcagggcttctctgctctg gaacccctggtggatctgcccatcggcatcaacatcacccggtttcagacactgctggccctgcac agaagctacctgacacctggcgatagcagcagcagctggacagctggtgccgccgcttactatgtg ggctacctgcagcctagaaccttcctgctgaagtacaacgagaacggcaccatcaccgacgccgtg gattgtgctctggatcctctgagcgagacaaagtgcaccctgaagtccttcaccgtggaaaagggc atctaccagaccagcaacttccgggtgcagcccaccgaatccatcgtgcggttccccaatatcacc aatctgtgccccttccacgaggtgttcaatgccaccaccttcgcctctgtgtacgcctggaaccgg aagcggatcagcaattgcgtggccgactactccgtgctgtacaacttcgcccccttcttcgcattc aagtgctacggcgtgtcccctaccaagctgaacgacctgtgcttcacaaacgtgtacgccgacagc ttcgtgatccggggaaacgaagtgtcacagattgcccctggacagacaggcaacatcgccgactac aactacaagctgcccgacgacttcaccggctgtgtgattgcctggaacagcaacaagctggactcc aaagtcagcggcaactacaattacctgtaccggctgttccggaagtccaagctgaagcccttcgag cgggacatctccaccgagatctatcaggccggcaacaagccttgtaacggcgtggcaggcagcaac tgctacttcccactgcagtcctacggctttaggcccacatacggcgtgggccaccagccctacaga gtggtggtgctgagcttcgaactgctgcatgcccctgccacagtgtgcggccctaagaaaagcacc aatctcgtgaagaacaaatgcgtgaacttcaacttcaacggcctgaccggcaccggcgtgctgaca gagagcaacaagaagttcctgccattccagcagtttggccgggatatcgccgataccacagacgcc gttagagatccccagacactggaaatcctggacatcaccccttgcagcttcggcggagtgtctgtg atcacccctggcaccaacaccagcaatcaggtggcagtgctgtaccaggcgtgaactgtaccgaa gtgcccgtggccattcacgccgatcagctgacacctacatggcgggtgtactccaccggcagcaat gtgtttcagaccagagccggctgtctgatcggagccgagtacgtgaacaatagctacgagtgcgac atccccatcggcgctggaatctgcgccagctaccagacacagacaaagagccaccggagagccaga agcgtggccagccagagcatcattgcctacacaatgtctctgggcgccgagaacagcgtggcctac tccaacaactctatcgctatccccaccaacttcaccatcagcgtgaccacagagatcctgcctgtg tccatgaccaagaccagcgtggactgcaccatgtacatctgcggcgattccaccgagtgctccaac ctgctgctgcagtacggcagcttctgcacccagctgaaaagagccctgacagggatcgccgtggaa caggacaagaacacccaagaggtgttcgcccaagtgaagcagatctacaagacccctcctatcaag tacttcggcggcttcaatttcagccagattctgcccgatcctagcaagcccagcaagcggagcttc |
| | | atcgaggacctgctgttcaacaaagtgacactggccgacgccggcttcatcaagcagtatggcgat tgtctgggcgacattgccgccagggatctgatttgcgcccagaagtttaacggactgacagtgctg cctcctctgctgaccgatgagatgatcgcccagtacacatctgccctgctggccggcacaatcaca agcggctggacatttggagcaggcgccgctctgcagatcccctttgctatgcagatggcctaccgg ttcaacggcatcggagtgacccagaatgtgctgtacgagaaccagaagctgatcgccaaccagttc aacagcgccatcggcaagatccaggacagcctgagcagcacagcaagcgccctgggaaagctgcag gacgtggtcaaccacaatgcccaggcactgaacaccctggtcaagcagctgtcctccaagttcggc gccatcagctctgtgctgaacgatatcctgagcagactggaccctcctgaggccgaggtgcagatc gacagactgatcacaggcagactgcagagcctccagacatacgtgacccagcagctgatcagagcc gccgagattagagcctctgccaatctggccgccaccaagatgtctgagtgtgtgctgggccagagc aagagagtggactttgcggcaagggctaccacctgatgagcttccctcagtctgcccctcacggc gtggtgtttctgcacgtgacatatgtgcccgctcaagagaagaatttcaccaccgctccagccatc tgccacgacggcaaagcccactttcctagagaaggcgtgttcgtgtccaacggcacccattggttc gtgacacagcggaacttctacgagccccagatcatcaccaccgacaacaccttcgtgtctggcaac tgcgacgtcgtgatcggcattgtgaacaataccgtgtacgaccctctgcagcccgagctggacagc ttcaaagaggaactggacaagtactttaagaaccacacaagccccgacgtggacctgggcgatatc agcggaatcaatgccagcgtcgtgaacatccagaaagagatcgaccggctgaacgaggtggccaag aatctgaacgagagcctgatcaacctgcaagaactggggaagtacgagcagtacatcaagtggccc tggtacatctggctgggctttatcgccggactgattgccatcgtgatggtcacaatcatgctgtgt tgcatgaccagctgctgtagctgcctgaagggctgttgtagctgtggcagctgctgcaagttcgac gaggacgattctgagcccgtgctgaagggcgtgaaactgcactacacatgatga |
| 88 | Full length RNA construct encoding a SARS-CoV-2 S protein from an Omicron BA.2.75.2 variant | agaauaaacuaguauucuucuggucccacagacucagagagaacccgccaccauguucguguucc uggugcugcugccucugguguccagccagugugugaaccugaucaccagaacacagucauacacca acagcuuuaccagaggcguguacuaccccgacaagguguucagauccagcgugcugcacucuaccc aggaccuguuccugccuuucuucagcaacgugaccugguuccacgccauccacguguccggcacca auggcaccaagagauucgacaaccccgugcugcccuucaacgacgggguacuuugccagcaccg agaaguccaacaucaucagaggcuggaucuucggcaccacacuggacagcaagacccagagccugc ugaucgugaacaacgccaccaacguggucaucaaagugugcgaguuccaguucugcaacgaccccu uccuggacgucuacuaccacgagaacaacaagagcaggauggaaagcgagcuccggguguacagca gcgccaacaacugcaccuucgaguacgugucccagccuuuccugauggaccuggaaggcaagcagg gcaacuucaagaaccugcgcgaguucguguuuaagaacaucgacggcuacuucaagaucuacagca agcacaccccugugaaccucggccgggaucugccucagggcuucucugcucuggaaccccuggugg aucugcccaucggcaucaacaucacccgguuucagacacugcuggcccugcacagaagcuaccuga caccuggcgauagcagcagcagcuggacagcuggugccgccgcuuacuaugugggcuaccugcagc cuagaaccuuccugcugaaguacaacgagaacggcaccaucaccgacgccguggauugugcucugg auccucugagcgagacaaagugcacccugaaguccuucaccguggaaaagggcaucuaccagacca gcaacuuccgggugcagcccaccgaauccaucgugcgguuccccaauaucaccaaucugugccccu uccacgagguguucaaugccaccaccuucgccucuguguacgccuggaaccggaagcggaucagca auugcguggccgacuacuccgugcuguacaacuucgccccuucuucgcauucaagugcuacggcg |
| | | ugucccuaccaagcugaacgaccugugcuucacaaacguguacgccgacagcuucgugauccggg gaaacgaagugucacagauugccccuggacagacaggcaacaucgccgacuacaacuacaagcugc ccgacgacuucaccggcugugugauugccuggaacagcaacaagcuggacuccaaagucagcggca acuacaauuaccuguaccggcuguuccggaaguccaagcugaagcccuucgagcgggacaucucca ccgagaucuaucaggccggcaacaagccuuguaacggcguggcaggcagcaacugcuacuucccac ugcaguccuacggcuuuaggcccacauacggcgugggccaccagcccuacagagugguggugcuga gcuucgaacugcugcaugccccugccacagugugcggcccuaagaaaagcaccaaucucgugaaga acaaaugcgugaacuucaacuucaacggccugaccggcaccggcgugcugacagagagcaacaaga aguuccugccauuccagcaguuuggccgggauaucgccgauaccacagacgccguuagagaucccc agacacuggaaauccuggacaucaccccuugcagcuucggcggagugucugugaucaccccuggca ccaacaccagcaaucagguggcagugcuguaccagggcgugaacuguaccgaagugcccguggcca uucacgccgaucagcugacaccuacauggcggguguacuccaccggcagcaauguguuucagacca gagccggcugucugaucggagccgaguacgugaacaauagcuacgagugcgacauccccaucggcg cuggaaucugcgccagcuaccagacacagacaaagagccaccggagagccagaagcguggccagcc agagcaucauugccuacacaaugucucugggcgccgagaacagcguggccuacuccaacaacucua ucgcuauccccaccaacuucaccaucagcgugaccacagagauccugccuguguccaugaccaaga ccagcguggacugcaccauguacaucugcggcgauuccaccgagugcuccaaccugcugcugcagu acggcagcuucugcacccagcugaaaagagcccugacagggaucgccguggaacaggacaagaaca cccaagagguguucgcccaagugaagcagaucuacaagaccccuccuaucaaguacuucggcggcu ucaauuucagccagauucugcccgauccuagcaagcccagcaagcggagcuucaucgaggaccugc uguucaacaaagugacacuggccgacgccggcuucaucaagcaguauggcgauugucugggcgaca uugccgccagggaucugauuugcgcccagaaguuuaacggacugacagugcugccuccucugcuga ccgaugagaugaucgcccaguacacaucugcccugcuggccggcacaaucacaagcggcuggacau uuggagcaggcgccgcucugcagauccccuuugcuaugcagauggccuaccgguucaacggcaucg gagugacccagaaugugcuguacgagaaccagaagcugaucgccaaccaguucaacagcgccaucg gcaagauccaggacagccugagcagcacagcaagcgcccugggaaagcugcaggacguggucaacc acaaugcccaggcacugaacacccuggucaagcagcuguccuccaaguucggcgccaucagcucug ugcugaacgauauccugagcagacuggacccuccugaggccgaggugcagaucgacagacugauca caggcagacugcagagccuccagacauacgugacccagcagcugaucagagccgccgagauuagag ccucugccaaucuggccgccaccaagaugucugagugugugcugggccagagcaagagaguggacu uuugcggcaagggcuaccaccugaugagcuucccucagucugccccucacggcgugguguuucugc acgugacauaugugcccgcucaagagaagaauuucaccaccgcuccagccaucugccacgacggca aagcccacuuuccuagagaaggcguguucguguccaacggcacccauugguucgugacacagcgga acuucuacgagccccagaucaucaccaccgacaacaccuucgugucuggcaacugcgacgucguga ucggcauugugaacaauaccguguacgacccucugcagcccgagcuggacagcuucaaagaggaac uggacaaguacuuuaagaaccacacaagccccgacguggaccugggcgauaucagcggaaucaaug ccagcgucgugaacauccagaaagagaucgaccggcugaacgagguggccaagaaucugaacgaga gccugaucaaccugcaagaacuggggaaguacgagcaguacaucaaguggcccugguacaucuggc ugggcuuuaucgccggacugauugccaucgugauggucacaaucaugcuguguugcaugaccagcu gcuguagcugccugaagggcuguuguagcuguggcagcugcugcaaguucgacgaggacgauucug |
| | | agcccgugcugaagggcgugaaacugcacuacacaugaugacucgagcugguacugcaugcacgca augcuagcugccccuuucccguccuggguaccccgagucucccccgaccucgggucccagguaugc ucccaccuccaccugccccacucaccaccucugcuaguuccagacaccucccaagcacgcagcaau gcagcucaaaacgcuuagccuagccacacccccacgggaaacagcagugauuaaccuuuagcaaua aacgaaaguuuaacuaagcuauacuaaccccaggguuggucaauuucgugccagccacacccugga gcuagcaaaaaaaaaaaaaaaaaaaaaaaaaaaaaaagcauaugacuaaaaaaaaaaaaaaaaaaaa aaaaaaaaaaaaaaaaaaaaaaaaaaaaaaaaaaaaaaaaaaaaaaaaa |
| 89 | Full length DNA construct encoding a SARS-CoV-2 S protein from an Omicron BA.2.75.2 variant | agaataaactagtattcttctggtccccacagactcagagagaacccgccaccatgttcgtgttcc tggtgctgctgcctctggtgtccagccagtgtgtgaacctgatcaccagaacacagtcatacacca acagctttaccagaggcgtgtactaccccgacaaggtgttcagatccagcgtgctgcactctaccc aggacctgttcctgcctttcttcagcaacgtgacctggttccacgccatccacgtgtccggcacca atggcaccaagagattcgacaaccccgtgctgcccttcaacgacggggtgtactttgccagcaccg agaagtccaacatcatcagaggctggatcttcggcaccacactggacagcaagacccagagcctgc tgatcgtgaacaacgccaccaacgtggtcatcaaagtgtgcgagttccagttctgcaacgacccct tcctggacgtctactaccacgagaacaacaagagcaggatggaaagcgagctccgggtgtacagca gcgccaacaactgcaccttcgagtacgtgtcccagcctttcctgatggacctggaaggcaagcagg gcaacttcaagaacctgcgcgagttcgtgtttaagaacatcgacggctacttcaagatctacagca agcacacccctgtgaacctcggccgggatctgcctcagggcttctctgctctggaacccctggtgg atctgcccatcggcatcaacatcacccggtttcagacactgctggccctgcacagaagctacctga cacctggcgatagcagcagcagctggacagctggtgccgccgcttactatgtgggctacctgcagc ctagaaccttcctgctgaagtacaacgagaacggcaccatcaccgacgccgtggattgtgctctgg atcctctgagcgagacaaagtgcaccctgaagtccttcaccgtggaaaagggcatctaccagacca gcaacttccgggtgcagcccaccgaatccatcgtgcggttccccaatatcaccaatctgtgcccct tccacgaggtgttcaatgccaccaccttcgcctctgtgtacgcctggaaccggaagcggatcagca attgcgtggccgactactccgtgctgtacaacttcgccccctttcttcgcattcaagtgctacggcg tgtcccctaccaagctgaacgacctgtgcttcacaaacgtgtacgccgacagcttcgtgatccggg gaaacgaagtgtcacagattgcccctggacagacaggcaacatcgccgactacaactacaagctgc ccgacgacttcaccggctgtgtgattgcctggaacagcaacaagctggactccaaagtcagcggca actacaattacctgtaccggctgttccggaagtccaagctgaagcccttcgagcgggacatctcca ccgagatctatcaggccggcaacaagccttgtaacggcgtggcaggcagcaactgctacttcccac tgcagtcctacggctttaggcccacatacggcgtgggccaccagccctacagagtggtggtgctga gcttcgaactgctgcatgcccctgccacagtgtgcggccctaagaaaagcaccaatctcgtgaaga acaaatgcgtgaacttcaacttcaacggcctgaccggcaccggcgtgctgacagagagcaacaaga agttcctgccattccagcagtttggccgggatatcgccgataccacagacgccgttagagatcccc agacactggaaatcctggacatcaccccttgcagcttcggcggagtgtctgtgatcacccctggca ccaacaccagcaatcaggtggcagtgctgtaccaggcgtgaactgtaccgaagtgcccgtggcca ttcacgccgatcagctgacacctacatggcgggtgtactccaccggcagcaatgtgtttcagacca gagccggctgtctgatcggagccgagtacgtgaacaatagctacgagtgcgacatccccatcggcg ctggaatctgcgccagctaccagacacagacaaagagccaccggagagccagaagcgtggccagcc |
| | | agagcatcattgcctacacaatgtctctgggcgccgagaacagcgtggcctactccaacaactcta tcgctatccccaccaacttcaccatcagcgtgaccacagagatcctgcctgtgtccatgaccaaga ccagcgtggactgcaccatgtacatctgcggcgattccaccgagtgctccaacctgctgctgcagt acggcagcttctgcacccagctgaaaagagccctgacagggatcgccgtggaacaggacaagaaca cccaagaggtgttcgcccaagtgaagcagatctacaagacccctcctatcaagtacttcggcggct tcaatttcagccagattctgcccgatcctagcaagcccagcaagcggagcttcatcgaggacctgc tgttcaacaaagtgacactggccgacgccggcttcatcaagcagtatggcgattgtctgggcgaca ttgccgccagggatctgatttgcgcccagaagtttaacggactgacagtgctgcctcctctgctga ccgatgagatgatcgcccagtacacatctgccctgctggccggcacaatcacaagcggctggacat ttggagcaggcgccgctctgcagatcccctttgctatgcagatggcctaccggttcaacggcatcg gagtgacccagaatgtgctgtacgagaaccagaagctgatcgccaaccagttcaacagcgccatcg gcaagatccaggacagcctgagcagcacagcaagcgccctgggaaagctgcaggacgtggtcaacc acaatgcccaggcactgaacaccctggtcaagcagctgtcctccaagttcggcgccatcagctctg tgctgaacgatatcctgagcagactggaccctcctgaggccgaggtgcagatcgacagactgatca caggcagactgcagagcctccagacatacgtgacccagcagctgatcagagccgccgagattagag cctctgccaatctggccgccaccaagatgtctgagtgtgtgctgggccagagcaagagagtggact tttgcggcaagggctaccacctgatgagcttccctcagtctgcccctcacggcgtggtgtttctgc acgtgacatatgtgcccgctcaagagaagaatttcaccaccgctccagccatctgccacgacggca aagcccactttcctagagaaggcgtgttcgtgtccaacggcacccattggttcgtgacacagcgga acttctacgagccccagatcatcaccaccgacaacaccttcgtgtctggcaactgcgacgtcgtga tcggcattgtgaacaataccgtgtacgaccctctgcagcccgagctggacagcttcaaagaggaac tggacaagtactttaagaaccacacaagccccgacgtggacctgggcgatatcagcggaatcaatg ccagcgtcgtgaacatccagaaagagatcgaccggctgaacgaggtggccaagaatctgaacgaga gcctgatcaacctgcaagaactggggaagtacgagcagtacatcaagtggccctggtacatctggc tgggctttatcgccggactgattgccatcgtgatggtcacaatcatgctgtgttgcatgaccagct gctgtagctgcctgaagggctgttgtagctgtggcagctgctgcaagttcgacgaggacgattctg agcccgtgctgaagggcgtgaaactgcactacacatgatgactcgagctggtactgcatgcacgca atgctagctgcccctttcccgtcctgggtaccccgagtctcccccgacctcgggtcccaggtatgc tcccacctccacctgccccactcaccacctctgctagttccagacacctcccaagcacgcagcaat gcagctcaaaacgcttagcctagccacacccccacgggaaacagcagtgattaacctttagcaata aacgaaagtttaactaagctatactaaccccagggttggtcaatttcgtgccagccacaccctgga gctagcaaaaaaaaaaaaaaaaaaaaaaaaaaaaaagcatatgactaaaaaaaaaaaaaaaaaaaa aaaaaaaaaaaaaaaaaaaaaaaaaaaaaaaaaaaaaaaaaaaaaaaaa |

**Table 14. Sequence of one embodiment of an exemplary Omicron BA.4.6/BF.7-specific RNA vaccine**

| SEQ ID NO. | Brief Description | Sequence |
|---|---|---|
| 90 | Amino acid sequence of RNA-encoded SARS-CoV-2 S protein from an Omicron BA.4.6/BF.7 variant (with PRO mutations at positions corresponding to K986P and V987P of SEQ ID NO: 1; i.e., PRO mutations at positions 981 and 982 of SEQ ID NO: 90) | MFVFLVLLPLVSSQCVNLITRTQSYTNSFTRGVYYPDKVFRSSVLHSTQDLFLPFFSNVTWFHAIS GTNGTKRFDNPVLPFNDGVYFASTEKSNIIRGWIFGTTLDSKTQSLLIVNNATNVVIKVCEFQFCN DPFLDVYYHKNNKSWMESEFRVYSSANNCTFEYVSQPFLMDLEGKQGNFKNLREFVFKNIDGYFKI YSKHTPINLGRDLPQGFSALEPLVDLPIGINITRFQTLLALHRSYLTPGDSSSGWTAGAAAYYVGY LQPRTFLLKYNENGTITDAVDCALDPLSETKCTLKSFTVEKGIYQTSNFRVQPTESIVRFPNITNL CPFDEVFNATTFASVYAWNRKRISNCVADYSVLYNFAPFFAFKCYGVSPTKLNDLCFTNVYADSFV IRGNEVRQIAPGQTGNIADYNYKLPDDFTGCVIAWNSNKLDSKVGGNYNYRYRLFRKSNLKPFERD ISTEIYQAGNKPCNGVAGVNCYFPLQSYGFRPTYGVGHQPYRVVVLSFELLHAPATVCGPKKSTNL VKNKCVNFNFNGLTGTGVLTESNKKFLPFQQFGRDIADTTDAVRDPQTLEILDITPCSFGGVSVIT PGTNTSNQVAVLYQGVNCTEVPVAIHADQLTPTWRVYSTGSNVFQTRAGCLIGAEYVNNSYECDIP IGAGICASYQTQTKSHRRARSVASQSIIAYTMSLGAENSVAYSNNSIAIPTNFTISVTTEILPVSM TKTSVDCTMYICGDSTECSNLLLQYGSFCTQLKRALTGIAVEQDKNTQEVFAQVKQIYKTPPIKYF GGFNFSQILPDPSKPSKRSFIEDLLFNKVTLADAGFIKQYGDCLGDIAARDLICAQKFNGLTVLPP LLTDEMIAQYTSALLAGTITSGWTFGAGAALQIPFAMQMAYRFNGIGVTQNVLYENQKLIANQFNS AIGKIQDSLSSTASALGKLQDVVNHNAQALNTLVKQLSSKFGAISSVLNDILSRLDPPEAEVQIDR LITGRLQSLQTYVTQQLIRAAEIRASANLAATKMSECVLGQSKRVDFCGKGYHLMSFPQSAPHGVV FLHVTYVPAQEKNFTTAPAICHDGKAHFPREGVFVSNGTHWFVTQRNFYEPQIITTDNTFVSGNCD VVIGIVNNTVYDPLQPELDSFKEELDKYFKNHTSPDVDLGDISGINASVVNIQKEIDRLNEVAKNL NESLIDLQELGKYEQYIKWPWYIWLGFIAGLIAIVMVTIMLCCMTSCCSCLKGCCSCGSCCKFDED DSEPVLKGVKLHYT |
| 91 | RNA sequence encoding a SARS-CoV-2 S protein from an Omicron BA.4.6/BF.7 variant | auguucguguuccuggugcugcugccucugguguccagccagugugugaaccugaucaccagaaca cagucauacaccaacagcuuuaccagaggcguguacuaccccgacaagguguucagauccagcgug cugcacucuacccaggaccuguuccugccuuucuucagcaacgugaccugguuccacgccaucucc ggcaccaauggcaccaagagauucgacaaccccgugcugcccuucaacgacgggguguacuuugcc agcaccgagaaguccaacaucaucagaggcuggaucuucggcaccacacuggacagcaagacccag agccugcugaucgugaacaacgccaccaacguggucaucaaagugugcgaguuccaguucugcaac gaccccuuccuggacgucuacuaccacaagaacaacaagagcuggauggaaagcgaguuccgggug uacagcagcgccaacaacugcaccuucgaguacgugucccagccuuuccugauggaccuggaaggc aagcagggcaacuucaagaaccugcgcgaguucguguuuaagaacaucgacggcuacuucaagauc uacagcaagcacaccccuaucaaccucggccgggaucugccucagggcuucucugcucuggaaccc cugguggaucugcccaucggcaucaacaucacccgguuucagacacugcuggcccugcacagaagc uaccugacaccuggcgauagcagcagcggauggacagcuggugccgccgcuuacuaugugggcuac cugcagccuagaaccuuccugcugaaguacaacgagaacggcaccaucaccgacgccguggauugu gcucuggaucccucugagcgagacaaagugcacccugaaguccuucaccguggaaaagggcaucuac cagaccagcaacuuccgggugcagcccaccgaauccaucgugcgguuccccaauaucaccaaucug ugccccuucgacgagguguucaaugccaccaccuucgccucuguguacgccuggaaccggaagcgg aucagcaauugcguggccgacuacuccgugcuguacaacuucgccccuuucuucgcauucaagugc uacggcguguccccuaccaagcugaacgaccugugcuucacaaacguguacgccgacagcuucgug auccggggaaacgaagugcggcagauugccccuggacagacaggcaacaucgccgacuacaacuac |
| | | aagcugcccgacgacuucaccggcugugugauugccuggaacagcaacaagcuggacuccaaaguc ggcggcaacuacaauuacagguaccggcuguuccggaaguccaaucugaagcccuucgagcgggac aucuccaccgagaucuaucaggccggcaacaagccuuguaacggcguggcaggcgugaacugcuac uucccacugcaguccuacggcuuuaggcccacauacggcguggccaccagcccuacagaguggug gugcugagcuucgaacugcugcaugccccugccacagugugcggcccuaagaaaagcaccaaucuc gugaagaacaaaugcgugaacuucaacuucaacggccugaccggcaccggcgugcugacagagagc aacaagaaguuccugccauuccagcaguuuggccgggauaucgccgauaccacagacgccguuaga gauccccagacacuggaaauccuggacaucaccccuugcagcuucggcggagugucugugaucacc ccuggcaccaacaccagcaaucagguggcagugcuguaccagggcgugaacuguaccgaagugccc guggccauucacgccgaucagcugacaccuacauggcggguguacuccaccggcagcaauguguuu cagaccagagccggcugucugaucggagccgaguacgugaacaauagcuacgagugcgacaucccc aucggcgcuggaaucugcgccagcuaccagacacagacaaagagccaccggagagccagaagcgug gccagccagagcaucauugccuacacaaugucucugggcgccgagaacagcguggccuacuccaac aacucuaucgcuauccccaccaacuucaccaucagcgugaccacagagauccugccuguguccaug accaagaccagcguggacugcaccauguacaucugcggcgauuccaccgagugcuccaaccugcug cugcaguacggcagcuucugcacccagcugaaaagagcccugacagggaucgccguggaacaggac aagaacacccaagagguguucgcccaagugaagcagaucuacaagaccccuccuaucaaguacuuc ggcggcuucaauuucagccagauucugcccgauccuagcaagcccagcaagcggagcuucaucgag gaccugcuguucaacaaagugacacuggccgacgccggcuucaucaagcaguauggcgauugucug ggcgacauugccgccagggaucugauuugcgcccagaaguuuaacggacugacagugcugccuccu cugcugaccgaugagaugaucgcccaguacacaucugcccugcuggccggcacaaucacaagcggc uggacauuuggagcaggcgccgcucugcagauccccuuugcuaugcagauggccuaccgguucaac ggcaucggagugacccagaaugugcuguacgagaaccagaagcugaucgccaaccaguucaacagc gccaucggcaagauccaggacagccugagcagcacagcaagcgcccugggaaagcugcaggacgug gucaaccacaaugcccaggcacugaacacccuggucaagcagcuguccuccaaguucggcgccauc agcucugugcugaacgauauccugagcagacuggacccuccugaggccgaggugcagaucgacaga cugaucacaggcagacugcagagccuccagacauacgugacccagcagcugaucagagccgccgag auuagagccucugccaaucuggccgccaccaagaugucugagugugugcugggccagagcaagaga guggacuuuugcggcaagggcuaccaccugaugagcuucccucagucugccccucacggcguggug uuucugcacgugacauaugugcccgcucaagagaagaauuucaccaccgcuccagccaucugccac gacggcaaagcccacuuuccuagagaaggcguguucguguccaacggcacccauugguucgugaca cagcggaacuucuacgagccccagaucaucaccaccgacaacaccuucgugucuggcaacugcgac gucgugaucggcauugugaacaauaccguguacgacccucugcagcccgagcuggacagcuucaaa gaggaacuggacaaguacuuuaagaaccacacaagccccgacguggaccugggcgauaucagcgga aucaaugccagcgucgugaacauccagaaagagaucgaccggcugaacgagguggccaagaaucug aacgagagccugaucgaccugcaagaacugggaaguacgagcaguacaucaaguggcccugguac aucuggcugggcuuuaucgccggacugauugccaucgugauggucacaaucaugcuguguugcaug accagcugcuguagcugccugaagggcuguuguagcuguggcagcugcugcaaguucgacgaggac gauucugagcccgugcugaagggcgugaaacugcacuacacaugauga |
| 92 | DNA sequence encoding a SARS-CoV-2 S protein from an Omicron BA.4.6/BF.7 variant | atgttcgtgttcctggtgctgctgcctctggtgtccagccagtgtgtgaacctgatcaccagaaca cagtcatacaccaacagctttaccagaggcgtgtactaccccgacaaggtgttcagatccagcgtg ctgcactctacccaggacctgttcctgcctttcttcagcaacgtgacctggttccacgccatctcc ggcaccaatggcaccaagagattcgacaaccccgtgctgcccttcaacgacggggtgtactttgcc agcaccgagaagtccaacatcatcagaggctggatcttcggcaccacactggacagcaagacccag agcctgctgatcgtgaacaacgccaccaacgtggtcatcaaagtgtgcgagttccagttctgcaac gaccccttcctgggcgtctactaccacaagaacaacaagagctggatggaaagcgagttccgggtg tacagcagcgccaacaactgcaccttcgagtacgtgtcccagcctttcctgatggacctggaaggc aagcagggcaacttcaagaacctgcgcgagttcgtgtttaagaacatcgacggctacttcaagatc tacagcaagcacacccctatcaacctcggccgggatctgcctcagggcttctctgctctggaaccc ctggtggatctgcccatcggcatcaacatcacccggtttcagacactgctggccctgcacagaagc tacctgacacctggcgatagcagcagcggatggacagctggtgccgccgcttactatgtgggctac ctgcagcctagaaccttcctgctgaagtacaacgagaacggcaccatcaccgacgccgtggattgt gctctggatcctctgagcgagacaaagtgcaccctgaagtccttcaccgtggaaaagggcatctac cagaccagcaacttccgggtgcagcccaccgaatccatcgtgcggttccccaatatcaccaatctg tgccccttcgacgaggtgttcaatgccaccaccttcgcctctgtgtacgcctggaaccggaagcgg atcagcaattgcgtggccgactactccgtgctgtacaacttcgcccccttcttcgcattcaagtgc tacggcgtgtcccctaccaagctgaacgacctgtgcttcacaaacgtgtacgccgacagcttcgtg atccggggaaacgaagtgcggcagattgcccctggacagacaggcaacatcgccgactacaactac aagctgcccgacgacttcaccggctgtgtgattgcctggaacagcaacaagctggactccaaagtc ggcggcaactacaattacaggtaccggctgttccggaagtccaatctgaagcccttcgagcgggac atctccaccgagatctatcaggccggcaacaagccttgtaacggcgtggcaggcgtgaactgctac ttcccactgcagtcctacggctttaggcccacatacggcgtgggccaccagccctacagagtggtg gtgctgagcttcgaactgctgcatgcccctgccacagtgtgcggccctaagaaaagcaccaatctc gtgaagaacaaatgcgtgaacttcaacttcaacggcctgaccggcaccggcgtgctgacagagagc aacaagaagttcctgccattccagcagtttggccgggatatcgccgataccacagacgccgttaga gatccccagacactggaaatcctggacatcaccccttgcagcttcggcggagtgtctgtgatcacc cctggcaccaacaccagcaatcaggtggcagtgctgtaccagggcgtgaactgtaccgaagtgccc gtggccattcacgccgatcagctgacacctacatggcgggtgtactccaccggcagcaatgtgttt cagaccagagccggctgtctgatcggagccgagtacgtgaacaatagctacgagtgcgacatcccc atcggcgctggaatctgcgccagctaccagacacagacaaagagccaccggagagccagaagcgtg gccagccagagcatcattgcctacacaatgtctctgggcgccgagaacagcgtggcctactccaac aactctatcgctatccccaccaacttcaccatcagcgtgaccacagagatcctgcctgtgtccatg accaagaccagcgtggactgcaccatgtacatctgcggcgattccaccgagtgctccaacctgctg ctgcagtacggcagcttctgcacccagctgaaaagagccctgacagggatcgccgtggaacaggac aagaacacccaagaggtgttcgcccaagtgaagcagatctacaagacccctcctatcaagtacttc ggcggcttcaatttcagccagattctgcccgatcctagcaagcccagcaagcggagcttcatcgag gacctgctgttcaacaaagtgacactggccgacgccggcttcatcaagcagtatggcgattgtctg ggcgacattgccgccagggatctgatttgcgcccagaagtttaacggactgacagtgctgcctcct ctgctgaccgatgagatgatcgcccagtacacatctgccctgctggccggcacaatcacaagcggc |
| | | tggacatttggagcaggcgccgctctgcagatcccctttgctatgcagatggcctaccggttcaac ggcatcggagtgacccagaatgtgctgtacgagaaccagaagctgatcgccaaccagttcaacagc gccatcggcaagatccaggacagcctgagcagcacagcaagcgccctgggaaagctgcaggacgtg gtcaaccacaatgcccaggcactgaacaccctggtcaagcagctgtcctccaagttcggcgccatc agctctgtgctgaacgatatcctgagcagactggaccctcctgaggccgaggtgcagatcgacaga ctgatcacaggcagactgcagagcctccagacatacgtgacccagcagctgatcagagccgccgag attagagcctctgccaatctggccgccaccaagatgtctgagtgtgtgctgggccagagcaagaga gtggacttttgcggcaagggctaccacctgatgagcttccctcagtctgcccctcacggcgtggtg tttctgcacgtgacatatgtgcccgctcaagagaagaatttcaccaccgctccagccatctgccac gacggcaaagcccactttcctagagaaggcgtgttcgtgtccaacggcacccattggttcgtgaca cagcggaacttctacgagccccagatcatcaccaccgacaacaccttcgtgtctggcaactgcgac gtcgtgatcggcattgtgaacaataccgtgtacgaccctctgcagcccgagctggacagcttcaaa gaggaactggacaagtactttaagaaccacacaagccccgacgtggacctgggcgatatcagcgga atcaatgccagcgtcgtgaacatccagaaagagatcgaccggctgaacgaggtggccaagaatctg aacgagagcctgatcgacctgcaagaactggggaagtacgagcagtacatcaagtggccctggtac atctggctgggctttatcgccggactgattgccatcgtgatggtcacaatcatgctgtgttgcatg accagctgctgtagctgcctgaagggctgttgtagctgtggcagctgctgcaagttcgacgaggac gattctgagcccgtgctgaagggcgtgaaactgcactacacatgatga |
| 93 | Full length RNA construct encoding a SARS-CoV-2 S protein from an Omicron BA.4.6/BF.7 variant | agaauaaacuaguauucuucuggucccacagacucagagagaacccgccaccauguucguguucc uggugcugcugccucugguguccagccagugugugaaccugaucaccagaacacagucauacacca acagcuuuaccagaggcguguacuaccccgacaagguguucagauccagcgugcugcacucuaccc aggaccuguuccugccuuucuucagcaacgugaccugguuccacgccaucuccggcaccaauggca ccaagagauucgacaaccccgugcugcccuucaacgacgggguguacuuugccagcaccgagaagu ccaacaucaucagaggcuggaucuucggcaccacacuggacagcaagacccagagccugcugaucg ugaacaacgccaccaacguggucaucaaagugugcgaguuccaguucugcaacgaccccuuccugg acgucuacuaccacaagaacaacaagagcuggauggaaagcgaguuccggguguacagcagcgcca acaacugcaccuucgaguacgugucccagccuuuccugauggaccuggaaggcaagcagggcaacu ucaagaaccugcgcgaguucguguuuaagaacaucgacggcuacuucaagaucuacagcaagcaca ccccuaucaaccucggccgggaucugccucagggcuucucugcucuggaaccccugguggaucugc ccaucggcaucaacaucacccgguuucagacacugcuggcccugcacagaagcuaccugacaccug gcgauagcagcagcggauggacagcugguggccgccgcuuacuaugugggcuaccugcagccuagaa ccuuccugcugaaguacaacgagaacggcaccaucaccgacgccguggauugugcucuggauccuc ugagcgagacaaagugcacccugaaguccuucaccguggaaaagggcaucuaccagaccagcaacu uccgggugcagcccaccgaauccaucgugcgguuccccaauaucaccaaucugugccccuucgacg agguguucaaugccaccaccuucgccucuguguacgccuggaaccggaagcggaucagcaauugcg uggccgacuacuccgugcuguacaacuucgccccuucuucgcauucaagugcuacggcgugucc cuaccaagcugaacgaccugugcuucacaaacguguacgccgacagcuucgugauccggggaaacg aagugcggcagauugccccuggacagacaggcaacaucgccgacuacaacuacaagcugcccgacg acuucaccggcugugugauugccuggaacagcaacaagcuggacuccaaagucggcggcaacuaca |
| | | auuacagguaccggcuguuccggaaguccaaucugaagcccuucgagcgggacaucuccaccgaga ucuaucaggccggcaacaagccuuguaacggcguggcaggcgugaacugcuacuucccacugcagu ccuacggcuuuaggcccacauacggcgugggccaccagcccuacagagugguggugcugagcuucg aacugcugcaugccccugccacagugugcggcccuaagaaaagcaccaaucucgugaagaacaaau gcgugaacuucaacuucaacggccugaccggcaccggcgugcugacagagagcaacaagaaguucc ugccauuccagcaguuuggccgggauaucgccgauaccacagacgccguuagagauccccagacac uggaaauccuggacaucaccccuugcagcuucggcggagugucugugaucaccccuggcaccaaca ccagcaaucagguggcagugcuguaccagggcgugaacuguaccgaagugcccguggccauucacg ccgaucagcugacaccuacauggcggguguacuccaccggcagcaauguguuucagaccagagccg gcugucugaucggagccgaguacgugaacaauagcuacgagugcgacauccccaucggcgcuggaa ucugcgccagcuaccagacacagacaaagagccaccggagagccagaagcguggccagccagagca ucauugccuacacaaugucucugggcgccgagaacagcguggccuacuccaacaacucuaucgcua uccccaccaacuucaccaucagcgugaccacagagauccugccuguguccaugaccaagaccagcg uggacugcaccauguacaucugcggcgauuccaccgagugcuccaaccugcugcugcaguacggca gcuucugcacccagcugaaaagagcccugacagggaucgccguggaacaggacaagaacacccaag agguguucgcccaagugaagcagaucuacaagaccccuccuaucaaguacuucggcggcuucaauu ucagccagauucugcccgauccuagcaagcccagcaagcggagcuucaucgaggaccugcuguuca acaaagugacacuggccgacgccggcuucaucaagcaguauggcgauugucugggcgacauugccg ccagggaucugauuugcgcccagaaguuuaacggacugacagugcugccuccucugcugaccgaug agaugaucgcccaguacacaucugcccugcuggccggcacaaucacaagcggcuggacauuuggag caggcgccgcucugcagauccccuuugcuaugcagauggccuaccgguucaacggcaucggaguga cccagaaugugcuguacgagaaccagaagcugaucgccaaccaguucaacagcgccaucggcaaga uccaggacagccugagcagcacagcaagcgcccugggaaagcugcaggacguggucaaccacaaug cccaggcacugaacacccuggucaagcagcuguccuccaaguucggcgccaucagcucugugcuga acgauauccugagcagacuggacccuccugaggccgaggugcagaucgacagacugaucacaggca gacugcagagccuccagacauacgugacccagcagcugaucagagccgccgagauuagagccucug ccaaucuggccgccaccaagaugucugagugugugcugggccagagcaagagaguggacuuuugcg gcaagggcuaccaccugaugagcuucccucagucugccccucacggcgugguguuucugcacguga cauaugugcccgcucaagagaagaauuucaccaccgcuccagccaucugccacgacggcaaagccc acuuuccuagagaaggcguguucguguccaacggcacccauugguucgugacacagcggaacuucu acgagccccagaucaucaccaccgacaacaccuucgugucuggcaacugcgacgucgugaucggca uugugaacaauaccguguacgacccucugcagcccgagcuggacagcuucaaagaggaacuggaca aguacuuuaagaaccacacaagccccgacguggaccugggcgauaucagcggaaucaaugccagcg ucgugaacauccagaaagagaucgaccggcugaacgagguggccaagaaucugaacgagagccuga ucgaccugcaagaacuggggaaguacgagcaguacaucaaguggcccugguacaucuggcugggcu uuaucgccggacugauugccaucgugauggucacaaucaugcuguguugcaugaccagcugcugua gcugccugaagggcuguuguagcuguggcagcugcugcaaguucgacgaggacgauucugagcccg ugcugaagggcgugaaacugcacuacacaugaugacucgagcugguacugcaugcacgcaaugcua gcugccccuuucccguccuggguaccccgagucucccccgaccucgggucccagguaugcucccac cuccaccugccccacucaccaccucugcuaguuccagacaccucccaagcacgcagcaaugcagcu |
| | | caaaacgcuuagccuagccacacccccacgggaaacagcagugauuaaccuuuagcaauaaacgaa aguuuaacuaagcuauacuaaccccaggguuggucaauuucgugccagccacacccuggagcuagc aaaaaaaaaaaaaaaaaaaaaaaaaaaaagcauaugacuaaaaaaaaaaaaaaaaaaaaaaaaaa aaaaaaaaaaaaaaaaaaaaaaaaaaaaaaaaaaaaaaaaaa |
| 94 | Full length DNA construct encoding a SARS-CoV-2 S protein from an Omicron BA.4.6/BF.7 variant | agaataaactagtattcttctggtccccacagactcagagagaacccgccaccatgttcgtgttcc tggtgctgctgcctctggtgtccagccagtgtgtgaacctgatcaccagaacacagtcatacacca acagctttaccagaggcgtgtactaccccgacaaggtgttcagatccagcgtgctgcactctaccc aggacctgttcctgcctttcttcagcaacgtgacctggttccacgccatctccggcaccaatggca ccaagagattcgacaaccccgtgctgcccttcaacgacggggtgtactttgccagcaccgagaagt ccaacatcatcagaggctggatcttcggcaccacactggacagcaagacccagagcctgctgatcg tgaacaacgccaccaacgtggtcatcaaagtgtgcgagttccagttctgcaacgaccccttcctgg acgtctactaccacaagaacaacaagagctggatggaaagcgagttccgggtgtacagcagcgcca acaactgcaccttcgagtacgtgtcccagcctttcctgatggacctggaaggcaagcagggcaact tcaagaacctgcgcgagttcgtgtttaagaacatcgacggctacttcaagatctacagcaagcaca cccctatcaacctcggccgggatctgcctcagggcttctctgctctggaacccctggtggatctgc ccatcggcatcaacatcacccggtttcagacactgctggccctgcacagaagctacctgacacctg gcgatagcagcagcggatggacagctggtgccgccgcttactatgtgggctacctgcagcctagaa ccttcctgctgaagtacaacgagaacggcaccatcaccgacgccgtggattgtgctctggatcctc tgagcgagacaaagtgcaccctgaagtccttcaccgtggaaaagggcatctaccagaccagcaact tccgggtgcagcccaccgaatccatcgtgcggttccccaatatcaccaatctgtgccccttcgacg aggtgttcaatgccaccaccttcgcctctgtgtacgcctggaaccggaagcggatcagcaattgcg tggccgactactccgtgctgtacaacttcgcccccttcttcgcattcaagtgctacggcgtgtccc ctaccaagctgaacgacctgtgcttcacaaacgtgtacgccgacagcttcgtgatccggggaaacg aagtgcggcagattgcccctggacagacaggcaacatcgccgactacaactacaagctgcccgacg acttcaccggctgtgtgattgcctggaacagcaacaagctggactccaaagtcggcggcaactaca attacaggtaccggctgttccggaagtccaatctgaagcccttcgagcgggacatctccaccgaga tctatcaggccggcaacaagccttgtaacggcgtggcaggcgtgaactgctacttcccactgcagt cctacggctttaggcccacatacggcgtgggccaccagccctacagagtggtggtgctgagcttcg aactgctgcatgcccctgccacagtgtgcggccctaagaaaagcaccaatctcgtgaagaacaaat gcgtgaacttcaacttcaacggcctgaccggcaccggcgtgctgacagagagcaacaagaagttcc tgccattccagcagtttggccgggatatcgccgataccacagacgccgttagagatccccagacac tggaaatcctggacatcaccccttgcagcttcggcggagtgtctgtgatcacccctggcaccaaca ccagcaatcaggtggcagtgctgtaccagggcgtgaactgtaccgaagtgcccgtggccattcacg ccgatcagctgacacctacatggcgggtgtactccaccggcagcaatgtgtttcagaccagagccg gctgtctgatcggagccgagtacgtgaacaatagctacgagtgcgacatccccatcggcgctggaa tctgcgccagctaccagacacagacaaagagccaccggagagccagaagcgtggccagccagagca tcattgcctacacaatgtctctgggcgccgagaacagcgtggcctactccaacaactctatcgcta tccccaccaacttcaccatcagcgtgaccacagagatcctgcctgtgtccatgaccaagaccagcg tggactgcaccatgtacatctgcggcgattccaccgagtgctccaacctgctgctgcagtacggca |
| | | gcttctgcacccagctgaaaagagccctgacagggatcgccgtggaacaggacaagaacacccaag aggtgttcgcccaagtgaagcagatctacaagacccctcctatcaagtacttcggcggcttcaatt tcagccagattctgcccgatcctagcaagcccagcaagcggagcttcatcgaggacctgctgttca acaaagtgacactggccgacgccggcttcatcaagcagtatggcgattgtctgggcgacattgccg ccagggatctgatttgcgcccagaagtttaacggactgacagtgctgcctcctctgctgaccgatg agatgatcgcccagtacacatctgccctgctggccggcacaatcacaagcggctggacatttggag caggcgccgctctgcagatcccctttgctatgcagatggcctaccggttcaacggcatcggagtga cccagaatgtgctgtacgagaaccagaagctgatcgccaaccagttcaacagcgccatcggcaaga tccaggacagcctgagcagcacagcaagcgccctgggaaagctgcaggacgtggtcaaccacaatg cccaggcactgaacaccctggtcaagcagctgtcctccaagttcggcgccatcagctctgtgctga acgatatcctgagcagactggaccctcctgaggccgaggtgcagatcgacagactgatcacaggca gactgcagagcctccagacatacgtgacccagcagctgatcagagccgccgagattagagcctctg ccaatctggccgccaccaagatgtctgagtgtgtgctgggccagagcaagagagtggacttttgcg gcaagggctaccacctgatgagcttccctcagtctgcccctcacggcgtggtgtttctgcacgtga catatgtgcccgctcaagagaagaatttcaccaccgctccagccatctgccacgacggcaaagccc actttcctagagaaggcgtgttcgtgtccaacggcacccattggttcgtgacacagcggaacttct acgagccccagatcatcaccaccgacaacaccttcgtgtctggcaactgcgacgtcgtgatcggca ttgtgaacaataccgtgtacgaccctctgcagcccgagctggacagcttcaaagaggaactggaca agtactttaagaaccacacaagccccgacgtggacctgggcgatatcagcggaatcaatgccagcg tcgtgaacatccagaaagagatcgaccggctgaacgaggtggccaagaatctgaacgagagcctga tcgacctgcaagaactggggaagtacgagcagtacatcaagtggccctggtacatctggctgggct ttatcgccggactgattgccatcgtgatggtcacaatcatgctgtgttgcatgaccagctgctgta gctgcctgaagggctgttgtagctgtggcagctgctgcaagttcgacgaggacgattctgagcccg tgctgaagggcgtgaaactgcactacacatgatgactcgagctggtactgcatgcacgcaatgcta gctgcccctttcccgtcctgggtaccccgagtctcccccgacctcgggtcccaggtatgctcccac ctccacctgccccactcaccacctctgctagttccagacacctcccaagcacgcagcaatgcagct caaaacgcttagcctagccacacccccacgggaaacagcagtgattaacctttagcaataaacgaa agtttaactaagctatactaaccccagggttggtcaatttcgtgccagccacaccctggagctagc aaaaaaaaaaaaaaaaaaaaaaaaaaaaaagcatatgactaaaaaaaaaaaaaaaaaaaaaaaaaa aaaaaaaaaaaaaaaaaaaaaaaaaaaaaaaaaaaaaaaaa |

**Table 15. Sequence of one embodiment of an exemplary Omicron XBB-specific RNA vaccine**

| SEQ ID NO. | Brief Description | Sequence |
|---|---|---|
| 95 | Amino acid sequence of RNA-encoded SARS-CoV-2 S protein | MFVFLVLLPLVSSQCVNLITRTQSYTNSFTRGVYYPDKVFRSSVLHSTQDLFLPFFSNVTWFHAIH VSGTNGTKRFDNPALPFNDGVYFASTEKSNIIRGWIFGTTLDSKTQSLLIVNNATNVVIKVCEFQF CNDPFLDVYQKNNKSWMESEFRVYSSANNCTFEYVSQPFLMDLEGKEGNFKNLREFVFKNIDGYFK IYSKHTPINLERDLPQGFSALEPLVDLPIGINITRFQTLLALHRSYLTPGDSSSGWTAGAAAYYVG YLQPRTFLLKYNENGTITDAVDCALDPLSETKCTLKSFTVEKGIYQTSNFRVQPTESIVRFPNITN |
| | from an Omicron XBB variant (with PRO mutations at positions corresponding to K986P and V987P of SEQ ID NO: 1; i.e., PRO mutations at positions 981 and 982 of SEQ ID NO: 95) | LCPFHEVFNATTFASVYAWNRKRISNCVADYSVIYNFAPFFAFKCYGVSPTKLNDLCFTNVYADSF VIRGNEVSQIAPGQTGNIADYNYKLPDDFTGCVIAWNSNKLDSKPSGNYNYLYRLFRKSKLKPFER DISTEIYQAGNKPCNGVAGSNCYSPLQSYGFRPTYGVGHQPYRVVVLSFELLHAPATVCGPKKSTN LVKNKCVNFNFNGLTGTGVLTESNKKFLPFQQFGRDIADTTDAVRDPQTLEILDITPCSFGGVSVI TPGTNTSNQVAVLYQGVNCTEVPVAIHADQLTPTWRVYSTGSNVFQTRAGCLIGAEYVNNSYECDI PIGAGICASYQTQTKSHRRARSVASQSIIAYTMSLGAENSVAYSNNSIAIPTNFTISVTTEILPVS MTKTSVDCTMYICGDSTECSNLLLQYGSFCTQLKRALTGIAVEQDKNTQEVFAQVKQIYKTPPIKY FGGFNFSQILPDPSKPSKRSFIEDLLFNKVTLADAGFIKQYGDCLGDIAARDLICAQKFNGLTVLP PLLTDEMIAQYTSALLAGTITSGWTFGAGAALQIPFAMQMAYRFNGIGVTQNVLYENQKLIANQFN SAIGKIQDSLSSTASALGKLQDVVNHNAQALNTLVKQLSSKFGAISSVLNDILSRLDPPEAEVQID RLITGRLQSLQTYVTQQLIRAAEIRASANLAATKMSECVLGQSKRVDFCGKGYHLMSFPQSAPHGV VFLHVTYVPAQEKNFTTAPAICHDGKAHFPREGVFVSNGTHWFVTQRNFYEPQIITTDNTFVSGNC DVVIGIVNNTVYDPLQPELDSFKEELDKYFKNHTSPDVDLGDISGINASVVNIQKEIDRLNEVAKN LNESLIDLQELGKYEQYIKWPWYIWLGFIAGLIAIVMVTIMLCCMTSCCSCLKGCCSCGSCCKFDE DDSEPVLKGVKLHYT |
| 96 | RNA sequence encoding a SARS-CoV-2 S protein from an Omicron XBB variant | auguucguguuccuggugcugcugccucugguguccagccagugugugaaccugaucaccagaaca cagUCAuacaccaacagcuuuaccagaggcguguacuaccccgacaagguguucagauccagcgug cugcacucuacccaggaccuguuccugccuuucuucagcaacgugaccugguuccacgccauccac guguccggcaccaauggcaccaagagauucgacaaccccgcccugcccuucaacgacgggguguac uuugccagcaccgagaaguccaacaucaucagaggcuggaucuucggcaccacacuggacagcaag acccagagccugcugaucgugaacaacgccaccaacgugguca[?]ucaaagugugcgaguuccaguuc ugcaacgaccccuuccuggacgucuaccagaagaacaacaagagcuggauggaaagcgaguuccgg guguacagcagcgccaacaacugcaccuucgaguacgugucccagccuuuccugauggaccuggaa ggcaaggagggcaacuucaagaaccugcgcgaguucguguuuaagaacaucgacggcuacuucaag aucuacagcaagcacaccccuaucaaccucgagcgggaucugccucagggcuucucugcucuggaa ccccugguggaucugcccaucggcaucaacaucacccgguuucagacacugcuggcccugcacaga agcuaccugacaccuggcgauagcagcagcggauggacagcuggugccgccgcuuacuaugugggc uaccugcagccuagaaccuuccugcugaaguacaacgagaacggcaccaucaccgacgccguggau ugugcucuggauccucugagcgagacaaagugcacccugaaguccuucaccguggaaaagggcauc uaccagaccagcaacuuccgggugcagcccaccgaauccaucgugcgguuccccaauaucaccaau cugugccccuuccacgagguguucaaugccaccaccuucgccucuguguacgccuggaaccggaag cggaucagcaauugcguggccgacuacuccgugaucuacaacuucgccccuuucuucgcauucaag ugcuacggcguguccccuaccaagcugaacgaccugugcuucacaaacguguacgccgacagcuuc gugauccggggaaacgaagugucacagauugccccuggacagacaggcaacaucgccgacuacaac uacaagcugcccgacgacuucaccggcugugugauugccuggaacagcaacaagcuggacuccaaa cccagcggcaacuacaauuaccuguaccggcuguuccggaaguccaagcugaagcccuucgagcgg gacaucuccaccgagaucuaucaggccggcaacaagccuuguaacggcguggcaggcagcaacugc uacagcccacugcaguccuacggcuuuaggcccacauacggcguggccaccagcccuacagagug guggugcugagcuucgaacugcugcaugccccugccacagugugcggcccuaagaaaagcaccaau |
| | | cucgugaagaacaaaugcgugaacuucaacuucaacggccugaccggcaccggcgugcugacagag agcaacaagaaguuccugccauuccagcaguuggccgggauaucgccgauaccacagacgccguu agagauccccagacacuggaaauccuggacaucaccccuugcagcuucggcggagugucugugauc accccuggcaccaacaccagcaaucagguggcagugcuguaccagggcgugaacuguaccgaagug cccguggccauucacgccgaucagcugacaccuacauggcggguguacuccaccggcagcaaugug uuucagaccagagccggcugucugaucggagccgaguacgugaacaauagcuacgagugcgacauc cccaucggcgcuggaaucugcgccagcuaccagacacagacaaagagccaccggagagccagaagc guggccagccagagcaucauugccuacacaaugucucugggcgccgagaacagcguggccuacucc aacaacucuaucgcuauccccaccaacuucaccaucagcgugaccacagagauccugccuguguccc augaccaagaccagcguggacugcaccauguacaucugcggcgauuccaccgagugcuccaaccug cugcugcaguacggcagcuucugcacccagcugaaaagagcccugacagggaucgccguggaacag gacaagaacacccaagagguguucgcccaagugaagcagaucuacaagaccccuccuaucaaguac uucggcggcuucaauuucagccagauucugcccgauccuagcaagcccagcaagcggagcuucauc gaggaccugcuguucaacaaagugacacuggccgacgccggcuucaucaagcaguauggcgauugu cugggcgacauugccgccagggaucugauuugcgcccagaaguuuaacggacugacagugcugccu ccucugcugaccgaugagaugaucgcccaguacacaucugcccugcuggccggcacaaucacaagc ggcuggacauuuggagcaggcgccgcucugcagaucccuuugcuaugcagauggccuaccgguuc aacggcaucggagugacccagaaugugcuguacgagaaccagaagcugaucgccaaccaguucaac agcgccaucggcaagauccaggacagccugagcagcacagcaagcgcccugggaaagcugcaggac guggucaaccacaaugcccaggcacugaacacccuggucaagcagcuguccuccaaguucggcgcc aucagcucugugcugaacgauauccugagcagacuggacccuccugaggccgaggugcagaucgac agacugaucacaggcagacugcagagccuccagacauacgugacccagcagcugaucagagccgcc gagauuagagccucugccaaucuggccgccaccaagaugucugagugugugcugggccagagcaag agaguggacuuuugcggcaagggcuaccaccugaugagcuucccucagucugccccucacggcgug guguuucugcacgugacauaugugcccgcucaagagaagaauuucaccaccgcuccagccaucugc cacgacggcaaagcccacuuuccuagagaaggcguguucguguccaacggcacccauugguucgug acacagcggaacuucuacgagccccagaucaucaccaccgacaacaccuucgugucuggcaacugc gacgucgugaucggcauugugaacaauaccguguacgacccucugcagcccgagcuggacagcuuc aaagaggaacuggacaaguacuuuaagaaccacacaagccccgacguggaccugggcgauaucagc ggaaucaaugccagcgucgugaacauccagaaagagaucgaccggcugaacgagguggccaagaau cugaacgagagccugaucgaccugcaagaacugggaaaguacgagcaguacaucaaguggcccugg uacaucuggcugggcuuuaucgccggacugauugccaucgugauggucacaaucaugcuguguugc augaccagcugcuguagcugccugaagggcuguuguagcuguggcagcugcugcaaguucgacgag gacgauucugagcccgugcugaagggcgugaaacugcacuacacaugauga |
| 97 | DNA sequence encoding a SARS-CoV-2 S protein from an Omicron XBB variant | atgttcgtgttcctggtgctgctgcctctggtgtccagccagtgtgtgaacctgatcaccagaaca cagTCAtacaccaacagctttaccagaggcgtgtactaccccgacaaggtgttcagatccagcgtg ctgcactctacccaggacctgttcctgcctttcttcagcaacgtgacctggttccacgccatccac gtgtccggcaccaatggcaccaagagattcgacaaccccgccctgcccttcaacgacggggtgtac tttgccagcaccgagaagtccaacatcatcagaggctggatcttcggcaccacactggacagcaag |
| | | acccagagcctgctgatcgtgaacaacgccaccaacgtggtcatcaaagtgtgcgagttccagttc tgcaacgaccccttcctggacgtctaccagaagaacaacaagagctggatggaaagcgagttccgg gtgtacagcagcgccaacaactgcaccttcgagtacgtgtcccagcctttcctgatggacctggaa ggcaaggagggcaacttcaagaacctgcgcgagttcgtgtttaagaacatcgacggctacttcaag atctacagcaagcacacccctatcaacctcgagcgggatctgcctcagggcttctctgctctggaa cccctggtggatctgcccatcggcatcaacatcacccggtttcagacactgctggccctgcacaga agctacctgacacctggcgatagcagcagcggatggacagctggtgccgccgcttactatgtgggc tacctgcagcctagaaccttcctgctgaagtacaacgagaacggcaccatcaccgacgccgtggat tgtgctctggatcctctgagcgagacaaagtgcaccctgaagtccttcaccgtggaaaagggcatc taccagaccagcaacttccgggtgcagcccaccgaatccatcgtgcggttccccaatatcaccaat ctgtgccccttccacgaggtgttcaatgccaccaccttcgcctctgtgtacgcctggaaccggaag cggatcagcaattgcgtggccgactactccgtgatctacaacttcgccccctttcttcgcattcaag tgctacggcgtgtcccctaccaagctgaacgacctgtgcttcacaaacgtgtacgccgacagcttc gtgatccggggaaacgaagtgtcacagattgcccctggacagacaggcaacatcgccgactacaac tacaagctgcccgacgacttcaccggctgtgtgattgcctggaacagcaacaagctggactccaaa cccagcggcaactacaattacctgtaccggctgttccggaagtccaagctgaagcccttcgagcgg gacatctccaccgagatctatcaggccggcaacaagccttgtaacggcgtggcaggcagcaactgc tacagcccactgcagtcctacggcttaggcccacatacggcgtgggccaccagccctacagagtg gtggtgctgagcttcgaactgctgcatgcccctgccacagtgtgcggccctaagaaaagcaccaat ctcgtgaagaacaaatgcgtgaacttcaacttcaacggcctgaccggcaccggcgtgctgacagag agcaacaagaagttcctgccattccagcagtttggccgggatatcgccgataccacagacgccgtt agagatccccagacactggaaatcctggacatcaccccttgcagcttcggcggagtgtctgtgatc acccctggcaccaacaccagcaatcaggtggcagtgctgtaccagggcgtgaactgtaccgaagtg cccgtggccattcacgccgatcagctgacacctacatggcgggtgtactccaccggcagcaatgtg tttcagaccagagccggctgtctgatcggagccgagtacgtgaacaatagctacgagtgcgacatc cccatcggcgctggaatctgcgccagctaccagacacagacaaagagccaccggagagccagaagc gtggccagccagagcatcattgcctacacaatgtctctgggcgccgagaacagcgtggcctactcc aacaactctatcgctatccccaccaacttcaccatcagcgtgaccacagagatcctgcctgtgtcc atgaccaagaccagcgtggactgcaccatgtacatctgcggcgattccaccgagtgctccaacctg ctgctgcagtacggcagcttctgcacccagctgaaaagagccctgacagggatcgccgtggaacag gacaagaacacccaagaggtgttcgcccaagtgaagcagatctacaagacccctcctatcaagtac ttcggcggcttcaatttcagccagattctgcccgatcctagcaagcccagcaagcggagcttcatc gaggacctgctgttcaacaaagtgacactggccgacgccggcttcatcaagcagtatggcgattgt ctgggcgacattgccgccagggatctgatttgcgcccagaagtttaacggactgacagtgctgcct cctctgctgaccgatgagatgatcgcccagtacacatctgccctgctggccggcacaatcacaagc ggctggacatttggagcaggcgccgctctgcagatcccctttgctatgcagatggcctaccggttc aacggcatcggagtgacccagaatgtgctgtacgagaaccagaagctgatcgccaaccagttcaac agcgccatcggcaagatccaggacagcctgagcagcacagcaagcgccctgggaaagctgcaggac gtggtcaaccacaatgcccaggcactgaacaccctggtcaagcagctgtcctccaagttcggcgcc atcagctctgtgctgaacgatatcctgagcagactggaccctcctgaggccgaggtgcagatcgac |
| | | agactgatcacaggcagactgcagagcctccagacatacgtgacccagcagctgatcagagccgcc gagattagagcctctgccaatctggccgccaccaagatgtctgagtgtgtgctgggccagagcaag agagtggacttttgcggcaagggctaccacctgatgagcttccctcagtctgcccctcacggcgtg gtgtttctgcacgtgacatatgtgcccgctcaagagaagaatttcaccaccgctccagccatctgc cacgacggcaaagcccactttcctagagaaggcgtgttcgtgtccaacggcacccattggttcgtg acacagcggaacttctacgagccccagatcatcaccaccgacaacaccttcgtgtctggcaactgc gacgtcgtgatcggcattgtgaacaataccgtgtacgaccctctgcagcccgagctggacagcttc aaagaggaactggacaagtactttaagaaccacacaagccccgacgtggacctgggcgatatcagc ggaatcaatgccagcgtcgtgaacatccagaaagagatcgaccggctgaacgaggtggccaagaat ctgaacgagagcctgatcgacctgcaagaactggggaagtacgagcagtacatcaagtggccctgg tacatctggctgggctttatcgccggactgattgccatcgtgatggtcacaatcatgctgtgttgc atgaccagctgctgtagctgcctgaagggctgttgtagctgtggcagctgctgcaagttcgacgag gacgattctgagcccgtgctgaagggcgtgaaactgcactacacatgatga |
| 98 | Full length RNA construct encoding a SARS-CoV-2 S protein from an Omicron XBB variant | agaauaaacuaguauucuucugguccccacagacucagagagaacccgccaccauguucguguucc uggugcugcugccucugguguccagccagugugugaaccugaucaccagaacacagUCAuacacca acagcuuuaccagaggcguguacuaccccgacaagguguucagauccagcgugcugcacucuaccc aggaccuguuccugccuuucuucagcaacgugaccugguuccacgccauccacguguccggcacca auggcaccaagagauucgacaaccccgcccugcccuucaacgacgggguguacuuugccagcaccg agaaguccaacaucaucagaggcuggaucuucggcaccacacuggacagcaagacccagagccugc ugaucgugaacaacgccaccaacguggucaucaaagugugcgaguuccaguucugcaacgaccccu uccuggacgucuaccagaagaacaacaagagcuggauggaaagcgaguuccggguguacagcagcg ccaacaacugcaccuucgaguacgugucccagccuuuccugauggaccuggaaggcaaggagggca acuucaagaaccugcgcgaguucgguguuuaagaacaucgacggcuacuucaagaucuacagcaagc acaccccuaucaaccucgagcgggaucugccucagggcuucucugcucuggaaccccugguggauc ugcccaucggcaucaacaucacccgguuucagacacugcuggcccugcacagaagcuaccugacac cuggcgauagcagcagcggauggacagcugguggccgccgcuuacuaugugggcuaccugcagccua gaaccuuccugcugaaguacaacgagaacggcaccaucaccgacgccguggauugugcucuggauc cucugagcgagacaaagugcacccugaaguccuucaccguggaaaagggcaucuaccagaccagca acuuccgggugcagcccaccgaauccaucgugcgguuccccaauaucaccaaucugugccccuucc acgagguguucaaugccaccaccuucgccucuguguacgccuggaaccggaagcggaucagcaauu gcguggccgacuacuccgugaucuacaacuucgccccuucuucgcauucaagugcuacggcgugu ccccuaccaagcugaacgaccugugcuucacaaacguguacgccgacagcuucgugauccggggaa acgaaguguccacagauugccccuggacagacaggcaacaucgccgacuacaacuacaagcugcccg acgacuucaccggcugugugauugccuggaacagcaacaagcuggacuccaaacccagcggcaacu acaauuaccuguaccggcuguuccggaaguccaagcugaagcccuucgagcgggacaucuccaccg agaucuaucaggccggcaacaagccuuguaacggcguggcaggcagcaacugcuacagcccacugc aguccuacggcuuuaggcccacauacggcguggccaccagcccuacagaguggugcugcugagcu ucgaacugcugcaugccccugccacagugugcggcccuaagaaaagcaccaaucucgugaagaaca aaugcgugaacuucaacuucaacggccugaccggcaccggcgugcugacagagagcaacaagaagu |
| | | uccugccauuccagcaguuuggccgggauaucgccgauaccacagacgccguuagagauccccaga cacuggaaauccuggacaucaccccuugcagcuucggcggagugucugugaucaccccuggcacca acaccagcaaucagguggcagugcuguaccagggcgugaacuguaccgaagugcccuggccauuc acgccgaucagcugacaccuacauggcggguguacuccaccggcagcaauguguuucagaccagag ccggcugucugaucggagccgaguacgugaacaauagcuacgagugcgacauccccaucggcgcug gaaucugcgccagcuaccagacacagacaaagagccaccggagagccagaagcgugccagccaga gcaucauugccuacacaaugucucugggcgccgagaacagcguggccuacuccaacaacucuaucg cuauccccaccaacuucaccaucagcgugaccacagagauccugccuguguccaugaccaagacca gcguggacugcaccauguacaucugcggcgauuccaccgagugcuccaaccugcugcugcaguacg gcagcuucugcacccagcugaaaagagcccugacagggaucgccguggaacaggacaagaacaccc aagagguguucgcccaagugaagcagaucuacaagaccccuccuaucaaguacuucggcggcuuca auuucagccagauucugcccgauccuagcaagcccagcaagcggagcuucaucgaggaccugcugu ucaacaaagugacacuggccgacgccggcuucaucaagcaguauggcgauugucuggcgacauug ccgccagggaucugauuugcgcccagaaguuuaacggacugacagugcugccuccucugcugaccg augagaugaucgcccaguacacaucugcccugcuggccggcacaaucacaagcggcuggacauuug gagcaggcgccgcucugcagauccccuuugcuaugcagauggccuaccgguucaacggcaucggag ugacccagaaugugcuguacgagaaccagaagcugaucgccaaccaguucaacagcgccaucggca agauccaggacagccugagcagcacagcaagcgcccugggaaagcugcaggacguggucaaccaca augcccaggcacugaacacccuggucaagcagcuguccuccaaguucggcgccaucagcucugugc ugaacgauauccugagcagacuggacccuccugaggccgaggugcagaucgacagacugaucacag gcagacugcagagccuccagacauacgugacccagcagcugaucagagccgccgagauuagagccu cugccaaucuggccgccaccaagaugucugagugugugcugggccagagcaagagaguggacuuuu gcggcaagggcuaccaccugaugagcuucccucagucugccccucacggcgugguguuucugcacg ugacauaugugcccgcucaagagaagaauuucaccaccgcuccagccaucugccacgacggcaaag cccacuuuccuagagaaggcguguucguguccaacggcacccauugguucgugacacagcggaacu ucuacgagccccagaucaucaccaccgacaacaccuucgugucuggcaacugcgacgucgugaucg gcauugugaacaauaccguguacgacccucugcagcccgagcuggacagcuucaaagaggaacugg acaaguacuuuaagaaccacacaagccccgacguggaccugggcgauaucagcggaaucaaugcca gcgucgugaacauccagaaagagaucgaccggcugaacgagguggccaagaaucugaacgagagcc ugaucgaccugcaagaacuggggaaguacgagcaguacaucaaguggcccugguacaucuggcugg gcuuuaucgccggacugauugccaucgugauggucacaaucaugcuguguugcaugaccagcugcu guagcugccugaagggcuguuguagcuguggcagcugcugcaaguucgacgaggacgauucugagc ccgugcugaagggcgugaaacugcacuacacaugaugacucgagcugguacugcaugcacgcaaug cuagcugccccuuucccguccuggguaccccgagucucccccgaccucggguccagguaugcucc caccuccaccugccccacucaccaccucugcuaguuccagacaccucccaagcacgcagcaaugca gcucaaaacgcuuagccuagccacacccccacgggaaacagcagugauuaaccuuuagcaauaaac gaaaguuuaacuaagcuauacuaaccccaggguuggucaauuucgugccagccacacccuggagcu agcaaaaaaaaaaaaaaaaaaaaaaaaaaaaagcauaugacuaaaaaaaaaaaaaaaaaaaaaaaa aaaaaaaaaaaaaaaaaaaaaaaaaaaaaaaaaaaaaaaaaaaa |
| 99 | Full length DNA construct encoding a SARS-CoV-2 S protein from an Omicron XBB variant | agaataaactagtattcttctggtccccacagactcagagagaacccgccaccatgttcgtgttcc tggtgctgctgcctctggtgtccagccagtgtgtgaacctgatcaccagaacacagTCAtacacca acagctttaccagaggcgtgtactaccccgacaaggtgttcagatccagcgtgctgcactctaccc aggacctgttcctgcctttcttcagcaacgtgacctggttccacgccatccacgtgtccggcacca atggcaccaagagattcgacaaccccgccctgcccttcaacgacggggtgtactttgccagcaccg agaagtccaacatcatcagaggctggatcttcggcaccacactggacagcaagacccagagcctgc tgatcgtgaacaacgccaccaacgtggtcatcaaagtgtgcgagttccagttctgcaacgacccct tcctggacgtctaccagaagaacaacaagagctggatggaaagcgagttccgggtgtacagcagcg ccaacaactgcaccttcgagtacgtgtcccagcctttcctgatggacctggaaggcaaggagggca acttcaagaacctgcgcgagttcgtgtttaagaacatcgacggctacttcaagatctacagcaagc acacccctatcaacctcgagcgggatctgcctcagggcttctctgctctggaacccctggtggatc tgcccatcggcatcaacatcacccggtttcagacactgctggccctgcacagaagctacctgacac ctggcgatagcagcagcggatggacagctggtgccgccgcttactatgtgggctacctgcagccta gaaccttcctgctgaagtacaacgagaacggcaccatcaccgacgccgtggattgtgctctggatc ctctgagcgagacaaagtgcaccctgaagtccttcaccgtggaaaagggcatctaccagaccagca acttccgggtgcagcccaccgaatccatcgtgcggttccccaatatcaccaatctgtgccccttcc acgaggtgttcaatgccaccaccttcgcctctgtgtacgcctggaaccggaagcggatcagcaatt gcgtggccgactactccgtgatctacaacttcgcccccttcttcgcattcaagtgctacggcgtgt cccctaccaagctgaacgacctgtgcttcacaaacgtgtacgccgacagcttcgtgatccggggaa acgaagtgtcacagattgcccctggacagacaggcaacatcgccgactacaactacaagctgcccg acgacttcaccggctgtgtgattgcctggaacagcaacaagctggactccaaacccagcggcaact acaattacctgtaccggctgttccggaagtccaagctgaagcccttcgagcgggacatctccaccg agatctatcaggccggcaacaagccttgtaacggcgtggcaggcagcaactgctacagcccactgc agtcctacggctttaggcccacatacggcgtgggccaccagccctacagagtggtggtgctgagct tcgaactgctgcatgcccctgccacagtgtgcggccctaagaaaagcaccaatctcgtgaagaaca aatgcgtgaacttcaacttcaacggcctgaccggcaccggcgtgctgacagagagcaacaagaagt tcctgccattccagcagtttggccgggatatcgccgataccacagacgccgttagagatccccaga cactggaaatcctggacatcaccccttgcagcttcggcggagtgtctgtgatcacccctggcacca acaccagcaatcaggtggcagtgctgtaccagggcgtgaactgtaccgaagtgcccgtggccattc acgccgatcagctgacacctacatggcgggtgtactccaccggcagcaatgtgtttcagaccagag ccggctgtctgatcggagccgagtacgtgaacaatagctacgagtgcgacatccccatcggcgctg gaatctgcgccagctaccagacacagacaaagagccaccggagagccagaagcgtggccagccaga gcatcattgcctacacaatgtctctgggcgccgagaacagcgtggcctactccaacaactctatcg ctatccccaccaacttcaccatcagcgtgaccacagagatcctgcctgtgtccatgaccaagacca gcgtggactgcaccatgtacatctgcggcgattccaccgagtgctccaacctgctgctgcagtacg gcagcttctgcacccagctgaaaagagccctgacagggatcgccgtggaacaggacaagaacaccc aagaggtgttcgcccaagtgaagcagatctacaagacccctcctatcaagtacttcggcggcttca atttcagccagattctgcccgatcctagcaagcccagcaagcggagcttcatcgaggacctgctgt tcaacaaagtgacactggccgacgccggcttcatcaagcagtatggcgattgtctgggcgacattg ccgccagggatctgatttgcgcccagaagtttaacggactgacagtgctgcctcctctgctgaccg |
| | | atgagatgatcgcccagtacacatctgccctgctggccggcacaatcacaagcggctggacatttg gagcaggcgccgctctgcagatccccttgctatgcagatggcctaccggttcaacggcatcggag tgacccagaatgtgctgtacgagaaccagaagctgatcgccaaccagttcaacagcgccatcggca agatccaggacagcctgagcagcacagcaagcgccctgggaaagctgcaggacgtggtcaaccaca atgcccaggcactgaacaccctggtcaagcagctgtcctccaagttcggcgccatcagctctgtgc tgaacgatatcctgagcagactggaccctcctgaggccgaggtgcagatcgacagactgatcacag gcagactgcagagcctccagacatacgtgacccagcagctgatcagagccgccgagattagagcct ctgccaatctggccgccaccaagatgtctgagtgtgtgctgggccagagcaagagagtggactttt gcggcaagggctaccacctgatgagcttccctcagtctgcccctcacggcgtggtgttctgcacg tgacatatgtgcccgctcaagagaagaatttcaccaccgctccagccatctgccacgacggcaaag cccactttcctagagaaggcgtgttcgtgtccaacggcacccattggttcgtgacacagcggaact tctacgagccccagatcatcaccaccgacaacaccttcgtgtctggcaactgcgacgtcgtgatcg gcattgtgaacaataccgtgtacgaccctctgcagcccgagctggacagcttcaaagaggaactgg acaagtactttaagaaccacacaagccccgacgtggacctgggcgatatcagcggaatcaatgcca gcgtcgtgaacatccagaaagagatcgaccggctgaacgaggtggccaagaatctgaacgagagcc tgatcgacctgcaagaactggggaagtacgagcagtacatcaagtggccctggtacatctggctgg gctttatcgccggactgattgccatcgtgatggtcacaatcatgctgtgttgcatgaccagctgct gtagctgcctgaagggctgttgtagctgtggcagctgctgcaagttcgacgaggacgattctgagc ccgtgctgaagggcgtgaaactgcactacacatgatgactcgagctggtactgcatgcacgcaatg ctagctgcccctttcccgtcctgggtaccccgagtctcccccgacctcgggtcccaggtatgctcc cacctccacctgccccactcaccacctctgctagttccagacacctcccaagcacgcagcaatgca gctcaaaacgcttagcctagccacacccccacgggaaacagcagtgattaacctttagcaataaac gaaagtttaactaagctatactaaccccagggttggtcaatttcgtgccagccacaccctggagct agcaaaaaaaaaaaaaaaaaaaaaaaaaaaaaagcatatgactaaaaaaaaaaaaaaaaaaaaaa aaaaaaaaaaaaaaaaaaaaaaaaaaaaaaaaaaaaaaaaaaaaaa |

**Table 16. Sequence of one embodiment of an exemplary Omicron BQ.1.1-specific RNA vaccine**

| SEQ ID NO. | Brief Description | Sequence |
|---|---|---|
| 100 | Amino acid sequence of RNA-encoded SARS-CoV-2 S protein from an Omicron BQ.1.1 variant (with PRO mutations at | MFVFLVLLPLVSSQCVNLITRTQSYTNSFTRGVYYPDKVFRSSVLHSTQDLFLPFFSNVTWFHAIS GTNGTKRFDNPVLPFNDGVYFASTEKSNIIRGWIFGTTLDSKTQSLLIVNNATNVVIKVCEFQFCN DPFLDVYYHKNNKSWMESEFRVYSSANNCTFEYVSQPFLMDLEGKQGNFKNLREFVFKNIDGYFKI YSKHTPINLGRDLPQGFSALEPLVDLPIGINITRFQTLLALHRSYLTPGDSSSGWTAGAAAYYVGY LQPRTFLLKYNENGTITDAVDCALDPLSETKCTLKSFTVEKGIYQTSNFRVQPTESIVRFPNITNL CPFDEVFNATTFASVYAWNRKRISNCVADYSVLYNFAPFFAFKCYGVSPTKLNDLCFTNVYADSFV IRGNEVSQIAPGQTGNIADYNYKLPDDFTGCVIAWNSNKLDSTVGGNYNYRYRLFRKSKLKPFERD ISTEIYQAGNKPCNGVAGVNCYFPLQSYGFRPTYGVGHQPYRVVVLSFELLHAPATVCGPKKSTNL |
| | positions corresponding to K986P and V987P of SEQ ID NO: 1; i.e., PRO mutations at positions 981 and 982 of SEQ ID NO: 100) | VKNKCVNFNFNGLTGTGVLTESNKKFLPFQQFGRDIADTTDAVRDPQTLEILDITPCSFGGVSVIT PGTNTSNQVAVLYQGVNCTEVPVAIHADQLTPTWRVYSTGSNVFQTRAGCLIGAEYVNNSYECDIP IGAGICASYQTQTKSHRRARSVASQSIIAYTMSLGAENSVAYSNNSIAIPTNFTISVTTEILPVSM TKTSVDCTMYICGDSTECSNLLLQYGSFCTQLKRALTGIAVEQDKNTQEVFAQVKQIYKTPPIKYF GGFNFSQILPDPSKPSKRSFIEDLLFNKVTLADAGFIKQYGDCLGDIAARDLICAQKFNGLTVLPP LLTDEMIAQYTSALLAGTITSGWTFGAGAALQIPFAMQMAYRFNGIGVTQNVLYENQKLIANQFNS AIGKIQDSLSSTASALGKLQDVVNHNAQALNTLVKQLSSKFGAISSVLNDILSRLDPPEAEVQIDR LITGRLQSLQTYVTQQLIRAAEIRASANLAATKMSECVLGQSKRVDFCGKGYHLMSFPQSAPHGVV FLHVTYVPAQEKNFTTAPAICHDGKAHFPREGVFVSNGTHWFVTQRNFYEPQIITTDNTFVSGNCD VVIGIVNNTVYDPLQPELDSFKEELDKYFKNHTSPDVDLGDISGINASVVNIQKEIDRLNEVAKNL NESLIDLQELGKYEQYIKWPWYIWLGFIAGLIAIVMVTIMLCCMTSCCSCLKGCCSCGSCCKFDED DSEPVLKGVKLHYT |
| 101 | RNA sequence encoding a SARS-CoV-2 S protein from an Omicron BQ.1.1 variant | AUGUUCGUGUUCCUGGUGCUGCUGCCUCUGGUGUCCAGCCAGUGUGUGAACCUGAUCACCAGAACA CAGUCAUACACCAACAGCUUUACCAGAGGCGUGUACUACCCCGACAAGGUGUUCAGAUCCAGCGUG CUGCACUCUACCCAGGACCUGUUCCUGCCUUUCUUCAGCAACGUGACCUGGUUCCACGCCAUCUCC GGCACCAAUGGCACCAAGAGAUUCGACAACCCCGUGCUGCCCUUCAACGACGGGGUGUACUUUGCC AGCACCGAGAAGUCCAACAUCAUCAGAGGCUGGAUCUUCGGCACCACACUGGACAGCAAGACCCAG AGCCUGCUGAUCGUGAACAACGCCACCAACGUGGUCAUCAAAGUGUGCGAGUUCCAGUUCUGCAAC GACCCCUUCCUGGACGUCUACUACCACAAGAACAACAAGAGCUGGAUGGAAAGCGAGUUCCGGGUG UACAGCAGCGCCAACAACUGCACCUUCGAGUACGUGUCCCAGCCUUUCCUGAUGGACCUGGAAGGC AAGCAGGGCAACUUCAAGAACCUGCGCGAGUUCGUGUUUAAGAACAUCGACGGCUACUUCAAGAUC UACAGCAAGCACACCCCUAUCAACCUCGGCCGGGAUCUGCCUCAGGGCUUCUCUGCUCUGGAACCC CUGGUGGAUCUGCCCAUCGGCAUCAACAUCACCCGGUUUCAGACACUGCUGGCCCUGCACAGAAGC UACCUGACACCUGGCGAUAGCAGCAGCGGAUGGACAGCUGGUGCCGCCGCUUACUAUGUGGGCUAC CUGCAGCCUAGAACCUUCCUGCUGAAGUACAACGAGAACGGCACCAUCACCGACGCCGUGGAUUGU GCUCUGGAUCCUCUGAGCGAGACAAAGUGCACCCUGAAGUCCUUCACCGUGGAAAAGGGCAUCUAC CAGACCAGCAACUUCCGGGUGCAGCCCACCGAAUCCAUCGUGCGGUUCCCCAAUAUCACCAAUCUG UGCCCCUUCGACGAGGUGUUCAAUGCCACCACCUUCGCCUCUGUGUACGCCUGGAACCGGAAGCGG AUCAGCAAUUGCGUGGCCGACUACUCCGUGCUGUACAACUUCGCCCCCUUCUUCGCAUUCAAGUGC UACGGCGUGUCCCCUACCAAGCUGAACGACCUGUGCUUCACAAACGUGUACGCCGACAGCUUCGUG AUCCGGGGAAACGAAGUGUCACAGAUUGCCCCUGGACAGACAGGCAACAUCGCCGACUACAACUAC AAGCUGCCCGACGACUUCACCGGCUGUGUGAUUGCCUGGAACAGCAACAAGCUGGACUCCACCGUC GGCGGCAACUACAAUUACAGGUACCGGCUGUUCCGGAAGUCCAAGCUGAAGCCCUUCGAGCGGGAC AUCUCCACCGAGAUCUAUCAGGCCGGCAACAAGCCUUGUAACGGCGUGGCAGGCGUGAACUGCUAC UUCCCACUGCAGUCCUACGGCUUUAGGCCCACAUACGGCGUGGGCCACCAGCCCUACAGAGUGGUG GUGCUGAGCUUCGAACUGCUGCAUGCCCCUGCCACAGUGUGCGGCCCUAAGAAAAGCACCAAUCUC GUGAAGAACAAAUGCGUGAACUUCAACUUCAACGGCCUGACCGGCACCGGCGUGCUGACAGAGAGC AACAAGAAGUUCCUGCCAUUCCAGCAGUUUGGCCGGGAUAUCGCCGAUACCACAGACGCCGUUAGA GAUCCCCAGACACUGGAAAUCCUGGACAUCACCCCUUGCAGCUUCGGCGGAGUGUCUGUGAUCACC |
| | | CCUGGCACCAACACCAGCAAUCAGGUGGCAGUGCUGUACCAGGGCGUGAACUGUACCGAAGUGCCC GUGGCCAUUCACGCCGAUCAGCUGACACCUACAUGGCGGGUGUACUCCACCGGCAGCAAUGUGUUU CAGACCAGAGCCGGCUGUCUGAUCGGAGCCGAGUACGUGAACAAUAGCUACGAGUGCGACAUCCCC AUCGGCGCUGGAAUCUGCGCCAGCUACCAGACACAGACAAAGAGCCACCGGAGAGCCAGAAGCGUG GCCAGCCAGAGCAUCAUUGCCUACACAAUGUCUCUGGGCGCCGAGAACAGCGUGGCCUACUCCAAC AACUCUAUCGCUAUCCCCACCAACUUCACCAUCAGCGUGACCACAGAGAUCCUGCCUGUGUCCAUG ACCAAGACCAGCGUGGACUGCACCAUGUACAUCUGCGGCGAUUCCACCGAGUGCUCCAACCUGCUG CUGCAGUACGGCAGCUUCUGCACCCAGCUGAAAAGAGCCCUGACAGGGAUCGCCGUGGAACAGGAC AAGAACACCCAAGAGGUGUUCGCCCAAGUGAAGCAGAUCUACAAGACCCCUCCUAUCAAGUACUUC GGCGGCUUCAAUUUCAGCCAGAUUCUGCCCGAUCCUAGCAAGCCCAGCAAGCGGAGCUUCAUCGAG GACCUGCUGUUCAACAAAGUGACACUGGCCGACGCCGGCUUCAUCAAGCAGUAUGGCGAUUGUCUG GGCGACAUUGCCGCCAGGGAUCUGAUUUGCGCCCAGAAGUUUAACGGACUGACAGUGCUGCCUCCU CUGCUGACCGAUGAGAUGAUCGCCCAGUACACAUCUGCCCUGCUGGCCGGCACAAUCACAAGCGGC UGGACAUUUGGAGCAGGCGCCGCUCUGCAGAUCCCCUUUGCUAUGCAGAUGGCCUACCGGUUCAAC GGCAUCGGAGUGACCCAGAAUGUGCUGUACGAGAACCAGAAGCUGAUCGCCAACCAGUUCAACAGC GCCAUCGGCAAGAUCCAGGACAGCCUGAGCAGCACAGCAAGCGCCCUGGGAAAGCUGCAGGACGUG GUCAACCACAAUGCCCAGGCACUGAACACCCUGGUCAAGCAGCUGUCCUCCAAGUUCGGCGCCAUC AGCUCUGUGCUGAACGAUAUCCUGAGCAGACUGGACCCUCCUGAGGCCGAGGUGCAGAUCGACAGA CUGAUCACAGGCAGACUGCAGAGCCUCCAGACAUACGUGACCCAGCAGCUGAUCAGAGCCGCCGAG AUUAGAGCCUCUGCCAAUCUGGCCGCCACCAAGAUGUCUGAGUGUGUGCUGGGCCAGAGCAAGAGA GUGGACUUUUGCGGCAAGGGCUACCACCUGAUGAGCUUCCCUCAGUCUGCCCCUCACGGCGUGGUG UUUCUGCACGUGACAUAUGUGCCCGCUCAAGAGAAGAAUUUCACCACCGCUCCAGCCAUCUGCCAC GACGGCAAAGCCCACUUUCCUAGAGAAGGCGUGUUCGUGUCCAACGGCACCCAUUGGUUCGUGACA CAGCGGAACUUCUACGAGCCCCAGAUCAUCACCACCGACAACACCUUCGUGUCUGGCAACUGCGAC GUCGUGAUCGGCAUUGUGAACAAUACCGUGUACGACCCUCUGCAGCCCGAGCUGGACAGCUUCAAA GAGGAACUGGACAAGUACUUUAAGAACCACACAAGCCCCGACGUGGACCUGGGCGAUAUCAGCGGA AUCAAUGCCAGCGUCGUGAACAUCCAGAAAGAGAUCGACCGGCUGAACGAGGUGGCCAAGAAUCUG AACGAGAGCCUGAUCGACCUGCAAGAACUGGGGAAGUACGAGCAGUACAUCAAGUGGCCCUGGUAC AUCUGGCUGGGCUUUAUCGCCGGACUGAUUGCCAUCGUGAUGGUCACAAUCAUGCUGUGUUGCAUG ACCAGCUGCUGUAGCUGCCUGAAGGGCUGUUGUAGCUGUGGCAGCUGCUGCAAGUUCGACGAGGAC GAUUCUGAGCCCGUGCUGAAGGGCGUGAAACUGCACUACACAUGAUGA |
| 102 | DNA sequence encoding a SARS-CoV-2 S protein from an Omicron BQ.1.1 variant | ATGTTCGTGTTCCTGGTGCTGCTGCCTCTGGTGTCCAGCCAGTGTGTGAACCTGATCACCAGAACA CAGTCATACACCAACAGCTTTACCAGAGGCGTGTACTACCCCGACAAGGTGTTCAGATCCAGCGTG CTGCACTCTACCCAGGACCTGTTCCTGCCTTTCTTCAGCAACGTGACCTGGTTCCACGCCATCTCC GGCACCAATGGCACCAAGAGATTCGACAACCCCGTGCTGCCCTTCAACGACGGGGTGTACTTTGCC AGCACCGAGAAGTCCAACATCATCAGAGGCTGGATCTTCGGCACCACACTGGACAGCAAGACCCAG AGCCTGCTGATCGTGAACAACGCCACCAACGTGGTCATCAAAGTGTGCGAGTTCCAGTTCTGCAAC GACCCCTTCCTGGACGTCTACTACCACAAGAACAACAAGAGCTGGATGGAAAGCGAGTTCCGGGTG TACAGCAGCGCCAACAACTGCACCTTCGAGTACGTGTCCCAGCCTTTCCTGATGGACCTGGAAGGC |
| | | AAGCAGGGCAACTTCAAGAACCTGCGCGAGTTCGTGTTTAAGAACATCGACGGCTACTTCAAGATC TACAGCAAGCACACCCCTATCAACCTCGGCCGGGATCTGCCTCAGGGCTTCTCTGCTCTGGAACCC CTGGTGGATCTGCCCATCGGCATCAACATCACCCGGTTTCAGACACTGCTGGCCCTGCACAGAAGC TACCTGACACCTGGCGATAGCAGCAGCGGATGGACAGCTGGTGCCGCCGCTTACTATGTGGGCTAC CTGCAGCCTAGAACCTTCCTGCTGAAGTACAACGAGAACGGCACCATCACCGACGCCGTGGATTGT GCTCTGGATCCTCTGAGCGAGACAAAGTGCACCCTGAAGTCCTTCACCGTGGAAAAGGGCATCTAC CAGACCAGCAACTTCCGGGTGCAGCCCACCGAATCCATCGTGCGGTTCCCCAATATCACCAATCTG TGCCCCTTCGACGAGGTGTTCAATGCCACCACCTTCGCCTCTGTGTACGCCTGGAACCGGAAGCGG ATCAGCAATTGCGTGGCCGACTACTCCGTGCTGTACAACTTCGCCCCCTTCTTCGCATTCAAGTGC TACGGCGTGTCCCCTACCAAGCTGAACGACCTGTGCTTCACAAACGTGTACGCCGACAGCTTCGTG ATCCGGGGAAACGAAGTGTCACAGATTGCCCCTGGACAGACAGGCAACATCGCCGACTACAACTAC AAGCTGCCCGACGACTTCACCGGCTGTGTGATTGCCTGGAACAGCAACAAGCTGGACTCCACCGTC GGCGGCAACTACAATTACAGGTACCGGCTGTTCCGGAAGTCCAAGCTGAAGCCCTTCGAGCGGGAC ATCTCCACCGAGATCTATCAGGCCGGCAACAAGCCTTGTAACGGCGTGGCAGGCGTGAACTGCTAC TTCCCACTGCAGTCCTACGGCTTTAGGCCCACATACGGCGTGGGCCACCAGCCCTACAGAGTGGTG GTGCTGAGCTTCGAACTGCTGCATGCCCCTGCCACAGTGTGCGGCCCTAAGAAAAGCACCAATCTC GTGAAGAACAAATGCGTGAACTTCAACTTCAACGGCCTGACCGGCACCGGCGTGCTGACAGAGAGC AACAAGAAGTTCCTGCCATTCCAGCAGTTTGGCCGGGATATCGCCGATACCACAGACGCCGTTAGA GATCCCCAGACACTGGAAATCCTGGACATCACCCCTTGCAGCTTCGGCGGAGTGTCTGTGATCACC CCTGGCACCAACACCAGCAATCAGGTGGCAGTGCTGTACCAGGGCGTGAACTGTACCGAAGTGCCC GTGGCCATTCACGCCGATCAGCTGACACCTACATGGCGGGTGTACTCCACCGGCAGCAATGTGTTT CAGACCAGAGCCGGCTGTCTGATCGGAGCCGAGTACGTGAACAATAGCTACGAGTGCGACATCCCC ATCGGCGCTGGAATCTGCGCCAGCTACCAGACACAGACAAAGAGCCACCGGAGAGCCAGAAGCGTG GCCAGCCAGAGCATCATTGCCTACACAATGTCTCTGGGCGCCGAGAACAGCGTGGCCTACTCCAAC AACTCTATCGCTATCCCCACCAACTTCACCATCAGCGTGACCACAGAGATCCTGCCTGTGTCCATG ACCAAGACCAGCGTGGACTGCACCATGTACATCTGCGGCGATTCCACCGAGTGCTCCAACCTGCTG CTGCAGTACGGCAGCTTCTGCACCCAGCTGAAAAGAGCCCTGACAGGGATCGCCGTGGAACAGGAC AAGAACACCCAAGAGGTGTTCGCCCAAGTGAAGCAGATCTACAAGACCCCTCCTATCAAGTACTTC GGCGGCTTCAATTTCAGCCAGATTCTGCCCGATCCTAGCAAGCCCAGCAAGCGGAGCTTCATCGAG GACCTGCTGTTCAACAAAGTGACACTGGCCGACGCCGGCTTCATCAAGCAGTATGGCGATTGTCTG GGCGACATTGCCGCCAGGGATCTGATTTGCGCCCAGAAGTTTAACGGACTGACAGTGCTGCCTCCT CTGCTGACCGATGAGATGATCGCCCAGTACACATCTGCCCTGCTGGCCGGCACAATCACAAGCGGC TGGACATTTGGAGCAGGCGCCGCTCTGCAGATCCCCTTTGCTATGCAGATGGCCTACCGGTTCAAC GGCATCGGAGTGACCCAGAATGTGCTGTACGAGAACCAGAAGCTGATCGCCAACCAGTTCAACAGC GCCATCGGCAAGATCCAGGACAGCCTGAGCAGCACAGCAAGCGCCCTGGGAAAGCTGCAGGACGTG GTCAACCACAATGCCCAGGCACTGAACACCCTGGTCAAGCAGCTGTCCTCCAAGTTCGGCGCCATC AGCTCTGTGCTGAACGATATCCTGAGCAGACTGGACCCTCCTGAGGCCGAGGTGCAGATCGACAGA CTGATCACAGGCAGACTGCAGAGCCTCCAGACATACGTGACCCAGCAGCTGATCAGAGCCGCCGAG ATTAGAGCCTCTGCCAATCTGGCCGCCACCAAGATGTCTGAGTGTGTGCTGGGCCAGAGCAAGAGA GTGGACTTTTGCGGCAAGGGCTACCACCTGATGAGCTTCCCTCAGTCTGCCCCTCACGGCGTGGTG |
| | | TTTCTGCACGTGACATATGTGCCCGCTCAAGAGAAGAATTTCACCACCGCTCCAGCCATCTGCCAC GACGGCAAAGCCCACTTTCCTAGAGAAGGCGTGTTCGTGTCCAACGGCACCCATTGGTTCGTGACA CAGCGGAACTTCTACGAGCCCCAGATCATCACCACCGACAACACCTTCGTGTCTGGCAACTGCGAC GTCGTGATCGGCATTGTGAACAATACCGTGTACGACCCTCTGCAGCCCGAGCTGGACAGCTTCAAA GAGGAACTGGACAAGTACTTTAAGAACCACACAAGCCCCGACGTGGACCTGGGCGATATCAGCGGA ATCAATGCCAGCGTCGTGAACATCCAGAAAGAGATCGACCGGCTGAACGAGGTGGCCAAGAATCTG AACGAGAGCCTGATCGACCTGCAAGAACTGGGGAAGTACGAGCAGTACATCAAGTGGCCCTGGTAC ATCTGGCTGGGCTTTATCGCCGGACTGATTGCCATCGTGATGGTCACAATCATGCTGTGTTGCATG ACCAGCTGCTGTAGCTGCCTGAAGGGCTGTTGTAGCTGTGGCAGCTGCTGCAAGTTCGACGAGGAC GATTCTGAGCCCGTGCTGAAGGGCGTGAAACTGCACTACACATGATGA |
| 103 | Full length RNA construct encoding a SARS-CoV-2 S protein from an Omicron BQ.1.1 variant | AgaauaaacuaguauucuucugguccccacagacucagagagaacccgccaccAUGUUCGUGUUCC UGGUGCUGCUGCCUCUGGUGUCCAGCCAGUGUGUGAACCUGAUCACCAGAACACAGUCAUACACCA ACAGCUUUACCAGAGGCGUGUACUACCCCGACAAGGUGUUCAGAUCCAGCGUGCUGCACUCUACCC AGGACCUGUUCCUGCCUUUCUUCAGCAACGUGACCUGGUUCCACGCCAUCUCCGGCACCAAUGGCA CCAAGAGAUUCGACAACCCCGUGCUGCCCUUCAACGACGGGGUGUACUUUGCCAGCACCGAGAAGU CCAACAUCAUCAGAGGCUGGAUCUUCGGCACCACACUGGACAGCAAGACCCAGAGCCUGCUGAUCG UGAACAACGCCACCAACGUGGUCAUCAAAGUGUGCGAGUUCCAGUUCUGCAACGACCCCUUCCUGG ACGUCUACUACCACAAGAACAACAAGAGCUGGAUGGAAAGCGAGUUCCGGGUGUACAGCAGCGCCA ACAACUGCACCUUCGAGUACGUGUCCCAGCCUUUCCUGAUGGACCUGGAAGGCAAGCAGGGCAACU UCAAGAACCUGCGCGAGUUCGUGUUUAAGAACAUCGACGGCUACUUCAAGAUCUACAGCAAGCACA CCCCUAUCAACCUCGGCCGGGAUCUGCCUCAGGGCUUCUCUGCUCUGGAACCCCUGGUGGAUCUGC CCAUCGGCAUCAACAUCACCCGGUUUCAGACACUGCUGGCCCUGCACAGAAGCUACCUGACACCUG GCGAUAGCAGCAGCGGAUGGACAGCUGGUGCCGCCGCUUACUAUGUGGGCUACCUGCAGCCUAGAA CCUUCCUGCUGAAGUACAACGAGAACGGCACCAUCACCGACGCCGUGGAUUGUGCUCUGGAUCCUC UGAGCGAGACAAAGUGCACCCUGAAGUCCUUCACCGUGGAAAAGGGCAUCUACCAGACCAGCAACU UCCGGGUGCAGCCCACCGAAUCCAUCGUGCGGUUCCCCAAUAUCACCAAUCUGUGCCCCUUCGACG AGGUGUUCAAUGCCACCACCUUCGCCUCUGUGUACGCCUGGAACCGGAAGCGGAUCAGCAAUUGCG UGGCCGACUACUCCGUGCUGUACAACUUCGCCCCCUUCUUCGCAUUCAAGUGCUACGGCGUGUCCC CUACCAAGCUGAACGACCUGUGCUUCACAAACGUGUACGCCGACAGCUUCGUGAUCCGGGGAAACG AAGUGUCACAGAUUGCCCCUGGACAGACAGGCAACAUCGCCGACUACAACUACAAGCUGCCCGACG ACUUCACCGGCUGUGUGAUUGCCUGGAACAGCAACAAGCUGGACUCCACCGUCGGCGGCAACUACA AUUACAGGUACCGGCUGUUCCGGAAGUCCAAGCUGAAGCCCUUCGAGCGGGACAUCUCCACCGAGA UCUAUCAGGCCGGCAACAAGCCUUGUAACGGCGUGGCAGGCGUGAACUGCUACUUCCCACUGCAGU CCUACGGCUUUAGGCCCACAUACGGCGUGGGCCACCAGCCCUACAGAGUGGUGGUGCUGAGCUUCG AACUGCUGCAUGCCCCUGCCACAGUGUGCGGCCCUAAGAAAAGCACCAAUCUCGUGAAGAACAAAU GCGUGAACUUCAACUUCAACGGCCUGACCGGCACCGGCGUGCUGACAGAGAGCAACAAGAAGUUCC UGCCAUUCCAGCAGUUUGGCCGGGAUAUCGCCGAUACCACAGACGCCGUUAGAGAUCCCCAGACAC UGGAAAUCCUGGACAUCACCCCUUGCAGCUUCGGCGGAGUGUCUGUGAUCACCCCUGGCACCAACA CCAGCAAUCAGGUGGCAGUGCUGUACCAGGGCGUGAACUGUACCGAAGUGCCCGUGGCCAUUCACG |
| | | CCGAUCAGCUGACACCUACAUGGCGGGUGUACUCCACCGGCAGCAAUGUGUUUCAGACCAGAGCCG GCUGUCUGAUCGGAGCCGAGUACGUGAACAAUAGCUACGAGUGCGACAUCCCCAUCGGCGCUGGAA UCUGCGCCAGCUACCAGACACAGACAAAGAGCCACCGGAGAGCCAGAAGCGUGGCCAGCCAGAGCA UCAUUGCCUACACAAUGUCUCUGGGCGCCGAGAACAGCGUGGCCUACUCCAACAACUCUAUCGCUA UCCCCACCAACUUCACCAUCAGCGUGACCACAGAGAUCCUGCCUGUGUCCAUGACCAAGACCAGCG UGGACUGCACCAUGUACAUCUGCGGCGAUUCCACCGAGUGCUCCAACCUGCUGCUGCAGUACGGCA GCUUCUGCACCCAGCUGAAAAGAGCCCUGACAGGGAUCGCCGUGGAACAGGACAAGAACACCCAAG AGGUGUUCGCCCAAGUGAAGCAGAUCUACAAGACCCCUCCUAUCAAGUACUUCGGCGGCUUCAAUU UCAGCCAGAUUCUGCCCGAUCCUAGCAAGCCCAGCAAGCGGAGCUUCAUCGAGGACCUGCUGUUCA ACAAAGUGACACUGGCCGACGCCGGCUUCAUCAAGCAGUAUGGCGAUUGUCUGGGCGACAUUGCCG CCAGGGAUCUGAUUUGCGCCCAGAAGUUUAACGGACUGACAGUGCUGCCUCCUCUGCUGACCGAUG AGAUGAUCGCCCAGUACACAUCUGCCCUGCUGGCCGGCACAAUCACAAGCGGCUGGACAUUUGGAG CAGGCGCCGCUCUGCAGAUCCCCUUUGCUAUGCAGAUGGCCUACCGGUUCAACGGCAUCGGAGUGA CCCAGAAUGUGCUGUACGAGAACCAGAAGCUGAUCGCCAACCAGUUCAACAGCGCCAUCGGCAAGA UCCAGGACAGCCUGAGCAGCACAGCAAGCGCCCUGGGAAAGCUGCAGGACGUGGUCAACCACAAUG CCCAGGCACUGAACACCCUGGUCAAGCAGCUGUCCUCCAAGUUCGGCGCCAUCAGCUCUGUGCUGA ACGAUAUCCUGAGCAGACUGGACCCUCCUGAGGCCGAGGUGCAGAUCGACAGACUGAUCACAGGCA GACUGCAGAGCCUCCAGACAUACGUGACCCAGCAGCUGAUCAGAGCCGCCGAGAUUAGAGCCUCUG CCAAUCUGGCCGCCACCAAGAUGUCUGAGUGUGUGCUGGGCCAGAGCAAGAGAGUGGACUUUUGCG GCAAGGGCUACCACCUGAUGAGCUUCCCUCAGUCUGCCCCUCACGGCGUGGUGUUUCUGCACGUGA CAUAUGUGCCCGCUCAAGAGAAGAAUUUCACCACCGCUCCAGCCAUCUGCCACGACGGCAAAGCCC ACUUUCCUAGAGAAGGCGUGUUCGUGUCCAACGGCACCCAUUGGUUCGUGACACAGCGGAACUUCU ACGAGCCCCAGAUCAUCACCACCGACAACACCUUCGUGUCUGGCAACUGCGACGUCGUGAUCGGCA UUGUGAACAAUACCGUGUACGACCCUCUGCAGCCCGAGCUGGACAGCUUCAAAGAGGAACUGGACA AGUACUUUAAGAACCACACAAGCCCCGACGUGGACCUGGGCGAUAUCAGCGGAAUCAAUGCCAGCG UCGUGAACAUCCAGAAAGAGAUCGACCGGCUGAACGAGGUGGCCAAGAAUCUGAACGAGAGCCUGA UCGACCUGCAAGAACUGGGGAAGUACGAGCAGUACAUCAAGUGGCCCUGGUACAUCUGGCUGGGCU UUAUCGCCGGACUGAUUGCCAUCGUGAUGGUCACAAUCAUGCUGUGUUGCAUGACCAGCUGCUGUA GCUGCCUGAAGGGCUGUUGUAGCUGUGGCAGCUGCUGCAAGUUCGACGAGGACGAUUCUGAGCCCG UGCUGAAGGGCGUGAAACUGCACUACACAUGAUGAcucgagcugguacugcaugcacgcaaugcua gcugccccuuucccguccuggguaccccgagucucccccgaccucgggucccagguaugcucccac cuccaccugccccacucaccaccucugcuaguuccagacaccucccaagcacgcagcaaugcagcu caaaacgcuuagccuagccacacccccacgggaaacagcagugauuaaccuuuagcaauaaacgaa aguuuaacuaagcuauacuaaccccaggguuggucaauuucgugccagccacacccuggagcuagc aaaaaaaaaaaaaaaaaaaaaaaaaaaaaagcaugacuaaaaaaaaaaaaaaaaaaaaaaaaaa aaaaaaaaaaaaaaaaaaaaaaaaaaaaaaaaaaaaaaaa |
| 104 | Full length DNA construct encoding a SARS-CoV-2 S | AgaataaactagtattcttctggtccccacagactcagagagaacccgccaccATGTTCGTGTTCC TGGTGCTGCTGCCTCTGGTGTCCAGCCAGTGTGTGAACCTGATCACCAGAACACAGTCATACACCA ACAGCTTTACCAGAGGCGTGTACTACCCCGACAAGGTGTTCAGATCCAGCGTGCTGCACTCTACCC |
| | protein from an Omicron BQ.1.1 variant | AGGACCTGTTCCTGCCTTTCTTCAGCAACGTGACCTGGTTCCACGCCATCTCCGGCACCAATGGCA CCAAGAGATTCGACAACCCCGTGCTGCCCTTCAACGACGGGGTGTACTTTGCCAGCACCGAGAAGT CCAACATCATCAGAGGCTGGATCTTCGGCACCACACTGGACAGCAAGACCCAGAGCCTGCTGATCG TGAACAACGCCACCAACGTGGTCATCAAAGTGTGCGAGTTCCAGTTCTGCAACGACCCCTTCCTGG ACGTCTACTACCACAAGAACAACAAGAGCTGGATGGAAAGCGAGTTCCGGGTGTACAGCAGCGCCA ACAACTGCACCTTCGAGTACGTGTCCCAGCCTTTCCTGATGGACCTGGAAGGCAAGCAGGGCAACT TCAAGAACCTGCGCGAGTTCGTGTTTAAGAACATCGACGGCTACTTCAAGATCTACAGCAAGCACA CCCCTATCAACCTCGGCCGGGATCTGCCTCAGGGCTTCTCTGCTCTGGAACCCCTGGTGGATCTGC CCATCGGCATCAACATCACCCGGTTTCAGACACTGCTGGCCCTGCACAGAAGCTACCTGACACCTG GCGATAGCAGCAGCGGATGGACAGCTGGTGCCGCCGCTTACTATGTGGGCTACCTGCAGCCTAGAA CCTTCCTGCTGAAGTACAACGAGAACGGCACCATCACCGACGCCGTGGATTGTGCTCTGGATCCTC TGAGCGAGACAAAGTGCACCCTGAAGTCCTTCACCGTGGAAAAGGGCATCTACCAGACCAGCAACT TCCGGGTGCAGCCCACCGAATCCATCGTGCGGTTCCCCAATATCACCAATCTGTGCCCCTTCGACG AGGTGTTCAATGCCACCACCTTCGCCTCTGTGTACGCCTGGAACCGGAAGCGGATCAGCAATTGCG TGGCCGACTACTCCGTGCTGTACAACTTCGCCCCCTTCTTCGCATTCAAGTGCTACGGCGTGTCCC CTACCAAGCTGAACGACCTGTGCTTCACAAACGTGTACGCCGACAGCTTCGTGATCCGGGGAAACG AAGTGTCACAGATTGCCCCTGGACAGACAGGCAACATCGCCGACTACAACTACAAGCTGCCCGACG ACTTCACCGGCTGTGTGATTGCCTGGAACAGCAACAAGCTGGACTCCACCGTCGGCGGCAACTACA ATTACAGGTACCGGCTGTTCCGGAAGTCCAAGCTGAAGCCCTTCGAGCGGGACATCTCCACCGAGA TCTATCAGGCCGGCAACAAGCCTTGTAACGGCGTGGCAGGCGTGAACTGCTACTTCCCACTGCAGT CCTACGGCTTTAGGCCCACATACGGCGTGGGCCACCAGCCCTACAGAGTGGTGGTGCTGAGCTTCG AACTGCTGCATGCCCCTGCCACAGTGTGCGGCCCTAAGAAAAGCACCAATCTCGTGAAGAACAAAT GCGTGAACTTCAACTTCAACGGCCTGACCGGCACCGGCGTGCTGACAGAGAGCAACAAGAAGTTCC TGCCATTCCAGCAGTTTGGCCGGGATATCGCCGATACCACAGACGCCGTTAGAGATCCCCAGACAC TGGAAATCCTGGACATCACCCCTTGCAGCTTCGGCGGAGTGTCTGTGATCACCCCTGGCACCAACA CCAGCAATCAGGTGGCAGTGCTGTACCAGGGCGTGAACTGTACCGAAGTGCCCGTGGCCATTCACG CCGATCAGCTGACACCTACATGGCGGGTGTACTCCACCGGCAGCAATGTGTTTCAGACCAGAGCCG GCTGTCTGATCGGAGCCGAGTACGTGAACAATAGCTACGAGTGCGACATCCCCATCGGCGCTGGAA TCTGCGCCAGCTACCAGACACAGACAAAGAGCCACCGGAGAGCCAGAAGCGTGGCCAGCCAGAGCA TCATTGCCTACACAATGTCTCTGGGCGCCGAGAACAGCGTGGCCTACTCCAACAACTCTATCGCTA TCCCCACCAACTTCACCATCAGCGTGACCACAGAGATCCTGCCTGTGTCCATGACCAAGACCAGCG TGGACTGCACCATGTACATCTGCGGCGATTCCACCGAGTGCTCCAACCTGCTGCTGCAGTACGGCA GCTTCTGCACCCAGCTGAAAAGAGCCCTGACAGGGATCGCCGTGGAACAGGACAAGAACACCCAAG AGGTGTTCGCCCAAGTGAAGCAGATCTACAAGACCCCTCCTATCAAGTACTTCGGCGGCTTCAATT TCAGCCAGATTCTGCCCGATCCTAGCAAGCCCAGCAAGCGGAGCTTCATCGAGGACCTGCTGTTCA ACAAAGTGACACTGGCCGACGCCGGCTTCATCAAGCAGTATGGCGATTGTCTGGGCGACATTGCCG CCAGGGATCTGATTTGCGCCCAGAAGTTTAACGGACTGACAGTGCTGCCTCCTCTGCTGACCGATG AGATGATCGCCCAGTACACATCTGCCCTGCTGGCCGGCACAATCACAAGCGGCTGGACATTTGGAG CAGGCGCCGCTCTGCAGATCCCCTTTGCTATGCAGATGGCCTACCGGTTCAACGGCATCGGAGTGA CCCAGAATGTGCTGTACGAGAACCAGAAGCTGATCGCCAACCAGTTCAACAGCGCCATCGGCAAGA |
| | | TCCAGGACAGCCTGAGCAGCACAGCAAGCGCCCTGGGAAAGCTGCAGGACGTGGTCAACCACAATG CCCAGGCACTGAACACCCTGGTCAAGCAGCTGTCCTCCAAGTTCGGCGCCATCAGCTCTGTGCTGA ACGATATCCTGAGCAGACTGGACCCTCCTGAGGCCGAGGTGCAGATCGACAGACTGATCACAGGCA GACTGCAGAGCCTCCAGACATACGTGACCCAGCAGCTGATCAGAGCCGCCGAGATTAGAGCCTCTG CCAATCTGGCCGCCACCAAGATGTCTGAGTGTGTGCTGGGCCAGAGCAAGAGAGTGGACTTTTGCG GCAAGGGCTACCACCTGATGAGCTTCCCTCAGTCTGCCCCTCACGGCGTGGTGTTTCTGCACGTGA CATATGTGCCCGCTCAAGAGAAGAATTTCACCACCGCTCCAGCCATCTGCCACGACGGCAAAGCCC ACTTTCCTAGAGAAGGCGTGTTCGTGTCCAACGGCACCCATTGGTTCGTGACACAGCGGAACTTCT ACGAGCCCCAGATCATCACCACCGACAACACCTTCGTGTCTGGCAACTGCGACGTCGTGATCGGCA TTGTGAACAATACCGTGTACGACCCTCTGCAGCCCGAGCTGGACAGCTTCAAAGAGGAACTGGACA AGTACTTTAAGAACCACACAAGCCCCGACGTGGACCTGGGCGATATCAGCGGAATCAATGCCAGCG TCGTGAACATCCAGAAAGAGATCGACCGGCTGAACGAGGTGGCCAAGAATCTGAACGAGAGCCTGA TCGACCTGCAAGAACTGGGGAAGTACGAGCAGTACATCAAGTGGCCCTGGTACATCTGGCTGGGCT TTATCGCCGGACTGATTGCCATCGTGATGGTCACAATCATGCTGTGTTGCATGACCAGCTGCTGTA GCTGCCTGAAGGGCTGTTGTAGCTGTGGCAGCTGCTGCAAGTTCGACGAGGACGATTCTGAGCCCG TGCTGAAGGGCGTGAAACTGCACTACACATGATGActcgagctggtactgcatgcacgcaatgcta gctgcccctttcccgtcctgggtaccccgagtctcccccgacctcgggtcccaggtatgctcccac ctccacctgccccactcaccacctctgctagttccagacacctcccaagcacgcagcaatgcagct caaaacgcttagcctagccacacccccacgggaaacagcagtgattaacctttagcaataaacgaa agtttaactaagctatactaaccccagggttggtcaatttcgtgccagccacaccctggagctagc aaaaaaaaaaaaaaaaaaaaaaaaaaaaaaagcatatgactaaaaaaaaaaaaaaaaaaaaaaaaaa aaaaaaaaaaaaaaaaaaaaaaaaaaaaaaaaaaaaaaaa |

Tables 3-16 show amino acid sequences of SARS-CoV-2 S proteins encoded by RNAs described herein from different variants with 2 proline substitutions at positions corresponding to K986P and V987P of SEQ ID NO: 1. In some embodiments, an RNA described herein encodes a SARS-CoV-2 S protein as described herein, for example, in some embodiments, as described in Tables 3-16, without the 2 proline substitutions at positions corresponding to K986P and V987P of SEQ ID NO: 1. In some embodiments, such an RNA described herein encodes a SARS-CoV-2 S protein comprising one or more mutations characteristic of a variant described herein, and further comprising at least four (including, e.g., at least five, at least six, or more) proline mutations. In some embodiments, at least four of such proline mutations include mutations at positions corresponding to residues 817, 892, 899, and 942 of SEQ ID NO: 1, e.g., as described in WO 2021243122 A2, the entire contents of which are incorporated herein by reference in its entirety. In some embodiments, such a SARS-CoV-2 S protein comprising proline substitutions at positions corresponding to residues 817, 892, 899, and 942 of SEQ ID NO: 1, may further comprise proline substitutions at positions corresponding to residues 986 and 987 of SEQ ID NO: 1.

### Self-amplifying RNA (saRNA)

The active principle of a self-amplifying RNA (saRNA) drug substance is a single-stranded RNA, which self-amplifies upon entering a cell, and a coronavirus vaccine antigen is translated thereafter. In contrast to uRNA and modRNA that preferably code for a single protein, the coding region of saRNA contains two open reading frames (ORFs). The 5'-ORF encodes an RNA-dependent RNA polymerase such as Venezuelan equine encephalitis virus (VEEV) RNA-dependent RNA polymerase (replicase). The replicase ORF is followed 3' by a subgenomic promoter and a second ORF encoding an antigen. Furthermore, saRNA UTRs contain 5' and 3' conserved sequence elements (CSEs) required for self-amplification. The saRNA contains common structural elements optimized for maximal efficacy of the RNA as the uRNA (including, e.g., 5'-cap, 5'-UTR, 3'-UTR, poly(A)-tail). In some embodiments, the saRNA preferably contains uridine. In some embodiments, the saRNA comprises one or more nucleoside modifications as described herein. The preferred 5' cap structure is beta-S-ARCA(D1) (m₂^{7,2'-O}GppSpG).

Cytoplasmic delivery of saRNA initiates an alphavirus-like life cycle. However, saRNA does not encode for alphaviral structural proteins required for genome packaging or cell entry, therefore generation of replication competent viral particles is very unlikely or not possible. Replication does not involve any intermediate steps that generate DNA. The use/uptake of saRNA therefore poses no risk of genomic integration or other permanent genetic modification within the target cell. Furthermore, the saRNA itself prevents its persistent replication by effectively activating innate immune response via recognition of dsRNA intermediates.

Different embodiments of this platform are as follows:
**RBS004.1 (SEQ ID NO: 24; SEQ ID NO: 7)**

| | |
|---|---|
| Structure | beta-S-ARCA(D1)-replicase-S1S2-PP-FI-A30L70 |
| Encoded | antigen Viral spike protein (S protein) of the SARS-CoV-2 (S1S2 full-length protein, sequence variant) |

**RBS004.2 (SEQ ID NO: 25; SEQ ID NO: 7)**

| | |
|---|---|
| Structure | beta-S-ARCA(D1)-replicase-S1S2-PP-FI-A30L70 |
| Encoded | antigen Viral spike protein (S protein) of the SARS-CoV-2 (S1S2 full-length protein, sequence variant) |

**BNT162c1; RBS004.3 (SEQ ID NO: 26; SEQ ID NO: 5)**

| | |
|---|---|
| Structure | beta-S-ARCA(D1)-replicase-RBD-GS-Fibritin-FI-A30L70 |
| Encoded antigen | Viral spike protein (S protein) of the SARS-CoV-2 (partial sequence, Receptor Binding Domain (RBD) of S1S2 protein) |

Receptor Binding Domain (RBD) of S1S2 protein)
**RBS004.4 (SEQ ID NO: 27; SEQ ID NO: 28)**

| | |
|---|---|
| Structure | beta-S-ARCA(D1)-replicase-RBD-GS-Fibritin-TM-FI-A30L70 |
| Encoded antigen | Viral spike protein (S protein) of the SARS-CoV-2 (partial sequence, Receptor Binding Domain (RBD) of S1S2 protein) |

Receptor Binding Domain (RBD) of S1S2 protein)

Figure 5 schematizes the general structure of the antigen-encoding RNAs.

In some embodiments, vaccine RNA described herein comprises a nucleotide sequence selected from the group consisting of SEQ ID NO: 15, 16, 17, 19, 20, 21, 24, 25, 26, 27, 30, and 32. A particularly preferred vaccine RNA described herein comprises a nucleotide sequence selected from the group consisting of SEQ ID NO: 15, 17, 19, 21, 25, 26, 30, and 32 such as selected from the group consisting of SEQ ID NO: 17, 19, 21, 26, 30, and 32.

In some embodiments, RNA described herein is formulated in lipid nanoparticles, lipoplex, polyplexes (PLX), lipidated polyplexes (LPLX), liposomes, or polysaccharide nanoparticles. In some embodiments, RNA described herein is preferably formulated in lipid nanoparticles (LNP). In one embodiment, LNP comprise a cationic lipid, a neutral lipid, a steroid, a polymer conjugated lipid; and RNA. In one embodiment, the cationic lipid is ALC-0315, the neutral lipid is DSPC, the steroid is cholesterol, and the polymer conjugated lipid is ALC-0159. The preferred mode of administration is intramuscular administration, more preferably in aqueous cryoprotectant buffer for intramuscular administration. Drug product is a preferably a preservative-free, sterile dispersion of RNA formulated in lipid nanoparticles (LNP) in aqueous cryoprotectant buffer for intramuscular administration.

In different embodiments, drug product comprises the components shown below, preferably at the proportions or concentrations shown below:

| **Component** | **Function** | **Proportion (mol%)** |
|---|---|---|
| ALC-0315 | _{[1]} Functional lipid | 47.5 |
| ALC-0159 | _{[2]} Functional lipid | 1.8 |
| DSPC | _{[3]} Structural lipid | 10.0 |
| Cholesterol, | synthetic Structural lipid | 40.7 |

| **Component** | **Function** | **Concentration (mg/mL)** |
|---|---|---|
| Drug | Substance Active | 0.5 |
| ALC-0315 | [1] Functional lipid | 7.17 |
| ALC-0159 | [2] Functional lipid | 0.89 |
| DSPC | [3] Structural lipid | 1.56 |
| Cholesterol, | synthetic Structural lipid | 3.1 |
| Sucrose | Cryoprotectant | 102.69 |
| NaCl | Buffer | 6.0 |
| KCI | Buffer | 0.15 |
| Na₂HPO₄ | Buffer | 1.08 |
| KH₂PO₄ | Buffer | 0.18 |
| Water | for injection Solvent/Vehicle | q.s. |

| **Component** | **Function** | **Concentration (mg/mL)** |
|---|---|---|
| Drug | Substance Active | 1.0 |
| ALC-0315 | _{[1]} Functional lipid | 13.56 |
| ALC-0159 | _{[2]} Functional lipid | 1.77 |
| DSPC | _{[3]} Structural lipid | 3.11 |
| Cholesterol, | synthetic Structural lipid | 6.20 |
| Sucrose | Cryoprotectant | 102.69 |
| NaCl | Buffer | 6.0 |
| KCI | Buffer | 0.15 |
| Na₂HPO₄ | Buffer | 1.08 |
| KH₂PO₄ | Buffer | 0.15 |
| Water | for injection Solvent/Vehicle | q.s. |

| | | |
|---|---|---|
| _{[1]} ALC-0315 = ((4-hydroxybutyl)azanediyl)bis(hexane-6,1-diyl)bis(2-hexyldecanoate) / 6-[N-6-(2-hexyldecanoyloxy)hexyl-N-(4-hydroxybutyl)amino]hexyl 2-hexyldecanoate _{[2]} ALC-0159 = 2-[(polyethylene glycol)-2000]-*N*,*N*-ditetradecylacetamide / 2-[2-(ω-methoxy (polyethyleneglycol2000) ethoxy]-N,N-ditetradecylacetamide _{[3]} DSPC = 1,2-Distearoyl-sn-glycero-3-phosphocholine q.s. = quantum satis (as much as may suffice) | | |

### ALC-0315:

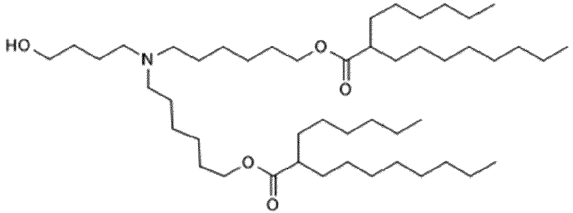

### ALC-0159:

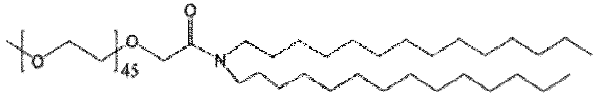

### DSPC:

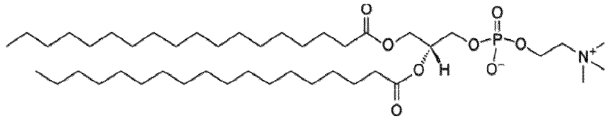

### Cholesterol:

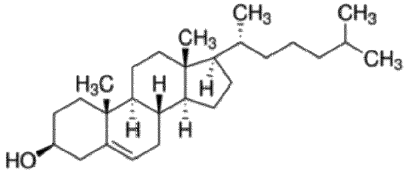

In some embodiments, particles disclosed herein are formulated in a solution comprising 10 mM Tris and 10% sucrose, and optionally having a pH of about 7.4. In some embodiments, particles disclosed herein are formulated in a solution comprising about 103 mg/ml sucrose, about 0.20 mg/ml tromethamine (Tris base), and about 1.32 mg/ml Tris.

In some embodiments, a composition comprises:
(a) about 0.1 mg/mL RNA comprising an open reading frame encoding a polypeptide that comprises a SARS-CoV-2 protein or an immunogenic fragment or variant thereof,
(b) about 1.43 mg/ml ALC-0315,
(c) about 0.18 mg/ml ALC-0159,
(d) about 0.31 mg/ml DSPC,
(e) about 0.62 mg/ml cholesterol,
(f) about 103 mg/ml sucrose,
(g) about 0.20 mg/ml tromethamine (Tris base),
(h) about 1.32 mg/ml Tris (hydroxymethyl) aminomethane hydrochloride (Tris HCl), and
(i) q.s. water.

In one embodiment, the ratio of RNA (e.g., mRNA) to total lipid (N/P) is between 6.0 and 6.5 such as about 6.0 or about 6.3.

### Nucleic acid containing particles

Nucleic acids described herein such as RNA encoding a vaccine antigen may be administered formulated as particles.

In the context of the present disclosure, the term "particle" relates to a structured entity formed by molecules or molecule complexes. In one embodiment, the term "particle" relates to a micro- or nano-sized structure, such as a micro- or nano-sized compact structure dispersed in a medium. In one embodiment, a particle is a nucleic acid containing particle such as a particle comprising DNA, RNA or a mixture thereof.

Electrostatic interactions between positively charged molecules such as polymers and lipids and negatively charged nucleic acid are involved in particle formation. This results in complexation and spontaneous formation of nucleic acid particles. In one embodiment, a nucleic acid particle is a nanoparticle.

As used in the present disclosure, "nanoparticle" refers to a particle having an average diameter suitable for parenteral administration.

A "nucleic acid particle" can be used to deliver nucleic acid to a target site of interest (e.g., cell, tissue, organ, and the like). A nucleic acid particle may be formed from at least one cationic or cationically ionizable lipid or lipid-like material, at least one cationic polymer such as protamine, or a mixture thereof and nucleic acid. In some embodiments, exemplary nanoparticles include lipid nanoparticles, polyplexes (PLX), lipidated polyplexes (LPLX), liposomes, or polysaccharide nanoparticles.

In some embodiments, the RNA encoding an amino acid sequence comprising a SARS-CoV-2 S protein, an immunogenic variant thereof, or an immunogenic fragment of the SARS-CoV-2 S protein or the immunogenic variant thereof is formulated as LNPs. In some embodiments, the LNPs comprise one or more cationically ionizable lipids; one or more neutral lipids (e.g., in some embodiments sterol such as, e.g., cholesterol; and/or phospholipids), and one or more polymer-conjugated lipids. In some embodiments, the formulation comprises ALC-0315 (4-hydroxybutyl)azanediyl)bis(hexane-6,1-diyl)bis(2-hexyldecanoate), ALC-0159 (2-[(polyethylene glycol)-2000]-N,N-ditetradecylacetamide), DSPC (1,2-distearoyl-sn-glycero-3-phosphocholine), cholesterol, sucrose, trometamol (Tris), trometamol hydrochloride and water.

RNA particles described herein include nanoparticles. In some embodiments, exemplary nanoparticles include lipid nanoparticles, lipoplex, polyplexes (PLX), lipidated polyplexes (LPLX), liposomes, or polysaccharide nanoparticles.

Polyplexes (PLX), polysaccharide nanoparticles, and liposomes, are all delivery technologies that are well known to a person of skill in the art. See, e.g., Lächelt, Ulrich, and Ernst Wagner. "Nucleic acid therapeutics using polyplexes: a journey of 50 years (and beyond)" Chemical reviews 115.19 (2015): 11043-11078; Plucinski, Alexander, Zan Lyu, and Bernhard VKJ Schmidt, "Polysaccharide nanoparticles: from fabrication to applications." Journal of Materials Chemistry B (2021); and Tenchov, Rumiana, et al. "Lipid Nanoparticles- From Liposomes to mRNA Vaccine Delivery, a Landscape of Research Diversity and Advancement," ACS nano 15.11 (2021): 16982-17015, respectively, the contents of each of which are hereby incorporated by reference herein in their entirety.

In some embodiments, the concentration of the RNA in the pharmaceutical RNA preparation is about 0.1 mg/ml. In some embodiments, the concentration of the RNA in the pharmaceutical RNA preparation is about 30 µg/ml to about 100 µg/ml. In some embodiments, the concentration of the RNA in the pharmaceutical RNA preparation is about 50 µg/ml to about 100 µg/ml.

Without intending to be bound by any theory, it is believed that the cationic or cationically ionizable lipid or lipid-like material and/or the cationic polymer combine together with the nucleic acid to form aggregates, and this aggregation results in colloidally stable particles.

In one embodiment, particles described herein further comprise at least one lipid or lipid-like material other than a cationic or cationically ionizable lipid or lipid-like material, at least one polymer other than a cationic polymer, or a mixture thereof

In some embodiments, nucleic acid particles comprise more than one type of nucleic acid molecules, where the molecular parameters of the nucleic acid molecules may be similar or different from each other, like with respect to molar mass or fundamental structural elements such as molecular architecture, capping, coding regions or other features.

Nucleic acid particles described herein may have an average diameter that in one embodiment ranges from about 30 nm to about 1000 nm, from about 50 nm to about 800 nm, from about 70 nm to about 600 nm, from about 90 nm to about 400 nm, or from about 100 nm to about 300 nm.

Nucleic acid particles described herein may exhibit a polydispersity index less than about 0.5, less than about 0.4, less than about 0.3, or about 0.2 or less. By way of example, the nucleic acid particles can exhibit a polydispersity index in a range of about 0.1 to about 0.3 or about 0.2 to about 0.3.

With respect to RNA lipid particles, the N/P ratio gives the ratio of the nitrogen groups in the lipid to the number of phosphate groups in the RNA. It is correlated to the charge ratio, as the nitrogen atoms (depending on the pH) are usually positively charged and the phosphate groups are negatively charged. The N/P ratio, where a charge equilibrium exists, depends on the pH. Lipid formulations are frequently formed at N/P ratios larger than four up to twelve, because positively charged nanoparticles are considered favorable for transfection. In that case, RNA is considered to be completely bound to nanoparticles.

Nucleic acid particles described herein can be prepared using a wide range of methods that may involve obtaining a colloid from at least one cationic or cationically ionizable lipid or lipid-like material and/or at least one cationic polymer and mixing the colloid with nucleic acid to obtain nucleic acid particles.

The term "colloid" as used herein relates to a type of homogeneous mixture in which dispersed particles do not settle out. The insoluble particles in the mixture are microscopic, with particle sizes between 1 and 1000 nanometers. The mixture may be termed a colloid or a colloidal suspension. Sometimes the term "colloid" only refers to the particles in the mixture and not the entire suspension.

For the preparation of colloids comprising at least one cationic or cationically ionizable lipid or lipid-like material and/or at least one cationic polymer methods are applicable herein that are conventionally used for preparing liposomal vesicles and are appropriately adapted. The most commonly used methods for preparing liposomal vesicles share the following fundamental stages: (i) lipids dissolution in organic solvents, (ii) drying of the resultant solution, and (iii) hydration of dried lipid (using various aqueous media).

In the film hydration method, lipids are firstly dissolved in a suitable organic solvent, and dried down to yield a thin film at the bottom of the flask. The obtained lipid film is hydrated using an appropriate aqueous medium to produce a liposomal dispersion. Furthermore, an additional downsizing step may be included.

Reverse phase evaporation is an alternative method to the film hydration for preparing liposomal vesicles that involves formation of a water-in-oil emulsion between an aqueous phase and an organic phase containing lipids. A brief sonication of this mixture is required for system homogenization. The removal of the organic phase under reduced pressure yields a milky gel that turns subsequently into a liposomal suspension.

The term "ethanol injection technique" refers to a process, in which an ethanol solution comprising lipids is rapidly injected into an aqueous solution through a needle. This action disperses the lipids throughout the solution and promotes lipid structure formation, for example lipid vesicle formation such as liposome formation. Generally, the RNA lipoplex particles described herein are obtainable by adding RNA to a colloidal liposome dispersion. Using the ethanol injection technique, such colloidal liposome dispersion is, in one embodiment, formed as follows: an ethanol solution comprising lipids, such as cationic lipids and additional lipids, is injected into an aqueous solution under stirring. In one embodiment, the RNA lipoplex particles described herein are obtainable without a step of extrusion.

The term "extruding" or "extrusion" refers to the creation of particles having a fixed, cross-sectional profile. In particular, it refers to the downsizing of a particle, whereby the particle is forced through filters with defined pores.

Other methods having organic solvent free characteristics may also be used according to the present disclosure for preparing a colloid.

LNPs typically comprise four components: ionizable cationic lipids, neutral lipids such as phospholipids, a steroid such as cholesterol, and a polymer conjugated lipid such as polyethylene glycol (PEG)-lipids. Each component is responsible for payload protection, and enables effective intracellular delivery. LNPs may be prepared by mixing lipids dissolved in ethanol rapidly with nucleic acid in an aqueous buffer.

The term "average diameter" refers to the mean hydrodynamic diameter of particles as measured by dynamic laser light scattering (DLS) with data analysis using the so-called cumulant algorithm, which provides as results the so-called Z_{average} with the dimension of a length, and the polydispersity index (PI), which is dimensionless (Koppel, D., J. Chem. Phys. 57, 1972, pp 4814-4820, ISO 13321). Here "average diameter", "diameter" or "size" for particles is used synonymously with this value of the Z_{average}.

The "polydispersity index" is preferably calculated based on dynamic light scattering measurements by the so-called cumulant analysis as mentioned in the definition of the "average diameter". Under certain prerequisites, it can be taken as a measure of the size distribution of an ensemble of nanoparticles.

Different types of nucleic acid containing particles have been described previously to be suitable for delivery of nucleic acid in particulate form (e.g. Kaczmarek, J. C. et al., 2017, Genome Medicine 9, 60). For non-viral nucleic acid delivery vehicles, nanoparticle encapsulation of nucleic acid physically protects nucleic acid from degradation and, depending on the specific chemistry, can aid in cellular uptake and endosomal escape.

The present disclosure describes particles comprising nucleic acid, at least one cationic or cationically ionizable lipid or lipid-like material, and/or at least one cationic polymer which associate with nucleic acid to form nucleic acid particles and compositions comprising such particles. The nucleic acid particles may comprise nucleic acid which is complexed in different forms by non-covalent interactions to the particle. The particles described herein are not viral particles, in particular infectious viral particles, i.e., they are not able to virally infect cells.

Suitable cationic or cationically ionizable lipids or lipid-like materials and cationic polymers are those that form nucleic acid particles and are included by the term "particle forming components" or "particle forming agents". The term "particle forming components" or "particle forming agents" relates to any components which associate with nucleic acid to form nucleic acid particles. Such components include any component which can be part of nucleic acid particles.

In some embodiments, a nucleic acid containing particle (e.g., a lipid nanoparticle (LNP)) comprises two or more RNA molecules, each comprising a different nucleic acid sequence. In some embodiments, a nucleic acid containing particle comprises two or more RNA molecules, each encoding a different immunogenic polypeptide or immunogenic fragment thereof. In some embodiments, two or more RNA molecules present in a nucleic acid containing particle comprise: a first RNA molecule encodes an immunogenic polypeptide or immunogenic fragment thereof from a coronavirus and a second RNA molecule encodes an immunogenic polypeptide or immunogenic fragment thereof from an infectious disease pathogen (e.g., virus, bacteria, parasite, etc.). For example, in some embodiments, two or more RNA molecules present in a nucleic acid containing particle comprise: a first RNA molecule encoding an immunogenic polypeptide or immunogenic fragment thereof from a coronavirus (e.g., in some embodiments SARS-CoV-2 Wuhan strain or a variant thereof, e.g., a SARS-CoV-2 having one or more mutations characteristic of an Omicron variant) and a second RNA molecule encoding an immunogenic polypeptide or immunogenic fragment thereof from an influenza virus. In some embodiments, two or more RNA molecules present in a nucleic acid containing particle comprise: a first RNA molecule encoding an immunogenic polypeptide or immunogenic fragment thereof from a first coronavirus (e.g., as described herein) and a second RNA molecule encoding an immunogenic polypeptide or immunogenic fragment thereof from a second coronavirus (e.g., as described herein). In some embodiments, a first coronavirus is different from a second coronavirus. In some embodiments, a first and/or second coronavirus is independently from a SARS-CoV-2 Wuhan strain or a variant thereof, e.g., a SARS-CoV-2 having one or more mutations characteristic of an Omicron variant.

In some embodiments, two or more RNA molecules present in a nucleic acid containing particle each encode an immunogenic polypeptide or an immunogenic fragment thereof from the same and/or different strains and/or variants of coronavirus (e.g., in some embodiments SARS-CoV-2 strains or variants). For example, in some embodiments, two or more RNA molecules present in a nucleic acid containing particle each encode a different immunogenic polypeptide or immunogenic fragment thereof from a coronavirus membrane protein, a coronavirus nucleocapsid protein, a coronavirus spike protein, a coronavirus non-structural protein and/or a coronavirus accessory protein. In some embodiments, such immunogenic polypeptides or immunogenic fragments thereof may be from the same or a different coronavirus (e.g., in some embodiments a SARS-CoV-2 Wuhan strain or variants thereof, for example, in some embodiments a variant having one or more mutations characteristic of a prevalent variant such as an Omicron variant). In some embodiments, a nucleic acid containing particle comprises a first RNA molecule encoding a SARS-CoV-2 S protein or an immunogenic fragment thereof from a first strain or variant, and a second RNA molecule encoding a SARS-CoV-2 S protein or an immunogenic fragment thereof from a second strain or variant, wherein the second strain or variant is different from the first strain or variant.

In some embodiments, a nucleic acid containing particle (e.g., in some embodiments an LNP as described herein) comprises a first RNA molecule encoding a SARS-CoV-2 S protein from a Wuhan strain and a second RNA molecule encoding a SARS-CoV-2 S protein comprising one or more mutations that are characteristic of an Omicron variant (e.g., a BA.1, BA.2, BA.3, BA.4, or BA.5 Omicron variant).

In some embodiments, a nucleic acid containing particle comprises a first RNA molecule encoding a SARS-CoV-2 S protein from a Wuhan strain and a second RNA molecule encoding a SARS-CoV-2 S protein comprising one or more mutations that are characteristic of an Omicron BA.1 variant. In some embodiments, the ratio of the first RNA molecule encoding a SARS-CoV-2 S protein from a Wuhan strain and the second RNA molecule encoding a SARS-CoV-2 S protein comprising one or more mutations that are characteristic of an Omicron BA.1 variant is 1:1. In some embodiments, the ratio of the first RNA molecule encoding a SARS-CoV-2 S protein from a Wuhan strain and the second RNA molecule encoding a SARS-CoV-2 S protein comprising one or more mutations that are characteristic of an Omicron BA.1 variant is 1:2. In some embodiments, the ratio of the first RNA molecule encoding a SARS-CoV-2 S protein from a Wuhan strain and the second RNA molecule encoding a SARS-CoV-2 S protein comprising one or more mutations that are characteristic of an Omicron BA.1 variant is 1:3.

In some embodiments, a nucleic acid containing particle comprises a first RNA molecule encoding a SARS-CoV-2 S protein from a Wuhan strain and a second RNA molecule encoding a SARS-CoV-2 S protein comprising one or more mutations that are characteristic of an Omicron BA.2 variant. In some embodiments, the ratio of the first RNA molecule encoding a SARS-CoV-2 S protein from a Wuhan strain and the second RNA molecule encoding a SARS-CoV-2 S protein comprising one or more mutations that are characteristic of an Omicron BA.2 variant is 1:1. In some embodiments, the ratio of the first RNA molecule encoding a SARS-CoV-2 S protein from a Wuhan strain and the second RNA molecule encoding a SARS-CoV-2 S protein comprising one or more mutations that are characteristic of an Omicron BA.2 variant is 1:2. In some embodiments, the ratio of the first RNA molecule encoding a SARS-CoV-2 S protein from a Wuhan strain and the second RNA molecule encoding a SARS-CoV-2 S protein comprising one or more mutations that are characteristic of an Omicron BA.2 variant is 1:3. In some embodiments, a nucleic acid containing particle comprises a first RNA molecule encoding a SARS-CoV-2 S protein from a Wuhan strain and a second RNA molecule encoding a SARS-CoV-2 S protein comprising one or more mutations that are characteristic of an Omicron BA.3 variant. In some embodiments, a nucleic acid containing particle comprises a first RNA molecule encoding a SARS-CoV-2 S protein from a Wuhan strain and a second RNA molecule encoding a SARS-CoV-2 S protein comprising one or more mutations that are characteristic of an Omicron BA.4 or BA.5 variant. In some embodiments, the ratio of the first RNA molecule encoding a SARS-CoV-2 S protein from a Wuhan strain and the second RNA molecule encoding a SARS-CoV-2 S protein comprising one or more mutations that are characteristic of an Omicron BA.4 or BA.5 variant is 1:1. In some embodiments, the ratio of the first RNA molecule encoding a SARS-CoV-2 S protein from a Wuhan strain and the second RNA molecule encoding a SARS-CoV-2 S protein comprising one or more mutations that are characteristic of an Omicron BA.4 or BA.5 variant is 1:2. In some embodiments, the ratio of the first RNA molecule encoding a SARS-CoV-2 S protein from a Wuhan strain and the second RNA molecule encoding a SARS-CoV-2 S protein comprising one or more mutations that are characteristic of an Omicron BA.4 or BA.5 variant is 1:3.

In some embodiments, a nucleic acid containing particle comprises a first RNA molecule encoding a SARS-CoV-2 S protein from a first Omicron variant and a second RNA molecule encoding a SARS-CoV-2 S protein comprising one or more mutations that are characteristic of a second Omicron variant.

In some embodiments, a nucleic acid containing particle comprises a first RNA molecule encoding a SARS-CoV-2 S protein from a BA.1 Omicron variant and a second RNA molecule encoding a SARS-CoV-2 S protein comprising one or more mutations that are characteristic of a BA.2 Omicron variant. In some embodiments, the ratio of the first RNA molecule encoding a SARS-CoV-2 S protein comprising one or more mutations characteristic of a BA.1 Omicron variant strain and the second RNA molecule encoding a SARS-CoV-2 S protein comprising one or more mutations that are characteristic of an Omicron BA.2 variant is 1:1. In some embodiments, the ratio of the first RNA molecule encoding a SARS-CoV-2 S protein comprising one or more mutations characteristic of a BA.1 Omicron variant strain and the second RNA molecule encoding a SARS-CoV-2 S protein comprising one or more mutations that are characteristic of an Omicron BA.2 variant is 1:2. In some embodiments, the ratio of the first RNA molecule encoding a SARS-CoV-2 S protein comprising one or more mutations characteristic of a BA.1 Omicron variant strain and the second RNA molecule encoding a SARS-CoV-2 S protein comprising one or more mutations that are characteristic of an Omicron BA.2 variant is 1:3.

In some embodiments, a nucleic acid containing particle comprises a first RNA molecule encoding a SARS-CoV-2 S protein from a BA.1 Omicron variant and a second RNA molecule encoding a SARS-CoV-2 S protein comprising one or more mutations that are characteristic of a BA.3 Omicron variant. In some embodiments, the ratio of the first RNA molecule encoding a SARS-CoV-2 S protein comprising one or more mutations characteristic of a BA.1 Omicron variant strain and the second RNA molecule encoding a SARS-CoV-2 S protein comprising one or more mutations that are characteristic of an Omicron BA.3 variant is 1:1. In some embodiments, the ratio of the first RNA molecule encoding a SARS-CoV-2 S protein comprising one or more mutations characteristic of a BA.1 Omicron variant strain and the second RNA molecule encoding a SARS-CoV-2 S protein comprising one or more mutations that are characteristic of an Omicron BA.3 variant is 1:2. In some embodiments, the ratio of the first RNA molecule encoding a SARS-CoV-2 S protein comprising one or more mutations characteristic of a BA.1 Omicron variant strain and the second RNA molecule encoding a SARS-CoV-2 S protein comprising one or more mutations that are characteristic of an Omicron BA.3 variant is 1:3.

In some embodiments, a nucleic acid containing particle comprises a first RNA molecule encoding a SARS-CoV-2 S protein from a BA.1 Omicron variant and a second RNA molecule encoding a SARS-CoV-2 S protein comprising one or more mutations that are characteristic of a BA.4 or BA.5 Omicron variant. In some embodiments, the ratio of the first RNA molecule encoding a SARS-CoV-2 S protein comprising one or more mutations characteristic of a BA.1 Omicron variant strain and the second RNA molecule encoding a SARS-CoV-2 S protein comprising one or more mutations that are characteristic of an Omicron BA.4 or BA.5 variant is 1:1. In some embodiments, the ratio of the first RNA molecule encoding a SARS-CoV-2 S protein comprising one or more mutations characteristic of a BA.1 Omicron variant strain and the second RNA molecule encoding a SARS-CoV-2 S protein comprising one or more mutations that are characteristic of an Omicron BA.4 or BA.5 variant is 1:2. In some embodiments, the ratio of the first RNA molecule encoding a SARS-CoV-2 S protein comprising one or more mutations characteristic of a BA.1 Omicron variant strain and the second RNA molecule encoding a SARS-CoV-2 S protein comprising one or more mutations that are characteristic of an Omicron BA.4 or BA.5 variant is 1:3.

In some embodiments, a nucleic acid containing particle comprises a first RNA molecule encoding a SARS-CoV-2 S protein from a BA.2 Omicron variant and a second RNA molecule encoding a SARS-CoV-2 S protein comprising one or more mutations that are characteristic of a BA.3 Omicron variant. In some embodiments, the ratio of the first RNA molecule encoding a SARS-CoV-2 S protein comprising one or more mutations characteristic of a BA.2 Omicron variant strain and the second RNA molecule encoding a SARS-CoV-2 S protein comprising one or more mutations that are characteristic of an Omicron BA.3 variant is 1:1. In some embodiments, the ratio of the first RNA molecule encoding a SARS-CoV-2 S protein comprising one or more mutations characteristic of a BA.2 Omicron variant strain and the second RNA molecule encoding a SARS-CoV-2 S protein comprising one or more mutations that are characteristic of an Omicron BA.3 variant is 1:2. In some embodiments, the ratio of the first RNA molecule encoding a SARS-CoV-2 S protein comprising one or more mutations characteristic of a BA.2 Omicron variant strain and the second RNA molecule encoding a SARS-CoV-2 S protein comprising one or more mutations that are characteristic of an Omicron BA.3 variant is 1:3.

In some embodiments, a nucleic acid containing particle comprises a first RNA molecule encoding a SARS-CoV-2 S protein from a BA.2 Omicron variant and a second RNA molecule encoding a SARS-CoV-2 S protein comprising one or more mutations that are characteristic of a BA.4 or BA.5 Omicron variant. In some embodiments, the ratio of the first RNA molecule encoding a SARS-CoV-2 S protein comprising one or more mutations characteristic of a BA.2 Omicron variant strain and the second RNA molecule encoding a SARS-CoV-2 S protein comprising one or more mutations that are characteristic of an Omicron BA.4 or BA.5 variant is 1:1. In some embodiments, the ratio of the first RNA molecule encoding a SARS-CoV-2 S protein comprising one or more mutations characteristic of a BA.2 Omicron variant strain and the second RNA molecule encoding a SARS-CoV-2 S protein comprising one or more mutations that are characteristic of an Omicron BA.4 or BA.5 variant is 1:2. In some embodiments, the ratio of the first RNA molecule encoding a SARS-CoV-2 S protein comprising one or more mutations characteristic of a BA.2 Omicron variant strain and the second RNA molecule encoding a SARS-CoV-2 S protein comprising one or more mutations that are characteristic of an Omicron BA.4 or BA.5 variant is 1:3.

In some embodiments, a nucleic acid containing particle comprises a first RNA molecule encoding a SARS-CoV-2 S protein from a BA.3 Omicron variant and a second RNA molecule encoding a SARS-CoV-2 S protein comprising one or more mutations that are characteristic of a BA.4 or BA.5 Omicron variant. In some embodiments, the ratio of the first RNA molecule encoding a SARS-CoV-2 S protein comprising one or more mutations characteristic of a BA.3 Omicron variant strain and the second RNA molecule encoding a SARS-CoV-2 S protein comprising one or more mutations that are characteristic of an Omicron BA.4 or BA.5 variant is 1:1. In some embodiments, the ratio of the first RNA molecule encoding a SARS-CoV-2 S protein comprising one or more mutations characteristic of a BA.3 Omicron variant strain and the second RNA molecule encoding a SARS-CoV-2 S protein comprising one or more mutations that are characteristic of an Omicron BA.4 or BA.5 variant is 1:2. In some embodiments, the ratio of the first RNA molecule encoding a SARS-CoV-2 S protein comprising one or more mutations characteristic of a BA.3 Omicron variant strain and the second RNA molecule encoding a SARS-CoV-2 S protein comprising one or more mutations that are characteristic of an Omicron BA.4 or BA.5 variant is 1:3.

In some embodiments, a nucleic acid containing particle (e.g., in some embodiments an LNP as described herein) comprises three or more RNA molecules, each encoding a SARS-CoV-2 S protein comprising mutations of a different SARS-CoV-2 variant. In some embodiments, a nucleic acid containing particle comprises a first RNA molecule encoding a SARS-CoV-2 S protein from a Wuhan strain, a second RNA molecule encoding a SARS-CoV-2 S protein comprising one or more mutations that are characteristic of an Omicron variant (e.g., a BA.1, BA.2, BA.3, BA.4, or BA.5 Omicron variant), and a third RNA molecule encoding a SARS-CoV-2 S protein comprising one or more mutations that are characteristic of an Omicron variant (e.g., a BA.1, BA.2, BA.3, BA.4, or BA.5 Omicron variant), wherein the second and third RNA molecules encode a SARS-CoV-2 S protein comprising one or mutations characteristic of different Omicron subvariants. In some embodiments, a nucleic acid containing particle comprises a first RNA molecule encoding a SARS-CoV-2 S protein from a Wuhan strain, a second RNA molecule encoding a SARS-CoV-2 S protein comprising one or more mutations that are characteristic of a BA.1 Omicron variant, and a third RNA molecule encoding a SARS-CoV-2 S protein comprising one or more mutations that are characteristic of a BA.2 Omicron variant. In some embodiments, a nucleic acid containing particle comprises a first RNA molecule encoding a SARS-CoV-2 S protein from a Wuhan strain, a second RNA molecule encoding a SARS-CoV-2 S protein comprising one or more mutations that are characteristic of a BA.1 Omicron variant, and a third RNA molecule encoding a SARS-CoV-2 S protein comprising one or more mutations that are characteristic of a BA.2 Omicron variant. In some embodiments, a nucleic acid containing particle comprises a first RNA molecule encoding a SARS-CoV-2 S protein from a Wuhan strain, a second RNA molecule encoding a SARS-CoV-2 S protein comprising one or more mutations that are characteristic of a BA.1 Omicron variant, and a third RNA molecule encoding a SARS-CoV-2 S protein comprising one or more mutations that are characteristic of a BA.4/5 Omicron variant. In some embodiments, a nucleic acid containing particle comprises a first RNA molecule encoding a SARS-CoV-2 S protein from a Wuhan strain, a second RNA molecule encoding a SARS-CoV-2 S protein comprising one or more mutations that are characteristic of a BA.2 Omicron variant, and a third RNA molecule encoding a SARS-CoV-2 S protein comprising one or more mutations that are characteristic of a BA.4/5 Omicron variant. In some embodiments, a nucleic acid containing particle comprises a first RNA molecule encoding a SARS-CoV-2 S protein comprising one or more mutations that are characteristic of a BA.1 Omicron variant, a second RNA molecule encoding a SARS-CoV-2 S protein comprising one or more mutations that are characteristic of a BA.2 Omicron variant, and a third RNA molecule encoding a SARS-CoV-2 S protein comprising one or more mutations that are characteristic of a BA.4/5 Omicron variant.

In some embodiments, a nucleic acid containing particle (e.g., in some embodiments an LNP as described herein) comprising two or more RNA molecules, comprises each RNA molecule in the same amount (i.e., at a 1:1 ratio).

In some embodiments, a nucleic acid containing particle (e.g., in some embodiments an LNP as described herein) comprising two or more RNA molecules, comprises a different amount of each RNA molecule. For example, in some embodiments, a nucleic acid containing particle comprises a first RNA molecule and a second RNA molecule, where the first RNA molecule is present in an amount that is 0.01 to 100 times that of the second RNA molecule (e.g., wherein the amount of the first RNA molecule is 0.01 to 50, 0.01 to 4, 0.01 to 30, 0.01 to 25, 0.01 to 20, 0.01 to 15, 0.01 to 10, 0.01 to 9, 0.01 to 8, 0.01 to 7, 0.01 to 6, 0.01 to 5, 0.01 to 4, 0.01 to 3, 0.01 to 2, 0.01 to 1.5, 1 to 50, 1 to 4, 1 to 30, 1 to 25, 1 to 20, 1 to 15, 1 to 10, 1 to 9, 1 to 8, 1 to 7, 1 to 6, 1 to 5, 1 to 4, 1 to 3, 1 to 2, or 1 to 1.5 times higher than the second RNA molecule). In some embodiments, a nucleic acid containing particle comprises a first RNA molecule and a second RNA molecule, wherein the concentration of the first RNA molecule is 1 to 10 times that of the second RNA molecule. In some embodiments, a nucleic acid containing particle comprises a first RNA molecule and a second RNA molecule, wherein the concentration of the first RNA molecule is 1 to 5 times that of the second RNA molecule. In some embodiments, a nucleic acid containing particle comprises a first RNA molecule and a second RNA molecule, wherein the concentration of the first RNA molecule is 1 to 3 times that of the second RNA molecule. In some embodiments, a nucleic acid containing particle comprises a first RNA molecule and a second RNA molecule, wherein the concentration of the first RNA molecule is 2 times that of the second RNA molecule. In some embodiments, a nucleic acid containing particle comprises a first RNA molecule and a second RNA molecule, wherein the concentration of the first RNA molecule is 3 times that of the second RNA molecule.

In some embodiments, a nucleic acid containing particle (e.g., in some embodiments an LNP as described herein) comprising three RNA molecules, comprises each RNA molecule in the same amount (i.e., at a 1:1:1 ratio).

In some embodiments, a nucleic acid containing particle (e.g., in some embodiments an LNP as described herein) comprising three RNA molecules, comprises a different amount of each RNA molecule. For example, in some embodiments, the ratio of first RNA molecule: second RNA molecule: third RNA molecule is 1: 0.01-100: 0.01-100 (e.g., 1: 0.01-50: 0.01-50; 1: 0.01-40: 0.01-40; 1: 0.01-30: 0.01-25; 1: 0.01-25: 0.01-25; 1: 0.01-20: 0.01-20; 1: 0.01-15: 0.01-15; 1: 0.01-10: 0.01-9; 1: 0.01-9: 0.01-9; 1: 0.01-8: 0.01-8; 1: 0.01-7: 0.01-7; 1: 0.01-6: 0.01-6; 1: 0.01-5: 0.01-5; 1: 0.01-4: 0.01-4; 1: 0.01-3: 0.01-3; 1: 0.01-2: 0.01-2; or 1: 0.01-1.5: 0.01-1.5). In some embodiments, the ratio of first RNA molecule: second RNA molecule: third RNA molecule is 1:1:3. In some embodiments, the ratio of first RNA molecule: second RNA molecule: third RNA molecule is 1:3:3.

In some embodiments, a first RNA molecule encoding a SARS-CoV-2 S protein from a Wuhan strain comprises a nucleotide sequence that encodes the amino acid sequence of SEQ ID NO: 7. In some embodiments, a first RNA molecule encoding a SARS-CoV-2 S protein from a Wuhan strain comprises a nucleotide sequence that is at least 80% identical (e.g., at least 85%, at least 90%, at least 91%, at least 92%, at least 93%, at least 94%, at least 95%, at least 96%, at least 97%, at least 98%, or at least 99% identical) to SEQ ID NO: 9. In some embodiments, a first RNA molecule encoding a SARS-COV-2 S protein from a Wuhan strain comprises a nucleotide sequence that is at least 80% identical to (e.g., at least 85%, at least 90%, at least 91%, at least 92%, at least 93%, at least 94%, at least 95%, at least 96%, at least 97%, at least 98%, or at least 99% identical to) SEQ ID NO: 20. In some embodiments, a first RNA molecule encoding a SARS-COV-2 S protein from a Wuhan strain comprises a nucleotide sequence that encodes an amino acid sequence that is at least 80% identical to (e.g., at least 85%, at least 90%, at least 91%, at least 92%, at least 93%, at least 94%, at least 95%, at least 96%, at least 97%, at least 98%, or at least 99% identical to) SEQ ID NO: 7. In some embodiments, a second RNA molecule encoding a SARS-CoV-2 S protein having one or more mutations that are characteristic of an Omicron variant comprises a nucleotide sequence that encodes the amino acid sequence of SEQ ID NO: 49. In some embodiments, a second RNA molecule encoding a SARS-CoV-2 S protein comprising one or more mutations characteristic of an Omicron variant comprises a nucleotide sequence that is at least 80% identical (e.g., at least 85%, at least 90%, at least 91%, at least 92%, at least 93%, at least 94%, at least 95%, at least 96%, at least 97%, at least 98%, or at least 99% identical) to SEQ ID NO: 50. In some embodiments, a second RNA molecule encoding a SARS-COV-2 S protein comprising one or more mutations characteristic of an Omicron variant comprises a nucleotide sequence that is at least 80% identical to (e.g., at least 85%, at least 90%, at least 91%, at least 92%, at least 93%, at least 94%, at least 95%, at least 96%, at least 97%, at least 98%, or at least 99% identical to) SEQ ID NO: 51. In some embodiments, a second RNA molecule encoding a SARS-COV-2 S protein comprising one or more mutations characteristic of an Omicron variant comprises a nucleotide sequence that encodes an amino acid sequence that is at least 80% identical to (e.g., at least 85%, at least 90%, at least 91%, at least 92%, at least 93%, at least 94%, at least 95%, at least 96%, at least 97%, at least 98%, or at least 99% identical to) SEQ ID NO: 49.

In some embodiments, a second RNA molecule encoding a SARS-CoV-2 S protein having one or more mutations that are characteristic of an Omicron variant comprises a nucleotide sequence that encodes the amino acid sequence of SEQ ID NO: 64. In some embodiments, a second RNA molecule encoding a SARS-CoV-2 S protein comprising one or more mutations characteristic of an Omicron variant comprises a nucleotide sequence that is at least 80% identical (e.g., at least 85%, at least 90%, at least 91%, at least 92%, at least 93%, at least 94%, at least 95%, at least 96%, at least 97%, at least 98%, or at least 99% identical) to SEQ ID NO: 65. In some embodiments, a second RNA molecule encoding a SARS-COV-2 S protein comprising one or more mutations characteristic of an Omicron variant comprises a nucleotide sequence that is at least 80% identical to (e.g., at least 85%, at least 90%, at least 91%, at least 92%, at least 93%, at least 94%, at least 95%, at least 96%, at least 97%, at least 98%, or at least 99% identical to) SEQ ID NO: 67. In some embodiments, a second RNA molecule encoding a SARS-COV-2 S protein comprising one or more mutations characteristic of an Omicron variant comprises a nucleotide sequence that encodes an amino acid sequence that is at least 80% identical to (e.g., at least 85%, at least 90%, at least 91%, at least 92%, at least 93%, at least 94%, at least 95%, at least 96%, at least 97%, at least 98%, or at least 99% identical to) SEQ ID NO: 64.

In some embodiments, a second RNA molecule encoding a SARS-CoV-2 S protein having one or more mutations that are characteristic of an Omicron variant comprises a nucleotide sequence that encodes the amino acid sequence of SEQ ID NO: 69. In some embodiments, a second RNA molecule encoding a SARS-CoV-2 S protein comprising one or more mutations characteristic of an Omicron variant comprises a nucleotide sequence that is at least 80% identical (e.g., at least 85%, at least 90%, at least 91%, at least 92%, at least 93%, at least 94%, at least 95%, at least 96%, at least 97%, at least 98%, or at least 99% identical) to SEQ ID NO: 70. In some embodiments, a second RNA molecule encoding a SARS-COV-2 S protein comprising one or more mutations characteristic of an Omicron variant comprises a nucleotide sequence that is at least 80% identical to (e.g., at least 85%, at least 90%, at least 91%, at least 92%, at least 93%, at least 94%, at least 95%, at least 96%, at least 97%, at least 98%, or at least 99% identical to) SEQ ID NO: 72. In some embodiments, a second RNA molecule encoding a SARS-COV-2 S protein comprising one or more mutations characteristic of an Omicron variant comprises a nucleotide sequence that encodes an amino acid sequence that is at least 80% identical to (e.g., at least 85%, at least 90%, at least 91%, at least 92%, at least 93%, at least 94%, at least 95%, at least 96%, at least 97%, at least 98%, or at least 99% identical to) SEQ ID NO: 69.

In some embodiments, a second RNA molecule encoding a SARS-CoV-2 S protein having one or more mutations that are characteristic of an Omicron variant comprises a nucleotide sequence that encodes the amino acid sequence of SEQ ID NO: 74. In some embodiments, a second RNA molecule encoding a SARS-CoV-2 S protein comprising one or more mutations characteristic of an Omicron variant comprises a nucleotide sequence that is at least 80% identical (e.g., at least 85%, at least 90%, at least 91%, at least 92%, at least 93%, at least 94%, at least 95%, at least 96%, at least 97%, at least 98%, or at least 99% identical) to SEQ ID NO: 75. In some embodiments, a second RNA molecule encoding a SARS-COV-2 S protein comprising one or more mutations characteristic of an Omicron variant comprises a nucleotide sequence that is at least 80% identical to (e.g., at least 85%, at least 90%, at least 91%, at least 92%, at least 93%, at least 94%, at least 95%, at least 96%, at least 97%, at least 98%, or at least 99% identical to) SEQ ID NO: 77. In some embodiments, a second RNA molecule encoding a SARS-COV-2 S protein comprising one or more mutations characteristic of an Omicron variant comprises a nucleotide sequence that encodes an amino acid sequence that is at least 80% identical to (e.g., at least 85%, at least 90%, at least 91%, at least 92%, at least 93%, at least 94%, at least 95%, at least 96%, at least 97%, at least 98%, or at least 99% identical to) SEQ ID NO: 74.

In some embodiments, a nucleic acid containing particle (e.g., in some embodiments an LNP as described herein) comprises: a first RNA molecule comprising a nucleotide sequence that encodes the amino acid sequence of SEQ ID NO: 7 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 7); and a second RNA molecule comprising a nucleotide sequence that encodes the amino acid sequence of SEQ ID NO: 49 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 49. In some embodiments, the ratio of the first RNA molecule that encodes the amino acid sequence of SEQ ID NO: 7 or a sequence that is at least 80% identical to SEQ ID NO: 7 to the second RNA molecule that encodes the amino acid sequence of SEQ ID NO: 49 or a sequence that is at least 80% identical to SEQ ID NO: 49 is 1:1. In some embodiments, the ratio of the first RNA molecule that encodes the amino acid sequence of SEQ ID NO: 7 or a sequence that is at least 80% identical to SEQ ID NO: 7 to the second RNA molecule that encodes the amino acid sequence of SEQ ID NO: 49 or a sequence that is at least 80% identical to SEQ ID NO: 49 is 1:2. In some embodiments, the ratio of the first RNA molecule that encodes the amino acid sequence of SEQ ID NO: 7 or a sequence that is at least 80% identical to SEQ ID NO: 7 to the second RNA molecule that encodes the amino acid sequence of SEQ ID NO: 49 or a sequence that is at least 80% identical to SEQ ID NO: 49 is 1:3.

In some embodiments, a nucleic acid containing particle (e.g., in some embodiments an LNP as described herein) comprises: a first RNA molecule comprising a nucleotide sequence of SEQ ID NO: 9 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 9); and a second RNA molecule comprising a nucleotide sequence of SEQ ID NO: 50 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 50. In some embodiments, the ratio of the first RNA molecule comprising the nucleotide sequence of SEQ ID NO: 9 or a sequence that is at least 80% identical to SEQ ID NO: 9 to the second RNA molecule that comprises a nucleotide sequence of SEQ ID NO: 50 or a sequence that is at least 80% identical to SEQ ID NO: 50 is 1:1. In some embodiments, the ratio of the first RNA molecule comprising the nucleotide sequence of SEQ ID NO: 9 or a sequence that is at least 80% identical to SEQ ID NO: 9 to the second RNA molecule that comprises a nucleotide sequence of SEQ ID NO: 50 or a sequence that is at least 80% identical to SEQ ID NO: 50 is 1:2. In some embodiments, the ratio of the first RNA molecule comprising the nucleotide sequence of SEQ ID NO: 9 or a sequence that is at least 80% identical to SEQ ID NO: 9 to the second RNA molecule that comprises a nucleotide sequence of SEQ ID NO: 50 or a sequence that is at least 80% identical to SEQ ID NO: 50 is 1:3.

In some embodiments, a nucleic acid containing particle (e.g., in some embodiments an LNP as described herein) comprises: a first RNA molecule comprising a nucleotide sequence of SEQ ID NO: 20 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 20; and a second RNA molecule comprising a nucleotide sequence of SEQ ID NO: 51 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 51. In some embodiments, the ratio of the first RNA molecule comprising the nucleotide sequence of SEQ ID NO: 20 or a sequence that is at least 80% identical to SEQ ID NO: 20 to the second RNA molecule that comprises a nucleotide sequence of SEQ ID NO: 51 or a sequence that is at least 80% identical to SEQ ID NO: 51 is 1:1. In some embodiments, the ratio of the first RNA molecule comprising the nucleotide sequence of SEQ ID NO: 20 or a sequence that is at least 80% identical to SEQ ID NO: 20 to the second RNA molecule that comprises a nucleotide sequence of SEQ ID NO: 51 or a sequence that is at least 80% identical to SEQ ID NO: 51 is 1:2. In some embodiments, the ratio of the first RNA molecule comprising the nucleotide sequence of SEQ ID NO: 20 or a sequence that is at least 80% identical to SEQ ID NO: 20 to the second RNA molecule that comprises a nucleotide sequence of SEQ ID NO: 51 or a sequence that is at least 80% identical to SEQ ID NO: 51 is 1:3.

In some embodiments, a nucleic acid containing particle (e.g., in some embodiments an LNP as described herein) comprises: a first RNA molecule comprising a nucleotide sequence that encodes the amino acid sequence of SEQ ID NO: 7 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 7); and a second RNA molecule comprising a nucleotide sequence that encodes the amino acid sequence of SEQ ID NO: 64 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 64. In some embodiments, the ratio of the first RNA molecule that encodes the amino acid sequence of SEQ ID NO: 7 or a sequence that is at least 80% identical to SEQ ID NO: 7 to the second RNA molecule that encodes the amino acid sequence of SEQ ID NO: 49 or a sequence that is at least 80% identical to SEQ ID NO: 64 is 1:1. In some embodiments, the ratio of the first RNA molecule that encodes the amino acid sequence of SEQ ID NO: 7 or a sequence that is at least 80% identical to SEQ ID NO: 7 to the second RNA molecule that encodes the amino acid sequence of SEQ ID NO: 64 or a sequence that is at least 80% identical to SEQ ID NO: 64 is 1:2. In some embodiments, the ratio of the first RNA molecule that encodes the amino acid sequence of SEQ ID NO: 7 or a sequence that is at least 80% identical to SEQ ID NO: 7 to the second RNA molecule that encodes the amino acid sequence of SEQ ID NO: 64 or a sequence that is at least 80% identical to SEQ ID NO: 64 is 1:3.

In some embodiments, a nucleic acid containing particle (e.g., in some embodiments an LNP as described herein) comprises: a first RNA molecule comprising a nucleotide sequence of SEQ ID NO: 9 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 9); and a second RNA molecule comprising a nucleotide sequence of SEQ ID NO: 65 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 65. In some embodiments, the ratio of the first RNA molecule comprising the nucleotide sequence of SEQ ID NO: 9 or a sequence that is at least 80% identical to SEQ ID NO: 9 to the second RNA molecule that comprises a nucleotide sequence of SEQ ID NO: 65 or a sequence that is at least 80% identical to SEQ ID NO: 65 is 1:1. In some embodiments, the ratio of the first RNA molecule comprising the nucleotide sequence of SEQ ID NO: 9 or a sequence that is at least 80% identical to SEQ ID NO: 9 to the second RNA molecule that comprises a nucleotide sequence of SEQ ID NO: 65 or a sequence that is at least 80% identical to SEQ ID NO: 65 is 1:2. In some embodiments, the ratio of the first RNA molecule comprising the nucleotide sequence of SEQ ID NO: 9 or a sequence that is at least 80% identical to SEQ ID NO: 9 to the second RNA molecule that comprises a nucleotide sequence of SEQ ID NO: 65 or a sequence that is at least 80% identical to SEQ ID NO: 65 is 1:3.

In some embodiments, a nucleic acid containing particle (e.g., in some embodiments an LNP as described herein) comprises: a first RNA molecule comprising a nucleotide sequence of SEQ ID NO: 20 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 20; and a second RNA molecule comprising a nucleotide sequence of SEQ ID NO: 51 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 67. In some embodiments, the ratio of the first RNA molecule comprising the nucleotide sequence of SEQ ID NO: 20 or a sequence that is at least 80% identical to SEQ ID NO: 20 to the second RNA molecule that comprises a nucleotide sequence of SEQ ID NO: 67 or a sequence that is at least 80% identical to SEQ ID NO: 67 is 1:1. In some embodiments, the ratio of the first RNA molecule comprising the nucleotide sequence of SEQ ID NO: 20 or a sequence that is at least 80% identical to SEQ ID NO: 20 to the second RNA molecule that comprises a nucleotide sequence of SEQ ID NO: 67 or a sequence that is at least 80% identical to SEQ ID NO: 67 is 1:2. In some embodiments, the ratio of the first RNA molecule comprising the nucleotide sequence of SEQ ID NO: 20 or a sequence that is at least 80% identical to SEQ ID NO: 20 to the second RNA molecule that comprises a nucleotide sequence of SEQ ID NO: 67 or a sequence that is at least 80% identical to SEQ ID NO: 67 is 1:3.

In some embodiments, a nucleic acid containing particle (e.g., in some embodiments an LNP as described herein) comprises: a first RNA molecule comprising a nucleotide sequence that encodes the amino acid sequence of SEQ ID NO: 7 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 7); and a second RNA molecule comprising a nucleotide sequence that encodes the amino acid sequence of SEQ ID NO: 69 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 69. In some embodiments, the ratio of the first RNA molecule that encodes the amino acid sequence of SEQ ID NO: 7 or a sequence that is at least 80% identical to SEQ ID NO: 7 to the second RNA molecule that encodes the amino acid sequence of SEQ ID NO: 69 or a sequence that is at least 80% identical to SEQ ID NO: 69 is 1:1. In some embodiments, the ratio of the first RNA molecule that encodes the amino acid sequence of SEQ ID NO: 7 or a sequence that is at least 80% identical to SEQ ID NO: 7 to the second RNA molecule that encodes the amino acid sequence of SEQ ID NO: 69 or a sequence that is at least 80% identical to SEQ ID NO: 69 is 1:2. In some embodiments, the ratio of the first RNA molecule that encodes the amino acid sequence of SEQ ID NO: 7 or a sequence that is at least 80% identical to SEQ ID NO: 7 to the second RNA molecule that encodes the amino acid sequence of SEQ ID NO: 69 or a sequence that is at least 80% identical to SEQ ID NO: 69 is 1:3.

In some embodiments, a nucleic acid containing particle (e.g., in some embodiments an LNP as described herein) comprises: a first RNA molecule comprising a nucleotide sequence of SEQ ID NO: 9 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 9); and a second RNA molecule comprising a nucleotide sequence of SEQ ID NO: 70 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 70. In some embodiments, the ratio of the first RNA molecule comprising the nucleotide sequence of SEQ ID NO: 9 or a sequence that is at least 80% identical to SEQ ID NO: 9 to the second RNA molecule that comprises a nucleotide sequence of SEQ ID NO: 70 or a sequence that is at least 80% identical to SEQ ID NO: 70 is 1:1. In some embodiments, the ratio of the first RNA molecule comprising the nucleotide sequence of SEQ ID NO: 9 or a sequence that is at least 80% identical to SEQ ID NO: 9 to the second RNA molecule that comprises a nucleotide sequence of SEQ ID NO: 70 or a sequence that is at least 80% identical to SEQ ID NO: 70 is 1:2. In some embodiments, the ratio of the first RNA molecule comprising the nucleotide sequence of SEQ ID NO: 9 or a sequence that is at least 80% identical to SEQ ID NO: 9 to the second RNA molecule that comprises a nucleotide sequence of SEQ ID NO: 50 or a sequence that is at least 80% identical to SEQ ID NO: 70 is 1:3.

In some embodiments, a nucleic acid containing particle (e.g., in some embodiments an LNP as described herein) comprises: a first RNA molecule comprising a nucleotide sequence of SEQ ID NO: 20 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 20; and a second RNA molecule comprising a nucleotide sequence of SEQ ID NO: 72 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 72. In some embodiments, the ratio of the first RNA molecule comprising the nucleotide sequence of SEQ ID NO: 20 or a sequence that is at least 80% identical to SEQ ID NO: 20 to the second RNA molecule that comprises a nucleotide sequence of SEQ ID NO: 72 or a sequence that is at least 80% identical to SEQ ID NO: 72 is 1:1. In some embodiments, the ratio of the first RNA molecule comprising the nucleotide sequence of SEQ ID NO: 20 or a sequence that is at least 80% identical to SEQ ID NO: 20 to the second RNA molecule that comprises a nucleotide sequence of SEQ ID NO: 72 or a sequence that is at least 80% identical to SEQ ID NO: 72 is 1:2. In some embodiments, the ratio of the first RNA molecule comprising the nucleotide sequence of SEQ ID NO: 20 or a sequence that is at least 80% identical to SEQ ID NO: 20 to the second RNA molecule that comprises a nucleotide sequence of SEQ ID NO: 72 or a sequence that is at least 80% identical to SEQ ID NO: 72 is 1:3.

In some embodiments, a nucleic acid containing particle (e.g., in some embodiments an LNP as described herein) comprises: a first RNA molecule comprising a nucleotide sequence that encodes the amino acid sequence of SEQ ID NO: 7 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 7); and a second RNA molecule comprising a nucleotide sequence that encodes the amino acid sequence of SEQ ID NO: 74 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 74. In some embodiments, the ratio of the first RNA molecule that encodes the amino acid sequence of SEQ ID NO: 7 or a sequence that is at least 80% identical to SEQ ID NO: 7 to the second RNA molecule that encodes the amino acid sequence of SEQ ID NO: 74 or a sequence that is at least 80% identical to SEQ ID NO: 74 is 1:1. In some embodiments, the ratio of the first RNA molecule that encodes the amino acid sequence of SEQ ID NO: 7 or a sequence that is at least 80% identical to SEQ ID NO: 7 to the second RNA molecule that encodes the amino acid sequence of SEQ ID NO: 74 or a sequence that is at least 80% identical to SEQ ID NO: 74 is 1:2. In some embodiments, the ratio of the first RNA molecule that encodes the amino acid sequence of SEQ ID NO: 7 or a sequence that is at least 80% identical to SEQ ID NO: 7 to the second RNA molecule that encodes the amino acid sequence of SEQ ID NO: 74 or a sequence that is at least 80% identical to SEQ ID NO: 74 is 1:3.

In some embodiments, a nucleic acid containing particle (e.g., in some embodiments an LNP as described herein) comprises: a first RNA molecule comprising a nucleotide sequence of SEQ ID NO: 9 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 9); and a second RNA molecule comprising a nucleotide sequence of SEQ ID NO: 75 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 75. In some embodiments, the ratio of the first RNA molecule comprising the nucleotide sequence of SEQ ID NO: 9 or a sequence that is at least 80% identical to SEQ ID NO: 9 to the second RNA molecule that comprises a nucleotide sequence of SEQ ID NO: 75 or a sequence that is at least 80% identical to SEQ ID NO: 75 is 1:1. In some embodiments, the ratio of the first RNA molecule comprising the nucleotide sequence of SEQ ID NO: 9 or a sequence that is at least 80% identical to SEQ ID NO: 9 to the second RNA molecule that comprises a nucleotide sequence of SEQ ID NO: 75 or a sequence that is at least 80% identical to SEQ ID NO: 75 is 1:2. In some embodiments, the ratio of the first RNA molecule comprising the nucleotide sequence of SEQ ID NO: 9 or a sequence that is at least 80% identical to SEQ ID NO: 9 to the second RNA molecule that comprises a nucleotide sequence of SEQ ID NO: 75 or a sequence that is at least 80% identical to SEQ ID NO: 75 is 1:3.

In some embodiments, a nucleic acid containing particle (e.g., in some embodiments an LNP as described herein) comprises: a first RNA molecule comprising a nucleotide sequence of SEQ ID NO: 20 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 20; and a second RNA molecule comprising a nucleotide sequence of SEQ ID NO: 77 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 77. In some embodiments, the ratio of the first RNA molecule comprising the nucleotide sequence of SEQ ID NO: 20 or a sequence that is at least 80% identical to SEQ ID NO: 20 to the second RNA molecule that comprises a nucleotide sequence of SEQ ID NO: 77 or a sequence that is at least 80% identical to SEQ ID NO: 77 is 1:1. In some embodiments, the ratio of the first RNA molecule comprising the nucleotide sequence of SEQ ID NO: 20 or a sequence that is at least 80% identical to SEQ ID NO: 20 to the second RNA molecule that comprises a nucleotide sequence of SEQ ID NO: 77 or a sequence that is at least 80% identical to SEQ ID NO: 77 is 1:2. In some embodiments, the ratio of the first RNA molecule comprising the nucleotide sequence of SEQ ID NO: 20 or a sequence that is at least 80% identical to SEQ ID NO: 20 to the second RNA molecule that comprises a nucleotide sequence of SEQ ID NO: 77 or a sequence that is at least 80% identical to SEQ ID NO: 77 is 1:3.

In some embodiments, a nucleic acid containing particle (e.g., in some embodiments an LNP as described herein) comprises: a first RNA molecule comprising a nucleotide sequence that encodes the amino acid sequence of SEQ ID NO: 7 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 7); and a second RNA molecule comprising a nucleotide sequence that encodes the amino acid sequence of SEQ ID NO: 55, 58, or 61 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 55, 58, or 61.

In some embodiments, a nucleic acid containing particle (e.g., in some embodiments an LNP as described herein) comprises: a first RNA molecule comprising a nucleotide sequence of SEQ ID NO: 9 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 9; and a second RNA molecule comprising a nucleotide sequence of SEQ ID NO: 56, 59, or 62a or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 56, 59, or 62a.

In some embodiments, a nucleic acid containing particle (e.g., in some embodiments an LNP as described herein) comprises: a first RNA molecule comprising a nucleotide sequence of SEQ ID NO: 20 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 20; and a second RNA molecule comprising a nucleotide sequence of SEQ ID NO: 57, 60, or 63a or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 57, 60, or 63a.

In some embodiments, a nucleic acid containing particle (e.g., in some embodiments an LNP as described herein) comprises: a first RNA molecule comprising a nucleotide sequence that encodes the amino acid sequence of SEQ ID NO: 58 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 58; and a second RNA molecule comprising a nucleotide sequence that encodes an amino acid sequence of SEQ ID NO: 49, 55, or 61 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 49, 55, or 61.

In some embodiments, a nucleic acid containing particle (e.g., in some embodiments an LNP as described herein) comprises: a first RNA molecule comprising a nucleotide sequence of SEQ ID NO: 59 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 59; and a second RNA molecule comprising a nucleotide sequence of SEQ ID NO: 50, 56, or 62a, or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 50, 56, or 62a.

In some embodiments, a nucleic acid containing particle (e.g., in some embodiments an LNP as described herein) comprises: a first RNA molecule comprising a nucleotide sequence of SEQ ID NO: 60 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 60; and a second RNA molecule comprising a nucleotide sequence of SEQ ID NO: 51, 57, or 63a, or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 51, 57, or 63a.

In some embodiments, a nucleic acid containing particle (e.g., in some embodiments an LNP as described herein) comprises: a first RNA molecule comprising a nucleotide sequence that encodes the amino acid sequence of SEQ ID NO: 49 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 49; and a second RNA molecule comprising a nucleotide sequence that encodes the amino acid sequence of SEQ ID NO: 55 or 61 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 55 or 61. In some embodiments, a nucleic acid containing particle (e.g., in some embodiments an LNP as described herein) comprises: a first RNA molecule comprising a nucleotide sequence of SEQ ID NO: 50 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 50; and a second RNA molecule comprising a nucleotide sequence of SEQ ID NO: 56 or 62a or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 56 or 62a.

In some embodiments, a nucleic acid containing particle (e.g., in some embodiments an LNP as described herein) comprises: a first RNA molecule comprising a nucleotide sequence of SEQ ID NO: 51 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 51; and a second RNA molecule comprising a nucleotide sequence of SEQ ID NO: 57 or 63a or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 57 or 63a.

In some embodiments, a nucleic acid containing particle (e.g., in some embodiments an LNP as described herein) comprises: a first RNA molecule comprising a nucleotide sequence that encodes the amino acid sequence of SEQ ID NO: 55 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 55; and a second RNA molecule comprising a nucleotide sequence that encodes the amino acid sequence of SEQ ID NO: 61 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 61.

In some embodiments, a nucleic acid containing particle (e.g., in some embodiments an LNP as described herein) comprises: a first RNA molecule comprising a nucleotide sequence of SEQ ID NO: 56 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 56; and a second RNA molecule comprising a nucleotide sequence of SEQ ID NO: 62a, or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 62a.

In some embodiments, a nucleic acid containing particle (e.g., in some embodiments an LNP as described herein) comprises: a first RNA molecule comprising a nucleotide sequence of SEQ ID NO: 57 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 57; and a second RNA molecule comprising a nucleotide sequence of SEQ ID NO: 63a or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 63a.

In some embodiments, a particle (e.g., in some embodiments an LNP) containing nucleic acids (e.g., RNAs) encoding different polypeptides can be formed by mixing a plurality of (e.g., at least two, at least three, or more) RNA molecules with particle-forming components (e.g., lipids). In some embodiments, nucleic acids (e.g., RNAs) encoding different polypeptides can be mixed (e.g., in some embodiments in substantially equal proportions, e.g., in some embodiments at a 1:1 ratio when two RNA molecules are present) prior to mixing with particle-forming components (e.g., lipids).

In some embodiments, two or more RNA molecules each encoding a different polypeptide (e.g., as described herein) can be mixed with particle-forming agents to form nucleic acid containing particles as described above. In alternative embodiments, two or more RNA molecules each encoding a different polypeptide (e.g., as described herein) can be formulated into separate particle compositions, which are then mixed together. For example, in some embodiments, individual populations of nucleic acid containing particles, each population comprising an RNA molecule encoding a different immunogenic polypeptide or immunogenic fragment thereof (e.g., as described herein), can be separately formed and then mixed together, for example, prior to filling into vials during a manufacturing process, or immediately prior to administration (e.g., by an administering health-care professional)). Accordingly, in some embodiments, described herein is a composition comprises two or more populations of particles (e.g., in some embodiments, lipid nanoparticles), each population comprising at least one RNA molecule encoding a different immunogenic polypeptide or immunogenic fragment thereof (e.g., a SARS-CoV-2 S protein, or fragments thereof, from a different variant). In some embodiments, each population may be provided in a composition at a desirable proportion (e.g., in some embodiments, each population may be provided in a composition in an amount that provides the same amount of RNA molecules).

### Cationic polymer

Given their high degree of chemical flexibility, polymers are commonly used materials for nanoparticle-based delivery. Typically, cationic polymers are used to electrostatically condense the negatively charged nucleic acid into nanoparticles. These positively charged groups often consist of amines that change their state of protonation in the pH range between 5.5 and 7.5, thought to lead to an ion imbalance that results in endosomal rupture. Polymers such as poly-L-lysine, polyamidoamine, protamine and polyethyleneimine, as well as naturally occurring polymers such as chitosan have all been applied to nucleic acid delivery and are suitable as cationic polymers herein. In addition, some investigators have synthesized polymers specifically for nucleic acid delivery. Poly(β-amino esters), in particular, have gained widespread use in nucleic acid delivery owing to their ease of synthesis and biodegradability. Such synthetic polymers are also suitable as cationic polymers herein.

A "polymer," as used herein, is given its ordinary meaning, i.e., a molecular structure comprising one or more repeat units (monomers), connected by covalent bonds. The repeat units can all be identical, or in some cases, there can be more than one type of repeat unit present within the polymer. In some cases, the polymer is biologically derived, i.e., a biopolymer such as a protein. In some cases, additional moieties can also be present in the polymer, for example targeting moieties such as those described herein.

If more than one type of repeat unit is present within the polymer, then the polymer is said to be a "copolymer." It is to be understood that the polymer being employed herein can be a copolymer. The repeat units forming the copolymer can be arranged in any fashion. For example, the repeat units can be arranged in a random order, in an alternating order, or as a "block" copolymer, i.e., comprising one or more regions each comprising a first repeat unit (e.g., a first block), and one or more regions each comprising a second repeat unit (e.g., a second block), etc. Block copolymers can have two (a diblock copolymer), three (a triblock copolymer), or more numbers of distinct blocks.

In certain embodiments, the polymer is biocompatible. Biocompatible polymers are polymers that typically do not result in significant cell death at moderate concentrations. In certain embodiments, the biocompatible polymer is biodegradable, i.e., the polymer is able to degrade, chemically and/or biologically, within a physiological environment, such as within the body.

In certain embodiments, polymer may be protamine or polyalkyleneimine, in particular protamine.

The term "protamine" refers to any of various strongly basic proteins of relatively low molecular weight that are rich in arginine and are found associated especially with DNA in place of somatic histones in the sperm cells of various animals (as fish). In particular, the term "protamine" refers to proteins found in fish sperm that are strongly basic, are soluble in water, are not coagulated by heat, and yield chiefly arginine upon hydrolysis. In purified form, they are used in a long-acting formulation of insulin and to neutralize the anticoagulant effects of heparin.

According to the present disclosure, the term "protamine" as used herein is meant to comprise any protamine amino acid sequence obtained or derived from natural or biological sources including fragments thereof and multimeric forms of said amino acid sequence or fragment thereof as well as (synthesized) polypeptides which are artificial and specifically designed for specific purposes and cannot be isolated from native or biological sources.

In one embodiment, the polyalkyleneimine comprises polyethylenimine and/or polypropylenimine, preferably polyethyleneimine. A preferred polyalkyleneimine is polyethyleneimine (PEI). The average molecular weight of PEI is preferably 0.75·10² to 10⁷ Da, preferably 1000 to 10⁵ Da, more preferably 10000 to 40000 Da, more preferably 15000 to 30000 Da, even more preferably 20000 to 25000 Da.

Preferred according to the present disclosure is linear polyalkyleneimine such as linear polyethyleneimine (PEI).

Cationic polymers (including polycationic polymers) contemplated for use herein include any cationic polymers which are able to electrostatically bind nucleic acid. In one embodiment, cationic polymers contemplated for use herein include any cationic polymers with which nucleic acid can be associated, e.g. by forming complexes with the nucleic acid or forming vesicles in which the nucleic acid is enclosed or encapsulated.

Particles described herein may also comprise polymers otherthan cationic polymers, i.e., non-cationic polymers and/or anionic polymers. Collectively, anionic and neutral polymers are referred to herein as non-cationic polymers.

### Lipid and lipid-like material

The terms "lipid" and "lipid-like material" are broadly defined herein as molecules which comprise one or more hydrophobic moieties or groups and optionally also one or more hydrophilic moieties or groups. Molecules comprising hydrophobic moieties and hydrophilic moieties are also frequently denoted as amphiphiles. Lipids are usually poorly soluble in water. In an aqueous environment, the amphiphilic nature allows the molecules to self-assemble into organized structures and different phases. One of those phases consists of lipid bilayers, as they are present in vesicles, multilamellar/unilamellar liposomes, or membranes in an aqueous environment. Hydrophobicity can be conferred by the inclusion of apolar groups that include, but are not limited to, long-chain saturated and unsaturated aliphatic hydrocarbon groups and such groups substituted by one or more aromatic, cycloaliphatic, or heterocyclic group(s). The hydrophilic groups may comprise polar and/or charged groups and include carbohydrates, phosphate, carboxylic, sulfate, amino, sulfhydryl, nitro, hydroxyl, and other like groups.

As used herein, the term "amphiphilic" refers to a molecule having both a polar portion and a non-polar portion. Often, an amphiphilic compound has a polar head attached to a long hydrophobic tail. In some embodiments, the polar portion is soluble in water, while the non-polar portion is insoluble in water. In addition, the polar portion may have either a formal positive charge, or a formal negative charge. Alternatively, the polar portion may have both a formal positive and a negative charge, and be a zwitterion or inner salt. For purposes of the present disclosure, the amphiphilic compound can be, but is not limited to, one or a plurality of natural or non-natural lipids and lipid-like compounds.

The term "lipid-like material", "lipid-like compound" or "lipid-like molecule" relates to substances that structurally and/or functionally relate to lipids but may not be considered as lipids in a strict sense. For example, the term includes compounds that are able to form amphiphilic layers as they are present in vesicles, multilamellar/unilamellar liposomes, or membranes in an aqueous environment and includes surfactants, or synthesized compounds with both hydrophilic and hydrophobic moieties. Generally speaking, the term refers to molecules, which comprise hydrophilic and hydrophobic moieties with different structural organization, which may or may not be similar to that of lipids. As used herein, the term "lipid" is to be construed to cover both lipids and lipid-like materials unless otherwise indicated herein or clearly contradicted by context.

Specific examples of amphiphilic compounds that may be included in an amphiphilic layer include, but are not limited to, phospholipids, aminolipids and sphingolipids.

In certain embodiments, the amphiphilic compound is a lipid. The term "lipid" refers to a group of organic compounds that are characterized by being insoluble in water, but soluble in many organic solvents. Generally, lipids may be divided into eight categories: fatty acids, glycerolipids, glycerophospholipids, sphingolipids, saccharolipids, polyketides (derived from condensation of ketoacyl subunits), sterol lipids and prenol lipids (derived from condensation of isoprene subunits). Although the term "lipid" is sometimes used as a synonym for fats, fats are a subgroup of lipids called triglycerides. Lipids also encompass molecules such as fatty acids and their derivatives (including tri-, di-, monoglycerides, and phospholipids), as well as sterol-containing metabolites such as cholesterol.

Fatty acids, or fatty acid residues are a diverse group of molecules made of a hydrocarbon chain that terminates with a carboxylic acid group; this arrangement confers the molecule with a polar, hydrophilic end, and a nonpolar, hydrophobic end that is insoluble in water. The carbon chain, typically between four and 24 carbons long, may be saturated or unsaturated, and may be attached to functional groups containing oxygen, halogens, nitrogen, and sulfur. If a fatty acid contains a double bond, there is the possibility of either a cis or trans geometric isomerism, which significantly affects the molecule's configuration. Cis-double bonds cause the fatty acid chain to bend, an effect that is compounded with more double bonds in the chain. Other major lipid classes in the fatty acid category are the fatty esters and fatty amides. Glycerolipids are composed of mono-, di-, and tri-substituted glycerols, the best-known being the fatty acid triesters of glycerol, called triglycerides. The word "triacylglycerol" is sometimes used synonymously with "triglyceride". In these compounds, the three hydroxyl groups of glycerol are each esterified, typically by different fatty acids. Additional subclasses of glycerolipids are represented by glycosylglycerols, which are characterized by the presence of one or more sugar residues attached to glycerol via a glycosidic linkage.

The glycerophospholipids are amphipathic molecules (containing both hydrophobic and hydrophilic regions) that contain a glycerol core linked to two fatty acid-derived "tails" by ester linkages and to one "head" group by a phosphate ester linkage. Examples of glycerophospholipids, usually referred to as phospholipids (though sphingomyelins are also classified as phospholipids) are phosphatidylcholine (also known as PC, GPCho or lecithin), phosphatidylethanolamine (PE or GPEtn) and phosphatidylserine (PS or GPSer).

Sphingolipids are a complex family of compounds that share a common structural feature, a sphingoid base backbone. The major sphingoid base in mammals is commonly referred to as sphingosine. Ceramides (N-acyl-sphingoid bases) are a major subclass of sphingoid base derivatives with an amide-linked fatty acid. The fatty acids are typically saturated or mono-unsaturated with chain lengths from 16 to 26 carbon atoms. The major phosphosphingolipids of mammals are sphingomyelins (ceramide phosphocholines), whereas insects contain mainly ceramide phosphoethanolamines and fungi have phytoceramide phosphoinositols and mannose-containing headgroups. The glycosphingolipids are a diverse family of molecules composed of one or more sugar residues linked via a glycosidic bond to the sphingoid base.

Examples of these are the simple and complex glycosphingolipids such as cerebrosides and gangliosides.

Sterol lipids, such as cholesterol and its derivatives, or tocopherol and its derivatives, are an important component of membrane lipids, along with the glycerophospholipids and sphingomyelins.

Saccharolipids describe compounds in which fatty acids are linked directly to a sugar backbone, forming structures that are compatible with membrane bilayers. In the saccharolipids, a monosaccharide substitutes for the glycerol backbone present in glycerolipids and glycerophospholipids. The most familiar saccharolipids are the acylated glucosamine precursors of the Lipid A component of the lipopolysaccharides in Gram-negative bacteria. Typical lipid A molecules are disaccharides of glucosamine, which are derivatized with as many as seven fatty-acyl chains. The minimal lipopolysaccharide required for growth in E. coli is Kdo2-Lipid A, a hexa-acylated disaccharide of glucosamine that is glycosylated with two 3-deoxy-D-manno-octulosonic acid (Kdo) residues.

Polyketides are synthesized by polymerization of acetyl and propionyl subunits by classic enzymes as well as iterative and multimodular enzymes that share mechanistic features with the fatty acid synthases. They comprise a large number of secondary metabolites and natural products from animal, plant, bacterial, fungal and marine sources, and have great structural diversity. Many polyketides are cyclic molecules whose backbones are often further modified by glycosylation, methylation, hydroxylation, oxidation, or other processes.

According to the present disclosure, lipids and lipid-like materials may be cationic, anionic or neutral. Neutral lipids or lipid-like materials exist in an uncharged or neutral zwitterionic form at a selected pH.

### Cationic or cationically ionizable lipids or lipid-like materials

The nucleic acid particles described herein may comprise at least one cationic or cationically ionizable lipid or lipid-like material as particle forming agent. Cationic or cationically ionizable lipids or lipid-like materials contemplated for use herein include any cationic or cationically ionizable lipids or lipid-like materials which are able to electrostatically bind nucleic acid. In one embodiment, cationic or cationically ionizable lipids or lipid-like materials contemplated for use herein can be associated with nucleic acid, e.g. by forming complexes with the nucleic acid or forming vesicles in which the nucleic acid is enclosed or encapsulated.

As used herein, a "cationic lipid" or "cationic lipid-like material" refers to a lipid or lipid-like material having a net positive charge. Cationic lipids or lipid-like materials bind negatively charged nucleic acid by electrostatic interaction. Generally, cationic lipids possess a lipophilic moiety, such as a sterol, an acyl chain, a diacyl or more acyl chains, and the head group of the lipid typically carries the positive charge.

In certain embodiments, a cationic lipid or lipid-like material has a net positive charge only at certain pH, in particular acidic pH, while it has preferably no net positive charge, preferably has no charge, i.e., it is neutral, at a different, preferably higher pH such as physiological pH. This ionizable behavior is thought to enhance efficacy through helping with endosomal escape and reducing toxicity as compared with particles that remain cationic at physiological pH.

For purposes of the present disclosure, such "cationically ionizable" lipids or lipid-like materials are comprised by the term "cationic lipid or lipid-like material" unless contradicted by the circumstances.

In one embodiment, the cationic or cationically ionizable lipid or lipid-like material comprises a head group which includes at least one nitrogen atom (N) which is positive charged or capable of being protonated.

Examples of cationic lipids include, but are not limited to 1,2-dioleoyl-3-trimethylammonium propane (DOTAP); N,N-dimethyl-2,3-dioleyloxypropylamine (DODMA), 1,2-di-O-octadecenyl-3-trimethylammonium propane (DOTMA), 3-(N-(N',N'-dimethylaminoethane)-carbamoyl)cholesterol (DC-Chol), dimethyldioctadecylammonium (DDAB); 1,2-dioleoyl-3-dimethylammonium-propane (DODAP); 1,2-diacyloxy-3-dimethylammonium propanes; 1,2-dialkyloxy-3-dimethylammonium propanes; dioctadecyldimethyl ammonium chloride (DODAC), 1,2-distearyloxy-N,N-dimethyl-3-aminopropane (DSDMA), 2,3-di(tetradecoxy)propyl-(2-hydroxyethyl)-dimethylazanium (DMRIE), 1,2-dimyristoyl-sn-glycero-3-ethylphosphocholine (DMEPC), l,2-dimyristoyl-3-trimethylammonium propane (DMTAP), 1,2-dioleyloxypropyl-3-dimethyl-hydroxyethyl ammonium bromide (DORIE), and 2,3-dioleoyloxy- N-[2(spermine carboxamide)ethyl]-N,N-dimethyl-l-propanamium trifluoroacetate (DOSPA), 1,2-dilinoleyloxy-N,N-dimethylaminopropane (DLinDMA), 1,2-dilinolenyloxy-N,N-dimethylaminopropane (DLenDMA), dioctadecylamidoglycyl spermine (DOGS), 3-dimethylamino-2-(cholest-5-en-3-beta-oxybutan-4-oxy)-1-(cis,cis-9,12-oc-tadecadienoxy)propane (CLinDMA), 2-[5'-(cholest-5-en-3-beta-oxy)-3'-oxapentoxy)-3-dimethyl-1-(cis,cis-9',12'-octadecadienoxy)propane (CpLinDMA), N,N-dimethyl-3,4-dioleyloxybenzylamine (DMOBA), 1,2-N,N'-dioleylcarbamyl-3-dimethylaminopropane (DOcarbDAP), 2,3-Dilinoleoyloxy-N,N-dimethylpropylamine (DLinDAP), 1,2-N,N'-Dilinoleylcarbamyl-3-dimethylaminopropane (DLincarbDAP), 1,2-Dilinoleoylcarbamyl-3-dimethylaminopropane (DLinCDAP), 2,2-dilinoleyl-4-dimethylaminomethyl-[1,3]-dioxolane (DLin-K-DMA), 2,2-dilinoleyl-4-dimethylaminoethyl-[1,3]-dioxolane (DLin-K-XTC2-DMA), 2,2-dilinoleyl-4-(2-dimethylaminoethyl)-[1,3]-dioxolane (DLin-KC2-DMA), heptatriaconta-6,9,28,31-tetraen-19-yl-4-(dimethylamino)butanoate (DLin-MC3-DMA), N-(2-Hydroxyethyl)-N,N-dimethyl-2,3-bis(tetradecyloxy)-1-propanaminium bromide (DMRIE), (±)-N-(3-aminopropyl)-N,N-dimethyl-2,3-bis(cis-9-tetradecenyloxy)-1-propanaminium bromide (GAP-DMORIE), (±)-N-(3-aminopropyl)-N,N-dimethyl-2,3-bis(dodecyloxy)-1-propanaminium bromide (GAP-DLRIE), (±)-N-(3-aminopropyl)-N,N-dimethyl-2,3-bis(tetradecyloxy)-1-propanaminium bromide (GAP-DMRIE), N-(2-Aminoethyl)-N,N-dimethyl-2,3-bis(tetradecyloxy)-1-propanaminium bromide (βAE-DMRIE), N-(4-carboxybenzyl)-N,N-dimethyl-2,3-bis(oleoyloxy)propan-1-aminium (DOBAQ), 2-({8-[(3β)-cholest-5-en-3-yloxy]octyl}oxy)-N,N-dimethyl-3-[(9Z,12Z)-octadeca-9,12-dien-1-yloxy]propan-1-amine (Octyl-CLinDMA), 1,2-dimyristoyl-3-dimethylammonium-propane (DMDAP), 1,2-dipalmitoyl-3-dimethylammonium-propane (DPDAP), N1-[2-((1S)-1-[(3-aminopropyl)amino]-4-[di(3-amino-propyl)amino]butylcarboxamido)ethyl]-3,4-di[oleyloxy]-benzamide (MVL5), 1,2-dioleoyl-sn-glycero-3-ethylphosphocholine (DOEPC), 2,3-bis(dodecyloxy)-N-(2-hydroxyethyl)-N,N-dimethylpropan-1-amonium bromide (DLRIE), N-(2-aminoethyl)-N,N-dimethyl-2,3-bis(tetradecyloxy)propan-1-aminium bromide (DMORIE), di((Z)-non-2-en-1-yl) 8,8'-((((2(dimethylamino)ethyl)thio)carbonyl)azanediyl)dioctanoate (ATX), N,N-dimethyl-2,3-bis(dodecyloxy)propan-1-amine (DLDMA), N,N-dimethyl-2,3-bis(tetradecyloxy)propan-1-amine (DMDMA), Di((Z)-non-2-en-1-yl)-9-((4-(dimethylaminobutanoyl)oxy)heptadecanedioate (L319), N-Dodecyl-3-((2-dodecylcarbamoyl-ethyl)-{2-[(2-dodecylcarbamoyl-ethyl)-2-{(2-dodecylcarbamoyl-ethyl)-[2-(2-dodecylcarbamoyl-ethylamino)-ethyl]-amino}-ethylamino)propionamide (lipidoid 98N₁₂-5), 1-[2-[bis(2-hydroxydodecyl)amino]ethyl-[2-[4-[2-[bis(2 hydroxydodecyl)amino]ethyl]piperazin-1-yl]ethyl]amino]dodecan-2-ol (lipidoid C12-200).

In some embodiments, the cationic lipid may comprise from about 10 mol % to about 100 mol %, about 20 mol % to about 100 mol %, about 30 mol % to about 100 mol %, about 40 mol % to about 100 mol %, or about 50 mol % to about 100 mol % of the total lipid present in the particle.

### Additional lipids or lipid-like materials

Particles described herein may also comprise lipids or lipid-like materials other than cationic or cationically ionizable lipids or lipid-like materials, i.e., non-cationic lipids or lipid-like materials (including non-cationically ionizable lipids or lipid-like materials). Collectively, anionic and neutral lipids or lipid-like materials are referred to herein as non-cationic lipids or lipid-like materials. Optimizing the formulation of nucleic acid particles by addition of other hydrophobic moieties, such as cholesterol and lipids, in addition to an ionizable/cationic lipid or lipid-like material may enhance particle stability and efficacy of nucleic acid delivery.

An additional lipid or lipid-like material may be incorporated which may or may not affect the overall charge of the nucleic acid particles. In certain embodiments, the additional lipid or lipid-like material is a non-cationic lipid or lipid-like material. The non-cationic lipid may comprise, e.g., one or more anionic lipids and/or neutral lipids. As used herein, an "anionic lipid" refers to any lipid that is negatively charged at a selected pH. As used herein, a "neutral lipid" refers to any of a number of lipid species that exist either in an uncharged or neutral zwitterionic form at a selected pH. In preferred embodiments, the additional lipid comprises one of the following neutral lipid components: (1) a phospholipid, (2) cholesterol or a derivative thereof; or (3) a mixture of a phospholipid and cholesterol or a derivative thereof. Examples of cholesterol derivatives include, but are not limited to, cholestanol, cholestanone, cholestenone, coprostanol, cholesteryl-2'-hydroxyethyl ether, cholesteryl-4'- hydroxybutyl ether, tocopherol and derivatives thereof, and mixtures thereof.

Specific phospholipids that can be used include, but are not limited to, phosphatidylcholines, phosphatidylethanolamines, phosphatidylglycerols, phosphatidic acids, phosphatidylserines or sphingomyelin. Such phospholipids include in particular diacylphosphatidylcholines, such as distearoylphosphatidylcholine (DSPC), dioleoylphosphatidylcholine (DOPC), dimyristoylphosphatidylcholine (DMPC), dipentadecanoylphosphatidylcholine, dilauroylphosphatidylcholine, dipalmitoylphosphatidylcholine (DPPC), diarachidoylphosphatidylcholine (DAPC), dibehenoylphosphatidylcholine (DBPC), ditricosanoylphosphatidylcholine (DTPC), dilignoceroylphatidylcholine (DLPC), palmitoyloleoyl-phosphatidylcholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC) and phosphatidylethanolamines, in particular diacylphosphatidylethanolamines, such as dioleoylphosphatidylethanolamine (DOPE), distearoyl-phosphatidylethanolamine (DSPE), dipalmitoyl-phosphatidylethanolamine (DPPE), dimyristoyl-phosphatidylethanolamine (DMPE), dilauroyl-phosphatidylethanolamine (DLPE), diphytanoyl-phosphatidylethanolamine (DPyPE), and further phosphatidylethanolamine lipids with different hydrophobic chains.

In certain preferred embodiments, the additional lipid is DSPC or DSPC and cholesterol.

In certain embodiments, the nucleic acid particles include both a cationic lipid and an additional lipid.

In one embodiment, particles described herein include a polymer conjugated lipid such as a pegylated lipid. The term "pegylated lipid" refers to a molecule comprising both a lipid portion and a polyethylene glycol portion. Pegylated lipids are known in the art.

Without wishing to be bound by theory, the amount of the at least one cationic lipid compared to the amount of the at least one additional lipid may affect important nucleic acid particle characteristics, such as charge, particle size, stability, tissue selectivity, and bioactivity of the nucleic acid. Accordingly, in some embodiments, the molar ratio of the at least one cationic lipid to the at least one additional lipid is from about 10:0 to about 1:9, about 4:1 to about 1:2, or about 3:1 to about 1:1.

In some embodiments, the non-cationic lipid, in particular neutral lipid, (e.g., one or more phospholipids and/or cholesterol) may comprise from about 0 mol % to about 90 mol %, from about 0 mol % to about 80 mol %, from about 0 mol % to about 70 mol %, from about 0 mol % to about 60 mol %, or from about 0 mol % to about 50 mol %, of the total lipid present in the particle.

### Lipoplex Particles

In certain embodiments of the present disclosure, the RNA described herein may be present in RNA lipoplex particles.

In the context of the present disclosure, the term "RNA lipoplex particle" relates to a particle that contains lipid, in particular cationic lipid, and RNA. Electrostatic interactions between positively charged liposomes and negatively charged RNA results in complexation and spontaneous formation of RNA lipoplex particles. Positively charged liposomes may be generally synthesized using a cationic lipid, such as DOTMA, and additional lipids, such as DOPE. In one embodiment, a RNA lipoplex particle is a nanoparticle.

In certain embodiments, the RNA lipoplex particles include both a cationic lipid and an additional lipid. In an exemplary embodiment, the cationic lipid is DOTMA and the additional lipid is DOPE.

In some embodiments, the molar ratio of the at least one cationic lipid to the at least one additional lipid is from about 10:0 to about 1:9, about 4:1 to about 1:2, or about 3:1 to about 1:1. In specific embodiments, the molar ratio may be about 3:1, about 2.75:1, about 2.5:1, about 2.25:1, about 2:1, about 1.75:1, about 1.5:1, about 1.25:1, or about 1:1. In an exemplary embodiment, the molar ratio of the at least one cationic lipid to the at least one additional lipid is about 2:1.

RNA lipoplex particles described herein have an average diameter that in one embodiment ranges from about 200 nm to about 1000 nm, from about 200 nm to about 800 nm, from about 250 to about 700 nm, from about 400 to about 600 nm, from about 300 nm to about 500 nm, or from about 350 nm to about 400 nm. In specific embodiments, the RNA lipoplex particles have an average diameter of about 200 nm, about 225 nm, about 250 nm, about 275 nm, about 300 nm, about 325 nm, about 350 nm, about 375 nm, about 400 nm, about 425 nm, about 450 nm, about 475 nm, about 500 nm, about 525 nm, about 550 nm, about 575 nm, about 600 nm, about 625 nm, about 650 nm, about 700 nm, about 725 nm, about 750 nm, about 775 nm, about 800 nm, about 825 nm, about 850 nm, about 875 nm, about 900 nm, about 925 nm, about 950 nm, about 975 nm, or about 1000 nm. In an embodiment, the RNA lipoplex particles have an average diameter that ranges from about 250 nm to about 700 nm. In another embodiment, the RNA lipoplex particles have an average diameter that ranges from about 300 nm to about 500 nm. In an exemplary embodiment, the RNA lipoplex particles have an average diameter of about 400 nm.

The RNA lipoplex particles and compositions comprising RNA lipoplex particles described herein are useful for delivery of RNA to a target tissue after parenteral administration, in particular after intravenous administration. The RNA lipoplex particles may be prepared using liposomes that may be obtained by injecting a solution of the lipids in ethanol into water or a suitable aqueous phase. In one embodiment, the aqueous phase has an acidic pH. In one embodiment, the aqueous phase comprises acetic acid, e.g., in an amount of about 5 mM. Liposomes may be used for preparing RNA lipoplex particles by mixing the liposomes with RNA. In one embodiment, the liposomes and RNA lipoplex particles comprise at least one cationic lipid and at least one additional lipid. In one embodiment, the at least one cationic lipid comprises 1,2-di-O-octadecenyl-3-trimethylammonium propane (DOTMA) and/or 1,2-dioleoyl-3-trimethylammonium-propane (DOTAP). In one embodiment, the at least one additional lipid comprises 1,2-di-(9Z-octadecenoyl)-sn-glycero-3-phosphoethanolamine (DOPE), cholesterol (Chol) and/or 1,2-dioleoyl-sn-glycero-3-phosphocholine (DOPC). In one embodiment, the at least one cationic lipid comprises 1,2-di-O-octadecenyl-3-trimethylammonium propane (DOTMA) and the at least one additional lipid comprises 1,2-di-(9Z-octadecenoyl)-sn-glycero-3-phosphoethanolamine (DOPE). In one embodiment, the liposomes and RNA lipoplex particles comprise 1,2-di-O-octadecenyl-3-trimethylammonium propane (DOTMA) and 1,2-di-(9Z-octadecenoyl)-sn-glycero-3-phosphoethanolamine (DOPE). Spleen targeting RNA lipoplex particles are described in WO 2013/143683, herein incorporated by reference. It has been found that RNA lipoplex particles having a net negative charge may be used to preferentially target spleen tissue or spleen cells such as antigen-presenting cells, in particular dendritic cells. Accordingly, following administration of the RNA lipoplex particles, RNA accumulation and/or RNA expression in the spleen occurs. Thus, RNA lipoplex particles of the present disclosure may be used for expressing RNA in the spleen. In an embodiment, after administration of the RNA lipoplex particles, no or essentially no RNA accumulation and/or RNA expression in the lung and/or liver occurs. In one embodiment, after administration of the RNA lipoplex particles, RNA accumulation and/or RNA expression in antigen presenting cells, such as professional antigen presenting cells in the spleen occurs. Thus, RNA lipoplex particles of the present disclosure may be used for expressing RNA in such antigen presenting cells. In one embodiment, the antigen presenting cells are dendritic cells and/or macrophages.

### Lipid nanoparticles (LNPs)

In one embodiment, nucleic acid such as RNA described herein is administered in the form of lipid nanoparticles (LNPs). The LNP may comprise any lipid capable of forming a particle to which the one or more nucleic acid molecules are attached, or in which the one or more nucleic acid molecules are encapsulated.

In one embodiment, the LNP comprises one or more cationic lipids, and one or more stabilizing lipids. Stabilizing lipids include neutral lipids and pegylated lipids.

In one embodiment, the LNP comprises a cationic lipid, a neutral lipid, a steroid, a polymer conjugated lipid; and the RNA, encapsulated within or associated with the lipid nanoparticle. In one embodiment, the LNP comprises from 40 to 55 mol percent, from 40 to 50 mol percent, from 41 to 49 mol percent, from 41 to 48 mol percent, from 42 to 48 mol percent, from 43 to 48 mol percent, from 44 to 48 mol percent, from 45 to 48 mol percent, from 46 to 48 mol percent, from 47 to 48 mol percent, or from 47.2 to 47.8 mol percent of the cationic lipid. In one embodiment, the LNP comprises about 47.0, 47.1, 47.2, 47.3, 47.4, 47.5, 47.6, 47.7, 47.8, 47.9 or 48.0 mol percent of the cationic lipid.

In one embodiment, the neutral lipid is present in a concentration ranging from 5 to 15 mol percent, from 7 to 13 mol percent, or from 9 to 11 mol percent. In one embodiment, the neutral lipid is present in a concentration of about 9.5, 10 or 10.5 mol percent.

In one embodiment, the steroid is present in a concentration ranging from 30 to 50 mol percent, from 35 to 45 mol percent or from 38 to 43 mol percent. In one embodiment, the steroid is present in a concentration of about 40, 41, 42, 43, 44, 45 or 46 mol percent.

In one embodiment, the LNP comprises from 1 to 10 mol percent, from 1 to 5 mol percent, or from 1 to 2.5 mol percent of the polymer conjugated lipid.

In one embodiment, the LNP comprises from 40 to 50 mol percent a cationic lipid; from 5 to 15 mol percent of a neutral lipid; from 35 to 45 mol percent of a steroid; from 1 to 10 mol percent of a polymer conjugated lipid; and the RNA, encapsulated within or associated with the lipid nanoparticle.

In one embodiment, the mol percent is determined based on total mol of lipid present in the lipid nanoparticle.

In one embodiment, the neutral lipid is selected from the group consisting of DSPC, DPPC, DMPC, DOPC, POPC, DOPE, DOPG, DPPG, POPE, DPPE, DMPE, DSPE, and SM. In one embodiment, the neutral lipid is selected from the group consisting of DSPC, DPPC, DMPC, DOPC, POPC, DOPE and SM. In one embodiment, the neutral lipid is DSPC.

In one embodiment, the steroid is cholesterol.

In one embodiment, the polymer conjugated lipid is a pegylated lipid. In one embodiment, the pegylated lipid has the following structure: 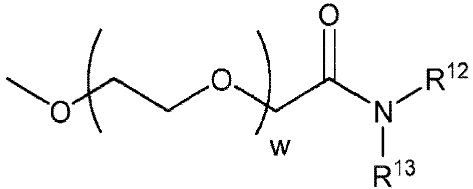 or a pharmaceutically acceptable salt, tautomer or stereoisomer thereof, wherein:
R¹² and R¹³ are each independently a straight or branched, saturated or unsaturated alkyl chain containing from 10 to 30 carbon atoms, wherein the alkyl chain is optionally interrupted by one or more ester bonds; and w has a mean value ranging from 30 to 60. In one embodiment, R¹² and R¹³ are each independently straight, saturated alkyl chains containing from 12 to 16 carbon atoms. In one embodiment, w has a mean value ranging from 40 to 55. In one embodiment, the average w is about 45. In one embodiment, R¹² and R¹³ are each independently a straight, saturated alkyl chain containing about 14 carbon atoms, and w has a mean value of about 45.

In one embodiment, the pegylated lipid is DMG-PEG 2000, e.g., having the following structure:

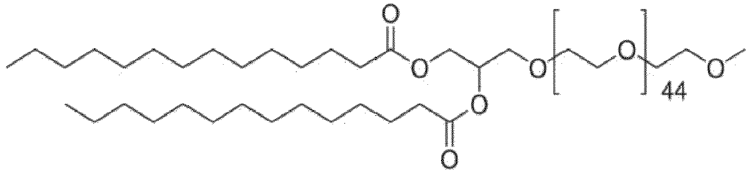

In some embodiments, the cationic lipid component of the LNPs has the structure of Formula (III): 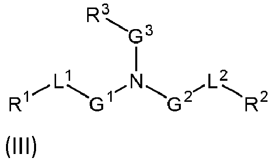 or a pharmaceutically acceptable salt, tautomer, prodrug or stereoisomer thereof, wherein:
one of L¹ or L² is -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, NR^{a}C(=O)NR^{a}-, -OC(=O)NR^{a}- or -NR^{a}C(=O)O-, and the other of L¹ or L² is -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, NR^{a}C(=O)NR^{a}-, -OC(=O)NR^{a}- or -NR^{a}C(=O)O- or a direct bond;
G¹ and G² are each independently unsubstituted C₁-C₁₂ alkylene or C₁-C₁₂ alkenylene;
G³ is C₁-C₂₄ alkylene, C₁-C₂₄ alkenylene, C₃-C₈ cycloalkylene, C₃-C₈ cycloalkenylene;
R^{a} is H or C₁-C₁₂ alkyl;
R¹ and R² are each independently C₆-C₂₄ alkyl or C₆-C₂₄ alkenyl;
R³ is H, OR⁵, CN, -C(=O)OR⁴, -OC(=O)R⁴ or -NR⁵C(=O)R⁴;
R⁴ is C₁-C₁₂ alkyl;
R⁵ is H or C₁-C₆ alkyl; and
x is 0, 1 or 2.

In some of the foregoing embodiments of Formula (III), the lipid has one of the following structures (IIIA) or (IIIB): 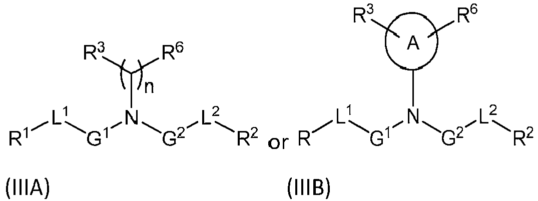 wherein:
A is a 3 to 8-membered cycloalkyl or cycloalkylene ring;
R⁶ is, at each occurrence, independently H, OH or C₁-C₂₄ alkyl;
n is an integer ranging from 1 to 15.

In some of the foregoing embodiments of Formula (III), the lipid has structure (IIIA), and in other embodiments, the lipid has structure (IIIB).

In other embodiments of Formula (III), the lipid has one of the following structures (IIIC) or (IIID): 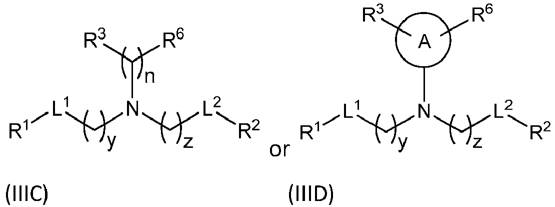 wherein y and z are each independently integers ranging from 1 to 12.

In any of the foregoing embodiments of Formula (III), one of L¹ or L² is -O(C=O)-. For example, in some embodiments each of L¹ and L² are -O(C=O)-. In some different embodiments of any of the foregoing, L¹ and L² are each independently -(C=O)O- or -O(C=O)-. For example, in some embodiments each of L¹ and L² is -(C=O)O-.

In some different embodiments of Formula (III), the lipid has one of the following structures (IIIE) or (IIIF):

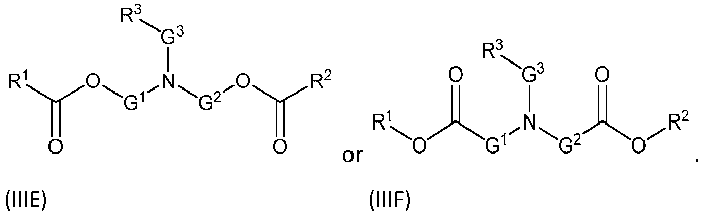

In some of the foregoing embodiments of Formula (III), the lipid has one of the following structures (IIIG), (IIIH), (IIII), or (IIIJ):

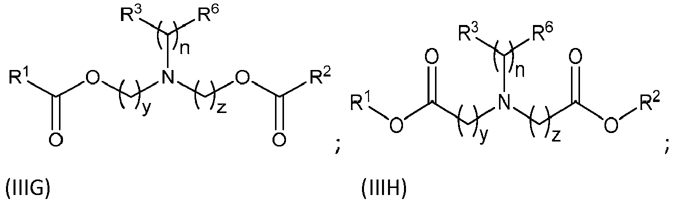

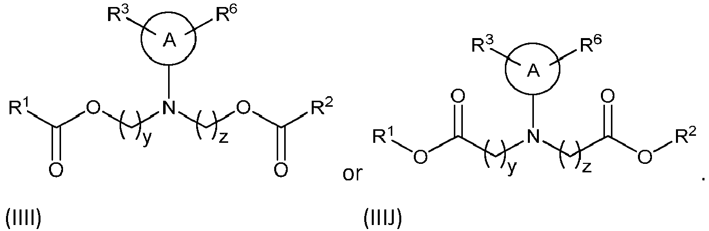

In some of the foregoing embodiments of Formula (III), n is an integer ranging from 2 to 12, for example from 2 to 8 or from 2 to 4. For example, in some embodiments, n is 3, 4, 5 or 6. In some embodiments, n is 3. In some embodiments, n is 4. In some embodiments, n is 5. In some embodiments, n is 6.

In some other of the foregoing embodiments of Formula (III), y and z are each independently an integer ranging from 2 to 10. For example, in some embodiments, y and z are each independently an integer ranging from 4 to 9 or from 4 to 6.

In some of the foregoing embodiments of Formula (III), R⁶ is H. In other of the foregoing embodiments, R⁶ is C₁-C₂₄ alkyl. In other embodiments, R⁶ is OH.

In some embodiments of Formula (III), G³ is unsubstituted. In other embodiments, G3 is substituted. In various different embodiments, G³ is linear C₁-C₂₄ alkylene or linear C₁-C₂₄ alkenylene.

In some other foregoing embodiments of Formula (III), R¹ or R², or both, is C₆-C₂₄ alkenyl. For example, in some embodiments, R¹ and R² each, independently have the following structure: 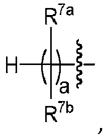 wherein:
R^{7a} and R^{7b} are, at each occurrence, independently H or C₁-C₁₂ alkyl; and
a is an integer from 2 to 12,
wherein R^{7a}, R^{7b} and a are each selected such that R¹ and R² each independently comprise from 6 to 20 carbon atoms. For example, in some embodiments a is an integer ranging from 5 to 9 or from 8 to 12.

In some of the foregoing embodiments of Formula (III), at least one occurrence of R^{7a} is H. For example, in some embodiments, R^{7a} is H at each occurrence. In other different embodiments of the foregoing, at least one occurrence of R^{7b} is C₁-C₈ alkyl. For example, in some embodiments, C₁-C₈ alkyl is methyl, ethyl, n-propyl, iso-propyl, n-butyl, iso-butyl, tert-butyl, n-hexyl or n-octyl.

In different embodiments of Formula (III), R¹ or R², or both, has one of the following structures:

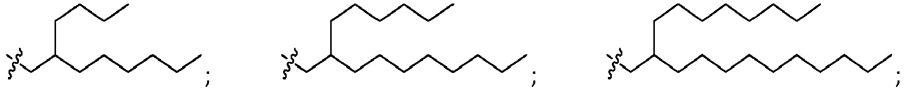

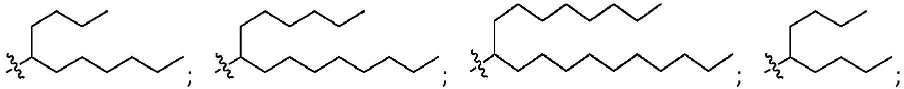

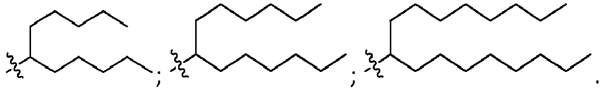

In some of the foregoing embodiments of Formula (III), R³ is OH, CN, -C(=O)OR⁴, -OC(=O)R⁴ or -NHC(=O)R⁴. In some embodiments, R⁴ is methyl or ethyl.

In various different embodiments, the cationic lipid of Formula (III) has one of the structures set forth in the table below.

**Table 17: Representative Compounds of Formula (III).**

| **No.** | **Structure** |
|---|---|
| III-1 | 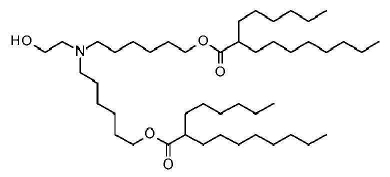 |
| III-2 | 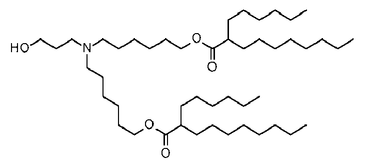 |
| III-3 | 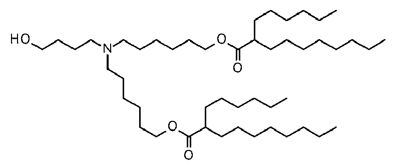 |
| III-4 | 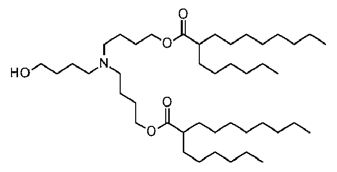 |
| III-5 | 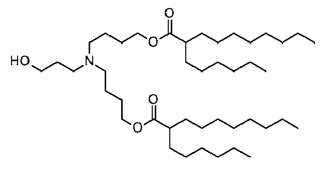 |
| III-6 | 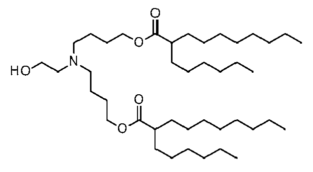 |
| III-7 | 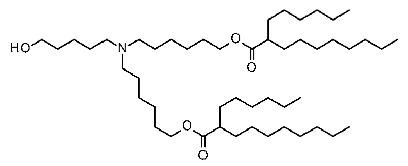 |
| III-8 | 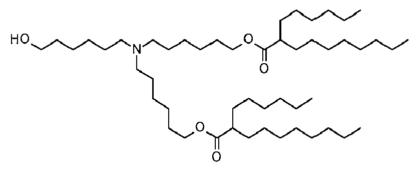 |
| III-9 | 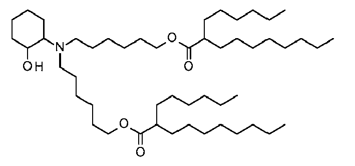 |
| III-10 | 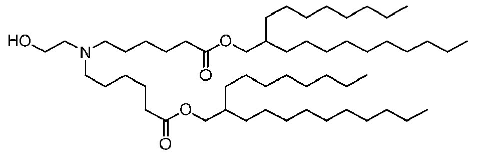 |
| III-11 | 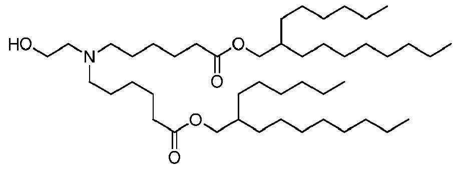 |
| III-12 | 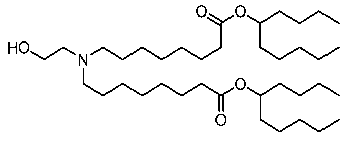 |
| III-13 | 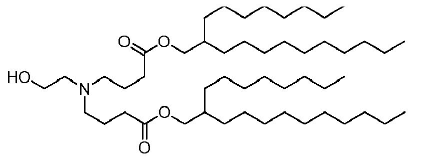 |
| III-14 | 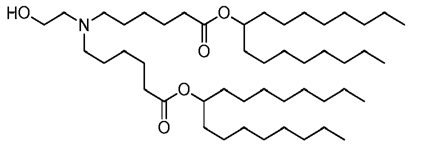 |
| III-15 | 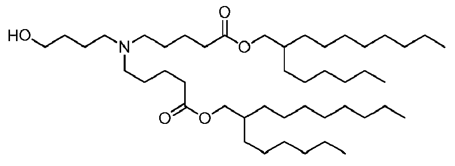 |
| III-16 | 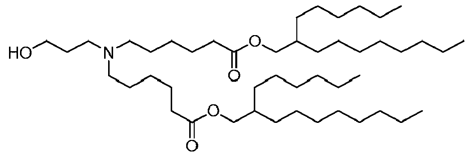 |
| III-17 | 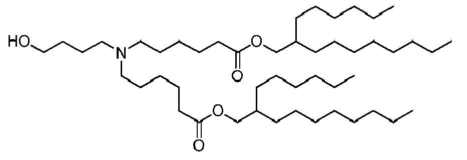 |
| III-18 | 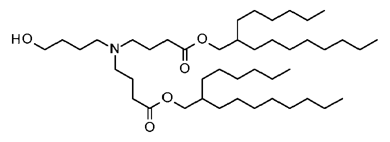 |
| III-19 | 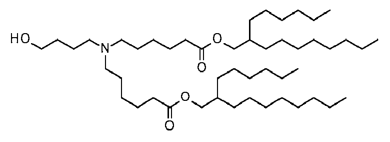 |
| III-20 | 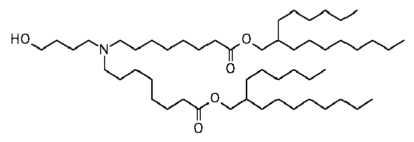 |
| III-21 | 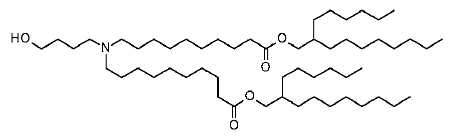 |
| III-22 | 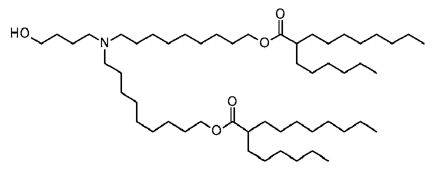 |
| III-23 | 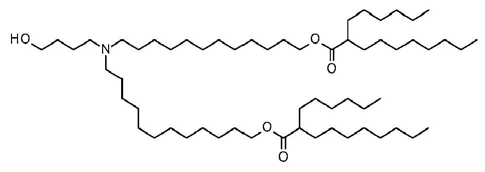 |
| III-24 | 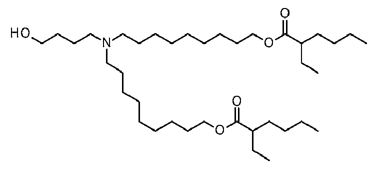 |
| III-25 | 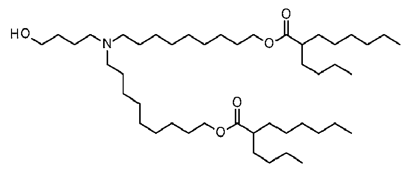 |
| III-26 | 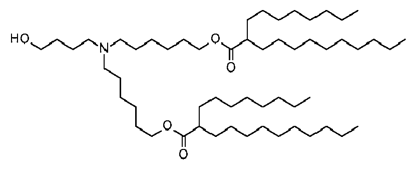 |
| III-27 | 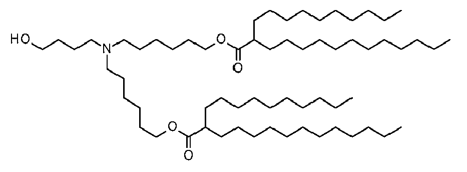 |
| III-28 | 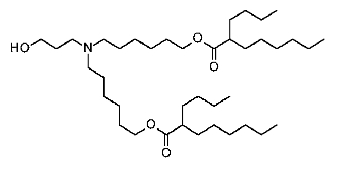 |
| III-29 | 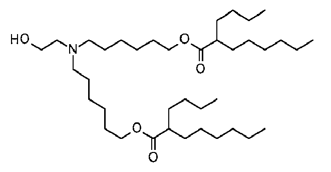 |
| III-30 | 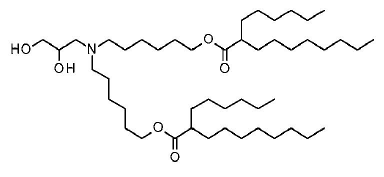 |
| III-31 | 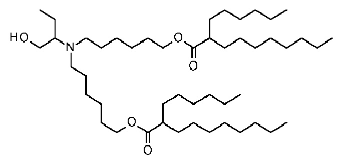 |
| III-32 | 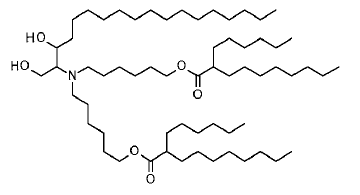 |
| III-33 | 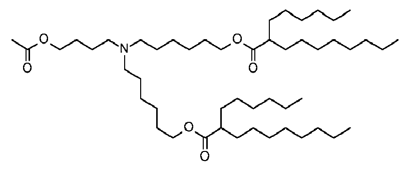 |
| III-34 | 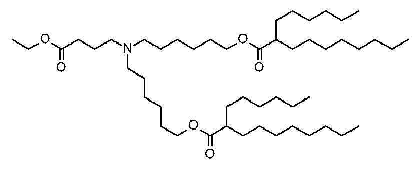 |
| III-35 | 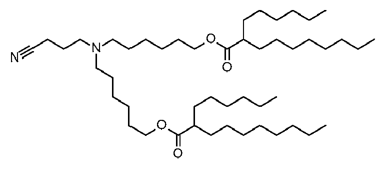 |
| III-36 | 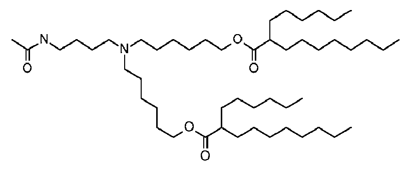 |

In some embodiments, the LNP comprises a lipid of Formula (III), RNA, a neutral lipid, a steroid and a pegylated lipid. In some embodiments, the lipid of Formula (III) is compound III-3. In some embodiments, the neutral lipid is DSPC. In some embodiments, the steroid is cholesterol. In some embodiments, the pegylated lipid is ALC-0159.

In some embodiments, the cationic lipid is present in the LNP in an amount from about 40 to about 50 mole percent. In one embodiment, the neutral lipid is present in the LNP in an amount from about 5 to about 15 mole percent. In one embodiment, the steroid is present in the LNP in an amount from about 35 to about 45 mole percent. In one embodiment, the pegylated lipid is present in the LNP in an amount from about 1 to about 10 mole percent.

In some embodiments, the LNP comprises compound III-3 in an amount from about 40 to about 50 mole percent, DSPC in an amount from about 5 to about 15 mole percent, cholesterol in an amount from about 35 to about 45 mole percent, and ALC-0159 in an amount from about 1 to about 10 mole percent.

In some embodiments, the LNP comprises compound III-3 in an amount of about 47.5 mole percent, DSPC in an amount of about 10 mole percent, cholesterol in an amount of about 40.7 mole percent, and ALC-0159 in an amount of about 1.8 mole percent.

In various different embodiments, the cationic lipid has one of the structures set forth in the table below.

**Table 18: Representative cationic lipids.**

| **No.** | **Structure** |
|---|---|
| A | 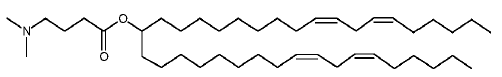 O N O |
| B | 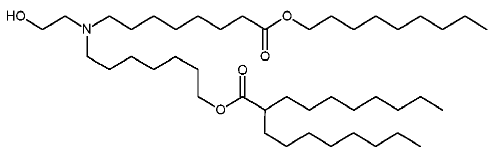 HO                    O N O O O |
| C | 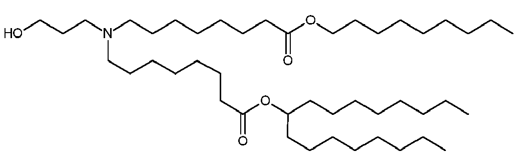 |
| D | 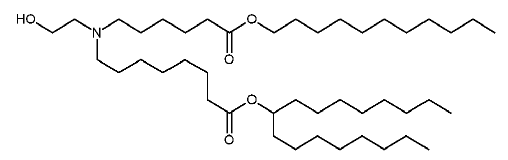 HO N O O O O O |
| E | 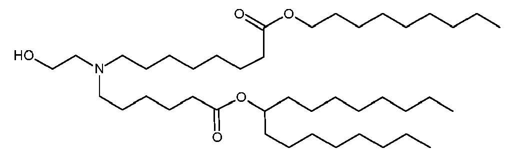 |
| F | 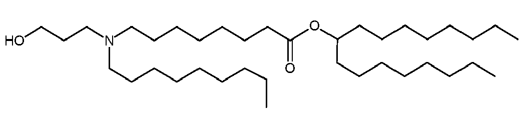 HO N O O |

In some embodiments, the LNP comprises a cationic lipid shown in the above table, e.g., a cationic lipid of Formula (B) or Formula (D), in particular a cationic lipid of Formula (D), RNA, a neutral lipid, a steroid and a pegylated lipid. In some embodiments, the neutral lipid is DSPC. In some embodiments, the steroid is cholesterol. In some embodiments, the pegylated lipid is DMG-PEG 2000.

In one embodiment, the LNP comprises a cationic lipid that is an ionizable lipid-like material (lipidoid). In one embodiment, the cationic lipid has the following structure:

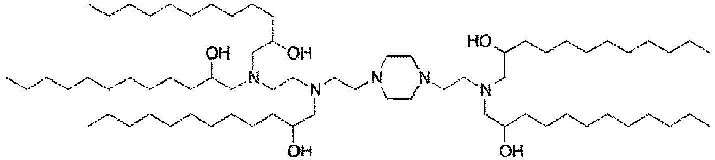

The N/P value is preferably at least about 4. In some embodiments, the N/P value ranges from 4 to 20, 4 to 12, 4 to 10, 4 to 8, or 5 to 7. In one embodiment, the N/P value is about 6.

LNP described herein may have an average diameter that in one embodiment ranges from about 30 nm to about 200 nm, or from about 60 nm to about 120 nm.

### RNA Targeting

Some aspects of the present disclosure involve the targeted delivery of the RNA disclosed herein (e.g., RNA encoding vaccine antigens and/or immunostimulants).

In one embodiment, the present disclosure involves targeting lung. Targeting lung is in particular preferred if the RNA administered is RNA encoding vaccine antigen. RNA may be delivered to lung, for example, by administering the RNA which may be formulated as particles as described herein, e.g., lipid particles, by inhalation.

In one embodiment, the present disclosure involves targeting the lymphatic system, in particular secondary lymphoid organs, more specifically spleen. Targeting the lymphatic system, in particular secondary lymphoid organs, more specifically spleen is in particular preferred if the RNA administered is RNA encoding vaccine antigen.

In one embodiment, the target cell is a spleen cell. In one embodiment, the target cell is an antigen presenting cell such as a professional antigen presenting cell in the spleen. In one embodiment, the target cell is a dendritic cell in the spleen.

The "lymphatic system" is part of the circulatory system and an important part of the immune system, comprising a network of lymphatic vessels that carry lymph. The lymphatic system consists of lymphatic organs, a conducting network of lymphatic vessels, and the circulating lymph. The primary or central lymphoid organs generate lymphocytes from immature progenitor cells. The thymus and the bone marrow constitute the primary lymphoid organs. Secondary or peripheral lymphoid organs, which include lymph nodes and the spleen, maintain mature naïve lymphocytes and initiate an adaptive immune response.

RNA may be delivered to spleen by so-called lipoplex formulations, in which the RNA is bound to liposomes comprising a cationic lipid and optionally an additional or helper lipid to form injectable nanoparticle formulations. The liposomes may be obtained by injecting a solution of the lipids in ethanol into water or a suitable aqueous phase. RNA lipoplex particles may be prepared by mixing the liposomes with RNA. Spleen targeting RNA lipoplex particles are described in WO 2013/143683, herein incorporated by reference. It has been found that RNA lipoplex particles having a net negative charge may be used to preferentially target spleen tissue or spleen cells such as antigen-presenting cells, in particular dendritic cells. Accordingly, following administration of the RNA lipoplex particles, RNA accumulation and/or RNA expression in the spleen occurs. Thus, RNA lipoplex particles of the present disclosure may be used for expressing RNA in the spleen. In an embodiment, after administration of the RNA lipoplex particles, no or essentially no RNA accumulation and/or RNA expression in the lung and/or liver occurs. In one embodiment, after administration of the RNA lipoplex particles, RNA accumulation and/or RNA expression in antigen presenting cells, such as professional antigen presenting cells in the spleen occurs. Thus, RNA lipoplex particles of the present disclosure may be used for expressing RNA in such antigen presenting cells. In one embodiment, the antigen presenting cells are dendritic cells and/or macrophages.

The electric charge of the RNA lipoplex particles of the present disclosure is the sum of the electric charges present in the at least one cationic lipid and the electric charges present in the RNA. The charge ratio is the ratio of the positive charges present in the at least one cationic lipid to the negative charges present in the RNA. The charge ratio of the positive charges present in the at least one cationic lipid to the negative charges present in the RNA is calculated by the following equation: charge ratio=[(cationic lipid concentration (mol)) * (the total number of positive charges in the cationic lipid)] / [(RNA concentration (mol)) * (the total number of negative charges in RNA)].

The spleen targeting RNA lipoplex particles described herein at physiological pH preferably have a net negative charge such as a charge ratio of positive charges to negative charges from about 1.9:2 to about 1:2, or about 1.6:2 to about 1:2, or about 1.6:2 to about 1.1:2. In specific embodiments, the charge ratio of positive charges to negative charges in the RNA lipoplex particles at physiological pH is about 1.9:2.0, about 1.8:2.0, about 1.7:2.0, about 1.6:2.0, about 1.5:2.0, about 1.4:2.0, about 1.3:2.0, about 1.2:2.0, about 1.1:2.0, or about 1:2.0.

Immunostimulants may be provided to a subject by administering to the subject RNA encoding an immunostimulant in a formulation for preferential delivery of RNA to liver or liver tissue. The delivery of RNA to such target organ or tissue is preferred, in particular, if it is desired to express large amounts of the immunostimulant and/or if systemic presence of the immunostimulant, in particular in significant amounts, is desired or required.

RNA delivery systems have an inherent preference to the liver. This pertains to lipid-based particles, cationic and neutral nanoparticles, in particular lipid nanoparticles such as liposomes, nanomicelles and lipophilic ligands in bioconjugates. Liver accumulation is caused by the discontinuous nature of the hepatic vasculature or the lipid metabolism (liposomes and lipid or cholesterol conjugates).

For *in vivo* delivery of RNA to the liver, a drug delivery system may be used to transport the RNA into the liver by preventing its degradation. For example, polyplex nanomicelles consisting of a poly(ethylene glycol) (PEG)-coated surface and an RNA (e.g., mRNA)-containing core is a useful system because the nanomicelles provide excellent *in vivo* stability of the RNA, under physiological conditions. Furthermore, the stealth property provided by the polyplex nanomicelle surface, composed of dense PEG palisades, effectively evades host immune defenses.

Examples of suitable immunostimulants for targeting liver are cytokines involved in T cell proliferation and/or maintenance. Examples of suitable cytokines include IL2 or IL7, fragments and variants thereof, and fusion proteins of these cytokines, fragments and variants, such as extended-PK cytokines.

In another embodiment, RNA encoding an immunostimulant may be administered in a formulation for preferential delivery of RNA to the lymphatic system, in particular secondary lymphoid organs, more specifically spleen. The delivery of an immunostimulant to such target tissue is preferred, in particular, if presence of the immunostimulant in this organ or tissue is desired (e.g., for inducing an immune response, in particular in case immunostimulants such as cytokines are required during T-cell priming or for activation of resident immune cells), while it is not desired that the immunostimulant is present systemically, in particular in significant amounts (e.g., because the immunostimulant has systemic toxicity).

Examples of suitable immunostimulants are cytokines involved in T cell priming. Examples of suitable cytokines include IL12, IL15, IFN-α, or IFN-β, fragments and variants thereof, and fusion proteins of these cytokines, fragments and variants, such as extended-PK cytokines.

### Immunostimulants

In one embodiment, the RNA encoding vaccine antigen may be non-immunogenic. In this and other embodiments, the RNA encoding vaccine antigen may be co-administered with an immunostimulant or RNA encoding an immunostimulant. The methods and agents described herein are particularly effective if the immunostimulant is attached to a pharmacokinetic modifying group (hereafter referred to as "extended-pharmacokinetic (PK)" immunostimulant). The methods and agents described herein are particularly effective if the immunostimulant is administered in the form of RNA encoding an immunostimulant. In one embodiment, said RNA is targeted to the liver for systemic availability. Liver cells can be efficiently transfected and are able to produce large amounts of protein.

An "immunostimulant" is any substance that stimulates the immune system by inducing activation or increasing activity of any of the immune system's components, in particular immune effector cells. The immunostimulant may be pro-inflammatory.

According to one aspect, the immunostimulant is a cytokine or a variant thereof. Examples of cytokines include interferons, such as interferon-alpha (IFN-α) or interferon-gamma (IFN-γ), interleukins, such as IL2, IL7, IL12, IL15 and IL23, colony stimulating factors, such as M-CSF and GM-CSF, and tumor necrosis factor. According to another aspect, the immunostimulant includes an adjuvant-type immunostimulatory agent such as APC Toll-like Receptor agonists or costimulatory/cell adhesion membrane proteins. Examples of Toll-like Receptor agonists include costimulatory/adhesion proteins such as CD80, CD86, and ICAM-1.

Cytokines are a category of small proteins (~5-20 kDa) that are important in cell signaling. Their release has an effect on the behavior of cells around them. Cytokines are involved in autocrine signaling, paracrine signaling and endocrine signaling as immunomodulating agents. Cytokines include chemokines, interferons, interleukins, lymphokines, and tumour necrosis factors but generally not hormones or growth factors (despite some overlap in the terminology). Cytokines are produced by a broad range of cells, including immune cells like macrophages, B lymphocytes, T lymphocytes and mast cells, as well as endothelial cells, fibroblasts, and various stromal cells. A given cytokine may be produced by more than one type of cell. Cytokines act through receptors, and are especially important in the immune system; cytokines modulate the balance between humoral and cell-based immune responses, and they regulate the maturation, growth, and responsiveness of particular cell populations. Some cytokines enhance or inhibit the action of other cytokines in complex ways.

According to the present disclosure, a cytokine may be a naturally occurring cytokine or a functional fragment or variant thereof. A cytokine may be human cytokine and may be derived from any vertebrate, especially any mammal. One particularly preferred cytokine is interferon-a.

### Interferons

Interferons (IFNs) are a group of signaling proteins made and released by host cells in response to the presence of several pathogens, such as viruses, bacteria, parasites, and also tumor cells. In a typical scenario, a virus-infected cell will release interferons causing nearby cells to heighten their anti-viral defenses.

Based on the type of receptor through which they signal, interferons are typically divided among three classes: type I interferon, type II interferon, and type III interferon.

All type I interferons bind to a specific cell surface receptor complex known as the IFN-α/β receptor (IFNAR) that consists of IFNAR1 and IFNAR2 chains.

The type I interferons present in humans are IFNα, IFNβ, IFNε, IFNκ and IFNω. In general, type I interferons are produced when the body recognizes a virus that has invaded it. They are produced by fibroblasts and monocytes. Once released, type I interferons bind to specific receptors on target cells, which leads to expression of proteins that will prevent the virus from producing and replicating its RNA and DNA.

The IFNα proteins are produced mainly by plasmacytoid dendritic cells (pDCs). They are mainly involved in innate immunity against viral infection. The genes responsible for their synthesis come in 13 subtypes that are called IFNA1, IFNA2, IFNA4, IFNA5, IFNA6, IFNA7, IFNA8, IFNA10, IFNA13, IFNA14, IFNA16, IFNA17, IFNA21. These genes are found together in a cluster on chromosome 9.

The IFNβ proteins are produced in large quantities by fibroblasts. They have antiviral activity that is involved mainly in innate immune response. Two types of IFNβ have been described, IFNβ1 and IFNβ3. The natural and recombinant forms of IFNβ1 have antiviral, antibacterial, and anticancer properties.

Type II interferon (IFNy in humans) is also known as immune interferon and is activated by IL12. Furthermore, type II interferons are released by cytotoxic T cells and T helper cells.

Type III interferons signal through a receptor complex consisting of IL10R2 (also called CRF2-4) and IFNLR1 (also called CRF2-12). Although discovered more recently than type I and type II IFNs, recent information demonstrates the importance of type III IFNs in some types of virus or fungal infections.

In general, type I and II interferons are responsible for regulating and activating the immune response.

According to the present disclosure, a type I interferon is preferably IFNα or IFNβ, more preferably IFNα.

According to the present disclosure, an interferon may be a naturally occurring interferon or a functional fragment or variant thereof. An interferon may be human interferon and may be derived from any vertebrate, especially any mammal.

### Interleukins

Interleukins (ILs) are a group of cytokines (secreted proteins and signal molecules) that can be divided into four major groups based on distinguishing structural features. However, their amino acid sequence similarity is rather weak (typically 15-25% identity). The human genome encodes more than 50 interleukins and related proteins.

According to the present disclosure, an interleukin may be a naturally occurring interleukin or a functional fragment or variant thereof. An interleukin may be human interleukin and may be derived from any vertebrate, especially any mammal.

### Extended-PK group

Immunostimulant polypeptides described herein can be prepared as fusion or chimeric polypeptides that include an immunostimulant portion and a heterologous polypeptide (i.e., a polypeptide that is not an immunostimulant). The immunostimulant may be fused to an extended-PK group, which increases circulation half-life. Non-limiting examples of extended-PK groups are described infra. It should be understood that other PK groups that increase the circulation half-life of immunostimulants such as cytokines, or variants thereof, are also applicable to the present disclosure. In certain embodiments, the extended-PK group is a serum albumin domain (e.g., mouse serum albumin, human serum albumin).

As used herein, the term "PK" is an acronym for "pharmacokinetic" and encompasses properties of a compound including, by way of example, absorption, distribution, metabolism, and elimination by a subject. As used herein, an "extended-PK group" refers to a protein, peptide, or moiety that increases the circulation half-life of a biologically active molecule when fused to or administered together with the biologically active molecule. Examples of an extended-PK group include serum albumin (e.g., HSA), Immunoglobulin Fc or Fc fragments and variants thereof, transferrin and variants thereof, and human serum albumin (HSA) binders (as disclosed in U.S. Publication Nos. 2005/0287153 and 2007/0003549). Other exemplary extended-PK groups are disclosed in Kontermann, Expert Opin Biol Ther, 2016 Jul;16(7):903-15 which is herein incorporated by reference in its entirety. As used herein, an "extended-PK" immunostimulant refers to an immunostimulant moiety in combination with an extended-PK group. In one embodiment, the extended-PK immunostimulant is a fusion protein in which an immunostimulant moiety is linked or fused to an extended-PK group.

In certain embodiments, the serum half-life of an extended-PK immunostimulant is increased relative to the immunostimulant alone (i.e., the immunostimulant not fused to an extended-PK group). In certain embodiments, the serum half-life of the extended-PK immunostimulant is at least 20, 40, 60, 80, 100, 120, 150, 180, 200, 400, 600, 800, or 1000% longer relative to the serum half-life of the immunostimulant alone. In certain embodiments, the serum half-life of the extended-PK immunostimulant is at least 1.5-fold, 2-fold, 2.5-fold, 3-fold, 3.5 fold, 4-fold, 4.5-fold, 5-fold, 6-fold, 7-fold, 8-fold, 10- fold, 12-fold, 13-fold, 15-fold, 17-fold, 20-fold, 22- fold, 25-fold, 27-fold, 30-fold, 35-fold, 40-fold, or 50-fold greater than the serum half-life of the immunostimulant alone. In certain embodiments, the serum half-life of the extended-PK immunostimulant is at least 10 hours, 15 hours, 20 hours, 25 hours, 30 hours, 35 hours, 40 hours, 50 hours, 60 hours, 70 hours, 80 hours, 90 hours, 100 hours, 110 hours, 120 hours, 130 hours, 135 hours, 140 hours, 150 hours, 160 hours, or 200 hours.

As used herein, "half-life" refers to the time taken for the serum or plasma concentration of a compound such as a peptide or protein to reduce by 50%, *in vivo,* for example due to degradation and/or clearance or sequestration by natural mechanisms. An extended-PK immunostimulant suitable for use herein is stabilized *in vivo* and its half-life increased by, e.g., fusion to serum albumin (e.g., HSA or MSA), which resist degradation and/or clearance or sequestration. The half-life can be determined in any manner known per se, such as by pharmacokinetic analysis. Suitable techniques will be clear to the person skilled in the art, and may for example generally involve the steps of suitably administering a suitable dose of the amino acid sequence or compound to a subject; collecting blood samples or other samples from said subject at regular intervals; determining the level or concentration of the amino acid sequence or compound in said blood sample; and calculating, from (a plot of) the data thus obtained, the time until the level or concentration of the amino acid sequence or compound has been reduced by 50% compared to the initial level upon dosing. Further details are provided in, e.g., standard handbooks, such as Kenneth, A. et al., Chemical Stability of Pharmaceuticals: A Handbook for Pharmacists and in Peters et al., Pharmacokinetic Analysis: A Practical Approach (1996). Reference is also made to Gibaldi, M. et al., Pharmacokinetics, 2nd Rev. Edition, Marcel Dekker (1982).

In certain embodiments, the extended-PK group includes serum albumin, or fragments thereof or variants of the serum albumin or fragments thereof (all of which for the purpose of the present disclosure are comprised by the term "albumin"). Polypeptides described herein may be fused to albumin (or a fragment or variant thereof) to form albumin fusion proteins. Such albumin fusion proteins are described in U.S. Publication No. 20070048282.

As used herein, "albumin fusion protein" refers to a protein formed by the fusion of at least one molecule of albumin (or a fragment or variant thereof) to at least one molecule of a protein such as a therapeutic protein, in particular an immunostimulant. The albumin fusion protein may be generated by translation of a nucleic acid in which a polynucleotide encoding a therapeutic protein is joined in-frame with a polynucleotide encoding an albumin. The therapeutic protein and albumin, once part of the albumin fusion protein, may each be referred to as a "portion", "region" or "moiety" of the albumin fusion protein (e.g., a "therapeutic protein portion" or an "albumin protein portion"). In a highly preferred embodiment, an albumin fusion protein comprises at least one molecule of a therapeutic protein (including, but not limited to a mature form of the therapeutic protein) and at least one molecule of albumin (including but not limited to a mature form of albumin). In one embodiment, an albumin fusion protein is processed by a host cell such as a cell of the target organ for administered RNA, e.g. a liver cell, and secreted into the circulation. Processing of the nascent albumin fusion protein that occurs in the secretory pathways of the host cell used for expression of the RNA may include, but is not limited to signal peptide cleavage; formation of disulfide bonds; proper folding; addition and processing of carbohydrates (such as for example, N- and O-linked glycosylation); specific proteolytic cleavages; and/or assembly into multimeric proteins. An albumin fusion protein is preferably encoded by RNA in a non-processed form which in particular has a signal peptide at its N-terminus and following secretion by a cell is preferably present in the processed form wherein in particular the signal peptide has been cleaved off. In a most preferred embodiment, the "processed form of an albumin fusion protein" refers to an albumin fusion protein product which has undergone N-terminal signal peptide cleavage, herein also referred to as a "mature albumin fusion protein". In preferred embodiments, albumin fusion proteins comprising a therapeutic protein have a higher plasma stability compared to the plasma stability of the same therapeutic protein when not fused to albumin. Plasma stability typically refers to the time period between when the therapeutic protein is administered *in vivo* and carried into the bloodstream and when the therapeutic protein is degraded and cleared from the bloodstream, into an organ, such as the kidney or liver, that ultimately clears the therapeutic protein from the body. Plasma stability is calculated in terms of the half-life of the therapeutic protein in the bloodstream. The half-life of the therapeutic protein in the bloodstream can be readily determined by common assays known in the art.

As used herein, "albumin" refers collectively to albumin protein or amino acid sequence, or an albumin fragment or variant, having one or more functional activities (e.g., biological activities) of albumin. In particular, "albumin" refers to human albumin or fragments or variants thereof especially the mature form of human albumin, or albumin from other vertebrates or fragments thereof, or variants of these molecules. The albumin may be derived from any vertebrate, especially any mammal, for example human, cow, sheep, or pig. Non-mammalian albumins include, but are not limited to, hen and salmon. The albumin portion of the albumin fusion protein may be from a different animal than the therapeutic protein portion.

In certain embodiments, the albumin is human serum albumin (HSA), or fragments or variants thereof, such as those disclosed in US 5,876,969, WO 2011/124718, WO 2013/075066, and WO 2011/0514789.

The terms, human serum albumin (HSA) and human albumin (HA) are used interchangeably herein. The terms, "albumin and "serum albumin" are broader, and encompass human serum albumin (and fragments and variants thereof) as well as albumin from other species (and fragments and variants thereof).

As used herein, a fragment of albumin sufficient to prolong the therapeutic activity or plasma stability of the therapeutic protein refers to a fragment of albumin sufficient in length or structure to stabilize or prolong the therapeutic activity or plasma stability of the protein so that the plasma stability of the therapeutic protein portion of the albumin fusion protein is prolonged or extended compared to the plasma stability in the non-fusion state.

The albumin portion of the albumin fusion proteins may comprise the full length of the albumin sequence, or may include one or more fragments thereof that are capable of stabilizing or prolonging the therapeutic activity or plasma stability. Such fragments may be of 10 or more amino acids in length or may include about 15, 20, 25, 30, 50, or more contiguous amino acids from the albumin sequence or may include part or all of specific domains of albumin. For instance, one or more fragments of HSA spanning the first two immunoglobulin-like domains may be used. In a preferred embodiment, the HSA fragment is the mature form of HSA.

Generally speaking, an albumin fragment or variant will be at least 100 amino acids long, preferably at least 150 amino acids long.

According to the present disclosure, albumin may be naturally occurring albumin or a fragment or variant thereof. Albumin may be human albumin and may be derived from any vertebrate, especially any mammal.

Preferably, the albumin fusion protein comprises albumin as the N-terminal portion, and a therapeutic protein as the C-terminal portion. Alternatively, an albumin fusion protein comprising albumin as the C-terminal portion, and a therapeutic protein as the N-terminal portion may also be used. In other embodiments, the albumin fusion protein has a therapeutic protein fused to both the N-terminus and the C-terminus of albumin. In a preferred embodiment, the therapeutic proteins fused at the N- and C-termini are the same therapeutic proteins. In another preferred embodiment, the therapeutic proteins fused at the N- and C-termini are different therapeutic proteins. In one embodiment, the different therapeutic proteins are both cytokines.

In one embodiment, the therapeutic protein(s) is (are) joined to the albumin through (a) peptide linker(s). A linker peptide between the fused portions may provide greater physical separation between the moieties and thus maximize the accessibility of the therapeutic protein portion, for instance, for binding to its cognate receptor. The linker peptide may consist of amino acids such that it is flexible or more rigid. The linker sequence may be cleavable by a protease or chemically.

As used herein, the term "Fc region" refers to the portion of a native immunoglobulin formed by the respective Fc domains (or Fc moieties) of its two heavy chains. As used herein, the term "Fc domain" refers to a portion or fragment of a single immunoglobulin (Ig) heavy chain wherein the Fc domain does not comprise an Fv domain. In certain embodiments, an Fc domain begins in the hinge region just upstream of the papain cleavage site and ends at the C-terminus of the antibody. Accordingly, a complete Fc domain comprises at least a hinge domain, a CH2 domain, and a CH3 domain. In certain embodiments, an Fc domain comprises at least one of: a hinge (e.g., upper, middle, and/or lower hinge region) domain, a CH2 domain, a CH3 domain, a CH4 domain, or a variant, portion, or fragment thereof. In certain embodiments, an Fc domain comprises a complete Fc domain (i.e., a hinge domain, a CH2 domain, and a CH3 domain). In certain embodiments, an Fc domain comprises a hinge domain (or portion thereof) fused to a CH3 domain (or portion thereof). In certain embodiments, an Fc domain comprises a CH2 domain (or portion thereof) fused to a CH3 domain (or portion thereof). In certain embodiments, an Fc domain consists of a CH3 domain or portion thereof. In certain embodiments, an Fc domain consists of a hinge domain (or portion thereof) and a CH3 domain (or portion thereof). In certain embodiments, an Fc domain consists of a CH2 domain (or portion thereof) and a CH3 domain. In certain embodiments, an Fc domain consists of a hinge domain (or portion thereof) and a CH2 domain (or portion thereof). In certain embodiments, an Fc domain lacks at least a portion of a CH2 domain (e.g., all or part of a CH2 domain). An Fc domain herein generally refers to a polypeptide comprising all or part of the Fc domain of an immunoglobulin heavy-chain. This includes, but is not limited to, polypeptides comprising the entire CH1, hinge, CH2, and/or CH3 domains as well as fragments of such peptides comprising only, e.g., the hinge, CH2, and CH3 domain. The Fc domain may be derived from an immunoglobulin of any species and/or any subtype, including, but not limited to, a human IgG1, IgG2, IgG3, IgG4, IgD, IgA, IgE, or IgM antibody. The Fc domain encompasses native Fc and Fc variant molecules. As set forth herein, it will be understood by one of ordinary skill in the art that any Fc domain may be modified such that it varies in amino acid sequence from the native Fc domain of a naturally occurring immunoglobulin molecule. In certain embodiments, the Fc domain has reduced effector function (e.g., FcγR binding).

The Fc domains of a polypeptide described herein may be derived from different immunoglobulin molecules. For example, an Fc domain of a polypeptide may comprise a CH2 and/or CH3 domain derived from an IgG1 molecule and a hinge region derived from an IgG3 molecule. In another example, an Fc domain can comprise a chimeric hinge region derived, in part, from an IgG1 molecule and, in part, from an IgG3 molecule. In another example, an Fc domain can comprise a chimeric hinge derived, in part, from an IgG1 molecule and, in part, from an IgG4 molecule.

In certain embodiments, an extended-PK group includes an Fc domain or fragments thereof or variants of the Fc domain or fragments thereof (all of which for the purpose of the present disclosure are comprised by the term "Fc domain"). The Fc domain does not contain a variable region that binds to antigen. Fc domains suitable for use in the present disclosure may be obtained from a number of different sources. In certain embodiments, an Fc domain is derived from a human immunoglobulin. In certain embodiments, the Fc domain is from a human IgG1 constant region. It is understood, however, that the Fc domain may be derived from an immunoglobulin of another mammalian species, including for example, a rodent (e.g. a mouse, rat, rabbit, guinea pig) or non- human primate (e.g. chimpanzee, macaque) species. Moreover, the Fc domain (or a fragment or variant thereof) may be derived from any immunoglobulin class, including IgM, IgG, IgD, IgA, and IgE, and any immunoglobulin isotype, including IgG1, IgG2, IgG3, and IgG4.

A variety of Fc domain gene sequences (e.g., mouse and human constant region gene sequences) are available in the form of publicly accessible deposits. Constant region domains comprising an Fc domain sequence can be selected lacking a particular effector function and/or with a particular modification to reduce immunogenicity. Many sequences of antibodies and antibody-encoding genes have been published and suitable Fc domain sequences (e.g. hinge, CH2, and/or CH3 sequences, or fragments or variants thereof) can be derived from these sequences using art recognized techniques.

In certain embodiments, the extended-PK group is a serum albumin binding protein such as those described in US2005/0287153, US2007/0003549, US2007/0178082, US2007/0269422, US2010/0113339, WO2009/083804, and WO2009/133208, which are herein incorporated by reference in their entirety. In certain embodiments, the extended-PK group is transferrin, as disclosed in US 7,176,278 and US 8,158,579, which are herein incorporated by reference in their entirety. In certain embodiments, the extended-PK group is a serum immunoglobulin binding protein such as those disclosed in US2007/0178082, US2014/0220017, and US2017/0145062, which are herein incorporated by reference in their entirety. In certain embodiments, the extended-PK group is a fibronectin (Fn)-based scaffold domain protein that binds to serum albumin, such as those disclosed in US2012/0094909, which is herein incorporated by reference in its entirety. Methods of making fibronectin-based scaffold domain proteins are also disclosed in US2012/0094909. A non-limiting example of a Fn3-based extended-PK group is Fn3(HSA), i.e., a Fn3 protein that binds to human serum albumin.

In certain aspects, the extended-PK immunostimulant, suitable for use according to the present disclosure, can employ one or more peptide linkers. As used herein, the term "peptide linker" refers to a peptide or polypeptide sequence which connects two or more domains (e.g., the extended-PK moiety and an immunostimulant moiety) in a linear amino acid sequence of a polypeptide chain. For example, peptide linkers may be used to connect an immunostimulant moiety to a HSA domain.

Linkers suitable for fusing the extended-PK group to e.g. an immunostimulant are well known in the art. Exemplary linkers include glycine-serine-polypeptide linkers, glycine-proline-polypeptide linkers, and proline-alanine polypeptide linkers. In certain embodiments, the linker is a glycine-serine-polypeptide linker, i.e., a peptide that consists of glycine and serine residues.

In addition to, or in place of, the heterologous polypeptides described above, an immunostimulant polypeptide described herein can contain sequences encoding a "marker" or "reporter". Examples of marker or reporter genes include P-lactamase, chloramphenicol acetyltransferase (CAT), adenosine deaminase (ADA), aminoglycoside phosphotransferase, dihydrofolate reductase (DHFR), hygromycin-B-hosphotransferase (HPH), thymidine kinase (TK), β-galactosidase, and xanthine guanine phosphoribosyltransferase (XGPRT).

### Pharmaceutical compositions

The agents described herein may be administered in pharmaceutical compositions or medicaments and may be administered in the form of any suitable pharmaceutical composition.

In one embodiment, the pharmaceutical composition described herein is an immunogenic composition for inducing an immune response against coronavirus in a subject. For example, in one embodiment, the immunogenic composition is a vaccine.

In one embodiment of all aspects of the present disclosure, the components described herein such as RNA encoding a vaccine antigen may be administered in a pharmaceutical composition which may comprise a pharmaceutically acceptable carrier and may optionally comprise one or more adjuvants, stabilizers etc. In one embodiment, the pharmaceutical composition is for therapeutic or prophylactic treatments, e.g., for use in treating or preventing a coronavirus infection.

The term "pharmaceutical composition" relates to a formulation comprising a therapeutically effective agent, preferably together with pharmaceutically acceptable carriers, diluents and/or excipients. Said pharmaceutical composition is useful for treating, preventing, or reducing the severity of a disease or disorder by administration of said pharmaceutical composition to a subject. A pharmaceutical composition is also known in the art as a pharmaceutical formulation.

The pharmaceutical compositions of the present disclosure may comprise one or more adjuvants or may be administered with one or more adjuvants. The term "adjuvant" relates to a compound which prolongs, enhances or accelerates an immune response. Adjuvants comprise a heterogeneous group of compounds such as oil emulsions (e.g., Freund's adjuvants), mineral compounds (such as alum), bacterial products (such as Bordetella pertussis toxin), or immune-stimulating complexes. Examples of adjuvants include, without limitation, LPS, GP96, CpG oligodeoxynucleotides, growth factors, and cytokines, such as monokines, lymphokines, interleukins, chemokines. The cytokines may be IL1, IL2, IL3, IL4, IL5, IL6, IL7, IL8, IL9, IL10, IL12, IFNα, IFNγ, GM-CSF, LT-a. Further known adjuvants are aluminium hydroxide, Freund's adjuvant or oil such as Montanide^{®} ISA51. Other suitable adjuvants for use in the present disclosure include lipopeptides, such as Pam3Cys.

The pharmaceutical compositions according to the present disclosure are generally applied in a "pharmaceutically effective amount" and in "a pharmaceutically acceptable preparation". The term "pharmaceutically acceptable" refers to the non-toxicity of a material which does not interact with the action of the active component of the pharmaceutical composition.

The term "pharmaceutically effective amount" or "therapeutically effective amount" refers to the amount which achieves a desired reaction or a desired effect alone or together with further doses. In the case of the treatment of a particular disease, the desired reaction preferably relates to inhibition of the course of the disease. This comprises slowing down the progress of the disease and, in particular, interrupting or reversing the progress of the disease. The desired reaction in a treatment of a disease may also be delay of the onset or a prevention of the onset of said disease or said condition. An effective amount of the compositions described herein will depend on the condition to be treated, the severeness of the disease, the individual parameters of the patient, including age, physiological condition, size and weight, the duration of treatment, the type of an accompanying therapy (if present), the specific route of administration and similar factors. Accordingly, the doses administered of the compositions described herein may depend on various of such parameters. In the case that a reaction in a patient is insufficient with an initial dose, higher doses (or effectively higher doses achieved by a different, more localized route of administration) may be used.

The pharmaceutical compositions of the present disclosure may contain salts, buffers, preservatives, and optionally other therapeutic agents. In one embodiment, the pharmaceutical compositions of the present disclosure comprise one or more pharmaceutically acceptable carriers, diluents and/or excipients.

Suitable preservatives for use in the pharmaceutical compositions of the present disclosure include, without limitation, benzalkonium chloride, chlorobutanol, paraben and thimerosal.

The term "excipient" as used herein refers to a substance which may be present in a pharmaceutical composition of the present disclosure but is not an active ingredient. Examples of excipients, include without limitation, carriers, binders, diluents, lubricants, thickeners, surface active agents, preservatives, stabilizers, emulsifiers, buffers, flavoring agents, or colorants.

The term "diluent" relates a diluting and/or thinning agent. Moreover, the term "diluent" includes any one or more of fluid, liquid or solid suspension and/or mixing media. Examples of suitable diluents include ethanol, glycerol and water.

The term "carrier" refers to a component which may be natural, synthetic, organic, inorganic in which the active component is combined in order to facilitate, enhance or enable administration of the pharmaceutical composition. A carrier as used herein may be one or more compatible solid or liquid fillers, diluents or encapsulating substances, which are suitable for administration to subject. Suitable carrier include, without limitation, sterile water, Ringer, Ringer lactate, sterile sodium chloride solution, isotonic saline, polyalkylene glycols, hydrogenated naphthalenes and, in particular, biocompatible lactide polymers, lactide/glycolide copolymers or polyoxyethylene/polyoxy-propylene copolymers. In one embodiment, the pharmaceutical composition of the present disclosure includes isotonic saline.

Pharmaceutically acceptable carriers, excipients or diluents for therapeutic use are well known in the pharmaceutical art, and are described, for example, in Remington's Pharmaceutical Sciences, Mack Publishing Co. (A. R Gennaro edit. 1985).

Pharmaceutical carriers, excipients or diluents can be selected with regard to the intended route of administration and standard pharmaceutical practice.

In one embodiment, pharmaceutical compositions described herein may be administered intravenously, intraarterially, subcutaneously, intradermally or intramuscularly. In certain embodiments, the pharmaceutical composition is formulated for local administration or systemic administration. Systemic administration may include enteral administration, which involves absorption through the gastrointestinal tract, or parenteral administration. As used herein, "parenteral administration" refers to the administration in any manner other than through the gastrointestinal tract, such as by intravenous injection. In a preferred embodiment, the pharmaceutical composition is formulated for intramuscular administration. In another embodiment, the pharmaceutical composition is formulated for systemic administration, e.g., for intravenous administration.

The term "co-administering" as used herein means a process whereby different compounds or compositions (e.g., RNA encoding an antigen and RNA encoding an immunostimulant) are administered to the same patient. The different compounds or compositions may be administered simultaneously, at essentially the same time, or sequentially.

The pharmaceutical compositions and products described herein may be provided as a frozen concentrate for solution for injection, e.g., at a concentration of 0.50 mg/mL. In one embodiment, for preparation of solution for injection, a drug product is thawed and diluted with isotonic sodium chloride solution (e.g., 0.9% NaCl, saline), e.g., by a one-step dilution process. In some embodiments, bacteriostatic sodium chloride solution (e.g., 0.9% NaCl, saline) cannot be used as a diluent. In some embodiments, a diluted drug product is an off-white suspension. The concentration of the final solution for injection varies depending on the respective dose level to be administered.

In one embodiment, administration is performed within 6 h after begin of preparation due to the risk of microbial contamination and considering the multiple-dose approach of the preparation process. In one embodiment, in this period of 6 h, two conditions are allowed: room temperature for preparation, handling and transfer as well as 2 to 8°C for storage.

Compositions described herein may be shipped and/or stored under temperature-controlled conditions, e.g., temperature conditions of about 4-5°C or below, about -20°C or below, - 70°C±10°C (e.g., -80°C to -60°C), e.g., utilizing a cooling system (e.g., that may be or include dry ice) to maintain the desired temperature. In one embodiment, compositions described herein are shipped in temperature-controlled thermal shippers. Such shippers may contain a GPS-enabled thermal sensor to track the location and temperature of each shipment. The compositions can be stored by refilling with, e.g., dry ice.

### Treatments

The present disclosure provides methods and agents for inducing an adaptive immune response against coronavirus in a subject comprising administering an effective amount of a composition comprising RNA encoding a coronavirus vaccine antigen described herein.

In one embodiment, the methods and agents described herein provide immunity in a subject to coronavirus, coronavirus infection, or to a disease or disorder associated with coronavirus. The present disclosure thus provides methods and agents for treating or preventing the infection, disease, or disorder associated with coronavirus.

In one embodiment, the methods and agents described herein are administered to a subject having an infection, disease, or disorder associated with coronavirus. In one embodiment, the methods and agents described herein are administered to a subject at risk for developing the infection, disease, or disorder associated with coronavirus. For example, the methods and agents described herein may be administered to a subject who is at risk for being in contact with coronavirus. In one embodiment, the methods and agents described herein are administered to a subject who lives in, traveled to, or is expected to travel to a geographic region in which coronavirus is prevalent. In one embodiment, the methods and agents described herein are administered to a subject who is in contact with or expected to be in contact with another person who lives in, traveled to, or is expected to travel to a geographic region in which coronavirus is prevalent. In one embodiment, the methods and agents described herein are administered to a subject who has knowingly been exposed to coronavirus through their occupation, or other contact. In one embodiment, a coronavirus is SARS-CoV-2. In some embodiments, methods and agents described herein are administered to a subject with evidence of prior exposure to and/or infection with SARS-CoV-2 and/or an antigen or epitope thereof or cross-reactive therewith. For example, in some embodiments, methods and agents described herein are administered to a subject in whom antibodies, B cells, and/or T cells reactive with one or more epitopes of a SARS-CoV-2 spike protein are detectable and/or have been detected.

For a composition to be useful as a vaccine, the composition must induce an immune response against the coronavirus antigen in a cell, tissue or subject (e.g., a human). In some embodiments, the composition induces an immune response against the coronavirus antigen in a cell, tissue or subject (e.g., a human). In some instances, the vaccine induces a protective immune response in a mammal. The therapeutic compounds or compositions of the present disclosure may be administered prophylactically (i.e., to prevent a disease or disorder) or therapeutically (i.e., to treat a disease or disorder) to subjects suffering from, or at risk of (or susceptible to) developing a disease or disorder. Such subjects may be identified using standard clinical methods. In the context of the present disclosure, prophylactic administration occurs prior to the manifestation of overt clinical symptoms of disease, such that a disease or disorder is prevented or alternatively delayed in its progression. In the context of the field of medicine, the term "prevent" encompasses any activity, which reduces the burden of mortality or morbidity from disease. Prevention can occur at primary, secondary and tertiary prevention levels. While primary prevention avoids the development of a disease, secondary and tertiary levels of prevention encompass activities aimed at preventing the progression of a disease and the emergence of symptoms as well as reducing the negative impact of an already established disease by restoring function and reducing disease-related complications.

The term "dose" as used herein refers in general to a "dose amount" which relates to the amount of RNA administered per administration, i.e., per dosing.

In some embodiments, administration of an immunogenic composition or vaccine of the present disclosure may be performed by single administration or boosted by multiple administrations.

In some embodiments, a regimen described herein includes at least one dose. In some embodiments, a regimen includes a first dose and at least one subsequent dose. In some embodiments, the first dose is the same amount as at least one subsequent dose. In some embodiments, the first dose is the same amount as all subsequent doses. In some embodiments, the first dose is a different amount as at least one subsequent dose. In some embodiments, the first dose is a different amount than all subsequent doses. In some embodiments, a regimen comprises two doses. In some embodiments, a provided regimen consists of two doses. In some embodiments, a regimen comprises three doses.

In one embodiment, the present disclosure envisions administration of a single dose. In one embodiment, the present disclosure envisions administration of a priming dose followed by one or more booster doses. The booster dose or the first booster dose may be administered 7 to 28 days or 14 to 24 days following administration of the priming dose. In some embodiments, a first booster dose may be administered 1 week to 3 months (e.g., 1 week, 2 weeks, 3 weeks, 4 weeks, 5 weeks, 6 weeks, 7 weeks, 8 weeks, 9 weeks, 10 weeks, 11 weeks, 12 weeks) following administration of a priming dose. In some embodiments, a subsequent booster dose may be administered at least 1 week or longer, including, e.g., at least 2 weeks, at least 3 weeks, at least 4 weeks, at least 5 weeks, at least 6 weeks, at least 7 weeks, at least 8 weeks, at least 9 weeks, at least 10 weeks, at least 11 weeks, at least 12 weeks, or longer, following a preceding booster dose. In some embodiments, subsequent booster doses may be administered about 5-9 weeks or 6-8 weeks apart. In some embodiments, at least one subsequent booster dose (e.g., after a first booster dose) may be administered at least 3 months or longer, including, e.g., at least 4 months, at least 5 months, at least 6 months, at least 7 months, at least 8 months, at least 9 months, at least 10 months, or longer, following a preceding dose.

In some embodiments, a subsequent dose given to an individual (e.g., as part of a primary regimen or booster regimen) can have the same amount of RNA as previously given to the individual. In some embodiments, a subsequent dose given to an individual (e.g., as part of a primary regimen or booster regimen) can differ in the amount of RNA, as compared to the amount previously given to the individual. For example, in some embodiments, a subsequent dose can be higher or lower than the prior dose, for example, based on consideration of various factors, including, e.g., immunogenicity and/or reactogenicity induced by the prior dose, prevalence of the disease, etc. In some embodiments, a subsequent dose can be higher than a prior dose by at least 30%, at least 40%, at least 50%, at least 60%, at least 70%, at least 80%, at least 90%, or higher. In some embodiments, a subsequent dose can be higher than a prior dose by at least 1.5-fold, at least 2-fold, at least 2.5 fold, at least 3-fold, or higher. In some embodiments, a subsequent dose can be higher than a prior dose by at least 30%, at least 40%, at least 50%, at least 60%, at least 70%, at least 80%, at least 90%, or higher. In some embodiments, a subsequent dose can be lower than a prior dose by at least 10%, at least 20%, at least 30%, at least 40%, at least 50%, at least 60%, at least 70% or lower.In some embodiments, an amount the RNA described herein from 0.1 µg to 300 µg, 0.5 µg to 200 µg, or 1 µg to 100 µg, such as about 1 µg, about 2 µg, about 3 µg, about 10 µg, about 15 µg, about 20 µg, about 25 µg, about 30 µg, about 35 µg, about 40 µg, about 45 µg, about 50 µg, about 55 µg, about 60 µg, about 70 µg, about 80 µg, about 90 µg, or about 100 µg may be administered per dose (e.g., in a given dose).

In some embodiments, an amount of the RNA described herein of 60 µg or lower, 55 µg or lower, 50 µg or lower, 45 µg or lower, 40 µg or lower, 35 µg or lower, 30 µg or lower, 25 µg or lower, 20 µg or lower, 15 µg or lower, 10 µg or lower, 5 µg or lower, 3 µg or lower, 2.5 µg or lower, or 1 µg or lower may be administered per dose (e.g., in a given dose).

In some embodiments, an amount of the RNA described herein of at least 0.25 µg, at least 0.5 µg, at least 1 µg, at least 2 µg, at least 3 µg, at least 4 µg, at least 5 µg, at least 10 µg, at least 15 µg, at least 20 µg, at least 25 µg, at least 30 µg, at least 40 µg, at least 50 µg, or at least 60 µg may be administered per dose (e.g., in a given dose). In some embodiments, an amount of the RNA described herein of at least 3 ug may be administered in at least one of given doses. In some embodiments, an amount of the RNA described herein of at least 10 ug may be administered in at least one of given doses. In some embodiments, an amount of the RNA described herein of at least 15 ug may be administered in at least one of given doses. In some embodiments, an amount of the RNA described herein of at least 20 ug may be administered in at least one of given doses. In some embodiments, an amount of the RNA described herein of at least 25 ug may be administered in at least one of given doses. In some embodiments, an amount of the RNA described herein of at least 30 ug may be administered in at least one of given doses. In some embodiments, an amount of the RNA described herein of at least 50 ug may be administered in at least one of given doses. In some embodiments, an amount of the RNA described herein of at least 60 ug may be administered in at least one of given doses. In some embodiments, combinations of aforementioned amounts may be administered in a regimen comprising two or more doses (e.g., a prior dose and a subsequent dose can be of different amounts as described herein). In some embodiments, combinations of aforementioned amounts may be administered in a primary regimen and a booster regimen (e.g., different doses can be given in a primary regimen and a booster regimen).

In some embodiments, an amount of the RNA described herein of 0.25 µg to 60 µg, 0.5 µg to 55 µg, 1 µg to 50 µg, 5 µg to 40 µg, or 10 µg to 30 µg may be administered per dose. In some embodiments, an amount of the RNA described herein of 3 µg to 30 µg may be administered in at least one of given doses. In some embodiments, an amount of the RNA described herein of 3 µg to 20 µg may be administered in at least one of given doses. In some embodiments, an amount of the RNA described herein of 3 µg to 15 µg may be administered in at least one of given doses. In some embodiments, an amount of the RNA described herein of 3 µg to 10 µg may be administered in at least one of given doses. In some embodiments, an amount of the RNA described herein of 10 µg to 30 µg may be administered in at least one of given doses.

In some embodiments, a regimen administered to a subject may comprise a plurality of doses (e.g., at least two doses, at least three doses, or more). In some embodiments, a regimen administered to a subject may comprise a first dose and a second dose, which are given at least 2 weeks apart, at least 3 weeks apart, at least 4 weeks apart, or more. In some embodiments, such doses may be at least 1 month, at least 2 months, at least 3 months, at least 4 months, at least 5 months, at least 6 months, at least 7 months, at least 8 months, at least 9 months, at least 10 months, at least 11 months, at least 12 months, or more apart. In some embodiments, doses may be administered days apart, such as 1, 2, 3, 4, 5, 6, 7, 8, 9 ,10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or more days apart. In some embodiments, doses may be administered about 1 to about 3 weeks apart, or about 1 to about 4 weeks apart, or about 1 to about 5 weeks apart, or about 1 to about 6 weeks apart, or about 1 to more than 6 weeks apart. In some embodiments, doses may be separated by a period of about 7 to about 60 days, such as for example about 14 to about 48 days, etc. In some embodiments, a minimum number of days between doses may be about 1, 2, 3, 4, 5, 6, 7, 8, 9, 10, 11, 12, 13, 14, 15, 16, 17, 18, 19, 20, 21 or more. In some embodiments, a maximum number of days between doses may be about 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, or fewer. In some embodiments, doses may be about 21 to about 28 days apart. In some embodiments, doses may be about 19 to about 42 days apart. In some embodiments, doses may be about 7 to about 28 days apart. In some embodiments, doses may be about 14 to about 24 days. In some embodiments, doses may be about 21 to about 42 days.

In some embodiments, a vaccination regimen comprises a first dose and a second dose. In some embodiments, a first dose and a second dose are administered by at least 21 days apart. In some embodiments, a first dose and a second dose are administered by at least 28 days apart.

In some embodiments, a vaccination regimen comprises a first dose and a second dose, wherein the amount of RNA administered in the first dose is the same as the amount of RNA administered in the second dose. In some embodiments, a vaccination regimen comprises a first dose and a second dose wherein the amount of RNA administered in the first dose differs from that administered in the second dose.

In some embodiments, a vaccination regimen comprises a first dose and a second dose, wherein the amount of RNA administered in the first dose is less than that administered in the second dose. In some embodiments, the amount of RNA administered in the first dose is 10%-90% of the second dose. In some embodiments, the amount of RNA administered in the first dose is 10%-50% of the second dose. In some embodiments, the amount of RNA administered in the first dose is 10%-20% of the second dose. In some embodiments, the first dose and the second dose are administered at least 2 weeks apart, including, at least 3 weeks apart, at least 4 weeks apart, at least 5 weeks apart, at least 6 weeks apart or longer. In some embodiments, the first dose and the second dose are administered at least 3 weeks apart.

In some embodiments, a first dose comprises less than about 30 ug of RNA and a second dose comprises at least about 30 ug of RNA. In some embodiments, a first dose comprises about 1 to less than about 30 ug of RNA (e.g., about 0.1, about 1, about 3, about 5, about 10, about 15, about 20, about 25, or less than about 30 ug of RNA) and a second dose comprises about 30 to about 100 ug of RNA (e.g., about 30, about 40, about 50, or about 60 ug of RNA). In some embodiments, a first dose comprises about 1 to about 20 ug of RNA, about 1 to about 10 ug of RNA, or about 1 to about 5 ug of RNA and a second dose comprises about 30 to about 60 ug of RNA.

In some embodiments, a first dose comprises about 1 to about 10 ug of RNA (e.g., about 1, about 2, about 3, about 4, about 5, about 6, about 7, about 8, about 9, or about 10 ug of RNA) and a second dose comprises about 30 to about 60 ug of RNA (e.g., about 30, about 35, about 40, about 45, about 50, about 55, or about 60 ug of RNA).

In some embodiments, a first dose comprises about 1 ug of RNA and a second dose comprises about 30 ug of RNA. In some embodiments, a first dose comprises about 3 ug of RNA and a second dose comprises about 30 ug of RNA. In some embodiments, a first dose comprises about 5 ug of RNA and a second dose comprises about 30 ug of RNA. In some embodiments, a first dose comprises about 10 ug of RNA and a second dose comprises about 30 ug of RNA. In some embodiments, a first dose comprises about 15 ug of RNA and a second dose comprises about 30 ug of RNA.

In some embodiments, a first dose comprises about 1 ug of RNA and a second dose comprises about 60 ug of RNA. In some embodiments, a first dose comprises about 3 ug of RNA and a second dose comprises about 60 ug of RNA. In some embodiments, a first dose comprises about 5 ug of RNA and a second dose comprises about 60 ug of RNA. In some embodiments, a first dose comprises about 6 ug of RNA and a second dose comprises about 60 ug of RNA. In some embodiments, a first dose comprises about 10 ug of RNA and a second dose comprises about 60 ug of RNA. In some embodiments, a first dose comprises about 15 ug of RNA and a second dose comprises about 60 ug of RNA. In some embodiments, a first dose comprises about 20 ug of RNA and a second dose comprises about 60 ug of RNA. In some embodiments, a first dose comprises about 25 ug of RNA and a second dose comprises about 60 ug of RNA. In some embodiments, a first dose comprises about 30 ug of RNA and a second dose comprises about 60 ug of RNA.

In some embodiments, a first dose comprises less than about 10 ug of RNA and a second dose comprises at least about 10 ug of RNA. In some embodiments, a first dose comprises about 0.1 to less than about 10 ug of RNA (e.g., about 0.1, about 0.5, about 1, about 2, about 3, about 4, about 5, about 6, about 7, about 8, or less than about 10 ug of RNA) and a second dose comprises about 10 to about 30 ug of RNA (e.g., about 10, about 15, about 20, about 25, or about 30 ug of RNA). In some embodiments, a first dose comprises about 0.1 to about 10 ug of RNA, about 1 to about 5 ug of RNA, or about 0.1 to about 3 ug of RNA and a second dose comprises about 10 to about 30 ug of RNA.

In some embodiments, a first dose comprises about 0.1 to about 5 ug of RNA (e.g., about 0.1, about 0.5, about 1, about 2, about 3, about 4, about 5ug of RNA) and a second dose comprises about 10 to about 20 ug of RNA (e.g., about 10, about 12, about 14, about 16, about 18, about 20ug of RNA).

In some embodiments, a first dose comprises about 0.1 ug of RNA and a second dose comprises about 10 ug of RNA. In some embodiments, a first dose comprises about 0.3 ug of RNA and a second dose comprises about 10 ug of RNA. In some embodiments, a first dose comprises about 1 ug of RNA and a second dose comprises about 10 ug of RNA. In some embodiments, a first dose comprises about 3 ug of RNA and a second dose comprises about 10 ug of RNA.

In some embodiments, a first dose comprises less than about 3 ug of RNA and a second dose comprises at least about 3 ug of RNA. In some embodiments, a first dose comprises about 0.1 to less than about 3 ug of RNA (e.g., about 0.1, about 0.2, about 0.3, about 0.5, about 0.6, about 0.7, about 0.8, about 0.9, about 1.0, about 1.5, about 2.0, or about 2.5 ug of RNA) and a second dose comprises about 3 to about 10 ug of RNA (e.g., about 3, about 4, about 5, about 6, or about 7, about 8, about 9, or about 10 ug of RNA). In some embodiments, a first dose comprises about 0.1 to about 3 ug of RNA, about 0.1 to about 1 ug of RNA, or about 0.1 to about 0.5 ug of RNA and a second dose comprises about 3 to about 10 ug of RNA.

In some embodiments, a first dose comprises about 0.1 to about 1.0 ug of RNA (e.g., about 0.1, about 0.2, about 0.3, about 0.4, about 0.5, about 0.6, about 0.7, about 0.8, about 0.9, or about 1.0 ug of RNA) and a second dose comprises about 1 to about 3 ug of RNA (e.g., about 1.0, about 1.5, about 2.0, about 2.5, or about 3.0 ug of RNA).

In some embodiments, a first dose comprises about 0.1 ug of RNA and a second dose comprises about 3 ug of RNA. In some embodiments, a first dose comprises about 0.3 ug of RNA and a second dose comprises about 3 ug of RNA. In some embodiments, a first dose comprises about 0.5 ug of RNA and a second dose comprises about 3 ug of RNA. In some embodiments, a first dose comprises about 1 ug of RNA and a second dose comprises about 3 ug of RNA.

In some embodiments, a vaccination regimen comprises a first dose and a second dose, wherein the amount of RNA administered in the first dose is greater than that administered in the second dose. In some embodiments, the amount of RNA administered in the second dose is 10%-90% of the first dose. In some embodiments, the amount of RNA administered in the second dose is 10%-50% of the first dose. In some embodiments, the amount of RNA administered in the second dose is 10%-20% of the first dose. In some embodiments, the first dose and the second dose are administered at least 2 weeks apart, including, at least 3 weeks apart, at least 4 weeks apart, at least 5 weeks apart, at least 6 weeks apart or longer. In some embodiments, the first dose and the second dose are administered at least 3 weeks apart

In some embodiments, a first dose comprises at least about 30 ug of RNA and a second dose comprises less than about 30 ug of RNA. In some embodiments, a first dose comprises about 30 to about 100 ug of RNA (e.g., about 30, about 40, about 50, or about 60 ug of RNA) and a second dose comprises about 1 to about 30 ug of RNA (e.g., about 0.1, about 1, about 3, about 5, about 10, about 15, about 20, about 25, or about 30 ug of RNA). In some embodiments, a second dose comprises about 1 to about 20 ug of RNA, about 1 to about 10 ug of RNA, or about 1 to 5 ug of RNA. In some embodiments, a first dose comprises about 30 to about 60 ug of RNA and a second dose comprises about 1 to about 20 ug of RNA, about 1 to about 10 ug of RNA, or about 0.1 to about 3 ug of RNA.

In some embodiments, a first dose comprises about 30 to about 60 ug of RNA (e.g., about 30, about 35, about 40, about 45, about 50, about 55, or about 60 ug of RNA) and a second dose comprises about 1 to about 10 ug of RNA (e.g., about 1, about 2, about 3, about 4, about 5, about 6, about 7, about 8, about 9, or about 10 ug of RNA).

In some embodiments, a first dose comprises about 30 ug of RNA and a second dose comprises about 1 ug of RNA. In some embodiments, a first dose comprises about 30 ug of RNA and a second dose comprises about 3 ug of RNA. In some embodiments, a first dose comprises about 30 ug of RNA and a second dose comprises about 5 ug of RNA. In some embodiments, a first dose comprises about 30 ug of RNA and a second dose comprises about 10 ug of RNA. In some embodiments, a first dose comprises about 30 ug of RNA and a second dose comprises about 15 ug of RNA.

In some embodiments, a first dose comprises about 60 ug of RNA and a second dose comprises about 1 ug of RNA. In some embodiments, a first dose comprises about 60 ug of RNA and a second dose comprises about 3 ug of RNA. In some embodiments, a first dose comprises about 60 ug of RNA and a second dose comprises about 5 ug of RNA. In some embodiments, a first dose comprises about 60 ug of RNA and a second dose comprises about 6 ug of RNA. In some embodiments, a first dose comprises about 60 ug of RNA and a second dose comprises about 10 ug of RNA. In some embodiments, a first dose comprises about 60 ug of RNA and a second dose comprises about 15 ug of RNA. In some embodiments, a first dose comprises about 60 ug of RNA and a second dose comprises about 20 ug of RNA. In some embodiments, a first dose comprises about 60 ug of RNA and a second dose comprises about 25 ug of RNA. In some embodiments, a first dose comprises about 60 ug of RNA and a second dose comprises about 30 ug of RNA.

In some embodiments, a first dose comprises at least about 10 ug of RNA and a second dose comprises less than about 10 ug of RNA. In some embodiments, a first dose comprises about 10 to about 30 ug of RNA (e.g., about 10, about 15, about 20, about 25, or about 30 ug of RNA) and a second dose comprises about 0.1 to less than about 10 ug of RNA (e.g., about 0.1, about 0.5, about 1, about 2, about 3, about 4, about 5, about 6, about 7, about 8, or less than about 10 ug of RNA). In some embodiments, a first dose comprises about 10 to about 30 ug of RNA, or about 0.1 to about 3 ug of RNA and a second dose comprises about 1 to about 10 ug of RNA, or about 1 to about 5 ug of RNA.

In some embodiments, a first dose comprises about 10 to about 20 ug of RNA (e.g., about 10, about 12, about 14, about 16, about 18, about 20 ug of RNA) and a second dose comprises about 0.1 to about 5 ug of RNA (e.g., about 0.1, about 0.5, about 1, about 2, about 3, about 4, or about 5 ug of RNA).

In some embodiments, a first dose comprises about 10 ug of RNA and a second dose comprises about 0.1 ug of RNA. In some embodiments, a first dose comprises about 10 ug of RNA and a second dose comprises about 0.3 ug of RNA. In some embodiments, a first dose comprises about 10 ug of RNA and a second dose comprises about 1 ug of RNA. In some embodiments, a first dose comprises about 10 ug of RNA and a second dose comprises about 3 ug of RNA.

In some embodiments, a first dose comprises at least about 3 ug of RNA and a second dose comprises less than about 3 ug of RNA. In some embodiments, a first dose comprises about 3 to about 10 ug of RNA (e.g., about 3, about 4, about 5, about 6, or about 7, about 8, about 9, or about 10 ug of RNA) and a second dose comprises 0.1 to less than about 3 ug of RNA (e.g., about 0.1, about 0.2, about 0.3, about 0.5, about 0.6, about 0.7, about 0.8, about 0.9, about 1.0, about 1.5 about 2.0, or about 2.5 ug of RNA). In some embodiments, a first dose comprises about 3 to about 10 ug of RNA and a second dose comprises about 0.1 to about 3 ug of RNA, about 0.1 to about 1 ug of RNA, or about 0.1 to about 0.5 ug of RNA.

In some embodiments, a first dose comprises about 1 to about 3 ug of RNA (e.g., about 1, about 1.5, about 2.0, about 2.5, or about 3.0 ug of RNA) and a second dose comprises about 0.1 to 0.3 ug of RNA (e.g., about 0.1, about 0.2, about 0.3, about 0.4, about 0.5, about 0.6, about 0.7, about 0.8, about 0.9, or about 1.0 ug of RNA).

In some embodiments, a first dose comprises about 3 ug of RNA and a second dose comprises about 0.1 ug of RNA. In some embodiments, a first dose comprises about 3 ug of RNA and a second dose comprises about 0.3 ug of RNA. In some embodiments, a first dose comprises about 3 ug of RNA and a second dose comprises about 0.6 ug of RNA. In some embodiments, a first dose comprises about 3 ug of RNA and a second dose comprises about 1 ug of RNA.

In some embodiments, a vaccination regimen comprises at least two doses, including, e.g., at least three doses, at least four doses or more. In some embodiments, a vaccination regimen comprises three doses. In some embodiments, the time interval between the first dose and the second dose can be the same as the time interval between the second dose and the third dose. In some embodiments, the time interval between the first dose and the second dose can be longer than the time interval between the second dose and the third dose, e.g., by days or weeks (including, e.g., at least 3 days, at least 4 days, at least 5 days, at least 6 days, at least 1 week, at least 2 weeks, at least 3 weeks, at least 4 weeks, at least 5 weeks, at least 6 weeks, or longer). In some embodiments, the time interval between the first dose and the second dose can be shorter than the time interval between the second dose and the third dose, e.g., by days or weeks (including, e.g., at least 3 days, at least 4 days, at least 5 days, at least 6 days, at least 1 week, at least 2 weeks, at least 3 weeks, at least 4 weeks, at least 5 weeks, at least 6 weeks, or longer). In some embodiments, the time interval between the first dose and the second dose can be shorter than the time interval between the second dose and the third dose, e.g., by at least 1 month (including, e.g., at least 2 months, at least 3 months, at least 4 months, at least 5 months, at least 6 months, at least 7 months, at least 8 months, at least 9 months, at least 10 months, at least 11 months, at least 12 months, or longer).

In some embodiments, a last dose of a primary regimen and a first dose of a booster regimen are given at least 2 months, at least 3 months, at least 4 months, at least 5 months, at least 6 months, at least 7 months, at least 8 months, at least 9 months, at least 10 months, at least 11 months, at least 12 months, or more apart. In some embodiments, a primary regimen may comprises two doses. In some embodiments, a primary regimen may comprises three doses.

In some embodiments, a first dose and a second dose (and/or other subsequent dose) may be administered by intramuscular injection. In some embodiments, a first dose and a second dose (and/or other subsequent dose) may be administered in the deltoid muscle. In some embodiments, a first dose and a second dose (and/or other subsequent dose) may be administered in the same arm.

In some embodiments, an RNA (e.g., mRNA) composition described herein is administered (e.g., by intramuscular injection) as a series of two doses (e.g., 0.3 mL each) 21 days apart. In some embodiments, an RNA (e.g., mRNA) composition described herein is administered (e.g., by intramuscular injection) as a series of two doses (e.g., 0.2 mL each) 21 days apart. In some embodiments, an RNA (e.g., mRNA) composition described herein is administered (e.g., by intramuscular injection) as a series of three doses (e.g., 0.3 mL or lower including, e.g., 0.2 mL), wherein doses are given at least 3 weeks apart. In some embodiments, the first and second doses may be administered 3 weeks apart, while the second and third doses may be administered at a longer time interval than that between the first and the second doses, e.g., at least 4 weeks apart or longer (including, at least 5 weeks, at least 6 weeks, at least 7 weeks, at least 8 weeks, at least 9 weeks, or longer). In some embodiments, each dose is about 60 ug. In some embodiments, each dose is about 50 ug. In some embodiments, each dose is about 30 ug. In some embodiments, each dose is about 25 ug. In some embodiments, each dose is about 20 ug. In some embodiments, each dose is about 15 ug. In some embodiments, each dose is about 10 ug. In some embodiments, each dose is about 3 ug.

In some embodiments, at least one dose given in a vaccination regimen (e.g., a primary vaccination regimen and/or a booster vaccination regimen) is about 60 ug. In some embodiments, at least one dose given in a vaccination regimen (e.g., a primary vaccination regimen and/or a booster vaccination regimen) is about 50 ug. In some embodiments, at least one dose given in a vaccination regimen (e.g., a primary vaccination regimen and/or a booster vaccination regimen) is about 30 ug. In some embodiments, at least one dose given in a vaccination regimen (e.g., a primary vaccination regimen and/or a booster vaccination regimen) is about 25 ug. In some embodiments, at least one dose given in a vaccination regimen (e.g., a primary vaccination regimen and/or a booster vaccination regimen) is about 20 ug. In some embodiments, at least one dose given in a vaccination regimen (e.g., a primary vaccination regimen and/or a booster vaccination regimen) is about 15 ug. In some embodiments, at least one dose given in a vaccination regimen (e.g., a primary vaccination regimen and/or a booster vaccination regimen) is about 10 ug. In some embodiments, at least one dose given in a vaccination regimen (e.g., a primary vaccination regimen and/or a booster vaccination regimen) is about 3 ug.

In one embodiment, an amount of the RNA described herein of about 60 µg is administered per dose. In one embodiment, an amount of the RNA described herein of about 50 µg is administered per dose. In one embodiment, an amount of the RNA described herein of about 30 µg is administered per dose. In one embodiment, an amount of the RNA described herein of about 25 µg is administered per dose. In one embodiment, an amount of the RNA described herein of about 20 µg is administered per dose. In one embodiment, an amount of the RNA described herein of about 15 µg is administered per dose. In one embodiment, an amount of the RNA described herein of about 10 µg is administered per dose. In one embodiment, an amount of the RNA described herein of about 5 µg is administered per dose. In one embodiment, an amount of the RNA described herein of about 3 µg is administered per dose. In one embodiment, at least two of such doses are administered. For example, a second dose may be administered about 21 days following administration of the first dose.

In some embodiments, the efficacy of the RNA vaccine described herein (e.g., administered in two doses, wherein a second dose may be administered about 21 days following administration of the first dose, and administered, for example, in an amount of about 30 µg per dose) is at least 70%, at least 80%, at least 90, or at least 95% beginning 7 days after administration of the second dose (e.g., beginning 28 days after administration of the first dose if a second dose is administered 21 days following administration of the first dose). In some embodiments, such efficacy is observed in populations of age of at least 50, at least 55, at least 60, at least 65, at least 70, or older. In some embodiments, the efficacy of the RNA vaccine described herein (e.g., administered in two doses, wherein a second dose may be administered about 21 days following administration of the first dose, and administered, for example, in an amount of about 30 µg per dose) beginning 7 days after administration of the second dose (e.g., beginning 28 days after administration of the first dose if a second dose is administered 21 days following administration of the first dose) in populations of age of at least 65, such as 65 to 80, 65 to 75, or 65 to 70, is at least 90%, at least 91%, at least 92%, at least 93%, at least 94%, or at least 95%. Such efficacy may be observed over time periods of up to 1 month, 2 months, 3 months, 6 months or even longer.

In one embodiment, vaccine efficacy is defined as the percent reduction in the number of subjects with evidence of infection (vaccinated subjects vs. non-vaccinated subjects).

In one embodiment, efficacy is assessed through surveillance for potential cases of COVID-19. If, at any time, a patient develops acute respiratory illness, for the purposes herein, the patient can be considered to potentially have COVID-19 illness. The assessments can include a nasal (midturbinate) swab, which may be tested using a reverse transcription-polymerase chain reaction (RT-PCR) test to detect SARS-CoV-2. In addition, clinical information and results from local standard-of-care tests can be assessed.

In some embodiments, efficacy assessments may utilize a definition of SARS-CoV-2-related cases wherein:
- Confirmed COVID-19: presence of at least 1 of the following symptoms and SARS-CoV-2 NAAT (nucleic acid amplification-based test) positive during, or within 4 days before or after, the symptomatic period: fever; new or increased cough; new or increased shortness of breath; chills; new or increased muscle pain; new loss of taste or smell; sore throat; diarrhea; vomiting. Alternatively or additionally, in some embodiments, efficacy assessments may utilize a definition of SARS-CoV-2-related cases wherein one or more of the following additional symptoms defined by the CDC can be considered: fatigue; headache; nasal congestion or runny nose; nausea.

In some embodiments, efficacy assessments may utilize a definition of SARS-CoV-2-related severe cases
- Confirmed severe COVID-19: confirmed COVID-19 and presence of at least 1 of the following: clinical signs at rest indicative of severe systemic illness (e.g., RR ≥30 breaths per minute, HR ≥125 beats per minute, SpO₂≤93% on room air at sea level, or PaO₂/FiO₂<300mm Hg); respiratory failure (which can be defined as needing high-flow oxygen, noninvasive ventilation, mechanical ventilation, or ECMO); evidence of shock (e.g., SBP <90 mm Hg, DBP <60 mm Hg, or requiring vasopressors); significant acute renal, hepatic, or neurologic dysfunction; admission to an ICU; death.

Alternatively or additionally, in some embodiments a serological definition can be used for patients without clinical presentation of COVID-19: e.g., confirmed seroconversion to SARS-CoV-2 without confirmed COVID-19: e.g., positive N-binding antibody result in a patient with a prior negative N-binding antibody result.

In some embodiments, any or all of the following assays can be performed on serum samples: SARS-CoV-2 neutralization assay; S1-binding IgG level assay; RBD-binding IgG level assay; N-binding antibody assay.

In one embodiment, methods and agents described herein are administered to a paediatric population. In various embodiments, the paediatric population comprises or consists of subjects under 18 years, e.g., 5 to less than 18 years of age, 12 to less than 18 years of age, 16 to less than 18 years of age, 12 to less than 16 years of age, 5 to less than 12 years of age, or 6 months to less than 12 years of age. In various embodiments, the paediatric population comprises or consists of subjects under 5 years, e.g., 2 to less than 5 years of age, 12 to less than 24 months of age, 7 to less than 12 months of age, or less than 6 months of age. In some such embodiments, an RNA (e.g., mRNA) composition described herein is administered to subjects of less than 2 years old, for example, 6 months to less than 2 years old. In some such embodiments, an RNA (e.g., mRNA) composition described herein is administered to subjects of less than 6 months old, for example, 1 month to less than 4 months old. In some embodiments, a dosing regimen (e.g., doses and/or dosing schedule) for a paediatric population may vary for different age groups. For example, in some embodiments, a subject 6 months through 4 years of age may be administered according to a primary regimen comprising at least three doses, in which the initial two doses are administered at least 3 weeks (including, e.g., at least 4 weeks, at least 5 weeks, at least 6 weeks, or longer) apart followed by a third dose administered at least 8 weeks (including, e.g., at least 9 weeks, at least 10 weeks, at least 11 weeks, at least 12 weeks, or longer) after the second dose. In some such embodiments, at least one dose administered is 3 ug RNA described herein. In some embodiments, a subject 5 years of age and older may be administered according to a primary regimen comprising at least two doses, in which the two doses are administered at least 3 weeks (including, e.g., at least 3 weeks, at least 4 weeks, at least 5 weeks, at least 6 weeks, or longer) apart. In some such embodiments, at least one dose administered is 10 ug RNA described herein. In some embodiments, a subject 5 years of age and older who are immunocompromised (e.g., in some embodiments subjects who have undergone solid organ transplantation, or who are diagnosed with conditions that are considered to have an equivalent of immunocompromise) may be administered according to a primary regimen comprising at least three doses, in which the initial two doses are administered at least 3 weeks (including, e.g., at least 3 weeks, at least 4 weeks, at least 5 weeks, at least 6 weeks, or longer) apart, followed by a third dose administered at least 4 weeks (including, e.g., at least 5 weeks, at least 6 weeks, at least 7 weeks, at least 8 weeks, at least 9 weeks, at least 10 weeks, at least 11 weeks, at least 12 weeks, or longer) after the second dose.

In some embodiments, an RNA (e.g., mRNA) composition described herein is administered to subjects of age 12 or older and each dose is about 30 ug. In some embodiments, an RNA (e.g., mRNA) composition described herein is administered to subjects of age 12 or older (including, e.g., age 18 or older) and each dose is higher than 30 ug, including, e.g., 35 ug, 40 ug, 45 ug, 50 ug, 55 ug, 60 ug, 65 ug, 70 ug, or higher. In some such embodiments, an RNA (e.g., mRNA) composition described herein is administered to subjects of age 12 or older and each dose is about 60 ug. In some such embodiments, an RNA (e.g., mRNA) composition described herein is administered to subjects of age 12 or older and each dose is about 50 ug. In one embodiment, the paediatric population comprises or consists of subjects 12 to less than 18 years of age including subjects 16 to less than 18 years of age and/or subjects 12 to less than 16 years of age. In this embodiment, treatments may comprise 2 vaccinations 21 days apart, wherein, in one embodiment, the vaccine is administered in an amount of 30 µg RNA per dose, e.g., by intramuscular administration. In some embodiments, higher doses are administered to older pediatric patients and adults, e.g., to patients 12 years or older, compared to younger children or infants, e.g. 2 to less than 5 years old, 6 months to less than 2 years old, or less than 6 months old. In some embodiments, higher doses are administered to children who are 2 to less than 5 years old, as compared to toddlers and/or infants, e.g., who are 6 months to less than 2 years old, or less than 6 months old.

In one embodiment, the paediatric population comprises or consists of subjects 5 to less than 18 years of age including subjects 12 to less than 18 years of age and/or subjects 5 to less than 12 years of age. In this embodiment, treatments may comprise 2 vaccinations 21 days apart, wherein, in various embodiments, the vaccine is administered in an amount of 10 µg, 20µg, or 30 µg RNA per dose, e.g., by intramuscular administration. In some such embodiments, an RNA (e.g., mRNA) composition described herein is administered to subjects of age 5 to 11 and each dose is about 10 ug

In some embodiments, each dose comprises about 5 ug of RNA encoding a SARS-CoV-2 S protein of a first variant and about 5 ug of RNA encoding a SARS-CoV-2 S protein of a second variant. In some embodiments, each dose comprises about 5 ug of RNA encoding a SARS-CoV-2 S protein of a Wuhan strain and about 5 ug of RNA encoding a SARS-CoV-2 S protein of an Omicron variant. In some embodiments, each dose comprises about 5 ug of RNA encoding a SARS-CoV-2 S protein of a Wuhan strain (e.g., RNA comprising SEQ ID NO: 20) and about 5 ug of RNA encoding a SARS-CoV-2 S protein of a BA.1 Omicron variant (e.g., RNA comprising SEQ ID NO: 51). In some embodiments, each dose comprises about 5 ug of RNA encoding a SARS-CoV-2 S protein of a Wuhan strain (e.g., RNA comprising SEQ ID NO: 20)and about 5 ug of RNA encoding a SARS-CoV-2 S protein of a BA.4/5 Omicron variant (e.g., RNA comprising SEQ ID NO: 72).

In one embodiment, the paediatric population comprises or consists of subjects less than 5 years of age including subjects 2 to less than 5 years of age, subjects 12 to less than 24 months of age, subjects 7 to less than 12 months of age, subjects 6 to less than 12 months of age and/or subjects less than 6 months of age. In this embodiment, treatments may comprise 2 vaccinations, e.g., 21 to 42 days apart, e.g., 21 days apart, wherein, in various embodiments, the vaccine is administered in an amount of 3 µg, 10 µg, 20 µg, or 30 µg RNA per dose, e.g., by intramuscular administration. In some such embodiments, an RNA (e.g., mRNA) composition described herein is administered to subjects of age 2 to less than 5 and each dose is about 3 ug. In some such embodiments, an RNA (e.g., mRNA) composition described herein is administered to subjects of about 6 months to less than about 5 years and each dose is about 3 ug.

In some embodiments, each dose comprises about 1.5 ug of RNA encoding a SARS-CoV-2 S protein of a first variant and about 1.5 ug of RNA encoding a SARS-CoV-2 S protein of a second variant. In some embodiments, each dose comprises about 1.5 ug of RNA encoding a SARS-CoV-2 S protein of a Wuhan strain and about 1.5 ug of RNA encoding a SARS-CoV-2 S protein of an Omicron variant. In some embodiments, each dose comprises about 1.5 ug of RNA encoding a SARS-CoV-2 S protein of a Wuhan strain (e.g., RNA comprising SEQ ID NO: 20) and about 1.5 ug of RNA encoding a SARS-CoV-2 S protein of a BA.1 Omicron variant (e.g., RNA comprising SEQ ID NO: 51). In some embodiments, each dose comprises about 1.5 ug of RNA encoding a SARS-CoV-2 S protein of a Wuhan strain (e.g., RNA comprising SEQ ID NO: 20) and about 1.5 ug of RNA encoding a SARS-CoV-2 S protein of a BA.4/5 Omicron variant (e.g., RNA comprising SEQ ID NO: 72).

In some embodiments, an RNA (e.g., mRNA) composition described herein is administered to subjects of age 12 or older and at least one dose given in a vaccination regimen (e.g., a primary vaccination regimen and/or a booster vaccination regimen) is about 60 ug. In some embodiments, an RNA (e.g., mRNA) composition described herein is administered to subjects of age 12 or older and at least one dose given in a vaccination regimen (e.g., a primary vaccination regimen and/or a booster vaccination regimen) is about 30 ug. In some embodiments, an RNA (e.g., mRNA) composition described herein is administered to subjects of age 12 or older and at least one dose given in a vaccination regimen (e.g., a primary vaccination regimen and/or a booster vaccination regimen) is about 15 ug. In some embodiments, an RNA (e.g., mRNA) composition described herein is administered to subjects of age 5 to less than 12 years of age and at least one dose given in a vaccination regimen (e.g., a primary vaccination regimen and/or a booster vaccination regimen) is about 10 ug. In some embodiments, an RNA (e.g., mRNA) composition described herein is administered to subjects of age 2 to less than 5 and at least one dose given in a vaccination regimen (e.g., a primary vaccination regimen and/or a booster vaccination regimen) is about 3 ug. In some embodiments, an RNA (e.g., mRNA) composition described herein is administered to subjects of 6 months to less than age 2 and at least one dose given in a vaccination regimen (e.g., a primary vaccination regimen and/or a booster vaccination regimen) is about 3 ug or lower, including, e.g., 2 ug, 1 ug, or lower). In some embodiments, an RNA (e.g., mRNA) composition described herein is administered to infants of less than 6 months and at least one dose given in a vaccination regimen (e.g., a primary vaccination regimen and/or a booster vaccination regimen) is about 3 ug or lower, including, e.g., 2 ug, 1 ug, 0.5 ug, or lower).

In some embodiments, an RNA (e.g., mRNA) composition described herein is administered (e.g., by intramuscular injection) as a single dose. In some embodiments, a single dose comprise a single RNA encoding a SARS-CoV-2 S protein or an immunogenic fragment thereof (e.g., an RBD domain). In some embodiments, a single dose comprise at least two RNAs described herein, for example, each RNA encoding a SARS-CoV-2 S protein or an immunogenic fragment thereof (e.g., an RBD domain) from different strains. In some embodiments, such at least two RNAs described herein can be administered as a single mixture. For example, in some such embodiments, two separate RNA compositions described herein can be mixed to generate a single mixture prior to injection. In some embodiments, such at least two RNAs described herein can be administered as two separate compositions, which, for example, can be administered at different injection sites (e.g., on different arms, or different sites on the same arm).

In some embodiments, a dose administered to subjects in need thereof may comprise administration of a single RNA (e.g., mRNA) composition described herein.

In some embodiments, a dose administered to subjects in need thereof may comprise administration of at least two or more (including, e.g., at least three or more) different drug products/formulations. For example, in some embodiments, at least two or more different drug products/formulations may comprise at least two different RNA (e.g., mRNA) compositions described herein (e.g., in some embodiments each comprising a different RNA construct).

In some embodiments, an RNA (e.g., mRNA) composition disclosed herein may be administered in conjunction with a vaccine targeting a different infectious agent. In some embodiments, the different infectious agent is one that increases the likelihood of a subject experiencing deleterious symptoms when coinfected with SARS-CoV-2 and the infectious agent. In some embodiments, the infectious agent is one that increases the infectivity of SARS-CoV-2 when a subject is coinfected with SARS-CoV-2 and the infectious agent. In some embodiments, at least one RNA (e.g., mRNA) composition described herein may be administered in combination with a vaccine that targets influenza. In some embodiments, at least two or more different drug products/formulations may comprise at least one RNA (e.g., mRNA) composition described herein and a vaccine targeting a different infectious agent (e.g., an influenza vaccine). In some embodiments, different drug products/ formulations are separately administered. In some embodiments, such different drug product/formulations are separately adminsitered at the same time (e.g., at the same vaccination session) at different sites of a subject (e.g., at different arms of the subject).

In one embodiment, at least two doses are administered. For example, a second dose may be administered about 21 days following administration of the first dose.

In some embodiments, at least one single dose is administered. In some embodiments, such single dose is administered to subjects, for example, who may have previously received one or more doses of, or a complete regimen of, a SARS-CoV-2 vaccine (e.g., of a BNT162b2 vaccine [including, e.g., as described herein], an mRNA-1273 vaccine, an Ad26.CoV2.S vaccine, a ChAdxOx1 vaccine, an NVX-CoV2373 vaccine, a CvnCoV vaccine, a GAM-COVID0Vac vaccine, a CoronaVac vaccine, a BBIBP-CorV vaccine, an Ad5-nCoV vaccine, a zf2001 vaccine, a SCB-2019 vaccine, or other approved RNA (e.g., mRNA) or adenovector vaccines, etc. Alternatively or additionally, in some embodiments, a single dose is administered to subjects who have been exposed to and/or infected by SARS-CoV-2. In some embodiments, at least one single dose is administered to subjects who both have received one or more doses of, or a complete regimen of, a SARS-CoV-2 vaccine and have been exposed to and/or infected with SARS-CoV-2.

In some particular embodiments where at least one single dose is administered to subjects who have received one or more doses of a prior SARS-CoV-2 vaccine, such prior SARS-CoV-2 vaccine is a different vaccine, or a different form (e.g., formulation) and/or dose of a vaccine with the same active (e.g., BNT162b2); in some such embodiments, such subjects have not received a complete regimen of such prior vaccine and/or have experienced one or more undesirable reactions to or effects of one or more received doses of such prior vaccine. In some particular embodiments, such prior vaccine is or comprises higher dose(s) of the same active (e.g., BNT162b2). Alternatively or additionally, in some such embodiments, such subjects were exposed to and/or infected by SARS-CoV-2 prior to completion (but, in some embodiments, after initiation) of a full regimen of such prior vaccine..

In one embodiment, at least two doses are administered. For example, a second dose may be administered about 21 days following administration of the first dose.

In one embodiment, at least three doses are administered. In some embodiments, such third dose is administered a period of time after the second dose that is comparable to (e.g., the same as) the period of time between the first and second doses. For example, in some embodiments, a third dose may be administered about 21 days following administration of the second dose. In some embodiments, a third dose is administered after a longer period of time relative to the second dose than the second dose was relative to the first dose. In some embodiments, a three-dose regimen is administered to an immunocompromised patient, e.g., a cancer patient, an HIV patient, a patient who has received and/or is receiving immunosuppressant therapy (e.g., an organ transplant patient). In some embodiments, the length of time between the second and third dose (e.g., a second and third dose administered to an immunocompromised patient) is at least about 21 days (e.g., at least about 28 days).

In some embodiments, a vaccination regimen comprises administering the same amount of RNA in different doses (e.g., in first and/or second and/or third and/or subsequent doses). In some embodiments, a vaccination regimen comprises administering different amounts of RNA in different doses. In some embodiments, one or more later doses is larger than one or more earlier doses (e.g., in situations where waning of vaccine efficacy from one or more earlier doses is observed and/or immune escape by a variant (e.g., one described herein) that is prevalent or rapidly spreading is observed in a relevant jurisdiction at the time of administration is observed). In some embodiments, one or more later doses may be larger than one or more earlier doses by at least 30%, at least 40%, at least 50%, at least 60%, at least 70%, at least 80%, at least 90%, at least 95%, or higher, provided that safety and/or tolerability of such a dose is clinically acceptable. In some embodiments, one or more later doses may be larger than one or more earlier doses by at least 1.1-fold, at least 1.5-fold, at least 2-fold, at least 3-fold, at least 4-fold, or higher provided that safety and/or tolerability of such a dose is clinically acceptable. In some embodiments, one or more later doses is smaller than one or more earlier doses (e.g., in a negative reaction was experienced after one or more earlier doses and/or if exposure to and/or infection by SARS-CoV-2 between an earlier dose and a subsequent dose). In some embodiments, one or more later doses may be smaller than one or more earlier doses by at least 10%, at least 20%, at least 30%, at least 40%, at least 50%, at least 60%, or higher. In some embodiments, where different doses are utilized, they are related to one another by identity with and/or dilution of a common stock as described herein. In some embodiments, where at least two or more doses are administered (e.g., at least two doses administered in a primary regimen, at least two doses administered in a booster regimen, or at least one dose administered in a primary regimen and at least one dose in a booster regimen), the same RNA compositions described herein may be administered in such doses and each of such doses can be the same or different (as described herein). In some embodiments, where at least two or more doses are administered (e.g., at least two doses administered in a primary regimen, at least two doses administered in a booster regimen, or at least one dose administered in a primary regimen and at least one dose in a booster regimen), different RNA compositions described herein (e.g., different encoded viral polypeptides, e.g., from different coronavirus clades, or from different strains of the same coronavirus clade; different construct elements such as 5' cap, 3' UTR, 5' UTR, etc.; different formulations, e.g., different excipients and/or buffers (e.g., PBS vs. Tris); different LNP compositions; or combinations thereof) may be administered in such doses and each of such doses can be the same or different (e.g., as described herein).

In some embodiments, a subject is administered two or more RNAs (e.g., as part of either a primary regimen or a booster regimen), wherein the two or more RNAs are administered on the same day or same visit. In some embodiments, the two or more RNAs are administered in separate compositions, e.g., by administering each RNA to a separate part of the subject (e.g., by intramuscular administration to different arms of the subject or to different sites of the same arm of the subject). In some embodiments, the two or more RNAs are mixed prior to administration (e.g., mixed immediately prior to administration, e.g., by the administering practitioner). In some embodiments, the two or more RNAs are formulated together (e.g., by (a) mixing separate populations of LNPs, each population comprising a different RNA; or (b) by mixing two or more RNAs prior to LNP formulation, so that each LNP comprises two or more RNAs). In some embodiments, the two or more RNAs comprise an RNA that encode a coronavirus S protein or immunogenic fragment thereof (e.g., RBD or other relevant domains) from one strain (e.g., Wuhan strain) and a variant that is prevalent or rapidly spreading in a relevant jurisdiction at the time of administration (e.g., a variant described herein). In some embodiments, such a variant is an Omicron variant (e.g., a BA.1, BA.2, or BA.3 variant). In some embodiments, the two or more RNAs comprise a first RNA and a second RNA that have been shown to elicit a broad immune response in subject. In some embodiments the two or more RNAs comprise an RNA encoding a SARS-CoV-2 S protein from a Wuhan strain and an RNA encoding a SARS-CoV-2 S protein from a BA.1 Omicron variant. In some embodiments the two or more RNAs comprise an RNA encoding a SARS-CoV-2 S protein from a Wuhan strain and an RNA encoding a SARS-CoV-2 S protein from a BA.2 Omicron variant. In some embodiments the two or more RNAs comprise an RNA encoding a SARS-CoV-2 S protein from a Wuhan strain and an RNA encoding a SARS-CoV-2 S protein from a BA.4 or BA.5 Omicron variant. In some embodiments the two or more RNAs comprise an RNA encoding a SARS-CoV-2 S protein from a BA.1 Omicron variant and an RNA encoding a SARS-CoV-2 S protein from a BA.2 Omicron variant. In some embodiments the two or more RNAs comprise an RNA encoding a SARS-CoV-2 S protein from a BA.1 Omicron variant and an RNA encoding a SARS-CoV-2 S protein from a BA.4 or BA.5 Omicron variant. In some embodiments the two or more RNAs comprise an RNA encoding a SARS-CoV-2 S protein from a BA.2 Omicron variant and an RNA encoding a SARS-CoV-2 S protein from a BA.4 or 5 Omicron variant. In some embodiments the two or more RNAs comprise an RNA encoding a SARS-CoV-2 S protein from a Wuhan strain, an alpha variant, a beta variant, or a delta variant, or sublineages derived therefrom; and an RNA encoding a SARS-CoV-2 S protein from a BA.2, BA.4 or 5 Omicron variant, or sublineages derived therefrom.

In some embodiments, a subject may be administered any one of combinations 1 to 66, listed in the below table. In some embodiments, such combinations can be administered using an LNP formulation, where the first RNA and the second RNA are encapsulated in the same LNP or in separate LNPs. In some embodiments, such combinations can be administered as separate LNP formulations (e.g., by administering at separate sites to a subject).

| Combination | SARS-CoV-2 S protein encoded by a first RNA¹ | SARS-CoV-2 S protein encoded by a second RNA¹ |
|---|---|---|
| 1 | Wuhan | Alpha |
| 2 | Wuhan | Beta |
| 3 | Wuhan | Delta |
| 4 | Wuhan | BA.1 |
| 5 | Wuhan | BA.2 |
| 6 | Wuhan | BA.2.12.1 |
| 7 | Wuhan | BA.3 |
| 8 | Wuhan | BA.4/5 |
| 9 | Wuhan | XBB |
| 10 | Wuhan | XBB variant (e.g., XBB.1, XBB.2, XBB.1.3) |
| 11 | Wuhan | BQ.1.1 |
| 12 | Alpha | Beta |
| 13 | Alpha | Delta |
| 14 | Alpha | BA.1 |
| 15 | Alpha | BA.2 |
| 16 | Alpha | BA.2.12.1 |
| 17 | Alpha | BA.3 |
| 18 | Alpha | BA.4/5 |
| 19 | Alpha | XBB |
| 20 | Alpha | XBB variant (e.g., XBB.1, XBB.2, XBB.1.3) |
| 21 | Alpha | BQ.1.1 |
| 22 | Beta | Delta |
| 23 | Beta | BA.1 |
| 24 | Beta | BA.2 |
| 25 | Beta | BA.2.12.1 |
| 26 | Beta | BA.3 |
| 27 | Beta | BA.4/5 |
| 28 | Beta | XBB |
| 29 | Beta | XBB variant (e.g., XBB.1, XBB.2, XBB.1.3) |
| 30 | Beta | BQ.1.1 |
| 31 | Delta | BA.1 |
| 32 | Delta | BA.2 |
| 33 | Delta | BA.2.12.1 |
| 34 | Delta | BA.3 |
| 35 | Delta | BA.4/5 |
| 36 | Delta | XBB |
| 37 | Delta | XBB variant (e.g., XBB.1, XBB.2, XBB.1.3) |
| 38 | Delta | BQ.1.1 |
| 39 | BA.1 | BA.2 |
| 40 | BA.1 | BA.2.12.1 |
| 41 | BA.1 | BA.3 |
| 42 | BA.1 | BA.4/5 |
| 43 | BA.1 | XBB |
| 44 | BA.1 | XBB variant (e.g., XBB.1, XBB.2, XBB.1.3) |
| 45 | BA.1 | BQ.1.1 |
| 46 | BA.2 | BA.2.12.1 |
| 47 | BA.2 | BA.3 |
| 48 | BA.2 | BA.4/5 |
| 49 | BA.2 | XBB |
| 50 | BA.2 | XBB variant (e.g., XBB.1, XBB.2, XBB.1.3) |
| 51 | BA.2 | BQ.1.1 |
| 52 | BA.2.12.1 | BA.3 |
| 53 | BA.2.12.1 | BA.4/5 |
| 54 | BA.2.12.1 | XBB |
| 55 | BA.2.12.1 | XBB variant (e.g., XBB.1, XBB.2, XBB.1.3) |
| 56 | BA.2.12.1 | BQ.1.1 |
| 57 | BA.3 | BA.4/5 |
| 58 | BA.3 | XBB |
| 59 | BA.3 | XBB variant (e.g., XBB.1, XBB.2, XBB.3) |
| 60 | BA.3 | BQ.1.1 |
| 61 | BA.4/5 | XBB |
| 62 | BA.4/5 | XBB variant (e.g., XBB.1, XBB.2, XBB.1.3) |
| 63 | BA.4/5 | BQ.1.1 |
| 64 | XBB | XBB variant (e.g., XBB.1, XBB.2, XBB.1.3) |
| 65 | XBB | BQ.1.1 |
| 66 | XBB variant (e.g., XBB.1, XBB.2, XBB.1.3) | BQ.1.1 |

| | | |
|---|---|---|
| ¹Listed RNAs encode a SARS-CoV-2 S protein having mutations characteristic of the indicated SARS-CoV-2 variant. | | |

In some embodiments, a subject is administered a first RNA and a second RNA, each in the same amount (i.e., at a 1:1 ratio).

In some embodiments, a subject is administered a first RNA and a second RNA, each in a different amount. For example, in some embodiments, a subject is administered a first RNA in an amount that is 0.01 to 100 times that of a second RNA (e.g., wherein the amount of a first RNA is 0.01 to 50, 0.01 to 4, 0.01 to 30, 0.01 to 25, 0.01 to 20, 0.01 to 15, 0.01 to 10, 0.01 to 9, 0.01 to 8, 0.01 to 7, 0.01 to 6, 0.01 to 5, 0.01 to 4, 0.01 to 3, 0.01 to 2, 0.01 to 1.5, 1 to 50, 1 to 4, 1 to 30, 1 to 25, 1 to 20, 1 to 15, 1 to 10, 1 to 9, 1 to 8, 1 to 7, 1 to 6, 1 to 5, 1 to 4, 1 to 3, 1 to 2, or 1 to 1.5 times that of a second RNA). In some embodiments, a subject is administered a first RNA and a second RNA, wherein the concentration of the first RNA is 1 to 10 times that of the second RNA. In some embodiments, a subject is administered a first RNA and a second RNA, wherein the amount of the first RNA is 1 to 5 times that of the second RNA. In some embodiments, a subject is administered a first RNA and a second RNA, wherein the concentration of the first RNA is 1 to 3 times that of the second RNA. In some embodiments, a subject is administered a first RNA and a second RNA, wherein the amount of the first RNA is 2 times that of the second RNA. In some embodiments, a subject is administered a first RNA and a second RNA, wherein the concentration of the first RNA is 3 times that of the second RNA.

In some embodiments, a subject is administered three RNAs, each encoding a SARS-CoV-2 S protein comprising one or more mutations characteristic of a different SARS-CoV-2 variant, and each in the same amount (i.e., at a 1:1:1 ratio).

In some embodiments, a subject is administered three RNAs, each encoding a SARS-CoV-2 S protein comprising one or more mutations characteristic of a different SARS-CoV-2 variant, wherein the amount of each RNA is not the same (e.g., one RNA is present in an amount that is different than the other two RNA, or all three RNAs are present in different amounts). For example, in some embodiments, the ratio of first RNA:second RNA:third RNA is 1: 0.01-100: 0.01-100 (e.g., 1: 0.01-50: 0.01-50; 1: 0.01-40: 0.01-40; 1: 0.01-30: 0.01-25; 1: 0.01-25: 0.01-25; 1: 0.01-20: 0.01-20; 1: 0.01-15: 0.01-15; 1: 0.01-10: 0.01-9; 1: 0.01-9: 0.01-9; 1: 0.01-8: 0.01-8; 1: 0.01-7: 0.01-7; 1: 0.01-6: 0.01-6; 1: 0.01-5: 0.01-5; 1: 0.01-4: 0.01-4; 1: 0.01-3: 0.01-3; 1: 0.01-2: 0.01-2; or 1: 0.01-1.5: 0.01-1.5). In some embodiments, a subject is administered three RNAs at a ratio of 1:1:3. In some embodiments, a subject is administered three RNAs at a ratio of 1:3:3.

In some embodiments, a vaccination regimen comprises a first vaccination regimen (e.g., a primary regimen) that includes at least two doses of an RNA composition as described herein, e.g., wherein the second dose may be administered about 21 days following administration of the first dose, and a second vaccination (e.g., a booster regimen) that comprises a single dose or multiple doses, e.g., two doses, of an RNA composition as described herein. In some embodiments, doses of a booster regimen are related to those of a primary regimen by identity with or dilution from a common stock as described herein. In various embodiments, a booster regimen is administered (e.g., is initiated) at least 1 week, at least 2 weeks, at least 3 weeks, at least 1 month, at least 2 months, at least 3 months, at least 4 months, at least 5 months, or at least 6 months, at least 7 months, at least 8 months, at least 9 months, at least 10 months, at least 11 months, at least 12 months, or longer, after administration of a primary regimen, e.g., after completion of a primary regimen comprising at least two doses. In various embodiments, a booster regimen is administered (e.g., is initiated) 1-12 months, 2-12 months, 3-12 months, 4-12 months, 6-12 months, 1-6 months, 1-5 months, 1-4 months, 1-3 months, or 2-3 months after administration of a primary regimen, e.g., after completion of a primary regimen comprising at least two doses. In various embodiments, a booster regimen is administered (e.g., is initiated) 1 to 60 months, 2 to 48 months, 2 to 24 months, 3 to 24 months, 6 to 18 months, 6 to 12 months, or 5 to 7 months after administration of a primary regimen, e.g., after completion of a two-dose primary regimen. In some embodiments, each dose of a primary regimen is about 60 µg per dose. In some embodiments, each dose of a primary regimen is about 50 µg per dose. In some embodiments, each dose of a primary regimen is about 30 µg per dose. In some embodiments, each dose of a primary regimen is about 25 µg per dose. In some embodiments, each dose of a primary regimen is about 20 µg per dose. In some embodiments, each dose of a primary regimen is about 15 µg per dose. In some embodiments, each dose of a primary regimen is about 10 µg per dose. In some embodiments, each dose of a primary regimen is about 3 µg per dose. In some embodiments, each dose of a booster regimen is the same as that of the primary regimen. In some embodiments, each dose of a booster regimen comprises the same amount of RNA as a dose administered in a primary regimen. In some embodiments, at least one dose of a booster regimen is the same as that of the primary regimen. In some embodiments, at least one dose of a booster regimen comprises the same amount of RNA as at least one dose of a primary regimen. In some embodiments, at least one dose of a booster regimen is lower than that of the primary regimen. In some embodiments, at least one dose of a booster regimen comprises an amount of RNA that is lower than that of a primary regimen. In some embodiments, at least one dose of a booster regimen is higher than that of the primary regimen. In some embodiments, at least one dose of a booster regimen comprises an amount of RNA that is higher than that of a primary regimen.

In some embodiments, a booster regimen (e.g., as described herein) is administered to a pediatric patient (e.g., a patient aged 2 through 5 years old, a patient aged 5 through 11 years old, or a patient aged 12 through 15 years old). In some embodiments, a booster regimen is administered to a pediatric patient who is 6 months old to less than 2 years old. In some embodiments, a booster regimen is administered to a pediatric patient who is less than 6 months old. In some embodiments, a booster regimen is administered to a pediatric patient who is 6 months old to less than 5 years old. In some embodiments, a booster regimen is administered to a pediatric patient who is 2 years old to less than 5 years old. In some embodiments, a booster regimen is administered to a pediatric patient who is 5 years old to less than 12 years old. In some embodiments, a booster regimen is administered to a pediatric patient who is 12 years old to less than 16 years old. In some embodiments, each dose of a pediatric booster regimen comprises about 3 µg of RNA. In some embodiments, each dose of a pediatric booster regimen comprises about 10 µg of RNA. In some embodiments, each dose of a pediatric booster regimen comprises about 15 µg of RNA. In some embodiments, each dose of a pediatric booster regimen comprises about 20 µg of RNA. In some embodiments, each dose of a pediatric booster regimen comprises about 25 µg of RNA. In some embodiments, each dose of a pediatric booster regimen comprises about 30 µg of RNA. In some embodiments, a booster regimen is administered to a non-pediatric patient (e.g., a patient 16 years or older, a patient aged 18 through 64 years old, and/or a patient 65 years and older). In some embodiments, each dose of a non-pediatric booster regimen comprises about 3 ug of RNA, about 10 ug of RNA, about 25 µg or RNA, about 30 µg of RNA, about 40 µg of RNA, about 45 µg of RNA, about 50 µg of RNA, about 55 µg of RNA, or about 60 µg of RNA . In some embodiments, the same booster regimen may be administered to both pediatric and non-pediatric patients (e.g., to a patient 12 years or older). In some embodiments, a booster regimen that is administered to a non-pediatric patient is administered in a formulation and dose that is related to that of a primary regimen previously received by the patient by identity with or by dilution as described herein. In some embodiments, a non-pediatric patient who receives a booster regimen at a lower dose than a primary regimen may have experienced an adverse reaction to one or more doses of such primary regimen and/or may have been exposed to and/or infected by SARS-CoV-2 between such primary regimen and such booster regimen, or between doses of such primary regimen and/or of such booster regimen. In some embodiments, pediatric and non-pediatric patients may receive a booster regimen at a higher dose than a primary regimen when waning of vaccine efficacy at lower doses is observed, and/or when immune escape of a variant that is prevalent and/or spreading rapidly at a relevant jurisdiction at the time of administration is observed.

In some embodiments one or more doses of a booster regimen differs from that of a primary regimen. For example, in some embodiments, an administered dose may correspond to a subject's age and a patient may age out of one treatment age group and into a next. Alternatively or additionally, in some embodiments, an administered dose may correspond to a patient's condition (e.g., immunocompromised state) and a different dose may be selected for one or more doses of a booster regimen than for a primary regimen (e.g., due to intervening cancer treatment, infection with HIV, receipt of immunosuppressive therapy, for example associated with an organ transplant. In some embodiments, at least one dose of a booster regimen may comprise an amount of RNA that is higher than at least one dose administered in a primary regimen (e.g., in situations where waning of vaccine efficacy from one or more earlier doses is observed and/or immune escape by a variant (e.g., one described herein) that is prevalent or rapidly spreading is observed in a relevant jurisdiction at the time of administration).

In some embodiments, a primary regimen may involve one or more 3 ug doses and a booster regimen may involve one or more 10 ug doses, and/or one or more 20 ug doses, or one or more 30 ug doses. In some embodiments, a primary regimen may involve one or more 3 ug doses and a booster regimen may involve one or more 3 ug doses. In some embodiments, a primary regimen may involve two or more 3 ug doses (e.g., at least two doses, each comprising 3 ug of RNA, and administered about 21 days after one another) and a booster regimen may involve one or more 3 ug doses. In some embodiments, a primary regimen may involve one or more 10 ug doses and a booster regimen may involve one or more 20 ug doses, and/or one or more 30 ug doses. In some embodiments, a primary regimen may involve one or more 10 ug doses and a booster regimen may involve one or more 10 ug doses. In some embodiments, a primary regimen may involve two or more 10 ug doses (e.g., two doses, each comprising 10 ug of RNA, administered about 21 days apart) and a booster regimen may involve one or more 10 ug doses. In some embodiments, a primary regimen may involve one or more 20 ug doses and a booster regimen may involve one or more 30 ug doses. In some embodiments, a primary regimen may involve one or more 20 ug doses and a booster regimen may involve one or more 20 ug doses. In some embodiments, a primary regimen may involve one or more 30 ug doses, and a booster regimen may also involve one or more 30 ug doses. In some embodiments, a primary regimen may involve two or more 30 ug doses (e.g., two doses, each comprising 30 ug of RNA, administered about 21 days apart), and a booster regimen may also involve one or more 30 ug doses. In some embodiments, a primary regimen may involve two or more 30 ug doses (e.g., two doses, each comprising 30 ug of RNA, administered about 21 days apart), and a booster regimen may involve one or more 50 ug doses. In some embodiments, a primary regimen may involve two or more 30 ug doses (e.g., two doses, each comprising 30 ug of RNA, administered about 21 days apart), and a booster regimen may involve one or more 60 ug doses.

In some embodiments, a subject is administered a booster regimen comprising at least one 30 ug dose of RNA. In some embodiments, a subject is administered a booster regimen comprising at least one 30 ug dose of RNA encoding a SARS-CoV-2 S protein from a Wuhan strain of SARS-CoV-2 (e.g., BNT162b2). In some embodiments, a subject is administered a booster regimen comprising at least one dose of 30 ug of RNA encoding a SARS-CoV-2 S protein having one or more mutations that are characteristic of a SARS-CoV-2 variant (e.g., a variant described herein). In some embodiments, a subject is administered a booster regimen comprising at least one dose of 30 ug of RNA encoding a SARS-CoV-2 S protein having one or more mutations that are characteristic of an Omicron variant (e.g., a BA.1, BA.2, BA.3, or BA.4 or BA.5 Omicron variant). In some embodiments, a subject is administered a booster regimen comprising at least one dose of 30 ug of RNA, wherein the 30 ug of RNA comprises RNA encoding a SARS-CoV-2 S protein from a Wuhan strain and RNA encoding a SARS-CoV-2 S protein comprising mutations that are characteristic of a SARS-CoV-2 variant (e.g., in some embodiments, a subject is administered a booster regimen comprising at least one dose comprising 15 ug of RNA encoding a SARS-CoV-2 S protein from a Wuhan strain and 15 ug of RNA encoding a SARS-CoV-2 S protein having one or more mutations that are characteristic of an Omicron variant). In some embodiments, a subject is administered a booster regimen comprising at least one dose comprising 15 ug of RNA encoding a SARS-CoV-2 S protein from a Wuhan strain and 15 ug of RNA encoding a SARS-CoV-2 S protein having one or more mutations that are characteristic of a BA.1 Omicron variant. In some embodiments, a subject is administered a booster regimen comprising at least one dose comprising 10 ug of RNA encoding a SARS-CoV-2 S protein from a Wuhan strain and 20 ug of RNA encoding a SARS-CoV-2 S protein having one or more mutations that are characteristic of a BA.1 Omicron variant. In some embodiments, a subject is administered a booster regimen comprising at least one dose comprising 7.5 ug of RNA encoding a SARS-CoV-2 S protein from a Wuhan strain and 22.5 ug of RNA encoding a SARS-CoV-2 S protein having one or more mutations that are characteristic of a BA.1 Omicron variant. In some embodiments, a subject is administered a booster regimen comprising at least one dose comprising 15 ug of RNA encoding a SARS-CoV-2 S protein from a Wuhan strain and 15 ug of RNA encoding a SARS-CoV-2 S protein having one or more mutations that are characteristic of a BA.2 Omicron variant. In some embodiments, a subject is administered a booster regimen comprising at least one dose comprising 10 ug of RNA encoding a SARS-CoV-2 S protein from a Wuhan strain and 20 ug of RNA encoding a SARS-CoV-2 S protein having one or more mutations that are characteristic of a BA.2 Omicron variant. In some embodiments, a subject is administered a booster regimen comprising at least one dose comprising 7.5 ug of RNA encoding a SARS-CoV-2 S protein from a Wuhan strain and 22.5 ug of RNA encoding a SARS-CoV-2 S protein having one or more mutations that are characteristic of a BA.2 Omicron variant. In some embodiments, a subject is administered a booster regimen comprising at least one dose comprising 15 ug of RNA encoding a SARS-CoV-2 S protein from a Wuhan strain and 15 ug of RNA encoding a SARS-CoV-2 S protein having one or more mutations that are characteristic of a BA.3 Omicron variant. In some embodiments, a subject is administered a booster regimen comprising at least one dose comprising 10 ug of RNA encoding a SARS-CoV-2 S protein from a Wuhan strain and 20 ug of RNA encoding a SARS-CoV-2 S protein having one or more mutations that are characteristic of a BA.3 Omicron variant. In some embodiments, a subject is administered a booster regimen comprising at least one dose comprising 7.5 ug of RNA encoding a SARS-CoV-2 S protein from a Wuhan strain and 22.5 ug of RNA encoding a SARS-CoV-2 S protein having one or more mutations that are characteristic of a BA.3 Omicron variant. In some embodiments, a subject is administered a booster regimen comprising at least one dose comprising 15 ug of RNA encoding a SARS-CoV-2 S protein from a Wuhan strain and 15 ug of RNA encoding a SARS-CoV-2 S protein having one or more mutations that are characteristic of a BA.4 or BA.5 Omicron variant. In some embodiments, a subject is administered a booster regimen comprising at least one dose comprising 10 ug of RNA encoding a SARS-CoV-2 S protein from a Wuhan strain and 20 ug of RNA encoding a SARS-CoV-2 S protein having one or more mutations that are characteristic of a BA.4 or BA.5 Omicron variant. In some embodiments, a subject is administered a booster regimen comprising at least one dose comprising 7.5 ug of RNA encoding a SARS-CoV-2 S protein from a Wuhan strain and 22.5 ug of RNA encoding a SARS-CoV-2 S protein having one or more mutations that are characteristic of a BA.4 or BA.5 Omicron variant.

In some embodiments, a subject is administered a booster regimen comprising at least one dose comprising 15 ug of RNA encoding a SARS-CoV-2 S protein from a BA.1 Omicron variant and 15 ug of RNA encoding a SARS-CoV-2 S protein having one or more mutations that are characteristic of a BA.2 Omicron variant. In some embodiments, a subject is administered a booster regimen comprising at least one dose comprising 10 ug of RNA encoding a SARS-CoV-2 S protein from a BA.1 Omicron variant and 20 ug of RNA encoding a SARS-CoV-2 S protein having one or more mutations that are characteristic of a BA.2 Omicron variant. In some embodiments, a subject is administered a booster regimen comprising at least one dose comprising 7.5 ug of RNA encoding a SARS-CoV-2 S protein from a BA.1 Omicron variant and 22.5 ug of RNA encoding a SARS-CoV-2 S protein having one or more mutations that are characteristic of a BA.2 Omicron variant. In some embodiments, a subject is administered a booster regimen comprising at least one dose comprising 15 ug of RNA encoding a SARS-CoV-2 S protein from a BA.1 Omicron variant and 15 ug of RNA encoding a SARS-CoV-2 S protein having one or more mutations that are characteristic of a BA.3 Omicron variant. In some embodiments, a subject is administered a booster regimen comprising at least one dose comprising 10 ug of RNA encoding a SARS-CoV-2 S protein from a BA.1 Omicron variant and 20 ug of RNA encoding a SARS-CoV-2 S protein having one or more mutations that are characteristic of a BA.3 Omicron variant. In some embodiments, a subject is administered a booster regimen comprising at least one dose comprising 7.5 ug of RNA encoding a SARS-CoV-2 S protein from a BA.1 Omicron variant and 22.5 ug of RNA encoding a SARS-CoV-2 S protein having one or more mutations that are characteristic of a BA.3 Omicron variant. In some embodiments, a subject is administered a booster regimen comprising at least one dose comprising 15 ug of RNA encoding a SARS-CoV-2 S protein from a BA.1 Omicron variant and 15 ug of RNA encoding a SARS-CoV-2 S protein having one or more mutations that are characteristic of a BA.4 or BA.5 Omicron variant. In some embodiments, a subject is administered a booster regimen comprising at least one dose comprising 10 ug of RNA encoding a SARS-CoV-2 S protein from a BA.1 Omicron variant and 20 ug of RNA encoding a SARS-CoV-2 S protein having one or more mutations that are characteristic of a BA.4 or BA.5 Omicron variant. In some embodiments, a subject is administered a booster regimen comprising at least one dose comprising 7.5 ug of RNA encoding a SARS-CoV-2 S protein from a BA.1 Omicron variant and 22.5 ug of RNA encoding a SARS-CoV-2 S protein having one or more mutations that are characteristic of a BA.4 or BA.5 Omicron variant.

In some embodiments, a subject is administered a booster regimen comprising at least one dose comprising 15 ug of RNA encoding a SARS-CoV-2 S protein from a BA.2 Omicron variant and 15 ug of RNA encoding a SARS-CoV-2 S protein having one or more mutations that are characteristic of a BA.4 or BA.5 Omicron variant. In some embodiments, a subject is administered a booster regimen comprising at least one dose comprising 10 ug of RNA encoding a SARS-CoV-2 S protein from a BA.2 Omicron variant and 20 ug of RNA encoding a SARS-CoV-2 S protein having one or more mutations that are characteristic of a BA.4 or BA.5 Omicron variant. In some embodiments, a subject is administered a booster regimen comprising at least one dose comprising 7.5 ug of RNA encoding a SARS-CoV-2 S protein from a BA.2 Omicron variant and 22.5 ug of RNA encoding a SARS-CoV-2 S protein having one or more mutations that are characteristic of a BA.4 or BA.5 Omicron variant. In some embodiments, a subject is administered a booster regimen comprising at least one dose comprising 15 ug of RNA encoding a SARS-CoV-2 S protein from a BA.3 Omicron variant and 15 ug of RNA encoding a SARS-CoV-2 S protein having one or more mutations that are characteristic of a BA.4 or BA.5 Omicron variant. In some embodiments, a subject is administered a booster regimen comprising at least one dose comprising 10 ug of RNA encoding a SARS-CoV-2 S protein from a BA.3 Omicron variant and 20 ug of RNA encoding a SARS-CoV-2 S protein having one or more mutations that are characteristic of a BA.4 or BA.5 Omicron variant. In some embodiments, a subject is administered a booster regimen comprising at least one dose comprising 7.5 ug of RNA encoding a SARS-CoV-2 S protein from a BA.3 Omicron variant and 22.5 ug of RNA encoding a SARS-CoV-2 S protein having one or more mutations that are characteristic of a BA.4 or BA.5 Omicron variant.

In some embodiments, a subject is administered a booster regimen comprising two or more doses of 30 ug of RNA, administered at least two months apart from each other. For example, in some embodiments, subjects are administered a booster regimen comprising two doses of 30 ug of RNA encoding a SARS-CoV-2 S protein having one or more mutations that are characteristic of an Omicron variant (e.g., a BA.1, BA.2, or BA.4 or BA.5 Omicron variant).

In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a dose of 30 ug of RNA encoding a SARS-CoV-2 S protein having one or mutations that are characteristic of an Omicron variant of SARS-CoV-2 (e.g., a BA.1, BA.2, BA.3, BA.4, or BA.5 Omicron variant), wherein the booster regimen is administered at least two months (including, e.g., at least three months, at least four months, at least five months, at least six months, or more) after completion of the primary regimen. In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a 30 ug dose of RNA comprising 15 ug RNA encoding a SARS-CoV-2 S protein from a Wuhan strain and 15 ug of RNA encoding a SARS-CoV-2 S protein having one or more mutations that are characteristic of an Omicron variant.

In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a dose of 30 ug of RNA encoding a SARS-CoV-2 S protein having one or mutations that are characteristic of a BA.1 Omicron variant of SARS-CoV-2, wherein the booster regimen is administered at least two months (including, e.g., at least three months, at least four months, at least five months, at least six months, or more) after completion of the primary regimen. In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a 30 ug dose of RNA comprising 15 ug RNA encoding a SARS-CoV-2 S protein from a Wuhan strain and 15 ug of RNA encoding a SARS-CoV-2 S protein having one or more mutations that are characteristic of a BA.1 Omicron variant.

In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a dose of 30 ug of RNA encoding a SARS-CoV-2 S protein having one or mutations that are characteristic of a BA.2 Omicron variant of SARS-CoV-2, wherein the booster regimen is administered at least two months (including, e.g., at least three months, at least four months, at least five months, at least six months, or more) after completion of the primary regimen. In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a 30 ug dose of RNA comprising 15 ug RNA encoding a SARS-CoV-2 S protein from a Wuhan strain and 15 ug of RNA encoding a SARS-CoV-2 S protein having one or more mutations that are characteristic of a BA.2 Omicron variant.

In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a dose of 30 ug of RNA encoding a SARS-CoV-2 S protein having one or mutations that are characteristic of a BA.4 or BA.5 Omicron variant of SARS-CoV-2, wherein the booster regimen is administered at least two months (including, e.g., at least three months, at least four months, at least five months, at least six months, or more) after completion of the primary regimen. In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a 30 ug dose of RNA comprising 15 ug RNA encoding a SARS-CoV-2 S protein from a Wuhan strain and 15 ug of RNA encoding a SARS-CoV-2 S protein having one or more mutations that are characteristic of a BA.4 or BA.5 Omicron variant.

In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a 30 ug dose of RNA comprising 15 ug RNA encoding a SARS-CoV-2 S protein from a BA.1 Omicron variant and 15 ug of RNA encoding a SARS-CoV-2 S protein having one or more mutations that are characteristic of a BA.2 Omicron variant.

In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a 30 ug dose of RNA comprising 15 ug RNA encoding a SARS-CoV-2 S protein from a BA.1 Omicron variant and 15 ug of RNA encoding a SARS-CoV-2 S protein having one or more mutations that are characteristic of a BA.4 or BA.5 Omicron variant.

In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a 30 ug dose of RNA comprising 15 ug RNA encoding a SARS-CoV-2 S protein from a BA.2 Omicron variant and 15 ug of RNA encoding a SARS-CoV-2 S protein having one or more mutations that are characteristic of a BA.4 or BA.5 Omicron variant.

In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising at least one 30 ug dose of RNA encoding a SARS-CoV-2 S protein from a non-BA.1 Omicron variant.

In some embodiments, a subject is administered (i) a primary regimen comprising two 30 ug doses of RNA encoding a SARS-CoV-2 S protein from a Wuhan strain, wherein the two doses are administered at least approximately 21 days apart, and (ii) a booster regimen comprising at least two 30 ug doses of RNA encoding a SARS-CoV-2 S protein having one or more mutations characteristic of an Omicron variant, wherein the booster regimen is administered at least two months (including, e.g., at least three months, at least four months, at least five months, at least six months, or more) after completion of the primary regimen, and the two booster doses are administered at least two months apart from each other.

In some embodiments, a subject is administered a booster regimen comprising at least one 50 ug dose of RNA. In some embodiments, a subject is administered a booster regimen comprising at least one dose of 50 ug of RNA encoding a SARS-CoV-2 S protein from a Wuhan strain (e.g., BNT162b2). In some embodiments, a subject is administered a booster regimen comprising at least one dose of 50 ug of RNA encoding a SARS-CoV-2 S protein having one or more mutations that are characteristic of a SARS-CoV-2 variant (e.g., a variant described herein). In some embodiments, a subject is administered a booster regimen comprising at least one dose of 50 ug of RNA encoding a SARS-CoV-2 S protein having one or more mutations that are characteristic of an Omicron variant. In some embodiments, a subject is administered a booster regimen comprising at least one 50 ug dose of RNA, wherein the 50 ug of RNA comprises RNA encoding a SARS-CoV-2 S protein from a Wuhan strain and RNA encoding a SARS-CoV-2 S protein comprising mutations that are characteristic of a SARS-CoV-2 variant (e.g., in some embodiments, a subject is administered a booster regimen comprising a 50 ug dose of RNA comprising 25 ug of RNA encoding a SARS-CoV-2 S protein from a Wuhan strain and 25 ug of RNA encoding a SARS-CoV-2 S protein having one or more mutations that are characteristic of an Omicron variant).

In some embodiments, a subject is administered a booster regimen comprising at least one dose comprising 25 ug of RNA encoding a SARS-CoV-2 S protein from a Wuhan strain and 25 ug of RNA encoding a SARS-CoV-2 S protein having one or more mutations that are characteristic of a BA.1 Omicron variant. In some embodiments, a subject is administered a booster regimen comprising at least one dose comprising 25 ug of RNA encoding a SARS-CoV-2 S protein from a Wuhan strain and 25 ug of RNA encoding a SARS-CoV-2 S protein having one or more mutations that are characteristic of a BA.2 Omicron variant. In some embodiments, a subject is administered a booster regimen comprising at least one dose comprising 25 ug of RNA encoding a SARS-CoV-2 S protein from a Wuhan strain and 25 ug of RNA encoding a SARS-CoV-2 S protein having one or more mutations that are characteristic of a BA.3 Omicron variant. In some embodiments, a subject is administered a booster regimen comprising at least one dose comprising 25 ug of RNA encoding a SARS-CoV-2 S protein from a Wuhan strain and 25 ug of RNA encoding a SARS-CoV-2 S protein having one or more mutations that are characteristic of a BA.4 or BA.5 Omicron variant.

In some embodiments, a subject is administered a booster regimen comprising at least one dose comprising 25 ug of RNA encoding a SARS-CoV-2 S protein from a BA.1 Omicron variant and 25 ug of RNA encoding a SARS-CoV-2 S protein having one or more mutations that are characteristic of a BA.2 Omicron variant. In some embodiments, a subject is administered a booster regimen comprising at least one dose comprising 25 ug of RNA encoding a SARS-CoV-2 S protein from a BA.1 Omicron variant and 25 ug of RNA encoding a SARS-CoV-2 S protein having one or more mutations that are characteristic of a BA.3 Omicron variant. In some embodiments, a subject is administered a booster regimen comprising at least one dose comprising 25 ug of RNA encoding a SARS-CoV-2 S protein from a BA.1 Omicron variant and 25 ug of RNA encoding a SARS-CoV-2 S protein having one or more mutations that are characteristic of a BA.4 or BA.5 Omicron variant.

In some embodiments, a subject is administered a booster regimen comprising at least one dose comprising 25 ug of RNA encoding a SARS-CoV-2 S protein from a BA.2 Omicron variant and 25 ug of RNA encoding a SARS-CoV-2 S protein having one or more mutations that are characteristic of a BA.4 or BA.5 Omicron variant. In some embodiments, a subject is administered a booster regimen comprising at least one dose comprising 25 ug of RNA encoding a SARS-CoV-2 S protein from a BA.3 Omicron variant and 25 ug of RNA encoding a SARS-CoV-2 S protein having one or more mutations that are characteristic of a BA.4 or BA.5 Omicron variant.

In some embodiments, a subject is administered (i) a primary regimen comprising two 30 ug doses of RNA encoding a SARS-CoV-2 S protein from a Wuhan strain, wherein the two doses are administered approximately 21 days apart, and (ii) a booster regimen comprising at least one 50 ug dose of RNA encoding a SARS-CoV-2 S protein having one or mutations that are characteristic of an Omicron variant of SARS-CoV-2 (e.g., a BA.1, BA.2, BA.4 or BA.5 Omicron variant), wherein the booster regimen is administered at least two months (including, e.g., at least three months, at least four months, at least five months, at least six months, or more) after completion of the primary regimen. In some embodiments, a subject is administered (i) a primary regimen comprising two 30 ug doses of RNA encoding a SARS-CoV-2 S protein from a Wuhan strain, wherein the two doses are administered approximately 21 days apart, and (ii) a booster regimen comprising at least one 50 ug dose of RNA, wherein the 50 ug of RNA comprises 25 ug of RNA encoding a SARS-CoV-2 S protein of a Wuhan strain and 25 ug of RNA encoding a SARS-CoV-2 S protein having one or mutations that are characteristic of an Omicron variant (e.g., a BA.1, BA.2, BA.4, or BA.5 variant), wherein the booster regimen is administered at least two months (including, e.g., at least three months, at least four months, at least five months, at least six months, or more) after completion of a first booster regimen. In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a dose of 50 ug of RNA encoding a SARS-CoV-2 S protein having one or mutations that are characteristic of a BA.1 Omicron variant of SARS-CoV-2, wherein the booster regimen is administered at least two months (including, e.g., at least three months, at least four months, at least five months, at least six months, or more) after completion of the primary regimen. In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a 50 ug dose of RNA comprising 25 ug RNA encoding a SARS-CoV-2 S protein from a Wuhan strain and 25 ug of RNA encoding a SARS-CoV-2 S protein having one or more mutations that are characteristic of a BA.1 Omicron variant.

In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a dose of 50 ug of RNA encoding a SARS-CoV-2 S protein having one or mutations that are characteristic of a BA.2 Omicron variant of SARS-CoV-2, wherein the booster regimen is administered at least two months (including, e.g., at least three months, at least four months, at least five months, at least six months, or more) after completion of the primary regimen. In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a 50 ug dose of RNA comprising 25 ug RNA encoding a SARS-CoV-2 S protein from a Wuhan strain and 25 ug of RNA encoding a SARS-CoV-2 S protein having one or more mutations that are characteristic of a BA.2 Omicron variant.

In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a dose of 50 ug of RNA encoding a SARS-CoV-2 S protein having one or mutations that are characteristic of a BA.4 or BA.5 Omicron variant of SARS-CoV-2, wherein the booster regimen is administered at least two months (including, e.g., at least three months, at least four months, at least five months, at least six months, or more) after completion of the primary regimen. In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a 50 ug dose of RNA comprising 25 ug RNA encoding a SARS-CoV-2 S protein from a Wuhan strain and 25 ug of RNA encoding a SARS-CoV-2 S protein having one or more mutations that are characteristic of a BA.4 or BA.5 Omicron variant.

In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a 50 ug dose of RNA comprising 25 ug RNA encoding a SARS-CoV-2 S protein from a BA.1 Omicron variant and 25 ug of RNA encoding a SARS-CoV-2 S protein having one or more mutations that are characteristic of a BA.2 Omicron variant.

In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a 50 ug dose of RNA comprising 25 ug RNA encoding a SARS-CoV-2 S protein from a BA.1 Omicron variant and 25 ug of RNA encoding a SARS-CoV-2 S protein having one or more mutations that are characteristic of a BA.4 or BA.5 Omicron variant.

In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a 50 ug dose of RNA comprising 25 ug RNA encoding a SARS-CoV-2 S protein from a BA.2 Omicron variant and 25 ug of RNA encoding a SARS-CoV-2 S protein having one or more mutations that are characteristic of a BA.4 or BA.5 Omicron variant.

In some embodiments, a subject is administered a booster regimen comprising at least one 60 ug dose of RNA. In some embodiments, a subject is administered a booster regimen comprising 60 ug of RNA encoding a SARS-CoV-2 S protein from a Wuhan variant. In some embodiments, a subject is administered 60 ug of RNA encoding a SARS-CoV-2 S protein having one or more mutations that are characteristic of a SARS-CoV-2 variant (e.g., a variant described herein). In some embodiments, a subject is administered a booster regimen comprising 60 ug of RNA encoding a SARS-CoV-2 S protein having one or more mutations that are characteristic of an Omicron variant (e.g., a BA.1, BA.2, BA.4, or BA.5 Omicron variant). In some embodiments, a subject is administered a booster regimen comprising 60 ug of RNA, wherein the RNA comprises a first RNA encoding a SARS-CoV-2 S protein from a Wuhan strain, and at least one additional RNA encoding a SARS-CoV-2 S protein comprising mutations that are characteristic of a SARS-CoV-2 variant (e.g., in some embodiments, a subject is administered a booster regimen comprising 30 ug of RNA encoding a SARS-CoV-2 S protein from a Wuhan strain and 30 ug of RNA encoding a SARS-CoV-2 S protein having one or more mutations that are characteristic of an Omicron variant (e.g., a BA.1, BA.2, BA.4, or BA.5 variant).

In some embodiments, a subject is administered a booster regimen comprising at least one dose comprising 30 ug of RNA encoding a SARS-CoV-2 S protein from a Wuhan strain and 30 ug of RNA encoding a SARS-CoV-2 S protein having one or more mutations that are characteristic of a BA.1 Omicron variant. In some embodiments, a subject is administered a booster regimen comprising at least one dose comprising 20 ug of RNA encoding a SARS-CoV-2 S protein from a Wuhan strain and 40 ug of RNA encoding a SARS-CoV-2 S protein having one or more mutations that are characteristic of a BA.1 Omicron variant. In some embodiments, a subject is administered a booster regimen comprising at least one dose comprising 15 ug of RNA encoding a SARS-CoV-2 S protein from a Wuhan strain and 45 ug of RNA encoding a SARS-CoV-2 S protein having one or more mutations that are characteristic of a BA.1 Omicron variant. In some embodiments, a subject is administered a booster regimen comprising at least one dose comprising 30 ug of RNA encoding a SARS-CoV-2 S protein from a Wuhan strain and 30 ug of RNA encoding a SARS-CoV-2 S protein having one or more mutations that are characteristic of a BA.2 Omicron variant. In some embodiments, a subject is administered a booster regimen comprising at least one dose comprising 20 ug of RNA encoding a SARS-CoV-2 S protein from a Wuhan strain and 40 ug of RNA encoding a SARS-CoV-2 S protein having one or more mutations that are characteristic of a BA.2 Omicron variant. In some embodiments, a subject is administered a booster regimen comprising at least one dose comprising 15 ug of RNA encoding a SARS-CoV-2 S protein from a Wuhan strain and 45 ug of RNA encoding a SARS-CoV-2 S protein having one or more mutations that are characteristic of a BA.2 Omicron variant. In some embodiments, a subject is administered a booster regimen comprising at least one dose comprising 30 ug of RNA encoding a SARS-CoV-2 S protein from a Wuhan strain and 30 ug of RNA encoding a SARS-CoV-2 S protein having one or more mutations that are characteristic of a BA.3 Omicron variant. In some embodiments, a subject is administered a booster regimen comprising at least one dose comprising 20 ug of RNA encoding a SARS-CoV-2 S protein from a Wuhan strain and 40 ug of RNA encoding a SARS-CoV-2 S protein having one or more mutations that are characteristic of a BA.3 Omicron variant. In some embodiments, a subject is administered a booster regimen comprising at least one dose comprising 15 ug of RNA encoding a SARS-CoV-2 S protein from a Wuhan strain and 45 ug of RNA encoding a SARS-CoV-2 S protein having one or more mutations that are characteristic of a BA.3 Omicron variant. In some embodiments, a subject is administered a booster regimen comprising at least one dose comprising 30 ug of RNA encoding a SARS-CoV-2 S protein from a Wuhan strain and 30 ug of RNA encoding a SARS-CoV-2 S protein having one or more mutations that are characteristic of a BA.4 or BA.5 Omicron variant. In some embodiments, a subject is administered a booster regimen comprising at least one dose comprising 20 ug of RNA encoding a SARS-CoV-2 S protein from a Wuhan strain and 40 ug of RNA encoding a SARS-CoV-2 S protein having one or more mutations that are characteristic of a BA.4 or BA.5 Omicron variant. In some embodiments, a subject is administered a booster regimen comprising at least one dose comprising 15 ug of RNA encoding a SARS-CoV-2 S protein from a Wuhan strain and 45 ug of RNA encoding a SARS-CoV-2 S protein having one or more mutations that are characteristic of a BA.4 or BA.5 Omicron variant.

In some embodiments, a subject is administered a booster regimen comprising at least one dose comprising 30 ug of RNA encoding a SARS-CoV-2 S protein from a BA.1 Omicron variant and 30 ug of RNA encoding a SARS-CoV-2 S protein having one or more mutations that are characteristic of a BA.2 Omicron variant. In some embodiments, a subject is administered a booster regimen comprising at least one dose comprising 20 ug of RNA encoding a SARS-CoV-2 S protein from a BA.1 Omicron variant and 40 ug of RNA encoding a SARS-CoV-2 S protein having one or more mutations that are characteristic of a BA.2 Omicron variant. In some embodiments, a subject is administered a booster regimen comprising at least one dose comprising 15 ug of RNA encoding a SARS-CoV-2 S protein from a BA.1 Omicron variant and 45 ug of RNA encoding a SARS-CoV-2 S protein having one or more mutations that are characteristic of a BA.2 Omicron variant. In some embodiments, a subject is administered a booster regimen comprising at least one dose comprising 30 ug of RNA encoding a SARS-CoV-2 S protein from a BA.1 Omicron variant and 30 ug of RNA encoding a SARS-CoV-2 S protein having one or more mutations that are characteristic of a BA.3 Omicron variant. In some embodiments, a subject is administered a booster regimen comprising at least one dose comprising 20 ug of RNA encoding a SARS-CoV-2 S protein from a BA.1 Omicron variant and 40 ug of RNA encoding a SARS-CoV-2 S protein having one or more mutations that are characteristic of a BA.3 Omicron variant. In some embodiments, a subject is administered a booster regimen comprising at least one dose comprising 15 ug of RNA encoding a SARS-CoV-2 S protein from a BA.1 Omicron variant and 45 ug of RNA encoding a SARS-CoV-2 S protein having one or more mutations that are characteristic of a BA.3 Omicron variant. In some embodiments, a subject is administered a booster regimen comprising at least one dose comprising 30 ug of RNA encoding a SARS-CoV-2 S protein from a BA.1 Omicron variant and 30 ug of RNA encoding a SARS-CoV-2 S protein having one or more mutations that are characteristic of a BA.4 or BA.5 Omicron variant. In some embodiments, a subject is administered a booster regimen comprising at least one dose comprising 20 ug of RNA encoding a SARS-CoV-2 S protein from a BA.1 Omicron variant and 40 ug of RNA encoding a SARS-CoV-2 S protein having one or more mutations that are characteristic of a BA.4 or BA.5 Omicron variant. In some embodiments, a subject is administered a booster regimen comprising at least one dose comprising 15 ug of RNA encoding a SARS-CoV-2 S protein from a BA.1 Omicron variant and 45 ug of RNA encoding a SARS-CoV-2 S protein having one or more mutations that are characteristic of a BA.4 or BA.5 Omicron variant.

In some embodiments, a subject is administered a booster regimen comprising at least one dose comprising 30 ug of RNA encoding a SARS-CoV-2 S protein from a BA.2 Omicron variant and 30 ug of RNA encoding a SARS-CoV-2 S protein having one or more mutations that are characteristic of a BA.4 or BA.5 Omicron variant. In some embodiments, a subject is administered a booster regimen comprising at least one dose comprising 20 ug of RNA encoding a SARS-CoV-2 S protein from a BA.2 Omicron variant and 40 ug of RNA encoding a SARS-CoV-2 S protein having one or more mutations that are characteristic of a BA.4 or BA.5 Omicron variant. In some embodiments, a subject is administered a booster regimen comprising at least one dose comprising 15 ug of RNA encoding a SARS-CoV-2 S protein from a BA.2 Omicron variant and 45 ug of RNA encoding a SARS-CoV-2 S protein having one or more mutations that are characteristic of a BA.4 or BA.5 Omicron variant. In some embodiments, a subject is administered a booster regimen comprising at least one dose comprising 30 ug of RNA encoding a SARS-CoV-2 S protein from a BA.3 Omicron variant and 30 ug of RNA encoding a SARS-CoV-2 S protein having one or more mutations that are characteristic of a BA.4 or BA.5 Omicron variant. In some embodiments, a subject is administered a booster regimen comprising at least one dose comprising 20 ug of RNA encoding a SARS-CoV-2 S protein from a BA.3 Omicron variant and 40 ug of RNA encoding a SARS-CoV-2 S protein having one or more mutations that are characteristic of a BA.4 or BA.5 Omicron variant. In some embodiments, a subject is administered a booster regimen comprising at least one dose comprising 15 ug of RNA encoding a SARS-CoV-2 S protein from a BA.3 Omicron variant and 45 ug of RNA encoding a SARS-CoV-2 S protein having one or more mutations that are characteristic of a BA.4 or BA.5 Omicron variant.

In some embodiments, a subject is administered (i) a primary regimen comprising two 30 ug doses of RNA encoding a SARS-CoV-2 S protein from a Wuhan strain of SARS-CoV-2, wherein the two doses are administered approximately 21 days apart, and (ii) a booster regimen comprising at least one 60 ug dose of RNA encoding a SARS-CoV-2 S protein from a Wuhan strain of SARS-CoV-2, wherein the booster regimen is administered at least two months (including, e.g., at least three months, at least four months, at least five months, at least six months, or more) after completion of the primary regimen.

In some embodiments, a subject is administered (i) a primary regimen comprising two 30 ug doses of RNA encoding a SARS-CoV-2 S protein from a Wuhan strain, wherein the two doses are administered approximately 21 days apart, and (ii) a booster regimen comprising at least one 60 ug dose of RNA encoding a SARS-CoV-2 S protein having one or mutations that are characteristic of an Omicron variant of SARS-CoV-2 (e.g., a BA.1, BA.2, BA.4 or BA.5 Omicron variant), wherein the booster regimen is administered at least two months (including, e.g., at least three months, at least four months, at least five months, at least six months, or more) after completion of the primary regimen. In some embodiments, a subject is administered (i) a primary regimen comprising two 30 ug doses of RNA encoding a SARS-CoV-2 S protein from a Wuhan strain, wherein the two doses are administered approximately 21 days apart, and (iii) a booster regimen comprising at least one 60 ug dose of RNA comprising 30 ug of RNA encoding a SARS-CoV-2 S protein having one or mutations that are characteristic of an Omicron variant of SARS-CoV-2 and 30 ug of RNA encoding a SARS-CoV-2 S protein from a Wuhan strain, wherein a second booster regimen is administered at least two months (including, e.g., at least three months, at least four months, at least five months, at least six months, or more) after completion of a first booster regimen.

In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a dose of 60 ug of RNA encoding a SARS-CoV-2 S protein having one or mutations that are characteristic of a BA.1 Omicron variant of SARS-CoV-2, wherein the booster regimen is administered at least two months (including, e.g., at least three months, at least four months, at least five months, at least six months, or more) after completion of the primary regimen. In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a 60 ug dose of RNA comprising 30 ug RNA encoding a SARS-CoV-2 S protein from a Wuhan strain and 30 ug of RNA encoding a SARS-CoV-2 S protein having one or more mutations that are characteristic of a BA.1 Omicron variant.

In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a dose of 60 ug of RNA encoding a SARS-CoV-2 S protein having one or mutations that are characteristic of a BA.2 Omicron variant of SARS-CoV-2, wherein the booster regimen is administered at least two months (including, e.g., at least three months, at least four months, at least five months, at least six months, or more) after completion of the primary regimen. In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a 60 ug dose of RNA comprising 30 ug RNA encoding a SARS-CoV-2 S protein from a Wuhan strain and 30 ug of RNA encoding a SARS-CoV-2 S protein having one or more mutations that are characteristic of a BA.2 Omicron variant.

In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a dose of 60 ug of RNA encoding a SARS-CoV-2 S protein having one or mutations that are characteristic of a BA.4 or BA.5 Omicron variant of SARS-CoV-2, wherein the booster regimen is administered at least two months (including, e.g., at least three months, at least four months, at least five months, at least six months, or more) after completion of the primary regimen. In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a 60 ug dose of RNA comprising 30 ug RNA encoding a SARS-CoV-2 S protein from a Wuhan strain and 30 ug of RNA encoding a SARS-CoV-2 S protein having one or more mutations that are characteristic of a BA.4/5 Omicron variant.

In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a 60 ug dose of RNA comprising 30 ug RNA encoding a SARS-CoV-2 S protein from a BA.1 Omicron variant and 30 ug of RNA encoding a SARS-CoV-2 S protein having one or more mutations that are characteristic of a BA.2 Omicron variant.

In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a 60 ug dose of RNA comprising 30 ug RNA encoding a SARS-CoV-2 S protein from a BA.1 Omicron variant and 30 ug of RNA encoding a SARS-CoV-2 S protein having one or more mutations that are characteristic of a BA.4 or BA.5 Omicron variant.

In some embodiments, a subject is administered (i) a primary regimen comprising at least two 30 ug doses of RNA encoding a SARS-CoV-2 S protein from a Wuhan strain, wherein the two doses are administered at least approximately 21 days apart, and (ii) a booster regimen comprising a 60 ug dose of RNA comprising 30 ug RNA encoding a SARS-CoV-2 S protein from a BA.2 Omicron variant and 30 ug of RNA encoding a SARS-CoV-2 S protein having one or more mutations that are characteristic of a BA.4 or BA.5 Omicron variant.

In some embodiments, a patient is administered a primary regimen comprising two 30 ug doses, administered approximately 21 days apart, and a booster regimen comprising at least one 60 ug dose of RNA (e.g., 60 ug of RNA encoding a SARS-CoV-2 S protein from a Wuhan strain, 60 ug of RNA encoding a SARS-CoV-2 S protein having one or more mutations that are characteristic of an Omicron variant (e.g., a BA.1, BA.2, BA.4, or BA.5 Omicron variant), or 30 ug of RNA encoding a SARS-CoV-2 S protein from a Wuhan strain and 30 ug of RNA encoding a SARS-CoV-2 S protein having one or more mutations that are characteristic of an Omicron variant). In some embodiments, a patient is administered a primary regimen comprising two 30 ug doses, administered approximately 21 days apart, and a booster regimen comprising at least one 50 ug dose of RNA (e.g., 50 ug of RNA encoding a SARS-CoV-2 S protein from a Wuhan strain, 50 ug of RNA encoding a SARS-CoV-2 S protein having one or more mutations that are characteristic of an Omicron variant, or 25 ug of RNA encoding a SARS-CoV-2 S protein from a Wuhan strain and 25 ug of RNA encoding a SARS-CoV-2 S protein having one or more mutations that are characteristic of an Omicron variant). In some embodiments, a patient is administered a primary regimen comprising two 30 ug doses, administered approximately 21 days apart, and a booster regimen comprising at least one 30 ug dose of RNA (e.g., 30 ug of RNA encoding a SARS-CoV-2 S protein from a Wuhan strain, 30 ug of RNA encoding a SARS-CoV-2 S protein having one or more mutations that are characteristic of an Omicron variant, or 15 ug of RNA encoding a SARS-CoV-2 S protein from a Wuhan strain and 15 ug of RNA encoding a SARS-CoV-2 S protein having one or more mutations that are characteristic of an Omicron variant).

In some embodiments, a primary regimen may involve one or more 30 ug doses and a booster regimen may involve one or more 20 ug doses, one or more 10 ug doses, and/or one or more 3 ug doses. In some embodiments, a primary regimen may involve one or more 20 ug doses and a booster regimen may involve one or more 10 ug doses, and/or one or more 3 ug doses. In some embodiments, a primary regimen may involve one or more 10 ug doses and a booster regimen may involve one or more 3 ug doses. In some embodiments, a primary regimen may involve one or more 3 ug doses, and a booster regimen may also involve one or more 3 ug doses.

In some embodiments, a booster regimen comprises a single dose, e.g., for patients who experienced an adverse reaction while receiving the primary regimen.

In some embodiments, the same RNA as used in a primary regimen is used in a booster regimen. In some embodiment, an RNA used in primary and booster regimens is BNT162b2.

In some embodiments, a different RNA is used in a booster regimen relative to that used in a primary regimen administered to the same subject. In some embodiments, BNT162b2 is used in a primary regimen but not in a booster regimen. In some embodiments, BNT162b2 is used in a booster regimen but not in a primary regimen. In some embodiments, a similar BNT162b2 construct can be used in a primary regimen and in a booster regimen, except that the RNA constructs used in the primary and booster regimens encode a SARS-CoV-2 S protein (or an immunogenic portion thereof) of different SARS-CoV-2 strains (e.g., as described herein).

In some embodiments, where BNT162b2 is used for a primary regimen or a booster regimen but not both, and a different RNA is used in the other, such different RNA may be an RNA encoding the same SARS-CoV-2 S protein but with different codon optimization or other different RNA sequence. In some embodiments, such different RNA may encode a SARS-CoV-2 S protein (or an immunogenic portion thereof) of a different SARS-CoV-2 strain, e.g., of a variant strain discussed herein. In some such embodiments, such variant strain that is prevalent or rapidly spreading in a relevant jurisdiction. In some embodiments, such different RNA may be an RNA encoding a SARS-CoV-2 S protein or variant thereof (or immunogenic portion of either) comprising one or more mutations described herein for S protein variants such as SARS-CoV-2 S protein variants, in particular naturally occurring S protein variants; in some such embodiments, a SARS-CoV-2 variant may be selected from the group consisting of VOC-202012/01, 501.V2, Cluster 5 and B.1.1.248. In some embodiments, a SARS-CoV-2 variant may be selected from the group consisting of VOC-202012/01, 501.V2, Cluster 5 and B.1.1.248, B.1.1.7, B.1.617.2, and B.1.1.529. In some embodiments, a booster regimen comprises at least one dose of RNA that encodes a SARS-CoV-2 S protein (or an immunogenic fragment thereof) of a variant that is spreading rapidly in a relevant jurisdiction at the time of administration. In some such embodiments, a variant that is encoded by RNA administered in a booster regimen may be different from that encoded by RNA administered in a primary regimen.

In some embodiments, a booster regimen comprises administering (i) a dose of RNA encoding the same SARS-CoV-2 S protein (or an immunogenic fragment thereof) as the RNA administered in the primary regimen (e.g., an RNA encoding a SARS-CoV-2the S protein (or an immunogenic fragment thereof) from the SARS-CoV-2 Wuhan strain) and (ii) a dose of RNA encoding a SARS-CoV-2 S protein (or an immunogenic fragment thereof) of a variant that is spreading rapidly in a relevant jurisdiction at the time of administration (e.g., a SARS-CoV-2 S protein (or an immunogenic fragment thereof) from one of the SARS-CoV-2 variants discussed herein).

In some embodiments, a booster regimen comprises multiple doses (e.g., at least two doses, at least three doses, or more). For example, in some embodiments, a first dose of a booster regimen may comprise an RNA encoding the same SARS-CoV-2 S protein (or an immunogenic fragment thereof) administered in the primary regimen and a second dose of a booster regimen may comprise the RNA encoding a SARS-CoV-2 S protein of a variant that is spreading rapidly in a relevant jurisdiction at the time of administration. In some embodiments, a first dose of a booster regimen may comprise RNA encoding a SARS-CoV-2 S protein (or an immunogenic fragment thereof) of a variant that is spreading rapidly in a relevant jurisdiction at the time of administration and a second dose of a booster regimen may comprise RNA encoding the same SARS-CoV-2 S protein (or an immunogenic fragment thereof) administered in the primary regimen. In some embodiments, the booster regimen comprises multiple doses, and the RNA encoding the S protein of a variant that is spreading rapidly in a relevant jurisdiction is administered in a first dose and the RNA encoding the S protein administered in the primary regimen is administered in a second dose.

In some embodiments, doses (e.g., a first and a second dose or any two consecutive doses) in a booster regimen are administered at least 2 weeks apart, including, e.g., at least 3 weeks, at least 4 weeks, at least 5 weeks, at least 6 weeks, at least 7 week, at least 8 weeks, at least 9 weeks, at least 10 weeks, at least 11 weeks, at least 12 weeks, at least 13 weeks, at least 14 weeks, at least 15 weeks, at least 16 weeks, or longer, apart. In some embodiments, doses (e.g., a first and a second dose or any two consecutive doses) in a booster regimen are administered approximately 2 to 168 weeks apart. In some embodiments, doses (e.g., a first and a second dose or any two consecutive doses) in a booster regimen are administered approximately 3 to 12 weeks apart. In some embodiments, doses (e.g., a first and a second dose or any two consecutive doses) in a booster regimen are administered approximately 4 to 10 weeks apart. In some embodiments, doses (e.g., a first and a second dose or any two consecutive doses) in a booster regimen are administered approximately 6 to 8 weeks apart. (e.g., about 21 days apart, or about 6 to 8 weeks apart). In some embodiments, the first and second dose are administered on the same day (e.g., by intramuscular injection at different sites on the subject).

In such embodiments, the booster regimen can optionally further comprise a third and fourth dose, administered approximately 2 to 8 weeks after the first and second dose (e.g., about 21 days after the first and second dose, or about 6 weeks to about 8 weeks after the first and second dose), where the third and fourth dose are also administered on the same day (e.g., by intramuscular injection at different sites on the subject), and comprise the same RNAs administered in the first and second doses of the booster regimen.

In some embodiments, multiple booster regimens may be administered. In some embodiments, a booster regimen is administered to a patient who has previously been administered a booster regimen.

In some embodiments, a second booster regimen is administered to a patient who has previously received a first booster regimen, and the amount of RNA administered in at least one dose of a second booster regimen is higher than the amount of RNA administered in at least one dose of a first booster regimen.

In some embodiments, a second booster regimen comprises administering at least one dose of 3 ug of RNA. In some embodiments, a second booster regimen comprises administering at least one dose of 5 ug of RNA. In some embodiments, a second booster regimen comprises administering at least one dose of 10 ug of RNA. In some embodiments, a second booster regimen comprises administering at least one dose of 15 ug of RNA. In some embodiments, a second booster regimen comprises administering at least one dose of 20 ug of RNA. In some embodiments, a second booster regimen comprises administering at least one dose of 25 ug of RNA. In some embodiments, a second booster regimen comprises administering at least one dose of 30 ug of RNA. In some embodiments, a second booster regimen comprises administering at least one dose of 50 ug of RNA. In some embodiments, a second booster regimen comprises administering at least one dose of 60 ug of RNA.

In some embodiments, a subject is administered a primary regimen that comprises two doses of 30 ug of RNA, administered approximately 21 days apart, and a booster regimen comprising at least one dose of approximately 30 ug of RNA. In some embodiments, a subject is administered a primary regimen that comprises two doses of 30 ug of RNA, administered approximately 21 days apart, and a booster regimen comprising at least one dose of approximately 50 ug of RNA. In some embodiments, a subject is administered a primary regimen that comprises two doses of 30 ug of RNA, administered approximately 21 days apart, and a booster regimen comprising at least one dose of approximately 60 ug of RNA.

In some embodiments, a subject is administered a primary regimen that comprises two doses of 30 ug of RNA, administered approximately 21 days apart, a first booster regimen comprising at least one dose of approximately 30 ug of RNA, and a second booster regimen comprising at least one dose of approximately 30 ug of RNA. In some embodiments, a subject is administered a primary regimen that comprises two doses of 30 ug of RNA, administered approximately 21 days apart, a first booster regimen comprising at least one dose of approximately 30 ug of RNA, and a second booster regimen comprising at least one dose of approximately 50 ug of RNA. In some embodiments, a subject is administered a primary regimen that comprises two doses of 30 ug of RNA, administered approximately 21 days apart, a first booster regimen comprising at least one dose of approximately 30 ug of RNA, and a second booster regimen comprising at least one dose of approximately 60 ug of RNA. In some embodiments, a first booster regimen comprises two doses of RNA, wherein each dose comprises an RNA encoding a Spike protein from a different SARS-CoV-2 variant. In some embodiments, a first booster regimen comprises two doses of RNA, wherein each dose comprises an RNA encoding a Spike protein from a different SARS-CoV-2 variant, and wherein the two doses of RNA are administered on the same day. In some embodiments, the two doses of RNA are administered in a single composition (e.g., by mixing a first composition comprising an RNA encoding a Spike protein from a first SARS-CoV-2 variant with a second composition comprising an RNA encoding a Spike protein from a second SARS-CoV-2 variant).

In some embodiments, a subject is administered a booster regimen comprising a first dose comprising an RNA that encodes a Spike protein from a Wuhan strain of SARS-CoV-2 and a second dose comprising an RNA that encodes a Spike protein comprising mutations from a variant that is prevalent and/or rapidly spreading in a relevant jurisdiction at the time of administering the booster regimen, wherein the first dose and the second dose of RNA may be administered on the same day. In some embodiments, a subject is administered a booster regimen comprising a first dose comprising an RNA that encodes a Spike protein from a Wuhan strain of SARS-CoV-2 and a second dose comprising an RNA that encodes a Spike protein comprising mutations from an alpha variant of SARS-CoV-2, wherein the first dose and the second dose may be administered on the same day. In some embodiments, a subject is administered a booster regimen comprising a first dose comprising an RNA that encodes a Spike protein from a Wuhan strain of SARS-CoV-2 and a second dose comprising an RNA that encodes a Spike protein comprising mutations from a beta variant of SARS-CoV-2, wherein the first dose and the second dose may be administered on the same day. In some embodiments, a subject is administered a booster regimen comprising a first dose comprising an RNA that encodes a Spike protein from a Wuhan strain of SARS-CoV-2 and a second dose comprising an RNA that encodes a Spike protein comprising mutations from a delta variant of SARS-CoV-2, wherein the first dose and the second dose may be administered on the same day. In some embodiments, a subject is administered a booster regimen comprising a first dose comprising an RNA that encodes a Spike protein from a Wuhan strain of SARS-CoV-2 and a second dose comprising an RNA that encodes a Spike protein comprising mutations from an Omicron variant of SARS-CoV-2, wherein the first dose and the second dose may be administered on the same day. Such booster regimens may be administered, e.g., to a subject previously administered a primary dosing regimen and/or to a subject previously administered a primary dosing regimen and a booster regimen.

In some embodiments, a subject is administered a first booster regimen comprising a first dose of 15 ug of RNA encoding a Spike protein from a Wuhan variant and a second dose of 15 ug of RNA encoding a Spike protein from an Omicron variant of SARS-CoV-2, where the first and the second dose are administered on the same day (e.g., wherein compositions comprising the RNA are mixed prior to administration, and the mixture is then administered to a patient). In some embodiments, a subject is administered a first booster regimen comprising a first dose of 25 ug of RNA encoding a Spike protein from a Wuhan variant and a second dose of 25 ug of RNA encoding a Spike protein from an Omicron variant of SARS-CoV-2. In some embodiments, the first and the second doses are optionally administered on the same day. In some embodiments, a subject is administered a first booster regimen comprising a first dose of 25 ug of RNA encoding a Spike protein from a Wuhan variant and a second dose of 25 ug of RNA encoding a Spike protein from an Omicron variant of SARS-CoV-2. In some embodiments, the first and the second doses are administered on the same day. In some embodiments, a subject is administered a first booster regimen comprising a first dose of 30 ug of RNA encoding a Spike protein from a Wuhan variant and a second dose of 30 ug of RNA encoding a Spike protein from an Omicron variant of SARS-CoV-2, wherein the first and the second dose are optionally administered on the same day (e.g., in separate administrations or as administration of a multivalent vaccine). In some embodiments, such a first booster regimen is administered to a subject previously administered a primary regimen comprising two doses of 30 ug of RNA, administered about 21 days apart wherein the first booster regimen is administered at least 3 months (e.g., at least 4, at least 5, or at least 6 months) after administration of a primary regimen.

In some embodiments, a subject is administered a second booster regimen comprising a first dose of 15 ug of RNA encoding a Spike protein from a Wuhan variant and a second dose of 15 ug of RNA encoding a Spike protein from an Omicron variant of SARS-CoV-2, where the first and the second dose are administered on the same day (e.g., wherein compositions comprising the RNA are mixed prior to administration to form a multivalent vaccine, and the mixture is then administered to a patient). In some embodiments, a subject is administered a second booster regimen comprising a first dose of 25 ug of RNA encoding a Spike protein from a Wuhan variant and a second dose of 25 ug of RNA encoding a Spike protein from an Omicron variant of SARS-CoV-2, wherein the first dose and the second dose are optionally administered on the same day (e.g., via administration of a multivalent vaccine or via administration of separate compositions). In some embodiments, a subject is administered a second booster regimen comprising a first dose of 25 ug of RNA encoding a Spike protein from a Wuhan variant and a second dose of 25 ug of RNA encoding a Spike protein from an Omicron variant of SARS-CoV-2. In some embodiments, a subject is administered a second booster regimen comprising a first dose of 30 ug of RNA encoding a Spike protein from a Wuhan variant and a second dose of 30 ug of RNA encoding a Spike protein from an Omicron variant of SARS-CoV-2, wherein the first dose and the second dose are optionally administered on the same day (e.g., via administration of a multivalent vaccine or via administration of separate compositions). In some embodiments, such a second booster regimen is administered to a subject previously administered a primary regimen comprising two doses of 30 ug of RNA, administered about 21 days apart. In some embodiments, such a second booster regimen is administered to a subject previously administered a primary regimen comprising two doses of 30 ug of RNA, administered about 21 days apart, and a first booster regimen comprising a dose of 30 ug of RNA, wherein the second booster regimen is administered at least 3 months (e.g., at least 4, at least 5, or at least 6 months) after administration of a first booster regimen.

In some embodiments, patients receiving dose(s) of RNA compositions as described herein are monitored for one or more particular conditions, e.g., following administration of one or more doses. In some embodiments, such condition(s) may be or comprise allergic reaction(s) (particularly in subject(s) with a history of relevant allergies or allergic reactions), myocarditis (inflammation of the heart muscle, particularly where the subject is a young male and/or may have experienced prior such inflammation), pericarditis (inflammation of the lining outside the heart, particularly where the subject is a young males and/or may have experienced prior such inflammation), fever, bleeding (particularly where the subject is known to have a bleeding disorder or to be receiving therapy with a blood thinner). Alternatively or additionally, patients who may receive closer monitoring may be or include patients who are immunocompromised or are receiving therapy with a medicine that affects the immune system, are pregnant or planning to become pregnant, are breastfeeding, have received another COVID-19 vaccine, and/or have ever fainted in association with an injection. In some embodiments, patients are monitored for myocarditis following administration of one of the compositions disclosed herein. In some embodiments, patients are monitored for pericarditis following administration of one of the compositions disclosed herein. Patients may be monitored and/or treated for the condition using current standards of care.

In some embodiments, efficacy for RNA (e.g., mRNA) compositions described in pediatric populations (e.g., described herein) may be assessed by various metrics described herein (including, e.g., but not limited to COVID-19 incidence per 1000 person-years in subjects with no serological or virological evidence of past SARS-CoV-2 infection; geometric mean ratio (GMR) of SARS CoV-2 neutralizing titers measured, e.g., 7 days after a second dose; etc.)

In some embodiments, pediatric populations described herein (e.g., from 12 to less than 16 years of age) may be monitored for occurrence of multisystem inflammatory syndrome (MIS) (e.g., inflammation in different body parts such as, e.g., heart, lung, kidneys, brain, skin eyes, and/or gastrointestinal organs), after administration of an RNA composition (e.g., mRNA) described herein. Exemplary symptoms of MIS in children may include, but are not limited to fever, abdominal pain, vomiting, diarrhea, neck pain, rash, bloodshot eyes, feeling extra tried, and combinations thereof.

In one embodiment, RNA administered as described above is nucleoside modified messenger RNA (modRNA) described herein as BNT162b1 (RBP020.3), BNT162b2 (RBP020.1 or RBP020.2), or BNT162b3 (e.g., BNT162b3c). In one embodiment, RNA administered as described above is nucleoside modified messenger RNA (modRNA) described herein as RBP020.2. In one embodiment, RNA encoding a vaccine antigen is nucleoside modified messenger RNA (modRNA) described herein as BNT162b3 (e.g., BNT162b3c).

In one embodiment, RNA administered as described above is nucleoside modified messenger RNA (modRNA) and (i) comprises the nucleotide sequence of SEQ ID NO: 21, a nucleotide sequence having at least 99%, 98%, 97%, 96%, 95%, 90%, 85%, or 80% identity to the nucleotide sequence of SEQ ID NO: 21, and/or (ii) encodes an amino acid sequence comprising the amino acid sequence of SEQ ID NO: 5, or an amino acid sequence having at least 99%, 98%, 97%, 96%, 95%, 90%, 85%, or 80% identity to the amino acid sequence of SEQ ID NO: 5. In one embodiment, RNA administered as described above is nucleoside modified messenger RNA (modRNA) and (i) comprises the nucleotide sequence of SEQ ID NO: 21; and/or (ii) encodes an amino acid sequence comprising the amino acid sequence of SEQ ID NO: 5.

In one embodiment, RNA administered as described above is nucleoside modified messenger RNA (modRNA) and (i) comprises the nucleotide sequence of SEQ ID NO: 19, or 20, a nucleotide sequence having at least 99%, 98%, 97%, 96%, 95%, 90%, 85%, or 80% identity to the nucleotide sequence of SEQ ID NO: 19, or 20, and/or (ii) encodes an amino acid sequence comprising the amino acid sequence of SEQ ID NO: 7, or an amino acid sequence having at least 99%, 98%, 97%, 96%, 95%, 90%, 85%, or 80% identity to the amino acid sequence of SEQ ID NO: 7. In one embodiment, RNA administered as described above is nucleoside modified messenger RNA (modRNA) and (i) comprises the nucleotide sequence of SEQ ID NO: 19, or 20; and/or (ii) encodes an amino acid sequence comprising the amino acid sequence of SEQ ID NO: 7.

In one embodiment, RNA administered as described above is nucleoside modified messenger RNA (modRNA) and (i) comprises the nucleotide sequence of SEQ ID NO: 20, a nucleotide sequence having at least 99%, 98%, 97%, 96%, 95%, 90%, 85%, or 80% identity to the nucleotide sequence of SEQ ID NO: 20, and/or (ii) encodes an amino acid sequence comprising the amino acid sequence of SEQ ID NO: 7, or an amino acid sequence having at least 99%, 98%, 97%, 96%, 95%, 90%, 85%, or 80% identity to the amino acid sequence of SEQ ID NO: 7. In one embodiment, RNA administered as described above is nucleoside modified messenger RNA (modRNA) and (i) comprises the nucleotide sequence of SEQ ID NO: 20; and/or (ii) encodes an amino acid sequence comprising the amino acid sequence of SEQ ID NO: 7.

In one embodiment, RNA administered as described above is nucleoside modified messenger RNA (modRNA) and (i) comprises the nucleotide sequence of SEQ ID NO: 30, a nucleotide sequence having at least 99%, 98%, 97%, 96%, 95%, 90%, 85%, or 80% identity to the nucleotide sequence of SEQ ID NO: 30, and/or (ii) encodes an amino acid sequence comprising the amino acid sequence of SEQ ID NO: 29, or an amino acid sequence having at least 99%, 98%, 97%, 96%, 95%, 90%, 85%, or 80% identity to the amino acid sequence of SEQ ID NO: 29. In one embodiment, RNA administered as described above is nucleoside modified messenger RNA (modRNA) and (i) comprises the nucleotide sequence of SEQ ID NO: 30; and/or (ii) encodes an amino acid sequence comprising the amino acid sequence of SEQ ID NO: 29.

In one embodiment, RNA administered is nucleoside modified messenger RNA (modRNA), (i) comprises the nucleotide sequence of SEQ ID NO: 20; and/or (ii) encodes an amino acid sequence comprising the amino acid sequence of SEQ ID NO: 7, and is administered in an amount of about 30 µg per dose. In one embodiment, at least two of such doses are administered. For example, a second dose may be administered about 21 days following administration of the first dose.

In some embodiments, populations to be treated with RNA described herein comprise, essentially consist of, or consist of subjects of age of at least 50, at least 55, at least 60, or at least 65. In some embodiments, populations to be treated with RNA described herein comprise, essentially consist of, or consist of subjects of age of between 55 to 90, 60 to 85, or 65 to 85.

In some embodiments, the period of time between the doses administered is at least 7 days, at least 14 days, or at least 21 days. In some embodiments, the period of time between the doses administered is between 7 days and 28 days such as between 14 days and 23 days.

In some embodiments, no more than 5 doses, no more than 4 doses, or no more than 3 doses of the RNA described herein may be administered to a subject.

In some embodiments, the methods and agents described herein are administered (in a regimen, e.g., at a dose, frequency of doses and/or number of doses) such that adverse events (AE), i.e., any unwanted medical occurrence in a patient, e.g., any unfavourable and unintended sign, symptom, or disease associated with the use of a medicinal product, whether or not related to the medicinal product, are mild or moderate in intensity. In some embodiments, the methods and agents described herein are administered such that adverse events (AE) can be managed with interventions such as treatment with, e.g., paracetamol or other drugs that provide analgesic, antipyretic (fever-reducing) and/or anti-inflammatory effects, e.g., nonsteroidal anti-inflammatory drugs (NSAIDs), e.g., aspirin, ibuprofen, and naproxen. Paracetamol or "acetaminophen" which is not classified as a NSAID exerts weak anti-inflammatory effects and can be administered as analgesic according to the present disclosure.

In some embodiments, the methods and agents described herein provide a neutralizing effect in a subject to coronavirus, coronavirus infection, or to a disease or disorder associated with coronavirus.

In some embodiments, the methods and agents described herein following administration to a subject induce an immune response that blocks or neutralizes coronavirus in the subject. In some embodiments, the methods and agents described herein following administration to a subject induce the generation of antibodies such as IgG antibodies that block or neutralize coronavirus in the subject. In some embodiments, the methods and agents described herein following administration to a subject induce an immune response that blocks or neutralizes coronavirus S protein binding to ACE2 in the subject. In some embodiments, the methods and agents described herein following administration to a subject induce the generation of antibodies that block or neutralize coronavirus S protein binding to ACE2 in the subject.

In some embodiments, the methods and agents described herein following administration to a subject induce geometric mean concentrations (GMCs) of RBD domain-binding antibodies such as IgG antibodies of at least 500 U/ml, 1000 U/ml, 2000 U/ml, 3000 U/ml, 4000 U/ml, 5000 U/ml, 10000 U/ml, 15000 U/ml, 20000 U/ml, 25000 U/ml, 30000 U/ml or even higher. In some embodiments, the elevated GMCs of RBD domain-binding antibodies persist for at least 14 days, 21 days, 28 days, 1 month, 3 months, 6 months, 12 months or even longer.

In some embodiments, the methods and agents described herein following administration to a subject induce geometric mean titers (GMTs) of neutralizing antibodies such as IgG antibodies of at least 100 U/ml, 200 U/ml, 300 U/ml, 400 U/ml, 500 U/ml, 1000 U/ml, 1500 U/ml, or even higher. In some embodiments, the elevated GMTs of neutralizing antibodies persist for at least 14 days, 21 days, 28 days, 1 month, 3 months, 6 months, 12 months or even longer.

As used herein, the term "neutralization" refers to an event in which binding agents such as antibodies bind to a biological active site of a virus such as a receptor binding protein, thereby inhibiting the viral infection of cells. As used herein, the term "neutralization" with respect to coronavirus, in particular coronavirus S protein, refers to an event in which binding agents such as antibodies bind to the RBD domain of the S protein, thereby inhibiting the viral infection of cells. In particular, the term "neutralization" refers to an event in which binding agents eliminate or significantly reduce virulence (e.g. ability of infecting cells) of viruses of interest.

The type of immune response generated in response to an antigenic challenge can generally be distinguished by the subset of T helper (Th) cells involved in the response. Immune responses can be broadly divided into two types: Th1 and Th2. Th1 immune activation is optimized for intracellular infections such as viruses, whereas Th2 immune responses are optimized for humoral (antibody) responses. Th1 cells produce interleukin 2 (IL-2), tumor necrosis factor (TNFα) and interferon gamma (IFNγ). Th2 cells produce IL-4, IL-5, IL-6, IL-9, IL-10 and IL-13. Th1 immune activation is the most highly desired in many clinical situations. Vaccine compositions specialized in eliciting Th2 or humoral immune responses are generally not effective against most viral diseases.

In some embodiments, the methods and agents described herein following administration to a subject induce or promote a Th1-mediated immune response in the subject. In some embodiments, the methods and agents described herein following administration to a subject induce or promote a cytokine profile that is typical for a Th1-mediated immune response in the subject. In some embodiments, the methods and agents described herein following administration to a subject induce or promote the production of interleukin 2 (IL-2), tumor necrosis factor (TNFα) and/or interferon gamma (IFNγ) in the subject. In some embodiments, the methods and agents described herein following administration to a subject induce or promote the production of interleukin 2 (IL-2) and interferon gamma (IFNγ) in the subject. In some embodiments, the methods and agents described herein following administration to a subject do not induce or promote a Th2-mediated immune response in the subject, or induce or promote a Th2-mediated immune response in the subject to a significant lower extent compared to the induction or promotion of a Th1-mediated immune response. In some embodiments, the methods and agents described herein following administration to a subject do not induce or promote a cytokine profile that is typical for a Th2-mediated immune response in the subject, or induce or promote a cytokine profile that is typical for a Th2-mediated immune response in the subject to a significant lower extent compared to the induction or promotion of a cytokine profile that is typical for a Th1-mediated immune response. In some embodiments, the methods and agents described herein following administration to a subject do not induce or promote the production of IL-4, IL-5, IL-6, IL-9, IL-10 and/or IL-13, or induce or promote the production of IL-4, IL-5, IL-6, IL-9, IL-10 and/or IL-13 in the subject to a significant lower extent compared to the induction or promotion of interleukin 2 (IL-2), tumor necrosis factor (TNFα) and/or interferon gamma (IFNγ) in the subject. In some embodiments, the methods and agents described herein following administration to a subject do not induce or promote the production of IL-4, or induce or promote the production of IL-4 in the subject to a significant lower extent compared to the induction or promotion of interleukin 2 (IL-2) and interferon gamma (IFNγ) in the subject.

In some embodiments, the methods and agents described herein following administration to a subject induce an antibody response, in particular a neutralizing antibody response, in the subject that targets a panel of different S protein variants such as SARS-CoV-2 S protein variants, in particular naturally occurring S protein variants. In some embodiments, the panel of different S protein variants comprises at least 5, at least 10, at least 15, or even more S protein variants. In some embodiments, such S protein variants comprise variants having amino acid modifications in the RBD domain and/or variants having amino acid modifications outside the RBD domain. In one embodiment, such S protein variant comprises SARS-CoV-2 S protein or a naturally occurring variant thereof wherein the amino acid corresponding to position 321 (Q) in SEQ ID NO: 1 is S. In one embodiment, such S protein variant comprises SARS-CoV-2 S protein or a naturally occurring variant thereof wherein the amino acid corresponding to position 321 (Q) in SEQ ID NO: 1 is L. In one embodiment, such S protein variant comprises SARS-CoV-2 S protein or a naturally occurring variant thereof wherein the amino acid corresponding to position 341 (V) in SEQ ID NO: 1 is I. In one embodiment, such S protein variant comprises SARS-CoV-2 S protein or a naturally occurring variant thereof wherein the amino acid corresponding to position 348 (A) in SEQ ID NO: 1 is T. In one embodiment, such S protein variant comprises SARS-CoV-2 S protein or a naturally occurring variant thereof wherein the amino acid corresponding to position 354 (N) in SEQ ID NO: 1 is D. In one embodiment, such S protein variant comprises SARS-CoV-2 S protein or a naturally occurring variant thereof wherein the amino acid corresponding to position 359 (S) in SEQ ID NO: 1 is N. In one embodiment, such S protein variant comprises SARS-CoV-2 S protein or a naturally occurring variant thereof wherein the amino acid corresponding to position 367 (V) in SEQ ID NO: 1 is F. In one embodiment, such S protein variant comprises SARS-CoV-2 S protein or a naturally occurring variant thereof wherein the amino acid corresponding to position 378 (K) in SEQ ID NO: 1 is S. In one embodiment, such S protein variant comprises SARS-CoV-2 S protein or a naturally occurring variant thereof wherein the amino acid corresponding to position 378 (K) in SEQ ID NO: 1 is R. In one embodiment, such S protein variant comprises SARS-CoV-2 S protein or a naturally occurring variant thereof wherein the amino acid corresponding to position 408 (R) in SEQ ID NO: 1 is I. In one embodiment, such S protein variant comprises SARS-CoV-2 S protein or a naturally occurring variant thereof wherein the amino acid corresponding to position 409 (Q) in SEQ ID NO: 1 is E. In one embodiment, such S protein variant comprises SARS-CoV-2 S protein or a naturally occurring variant thereof wherein the amino acid corresponding to position 435 (A) in SEQ ID NO: 1 is S. In one embodiment, such S protein variant comprises SARS-CoV-2 S protein or a naturally occurring variant thereof wherein the amino acid corresponding to position 439 (N) in SEQ ID NO: 1 is K. In one embodiment, such S protein variant comprises SARS-CoV-2 S protein or a naturally occurring variant thereof wherein the amino acid corresponding to position 458 (K) in SEQ ID NO: 1 is R. In one embodiment, such S protein variant comprises SARS-CoV-2 S protein or a naturally occurring variant thereof wherein the amino acid corresponding to position 472 (I) in SEQ ID NO: 1 is V. In one embodiment, such S protein variant comprises SARS-CoV-2 S protein or a naturally occurring variant thereof wherein the amino acid corresponding to position 476 (G) in SEQ ID NO: 1 is S. In one embodiment, such S protein variant comprises SARS-CoV-2 S protein or a naturally occurring variant thereof wherein the amino acid corresponding to position 477 (S) in SEQ ID NO: 1 is N. In one embodiment, such S protein variant comprises SARS-CoV-2 S protein or a naturally occurring variant thereof wherein the amino acid corresponding to position 483 (V) in SEQ ID NO: 1 is A. In one embodiment, such S protein variant comprises SARS-CoV-2 S protein or a naturally occurring variant thereof wherein the amino acid corresponding to position 508 (Y) in SEQ ID NO: 1 is H. In one embodiment, such S protein variant comprises SARS-CoV-2 S protein or a naturally occurring variant thereof wherein the amino acid corresponding to position 519 (H) in SEQ ID NO: 1 is P. In one embodiment, such S protein variant comprises SARS-CoV-2 S protein or a naturally occurring variant thereof wherein the amino acid corresponding to position 614 (D) in SEQ ID NO: 1 is G.

In some embodiments, the methods and agents described herein following administration to a subject induce an antibody response, in particular a neutralizing antibody response, in the subject that targets a S protein variant such as SARS-CoV-2 S protein variant, in particular naturally occurring S protein variant comprising a mutation at a position corresponding to position 501 (N) in SEQ ID NO: 1. In one embodiment, the amino acid corresponding to position 501 (N) in SEQ ID NO: 1 is Y.

Said S protein variant comprising a mutation at a position corresponding to position 501 (N) in SEQ ID NO: 1 may comprise one or more further mutations. Such one or more further mutations may be selected from mutations at positions corresponding to the following positions in SEQ ID NO: 1: 69 (H), 70 (V), 144 (Y), 570 (A), 614 (D), 681 (P), 716 (T), 982 (S), 1118 (D), 80 (D), 215 (D), 484 (E), 701 (A), 18 (L), 246 (R), 417 (K), 242 (L), 243 (A), and 244 (L). In one embodiment, the amino acid corresponding to position 69 (H) in SEQ ID NO: 1 is deleted. In one embodiment, the amino acid corresponding to position 70 (V) in SEQ ID NO: 1 is deleted.

In one embodiment, the amino acid corresponding to position 144 (Y) in SEQ ID NO: 1 is deleted. In one embodiment, the amino acid corresponding to position 570 (A) in SEQ ID NO: 1 is D. In one embodiment, the amino acid corresponding to position 614 (D) in SEQ ID NO: 1 is G. In one embodiment, the amino acid corresponding to position 681 (P) in SEQ ID NO: 1 is H. In one embodiment, the amino acid corresponding to position 716 (T) in SEQ ID NO: 1 is I. In one embodiment, the amino acid corresponding to position 982 (S) in SEQ ID NO: 1 is A. In one embodiment, the amino acid corresponding to position 1118 (D) in SEQ ID NO: 1 is H. In one embodiment, the amino acid corresponding to position 80 (D) in SEQ ID NO: 1 is A. In one embodiment, the amino acid corresponding to position 215 (D) in SEQ ID NO: 1 is G. In one embodiment, the amino acid corresponding to position 484 (E) in SEQ ID NO: 1 is K. In one embodiment, the amino acid corresponding to position 701 (A) in SEQ ID NO: 1 is V. In one embodiment, the amino acid corresponding to position 18 (L) in SEQ ID NO: 1 is F. In one embodiment, the amino acid corresponding to position 246 (R) in SEQ ID NO: 1 is I. In one embodiment, the amino acid corresponding to position 417 (K) in SEQ ID NO: 1 is N. In one embodiment, the amino acid corresponding to position 242 (L) in SEQ ID NO: 1 is deleted. In one embodiment, the amino acid corresponding to position 243 (A) in SEQ ID NO: 1 is deleted. In one embodiment, the amino acid corresponding to position 244 (L) in SEQ ID NO: 1 is deleted.

In some embodiments, the methods and agents described herein following administration to a subject induce an antibody response, in particular a neutralizing antibody response, in the subject that targets VOC-202012/01.

In some embodiments, the methods and agents described herein following administration to a subject induce an antibody response, in particular a neutralizing antibody response, in the subject that targets a S protein variant comprising the following mutations at positions corresponding to the following positions in SEQ ID NO: 1: deletion 69-70, deletion 144, N501Y, A570D, D614G, P681H, T716I, S982A, and D1118H.

In some embodiments, the methods and agents described herein following administration to a subject induce an antibody response, in particular a neutralizing antibody response, in the subject that targets 501.V2.

In some embodiments, the methods and agents described herein following administration to a subject induce an antibody response, in particular a neutralizing antibody response, in the subject that targets a S protein variant comprising the following mutations at positions corresponding to the following positions in SEQ ID NO: 1: D80A, D215G, E484K, N501Y and A701V, and optionally: L18F, R246I, K417N, and deletion 242-244. Said S protein variant may also comprise a D->G mutation at a position corresponding to position 614 in SEQ ID NO: 1.

In some embodiments, the methods and agents described herein following administration to a subject induce an antibody response, in particular a neutralizing antibody response, in the subject that targets a S protein variant such as SARS-CoV-2 S protein variant, in particular naturally occurring S protein variant comprising a deletion at a position corresponding to positions 69 (H) and 70 (V) in SEQ ID NO: 1.

In some embodiments, a S protein variant comprising a deletion at a position corresponding to positions 69 (H) and 70 (V) in SEQ ID NO: 1 may comprise one or more further mutations. Such one or more further mutations may be selected from mutations at positions corresponding to the following positions in SEQ ID NO: 1: 144 (Y), 501 (N), 570 (A), 614 (D), 681 (P), 716 (T), 982 (S), 1118 (D), 80 (D), 215 (D), 484 (E), 701 (A), 18 (L), 246 (R), 417 (K), 242 (L), 243 (A), 244 (L), 453 (Y), 692 (I), 1147 (S), and 1229 (M). In one embodiment, the amino acid corresponding to position 144 (Y) in SEQ ID NO: 1 is deleted. In one embodiment, the amino acid corresponding to position 501 (N) in SEQ ID NO: 1 is Y. In one embodiment, the amino acid corresponding to position 570 (A) in SEQ ID NO: 1 is D. In one embodiment, the amino acid corresponding to position 614 (D) in SEQ ID NO: 1 is G. In one embodiment, the amino acid corresponding to position 681 (P) in SEQ ID NO: 1 is H. In one embodiment, the amino acid corresponding to position 716 (T) in SEQ ID NO: 1 is I. In one embodiment, the amino acid corresponding to position 982 (S) in SEQ ID NO: 1 is A. In one embodiment, the amino acid corresponding to position 1118 (D) in SEQ ID NO: 1 is H. In one embodiment, the amino acid corresponding to position 80 (D) in SEQ ID NO: 1 is A. In one embodiment, the amino acid corresponding to position 215 (D) in SEQ ID NO: 1 is G. In one embodiment, the amino acid corresponding to position 484 (E) in SEQ ID NO: 1 is K. In one embodiment, the amino acid corresponding to position 701 (A) in SEQ ID NO: 1 is V. In one embodiment, the amino acid corresponding to position 18 (L) in SEQ ID NO: 1 is F. In one embodiment, the amino acid corresponding to position 246 (R) in SEQ ID NO: 1 is I. In one embodiment, the amino acid corresponding to position 417 (K) in SEQ ID NO: 1 is N. In one embodiment, the amino acid corresponding to position 242 (L) in SEQ ID NO: 1 is deleted. In one embodiment, the amino acid corresponding to position 243 (A) in SEQ ID NO: 1 is deleted. In one embodiment, the amino acid corresponding to position 244 (L) in SEQ ID NO: 1 is deleted. In one embodiment, the amino acid corresponding to position 453 (Y) in SEQ ID NO: 1 is F. In one embodiment, the amino acid corresponding to position 692 (I) in SEQ ID NO: 1 is V. In one embodiment, the amino acid corresponding to position 1147 (S) in SEQ ID NO: 1 is L. In one embodiment, the amino acid corresponding to position 1229 (M) in SEQ ID NO: 1 is I.

In some embodiments, the methods and agents described herein following administration to a subject induce an antibody response, in particular a neutralizing antibody response, in the subject that targets VOC-202012/01.

In some embodiments, the methods and agents described herein following administration to a subject induce an antibody response, in particular a neutralizing antibody response, in the subject that targets a S protein variant comprising the following mutations at positions corresponding to the following positions in SEQ ID NO: 1: deletion 69-70, deletion 144, N501Y, A570D, D614G, P681H, T716I, S982A, and D1118H.

In some embodiments, the methods and agents described herein following administration to a subject induce an antibody response, in particular a neutralizing antibody response, in the subject that targets "Cluster 5".

In some embodiments, the methods and agents described herein following administration to a subject induce an antibody response, in particular a neutralizing antibody response, in the subject that targets a S protein variant comprising the following mutations at positions corresponding to the following positions in SEQ ID NO: 1: deletion 69-70, Y453F, 1692V, M1229I, and optionally S1147L.

In some embodiments, the methods and agents described herein following administration to a subject induce an antibody response, in particular a neutralizing antibody response, in the subject that targets a S protein variant such as SARS-CoV-2 S protein variant, in particular naturally occurring S protein variant comprising a mutation at a position corresponding to position 614 (D) in SEQ ID NO: 1. In one embodiment, the amino acid corresponding to position 614 (D) in SEQ ID NO: 1 is G.

In some embodiments, a S protein variant comprising a mutation at a position corresponding to position 614 (D) in SEQ ID NO: 1 may comprise one or more further mutations. Such one or more further mutations may be selected from mutations at positions corresponding to the following positions in SEQ ID NO: 1: 69 (H), 70 (V), 144 (Y), 501 (N), 570 (A), 681 (P), 716 (T), 982 (S), 1118 (D), 80 (D), 215 (D), 484 (E), 701 (A), 18 (L), 246 (R), 417 (K), 242 (L), 243 (A), 244 (L), 453 (Y), 692 (I), 1147 (S), and 1229 (M). In one embodiment, the amino acid corresponding to position 69 (H) in SEQ ID NO: 1 is deleted. In one embodiment, the amino acid corresponding to position 70 (V) in SEQ ID NO: 1 is deleted. In one embodiment, the amino acid corresponding to position 144 (Y) in SEQ ID NO: 1 is deleted. In one embodiment, the amino acid corresponding to position 501 (N) in SEQ ID NO: 1 is Y. In one embodiment, the amino acid corresponding to position 570 (A) in SEQ ID NO: 1 is D. In one embodiment, the amino acid corresponding to position 681 (P) in SEQ ID NO: 1 is H. In one embodiment, the amino acid corresponding to position 716 (T) in SEQ ID NO: 1 is I. In one embodiment, the amino acid corresponding to position 982 (S) in SEQ ID NO: 1 is A. In one embodiment, the amino acid corresponding to position 1118 (D) in SEQ ID NO: 1 is H. In one embodiment, the amino acid corresponding to position 80 (D) in SEQ ID NO: 1 is A. In one embodiment, the amino acid corresponding to position 215 (D) in SEQ ID NO: 1 is G. In one embodiment, the amino acid corresponding to position 484 (E) in SEQ ID NO: 1 is K. In one embodiment, the amino acid corresponding to position 701 (A) in SEQ ID NO: 1 is V. In one embodiment, the amino acid corresponding to position 18 (L) in SEQ ID NO: 1 is F. In one embodiment, the amino acid corresponding to position 246 (R) in SEQ ID NO: 1 is I. In one embodiment, the amino acid corresponding to position 417 (K) in SEQ ID NO: 1 is N. In one embodiment, the amino acid corresponding to position 242 (L) in SEQ ID NO: 1 is deleted. In one embodiment, the amino acid corresponding to position 243 (A) in SEQ ID NO: 1 is deleted. In one embodiment, the amino acid corresponding to position 244 (L) in SEQ ID NO: 1 is deleted. In one embodiment, the amino acid corresponding to position 453 (Y) in SEQ ID NO: 1 is F. In one embodiment, the amino acid corresponding to position 692 (I) in SEQ ID NO: 1 is V. In one embodiment, the amino acid corresponding to position 1147 (S) in SEQ ID NO: 1 is L. In one embodiment, the amino acid corresponding to position 1229 (M) in SEQ ID NO: 1 is I.

In some embodiments, the methods and agents described herein following administration to a subject induce an antibody response, in particular a neutralizing antibody response, in the subject that targets VOC-202012/01.

In some embodiments, the methods and agents described herein following administration to a subject induce an antibody response, in particular a neutralizing antibody response, in the subject that targets a S protein variant comprising the following mutations at positions corresponding to the following positions in SEQ ID NO: 1: deletion 69-70, deletion 144, N501Y, A570D, D614G, P681H, T716I, S982A, and D1118H.

In some embodiments, the methods and agents described herein following administration to a subject induce an antibody response, in particular a neutralizing antibody response, in the subject that targets a S protein variant comprising the following mutations at positions corresponding to the following positions in SEQ ID NO: 1: D80A, D215G, E484K, N501Y, D614G and A701V, and optionally: L18F, R246I, K417N, and deletion 242-244.

In some embodiments, the methods and agents described herein following administration to a subject induce an antibody response, in particular a neutralizing antibody response, in the subject that targets a S protein variant such as SARS-CoV-2 S protein variant, in particular naturally occurring S protein variant comprising a mutation at positions corresponding to positions 501 (N) and 614 (D) in SEQ ID NO: 1. In one embodiment, the amino acid corresponding to position 501 (N) in SEQ ID NO: 1 is Y and the amino acid corresponding to position 614 (D) in SEQ ID NO: 1 is G.

In some embodiments, a S protein variant comprising a mutation at positions corresponding to positions 501 (N) and 614 (D) in SEQ ID NO: 1 may comprise one or more further mutations. Such one or more further mutations may be selected from mutations at positions corresponding to the following positions in SEQ ID NO: 1: 69 (H), 70 (V), 144 (Y), 570 (A), 681 (P), 716 (T), 982 (S), 1118 (D), 80 (D), 215 (D), 484 (E), 701 (A), 18 (L), 246 (R), 417 (K), 242 (L), 243 (A), 244 (L), 453 (Y), 692 (I), 1147 (S), and 1229 (M). In one embodiment, the amino acid corresponding to position 69 (H) in SEQ ID NO: 1 is deleted. In one embodiment, the amino acid corresponding to position 70 (V) in SEQ ID NO: 1 is deleted. In one embodiment, the amino acid corresponding to position 144 (Y) in SEQ ID NO: 1 is deleted. In one embodiment, the amino acid corresponding to position 570 (A) in SEQ ID NO: 1 is D. In one embodiment, the amino acid corresponding to position 681 (P) in SEQ ID NO: 1 is H. In one embodiment, the amino acid corresponding to position 716 (T) in SEQ ID NO: 1 is I. In one embodiment, the amino acid corresponding to position 982 (S) in SEQ ID NO: 1 is A. In one embodiment, the amino acid corresponding to position 1118 (D) in SEQ ID NO: 1 is H. In one embodiment, the amino acid corresponding to position 80 (D) in SEQ ID NO: 1 is A. In one embodiment, the amino acid corresponding to position 215 (D) in SEQ ID NO: 1 is G. In one embodiment, the amino acid corresponding to position 484 (E) in SEQ ID NO: 1 is K. In one embodiment, the amino acid corresponding to position 701 (A) in SEQ ID NO: 1 is V. In one embodiment, the amino acid corresponding to position 18 (L) in SEQ ID NO: 1 is F. In one embodiment, the amino acid corresponding to position 246 (R) in SEQ ID NO: 1 is I. In one embodiment, the amino acid corresponding to position 417 (K) in SEQ ID NO: 1 is N. In one embodiment, the amino acid corresponding to position 242 (L) in SEQ ID NO: 1 is deleted. In one embodiment, the amino acid corresponding to position 243 (A) in SEQ ID NO: 1 is deleted. In one embodiment, the amino acid corresponding to position 244 (L) in SEQ ID NO: 1 is deleted. In one embodiment, the amino acid corresponding to position 453 (Y) in SEQ ID NO: 1 is F. In one embodiment, the amino acid corresponding to position 692 (I) in SEQ ID NO: 1 is V. In one embodiment, the amino acid corresponding to position 1147 (S) in SEQ ID NO: 1 is L. In one embodiment, the amino acid corresponding to position 1229 (M) in SEQ ID NO: 1 is I.

In some embodiments, the methods and agents described herein following administration to a subject induce an antibody response, in particular a neutralizing antibody response, in the subject that targets VOC-202012/01.

In some embodiments, the methods and agents described herein following administration to a subject induce an antibody response, in particular a neutralizing antibody response, in the subject that targets a S protein variant comprising the following mutations at positions corresponding to the following positions in SEQ ID NO: 1: deletion 69-70, deletion 144, N501Y, A570D, D614G, P681H, T716I, S982A, and D1118H.

In some embodiments, the methods and agents described herein following administration to a subject induce an antibody response, in particular a neutralizing antibody response, in the subject that targets a S protein variant comprising the following mutations at positions corresponding to the following positions in SEQ ID NO: 1: D80A, D215G, E484K, N501Y, D614G and A701V, and optionally: L18F, R246I, K417N, and deletion 242-244.

In some embodiments, the methods and agents described herein following administration to a subject induce an antibody response, in particular a neutralizing antibody response, in the subject that targets a S protein variant such as SARS-CoV-2 S protein variant, in particular naturally occurring S protein variant comprising a mutation at a position corresponding to position 484 (E) in SEQ ID NO: 1. In one embodiment, the amino acid corresponding to position 484 (E) in SEQ ID NO: 1 is K.

In some embodiments, a S protein variant comprising a mutation at a position corresponding to position 484 (E) in SEQ ID NO: 1 may comprise one or more further mutations. Such one or more further mutations may be selected from mutations at positions corresponding to the following positions in SEQ ID NO: 1: 69 (H), 70 (V), 144 (Y), 501 (N), 570 (A), 614 (D), 681 (P), 716 (T), 982 (S), 1118 (D), 80 (D), 215 (D), 701 (A), 18 (L), 246 (R), 417 (K), 242 (L), 243 (A), 244 (L), 453 (Y), 692 (I), 1147 (S), 1229 (M), 20 (T), 26 (P), 138 (D), 190 (R), 417 (K), 655 (H), 1027 (T), and 1176 (V). In one embodiment, the amino acid corresponding to position 69 (H) in SEQ ID NO: 1 is deleted. In one embodiment, the amino acid corresponding to position 70 (V) in SEQ ID NO: 1 is deleted. In one embodiment, the amino acid corresponding to position 144 (Y) in SEQ ID NO: 1 is deleted. In one embodiment, the amino acid corresponding to position 501 (N) in SEQ ID NO: 1 is Y. In one embodiment, the amino acid corresponding to position 570 (A) in SEQ ID NO: 1 is D. In one embodiment, the amino acid corresponding to position 614 (D) in SEQ ID NO: 1 is G. In one embodiment, the amino acid corresponding to position 681 (P) in SEQ ID NO: 1 is H. In one embodiment, the amino acid corresponding to position 716 (T) in SEQ ID NO: 1 is I. In one embodiment, the amino acid corresponding to position 982 (S) in SEQ ID NO: 1 is A. In one embodiment, the amino acid corresponding to position 1118 (D) in SEQ ID NO: 1 is H. In one embodiment, the amino acid corresponding to position 80 (D) in SEQ ID NO: 1 is A. In one embodiment, the amino acid corresponding to position 215 (D) in SEQ ID NO: 1 is G. In one embodiment, the amino acid corresponding to position 701 (A) in SEQ ID NO: 1 is V. In one embodiment, the amino acid corresponding to position 18 (L) in SEQ ID NO: 1 is F. In one embodiment, the amino acid corresponding to position 246 (R) in SEQ ID NO: 1 is I. In one embodiment, the amino acid corresponding to position 417 (K) in SEQ ID NO: 1 is N. In one embodiment, the amino acid corresponding to position 242 (L) in SEQ ID NO: 1 is deleted. In one embodiment, the amino acid corresponding to position 243 (A) in SEQ ID NO: 1 is deleted. In one embodiment, the amino acid corresponding to position 244 (L) in SEQ ID NO: 1 is deleted. In one embodiment, the amino acid corresponding to position 453 (Y) in SEQ ID NO: 1 is F. In one embodiment, the amino acid corresponding to position 692 (I) in SEQ ID NO: 1 is V. In one embodiment, the amino acid corresponding to position 1147 (S) in SEQ ID NO: 1 is L. In one embodiment, the amino acid corresponding to position 1229 (M) in SEQ ID NO: 1 is I. In one embodiment, the amino acid corresponding to position 20 (T) in SEQ ID NO: 1 is N. In one embodiment, the amino acid corresponding to position 26 (P) in SEQ ID NO: 1 is S. In one embodiment, the amino acid corresponding to position 138 (D) in SEQ ID NO: 1 is Y. In one embodiment, the amino acid corresponding to position 190 (R) in SEQ ID NO: 1 is S. In one embodiment, the amino acid corresponding to position 417 (K) in SEQ ID NO: 1 is T. In one embodiment, the amino acid corresponding to position 655 (H) in SEQ ID NO: 1 is Y. In one embodiment, the amino acid corresponding to position 1027 (T) in SEQ ID NO: 1 is I. In one embodiment, the amino acid corresponding to position 1176 (V) in SEQ ID NO: 1 is F.

In some embodiments, the methods and agents described herein following administration to a subject induce an antibody response, in particular a neutralizing antibody response, in the subject that targets 501.V2.

In some embodiments, the methods and agents described herein following administration to a subject induce an antibody response, in particular a neutralizing antibody response, in the subject that targets a S protein variant comprising the following mutations at positions corresponding to the following positions in SEQ ID NO: 1: D80A, D215G, E484K, N501Y and A701V, and optionally: L18F, R246I, K417N, and deletion 242-244. Said S protein variant may also comprise a D->G mutation at a position corresponding to position 614 in SEQ ID NO: 1. In some embodiments, the methods and agents described herein following administration to a subject induce an antibody response, in particular a neutralizing antibody response, in the subject that targets "B.1.1.28".

In some embodiments, the methods and agents described herein following administration to a subject induce an antibody response, in particular a neutralizing antibody response, in the subject that targets "B.1.1.248".

In some embodiments, the methods and agents described herein following administration to a subject induce an antibody response, in particular a neutralizing antibody response, in the subject that targets a S protein variant comprising the following mutations at positions corresponding to the following positions in SEQ ID NO: 1: L18F, T20N, P26S, D138Y, R190S, K417T, E484K, N501Y, H655Y, T1027I, and V1176F.

In some embodiments, the methods and agents described herein following administration to a subject induce an antibody response, in particular a neutralizing antibody response, in the subject that targets a S protein variant such as SARS-CoV-2 S protein variant, in particular naturally occurring S protein variant comprising a mutation at positions corresponding to positions 501 (N) and 484 (E) in SEQ ID NO: 1. In one embodiment, the amino acid corresponding to position 501 (N) in SEQ ID NO: 1 is Y and the amino acid corresponding to position 484 (E) in SEQ ID NO: 1 is K.

In some embodiments, a S protein variant comprising a mutation at positions corresponding to positions 501 (N) and 484 (E) in SEQ ID NO: 1 may comprise one or more further mutations. Such one or more further mutations may be selected from mutations at positions corresponding to the following positions in SEQ ID NO: 1: 69 (H), 70 (V), 144 (Y), 570 (A), 614 (D), 681 (P), 716 (T), 982 (S), 1118 (D), 80 (D), 215 (D), 701 (A), 18 (L), 246 (R), 417 (K), 242 (L), 243 (A), 244 (L), 453 (Y), 692 (I), 1147 (S), 1229 (M), 20 (T), 26 (P), 138 (D), 190 (R), 417 (K), 655 (H), 1027 (T), and 1176 (V). In one embodiment, the amino acid corresponding to position 69 (H) in SEQ ID NO: 1 is deleted. In one embodiment, the amino acid corresponding to position 70 (V) in SEQ ID NO: 1 is deleted. In one embodiment, the amino acid corresponding to position 144 (Y) in SEQ ID NO: 1 is deleted. In one embodiment, the amino acid corresponding to position 570 (A) in SEQ ID NO: 1 is D. In one embodiment, the amino acid corresponding to position 614 (D) in SEQ ID NO: 1 is G. In one embodiment, the amino acid corresponding to position 681 (P) in SEQ ID NO: 1 is H. In one embodiment, the amino acid corresponding to position 716 (T) in SEQ ID NO: 1 is I. In one embodiment, the amino acid corresponding to position 982 (S) in SEQ ID NO: 1 is A. In one embodiment, the amino acid corresponding to position 1118 (D) in SEQ ID NO: 1 is H. In one embodiment, the amino acid corresponding to position 80 (D) in SEQ ID NO: 1 is A. In one embodiment, the amino acid corresponding to position 215 (D) in SEQ ID NO: 1 is G. In one embodiment, the amino acid corresponding to position 701 (A) in SEQ ID NO: 1 is V. In one embodiment, the amino acid corresponding to position 18 (L) in SEQ ID NO: 1 is F. In one embodiment, the amino acid corresponding to position 246 (R) in SEQ ID NO: 1 is I. In one embodiment, the amino acid corresponding to position 417 (K) in SEQ ID NO: 1 is N. In one embodiment, the amino acid corresponding to position 242 (L) in SEQ ID NO: 1 is deleted. In one embodiment, the amino acid corresponding to position 243 (A) in SEQ ID NO: 1 is deleted. In one embodiment, the amino acid corresponding to position 244 (L) in SEQ ID NO: 1 is deleted. In one embodiment, the amino acid corresponding to position 453 (Y) in SEQ ID NO: 1 is F. In one embodiment, the amino acid corresponding to position 692 (I) in SEQ ID NO: 1 is V. In one embodiment, the amino acid corresponding to position 1147 (S) in SEQ ID NO: 1 is L. In one embodiment, the amino acid corresponding to position 1229 (M) in SEQ ID NO: 1 is I. In one embodiment, the amino acid corresponding to position 20 (T) in SEQ ID NO: 1 is N. In one embodiment, the amino acid corresponding to position 26 (P) in SEQ ID NO: 1 is S. In one embodiment, the amino acid corresponding to position 138 (D) in SEQ ID NO: 1 is Y. In one embodiment, the amino acid corresponding to position 190 (R) in SEQ ID NO: 1 is S. In one embodiment, the amino acid corresponding to position 417 (K) in SEQ ID NO: 1 is T. In one embodiment, the amino acid corresponding to position 655 (H) in SEQ ID NO: 1 is Y. In one embodiment, the amino acid corresponding to position 1027 (T) in SEQ ID NO: 1 is I. In one embodiment, the amino acid corresponding to position 1176 (V) in SEQ ID NO: 1 is F.

In some embodiments, the methods and agents described herein following administration to a subject induce an antibody response, in particular a neutralizing antibody response, in the subject that targets 501.V2.

In some embodiments, the methods and agents described herein following administration to a subject induce an antibody response, in particular a neutralizing antibody response, in the subject that targets a S protein variant comprising the following mutations at positions corresponding to the following positions in SEQ ID NO: 1: D80A, D215G, E484K, N501Y and A701V, and optionally: L18F, R246I, K417N, and deletion 242-244. Said S protein variant may also comprise a D->G mutation at a position corresponding to position 614 in SEQ ID NO: 1. In some embodiments, the methods and agents described herein following administration to a subject induce an antibody response, in particular a neutralizing antibody response, in the subject that targets "B.1.1.248".

In some embodiments, the methods and agents described herein following administration to a subject induce an antibody response, in particular a neutralizing antibody response, in the subject that targets a S protein variant comprising the following mutations at positions corresponding to the following positions in SEQ ID NO: 1: L18F, T20N, P26S, D138Y, R190S, K417T, E484K, N501Y, H655Y, T1027I, and V1176F.

In some embodiments, the methods and agents described herein following administration to a subject induce an antibody response, in particular a neutralizing antibody response, in the subject that targets a S protein variant such as SARS-CoV-2 S protein variant, in particular naturally occurring S protein variant comprising a mutation at positions corresponding to positions 501 (N), 484 (E) and 614 (D) in SEQ ID NO: 1. In one embodiment, the amino acid corresponding to position 501 (N) in SEQ ID NO: 1 is Y, the amino acid corresponding to position 484 (E) in SEQ ID NO: 1 is K and the amino acid corresponding to position 614 (D) in SEQ ID NO: 1 is G.

In some embodiments, a S protein variant comprising a mutation at positions corresponding to positions 501 (N), 484 (E) and 614 (D) in SEQ ID NO: 1 may comprise one or more further mutations. Such one or more further mutations may be selected from mutations at positions corresponding to the following positions in SEQ ID NO: 1: 69 (H), 70 (V), 144 (Y), 570 (A), 681 (P), 716 (T), 982 (S), 1118 (D), 80 (D), 215 (D), 701 (A), 18 (L), 246 (R), 417 (K), 242 (L), 243 (A), 244 (L), 453 (Y), 692 (I), 1147 (S), 1229 (M), 20 (T), 26 (P), 138 (D), 190 (R), 417 (K), 655 (H), 1027 (T), and 1176 (V). In one embodiment, the amino acid corresponding to position 69 (H) in SEQ ID NO: 1 is deleted. In one embodiment, the amino acid corresponding to position 70 (V) in SEQ ID NO: 1 is deleted. In one embodiment, the amino acid corresponding to position 144 (Y) in SEQ ID NO: 1 is deleted. In one embodiment, the amino acid corresponding to position 570 (A) in SEQ ID NO: 1 is D. In one embodiment, the amino acid corresponding to position 681 (P) in SEQ ID NO: 1 is H. In one embodiment, the amino acid corresponding to position 716 (T) in SEQ ID NO: 1 is I. In one embodiment, the amino acid corresponding to position 982 (S) in SEQ ID NO: 1 is A. In one embodiment, the amino acid corresponding to position 1118 (D) in SEQ ID NO: 1 is H. In one embodiment, the amino acid corresponding to position 80 (D) in SEQ ID NO: 1 is A. In one embodiment, the amino acid corresponding to position 215 (D) in SEQ ID NO: 1 is G. In one embodiment, the amino acid corresponding to position 701 (A) in SEQ ID NO: 1 is V. In one embodiment, the amino acid corresponding to position 18 (L) in SEQ ID NO: 1 is F. In one embodiment, the amino acid corresponding to position 246 (R) in SEQ ID NO: 1 is I. In one embodiment, the amino acid corresponding to position 417 (K) in SEQ ID NO: 1 is N. In one embodiment, the amino acid corresponding to position 242 (L) in SEQ ID NO: 1 is deleted. In one embodiment, the amino acid corresponding to position 243 (A) in SEQ ID NO: 1 is deleted. In one embodiment, the amino acid corresponding to position 244 (L) in SEQ ID NO: 1 is deleted. In one embodiment, the amino acid corresponding to position 453 (Y) in SEQ ID NO: 1 is F. In one embodiment, the amino acid corresponding to position 692 (I) in SEQ ID NO: 1 is V. In one embodiment, the amino acid corresponding to position 1147 (S) in SEQ ID NO: 1 is L. In one embodiment, the amino acid corresponding to position 1229 (M) in SEQ ID NO: 1 is I. In one embodiment, the amino acid corresponding to position 20 (T) in SEQ ID NO: 1 is N. In one embodiment, the amino acid corresponding to position 26 (P) in SEQ ID NO: 1 is S. In one embodiment, the amino acid corresponding to position 138 (D) in SEQ ID NO: 1 is Y. In one embodiment, the amino acid corresponding to position 190 (R) in SEQ ID NO: 1 is S. In one embodiment, the amino acid corresponding to position 417 (K) in SEQ ID NO: 1 is T. In one embodiment, the amino acid corresponding to position 655 (H) in SEQ ID NO: 1 is Y. In one embodiment, the amino acid corresponding to position 1027 (T) in SEQ ID NO: 1 is I. In one embodiment, the amino acid corresponding to position 1176 (V) in SEQ ID NO: 1 is F.

In some embodiments, the methods and agents described herein following administration to a subject induce an antibody response, in particular a neutralizing antibody response, in the subject that targets a S protein variant comprising the following mutations at positions corresponding to the following positions in SEQ ID NO: 1: D80A, D215G, E484K, N501Y, A701V, and D614G, and optionally: L18F, R246I, K417N, and deletion 242-244.

In some embodiments, the methods and agents described herein following administration to a subject induce an antibody response, in particular a neutralizing antibody response, in the subject that targets a S protein variant such as SARS-CoV-2 S protein variant, in particular naturally occurring S protein variant comprising a deletion at a position corresponding to positions 242 (L), 243 (A) and 244 (L) in SEQ ID NO: 1.

In some embodiments, a S protein variant comprising a deletion at a position corresponding to positions 242 (L), 243 (A) and 244 (L) in SEQ ID NO: 1 may comprise one or more further mutations. Such one or more further mutations may be selected from mutations at positions corresponding to the following positions in SEQ ID NO: 1: 69 (H), 70 (V), 144 (Y), 501 (N), 570 (A), 614 (D), 681 (P), 716 (T), 982 (S), 1118 (D), 80 (D), 215 (D), 484 (E), 701 (A), 18 (L), 246 (R), 417 (K), 453 (Y), 692 (I), 1147 (S), 1229 (M), 20 (T), 26 (P), 138 (D), 190 (R), 417 (K), 655 (H), 1027 (T), and 1176 (V). In one embodiment, the amino acid corresponding to position 69 (H) in SEQ ID NO: 1 is deleted. In one embodiment, the amino acid corresponding to position 70 (V) in SEQ ID NO: 1 is deleted. In one embodiment, the amino acid corresponding to position 144 (Y) in SEQ ID NO: 1 is deleted. In one embodiment, the amino acid corresponding to position 501 (N) in SEQ ID NO: 1 is Y. In one embodiment, the amino acid corresponding to position 570 (A) in SEQ ID NO: 1 is D. In one embodiment, the amino acid corresponding to position 614 (D) in SEQ ID NO: 1 is G. In one embodiment, the amino acid corresponding to position 681 (P) in SEQ ID NO: 1 is H. In one embodiment, the amino acid corresponding to position 716 (T) in SEQ ID NO: 1 is I. In one embodiment, the amino acid corresponding to position 982 (S) in SEQ ID NO: 1 is A. In one embodiment, the amino acid corresponding to position 1118 (D) in SEQ ID NO: 1 is H. In one embodiment, the amino acid corresponding to position 80 (D) in SEQ ID NO: 1 is A. In one embodiment, the amino acid corresponding to position 215 (D) in SEQ ID NO: 1 is G. In one embodiment, the amino acid corresponding to position 484 (E) in SEQ ID NO: 1 is K. In one embodiment, the amino acid corresponding to position 701 (A) in SEQ ID NO: 1 is V. In one embodiment, the amino acid corresponding to position 18 (L) in SEQ ID NO: 1 is F. In one embodiment, the amino acid corresponding to position 246 (R) in SEQ ID NO: 1 is I. In one embodiment, the amino acid corresponding to position 417 (K) in SEQ ID NO: 1 is N. In one embodiment, the amino acid corresponding to position 453 (Y) in SEQ ID NO: 1 is F. In one embodiment, the amino acid corresponding to position 692 (I) in SEQ ID NO: 1 is V. In one embodiment, the amino acid corresponding to position 1147 (S) in SEQ ID NO: 1 is L. In one embodiment, the amino acid corresponding to position 1229 (M) in SEQ ID NO: 1 is I. In one embodiment, the amino acid corresponding to position 20 (T) in SEQ ID NO: 1 is N. In one embodiment, the amino acid corresponding to position 26 (P) in SEQ ID NO: 1 is S. In one embodiment, the amino acid corresponding to position 138 (D) in SEQ ID NO: 1 is Y. In one embodiment, the amino acid corresponding to position 190 (R) in SEQ ID NO: 1 is S. In one embodiment, the amino acid corresponding to position 417 (K) in SEQ ID NO: 1 is T. In one embodiment, the amino acid corresponding to position 655 (H) in SEQ ID NO: 1 is Y. In one embodiment, the amino acid corresponding to position 1027 (T) in SEQ ID NO: 1 is I. In one embodiment, the amino acid corresponding to position 1176 (V) in SEQ ID NO: 1 is F.

In some embodiments, the methods and agents described herein following administration to a subject induce an antibody response, in particular a neutralizing antibody response, in the subject that targets 501.V2.

In some embodiments, the methods and agents described herein following administration to a subject induce an antibody response, in particular a neutralizing antibody response, in the subject that targets a S protein variant comprising the following mutations at positions corresponding to the following positions in SEQ ID NO: 1: D80A, D215G, E484K, N501Y, A701V and deletion 242-244, and optionally: L18F, R246I, and K417N. Said S protein variant may also comprise a D->G mutation at a position corresponding to position 614 in SEQ ID NO: 1.

In some embodiments, the methods and agents described herein following administration to a subject induce an antibody response, in particular a neutralizing antibody response, in the subject that targets a S protein variant such as SARS-CoV-2 S protein variant, in particular naturally occurring S protein variant comprising a mutation at a position corresponding to position 417 (K) in SEQ ID NO: 1. In one embodiment, the amino acid corresponding to position 417 (K) in SEQ ID NO: 1 is N. In one embodiment, the amino acid corresponding to position 417 (K) in SEQ ID NO: 1 is T.

In some embodiments, a S protein variant comprising a mutation at a position corresponding to position 417 (K) in SEQ ID NO: 1 may comprise one or more further mutations. Such one or more further mutations may be selected from mutations at positions corresponding to the following positions in SEQ ID NO: 1: 69 (H), 70 (V), 144 (Y), 501 (N), 570 (A), 614 (D), 681 (P), 716 (T), 982 (S), 1118 (D), 80 (D), 215 (D), 484 (E), 701 (A), 18 (L), 246 (R), 242 (L), 243 (A), 244 (L), 453 (Y), 692 (I), 1147 (S), 1229 (M), 20 (T), 26 (P), 138 (D), 190 (R), 655 (H), 1027 (T), and 1176 (V). In one embodiment, the amino acid corresponding to position 69 (H) in SEQ ID NO: 1 is deleted. In one embodiment, the amino acid corresponding to position 70 (V) in SEQ ID NO: 1 is deleted. In one embodiment, the amino acid corresponding to position 144 (Y) in SEQ ID NO: 1 is deleted. In one embodiment, the amino acid corresponding to position 501 (N) in SEQ ID NO: 1 is Y. In one embodiment, the amino acid corresponding to position 570 (A) in SEQ ID NO: 1 is D. In one embodiment, the amino acid corresponding to position 614 (D) in SEQ ID NO: 1 is G. In one embodiment, the amino acid corresponding to position 681 (P) in SEQ ID NO: 1 is H. In one embodiment, the amino acid corresponding to position 716 (T) in SEQ ID NO: 1 is I. In one embodiment, the amino acid corresponding to position 982 (S) in SEQ ID NO: 1 is A. In one embodiment, the amino acid corresponding to position 1118 (D) in SEQ ID NO: 1 is H. In one embodiment, the amino acid corresponding to position 80 (D) in SEQ ID NO: 1 is A. In one embodiment, the amino acid corresponding to position 215 (D) in SEQ ID NO: 1 is G. In one embodiment, the amino acid corresponding to position 484 (E) in SEQ ID NO: 1 is K. In one embodiment, the amino acid corresponding to position 701 (A) in SEQ ID NO: 1 is V. In one embodiment, the amino acid corresponding to position 18 (L) in SEQ ID NO: 1 is F. In one embodiment, the amino acid corresponding to position 246 (R) in SEQ ID NO: 1 is I. In one embodiment, the amino acid corresponding to position 242 (L) in SEQ ID NO: 1 is deleted. In one embodiment, the amino acid corresponding to position 243 (A) in SEQ ID NO: 1 is deleted. In one embodiment, the amino acid corresponding to position 244 (L) in SEQ ID NO: 1 is deleted. In one embodiment, the amino acid corresponding to position 453 (Y) in SEQ ID NO: 1 is F. In one embodiment, the amino acid corresponding to position 692 (I) in SEQ ID NO: 1 is V. In one embodiment, the amino acid corresponding to position 1147 (S) in SEQ ID NO: 1 is L. In one embodiment, the amino acid corresponding to position 1229 (M) in SEQ ID NO: 1 is I. In one embodiment, the amino acid corresponding to position 20 (T) in SEQ ID NO: 1 is N. In one embodiment, the amino acid corresponding to position 26 (P) in SEQ ID NO: 1 is S. In one embodiment, the amino acid corresponding to position 138 (D) in SEQ ID NO: 1 is Y. In one embodiment, the amino acid corresponding to position 190 (R) in SEQ ID NO: 1 is S. In one embodiment, the amino acid corresponding to position 655 (H) in SEQ ID NO: 1 is Y. In one embodiment, the amino acid corresponding to position 1027 (T) in SEQ ID NO: 1 is I. In one embodiment, the amino acid corresponding to position 1176 (V) in SEQ ID NO: 1 is F.

In some embodiments, the methods and agents described herein following administration to a subject induce an antibody response, in particular a neutralizing antibody response, in the subject that targets 501.V2.

In some embodiments, the methods and agents described herein following administration to a subject induce an antibody response, in particular a neutralizing antibody response, in the subject that targets a S protein variant comprising the following mutations at positions corresponding to the following positions in SEQ ID NO: 1: D80A, D215G, E484K, N501Y, A701V, and K417N, and optionally: L18F, R246I, and deletion 242-244. Said S protein variant may also comprise a D->G mutation at a position corresponding to position 614 in SEQ ID NO: 1.

In some embodiments, the methods and agents described herein following administration to a subject induce an antibody response, in particular a neutralizing antibody response, in the subject that targets "B.1.1.248".

In some embodiments, the methods and agents described herein following administration to a subject induce an antibody response, in particular a neutralizing antibody response, in the subject that targets a S protein variant comprising the following mutations at positions corresponding to the following positions in SEQ ID NO: 1: L18F, T20N, P26S, D138Y, R190S, K417T, E484K, N501Y, H655Y, T1027I, and V1176F.

In some embodiments, the methods and agents described herein following administration to a subject induce an antibody response, in particular a neutralizing antibody response, in the subject that targets a S protein variant such as SARS-CoV-2 S protein variant, in particular naturally occurring S protein variant comprising a mutation at positions corresponding to positions 417 (K) and 484 (E) and/or 501 (N) in SEQ ID NO: 1. In one embodiment, the amino acid corresponding to position 417 (K) in SEQ ID NO: 1 is N, and the amino acid corresponding to position 484 (E) in SEQ ID NO: 1 is K and/or the amino acid corresponding to position 501 (N) in SEQ ID NO: 1 is Y. In one embodiment, the amino acid corresponding to position 417 (K) in SEQ ID NO: 1 is T, and the amino acid corresponding to position 484 (E) in SEQ ID NO: 1 is K and/or the amino acid corresponding to position 501 (N) in SEQ ID NO: 1 is Y.

In some embodiments, a S protein variant comprising a mutation at positions corresponding to positions 417 (K) and 484 (E) and/or 501 (N) in SEQ ID NO: 1 may comprise one or more further mutations. Such one or more further mutations may be selected from mutations at positions corresponding to the following positions in SEQ ID NO: 1: 69 (H), 70 (V), 144 (Y), 570 (A), 614 (D), 681 (P), 716 (T), 982 (S), 1118 (D), 80 (D), 215 (D), 701 (A), 18 (L), 246 (R), 242 (L), 243 (A), 244 (L), 453 (Y), 692 (I), 1147 (S), 1229 (M), 20 (T), 26 (P), 138 (D), 190 (R), 655 (H), 1027 (T), and 1176 (V). In one embodiment, the amino acid corresponding to position 69 (H) in SEQ ID NO: 1 is deleted. In one embodiment, the amino acid corresponding to position 70 (V) in SEQ ID NO: 1 is deleted. In one embodiment, the amino acid corresponding to position 144 (Y) in SEQ ID NO: 1 is deleted. In one embodiment, the amino acid corresponding to position 570 (A) in SEQ ID NO: 1 is D. In one embodiment, the amino acid corresponding to position 614 (D) in SEQ ID NO: 1 is G. In one embodiment, the amino acid corresponding to position 681 (P) in SEQ ID NO: 1 is H. In one embodiment, the amino acid corresponding to position 716 (T) in SEQ ID NO: 1 is I. In one embodiment, the amino acid corresponding to position 982 (S) in SEQ ID NO: 1 is A. In one embodiment, the amino acid corresponding to position 1118 (D) in SEQ ID NO: 1 is H. In one embodiment, the amino acid corresponding to position 80 (D) in SEQ ID NO: 1 is A. In one embodiment, the amino acid corresponding to position 215 (D) in SEQ ID NO: 1 is G. In one embodiment, the amino acid corresponding to position 701 (A) in SEQ ID NO: 1 is V. In one embodiment, the amino acid corresponding to position 18 (L) in SEQ ID NO: 1 is F. In one embodiment, the amino acid corresponding to position 246 (R) in SEQ ID NO: 1 is I. In one embodiment, the amino acid corresponding to position 242 (L) in SEQ ID NO: 1 is deleted. In one embodiment, the amino acid corresponding to position 243 (A) in SEQ ID NO: 1 is deleted. In one embodiment, the amino acid corresponding to position 244 (L) in SEQ ID NO: 1 is deleted. In one embodiment, the amino acid corresponding to position 453 (Y) in SEQ ID NO: 1 is F. In one embodiment, the amino acid corresponding to position 692 (I) in SEQ ID NO: 1 is V. In one embodiment, the amino acid corresponding to position 1147 (S) in SEQ ID NO: 1 is L. In one embodiment, the amino acid corresponding to position 1229 (M) in SEQ ID NO: 1 is I. In one embodiment, the amino acid corresponding to position 20 (T) in SEQ ID NO: 1 is N. In one embodiment, the amino acid corresponding to position 26 (P) in SEQ ID NO: 1 is S. In one embodiment, the amino acid corresponding to position 138 (D) in SEQ ID NO: 1 is Y. In one embodiment, the amino acid corresponding to position 190 (R) in SEQ ID NO: 1 is S. In one embodiment, the amino acid corresponding to position 655 (H) in SEQ ID NO: 1 is Y. In one embodiment, the amino acid corresponding to position 1027 (T) in SEQ ID NO: 1 is I. In one embodiment, the amino acid corresponding to position 1176 (V) in SEQ ID NO: 1 is F.

In some embodiments, the methods and agents described herein following administration to a subject induce an antibody response, in particular a neutralizing antibody response, in the subject that targets 501.V2.

In some embodiments, the methods and agents described herein following administration to a subject induce an antibody response, in particular a neutralizing antibody response, in the subject that targets a S protein variant comprising the following mutations at positions corresponding to the following positions in SEQ ID NO: 1: D80A, D215G, E484K, N501Y, A701V, and K417N and optionally: L18F, R246I, and deletion 242-244. Said S protein variant may also comprise a D->G mutation at a position corresponding to position 614 in SEQ ID NO: 1.

In some embodiments, the methods and agents described herein following administration to a subject induce an antibody response, in particular a neutralizing antibody response, in the subject that targets "B.1.1.248".

In some embodiments, the methods and agents described herein following administration to a subject induce an antibody response, in particular a neutralizing antibody response, in the subject that targets a S protein variant comprising the following mutations at positions corresponding to the following positions in SEQ ID NO: 1: L18F, T20N, P26S, D138Y, R190S, K417T, E484K, N501Y, H655Y, T1027I, and V1176F.

In some embodiments, the methods and agents described herein following administration to a subject induce an antibody response, in particular a neutralizing antibody response, in the subject that targets the Omicron (B.1.1.529) variant.

In some embodiments, the methods and agents described herein following administration to a subject induce an antibody response, in particular a neutralizing antibody response, in the subject that targets a S protein variant comprising at least 10, at least 15, at least 20, at least 21, at least 22, at least 23, at least 24, at least 25, at least 26, at least 27, at least 28, at least 29, at least 30, at least 31, at least 32, at least 33, at least 34, at least 35, at least 36, or at least 37 of the following mutations: T547K, H655Y, D614G, N679K, P681H, N969K, S373P, S371L, N440K, G339D, G446S, N856K, N764K, K417N, D796Y, Q954H, T95I, A67V, L981F, S477N, G496S, T478K, Q498R, Q493R, E484A, N501Y, S375F, Y505H, V143del, H69del, V70del, N211del, L212I, ins214EPE, G142D, Y144del, Y145del, L141del, Y144F, Y145D, G142del, as compared to SEQ ID NO: 1.

In some embodiments, the methods and agents described herein following administration to a subject induce an antibody response, in particular a neutralizing antibody response, in the subject that targets a S protein variant comprising at least 10, at least 15, at least 20, at least 21, at least 22, at least 23, at least 24, or all of the following mutations: T547K, H655Y, D614G, N679K, P681H, N969K, S373P, S371L, N440K, G339D, G446S, N856K, N764K, K417N, D796Y, Q954H, T95I, A67V, L981F, S477N, G496S, T478K, Q498R, Q493R, E484A, as compared to SEQ ID NO: 1. Said S protein variant may include at least 1, at least 2, at least 3, at least 4, at least 5, or all of the following mutations: N501Y, S375F, Y505H, V143del, H69del, V70del, as compared to SEQ ID NO: 1 and/or may include at least 1, at least 2, at least 3, at least 4, at least 5, or all of the following mutations: N211del, L212I, ins214EPE, G142D, Y144del, Y145del, as compared to SEQ ID NO: 1. In some embodiments, said S protein variant may include at least 1, at least 2, at least 3, or all of the following mutations: L141del, Y144F, Y145D, G142del, as compared to SEQ ID NO: 1.

In some embodiments, the methods and agents described herein following administration to a subject induce an antibody response, in particular a neutralizing antibody response, in the subject that targets a S protein variant comprising at least 10, at least 15, at least 20, at least 21, at least 22, at least 23, at least 24, at least 25, at least 26, at least 27, at least 28, at least 29, at least 30, at least 31, at least 32, or at least 33 of the following mutations:
A67V, Δ69-70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, and L981F, as compared to SEQ ID NO: 1.

In some embodiments, the methods and agents described herein following administration to a subject induce an antibody response, in particular a neutralizing antibody response, in the subject that targets a S protein variant comprising the following mutations:
A67V, Δ69-70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, and L981F, as compared to SEQ ID NO: 1.

In some embodiments, the methods and agents described herein following administration to a subject induce an antibody response, in particular a neutralizing antibody response, in the subject that targets a S protein variant comprising the following mutations:
A67V, Δ69-70, T95I, G142D, Δ143-145, Δ211, L212I, ins214EPE, G339D, S371L, S373P, S375F, S477N, T478K, E484A, Q493R, G496S, Q498R, N501Y, Y505H, T547K, D614G, H655Y, N679K, P681H, N764K, D796Y, N856K, Q954H, N969K, and L981F, as compared to SEQ ID NO: 1.

In some embodiments, the methods and agents described herein following administration to a subject induce an immune response (cellular and/or antibody response, in particular neutralizing antibody response) in the subject that targets the Omicron (B.1.1.529) variant. In some embodiments, the methods and agents described herein following administration to a subject induce an immune response (cellular and/or antibody response, in particular neutralizing antibody response) in the subject that targets a S protein variant comprising at least 10, at least 15, at least 20, at least 21, at least 22, at least 23, at least 24, at least 25, at least 26, at least 27, at least 28, at least 29, at least 30, at least 31, at least 32, at least 33, at least 34, at least 35, at least 36, or at least 37 of the following mutations: T547K, H655Y, D614G, N679K, P681H, N969K, S373P, S371L, N440K, G339D, G446S, N856K, N764K, K417N, D796Y, Q954H, T95I, A67V, L981F, S477N, G496S, T478K, Q498R, Q493R, E484A, N501Y, S375F, Y505H, V143del, H69del, V70del, N211del, L212I, ins214EPE, G142D, Y144del, Y145del, L141del, Y144F, Y145D, G142del, as compared to SEQ ID NO: 1.

In some embodiments, the methods and agents described herein following administration to a subject induce an immune response (cellular and/or antibody response, in particular neutralizing antibody response) in the subject that targets a S protein variant comprising at least 10, at least 15, at least 20, at least 21, at least 22, at least 23, at least 24, or all of the following mutations: T547K, H655Y, D614G, N679K, P681H, N969K, S373P, S371L, N440K, G339D, G446S, N856K, N764K, K417N, D796Y, Q954H, T95I, A67V, L981F, S477N, G496S, T478K, Q498R, Q493R, E484A, as compared to SEQ ID NO: 1. Said S protein variant may include at least 1, at least 2, at least 3, at least 4, at least 5, or all of the following mutations: N501Y, S375F, Y505H, V143del, H69del, V70del, as compared to SEQ ID NO: 1 and/or may include at least 1, at least 2, at least 3, at least 4, at least 5, or all of the following mutations: N211del, L212I, ins214EPE, G142D, Y144del, Y145del, as compared to SEQ ID NO: 1. In some embodiments, said S protein variant may include at least 1, at least 2, at least 3, or all of the following mutations: L141del, Y144F, Y145D, G142del, as compared to SEQ ID NO: 1.

In some embodiments, the methods and agents described herein following administration to a subject induce an immune response (cellular and/or antibody response, in particular neutralizing antibody response) in the subject that targets a S protein variant comprising at least 10, at least 15, at least 20, at least 21, at least 22, at least 23, at least 24, at least 25, at least 26, at least 27, at least 28, at least 29, at least 30, at least 31, at least 32, or at least 33 of the following mutations:
A67V, Δ69-70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, and L981F, as compared to SEQ ID NO: 1.

In some embodiments, the methods and agents described herein following administration to a subject induce an immune response (cellular and/or antibody response, in particular neutralizing antibody response) in the subject that targets a S protein variant comprising the following mutations:
A67V, Δ69-70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, and L981F, as compared to SEQ ID NO: 1.

In some embodiments, the methods and agents described herein following administration to a subject induce an immune response (cellular and/or antibody response, in particular neutralizing antibody response) in the subject that targets a S protein variant comprising the following mutations:
A67V, Δ69-70, T95I, G142D, Δ143-145, Δ211, L212I, ins214EPE, G339D, S371L, S373P, S375F, S477N, T478K, E484A, Q493R, G496S, Q498R, N501Y, Y505H, T547K, D614G, H655Y, N679K, P681H, N764K, D796Y, N856K, Q954H, N969K, and L981F, as compared to SEQ ID NO: 1.

The term "amino acid corresponding to position..." as used herein refers to an amino acid position number corresponding to an amino acid position number in SARS-CoV-2 S protein, in particular the amino acid sequence shown in SEQ ID NO: 1. The phrase "as compared to SEQ ID NO: 1" is equivalent to "at positions corresponding to the following positions in SEQ ID NO: 1". Corresponding amino acid positions in other coronavirus S protein variants such as SARS-CoV-2 S protein variants may be found by alignment with SARS-CoV-2 S protein, in particular the amino acid sequence shown in SEQ ID NO: 1. It is considered well-known in the art how to align a sequence or segment in a sequence and thereby determine the corresponding position in a sequence to an amino acid position according to the present disclosure. Standard sequence alignment programs such as ALIGN, ClustalW or similar, typically at default settings may be used.

In some embodiments, the panel of different S protein variants to which an antibody response is targeted comprises at least 5, at least 10, at least 15, or even more S protein variants selected from the group consisting of the Q321S, V341I, A348T, N354D, S359N, V367F, K378S, R408I, Q409E, A435S, K458R, 1472V, G476S, V483A, Y508H, H519P and D614G variants described above. In some embodiments, the panel of different S protein variants to which an antibody response is targeted comprises all S protein variants from the group consisting of the Q321S, V341I, A348T, N354D, S359N, V367F, K378S, R408I, Q409E, A435S, K458R, 1472V, G476S, V483A, Y508H, H519P and D614G variants described above.

In some embodiments, the panel of different S protein variants to which an antibody response is targeted comprises at least 5, at least 10, at least 15, or even more S protein variants selected from the group consisting of the Q321L, V341I, A348T, N354D, S359N, V367F, K378R, R408I, Q409E, A435S, N439K, K458R, 1472V, G476S, S477N, V483A, Y508H, H519P and D614G variants described above. In some embodiments, the panel of different S protein variants to which an antibody response is targeted comprises all S protein variants from the group consisting of the Q321L, V341I, A348T, N354D, S359N, V367F, K378R, R408I, Q409E, A435S, N439K, K458R, 1472V, G476S, S477N, V483A, Y508H, H519P and D614G variants described above.

In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprises one or more of the mutations described herein for S protein variants such as SARS-CoV-2 S protein variants, in particular naturally occurring S protein variants. In one embodiment, a SARS-CoV-2 S protein, an immunogenic variant thereof, or an immunogenic fragment of the SARS-CoV-2 S protein or the immunogenic variant thereof, e.g., as encoded by the RNA described herein, comprises a mutation at a position corresponding to position 501 (N) in SEQ ID NO: 1. In one embodiment, the amino acid corresponding to position 501 (N) in SEQ ID NO: 1 is Y. In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprises one or more mutations, such as all mutations, of a SARS-CoV-2 S protein of a SARS-CoV-2 variant selected from the group consisting of VOC-202012/01, 501.V2, Cluster 5 and B.1.1.248. In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprises an amino acid sequence with alanine substitution at position 80, glycine substitution at position 215, lysine substitution at position 484, tyrosine substitution at position 501, valine substitution at position 701, phenylalanine substitution at position 18, isoleucine substitution at position 246, asparagine substitution at position 417, glycine substitution at position 614, deletions at positions 242 to 244, and proline substitutions at positions 986 and 987 of SEQ ID NO:1.

In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprises at least 10, at least 15, at least 20, at least 21, at least 22, at least 23, at least 24, at least 25, at least 26, at least 27, at least 28, at least 29, at least 30, at least 31, at least 32, at least 33, at least 34, at least 35, at least 36, or at least 37 of the following mutations: T547K, H655Y, D614G, N679K, P681H, N969K, S373P, S371L, N440K, G339D, G446S, N856K, N764K, K417N, D796Y, Q954H, T95I, A67V, L981F, S477N, G496S, T478K, Q498R, Q493R, E484A, N501Y, S375F, Y505H, V143del, H69del, V70del, N211del, L212I, ins214EPE, G142D, Y144del, Y145del, L141del, Y144F, Y145D, G142del, as compared to SEQ ID NO: 1. In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprising said mutations comprises K986P and V987P, as compared to SEQ ID NO: 1.

In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprises at least 10, at least 15, at least 20, at least 21, at least 22, at least 23, at least 24, or all of the following mutations: T547K, H655Y, D614G, N679K, P681H, N969K, S373P, S371L, N440K, G339D, G446S, N856K, N764K, K417N, D796Y, Q954H, T95I, A67V, L981F, S477N, G496S, T478K, Q498R, Q493R, E484A, as compared to SEQ ID NO: 1. Said SARs-CoV-2 S protein, variant, or fragment may include at least 1, at least 2, at least 3, at least 4, at least 5, or all of the following mutations: N501Y, S375F, Y505H, V143del, H69del, V70del, as compared to SEQ ID NO: 1 and/or may include at least 1, at least 2, at least 3, at least 4, at least 5, or all of the following mutations: N211del, L212I, ins214EPE, G142D, Y144del, Y145del, as compared to SEQ ID NO: 1. In some embodiments, said SARs-CoV-2 S protein, variant, or fragment may include at least 1, at least 2, at least 3, or all of the following mutations: L141del, Y144F, Y145D, G142del, as compared to SEQ ID NO: 1. In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprising said mutations comprises K986P and V987P, as compared to SEQ ID NO: 1.

In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprises at least 10, at least 15, at least 20, at least 21, at least 22, at least 23, at least 24, at least 25, at least 26, at least 27, at least 28, at least 29, at least 30, at least 31, at least 32, or at least 33 of the following mutations:
A67V, Δ69-70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, and L981F, as compared to SEQ ID NO: 1. In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprising said mutations comprises K986P and V987P, as compared to SEQ ID NO: 1.

In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprises the following mutations:
A67V, Δ69-70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, and L981F, as compared to SEQ ID NO: 1.

In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprising said mutations comprises K986P and V987P, as compared to SEQ ID NO: 1.

In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprises the following mutations:
A67V, Δ69-70, T95I, G142D, Δ143-145, Δ211, L212I, ins214EPE, G339D, S371L, S373P, S375F, S477N, T478K, E484A, Q493R, G496S, Q498R, N501Y, Y505H, T547K, D614G, H655Y, N679K, P681H, N764K, D796Y, N856K, Q954H, N969K, and L981F, as compared to SEQ ID NO: 1.

In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprising said mutations comprises K986P and V987P, as compared to SEQ ID NO: 1.

In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprises the following mutations:
A67V, Δ69-70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F, K986P and V987P, as compared to SEQ ID NO: 1.

In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprises the following mutations:
A67V, Δ69-70, T95I, G142D, Δ143-145, Δ211, L212I, ins214EPE, G339D, S371L, S373P, S375F, S477N, T478K, E484A, Q493R, G496S, Q498R, N501Y, Y505H, T547K, D614G, H655Y, N679K, P681H, N764K, D796Y, N856K, Q954H, N969K, L981F, K986P and V987P, as compared to SEQ ID NO: 1.

In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprises the following mutations:
In some embodiments, the spike changes in Omicron BA.2 variant include T19I, Δ24-26, A27S, G142D, V213G, G339D, S371F, S373P, S375F, T376A, D405N, R408S, K417N, N440K, S477N, T478K, E484A, Q493R, Q498R, N501Y, Y505H, D614G, H655Y, N679K, P681H, N764K, D796Y, Q954H, N969K, K986P, and V987P, as compared to SEQ ID NO: 1.

In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprises the following mutations:
T19I, Δ24-26, A27S, Δ69/70, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, N969K, K986P, and V987P, as compared to SEQ ID NO: 1. In some embodiments, administration of a variant specific vaccine (e.g., a variant specific vaccine disclosed herein) may result in an improved immune response in a patient as compared to administration of vaccine encoding or comprising a SARS-CoV-2 S protein from a Wuhan strain, or an immunogenic fragment thereof. In some embodiments, administration of a variant-specific vaccine may result in induction of a broader immune response in a subject as compared to a patient administered a vaccine comprising or encoding a SARS-CoV-2 S protein from a Wuhan strain (or an immunogenic fragment thereof) (e.g., induce a stronger neutralization response against a greater number of SARS-CoV-2 variants and/or a neutralization response that recognizes epitopes in a greater number of SARS-CoV-2 variants). In particular embodiments, a broader immune response may be induced when a variant specific vaccine is administered in combination with a vaccine comprising or encoding a SARS-CoV-2 S protein from a different variant or from a Wuhan strain (e.g., in some embodiments, a broader immune response may be induced when a variant specific vaccine is administered in combination with a vaccine comprising or encoding a SARS-CoV-2 S protein from a Wuhan strain or a vaccine comprising or encoding a SARS-CoV-2 S protein comprising mutations characteristic of a different SARS-CoV-2 variant). For example, a broader immune response may be induced when an RNA vaccine encoding a SARS-CoV-2 S protein from a Wuhan strain is administered in combination with an RNA vaccine encoding a SARS-CoV-2 S protein having mutations characteristic of an Omicron variant. In another embodiment, a broader immune response may be induced when an RNA vaccine encoding a SARS-CoV-2 S protein comprising one or more mutations characteristic of a delta variant is administered in combination with an RNA vaccine encoding a SARS-CoV-2 S protein comprising one or more mutations characteristic of an Omicron variant. In such embodiments, a "broader" immune response may be defined relative to a patient administered a vaccine comprising or encoding a SARS-CoV-2 S protein from a single variant (e.g., an RNA vaccine encoding a SARS-CoV-2 S protein from a Wuhan strain). Vaccines comprising or encoding S proteins from different SARS-CoV-2 variants, or immunogenic fragments thereof, may be administered in combination by administering at different time points (e.g., administering a vaccine encoding a SARS-CoV-2 S protein from a Wuhan strain and a vaccine encoding a SARS-CoV-2 S protein having one or more mutations characteristic of a variant strain at different time points, e.g., both administered as part of a primary regimen or part of a booster regimen; or one is administered as part of a primary regimen while another is administered as part of a booster regimen). In some embodiments, vaccines comprising or encoding S proteins from different SARS-CoV-2 variants, or immunogenic fragments thereof, may be administered in combination by administering a multivalent vaccine (e.g., a composition comprising RNA encoding a SARS-CoV-2 S protein from a Wuhan strain and RNA encoding a SARS-CoV-2 S protein having mutations characteristic of an Omicron variant). In some embodiments, a variant specific vaccine may induce a superior immune response (e.g., inducing higher concentrations of neutralizing antibodies) against a variant against which the vaccine is specifically designed to immunize, and an immune response against one or more other variants. In some such embodiments, an immune response against other variant(s) may be comparable to or higher than that as observed with a vaccine that encodes or comprises a SARS-CoV-2 S protein from a Wuhan strain.

In some embodiments, the geometric mean ratio (GMR) or geometric mean fold rise (GMFR) of neutralization antibodies induced by a variant specific vaccine is at least 1.1, 1.2, 1.3, 1.4, 1.5,1.6,1.7,1.8,1.9, or 2.0 (e.g., 1.1 to 4,1.1 to 3.5,1.1 to 3, 1.5 to 3, or 1.1 to 1.5) fold higher than that induced by a non-variant specific vaccine (e.g., as measured 1 day to 3 months after immunization, 7 days to 2 months after administration, about 7 days, or about 1 month after administration).

In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprising said mutations comprises the amino acid sequence of SEQ ID NO: 49, an amino acid sequence having at least 99.5%, 99%, 98.5%, 98%, 98.5%, 97%, 96%, 95%, 90%, 85%, or 80% identity to the amino acid sequence of SEQ ID NO: 49, or an immunogenic fragment of the amino acid sequence of SEQ ID NO: 49, or the amino acid sequence having at least 99.5%, 99%, 98.5%, 98%, 98.5%, 97%, 96%, 95%, 90%, 85%, or 80% identity to the amino acid sequence of SEQ ID NO: 49. In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprising said mutations comprises the amino acid sequence of SEQ ID NO: 49.

In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprising said mutations comprises the amino acid sequence of SEQ ID NO: 52, an amino acid sequence having at least 99.5%, 99%, 98.5%, 98%, 98.5%, 97%, 96%, 95%, 90%, 85%, or 80% identity to the amino acid sequence of SEQ ID NO: 52, or an immunogenic fragment of the amino acid sequence of SEQ ID NO: 52, or the amino acid sequence having at least 99.5%, 99%, 98.5%, 98%, 98.5%, 97%, 96%, 95%, 90%, 85%, or 80% identity to the amino acid sequence of SEQ ID NO: 52. In some embodiments, a SARS-CoV-2 S protein, an immunogenic variant thereof, or an immunogenic fragment of the SARS-CoV-2 S protein or the immunogenic variant thereof, e.g., as encoded by the RNA described herein, comprising said mutations comprises the amino acid sequence of SEQ ID NO: 52.

In some embodiments, the methods and agents, e.g., RNA (e.g., mRNA) compositions, described herein following administration to a subject induce a cell-mediated immune response (e.g., CD4+ and/or CD8+ T cell response). In some embodiments, T cells are induced that recognize one or more epitopes (e.g., MHC class I-restricted epitopes) selected from the group consisting of LPFNDGVYF, GVYFASTEK, YLQPRTFLL, QPTESIVRF, CVADYSVLY, KCYGVSPTK, NYNYLYRLF, FQPTNGVGY, IPFAMQMAY, RLQSLQTYV, GTHWFVTQR, VYDPLQPEL, QYIKWPWYI, and KWPWYIWLGF. In one embodiment, T cells are induced that recognize the epitope YLQPRTFLL. In one embodiment, T cells are induced that recognize the epitope NYNYLYRLF. In one embodiment, T cells are induced that recognize the epitope QYIKWPWYI. In one embodiment, T cells are induced that recognize the epitope KCYGVSPTK. In one embodiment, T cells are induced that recognize the epitope RLQSLQTYV. In some embodiments, the methods and agents, e.g., RNA (e.g., mRNA) compositions, described herein are administered according to a regimen which achieves such induction of T cells.

In some embodiments, the methods and agents, e.g., RNA (e.g., mRNA) compositions, described herein following administration to a subject induce a cell-mediated immune response (e.g., CD4+ and/or CD8+ T cell response) that is detectable 15 weeks or later, 16 weeks or later, 17 weeks or later, 18 weeks or later, 19 weeks or later, 20 weeks or later, 21 weeks or later, 22 weeks or later, 23 weeks or later, 24 weeks or later or 25 weeks or later after administration, e.g., using two doses of the RNA described herein (wherein the second dose may be administered about 21 days following administration of the first dose). In some embodiments, the methods and agents, e.g., RNA (e.g., mRNA) compositions, described herein are administered according to a regimen which achieves such induction of a cell-mediated immune response.

In one embodiment, vaccination against Coronavirus described herein, e.g., using RNA described herein which may be administered in the amounts and regimens described herein, e.g., at two doses of 30 µg per dose e.g. administered 21 days apart, may be repeated after a certain period of time, e.g., once it is observed that protection against Coronavirus infection diminishes, using the same or a different vaccine as used for the first vaccination. Such certain period of time may be at least 6 months, 1 year, two years etc. In one embodiment, the same RNA as used for the first vaccination is used for the second or further vaccination, however, at a lower dose or a lower frequency of administration. For example, the first vaccination may comprise vaccination using a dose of about 30 µg per dose, wherein in one embodiment, at least two of such doses are administered, (for example, a second dose may be administered about 21 days following administration of the first dose) and the second or further vaccination may comprise vaccination using a dose of less than about 30 µg per dose, wherein in one embodiment, only one of such doses is administered. In one embodiment, a different RNA as used for the first vaccination is used for the second or further vaccination, e.g., BNT162b2 is used for the first vaccination and BNT162B1 or BNT162b3 is used for the second or further vaccination.

In one embodiment, the vaccination regimen comprises a first vaccination using at least two doses of the RNA described herein, e.g., two doses of the RNA described herein (wherein the second dose may be administered about 21 days following administration of the first dose), and a second vaccination using a single dose or multiple doses, e.g., two doses, of the RNA described herein. In various embodiments, the second vaccination is administered 3 to 24 months, 6 to 18 months, 6 to 12 months, or 5 to 7 months after administration of the first vaccination, e.g., after the initial two-dose regimen. The amount of RNA used in each dose of the second vaccination may be equal or different to the amount of RNA used in each dose of the first vaccination. In one embodiment, the amount of RNA used in each dose of the second vaccination is equal to the amount of RNA used in each dose of the first vaccination. In one embodiment, the amount of RNA used in each dose of the second vaccination and the amount of RNA used in each dose of the first vaccination is about 30 µg per dose. In one embodiment, the same RNA as used for the first vaccination is used for the second vaccination.

In one embodiment, the RNA used for the first vaccination and for the second vaccination is BNT162b2.

In some embodiments, when the RNA used for the first vaccination and for the second vaccination is BNT162b2, the aim is to induce an immune response that targets SARS-CoV-2 variants including, but not limited to, the Omicron (B.1.1.529) variant. Accordingly, in some embodiments, when the RNA used for the first vaccination and for the second vaccination is BNT162b2, the aim is to protect a subject from infection with SARS-CoV-2 variants including, but not limited to, the Omicron (B.1.1.529) variant.

In one embodiment, a different RNA as used for the first vaccination is used for the second vaccination. In one embodiment, the RNA used for the first vaccination is BNT162b2 and the RNA used for the second vaccination is RNA encoding a SARS-CoV-2 S protein of a SARS-CoV-2 variant strain, e.g., a strain discussed herein. In one embodiment, the RNA used for the first vaccination is BNT162b2 and the RNA used for the second vaccination is RNA encoding a SARS-CoV-2 S protein of a SARS-CoV-2 variant strain that is prevalent or rapidly spreading at the time of the second vaccination. In one embodiment, the RNA used for the first vaccination is BNT162b2 and the RNA used for the second vaccination is RNA encoding a SARS-CoV-2 S protein, an immunogenic variant thereof, or an immunogenic fragment of the SARS-CoV-2 S protein or the immunogenic variant thereof comprising one or more of the mutations described herein for S protein variants such as SARS-CoV-2 S protein variants, in particular naturally occurring S protein variants. In one embodiment, the RNA used for the first vaccination is BNT162b2 and the RNA used for the second vaccination is RNA encoding a SARS-CoV-2 S protein, an immunogenic variant thereof, or an immunogenic fragment of the SARS-CoV-2 S protein or the immunogenic variant thereof comprising one or more mutations, such as all mutations, of a SARS-CoV-2 S protein of a SARS-CoV-2 variant selected from the group consisting of VOC-202012/01, 501.V2, Cluster 5, B.1.1.248, and Omicron (B.1.1.529).

In one embodiment, the RNA used for the first vaccination encodes a polypeptide comprising an amino acid sequence with proline residue substitutions at positions 986 and 987 of SEQ ID NO:1 and the RNA used for the second vaccination is RNA encoding a polypeptide comprising an amino acid sequence with alanine substitution at position 80, glycine substitution at position 215, lysine substitution at position 484, tyrosine substitution at position 501, valine substitution at position 701, phenylalanine substitution at position 18, isoleucine substitution at position 246, asparagine substitution at position 417, glycine substitution at position 614, deletions at positions 242 to 244, and proline substitutions at positions 986 and 987 of SEQ ID NO:1.

In one embodiment, the RNA used for the first vaccination encodes a polypeptide comprising an amino acid sequence with proline residue substitutions at positions 986 and 987 of SEQ ID NO:1 and the RNA used for the second vaccination is RNA encoding a polypeptide comprising an amino acid sequence with the following mutations in SEQ ID NO:1:
A67V, Δ69-70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F, K986P and V987P, as compared to SEQ ID NO: 1.

In one embodiment, the RNA used for the first vaccination encodes a polypeptide comprising an amino acid sequence with following mutations in SEQ ID NO: 1: residue substitutions at positions 986 and 987 of SEQ ID NO:1 and the RNA used for the second vaccination is RNA encoding a polypeptide comprising an amino acid sequence with the following mutations in SEQ ID NO:1:
T19I, Δ24-26, A27S, G142D, V213G, G339D, S371F, S373P, S375F, T376A, D405N, R408S, K417N, N440K, S477N, T478K, E484A, Q493R, Q498R, N501Y, Y505H, D614G, H655Y, N679K, P681H, N764K, D796Y, Q954H, N969K, K986P, and V987P, as compared to SEQ ID NO: 1.

In one embodiment, the RNA used for the first vaccination encodes a polypeptide comprising an amino acid sequence with proline residue substitutions at positions 986 and 987 of SEQ ID NO:1 and the RNA used for the second vaccination is RNA encoding a polypeptide comprising an amino acid sequence with the following mutations in SEQ ID NO:1:
T19I, Δ24-26, A27S, Δ69/70, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, N969K, K986P, and V987P, as compared to SEQ ID NO: 1.

In one embodiment, the RNA used for the first vaccination encodes a polypeptide comprising an amino acid sequence with following mutations in SEQ ID NO: 1:
A67V, Δ69-70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F, K986P and V987P, and the RNA used for the second vaccination encodes a polypeptide comprising an amino acid sequence with the following mutations in SEQ ID NO: 1:
T19I, Δ24-26, A27S, G142D, V213G, G339D, S371F, S373P, S375F, T376A, D405N, R408S, K417N, N440K, S477N, T478K, E484A, Q493R, Q498R, N501Y, Y505H, D614G, H655Y, N679K, P681H, N764K, D796Y, Q954H, N969K, K986P, and V987P, as compared to SEQ ID NO: 1.

In one embodiment, the RNA used for the first vaccination encodes a polypeptide comprising an amino acid sequence with following mutations in SEQ ID NO: 1:
A67V, Δ69-70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F, K986P and V987P, and the RNA used for the second vaccination encodes a polypeptide comprising an amino acid sequence with the following mutations in SEQ ID NO: 1:
T19I, Δ24-26, A27S, Δ69/70, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, N969K, K986P, and V987P, as compared to SEQ ID NO: 1.

In one embodiment, the RNA used for the first vaccination encodes a polypeptide comprising an amino acid sequence with following mutations in SEQ ID NO: 1:
T19I, Δ24-26, A27S, G142D, V213G, G339D, S371F, S373P, S375F, T376A, D405N, R408S, K417N, N440K, S477N, T478K, E484A, Q493R, Q498R, N501Y, Y505H, D614G, H655Y, N679K, P681H, N764K, D796Y, Q954H, N969K, K986P, and V987P, as compared to SEQ ID NO: 1, and the RNA used for the second vaccination encodes a polypeptide comprising an amino acid sequence with the following mutations in SEQ ID NO: 1:
T19I, Δ24-26, A27S, Δ69/70, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, N969K, K986P, and V987P, as compared to SEQ ID NO: 1.

In one embodiment, the RNA used for the first vaccination encodes a polypeptide comprising the amino acid sequence of SEQ ID NO: 7 and the RNA used for the second vaccination is RNA encoding a polypeptide comprising the amino acid sequence of SEQ ID NO: 49.

In one embodiment, the RNA used for the first vaccination comprises the nucleotide sequence of SEQ ID NO: 20 and the RNA used for the second vaccination is RNA comprising the nucleotide sequence of SEQ ID NO: 51.

In one embodiment, the RNA used for the first vaccination encodes a polypeptide comprising the amino acid sequence of SEQ ID NO: 7 and the RNA used for the second vaccination is RNA encoding a polypeptide comprising the amino acid sequence of SEQ ID NO: 55, 58 or 61.

In one embodiment, the RNA used for the first vaccination comprises the nucleotide sequence of SEQ ID NO: 20 and the RNA used for the second vaccination is RNA comprising the nucleotide sequence of SEQ ID NO: 57, 60, or 63a.

In one embodiment, the RNA used for the first vaccination encodes a polypeptide comprising the amino acid sequence of SEQ ID NO: 58 and the RNA used for the second vaccination is RNA encoding a polypeptide comprising the amino acid sequence of SEQ ID NO: 49, 55 or 61.

In one embodiment, the RNA used for the first vaccination comprises the nucleotide sequence of SEQ ID NO: 60 and the RNA used for the second vaccination is RNA comprising the nucleotide sequence of SEQ ID NO: 51, 57, or 63a.

In one embodiment, the RNA used for the first vaccination encodes a polypeptide comprising the amino acid sequence of SEQ ID NO: 49 and the RNA used for the second vaccination is RNA encoding a polypeptide comprising the amino acid sequence of SEQ ID NO: 55 or 61.

In one embodiment, the RNA used for the first vaccination comprises the nucleotide sequence of SEQ ID NO: 51 and the RNA used for the second vaccination is RNA comprising the nucleotide sequence of SEQ ID NO: 57 or 63a.

In one embodiment, the RNA used for the first vaccination encodes a polypeptide comprising the amino acid sequence of SEQ ID NO: 55 and the RNA used for the second vaccination is RNA encoding a polypeptide comprising the amino acid sequence of SEQ ID NO: 61.

In one embodiment, the RNA used for the first vaccination comprises the nucleotide sequence of SEQ ID NO: 57 and the RNA used for the second vaccination is RNA comprising the nucleotide sequence of SEQ ID NO: 63a.

In one embodiment, the RNA used for the first vaccination encodes a polypeptide comprising an amino acid sequence with proline residue substitutions at positions 986 and 987 of SEQ ID NO:1 and the RNA used for the second vaccination is RNA encoding a polypeptide comprising an amino acid sequence with the following mutations in SEQ ID NO:1:
A67V, Δ69-70, T95I, G142D, Δ143-145, Δ211, L212I, ins214EPE, G339D, S371L, S373P, S375F, S477N, T478K, E484A, Q493R, G496S, Q498R, N501Y, Y505H, T547K, D614G, H655Y, N679K, P681H, N764K, D796Y, N856K, Q954H, N969K, L981F, K986P and V987P, as compared to SEQ ID NO: 1. In some embodiments, the polypeptide encoded by the RNA used in the second vaccination further comprises proline residue substitutions at positions corresponding to 986 and 987 of SEQ ID NO:1.

In one embodiment, the RNA used for the first vaccination encodes a polypeptide comprising the amino acid sequence of SEQ ID NO: 7 and the RNA used for the second vaccination is RNA encoding a polypeptide comprising the amino acid sequence of SEQ ID NO: 52.

In one embodiment, a subject is administered a primary regimen comprising two 30 ug doses of RNA comprising a sequence encoding a polypeptide comprising the amino acid sequence of SEQ ID NO: 7, and a booster regimen comprising at least one dose of 30 ug of RNA comprising a sequence encoding a polypeptide comprising the amino acid sequence of SEQ ID NO: 7, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a sequence encoding a polypeptide comprising the amino acid sequence of SEQ ID NO: 7.

In one embodiment, a subject is administered a primary regimen comprising two 30 ug doses of RNA comprising a sequence encoding a polypeptide comprising the amino acid sequence of SEQ ID NO: 7, and a booster regimen comprising at least one dose of 50 ug of RNA comprising a sequence encoding a polypeptide comprising the amino acid sequence of SEQ ID NO: 7, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a sequence encoding a polypeptide comprising the amino acid sequence of SEQ ID NO: 7.

In one embodiment, a subject is administered a primary regimen comprising two 30 ug doses of RNA comprising a sequence encoding a polypeptide comprising the amino acid sequence of SEQ ID NO: 7, and a booster regimen comprising at least one dose of 60 ug of RNA comprising a sequence encoding a polypeptide comprising the amino acid sequence of SEQ ID NO: 7, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a sequence encoding a polypeptide comprising the amino acid sequence of SEQ ID NO: 7.

In one embodiment, a subject is administered a primary regimen comprising two 30 ug doses of RNA comprising a sequence encoding a polypeptide comprising the amino acid sequence of SEQ ID NO: 7, and a booster regimen comprising at least one dose of 30 ug of RNA comprising a sequence encoding a polypeptide comprising the amino acid sequence of SEQ ID NO: 49, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a sequence encoding a polypeptide comprising the amino acid sequence of SEQ ID NO: 7.

In one embodiment, a subject is administered a primary regimen comprising at least two 30 ug doses of RNA comprising a sequence encoding a polypeptide comprising the amino acid sequence of SEQ ID NO: 7, and a booster regimen comprising at least two doses of 30 ug of RNA comprising a sequence encoding a polypeptide comprising the amino acid sequence of SEQ ID NO: 49, wherein in some embodiments the two doses of the booster regimen are administered at least 2 months apart from each other (e.g., at least 3 months, at least 4 months, at least 5 months, or at least 6 months apart from each other). In some embodiments, such a subject may have previously been administered a 30 ug dose of RNA comprising a sequence encoding a polypeptide comprising the amino acid sequence of SEQ ID NO: 7 as a booster dose.

In one embodiment, a subject is administered a primary regimen comprising two 30 ug doses of RNA comprising a sequence encoding a polypeptide comprising the amino acid sequence of SEQ ID NO: 7, and a booster regimen comprising at least one dose of 50 ug of RNA comprising a nucleotide sequence that encodes the amino acid sequence of SEQ ID NO: 49, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a sequence encoding a polypeptide comprising the amino acid sequence of SEQ ID NO: 7.

In one embodiment, a subject is administered a primary regimen comprising two 30 ug doses of RNA comprising a sequence encoding a polypeptide comprising the amino acid sequence of SEQ ID NO: 7, and a booster regimen comprising at least one dose of 60 ug of RNA comprising a nucleotide sequence that encodes the amino acid sequence of SEQ ID NO: 49, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a sequence encoding a polypeptide comprising the amino acid sequence of SEQ ID NO: 7.

In some embodiments, a subject is administered a primary regimen comprising two doses of 30 ug of RNA (administered, e.g., about 21 days after one another), wherein each 30 ug dose of RNA comprises 15 ug of RNA comprising a nucleotide sequence encoding the amino acid sequence of SEQ ID NO: 7 and 15 ug of RNA comprising a nucleotide sequence encoding the amino acid sequence of SEQ ID NO: 49. In some embodiments, such a primary regimen is administered to a vaccine naive subject.

In some embodiments, a subject is administered a primary regimen comprising two doses of 30 ug of RNA (administered, e.g., about 21 days after one another), wherein each 30 ug dose of RNA comprises a nucleotide sequence encoding the amino acid sequence of SEQ ID NO: 49. In some embodiments, such a primary regimen is administered to a vaccine naive subject.

In one embodiment, a subject is administered a primary regimen comprising two 30 ug doses of RNA comprising a sequence encoding a polypeptide comprising the amino acid sequence of SEQ ID NO: 7, and a booster regimen comprising at least one dose of 30 ug of RNA, wherein the 30 ug of RNA comprises 15 ug of RNA comprising a nucleotide sequence encoding the amino acid sequence of SEQ ID NO: 7 and 15 ug of RNA comprising a nucleotide sequence encoding the amino acid sequence of SEQ ID NO: 49, wherein the two RNAs are optionally administered in the same composition, and wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a sequence encoding a polypeptide comprising the amino acid sequence of SEQ ID NO: 7.

In one embodiment, a subject is administered a primary regimen comprising two 30 ug doses of RNA comprising a sequence encoding a polypeptide comprising the amino acid sequence of SEQ ID NO: 7, and a booster regimen comprising at least one dose of 50 ug of RNA, wherein the 50 ug of RNA comprises 25 ug of RNA comprising a nucleotide sequence encoding the amino acid sequence of SEQ ID NO: 7 and 25 ug of RNA comprising a nucleotide sequence encoding the amino acid sequence of SEQ ID NO: 49, wherein the two RNAs are optionally administered in the same composition (e.g., a formulation comprising both RNAs), and wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a sequence encoding a polypeptide comprising the amino acid sequence of SEQ ID NO: 7.

In one embodiment, a subject is administered a primary regimen comprising two 30 ug doses of RNA comprising a sequence encoding a polypeptide comprising the amino acid sequence of SEQ ID NO: 7, and a booster regimen comprising at least one dose of 60 ug of RNA, wherein the 60 ug of RNA comprises 30 ug of RNA comprising a nucleotide sequence encoding the amino acid sequence of SEQ ID NO: 7 and 30 ug of an RNA comprising a nucleotide sequence encoding the amino acid sequence of SEQ ID NO: 49, wherein the two RNAs are optionally administered in the same composition, and wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a sequence encoding a polypeptide comprising the amino acid sequence of SEQ ID NO: 7.

In one embodiment, the RNA used for the first vaccination comprises the nucleotide sequence of SEQ ID NO: 20 and the RNA used for the second vaccination is RNA comprising the nucleotide sequence of SEQ ID NO: 54.

In one embodiment, a subject is administered a primary regimen comprising two 30 ug doses of RNA comprising a nucleotide sequence of SEQ ID NO: 20, and a booster regimen comprising at least one dose of 30 ug of RNA comprising a nucleotide sequence of SEQ ID NO: 20, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a nucleotide sequence of SEQ ID NO: 20.

In one embodiment, a subject is administered a primary regimen comprising two 30 ug doses of RNA comprising a nucleotide sequence of SEQ ID NO: 20, and a booster regimen comprising at least one dose of 50 ug of RNA comprising a nucleotide sequence of SEQ ID NO: 20, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a nucleotide sequence of SEQ ID NO: 20.

In one embodiment, a subject is administered a primary regimen comprising two 30 ug doses of RNA comprising a nucleotide sequence of SEQ ID NO: 20, and a booster regimen comprising at least one dose of 60 ug of RNA comprising a nucleotide sequence of SEQ ID NO: 20, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a nucleotide sequence of SEQ ID NO: 20.

In one embodiment, a subject is administered a primary regimen comprising two 30 ug doses of RNA comprising a nucleotide sequence of SEQ ID NO: 20, and a booster regimen comprising at least one dose of 30 ug of RNA comprising a nucleotide sequence of SEQ ID NO: 51, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a nucleotide sequence of SEQ ID NO: 20.

In one embodiment, a subject is administered a primary regimen comprising two 30 ug doses of RNA comprising a nucleotide sequence of SEQ ID NO: 20, and a booster regimen comprising at least one dose of 50 ug of RNA comprising a nucleotide sequence of SEQ ID NO: 51, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a nucleotide sequence of SEQ ID NO: 20.

In one embodiment, a subject is administered a primary regimen comprising two 30 ug doses of RNA comprising a nucleotide sequence of SEQ ID NO: 20, and a booster regimen comprising at least one dose of 60 ug of RNA comprising a nucleotide sequence of SEQ ID NO: 51, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a nucleotide sequence of SEQ ID NO: 20.

In one embodiment, a subject is administered a primary regimen comprising two 30 ug doses of RNA comprising a nucleotide sequence of SEQ ID NO: 20, and a booster regimen comprising at least one dose of 30 ug of RNA, wherein the 30 ug of RNA comprises 15 ug of RNA comprising a nucleotide sequence of SEQ ID NO: 20 and 15 ug of RNA comprising a nucleotide sequence of SEQ ID NO: 51, wherein the two RNAs are optionally administered in the same composition, and wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a nucleotide sequence of SEQ ID NO: 20. In one embodiment, a subject is administered a primary regimen comprising two 30 ug doses of RNA comprising a nucleotide sequence of SEQ ID NO: 20, and a booster regimen comprising at least one dose comprising 50 ug of a RNA, wherein the 50 ug of RNA comprises 25 ug of RNA comprising a nucleotide sequence of SEQ ID NO: 20 and 25 ug of RNA comprising a nucleotide sequence of SEQ ID NO: 51, wherein the two RNAs are optionally administered in the same composition, and wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a nucleotide sequence of SEQ ID NO: 20.

In one embodiment, a subject is administered a primary regimen comprising two 30 ug doses of RNA comprising a nucleotide sequence of SEQ ID NO: 20, and a booster regimen comprising at least one dose comprising 60 ug of RNA, wherein the 60 ug of RNA comprises 30 ug of an RNA comprising a nucleotide sequence of SEQ ID NO: 20 and 30 ug of an RNA comprising a nucleotide sequence of SEQ ID NO: 51, wherein the two RNAs are optionally administered in the same composition, and wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a nucleotide sequence of SEQ ID NO: 20.

In one embodiment, a subject is administered a primary regimen comprising two 30 ug doses of RNA comprising a nucleotide sequence of SEQ ID NO: 20, and a booster regimen comprising at least one dose of 30 ug of RNA comprising a nucleotide sequence of SEQ ID NO: 57, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a nucleotide sequence of SEQ ID NO: 20.

In one embodiment, a subject is administered a primary regimen comprising two 30 ug doses of RNA comprising a nucleotide sequence of SEQ ID NO: 20, and a booster regimen comprising at least one dose of 50 ug of RNA comprising a nucleotide sequence of SEQ ID NO: 57, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a nucleotide sequence of SEQ ID NO: 20.

In one embodiment, a subject is administered a primary regimen comprising two 30 ug doses of RNA comprising a nucleotide sequence of SEQ ID NO: 20, and a booster regimen comprising at least one dose of 60 ug of RNA comprising a nucleotide sequence of SEQ ID NO: 57, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a nucleotide sequence of SEQ ID NO: 20.

In one embodiment, a subject is administered a primary regimen comprising two 30 ug doses of RNA comprising a nucleotide sequence of SEQ ID NO: 20, and a booster regimen comprising at least one dose of 30 ug of RNA comprising a nucleotide sequence of SEQ ID NO: 60, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a nucleotide sequence of SEQ ID NO: 20.

In one embodiment, a subject is administered a primary regimen comprising two 30 ug doses of RNA comprising a nucleotide sequence of SEQ ID NO: 20, and a booster regimen comprising at least one dose of 50 ug of RNA comprising a nucleotide sequence of SEQ ID NO: 60, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a nucleotide sequence of SEQ ID NO: 20.

In one embodiment, a subject is administered a primary regimen comprising two 30 ug doses of RNA comprising a nucleotide sequence of SEQ ID NO: 20, and a booster regimen comprising at least one dose of 60 ug of RNA comprising a nucleotide sequence of SEQ ID NO: 60, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a nucleotide sequence of SEQ ID NO: 20.

In one embodiment, a subject is administered a primary regimen comprising two 30 ug doses of RNA comprising a nucleotide sequence of SEQ ID NO: 20, and a booster regimen comprising at least one dose of 30 ug of RNA comprising a nucleotide sequence of SEQ ID NO: 63a, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a nucleotide sequence of SEQ ID NO: 20.

In one embodiment, a subject is administered a primary regimen comprising two 30 ug doses of RNA comprising a nucleotide sequence of SEQ ID NO: 20, and a booster regimen comprising at least one dose of 50 ug of RNA comprising a nucleotide sequence of SEQ ID NO: 63a, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a nucleotide sequence of SEQ ID NO: 20.

In one embodiment, a subject is administered a primary regimen comprising two 30 ug doses of RNA comprising a nucleotide sequence of SEQ ID NO: 63a, and a booster regimen comprising at least one dose of 60 ug of RNA comprising a nucleotide sequence of SEQ ID NO: 57, wherein the booster regimen is administered at least 2 months (e.g., at least 3 months, at least 4 months, at least 5 months, or at least 6 months) after administration of the primary regimen, and wherein the subject has optionally previously been administered a first booster regimen comprising a 30 ug dose of RNA comprising a nucleotide sequence of SEQ ID NO: 20. In one embodiment, the vaccination regimen comprises a first vaccination using two doses of RNA encoding a polypeptide comprising an amino acid sequence with proline residue substitutions at positions 986 and 987 of SEQ ID NO:1 administered about 21 days apart and a second vaccination using a single dose or multiple doses of RNA encoding a polypeptide comprising an amino acid sequence with proline residue substitutions at positions 986 and 987 of SEQ ID NO:1 administered about 4 to 12 months, 5 to 12 months, or 6 to 12 months after administration of the first vaccination, i.e., after the initial two-dose regimen. In one embodiment, each RNA dose comprises 30 µg RNA. In this embodiment, the aim in one embodiment is to induce an immune response that targets SARS-CoV-2 variants including, but not limited to, the Omicron (B.1.1.529) variant. Accordingly, in this embodiment, the aim in one embodiment is to protect a subject from infection with SARS-CoV-2 variants including, but not limited to, the Omicron (B.1.1.529) variant.

In one embodiment, the vaccination regimen comprises a first vaccination using two doses of RNA encoding a polypeptide comprising an amino acid sequence with proline residue substitutions at positions 986 and 987 of SEQ ID NO:1 administered about 21 days apart and a second vaccination using a single dose or multiple doses of RNA encoding a polypeptide comprising an amino acid sequence with alanine substitution at position 80, glycine substitution at position 215, lysine substitution at position 484, tyrosine substitution at position 501, valine substitution at position 701, phenylalanine substitution at position 18, isoleucine substitution at position 246, asparagine substitution at position 417, glycine substitution at position 614, deletions at positions 242 to 244, and proline substitutions at positions 986 and 987 of SEQ ID NO:1 administered about 6 to 12 months after administration of the first vaccination, i.e., after the initial two-dose regimen. In one embodiment, each RNA dose comprises 30 µg RNA.

In one embodiment, the vaccination regimen comprises a first vaccination using two doses of RNA encoding a polypeptide comprising an amino acid sequence with proline residue substitutions at positions 986 and 987 of SEQ ID NO:1 administered about 21 days apart and a second vaccination using a single dose or multiple doses of RNA encoding a polypeptide comprising an amino acid sequence with the following mutations in SEQ ID NO:1:
A67V, Δ69-70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F, K986P and V987P, as compared to SEQ ID NO: 1, administered after, e.g., about 6 to 12 months after administration of the first vaccination, i.e., after the initial two-dose regimen. In one embodiment, each RNA dose comprises 30 µg RNA.

In one embodiment, the vaccination regimen comprises a first vaccination using two doses of RNA encoding a polypeptide comprising an amino acid sequence with proline residue substitutions at positions 986 and 987 of SEQ ID NO:1 administered about 21 days apart and a second vaccination using a single dose or multiple doses of RNA encoding a polypeptide comprising an amino acid sequence with the following mutations in SEQ ID NO:1:
A67V, Δ69-70, T95I, G142D, Δ143-145, Δ211, L212I, ins214EPE, G339D, S371L, S373P, S375F, S477N, T478K, E484A, Q493R, G496S, Q498R, N501Y, Y505H, T547K, D614G, H655Y, N679K, P681H, N764K, D796Y, N856K, Q954H, N969K, L981F, K986P and V987P, as compared to SEQ ID NO: 1, administered after, e.g., about 6 to 12 months after administration of the first vaccination, i.e., after the initial two-dose regimen. In one embodiment, each RNA dose comprises 30 µg RNA. In some embodiments, the encoded polypeptide further comprises proline residue substitutions at positions corresponding to 986 and 987 of SEQ ID NO:1.

In one embodiment, the vaccination regimen comprises a first vaccination involving at least two doses of RNA encoding a polypeptide comprising an amino acid sequence with proline residue substitutions at positions 986 and 987 of SEQ ID NO: 1 administered about 21 days apart and a second vaccination involving a single dose or multiple doses of RNA encoding a polypeptide comprising an amino acid sequence with the following mutations in SEQ ID NO: 1:
T19I, Δ24-26, A27S, G142D, V213G, G339D, S371F, S373P, S375F, T376A, D405N, R408S, K417N, N440K, S477N, T478K, E484A, Q493R, Q498R, N501Y, Y505H, D614G, H655Y, N679K, P681H, N764K, D796Y, Q954H, N969K, K986P, and V987P, as compared to SEQ ID NO: 1, administered after, e.g., about 6 to 12 months after administration of the first vaccination, i.e., after the initial two-dose regimen. In one embodiment, each or at least one RNA dose comprises 30 µg RNA.

In one embodiment, the vaccination regimen comprises a first vaccination involving at least two doses of RNA encoding a polypeptide comprising an amino acid sequence with proline residue substitutions at positions 986 and 987 of SEQ ID NO:1 administered about 21 days apart and a second vaccination involving a single dose or multiple doses of RNA encoding a polypeptide comprising an amino acid sequence with the following mutations in SEQ ID NO:1: T19I, Δ24-26, A27S, Δ69/70, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, N969K, K986P, and V987P, as compared to SEQ ID NO: 1 administered after, e.g., about 6 to 12 months after administration of the first vaccination, i.e., after the initial two-dose regimen. In one embodiment, each or at least one RNA dose comprises 30 µg RNA.

In one embodiment, the vaccination regimen comprises a first vaccination involving at least two doses of RNA encoding a polypeptide comprising an amino acid sequence with the following mutations in SEQ ID NO:1:
A67V, Δ69-70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F, K986P and V987P, wherein the two doses of the first vaccination are administered about 21 days apart and wherein the vaccination regimen comprises a second vaccination involving a single dose or multiple doses of RNA encoding a polypeptide comprising an amino acid sequence with the following mutations in SEQ ID NO:1:
T19I, Δ24-26, A27S, G142D, V213G, G339D, S371F, S373P, S375F, T376A, D405N, R408S, K417N, N440K, S477N, T478K, E484A, Q493R, Q498R, N501Y, Y505H, D614G, H655Y, N679K, P681H, N764K, D796Y, Q954H, N969K, K986P, and V987P, as compared to SEQ ID NO: 1 administered after, e.g., about 6 to 12 months after administration of the first vaccination, i.e., after the initial two-dose regimen. In one embodiment, each or at least one RNA dose comprises 30 µg RNA.

In one embodiment, the vaccination regimen comprises a first vaccination involving at least two doses of RNA encoding a polypeptide comprising an amino acid sequence with the following mutations in SEQ ID NO:1:
A67V, Δ69-70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F, K986P and V987P, wherein the two doses of the first vaccination are administered about 21 days apart and wherein the vaccination regimen comprises a second vaccination involving a single dose or multiple doses of RNA encoding a polypeptide comprising an amino acid sequence with the following mutations in SEQ ID NO:1:
T19I, Δ24-26, A27S, Δ69/70, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, N969K, K986P, and V987P, as compared to SEQ ID NO: 1 administered after, e.g., about 6 to 12 months after administration of the first vaccination, i.e., after the initial two-dose regimen. In one embodiment, each or at least one RNA dose comprises 30 µg RNA.

In one embodiment, the vaccination regimen comprises a first vaccination involving at least two doses of RNA encoding a polypeptide comprising an amino acid sequence with the following mutations in SEQ ID NO:1:
T19I, Δ24-26, A27S, G142D, V213G, G339D, S371F, S373P, S375F, T376A, D405N, R408S, K417N, N440K, S477N, T478K, E484A, Q493R, Q498R, N501Y, Y505H, D614G, H655Y, N679K, P681H, N764K, D796Y, Q954H, N969K, K986P, and V987P, as compared to SEQ ID NO: 1, wherein the two doses of the first vaccination are administered about 21 days apart and wherein the vaccination regimen comprises a second vaccination involving a single dose or multiple doses of RNA encoding a polypeptide comprising an amino acid sequence with the following mutations in SEQ ID NO:1:
T19I, Δ24-26, A27S, Δ69/70, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, N969K, K986P, and V987P, as compared to SEQ ID NO: 1 administered after, e.g., about 6 to 12 months after administration of the first vaccination, i.e., after the initial two-dose regimen. In one embodiment, each or at least one RNA dose comprises 30 µg RNA.

In one embodiment, a vaccination regimen comprises (i) a first vaccination comprising at least three doses of an RNA described herein (e.g., where each dose comprises about 30 ug of an RNA comprising a nucleotide sequence of SEQ ID NO: 20), wherein a second dose may be administered about 21 days following administration of a first dose, and a third dose may be administered at least about 1, 2, 3, 4, 5, 6, 7, 8, 9, 10, 11, or 12 months after a second dose; and (ii) a second vaccination comprising at least one dose of an RNA described herein (e.g., wherein each dose comprises about 30 µg RNA per dose). In some embodiments, a second vaccination comprises at least one dose of a bivalent vaccine described herein, e.g., about 30 µg total of a bivalent vaccine, e.g., a bivalent vaccine comprising about 15 µg RNA encoding a SARS-CoV-2 S protein from a Wuhan strain and about 15 µg RNA encoding a SARS-CoV-2 S protein comprising mutations characteristic of an Omicron variant (b2+Omi). In some embodiments, the bivalent vaccine comprises about 15 µg RNA encoding a SARS-CoV-2 S protein from a Wuhan strain and about 15 µg RNA encoding a SARS-CoV-2 S protein comprising mutations characteristic of a BA.1 Omicron variant (e.g., 15 µg RNA comprising a sequence of SEQ ID NO: 20 and 15 µg of RNA comprising a sequence of SEQ ID NO: 51). In some embodiments, the bivalent vaccine comprises about 15 µg RNA encoding a SARS-CoV-2 S protein from a Wuhan strain and about 15 µg RNA encoding a SARS-CoV-2 S protein comprising mutations characteristic of a BA.4/5 Omicron variant (e.g., 15 µg RNA comprising a sequence of SEQ ID NO: 20 and 15 µg of RNA comprising a sequence of SEQ ID NO: 72). In some embodiments, the vaccination regimen is administered to a subject who is at least about 12 years old. In some embodiments, the vaccination regimen is administered to a subject who is at least about 6 months old to less than about 12 years old.

In one embodiment, the second vaccination results in a boosting of the immune response.

In one embodiment, RNA described herein is co-administered with other vaccines. In some embodiments, RNA described herein is co-administered with a composition comprising one or more T-cell epitopes of SARS-CoV-2 or RNA encoding the same. In some embodiments, RNA described herein is co-administered one or more T-cell epitopes, or RNA encoding the same, derived from an M protein, an N protein, and/or an ORF1ab protein of SARS-CoV-2, e.g., a composition disclosed in WO2021188969, the contents of which is incorporated by reference herein in its entirety. In some embodiments, RNA described herein (e.g., RNA encoding a SARS-CoV-2 S protein comprising mutations characteristic of a BA.1, BA.2, or BA.4/5 Omicron variant, optionally administered with RNA encoding a SARS-CoV-2 S protein of a Wuhan variant) is co-administered with a T-string construct described in WO2021188969 (e.g., an RNA encoding SEQ ID NO: RS C7p2full of WO2021/188969). In some embodiments, RNA described herein and a T-string construct described in WO2021188969 are administered in a combination of up to about 100 ug RNA total. In some embodiments, subjects are administered with at least 2 doses of RNA described herein (e.g., in some embodiments at 30 ug each) in combination with a T-string construct (e.g., an RNA encoding SEQ ID NO: RS C7p2full of WO2021/188969), e.g., each dose of a combination of RNA described herein and an RNA encoding SEQ ID NO: RS C7p2full of up to about 100 ug RNA total, wherein the two doses are administered, for example, at least 4 weeks or longer (including, e.g., at least 5 weeks, at least 6 weeks, at least 7 weeks, at least 8 weeks, at least 9 weeks, at least 10 weeks, at least 11 weeks, or at least 12 weeks, or longer) apart from one another. In some embodiments, subjects are administered at least 3 doses of RNA described herein (e.g., in some embodiments at 30 ug each) in combination with a T-string construct (e.g., an RNA encoding SEQ ID NO: RS C7p2full of WO2021/188969), e.g., each dose of a combination of RNA described herein and an RNA encoding SEQ ID NO: RS C7p2full of up to about 100 ug RNA total, wherein the first and the second doses and the second and third doses are each independently administered at least 4 weeks or longer (including, e.g., at least 5 weeks, at least 6 weeks, at least 7 weeks, at least 8 weeks, at least 9 weeks, at least 10 weeks, at least 11 weeks, or at least 12 weeks, or longer) apart from one another. In some embodiments, the RNA described herein and the T-string construct may be co-administered as separate formulations (e.g., formulations administered on the same day to separate injection sites). In some embodiments, the RNA described herein and the T-string construct may be co-administered as a co-formulation (e.g., a formulation comprising RNA described herein and the T-string construct as separate LNP formulations or as LNP formulations comprising both a T-string construct and RNA described herein).

In some embodiments, an RNA composition described herein is co-administered with one or more vaccines against a non-SARS-CoV-2 disease. In some embodiments, an RNA composition described herein is co-administered with one or more vaccines against a non-SARS-COV-2 viral disease. In some embodiments, an RNA composition described herein is co-administered with one or more vaccines against a non-SARS-CoV-2 respiratory disease. In some embodiments, the non-SARS-CoV-2 respiratory disease is a non-SARS-CoV-2 Coronavirus, an Influenza virus, a Pneumoviridae virus, or a Paramyxoviridae virus. In some embodiments, the Pneumoviridae virus is a Respiratory syncytial virus or a Metapneumovirus. In some embodiments, the Metapneumovirus is a human metapneumovirus (hMPV). In some embodiments, the Paramyxoviridae virus is a Parainfluenza virus or a Henipavirus. In some embodiments the parainfluenzavirus is PIV3. In some embodiments, the non-SAR-CoV-2 coronavirus is a betacoronavirus (e.g., SARS-CoV-1). In come embodiments the non-SARS-CoV-2 coronavirus is a Merbecovirus (e.g., a MERS-CoV virus).

In some embodiments, an RNA composition described herein is co-administered with an RSV vaccine (e.g., an RSV A or RSV B vaccine). In some embodiments, the RSV vaccine comprises an RSV fusion protein (F), an RSV attachment protein (G), an RSV small hydrophobic protein (SH), an RSV matrix protein (M), an RSV nucleoprotein (N), an RSV M2-1 protein, an RSV Large polymerase (L), and/or an RSV phosphoprotein (P), or an immunogenic fragment of immunogenic variant thereof, or a nucleic acid (e.g., RNA), encoding any one of the same.

In some embodiments, an RNA composition described herein is co-administered with an influenza vaccine. In some embodiments, the influenza vaccine is an alphainfluenza virus, a betainfluenza virus, a gammainfluenza virus or a deltainfluenza virus vaccine. In some embodiments the vaccine is an Influenza A virus, an Influenza B virus, an Influenza C virus, or an Influenza D virus vaccine. In some embodiments, the influenza A virus vaccine comprises a hemagglutinin selected from H1, H2, H3, H4, H5, H6, H7, H8, H9, H10, H11, H12, H13, H14, H15, H16, H17, and H18, or an immunogenic fragment or variant of the same, or a nucleic acid (e.g., RNA) encoding any one of the same. In some embodiments the influenza A vaccine comprises or encodes a neuraminidase (NA) selected from N1, N2, N3, N4, N5, N6, N7, N8, N9, N10, and N11, or an immunogenic fragment or variant of the same, or a nucleic acid (e.g., RNA) encoding any one of the same. In some embodiments, the influenza vaccine comprises at least one Influenza virus hemagglutinin (HA), neuraminidase (NA), nucleoprotein (NP), matrix protein 1 (M1), matrix protein 2 (M2), non-structural protein 1 (NS1 ), non-structural protein 2 (NS2), nuclear export protein (NEP), polymerase acidic protein (PA), polymerase basic protein PB1, PB1-F2, and/or polymerase basic protein 2 (PB2), or an immunogenic fragment or variant thereof, or a nucleic acid (e.g., RNA) encoding any of one of the same.

In some embodiments, an RNA composition provided herein and other injectable vaccine(s) are administered at different times. In some embodiments, an RNA composition provided herein is administered at the same time as other injectable vaccine(s). In some such embodiments, an RNA composition provided herein and at least one another injectable vaccine(s) are administered at different injection sites. In some embodiments, an RNA composition provided herein is not mixed with any other vaccine in the same syringe. In some embodiments, an RNA composition provided herein is not combined with other coronavirus vaccines as part of vaccination against coronavirus, e.g., SARS-CoV-2.

The term "disease" refers to an abnormal condition that affects the body of an individual. A disease is often construed as a medical condition associated with specific symptoms and signs.

A disease may be caused by factors originally from an external source, such as infectious disease, or it may be caused by internal dysfunctions, such as autoimmune diseases. In humans, "disease" is often used more broadly to refer to any condition that causes pain, dysfunction, distress, social problems, or death to the individual afflicted, or similar problems for those in contact with the individual. In this broader sense, it sometimes includes injuries, disabilities, disorders, syndromes, infections, isolated symptoms, deviant behaviors, and atypical variations of structure and function, while in other contexts and for other purposes these may be considered distinguishable categories. Diseases usually affect individuals not only physically, but also emotionally, as contracting and living with many diseases can alter one's perspective on life, and one's personality.

In the present context, the term "treatment", "treating" or "therapeutic intervention" relates to the management and care of a subject for the purpose of combating a condition such as a disease or disorder. The term is intended to include the full spectrum of treatments for a given condition from which the subject is suffering, such as administration of the therapeutically effective compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of an individual for the purpose of combating the disease, condition or disorder and includes the administration of the active compounds to prevent the onset of the symptoms or complications.

The term "therapeutic treatment" relates to any treatment which improves the health status and/or prolongs (increases) the lifespan of an individual. Said treatment may eliminate the disease in an individual, arrest or slow the development of a disease in an individual, inhibit or slow the development of a disease in an individual, decrease the frequency or severity of symptoms in an individual, and/or decrease the recurrence in an individual who currently has or who previously has had a disease.

The terms "prophylactic treatment" or "preventive treatment" relate to any treatment that is intended to prevent a disease from occurring in an individual. The terms "prophylactic treatment" or "preventive treatment" are used herein interchangeably.

The terms "individual" and "subject" are used herein interchangeably. They refer to a human or another mammal (e.g. mouse, rat, rabbit, dog, cat, cattle, swine, sheep, horse or primate) that can be afflicted with or is susceptible to a disease or disorder but may or may not have the disease or disorder. In many embodiments, the individual is a human being. Unless otherwise stated, the terms "individual" and "subject" do not denote a particular age, and thus encompass adults, elderlies, children, and newborns. In some embodiments, the term "subject" includes humans of age of at least 50, at least 55, at least 60, at least 65, at least 70, or older. In some embodiments, the term "subject" includes humans of age of at least 65, such as 65 to 80, 65 to 75, or 65 to 70. In embodiments of the present disclosure, the "individual" or "subject" is a "patient".

The term "patient" means an individual or subject for treatment, in particular a diseased individual or subject.

In one embodiment of the present disclosure, the aim is to provide an immune response against coronavirus, and to prevent or treat coronavirus infection.

A pharmaceutical composition comprising RNA encoding a peptide or protein comprising an epitope may be administered to a subject to elicit an immune response against an antigen comprising said epitope in the subject which may be therapeutic or partially or fully protective. A person skilled in the art will know that one of the principles of immunotherapy and vaccination is based on the fact that an immunoprotective reaction to a disease is produced by immunizing a subject with an antigen or an epitope, which is immunologically relevant with respect to the disease to be treated. Accordingly, pharmaceutical compositions described herein are applicable for inducing or enhancing an immune response. Pharmaceutical compositions described herein are thus useful in a prophylactic and/or therapeutic treatment of a disease involving an antigen or epitope.

As used herein, "immune response" refers to an integrated bodily response to an antigen or a cell expressing an antigen and refers to a cellular immune response and/or a humoral immune response. The immune system is divided into a more primitive innate immune system, and acquired or adaptive immune system of vertebrates, each of which contains humoral and cellular components.

"Cell-mediated immunity", "cellular immunity", "cellular immune response", or similar terms are meant to include a cellular response directed to cells characterized by expression of an antigen, in particular characterized by presentation of an antigen with class I or class II MHC. The cellular response relates to immune effector cells, in particular to cells called T cells or T lymphocytes which act as either "helpers" or "killers". The helper T cells (also termed CD4⁺ T cells) play a central role by regulating the immune response and the killer cells (also termed cytotoxic T cells, cytolytic T cells, CD8⁺ T cells or CTLs) kill diseased cells such as virus-infected cells, preventing the production of more diseased cells.

An immune effector cell includes any cell which is responsive to vaccine antigen. Such responsiveness includes activation, differentiation, proliferation, survival and/or indication of one or more immune effector functions. The cells include, in particular, cells with lytic potential, in particular lymphoid cells, and are preferably T cells, in particular cytotoxic lymphocytes, preferably selected from cytotoxic T cells, natural killer (NK) cells, and lymphokine-activated killer (LAK) cells. Upon activation, each of these cytotoxic lymphocytes triggers the destruction of target cells. For example, cytotoxic T cells trigger the destruction of target cells by either or both of the following means. First, upon activation T cells release cytotoxins such as perforin, granzymes, and granulysin. Perforin and granulysin create pores in the target cell, and granzymes enter the cell and trigger a caspase cascade in the cytoplasm that induces apoptosis (programmed cell death) of the cell. Second, apoptosis can be induced via Fas-Fas ligand interaction between the T cells and target cells.

The term "effector functions" in the context of the present disclosure includes any functions mediated by components of the immune system that result, for example, in the neutralization of a pathogenic agent such as a virus and/or in the killing of diseased cells such as virus-infected cells. In one embodiment, the effector functions in the context of the present disclosure are T cell mediated effector functions. Such functions comprise in the case of a helper T cell (CD4⁺ T cell) the release of cytokines and/or the activation of CD8⁺ lymphocytes (CTLs) and/or B cells, and in the case of CTL the elimination of cells, i.e., cells characterized by expression of an antigen, for example, via apoptosis or perforin-mediated cell lysis, production of cytokines such as IFN-γ and TNF-α, and specific cytolytic killing of antigen expressing target cells.

The term "immune effector cell" or "immunoreactive cell" in the context of the present disclosure relates to a cell which exerts effector functions during an immune reaction. An "immune effector cell" in one embodiment is capable of binding an antigen such as an antigen presented in the context of MHC on a cell or expressed on the surface of a cell and mediating an immune response. For example, immune effector cells comprise T cells (cytotoxic T cells, helper T cells, tumor infiltrating T cells), B cells, natural killer cells, neutrophils, macrophages, and dendritic cells. Preferably, in the context of the present disclosure, "immune effector cells" are T cells, preferably CD4⁺ and/or CD8⁺ T cells, most preferably CD8⁺ T cells. According to the present disclosure, the term "immune effector cell" also includes a cell which can mature into an immune cell (such as T cell, in particular T helper cell, or cytolytic T cell) with suitable stimulation. Immune effector cells comprise CD34⁺ hematopoietic stem cells, immature and mature T cells and immature and mature B cells. The differentiation of T cell precursors into a cytolytic T cell, when exposed to an antigen, is similar to clonal selection of the immune system.

A "lymphoid cell" is a cell which is capable of producing an immune response such as a cellular immune response, or a precursor cell of such cell, and includes lymphocytes, preferably T lymphocytes, lymphoblasts, and plasma cells. A lymphoid cell may be an immune effector cell as described herein. A preferred lymphoid cell is a T cell.

The terms "T cell" and "T lymphocyte" are used interchangeably herein and include T helper cells (CD4+ T cells) and cytotoxic T cells (CTLs, CD8+ T cells) which comprise cytolytic T cells. The term "antigen-specific T cell" or similar terms relate to a T cell which recognizes the antigen to which the T cell is targeted and preferably exerts effector functions of T cells.

T cells belong to a group of white blood cells known as lymphocytes, and play a central role in cell-mediated immunity. They can be distinguished from other lymphocyte types, such as B cells and natural killer cells by the presence of a special receptor on their cell surface called T cell receptor (TCR). The thymus is the principal organ responsible for the maturation of T cells. Several different subsets of T cells have been discovered, each with a distinct function.

T helper cells assist other white blood cells in immunologic processes, including maturation of B cells into plasma cells and activation of cytotoxic T cells and macrophages, among other functions. These cells are also known as CD4+ T cells because they express the CD4 glycoprotein on their surface. Helper T cells become activated when they are presented with peptide antigens by MHC class II molecules that are expressed on the surface of antigen presenting cells (APCs). Once activated, they divide rapidly and secrete small proteins called cytokines that regulate or assist in the active immune response.

Cytotoxic T cells destroy virally infected cells and tumor cells, and are also implicated in transplant rejection. These cells are also known as CD8+ T cells since they express the CD8 glycoprotein on their surface. These cells recognize their targets by binding to antigen associated with MHC class I, which is present on the surface of nearly every cell of the body.

A majority of T cells have a T cell receptor (TCR) existing as a complex of several proteins. The TCR of a T cell is able to interact with immunogenic peptides (epitopes) bound to major histocompatibility complex (MHC) molecules and presented on the surface of target cells. Specific binding of the TCR triggers a signal cascade inside the T cell leading to proliferation and differentiation into a maturated effector T cell. The actual T cell receptor is composed of two separate peptide chains, which are produced from the independent T cell receptor alpha and beta (TCRα and TCRβ) genes and are called α- and β-TCR chains. γδ T cells (gamma delta T cells) represent a small subset of T cells that possess a distinct T cell receptor (TCR) on their surface. However, in γδ T cells, the TCR is made up of one γ-chain and one δ-chain. This group of T cells is much less common (2% of total T cells) than the αβ T cells.

"Humoral immunity" or "humoral immune response" is the aspect of immunity that is mediated by macromolecules found in extracellular fluids such as secreted antibodies, complement proteins, and certain antimicrobial peptides. It contrasts with cell-mediated immunity. Its aspects involving antibodies are often called antibody-mediated immunity.

Humoral immunity refers to antibody production and the accessory processes that accompany it, including: Th2 activation and cytokine production, germinal center formation and isotype switching, affinity maturation and memory cell generation. It also refers to the effector functions of antibodies, which include pathogen neutralization, classical complement activation, and opsonin promotion of phagocytosis and pathogen elimination.

In humoral immune response, first the B cells mature in the bone marrow and gain B-cell receptors (BCR's) which are displayed in large number on the cell surface. These membrane-bound protein complexes have antibodies which are specific for antigen detection. Each B cell has a unique antibody that binds with an antigen. The mature B cells migrate from the bone marrow to the lymph nodes or other lymphatic organs, where they begin to encounter pathogens. When a B cell encounters an antigen, the antigen is bound to the receptor and taken inside the B cell by endocytosis. The antigen is processed and presented on the B cell's surface again by MHC-II proteins. The B cell waits for a helper T cell (TH) to bind to the complex. This binding will activate the TH cell, which then releases cytokines that induce B cells to divide rapidly, making thousands of identical clones of the B cell. These daughter cells either become plasma cells or memory cells. The memory B cells remain inactive here; later when these memory B cells encounter the same antigen due to reinfection, they divide and form plasma cells. On the other hand, the plasma cells produce a large number of antibodies which are released free into the circulatory system. These antibodies will encounter antigens and bind with them. This will either interfere with the chemical interaction between host and foreign cells, or they may form bridges between their antigenic sites hindering their proper functioning, or their presence will attract macrophages or killer cells to attack and phagocytose them.

The term "antibody" includes an immunoglobulin comprising at least two heavy (H) chains and two light (L) chains inter-connected by disulfide bonds. Each heavy chain is comprised of a heavy chain variable region (abbreviated herein as VH) and a heavy chain constant region. Each light chain is comprised of a light chain variable region (abbreviated herein as VL) and a light chain constant region. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system. An antibody binds, preferably specifically binds with an antigen.

Antibodies expressed by B cells are sometimes referred to as the BCR (B cell receptor) or antigen receptor. The five members included in this class of proteins are IgA, IgG, IgM, IgD, and IgE. IgA is the primary antibody that is present in body secretions, such as saliva, tears, breast milk, gastrointestinal secretions and mucus secretions of the respiratory and genitourinary tracts. IgG is the most common circulating antibody. IgM is the main immunoglobulin produced in the primary immune response in most subjects. It is the most efficient immunoglobulin in agglutination, complement fixation, and other antibody responses, and is important in defense against bacteria and viruses. IgD is the immunoglobulin that has no known antibody function, but may serve as an antigen receptor. IgE is the immunoglobulin that mediates immediate hypersensitivity by causing release of mediators from mast cells and basophils upon exposure to allergen.

An "antibody heavy chain", as used herein, refers to the larger of the two types of polypeptide chains present in antibody molecules in their naturally occurring conformations.

An "antibody light chain", as used herein, refers to the smaller of the two types of polypeptide chains present in antibody molecules in their naturally occurring conformations, κ and λ light chains refer to the two major antibody light chain isotypes.

The present disclosure contemplates an immune response that may be protective, preventive, prophylactic and/or therapeutic. As used herein, "induces [or inducing] an immune response" may indicate that no immune response against a particular antigen was present before induction or it may indicate that there was a basal level of immune response against a particular antigen before induction, which was enhanced after induction. Therefore, "induces [or inducing] an immune response" includes "enhances [or enhancing] an immune response". The term "immunotherapy" relates to the treatment of a disease or condition by inducing, or enhancing an immune response. The term "immunotherapy" includes antigen immunization or antigen vaccination.

The terms "immunization" or "vaccination" describe the process of administering an antigen to an individual with the purpose of inducing an immune response, for example, for therapeutic or prophylactic reasons.

The term "macrophage" refers to a subgroup of phagocytic cells produced by the differentiation of monocytes. Macrophages which are activated by inflammation, immune cytokines or microbial products nonspecifically engulf and kill foreign pathogens within the macrophage by hydrolytic and oxidative attack resulting in degradation of the pathogen. Peptides from degraded proteins are displayed on the macrophage cell surface where they can be recognized by T cells, and they can directly interact with antibodies on the B cell surface, resulting in T and B cell activation and further stimulation of the immune response. Macrophages belong to the class of antigen presenting cells. In one embodiment, the macrophages are splenic macrophages.

The term "dendritic cell" (DC) refers to another subtype of phagocytic cells belonging to the class of antigen presenting cells. In one embodiment, dendritic cells are derived from hematopoietic bone marrow progenitor cells. These progenitor cells initially transform into immature dendritic cells. These immature cells are characterized by high phagocytic activity and low T cell activation potential. Immature dendritic cells constantly sample the surrounding environment for pathogens such as viruses and bacteria. Once they have come into contact with a presentable antigen, they become activated into mature dendritic cells and begin to migrate to the spleen or to the lymph node. Immature dendritic cells phagocytose pathogens and degrade their proteins into small pieces and upon maturation present those fragments at their cell surface using MHC molecules. Simultaneously, they upregulate cell-surface receptors that act as co-receptors in T cell activation such as CD80, CD86, and CD40 greatly enhancing their ability to activate T cells. They also upregulate CCR7, a chemotactic receptor that induces the dendritic cell to travel through the blood stream to the spleen or through the lymphatic system to a lymph node. Here they act as antigen-presenting cells and activate helper T cells and killer T cells as well as B cells by presenting them antigens, alongside non-antigen specific co-stimulatory signals. Thus, dendritic cells can actively induce a T cell- or B cell-related immune response. In one embodiment, the dendritic cells are splenic dendritic cells.

The term "antigen presenting cell" (APC) is a cell of a variety of cells capable of displaying, acquiring, and/or presenting at least one antigen or antigenic fragment on (or at) its cell surface. Antigen-presenting cells can be distinguished in professional antigen presenting cells and non-professional antigen presenting cells.

The term "professional antigen presenting cells" relates to antigen presenting cells which constitutively express the Major Histocompatibility Complex class II (MHC class II) molecules required for interaction with naive T cells. If a T cell interacts with the MHC class II molecule complex on the membrane of the antigen presenting cell, the antigen presenting cell produces a co-stimulatory molecule inducing activation of the T cell. Professional antigen presenting cells comprise dendritic cells and macrophages.

The term "non-professional antigen presenting cells" relates to antigen presenting cells which do not constitutively express MHC class II molecules, but upon stimulation by certain cytokines such as interferon-gamma. Exemplary, non-professional antigen presenting cells include fibroblasts, thymic epithelial cells, thyroid epithelial cells, glial cells, pancreatic beta cells or vascular endothelial cells.

"Antigen processing" refers to the degradation of an antigen into procession products, which are fragments of said antigen (e.g., the degradation of a protein into peptides) and the association of one or more of these fragments (e.g., via binding) with MHC molecules for presentation by cells, such as antigen presenting cells to specific T cells.

The term "disease involving an antigen" refers to any disease which implicates an antigen, e.g. a disease which is characterized by the presence of an antigen. The disease involving an antigen can be an infectious disease. As mentioned above, the antigen may be a disease-associated antigen, such as a viral antigen. In one embodiment, a disease involving an antigen is a disease involving cells expressing an antigen, preferably on the cell surface.

The term "infectious disease" refers to any disease which can be transmitted from individual to individual or from organism to organism, and is caused by a microbial agent (e.g. common cold). Infectious diseases are known in the art and include, for example, a viral disease, a bacterial disease, or a parasitic disease, which diseases are caused by a virus, a bacterium, and a parasite, respectively. In this regard, the infectious disease can be, for example, hepatitis, sexually transmitted diseases (e.g. chlamydia or gonorrhea), tuberculosis, HIV/acquired immune deficiency syndrome (AIDS), diphtheria, hepatitis B, hepatitis C, cholera, severe acute respiratory syndrome (SARS), the bird flu, and influenza.

### Exemplary Dosing Regimens

In some embodiments, compositions and methods disclosed herein can be used in accordance with an exemplary vaccination regimen as illustrated in Figure 14.

### Primary Dosing Regimens

In some embodiments, subjects are administered a primary dosing regimen. A primary dosing regimen can comprise one or more doses. For example, in some embodiments, a primary dosing regimen comprises a single dose (PD₁). In some embodiments a primary dosing regimen comprises a first dose (PD₁) and a second dose (PD₂). In some embodiments, a primary dosing regimen comprises a first dose, a second dose, and a third dose (PD₃). In some embodiments, a primary dosing regimen comprises a first dose, a second dose, a third dose, and one or more additional doses (POₙ) of any one of the pharmaceutical compositions described herein.

In some embodiments, PD₁ comprises administering 1 to 100 ug of RNA. In some embodiments, PD₁ comprises administering 1 to 60 ug of RNA In some embodiments, PD₁ comprises administering 1 to 50 ug of RNA. In some embodiments, PD₁ comprises administering 1 to 30 ug of RNA. In some embodiments, PD₁ comprises administering about 3 ug of RNA. In some embodiments, PD₁ comprises administering about 5 ug of RNA. In some embodiments, PD₁ comprises administering about 10 ug of RNA. In some embodiments, PD₁ comprises administering about 15 ug of RNA. In some embodiments, PD₁ comprises administering about 20 ug of RNA. In some embodiments, PD₁ comprises administering about 30 ug of RNA. In some embodiments, PD₁ comprises administering about 50 ug of RNA. In some embodiments, PD₁ comprises administering about 60 ug of RNA.

In some embodiments, PD₂ comprises administering 1 to 100 ug of RNA. In some embodiments, PD₂ comprises administering 1 to 60 ug of RNA. In some embodiments, PD₂ comprises administering 1 to 50 ug of RNA. In some embodiments, PD₂ comprises administering 1 to 30 ug of RNA. In some embodiments, PD₂ comprises administering about 3 ug. In some embodiments, PD₂ comprises administering about 5 ug of RNA. In some embodiments, PD₂ comprises administering about 10 ug of RNA. In some embodiments, PD₂ comprises administering about 15 ug of RNA. In some embodiments, PD₂ comprises administering about 20 ug RNA. In some embodiments, PD₂ comprises administering about 30 ug of RNA. In some embodiments, PD₂ comprises administering about 50 ug of RNA. In some embodiments, PD₂ comprises administering about 60 ug of RNA.

In some embodiments, PD₃ comprises administering 1 to 100 ug of RNA. In some embodiments, PD₃ comprises administering 1 to 60 ug of RNA. In some embodiments, PD₃ comprises administering 1 to 50 ug of RNA. In some embodiments, PD₃ comprises administering 1 to 30 ug of RNA. In some embodiments, PD₃ comprises administering about 3 ug of RNA. In some embodiments, PD₃ comprises administering about 5 ug of RNA. In some embodiments, PD₃ comprises administering about 10 ug of RNA. In some embodiments, PD₃ comprises administering about 15 ug of RNA. In some embodiments, PD₃ comprises administering about 20 ug of RNA. In some embodiments, PD₃ comprises administering about 30 ug of RNA. In some embodiments, PD₃ comprises administering about 50 ug of RNA. In some embodiments, PD₃ comprises administering about 60 ug of RNA.

In some embodiments, PDₙ comprises administering 1 to 100 ug of RNA. In some embodiments, PDₙ comprises administering 1 to 60 ug of RNA. In some embodiments, PDₙ comprises administering 1 to 50 ug of RNA. In some embodiments, PDₙ comprises administering 1 to 30 ug of RNA. In some embodiments, PDₙ comprises administering about 3 ug of RNA. In some embodiments, PDₙ comprises administering about 5 ug of RNA. In some embodiments, PDₙ comprises administering about 10 ug of RNA. In some embodiments, PDₙ comprises administering about 15 ug of RNA. In some embodiments, PDₙ comprises administering about 20 ug of RNA. In some embodiments, PDₙ comprises administering about 30 ug of RNA. In some embodiments, PDₙ comprises administering about 50 ug of RNA. In some embodiments, PDₙ comprises administering about 60 ug of RNA.

In some embodiments, PD₁ comprises an RNA encoding a SARS-CoV-2 Spike protein or an immunogenic fragment thereof from the Wuhan strain. In some embodiments, PD₁ comprises an RNA encoding a Spike protein or an immunogenic fragment thereof from a SARS-CoV-2 strain that is prevalent and/or spreading rapidly in a relevant jurisdiction. In some embodiments, PD₁ comprises an RNA encoding a SARS-CoV-2 Spike protein or an immunogenic fragment thereof comprising one or more mutations from an alpha variant. In some embodiments, PD₁ comprises an RNA encoding a SARS-CoV-2 Spike protein or an immunogenic fragment thereof comprising one or more mutations from a delta variant. In some embodiments, PD₁ comprises an RNA encoding a SARS-CoV-2 Spike protein or an immunogenic fragment thereof comprising one or more mutations from a beta variant. In some embodiments, PD₁ comprises an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant). In some embodiments, PD₁ comprises an RNA encoding a SARS-CoV-2 Spike protein or an immunogenic fragment thereof from the Wuhan strain and one or more additional RNAs encoding a Spike protein or an immunogenic fragment thereof from a SARS-CoV-2 strain that is prevalent and/or spreading rapidly in a relevant jurisdiction. In some embodiments, PD₁ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an alpha variant. In some embodiments, PD₁ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, PD₁ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, PD₁ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, PD₂ comprises an RNA encoding a SARS-CoV-2 Spike protein or an immunogenic fragment thereof from the Wuhan strain. In some embodiments, PD₂ comprises an RNA encoding a Spike protein or an immunogenic fragment thereof from a SARS-CoV-2 strain that is prevalent and/or spreading rapidly in a relevant jurisdiction. In some embodiments, PD₂ comprises an RNA encoding a SARS-CoV-2 Spike protein or an immunogenic fragment thereof comprising one or more mutations from an alpha variant. In some embodiments, PD₂ comprises an RNA encoding a SARS-CoV-2 Spike protein or an immunogenic fragment thereof comprising one or more mutations from a delta variant. In some embodiments, PD₂ comprises an RNA encoding a SARS-CoV-2 Spike protein or an immunogenic fragment thereof comprising one or more mutations from a beta variant. In some embodiments, PD₂ comprises an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant). In some embodiments, PD₂ comprises an RNA encoding a SARS-CoV-2 Spike protein or an immunogenic fragment thereof from the Wuhan strain and one or more additional RNAs encoding a Spike protein or an immunogenic fragment thereof from a SARS-CoV-2 strain that is prevalent and/or spreading rapidly in a relevant jurisdiction. In some embodiments, PD₂ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an alpha variant. In some embodiments, PD₂ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, PD₂ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, PD₂ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, PD₃ comprises an RNA encoding a SARS-CoV-2 Spike protein or an immunogenic fragment thereof from the Wuhan strain. In some embodiments, PD₃ comprises an RNA encoding a Spike protein or an immunogenic fragment thereof from a SARS-CoV-2 strain that is prevalent and/or spreading rapidly in a relevant jurisdiction. In some embodiments, PD₃ comprises an RNA encoding a SARS-CoV-2 Spike protein or an immunogenic fragment thereof comprising one or more mutations from an alpha variant. In some embodiments, PD₃ comprises an RNA encoding a SARS-CoV-2 Spike protein or an immunogenic fragment thereof comprising one or more mutations from a delta variant. In some embodiments, PD₃ comprises an RNA encoding a SARS-CoV-2 Spike protein or an immunogenic fragment thereof comprising one or more mutations from a beta variant. In some embodiments, PD₃ comprises an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant). In some embodiments, PD₃ comprises an RNA encoding a SARS-CoV-2 Spike protein or an immunogenic fragment thereof from the Wuhan strain and one or more additional RNAs encoding a Spike protein or an immunogenic fragment thereof from a SARS-CoV-2 strain that is prevalent and/or spreading rapidly in a relevant jurisdiction. In some embodiments, PD₃ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an alpha variant. In some embodiments, PD₃ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, PD₃ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, PD₃ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, PDₙ comprises an RNA encoding a SARS-CoV-2 Spike protein or an immunogenic fragment thereof from the Wuhan strain. In some embodiments, PDₙ comprises an RNA encoding a Spike protein or an immunogenic fragment thereof from a SARS-CoV-2 strain that is prevalent and/or spreading rapidly in a relevant jurisdiction. In some embodiments, PDₙ comprises an RNA encoding a SARS-CoV-2 Spike protein or an immunogenic fragment thereof comprising one or more mutations from an alpha variant. In some embodiments, PDₙ comprises an RNA encoding a SARS-CoV-2 Spike protein or an immunogenic fragment thereof comprising one or more mutations from a delta variant. In some embodiments, PDₙ comprises an RNA encoding a SARS-CoV-2 Spike protein or an immunogenic fragment thereof comprising one or more mutations from a beta variant. In some embodiments, PDₙ comprises an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant). In some embodiments, PDₙ comprises an RNA encoding a SARS-CoV-2 Spike protein or an immunogenic fragment thereof from the Wuhan strain and one or more additional RNAs encoding a Spike protein or an immunogenic fragment thereof from a SARS-CoV-2 strain that is prevalent and/or spreading rapidly in a relevant jurisdiction. In some embodiments, PDₙ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an alpha variant. In some embodiments, PDₙ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, PDₙ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, PDₙ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, PD₁, PD₂, PD₃, and PDᵣ, can each independently comprise a plurality of (e.g., at least two) mRNA compositions described herein. In some embodiments PD₁, PD₂, PD₃, and PDₙ can each independently comprise a first and a second mRNA composition. In some embodiments, at least one of a plurality of mRNA compositions comprises BNT162b2 (e.g., as described herein). In some embodiments, at least one of a plurality of mRNA compositions comprises an mRNA encoding a SARS-CoV-2 S protein or an immunogenic fragment thereof from a different SARS-CoV-2 variant. In some embodiments, at least one of a plurality of mRNA compositions comprises an mRNA encoding a SARS-CoV-2 S protein or an immunogenic fragment thereof from a Wuhan strain of SARS-CoV-2. In some embodiments, at least one of a plurarity of mRNA compositions comprises an RNA encoding a SARS-CoV-2 S protein or an immunogenic fragment thereof comprising one or more mutations from a variant that is prevalent and/or spreading rapidly in a relevant jurisdiction. In some embodiments, at least one of a plurality of mRNA compositions comprises an mRNA encoding a SARS-CoV-2 S protein or an immunogenic fragment thereof comprising one or more mutations from an alpha variant. In some embodiments, at least one of a plurality of mRNA compositions comprises an mRNA encoding a SARS-CoV-2 S protein or an immunogenic fragment thereof comprising one or more mutations from a delta variant. In some embodiments, at least one of a plurality of mRNA compositions comprises an mRNA encoding a SARS-CoV-2 S protein or an immunogenic fragment thereof comprising one or more mutations from an Omicron variant (e.g., a BA.4/5, BA.1, BA.2, XBB, XBB.1, or BQ.1 Omicron variant).

In some embodiments, a plurality of mRNA compositions given in PD₁, PD₂, PD_{3,} and/or PDₙ can each independently comprise at least two different mRNA constructs (e.g., differing in at protein-encoding sequences). For example, in some embodiments a plurality of mRNA compositions given in PD₁, PD₂, PD₃, and/or PDₙ can each independently comprise an mRNA encoding a SARS-CoV-2 S protein or an immunogenic fragment thereof from a Wuhan strain of SARS-CoV-2 and an mRNA encoding a SARS-CoV-2 S protein or an immunogenic fragment thereof comprising one or more mutations from a variant that is prevalent and/or spreading rapidly in a relevant jurisdiction. In some embodiments a plurality of mRNA compositions given in PD₁, PD₂, PD₃, and/or PDₙ can each independently comprise an mRNA encoding a SARS-CoV-2 S protein or an immunogenic fragment thereof derived from a Wuhan strain of SARS-CoV-2 and an mRNA encoding a SARS-CoV-2 S protein or an immunogenic fragment thereof comprising one or more mutations from a variant that is prevalent and/or spreading rapidly in a relevant jurisdiction. In some such embodiments, a variant can be an alpha variant. In some such embodiments, a variant can be a delta variant. In some such embodiments a variant can be an Omicron variant (e.g., a BA.4/5, BA.1, BA.2, XBB, XBB.1, or BQ.1 Omicron variant). In some embodiments, each of a plurality of mRNA compositions given in PD₁, PD₂, PD_{3,} and/or PDₙ can independently comprise at least two mRNAs, each encoding a SARS-CoV-2 S protein or an immunogenic fragment thereof comprising one or more mutations from a distinct variant that is prevalent and/or spreading rapidly in a relevant jurisdiction. In some embodiments, each of a plurality of mRNA compositions given in PD₁, PD₂, PD₃, and/or PDₙ can independently comprise an mRNA encoding a SARS-CoV-2 S protein or an immunogenic fragment thereof from an alpha variant and an mRNA encoding a SARS-CoV-2 S protein or an immunogenic fragment thereof comprising one or more mutations from a delta variant. In some embodiments, each of a plurality of mRNA compositions given in PD₁, PD₂, PD₃, and/or PDₙ can independently comprise an mRNA encoding a SARS-CoV-2 S protein or an immunogenic fragment thereof from an alpha variant and an mRNA encoding a SARS-CoV-2 S protein or an immunogenic fragment thereof comprising one or more mutations from an Omicron variant (e.g., a BA.4/5, BA.1, BA.2, XBB, XBB.1, or BQ.1 Omicron variant). In some embodiments, each of a plurality of mRNA compositions given in PD₁, PD₂, PD₃, and/or PDₙ can independently comprise an mRNA encoding a SARS-CoV-2 S protein or an immunogenic fragment thereof from a delta variant and an mRNA encoding a SARS-CoV-2 S protein or an immunogenic fragment thereof comprising one or more mutations from an Omicron variant (e.g., a BA.4/5, BA.1, BA.2, XBB, XBB.1, or BQ.1 Omicron variant).

In some embodiments, PD₁, PD₂, PD₃, and/or PDₙ each comprise a plurality of mRNA compositions, wherein each mRNA composition is separately administered to a subject. For example, in some embodiments each mRNA composition is administered via intramuscular injection at different injection sites. For example, in some embodiments, a first and second mRNA composition given in PD₁, PD₂, PD_{3,} and/or PDₙ are separately administered to different arms of a subject via intramuscular injection.

In some embodiments, PD₁, PD₂, PD₃, and/or PDₙ comprise administering a plurality of RNA molecules, wherein each RNA molecule encodes a Spike protein comprising mutations from a different SARS-CoV-2 variant, and wherein the plurality of RNA molecules are administered to the subject in a single formulation. In some embodiments, the single formulation comprises an RNA encoding a Spike protein or an immunogenic variant thereof from the Wuhan strain and an RNA encoding a SARS-CoV-2 Spike protein or an immunogenic fragment thereof comprising one or more mutations from an alpha variant. In some embodiments, the single formulation comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, the single formulation comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, the single formulation comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, the length of time between PD₁ and PD₂ (PI₁) is at least about 1 week, at least about 2 weeks, at least about 3 weeks, or at least about 4 weeks. In some embodiments, PI₁ is about 1 week to about 12 weeks. In some embodiments, PI₁ is about 1 week to about 10 weeks. In some embodiments, PI₁ is about 2 weeks to about 10 weeks. In some embodiments, PI₁ is about 2 weeks to about 8 weeks. In some embodiments, PI₁ is about 3 weeks to about 8 weeks. In some embodiments, PI₁ is about 4 weeks to about 8 weeks. In some embodiments, PI₁ is about 6 weeks to about 8 weeks. In some embodiments PI₁ is about 3 to about 4 weeks. In some embodiments, PI₁ is about 1 week. In some embodiments, PI₁ is about 2 weeks. In some embodiments, PI₁ is about 3 weeks. In some embodiments, PI₁ is about 4 weeks. In some embodiments, PI₁ is about 5 weeks. In some embodiments, PI₁ is about 6 weeks. In some embodiments, PI₁ is about 7 weeks. In some embodiments, PI₁ is about 8 weeks. In some embodiments, PI₁ is about 9 weeks. In some embodiments, PI₁ is about 10 weeks. In some embodiments, PI₁ is about 11 weeks. In some embodiments, PI₁ is about 12 weeks.

In some embodiments, the length of time between PD₂ and PD₃ (PI₂) is at least about 1 week, at least about 2 weeks, or at least about 3 weeks. In some embodiments, PI₂ is about 1 week to about 12 weeks. In some embodiments, PI₂ is about 1 week to about 10 weeks. In some embodiments, PI₂ is about 2 weeks to about 10 weeks. In some embodiments, PI₂ is about 2 weeks to about 8 weeks. In some embodiments, PI₂ is about 3 weeks to about 8 weeks. In some embodiments, PI₂ is about 4 weeks to about 8 weeks. In some embodiments, PI₂ is about 6 weeks to about 8 weeks. In some embodiments PI₂ is about 3 to about 4 weeks. In some embodiments, PI₂ is about 1 week. In some embodiments, PI₂ is about 2 weeks. In some embodiments, PI₂ is about 3 weeks. In some embodiments, PI₂ is about 4 weeks. In some embodiments, PI₂ is about 5 weeks. In some embodiments, PI₂ is about 6 weeks. In some embodiments, PI₂ is about 7 weeks. In some embodiments, PI₂ is about 8 weeks. In some embodiments, PI₂ is about 9 weeks. In some embodiments, PI₂ is about 10 weeks. In some embodiments, PI₂ is about 11 weeks. In some embodiments, PI₂ is about 12 weeks.

In some embodiments, the length of time between PD₃ and a subsequent dose that is part of the Primary Dosing Regimen, or between doses for any dose beyond PD₃ (PIₙ) is each separately and independently selected from: about 1 week or more, about 2 weeks or more, or about 3 weeks or more. In some embodiments, PIₙ is about 1 week to about 12 weeks. In some embodiments, PIₙ is about 1 week to about 10 weeks. In some embodiments, PIₙ is about 2 weeks to about 10 weeks. In some embodiments, PIₙ is about 2 weeks to about 8 weeks. In some embodiments, PIₙ is about 3 weeks to about 8 weeks. In some embodiments, PIₙ is about 4 weeks to about 8 weeks. In some embodiments, PIₙ is about 6 weeks to about 8 weeks. In some embodiments PIₙ is about 3 to about 4 weeks. In some embodiments, PI₂ is about 1 week. In some embodiments, PIₙ is about 2 weeks. In some embodiments, PIₙ is about 3 weeks. In some embodiments, PIₙ is about 4 weeks. In some embodiments, PIₙ is about 5 weeks. In some embodiments, PIₙis about 6 weeks. In some embodiments, PIₙ is about 7 weeks. In some embodiments, PIₙis about 8 weeks. In some embodiments, PIₙ is about 9 weeks. In some embodiments, PIₙ is about 10 weeks. In some embodiments, PIₙ is about 11 weeks. In some embodiments, PIₙ is about 12 weeks.

In some embodiments, one or more compositions adminstered in PD₁ are formulated in a Tris buffer. In some embodiments, one or more compositions administered in PD₂ are formulated in a Tris buffer. In some embodiments, one or more compositions administering in PD₃ are formulated in a Tris buffer. In some embodiments, one or more compositions adminsitered in PDₙ are formulated in a Tris buffer.

In some embodiments, the primary dosing regimen comprises administering two or more mRNA compositions described herein, and at least two of the mRNA compositions have different formulations. In some embodiments, the primary dosing regimen comprises PD₁ and PD₂, where PD₁ comprises administering an mRNA formulated in a Tris buffer and PD₂ comprises administering an mRNA formulated in a PBS buffer. In some embodiments, the primary dosing regimen comprises PD₁ and PD₂, where PD₁ comprises administering an mRNA formulated in a PBS buffer and PD₂ comprises administering an mRNA formulated in a Tris buffer.

In some embodiments, one or more mRNA compositions given in PD₁, PD₂, PD₃, and/or PDₙ can be administered in combination with another vaccine. In some embodiments, another vaccine is for a disease that is not COVID-19. In some embodiments, the disease is one that increases deleterious effects of SARS-CoV-2 when a subject is coinfected with the disease and SARS-CoV-2. In some embodiments, the disease is one that increases the transmission rate of SARS-CoV-2 when a subject is coinfected with the disease and SARS-CoV-2. In some embodiments, another vaccine is a different commerically available vaccine. In some embodiments, the different commercially available vaccine is an RNA vaccine. In some embodiments, the different commercially available vaccine is a polypeptide-based vaccine. In some embodiments, another vaccine (e.g., as described herein) and one or more mRNA compositions given in PD₁, PD₂, PD₃, and/or PDₙ are separately administered, for example, in some embodiments via intramuscular injection, at different injection sites. For example, in some embodiments, an influenza vaccine and one or more SARS-CoV-2 mRNA compositions described herein given in PD₁, PD₂, PD₃, and/or PDₙ are separately administered to different arms of a subject via intramuscular injection.

### Booster Dosing Regimens

In some embodiments, methods of vaccination disclosed herein comprise one or more Booster Dosing Regimens. The Booster Dosing Regimens disclosed herein comprise one or more doses. In some embodiments, a Booster Dosing Regimen is administered to patients who have been administered a Primary Dosing Regimen (e.g., as described herein). In some embodiments a Booster Dosing Regimen is administed to patients who have not received a pharmaceutical composition disclosed herein. In some embodiments a Booster Dosing Regimen is administered to patients who have been previously vaccinated with a COVID-19 vaccine that is different from the vaccine administered in a Primary Dosing Regimen.

In some embodiments, the length of time between the Primary Dosing Regimen and the Booster Dosing Regimen is at least 1 week, at least 2 weeks, at least 3 weeks, at least 4 weeks, at least 5 weeks, at least 6 weeks, at least 7 weeks, at least 8 weeks, at least 9 weeks, at least 10 weeks, at least 11 weeks, at least 12 weeks, at least 2 months, at least 3 months, at least 4 months, at least 5 months, at least 6 months, at least 7 months, at least 8 months, at least 9 months, at least 10 months, at least 11 months, or at least 12 months or longer. In some embodiments, the length of time between the Primary Dosing Regimen and the Booster Dosing Regimen is about 1 month. In some embodiments, the length of time between the Primary Dosing Regimen and the Booster Dosing Regimen is at least about 2 months. In some embodiments, the length of time between the Primary Dosing Regimen and the Booster Dosing Regimen is at least about 3 months. In some embodiments, the length of time between the Primary Dosing Regimen and the Booster Dosing Regimen is at least about 4 months. In some embodiments, the length of time between the Primary Dosing Regimen and the Booster Dosing Regimen is at least about 5 months. In some embodiments, the length of time between the Primary Dosing Regimen and the Booster Dosing Regimen is at least about 6 months. In some embodiments, the length of time between the Primary Dosing Regimen and the Booster Dosing Regimen is from about 1 month to about 48 months. In some embodiments, the length of time between the Primary Dosing Regimen and the Booster Dosing Regimen is from about 1 month to about 36 months. In some embodiments, the length of time between the primary dosing regimen and the Booster Dosing Regimen is from about 1 month to about 24 months. In some embodiments, the length of time between the Primary Dosing Regimen and the Booster Dosing Regimen is from about 2 months to about 24 months. In some embodiments, the length of time between the Primary Dosing Regimen and the Booster Dosing Regimen is from about 3 months to about 24 months. In some embodiments, the length of time between the primary dosing regimen and the Booster Dosing Regimen is from about 3 months to about 18 months. In some embodiments, the length of time between the primary dosing regimen and the Booster Dosing Regimen is from about 3 months to about 12 months. In some embodiments, the length of time between the primary dosing regimen and the Booster Dosing Regimen is from about 6 months to about 12 months. In some embodiments, the length of time between the Primary Dosing Regimen and the Booster Dosing Regimen is from about 3 months to about 9 months. In some embodiments, the length of time between the Primary Dosing Regimen and the Booster Dosing Regimen is from about 5 months to about 7 months. In some embodiments, the length of time between the Primary Dosing Regimen and the Booster Dosing Regimen is about 6 months.

In some embodiments, subjects are administered a Booster Dosing Regimen. A Booster dosing regimen can comprise one or more doses. For example, in some embodiments, a Booster Dosing Regimen comprises a single dose (BD₁). In some embodiments a Booster Dosing Regimen comprises a first dose (BD₁) and a second dose (BD₂). In some embodiments, a Booster Dosing Regimen comprises a first dose, a second dose, and a third dose (BD₃). In some embodiments, a Booster Dosing Regimen comprises a first dose, a second dose, a third dose, and one or more additional doses (BDₙ) of any one of the pharmaceutical compositions described herein.

In some embodiments, BD₁ comprises administering 1 to 100 ug of RNA. In some embodiments, BD₁ comprises administering 1 to 60 ug of RNA. In some embodiments, BD₁ comprises administering 1 to 50 ug of RNA. In some embodiments, BD₁ comprises administering 1 to 30 ug of RNA. In some embodiments, BD₁ comprises administering about 3 ug of RNA. In some embodiments, BD₁ comprises administering about 5 ug of RNA. In some embodiments, BD₁ comprises administering about 10 ug of RNA. In some embodiments, BD₁ comprises administering about 15 ug of RNA. In some embodiments, BD₁ comprises administering about 20 ug of RNA. In some embodiments, BD₁ comprises administering about 30 ug of RNA. In some embodiments, BD₁ comprises administering about 50 ug of RNA. In some embodiments, BD₁ comprises administering about 60 ug of RNA.

In some embodiments, BD₂ comprises administering 1 to 100 ug of RNA. In some embodiments, BD₂ comprises administering 1 to 60 ug of RNA. In some embodiments, BD₂ comprises administering 1 to 50 ug of RNA. In some embodiments, BD₂ comprises administering 1 to 30 ug of RNA. In some embodiments, BD₂ comprises administering about 3 ug. In some embodiments, BD₂ comprises administering about 5 ug of RNA. In some embodiments, BD₂ comprises administering about 10 ug of RNA. In some embodiments, BD₂ comprises administering about 15 ug of RNA. In some embodiments, BD₂ comprises administering about 20 ug RNA. In some embodiments, BD₂ comprises administering about 30 ug of RNA. In some embodiments, BD₂ comprises administering about 50 ug of RNA. In some embodiments, BD₂ comprises administering about 60 ug of RNA.

In some embodiments, BD₃ comprises administering 1 to 100 ug of RNA. In some embodiments, BD₃ comprises administering 1 to 60 ug of RNA. In some embodiments, BD₃ comprises administering 1 to 50 ug of RNA. In some embodiments, BD₃ comprises administering 1 to 30 ug of RNA. In some embodiments, BD₃ comprises administering about 3 ug of RNA. In some embodiments, BD₃ comprises administering about 5 ug of RNA. In some embodiments, BD₃ comprises administering about 10 ug of RNA. In some embodiments, BD₃ comprises administering about 15 ug of RNA. In some embodiments, BD₃ comprises administering about 20 ug of RNA. In some embodiments, BD₃ comprises administering about 30 ug of RNA. In some embodiments, BD₃ comprises administering about 50 ug of RNA. In some embodiments, BD₃ comprises administering about 60 ug of RNA.

In some embodiments, BDₙ comprises administering 1 to 100 ug of RNA. In some embodiments, BDₙ comprises administering 1 to 60 ug of RNA. In some embodiments, BDₙ comprises administering 1 to 50 ug of RNA. In some embodiments, BDₙ comprises administering 1 to 30 ug of RNA. In some embodiments, BDₙ comprises administering about 3 ug of RNA. In some embodiments, BDₙ comprises administering about 5 ug of RNA. In some embodiments, BDₙ comprises administering about 10 ug of RNA. In some embodiments, BDₙ comprises administering about 15 ug of RNA. In some embodiments, BDₙ comprises administering about 20 ug of RNA. In some embodiments, BDₙ comprises administering about 30 ug of RNA. In some embodiments, BDₙ comprises administering about 60 ug of RNA. In some embodiments, BDₙ comprises administering about 50 ug of RNA.

In some embodiments, BD₁ comprises an RNA encoding a SARS-CoV-2 Spike protein or an immunogenic fragment thereof from the Wuhan strain. In some embodiments, BD₁ comprises an RNA encoding a Spike protein or an immunogenic fragment thereof from a SARS-CoV-2 strain that is prevalent and/or spreading rapidly in a relevant jurisdiction. In some embodiments, BD₁ comprises an RNA encoding a SARS-CoV-2 Spike protein or an immunogenic fragment thereof comprising one or more mutations from an alpha variant. In some embodiments, BD₁ comprises an RNA encoding a SARS-CoV-2 Spike protein or an immunogenic fragment thereof comprising one or more mutations from a delta variant. In some embodiments, BD₁ comprises an RNA encoding a SARS-CoV-2 Spike protein or an immunogenic fragment thereof comprising one or more mutations from a beta variant. In some embodiments, BD₁ comprises an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, BD₁ comprises an RNA encoding a SARS-CoV-2 Spike protein or an immunogenic fragment thereof from the Wuhan strain and one or more RNA encoding a Spike protein or an immunogenic fragment thereof from a SARS-CoV-2 strain that is prevalent and/or spreading rapidly in a relevant jurisdiction. In some embodiments, BD₁ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a alpha variant. In some embodiments, BD₁ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, BD₁ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, BD₁ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, BD₂ comprises an RNA encoding a SARS-CoV-2 Spike protein or an immunogenic fragment thereof from the Wuhan strain. In some embodiments, BD₂ comprises an RNA encoding a Spike protein or an immunogenic fragment thereof from a SARS-CoV-2 strain that is prevalent and/or spreading rapidly in a relevant jurisdiction. In some embodiments, BD₂ comprises an RNA encoding a SARS-CoV-2 Spike protein or an immunogenic fragment thereof comprising one or more mutations from an alpha variant. In some embodiments, BD₂ comprises an RNA encoding a SARS-CoV-2 Spike protein or an immunogenic fragment thereof comprising one or more mutations from a delta variant. In some embodiments, BD₂ comprises an RNA encoding a SARS-CoV-2 Spike protein or an immunogenic fragment thereof comprising one or more mutations from a beta variant. In some embodiments, BD₂ comprises an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant). In some embodiments, BD₂ comprises an RNA encoding a SARS-CoV-2 Spike protein or an immunogenic fragment thereof from the Wuhan strain and one or more RNA encoding a Spike protein or an immunogenic fragment thereof from a SARS-CoV-2 strain that is prevalent and/or spreading rapidly in a relevant jurisdiction. In some embodiments, BD₂ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a alpha variant. In some embodiments, BD₂ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, BD₂ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, BD₂ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, BD₃ comprises an RNA encoding a SARS-CoV-2 Spike protein or an immunogenic fragment thereof from the Wuhan strain. In some embodiments, BD₃ comprises an RNA encoding a Spike protein or an immunogenic fragment thereof from a SARS-CoV-2 strain that is prevalent and/or spreading rapidly in a relevant jurisdiction. In some embodiments, BD₃ comprises an RNA encoding a SARS-CoV-2 Spike protein or an immunogenic fragment thereof comprising one or more mutations from an alpha variant. In some embodiments, BD₃ comprises an RNA encoding a SARS-CoV-2 Spike protein or an immunogenic fragment thereof comprising one or more mutations from a delta variant. In some embodiments, BD₃ comprises an RNA encoding a SARS-CoV-2 Spike protein or an immunogenic fragment thereof comprising one or more mutations from a beta variant. In some embodiments, BD₃ comprises an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, BD₃ comprises an RNA encoding a SARS-CoV-2 Spike protein or an immunogenic fragment thereof from the Wuhan strain and one or more RNA encoding a Spike protein or an immunogenic fragment thereof from a SARS-CoV-2 strain that is prevalent and/or spreading rapidly in a relevant jurisdiction. In some embodiments, BD₃ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a alpha variant. In some embodiments, BD₃ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, BD₃ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, BD₃ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, BDₙ comprises an RNA encoding a SARS-CoV-2 Spike protein or an immunogenic fragment thereof from the Wuhan strain. In some embodiments, BDₙ comprises an RNA encoding a Spike protein or an immunogenic fragment thereof from a SARS-CoV-2 strain that is prevalent and/or spreading rapidly in a relevant jurisdiction. In some embodiments, BDₙ comprises an RNA encoding a SARS-CoV-2 Spike protein or an immunogenic fragment thereof comprising one or more mutations from an alpha variant. In some embodiments, BDₙ comprises an RNA encoding a SARS-CoV-2 Spike protein or an immunogenic fragment thereof comprising one or more mutations from a delta variant. In some embodiments, BDₙ comprises an RNA encoding a SARS-CoV-2 Spike protein or an immunogenic fragment thereof comprising one or more mutations from a beta variant. In some embodiments, BDₙ comprises an RNA encoding a SARS-CoV-2 Spike protein or an immunogenic fragment thereof comprising one or more mutations from an Omicron variant(e.g., a BA.4/5, BA.1, BA.2, XBB, XBB.1, or BQ.1 Omicron variant).

In some embodiments, BDₙ comprises an RNA encoding a SARS-CoV-2 Spike protein or an immunogenic fragment thereof from the Wuhan strain and one or more RNA encoding a Spike protein or an immunogenic fragment thereof from a SARS-CoV-2 strain that is prevalent and/or spreading rapidly in a relevant jurisdiction. In some embodiments, BDₙ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a alpha variant. In some embodiments, BDₙ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, BDₙ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, BDₙ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, BD₁, BD₂, BD₃, and BDₙ can each independently comprise a plurality of (e.g., at least two) mRNA compositions described herein. In some embodiments BD₁, BD₂, BD₃, and BDₙ can each independently comprise a first and a second mRNA composition. In some embodiments, BD₁, BD₂, BD₃, and BDₙ can each independently comprise a plurality of (e.g., at least two) mRNA compositions, wherein , at least one of the plurality of mRNA compositions comprises BNT162b2 (e.g., as described herein). In some embodiments, at least one of a plurality of mRNA compositions comprises an mRNA encoding a SARS-CoV-2 S protein or an immunogenic fragment thereof from a different SARS-CoV-2 variant (e.g., a variant that is prevalent or rapidly spreading in a relevant jurisdiction, e.g., a variant disclosed herein). In some embodiments, at least one of a plurality of mRNA compositions comprises an mRNA encoding a SARS-CoV-2 S protein or an immunogenic fragment thereof from a Wuhan strain of SARS-CoV-2. In some embodiments, at least one of a plurality of mRNA compositions comprises an RNA encoding a SARS-CoV-2 S protein or an immunogenic fragment thereof comprising one or more mutations from a variant that is prevalent and/or spreading rapidly in a relevant jurisdiction. In some embodiments, at least one of a plurality of mRNA compositions comprises an mRNA encoding a SARS-CoV-2 S protein or an immunogenic fragment thereof comprising one or more mutations from an alpha variant. In some embodiments, at least one of a plurality of mRNA compositions comprises an mRNA encoding a SARS-CoV-2 S protein or an immunogenic fragment thereof comprising one or more mutations from a delta variant. In some embodiments, at least one of a plurality of mRNA compositions comprises an mRNA encoding a SARS-CoV-2 S protein or an immunogenic fragment thereof comprising one or more mutations from an Omicron variant (e.g., a BA.4/5, BA.1, BA.2, XBB, XBB.1, or BQ.1 Omicron variant).

In some embodiments, a plurality of mRNA compositions given in BD₁, BD₂, BD₃, and/or BDₙ can each indendently comprise at least two different mRNA constructs (e.g., mRNA constructs having differing protein-encoding sequences). For example, in some embodiments a plurality of mRNA compositions given in BD₁, BD₂, BD₃, and/or BDₙ can each indendently comprise an mRNA encoding a SARS-CoV-2 S protein or an immunogenic fragment thereof from a Wuhan strain of SARS-CoV-2 and an mRNA encoding a SARS-CoV-2 S protein or an immunogenic fragment thereof comprising one or more mutations from a variant that is prevalent and/or spreading rapidly in a relevant jurisdiction. In some embodiments a plurality of mRNA compositions given in BD₁, BD₂, BD₃, and/or BDₙ can each independently comprise an mRNA encoding a SARS-CoV-2 S protein or an immunogenic fragment thereof derived from a Wuhan strain of SARS-CoV-2 and an mRNA encoding a SARS-CoV-2 S protein or an immunogenic fragment thereof comprising one or more mutations from a variant that is prevalent and/or spreading rapidly in a relevant jurisdiction. In some such embodiments, a variant can be an alpha variant. In some such embodiments, a variant can be a delta variant. In some such embodiments a variant can be an Omicron variant (e.g., a BA.4/5, BA.1, BA.2, XBB, XBB.1, or BQ.1 Omicron variant).

In some embodiments, a plurality of mRNA compositions given in BD₁, BD₂, BD₃, and/or BDₙ can each independently comprise at least two mRNAs each encoding a SARS-CoV-2 S protein or an immunogenic fragment thereof comprising one or more mutations from a distinct variant that is prevalent and/or spreading rapidly in a relevant jurisdiction. In some embodiments a plurality of mRNA compositions given in BD₁, BD₂, BD₃, and/or BDₙ can each independently comprise an mRNA encoding a SARS-CoV-2 S protein or an immunogenic fragment thereof from an alpha variant and an mRNA encoding a SARS-CoV-2 S protein or an immunogenic fragment thereof comprising one or more mutations from a delta variant. In some embodiments a plurality of mRNA compositions given in BD₁, BD₂, BD₃, and/or BDₙ can each independently comprise an mRNA encoding a SARS-CoV-2 S protein or an immunogenic fragment thereof from an alpha variant and an mRNA encoding a SARS-CoV-2 S protein or an immunogenic fragment thereof comprising one or more mutations from an Omicron variant (e.g., a BA.4/5, BA.1, BA.2, XBB, XBB.1, or BQ.1 Omicron variant). In some embodiments a plurality of mRNA compositions given in BD₁, BD₂, BD₃, and/or BDₙ can each independently comprise an mRNA encoding a SARS-CoV-2 S protein or an immunogenic fragment thereof from a delta variant and an mRNA encoding a SARS-CoV-2 S protein or an immunogenic fragment thereof comprising one or more mutations from an Omicron variant (e.g., a BA.4/5, BA.1, BA.2, XBB, XBB.1, or BQ.1 Omicron variant).

In some embodiments, a plurality of mRNA compositions given in BD₁, BD₂, BD₃, and/or BDₙ are separately administered to a subject, for example, in some embodiments via intramuscular injection, at different injection sites. For example, in some embodiments, a first and second mRNA composition given in BD₁, BD₂, BD₃, and/or BDₙ are separately administered to different arms of a subject via intramuscular injection.

In some embodiments, the length of time between BD₁ and BD₂ (BI₁) is at least about 1 week, at least about 2 weeks, at least about 3 weeks, or at least about 4 weeks. In some embodiments, BI₁ is about 1 week to about 12 weeks. In some embodiments, BI₁ is about 1 week to about 10 weeks. In some embodiments, BI₁ is about 2 weeks to about 10 weeks. In some embodiments, BI₁ is about 2 weeks to about 8 weeks. In some embodiments, BI₁ is about 3 weeks to about 8 weeks. In some embodiments, BI₁ is about 4 weeks to about 8 weeks. In some embodiments, BI₁ is about 6 weeks to about 8 weeks. In some embodiments BI₁ is about 3 to about 4 weeks. In some embodiments, BI₁ is about 1 week. In some embodiments, BI₁ is about 2 weeks. In some embodiments, BI₁ is about 3 weeks. In some embodiments, BI₁ is about 4 weeks. In some embodiments, BI₁ is about 5 weeks. In some embodiments, BI₁ is about 6 weeks. In some embodiments, BI₁ is about 7 weeks. In some embodiments, BI₁ is about 8 weeks. In some embodiments, BI₁ is about 9 weeks. In some embodiments, BI₁ is about 10 weeks.

In some embodiments, the length of time between BD₂ and BD₃ (BI₂) is at least about 1 week, at least about 2 weeks, or at least about 3 weeks. In some embodiments, BI₂ is about 1 week to about 12 weeks. In some embodiments, BI₂ is about 1 week to about 10 weeks. In some embodiments, BI₂ is about 2 weeks to about 10 weeks. In some embodiments, BI₂ is about 2 weeks to about 8 weeks. In some embodiments, BI₂ is about 3 weeks to about 8 weeks. In some embodiments, BI₂ is about 4 weeks to about 8 weeks. In some embodiments, BI₂ is about 6 weeks to about 8 weeks. In some embodiments BI₂ is about 3 to about 4 weeks. In some embodiments, BI₂ is about 1 week. In some embodiments, BI₂ is about 2 weeks. In some embodiments, BI₂ is about 3 weeks. In some embodiments, BI₂ is about 4 weeks. In some embodiments, BI₂ is about 5 weeks. In some embodiments, BI₂ is about 6 weeks. In some embodiments, BI₂ is about 7 weeks. In some embodiments, BI₂ is about 8 weeks. In some embodiments, BI₂ is about 9 weeks. In some embodiments, BI₂ is about 10 weeks.

In some embodiments, the length of time between BD₃ and a subsequent dose that is part of the Booster Dosing Regimen, or between doses for any dose beyond BD₃ (BIₙ) is each separately and independently selected from: about 1 week or more, about 2 weeks or more, or about 3 weeks or more. In some embodiments, BIₙ is about 1 week to about 12 weeks. In some embodiments, BIₙ is about 1 week to about 10 weeks. In some embodiments, BIₙ is about 2 weeks to about 10 weeks. In some embodiments, BIₙ is about 2 weeks to about 8 weeks. In some embodiments, BIₙ is about 3 weeks to about 8 weeks. In some embodiments, BIₙ is about 4 weeks to about 8 weeks. In some embodiments, BIₙ is about 6 weeks to about 8 weeks. In some embodiments BIₙ is about 3 to about 4 weeks. In some embodiments, BIₙ is about 1 week. In some embodiments, BIₙ is about 2 weeks. In some embodiments, BIₙ is about 3 weeks. In some embodiments, BIₙ is about 4 weeks. In some embodiments, BIₙ is about 5 weeks. In some embodiments, BIₙ is about 6 weeks. In some embodiments, BIₙ is about 7 weeks. In some embodiments, BIₙ is about 8 weeks. In some embodiments, BIₙ is about 9 weeks. In some embodiments, BIₙ is about 10 weeks.

In some embodiments, one or more compositions adminstered in BD₁ are formulated in a Tris buffer. In some embodiments, one or more compositions administered in BD₂ are formulated in a Tris buffer. In some embodiments, one or more compositions administering in BD₃ are formulated in a Tris buffer. In some embodiments, one or more compositions adminsitered in BD₃ are formulated in a Tris buffer.

In some embodiments, the Booster dosing regimen comprises administering two or more mRNA compositions described herein, and at least two of the mRNA compositions have differnent formulations. In some embodiments, the Booster dosing regimen comprises BD₁ and BD₂, where BD₁ comprises administering an mRNA formulated in a Tris buffer and BD₂ comprises administering an mRNA formulated in a PBS buffer. In some embodiments, the Booster dosing regimen comprises BD₁ and BD₂, where BD₁ comprises administering an mRNA formulated in a PBS buffer and BD₂ comprises administering an mRNA formulated in a Tris buffer.

In some embodiments, one or more mRNA compositions given in BD₁, BD₂, BD₃, and/or BDₙ can be administered in combination with another vaccine. In some embodiments, another vaccine is for a disease that is not COVID-19. In some embodiments, the disease is one that increases deleterious effects of SARS-CoV-2 when a subject is coinfected with the disease and SARS-CoV-2. In some embodiments, the disease is one that increases the transmission rate of SARS-CoV-2 when a subject is coinfected with the disease and SARS-CoV-2. In some embodiments, another vaccine is a different commerically available vaccine. In some embodiments, the different commercially available vaccine is an RNA vaccine. In some embodiments, the different commercially available vaccine is a polypeptide-based vaccine. In some embodiments, another vaccine (e.g., as described herein) and one or more mRNA compositions given in BD₁, BD₂, BD₃, and/or BDₙ are separately administered, for example, in some embodiments via intramuscular injection, at different injection sites. For example, in some embodiments, an influenza vaccine and one or more SARS-CoV-2 mRNA compositions described herein given in BD₁, BD₂, BD₃, and/or BDₙ are separately administered to different arms of a subject via intramuscular injection.

### Additional Booster Regimens

In some embodiments, methods of vaccination disclosed herein comprise administering more than one Booster Dosing Regimen. In some embodiments, more than one Booster Dosing Regimen may need to be administered to increase neutralizing antibody response. In some embodiments, more than one booster dosing regimen may be needed to counteract a SARS-CoV-2 strain that has been shown to have a high likelihood of evading immune response elicited by vaccines that a patient has previously received. In some embodiments, an additional Booster Dosing Regimen is administered to a patient who has been determined to produce low concentrations of neutralizing antibodies. In some embodiments, an additional booster dosing regimen is administered to a patient who has been determined to have a high likelihood of being susceptible to SARS-CoV-2 infection, despite previous vaccination (e.g., an immunocompromised patient, a cancer patient, and/or an organ transplant patient).

The description provided above for the first Booster Dosing Regimen also describes the one or more additional Booster Dosing Regimens. The interval of time between the first Booster Dosing Regimen and a second Booster Dosing Regimen, or between subsequent Booster Dosing Regimens can be any of the acceptable intervals of time described above between the Primary Dosing Regimen and the First Booster Dosing Regimen.

In some embodiments, a dosing regimen comprises a primary regimen and a booster regimen, wherein at least one dose given in the primary regimen and/or the booster regimen comprises a composition comprising an RNA that encodes a S protein or immungenic fragment thereof from a variant that is prevalent or is spreading rapidly in a relevant jurisdiction (e.g., Omicron variant as described herein). For example, in some embodiments, a primary regimen comprises at least 2 doses of BNT162b2 (e.g., encoding a Wuhan strain), for example, given at least 3 weeks apart, and a booster regimen comprises at least 1 dose of a composition comprising RNA that encodes a S protein or immungenic fragment thereof from a variant that is prevalent or is spreading rapidly in a relevant jurisdiction (e.g., Omicron variant as described herein). In some such embodiments, such a dose of a booster regimen may further comprise an RNA that encodes a S protein or immungenic fragement thereof from a Wuhan strain, which can be administered with an RNA that encodes a S protein or immungenic fragment thereof from a variant that is prevalent or is spreading rapidly in a relevant jurisdiction (e.g., Omicron variant as described herein), as a single mixture, or as two separate compositions, for example, in 1:1 weight ratio. In some embodiments, a booster regimen can also comprise at least 1 dose of BNT162b2, which can be administered as a first booster dose or a subsequent booster dose.

In some embodiments, an RNA composition described herein is given as a booster at a dose that is higher than the doses given during a primary regimen (primary doses) and/or the dose given for a first booster, if any. For example, in some embodiments, such a dose may be 60 ug; or in some embodiments such a dose may be higher than 30 ug and lower than 60 ug (e.g., 55 ug, 50 ug, or lower). In some embodiments, an RNA composition described herein is given as a booster at least 3-12 months or 4-12 months, or 5-12 months, or 6-12 months after the last dose (e.g., the last dose of a primary regimen or a first dose of a booster regimen). In some embodiments, the primary doses and/or the first booster dose (if any) may comprise BNT162b2, for example at 30 ug per dose.

In some embodiments, an RNA composition described herein comprises an RNA encoding a polypeptide as set forth in SEQ ID NO: 49 or an immunogenic fragment thereof, or a variant thereof (e.g., having at least 70% or more, including, e.g., at least 80%, at least 85%, at least 90%, at least 95%, at least 96%, at least 97%, at least 98%, or higher, identity to SEQ ID NO: 49). In some embodiments, an RNA composition comprises an RNA that includes the sequence of SEQ ID NO: 50 or a variant thereof (e.g., having at least 70% or more, including, e.g., at least 80%, at least 85%, at least 90%, at least 95%, at least 96%, at least 97%, at least 98%, or higher, identity to SEQ ID NO: 50). In some embodiments, an RNA composition comprises an RNA that includes the sequence of SEQ ID NO: 51 or a variant thereof (e.g., having at least 70% or more, including, e.g., at least 80%, at least 85%, at least 90%, at least 95%, at least 96%, at least 97%, at least 98%, or higher, identity to SEQ ID NO: 51).

In some embodiments, an RNA composition described herein comprises an RNA encoding a polypeptide as set forth in SEQ ID NO: 55 or an immunogenic fragment thereof, or a variant thereof (e.g., having at least 70% or more, including, e.g., at least 80%, at least 85%, at least 90%, at least 95%, at least 96%, at least 97%, at least 98%, or higher, identity to SEQ ID NO: 55. In some embodiments, an RNA composition comprises an RNA that includes the sequence of SEQ ID NO: 56 or a variant thereof (e.g., having at least 70% or more, including, e.g., at least 80%, at least 85%, at least 90%, at least 95%, at least 96%, at least 97%, at least 98%, or higher, identity to SEQ ID NO: 56). In some embodiments, an RNA composition comprises an RNA that includes the sequence of SEQ ID NO: 57 or a variant thereof (e.g., having at least 70% or more, including, e.g., at least 80%, at least 85%, at least 90%, at least 95%, at least 96%, at least 97%, at least 98%, or higher, identity to SEQ ID NO: 57).

In some embodiments, an RNA composition described herein comprises an RNA encoding a polypeptide as set forth in SEQ ID NO: 58 or an immunogenic fragment thereof, or a variant thereof (e.g., having at least 70% or more, including, e.g., at least 80%, at least 85%, at least 90%, at least 95%, at least 96%, at least 97%, at least 98%, or higher, identity to SEQ ID NO: 58). In some embodiments, an RNA composition comprises an RNA that includes the sequence of SEQ ID NO: 59 or a variant thereof (e.g., having at least 70% or more, including, e.g., at least 80%, at least 85%, at least 90%, at least 95%, at least 96%, at least 97%, at least 98%, or higher, identity to SEQ ID NO: 59). In some embodiments, an RNA composition comprises an RNA that includes the sequence of SEQ ID NO: 60 or a variant thereof (e.g., having at least 70% or more, including, e.g., at least 80%, at least 85%, at least 90%, at least 95%, at least 96%, at least 97%, at least 98%, or higher, identity to SEQ ID NO: 60).

In some embodiments, an RNA composition described herein comprises an RNA encoding a polypeptide as set forth in SEQ ID NO: 61 or an immunogenic fragment thereof, or a variant thereof (e.g., having at least 70% or more, including, e.g., at least 80%, at least 85%, at least 90%, at least 95%, at least 96%, at least 97%, at least 98%, or higher, identity to SEQ ID NO: 61). In some embodiments, an RNA composition comprises an RNA that includes the sequence of SEQ ID NO: 62a or a variant thereof (e.g., having at least 70% or more, including, e.g., at least 80%, at least 85%, at least 90%, at least 95%, at least 96%, at least 97%, at least 98%, or higher, identity to SEQ ID NO: 62a). In some embodiments, an RNA composition comprises an RNA that includes the sequence of SEQ ID NO: 63a or a variant thereof (e.g., having at least 70% or more, including, e.g., at least 80%, at least 85%, at least 90%, at least 95%, at least 96%, at least 97%, at least 98%, or higher, identity to SEQ ID NO: 63a).

In some embodiments, the formulations disclosed herein can be used to carry out any of the dosing regimens described in Table C (below).

**Table C: Exemplary Dosing Regimens:**

| | Primary Regimen | | | | Time between the last dose of a Primary regimen and a first dose of Booster Regimen | Booster Regimen | | | |
|---|---|---|---|---|---|---|---|---|---|
| # | Dose 1 (µg RNA) | Dose 2 (µg RNA) | Time Between Doses 1 and 2 | Dose 1 and Dose 2 Formulation | | Dose 1 (µg RNA) | Dose 2 (µg RNA) | Time Between Doses 1 and 2 | Dose 1 and Dose 2 Formulation |
| 1 | 30 | 30 | 2 to 8 weeks | PBS | At least 2 months | 30 | N/A¹ | N/A | PBS |
| 2 | 30 | 30 | 2 to 8 weeks | PBS | At least 3 months | 30 | N/A¹ | N/A | PBS |
| 3 | 30 | 30 | 2 to 8 weeks | PBS | 6 to 12 months | 30 | N/A¹ | N/A | PBS |
| 4 | 30 | 30 | 2 to 8 weeks | PBS or Tris | 4 to 12 months | 15 | N/A¹ | N/A | PBS or Tris |
| 5 | 30 | 30 | 2 to 8 weeks | PBS or Tris | 4 to 12 months | 10 | N/A¹ | N/A | PBS or Tris |
| 6 | 30 | 30 | 2 to 8 weeks | PBS or Tris | 4 to 12 months | 30 | 30 | 4 to 12 months | PBS or Tris |
| 7 | 30 | 30 | 2 to 8 weeks | PBS or Tris | 4 to 12 months | 30 | 15 | 4 to 12 months | PBS or Tris |
| 8 | 30 | 30 | 2 to 8 weeks | PBS or Tris | 4 to 12 months | 30 | 10 | 4 to 12 months | PBS or Tris |
| 9 | 30 | 30 | 2 to 8 weeks | PBS or Tris | 4 to 12 months | 30 | 60 | 4 to 12 months | PBS or Tris |
| 10 | 30 | 30 | 2 to 8 weeks | PBS or Tris | 4 to 12 months | 30 | >30 to < 60 | 4 to 12 months | PBS or Tris |
| 11 | 30 | 30 | 2 to 8 weeks | PBS or Tris | 4 to 12 months | 30 | 50 | 4 to 12 months | PBS or Tris |
| 12 | 30 | 30 | 2 to 8 weeks | PBS | At least 6 months | 30 | N/A¹ | N/A | PBS |
| 13 | 30 | 30 | ^{∼}21 days | PBS | At least 2 months | 30 | N/A¹ | N/A | PBS |
| 14 | 30 | 30 | ^{∼}21 days | PBS | At least 3 months | 30 | N/A¹ | N/A | PBS |
| 15 | 30 | 30 | ^{∼}21 days | PBS | 6 to 12 months | 30 | N/A¹ | N/A | PBS |
| 16 | 30 | 30 | ^{∼}21 days | PBS | At least 6 months | 30 | N/A¹ | N/A | PBS |
| 17 | 30 | 30 | 21 days | PBS | At least 6 months | 15 | 15 | ^{∼}21 days | PBS |
| 18 | 30 | 30 | 21 days | PBS | At least 6 months | 15 | 15 | ^{∼}21 days | PBS |
| | | | | | | | | | |
| 19 | 30 | 30 | 2 to 8 weeks | PBS | At least 2 months | 30 | N/A¹ | N/A | Tris |
| 20 | 30 | 30 | 2 to 8 weeks | PBS | At least 3 months | 30 | N/A¹ | N/A | Tris |
| 21 | 30 | 30 | 2 to 8 weeks | PBS | 6 to 12 months | 30 | N/A¹ | N/A | Tris |
| 22 | 30 | 30 | 2 to 8 weeks | PBS | At least 6 months | 30 | N/A¹ | N/A | Tris |
| 23 | 30 | 30 | ^{∼}21 days | PBS | At least 2 months | 30 | N/A¹ | N/A | Tris |
| 24 | 30 | 30 | ^{∼}21 days | PBS | At least 3 months | 30 | N/A¹ | N/A | Tris |
| 25 | 30 | 30 | ^{∼}21 days | PBS | 6 to 12 months | 30 | N/A¹ | N/A | Tris |
| 26 | 30 | 30 | ^{∼}21 days | PBS | At least 6 months | 30 | N/A¹ | N/A | Tris |
| 27 | 30 | 30 | 21 days | PBS | At least 6 months | 15 | 15 | ^{∼}21 days | Tris |
| 28 | 30 | 30 | 21 days | PBS | At least 6 months | 15 | 15 | ^{∼}21 days | Tris |
| | | | | | | | | | |
| 29 | 30 | 30 | 2 to 8 weeks | Tris | At least 2 months | 30 | N/A¹ | N/A | Tris |
| 30 | 30 | 30 | 2 to 8 weeks | Tris | At least 3 months | 30 | N/A¹ | N/A | Tris |
| 31 | 30 | 30 | 2 to 8 weeks | Tris | 6 to 12 months | 30 | N/A¹ | N/A | Tris |
| 32 | 30 | 30 | 2 to 8 weeks | Tris | At least 6 months | 30 | N/A¹ | N/A | Tris |
| 33 | 30 | 30 | ^{∼}21 days | Tris | At least 2 months | 30 | N/A¹ | N/A | Tris |
| 34 | 30 | 30 | ^{∼}21 days | Tris | At least 3 months | 30 | N/A¹ | N/A | Tris |
| 35 | 30 | 30 | ^{∼}21 days | Tris | 6 to 12 months | 30 | N/A¹ | N/A | Tris |
| 36 | 30 | 30 | ^{∼}21 days | Tris | At least 6 months | 30 | N/A¹ | N/A | Tris |
| 37 | 30 | 30 | 21 days | Tris | At least 6 months | 15 | 15 | ^{∼}21 days | Tris |
| 38 | 30 | 30 | 21 days | Tris | At least 6 months | 15 | 15 | ^{∼}21 days | Tris |
| | | | | | | | | | |
| 39 | 10 | 10 | 2 to 8 weeks | Tris | At least 2 months | 10 | N/A¹ | N/A | Tris |
| 40 | 10 | 10 | 2 to 8 weeks | Tris | At least 3 months | 10 | N/A¹ | N/A | Tris |
| 41 | 10 | 10 | 2 to 8 weeks | Tris | 6 to 12 months | 10 | N/A¹ | N/A | Tris |
| 42 | 10 | 10 | 2 to 8 weeks | Tris | At least 6 months | 10 | N/A¹ | N/A | Tris |
| 43 | 10 | 10 | ^{∼}21 days | Tris | At least 2 months | 10 | N/A¹ | N/A | Tris |
| 44 | 10 | 10 | ^{∼}21 days | Tris | At least 3 months | 10 | N/A¹ | N/A | Tris |
| 45 | 10 | 10 | ^{∼}21 days | Tris | 6 to 12 months | 10 | N/A¹ | N/A | Tris |
| 46 | 10 | 10 | ^{∼}21 days | Tris | At least 6 months | 10 | N/A¹ | N/A | Tris |
| | | | | | | | | | |
| 47 | 3 | 3 | 2 to 8 weeks | Tris | At least 2 months | 3 | N/A¹ | N/A | Tris |
| 48 | 3 | 3 | 2 to 8 weeks | Tris | At least 3 months | 3 | N/A¹ | N/A | Tris |
| 49 | 3 | 3 | 2 to 8 weeks | Tris | 6 to 12 months | 3 | N/A¹ | N/A | Tris |
| 50 | 3 | 3 | 2 to 8 weeks | Tris | At least 6 months | 3 | N/A¹ | N/A | Tris |
| 51 | 3 | 3 | ^{∼}21 days | Tris | At least 2 months | 3 | N/A¹ | N/A | Tris |
| 52 | 3 | 3 | ^{∼}21 days | Tris | At least 3 months | 3 | N/A¹ | N/A | Tris |
| 53 | 3 | 3 | ^{∼}21 days | Tris | 6 to 12 months | 3 | N/A¹ | N/A | Tris |
| 54 | 3 | 3 | ^{∼}21 days | Tris | At least 6 months | 3 | N/A¹ | N/A | Tris |

| | | | | | | | | | |
|---|---|---|---|---|---|---|---|---|---|
| ¹N/A refers to no dose necessary. | | | | | | | | | |

In some embodiments of certain exemplary dosing regimens as described in Table C above, an RNA composition described herein (e.g., comprising RNA encoding a variant described herein) is given in a first dose of a primary regimen. In some embodiments of certain exemplary dosing regimens as described in Table C above, an RNA composition described herein (e.g., comprising RNA encoding a variant described herein) is given in a second dose of a primary regimen. In some embodiments of certain exemplary dosing regimens as described in Table C above, an RNA composition described herein (e.g., comprising RNA encoding a variant described herein) is given in a first dose and a second dose of a primary regimen. In some embodiments of certain exemplary dosing regimens as described in Table C above, an RNA composition described herein (e.g., comprising RNA encoding a variant described herein) is given in a first dose of a booster regimen. In some embodiments of certain exemplary dosing regimens as described in Table C above, an RNA composition described herein (e.g., comprising RNA encoding a variant described herein) is given in a second dose of a booster regimen. In some embodiments of certain exemplary dosing regimens as described in Table C above, an RNA composition described herein (e.g., comprising RNA encoding a variant described herein) is given in a first dose and a second dose of a booster regimen. In some embodiments of certain exemplary dosing regimens as described in Table C above, an RNA composition described herein (e.g., comprising RNA encoding a variant described herein) is given in a first dose and a second dose of a primary regimen and also in at least one dose of a booster regimen. In some embodiments of certain exemplary dosing regimens as described in Table C above, an RNA composition described herein (e.g., comprising RNA encoding a variant described herein) is given in at least one dose (including, e.g., at least two doses) of a booster regimen and BNT162b2 is given in a primary regimen. In some embodiments of certain exemplary dosing regimens as described in Table C above, an RNA composition described herein (e.g., comprising RNA encoding a variant described herein) is given in a second dose of a booster regimen and BNT162b2 is given in a primary regimen and in a first dose of a booster regimen. In some embodiments, an RNA composition described herein (e.g., comprising RNA encoding a variant described herein) comprises an RNA encoding a polypeptide as set forth in SEQ ID NO: 49 or an immunogenic fragment thereof, or a variant thereof (e.g., having at least 70% or more, including, e.g., at least 80%, at least 85%, at least 90%, at least 95%, at least 96%, at least 97%, at least 98%, or higher, identity to SEQ ID NO: 49). In some embodiments, an RNA composition described herein (e.g., comprising RNA encoding a variant described herein) comprises an RNA that includes the sequence of SEQ ID NO: 50 or a variant thereof (e.g., having at least 70% or more, including, e.g., at least 80%, at least 85%, at least 90%, at least 95%, at least 96%, at least 97%, at least 98%, or higher, identity to SEQ ID NO: 50). In some embodiments, an RNA composition described herein (e.g., comprising RNA encoding a variant described herein) comprises an RNA that includes the sequence of SEQ ID NO: 51 or a variant thereof (e.g., having at least 70% or more, including, e.g., at least 80%, at least 85%, at least 90%, at least 95%, at least 96%, at least 97%, at least 98%, or higher, identity to SEQ ID NO: 51).

In some embodiments, an RNA composition described herein comprises an RNA encoding a polypeptide as set forth in SEQ ID NO: 55 or an immunogenic fragment thereof, or a variant thereof (e.g., having at least 70% or more, including, e.g., at least 80%, at least 85%, at least 90%, at least 95%, at least 96%, at least 97%, at least 98%, or higher, identity to SEQ ID NO: 55). In some embodiments, an RNA composition comprises an RNA that includes the sequence of SEQ ID NO: 56 or a variant thereof (e.g., having at least 70% or more, including, e.g., at least 80%, at least 85%, at least 90%, at least 95%, at least 96%, at least 97%, at least 98%, or higher, identity to SEQ ID NO: 56). In some embodiments, an RNA composition comprises an RNA that includes the sequence of SEQ ID NO: 57 or a variant thereof (e.g., having at least 70% or more, including, e.g., at least 80%, at least 85%, at least 90%, at least 95%, at least 96%, at least 97%, at least 98%, or higher, identity to SEQ ID NO: 57).

In some embodiments, an RNA composition described herein comprises an RNA encoding a polypeptide as set forth in SEQ ID NO: 58 or an immunogenic fragment thereof, or a variant thereof (e.g., having at least 70% or more, including, e.g., at least 80%, at least 85%, at least 90%, at least 95%, at least 96%, at least 97%, at least 98%, or higher, identity to SEQ ID NO: 58). In some embodiments, an RNA composition comprises an RNA that includes the sequence of SEQ ID NO: 59 or a variant thereof (e.g., having at least 70% or more, including, e.g., at least 80%, at least 85%, at least 90%, at least 95%, at least 96%, at least 97%, at least 98%, or higher, identity to SEQ ID NO: 59). In some embodiments, an RNA composition comprises an RNA that includes the sequence of SEQ ID NO: 60 or a variant thereof (e.g., having at least 70% or more, including, e.g., at least 80%, at least 85%, at least 90%, at least 95%, at least 96%, at least 97%, at least 98%, or higher, identity to SEQ ID NO: 60).

In some embodiments, an RNA composition described herein comprises an RNA encoding a polypeptide as set forth in SEQ ID NO: 61 or an immunogenic fragment thereof, or a variant thereof (e.g., having at least 70% or more, including, e.g., at least 80%, at least 85%, at least 90%, at least 95%, at least 96%, at least 97%, at least 98%, or higher, identity to SEQ ID NO: 61). In some embodiments, an RNA composition comprises an RNA that includes the sequence of SEQ ID NO: 62a or a variant thereof (e.g., having at least 70% or more, including, e.g., at least 80%, at least 85%, at least 90%, at least 95%, at least 96%, at least 97%, at least 98%, or higher, identity to SEQ ID NO: 62a). In some embodiments, an RNA composition comprises an RNA that includes the sequence of SEQ ID NO: 63a or a variant thereof (e.g., having at least 70% or more, including, e.g., at least 80%, at least 85%, at least 90%, at least 95%, at least 96%, at least 97%, at least 98%, or higher, identity to SEQ ID NO: 63a).

In some embodiments, such an RNA composition described herein (e.g., comprising RNA encoding a variant described herein) can further comprise RNA encoding a S protein or an immungenic fragment thereof of a different strain (e.g., a Wuhan strain). By way of example, in some embodiments, a second dose of a booster regimen of Regimens #9-11 as described in Table C above can comprise an RNA composition described herein (e.g., comprising RNA encoding a variant described herein such as Omicron, for example, in one embodiment RNA as described in this Example) and a BNT162b2 construct, for example, in 1: 1 weight ratio.

In some embodiments of Regimen #6 as described in Table C above, a first dose and a second dose of a primary regimen and a first dose and a second dose of a booster regimen each comprise an RNA composition described herein (e.g., comprising RNA encoding a variant described herein such as Omicron, for example, in one embodiment RNA as described in this Example). In some such embodiments, a second dose of a booster regimen may not be necessary.

In some embodiments of Regimen #6 as described in Table C above, a first dose and a second dose of a primary regimen and a first dose and a second dose of a booster regimen each comprise an RNA composition described herein (e.g., comprising RNA encoding a variant described herein such as Omicron, for example, in one embodiment RNA as described in this Example). In some such embodiments, a second dose of a booster regimen may not be necessary.

In some embodiments of Regimen #6 as described in Table C above, a first dose and a second dose of a primary regimen each comprise a BNT162b2 construct, and a first dose and a second dose of a booster regimen each comprise an RNA composition described herein (e.g., comprising RNA encoding a variant described herein such as Omicron, for example, in one embodiment RNA as described in this Example). In some such embodiments, a second dose of a booster regimen may not be necessary.

In some embodiments of Regimen #6 as described in Table C above, a first dose and a second dose of a primary regimen and a first dose of a booster regimen each comprise a BNT162b2 construct, and a second dose of a booster regimen comprises an RNA composition described herein (e.g., comprising RNA encoding a variant described herein such as Omicron, for example, in one embodiment RNA as described in this Example).

Certain exemplary embodiments below are also within the scope of the present disclosure:
1. A composition or medical preparation comprising RNA comprising a nucleotide sequence encoding a SARS-CoV-2 S protein comprising one or more mutations characteristic of a SARS-CoV-2 variant (e.g., in some embodiments a SARS-CoV-2 Omicron variant), or an immunogenic fragment thereof.
2. The composition or medical preparation of embodiment 1, wherein the immunogenic fragment of the SARS-CoV-2 S protein comprises the S1 subunit of the SARS-CoV-2 S protein, or the receptor binding domain (RBD) of the S1 subunit of the SARS-CoV-2 S protein.
3. The composition or medical preparation of embodiment 1 or 2, wherein the SARS-CoV-2 S protein or immunogenic fragment thereof comprises one or more mutations characteristic of a BA.1, BA.2, BA.4/5, XBB, XBB.1, BQ.1.1, or a BA.4.6/BF.7 Omicron variant or sublineages thereof.
4. The composition or medical preparation of embodiment 3, wherein the SARS-CoV-2 S protein or immunogenic fragment thereof comprises one or more mutations characteristic of a BA.4/5 variant, wherein the one or more mutations are selected from the group consisting of T19I, Δ24-26, A27S, Δ69/70, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, and N969K.
5. The composition or medical preparation of embodiment 3, wherein the SARS-CoV-2 S protein or immunogenic fragment thereof comprises one or more mutations characteristic of a BA.1 variant, wherein the one or more mutations are selected from the group consisting of A67V, Δ69-70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F.
6. The composition or medical preparation of embodiment 3, wherein the SARS-CoV-2 S protein or immunogenic fragment thereof comprises one or more mutations characteristic of a BA.2 variant, wherein the one or more mutations are selected from the group consisting of T19I, Δ24-26, A27S, G142D, V213G, G339D, S371F, S373P, S375F, T376A, D405N, R408S, K417N, N440K, S477N, T478K, E484A, Q493R, Q498R, N501Y, Y505H, D614G, H655Y, N679K, P681H, N764K, D796Y, Q954H, and N969K.
7. The composition or medical preparation of embodiment 3, wherein the SARS-CoV-2 S protein or immunogenic fragment thereof comprises one or more mutations characteristic of a BA.2.75.1 variant, wherein the one or more mutations are selected from the group consisting of T19I, Δ24-26, A27S, G142D, V213G, G339D, S371F, S373P, S375F, T376A, D405N, R408S, K417N, N440K, S477N, T478K, E484A, Q493R, Q498R, N501Y, Y505H, D614G, H655Y, N679K, P681H, N764K, D796Y, Q954H, and N969K.
8. The composition or medical preparation of embodiment 3, wherein the SARS-CoV-2 S protein or immunogenic fragment thereof comprises one or more mutations characteristic of a BA.4.6/BF.7 variant, wherein the one or more mutations are selected from the group consisting of T19I, Δ24-26, A27S, Δ69/70, G142D, V213G, G339D, R346T, S371F, S373P, S375F, T376A, D405N, R408S, K417N, N440K, L452R, S477N, T478K, E484A, F486V, Q498R, N501Y, Y505H, D614G, H655Y, N658S, N679K, P681H, N764K, D796Y, Q954H, and N969K.
9. The composition or medical preparation of embodiment 3, wherein the SARS-CoV-2 S protein or immunogenic fragment thereof comprises one or more mutations characteristic of an XBB variant, wherein the one or more mutations are selected from the group consisting of T19I, Δ24-26, A27S, V83A, G142D, Δ144, H146Q, Q183E, V213E, G339H, R346T, L368I, S371F, S373P, S375F, T376A, D405N, R408S, K417N, N440K, V445P, G446S, N460K, S477N, T478K, E484A, F486S, F490S, Q493R, Q498R, N501Y, Y505H, D614G, H655Y, N679K, P681H, N764K, D796Y, Q954H, and N969K.
10. The composition or medical preparation of embodiment 3, wherein the SARS-CoV-2 S protein or immunogenic fragment thereof comprises one or more mutations characteristic of an XBB.1 variant, wherein the one or more mutations are selected from the group consisting of T19I, Δ24-26, A27S, V83A, G142D, Δ144, H146Q, Q183E, V213E, G252V, G339H, R346T, L368I, S371F, S373P, S375F, T376A, D405N, R408S, K417N, N440K, V445P, G446S, N460K, S477N, T478K, E484A, F486S, F490S, Q493R, Q498R, N501Y, Y505H, D614G, H655Y, N679K, P681H, N764K, D796Y, Q954H, and N969K.
11. The composition or medical preparation of embodiment 3, wherein the SARS-CoV-2 S protein or immunogenic fragment thereof comprises one or more mutations characteristic of a BQ.1.1 variant, wherein the one or more mutations are selected from the group consisting of T19I, Δ24-26, A27S, Δ69/70, G142D, V213G, G339D, R346T, S371F, S373P, S375F, T376A, D405N, R408S, K417N, N440K, K444T, L452R, N460K, S477N, T478K, E484A, F486V, Q498R, N501Y, Y505H, D614G, H655Y, N679K, P681H, N764K, D796Y, Q954H, N969K.
12. The composition or medical preparation of any one of embodiments 1-11, wherein the SARS-CoV-2 S protein comprising one or more mutations characteristic of an Omicron variant, or an immunogenic fragment thereof is encoded by a sequence that is codon-optimized (e.g., codon-optimized for expression in human cells) and/or which has a G/C content that is increased compared to a wild type coding sequence.
13. The composition or medical preparation of embodiment 3 or 4, wherein the SARS-CoV-2 S protein comprises one or more mutations characteristic of an Omicron BA.4/5 variant and wherein:
   a) the SARS-CoV-2 S protein comprises an amino acid sequence having at least 99%, 98%, 97%, 96%, 95%, 90%, 85%, or 80% identity to SEQ ID NO: 69, or an immunogenic fragment thereof; and/or
   b) the RNA encoding the SARS-CoV-2 S protein comprises a nucleotide sequence having at least 99%, 98%, 97%, 96%, 95%, 90%, 85%, or 80% identity to SEQ ID NO: 70; and/or at least 99%, 98%, 97%, 96%, 95%, 90%, 85%, or 80% identity to SEQ ID NO: 71.
14. The composition or medical preparation of embodiment 3 or 5, wherein the SARS-CoV-2 S protein comprises one or more mutations characteristic of an Omicron BA.1 variant and wherein:
   a) the SARS-CoV-2 S protein comprises an amino acid sequence having at least 99%, 98%, 97%, 96%, 95%, 90%, 85%, or 80% identity to SEQ ID NO: 49, or an immunogenic fragment thereof; and/or
   b) the RNA encoding the SARS-CoV-2 S protein comprises a nucleotide sequence having at least 99%, 98%, 97%, 96%, 95%, 90%, 85%, or 80% identity to SEQ ID NO: 50 and/or at least 99%, 98%, 97%, 96%, 95%, 90%, 85%, or 80% identity to SEQ ID NO: 51.
15. The composition or medical preparation of embodiment 3 or 6, wherein the SARS-CoV-2 S protein comprises one or more mutations characteristic of an Omicron BA.2 variant and wherein:
   a) the SARS-CoV-2 S protein comprises an amino acid sequence having at least 99%, 98%, 97%, 96%, 95%, 90%, 85%, or 80% identity to SEQ ID NO: 64, or an immunogenic fragment thereof; and/or
   b) the RNA encoding the SARS-CoV-2 S protein comprises a nucleotide sequence having at least 99%, 98%, 97%, 96%, 95%, 90%, 85%, or 80% identity to SEQ ID NO: 65 and/or at least 99%, 98%, 97%, 96%, 95%, 90%, 85%, or 80% identity to SEQ ID NO: 66.
16. The composition or medical preparation of embodiment 3 or 7, wherein the SARS-CoV-2 S protein comprises one or more mutations characteristic of an Omicron BA.2.75.1 variant and wherein:
   a) the SARS-CoV-2 S protein comprises an amino acid sequence having at least 99%, 98%, 97%, 96%, 95%, 90%, 85%, or 80% identity to SEQ ID NO: 80, or an immunogenic fragment thereof; and/or
   b) the RNA encoding the SARS-CoV-2 S protein comprises a nucleotide sequence having at least 99%, 98%, 97%, 96%, 95%, 90%, 85%, or 80% identity to SEQ ID NO: 81 and/or at least 99%, 98%, 97%, 96%, 95%, 90%, 85%, or 80% identity to SEQ ID NO: 83.
17. The composition or medical preparation of embodiment 3 or 8, wherein the SARS-CoV-2 S protein comprises one or more mutations characteristic of an Omicron BA.4.6/BF.7 variant and wherein:
   a) the SARS-CoV-2 S protein comprises an amino acid sequence having at least 99%, 98%, 97%, 96%, 95%, 90%, 85%, or 80% identity to SEQ ID NO: 90, or an immunogenic fragment thereof; and/or
   b) the RNA encoding the SARS-CoV-2 S protein comprises a nucleotide sequence having at least 99%, 98%, 97%, 96%, 95%, 90%, 85%, or 80% identity to SEQ ID NO: 91 and/or at least 99%, 98%, 97%, 96%, 95%, 90%, 85%, or 80% identity to SEQ ID NO: 92.
18. The composition or medical preparation of embodiment 3 or 9, wherein the SARS-CoV-2 S protein comprises one or more mutations characteristic of an Omicron XBB variant and wherein:
   a) the SARS-CoV-2 S protein comprises an amino acid sequence having at least 99%, 98%, 97%, 96%, 95%, 90%, 85%, or 80% identity to SEQ ID NO: 95, or an immunogenic fragment thereof; and/or
   b) the RNA encoding the SARS-CoV-2 S protein comprises a nucleotide sequence having at least 99%, 98%, 97%, 96%, 95%, 90%, 85%, or 80% identity to SEQ ID NO: 96; and/or at least 99%, 98%, 97%, 96%, 95%, 90%, 85%, or 80% identity to SEQ ID NO: 98.
19. The composition or medical preparation of embodiment 3 or 11, wherein the SARS-CoV-2 S protein comprises one or more mutations characteristic of an Omicron BQ.1.1 variant and wherein:
   a) the SARS-CoV-2 S protein comprises an amino acid sequence having at least 99%, 98%, 97%, 96%, 95%, 90%, 85%, or 80% identity to SEQ ID NO: 100, or an immunogenic fragment thereof; and/or
   b) the RNA encoding the SARS-CoV-2 S protein comprises a nucleotide sequence having at least 99%, 98%, 97%, 96%, 95%, 90%, 85%, or 80% identity to SEQ ID NO: 101 and/or at least 99%, 98%, 97%, 96%, 95%, 90%, 85%, or 80% identity to SEQ ID NO: 102.
20. The composition or medical preparation of any one of embodiments 1-19, wherein the SARS-CoV-2 S protein comprises one or more mutations that improves expression, stability, and/or immunogenicity.
21. The composition or medical preparation of embodiment 20, wherein the SARS-CoV-2 S protein comprises one or more mutations that stabilizes the prefusion conformation.
22. The composition or medical preparation of embodiment 20, wherein the SARS-CoV-2 S protein comprises proline mutations at positions corresponding to residues 986 and 987 of SEQ ID NO: 1.
23. The composition or medical preparation of embodiment 21 or 22, wherein the SARS-CoV-2 S protein comprises one or more proline residues at positions corresponding to positions 817, 892, 899, and/or 942 of SEQ ID NO: 1.
24. The composition or medical preparation of any one of embodiments 20-23, wherein the SARS-CoV-2 S protein comprises a mutation that prevents furin cleavage.
25. The composition or medical preparation of embodiment 24, wherein the SARS-CoV-2 S protein comprises a mutation at a location corresponding to residues 682-685 of SEQ ID NO: 1 that prevents cleavage by a furin protease (e.g., a GSAS mutation).
26. The composition or medical preparation of any one of embodiments 20-25, wherein the SARS-CoV-2 S protein comprises one or more mutations that decreases S protein shedding (e.g., an aspartate to glycine mutation at a position corresponding to residue 614 of SEQ ID NO: 1).
27. The composition or medical preparation of any one of embodiments 1-26, wherein the RNA comprises a modified nucleoside in place of uridine.
28. The composition or medical preparation of embodiment 27, wherein the RNA comprises a modified nucleoside in place of each uridine.
29. The composition or medical preparation of embodiment 27 or 28, wherein the modified nucleoside is selected from pseudouridine (ψ), N1-methyl-pseudouridine (m1ψ), and 5-methyl-uridine (m5U).
30. The composition or medical preparation of embodiment 29, wherein the modified nucleoside is N1-methyl-pseudouridine (m1ψ).
31. The composition or medical preparation of any one of embodiments 1-30, wherein the RNA comprises a 5' cap.
32. The composition or medical preparation of embodiment 31, wherein the 5' cap is or comprises a cap1 structure.
33. The composition or medical preparation of embodiment 32, wherein the RNA comprises a 5'-cap that is or comprises m₂^{7,3'-}OGppp(m₁^{2'-O})ApG.
34. The composition or medical preparation of any one of embodiments 1-33, wherein the composition comprises a poly(A) sequence.
35. The composition or medical preparation of embodiment 34, wherein the poly(A) sequence comprises at least 100 A nucleotides.
36. The composition or medical preparation of embodiment 34 or 35, wherein the poly(A) sequence is an interrupted sequence of A nucleotides.
37. The composition or medical preparation of embodiment 36, wherein the poly(A) sequence comprises 30 adenine nucleotides followed by 70 adenine nucleotides, wherein the 30 adenine nucleotides and 70 adenine nucleotides are separated by a linker sequence.
38. The composition or medical preparation of embodiment 37, wherein the poly(A) sequence comprises or consists of the nucleotide sequence of SEQ ID NO: 14, or a nucleotide sequence that is at least 99%, 98%, 97%, 96%, 95%, 90%, 85%, or 80% identical to the nucleotide sequence of SEQ ID NO: 14.
39. The composition or medical preparation of any one of embodiments 1-38, wherein the RNA comprises a 5'-UTR that is or comprises a modified human alpha-globin 5'-UTR.
40. The composition or medical preparation of embodiment 39, wherein the 5' UTR comprises the nucleotide sequence of SEQ ID NO: 12, or a nucleotide sequence that is at least 99%, 98%, 97%, 96%, 95%, 90%, 85%, or 80% identical to the nucleotide sequence of SEQ ID NO: 12.
41. The composition or medical preparation of any one of embodiments 1-40, wherein the RNA comprises a 3'-UTR that is or comprises a first sequence from the amino terminal enhancer of split (AES) messenger RNA and a second sequence from the mitochondrial encoded 12S ribosomal RNA.
42. The composition or medical preparation of embodiment 41, wherein the RNA comprises a 3' UTR comprising the nucleotide sequence of SEQ ID NO: 13, or a nucleotide sequence having at least 99%, 98%, 97%, 96%, 95%, 90%, 85%, or 80% identity to the nucleotide sequence of SEQ ID NO: 13.
43. The composition or medical preparation of any one of embodiments 1-42, wherein the RNA is formulated or is to be formulated as particles.
44. The composition or medical preparation of embodiment 43, wherein the particles are lipid nanoparticles (LNP) or lipoplex (LPX) particles.
45. The composition or medical preparation of embodiment 44, wherein the LNPs comprise a cationically ionizable lipid, a neutral lipid, a sterol and a polymer-lipid conjugate.
46. The composition or medical preparation of embodiment 45, wherein the neutral lipid is present in a concentration ranging from about 5 to about 15 mol percent of the total lipids.
47. The composition or medical preparation of embodiment 45 or 46, wherein the cationically ionizable lipid is present in a concentration ranging from about 40 to about 50 mol percent of the total lipids.
48. The composition or medical preparation of any one of embodiments 45-47, wherein the sterol is present in a concentration ranging from about 30 to about 50 mol percent of the total lipids.
49. The composition or medical preparation of any one of embodiments 45-48, wherein the polymer-lipid conjugate is present in a concentration ranging from about 1 to about 10 mol percent of the total lipids.
50. The composition or medical preparation of any one of embodiments 45-49, wherein the lipid nanoparticles comprise from about 40 to about 50 mol percent of the cationically ionizable lipid; from about 5 to about 15 mol percent of the neutral lipid; from about 35 to about 45 mol percent of the sterol; and from about 1 to about 10 mol percent of the polymer conjugated-lipid.
51. The composition or medical preparation of embodiment 44, wherein the RNA lipoplex particles are obtainable by mixing the RNA with liposomes.
52. A composition or medical preparation comprising: a lipid nanoparticle (LNP) comprising an RNA, wherein the RNA comprises a nucleotide sequence that (a) encodes a SARS-CoV-2 S protein comprising one or more mutations characteristic of an Omicron BA.1 variant and comprising an amino acid sequence of SEQ ID NO: 49 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 49 and/or (b) comprises the nucleotide sequence of SEQ ID NO: 50 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 50, and wherein the RNA comprises:
   (a) modified uridines;
   (b) a 5' cap; and
      wherein the LNP comprises a cationically ionizable lipid, a neutral lipid, a sterol and a polymer-lipid conjugate.
53. The composition or medical preparation of embodiment 52, wherein the nucleotide sequence includes modified uridines in place of all uridines.
54. The composition or medical preparation of embodiment 52 or 53, wherein the modified uridines are each N1-methyl-pseudouridine.
55. The composition or medical preparation of any one of embodiments 52-54, wherein the RNA further comprises at least one, at least two, or all of the following features:
   a 5' untranslated region (UTR) comprising SEQ ID NO: 12;
   a 3' untranslated region (UTR) comprising SEQ ID NO: 13; and
   a poly-A sequence of at least 100 A nucleotides.
56. The composition or medical preparation of embodiment 55, wherein the poly-A sequence comprises 30 adenine nucleotides followed by 70 adenine nucleotides, wherein the 30 adenine nucleotides and 70 adenine nucleotides are separated by a linker sequence.
57. The composition or medical preparation of embodiment 56, wherein the poly-A sequence comprises SEQ ID NO: 14.
58. The composition or medical preparation of any one of embodiments 52-57, wherein the RNA comprises SEQ ID NO: 51.
59. A composition or medical preparation comprising a lipid nanoparticle (LNP) comprising an RNA, wherein the RNA comprises a nucleotide sequence (a) that encodes a SARS-CoV-2 S protein comprising one or more mutations characteristic of a Beta variant and comprising the amino acid sequence of SEQ ID NO: 55 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 55 and/or (b) comprises the nucleotide sequence of SEQ ID NO: 56 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 56, and wherein the RNA comprises:
   (a) modified uridines;
   (b) a 5' cap; and
      wherein the LNP comprises a cationically ionizable lipid, a neutral lipid, a sterol and a polymer-lipid conjugate.
60. The composition or medical preparation of embodiment 59, wherein the nucleotide sequence includes modified uridines in place of all uridines.
61. The composition or medical preparation of embodiment 59 or 60, wherein the modified uridines are each N1-methyl-pseudouridine.
62. The composition or medical preparation of any one of embodiments 59 to 61, wherein the RNA further comprises at least one, at least two, or all of the following features:
   a 5' untranslated region (UTR) comprising SEQ ID NO: 12;
   a 3' untranslated region (UTR) comprising SEQ ID NO: 13; and
   a poly-A sequence of at least 100 A nucleotides.
63. The composition or medical preparation of embodiment 62, wherein the poly-A sequence comprises 30 adenine nucleotides followed by 70 adenine nucleotides, wherein the 30 adenine nucleotides and 70 adenine nucleotides are separated by a linker sequence.
64. The composition or medical preparation of embodiment 63, wherein the poly-A sequence comprises SEQ ID NO: 14.
65. The composition or medical preparation of any one of embodiments 59-64, wherein the RNA comprises SEQ ID NO: 57.
66. A composition or medical preparation comprising a a lipid nanoparticle (LNP) comprising an RNA, wherein the RNA comprises a nucleotide sequence that (a) encodes a SARS-CoV-2 S protein comprising one or more mutations characteristic of an Alpha variant and comprising the polypeptide of SEQ ID NO: 58 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 58 and/or (b) comprises the nucleotide sequence of SEQ ID NO: 59 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 59, and wherein the RNA comprises:
   (a) modified uridines;
   (b) a 5' cap; and
      wherein the LNP comprises a cationically ionizable lipid, a neutral lipid, a sterol and a polymer-lipid conjugate.
67. The composition or medical preparation of embodiment 66, wherein the nucleotide sequence includes modified uridines in place of all uridines.
68. The composition or medical preparation of embodiment 66 or 67, wherein the modified uridines are each N1-methyl-pseudouridine.
69. The composition or medical preparation of any one of embodiments 66-68, wherein the RNA further comprises at least one, at least two, or all of the following features:
   a 5' untranslated region (UTR) comprising SEQ ID NO: 12;
   a 3' untranslated region (UTR) comprising SEQ ID NO: 13; and
   a poly-A sequence of at least 100 A nucleotides.
70. The composition or medical preparation of embodiment 69, wherein the poly-A sequence comprises 30 adenine nucleotides followed by 70 adenine nucleotides, wherein the 30 adenine nucleotides and 70 adenine nucleotides are separated by a linker sequence.
71. The composition or medical preparation of embodiment 70, wherein the poly-A sequence comprises SEQ ID NO: 14.
72. The composition or medical preparation of any one of embodiments 66-71, wherein the RNA comprises SEQ ID NO: 60.
73. A composition or medical preparation comprising a lipid nanoparticle (LNP) comprising an RNA, wherein the RNA comprises a nucleotide sequence that (a) encodes a SARS-CoV-2 S protein comprising one or more mutations characteristic of a Delta variant and comprises an amino acid sequence of of SEQ ID NO: 61 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 61 and/or (b) comprises the nucleotide sequence of SEQ ID NO: 62a or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 62a, and wherein the RNA comprises:
   (a) modified uridines;
   (b) a 5' cap; and
      wherein the LNP comprises a cationically ionizable lipid, a neutral lipid, a sterol and a polymer-lipid conjugate.
74. The composition or medical preparation of embodiment 73, wherein the nucleotide sequence includes modified uridines in place of all uridines.
75. The composition or medical preparation of embodiment 73 or 74, wherein the modified uridines are each N1-methyl-pseudouridine.
76. The composition or medical preparation of any one of embodiments 73-75, wherein the RNA further comprises at least one, at least two, or all of the following features:
   a 5' untranslated region (UTR) comprising SEQ ID NO: 12;
   a 3' untranslated region (UTR) comprising SEQ ID NO: 13; and a poly-A sequence of at least 100 A nucleotides.
77. The composition or medical preparation of embodiment 76, wherein the poly-A sequence comprises 30 adenine nucleotides followed by 70 adenine nucleotides, wherein the 30 adenine nucleotides and 70 adenine nucleotides are separated by a linker sequence.
78. The composition or medical preparation of embodiment 77, wherein the poly-A sequence comprises SEQ ID NO: 14.
79. The composition or medical preparation of any one of embodiments 73-78, wherein the RNA comprises SEQ ID NO: 63a.
80. A composition or medical preparation comprising a lipid nanoparticle (LNP) comprising an RNA, wherein the RNA comprises a nucleotide sequence that (a) encodes a SARS-CoV-2 S protein comprising one or more mutations characteristic of a BA.2 Omicron variant and encodes a polypeptide comprising an amino acid sequence of SEQ ID NO: 64 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 64 and/or (b) comprises the nucleotide sequence of SEQ ID NO: 65 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 65, and wherein the RNA comprises:
   (a) modified uridines;
   (b) a 5' cap; and
      wherein the LNP comprises a cationically ionizable lipid, a neutral lipid, a sterol and a polymer-lipid conjugate.
81. The composition or medical preparation of embodiment 80, wherein the nucleotide sequence includes modified uridines in place of all uridines.
82. The composition or medical preparation of embodiment 80 or 81, wherein the modified uridines are each N1-methyl-pseudouridine.
83. The composition or medical preparation of any one of embodiments 80-82, wherein the RNA further comprises at least one, at least two, or all of the following features:
   a 5' untranslated region (UTR) comprising SEQ ID NO: 12;
   a 3' untranslated region (UTR) comprising SEQ ID NO: 13; and
   a poly-A sequence of at least 100 A nucleotides.
84. The composition or medical preparation of embodiment 83, wherein the poly-A sequence comprises 30 adenine nucleotides followed by 70 adenine nucleotides, wherein the 30 adenine nucleotides and 70 adenine nucleotides are separated by a linker sequence.
85. The composition or medical preparation of embodiment 84, wherein the poly-A sequence comprises SEQ ID NO: 14.
86. The composition or medical preparation of any one of embodiments 80-85, wherein the RNA comprises SEQ ID NO: 67.
87. A composition or medical preparation comprising a lipid nanoparticle (LNP) comprising an RNA, wherein the RNA comprises a nucleotide sequence that (a) encodes a SARS-CoV-2 S protein that comprises one or more mutations characteristic of a BA.2.75 Omicron variant and comprises an amino acid sequence of SEQ ID NO 80 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 80 and/or (b) comprises the nucleotide sequence of SEQ ID NO: 81 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 81, and wherein the RNA comprises:
   (a) modified uridines;
   (b) a 5' cap; and
      wherein the LNP comprises a cationically ionizable lipid, a neutral lipid, a sterol and a polymer-lipid conjugate.
88. The composition or medical preparation of embodiment 87, wherein the nucleotide sequence includes modified uridines in place of all uridines.
89. The composition or medical preparation of embodiment 87 or 88, wherein the modified uridines are each N1-methyl-pseudouridine.
90. The composition or medical preparation of any one of embodiments 87-89, wherein the RNA further comprises at least one, at least two, or all of the following features:
   a 5' untranslated region (UTR) comprising SEQ ID NO: 12;
   a 3' untranslated region (UTR) comprising SEQ ID NO: 13; and a poly-A sequence of at least 100 A nucleotides.
91. The composition or medical preparation of embodiment 90, wherein the poly-A sequence comprises 30 adenine nucleotides followed by 70 adenine nucleotides, wherein the 30 adenine nucleotides and 70 adenine nucleotides are separated by a linker sequence.
92. The composition or medical preparation of embodiment 91, wherein the poly-A sequence comprises SEQ ID NO: 14.
93. The composition or medical preparation of any one of embodiments 90-92, wherein the RNA comprises SEQ ID NO: 83.
94. A composition or medical preparation comprising a lipid nanoparticle (LNP) comprising an RNA, wherein the RNA comprises a nucleotide sequence that encodes a SARS-CoV-2 S protein comprising one or more mutations characteristic of a BA.2.75.2 Omicron variant and comprises an amino acid sequence of SEQ ID NO 85 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 85 and/or (b) comprises the nucleotide sequence of 86 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 86, and wherein the RNA comprises:
   (a) modified uridines;
   (b) a 5' cap; and
      wherein the LNP comprises a cationically ionizable lipid, a neutral lipid, a sterol and a polymer-lipid conjugate.
95. The composition or medical preparation of embodiment 94, wherein the nucleotide sequence includes modified uridines in place of all uridines.
96. The composition or medical preparation of embodiment 94 or 95, wherein the modified uridines are each N1-methyl-pseudouridine.
97. The composition or medical preparation of any one of embodiments 94-96, wherein the RNA further comprises at least one, at least two, or all of the following features:
   a 5' untranslated region (UTR) comprising SEQ ID NO: 12;
   a 3' untranslated region (UTR) comprising SEQ ID NO: 13; and
   a poly-A sequence of at least 100 A nucleotides.
98. The composition or medical preparation of embodiment 97, wherein the poly-A sequence comprises 30 adenine nucleotides followed by 70 adenine nucleotides, wherein the 30 adenine nucleotides and 70 adenine nucleotides are separated by a linker sequence.
99. The composition or medical preparation of embodiment 98, wherein the poly-A sequence comprises SEQ ID NO: 14.
100. The composition or medical preparation of any one of embodiments 94-99, wherein the RNA comprises SEQ ID NO: 88.
101. A composition or medical preparation comprising a lipid nanoparticle (LNP) comprising an RNA, wherein the RNA comprises a nucleotide sequence that (a) encodes a SARS-CoV-2 S protein comprising one or more mutations characteristic of an Omicron BA.4/5 varaint and comprises an amino acid sequence of SEQ ID NO: 69 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 69 and/or (b) comprises the nucleotide sequence of SEQ ID NO: 70 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 70, and wherein the RNA comprises:
   (a) modified uridines;
   (b) a 5' cap; and
      wherein the LNP comprises a cationically ionizable lipid, a neutral lipid, a sterol and a polymer-lipid conjugate.
102. The composition or medical preparation of embodiment 101, wherein the nucleotide sequence includes modified uridines in place of all uridines.
103. The composition or medical preparation of embodiment 101 or 102, wherein the modified uridines are each N1-methyl-pseudouridine.
104. The composition or medical preparation of any one of embodiments 101-103, wherein the RNA further comprises at least one, at least two, or all of the following features:
   a 5' untranslated region (UTR) comprising SEQ ID NO: 12;
   a 3' untranslated region (UTR) comprising SEQ ID NO: 13; and a poly-A sequence of at least 100 A nucleotides.
105. The composition or medical preparation of embodiment 104, wherein the poly-A sequence comprises 30 adenine nucleotides followed by 70 adenine nucleotides, wherein the 30 adenine nucleotides and 70 adenine nucleotides are separated by a linker sequence.
106. The composition or medical preparation of embodiment 105, wherein the poly-A sequence comprises SEQ ID NO: 14.
107. The composition or medical preparation of any one of embodiments 101-106, wherein the RNA comprises SEQ ID NO: 72.
108. A composition or medical preparation comprising a lipid nanoparticle (LNP) comprising an RNA, wherein the RNA comprises a nucleotide sequence that (a) encodes a SARS-CoV-2 S protein comprising one or more mutations that are characteristic of an Omicron BA.4.6/BF.7 variant and comprising an amino acid sequence of SEQ ID NO: 90 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 90 and (b) comprises the nucleotide sequence of SEQ ID NO: 91 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 91, and wherein the RNA comprises:
   (a) modified uridines;
   (b) a 5' cap; and
      wherein the LNP comprises a cationically ionizable lipid, a neutral lipid, a sterol and a polymer-lipid conjugate.
109. The composition or medical preparation of embodiment 108, wherein the nucleotide sequence includes modified uridines in place of all uridines.
110. The composition or medical preparation of embodiment 108 or 109, wherein the modified uridines are each N1-methyl-pseudouridine.
111. The composition or medical preparation of any one of embodiments 108-110, wherein the RNA further comprises at least one, at least two, or all of the following features:
   a 5' untranslated region (UTR) comprising SEQ ID NO: 12;
   a 3' untranslated region (UTR) comprising SEQ ID NO: 13; and
   a poly-A sequence of at least 100 A nucleotides.
112. The composition or medical preparation of embodiment 111, wherein the poly-A sequence comprises 30 adenine nucleotides followed by 70 adenine nucleotides, wherein the 30 adenine nucleotides and 70 adenine nucleotides are separated by a linker sequence.
113. The composition or medical preparation of embodiment 112, wherein the poly-A sequence comprises SEQ ID NO: 14.
114. The composition or medical preparation of any one of embodiments 108-112, wherein the RNA comprises SEQ ID NO: 93.
115. A composition or medical preparation comprising a lipid nanoparticle (LNP) comprising an RNA, wherein the RNA comprises a nucleotide sequence that (a) encodes a SARS-CoV-2 S protein comprising one or more mutations characteristic of an XBB Omicron variant and comprises an amino acid sequence of SEQ ID NO 85 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 95 and/or (b) comprises the nucleotide sequence of SEQ ID NO: 96 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 96, and wherein the RNA comprises:
   (a) modified uridines;
   (b) a 5' cap; and
      wherein the LNP comprises a cationically ionizable lipid, a neutral lipid, a sterol and a polymer-lipid conjugate.
116. The composition or medical preparation of embodiment 115, wherein the nucleotide sequence includes modified uridines in place of all uridines.
117. The composition or medical preparation of embodiment 115 or 116, wherein the modified uridines are each N1-methyl-pseudouridine.
118. The composition or medical preparation of any one of embodiments 115-117, wherein the RNA further comprises at least one, at least two, or all of the following features:
   a 5' untranslated region (UTR) comprising SEQ ID NO: 12;
   a 3' untranslated region (UTR) comprising SEQ ID NO: 13; and
   a poly-A sequence of at least 100 A nucleotides.
119. The composition or medical preparation of embodiment 118, wherein the poly-A sequence comprises 30 adenine nucleotides followed by 70 adenine nucleotides, wherein the 30 adenine nucleotides and 70 adenine nucleotides are separated by a linker sequence.
120. The composition or medical preparation of embodiment 119, wherein the poly-A sequence comprises SEQ ID NO: 14.
121. The composition or medical preparation of any one of embodiments 115-120, wherein the RNA comprises SEQ ID NO: 98.
122. A composition or medical preparation comprising a lipid nanoparticle (LNP) comprising an RNA, wherein the RNA comprises a nucleotide sequence that (a) encodes a SARS-CoV-2 S protein comprising one or more mutations characteristic of a BQ.1.1 Omicron variant and comprising an amino acid sequence of SEQ ID NO 100 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 100 and/or (b) comprises a nucleotide sequence of SEQ ID NO: 101 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 101, and wherein the RNA comprises:
   (a) modified uridines;
   (b) a 5' cap; and
      wherein the LNP comprises a cationically ionizable lipid, a neutral lipid, a sterol and a polymer-lipid conjugate.
123. The composition or medical preparation of embodiment 122, wherein the nucleotide sequence includes modified uridines in place of all uridines.
124. The composition or medical preparation of embodiment 122 or 123, wherein the modified uridines are each N1-methyl-pseudouridine.
125. The composition or medical preparation of any one of embodiments 122-124, wherein the RNA further comprises at least one, at least two, or all of the following features:
   a 5' untranslated region (UTR) comprising SEQ ID NO: 12;
   a 3' untranslated region (UTR) comprising SEQ ID NO: 13; and
   a poly-A sequence of at least 100 A nucleotides.
126. The composition or medical preparation of embodiment 125, wherein the poly-A sequence comprises 30 adenine nucleotides followed by 70 adenine nucleotides, wherein the 30 adenine nucleotides and 70 adenine nucleotides are separated by a linker sequence.
127. The composition or medical preparation of embodiment 126, wherein the poly-A sequence comprises SEQ ID NO: 14.
128. The composition or medical preparation of any one of embodiments 121-127, wherein the RNA comprises SEQ ID NO: 103.
129. The composition or medical preparation of any one of embodiments 1-128, wherein the RNA is present in an amount within a range of about 1 µg to about 100 µg per dose in the composition.
130. The composition or medical preparation of embodiment 129, wherein the RNA is present in an amount within a range of about 1 µg to about 60 µg per dose in the composition.
131. The composition or medical preparation of embodiment 130, wherein the RNA is present in an amount of about 1.5 µg, about 2.5 µg, about 3.0 µg, about 5.0 µg, about 10 µg, about 15 µg, about 30 µg, or about 60 µg per dose in the composition.
132. The composition or medical preparation of any one of embodiments 1-131, further comprising a second RNA comprising a nucleotide sequence encoding a SARS-CoV-2 S protein, or an immunogenic fragment thereof, wherein the SARS-CoV-2 S protein comprises one or more mutations that are characteristic of a second SARS-CoV-2 variant.
133. A composition or medical preparation comprising:
   (a) a first RNA comprising a nucleotide sequence encoding a SARS-CoV-2 S protein of a first strain or variant, or an immunogenic fragment thereof; and
   (b) a second RNA comprising a nucleotide sequence encoding a SARS-CoV-2 S protein of a second variant, or an immunogenic fragment thereof,
      wherein the first variant is different from the second variant, and optionally wherein the first and/or second variant is an Omicron variant.
134. The composition or medical preparation of embodiment 133, wherein the first RNA comprises a nucleotide sequence that encodes a SARS-CoV-2 S protein of a Wuhan strain, or an immungenic fragment thereof, and the second RNA comprises a nucleotide sequence that encodes a SARS-CoV-2 S protein comprising one or more mutations that are characteristic of an Omicron variant, or an immunogenic fragment thereof.
135. The compostion or medical preparation of embodiments 133 or 134, wherein the first RNA comprises a nucleotide sequence that encodes a SARS-CoV-2 S protein of a Wuhan strain, or an immunogenic fragment thereof, and the second RNA comprises a nucleotide sequence that encodes a SARS-CoV-2 S proten comprising one or more mutations that are characteristic of a BA.4/5, BA.1, BA.2, XBB, XBB.1, or BQ.1.1 Omicron variant, or sublineages thereof or an immunogenic fragment thereof.
136. The composition or medical preparation of embodiment 133, wherein the second RNA comprises a nucleotide sequence that encodes a SARS-CoV-2 S protein that is antigenically distinct from the S protein encoded by the first RNA, or an immunogenic fragment thereof.
137. A composition or medical preparation comprising (a) a first RNA comprising a nucleotide sequence encoding a SARS-CoV-2 S protein of a Wuhan strain, an Alpha variant, a Beta variant, a Delta variant, or a BA.1 Omicron variant, or an immunogenic fragment thereof and (b) a second RNA comprising a nucleotide sequence encoding a SARS-CoV-2 S protein comprising one or more mutations that are characteristic of an Omicron variant that is not a BA.1 Omicron variant, or an immunogenic fragment thereof.
138. The composition or medical preparation of any one of embodiments 133-137, wherein the second RNA comprises a nucleotide sequence that encodes a SARS-CoV-2 S protein comprising one or more mutations characteristic of a BA.4/5 Omicron variant, or a variant that has evolved from a BA.4/5 Omicron variant, or an immunogenic fragment thereof.
139. The composition or medical preparation of any one of embodiments 133-135, wherein the first RNA comprises a nucleotide sequence encoding a SARS-CoV-2 S protein of a Wuhan strain, or an immunogenic fragment thereof, and the second RNA comprises a nucleotide sequence that encodes a SARS-CoV-2 S protein comprising one or more mutations that are characteristic of a BA.1 Omicron variant, or an immunogenic fragment thereof.
140. The composition or medical preparation of any one of embodiments 133-138, wherein the first RNA comprises a nucleotide sequence that encodes a SARS-CoV-2 S protein of a Wuhan strain, or an immunogenic fragment thereof, and the second RNA comprises a nucleotide sequence that encodes a SARS-CoV-2 S protein comprising one or more mutations characteristic of a BA.2 Omicron variant, or an immunogenic fragment thereof.
141. The composition or medical preparation of any one of embodiments 133-138, wherein the first RNA comprises a nucleotide sequence that encodes a SARS-CoV-2 S protein of a Wuhan strain, or an immunogenic fragment thereof, and the second RNA comprises a nucleotide sequence that encodes a SARS-CoV-2 S protein comprising one or more mutations characteristic of a BA.4 or BA.5 Omicron variant, or an immunogenic fragment thereof.
142. The composition or medical preparation of any one of embodiments 133-138, wherein the first RNA comprises a nucleotide sequence encoding a SARS-CoV-2 S protein of a Wuhan strain, or an immunogenic fragment thereof, and the second RNA comprises a nucleotide sequence encoding a SARS-CoV-2 S protein comprising one or more mutations characteristic of a BA.2 Omicron variant, or an immunogenic fragment thereof.
143. The composition or medical preparation of any one of embodiments 133-138, wherein the first RNA comprises a nucleotide sequence encoding a SARS-CoV-2 S protein of a Wuhan strain, or an immunogenic fragment thereof, and the second RNA comprises a nucleotide sequence encoding a SARS-CoV-2 S protein comprising one or more mutations characteristic of a BA.4 or BA.5 Omicron variant, or an immunogenic fragment thereof.
144. The composition or medical preparation of any one of embodiments 133-143, wherein the first RNA comprises a nucleotide sequence encoding a SARS-CoV-2 S protein of a Wuhan strain, or an immunogenic fragment thereof, and the second RNA comprises a nucleotide sequence encoding a SARS-CoV-2 S protein comprising one or more mutations characteristic of an XBB Omicron variant, or an immunogenic fragment thereof.
145. The composition or medical preparation of any one of embodiments 133-138, wherein the first RNA comprises a nucleotide sequence encoding a SARS-CoV-2 S protein of a Wuhan strain, or an immunogenic fragment thereof, and the second RNA comprises a nucleotide sequence encoding a SARS-CoV-2 S protein comprising one or more mutations characteristic of a BQ.1.1 Omicron variant or sublineages thereof, or an immunogenic fragment thereof.
146. The composition or medical preparation of any one of embodiments 133-145, wherein the immunogenic fragment of the SARS-CoV-2 S protein encoded by the first RNA and/or the second RNA comprises the S1 subunit of the SARS-CoV-2 S protein, or the receptor binding domain (RBD) of the S1 subunit of the SARS-CoV-2 S protein.
147. The composition or medical preparation of any one of embodiments 133-146, wherein each of the first RNA and the second RNA is codon-optimized (e.g., codon-optimized for expression in human cells) and/or has a G/C content that is increased compared to a wild type coding sequence.
148. A composition or medical preparation comprising a first RNA and a second RNA, wherein:
   a) the first RNA comprises a nucleotide sequence encoding a SARS-CoV-2 S protein of a Wuhan strain wherein (i) the SARS-CoV-2 S protein of a Wuhan strain comprises SEQ ID NO: 7 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 7, and/or (ii) the first RNA comprises a nucleotide sequence of SEQ ID NO: 9 or 20 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 9 or 20, and
   b) the second RNA comprises a nucleotide sequence encoding a SARS-CoV-2 protein comprising one or more mutations characteristic of a BA.4/5 Omicron variant, wherein (i) the SARS-CoV-2 S protein comprising one or more mutations characteristic of a BA.4/5 Omicron variant comprises an amino acid sequence of 90 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 90 and/or (ii) the second RNA comprises the nucleotide sequence of SEQ ID NO: 91 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 91.
149. A composition or medical preparation comprising a first RNA and a second RNA, wherein:
   a) the first RNA comprises a nucleotide sequence encoding a SARS-CoV-2 S protein of a Wuhan strain wherein (i) the SARS-CoV-2 S protein of a Wuhan strain comprises SEQ ID NO: 7 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 7, and/or (ii) the first RNA comprises a nucleotide sequence of SEQ ID NO: 9 or 20 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 9 or 20, and
   b) the second RNA comprises a nucleotide sequence encoding a SARS-CoV-2 protein comprising one or more mutations characteristic of a BA.1 Omicron variant, wherein (i) the SARS-CoV-2 S protein comprising one or more mutations characteristic of a BA.1 Omicron variant comprises an amino acid sequence of SEQ ID NO: 49 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 49 and/or (ii) the second RNA comprises the nucleotide sequence of SEQ ID NO: 70 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 70.
150. A composition or medical preparation comprising a first RNA and a second RNA, wherein:
   a) the first RNA comprises a nucleotide sequence encoding a SARS-CoV-2 S protein of a Wuhan strain wherein (i) the SARS-CoV-2 S protein of a Wuhan strain comprises SEQ ID NO: 7 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 7, and/or (ii) the first RNA comprises a nucleotide sequence of SEQ ID NO: 9 or 20 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 9 or 20, and
   b) the second RNA comprises a nucleotide sequence encoding a SARS-CoV-2 protein comprising one or more mutations characteristic of a BA.2 Omicron variant, wherein (i) the SARS-CoV-2 S protein comprising one or more mutations characteristic of a BA.2 Omicron variant comprises an amino acid sequence of SEQ ID NO: 64 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 69 and/or (ii) the second RNA comprises the nucleotide sequence of SEQ ID NO: 70 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 70.
151. A composition or medical preparation comprising a first RNA and a second RNA, wherein:
   a) the first RNA comprises a nucleotide sequence encoding a SARS-CoV-2 S protein of a Wuhan strain wherein (i) the SARS-CoV-2 S protein of a Wuhan strain comprises SEQ ID NO: 7 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 7, and/or (ii) the first RNA comprises a nucleotide sequence of SEQ ID NO: 9 or 20 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 9 or 20, and
   b) the second RNA comprises a nucleotide sequence encoding a SARS-CoV-2 protein comprising one or more mutations characteristic of a BA.2.75 Omicron variant, wherein (i) the SARS-CoV-2 S protein comprising one or more mutations characteristic of a BA.2.75 Omicron variant comprises an amino acid sequence of SEQ ID NO: 80 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 80 and/or (ii) the second RNA comprises the nucleotide sequence of SEQ ID NO: 81 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 81.
152. A composition or medical preparation comprising a first RNA and a second RNA, wherein:
   a) the first RNA comprises a nucleotide sequence encoding a SARS-CoV-2 S protein of a Wuhan strain wherein (i) the SARS-CoV-2 S protein of a Wuhan strain comprises SEQ ID NO: 7 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 7, and/or (ii) the first RNA comprises a nucleotide sequence of SEQ ID NO: 9 or 20 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 9 or 20, and
   b) the second RNA comprises a nucleotide sequence encoding a SARS-CoV-2 protein comprising one or more mutations characteristic of a BA.2.75.2 Omicron variant, wherein (i) the SARS-CoV-2 S protein comprising one or more mutations characteristic of a BA.2.75.2 Omicron variant comprises an amino acid sequence of SEQ ID NO: 85 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 85 and/or (ii) the second RNA comprises the nucleotide sequence of SEQ ID NO: 86 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 86.
153. A composition or medical preparation comprising a first RNA and a second RNA, wherein:
   a) the first RNA comprises a nucleotide sequence encoding a SARS-CoV-2 S protein of a Wuhan strain wherein (i) the SARS-CoV-2 S protein of a Wuhan strain comprises SEQ ID NO: 7 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 7, and/or (ii) the first RNA comprises a nucleotide sequence of SEQ ID NO: 9 or 20 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 9 or 20, and
   b) the second RNA comprises a nucleotide sequence encoding a SARS-CoV-2 protein comprising one or more mutations characteristic of a BA.4.6 or BF.7 Omicron variant, wherein (i) the SARS-CoV-2 S protein comprising one or more mutations characteristic of a BA.4.6 or BF.7 Omicron variant comprises an amino acid sequence of SEQ ID NO: 90 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 90 and/or (ii) the second RNA comprises the nucleotide sequence of SEQ ID NO: 91 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 91.
154. A composition or medical preparation comprising a first RNA and a second RNA, wherein:
   a) the first RNA comprises a nucleotide sequence encoding a SARS-CoV-2 S protein of a Wuhan strain wherein (i) the SARS-CoV-2 S protein of a Wuhan strain comprises SEQ ID NO: 7 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 7, and/or (ii) the first RNA comprises a nucleotide sequence of SEQ ID NO: 9 or 20 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 9 or 20, and
   b) the second RNA comprises a nucleotide sequence encoding a SARS-CoV-2 protein comprising one or more mutations characteristic of an XBB Omicron variant, wherein (i) the SARS-CoV-2 S protein comprising one or more mutations characteristic of an XBB Omicron variant comprises an amino acid sequence of SEQ ID NO: 95 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 95 and/or (ii) the second RNA comprises the nucleotide sequence of SEQ ID NO: 96 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 96.
155. A composition or medical preparation comprising a first RNA and a second RNA, wherein:
   a) the first RNA comprises a nucleotide sequence encoding a SARS-CoV-2 S protein of a Wuhan strain wherein (i) the SARS-CoV-2 S protein of a Wuhan strain comprises SEQ ID NO: 7 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 7, and/or (ii) the first RNA comprises a nucleotide sequence of SEQ ID NO: 9 or 20 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 9 or 20, and
   b) the second RNA comprises a nucleotide sequence encoding a SARS-CoV-2 protein comprising one or more mutations characteristic of an BQ.1.1 Omicron variant, wherein (i) the SARS-CoV-2 S protein comprising one or more mutations characteristic of a BQ.1.1 Omicron variant comprises an amino acid sequence of SEQ ID NO: 100 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 100 and/or (ii) the second RNA comprises the nucleotide sequence of SEQ ID NO: 100 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 100.
156. The composition or medical preparation of any one of embodiments 133-155, wherein each of the first RNA and the second RNA encode a SARS-CoV-2 S protein comprising one or more mutations that improves expression, stability, and/or immunogenicity.
157. The composition or medical preparation of embodiment 156, wherein each of the first RNA and the second RNA encode a SARS-CoV-2 S protein comprising one or more mutations that stabilize the prefusion confirmation.
158. The composition or medical preparation of embodiment 157, wherein each of the first RNA and the second RNA encode a SARS-CoV-2 S protein comprising proline mutations at positions corresponding to residues 986 and 987 of SEQ ID NO: 1.
159. The composition or medical preparation of embodiment 157 or 158, wherein each of the first RNA and the second RNA encode a SARS-CoV-2 S protein comprising one or more proline mutations at positions corresponding to residues 817, 892, 899, and/or 942 of SEQ ID NO: 1.
160. The composition or medical preparation of any one of embodiments 156-159, wherein each of the first RNA and the second RNA encode a SARS-CoV-2 S protein comprising a mutation that prevents furin cleavage.
161. The composition or medical preparation of embodiment 160, wherein each of the first RNA and the second RNA encode a SARS-CoV-2 S protein comprising a mutation at a location corresponding to residues 682-685 of SEQ ID NO: 1 that prevents cleavage by a furin protease (e.g., a GSAS mutation).
162. The composition or medical preparation of any one of embodiments 156-161, wherein each of the first RNA and the second RNA encode a SARS-CoV-2 S protein comprising one or more mutations that decreases S protein shedding (e.g., an aspartate to glycine mutation at a position corresponding to residue 614 of SEQ ID NO: 1).
163. The composition or medical preparation of any one of embodiments 133-162, wherein the first RNA and the second RNA each comprise a modified nucleoside in place of uridine.
164. The composition or medical preparation of embodiment 163, wherein the first RNA and the second RNA each comprise a modified nucleoside in place of each uridine.
165. The composition or medical preparation of embodiment 163 or 164, wherein the modified nucleoside is selected from pseudouridine (ψ), N1-methyl-pseudouridine (m1ψ), and 5-methyl-uridine (m5U).
166. The composition or medical preparation of embodiment 165, wherein the modified nucleoside is N1-methyl-pseudouridine (m1ψ).
167. The composition or medical preparation of any one of embodiments 133-166, wherein the first RNA and the second RNA each comprise a 5' cap.
168. The composition or medical preparation of embodiment 167, wherein the 5' cap is or comprises a cap1 structure.
169. The composition or medical preparation of embodiment 168, wherein the 5'-cap is or comprises m₂^{7,3'-}OGppp(m₁^{2'-O})ApG.
170. The composition or medical preparation or medical preparation of any one of embodiments 133-169, wherein the first RNA and the second RNA each comprise a poly(A) sequence.
171. The composition or medical preparation of embodiment 170, wherein the poly(A) sequence comprises at least 100 A nucleotides.
172. The composition or medical preparation of embodiment 170 or 171, wherein the poly(A) sequence is an interrupted sequence of A nucleotides.
173. The composition or medical preparation of embodiment 172, wherein the poly(A) sequence comprises 30 adenine nucleotides followed by 70 adenine nucleotides, wherein the 30 adenine nucleotides and 70 adenine nucleotides are separated by a linker sequence.
174. The composition or medical preparation of any one of embodiments 170-173, wherein the poly(A) sequence comprises or consists of the nucleotide sequence of SEQ ID NO: 14, or a nucleotide sequence that is at least 99%, 98%, 97%, 96%, 95%, 90%, 85%, or 80% identical to the nucleotide sequence of SEQ ID NO: 14.
175. The composition or medical preparation of any one of embodiments 133-174, wherein the first RNA and the second RNA each comprise a 5'-UTR that is or comprises a modified human alpha-globin 5'-UTR.
176. The composition or medical preparation of embodiment 175, wherein the 5' UTR comprises the nucleotide sequence of SEQ ID NO: 12, or a nucleotide sequence that is at least 99%, 98%, 97%, 96%, 95%, 90%, 85%, or 80% identical to the nucleotide sequence of SEQ ID NO: 12.
178. The composition or medical preparation of any one of embodiments 133-176, wherein the first RNA and the second RNA each comprise a 3'-UTR that is or comprises a first sequence from the amino terminal enhancer of split (AES) messenger RNA and a second sequence from the mitochondrial encoded 12S ribosomal RNA.
179. The composition or medical preparation of embodiment 178, wherein the first RNA and the second RNA each comprise a 3' UTR comprises the nucleotide sequence of SEQ ID NO: 13, or a nucleotide sequence having at least 99%, 98%, 97%, 96%, 95%, 90%, 85%, or 80% identity to the nucleotide sequence of SEQ ID NO: 13.
180. The composition or medical preparation of any one of embodiments 133-179, wherein the first RNA and the second RNA are each formulated or are to be formulated as particles.
181. The composition or medical preparation of embodiment 180, wherein the particles are lipid nanoparticles (LNP) or lipoplex (LPX) particles.
182.The composition or medical preparation of embodiment 181, wherein the LNPs comprise a cationically ionizable lipid, a neutral lipid, a sterol and a polymer-lipid conjugate.
183. The composition or medical preparation of embodiment 181 or 182, wherein the first RNA and the second RNA are formulated in separate lipid nanoparticles.
184. The composition or medical preparation of embodiment 181 or 182, wherein the first RNA and the second RNA are formulated in the same lipid nanoparticles.
185. The composition or medical preparation of any one of embodiments 182-184, wherein the neutral lipid is present in a concentration ranging from about 5 to about 15 mol percent of the total lipids.
186. The composition or medical preparation of any one of embodiments 182-185, wherein the cationically ionizable lipid is present in a concentration ranging from about 40 to about 50 mol percent of the total lipids.
187. The composition or medical preparation of any one of embodiments 182-186, wherein the sterol is present in a concentration ranging from about 30 to about 50 mol percent of the total lipids.
188. The composition or medical preparation of any one of embodiments 182-187, wherein the polymer-lipid conjugate is present in a concentration ranging from about 1 to about 10 mol percent of the total lipids.
189. The composition or medical preparation of any one of embodiments 182-188, wherein the lipid nanoparticles comprise from about 40 to about 50 mol percent of the cationically ionizable lipid; from about 5 to about 15 mol percent of the neutral lipid; from about 35 to about 45 mol percent of the sterol; and from about 1 to about 10 mol percent of the polymer conjugated-lipid.
190. The composition or medical preparation of embodiment 181, wherein the RNA lipoplex particles are obtainable by mixing the first RNA and the second RNA with liposomes.
191. The composition or medical preparation of any one of embodiments 133-190, wherein the first RNA and the second RNA are present in a combined amount within a range of about 1 µg to about 100 µg per dose.
192. The composition or medical preparation of any one of embodiments 133-191, wherein the first RNA and the second RNA are present in a combined amount within a range of about 1 µg to about 60 µg per dose.
193. The composition or medical preparation of any one of embodiments 133-192, wherein the first RNA and the second RNA are present in a combined amount of about 3.0 µg, about 10 µg, about 30 µg, or about 60 µg per dose.
194. The composition or medical preparation of any one of embodiments 133-193, wherein the ratio of the first RNA to the second RNA is about 1:10 to about 10:1.
195. The composition or medical preparation of any one of embodiments 133-194, wherein the ratio of the first RNA to the second RNA is about 1:1.
196. The composition or medical preparation of any one of embodiments 133-195, wherein:
   a) the first RNA and the second RNA are each present in an amount of about 1.5 µg per dose in the composition;
   b) the first RNA and the second RNA are each present in an amount of about 5 µg per dose in the composition;
   c) the first RNA and the second RNA are each present in an amount of about 15 µg per dose in the composition; or
   d) the first RNA and the second RNA are each present in an amount of about 15 µg per dose in the composition.
197. The composition or medical preparation of any one of embodiments 124-169, further comprising an RNA encoding one or more T cell epitopes of SARS-CoV-2.
198. The composition or medical preparation of embodiment 170, wherein the RNA encoding one or more T cell epitopes of SARS-CoV-2 comprises one or more epitopes of each of the ORF1ab, M, and N regions of the SARS-CoV-2 genome.
199. The composition or medical preparation of any one of embodiments 1-198, which is formulated or is to be formulated as a liquid, a solid, or a combination thereof.
200. The composition or medical preparation of any one of embodiments 1-199, which is formulated or is to be formulated for injection.
201. The composition or medical preparation of any one of embodiments 1-200, wherein the composition or medical preparation is formulated or is to be formulated for intramuscular administration.
202. The composition or medical preparation of any one of embodiments 1-201, which is a pharmaceutical composition.
203. The composition or medical preparation of any one of embodiments 1-202, which is a vaccine.
204. The composition or medical preparation of embodiment 202 or 203, wherein the pharmaceutical composition further comprises one or more pharmaceutically acceptable carriers, diluents and/or excipients.
205. The composition or medical preparation of any one of embodiments 1-204, which is a kit.
206. The composition or medical preparation of embodiment 205, further comprising instructions for use of the composition or medical preparation for inducing an immune response against coronavirus in a subject.
207. The composition or medical preparation of any one of embodiments 1-206 for pharmaceutical use.
208. The composition or medical preparation of embodiment 207, wherein the pharmaceutical use comprises inducing an immune response against coronavirus in a subject.
209. The composition or medical preparation of embodiment 207 or 208, wherein the pharmaceutical use comprises a therapeutic or prophylactic treatment of a coronavirus infection.
210. The composition or medical preparation of embodiment 208 or 209, wherein the coronavirus is a sarbecovirus.
211. The composition or medical preparation of any one of embodiments 208-211, wherein the coronavirus is a betacoronavirus.
212. The composition or medical preparation of any one of embodiments 208-212, wherein the coronavirus is SARS-CoV-2.
213. The composition or medical preparation of any one of embodiments 1-212, which is for administration to a human.
214. A method of eliciting an immune response against SARS-CoV-2 in a subject comprising administering the composition or medical preparation of any one of embodiments 1-213.
215. The method of embodiment 214, wherein the immune response is elicited against an Omicron variant of SARS-CoV-2.
216. The method of embodiment 214 or 215, wherein the subject has previously been infected with or vaccinated against SARS-CoV-2.
217. The method of any one of embodiments 214-216, wherein an antigen of a Wuhan strain of SARS-CoV-2 has previously been delivered to the subject (e.g., as a polypeptide or an RNA encoding such a polypeptide).
218. The method of any one of embodiments 214-217, wherein the subject has previously been administered RNA encoding a SARS-CoV-2 S protein of a Wuhan strain.
219. The method of any one of embodiments 214-218, wherein the subject has previously been administered two or more doses of RNA encoding a SARS-CoV-2 S protein of a Wuhan strain.
220. The method of embodiment 219, wherein the RNA of any one of embodiments 1-206 is administered at least about 2 months after the two or more doses of RNA encoding a SARS-CoV-2 S protein of a Wuhan strain.
221. The method of any one of embodiments 214-220, wherein the method comprises administering RNA encoding an antigen of a SARS-CoV-2 virus that is not a BA.1 Omicron variant.
222. The method of any one of embodiments 214-221, wherein the method comprises administering RNA comprising a nucleotide sequence encoding a SARS-Cov-2 S protein comprising one or more mutations characteristic of an Omicron variant, wherein the Omicron variant is not a BA.1 Omicron variant.
223. The method of any one of embodiments 214-222, wherein the method comprises administering RNA comprising a nucleotide seuqence encoding a SARS-CoV-2 S protein comprising one or more mutations characteristic of a BA.2 Omicron variant.
224. The method of any one of embodiments 214-222, wherein the method comprises administering RNA comprising a nucleotide sequence encoding a SARS-CoV-2 S protein comprising one or more mutations characteristic of a BA.4 or BA.5 Omicron variant.
225. A method for inducing an immune response in a subject, wherein the method comprises administering (a) a first RNA comprising a nucleotide sequence encoding a SARS-CoV-2 S protein of a Wuhan strain, or comprising one or more mutations characteristic of an Alpha variant, a Beta variant, or a Delta variant, and (b) a second RNA comprising a nucleotide sequence encoding a SARS-CoV-2 S protein comprising one or more mutations characteristic of an Omicron variant that is not a BA.1 Omicron variant.
226. The method of embodiment 225, where the second RNA comprises a nucleotide sequence encoding a SARS-CoV-2 S protein of a BA.2 Omicron variant.
227. The method of embodiment 225, where the second RNA comprises a nucleotide sequence encoding an S protein comprising one or more mutations characteristic of a BA.4 or BA.5 Omicron variant.
228. A method for inducing an immune response in a subject, wherein the method comprises administering (a) a first RNA comprising a nucleotide sequence encoding a SARS-CoV-2 S protein of a Wuhan strain, or comprising one or more mutations characteristic of an Alpha variant, a Beta variant, a Delta variant, or a BA.1 Omicron variant and (b) a second RNA comprising a nucleotide sequence encoding a SARS-CoV-2 S protein that is antigenically distinct from the S protein encoded by the first RNA.
229. The method of embodiment 228, wherein the second RNA comprises a nucleotide sequence encoding a SARS-CoV-2 S protein comprising one or more mutations that are characteristic of an Omicron variant that is not a BA.1 Omicron variant.
230. The method of embodiment 228, wherein the second RNA comprises a nucleotide sequence encoding a SARS-CoV-2 S protein comprising one or more mutations that are charcteristic of a BA.2 Omicron variant.
231. The method of embodiment 228, wherein the second RNA comprises a nucleotide sequence encoding a SARS-CoV-2 S protein comprising one or more mutations characteristic of a BA.4 or BA.5 Omicron variant.
232. The method of any one of embodiments 214-231, wherein the first RNA and the second RNA are encapsulated in separate LNPs.
233. The method of any one of embodiments 214-231, wherein the first RNA and the second RNA are encapsulated in the same LNP.
234. The method of any one of embodiments 214-232, wherein the first RNA and the second RNA are administered separately, e.g., at different injection sites.
235. The method of any one of embodiments 214-234, further comprising administering an RNA comprising a nucleotide sequence encoding one or more T cell epitopes of SARS-CoV-2.
236. The method of embodiment 235, wherein the nucleotide sequence encoding one or more T cell epitopes of SARS-CoV-2 encodes one or more epitopes of each of the ORF1ab, M, and N regions of the SARS-CoV-2 genome.
237. The method of embodiment 235 or 236, wherein the first RNA, the second RNA, and the RNA encoding one or more T cell epitopes of SARS-CoV-2 are each formulated in separate LNPs.
238. The method of embodiment 235 or 236, wherein the first RNA and the second RNA are co-formulated in the same LNP, and the RNA encoding one or more T cell epitopes of SARS-CoV-2 is formulated in a separate LNP.
239. The method of embodiment 235 or 236, wherein each of the first RNA, the second RNA, and the RNA encoding one or more T cell epitopes of SARS-CoV-2 is co-formulated in the same LNP.
240. The method of any one of embodiments 235-239, wherein the first RNA, the second RNA, and the RNA encoding one or more T cell epitopes of SARS-CoV-2 are administered in a dose comprising:
   (a) 30 µg of the first RNA and the second RNA combined (e.g., 15 µg of the first RNA and 15 µg of the second RNA), and 5 µg of the RNA encoding one or more T cell epitopes of SARS-CoV-2;
   (b) 30 µg of the first RNA and the second RNA combined (e.g., 15 µg of the first RNA and 15 µg of the second RNA), and 10 µg of the RNA encoding one or more T cell epitopes of SARS-CoV-2; or
   (c) 30 µg of the first RNA and the second RNA combined (e.g., 15 µg of the first RNA and 15 µg of the second RNA), and 15 µg of the RNA encoding one or more T cell epitopes of SARS-CoV-2.
241. The method of any one of embodiments 235-240, wherein the RNA encoding one or more T-cell epitopes of SARS-CoV-2 encodes a polypeptide sequence of SEQ ID NO: RS C7p2full.
242. The method of any one of embodiments 207-241, further comprising administering one or more vaccines against a respiratory disease.
243. The method of embodiment 242, wherein the respiratory disease is RSV or influenza.
244. The method of embodiment 242 or 243, comprising administering a vaccine against RSV and a vaccine against influenza.
245. The method of any one of embodiments 242-244, wherein the one or more vaccines against a respiratory disease are RNA vaccines each comprise one or more RNAs encoding an antigenic protein.
246. The method of embodiment 245, comprising administering one or more RNAs encoding antigen(s) from an influenza virus and/or one or more RNAs encoding antigen(s) from an RSV.
247. The method of embodiment 246, wherein each RNA is formulated in a separate LNP.
248. The method of embodiment 246, wherein each RNA encoding a SARS-CoV-2 S protein or an immunogenic fragment thereof is co-formulated together in the same LNP, each RNA encoding an antigenic protein from an influenza virus is co-formulated together in the same LNP, and each RNA encoding an antigenic protein from an RSV is co-formulated together in the same LNP.
249. The method of embodiment 246, wherein each RNA encoding a SARS-CoV-2 S protein or an immunogenic fragment thereof, each RNA encoding an antigenic protein from an influenza virus, and each RNA encoding an antigenic protein from an RSV are co-formulated together in the same LNP.
250. A composition or medical preparation comprising RNA encoding an amino acid sequence comprising a SARS-CoV-2 S protein, an immunogenic variant thereof, or an immunogenic fragment of the SARS-CoV-2 S protein or the immunogenic variant thereof.
251. The composition or medical preparation of embodiment 250, wherein an immunogenic fragment of the SARS-CoV-2 S protein comprises the S1 subunit of the SARS-CoV-2 S protein, or the receptor binding domain (RBD) of the S1 subunit of the SARS-CoV-2 S protein .
252. The composition or medical preparation of embodiments 250 or 251, wherein the amino acid sequence comprising a SARS-CoV-2 S protein, an immunogenic variant thereof, or an immunogenic fragment of the SARS-CoV-2 S protein or the immunogenic variant thereof is encoded by a coding sequence which is codon-optimized and/or the G/C content of which is increased compared to wild type coding sequence, wherein the codon-optimization and/or the increase in the G/C content preferably does not change the sequence of the encoded amino acid sequence.
253. The composition or medical preparation of any one of embodiments 250 to 252, wherein
   (i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 979 to 1584 of SEQ ID NO: 2, 8 or 9, a nucleotide sequence having at least 99%, 98%, 97%, 96%, 95%, 90%, 85%, or 80% identity to the nucleotide sequence of nucleotides 979 to 1584 of SEQ ID NO: 2, 8 or 9, or a fragment of the nucleotide sequence of nucleotides 979 to 1584 of SEQ ID NO: 2, 8 or 9, or the nucleotide sequence having at least 99%, 98%, 97%, 96%, 95%, 90%, 85%, or 80% identity to the nucleotide sequence of nucleotides 979 to 1584 of SEQ ID NO: 2, 8 or 9; and/or
   (ii) a SARS-CoV-2 S protein, an immunogenic variant thereof, or an immunogenic fragment of the SARS-CoV-2 S protein or the immunogenic variant thereof comprises the amino acid sequence of amino acids 327 to 528 of SEQ ID NO: 1, an amino acid sequence having at least 99%, 98%, 97%, 96%, 95%, 90%, 85%, or 80% identity to the amino acid sequence of amino acids 327 to 528 of SEQ ID NO: 1, or an immunogenic fragment of the amino acid sequence of amino acids 327 to 528 of SEQ ID NO: 1, or the amino acid sequence having at least 99%, 98%, 97%, 96%, 95%, 90%, 85%, or 80% identity to the amino acid sequence of amino acids 327 to 528 of SEQ ID NO: 1.
254. The composition or medical preparation of any one of embodiments250 to 253, wherein
   (i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 111 to 986 of SEQ ID NO: 30, a nucleotide sequence having at least 99%, 98%, 97%, 96%, 95%, 90%, 85%, or 80% identity to the nucleotide sequence of nucleotides 111 to 986 of SEQ ID NO: 30, or a fragment of the nucleotide sequence of nucleotides 111 to 986 of SEQ ID NO: 30, or the nucleotide sequence having at least 99%, 98%, 97%, 96%, 95%, 90%, 85%, or 80% identity to the nucleotide sequence of nucleotides 111 to 986 of SEQ ID NO: 30; and/or
   (ii) a SARS-CoV-2 S protein, an immunogenic variant thereof, or an immunogenic fragment of the SARS-CoV-2 S protein or the immunogenic variant thereof comprises the amino acid sequence of amino acids 20 to 311 of SEQ ID NO: 29, an amino acid sequence having at least 99%, 98%, 97%, 96%, 95%, 90%, 85%, or 80% identity to the amino acid sequence of amino acids 20 to 311 of SEQ ID NO: 29, or an immunogenic fragment of the amino acid sequence of amino acids 20 to 311 of SEQ ID NO: 29, or the amino acid sequence having at least 99%, 98%, 97%, 96%, 95%, 90%, 85%, or 80% identity to the amino acid sequence of amino acids 20 to 311 of SEQ ID NO: 29..
255. The composition or medical preparation of any one of embodiments 250 to 254, wherein
   (i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 49 to 3819 of SEQ ID NO: 2, 8 or 9, a nucleotide sequence having at least 99%, 98%, 97%, 96%, 95%, 90%, 85%, or 80% identity to the nucleotide sequence of nucleotides 49 to 3819 of SEQ ID NO: 2, 8 or 9, or a fragment of the nucleotide sequence of nucleotides 49 to 3819 of SEQ ID NO: 2, 8 or 9, or the nucleotide sequence having at least 99%, 98%, 97%, 96%, 95%, 90%, 85%, or 80% identity to the nucleotide sequence of nucleotides 49 to 3819 of SEQ ID NO: 2, 8 or 9; and/or
   (ii) a SARS-CoV-2 S protein, an immunogenic variant thereof, or an immunogenic fragment of the SARS-CoV-2 S protein or the immunogenic variant thereof comprises the amino acid sequence of amino acids 17 to 1273 of SEQ ID NO: 1 or 7, an amino acid sequence having at least 99%, 98%, 97%, 96%, 95%, 90%, 85%, or 80% identity to the amino acid sequence of amino acids 17 to 1273 of SEQ ID NO: 1 or 7, or an immunogenic fragment of the amino acid sequence of amino acids 17 to 1273 of SEQ ID NO: 1 or 7, or the amino acid sequence having at least 99%, 98%, 97%, 96%, 95%, 90%, 85%, or 80% identity to the amino acid sequence of amino acids 17 to 1273 of SEQ ID NO: 1 or 7.
256. The composition or medical preparation of any one of embodiments 250 to 255, wherein the amino acid sequence comprising a SARS-CoV-2 S protein, an immunogenic variant thereof, or an immunogenic fragment of the SARS-CoV-2 S protein or the immunogenic variant thereof comprises a secretory signal peptide.
257. The composition or medical preparation of embodiment256, wherein the secretory signal peptide is fused, preferably N-terminally, to a SARS-CoV-2 S protein, an immunogenic variant thereof, or an immunogenic fragment of the SARS-CoV-2 S protein or the immunogenic variant thereof.
258. The composition or medical preparation of embodiment 256 or 257, wherein
   (i) the RNA encoding the secretory signal peptide comprises the nucleotide sequence of nucleotides 1 to 48 of SEQ ID NO: 2, 8 or 9, a nucleotide sequence having at least 99%, 98%, 97%, 96%, 95%, 90%, 85%, or 80% identity to the nucleotide sequence of nucleotides 1 to 48 of SEQ ID NO: 2, 8 or 9, or a fragment of the nucleotide sequence of nucleotides 1 to 48 of SEQ ID NO: 2, 8 or 9, or the nucleotide sequence having at least 99%, 98%, 97%, 96%, 95%, 90%, 85%, or 80% identity to the nucleotide sequence of nucleotides 1 to 48 of SEQ ID NO: 2, 8 or 9; and/or
   (ii) the secretory signal peptide comprises the amino acid sequence of amino acids 1 to 16 of SEQ ID NO: 1, an amino acid sequence having at least 99%, 98%, 97%, 96%, 95%, 90%, 85%, or 80% identity to the amino acid sequence of amino acids 1 to 16 of SEQ ID NO: 1, or a functional fragment of the amino acid sequence of amino acids 1 to 16 of SEQ ID NO: 1, or the amino acid sequence having at least 99%, 98%, 97%, 96%, 95%, 90%, 85%, or 80% identity to the amino acid sequence of amino acids 1 to 16 of SEQ ID NO: 1.
259. The composition or medical preparation of any one of embodiments 250 to 258, wherein
   (i) the RNA encoding a SARS-CoV-2 S protein, an immunogenic variant thereof, or an immunogenic fragment of the SARS-CoV-2 S protein or the immunogenic variant thereof comprises the nucleotide sequence of SEQ ID NO: 6, a nucleotide sequence having at least 99%, 98%, 97%, 96%, 95%, 90%, 85%, or 80% identity to the nucleotide sequence of SEQ ID NO: 6, or a fragment of the nucleotide sequence of SEQ ID NO: 6, or the nucleotide sequence having at least 99%, 98%, 97%, 96%, 95%, 90%, 85%, or 80% identity to the nucleotide sequence of SEQ ID NO: 6; and/or
   (ii) a SARS-CoV-2 S protein, an immunogenic variant thereof, or an immunogenic fragment of the SARS-CoV-2 S protein or the immunogenic variant thereof comprises the amino acid sequence of SEQ ID NO: 5, an amino acid sequence having at least 99%, 98%, 97%, 96%, 95%, 90%, 85%, or 80% identity to the amino acid sequence of SEQ ID NO: 5, or an immunogenic fragment of the amino acid sequence of SEQ ID NO: 5, or the amino acid sequence having at least 99%, 98%, 97%, 96%, 95%, 90%, 85%, or 80% identity to the amino acid sequence of SEQ ID NO: 5.
260. The composition or medical preparation of any one of embodiments 250 to 259, wherein
   (i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 54 to 986 of SEQ ID NO: 30, a nucleotide sequence having at least 99%, 98%, 97%, 96%, 95%, 90%, 85%, or 80% identity to the nucleotide sequence of nucleotides 54 to 986 of SEQ ID NO: 30, or a fragment of the nucleotide sequence of nucleotides 54 to 986 of SEQ ID NO: 30, or the nucleotide sequence having at least 99%, 98%, 97%, 96%, 95%, 90%, 85%, or 80% identity to the nucleotide sequence of nucleotides 54 to 986 of SEQ ID NO: 30; and/or
   (ii) a SARS-CoV-2 S protein, an immunogenic variant thereof, or an immunogenic fragment of the SARS-CoV-2 S protein or the immunogenic variant thereof comprises the amino acid sequence of amino acids 1 to 311 of SEQ ID NO: 29, an amino acid sequence having at least 99%, 98%, 97%, 96%, 95%, 90%, 85%, or 80% identity to the amino acid sequence of amino acids 1 to 311 of SEQ ID NO: 29, or an immunogenic fragment of the amino acid sequence of amino acids 1 to 311 of SEQ ID NO: 29, or the amino acid sequence having at least 99%, 98%, 97%, 96%, 95%, 90%, 85%, or 80% identity to the amino acid sequence of amino acids 1 to 311 of SEQ ID NO: 29.
261. The composition or medical preparation of any one of embodiments 250 to 258, wherein the RNA comprises a modified nucleoside in place of uridine, in particular wherein the modified nucleoside is selected from pseudouridine (ψ), N1-methyl-pseudouridine (m1ψ), and 5-methyl-uridine (m5U), in particular wherein the modified nucleoside is N1-methyl-pseudouridine (m1ψ).
262. The composition or medical preparation of any one of embodiments 250 to 261, wherein the RNA comprises a 5' cap.
263. The composition or medical preparation of any one of embodiments 250 to 262, wherein the RNA encoding an amino acid sequence comprising a SARS-CoV-2 S protein, an immunogenic variant thereof, or an immunogenic fragment of the SARS-CoV-2 S protein or the immunogenic variant thereof comprises a 5' UTR comprising the nucleotide sequence of SEQ ID NO: 12, or a nucleotide sequence having at least 99%, 98%, 97%, 96%, 95%, 90%, 85%, or 80% identity to the nucleotide sequence of SEQ ID NO: 12.
264. The composition or medical preparation of any one of embodiments 250 to 263, wherein the RNA encoding an amino acid sequence comprising a SARS-CoV-2 S protein, an immunogenic variant thereof, or an immunogenic fragment of the SARS-CoV-2 S protein or the immunogenic variant thereof comprises a 3' UTR comprising the nucleotide sequence of SEQ ID NO: 13, or a nucleotide sequence having at least 99%, 98%, 97%, 96%, 95%, 90%, 85%, or 80% identity to the nucleotide sequence of SEQ ID NO: 13.
265. The composition or medical preparation of any one of embodiments 250 to 264, wherein the RNA encoding an amino acid sequence comprising a SARS-CoV-2 S protein, an immunogenic variant thereof, or an immunogenic fragment of the SARS-CoV-2 S protein or the immunogenic variant thereof comprises a poly-A sequence.
266. The composition or medical preparation of embodiment 265, wherein the poly-A sequence comprises at least 100 nucleotides.
267. The composition or medical preparation of embodiment 265 or 266, wherein the poly-A sequence comprises or consists of the nucleotide sequence of SEQ ID NO: 14.
268. The composition or medical preparation of any one of embodiments 250 to 267, wherein the RNA is formulated or is to be formulated as a liquid, a solid, or a combination thereof.
269. The composition or medical preparation of any one of embodiments 250 to 268, wherein the RNA is formulated or is to be formulated for injection.
270. The composition or medical preparation of any one of embodiments 250 to 269, wherein the RNA is formulated or is to be formulated for intramuscular administration.
271. The composition or medical preparation of any one of embodiments 250 to 270, wherein the RNA is formulated or is to be formulated as particles.
272. The composition or medical preparation of embodiment 271, wherein the particles are lipid nanoparticles (LNP) or lipoplex (LPX) particles.
273. The composition or medical preparation of embodiment 272, wherein the LNP particles comprise ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoyl-sn-glycero-3-phosphocholine, and cholesterol.
274. The composition or medical preparation of embodiment 272, wherein the RNA lipoplex particles are obtainable by mixing the RNA with liposomes.
275. The composition or medical preparation of any one of embodiments 250 to 274, wherein the RNA is mRNA or saRNA.
276. The composition or medical preparation of any one of embodiments 250 to 275, which is a pharmaceutical composition.
277. The composition or medical preparation of any one of embodiments 250 to 276, which is a vaccine.
278. The composition or medical preparation of embodiment 276 or 277, wherein the pharmaceutical composition further comprises one or more pharmaceutically acceptable carriers, diluents and/or excipients.
279. The composition or medical preparation of any one of embodiments 250 to 275, which is a kit.
280. The composition or medical preparation of embodiment 279, wherein the RNA and optionally the particle forming components are in separate vials.
281. The composition or medical preparation of embodiment 279 or 280, further comprising instructions for use of the composition or medical preparation for inducing an immune response against coronavirus in a subject.
282. The composition or medical preparation of any one of embodiments 250 to 281 for pharmaceutical use.
283. The composition or medical preparation of embodiment 282, wherein the pharmaceutical use comprises inducing an immune response against coronavirus in a subject.
284. The composition or medical preparation of embodiment 282 or 283, wherein the pharmaceutical use comprises a therapeutic or prophylactic treatment of a coronavirus infection.
285. The composition or medical preparation of any one of embodiments 250 to 284, which is for administration to a human.
286. The composition or medical preparation of any one of embodiments 282 to 285, wherein the coronavirus is a betacoronavirus.
287. The composition or medical preparation of any one of embodiments 282 to 286, wherein the coronavirus is a sarbecovirus.
288. The composition or medical preparation of any one of embodiments 282 to 287, wherein the coronavirus is SARS-CoV-2.
289. A method of inducing an immune response against coronavirus in a subject comprising administering to the subject a composition comprising RNA encoding an amino acid sequence comprising a SARS-CoV-2 S protein, an immunogenic variant thereof, or an immunogenic fragment of the SARS-CoV-2 S protein or the immunogenic variant thereof.
290. The method of embodiment 289, wherein an immunogenic fragment of the SARS-CoV-2 S protein comprises the S1 subunit of the SARS-CoV-2 S protein, or the receptor binding domain (RBD) of the S1 subunit of the SARS-CoV-2 S protein.
291. The method of any one of embodiments 289 or 290, wherein the amino acid sequence comprising a SARS-CoV-2 S protein, an immunogenic variant thereof, or an immunogenic fragment of the SARS-CoV-2 S protein or the immunogenic variant thereof is encoded by a coding sequence which is codon-optimized and/or the G/C content of which is increased compared to wild type coding sequence, wherein the codon-optimization and/or the increase in the G/C content preferably does not change the sequence of the encoded amino acid sequence.
292. The method of any one of embodiments 289 to 291, wherein
   (i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 979 to 1584 of SEQ ID NO: 2, 8 or 9, a nucleotide sequence having at least 99%, 98%, 97%, 96%, 95%, 90%, 85%, or 80% identity to the nucleotide sequence of nucleotides 979 to 1584 of SEQ ID NO: 2, 8 or 9, or a fragment of the nucleotide sequence of nucleotides 979 to 1584 of SEQ ID NO: 2, 8 or 9, or the nucleotide sequence having at least 99%, 98%, 97%, 96%, 95%, 90%, 85%, or 80% identity to the nucleotide sequence of nucleotides 979 to 1584 of SEQ ID NO: 2, 8 or 9; and/or
   (ii) a SARS-CoV-2 S protein, an immunogenic variant thereof, or an immunogenic fragment of the SARS-CoV-2 S protein or the immunogenic variant thereof comprises the amino acid sequence of amino acids 327 to 528 of SEQ ID NO: 1, an amino acid sequence having at least 99%, 98%, 97%, 96%, 95%, 90%, 85%, or 80% identity to the amino acid sequence of amino acids 327 to 528 of SEQ ID NO: 1, or an immunogenic fragment of the amino acid sequence of amino acids 327 to 528 of SEQ ID NO: 1, or the amino acid sequence having at least 99%, 98%, 97%, 96%, 95%, 90%, 85%, or 80% identity to the amino acid sequence of amino acids 327 to 528 of SEQ ID NO: 1.
293. The method of any one of embodiments 289 to 292, wherein
   (i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 111 to 986 of SEQ ID NO: 30, a nucleotide sequence having at least 99%, 98%, 97%, 96%, 95%, 90%, 85%, or 80% identity to the nucleotide sequence of nucleotides 111 to 986 of SEQ ID NO: 30, or a fragment of the nucleotide sequence of nucleotides 111 to 986 of SEQ ID NO: 30, or the nucleotide sequence having at least 99%, 98%, 97%, 96%, 95%, 90%, 85%, or 80% identity to the nucleotide sequence of nucleotides 111 to 986 of SEQ ID NO: 30; and/or
   (ii) a SARS-CoV-2 S protein, an immunogenic variant thereof, or an immunogenic fragment of the SARS-CoV-2 S protein or the immunogenic variant thereof comprises the amino acid sequence of amino acids 20 to 311 of SEQ ID NO: 29, an amino acid sequence having at least 99%, 98%, 97%, 96%, 95%, 90%, 85%, or 80% identity to the amino acid sequence of amino acids 20 to 311 of SEQ ID NO: 29, or an immunogenic fragment of the amino acid sequence of amino acids 20 to 311 of SEQ ID NO: 29, or the amino acid sequence having at least 99%, 98%, 97%, 96%, 95%, 90%, 85%, or 80% identity to the amino acid sequence of amino acids 20 to 311 of SEQ ID NO: 29.
294. The method of any one of embodiments 289 to 293, wherein
   (i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 49 to 3819 of SEQ ID NO: 2, 8 or 9, a nucleotide sequence having at least 99%, 98%, 97%, 96%, 95%, 90%, 85%, or 80% identity to the nucleotide sequence of nucleotides 49 to 3819 of SEQ ID NO: 2, 8 or 9, or a fragment of the nucleotide sequence of nucleotides 49 to 3819 of SEQ ID NO: 2, 8 or 9, or the nucleotide sequence having at least 99%, 98%, 97%, 96%, 95%, 90%, 85%, or 80% identity to the nucleotide sequence of nucleotides 49 to 3819 of SEQ ID NO: 2, 8 or 9; and/or
   (ii) a SARS-CoV-2 S protein, an immunogenic variant thereof, or an immunogenic fragment of the SARS-CoV-2 S protein or the immunogenic variant thereof comprises the amino acid sequence of amino acids 17 to 1273 of SEQ ID NO: 1 or 7, an amino acid sequence having at least 99%, 98%, 97%, 96%, 95%, 90%, 85%, or 80% identity to the amino acid sequence of amino acids 17 to 1273 of SEQ ID NO: 1 or 7, or an immunogenic fragment of the amino acid sequence of amino acids 17 to 1273 of SEQ ID NO: 1 or 7, or the amino acid sequence having at least 99%, 98%, 97%, 96%, 95%, 90%, 85%, or 80% identity to the amino acid sequence of amino acids 17 to 1273 of SEQ ID NO: 1 or 7.
295. The method of any one of embodiments 289 to 294, wherein the amino acid sequence comprising a SARS-CoV-2 S protein, an immunogenic variant thereof, or an immunogenic fragment of the SARS-CoV-2 S protein or the immunogenic variant thereof comprises a secretory signal peptide.
296. The method of embodiment 295, wherein the secretory signal peptide is fused, preferably N-terminally, to a SARS-CoV-2 S protein, an immunogenic variant thereof, or an immunogenic fragment of the SARS-CoV-2 S protein or the immunogenic variant thereof.
297. The method of embodiment 295 or 297, wherein
   (i) the RNA encoding the secretory signal peptide comprises the nucleotide sequence of nucleotides 1 to 48 of SEQ ID NO: 2, 8 or 9, a nucleotide sequence having at least 99%, 98%, 97%, 96%, 95%, 90%, 85%, or 80% identity to the nucleotide sequence of nucleotides 1 to 48 of SEQ ID NO: 2, 8 or 9, or a fragment of the nucleotide sequence of nucleotides 1 to 48 of SEQ ID NO: 2, 8 or 9, or the nucleotide sequence having at least 99%, 98%, 97%, 96%, 95%, 90%, 85%, or 80% identity to the nucleotide sequence of nucleotides 1 to 48 of SEQ ID NO: 2, 8 or 9; and/or
   (ii) the secretory signal peptide comprises the amino acid sequence of amino acids 1 to 16 of SEQ ID NO: 1, an amino acid sequence having at least 99%, 98%, 97%, 96%, 95%, 90%, 85%, or 80% identity to the amino acid sequence of amino acids 1 to 16 of SEQ ID NO: 1, or a functional fragment of the amino acid sequence of amino acids 1 to 16 of SEQ ID NO: 1, or the amino acid sequence having at least 99%, 98%, 97%, 96%, 95%, 90%, 85%, or 80% identity to the amino acid sequence of amino acids 1 to 16 of SEQ ID NO: 1.
298. The method of any one of embodiments 289 to 297, wherein
   (i) the RNA encoding a SARS-CoV-2 S protein, an immunogenic variant thereof, or an immunogenic fragment of the SARS-CoV-2 S protein or the immunogenic variant thereof comprises the nucleotide sequence of SEQ ID NO: 6, a nucleotide sequence having at least 99%, 98%, 97%, 96%, 95%, 90%, 85%, or 80% identity to the nucleotide sequence of SEQ ID NO: 6, or a fragment of the nucleotide sequence of SEQ ID NO: 6, or the nucleotide sequence having at least 99%, 98%, 97%, 96%, 95%, 90%, 85%, or 80% identity to the nucleotide sequence of SEQ ID NO: 6; and/or
   (ii) a SARS-CoV-2 S protein, an immunogenic variant thereof, or an immunogenic fragment of the SARS-CoV-2 S protein or the immunogenic variant thereof comprises the amino acid sequence of SEQ ID NO: 5, an amino acid sequence having at least 99%, 98%, 97%, 96%, 95%, 90%, 85%, or 80% identity to the amino acid sequence of SEQ ID NO: 5, or an immunogenic fragment of the amino acid sequence of SEQ ID NO: 5, or the amino acid sequence having at least 99%, 98%, 97%, 96%, 95%, 90%, 85%, or 80% identity to the amino acid sequence of SEQ ID NO: 5.
299. The method of any one of embodiments 289 to 298, wherein
   (i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 54 to 986 of SEQ ID NO: 30, a nucleotide sequence having at least 99%, 98%, 97%, 96%, 95%, 90%, 85%, or 80% identity to the nucleotide sequence of nucleotides 54 to 986 of SEQ ID NO: 30, or a fragment of the nucleotide sequence of nucleotides 54 to 986 of SEQ ID NO: 30, or the nucleotide sequence having at least 99%, 98%, 97%, 96%, 95%, 90%, 85%, or 80% identity to the nucleotide sequence of nucleotides 54 to 986 of SEQ ID NO: 30; and/or
   (ii) a SARS-CoV-2 S protein, an immunogenic variant thereof, or an immunogenic fragment of the SARS-CoV-2 S protein or the immunogenic variant thereof comprises the amino acid sequence of amino acids 1 to 311 of SEQ ID NO: 29, an amino acid sequence having at least 99%, 98%, 97%, 96%, 95%, 90%, 85%, or 80% identity to the amino acid sequence of amino acids 1 to 311 of SEQ ID NO: 29, or an immunogenic fragment of the amino acid sequence of amino acids 1 to 311 of SEQ ID NO: 29, or the amino acid sequence having at least 99%, 98%, 97%, 96%, 95%, 90%, 85%, or 80% identity to the amino acid sequence of amino acids 1 to 311 of SEQ ID NO: 29.
300. The method of any one of embodiments 289 to 298, wherein the RNA comprises a modified nucleoside in place of uridine, in particular wherein the modified nucleoside is selected from pseudouridine (ψ), N1-methyl-pseudouridine (m1ψ), and 5-methyl-uridine (m5U), in particular wherein the modified nucleoside is N1-methyl-pseudouridine (m1ψ).
301. The method of any one of embodiments 289 to 300, wherein the RNA comprises a cap.
302. The method of any one of embodiments 289 to 301, wherein the RNA encoding an amino acid sequence comprising a SARS-CoV-2 S protein, an immunogenic variant thereof, or an immunogenic fragment of the SARS-CoV-2 S protein or the immunogenic variant thereof comprises a 5' UTR comprising the nucleotide sequence of SEQ ID NO: 12, or a nucleotide sequence having at least 99%, 98%, 97%, 96%, 95%, 90%, 85%, or 80% identity to the nucleotide sequence of SEQ ID NO: 12.
303. The method of any one of embodiments 289 to 302, wherein the RNA encoding an amino acid sequence comprising a SARS-CoV-2 S protein, an immunogenic variant thereof, or an immunogenic fragment of the SARS-CoV-2 S protein or the immunogenic variant thereof comprises a 3' UTR comprising the nucleotide sequence of SEQ ID NO: 13, or a nucleotide sequence having at least 99%, 98%, 97%, 96%, 95%, 90%, 85%, or 80% identity to the nucleotide sequence of SEQ ID NO: 13.
304. The method of any one of embodiments 289 to 303, wherein the RNA encoding an amino acid sequence comprising a SARS-CoV-2 S protein, an immunogenic variant thereof, or an immunogenic fragment of the SARS-CoV-2 S protein or the immunogenic variant thereof comprises a poly-A sequence.
305. The method of embodiment 304, wherein the poly-A sequence comprises at least 100 nucleotides.
306. The method of embodiment 304 or 305, wherein the poly-A sequence comprises or consists of the nucleotide sequence of SEQ ID NO: 14.
307. The method of any one of embodiments 289 to 306, wherein the RNA is formulated as a liquid, a solid, or a combination thereof.
308. The method of any one of embodiments 289 to 307, wherein the RNA is administered by injection.
309. The method of any one of embodiments 289 to 308, wherein the RNA is administered by intramuscular administration.
310. The method of any one of embodiments 289 to 309, wherein the RNA is formulated as particles.
311. The method of embodiment 310, wherein the particles are lipid nanoparticles (LNP) or lipoplex (LPX) particles.
312. The method of embodiment 311, wherein the LNP particles comprise ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoyl-sn-glycero-3-phosphocholine, and cholesterol.
313. The method of any one of embodiment 311, wherein the RNA lipoplex particles are obtainable by mixing the RNA with liposomes.
314. The method of any one of embodiments 289 to 313, wherein the RNA is mRNA or saRNA.
315. The method of any one of embodiments 289 to 314, which is a method for vaccination against coronavirus.
316. The method of any one of embodiments 289 to 315, which is a method for therapeutic or prophylactic treatment of a coronavirus infection.
317. The method of any one of embodiments 289 to 316, wherein the subject is a human.
318. The method of any one of embodiments 289 to 317, wherein the coronavirus is a betacoronavirus.
319. The method of any one of embodiments 289 to 318, wherein the coronavirus is a sarbecovirus.
320. The method of any one of embodiments 289 to 319, wherein the coronavirus is SARS-CoV-2.
321. The method of any one of embodiments 289 to 320, wherein the composition is a composition of any one of embodiments 1 to 39.
322. A composition or medical preparation of any one of embodiments 250 to 288 for use in a method of any one of embodiments 289 to 320.
323. An immunogenic composition comprising: a lipid nanoparticle (LNP) comprising an RNA, wherein the RNA encodes the polypeptide of SEQ ID NO: 49 and comprises the nucleotide sequence of SEQ ID NO: 50 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 50, and wherein the RNA comprises:
   (a) modified uridines;
   (b) a 5' cap; and
      wherein the LNP comprises ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoyl-sn-glycero-3-phosphocholine, and cholesterol.
324. The immunogenic composition of embodiment 323, wherein the nucleotide sequence includes modified uridines in place of all uridines.
325. The immunogenic of embodiment 323 or 324, wherein the modified uridines are each N1-methyl-pseudouridine.
326. The immunogenic composition of any one of embodiments 323 to 325, wherein the RNA further comprises at least one, at least two, or all of the following features:
   a 5' untranslated region (UTR) comprising SEQ ID NO: 12;
   a 3' untranslated region (UTR) comprising SEQ ID NO: 13; and
   a poly-A sequence of at least 100 A nucleotides.
327. The immunogenic composition of embodiment 326, wherein the poly-A sequence comprises 30 adenine nucleotides followed by 70 adenine nucleotides, wherein the 30 adenine nucleotides and 70 adenine nucleotides are separated by a linker sequence.
328. The immunogenic composition of embodiment 326, wherein the poly-A sequence comprises SEQ ID NO: 14.
329. The immunogenic composition of any one of embodiments 323 to 328, wherein the RNA comprises SEQ ID NO: 51.
330. An immunogenic composition comprising a lipid nanoparticle (LNP) comprising an RNA, wherein the RNA encodes the polypeptide of SEQ ID NO: 55 and comprises the nucleotide sequence of SEQ ID NO: 56 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 56, and wherein the RNA comprises:
   (a) modified uridines;
   (b) a 5' cap; and
      wherein the LNP comprises ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoyl-sn-glycero-3-phosphocholine, and cholesterol.
331. The immunogenic composition of embodiment 330, wherein the nucleotide sequence includes modified uridines in place of all uridines.
332. The immunogenic of embodiment 330 or 331, wherein the modified uridines are each N1-methyl-pseudouridine.
333. The immunogenic composition of any one of embodiments 330 to 332, wherein the RNA further comprises at least one, at least two, or all of the following features:
   a 5' untranslated region (UTR) comprising SEQ ID NO: 12;
   a 3' untranslated region (UTR) comprising SEQ ID NO: 13; and
   a poly-A sequence of at least 100 A nucleotides.
334. The immunogenic composition of embodiment 333, wherein the poly-A sequence comprises 30 adenine nucleotides followed by 70 adenine nucleotides, wherein the 30 adenine nucleotides and 70 adenine nucleotides are separated by a linker sequence.
335. The immunogenic composition of embodiment334, wherein the poly-A sequence comprises SEQ ID NO: 14.
336. The immunogenic composition of any one of embodiments 330 to 335, wherein the RNA comprises SEQ ID NO: 57.
337. An immunogenic composition comprising a a lipid nanoparticle (LNP) comprising an RNA, wherein the RNA encodes the polypeptide of SEQ ID NO: 58 and comprises the nucleotide sequence of SEQ ID NO: 59 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 59, and wherein the RNA comprises:
   (a) modified uridines;
   (b) a 5' cap; and
      wherein the LNP comprises ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoyl-sn-glycero-3-phosphocholine, and cholesterol.
338. The immunogenic composition of embodiment 337, wherein the nucleotide sequence includes modified uridines in place of all uridines.
339. The immunogenic of embodiment 337 or 338, wherein the modified uridines are each N1-methyl-pseudouridine.
340. The immunogenic composition of any one of embodiments 337 to 339, wherein the RNA further comprises at least one, at least two, or all of the following features:
   a 5' untranslated region (UTR) comprising SEQ ID NO: 12;
   a 3' untranslated region (UTR) comprising SEQ ID NO: 13; and
   a poly-A sequence of at least 100 A nucleotides.
341. The immunogenic composition of embodiment 340, wherein the poly-A sequence comprises 30 adenine nucleotides followed by 70 adenine nucleotides, wherein the 30 adenine nucleotides and 70 adenine nucleotides are separated by a linker sequence.
342. The immunogenic composition of embodiment 341, wherein the poly-A sequence comprises SEQ ID NO: 14.
343. The immunogenic composition of any one of embodiments 337 to 342, wherein the RNA comprises SEQ ID NO: 60.
344. An immunogenic composition comprising a lipid nanoparticle (LNP) comprising an RNA, wherein the RNA encodes the polypeptide of SEQ ID NO: 61 and comprises the nucleotide sequence of SEQ ID NO: 62a or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 62a, and wherein the RNA comprises:
   (a) modified uridines;
   (b) a 5' cap; and
      wherein the LNP comprises ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoyl-sn-glycero-3-phosphocholine, and cholesterol.
345. The immunogenic composition of embodiment 344, wherein the nucleotide sequence includes modified uridines in place of all uridines.
346. The immunogenic of embodiment 344 or 345, wherein the modified uridines are each N1-methyl-pseudouridine.
347. The immunogenic composition of any one of embodiments 344 to 346, wherein the RNA further comprises at least one, at least two, or all of the following features:
   a 5' untranslated region (UTR) comprising SEQ ID NO: 12;
   a 3' untranslated region (UTR) comprising SEQ ID NO: 13; and
   a poly-A sequence of at least 100 A nucleotides.
348. The immunogenic composition of embodiment 347, wherein the poly-A sequence comprises 30 adenine nucleotides followed by 70 adenine nucleotides, wherein the 30 adenine nucleotides and 70 adenine nucleotides are separated by a linker sequence.
349. The immunogenic composition of embodiment 348, wherein the poly-A sequence comprises SEQ ID NO: 14.
350. The immunogenic composition of any one of embodiments 344 to 349, wherein the RNA comprises SEQ ID NO: 63a.
351. An immunogenic composition comprising a first RNA and a second RNA, wherein:
   the first RNA encodes the polypeptide of SEQ ID NO: 7 and comprises the nucleotide sequence of SEQ ID NO: 9 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 9, and
   the second RNA encodes the polypeptide of SEQ ID NO: 49 and comprises the nucleotide sequence of SEQ ID NO: 50 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 50, and
   wherein each of the first RNA and the second RNA comprise:
      (a) modified uridines; and
      (b) a 5' cap, and
   wherein the first RNA and the second RNA are formulated in lipid nanoparticles (LNPs), wherein the LNPs comprise ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoyl-sn-glycero-3-phosphocholine, and cholesterol.
352. The immunogenic composition of embodiment 351, wherein the first RNA and the second RNA are formulated in the same lipid nanoparticles.
353. The immunogenic composition of embodiment 351, wherein the first RNA and the second RNA are formulated in seperate lipid nanoparticles.
354. The immunogenic composition of any one of embodiments 351 to 353, wherein each of the first RNA and the second RNA include modified uridines in place of all uridines.
355. The immunogenic of any one of embodiments 351 to 354, wherein the modified uridines are each N1-methyl-pseudouridine.
356. The immunogenic composition of any one of embodiments 351 to 355, wherein the first RNA and the second RNA each indepedently comprise at least one, at least two, or all of the following features:
   a 5' untranslated region (UTR) comprising SEQ ID NO: 12;
   a 3' untranslated region (UTR) comprising SEQ ID NO: 13; and
   a poly-A sequence of at least 100 A nucleotides.
357. The immunogenic composition of embodiment 357, wherein the poly-A sequence comprises 30 adenine nucleotides followed by 70 adenine nucleotides, wherein the 30 adenine nucleotides and 70 adenine nucleotides are separated by a linker sequence.
358. The immunogenic composition of embodiment 357, wherein the poly-A sequence comprises SEQ ID NO: 14.
359. The immunogenic composition of any one of embodiments 351 to 358, wherein the first RNA comprises SEQ ID NO: 20 and the second RNA comprises SEQ ID NO: 51.
360. An immunogenic composition comprising a first RNA and a second RNA, wherein:
   the first RNA encodes the polypeptide of SEQ ID NO: 7 and comprises the nucleotide sequence of SEQ ID NO: 9 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 9, and
   the second RNA encodes the polypeptide of SEQ ID NO: 55, 58, or 61 and comprises the nucleotide sequence of SEQ ID NO: 56, 59, or 62a, or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 56, 59, or 62a, and
   wherein each of the first RNA and the second RNA comprise:
      (a) modified uridines; and
      (b) a 5' cap, and
   wherein the first RNA and the second RNA are formulated in lipid nanoparticles (LNPs), wherein the LNPs comprise ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoyl-sn-glycero-3-phosphocholine, and cholesterol.
361. The immunogenic composition of embodiment 360, wherein the first RNA and the second RNA are formulated in separate lipid nanoparticles.
362. The immunogenic composition of embodiment 360, wherein the first RNA and the second RNA are formulated in the same lipid nanoparticles.
363. The immunogenic composition of any one of embodiments 360 to 362, wherein the first RNA and the second RNA each include modified uridines in place of all uridines.
364. The immunogenic composition of any one of embodiments 360 to 363, wherein the modified uridines are each N1-methyl-pseudouridine.
365. The immunogenic composition of any one of embodiments 360 to 364, wherein the first RNA and the second RNA each independently further comprise at least one, at least two, or all of the following features:
   a 5' untranslated region (UTR) comprising SEQ ID NO: 12;
   a 3' untranslated region (UTR) comprising SEQ ID NO: 13; and
   a poly-A sequence of at least 100 A nucleotides.
366. The immunogenic composition of any one of embodiments 360 to 365, wherein the poly-A sequence comprises 30 adenine nucleotides followed by 70 adenine nucleotides, wherein the 30 adenine nucleotides and 70 adenine nucleotides are separated by a linker sequence.
367. The immunogenic composition of embodiment 366, wherein the poly-A sequence comprises SEQ ID NO: 14.
368. The immunogenic composition of any one of embodiments 360 to 367, wherein the first RNA comprises SEQ ID NO: 9 and the second RNA comprises SEQ ID NO: 56.
369. The immunogenic composition of any one of embodiments 360 to 368, wherein the first RNA comprises SEQ ID NO: 9 and the second RNA comprises SEQ ID NO: 59.
370. The immunogenic composition of any one of embodiments 360 to 368, wherein the first RNA comprises SEQ ID NO: 9 and the second RNA comprises SEQ ID NO: 62a.
371. The immunogenic composition of any one of embodiments 360 to 368, wherein the first RNA comprises SEQ ID NO: 20 and the second RNA comprises SEQ ID NO: 57.
372. The immunogenic composition of any one of embodiments 360 to 368, wherein the first RNA comprises SEQ ID NO: 20 and the second RNA comprises SEQ ID NO: 60.
373. The immunogenic composition of any one of embodiments 360 to 368, wherein the first RNA comprises SEQ ID NO: 20 and the second RNA comprises SEQ ID NO: 63a.
374. An immunogenic composition comprising a first RNA and a second RNA, wherein:
   the first RNA encodes the polypeptide of SEQ ID NO: 58 and comprises the nucleotide sequence of SEQ ID NO: 59 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 59, and
   the second RNA encodes the polypeptide of SEQ ID NO: 49, 55, or 61 and comprises the nucleotide sequence of SEQ ID NO: 50, 56, or 62a, or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 50, 56, or 62a, and
   wherein each of the first RNA and the second RNA comprise:
      (a) modified uridines; and
      (b) a 5' cap, and
   wherein the first RNA and the second RNA are formulated in lipid nanoparticles (LNPs), wherein the LNPs comprise ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoyl-sn-glycero-3-phosphocholine, and cholesterol.
375. The immunogenic composition of embodiment 374, wherein the first RNA and the second RNA are formulated in separate lipid nanoparticles.
376. The immunogenic composition of embodiment 374, wherein the first RNA and the second RNA are formulated in the same lipid nanoparticles.
377. The immunogenic composition of any one of embodiments 374 to 376, wherein the first RNA and the second RNA each include modified uridines in place of all uridines.
378. The immunogenic of any one of embodiments 374 to 377, wherein the modified uridines are each N1-methyl-pseudouridine.
379. The immunogenic composition of any one of embodiments 374 to 378, wherein the first RNA and the second RNA each independently further comprise at least one, at least two, or all of the following features:
   a 5' untranslated region (UTR) comprising SEQ ID NO: 12;
   a 3' untranslated region (UTR) comprising SEQ ID NO: 13; and
   a poly-A sequence of at least 100 A nucleotides.
380. The immunogenic composition of embodiment 379, wherein the poly-A sequence comprises 30 adenine nucleotides followed by 70 adenine nucleotides, wherein the 30 adenine nucleotides and 70 adenine nucleotides are separated by a linker sequence.
381. The immunogenic composition of embodiment 379, wherein the poly-A sequence comprises SEQ ID NO: 14.
382. The immunogenic composition of any one of embodiments 374 to 381, wherein the first RNA comprises SEQ ID NO: 59 and the second RNA comprises SEQ ID NO: 50.
383. The immunogenic composition of any one of embodiments 374 to 381, wherein the first RNA comprises SEQ ID NO: 59 and the second RNA comprises SEQ ID NO: 56.
384. The immunogenic composition of any one of embodiments 374 to 381, wherein the first RNA comprises SEQ ID NO: 59 and the second RNA comprises SEQ ID NO: 62a.
385. The immunogenic composition of any one of embodiments 374 to 381, wherein the first RNA comprises SEQ ID NO: 60 and the second RNA comprises SEQ ID NO: 51.
386. The immunogenic composition of any one of embodiments 374 to 381, wherein the first RNA comprises SEQ ID NO: 60 and the second RNA comprises SEQ ID NO: 57.
387. The immunogenic composition of any one of embodiments 374 to 381, wherein the first RNA comprises SEQ ID NO: 60 and the second RNA comprises SEQ ID NO: 63a.
388. An immunogenic composition comprising a first RNA and a second RNA, wherein:
   the first RNA encodes the polypeptide of SEQ ID NO: 49 and comprises the nucleotide sequence of SEQ ID NO: 50 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 50, and
   the second RNA encodes the polypeptide of SEQ ID NO: 55 or 61 and comprises the nucleotide sequence of SEQ ID NO: 56 or 62a, or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 56 or 62a, and
   wherein each of the first RNA and the second RNA comprise:
      (a) modified uridines; and
      (b) a 5' cap, and
   wherein the first RNA and the second RNA are formulated in lipid nanoparticles (LNPs), wherein the LNPs comprise ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoyl-sn-glycero-3-phosphocholine, and cholesterol.
389. The immunogenic composition of embodiment 388, wherein the first RNA and the second RNA are formulated in separate lipid nanoparticles.
390. The immunogenic composition of embodiment 388, wherein the first RNA and the second RNA are formulated in the same lipid nanoparticles.
391. The immunogenic composition of any one of embodiments 388 to 390, wherein the first RNA and the second RNA each include modified uridines in place of all uridines.
392. The immunogenic of any one of embodiments 388 to 391, wherein the modified uridines are each N1-methyl-pseudouridine.
393. The immunogenic composition of any one of embodiments 388 to 392, wherein the first RNA and the second RNA further each independently further comprise at least one, at least two, or all of the following features:
   a 5' untranslated region (UTR) comprising SEQ ID NO: 12;
   a 3' untranslated region (UTR) comprising SEQ ID NO: 13; and
   a poly-A sequence of at least 100 A nucleotides.
394. The immunogenic composition of embodiment 393, wherein the poly-A sequence comprises 30 adenine nucleotides followed by 70 adenine nucleotides, wherein the 30 adenine nucleotides and 70 adenine nucleotides are separated by a linker sequence.
395. The immunogenic composition of embodiment 393, wherein the poly-A sequence comprises SEQ ID NO: 14.
396. The immunogenic composition of any one of embodiments 388 to 395, wherein the first RNA comprises SEQ ID NO: 50 and the second RNA comprises SEQ ID NO: 56.
397. The immunogenic composition of any one of embodiments 388 to 395, wherein the first RNA comprises SEQ ID NO: 50 and the second RNA comprises SEQ ID NO: 62a.
398. The immunogenic composition of any one of embodiments 388 to 395, wherein the first RNA comprises SEQ ID NO: 51 and the second RNA comprises SEQ ID NO: 57.
399. The immunogenic composition of any one of embodiments 388 to 395, wherein the first RNA comprises SEQ ID NO: 51 and the second RNA comprises SEQ ID NO: 63a.
400. An immunogenic composition comprising a first RNA and a second RNA, wherein:
   the first RNA encodes the polypeptide of SEQ ID NO: 55 and comprises the nucleotide sequence of SEQ ID NO: 56 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 56, and
   the second RNA encodes the polypeptide of SEQ ID NO: 61 and comprises the nucleotide sequence of SEQ ID NO: 62a, or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 62a, and
   wherein each of the first RNA and the second RNA comprise:
      (a) modified uridines; and
      (b) a 5' cap, and
   wherein the first RNA and the second RNA are formulated in lipid nanoparticles (LNPs), wherein the LNPs comprise ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoyl-sn-glycero-3-phosphocholine, and cholesterol.
401. The immunogenic composition of embodiment 400, wherein the first RNA and the second RNA are formulated in separate lipid nanoparticles.
402. The immunogenic composition of embodiment 400, wherein the first RNA and the second RNA are formulated in the same lipid nanoparticles.
403. The immunogenic composition of any one of embodiments 400 to 402, wherein the first RNA and the second RNA each include modified uridines in place of all uridines.
404. The immunogenic of any one of embodiments 400 to 403, wherein the modified uridines are each N1-methyl-pseudouridine.
405. The immunogenic composition of any one of embodiments 400 to 404, wherein the first RNA and the second RNA each independently further comprise at least one, at least two, or all of the following features:
   a 5' untranslated region (UTR) comprising SEQ ID NO: 12;
   a 3' untranslated region (UTR) comprising SEQ ID NO: 13; and
   a poly-A sequence of at least 100 A nucleotides.
406. The immunogenic composition of embodiment 405, wherein the poly-A sequence comprises 30 adenine nucleotides followed by 70 adenine nucleotides, wherein the 30 adenine nucleotides and 70 adenine nucleotides are separated by a linker sequence.
407. The immunogenic composition of embodiment 405, wherein the poly-A sequence comprises SEQ ID NO: 14.
408. The immunogenic composition of any one of embodiments 400 to 407, wherein the first RNA comprises SEQ ID NO: 57 and the second RNA comprises SEQ ID NO: 63a.
409. The immunogenic composition of any one of embodiments 323 to 408, wherein the 5'-cap is or comprises m₂^{7,3'-O}Gppp(m₁^{2'-O})ApG.
410. The immunogenic composition of any one of embodiments 323 to 409, wherein the LNP comprises about 40 to about 50 mole percent ((4-hydroxybutyl)azanediyl)bis(hexane-6,1-diyl)bis(2-hexyldecanoate), about 35 to about 45 mole percent cholesterol, about 5 to about 15 mole percent 1,2-Distearoyl-sn-glycero-3-phosphocholine, and about 1 to about 10 mole percent 2-[(polyethylene glycol)-2000]-N,N-ditetradecylacetamide.
411. The immunogenic composition of any one of embodiments 323 to 410, wherein the composition comprises a plurality of LNPs, wherein the average diameter of the plurality of LNPs is about 30 nm to about 200 nm or about 60 nm to about 120 nm (e.g., as determined by dynamic light scattering measurements).
412. A method of eliciting an immune response against SARS-CoV-2 comprising administering the immunogenic composition of any one of embodiments 74 to 162.
413. The method of embodiment 413, wherein the immune response is elicited against an Omicron variant of SARS-CoV-2.
414. The method of embodiment 412, wherein the immune response is elicited against a Beta variant of SARS-CoV-2.
415. The method of embodiment 412, wherein the immune response is elicited against an Alpha variant of SARS-CoV-2.
416. The method of embodiment 412, wherein the immune response is elicited against a Delta variant of SARS-CoV-2.
417. The method of embodiment 412, wherein the immune response is elicited against a Wuhan strain, an Omicron variant, a Beta variant, an Alpha variant, and a Delta variant of SARS-CoV-2.
418. A method for inducing an immune response in a subject, wherein the method comprises delivering (e.g., as a polypeptide or an RNA encoding such a polypeptide) an antigen of a SARS-CoV-2 virus that is not a BA.1 Omicron variant of SARS-CoV-2.
419. The method of embodiment 418, wherein the subject has previously been infected with or vaccinated against SARS-CoV-2.
420. The method of embodiment 418 or 419, wherein the subject has previously been delivered (e.g., as a polypeptide or an RNA encoding such a polypeptide) an antigen of a Wuhan strain of SARS-CoV-2.
421. The method of any one of embodiments 418-420, wherein the subject has previously been administered RNA encoding a SARS-CoV-2 S protein of a Wuhan strain.
422. The method of any one of embodiments 418-421, wherein the subject has previously been administered two or more doses of RNA encoding a SARS-CoV-2 S protein of a Wuhan strain.
423. The method of any one of embodiments 418-422, wherein the method comprises administering RNA encoding an antigen of a SARS-CoV-2 virus that is not a BA.1 Omicron variant.
424. The method of any one of embodiments 418-423, wherein the method comprises administering RNA encoding a SARS-CoV-2 S protein from a SARS-CoV-2 variant that is a BA.1 Omicron variant.
425. The method of any one of embodiments 418-424, wherein the method comprises administering RNA encoding an S protein of an Omicron variant of SARS-CoV-2, wherein the Omicron variant is not a BA.1 Omicron variant.
426. The method of any one of embodiments 418-425, wherein the method comprises administering RNA encoding an S protein of a BA.2 Omicron variant of SARS-CoV-2.
427. The method of any one of embodiments 418-425, wherein the method comprises administering RNA encoding an S protein of a BA.4 or BA.5 Omicron variant of SARS-CoV-2.
428. A method for inducing an immune response in a subject, wherein the method comprises administering (a) a first RNA encoding a SARS-CoV-2 S protein of a Wuhan strain, an Alpha variant, a Beta variant, or a Delta variant, and (b) a second RNA encoding a SARS-CoV-2 S protein of an Omicron variant that is not a BA.1 Omicron variant.
429. The method of embodiment 428, where the second RNA encodes an S protein of a BA.2 Omicron variant.
430. The method of embodiment 428, where the second RNA encodes an S protein of a BA.4 or BA.5 Omicron variant.
431. A method for inducing an immune response in a subject, wherein the method comprises administering (a) a first RNA encoding a SARS-CoV-2 S protein of a Wuhan strain, an Alpha variant, a Beta variant, a Delta variant, or a BA.1 Omicron variant and (b) a second RNA encoding a SARS-CoV-2 S protein that is antigenically distinct from the S protein encoded by the first RNA.
432. The method of embodiment 431, wherein the second RNA encodes a SARS-CoV-2 S protein of an Omicron variant that is not a BA.1 Omicron variant.
433. The method of embodiment 431, wherein the second RNA encodes a SARS-CoV-2 S protein of a BA.2 Omicron variant.
434. The method of embodiment 431, wherein the second RNA encodes a SARS-CoV-2 S protein of a BA.4 or BA.5 Omicron variant.
435. The method of any one of embodiments 418-434, wherein the first RNA and the second RNA are encapsulated in separate LNPs.
436. The method of any one of embodiments 418-435, wherein the first RNA and the second RNA are encapsulated in the same LNP.
437. The method of any one of embodiments 418-436, wherein the first RNA and the second RNA are administered separately, e.g., at different injection sites.
438. A composition comprising (a) a first RNA encoding a SARS-CoV-2 S protein of a Wuhan strain, an Alpha variant, a Beta variant, a Delta variant, or a BA.1 Omicron variant and (b) a second RNA encoding a SARS-CoV-2 S protein that is antigenically distinct from the S protein encoded by the first RNA.
439. A composition comprising (a) a first RNA encoding a SARS-CoV-2 S protein of a Wuhan strain, an Alpha variant, a Beta variant, a Delta variant, or a BA.1 Omicron variant and (b) a second RNA encoding a SARS-CoV-2 S protein an Omicron variant that is not a BA.1 Omicron variant.
440. The composition of embodiment 439, wherein the second RNA encodes a SARS-CoV-2 S protein of a BA.2 Omicron variant.
441. The composition of embodiment 439, wherein the second RNA encodes a a SARS-CoV-2 S protein of a BA.4 or BA.5 Omicron variant.
442. The composition of embodiment 439, wherein the first RNA encodes a SARS-CoV-2 S protein of a Wuhan strain and the second RNA encodes a SARS-CoV-2 S protein of a BA.2 Omicron variant.
443. The composition of embodiment 439, wherein the first RNA encodes a SARS-CoV-2 S protein of a Wuhan strain and the second RNA encodes a SARS-CoV-2 S protein of a BA.4 or BA.5 Omicron variant.
444. The composition of embodiment 439, wherein the first RNA encodes a SARS-CoV-2 S protein of a BA.1 Omicron variant and the second RNA encodes a SARS-CoV-2 S protein of a BA.2 Omicron variant.
445. The composition of embodiment 439, wherein the first RNA encodes a SARS-CoV-2 S protein of a BA.1 Omicron variant and the second RNA encodes a SARS-CoV-2 S protein of a BA.4 or BA.5 Omicron variant.
446. The composition of any one of embodiments 438-445, wherein the first RNA and the second RNA are administered in encapsulated in the same LNP.
447. The composition of any one of embodiments 438-446, wherein the first RNA and the second RNA are encapsulated in separate LNPs.
448. The method of any one of embodiments 214 to 249, 289 to 321, or 412 to 437, wherein the composition is a composition of any one of embodiments 1 to 213, 250 to 288, 322 to 411, or 438 to 448 .
448. A composition or medical preparation of any one of embodiments 1 to 213, 250 to 288, 322 to 411, or 438 to 448 for use in a method of any one of embodiments 214 to 249, 289 to 321, or 412 to 437.
449. The composition or medical preparation of any one of embodiments 1 to 213, 250 to 288, 322 to 411, or 438 to 448, which is a pharmaceutical composition.
450. The composition or medical preparation of any one of embodiments 1 to 213, 250 to 288, 322 to 411, or 438 to 448, which is a vaccine.
451. The composition or medical preparation of embodiment 449 or 450, wherein the pharmaceutical composition or vaccine further comprises one or more pharmaceutically acceptable carriers, diluents and/or excipients.
452. The composition or medical preparation of any one of embodiment 1 to 213, 250 to 288, 322 to 411, or 438 to 448, which is a kit.
453. The composition or medical preparation of embodiment 452, wherein the RNA and optionally the particle forming components are in separate vials.
454. The composition or medical preparation of embodiment 452 or 453, further comprising instructions for use of the composition or medical preparation for inducing an immune response against coronavirus in a subject.
455. The composition or medical preparation of any one of embodiments 1 to 213, 250 to 288, 322 to 411, or 438 to 448 for pharmaceutical use.
456. The composition or medical preparation of embodiment 455, wherein the pharmaceutical use comprises inducing an immune response against coronavirus in a subject.
457. The composition or medical preparation of embodiment 455 or 456, wherein the pharmaceutical use comprises a therapeutic or prophylactic treatment of a coronavirus infection.
458. The composition or medical preparation of any one of embodiments 1 to 213, 250 to 288, 322 to 411, or 438 to 448 for use in the manufacture of a medicament.
459. The composition or medical preparation of embodiment 458, wherein the medicament is for inducing an immune response against coronavirus in a subject.
460. The composition or medical preparation of embodiment 458 or 459, wherein the medicament is for therapeutic or prophylactic treatment of a coronavirus infection.
461. The composition or medical preparation of any one of embodiments 1 to 213, 250 to 288, 322 to 411, or 438 to 448, which is for administration to a human.
462. The composition or medical preparation of any one of embodiments 454, 456, 457, 459, or 460, wherein the coronavirus is a betacoronavirus.
463. The composition or medical preparation of embodiment 462, wherein the coronavirus is a sarbecovirus.
464. The composition or medical preparation of embodiment 463, wherein the coronavirus is SARS-CoV-2.

Citation of documents and studies referenced herein is not intended as an admission that any of the foregoing is pertinent prior art. All statements as to the contents of these documents are based on the information available to the applicants and do not constitute any admission as to the correctness of the contents of these documents.

The following description is presented to enable a person of ordinary skill in the art to make and use the various embodiments. Descriptions of specific devices, techniques, and applications are provided only as examples. Various modifications to the examples described herein will be readily apparent to those of ordinary skill in the art, and the general principles defined herein may be applied to other examples and applications without departing from the spirit and scope of the various embodiments. Thus, the various embodiments are not intended to be limited to the examples described herein and shown, but are to be accorded the scope consistent with the claims.

### Examples

### Example 1: Immunogenicity study of BNT162b3 variants BNT162b3c and BNT162b3d

To get an idea about the potential potency of transmembrane-anchored RBD-based vaccine antigens (Schematic in Figure 6; BNT162b3c (1) and BNT162b3d (2)), BALB/c mice were immunized IM once with 4 µg LNP-C12 formulated mRNA or with buffer as control. The non-clinical LNP-C12 formulated mRNAs were used as surrogate for the BNT162b3 variants BNT162b3c and BNT162b3d. The immunogenicity of the RNA vaccine was investigated by focusing on the antibody immune response.

ELISA data 6, 14 and 21 d after the first immunization show an early, dose-dependent immune activation against the S1 protein and the receptor binding domain (Figure 7). Sera obtained 6, 14 and 21 d after immunization show high SARS-CoV-2 pseudovirus neutralization, correlating with the increase of IgG antibody titers (Figure 8).

### Example 2: Neutralization of SARS-CoV-2 BA.1 Omicron lineage (a.k.a. B.1.1.529) pseudovirus by BNT162b2 vaccine-elicited human sera

### Materials and Methods:

A recombinant replication-deficient VSV vector that encodes green fluorescent protein (GFP) and luciferase (Luc) instead of the VSV-glycoprotein (VSV-G) was pseudotyped with Wuhan-Hu-1 isolate SARS-CoV-2 spike (S) (GenBank: QHD43416.1), and a variant spike harbouring the mutations found in the S protein of the Omicron (B.1.1.529) BA.1 lineage (A67V, Δ69-70, T951, G142D, Δ143-145, Δ211, L212!, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F) according to published pseudotyping protocols. In brief, HEK293T/17 monolayers transfected to express the respective SARS-CoV-2 S protein truncated of the C-terminal cytoplasmic 19 amino acids (SARS-CoV-2-S(CΔ19)) were inoculated with VSVΔG-GFP/Luc vector. After incubation for 2 h at 37 °C, the inoculum was removed, and cells were washed with PBS before medium supplemented with anti-VSV-G antibody (clone 8G5F11, Kerafast) was added to neutralise residual input virus. VSV-SARS-CoV-2 pseudovirus-containing medium was collected 20 h after inoculation, 0.2-µm-filtered and stored at -80 °C.

For pseudovirus neutralisation assays, 40,000 Vero 76 cells were seeded per 96-well. Sera were serially diluted 1:2 in culture medium starting with a 1:10 dilution (dilution range of 1:10 to 1:10,240). VSV-SARS-CoV-2-S pseudoparticles were diluted in culture medium for a fluorescent focus unit (ffu) count in the assay of ^{~}200 TU in the assay. Serum dilutions were mixed 1:1 with pseudovirus for 30 minutes at room temperature prior to addition to Vero 76 cell monolayers in 96-well plates and incubation at 37 °C for 16-24 hours. Supernatants were removed, and the cells were lysed with luciferase reagent (Promega). Luminescence was recorded, and neutralisation titers were calculated as the reciprocal of the highest serum dilution that still resulted in 50% reduction in luminescence. Results were reported as GMT of duplicates. If no neutralization was observed, an arbitrary titer value of 5 (half of the limit of detection [LOD]) was reported.

Sera (N=19-20) were collected from subjects 21 days after receiving the second 30 µg dose or one month after receiving the third 30 µg dose of BNT162b2. Each serum was tested for its neutralizing antibody titer against wild-type SARS-CoV-2 Wuhan Hu-1 and Omicron BA.1 lineage (B.1.1.529) spike protein pseudotyped VSV by a 50% neutralization assay (pVNT₅₀). The Omicron BA.1-strain spike protein used in the neutralization assay carried the following amino acid changes compared to the Wuhan reference: A67V, Δ69-70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F.

BNT162b2-immune sera generated at 21 days after the second dose displayed effective neutralization of the SARS-CoV-2 Wuhan Hu-1 pseudotyped reference. However, more than a 25-fold reduction in neutralization titers against the Omicron BA.1 variant was observed when compared to the Wuhan reference (geometric mean titer [GMT] of 6 vs. 155). Importantly, the third dose significantly increased the neutralizing antibody titers against the Omicron BA.1 strain pseudovirus by 25-fold. Hence, neutralization titers against the Omicron BA.1 variant pseudovirus after three doses of BNT162b2 were comparable to the neutralization titers against the wild-type strain observed in sera from individuals who received two doses of BNT162b2 (GMT of 154 vs. 155).

### Example 3: Additional data for neutralization of SARS-CoV-2 Omicron BA.1 lineage (a.k.a. B.1.1.529) pseudovirus by BNT162b2 vaccine-elicited human sera

Further to the study and data as described in Example 2, a longitudinal analysis of neutralizing titers was also performed in an independent smaller subset of subjects. Sera drawn at 21 days after dose 2 exhibited a 19.6-fold reduction in GMT against the Omicron BA.1 variant compared to the Wuhan reference pseudovirus (Fig. 12; GMT of 6 vs. 118). Serum obtained from study participants just prior to receiving the third dose of BNT162b2 (at a median 251 days following dose 2) had considerably reduced neutralizing titers against the Wuhan pseudovirus (GMT of 14) while the Omicron BA.1-specific titers were below the limit of detection. The third dose of BNT162b2 resulted in a significant increase in neutralizing titers against the Wuhan pseudovirus (GMT of 254) and a >26.6-fold increase in neutralizing titers against Omicron BA.1 at 1 month after dose 3 compared to titers at 21 days after dose 2 (GMT of 160 vs. 6). In all 9 subjects reduced but effective neutralization of Omicron BA.1 was observed up to 3 months after the third dose (3.2-fold reduction compared to 1 month after dose 3; GMT of 50 vs. 160), whereas Wuhan-specific neutralizing GMTs remained stable.

In summary, a third dose of BNT162b2 boosts Omicron BA.1 neutralization capability to a level similar to the one observed after two doses against the Wuhan pseudovirus. Thus, the data indicate that providing a third dose of BNT162b2 can improve protection against infection with the Omicron BA.1 variant.

### Example 4: Neutralization of other SARS-CoV-2 lineage pseudovirus by BNT162b2 vaccine-elicited human sera

As described in Example 2 and Example 3, each serum was also tested for its neutralizing antibody titer against Beta and Delta lineage spike protein pseudotyped VSV by a 50% neutralization assay (pVNT₅₀) (data not shown).

A recombinant replication-deficient VSV vector that encodes green fluorescent protein (GFP) and luciferase (Luc) instead of the VSV-glycoprotein (VSV-G) was pseudotyped with Wuhan-Hu-1 isolate SARS-CoV-2 spike (S) (GenBank: QHD43416.1), and a variant spike harbouring the mutations found in the S protein of the Beta lineage (mutations: L18F, D80A, D215G, R246I, Δ242-244, K417N, E484K, N501Y, D614G, A701V), and the Delta lineage (mutations: T19R, G142D, Δ157/158, K417N, L452R, T478K, D614G, P681R, D950N, K986P, V987P), according to published pseudotyping protocols.

For sera collected 21 days after a second dose of BNT162b2, PVNT₅₀ was reduced by approximately 6.7-fold (GMT of 24 vs 155) for the Beta variant and approximately 2.2-fold for the Delta variant (GMT of 73 vs 155) as compared to the Wuhan variant, but were significantly higher than the neutralization response against the delta variant. The third dose of BNT162b2 also increased neutralizing activity against Beta and Delta pseudoviruses, with GMTs of 279 and 413, respectively.

### Example 5: T cell epitope conservation in the Omicron BA.1 Spike variant

In addition to humoral immunity, T-cell mediated immunity is another layer of defense, in particular for preventing severe COVID-19. Previous observations that efficacy against disease is already established about 12 days after the first dose of BNT162b2 before the second dose has been administered and prior to the onset of high neutralizing titers further highlights the potential protective role of the T cell response. Prior reports have shown that CD8 T cell responses in individuals vaccinated with BNT162b2 are polyepitopic.

To assess the risk of immune evasion of CD8+ T cell responses by Omicron BA.1, a set of HLA class I restricted T cell epitopes from the Wuhan spike protein sequence that were reported in the Immune Epitope Database to be immunogenic (IEDB, n=244) were investigated (the procedure used to identify these epitopes is described in the below paragraph). Despite the multitude of mutations in the Omicron BA.1 spike protein, 85.25% (n=208) of the described epitopes were not impacted on the amino acid sequence level, indicating that the targets of the vast majority of T cell responses elicited by BNT162b2 may still be conserved in the Omicron BA.1 variant (Fig. 13). Early laboratory studies confirm that CD8+ T cell recognition of Omicron epitopes are preserved in COVID-19 recovered individuals exposed early in the pandemic and that the Omicron BA.1 VOC has not evolved extensive T-cell escape mutations at this time.

To estimate the rate of nonsynonymous mutation in T cell epitopes in the spike glycoprotein, the Immune Epitope Database (https://www.iedb.org/) was used to obtain epitopes confirmed for T cell reactivity in experimental assays. The database was filtered using the following criteria: Organism: SARS-COV2; Antigen: Spike glycoprotein; Positive Assay; No B cell assays; No MHC assays; MHC Restriction Type: Class I; Host: Homo sapiens (human). The resulting table was filtered by removing epitopes that were "deduced from a reactive overlapping peptide pool", as well as epitopes longer than 14 amino acids in order to restrict the dataset to confirmed minimal epitopes only. Of the 251 unique epitope sequences obtained in this approach, 244 were found in the Wuhan strain Spike glycoprotein. Of these, 36 epitopes (14.75%) included a position reported to be mutated in Omicron by the sequence analysis disclosed herein. Results are summarized in Figure 10. Also shown are the numbers of predicted MHC-I epitopes mutated in each of the Alpha, Beta, Gamma, Delta SARS-CoV-2 variants. Figure 13 depicts the locations of the T cell epitopes within the Spike Protein, and indicates which epitopes are conserved or mutated in the Spike protein from the Omicron BA.1 variant.

### Example 6: Exemplary Dosing Regimens

In some embodiments, compositions and methods disclosed herein can be used in accordance with an exemplary vaccination regimen as illustrated in Figure 14.

### Primary Dosing Regimens

In some embodiments, subjects are administered a primary dosing regimen. A primary dosing regimen can comprise one or more doses. For example, in some embodiments, a primary dosing regimen comprises a single dose (PD₁). In some embodiments a primary dosing regimen comprises a first dose (PD₁) and a second dose (PD₂). In some embodiments, a primary dosing regimen comprises a first dose, a second dose, and a third dose (PD₃). In some embodiments, a primary dosing regimen comprises a first dose, a second dose, a third dose, and one or more additional doses (PDₙ) of any one of the pharmaceutical compositions described herein.

In some embodiments, PD₁ comprises administering 1 to 100 ug of RNA. In some embodiments, PD₁ comprises administering 1 to 60 ug of RNA In some embodiments, PD₁ comprises administering 1 to 50 ug of RNA. In some embodiments, PD₁ comprises administering 1 to 30 ug of RNA. In some embodiments, PD₁ comprises administering about 3 ug of RNA. In some embodiments, PD₁ comprises administering about 5 ug of RNA. In some embodiments, PD₁ comprises administering about 10 ug of RNA. In some embodiments, PD₁ comprises administering about 15 ug of RNA. In some embodiments, PD₁ comprises administering about 20 ug of RNA. In some embodiments, PD₁ comprises administering about 30 ug of RNA. In some embodiments, PD₁ comprises administering about 50 ug of RNA. In some embodiments, PD₁ comprises administering about 60 ug of RNA.

In some embodiments, PD₂ comprises administering 1 to 100 ug of RNA. In some embodiments, PD₂ comprises administering 1 to 60 ug of RNA. In some embodiments, PD₂ comprises administering 1 to 50 ug of RNA. In some embodiments, PD₂ comprises administering 1 to 30 ug of RNA. In some embodiments, PD₂ comprises administering about 3 ug. In some embodiments, PD₂ comprises administering about 5 ug of RNA. In some embodiments, PD₂ comprises administering about 10 ug of RNA. In some embodiments, PD₂ comprises administering about 15 ug of RNA. In some embodiments, PD₂ comprises administering about 20 ug RNA. In some embodiments, PD₂ comprises administering about 30 ug of RNA. In some embodiments, PD₂ comprises administering about 50 ug of RNA. In some embodiments, PD₂ comprises administering about 60 ug of RNA.

In some embodiments, PD₃ comprises administering 1 to 100 ug of RNA. In some embodiments, PD₃ comprises administering 1 to 60 ug of RNA. In some embodiments, PD₃ comprises administering 1 to 50 ug of RNA. In some embodiments, PD₃ comprises administering 1 to 30 ug of RNA. In some embodiments, PD₃ comprises administering about 3 ug of RNA. In some embodiments, PD₃ comprises administering about 5 ug of RNA. In some embodiments, PD₃ comprises administering about 10 ug of RNA. In some embodiments, PD₃ comprises administering about 15 ug of RNA. In some embodiments, PD₃ comprises administering about 20 ug of RNA. In some embodiments, PD₃ comprises administering about 30 ug of RNA. In some embodiments, PD₃ comprises administering about 50 ug of RNA. In some embodiments, PD₃ comprises administering about 60 ug of RNA.

In some embodiments, PDₙ comprises administering 1 to 100 ug of RNA. In some embodiments, PDₙ comprises administering 1 to 60 ug of RNA. In some embodiments, PDₙ comprises administering 1 to 50 ug of RNA. In some embodiments, PDₙ comprises administering 1 to 30 ug of RNA. In some embodiments, PDₙ comprises administering about 3 ug of RNA. In some embodiments, PDₙ comprises administering about 5 ug of RNA. In some embodiments, PDₙ comprises administering about 10 ug of RNA. In some embodiments, PDₙ comprises administering about 15 ug of RNA. In some embodiments, PDₙ comprises administering about 20 ug of RNA. In some embodiments, PDₙ comprises administering about 30 ug of RNA. In some embodiments, PDₙ comprises administering about 50 ug of RNA. In some embodiments, PDₙ comprises administering about 60 ug of RNA.

In some embodiments, PD₁ comprises an RNA encoding a SARS-CoV-2 Spike protein or an immunogenic fragment thereof from the Wuhan strain. In some embodiments, PD₁ comprises an RNA encoding a Spike protein or an immunogenic fragment thereof from a SARS-CoV-2 strain that is prevalent and/or spreading rapidly in a relevant jurisdiction. In some embodiments, PD₁ comprises an RNA encoding a SARS-CoV-2 Spike protein or an immunogenic fragment thereof comprising one or more mutations from an alpha variant. In some embodiments, PD₁ comprises an RNA encoding a SARS-CoV-2 Spike protein or an immunogenic fragment thereof comprising one or more mutations from a delta variant. In some embodiments, PD₁ comprises an RNA encoding a SARS-CoV-2 Spike protein or an immunogenic fragment thereof comprising one or more mutations from a beta variant. In some embodiments, PD₁ comprises an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant). In some embodiments, PD₁ comprises an RNA encoding a SARS-CoV-2 Spike protein or an immunogenic fragment thereof from the Wuhan strain and one or more additional RNAs encoding a Spike protein or an immunogenic fragment thereof from a SARS-CoV-2 strain that is prevalent and/or spreading rapidly in a relevant jurisdiction. In some embodiments, PD₁ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an alpha variant. In some embodiments, PD₁ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, PD₁ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, PD₁ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, PD₂ comprises an RNA encoding a SARS-CoV-2 Spike protein or an immunogenic fragment thereof from the Wuhan strain. In some embodiments, PD₂ comprises an RNA encoding a Spike protein or an immunogenic fragment thereof from a SARS-CoV-2 strain that is prevalent and/or spreading rapidly in a relevant jurisdiction. In some embodiments, PD₂ comprises an RNA encoding a SARS-CoV-2 Spike protein or an immunogenic fragment thereof comprising one or more mutations from an alpha variant. In some embodiments, PD₂ comprises an RNA encoding a SARS-CoV-2 Spike protein or an immunogenic fragment thereof comprising one or more mutations from a delta variant. In some embodiments, PD₂ comprises an RNA encoding a SARS-CoV-2 Spike protein or an immunogenic fragment thereof comprising one or more mutations from a beta variant. In some embodiments, PD₂ comprises an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant). In some embodiments, PD₂ comprises an RNA encoding a SARS-CoV-2 Spike protein or an immunogenic fragment thereof from the Wuhan strain and one or more additional RNAs encoding a Spike protein or an immunogenic fragment thereof from a SARS-CoV-2 strain that is prevalent and/or spreading rapidly in a relevant jurisdiction. In some embodiments, PD₂ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an alpha variant. In some embodiments, PD₂ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, PD₂ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, PD₂ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, PD₃ comprises an RNA encoding a SARS-CoV-2 Spike protein or an immunogenic fragment thereof from the Wuhan strain. In some embodiments, PD₃ comprises an RNA encoding a Spike protein or an immunogenic fragment thereof from a SARS-CoV-2 strain that is prevalent and/or spreading rapidly in a relevant jurisdiction. In some embodiments, PD₃ comprises an RNA encoding a SARS-CoV-2 Spike protein or an immunogenic fragment thereof comprising one or more mutations from an alpha variant. In some embodiments, PD₃ comprises an RNA encoding a SARS-CoV-2 Spike protein or an immunogenic fragment thereof comprising one or more mutations from a delta variant. In some embodiments, PD₃ comprises an RNA encoding a SARS-CoV-2 Spike protein or an immunogenic fragment thereof comprising one or more mutations from a beta variant. In some embodiments, PD₃ comprises an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant). In some embodiments, PD₃ comprises an RNA encoding a SARS-CoV-2 Spike protein or an immunogenic fragment thereof from the Wuhan strain and one or more additional RNAs encoding a Spike protein or an immunogenic fragment thereof from a SARS-CoV-2 strain that is prevalent and/or spreading rapidly in a relevant jurisdiction. In some embodiments, PD₃ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an alpha variant. In some embodiments, PD₃ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, PD₃ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, PD₃ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, PDₙ comprises an RNA encoding a SARS-CoV-2 Spike protein or an immunogenic fragment thereof from the Wuhan strain. In some embodiments, PDₙ comprises an RNA encoding a Spike protein or an immunogenic fragment thereof from a SARS-CoV-2 strain that is prevalent and/or spreading rapidly in a relevant jurisdiction. In some embodiments, PDₙ comprises an RNA encoding a SARS-CoV-2 Spike protein or an immunogenic fragment thereof comprising one or more mutations from an alpha variant. In some embodiments, PDₙ comprises an RNA encoding a SARS-CoV-2 Spike protein or an immunogenic fragment thereof comprising one or more mutations from a delta variant. In some embodiments, PDₙ comprises an RNA encoding a SARS-CoV-2 Spike protein or an immunogenic fragment thereof comprising one or more mutations from a beta variant. In some embodiments, PDₙ comprises an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant). In some embodiments, PDₙ comprises an RNA encoding a SARS-CoV-2 Spike protein or an immunogenic fragment thereof from the Wuhan strain and one or more additional RNAs encoding a Spike protein or an immunogenic fragment thereof from a SARS-CoV-2 strain that is prevalent and/or spreading rapidly in a relevant jurisdiction. In some embodiments, PDₙ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an alpha variant. In some embodiments, PDₙ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, PDₙ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, PDₙ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, PD₁, PD₂, PD₃, and PDₙ can each independently comprise a plurality of (e.g., at least two) mRNA compositions described herein. In some embodiments PD₁, PD₂, PD₃, and PDₙ can each independently comprise a first and a second mRNA composition. In some embodiments, at least one of a plurality of mRNA compositions comprises BNT162b2 (e.g., as described herein). In some embodiments, at least one of a plurality of mRNA compositions comprises an mRNA encoding a SARS-CoV-2 S protein or an immunogenic fragment thereof from a different SARS-CoV-2 variant. In some embodiments, at least one of a plurality of mRNA compositions comprises an mRNA encoding a SARS-CoV-2 S protein or an immunogenic fragment thereof from a Wuhan strain of SARS-CoV-2. In some embodiments, at least one of a plurarity of mRNA compositions comprises an RNA encoding a SARS-CoV-2 S protein or an immunogenic fragment thereof comprising one or more mutations from a variant that is prevalent and/or spreading rapidly in a relevant jurisdiction. In some embodiments, at least one of a plurality of mRNA compositions comprises an mRNA encoding a SARS-CoV-2 S protein or an immunogenic fragment thereof comprising one or more mutations from an alpha variant. In some embodiments, at least one of a plurality of mRNA compositions comprises an mRNA encoding a SARS-CoV-2 S protein or an immunogenic fragment thereof comprising one or more mutations from a delta variant. In some embodiments, at least one of a plurality of mRNA compositions comprises an mRNA encoding a SARS-CoV-2 S protein or an immunogenic fragment thereof comprising one or more mutations from an Omicron variant (e.g., a BA.4/5, BA.1, BA.2, XBB, XBB.1, or BQ.1 Omicron variant).

In some embodiments, a plurality of mRNA compositions given in PD₁, PD₂, PD₃,and/or PDₙ can each independently comprise at least two different mRNA constructs (e.g., differing in at protein-encoding sequences). For example, in some embodiments a plurality of mRNA compositions given in PD₁, PD₂, PD₃, and/or PDₙ can each independently comprise an mRNA encoding a SARS-CoV-2 S protein or an immunogenic fragment thereof from a Wuhan strain of SARS-CoV-2 and an mRNA encoding a SARS-CoV-2 S protein or an immunogenic fragment thereof comprising one or more mutations from a variant that is prevalent and/or spreading rapidly in a relevant jurisdiction. In some embodiments a plurality of mRNA compositions given in PD₁, PD₂, PD₃, and/or PDₙ can each independently comprise an mRNA encoding a SARS-CoV-2 S protein or an immunogenic fragment thereof derived from a Wuhan strain of SARS-CoV-2 and an mRNA encoding a SARS-CoV-2 S protein or an immunogenic fragment thereof comprising one or more mutations from a variant that is prevalent and/or spreading rapidly in a relevant jurisdiction. In some such embodiments, a variant can be an alpha variant. In some such embodiments, a variant can be a delta variant. In some such embodiments a variant can be an Omicron variant (e.g., a BA.4/5, BA.1, BA.2, XBB, XBB.1, or BQ.1 Omicron variant). In some embodiments, each of a plurality of mRNA compositions given in PD₁, PD₂, PD₃, and/or PDₙ can independently comprise at least two mRNAs, each encoding a SARS-CoV-2 S protein or an immunogenic fragment thereof comprising one or more mutations from a distinct variant that is prevalent and/or spreading rapidly in a relevant jurisdiction. In some embodiments, each of a plurality of mRNA compositions given in PD₁, PD₂, PD₃, and/or PDₙ can independently comprise an mRNA encoding a SARS-CoV-2 S protein or an immunogenic fragment thereof from an alpha variant and an mRNA encoding a SARS-CoV-2 S protein or an immunogenic fragment thereof comprising one or more mutations from a delta variant. In some embodiments, each of a plurality of mRNA compositions given in PD₁, PD₂, PD₃, and/or PDₙ can independently comprise an mRNA encoding a SARS-CoV-2 S protein or an immunogenic fragment thereof from an alpha variant and an mRNA encoding a SARS-CoV-2 S protein or an immunogenic fragment thereof comprising one or more mutations from an Omicron variant (e.g., a BA.4/5, BA.1, BA.2, XBB, XBB.1, or BQ.1 Omicron variant). In some embodiments, each of a plurality of mRNA compositions given in PD₁, PD₂, PD₃, and/or PDₙ can independently comprise an mRNA encoding a SARS-CoV-2 S protein or an immunogenic fragment thereof from a delta variant and an mRNA encoding a SARS-CoV-2 S protein or an immunogenic fragment thereof comprising one or more mutations from an Omicron variant (e.g., a BA.4/5, BA.1, BA.2, XBB, XBB.1, or BQ.1 Omicron variant).

In some embodiments, PD₁, PD₂, PD₃, and/or PDₙ each comprise a plurality of mRNA compositions, wherein each mRNA composition is separately administered to a subject. For example, in some embodiments each mRNA composition is administered via intramuscular injection at different injection sites. For example, in some embodiments, a first and second mRNA composition given in PD₁, PD₂, PD₃, and/or PDₙ are separately administered to different arms of a subject via intramuscular injection.

In some embodiments, PD₁, PD₂, PD₃, and/or PDₙ comprise administering a plurality of RNA molecules, wherein each RNA molecule encodes a Spike protein comprising mutations from a different SARS-CoV-2 variant, and wherein the plurality of RNA molecules are administered to the subject in a single formulation. In some embodiments, the single formulation comprises an RNA encoding a Spike protein or an immunogenic variant thereof from the Wuhan strain and an RNA encoding a SARS-CoV-2 Spike protein or an immunogenic fragment thereof comprising one or more mutations from an alpha variant. In some embodiments, the single formulation comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, the single formulation comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, the single formulation comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, the length of time between PD₁ and PD₂ (PI₁) is at least about 1 week, at least about 2 weeks, at least about 3 weeks, or at least about 4 weeks. In some embodiments, PI₁ is about 1 week to about 12 weeks. In some embodiments, PI₁ is about 1 week to about 10 weeks. In some embodiments, PI₁ is about 2 weeks to about 10 weeks. In some embodiments, PI₁ is about 2 weeks to about 8 weeks. In some embodiments, PI₁ is about 3 weeks to about 8 weeks. In some embodiments, PI₁ is about 4 weeks to about 8 weeks. In some embodiments, PI₁ is about 6 weeks to about 8 weeks. In some embodiments PI₁ is about 3 to about 4 weeks. In some embodiments, PI₁ is about 1 week. In some embodiments, PI₁ is about 2 weeks. In some embodiments, PI₁ is about 3 weeks. In some embodiments, PI₁ is about 4 weeks. In some embodiments, PI₁ is about 5 weeks. In some embodiments, PI₁ is about 6 weeks. In some embodiments, PI₁ is about 7 weeks. In some embodiments, PI₁ is about 8 weeks. In some embodiments, PI₁ is about 9 weeks. In some embodiments, PI₁ is about 10 weeks. In some embodiments, PI₁ is about 11 weeks. In some embodiments, PI₁ is about 12 weeks.

In some embodiments, the length of time between PD₂ and PD₃ (PI₂) is at least about 1 week, at least about 2 weeks, or at least about 3 weeks. In some embodiments, PI₂ is about 1 week to about 12 weeks. In some embodiments, PI₂ is about 1 week to about 10 weeks. In some embodiments, PI₂ is about 2 weeks to about 10 weeks. In some embodiments, PI₂ is about 2 weeks to about 8 weeks. In some embodiments, PI₂ is about 3 weeks to about 8 weeks. In some embodiments, PI₂ is about 4 weeks to about 8 weeks. In some embodiments, PI₂ is about 6 weeks to about 8 weeks. In some embodiments PI₂ is about 3 to about 4 weeks. In some embodiments, PI₂ is about 1 week. In some embodiments, PI₂ is about 2 weeks. In some embodiments, PI₂ is about 3 weeks. In some embodiments, PI₂ is about 4 weeks. In some embodiments, PI₂ is about 5 weeks. In some embodiments, PI₂ is about 6 weeks. In some embodiments, PI₂ is about 7 weeks. In some embodiments, PI₂ is about 8 weeks. In some embodiments, PI₂ is about 9 weeks. In some embodiments, PI₂ is about 10 weeks. In some embodiments, PI₂ is about 11 weeks. In some embodiments, PI₂ is about 12 weeks.

In some embodiments, the length of time between PD₃ and a subsequent dose that is part of the Primary Dosing Regimen, or between doses for any dose beyond PD₃ (PIₙ) is each separately and independently selected from: about 1 week or more, about 2 weeks or more, or about 3 weeks or more. In some embodiments, PIₙ is about 1 week to about 12 weeks. In some embodiments, PIₙ is about 1 week to about 10 weeks. In some embodiments, PIₙ is about 2 weeks to about 10 weeks. In some embodiments, PIₙ is about 2 weeks to about 8 weeks. In some embodiments, PIₙ is about 3 weeks to about 8 weeks. In some embodiments, PIₙ is about 4 weeks to about 8 weeks. In some embodiments, PIₙ is about 6 weeks to about 8 weeks. In some embodiments PIₙ is about 3 to about 4 weeks. In some embodiments, PI₂ is about 1 week. In some embodiments, PIₙ is about 2 weeks. In some embodiments, PIₙ is about 3 weeks. In some embodiments, PIₙ is about 4 weeks. In some embodiments, PIₙ is about 5 weeks. In some embodiments, PIₙis about 6 weeks. In some embodiments, PIₙ is about 7 weeks. In some embodiments, PIₙis about 8 weeks. In some embodiments, PIₙ is about 9 weeks. In some embodiments, PIₙ is about 10 weeks. In some embodiments, PIₙ is about 11 weeks. In some embodiments, PIₙ is about 12 weeks.

In some embodiments, one or more compositions adminstered in PD₁ are formulated in a Tris buffer. In some embodiments, one or more compositions administered in PD₂ are formulated in a Tris buffer. In some embodiments, one or more compositions administering in PD₃ are formulated in a Tris buffer. In some embodiments, one or more compositions adminsitered in PDₙ are formulated in a Tris buffer.

In some embodiments, the primary dosing regimen comprises administering two or more mRNA compositions described herein, and at least two of the mRNA compositions have different formulations. In some embodiments, the primary dosing regimen comprises PD₁ and PD₂, where PD₁ comprises administering an mRNA formulated in a Tris buffer and PD₂ comprises administering an mRNA formulated in a PBS buffer. In some embodiments, the primary dosing regimen comprises PD₁ and PD₂, where PD₁ comprises administering an mRNA formulated in a PBS buffer and PD₂ comprises administering an mRNA formulated in a Tris buffer.

In some embodiments, one or more mRNA compositions given in PD₁, PD₂, PD₃, and/or PDₙ can be administered in combination with another vaccine. In some embodiments, another vaccine is for a disease that is not COVID-19. In some embodiments, the disease is one that increases deleterious effects of SARS-CoV-2 when a subject is coinfected with the disease and SARS-CoV-2. In some embodiments, the disease is one that increases the transmission rate of SARS-CoV-2 when a subject is coinfected with the disease and SARS-CoV-2. In some embodiments, another vaccine is a different commerically available vaccine. In some embodiments, the different commercially available vaccine is an RNA vaccine. In some embodiments, the different commercially available vaccine is a polypeptide-based vaccine. In some embodiments, another vaccine (e.g., as described herein) and one or more mRNA compositions given in PD₁, PD₂, PD₃, and/or PDₙ are separately administered, for example, in some embodiments via intramuscular injection, at different injection sites. For example, in some embodiments, an influenza vaccine and one or more SARS-CoV-2 mRNA compositions described herein given in PD₁, PD₂, PD₃, and/or PDₙ are separately administered to different arms of a subject via intramuscular injection.

### Booster Dosing Regimens

In some embodiments, methods of vaccination disclosed herein comprise one or more Booster Dosing Regimens. The Booster Dosing Regimens disclosed herein comprise one or more doses. In some embodiments, a Booster Dosing Regimen is administered to patients who have been administered a Primary Dosing Regimen (e.g., as described herein). In some embodiments a Booster Dosing Regimen is administed to patients who have not received a pharmaceutical composition disclosed herein. In some embodiments a Booster Dosing Regimen is administered to patients who have been previously vaccinated with a COVID-19 vaccine that is different from the vaccine administered in a Primary Dosing Regimen.

In some embodiments, the length of time between the Primary Dosing Regimen and the Booster Dosing Regimen is at least 1 week, at least 2 weeks, at least 3 weeks, at least 4 weeks, at least 5 weeks, at least 6 weeks, at least 7 weeks, at least 8 weeks, at least 9 weeks, at least 10 weeks, at least 11 weeks, at least 12 weeks, at least 2 months, at least 3 months, at least 4 months, at least 5 months, at least 6 months, at least 7 months, at least 8 months, at least 9 months, at least 10 months, at least 11 months, or at least 12 months or longer. In some embodiments, the length of time between the Primary Dosing Regimen and the Booster Dosing Regimen is about 1 month. In some embodiments, the length of time between the Primary Dosing Regimen and the Booster Dosing Regimen is at least about 2 months. In some embodiments, the length of time between the Primary Dosing Regimen and the Booster Dosing Regimen is at least about 3 months. In some embodiments, the length of time between the Primary Dosing Regimen and the Booster Dosing Regimen is at least about 4 months. In some embodiments, the length of time between the Primary Dosing Regimen and the Booster Dosing Regimen is at least about 5 months. In some embodiments, the length of time between the Primary Dosing Regimen and the Booster Dosing Regimen is at least about 6 months. In some embodiments, the length of time between the Primary Dosing Regimen and the Booster Dosing Regimen is from about 1 month to about 48 months. In some embodiments, the length of time between the Primary Dosing Regimen and the Booster Dosing Regimen is from about 1 month to about 36 months. In some embodiments, the length of time between the primary dosing regimen and the Booster Dosing Regimen is from about 1 month to about 24 months. In some embodiments, the length of time between the Primary Dosing Regimen and the Booster Dosing Regimen is from about 2 months to about 24 months. In some embodiments, the length of time between the Primary Dosing Regimen and the Booster Dosing Regimen is from about 3 months to about 24 months. In some embodiments, the length of time between the primary dosing regimen and the Booster Dosing Regimen is from about 3 months to about 18 months. In some embodiments, the length of time between the primary dosing regimen and the Booster Dosing Regimen is from about 3 months to about 12 months. In some embodiments, the length of time between the primary dosing regimen and the Booster Dosing Regimen is from about 6 months to about 12 months. In some embodiments, the length of time between the Primary Dosing Regimen and the Booster Dosing Regimen is from about 3 months to about 9 months. In some embodiments, the length of time between the Primary Dosing Regimen and the Booster Dosing Regimen is from about 5 months to about 7 months. In some embodiments, the length of time between the Primary Dosing Regimen and the Booster Dosing Regimen is about 6 months.

In some embodiments, subjects are administered a Booster Dosing Regimen. A Booster dosing regimen can comprise one or more doses. For example, in some embodiments, a Booster Dosing Regimen comprises a single dose (BD₁). In some embodiments a Booster Dosing Regimen comprises a first dose (BD₁) and a second dose (BD₂). In some embodiments, a Booster Dosing Regimen comprises a first dose, a second dose, and a third dose (BD₃). In some embodiments, a Booster Dosing Regimen comprises a first dose, a second dose, a third dose, and one or more additional doses (BDₙ) of any one of the pharmaceutical compositions described herein.

In some embodiments, BD₁ comprises administering 1 to 100 ug of RNA. In some embodiments, BD₁ comprises administering 1 to 60 ug of RNA. In some embodiments, BD₁ comprises administering 1 to 50 ug of RNA. In some embodiments, BD₁ comprises administering 1 to 30 ug of RNA. In some embodiments, BD₁ comprises administering about 3 ug of RNA. In some embodiments, BD₁ comprises administering about 5 ug of RNA. In some embodiments, BD₁ comprises administering about 10 ug of RNA. In some embodiments, BD₁ comprises administering about 15 ug of RNA. In some embodiments, BD₁ comprises administering about 20 ug of RNA. In some embodiments, BD₁ comprises administering about 30 ug of RNA. In some embodiments, BD₁ comprises administering about 50 ug of RNA. In some embodiments, BD₁ comprises administering about 60 ug of RNA.

In some embodiments, BD₂ comprises administering 1 to 100 ug of RNA. In some embodiments, BD₂ comprises administering 1 to 60 ug of RNA. In some embodiments, BD₂ comprises administering 1 to 50 ug of RNA. In some embodiments, BD₂ comprises administering 1 to 30 ug of RNA. In some embodiments, BD₂ comprises administering about 3 ug. In some embodiments, BD₂ comprises administering about 5 ug of RNA. In some embodiments, BD₂ comprises administering about 10 ug of RNA. In some embodiments, BD₂ comprises administering about 15 ug of RNA. In some embodiments, BD₂ comprises administering about 20 ug RNA. In some embodiments, BD₂ comprises administering about 30 ug of RNA. In some embodiments, BD₂ comprises administering about 50 ug of RNA. In some embodiments, BD₂ comprises administering about 60 ug of RNA.

In some embodiments, BD₃ comprises administering 1 to 100 ug of RNA. In some embodiments, BD₃ comprises administering 1 to 60 ug of RNA. In some embodiments, BD₃ comprises administering 1 to 50 ug of RNA. In some embodiments, BD₃ comprises administering 1 to 30 ug of RNA. In some embodiments, BD₃ comprises administering about 3 ug of RNA. In some embodiments, BD₃ comprises administering about 5 ug of RNA. In some embodiments, BD₃ comprises administering about 10 ug of RNA. In some embodiments, BD₃ comprises administering about 15 ug of RNA. In some embodiments, BD₃ comprises administering about 20 ug of RNA. In some embodiments, BD₃ comprises administering about 30 ug of RNA. In some embodiments, BD₃ comprises administering about 50 ug of RNA. In some embodiments, BD₃ comprises administering about 60 ug of RNA.

In some embodiments, BDₙ comprises administering 1 to 100 ug of RNA. In some embodiments, BDₙ comprises administering 1 to 60 ug of RNA. In some embodiments, BDₙ comprises administering 1 to 50 ug of RNA. In some embodiments, BDₙ comprises administering 1 to 30 ug of RNA. In some embodiments, BDₙ comprises administering about 3 ug of RNA. In some embodiments, BDₙ comprises administering about 5 ug of RNA. In some embodiments, BDₙ comprises administering about 10 ug of RNA. In some embodiments, BDₙ comprises administering about 15 ug of RNA. In some embodiments, BDₙ comprises administering about 20 ug of RNA. In some embodiments, BDₙ comprises administering about 30 ug of RNA. In some embodiments, BDₙ comprises administering about 60 ug of RNA. In some embodiments, BDₙ comprises administering about 50 ug of RNA.

In some embodiments, BD₁ comprises an RNA encoding a SARS-CoV-2 Spike protein or an immunogenic fragment thereof from the Wuhan strain. In some embodiments, BD₁ comprises an RNA encoding a Spike protein or an immunogenic fragment thereof from a SARS-CoV-2 strain that is prevalent and/or spreading rapidly in a relevant jurisdiction. In some embodiments, BD₁ comprises an RNA encoding a SARS-CoV-2 Spike protein or an immunogenic fragment thereof comprising one or more mutations from an alpha variant. In some embodiments, BD₁ comprises an RNA encoding a SARS-CoV-2 Spike protein or an immunogenic fragment thereof comprising one or more mutations from a delta variant. In some embodiments, BD₁ comprises an RNA encoding a SARS-CoV-2 Spike protein or an immunogenic fragment thereof comprising one or more mutations from a beta variant. In some embodiments, BD₁ comprises an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, BD₁ comprises an RNA encoding a SARS-CoV-2 Spike protein or an immunogenic fragment thereof from the Wuhan strain and one or more RNA encoding a Spike protein or an immunogenic fragment thereof from a SARS-CoV-2 strain that is prevalent and/or spreading rapidly in a relevant jurisdiction. In some embodiments, BD₁ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a alpha variant. In some embodiments, BD₁ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, BD₁ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, BD₁ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, BD₂ comprises an RNA encoding a SARS-CoV-2 Spike protein or an immunogenic fragment thereof from the Wuhan strain. In some embodiments, BD₂ comprises an RNA encoding a Spike protein or an immunogenic fragment thereof from a SARS-CoV-2 strain that is prevalent and/or spreading rapidly in a relevant jurisdiction. In some embodiments, BD₂ comprises an RNA encoding a SARS-CoV-2 Spike protein or an immunogenic fragment thereof comprising one or more mutations from an alpha variant. In some embodiments, BD₂ comprises an RNA encoding a SARS-CoV-2 Spike protein or an immunogenic fragment thereof comprising one or more mutations from a delta variant. In some embodiments, BD₂ comprises an RNA encoding a SARS-CoV-2 Spike protein or an immunogenic fragment thereof comprising one or more mutations from a beta variant. In some embodiments, BD₂ comprises an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant). In some embodiments, BD₂ comprises an RNA encoding a SARS-CoV-2 Spike protein or an immunogenic fragment thereof from the Wuhan strain and one or more RNA encoding a Spike protein or an immunogenic fragment thereof from a SARS-CoV-2 strain that is prevalent and/or spreading rapidly in a relevant jurisdiction. In some embodiments, BD₂ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a alpha variant. In some embodiments, BD₂ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, BD₂ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, BD₂ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, BD₃ comprises an RNA encoding a SARS-CoV-2 Spike protein or an immunogenic fragment thereof from the Wuhan strain. In some embodiments, BD₃ comprises an RNA encoding a Spike protein or an immunogenic fragment thereof from a SARS-CoV-2 strain that is prevalent and/or spreading rapidly in a relevant jurisdiction. In some embodiments, BD₃ comprises an RNA encoding a SARS-CoV-2 Spike protein or an immunogenic fragment thereof comprising one or more mutations from an alpha variant. In some embodiments, BD₃ comprises an RNA encoding a SARS-CoV-2 Spike protein or an immunogenic fragment thereof comprising one or more mutations from a delta variant. In some embodiments, BD₃ comprises an RNA encoding a SARS-CoV-2 Spike protein or an immunogenic fragment thereof comprising one or more mutations from a beta variant. In some embodiments, BD₃ comprises an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, BD₃ comprises an RNA encoding a SARS-CoV-2 Spike protein or an immunogenic fragment thereof from the Wuhan strain and one or more RNA encoding a Spike protein or an immunogenic fragment thereof from a SARS-CoV-2 strain that is prevalent and/or spreading rapidly in a relevant jurisdiction. In some embodiments, BD₃ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a alpha variant. In some embodiments, BD₃ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, BD₃ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, BD₃ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, BDₙ comprises an RNA encoding a SARS-CoV-2 Spike protein or an immunogenic fragment thereof from the Wuhan strain. In some embodiments, BDₙ comprises an RNA encoding a Spike protein or an immunogenic fragment thereof from a SARS-CoV-2 strain that is prevalent and/or spreading rapidly in a relevant jurisdiction. In some embodiments, BDₙ comprises an RNA encoding a SARS-CoV-2 Spike protein or an immunogenic fragment thereof comprising one or more mutations from an alpha variant. In some embodiments, BDₙ comprises an RNA encoding a SARS-CoV-2 Spike protein or an immunogenic fragment thereof comprising one or more mutations from a delta variant. In some embodiments, BDₙ comprises an RNA encoding a SARS-CoV-2 Spike protein or an immunogenic fragment thereof comprising one or more mutations from a beta variant. In some embodiments, BDₙ comprises an RNA encoding a SARS-CoV-2 Spike protein or an immunogenic fragment thereof comprising one or more mutations from an Omicron variant(e.g., a BA.4/5, BA.1, BA.2, XBB, XBB.1, or BQ.1 Omicron variant).

In some embodiments, BDₙ comprises an RNA encoding a SARS-CoV-2 Spike protein or an immunogenic fragment thereof from the Wuhan strain and one or more RNA encoding a Spike protein or an immunogenic fragment thereof from a SARS-CoV-2 strain that is prevalent and/or spreading rapidly in a relevant jurisdiction. In some embodiments, BDₙ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a alpha variant. In some embodiments, BDₙ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, BDₙ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, BDₙ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an Omicron variant (e.g., a BA.4/5, BA.1, BA.2, XBB, XBB.1, or BQ.1 Omicron variant).

In some embodiments, BD₁, BD₂, BD₃, and BDₙ can each independently comprise a plurality of (e.g., at least two) mRNA compositions described herein. In some embodiments BD₁, BD₂, BD₃, and BDₙ can each independently comprise a first and a second mRNA composition. In some embodiments, BD₁, BD₂, BD₃, and BDₙ can each independently comprise a plurality of (e.g., at least two) mRNA compositions, wherein , at least one of the plurality of mRNA compositions comprises BNT162b2 (e.g., as described herein). In some embodiments, at least one of a plurality of mRNA compositions comprises an mRNA encoding a SARS-CoV-2 S protein or an immunogenic fragment thereof from a different SARS-CoV-2 variant (e.g., a variant that is prevalent or rapidly spreading in a relevant jurisdiction, e.g., a variant disclosed herein). In some embodiments, at least one of a plurality of mRNA compositions comprises an mRNA encoding a SARS-CoV-2 S protein or an immunogenic fragment thereof from a Wuhan strain of SARS-CoV-2. In some embodiments, at least one of a plurality of mRNA compositions comprises an RNA encoding a SARS-CoV-2 S protein or an immunogenic fragment thereof comprising one or more mutations from a variant that is prevalent and/or spreading rapidly in a relevant jurisdiction. In some embodiments, at least one of a plurality of mRNA compositions comprises an mRNA encoding a SARS-CoV-2 S protein or an immunogenic fragment thereof comprising one or more mutations from an alpha variant. In some embodiments, at least one of a plurality of mRNA compositions comprises an mRNA encoding a SARS-CoV-2 S protein or an immunogenic fragment thereof comprising one or more mutations from a delta variant. In some embodiments, at least one of a plurality of mRNA compositions comprises an mRNA encoding a SARS-CoV-2 S protein or an immunogenic fragment thereof comprising one or more mutations from an Omicron variant (e.g., a BA.4/5, BA.1, BA.2, XBB, XBB.1, or BQ.1 Omicron variant).

In some embodiments, a plurality of mRNA compositions given in BD₁, BD₂, BD₃, and/or BDₙ can each indendently comprise at least two different mRNA constructs (e.g., mRNA constructs having differing protein-encoding sequences). For example, in some embodiments a plurality of mRNA compositions given in BD₁, BD₂, BD₃, and/or BDₙ can each indendently comprise an mRNA encoding a SARS-CoV-2 S protein or an immunogenic fragment thereof from a Wuhan strain of SARS-CoV-2 and an mRNA encoding a SARS-CoV-2 S protein or an immunogenic fragment thereof comprising one or more mutations from a variant that is prevalent and/or spreading rapidly in a relevant jurisdiction. In some embodiments a plurality of mRNA compositions given in BD₁, BD₂, BD₃, and/or BDₙ can each independently comprise an mRNA encoding a SARS-CoV-2 S protein or an immunogenic fragment thereof derived from a Wuhan strain of SARS-CoV-2 and an mRNA encoding a SARS-CoV-2 S protein or an immunogenic fragment thereof comprising one or more mutations from a variant that is prevalent and/or spreading rapidly in a relevant jurisdiction. In some such embodiments, a variant can be an alpha variant. In some such embodiments, a variant can be a delta variant. In some such embodiments a variant can be an Omicron variant (e.g., a BA.4/5, BA.1, BA.2, XBB, XBB.1, or BQ.1 Omicron variant).

In some embodiments, a plurality of mRNA compositions given in BD₁, BD₂, BD₃, and/or BDₙ can each independently comprise at least two mRNAs each encoding a SARS-CoV-2 S protein or an immunogenic fragment thereof comprising one or more mutations from a distinct variant that is prevalent and/or spreading rapidly in a relevant jurisdiction. In some embodiments a plurality of mRNA compositions given in BD₁, BD₂, BD₃, and/or BDₙ can each independently comprise an mRNA encoding a SARS-CoV-2 S protein or an immunogenic fragment thereof from an alpha variant and an mRNA encoding a SARS-CoV-2 S protein or an immunogenic fragment thereof comprising one or more mutations from a delta variant. In some embodiments a plurality of mRNA compositions given in BD₁, BD₂, BD₃, and/or BDₙ can each independently comprise an mRNA encoding a SARS-CoV-2 S protein or an immunogenic fragment thereof from an alpha variant and an mRNA encoding a SARS-CoV-2 S protein or an immunogenic fragment thereof comprising one or more mutations from an Omicron variant (e.g., a BA.4/5, BA.1, BA.2, XBB, XBB.1, or BQ.1 Omicron variant). In some embodiments a plurality of mRNA compositions given in BD₁, BD₂, BD₃, and/or BDₙ can each independently comprise an mRNA encoding a SARS-CoV-2 S protein or an immunogenic fragment thereof from a delta variant and an mRNA encoding a SARS-CoV-2 S protein or an immunogenic fragment thereof comprising one or more mutations from an Omicron variant (e.g., a BA.4/5, BA.1, BA.2, XBB, XBB.1, or BQ.1 Omicron variant).

In some embodiments, a plurality of mRNA compositions given in BD₁, BD₂, BD₃, and/or BDₙ are separately administered to a subject, for example, in some embodiments via intramuscular injection, at different injection sites. For example, in some embodiments, a first and second mRNA composition given in BD₁, BD₂, BD₃, and/or BDₙ are separately administered to different arms of a subject via intramuscular injection.

In some embodiments, the length of time between BD₁ and BD₂ (BI₁) is at least about 1 week, at least about 2 weeks, at least about 3 weeks, or at least about 4 weeks. In some embodiments, BI₁ is about 1 week to about 12 weeks. In some embodiments, BI₁ is about 1 week to about 10 weeks. In some embodiments, BI₁ is about 2 weeks to about 10 weeks. In some embodiments, BI₁ is about 2 weeks to about 8 weeks. In some embodiments, BI₁ is about 3 weeks to about 8 weeks. In some embodiments, BI₁ is about 4 weeks to about 8 weeks. In some embodiments, BI₁ is about 6 weeks to about 8 weeks. In some embodiments BI₁ is about 3 to about 4 weeks. In some embodiments, BI₁ is about 1 week. In some embodiments, BI₁ is about 2 weeks. In some embodiments, BI₁ is about 3 weeks. In some embodiments, BI₁ is about 4 weeks. In some embodiments, BI₁ is about 5 weeks. In some embodiments, BI₁ is about 6 weeks. In some embodiments, BI₁ is about 7 weeks. In some embodiments, BI₁ is about 8 weeks. In some embodiments, BI₁ is about 9 weeks. In some embodiments, BI₁ is about 10 weeks.

In some embodiments, the length of time between BD₂ and BD₃ (BI₂) is at least about 1 week, at least about 2 weeks, or at least about 3 weeks. In some embodiments, BI₂ is about 1 week to about 12 weeks. In some embodiments, BI₂ is about 1 week to about 10 weeks. In some embodiments, BI₂ is about 2 weeks to about 10 weeks. In some embodiments, BI₂ is about 2 weeks to about 8 weeks. In some embodiments, BI₂ is about 3 weeks to about 8 weeks. In some embodiments, BI₂ is about 4 weeks to about 8 weeks. In some embodiments, BI₂ is about 6 weeks to about 8 weeks. In some embodiments BI₂ is about 3 to about 4 weeks. In some embodiments, BI₂ is about 1 week. In some embodiments, BI₂ is about 2 weeks. In some embodiments, BI₂ is about 3 weeks. In some embodiments, BI₂ is about 4 weeks. In some embodiments, BI₂ is about 5 weeks. In some embodiments, BI₂ is about 6 weeks. In some embodiments, BI₂ is about 7 weeks. In some embodiments, BI₂ is about 8 weeks. In some embodiments, BI₂ is about 9 weeks. In some embodiments, BI₂ is about 10 weeks.

In some embodiments, the length of time between BD₃ and a subsequent dose that is part of the Booster Dosing Regimen, or between doses for any dose beyond BD₃ (BIₙ) is each separately and independently selected from: about 1 week or more, about 2 weeks or more, or about 3 weeks or more. In some embodiments, BIₙ is about 1 week to about 12 weeks. In some embodiments, BIₙ is about 1 week to about 10 weeks. In some embodiments, BIₙ is about 2 weeks to about 10 weeks. In some embodiments, BIₙ is about 2 weeks to about 8 weeks. In some embodiments, BIₙ is about 3 weeks to about 8 weeks. In some embodiments, BIₙ is about 4 weeks to about 8 weeks. In some embodiments, BIₙ is about 6 weeks to about 8 weeks. In some embodiments BIₙ is about 3 to about 4 weeks. In some embodiments, BIₙ is about 1 week. In some embodiments, BIₙ is about 2 weeks. In some embodiments, BIₙ is about 3 weeks. In some embodiments, BIₙ is about 4 weeks. In some embodiments, BIₙ is about 5 weeks. In some embodiments, BIₙ is about 6 weeks. In some embodiments, BIₙ is about 7 weeks. In some embodiments, BIₙ is about 8 weeks. In some embodiments, BIₙ is about 9 weeks. In some embodiments, BIₙ is about 10 weeks.

In some embodiments, one or more compositions adminstered in BD₁ are formulated in a Tris buffer. In some embodiments, one or more compositions administered in BD₂ are formulated in a Tris buffer. In some embodiments, one or more compositions administering in BD₃ are formulated in a Tris buffer. In some embodiments, one or more compositions adminsitered in BD₃ are formulated in a Tris buffer.

In some embodiments, the Booster dosing regimen comprises administering two or more mRNA compositions described herein, and at least two of the mRNA compositions have differnent formulations. In some embodiments, the Booster dosing regimen comprises BD₁ and BD₂, where BD₁ comprises administering an mRNA formulated in a Tris buffer and BD₂ comprises administering an mRNA formulated in a PBS buffer. In some embodiments, the Booster dosing regimen comprises BD₁ and BD₂, where BD₁ comprises administering an mRNA formulated in a PBS buffer and BD₂ comprises administering an mRNA formulated in a Tris buffer.

In some embodiments, one or more mRNA compositions given in BD₁, BD₂, BD₃, and/or BDₙ can be administered in combination with another vaccine. In some embodiments, another vaccine is for a disease that is not COVID-19. In some embodiments, the disease is one that increases deleterious effects of SARS-CoV-2 when a subject is coinfected with the disease and SARS-CoV-2. In some embodiments, the disease is one that increases the transmission rate of SARS-CoV-2 when a subject is coinfected with the disease and SARS-CoV-2. In some embodiments, another vaccine is a different commerically available vaccine. In some embodiments, the different commercially available vaccine is an RNA vaccine. In some embodiments, the different commercially available vaccine is a polypeptide-based vaccine. In some embodiments, another vaccine (e.g., as described herein) and one or more mRNA compositions given in BD₁, BD₂, BD₃, and/or BDₙ are separately administered, for example, in some embodiments via intramuscular injection, at different injection sites. For example, in some embodiments, an influenza vaccine and one or more SARS-CoV-2 mRNA compositions described herein given in BD₁, BD₂, BD₃, and/or BDₙ are separately administered to different arms of a subject via intramuscular injection.

### Additional Booster Regimens

In some embodiments, methods of vaccination disclosed herein comprise administering more than one Booster Dosing Regimen. In some embodiments, more than one Booster Dosing Regimen may need to be administered to increase neutralizing antibody response. In some embodiments, more than one booster dosing regimen may be needed to counteract a SARS-CoV-2 strain that has been shown to have a high likelihood of evading immune response elicited by vaccines that a patient has previously received. In some embodiments, an additional Booster Dosing Regimen is administered to a patient who has been determined to produce low concentrations of neutralizing antibodies. In some embodiments, an additional booster dosing regimen is administered to a patient who has been determined to have a high likelihood of being susceptible to SARS-CoV-2 infection, despite previous vaccination (e.g., an immunocompromised patient, a cancer patient, and/or an organ transplant patient).

The description provided above for the first Booster Dosing Regimen also describes the one or more additional Booster Dosing Regimens. The interval of time between the first Booster Dosing Regimen and a second Booster Dosing Regimen, or between subsequent Booster Dosing Regimens can be any of the acceptable intervals of time described above between the Primary Dosing Regimen and the First Booster Dosing Regimen.

In some embodiments, a dosing regimen comprises a primary regimen and a booster regimen, wherein at least one dose given in the primary regimen and/or the booster regimen comprises a composition comprising an RNA that encodes a S protein or immungenic fragment thereof from a variant that is prevalent or is spreading rapidly in a relevant jurisdiction (e.g., Omicron variant as described herein). For example, in some embodiments, a primary regimen comprises at least 2 doses of BNT162b2 (e.g., encoding a Wuhan strain), for example, given at least 3 weeks apart, and a booster regimen comprises at least 1 dose of a composition comprising RNA that encodes a S protein or immungenic fragment thereof from a variant that is prevalent or is spreading rapidly in a relevant jurisdiction (e.g., Omicron variant as described herein). In some such embodiments, such a dose of a booster regimen may further comprise an RNA that encodes a S protein or immungenic fragement thereof from a Wuhan strain, which can be administered with an RNA that encodes a S protein or immungenic fragment thereof from a variant that is prevalent or is spreading rapidly in a relevant jurisdiction (e.g., Omicron variant as described herein), as a single mixture, or as two separate compositions, for example, in 1:1 weight ratio. In some embodiments, a booster regimen can also comprise at least 1 dose of BNT162b2, which can be administered as a first booster dose or a subsequent booster dose.

In some embodiments, an RNA composition described herein is given as a booster at a dose that is higher than the doses given during a primary regimen (primary doses) and/or the dose given for a first booster, if any. For example, in some embodiments, such a dose may be 60 ug; or in some embodiments such a dose may be higher than 30 ug and lower than 60 ug (e.g., 55 ug, 50 ug, or lower). In some embodiments, an RNA composition described herein is given as a booster at least 3-12 months or 4-12 months, or 5-12 months, or 6-12 months after the last dose (e.g., the last dose of a primary regimen or a first dose of a booster regimen). In some embodiments, the primary doses and/or the first booster dose (if any) may comprise BNT162b2, for example at 30 ug per dose.

In some embodiments, an RNA composition described herein comprises an RNA encoding a polypeptide as set forth in SEQ ID NO: 49 or an immunogenic fragment thereof, or a variant thereof (e.g., having at least 70% or more, including, e.g., at least 80%, at least 85%, at least 90%, at least 95%, at least 96%, at least 97%, at least 98%, or higher, identity to SEQ ID NO: 49). In some embodiments, an RNA composition comprises an RNA that includes the sequence of SEQ ID NO: 50 or a variant thereof (e.g., having at least 70% or more, including, e.g., at least 80%, at least 85%, at least 90%, at least 95%, at least 96%, at least 97%, at least 98%, or higher, identity to SEQ ID NO: 50). In some embodiments, an RNA composition comprises an RNA that includes the sequence of SEQ ID NO: 51 or a variant thereof (e.g., having at least 70% or more, including, e.g., at least 80%, at least 85%, at least 90%, at least 95%, at least 96%, at least 97%, at least 98%, or higher, identity to SEQ ID NO: 51).

In some embodiments, an RNA composition described herein comprises an RNA encoding a polypeptide as set forth in SEQ ID NO: 55 or an immunogenic fragment thereof, or a variant thereof (e.g., having at least 70% or more, including, e.g., at least 80%, at least 85%, at least 90%, at least 95%, at least 96%, at least 97%, at least 98%, or higher, identity to SEQ ID NO: 55. In some embodiments, an RNA composition comprises an RNA that includes the sequence of SEQ ID NO: 56 or a variant thereof (e.g., having at least 70% or more, including, e.g., at least 80%, at least 85%, at least 90%, at least 95%, at least 96%, at least 97%, at least 98%, or higher, identity to SEQ ID NO: 56). In some embodiments, an RNA composition comprises an RNA that includes the sequence of SEQ ID NO: 57 or a variant thereof (e.g., having at least 70% or more, including, e.g., at least 80%, at least 85%, at least 90%, at least 95%, at least 96%, at least 97%, at least 98%, or higher, identity to SEQ ID NO: 57).

In some embodiments, an RNA composition described herein comprises an RNA encoding a polypeptide as set forth in SEQ ID NO: 58 or an immunogenic fragment thereof, or a variant thereof (e.g., having at least 70% or more, including, e.g., at least 80%, at least 85%, at least 90%, at least 95%, at least 96%, at least 97%, at least 98%, or higher, identity to SEQ ID NO: 58). In some embodiments, an RNA composition comprises an RNA that includes the sequence of SEQ ID NO: 59 or a variant thereof (e.g., having at least 70% or more, including, e.g., at least 80%, at least 85%, at least 90%, at least 95%, at least 96%, at least 97%, at least 98%, or higher, identity to SEQ ID NO: 59). In some embodiments, an RNA composition comprises an RNA that includes the sequence of SEQ ID NO: 60 or a variant thereof (e.g., having at least 70% or more, including, e.g., at least 80%, at least 85%, at least 90%, at least 95%, at least 96%, at least 97%, at least 98%, or higher, identity to SEQ ID NO: 60).

In some embodiments, an RNA composition described herein comprises an RNA encoding a polypeptide as set forth in SEQ ID NO: 61 or an immunogenic fragment thereof, or a variant thereof (e.g., having at least 70% or more, including, e.g., at least 80%, at least 85%, at least 90%, at least 95%, at least 96%, at least 97%, at least 98%, or higher, identity to SEQ ID NO: 61). In some embodiments, an RNA composition comprises an RNA that includes the sequence of SEQ ID NO: 62a or a variant thereof (e.g., having at least 70% or more, including, e.g., at least 80%, at least 85%, at least 90%, at least 95%, at least 96%, at least 97%, at least 98%, or higher, identity to SEQ ID NO: 62a). In some embodiments, an RNA composition comprises an RNA that includes the sequence of SEQ ID NO: 63a or a variant thereof (e.g., having at least 70% or more, including, e.g., at least 80%, at least 85%, at least 90%, at least 95%, at least 96%, at least 97%, at least 98%, or higher, identity to SEQ ID NO: 63a).

In some embodiments, the formulations disclosed herein can be used to carry out any of the dosing regimens described in Table 19 (below).

**Table 19: Exemplary Dosing Regimens:**

| | Primary Regimen | | | | Time between the last dose of a Primary regimen and a first dose of Booster Regimen | Booster Regimen | | | |
|---|---|---|---|---|---|---|---|---|---|
| # | Dose 1 (µg RNA) | Dose 2 (µg RNA) | Time Between Doses 1 and 2 | Dose 1 and Dose 2 Formulation | | Dose 1 (µg RNA) | Dose 2 (µg RNA) | Time Between Doses 1 and 2 | Dose 1 and Dose 2 Formulation |
| 1 | 30 | 30 | 2 to 8 weeks | PBS | At least 2 months | 30 | N/A¹ | N/A | PBS |
| 2 | 30 | 30 | 2 to 8 weeks | PBS | At least 3 months | 30 | N/A¹ | N/A | PBS |
| 3 | 30 | 30 | 2 to 8 weeks | PBS | 6 to 12 months | 30 | N/A¹ | N/A | PBS |
| 4 | 30 | 30 | 2 to 8 weeks | PBS or Tris | 4 to 12 months | 15 | N/A¹ | N/A | PBS or Tris |
| 5 | 30 | 30 | 2 to 8 weeks | PBS or Tris | 4 to 12 months | 10 | N/A¹ | N/A | PBS or Tris |
| 6 | 30 | 30 | 2 to 8 weeks | PBS or Tris | 4 to 12 months | 30 | 30 | 4 to 12 months | PBS or Tris |
| 7 | 30 | 30 | 2 to 8 weeks | PBS or Tris | 4 to 12 months | 30 | 15 | 4 to 12 months | PBS or Tris |
| 8 | 30 | 30 | 2 to 8 weeks | PBS or Tris | 4 to 12 months | 30 | 10 | 4 to 12 months | PBS or Tris |
| 9 | 30 | 30 | 2 to 8 weeks | PBS or Tris | 4 to 12 months | 30 | 60 | 4 to 12 months | PBS or Tris |
| 10 | 30 | 30 | 2 to 8 weeks | PBS or Tris | 4 to 12 months | 30 | >30 to < 60 | 4 to 12 months | PBS or Tris |
| 11 | 30 | 30 | 2 to 8 weeks | PBS or Tris | 4 to 12 months | 30 | 50 | 4 to 12 months | PBS or Tris |
| 12 | 30 | 30 | 2 to 8 weeks | PBS | At least 6 months | 30 | N/A¹ | N/A | PBS |
| 13 | 30 | 30 | ^{∼}21 days | PBS | At least 2 months | 30 | N/A¹ | N/A | PBS |
| 14 | 30 | 30 | ^{∼}21 days | PBS | At least 3 months | 30 | N/A¹ | N/A | PBS |
| 15 | 30 | 30 | ^{∼}21 days | PBS | 6 to 12 months | 30 | N/A¹ | N/A | PBS |
| 16 | 30 | 30 | ^{∼}21 days | PBS | At least 6 months | 30 | N/A¹ | N/A | PBS |
| 17 | 30 | 30 | 21 days | PBS | At least 6 months | 15 | 15 | ^{∼}21 days | PBS |
| 18 | 30 | 30 | 21 days | PBS | At least 6 months | 15 | 15 | ^{∼}21 days | PBS |
| | | | | | | | | | |
| 19 | 30 | 30 | 2 to 8 weeks | PBS | At least 2 months | 30 | N/A¹ | N/A | Tris |
| 20 | 30 | 30 | 2 to 8 weeks | PBS | At least 3 months | 30 | N/A¹ | N/A | Tris |
| 21 | 30 | 30 | 2 to 8 weeks | PBS | 6 to 12 months | 30 | N/A¹ | N/A | Tris |
| 22 | 30 | 30 | 2 to 8 weeks | PBS | At least 6 months | 30 | N/A¹ | N/A | Tris |
| 23 | 30 | 30 | ^{∼}21 days | PBS | At least 2 months | 30 | N/A¹ | N/A | Tris |
| 24 | 30 | 30 | ^{∼}21 days | PBS | At least 3 months | 30 | N/A¹ | N/A | Tris |
| 25 | 30 | 30 | ^{∼}21 days | PBS | 6 to 12 months | 30 | N/A¹ | N/A | Tris |
| 26 | 30 | 30 | ^{∼}21 days | PBS | At least 6 months | 30 | N/A¹ | N/A | Tris |
| 27 | 30 | 30 | 21 days | PBS | At least 6 months | 15 | 15 | ^{∼}21 days | Tris |
| 28 | 30 | 30 | 21 days | PBS | At least 6 months | 15 | 15 | ^{∼}21 days | Tris |
| | | | | | | | | | |
| 29 | 30 | 30 | 2 to 8 weeks | Tris | At least 2 months | 30 | N/A¹ | N/A | Tris |
| 30 | 30 | 30 | 2 to 8 weeks | Tris | At least 3 months | 30 | N/A¹ | N/A | Tris |
| 31 | 30 | 30 | 2 to 8 weeks | Tris | 6 to 12 months | 30 | N/A¹ | N/A | Tris |
| 32 | 30 | 30 | 2 to 8 weeks | Tris | At least 6 months | 30 | N/A¹ | N/A | Tris |
| 33 | 30 | 30 | ^{∼}21 days | Tris | At least 2 months | 30 | N/A¹ | N/A | Tris |
| 34 | 30 | 30 | ^{∼}21 days | Tris | At least 3 months | 30 | N/A¹ | N/A | Tris |
| 35 | 30 | 30 | ^{∼}21 days | Tris | 6 to 12 months | 30 | N/A¹ | N/A | Tris |
| 36 | 30 | 30 | ^{∼}21 days | Tris | At least 6 months | 30 | N/A¹ | N/A | Tris |
| 37 | 30 | 30 | 21 days | Tris | At least 6 months | 15 | 15 | ^{∼}21 days | Tris |
| 38 | 30 | 30 | 21 days | Tris | At least 6 months | 15 | 15 | ^{∼}21 days | Tris |
| | | | | | | | | | |
| 39 | 10 | 10 | 2 to 8 weeks | Tris | At least 2 months | 10 | N/A¹ | N/A | Tris |
| 40 | 10 | 10 | 2 to 8 weeks | Tris | At least 3 months | 10 | N/A¹ | N/A | Tris |
| 41 | 10 | 10 | 2 to 8 weeks | Tris | 6 to 12 months | 10 | N/A¹ | N/A | Tris |
| 42 | 10 | 10 | 2 to 8 weeks | Tris | At least 6 months | 10 | N/A¹ | N/A | Tris |
| 43 | 10 | 10 | ^{∼}21 days | Tris | At least 2 months | 10 | N/A¹ | N/A | Tris |
| 44 | 10 | 10 | ^{∼}21 days | Tris | At least 3 months | 10 | N/A¹ | N/A | Tris |
| 45 | 10 | 10 | ^{∼}21 days | Tris | 6 to 12 months | 10 | N/A¹ | N/A | Tris |
| 46 | 10 | 10 | ^{∼}21 days | Tris | At least 6 months | 10 | N/A¹ | N/A | Tris |
| | | | | | | | | | |
| 47 | 3 | 3 | 2 to 8 weeks | Tris | At least 2 months | 3 | N/A¹ | N/A | Tris |
| 48 | 3 | 3 | 2 to 8 weeks | Tris | At least 3 months | 3 | N/A¹ | N/A | Tris |
| 49 | 3 | 3 | 2 to 8 weeks | Tris | 6 to 12 months | 3 | N/A¹ | N/A | Tris |
| 50 | 3 | 3 | 2 to 8 weeks | Tris | At least 6 months | 3 | N/A¹ | N/A | Tris |
| 51 | 3 | 3 | ^{∼}21 days | Tris | At least 2 months | 3 | N/A¹ | N/A | Tris |
| 52 | 3 | 3 | ^{∼}21 days | Tris | At least 3 months | 3 | N/A¹ | N/A | Tris |
| 53 | 3 | 3 | ^{∼}21 days | Tris | 6 to 12 months | 3 | N/A¹ | N/A | Tris |
| 54 | 3 | 3 | ^{∼}21 days | Tris | At least 6 months | 3 | N/A¹ | N/A | Tris |

| | | | | | | | | | |
|---|---|---|---|---|---|---|---|---|---|
| ¹N/A refers to no dose necessary. | | | | | | | | | |

In some embodiments of certain exemplary dosing regimens as described in Table 19 above, an RNA composition described herein (e.g., comprising RNA encoding a variant described herein) is given in a first dose of a primary regimen. In some embodiments of certain exemplary dosing regimens as described in Table 19 above, an RNA composition described herein (e.g., comprising RNA encoding a variant described herein) is given in a second dose of a primary regimen. In some embodiments of certain exemplary dosing regimens as described in Table 19 above, an RNA composition described herein (e.g., comprising RNA encoding a variant described herein) is given in a first dose and a second dose of a primary regimen. In some embodiments of certain exemplary dosing regimens as described in Table 19 above, an RNA composition described herein (e.g., comprising RNA encoding a variant described herein) is given in a first dose of a booster regimen. In some embodiments of certain exemplary dosing regimens as described in Table 19 above, an RNA composition described herein (e.g., comprising RNA encoding a variant described herein) is given in a second dose of a booster regimen. In some embodiments of certain exemplary dosing regimens as described in Table 19 above, an RNA composition described herein (e.g., comprising RNA encoding a variant described herein) is given in a first dose and a second dose of a booster regimen. In some embodiments of certain exemplary dosing regimens as described in Table 19 above, an RNA composition described herein (e.g., comprising RNA encoding a variant described herein) is given in a first dose and a second dose of a primary regimen and also in at least one dose of a booster regimen. In some embodiments of certain exemplary dosing regimens as described in Table 19 above, an RNA composition described herein (e.g., comprising RNA encoding a variant described herein) is given in at least one dose (including, e.g., at least two doses) of a booster regimen and BNT162b2 is given in a primary regimen. In some embodiments of certain exemplary dosing regimens as described in Table 19 above, an RNA composition described herein (e.g., comprising RNA encoding a variant described herein) is given in a second dose of a booster regimen and BNT162b2 is given in a primary regimen and in a first dose of a booster regimen. In some embodiments, an RNA composition described herein (e.g., comprising RNA encoding a variant described herein) comprises an RNA encoding a polypeptide as set forth in SEQ ID NO: 49 or an immunogenic fragment thereof, or a variant thereof (e.g., having at least 70% or more, including, e.g., at least 80%, at least 85%, at least 90%, at least 95%, at least 96%, at least 97%, at least 98%, or higher, identity to SEQ ID NO: 49). In some embodiments, an RNA composition described herein (e.g., comprising RNA encoding a variant described herein) comprises an RNA that includes the sequence of SEQ ID NO: 50 or a variant thereof (e.g., having at least 70% or more, including, e.g., at least 80%, at least 85%, at least 90%, at least 95%, at least 96%, at least 97%, at least 98%, or higher, identity to SEQ ID NO: 50). In some embodiments, an RNA composition described herein (e.g., comprising RNA encoding a variant described herein) comprises an RNA that includes the sequence of SEQ ID NO: 51 or a variant thereof (e.g., having at least 70% or more, including, e.g., at least 80%, at least 85%, at least 90%, at least 95%, at least 96%, at least 97%, at least 98%, or higher, identity to SEQ ID NO: 51).

In some embodiments, an RNA composition described herein comprises an RNA encoding a polypeptide as set forth in SEQ ID NO: 55 or an immunogenic fragment thereof, or a variant thereof (e.g., having at least 70% or more, including, e.g., at least 80%, at least 85%, at least 90%, at least 95%, at least 96%, at least 97%, at least 98%, or higher, identity to SEQ ID NO: 55). In some embodiments, an RNA composition comprises an RNA that includes the sequence of SEQ ID NO: 56 or a variant thereof (e.g., having at least 70% or more, including, e.g., at least 80%, at least 85%, at least 90%, at least 95%, at least 96%, at least 97%, at least 98%, or higher, identity to SEQ ID NO: 56). In some embodiments, an RNA composition comprises an RNA that includes the sequence of SEQ ID NO: 57 or a variant thereof (e.g., having at least 70% or more, including, e.g., at least 80%, at least 85%, at least 90%, at least 95%, at least 96%, at least 97%, at least 98%, or higher, identity to SEQ ID NO: 57).

In some embodiments, an RNA composition described herein comprises an RNA encoding a polypeptide as set forth in SEQ ID NO: 58 or an immunogenic fragment thereof, or a variant thereof (e.g., having at least 70% or more, including, e.g., at least 80%, at least 85%, at least 90%, at least 95%, at least 96%, at least 97%, at least 98%, or higher, identity to SEQ ID NO: 58). In some embodiments, an RNA composition comprises an RNA that includes the sequence of SEQ ID NO: 59 or a variant thereof (e.g., having at least 70% or more, including, e.g., at least 80%, at least 85%, at least 90%, at least 95%, at least 96%, at least 97%, at least 98%, or higher, identity to SEQ ID NO: 59). In some embodiments, an RNA composition comprises an RNA that includes the sequence of SEQ ID NO: 60 or a variant thereof (e.g., having at least 70% or more, including, e.g., at least 80%, at least 85%, at least 90%, at least 95%, at least 96%, at least 97%, at least 98%, or higher, identity to SEQ ID NO: 60).

In some embodiments, an RNA composition described herein comprises an RNA encoding a polypeptide as set forth in SEQ ID NO: 61 or an immunogenic fragment thereof, or a variant thereof (e.g., having at least 70% or more, including, e.g., at least 80%, at least 85%, at least 90%, at least 95%, at least 96%, at least 97%, at least 98%, or higher, identity to SEQ ID NO: 61). In some embodiments, an RNA composition comprises an RNA that includes the sequence of SEQ ID NO: 62a or a variant thereof (e.g., having at least 70% or more, including, e.g., at least 80%, at least 85%, at least 90%, at least 95%, at least 96%, at least 97%, at least 98%, or higher, identity to SEQ ID NO: 62a). In some embodiments, an RNA composition comprises an RNA that includes the sequence of SEQ ID NO: 63a or a variant thereof (e.g., having at least 70% or more, including, e.g., at least 80%, at least 85%, at least 90%, at least 95%, at least 96%, at least 97%, at least 98%, or higher, identity to SEQ ID NO: 63a).

In some embodiments, such an RNA composition described herein (e.g., comprising RNA encoding a variant described herein) can further comprise RNA encoding a S protein or an immungenic fragment thereof of a different strain (e.g., a Wuhan strain). By way of example, in some embodiments, a second dose of a booster regimen of Regimens #9-11 as described in Table 19 above can comprise an RNA composition described herein (e.g., comprising RNA encoding a variant described herein such as Omicron, for example, in one embodiment RNA as described in this Example) and a BNT162b2 construct, for example, in 1: 1 weight ratio.

In some embodiments of Regimen #6 as described in Table 19 above, a first dose and a second dose of a primary regimen and a first dose and a second dose of a booster regimen each comprise an RNA composition described herein (e.g., comprising RNA encoding a variant described herein such as Omicron, for example, in one embodiment RNA as described in this Example). In some such embodiments, a second dose of a booster regimen may not be necessary.

In some embodiments of Regimen #6 as described in Table 19 above, a first dose and a second dose of a primary regimen and a first dose and a second dose of a booster regimen each comprise an RNA composition described herein (e.g., comprising RNA encoding a variant described herein such as Omicron, for example, in one embodiment RNA as described in this Example). In some such embodiments, a second dose of a booster regimen may not be necessary.

In some embodiments of Regimen #6 as described in Table 19 above, a first dose and a second dose of a primary regimen each comprise a BNT162b2 construct, and a first dose and a second dose of a booster regimen each comprise an RNA composition described herein (e.g., comprising RNA encoding a variant described herein such as Omicron, for example, in one embodiment RNA as described in this Example). In some such embodiments, a second dose of a booster regimen may not be necessary.

In some embodiments of Regimen #6 as described in Table 19 above, a first dose and a second dose of a primary regimen and a first dose of a booster regimen each comprise a BNT162b2 construct, and a second dose of a booster regimen comprises an RNA composition described herein (e.g., comprising RNA encoding a variant described herein such as Omicron, for example, in one embodiment RNA as described in this Example).

### Example 7: Omicron BA.1 breakthrough infection drives cross-variant neutralization and memory B cell formation

The present Example shows that an Omicron BA.1 breakthrough infection in individuals double- and triple-vaccinated with BNT162b2 drives cross variant neutralization and memory B cell formation, including production of neutralizing antibodies and B cell responses toward an Omicron BA.1 variant. One of ordinary skill in the art reading the present Example will understand that such findings can be extended to administration of an mRNA vaccine comprising an RNA encoding a SARS-CoV-2 S protein having mutations characteristic of an Omicron BA.1 variant (e.g., ones as described herein) to subjects who were previously administered two or three doses of SARS-CoV-2 vaccines (e.g., in some embodiments developed based on a S protein from a Wuhan-Hu-1 strain).

Omicron is the evolutionarily most distinct SARS-CoV-2 variant of concern (VOC) to date. To address how Omicron breakthrough infection can potentially reshape SARS-CoV-2 recognition in vaccinated individuals, the effects of Omicron BA.1 breakthrough infection were investigated on serum neutralization and B_{MEM} cell antigen recognition in BNT162b2 double- and triple-vaccinated individuals. Omicron BA.1 breakthrough infection induced broad neutralization of VOCs including Omicron BA.1, with substantially stronger neutralization compared to Omicron BA.1-naïve double- and triple-vaccinees. Broad recognition of VOCs by B_{MEM} cells from BNT162b2 double- and triple-vaccinated individuals was boosted by Omicron BA.1 breakthrough infection, with recognition primarily against conserved epitopes shared broadly between variants rather than Omicron BA.1-specific epitopes. The data presented herein demonstrate that an Omicron BA.1 breakthrough infection efficiently broadens neutralizing antibody and/or B cell responses towards multiple variants and suggest that a vaccine adapted to the Omicron BA.1 S protein may be able to reshape the immune repertoire.

### Introduction

Containment of the current COVID-19 pandemic requires the generation of durable and sufficiently broad immunity that provides protection against circulating and future variants of SARS-CoV-2. The titer of neutralizing antibodies to SARS-CoV-2, and the binding of antibodies to the spike (S) glycoprotein and its receptor-binding domain (RBD) are considered correlates of protection against infection (D. S. Khoury et al., "Neutralizing antibody levels are highly predictive of immune protection from symptomatic SARS-CoV-2 infection," Nature medicine. 27, 1205-1211 (2021), doi:10.1038/s41591-021-01377-8; and P. B. Gilbert et al., "Immune correlates analysis of the mRNA-1273 COVID-19 vaccine efficacy clinical trial," Science (New York, N.Y.). 375, 43-50 (2022), doi:10.1126/science.abm3425). Currently available vaccines are based on the ancestral Wuhan-Hu-1 strain and induce antibodies with a neutralizing capacity that exceeds the breadth elicited by infection with the Wuhan strain, or with variants of concern (VOCs) (K. Röltgen et al., "Immune imprinting, breadth of variant recognition, and germinal center response in human SARS-CoV-2 infection and vaccination," Cell (2022), doi:10.1016/j.cell.2022.01.018). However, protective titers wane over time (J. P. Evans et al., "Neutralizing antibody responses elicited by SARS-CoV-2 mRNA vaccination wane over time and are boosted by breakthrough infection," Science translational medicine, eabn8057 (2022), doi:10.1126/scitranslmed.abn8057; S. Yamayoshi et al., "Antibody titers against SARS-CoV-2 decline, but do not disappear for several months," Eclinical Medicine, 32, 100734 (2021), doi:10.1016/j.eclinm.2021.100734; W. N. Chia et al., "Dynamics of SARS-CoV-2 neutralising antibody responses and duration of immunity," The Lancet Microbe, 2, e240-e249 (2021), doi:10.1016/52666-5247(21)00025-2; Y. Goldberg et al., "Waning Immunity after the BNT162b2 Vaccine in Israel," The New England journal of medicine. 385, e85 (2021), doi:10.1056/NEJMoa2114228. and routine booster vaccinations are thought to be needed to trigger recall immunity and maintain efficacy against new VOCs (A. R. Falsey et al., "SARS-CoV-2 Neutralization with BNT162b2 Vaccine Dose 3," The New England journal of medicine. 385, 1627-1629 (2021), doi:10.1056/NEJMc2113468; A. Choi et al., "Safety and immunogenicity of SARS-CoV-2 variant mRNA vaccine boosters in healthy adults," Nature medicine. 27, 2025-2031 (2021), doi:10.1038/s41591-021-01527-y; and N. Andrews et al., "Effectiveness of COVID-19 booster vaccines against covid-19 related symptoms, hospitalisation and death in England," Nature medicine (2022), doi:10.1038/s41591-022-01699-1 .

Long-lived memory B (B_{MEM}) cells are the basis for the recall response upon antigen reencounter either by infection or booster vaccination. They play an important role in the maintenance and evolution of the antiviral antibody response against variants, since low-affinity selection mechanisms during the germinal center reaction and continued hypermutation of B_{MEM} cells expand the breadth of viral variant recognition over time (W. E. Purtha, et al., "Memory B cells, but not long-lived plasma cells, possess antigen specificities for viral escape mutants," The Journal of experimental medicine, 208, 2599-2606 (2011) doi:10.1084/jem.20110740; and Y. Adachi et al., "Distinct germinal center selection at local sites shapes memory B cell response to viral escape," The Journal of experimental medicine. 212, 1709-1723 (2015), doi:10.1084/jem.20142284).

How vaccine-mediated protective immunity will evolve over time and will be modified by iterations of exposure to COVID-19 vaccines and infections with increasingly divergent viral variants, is of particular relevance with the emergence of antigenically distinct VOCs. Omicron is the evolutionarily most distant reported VOC with a hitherto unprecedented number of amino acid alterations in its S glycoprotein, including at least 15 amino acid changes in the RBD and extensive changes in the N-terminal domain (NTD). These alterations are predicted to affect most neutralizing antibody epitopes. In addition, Omicron is highly transmissible, and its sublineages BA.1 and BA.2 have spread rapidly across the globe, outcompeting Delta within weeks to become the dominant circulating VOC (W. Dejnirattisai et al., "SARS-CoV-2 Omicron-B.1.1.529 leads to widespread escape from neutralizing antibody responses," Cell. 185, 467-484.e15 (2022), doi:10.1016/j.cell.2021.12.046; and M. Hoffmann et al., "The Omicron variant is highly resistant against antibody-mediated neutralization," Cell. 185, 447-456.e11 (2022), doi:10.1016/j.cell.2021.12.032).

To date, over 1 billion people worldwide have been vaccinated with the mRNA-based COVID-19 vaccine BNT162b2 and have received the primary 2-dose series or further boosters. This vaccine is contributing substantially to the pattern of population immunity in many regions on which further immune editing and effects of currently spreading variants will build upon.

To characterize the effect of Omicron BA.1 breakthrough infection on the magnitude and breadth of serum neutralizing activity and B_{MEM} cells, blood samples from individuals that were double- or triple-vaccinated with BNT162b2 were studied.

As understanding of the antigen-specific B cell memory pool is a critical determinant of an individual's ability to respond to newly emerging variants, this data can help to guide vaccine development.

### Results and discussion

### Cohorts and sampling

Blood samples have been sourced from the biosample collection of BNT162b2 vaccine trials, and a biobank of prospectively collected samples from vaccinated individuals with subsequent SARS-CoV-2 Omicron BA.1 breakthrough infection. Samples were selected to investigate biomarkers in four independent groups, namely individuals who were (i) double- or (ii) triple-vaccinated with BNT162b2 without a prior or breakthrough infection at the time of sample collection (BNT162b2², BNT162b2³) and individuals who were (iii) double- or (iv) triple-vaccinated with BNT162b2 and who experienced breakthrough infection with the SARS-CoV-2 Omicron BA.1 variant after a median of approximately 5 months or 4 weeks, respectively (BNT162b2² + Omi, BNT162b2³ + Omi) (see materials and methods below). Immune sera were used to characterize Omicron BA.1 infection-associated changes to the magnitude and the breadth of serum neutralizing activity. PBMCs were used to characterize the VOC-specificity of peripheral B_{MEM} cells recognizing the respective full-length SARS-CoV-2 S protein or its RBD (Fig. 15).

### Omicron breakthrough infection of BNT162b2 double- and triple-vaccinated individuals induces broad neutralization of Omicron BA.1, BA.2 and other VOCs

To evaluate the neutralizing activity of immune sera, two orthogonal test systems were used: a well-characterized pseudovirus neutralization test (pVNT) to investigate the breadth of inhibition of virus entry in a propagation-deficient set-up, as well as a live SARS-CoV-2 neutralization test (VNT) designed to evaluate neutralization during multicycle replication of authentic virus with the antibodies maintained throughout the entire test period. For the former, pseudoviruses bearing S proteins comprising mutations characteristic of Omicron sublineages BA.1 or BA.2, other SARS-CoV-2 VOCs (Wuhan, Alpha, Beta, Delta) were used to assess breadth while pseudoviruses bearing the S proteins of SARS-CoV-1 (T. Li et al., "Phylogenetic supertree reveals detailed evolution of SARS-CoV-2," Scientific reports, 10, 22366 (2020), doi:10.1038/s41598-020-79484-8) was used to detect potential pan-Sarbecovirus neutralizing activity (C.-W. Tan et al., "Pan-Sarbecovirus Neutralizing Antibodies in BNT162b2-Immunized SARS-CoV-1 Survivors," The New England journal of medicine, 385, 1401-1406 (2021), doi:10.1056/NEJMoa2108453).

As reported previously (A. R. Falsey et al., "SARS-CoV-2 Neutralization with BNT162b2 Vaccine Dose 3," The New England journal of medicine, 385, 1627-1629 (2021), doi:10.1056/NEJMc2113468; and C.-W. Tan et al., "Pan-Sarbecovirus Neutralizing Antibodies in BNT162b2-Immunized SARS-CoV-1 Survivors," The New England journal of medicine. 385, 1401-1406 (2021), doi:10.1056/NEJMoa2108453), in Omicron-naïve double-vaccinated individuals 50% pseudovirus neutralization (pVN₅₀) geometric mean titers (GMTs) of Beta and Delta VOCs were reduced, and neutralization of both Omicron sublineages was virtually undetectable. In Omicron-naïve triple-vaccinated individuals, pVNso GMTs against all tested VOCs were substantially higher with robust neutralization of Alpha, Beta and Delta variants. While GMTs against Omicron BA.1 were significantly lower compared to Wuhan (GMT 160 vs 398), titers against Omicron BA.2 were also considerably reduced at 211. Thus, triple vaccination induced a similar level of neutralization against the two Omicron sublineages (Fig. 16, A) (A. Muik et al., "Neutralization of SARS-CoV-2 Omicron by BNT162b2 mRNA vaccine-elicited human sera," Science (New York, N.Y.), 375, 678-680 (2022), doi:10.1126/science.abn7591; C.-W. Tan et al., "Pan-Sarbecovirus Neutralizing Antibodies in BNT162b2-Immunized SARS-CoV-1 Survivors," The New England journal of medicine, 385, 1401-1406 (2021), doi:10.1056/NEJMoa2108453; J. Liu et al., "BNT162b2-elicited neutralization of B.1.617 and other SARS-CoV-2 variants," Nature, 596, 273-275 (2021), doi:10.1038/s41586-021-03693-y; A. Muik et al., "Neutralization of SARS-CoV-2 lineage B.1.1.7 pseudovirus by BNT162b2 vaccine-elicited human sera," Science (New York, N.Y.), 371, 1152-1153 (2021), doi:10.1126/science.abg6105; and Y. Liu et al., "Neutralizing Activity of BNT162b2-Elicited Serum," The New England journal of medicine, 384, 1466-1468 (2021), doi:10.1056/NEJMc2102017).

Omicron BA.1-breakthrough infection had a marked effect on magnitude and breadth of the neutralizing antibody response of both double- and triple-vaccinated individuals, with slightly higher pVN₅₀ GMTs observed in the triple-vaccinated individuals (Fig. 16, A). The pVNso GMT of double-vaccinated individuals with breakthrough infection against Omicron BA.1 and BA.2 was more than 100-fold and 35-fold above the GMTs of Omicron BA.1-naïve double-vaccinated individuals. Immune sera from double-vaccinated individuals with breakthrough infection had broad neutralizing activity, with higher pVNso GMTs against Beta and Delta than observed in Omicron-naïve triple-vaccinated individuals (GMT 740 vs. 222 and 571 vs. 370). The effect of Omicron BA.1-breakthrough infection on the neutralization of Omicron BA.1 and BA.2 pseudovirus was less pronounced when looking at triple-vaccinated individuals (approximately 7-fold and 4-fold increased neutralization compared to Omicron-naïve triple-vaccinated individuals). pVN₅₀ GMTs against Omicron BA.1, BA.2 and Delta were 1029, 836 and 1103 in triple-vaccinated Omicron breakthrough individuals as compared to 160, 211 and 370 in the Omicron-naïve triple-vaccinated. GMTs against all SARS-CoV-2 VOCs, including Beta and Omicron, were close to titers against the Wuhan reference, while noticeably reduced in triple-vaccinated Omicron-naïve individuals.

Likewise, while sera from vaccinated Omicron-naïve individuals had no detectable or only poor pVN₅₀ titers against the phylogenetically more distant SARS-CoV-1, convalescent sera of double- and even more markedly of triple-vaccinated Omicron infected individuals robustly neutralized SARS-CoV-1 pseudovirus (Fig. 16, A and B). Nine out of 18 breakthrough infected individuals (four double-vaccinated and five triple-vaccinated) had SARS-CoV-1 pVNso GMTs comparable to or above those against the Wuhan reference in Omicron-naïve double-vaccinated individuals (GMT≥120).

Authentic live SARS-CoV-2 virus neutralization assays conducted with Wuhan, Beta, Delta and Omicron BA.1 pseudoviruses also showed similar findings (Fig. 16, B). In BNT162b2 double- and triple-vaccinated individuals, Omicron BA.1 infection was associated with a strongly increased neutralizing activity against Omicron BA.1 with 50% virus neutralization (VN₅₀) GMTs in the same range as against the Wuhan strain (Fig. 16, B; GMT 493 vs. 381 and GMT 538 vs. 613). Similarly, Omicron BA.1 convalescent double- and triple-vaccinated individuals showed comparable levels of neutralization against other variants as well (e.g., GMT 493 and 729 against Beta), indicating a wide breadth of neutralizing activity.

In aggregate, these data demonstrate that SARS-CoV-2 Omicron BA.1 breakthrough infection induces neutralization activity of profound breadth in vaccine-experienced individuals, a finding further supported by the calculated ratios of VNso GMTs against the Wuhan strain and SARS-CoV-2 VOCs (Fig. 16, C). While double- and to a lesser extent also triple-BNT162b2 vaccinated Omicron-naïve individuals displayed marked differences in neutralization proficiency against VOCs, neutralization activity of Omicron BA.1 convalescent subjects was leveled to almost the same range of high performance against all variant strains tested. Likewise, Omicron BA.1 breakthrough infection had a similarly broad neutralization augmenting effect in individuals vaccinated with other approved COVID-19 vaccines or heterologous regimens (Fig. 19; Table 20).

**Table 20. Individuals vaccinated with other approved COVID-19 vaccines or mixed regimens after subsequent Omicron BA.1 breakthrough infection**

| **Vaccination** | **Omicron subtype** | **Dose 1-2 interval** | **Dose 2-3 interval** | **Positive test after last after vaccination** | **Blood draw after positive test** |
|---|---|---|---|---|---|
| **AZ/BNT** | n/a | 62 | N/A | 142 | 46 |
| **AZ/BNT** | n/a | 68 | N/A | 135 | 45 |
| **J&J** | n/a | N/A | N/A | 161 | 44 |
| **MOD³** | BA.1 | 42 | 172 | 3 | 35 |
| **MOD²** | n/a | 42 | N/A | 169 | 44 |
| **J&J/BNT** | n/a | 138 | N/A | 45 | 40 |
| **AZ/BNT/MO D** | BA.1 | 68 | 154 | 9 | 43 |
| **MOD²/BNT** | n/a | 28 | 252 | 22 | 40 |
| **MOD²/BNT** | n/a | 42 | 154 | 13 | 42 |
| **MOD²/BNT** | n/a | 28 | 256 | 45 | 31 |
| | | | **Median:** | 45 | 43 |

| | | | | | |
|---|---|---|---|---|---|
| n/a, not available; N/A, not applicable; AZ, AstraZeneca AZD1222; BNT, BioNTech/Pfizer BNT162b2; J&J, Johnson & Johnson Ad26.COV2.S; MOD, Moderna mRNA-1273; BNT⁴, BNT162b2 four-dose series; MOD², mRNA-1273 two-dose series; MOD³, mRNA-1273 three-dose series | | | | | |

### B_{MEM} cells of BNT162b2 double- and of triple-vaccinated individuals broadly recognize VOCs and are further boosted by Omicron breakthrough infection

Next, the phenotype and quantity of SARS-CoV-2 S protein specific B cells were investigated. Flow cytometry-based B cell phenotyping assays were used for differential detection of variant-specific S protein-binding B cells in bulk PBMCs. All S protein- and RBD-specific B cells in the peripheral blood were found to be of a B_{MEM} phenotype (B_{MEM}; CD^{20high}CD^{38int/neg}), as antigen-specific plasmablasts or naïve B cells were not detected (data not shown). The assays therefore allowed the differentiation for each of the SARS-CoV-2 variants between B_{MEM} cells recognizing the full S protein or its RBD that is a hotspot for amino acid alterations, and variant-specific antigenic epitopes (Fig. 17, A).

The overall frequency of antigen-specific B_{MEM} cells varied across the different groups. The frequency of B_{MEM} cells in Omicron-naïve double-vaccinated individuals was low at an early time point after vaccination and increased over time: At 5 months as compared to 3 weeks after the second BNT162b2 dose, S protein-specific B_{MEM} cells almost quadrupled, RBD-specific ones tripled across all VOCs thereby reaching quantities similar to those observed in Omicron-naïve triple-vaccinated individuals (Fig. 17, B and C).

Double or triple BNT162b2-vaccinated individuals with a SARS-CoV-2 Omicron BA.1 breakthrough infection exhibited a strongly increased frequency of B_{MEM} cells, which was higher than those of Omicron-naïve triple-vaccinated individuals (Fig. 17, B and D).

In all groups, including Omicron-naïve and Omicron infected individuals, B_{MEM} cells against Omicron BA.1 S protein were detectable at frequencies comparable to those against Wuhan and other tested VOCs (Fig 17, B and D), whereas the frequency of B_{MEM} cells against Omicron BA.1 RBD was slightly lower compared to the other variants (Fig. 17, C and E).

The ratios of RBD protein to S protein binding within the different groups was then compared and found to be biased towards S protein recognition for the Omicron BA.1 VOC, particularly in the Omicron-naïve groups (Fig. 17, F). In the Omicron BA.1-experienced groups this ratio is higher, indicating that an Omicron BA.1 breakthrough infection improved Omicron BA.1 RBD recognition.

*Omicron* BA.1 *breakthrough infection in BNT162b2 double- and triple-vaccinated individuals primarily boosts B_{MEM} cells against conserved epitopes shared broadly between S proteins of Wuhan and other VOCs rather than strictly Omicron S-specific epitopes.*

These findings indicate that Omicron BA.1 infection in vaccinated individuals boosts not only neutralizing activity and B_{MEM} cells against Omicron BA.1, but broadly augments immunity against various VOCs. To investigate the specificity of antibody responses at a cellular level, multi-parameter analyses of B_{MEM} cells stained with fluorescently labeled variant-specific S or RBD proteins were performed.

A combinatorial gating strategy was applied to distinguish between B_{MEM} cell subsets that could identify only single variant-specific epitopes of Wuhan, Alpha, Delta or Omicron BA.1, versus those that could identify any given combination thereof (Fig 18, A).

In a first analysis, B_{MEM} cell recognition of Wuhan and Omicron BA.1 S and RBD proteins was evaluated (Fig. 18, B, C, and D). The SARS-CoV-2 Omicron BA.1 variant has 37 amino acid alterations in the S protein compared to the Wuhan parental strain, of which 15 alterations are in the RBD, an immunodominant target of neutralizing antibodies induced by COVID-19 vaccines or by SARS-CoV-2 infections.

Staining with full length S proteins showed that the largest proportion of B_{MEM} cells from Omicron-naïve double-vaccinated individuals, and even more predominantly from triple-vaccinated individuals were directed against epitopes shared by both Wuhan and Omicron BA.1 SARS-CoV-2 variants. Consistent with the observation that vaccination with BNT162b2 can elicit immune responses against wild-type epitopes that do not recognize the corresponding altered epitopes in the Omicron BA.1 S protein (Fig. 18, B and C), in most individuals a smaller but clearly detectable proportion of B_{MEM} cells was found that recognized only Wuhan S protein or RBD. Consistent with the lack of exposure, no B_{MEM} cells binding exclusively to Omicron BA.1 S or RBD protein were detected in these Omicron-naïve individuals.

In Omicron BA.1 convalescent individuals, frequencies of B_{MEM} cells recognizing S protein epitopes shared between Wuhan and Omicron BA.1 were significantly higher than in the Omicron-naïve ones (Fig. 18, B and C). In most of these subjects, a small proportion of exclusively Wuhan S protein-specific B_{MEM} cells was found, as well as a slightly lower frequency of exclusively Omicron BA.1 variant S protein-specific ones.

A similar but slightly different pattern was observed by B cell staining with labeled RBD proteins (Fig. 18, B and D). Again, Omicron BA.1 breakthrough infection of double-/triple-vaccinated individuals was found to primarily boost B_{MEM} cells reactive with conserved epitopes. A moderate boost of Wuhan-specific reactivities was observed; however, only small populations of Omicron-RBD-specific B_{MEM} cells were detected in the tested individuals (Fig. 18, D).

Next, the combinatorial gating approach was used to identify the subsets of S protein or RBD binding B_{MEM} cells that either bind exclusively to Wuhan or Omicron BA.1, or to common epitopes conserved broadly across all four variants, Wuhan, Alpha, Delta and Omicron BA.1 (Fig 18, E). Across all four study groups, the frequency of B_{MEM} cells recognizing S protein epitopes was found to be conserved across all tested variants, accounting for the largest fraction of the pool of S protein-binding B_{MEM} cells (Fig. 18, F, all 4+ve). The S protein of the Wuhan strain does not have an exclusive amino acid change that distinguishes it from the spike proteins of the Alpha, Delta, or Omicron BA.1 VOCs. Accordingly, B_{MEM} cells exclusively recognizing the Wuhan S protein were hardly detected in any individual (Fig. 18, F). In several individuals with Omicron BA.1 breakthrough infection, a small proportion of B_{MEM} cells was detected that bound exclusively to Omicron BA.1 S protein (Fig. 18, F)=, whereas almost none of the individuals displayed a strictly Omicron BA.1 RBD-specific response (Fig. 18, G).

These findings indicate that SARS-CoV-2 Omicron BA.1 breakthrough infection in vaccinated individuals primarily expands a broad B_{MEM} cell repertoire against conserved S protein and RBD epitopes, rather than inducing large numbers of Omicron-specific B_{MEM} cells.

To further dissect this response, the B_{MEM} subsets directed against the RBD were characterized. The combinatorial Boolean gating approach was used to discern B_{MEM} cells with distinct binding patterns in the spectrum of strictly variant-specific and common epitopes shared by several variants. Multiple sequence alignments revealed that the Omicron BA.1 RBD diverges from the RBD sequence regions conserved in Wuhan, Alpha and Delta by 13 single amino acid alterations. All Omicron BA.1 convalescent individuals were found to have robust frequencies of B_{MEM} cells that recognized Wuhan, Alpha as well as the Delta VOC RBDs, but not Omicron BA.1 RBD, while B_{MEM} cells exclusively reactive with Omicron BA.1 RBD were almost absent in most of those individuals (Fig. 18, H). B_{MEM} cells that exclusively recognized the Omicron BA.1 and Alpha RBDs, or the Omicron BA.1 and Delta RBDs were also not detected.

Furthermore, in all individuals two additional subsets of RBD-specific B_{MEM} cells were identified. One subset was characterized by binding to Wuhan, Alpha and Omicron BA.1, but not Delta, RBD. The other population exhibited binding to Wuhan and Alpha but not Omicron BA.1 or Delta RBD (Fig. 18, H). Sequence alignment identified L452R as the only RBD mutation unique for Delta that is not shared by the other 3 variant RBDs (Fig. 18, I top). Similarly, the only RBD site conserved in Wuhan and Alpha but altered in Delta and Omicron BA.1 was found to be T478K (Fig 18, I bottom). Both L452R and T478K alterations are known to be associated with the evasion of vaccine induced neutralizing antibody responses. Of note, no B_{MEM} cells were detected in all combinatorial subgroups in which multiple sequence alignment failed to identify unique epitopes in the RBD sequence that satisfied the Boolean selection criteria (e.g., Wuhan only or Wuhan and Omicron BA.1, but not Alpha, Delta). These findings indicate that the B_{MEM} cell response against RBD is driven by specificities induced through prior vaccination with BNT162b2 and not substantially redirected against new RBD epitopes mutated in the Omicron variant after infection.

### Summary

SARS-CoV-2 Omicron is a partial immune escape variant with an unprecedented number of amino acid alterations in the S protein at sites of neutralizing antibody binding, distinguishing it from previously reported variants. Recent neutralizing antibody mapping and molecular modeling studies strongly support the functional relevance of these alterations, and their importance is confirmed by the observation that double-vaccinated individuals have no detectable neutralizing activity against SARS-CoV-2 Omicron.

The findings presented herein show that Omicron BA.1 breakthrough infection of vaccinated individuals boosts not only neutralizing activity and B_{MEM} cells against Omicron BA.1 but broadly augments immunity against various VOCs, and also provide insights into how broad immunity is acheived

The data presented herein indicate that initial exposure to the Wuhan strain S protein may have shaped the formation of B_{MEM} cells and imprinted against the formation of novel B_{MEM} cell responses against the more distinctive epitopes of the Omicron BA.1 variant. Similar observations have been reported from vaccinated individuals who experienced breakthrough infections with the delta variant (K. Röltgen et al., "Immune imprinting, breadth of variant recognition, and germinal center response in human SARS-CoV-2 infection and vaccination," Cell (2022), doi:10.1016/j.cell.2022.01.018.). As demonstrated in the present Example, Omicron BA.1 breakthrough infection primarily expands a broad B_{MEM} cell repertoire against conserved S protein and RBD epitopes, rather than inducing considerable numbers of strictly Omicron-specific B_{MEM} cells.

Thus, Omicron BA.1 breakthrough infection in double-vaccinated individuals leads to expansion of the pre-existing B_{MEM} cell pool, similar to a third dose of booster vaccination. However, there are clear differences in the immune response pattern induced by a homologous vaccine booster as compared to an Omicron BA.1 breakthrough infection. Despite the focus of the B cell memory response on conserved epitopes, Omicron BA.1 breakthrough infection leads to a more substantial increase in antibody neutralization titers against Omicron BA.1, as well as pronounced cross-neutralization of both the ancestral and the novel SARS CoV-2 variants. These effects are particularly striking in double-vaccinated individuals.

Without wishing to be bound by theory, three findings may point to potentially complementary and synergistic mechanisms responsible for these results:
First, an overall increase of S protein-specific B_{MEM} cells. Omicron BA.1-convalescent double-vaccinated individuals have a higher frequency of B_{MEM} cells and higher neutralizing antibody titers against all VOCs as compared to triple-vaccinated individuals. That breakthrough infection elicits a stronger neutralizing antibody response than the 3rd vaccine dose in double-vaccinated individuals is not apparent from previous studies describing breakthrough infections with other variants (Evans et al., Science Translational Medicine (2022) 14, eabn8057) and may be explained by poor neutralization of the Omicron BA.1 variant in the initial phase of infection, potentially causing a greater or prolonged antigen exposure of the immune system to the altered S protein.
Second, a stronger bias on RBD-specific B_{MEM} cell responses. Omicron BA.1 breakthrough infection promotes proportionally more pronounced boosting of RBD-specific B_{MEM} cells than of B_{MEM} cells that recognize S protein-specific epitopes outside the RBD. Therefore, Omicron BA.1 -infected individuals have a significantly higher ratio of RBD/S protein-specific B_{MEM} cells compared to vaccinated Omicron-naïve individuals. The RBD is a key domain of the S protein that binds to the SARS-CoV-2 receptor ACE2 and has multiple neutralizing antibody binding sites in regions that are not affected by Omicron BA.1 alterations, e.g., position L452. An increased focus of the immune response on this domain could promote B_{MEM} cells producing neutralizing antibodies against RBD epitopes that are not altered in Omicron BA.1.
Third, the induction of broadly neutralizing antibodies. The majority of sera from Omicron-BA.1 convalescent but not from Omicron-naïve vaccinated individuals was found to robustly neutralize SARS-CoV-1. This may indicate that Omicron BA.1 infection in vaccinated individuals stimulates B_{MEM} cells that form neutralizing antibodies against spike protein epitopes conserved in the SARS-CoV-1 and SARS-CoV-2 families. It was reported that broadly neutralizing antibodies are present in SARS-CoV-1 infected individuals vaccinated with BNT162b2. Such pan-Serbecovirus immune responses are thought to be triggered by neutralizing antibodies to highly conserved S protein domains. The greater antigenic distance of the Omicron BA.1 spike protein from the other SARS-Cov-2 strains may promote targeting of conserved subdominant neutralizing epitopes as recently described to be located in the C-terminal portion of the spike protein.

In aggregate, these results indicate that despite possible imprinting of the immune response by previous vaccination, the preformed B-cell memory pool can be refocused and quantitatively remodeled by exposure to heterologous S proteins to allow neutralization of variants that evade a previously established neutralizing antibody response.

In conclusion, while the data are based on samples from individuals exposed to the Omicron BA.1 S protein as a result of infection, the findings presented herein support that a vaccine adapted to the Omicron BA.1 S protein can similarly reshape the B-cell memory repertoire and therefore can be more beneficial than an extended series of boosters with the existing Wuhan-Hu-1 spike based vaccines.

### Materials and Methods

### Recruitment of participants and sample collection

Individuals from the SARS-CoV-2 Omicron-naïve BNT162b2 double-vaccinated (BNT162b2²) and triple-vaccinated (BNT162b2³) cohorts provided informed consent as part of their participation in a clinical trial (the Phase 1/2 trial BNT162-01 [NCT04380701], the Phase 2 rollover trial BNT162-14 [NCT04949490], or as part of the BNT162-17 [NCT05004181] trial). Participants from the SARS-CoV-2 Omicron BA.1 convalescent double- and triple vaccinated cohorts (BNT162b2² + Omi and BNT162b2³ + Omi cohorts, respectively) and individuals vaccinated with other approved COVID-19 vaccines or mixed regimens with subsequent Omicron BA.1 breakthrough infection were recruited from University Hospital, Goethe University Frankfurt as part of a research program that recruited patients that had experienced Omicron BA.1 breakthrough infection following vaccination for COVID-19, to provide blood samples and clinical data for research. Infection with the Omicron BA.1 strain was confirmed with variant-specific PCR or sequencing, and participants were free of symptoms at the time of blood collection.

### Sampling timepoints are provided in Fig. 15.

Serum was isolated by centrifugation 2000 × g for 10 minutes and cryopreserved until use. Li-Heparin blood samples were isolated by density gradient centrifugation using Ficoll-Paque PLUS (Cytiva) and were subsequently cryopreserved until use.

### VSV-SARS-CoV-2 S variant pseudovirus generation

A recombinant replication-deficient vesicular stomatitis virus (VSV) vector that encodes green fluorescent protein (GFP) and luciferase instead of the VSV-glycoprotein (VSV-G) was pseudotyped with SARS-CoV-1 spike (S) (UniProt Ref: P59594) and with SARS-CoV-2 S derived from either the Wuhan reference strain (NCBI Ref: 43740568), the Alpha variant (mutations: Δ69/70, Δ144, N501Y, A570D, D614G, P681H, T716I, S982A, D1118H), the Beta variant (mutations: L18F, D80A, D215G, Δ242-244, R246I, K417N, E484K, N501Y, D614G, A701V), the Delta variant (mutations: T19R, G142D, E156G, Δ157/158, K417N, L452R, T478K, D614G, P681R, D950N) the Omicron BA.1 variant (mutations: A67V, Δ69/70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F) or the Omicron BA.2 variant (mutations: T19I, Δ24-26, A27S, G142D, V213G, G339D, S371F, S373P, S375F, T376A, D405N, R408S, K417N, N440K, S477N, T478K, E484A, Q493R, Q498R, N501Y, Y505H, D614G, H655Y, N679K, P681H, N764K, D796Y, Q954H, N969K) according to published pseudotyping protocols (M. Berger Rentsch, G. Zimmer, A vesicular stomatitis virus replicon-based bioassay for the rapid and sensitive determination of multi-species type I interferon. PloS one. 6, e25858 (2011), doi:10.1371/journal.pone.0025858).

In brief, HEK293T/17 monolayers (ATCC^{®} CRL-11268^{™}) cultured in Dulbecco's modified Eagle's medium (DMEM) with GlutaMAX^{™} (Gibco) supplemented with 10% heat-inactivated fetal bovine serum (FBS [Sigma-Aldrich]) (referred to as medium) were transfected with Sanger sequencing-verified SARS-CoV-1 or variant-specific SARS-CoV-2 S expression plasmid with Lipofectamine LTX (Life Technologies) following the manufacturer's instructions. At 24 hours VSV-G complemented VSVΔG vector. After incubation for 2 hours at 37 °C with 7.5% CO₂, cells were washed twice with phosphate buffered saline (PBS) before medium supplemented with anti-VSV-G antibody (clone 8G5F11, Kerafast Inc.) was added to neutralize residual VSV-G-complemented input virus. VSV-SARS-CoV-2-S pseudotype-containing medium was harvested 20 hours after inoculation, passed through a 0.2 µm filter (Nalgene) and stored at -80 °C. The pseudovirus batches were titrated on Vero 76 cells (ATCC^{®} CRL-1587^{™}) cultured in medium. The relative luciferase units induced by a defined volume of a Wuhan spike pseudovirus reference batch previously described in Muik et al. (Muik et al., "Neutralization of SARS-CoV-2 lineage B.1.1.7 pseudovirus by BNT162b2 vaccine-elicited human sera. Science (New York, N.Y.). 371, 1152-1153 (2021), doi:10.1126/science.abg6105") that corresponds to an infectious titer of 200 transducing units (TU) per mL, was used as a comparator. Input volumes for the SARS-CoV-2 variant pseudovirus batches were calculated to normalize the infectious titer based on the relative luciferase units relative to the reference.

### Pseudovirus neutralization assay

Vero 76 cells were seeded in 96-well white, flat-bottom plates (Thermo Scientific) at 40,000 cells/well in medium 4 hours prior to the assay and cultured at 37 °C with 7.5% CO₂. Each serum was serially diluted 2-fold in medium with the first dilution being 1:5 (Omicron naïve double- and triple BNT162b2 vaccinated; dilution range of 1:5 to 1:5,120) or 1:30 (double- and triple BNT162b2 vaccinated after subsequent Omicron breakthrough infection; dilution range of 1:30 to 1:30,720). VSV-SARS-CoV-2-S/VSV-SARS-CoV-1-S particles were diluted in medium to obtain 200 TU in the assay. Serum dilutions were mixed 1:1 with pseudovirus (n=2 technical replicates per serum per pseudovirus) for 30 minutes at room temperature before being added to Vero 76 cell monolayers and incubated at 37 °C with 7.5% CO₂ for 24 hours. Supernatants were removed and the cells were lysed with luciferase reagent (Promega). Luminescence was recorded on a CLARIOstar^{®} Plus microplate reader (BMG Labtech), and neutralization titers were calculated as the reciprocal of the highest serum dilution that still resulted in 50% reduction in luminescence. Results were expressed as geometric mean titers (GMT) of duplicates. If no neutralization was observed, an arbitrary titer value of half of the limit of detection [LOD] was reported.

### Live SARS-CoV-2 neutralization assay

SARS-CoV-2 virus neutralization titers were determined by a microneutralization assay based on cytopathic effect (CPE) at VisMederi S.r.l., Siena, Italy. In brief, heat-inactivated serum samples from participants were serially diluted 1:2 (starting at 1:10) and incubated for 1 hour at 37 °C with 100 TCID50 of live Wuhan-like SARS-CoV-2 virus strain 2019-nCOV/ITALY-INMI1 (GenBank: MT066156), Beta virus strain Human nCoV19 isolate/England ex-SA/HCM002/2021 (mutations: D80A, D215G, Δ242-244, K417N, E484K, N501Y, D614G, A701V), sequence-verified Delta strain isolated from a nasopharyngeal swab (mutations: T19R, G142D, E156G, Δ157/158, L452R, T478K, D614G, P681R, R682Q, D950N) or Omicron BA.1 strain hCoV-19/Belgium/rega-20174/2021 (mutations: A67V, Δ69/70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F) to allow any antigen-specific antibodies to bind to the virus. The 2019-nCOV/ITALY-INMI1 strain S protein is identical in sequence to the wild-type SARS-CoV-2 S (Wuhan-Hu-1 isolate). Vero E6 (ATCC^{®} CRL-1586^{™}) cell monolayers were inoculated with the serum/virus mix in 96-well plates and incubated for 3 days (2019-nCOV/ITALY-INMI1 strain) or 4 days (Beta, Delta and Omicron BA.1 variant strain) to allow infection by non-neutralized virus. The plates were observed under an inverted light microscope and the wells were scored as positive for SARS-CoV-2 infection (i.e., showing CPE) or negative for SARS-CoV-2 infection (i.e., cells were alive without CPE). The neutralization titer was determined as the reciprocal of the highest serum dilution that protected more than 50% of cells from CPE and reported as GMT of duplicates. If no neutralization was observed, an arbitrary titer value of 5 (half of the limit of detection [LOD]) was reported.

### Detection and characterization of SARS-CoV-2-specific B cells with flow cytometry

Spike/RBD-specific B cells were detected using recombinant, biotinylated SARS-CoV-2 Spike (Acro Biosystems: Wuhan - SPN-C82E9, Alpha - SPN-C82E5, Delta - SPN-C82Ec, Omicron - SPN-C82Ee) and RBD (Acro Biosystems: Wuhan - SPD-B28E9, Alpha - SPD-C82E6, Delta - SPD-C82Ed, Omicron - SPD-C82E4) proteins. Recombinant Spike and RBD proteins were tetramerized with fluorescently labeled Streptavidin (BioLegend, BD Biosciences) in a 4:1 molar ratio for 1 h at 4 °C in the dark. Afterwards samples were spun down for 10 min at 4°C to remove eventual precipitates.

For flow cytometric analysis, PBMCs were thawed and 5×10⁶ cells per sample were seeded into 96 U-bottom plates. Cells were blocked for Fc-receptor-binding (Human BD Fc Block^{™}, BD Biosciences) and statured with free biotin (D-Biotin, Invitrogen, 1 µM) in flow buffer (DPBS (Gibco) supplemented with 2% FBS (Sigma), 2 mM EDTA (Sigma-Aldrich)) for 20 min at 4 °C. Cells were washed and labeled with BCR bait tetramers supplemented with free Biotin in flow buffer (D-Biotin, Invitrogen, 2 µg/ml) for 1 h at 4 °C in the dark (2 µg/ml for Spike and 0,25 µg/ml for RBD proteins). Cells were washed with flow buffer and stained for viability (Fixable Viability Dye eFluor^{™} 780, eBioscience) and surface markers (CD3 - clone: UCHT1(BD Biosciences), CD4 - clone: SK3 (BD Biosciences), CD185 (CXCR5) - clone: RF8B2 (BioLegend), CD279 (PD-1) - clone: EH12.1(BD Biosciences), CD278 (ICOS) - clone: C398.4A (BioLegend) , CD19- clone: SJ25C1(BD Biosciences), CD20 - clone: 2H7(BD Biosciences), CD21 - clone: B-ly4(BD Biosciences), CD27 - clone: L128(BD Biosciences), CD38 - clone: HIT2(BD Biosciences), CD11c - clone: S-HCL-3(BD Biosciences), CD138 - clone: MI15(BD Biosciences), IgG - clone: G18-145(BD Biosciences), IgM - clone: G20-127(BD Biosciences), IgD - clone: IA6-2(BD Biosciences), CD14 - clone: MφP9 (BD Biosciences, dump channel), CD16 - clone: 3G8 (BD Biosciences, dump channel)) in flow buffer supplemented with Brilliant Stain Buffer Plus (BD Biosciences, according to the manufacturer's instructions) for 20 min at 4 °C. Samples were washed and fixed with BDTM Stabilizing Fixative (BD Biosciences, according to the manufacturer's instructions) prior to data acquisition on a BD Symphony A3 flow cytometer. FCS 3.0 files were exported from BD Diva Software and analyzed using FlowJo software (Version 10.7.1.).

Debris and doublets were discriminated via FSC/SSC. Then dead cells and monocytes (CD14, CD16 - Viability/Dump channel) were excluded. CD19 positive B cells were analyzed for IgD and CD27 expression, thereby naïve B cells were discriminated as IgD⁺ cells with the Boolean 'make non-gate' function. Within non-naïve B cells Plasmablasts (CD38^{high} CD20^{low}) and memory B cells (B_{MEM}s CD38^{int/low}CD20^{high}) were distinguished. B_{MEM} cells were analyzed for B cell bait binding. SARS-CoV-2 Spike reactivities were assessed by gating on each Spike/RBD variant tested by plotting against the CD20 signal. Bait gates were overlayed onto total B_{MEM} cells and displayed as NxN-Plots for the four bait channels.

### Statistical analysis

The statistical method of aggregation used for the analysis of antibody titers is the geometric mean and for the ratio of SARS-CoV-2 VOC titer and Wuhan titer the geometric mean and the corresponding 95% confidence interval. The use of the geometric mean accounts for the non-normal distribution of antibody titers, which span several orders of magnitude. The Friedman test with Dunn's correction for multiple comparisons was used to conduct pairwise signed-rank tests of group geometric mean neutralizing antibody titers with a common control group. Flow cytometric frequencies were analyzed with and tables were exported from FlowJo software (Version 10.7.1.). Statistical analysis of cumulative memory B cell frequencies was the mean and standard errors of the mean (SEM). All statistical analyses were performed using GraphPad Prism software version 9.

### Example 8: Induced antibody response of vaccines encoding a SARS-CoV-2 S protein from an Omicron variant

To test the efficacy of an RNA vaccine encoding a SARS-CoV-2 S protein comprising one or more mutations characteristic of an Omicron variant, subjects previously administered a primary regimen comprising two doses of 30 ug of RNA encoding a SARS-CoV-2 S protein from a Wuhan strain (e.g., BNT162b2), and a booster regimen comprising a dose of 30 ug of RNA encoding a SARS-CoV-2 S protein from a Wuhan strain (i.e., a Wuhan specific booster, e.g., BNT162b2) were administered a further booster dose comprising either (i) 30 ug of RNA encoding an SARS-CoV-2 S protein from a Wuhan strain (e.g., BNT162b2), or (ii) 30 ug of RNA encoding a SARS-CoV-2 S protein comprising mutations that are characteristic of an Omicron BA.1 variant (i.e., an Omicron BA.1-specific booster, e.g., RNA encoding a SARS-CoV-2 S protein comprising an amino acid sequence of SEQ ID NO: 49, and/or comprising a nucleotide sequence of SEQ ID NOs: 50 and/or 51) (the dose administered as part of the second booster regimen is referred to as a "4th dose" in the figures). Sera was collected from subjects at the time of administering the second booster regimen and one month afterwards.

Neutralization antibody titers were determined using a Fluorescent Focus Reduction Neutralization Test ("FFRNT"). Suitable FFRNT assays are known in the art, and include, e.g., the assays described in Zou J, Xia H, Xie X, et al. "Neutralization against Omicron SARS-CoV-2 from previous non-Omicron infection," Nat Commun 2022;13:852, the contents of which is incorporated by reference herein in its entirety. Additional exemplary neutralization assays include those described in the previous examples, as well as those described in Bewley, Kevin R., et al. "Quantification of SARS-CoV-2 neutralizing antibody by wild-type plaque reduction neutralization, microneutralization and pseudotyped virus neutralization assays." Nature Protocols 16.6 (2021): 3114-3140. As shown in Fig. 20, A, subjects administered a second booster regimen comprising a dose of RNA encoding a SARS-CoV-2 S protein comprising mutations characteristic of an Omicron BA.1 variant exhibited significant increases in concentrations of neutralization antibodies against an Omicron BA.1 variant, as compared to subjects administered a second booster regimen comprising a dose of RNA encoding a SARS-CoV-2 S protein of a Wuhan strain. Specifically, subjects administered an Omicron BA.1 specific booster exhibited a GMR that was 1.79-fold higher and a GMFR that was 2.31 fold higher than that observed in subjects administered a fourth dose of RNA encoding a SARS-CoV-2 S protein from a Wuhan strain. The superior immune response induced by an Omicron BA.1-specific booster against an Omicron BA.1 variant was further increased in subjects previously infected with SARS-CoV-2 (as determined by an antigen test) or currently infected with SARS-CoV-2 (as determined by PCR). See Fig. 20, B, which shows that a subject population including previously and/or currently infected subjects exhibited a GMR that is 2.94 fold higher, and a GMFR ratio that is 1.97 fold higher that observed in a subject population comprising previously and/or currently infected subjects administered an RNA vaccine encoding a SARS-CoV-2 S protein from a Wuhan strain.

Pseudovirus neutralization assays were also performed using a pseudovirus comprising a SARS-CoV-2 S protein of a Wuhan strain, using the same sera samples discussed above. Subjects administered RNA encoding a SARS-CoV-2 S protein from a Wuhan strain (e.g., BNT162b2) exhibited titers of neutralization antibodies that were similar to those observed in subjects administered an Omicron BA.1-specific booster, demonstrating that the two vaccines are at least similarly effective in their ability to induce an antibody response against a Wuhan strain. See Fig., 20C, which shows that the GMR and GMFR observed in subjects administered a Wuhan specific booster (e.g., BNT162b2) is similar to that observed in subjects administered an Omicron BA.1 specific booster (OMI). In subjects previously infected with SARS-CoV-2 (e.g., as determined by an antigen assay) or currently infected with SARS-CoV-2 (e.g., as determined by a PCR assay), subjects administered an Omicron BA.1 specific booster demonstrated an improved immune response as compared to subjects administered a booster specific for a Wuhan strain. See Fig., 20D, which shows that the GMR for subjects administered an Omicron BA.1 specific booster is about 1.4 fold that of subjects administered a Wuhan specific booster. Subjects administered an Omicron BA.1 specific booster also demonstrated a superior immune response against a delta variant in pseudovirus neutralization assays. See Fig., 20E, showing that the GMFR for subjects administered an Omicron BA.1-specific booster is about 1.20 fold higher than that observed in subjects administered a Wuhan specific booster. The superior immune response induced by an Omicron BA.1-specific booster against a delta variant was further increased in sera from subjects previously and/or currently infected with SARS-CoV-2. See Fig., 20F.

### Example 9: Immunogenicity study of Vaccines Encoding S proteins of SARS-CoV-2 Variants in Vaccine-Naive Subjects

To test the immunogenicity of various variant specific vaccines in vaccine naïve subjects, vaccine naïve mice were immunized twice with (a) saline (negative control), (b) an RNA vaccine encoding a SARS-CoV-2 S protein from a Wuhan strain, (c) an RNA vaccine encoding a SARS-CoV-2 S protein having mutations characteristic of an Omicron BA.1 variant (Omi), (d) an RNA vaccine encoding a SARS-CoV-2 S protein having mutations characteristic of a delta variant (Delta), (e) a bivalent vaccine comprising RNA encoding a SARS-CoV-2 S protein from a Wuhan strain and a SARS-CoV-2 S protein comprising mutations characteristic of an Omicron BA.1 variant (b2+Omi), and (f) a bivalent vaccine comprising RNA encoding a SARS-CoV-2 S protein having mutations characteristic of a delta variant and RNA encoding a SARS-CoV-2 S protein having mutations characteristics of an Omicron BA.1 variant (Delta+Omi). The immunogenicity of the RNA vaccines was investigated by focusing on the antibody immune response.

Sera was obtained 7 days after immunization, and analyzed using a pseudovirus neutralization assay (e.g., the assay described in Example 2), using pseudoviruses comprising a SARS-CoV-2 S protein from a Wuhan strain, a SARS-CoV-2 S protein comprising mutations characteristic of a beta variant, a SARS-CoV-2 S protein comprising mutations characteristic of a delta variant, or a SARS-CoV-2 S protein comprising mutations characteristic of an Omicron BA.1 variant. As shown in Fig., 21, bivalent vaccines were found to elicit the broadest immune response in vaccine naïve mice.

### Example 10: Induced antibody response of vaccines encoding a SARS-CoV-2 S protein comprising one or more mutations characteristic of a Beta variant in subjects previously administered an RNA vaccine encoding a SARS-CoV-2 S protein from a Wuhan strain

To test the efficacy of an RNA vaccine encoding a SARS-CoV-2 S protein comprising one or more mutations characteristic of a Beta variant, subjects previously administered a primary regimen comprising two doses each of 30 ug of RNA encoding a SARS-CoV-2 S protein from a Wuhan strain (in the present example, BNT162b2 (SEQ ID NO: 20)), were administered two booster doses, each comprising 30 ug of RNA encoding a SARS-CoV-2 S protein comprising one or more mutations characteristic of a Beta variant (referred to hereafter as a Beta-specific vaccine). In the present Example, construct RBP020.11 was administered as the Beta-specific vaccine. While in the present Example, the two booster doses were administered approximately one month apart, in some embodiments, the two booster doses can be administered at least 3 weeks apart, at least 4 weeks apart, at least 5 weeks apart, at least 6 weeks apart, at least 7 weeks apart, at least 8 weeks apart, or longer (e.g., in accordance with exemplary dosing regimens as described herein).

Sera were collected from subjects before administeration of BNT162b2, one month after administering two primary doses of BNT162b2, one month after administering a first dose of a Beta-specific vaccine, and one month after administering a second dose of a Beta-specific vaccine. Neutralization antibody titers against a pseudovirus comprising a SARS-CoV-2 S protein of a Wuhan strain or a SARS-CoV-2 S protein comprising one or more mutations characteristic of a Beta variant were measured using a pseudovirus neutralization assay (results shown in Fig. 22). Subjects exhibited an increase in neutralization antibody titers against both a Wuhan strain of SARS-CoV-2 and a Beta varaint following adminstration of the third and fourth doses of a Beta-specific vaccine.

### Example 11: Induced antibody response of vaccines encoding a SARS-CoV-2 S protein comprising one or more mutations characteristic of a Beta variant in vaccine naive subjects

To test the efficacy of an RNA vaccine encoding a SARS-CoV-2 S protein comprising one or more mutations characteristic of a Beta variant in vaccine naïve subjects, subjects who had not previouly been administered a SARS-CoV-2 vaccine, and did not show evidence of prior or current infection with SARS-CoV-2 (e.g., as assessed by an antibody test and/or a PCR test) were administered two doses each of 30 ug of RNA encoding a SARS-CoV-2 S protein comprising one or more mutations characteristic of a Beta variant (in the present example, RBP020.11). Sera was collected one month after administration of a second dose, and neutralization antibody titers were measured using a viral neutralization assay, using viral particles comprising either a SARS-CoV-2 S protein from a Wuhan strain or a SARS-CoV-2 S protein having one or more mutations characteristic of a Beta variant. Tables 15 and 16, below, show the results for the neutralization assay against Beta variant (results for the neutralization assay against a Wuhan strain are not shown). As shown in the tables, compared to vaccine-naïve subjects administered two doses of an RNA vaccine encoding a SARS-CoV-2 S protein from a Wuhan strain (in the present Example, BNT162b2), an RNA vaccine encoding a SARS-CoV-2 S protein having mutations characteristic of a Beta variant was found to induce a significantly stronger antibody response against a Beta variant.

**Table 21: Geometric Mean Fold Rise (GMFR) of titers of neutralization antibodies, from before Dose 1 to each subsequent time points, in BNT162b2-naïve subjects without evidence of infection up to 1 month after dose 2 and administered BNT162b2 or an RNA vaccine encoding a SARS-CoV-2 S protein having one or more mutations characteristic of a Beta variant**

| | | **Vaccine Group (as Assigned/Randomized)^{a}** | | | |
|---|---|---|---|---|---|
| | | **BNT162b2_{SA} (30 µg)** | | **BNT162b2 (30 µg)** | |
| **Assay** | **Dose/ Sampling Time Point^{b}** | **n^{c}** | **GMFR^{d} (95% CI^{d})** | **n^{c}** | **GMFR^{d} (95% CI^{d})** |
| SARS-CoV-2 neutralization assay-SA variant - NT50 | 2/1 Month | 272 | **40.0 (36.2, 44.2)** | 304 | **8.3 (7.4,9.2)** |

| | | | | | |
|---|---|---|---|---|---|
| Abbreviations: GMFR= geometric mean fold rise; LLOQ= lower limit of quantitation; N-binding = SARS-CoV-2 nucleoprotein-binding; NAAT=nucleic acid amplification test; NE = not estimable; NT50= 50% neutralizing titer; SA = South Africa (Beta variant); SARS-CoV-2 = severe acute respiratory syndrome coronavirus 2. Note: Subjects who had no serological or virological evidence (up to 1 month after receipt of Dose 2) of past SARS-CoV-2 infection (i.e., N-binding antibody [serum] negative at Dose 1 visit, 1-month post-Dose 2 visit, SARS-CoV-2 not detected by NAAT[nasal swab] at Dose 1 visit, Dose 2 visit, and negative NAAT[nasal swab] at any unscheduled visit up to 1 month after Dose 2) were included in the analysis. a. Subjects in the BNT162b2_{SA}/ Beta-specific vaccine (30 µg) vaccine group were not randomized. b. Protocol-specified timing for blood sample collection. c. n = Number of subjects with valid and determinate assay results for the specified assay at both prevaccinationtime point and the given dose/sampling time point. d. GMFRs and 2-sided 95% CIs were calculated by exponentiating the mean logarithm of fold rises and the corresponding CIs (based on the Student t distribution). Assay results below the LLOQwere set to 0.5 ×LLOQ in the analysis. | | | | | |

**Table 22: Geometric Mean Titers (GMT) of neutralization antibodies measured in vaccine-naïve subjects without evidence of infection up to 1 month after dose 2 and administered BNT162b2 or an RNA vaccine encoding a SARS-CoV-2 S protein having one or more mutations characteristic of a Beta variant**

| | | **Vaccine Group (as Assigned/Randomized)^{a}** | | | |
|---|---|---|---|---|---|
| | | **BNT162b2_{SA} (30 µg)** | | **BNT162b2 (30 µg)** | |
| **Assay** | **Dose/ Sampling Time Point^{b}** | **n^{c}** | **GMT^{d} (95% CI^{d})** | **n^{c}** | **GMT^{d} (95% CI^{d})** |
| | | | | | |
| SARS-CoV-2 neutralization assay - SA variant-NT50 (titer) | 1/Prevax | 272 | 34.0 (32.6,35.3) | 304 | 33.0 (33.0, 33.0) |
| | 2/1 Month | 272 | 1358.0 (1242.2,1484.5) | 304 | 273.1 (245.6, 303.7) |

| | | | | | |
|---|---|---|---|---|---|
| Abbreviations: GMT = geometric mean titer; LLOQ= lower limit of quantitation; N-binding = SARS-CoV-2 nucleoprotein-binding; NAAT= nucleic acid amplification test; NE = not estimable; NT50= 50% neutralizing titer; Prevax=prevaccination; SA = South Africa (Beta-variant); SARS-CoV-2 = severe acute respiratory syndrome coronavirus 2. Note: Subjects who had no serological or virological evidence (up to 1 month after receipt of Dose 2) of past SARS-CoV-2 infection (ie, N-binding antibody [serum] negative at Dose 1 visit, 1-month post-Dose 2 visit, SARS-CoV-2 not detected by NAAT[nasal swab] at Dose 1 visit, Dose 2 visit, and negative NAAT[nasal swab] at any unscheduled visit up to 1 month after Dose 2) were included in the analysis. a. Subjects in the BNT162b2_{SA}/ Beta-specific vaccine (30 µg) vaccine group were not randomized. b. Protocol-specified timing for blood sample collection. c. n = Number of subjects with valid and determinate assay results for the specified assay at the given dose/sampling time point. d. GMTs and 2-sided 95% CIs were calculated by exponentiating the mean logarithm of the titers and the corresponding CIs (based on the Student t distribution). Assay results below the LLOQwere set to 0.5 ×LLOQ. | | | | | |

### Example 12: Induced antibody response and reactogenecity of BNT162b2 or Omicron BA.1-specific vaccine as monovalent, bivalent and high dose in participants 55+ years of age

To test the efficacy and safety of (i) higher doses of RNA vaccines (e.g., as described herein), (ii) RNA vaccines encoding a SARS-CoV-2 S protein having one or more mutations characteristic of an Omicron BA.1 variant (an Omicron BA.1-specific vaccine), and (iii) a bivalent vaccine comprising an RNA encoding a SARS-CoV-2 S protein from a Wuhan variant and RNA encoding a SARS-CoV-2 S protein having one or more mutations characteristic of an Omicron BA.1 variant, subjects previously administered at least one dose of an RNA vaccine encoding a SARS-CoV-2 S protein of a Wuhan strain were administered one of several booster doses (e.g., as described herein). Specifically, subjects who had previously been administered two doses of 30 ug of an RNA vaccine encoding a SARS-CoV-2 S protein from a Wuhan strain (in the present example, BNT162b2), and a third dose of 30 ug of RNA encoding a SARS-CoV-2 S protein of a Wuhan strain (also BNT162b2 in the present example), were administered a fourth dose comprising:
(a) 30 ug of RNA encoding a SARS-CoV-2 S protein from a Wuhan strain,
(b) 60 ug of RNA encoding a SARS-CoV-2 S protein from a Wuhan strain,
(c) 30 ug of an Omicron BA.1-specific vaccine,
(d) 60 ug of an Omicron BA.1-specific vaccine,
e) 30 ug of a bivalent RNA vaccine (Omicron BA.1-adapted bivalent vaccine), comprising 15 ug of RNA encoding a SARS-CoV-2 S protein from a Wuhan strain and 15 ug of RNA encoding a SARS-CoV-2 S protein comprising mutations characteristic of an Omicron BA.1 variant, or
(f) 60 ug of a bivalent RNA vaccine (Omicron BA.1-adapted bivalent vaccine), comprising 30 ug of RNA encoding a SARS-CoV-2 S protein from a Wuhan strain and 30 ug of RNA encoding a SARS-CoV-2 S protein comprising mutations characteristic of an Omicron BA.1 variant.

In the present example, for the fourth dose, the RNA encoding a SARS-CoV-2 S protein from a Wuhan variant was BNT162b2, and the RNA encoding a SARS-CoV-2 S protein having mutations characteristic of an Omicron BA.1 variant comprised the nucleotide sequence of SEQ ID NO: 51.

Sera samples were collected at the time of administering the 4th dose and 7 days afterward, and tested for neutralization antibody titers against a viral particle comprising a SARS-CoV-2 S protein from a Wuhan strain, or a SARS-CoV-2 S protein comprising mutations characteristic of a Delta variant or an Omicron BA.1 variant.

Neutralization antibody titers were determined using a Fluorescent Focus Reduction Neutralization Test ("FFRNT"). Suitable FFRNT assays are known in the art, as discussed in Example 8. The neutralization responses are shown in Fig. 23.

As shown in Fig. 23 (A) subjects administered a fourth dose of 30 ug of an Omciron BA.1-specific vaccine exhibited an increase in neutralization antibodies against an Omicron BA.1 variant as compared to subjects administered a fourth dose of 30 ug of BNT162b2. Administering 60 ug of RNA increased neutralization responses both for BNT162b2 and an Omicron BA.1-specific vaccine, with 60 ug of an Omicron BA.1-specific vaccine showing a stronger immune response against an Omicron BA.1 variant. As shown in Fig. 23 (B), similar effects were observed in a population that included subjects previously or currently infected with SARS-CoV-2 (e.g., as determined by an antibody test and a PCR test, respectively).

Fig. 23 (C-D) provides data for neutalization responses against a Wuhan strain of SARS-CoV-2 in a population of subjects excluding subjects previously or currently infected with SARS-CoV-2 (Fig. 23(C)) and a population of subjects including these subjects (Fig. 23(D)).

Fig. 23 (E-F) provides data for neutralization responses against a Delta variant in a population of subjects excluding subjects previously or currently infected with SARS-CoV-2 (Fig. 23(E)) and a population of subjects including these subjects (Fig. 23(F)).

Fig. 23 (G) shows neutralization responses as compared to subjects administered a 4th dose of 30 ug of BNT162b2. As can be seen in the table, an Omicron BA.1-specific vaccine induced a strong response against an Omicron BA.1 variant, and responses that were at least comparable to that of BNT162b2 for other variants. A bivalent vaccine (Omicron BA.1-adapted bivalent vaccine) produced a strong immue response against each SARS-CoV-2 variant tested, both at 30 ug and 60 ug doses.

Reactogenicity of the tested 4^{th} doses was also monitored in patients for 7 days following administration of the 4^{th} dose. Fig. 24 (A) shows local immune responses observed in subjects of different groups as indicated. As can be seen in the figure, 60 ug doses of an Omicron BA.1 specific vaccine and a bivalent vaccine were found to be more likely to produce pain at the injection site, as compared to that observed with other tested booster doses; however, the pain was rated as mild or moderate for both doses. Redness and swelling responses were low and comparable at each dose tested.

Fig. 24 (B) shows systemic immune responses observed in subjects of different groups as indicated. Systemic responses (as characterized by fever, fatigue, headache, chills, vomiting, diarrhea, muscle pain, or joint pain) were similar for each dose, while fatigue trended higher with the 60 ug doses.

The immune responses and reactogenicity of Omicron BA.1-adapted vaccines (monovalent and bivalent vaccines as described in this Example) as a booster dose are also confirmed in a Phase 2/3 trial in over 1,000 participants 56 years of age and older. The geometric mean titers (GMTs) of each of the tested doses, as well as the geometric mean ratios (GMRs) as compared to subjects administered a 4th dose of 30 ug of BNT162b2 are shown in Table 23, below

**Table 23: Neutralization Responses in Subjects Administered 30 ug or 60 ug of a monovalent or bivalent Omicron BA.1-specific vaccine.**

| Vaccine Group | n | GMT **(**95% CI) 1 M post-dose | Vaccine group/BNT162b2 30 ug | |
|---|---|---|---|---|
| | | | GMR (95% CI) | Met Superiority (Y/N) |
| BNT162b2 30 ug | 163 | 455.8 (365.9, 567.6) | | |
| BNT162b2 OMI 30 ug | 169 | 1014.5 (825.6, 1246.7) | 2.23 (1.65, 3.00) | Y |
| | | | | |
| BNT162b2 OMI 60 ug | 174 | 1435.2 (1208.1, 1704.8) | 3.15 (2.38, 4.16) | Y |
| Bivalent OMI 30 ug | 178 | 711.0 (588.3, 829.2) | 1.56 (1.17, 2.08) | Y |
| Bivalent OMI 60 ug | 175 | 900.1 (726.3, 1115.6) | 1.97 (1.45, 2.68) | Y |

The Omicron BA.1-adapted vaccines (monovalent or bivalent; and 30 ug or 60 ug) given as a booster dose elicited substantially higher neutralizing antibody responses against Omicron BA.1 when compared to that induced by BNT162b2 (encoding a SARS-CoV-2 S protein from a Wuhan strain). The pre-specified criterion for simple superiority was measured by the ratio of neutralizing geometric mean titers (GMR) with the lower bound of the 95% confidence interval >1. The pre-specified criterion for super superiority was measured by the ratio of neutralizing geometric mean titers (GMR) with the lower bound of the 95% confidence interval >1.5. Based on these criteria, each of the tested doses was shown to be superior to 30 ug of BNT162b2, and administering 30 ug or 60 ug of BNT162b2 OMI was shown to exhibit super superiority as compared to 30 ug of BNT162b2.

Seroresponse rates are summarized in Table 24, below. As shown in the below table, each of the tested doses was found to be non-inferior to BNT162b2 30 ug.

**Table 24: Omicron BA.1 seroresponse rates**

| Vaccine Group | N | n (%) | (95% CI) 1M post-dose | Seroresponse difference in % (relative to BNT162b2 30 ug group) | |
|---|---|---|---|---|---|
| | | | | % (95% CI) | Met Non-Inferiority (Y/N)¹ |
| BNT162b2 30 ug | 149 | 85 (57.0) | (48.7, 65.1) | - | - |
| BNT162b2 OMI 30 ug | 163 | 125 (76.7) | (69.4, 82.9) | 19.6 (9.3, 29.7) | Y |
| BNT162b2 OMI 60 ug | 166 | 143 (86.1) | (79.9, 91.0) | 29.1 (19.4, 38.5) | Y |
| Bivalent OMI 30 ug | 169 | 121 (71.6) | (64.2, 78.3) | 14.6 (4.0, 24.9) | Y |
| Bivalent OMI 60 ug | 162 | 110 (67.9) | (60.1, 75.0) | 10.9 (0.1, 21.4) | Y |

| | | | | | |
|---|---|---|---|---|---|
| ¹Non-inferiority criterion: the lower bound of the 95% confidence interval is >-5% | | | | | |

Following the collection of the data summarized in Table 24, data continued to be collected on subjects administered an Omicron monovalent vaccine. The further data is summarized in Table 25, below, and confirms the findings shown in Table 24.

**Table 25: Further data on Omicron BA.1 seroresponse rates**

| | | Vaccine group (as randomized) | | | | |
|---|---|---|---|---|---|---|
| | | BNT162b2 OMI (30 ug) | | BNT162b2 (30 ug) | | Difference |
| Assay | Dose/sampling time point^{a} | N^{a} | n^{b} (%) | N^{a} | n^{b} (%) | %^{d} |
| | | | (95% Cl^{c}) | | (95% Cl^{c}) | (95% Cl^{e}) |
| SARS-CoV-2 neutralization assay - Omicron BA.1 - NT₅₀ (titre) | 1/1 month | 206 | 127 (61.7) | 226 | 91 (40.3) | 21.4 |
| | | | (54.6, 68.3) | | (33.8, 47.0) | (12.0, 30.4) |

| | | | | | | |
|---|---|---|---|---|---|---|
| Abbreviations: NT₅₀ = 50% neutralizing titer; SARS-CoV-2 = severe acute respiratory syndrome coronavirus 2. Note: Seroresponse is defined as achieving a ≥ 4-fold rise from baseline (before the first dose of study vaccination). If the baseline measurement is below the LLOQ, the postvaccination measure of ≥4 × LLOQ is considered seroresponse. Note: Participants who had no serological or virological evidence (prior to the 1-month post-first study vaccination blood sample collection) of past SARS-CoV-2 infection (i.e., N-binding antibody [serum] negative at the first study vaccination and the 1-month post-first study vaccination visits, negative NAAT [nasal swab] at the first study vaccination visit, and any unscheduled visit prior to the 1-month post-first study vaccination blood sample collection) and had no medical history of COVID-19 were included in the analysis. a. N = number of participants with valid and determinate assay results for the specified assay at both the prevaccination time point and the given sampling time point. This value is the denominator for the percentage calculations. b. n = Number of participants with seroresponse for the given assay at the given sampling time point. c. Exact 2-sided Cl based on the Clopper and Pearson method. d. Difference in proportions, expressed as a percentage (monovalent Omicron BA.1 [30 mcg] - Comirnaty [30 mcg]). e. 2-sided Cl based on the Miettinen and Nurminen method for the difference in proportions, expressed as a percentage. | | | | | | |

Changes in titers for each dose before administration of a 4th booster dose and 1 month after administration of a 4th booster dose are shown in Fig. 25. As shown in Fig. 25, one month after administration, a booster dose of the Omicron BA.1-adapted monovalent vaccine (30 µg and 60 µg) increased neutralizing geometric mean titers (GMT) against Omicron BA.1 13.5 and 19.6-fold above pre-booster dose levels, while a booster dose of the Omicron BA.1-adapted bivalent vaccine (30 µg and 60 µg) conferred a 9.1 and 10.9-fold increase in neutralizing GMTs against Omicron BA.1. A booster dose of the Omicron BA.1-adapted bivalent vaccine (30 µg) induced neutralization titers that were approximately 3-fold lower than those induced against BA.1. Both Omicron BA.1-adapted vaccines (e.g., monovalent and bivalent vaccines) were well-tolerated in participants who received one or the other Omicron BA.1-adapted vaccine, and demonstrated a favorable safety and tolerability profile similar to that of BNT162b2 (encoding a SARS-CoV-2 S protein from a Wuhan strain).

Additionally, in a SARS-CoV-2 live virus neutralization assay tested on sera from participants over 56 years of age and older receiving an Omicron-adapted vaccine (e.g., monovalent or bivalent vaccine as described in this Example), sera also neutralized Omicron BA.4/BA.5 with titers lower than Omicron BA.1.

### Example 13: Omicron breakthrough infection drives cross-variant neutralization and memory B cell formation, but to a lesser extent against Omicron BA.4 and BA.5

New Omicron sublineages that harbor further alterations in the SARS-CoV-2 S protein continue to arise, with BA.4 and BA.5 deemed VOCs by the European Centre for Disease Prevention and Control (ECDC) on the May 12, 2022 (Euopean Centre for Disease Prevention and Control, Epidemiological update: SARS-CoV-2 Omicron sub-lineages BA.4 and BA.5 (2022) (available at https://www.ecdc.europa.eu/en/news-events/epidemiological-update-sarscov-2-omicron-sub-lineages-ba4-and-ba5)).

The present Example 13 is an extension of Example 7, in which the serum samples collected from BA.1-breakthrough cases as described in Example 7 were further analyzed for their neutralization activity against Omicron BA.4 and BA.5 variants.

As described in Example 7, in Omicron-naïve double-vaccinated individuals, 50% pseudovirus neutralization (pVN₅₀) geometric mean titers (GMTs) of Beta and Delta VOCs were found to be reduced as compared to the Wuhan strain, while neutralization of Omicron sublineages BA.1 and BA.2 was virtually undetectable. In this present Example, Fig. 26(a) shows that neutralization titers of BA.4/5 was also virtually undetectable in double-vaccinated, BA.1-breakthrough patients.

As described in Example 7, Omicron-naïve triple-vaccinated individuals exhibited pVNso GMTs against all tested VOCs that were substantially higher as compared to double-vaccinated individuals. Robust neutralization of Alpha, Beta and Delta variants was observed, while neutralization of Omicron BA.1 and BA.2 was reduced as compared to Wuhan (GMT 160 and 211 vs 398). As shown in Fig. 26(A) of the present Example, neutralization of Omicron BA.4/5 was further reduced (GMT 74) in Omicron-naïve triple-vaccinating patients, corresponding to a 5-fold lower titer as compared to the Wuhan strain. As shown in Fig. 26(b), Omicron BA.1 breakthrough infection was found to have only a minor boosting effect on neutralization of BA.4/5. In double-vaccinated patients, pVN₅₀ GMTs against Omicron BA.4/5 were significantly below those against Wuhan (GMT 135 vs. 740). A similar pattern was observed with BA.1 convalescent and control sera from triple-vaccinated individuals. As noted in Example 7, BA.1 convalescent sera exhibited high pVNso GMTs against previous SARS-CoV-2 VOCs, including Beta (1182), Omicron BA.1 (1029), and Omicron BA.2 (836), which were close to titers against the Wuhan reference (1182). In contrast, as shown in Fig. 26(b), neutralization of BA.4/5 in triple-vaccinated individuals with a breakthrough infection of BA.1 was significantly reduced as compared to the Wuhan strain, with pVN₅₀ GMTs of 197, 6-fold lower than against the Wuhan strain.

Of note, in all cohorts, neutralizing titers against BA.4/5 were closer to the low level observed against the phylogenetically more distant SARS-CoV-1 pseudovirus than that seen against Wuhan. Comparing the ratios of SARS-CoV-2 VOC and SARS-CoV-1 pVN₅₀ GMTs normalized against Wuhan (Fig. 26(c)), it is remarkable that breakthrough infection with Omicron BA.1 does not lead to more efficient cross-neutralization of Omicron BA.4/5 in double-vaccinated and triple-vaccinated individuals. In aggregate, these data demonstrate that Omicron BA.1 breakthrough infection of vaccine-experienced individuals mediates broadly neutralizing activity against BA.1, BA.2 and several previous SARS-CoV-2 variants, but not for BA.4/5.

As shown in Fig. 27, similar results were found for patients previously administered a non-BNT162b2 vaccine and who had a BA.1 breakthrough infection.

As described in Example 7, Omicron BA.1 breakthrough infection in BNT162b2-vaccinated individuals was found to produce strong neutralizing activity against Omicron BA.1, BA.2 and previous SARS-CoV-2 VOCs, primarily by expanding B_{MEM} cells against epitopes shared broadly across the different SARS-CoV-2 strains. These data demonstrate that a vaccination-imprinted B_{MEM} cell pool has sufficient plasticity to be remodeled by exposure to a heterologous SARS-CoV-2 S protein. While selective amplification of B_{MEM} cells recognizing shared epitopes allows for effective neutralization of most variants that evade previously established immunity, susceptibility to escape by variants that acquire alterations at hitherto conserved sites may be heightened. The significantly reduced neutralizing activity against the Omicron BA.4/5 pseudovirus, which harbors the additional alterations L452R and F486V in the RBD, supports a mechanism of immune evasion by loss of the few remaining conserved epitopes.

### Discussion

Surprisingly, and contrary to the results observed in Example 7, neutralization of Omicron sublineages BA.4 and BA.5 was not enhanced in BA.1-breakthrough patients, with titers instead comparable to those against the phylogenetically more distant SARS-CoV-1. While the present Example focused on individuals vaccinated with the BNT162b2 mRNA vaccine, in individuals vaccinated with CoronaVac (a whole, inactivated virus vaccine developed by Sinovac Biotech), similar observations have recently been reported, suggesting that Omicron BA.4/5 can bypass BA.1 infection-mediated boosting of humoral immunity (Y. Cao et al., BA.2.12.1, BA.4 and BA.5 escape antibodies elicited by Omicron infection, bioRxiv: the preprint server for biology (2022)).

The present disclosure provides insights into how immunity against multiple variants is achieved in BA.1 breakthrough cases, why Omicron BA.4 and BA.5 sublineages can partially escape neutralization, and provides vaccination protocols and technologies to enhance protection across coronavirus strains and lineages, specifically including across Omicron lineages (e.g., including BA.4 and/or BA.5). Without wishing to be bound by any particular theory, the present disclosure proposes that initial exposure to the Wuhan strain S protein may shape formation of B_{MEM} cells and imprint against novel B_{MEM} cell responses recognizing epitopes distinctive for the Omicron BA.1 variant.

Omicron BA.1 breakthrough infection in BNT162b2-vaccinated individuals primarily expands a broad B_{MEM} cell repertoire against conserved SARS-CoV-2 S protein and RBD epitopes, rather than inducing strictly Omicron BA.1-specific B_{MEM} cells. As compared to the immune response induced by a homologous vaccine booster, an Omicron BA.1 breakthrough infection leads to a more substantial increase in antibody neutralization titers against Omicron and a robust cross-neutralization of many SARS CoV-2 variants.

As noted in Example 7, one potential explanation for the broad neutralization elicited by a BA.1 breakthrough infection is the induction of broadly neutralizing antibodies. Sera from Omicron BA.1-convalescent vaccinated individuals was found to neutralize SARS-CoV-2 Omicron BA.4/5 and SARS-CoV-1 to a far lesser extent than previous SARS-CoV-2 VOCs including BA.1 and BA.2. This finding indicates that Omicron BA.1 infection in vaccinated individuals stimulates B_{MEM} cells that produce neutralizing antibodies against S protein epitopes conserved in the SARS-CoV-2 variants up to and including Omicron BA.2, but that have mostly been lost in BA.4/5 and are for the most part not shared by SARS-CoV-1.

The greater antigenic distance of the Omicron BA.1 S protein from earlier SARS-CoV-2 strains may promote targeting of conserved subdominant neutralizing epitopes as recently described to be located, e.g., in cryptic sites within a portion of the RBD distinct from the receptor-binding motif (Li, Tingting, et al. "Cross-neutralizing antibodies bind a SARS-CoV-2 cryptic site and resist circulating variants," Nature communications 12.1 (2021): 1-12, and Yuan, Meng, et al. "A highly conserved cryptic epitope in the receptor binding domains of SARS-CoV-2 and SARS-CoV" Science 368.6491 (2020): 630-633) or in the membrane proximal S glycoprotein subunit designated S2 (Pinto, Dora, et al. "Broad betacoronavirus neutralization by a stem helix-specific human antibody." Science 373.6559 (2021): 1109-1116. Li, Wenwei, et al. "Structural basis and mode of action for two broadly neutralizing antibodies against SARS-CoV-2 emerging variants of concern." Cell reports 38.2 (2022): 110210; Hurlburt, Nicholas K., et al. "Structural definition of a pan-sarbecovirus neutralizing epitope on the spike S2 subunit." Communications biology 5.1 (2022): 1-13).

As noted in Example 7, Omicron BA.1-infected individuals appear to have a significantly higher RBD/S protein-specific B_{MEM} cell ratio as compared to vaccinated Omicron-naïve individuals. Omicron BA.1 carries multiple S protein alterations in key neutralizing antibody binding sites of the NTD (such as del69/70 and del143-145) that dramatically reduce the targeting surface for memory B cell responses in this region. Although the Omicron BA.1 RBD harbors multiple alterations, some neutralizing antibody binding sites are unaffected (20). An expansion of B_{MEM} cells that produce neutralizing antibodies against RBD epitopes that are not altered in Omicron BA.1, such as those at position L452 as indicated in the present Example, could help to rapidly restore neutralization of the BA.1 and BA.2 variants. Importantly, the strong neutralization of Omicron BA.1 and BA.2 should not mask the fact that the neutralizing B_{MEM} immune response in Omicron BA.1 convalescent vaccinated individuals is driven by a smaller number of epitopes. The significantly reduced neutralizing activity against the Omicron BA.4/5 pseudovirus, which harbors the additional alterations L452R and F486V in the RBD, demonstrates the mechanism of immune evasion by loss of the few remaining conserved epitopes. Meanwhile, further sublineages with L452 alterations (e.g., BA.2.12.1) are being reported to evade humoral immunity elicited by BA.1 breakthrough infection (Y. Cao et al., cited above).

The present disclosure proposes that immunity in the early stages of Omicron BA.1 infection in vaccinated individuals may be based on recognition of conserved epitopes, and narrowly focused on a small number of neutralizing sites that are not altered in Omicron BA.1 and BA.2. Such a narrow immune response bears a high risk that those few epitopes may be lost by acquisition of further alterations in the course of the on-going evolution of Omicron and may result in immune escape, as experienced with sublineages BA.2.12.1, BA.4 and BA.5 (Y. Cao et al., cited above, and K. Khan et al., Omicron sub-lineages BA.4/BA.5 escape BA.1 infection elicited neutralizing immunity (2022)). Importantly, Omicron BA.1 breakthrough infection does not appear to reduce the overall spectrum of (Wuhan) S glycoprotein-specific memory B cells, as memory B cells that do not recognize Omicron BA.1 S remain detectable in blood at similar frequencies. Wuhan-specific (non-Omicron BA.1 reactive) B_{MEM} cells were consistently detected in Omicron BA.1 breakthrough infected individuals at levels similar to those in Omicron-naïve double-/triple-vaccinated individuals. Without wishing to be bound by any particular theory, the present disclosure notes that these findings may reflect an increase of the total B_{MEM} cell repertoire by selective amplification of B_{MEM} cells that recognize shared epitopes.

The present Example, among other things, provides insights that it may be more beneficial for a subject who has been infected or administered at least one dose (including, e.g., at least two , at least three doses) of vaccine(s) adapted to a Wuhan strain (e.g., but not limited to a protein based vaccine or RNA-based vaccines such as BNT162b2, Moderna mRNA-1273) to receive at least one dose of a vaccine (e.g., a protein or RNA-based vaccine) adapted to a strain that is not an Omicron BA.1. In some embodiments, a vaccine that is adapted to a strain that is not an Omicron BA.1 can be or comprise a vaccine that is adapted to Omicron BA.4 and/or Omicron BA.5. The present Example, among other things, also provides insights that vaccine-naïve subjects without prior SARS-CoV-2 infection may be desirable to be administered a combination of vaccines, which comprises at least one dose of a vaccine adapted to a Wuhan strain (e.g., RNA vaccine such as in some embodiment BNT162b2) and at least one dose of a vaccine adapted to a strain that is not an Omicron BA.1. In some embodiments, such vaccines in a combination may be administered at different times, for example, in some embodiments as primary doses and/or booster doses administered apart by a pre-determined period of time (e.g., according to certain dosing regimens as described herein). In some embodiments, such vaccines in a combination may be administered as a single multivalent vaccine.

### MATERIALS AND METHODS

Serum samples, neutralization assays, and all other experiments described in the present Example were performed as in Example 7. The BA.4/5 VSV-SARS-CoV-2 S variant pseudovirus generation A recombinant replication-deficient vesicular stomatitis virus (VSV) vector that encodes green fluorescent protein (GFP) and luciferase instead of the VSV-glycoprotein (VSV-G) was pseudotyped comprised a SARS-CoV-2 S protein comprising the following mutations relative to the Wuhan strain: T19I, Δ 24-26, A27S, Δ69/70, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, N969K).

### Example 14. Omicron BA.2 breakthrough infection of vaccinated individuals induces broad cross neutralization against Omicron BA.1, BA.2 and other VOCs, including BA.4 and BA.5.

The present Example shows that a BA.2 Omicron breakthrough infection in individuals triple-vaccinated with BNT162b2 surprisingly drives superior cross variant neutralization as compared to a BA.1-breakthrough infection in individuals triple-vaccinated with BN162b2, including improved production of neutralizing antibodies against a BA.4/5 Omicron variant. Thus, among other things, the present disclosure demonstrates feasibility of defining immunologically synergistic categories of coronavirus strains and/or sequences (e.g., spike protein sequences).

In some embodiments of improved coronavirus vaccination strategies provided by the present disclosure, a subject is exposed to each of at least two different such synergistic categories. In some embodiments, a subject is, has been or becomes infected with a virus of a first category, and receives at least one dose of a vaccine of a second category, characterized by immunologic synergy with the first category. Alternatively or additionally, in some embodiments, a subject receives or has received doses of first and second vaccines of such first and second categories. In some embodiments, vaccines of different categories may be separately administered (e.g., at different points in time and/or to different sites on a subject). In some embodiments, vaccines of different categories may be administered together (e.g., at substantially the same time and/or to approximately or exactly the same site and/or in a single composition).

As compared to Omicron BA.1 breakthrough infection of vaccinated individuals, which induces lower neutralization against Omicron BA.4/BA.5 relative to neutralization against other SARS-CoV-2 variants (including, e.g., Wuhan-Hu-1 strain, alpha variant, beta variant, delta variant, Omicron BA.1, Omicron BA.2, and Omicron BA.2.11.2), the present Example shows that a BA.2 Omicron breakthrough infection in individuals vaccinated with BNT162b2 surprisingly drives superior cross variant neutralization, including improved production of neutralizing antibodies against a BA.4/5 Omicron variant. Thus, in some embodiments, the present disclosure, among other things, demonstrates that SARS-CoV-2 strains and/or variants can be grouped into at least two different categories such that a subject who is exposed to a SARS-CoV-2 strain and/or variant from each of such two different categories can benefit from immunologically synergistic protection conferred by such two different categories. In some embodiments, a first category of SARS-CoV-2 strains/variants comprises: Wuhan-Hu-1 strain, alpha variant, beta variant, delta variant, Omicron BA.1, and subvariants derived from aforementioned strains and/or variants; while a second category comprises Omicron BA.2, Omicron BA.2.12.1, Omicron BA.4/BA.5, and subvariants derived from aforementioned strains and/or variants. Thus, in some embodiments, the present disclosure, among other things, provide insights that a combination of at least one dose (including, e.g., at least 1, at least 2, at least 3, at least 4, or more) of a first vaccine (e.g., an mRNA vaccine as described herein that encodes a spike protein polypeptide) that comprises or delivers a SARS-CoV-2 spike protein polypeptide with a sequence characteristic of a first category as described above, and at least one dose (including, e.g., at least 1, at least 2, at least 3, at least 4, or more) of a second vaccine (e.g., an mRNA vaccine described herein that encodes a spike protein polypeptide) that comprises or delivers a SARS-CoV-2 spike protein polypeptide with a sequence characteristic of a second category as described above can synergistically provide superior cross variant neutralization, including enhanced production of neutralizing antibodies toward a BA.4/5 Omicron variant. In some embodiments, the present disclosure specifically teaches surprising efficacy of administering at least one dose of a vaccine (e.g., an mRNA vaccine that encodes a spike protein polypeptide as described herein) that comprises or delivers a SARS-CoV-2 spike protein polypeptide with sequences characteristic of a BA.2 Omicron variant to subjects who have received at least one (e.g., 2, 3, or more) doses of a vaccine (e.g., vaccine that encodes a spike protein polypeptide as described herein) that comprises or delivers a SARS-CoV-2 spike protein polypeptide with sequences characteristic of a Wuhan-Hu-1 strain).

### Background

Emergence of the SARS-CoV-2 Omicron variant of concern (VOC) in November 2021 (Ref. 1) can be considered a turning point in the COVID-19 pandemic, owing to its ability to substantially escape previously established immunity. Omicron BA.1, which displaced Delta within weeks as the predominant circulating VOC, had acquired significant alterations in the receptor binding domain (RBD) and N-terminal domain (NTD) (Ref. 2). These changes resulted in a loss of many epitopes recognized by neutralizing antibodies (Refs. 3-4) and drastically impaired humoral immunity induced by vaccines based on the ancestral Wuhan strain or exposure to the ancestral strain or previous variants (Refs. 5-7). BA.1 was subsequently displaced by the BA.2 variant, which in turn gave rise to further sub-lineages. BA.4 and BA.5, which are derived from BA.2, are currently becoming the dominant variants in many countries across the globe with multiple studies suggesting a significant change in antigenic properties compared to BA.2, and especially compared to BA.1 (Refs. 8-9). As BA.4 and BA.5 share an identical S glycoprotein sequence, they are referred herein as BA.4/5. While many of the amino acid changes in the RBD are shared between Omicron sub-lineages, alterations within the NTD of BA.2-derived sub-lineages including BA.4/5 are mostly distinct from those found in BA.1 (Figure 33).

A vast majority worldwide have been immunized with the vaccines adapted Wuhan strain, including, e.g., mRNA vaccines such as BNT162b2 and mRNA-1273 (Ref. 10), which have thus substantially shaped SARS-CoV-2 population immunity. However, emergence of the immune escape variant Omicron BA.1 led to a steep increase in the occurrence of breakthrough infections in vaccinated individuals. It has been reported that SARS-CoV-2 variant breakthrough infection can reshape humoral immunity, thereby modulating neutralizing antibody titers against other variants (Refs. 8, 11, 12). However, as previously reported, BA.1 breakthrough infection may not provide strong immunity against Omicron BA.4/5.

### Certain findings

In order to determine if BA.2 breakthrough infection would refocus immunity against Omicron BA.2 and BA.2-derived sub-lineages such as BA.4/5, the magnitude and breadth of the neutralizing antibody response was studied in samples from individuals who had received a triple vaccination scheme with mRNA vaccines (BNT162b2/mRNA-1273) and subsequently experienced SARS-CoV-2 breakthrough infections between March and May 2022, during which period the BA.2 lineage was dominant in Germany (All Vax + Omi BA.2). Such findings have important implications for ongoing efforts of vaccine design, as containment of the COVID-19 pandemic requires the generation of durable and sufficiently broad immunity to provide protection against current and future variants of SARS-CoV-2.

Two reference cohorts were generated from data previously published in Quandt et al. (Ref. 12), comprising (i) individuals triple-vaccinated with BNT162b2 without a prior or breakthrough SARS-CoV-2 infection at the time of sample collection (BNT162b2³) and (ii) individuals who were triple-vaccinated with mRNA vaccines with subsequent breakthrough-infection during a period of Omicron BA.1 dominance (All Vax + Omi BA.1).

Breakthrough infection with the SARS-CoV-2 Omicron BA.1 and BA.2 occurred at a median of approximately 4 months or 3 weeks, respectively, after triple-vaccination with an mRNA-based COVID-19 vaccine (BNT162b2, mRNA-1273, or heterologous regimens comprising both vaccines; all Vax + Omi BA.1, all Vax + Omi BA.2) (Fig. 29). Immune sera used to characterize serum neutralizing activity were collected at a median 28 days post-vaccination for the BNT162b2³ cohort, 43 days post-BA.1 breakthrough for the All Vax + Omi BA.1 cohort, and 39 days post BA.2 breakthrough infection for the All Vax + Omi BA.2 cohort. Median ages of the cohorts were similar (32-38 years). The BA.2.12.1 neutralization data was generated from serum samples from cohorts BNT162b³ and All Vax+Om BA. 1 for this study.

To evaluate the neutralizing activity of immune sera, a pseudovirus neutralization test (pVNT), for example, as described in Refs. 13, 14, were used. Pseudoviruses bearing the S glycoproteins of SARS-CoV-2 Wuhan, Alpha, Beta, Delta, Omicron BA.1, BA.2, BA.2.12.1, as well as the recently emerged Omicron sub-lineages BA.4 and BA.5 were applied to assess neutralization breadth. As BA.4 and BA.5 share an identical S glycoprotein sequence, including key alterations L452R and F486V, they are referred herein as BA.4/5. In addition, SARS-CoV (herein referred to as SARS-CoV-1; Ref. 15) was assayed to detect potential pan-Sarbecovirus neutralizing activity.

As reported previously in Ref. 12, 50% pseudovirus neutralization (pVN₅₀) geometric mean titers (GMTs) against Omicron BA.1 and BA.2 of immune sera from SARS-CoV-2 naïve triple-vaccinated individuals were considerably reduced compared to the Wuhan strain (GMT 160 and 221 versus 398). Neutralizing activity against BA.2.12.1 and BA.4/5 was even further reduced (GMTs 111 and 74), corresponding to a 5.4-fold lower titer for BA.4/5 as compared to the Wuhan strain (Fig. 30(A)).

Omicron BA.2 breakthrough infection markedly increased pVN₅₀ GMTs against BA.2 and BA.2.12.1 compared to SARS-CoV-2-naïve triple-vaccinated immune sera, such that neutralization of BA.2 after breakthrough infection was comparable to the Wuhan strain (Fig. 31 (B-C)). Similarly, BA.1 breakthrough infection conferred robust neutralizing activity against BA.1 (Fig. 30(B), Fig. 31(A). Importantly, while pVN₅₀ GMTs against BA.4/5 in BA.2 convalescent sera were lower than against the Wuhan strain (GMTs 391 versus 922, i.e., 2.4-fold reduction), this reduction was still less than that observed in the Omicron-naïve BNT162b2³ cohort, whose sera showed a 5.4-fold reduction of BA.4/5 neutralizing activity (Fig. 31(C)). By contrast, pVN₅₀ GMTs against BA.4/5 and Wuhan after BA.1 breakthrough infection were 266 and 1327, respectively (i.e., 5-fold reduction; Fig. 30(B)). Hence, Omicron BA.1 breakthrough infection of triple-vaccinated individuals did not lead to more efficient cross-neutralization of Omicron BA.4/5 as compared with triple-vaccinated Omicron-naïve individuals. In both cohorts, neutralizing titers against BA.4/5 were closer to the low level observed against the phylogenetically more distant SARS-CoV-1 than that seen against Wuhan (Fig. 30). Of note, the pVN₅₀ GMTs against the Wuhan strain after BA.1 breakthrough infection were slightly higher than those observed for BA.2 breakthrough infection (GMTs 1327 versus 922), which, without wishing to be bound by a particular theory, may relate to the longer interval between the third vaccination and the infection (median 22 days for BA.1 versus 127.5 days for BA.2) (Fig. 31). A separate analysis was conducted including only individuals triple-vaccinated with BNT162b2 (with BA.2 or BA.1 breakthrough infections, or Omicron-naïve). In these analyses similar observations regarding BA.4/5 neutralizing activities were made: pVN₅₀ GMTs against BA.4/5 in BA.2 convalescent sera were 2.4-fold lower than against the Wuhan strain, whereas the reduction was 6-fold after BA.1 breakthrough infections (Fig. 31). While relative neutralization of BA.2 and BA.2.12.1 was comparable in BA.2 and BA.1 convalescent sera, neutralizing activity against these variants remained slightly above that seen in Omicron-naïve sera.

Immune sera from triple-vaccinated Omicron naïve individuals had broad neutralizing activity against ancestral SARS-CoV-2 VOCs. Neutralizing activity against Beta was slightly higher in BA.1 convalescent sera, whereas neutralization of Alpha and Delta was not affected by BA.1 or BA.2 breakthrough infections (Fig. 31(C)).

In aggregate, these data demonstrate that Omicron BA.2 breakthrough infections of vaccine-experienced individuals mediate broadly neutralizing activity against BA.1, BA.2, BA.2.12.1 and several ancestral SARS-CoV-2 variants. Moreover, neutralizing activity against BA.4/5, while lower than against the Wuhan reference, is provided to a larger extent than in BA.1 convalescent sera.

Recent studies have demonstrated that Omicron BA.1 breakthrough infection in individuals vaccinated with an mRNA vaccine (BNT162b2 or mRNA-1273) boosts serum neutralizing titers not only against the ancestral Wuhan strain, but also against VOCs including BA.2 (Refs. 8, 11, 12). This effect was seen in triple-vaccinated individuals but was particularly evident in double-vaccinated individuals, whose sera contain little to no neutralizing activity against BA.2. However, BA.1 breakthrough infection did not induce strong neutralizing activity against BA.4/5, VOCs that are currently establishing dominance worldwide. Without wishing to be bound by a particular theory, this immune escape has been attributed to the amplification and/or recall of pre-existing neutralizing antibody responses that recognize epitopes absent in the Omicron sub-lineages BA.2.12.1, BA.4, and BA.5.

The present Example, among other things, provide insights that BA.2 breakthrough infections trigger recall responses which mediate enhanced neutralization of the BA.2-derived sub-lineages, including BA.4/5, indicating that higher S protein sequence similarity among BA.2, BA.2.12.1, and BA.4/5 drives more efficient cross-neutralization compared to breakthrough infections with the more distant BA.1 variant. Notwithstanding the importance of vaccination with currently approved Wuhan-derived vaccines such as BNT162b2 that offer effective protection from severe disease by current VOCs including Omicron BA.1 and BA.2, the present findings of broadly cross-neutralizing activity against current VOCs including BA.4/5 after BA.2 breakthrough infection provides insights, among other things, that a combination of a vaccine adapted to Wuhan strain sequence or a variant sequence from the same immunologically-related category as discussed above (e.g., alpha strain, beta strain, delta strain, Omicron BA.1) and a vaccine adapted to the BA.2 variant sequence or a variant sequence from the same immunologically-related category as discussed above (e.g., Omicron BA.2.12.1, Omicron BA.4/BA.5) can provide enhanced cross-neutralization activity against variants from two different categories. In some embodiments, the present Example provides evidence that supports implementation of licensure procedures modelled on that of seasonal flu vaccines that use the latest epidemiological data to select for COVID-19 vaccine strains. In some embodiments, the present Example further provides evidence that supports establishment of rapid strain selection for seasonal updates of COVID-19 vaccines, similar to the selection process practiced by the World Health Organization (WHO) Global Influenza Surveillance and Response System (GISRS), and/or agreement on accelerated approval pathways based on surrogate immunogenicity endpoints.

Neutralization titers from subjects vaccinated against SARS-CoV-2 and who have had a BA.1 or a BA.2 breakthrough infection are shown in Fig. 31(A) and (B), respectively, and GMRs for both groups of subjects are shown in Fig. 31(C). As shown in Figs. 31(A) and (B), sera from subjects previously vaccinated against SARS-CoV-2, and who had a breakthrough infection with either BA.1 or BA.2, were found to have significant neutralization titers against pseudovirus comprising a SARS-CoV-2 S protein of a Wuhan strain, an Alpha variant, a Beta variant, a Delta Variant, and an Omicron BA.1 variant. As noted previously, neutralization titers against BA.2 are somewhat lower in sera from BA.1 breakthrough patients (GMT of 875 for BA.2 vs 1327 for Wuhan strain) and are lower still against BA.2.12.1 and BA.4/5 (GMTs of 584 and 266, respectively as compared to 1327 for Wuhan). BA.2 breakthrough patients show similar neutralization responses as BA.1 breakthrough patients against a SARS-CoV-2 Wuhan strain, Alpha variant, Beta variant, and Delta variant. The neutralization response against Omicron BA.1 is somewhat higher in BA.1-breakthrough patients than in BA.2-breakthrough patients (GMR of 0.76 as compared to 0.60), while neutralization titers against Omicron BA.2 are higher in BA.2-breakthrough patients than in BA.1-breakthrough patients (GMR of 0.94 vs 0.66). Surprisingly, however, neutralization responses against BA.4/5 are significantly higher in BA.2-breakthrough patients (GMR of 0.39 in BA.2 breakthrough patients, as compared to a GMR of 0.2 in BA.1 breakthrough subjects). The present disclosure therefore documents that a broader immune response can be elicited by a BA.2-breakthrough infection as compared to a BA.1 breakthrough infection in subject vaccinated against SARS-CoV-2, and teaches that administering a booster vaccine comprising RNA encoding an S protein comprising mutations characteristic of a BA.2 Omicron variant can achieve surprising and unexpected benefits.

Furthermore, the present disclosure provides an insight that, given similarities among S protein sequences of BA.2 and BA.4/5 variants, combining vaccination doses that comprise or deliver BA.4 and/or BA.5 variant spike sequences with those of that comprise or deliver Wuhan spike sequences may also achieve particularly broad immunization (i.e., synergistic immunization as described herein).

In some embodiments, these findings suggest that synergistic categories of coronavirus strain and/or variant sequences (e.g., SARS-CoV-2 strain and/or variant sequences) can be defined, for example, in some embodiments based on shared amino acid alterations in S glycoprotein of coronavirus strain and/or variant sequences. For example, while many of the amino acid changes in the RBD of S protein are shared between Omicron sub-lineages (e.g., BA.1, BA.2, BA.2.12.1, and BA.4/5), alterations within the NTD of BA.2 and BA.2-derived sub-lineages including BA.4/5 are mostly distinct from those found in BA.1. Therefore, in some embodiments, synergistic categories of coronavirus strain and/or variant sequences (e.g., SARS-CoV-2 strain and/or variant sequences) can be defined based on the degree of shared amino acid mutations present with the NTD of a S protein. For example, in some embodiments where two SARS-CoV-2 strain and/or variant sequences share at least 50%

(including, e.g., at least 60%, at least 70%, at least 80%, at least 90%, or more) of the amino acid mutations present in the NTD of a S protein, both SARS-CoV-2 strain and variant sequences can be grouped into the same category. In some embodiments where two SARS-CoV-2 strain and/or variant sequences share no more 50% (including, e.g., no more than 45%, no more than 40%, no more than 30%, or lower) of the amino acid mutations present in the NTD of a S protein, both SARS-CoV-2 strain and variant sequences can be grouped into different categories. Among other things, the present findings provide insights that exposing subjects (e.g., via infection and/or vaccination) to at least two antigens that are of different synergistic categories (e.g., as shown in the table below) can produce a more robust immune response (e.g., broadening the spectrum of cross-neutralization against different variants and/or producing an immune response that is less prone to immune escape).

| **Category I** | **Category II** |
|---|---|
| Wuhan strain | Omicron BA.2 |
| Alpha variant | Omicron BA.2.11.2 |
| Beta variant | Omicron BA.4 |
| Delta variant | Omicron BA.5 |
| Omicron BA.1 | |
| | Sublineages derived from any one of the above |
| Sublineages derived from any one of the above | |

For example, in some embodiments, vaccine-naïve subjects without prior infection may be administered a combination of vaccines, at least two of which are each adapted to a SARS-CoV-2 strain of different synergistic categories (e.g., as described herein). In some embodiments, such vaccines in a combination may be administered at different times, for example, in some embodiments as a first dose and a second dose administered apart by a pre-determined period of time (e.g., according to certain dosing regimens as described herein). In some embodiments, such vaccines in a combination may be administered as a multivalent vaccine. In some embodiments, subject infected or vaccinated with a SARS-CoV-2 strain of one category may be administered with a vaccine adapted to a SARS-CoV-2 strain of a different category (e.g., as described herein). In some embodiments, such a vaccine may be a polypeptide-based or RNA-based vaccine.

In some embodiments, a combination of vaccines comprising a vaccine from Category I and a BA.4/5 vaccine may provide a particularly superior immune response (e.g., an immune response with particularly strong cross-neutralization effects).

Due to the large number of differences between XBB and its variants, in some embodiments, a particularly improved synergistic effect can be produced by a combination comprising a vaccine of Category I and an XBB vaccine. In some embodiments, a particularly improved synergistic effect can be produced by a combination comprising a BA.4/5 vaccine and an XBB vaccine.

While the present findings are based on retrospective analyses of samples derived from different studies, using relatively small samples sizes and cohorts that are not fully aligned regarding immunization intervals and demographic characteristics such as age and sex of individuals, the present findings provide useful insights for vaccine design and vaccination strategies for improving cross-neutralization against a broader spectrum of SARS-COV-2 variants.

In some embodiments, a vaccine can comprise a polypeptide (e.g., a non-natural polypeptide, e.g., a chimeric polypeptide) comprising one or more mutations that are characteristic of one or more different SARS-CoV-2 variants, or a nucleic acid (e.g., in some embodiments an RNA) encoding the polypeptide. In some embodiments, a vaccine can comprise a polypeptide (e.g., a non-natural polypeptide, e.g., a chimeric polypeptide) comprising one or more mutations that are characteristic of a first SARS-CoV-2 variant and one or more mutations that are characteristic of a second SARS-CoV-2 variant, or a nucleic acid (e.g., in some embodiments an RNA) encoding the polypeptide. In some embodiments, a first SARS-CoV-2 variant can be a SARS-CoV-2 strain/variant from Category I of the table above, while a second SARS-CoV-2 variant can be a SARS-CoV-2 strain/variant from Category II of the table above. For example, in some embodiments, a vaccine can comprise a polypeptide that comprises an RBD comprising one or more mutations that are characteristic of a first SARS-CoV-2 variant and an NTD comprising one or more mutations that are characteristic of a second SARS-CoV-2 variant, or a nucleic acid (e.g., in some embodiments an RNA) encoding the polypeptide.

In some embodiments, a vaccine can comprise a polypeptide that comprises an RBD comprising one or more mutations characteristic of a BA.1 Omicron variant and an NTD comprising one or more mutations characteristic of a second SARS-CoV-2 variant that is not a BA.1 Omicron variant, or a nucleic acid (e.g., in some embodiments an RNA) encoding the polypeptide. In some embodiments, a vaccine can comprise or encode a polypeptide comprising an NTD comprising one or more mutations characteristic of a BA.1 Omicron variant and an RBD comprising one or more mutations characteristic of a second SARS-CoV-2 variant that is not a BA.1 Omicron variant. In some embodiments, a vaccine can comprise a polypeptide that comprises an RBD comprising one or more mutations characteristic of a BA.1 Omicron variant and an NTD comprising one or more mutations characteristic of a BA.2 Omicron variant, or a nucleic acid (e.g., in some embodiments an RNA) encoding the polypeptide. In some embodiments, a vaccine can comprise a polypeptide that comprises an RBD comprising one or more mutations characteristic of a BA.1 Omicron variant and an NTD comprising one or more mutations characteristic of a BA.4/5 Omicron variant, or a nucleic acid (e.g., in some embodiments an RNA) encoding the polypeptide.

In some embodiments, a vaccine can comprise a polypeptide that comprises an NTD comprising one or more mutations characteristic of a BA.1 Omicron variant and an RBD comprising one or more mutations characteristic of a second SARS-CoV-2 variant that is not a BA.1 Omicron variant, or a nucleic acid (e.g., in some embodiments an RNA) encoding the polypeptide. In some embodiments, a vaccine can comprise a polypeptide that comprises an RBD comprising one or more mutations characteristic of a BA.1 Omicron variant and an NTD comprising one or more mutations characteristic of a second SARS-CoV-2 variant that is not a BA.1 Omicron variant, or a nucleic acid (e.g., in some embodiments an RNA) encoding the polypeptide. In some embodiments, a vaccine can comprise a polypeptide that comprises an NTD comprising one or more mutations characteristic of a BA.1 Omicron variant and an RBD comprising one or more mutations characteristic of a BA.2 Omicron variant, or a nucleic acid (e.g., in some embodiments an RNA) encoding the polypeptide. In some embodiments, a vaccine can comprise a polypeptide that comprises one or more mutations characteristic of an NTD of a BA.1 Omicron variant and an RBD comprising one or more mutations characteristic of a BA.4/5 Omicron variant, or a nucleic acid (e.g., in some embodiments an RNA) encoding the polypeptide.

In some embodiments, a vaccine can comprise a polypeptide that comprises an RBD comprising one or more mutations characteristic of a BA.1 Omicron variant and an NTD of a Wuhan S protein, or a nucleic acid (e.g., in some embodiments an RNA) encoding the polypeptide. In some embodiments, a vaccine can comprise a polypeptide that comprises an RBD comprising one or more mutations characteristic of a BA.2 Omicron variant and an NTD of a Wuhan S protein, or a nucleic acid (e.g., in some embodiments an RNA) encoding the polypeptide. In some embodiments, a vaccine can comprise a polypeptide that comprises an RBD comprising one or more mutations characteristic of a BA.4/5 Omicron variant and an NTD of a Wuhan S protein, or a nucleic acid (e.g., in some embodiments an RNA) encoding the polypeptide.

In some embodiments, a vaccine can comprise a polypeptide that comprises an NTD comprising one or more mutations characteristic of a BA.1 Omicron variant and an RBD of a Wuhan S protein, or a nucleic acid (e.g., in some embodiments an RNA) encoding the polypeptide. In some embodiments, a vaccine can comprise a polypeptide that comprises an NTD comprising one or more mutations characteristic of a BA.2 Omicron variant and an RBD of a Wuhan S protein, or a nucleic acid (e.g., in some embodiments an RNA) encoding the polypeptide. In some embodiments, a vaccine can comprise a polypeptide that comprises an NTD comprising one or more mutations characteristic of a BA.4/5 Omicron variant and an RBD of a Wuhan S protein, or a nucleic acid (e.g., in some embodiments an RNA) encoding the polypeptide.

### Materials and Methods

### Recruitment of participants and sample collection

Individuals from the SARS-CoV-2-naïve BNT162b2 triple-vaccinated (BNT162b2³) cohort provided informed consent as part of their participation in the Phase 2 trial BNT162-17 (NCT05004181). Individuals with Omicron BA.1 or BA.2 breakthrough infection (All Vax + Omi BA.1 and All Vax + Omi BA.2 cohorts) were triple-vaccinated, e.g., with one or more doses of BNT162b2, Moderna mRNA-1273, AstraZeneca ChAdOx1-S recombinant vaccine, or a combination thereof, and were recruited to provide blood samples and clinical data for research. Omicron infections were confirmed with variant-specific PCR either between November 2021 and mid-January 2022 (All Vax + Omi BA.1) or between March 2022 and May 2022, at times were sub-lineages BA.1 or BA.2, respectively, were dominant (Ref. 24). The infections of certain participants (e.g., at least 7 participants) in this study were further characterized by genome sequencing, and genome sequencing confirmed Omicron BA.1 or BA.2 infection.

Participants were free of symptoms at the time of blood collection. Table 26 is a summary of characteristics of vaccinated individuals analyzed for neutralizing antibody responses. All participants had no documented history of SARS-CoV-2 infection prior to vaccination.

**Table 26**

| **Characteristic** | | **all Vax** + **Omi BA.2 (n=14)** | **all Vax** + **Omi BA.1 (n=15)** | **BNT162b2³ (n=19)** |
|---|---|---|---|---|
| Sex, n (%) | | | | |
| | Male | 6 (43) | 12 (80) | 10 (53) |
| | Female | 8 (57) | 3 (20) | 9 (47) |
| Age, median (range) | | 34 (25-57) | 32 (23-60) | 38 (23-54) |
| Age group at vaccination, n (%) | | | | |
| | 18-55 yrs | 12 (86) | 13(87) | 19 (100) |
| | 56-85 yrs | 2(14) | 2 (13) | 0(0) |
| Baseline SARS-CoV-2 status, n (%) | | | | |
| | Positive | 14 (100)t | 15 (100)t | 0(0) |
| | Negative | 0(0) | 0(0) | 19 (100)^{#} |
| | Unknown | 0(0) | 0(0) | 0(0) |
| Interval, median (range) | | | | |
| | Days between D1/D2 | 41 (15-88) | 39 (20-92) | ‡ |
| | Days between D2/D3 | 189 (152-249) | 189 (156-256) | 202 (181-266) |
| | Days until serum draw after D3 | N/A | N/A | 28 (26-30) |
| | Days between last dose/infection | 127.5 (36-188) | 22 (3-112) | N/A |
| | Days until serum draw after infection | 43 (34-99) | 43 (25-55) | N/A |

| | | | | |
|---|---|---|---|---|
| N/A: not applicable; n/a, not available; D, Dose; Yrs, Years; n, Number. *, Negative SARS-CoV-2 PCR test at the time of enrollment ^{#}, No evidence of prior SARS-CoV-2 infection (based on COVID-19 symptoms/signs and SARS-CoV-2 PCR test) ^{‡}, Participants received the primary 2-dose series of BNT162b2 vaccine as part of a governmental vaccination program and the interval between doses was not recorded ^{†}, Omicron BA.1 infection confirmed at time of recruitment to the research study. | | | | |

Serum was isolated by centrifugation of drawn blood at 2000 × g for 10 minutes and cryopreserved until use.

### VSV-SARS-CoV-2 S variant pseudovirus generation

A recombinant replication-deficient vesicular stomatitis virus (VSV) vector that encodes green fluorescent protein (GFP) and luciferase instead of the VSV-glycoprotein (VSV-G) was pseudotyped with SARS-CoV-1 S glycoprotein (UniProt Ref: P59594) and with SARS-CoV-2 S glycoprotein derived from either the Wuhan reference strain (NCBI Ref: 43740568), the Alpha variant (alterations: Δ69/70, Δ144, N501Y, A570D, D614G, P681H, T716I, S982A, D1118H), the Beta variant (alterations: L18F, D80A, D215G, Δ242-244, R246I, K417N, E484K, N501Y, D614G, A701V), the Delta variant (alterations: T19R, G142D, E156G, Δ157/158, K417N, L452R, T478K, D614G, P681R, D950N) the Omicron BA.1 variant (alterations: A67V, Δ69/70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F), the Omicron BA.2 variant (alterations: T19I, Δ24-26, A27S, G142D, V213G, G339D, S371F, S373P, S375F, T376A, D405N, R408S, K417N, N440K, S477N, T478K, E484A, Q493R, Q498R, N501Y, Y505H, D614G, H655Y, N679K, P681H, N764K, D796Y, Q954H, N969K), the Omicron BA.2.12.1 variant (alterations: T19I, Δ24-26, A27S, G142D, V213G, G339D, S371F, S373P, S375F, T376A, D405N, R408S, K417N, N440K, L452Q, S477N, T478K, E484A, Q493R, Q498R, N501Y, Y505H, D614G, H655Y, N679K, P681H, S704L, N764K, D796Y, Q954H, N969K), or the Omicron BA.4/5 variant (alterations: T19I, Δ24-26, A27S, Δ69/70, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, N969K) according to published pseudotyping protocols (Ref. 49).

A diagram of SARS-CoV-2 S glycoprotein alterations is shown in Fig. 32 and a separate alignment of S glycoprotein alterations in Omicron sub-lineages is displayed in Fig. 33. In brief, HEK293T/17 monolayers (ATCC^{®} CRL-11268^{™}) cultured in Dulbecco's modified Eagle's medium (DMEM) with GlutaMAX^{™} (Gibco) supplemented with 10% heat-inactivated fetal bovine serum (FBS [Sigma-Aldrich]) (referred to as medium) were transfected with Sanger sequencing-verified SARS-CoV-1 or variant-specific SARS-CoV-2 S expression plasmid with Lipofectamine LTX (Life Technologies) following the manufacturer's instructions. At 24 hours VSV-G complemented VSVΔG vector. After incubation for 2 hours at 37 °C with 7.5% CO2, cells were washed twice with phosphate buffered saline (PBS) before medium supplemented with anti-VSV-G antibody (clone 8G5F11, Kerafast Inc.) was added to neutralize residual VSV-G-complemented input virus. VSV-SARS-CoV-2-S pseudotype-containing medium was harvested 20 hours after inoculation, passed through a 0.2 µm filter (Nalgene) and stored at -80 °C. The pseudovirus batches were titrated on Vero 76 cells (ATCC^{®} CRL-1587^{™}) cultured in medium.

The relative luciferase units induced by a defined volume of a Wuhan S glycoprotein pseudovirus reference batch previously described in Muik et al., 2021, that corresponds to an infectious titer of 200 transducing units (TU) per mL, was used as a comparator. Input volumes for the SARS-CoV-2 variant pseudovirus batches were calculated to normalize the infectious titer based on the relative luciferase units relative to the reference.

### Pseudovirus neutralization assay

Vero 76 cells were seeded in 96-well white, flat-bottom plates (Thermo Scientific) at 40,000 cells/well in medium 4 hours prior to the assay and cultured at 37 °C with 7.5% CO2. Each individual serum was serially diluted 2-fold in medium with the first dilution being 1:5 (Omicron-naïve triple BNT162b2 vaccinated; dilution range of 1:5 to 1:5,120) or 1:30 (triple vaccinated after subsequent Omicron BA.1 or BA.2 breakthrough infection; dilution range of 1:30 to 1:30,720). In the case of the SARS-CoV-1 pseudovirus assay, the serum of all individuals was initially diluted 1:5 (dilution range of 1:5 to 1:5,120). VSV-SARS-CoV-2-S/VSV-SARS-CoV-1-S particles were diluted in medium to obtain 200 TU in the assay. Serum dilutions were mixed 1:1 with pseudovirus (n=2 technical replicates per serum per pseudovirus) for 30 minutes at room temperature before being added to Vero 76 cell monolayers and incubated at 37 °C with 7.5% CO2 for 24 hours. Supernatants were removed and the cells were lysed with luciferase reagent (Promega). Luminescence was recorded on a CLARIOstar^{®} Plus microplate reader (BMG Labtech), and neutralization titers were calculated as the reciprocal of the highest serum dilution that still resulted in 50% reduction in luminescence. Results were expressed as geometric mean titers (GMT) of duplicates. If no neutralization was observed, an arbitrary titer value of half of the limit of detection [LOD] was reported.

### Statistical analysis

The statistical method of aggregation used for the analysis of antibody titers is the geometric mean and for the ratio of SARS-CoV-2 VOC titer and Wuhan titer the geometric mean and the corresponding 95% confidence interval. The use of the geometric mean accounts for the non-normal distribution of antibody titers, which span several orders of magnitude. The Friedman test with Dunn's correction for multiple comparisons was used to conduct pairwise signed-rank tests of group geometric mean neutralizing antibody titers with a common control group. All statistical analyses were performed using GraphPad Prism software version 9.

### References cited in Example 14

1. WHO Technical Advisory Group on SARS-CoV-2 Virus Evolution (TAG-VE), Classification of Omicron (B.1.1.529): SARS-CoV-2 Variant of Concern (2021).
2. WHO Headquarters (HQ), WHO Health Emergencies Programme, Enhancing response to Omicron SARS-CoV-2 variant: Technical brief and priority actions for Member States (2022).
3. M. Hoffmann et al., The Omicron variant is highly resistant against antibody-mediated neutralization. Cell. 185, 447-456.e11 (2022), doi:10.1016/j.cell.2021.12.032.
4. W. Dejnirattisai et al., SARS-CoV-2 Omicron-B.1.1.529 leads to widespread escape from neutralizing antibody responses. Cell. 185, 467-484.e15 (2022), doi:10.1016/j.cell.2021.12.046.
5. V. Servellita et al., Neutralizing immunity in vaccine breakthrough infections from the SARS-CoV-2 Omicron and Delta variants. Cell. 185, 1539-1548.e5 (2022), doi:10.1016/j.cell.2022.03.019.
6. C. Kurhade et al., Neutralization of Omicron BA.1, BA.2, and BA.3 SARS-CoV-2 by 3 doses of BNT162b2 vaccine. Nature communications. 13, 255 (2022), doi:10.1038/s41467-022-30681-1.
7. Y. Cao et al., Omicron escapes the majority of existing SARS-CoV-2 neutralizing antibodies. Nature. 602, 657-663 (2022), doi:10.1038/s41586-021-04385-3.
8. Y. Cao et al., BA.2.12.1, BA.4 and BA.5 escape antibodies elicited by Omicron infection. Nature (2022), doi:10.1038/s41586-022-04980-y.
9. N. P. Hachmann et al., Neutralization Escape by SARS-CoV-2 Omicron Subvariants BA.2.12.1, BA.4, and BA.5. The New England journal of medicine (2022), doi:10.1056/NEJMc2206576.
10. E. Mathieu et al., A global database of COVID-19 vaccinations. Nature human behaviour. 5, 947-953 (2021), doi:10.1038/s41562-021-01122-8.
11. C. I. Kaku et al., Recall of pre-existing cross-reactive B cell memory following Omicron BA.1 breakthrough infection. Science immunology, eabq3511 (2022), doi:10.1126/sciimmunol.abq3511.
12. J. Quandt et al., Omicron BA.1 breakthrough infection drives cross-variant neutralization and memory B cell formation against conserved epitopes. Science immunology, eabq2427 (2022), doi:10.1126/sciimmunol.abq2427.
13. A. Muik et al., Neutralization of SARS-CoV-2 Omicron by BNT162b2 mRNA vaccine-elicited human sera. Science (New York, N.Y.). 375, 678-680 (2022), doi:10.1126/science.abn7591.
14. A. Muik et al., Neutralization of SARS-CoV-2 lineage B.1.1.7 pseudovirus by BNT162b2 vaccine-elicited human sera. Science (New York, N.Y.). 371, 1152-1153 (2021), doi:10.1126/science.abg6105.
15. C.-W. Tan et al., Pan-Sarbecovirus Neutralizing Antibodies in BNT162b2-Immunized SARS-CoV-1 Survivors. The New England journal of medicine. 385, 1401-1406 (2021), doi:10.1056/NEJMoa2108453.

### Example 15: Further updates on immune responses elicited by vaccines encoding a SARS-CoV-2 S protein from an Omicron variant

Following the experiment described in Example 8, further subjects were enrolled in a clinical trial investigating RNA vaccines encoding a SARS-CoV-2 S protein comprising one or more mutations characteristic of a BA.1 Omicron variant. In the present Example, subjects (18 to 55 years of age with or without evidence of prior infection) were administered a 2nd booster (4th dose) of either 30 ug of RNA encoding a SARS-CoV-2 S protein of a Wuhan strain (in the present Example, BNT162b2) or 30 µg of RNA encoding an SARS-CoV-2 S protein having one or more mutations characteristic of an Omicron variant (in the present example, BNT162b2 OMI, which encodes a SARS-CoV-2 S protein having mutations characteristic of a BA.1 Omicron variant, comprises SEQ ID NOs: 50 and 51, and encodes an amino acid of SEQ ID NO: 49).

In a primary immunogenicity analysis of participants without evidence of prior infection, BNT162b2 OMI (N=132) elicited a superior neutralizing antibody response to the BA.1 Omicron SARS-CoV-2 virus compared to BNT162b2 (N=141). The BNT162b2 OMI GMT against BA.1 Omicron was 1929 (CI: 1632, 2281) compared to a BNT162b2 GMT of 1100 (CI: 932, 1297); GMT ratio 1.75 (95% Cl: 1.39, 2.22).

Compared to BNT162b2, BNT162b2 OMI elicited a similar neutralizing antibody response to a Wuhan strain of SARS-CoV-2. BNT162b2 OMI GMT was 11997 (CI: 10554, 13638) compared to a BNT162b2 GMT of 12009 (CI: 10744, 13425).

The data suggests that an Omicron monovalent vaccine as the 2nd booster vaccination (4th dose) improves a neutralizing antibody response to BA.1 Omicron compared to an RNA vaccine encoding an S protein of a Wuhan strain, and does not negatively affect a neutralizing antibody response to a Wuhan strain of SARS-CoV-2.

### Example 16. Immunological impact of VOC vaccination

The present Example describes immunological impacts of administration of BNT162b2 vaccines encoding spike proteins from certain variants of concern ("VOC"). In particular, the present Example describes immunological impacts of administration of a "booster dose" to subjects (in this Example, mice) who have received two doses (i.e., according to an established model immunization protocol) of the "original" BNT162b2 vaccine (i.e., encoding the Wuhan spike protein, as described herein).

Figure 34 presents the immunization protocol utilized in the present Example. Specifically, BALB/c mice were immunized twice (1 ug each dose) with BNT 162b2, and then at a later time point with a BNT162b2/VOC (1 ug each dose). Immunization occurred up to 3 or 4 times. Animals were bled regularly to analyze antibody immune response by ELISA and pseudovirus neutralization assay. At the end of the trial, animals were euthanized and T cell response in the spleen was analyzed.

Boosting was performed with: (a) the original BNT162b2 ("BNT162b2"); (b) BNT162b2 OMI BA.1 ("OMI BA.1"); (c) BNT162b2 OMI BA.4/5 ("OMI BA.4/5"); (d) BNT162b2 + OMI BA.1 (0.5 g each) (e) BNT162b2 + OMI BA.4/5 (0.5 ug each); (f) OMI BA.1 + OMI BA.4/5 (0.5 ug each); and (g) BNT162b2 + OMI BA.1 + OMI BA.4/5 (0.33 ug each).

Omicron variants BA.4 and BA.5 were first reported in circulation in January 2022, and were becoming dominant variants by June 2022. Both of these lineages contain the amino-acid substitutions F486V, and R493Q. Preliminary studies suggest a significant change in antigenic properties of BA.4 and BA.5 compared to BA.1 and BA.2, especially compared to BA.1. Additionally, as increasing trends in BA.5 variant proportions are observed in particular locations (e.g., in Portugal), COVID-19 case numbers and test positivity rate have also increased. The present disclosure proposes that BA.4/5 (which, given their common spike protein mutations, are considered together in the present Example) could represent escape VOC. The present disclosure demonstrates particular benefits of dosing regimens (e.g., as described herein and specifically as exemplified in the present Example) that include one or more doses of a vaccine that comprises or delivers (e.g., via expression of an administered RNA) a spike protein that includes relevant BA.4/5 sequences (e.g., amino acid substitutions). Figures 35 and 36 present baseline (determined at day 104, pre-boost) geometric mean titers ("GMT"s) relative to various SARS-CoV-2 strains, as indicated. As can be seen, baseline immunization of the different mouse cohorts was comparable. Specifically, group GMTs per pseudovirus were consistently in the same ballpark between cohorts; no difference greater than about 2-fold was observed. Consistent with observations made in human populations, as noted above, neutralizing GMTs against the Wuhan strain were considerably higher (GMT of up to 3,044) as compared to those against VOCs. Overall, the order of GMTs was Wuhan > BA.1 - BA.2 > BA.2.12.1 > BA.4/5.

Figure 37 shows baseline (determined at day 104, pre-boost) cross-neutralization analysis and demonstrates that baseline immunization of cohorts with respect to cross-neutralization capacity is comparable. Specifically, at baseline, calculated variant/Wuhan ref GMT ratios indicated that cross-neutralization capacity was quite comparable between cohorts (only one outlier in the BNT162b2 monovalent group re. BA.1 neutralization was observed). Again consistent with observations of human populations, BA.1 = BA.2 > BA.2.12.1 > BA.4/5 Figures 38 - 40 present data obtained seven days post-boost and document remarkable effectiveness of BA.4/5, and in particular of monovalent BA.4/5, in achieving significant geometric mean fold increase of GMTs (Figures 38 and 39) and effective cross-neutralization (Figure 40). As can be seen, BNT162b2 booster immunization resulted in a comparable titer increase against all VOCs (3.9-7.1 fold), whereas monovalent BA.1 and BA.4/5 boosters resulted in a considerably stronger increase in the homologous VOC titer (16.8-fold for BA.1, 67.3-fold for BA.4/5).

The monovalent BA.4/5 booster was the most effective in driving titer increases across the pseudovirus panel tested. Bivalent boosters showed a similar but attenuated trend compared to the monovalent VOC boosters; amongst bivalent boosters the b2 + BA.4/5 combination was most effective in driving broad cross-neutralization. The trivalent booster (b2 + BA.1 + BA.4/5) was superior to the bivalent boosters and gave intermediate immunization between the bivalent b2 + BA.4/5 and monovalent BA.4/5 booster.

Figure 40, among other things, presents calculated variant/Wuhan ref GMT ratios, which indicate that:
(i) BNT162b2 booster results in relatively poor cross-neutralization, especially of BA.2 and descendants (BA.2.12.1, BA.4/5)
(ii) BA.1 booster results in superior cross-neutralization of BA.1, but still relatively poor neutralization of BA.2.12.1, BA.4/5
(iii) BA.4/5 booster results in balanced pan-Omicron neutralization with very encouraging neutralization against BA.2, BA.2.12.1 and BA.4/5
Bivalent boosters showed a similar but attenuated trend compared to the monovalent VOC boosters; among bivalent boosters the b2 + BA.4/5 combination was most effective in driving broad cross-neutralization; the trivalent booster (b2 + BA.1 + BA.4/5) elicited comparable cross-neutralization to the BA.1/BA.4/5 booster.

Mice can also be administered two RNA molecules, where the ratio of the two RNA molecules is not 1:1. For example, mice can be administered a bivalent vaccine comprising BNT162b2 and BA.4/5 at a ratio of 1:2 (e.g., by administering 0.33 ug of BNT162b2 and 0.66 ug of BA.4/5). Mice can also be administered a bivalent vaccine comprising BNT162b2 and BA.4/5 at a ratio of 1:3 (e.g., by administering 0.25 ug of BNT162b2 and 0.75 ug of BA.4/5). Such compositions can be administered to vaccine naive mice or to mice previously vaccinated with BNT162b2 (e.g., previously administered two doses fo 1 ug of BNT162b2).

The present specification demonstrates remarkable efficacy of BA.4/5 immunization (and specifically of BNT162b2 + BA.4/5 immnunization, e.g., with sequences provided herein). Futhermore, the present specification demonstrates efficacy of BA.4/5 immunization in monovalent, bivalent, and trivalent formats, and documents surprising efficacy of monovalent BA.4/5.

The present disclosure specifically demonstrates remarkable usefulness of one or more BA.4/5 doses administered to subjects who have previously been immunized (e.g., with a Wuhan vaccine, such as with at least (or exactly) two doses of a Wuhan vaccine. Without wishing to be bound by any particular theory, the present disclosure teahces that immunological characteristics of the omicron BA.4/5 spike may render it particularly useful or effective for immunization of subjects, including those who have been immunized (e.g., via prior administration of one or more vaccine doses and/or by prior infection) with the Wuhan strain (and/or with one or more strains immnuologically related to the Wuhan strain), including specifically by vaccination with one or more (e.g., 1, 2, 3, 4 or more) doses of original BNT162b2.

### Example 17: Omicron BA.2 breakthrough infection enhances cross-neutralization of BA.2.12.1 and BA.4/BA.5

The present Example 17 is an extension of Example 14, and describes experiments in which serum samples collected from BA.1- and BA.2-breakthrough cases were analyzed for neutralization activity against Omicron BA.4 and BA.5 variants. In addition to confirming the results described in Example 14, the present Example 17 also provides further characterization of antibody responses induced by BA.1 and BA.2 breakthrough infections, and provides insights as to what aspects may contribute to increased neutralization of a BA.4/5 Omicron variant as observed in a BA.2 breakthrough infection, as compared to a BA.1 breakthrough infection. In particular, the present Example demonstrates that a BA.2 breakthrough infection can induce higher titers of neutralization antibodies that bind the N-terminal domain (NTD) of a SARS-CoV-2 S protein (e.g., a BA.4/5 SARS-CoV-2 Omicron variant), which can result in increased neutralization of a BA.4/5 Omicron variant.

As demonstrated in the previous examples, individuals previously administered RNA encoding a SARS-CoV-2 S protein from a Wuhan strain, who subsequently had an Omicron BA.1 breakthrough infection, have strong serum neutralizing activity against Omicron BA.1, BA.2, and previous SARS-CoV-2 variants of concern (VOCs), yet less against highly contagious Omicron sublineages BA.4 and BA.5 that have displaced previous variants. As the latter sublineages are derived from Omicron BA.2, the serum neutralizing activity of COVID-19 mRNA vaccine triple-immunized individuals who experienced BA.2 breakthrough infection was analyzed. The present Example demonstrates that sera of these individuals have broadly neutralizing activity against previous VOCs as well as all tested Omicron sublineages, including BA.2 derived variants BA.2.12.1, BA.4/BA.5 (confirming the results of previous Example 14). Furthermore, applying antibody depletion the present Example shows that neutralization of BA.2 and BA.4/BA.5 sublineages by BA.2 convalescent sera is driven to a significant extent by antibodies targeting the N-terminal domain (NTD) of the spike glycoprotein, whereas their neutralization by Omicron BA.1 convalescent sera depends exclusively on antibodies targeting the receptor binding domain (RBD). These findings suggest that exposure to Omicron BA.2, in contrast to BA.1 spike glycoprotein, triggers significant NTD specific recall responses in vaccinated individuals and thereby enhances the neutralization of BA.4/BA.5 sublineages. Given the current epidemiology with a predominance of BA.2 derived sublineages like BA.4/BA.5 and rapidly ongoing evolution, these findings are of high relevance for the development of Omicron adapted vaccines.

### Introduction

Emergence of the SARS-CoV-2 Omicron variant of concern (VOC) in November 2021 (Ref. 1) can be considered a turning point in the COVID-19 pandemic. Omicron BA.1, which is significantly altered in the spike (S) glycoprotein receptor binding domain (RBD) and N-terminal domain (NTD) relative to an S protein from a Wuhan strain, partially escapes previously established immunity (Ref. 2). The loss of many epitopes (Refs. 3, 4) decreased susceptibility to neutralizing antibodies induced by wild-type strain (Wuhan-Hu-1) S glycoprotein-based vaccines or by infection with previous strains (Refs. 5-7), necessitating a third vaccine dose to establish full immunity (Refs. 8-10). Omicron BA.1 was displaced by the BA.2 variant, which in turn was displaced by its descendants BA.2.12.1, BA.4 and BA.5 that now dominate in many regions (Refs. 11-14).

Antigenically, BA.2.12.1 exhibits high similarity with BA.2 but not BA.1, whereas BA.4 and BA.5 differ considerably from BA.2 and even more so from BA.1, in line with their genealogy (Refs. 15 and 16). While some amino acid changes in the RBD are shared between all Omicron sub-lineages, the alteration L452Q is only found in BA.2.12.1 and is the only residue which distinguishes its RBD from that of the BA.2 variant. The L452R and F486V alterations are BA.4/BA.5-specific, whereas S371F, T376A, D405N, and R408S are shared by BA.2 and its descendants BA.2.12.1 and BA.4/BA.5, but not BA.1 (Fig. 33). These amino acid exchanges are associated with further escape from vaccine-induced neutralizing antibodies and therapeutic antibody drugs targeting the wild-type S glycoprotein (Refs. 6, 15, 17-20). The NTDs of BA.2 and its descendants are antigenically closer to the wild-type strain and lack several amino acid changes, insertions, and deletions that occurred in BA.1 (Fig. 33). For instance, Δ143-145, L212I, or ins214EPE, which rendered the BA.1 variant resistant to a panel of NTD-directed monoclonal antibodies raised against the wild-type S glycoprotein, are not found in BA.2 and descendants (Refs. 21, 22).

As demonstrated in the previous Examples, Omicron BA.1 breakthrough infection of BNT162b2 vaccinated individuals augments broadly neutralizing activity against Omicron BA.1, BA.2 and previous VOCs at levels similar to those observed against SARS-CoV-2 wild-type. BA.1 breakthrough infection of triple BNT162b2-vaccinated individuals induces a robust recall response, primarily expanding memory B cells against epitopes shared broadly amongst variants, rather than inducing B cells specific to BA.1 only. Neutralization of Omicron sublineages BA.4 and BA.5 was increased to a lesser extent in BA.1 breakthrough patients as compared to the BA.1 variant, and geometric mean titers were comparable to those against the phylogenetically more distant SARS-CoV-1.

Given that Omicron BA.2 is more closely related to BA.4/BA.5 than to BA.1, whether BA.2 breakthrough infection would shift cross-neutralization activity more towards these most recent Omicron sublineages, as compared to BA.1 breakthrough infection, was assessed. The neutralization of different Omicron sublineages by serum samples was compared from three different cohorts of individuals triple-vaccinated with mRNA COVID-19 vaccines, namely from individuals with no history of SARS-CoV-2 infection and individuals that experienced breakthrough infection with either BA.1 or BA.2. In addition, the contribution of serum antibodies targeting the S glycoprotein RBD versus the NTD to Omicron sublineage neutralization was characterized. The resulting data increase current understanding on Omicron immune escape mechanisms and the effects of immunization on variant cross-neutralization, and thus help guide further vaccine development.

### Results

### Cohorts and sampling

This study investigated serum samples from three cohorts: from BNT162b2 triple-vaccinated individuals who were SARS-CoV-2-naïve at the time of sampling (BNT162b2³, n=18), from individuals vaccinated with three doses of mRNA COVID-19 vaccine (BNT162b2/mRNA-1273 homologous or heterologous regimens) who subsequently had a breakthrough infection with Omicron at a time of BA.1 dominance (mRNA-Vax³ + BA.1, n=14), or from triple mRNA vaccinated individuals with a breakthrough infection at a time of BA.2 dominance (mRNA-Vax³ + BA.2, n=13). For convalescent cohorts, relevant intervals between key events such as the most recent vaccination and infection are provided in Fig. 41. Sera were derived from the biosample collections of BNT162b2 vaccine trials and from a non-interventional study researching vaccinated patients that had experienced Omicron breakthrough infection.

### Omicron BA.2 breakthrough infection of triple mRNA-vaccinated individuals induces broad neutralization of VOCs including Omicron BA.4/BA.5

Neutralizing activity of immune sera was tested in a well-characterized pseudovirus neutralization test (pVNT) (Refs. 24, 25) by determining 50% pseudovirus neutralization (pVN₅₀) geometric mean titers (GMTs) with pseudoviruses bearing the S glycoproteins of the SARS-CoV-2 wild-type strain, or Alpha, Beta, Delta, Omicron BA.1, BA.2, and the BA.2-derived sublineages BA.2.12.1, BA.4 and BA.5. As BA.4 and BA.5 share an identical S glycoprotein sequence, in the present Example they are referred to as BA.4/5 in the context of the pVNT. In addition, SARS-CoV (herein referred to as SARS-CoV-1) was assayed to detect potential pan-Sarbecovirus neutralizing activity (Ref. 26). As an orthogonal test system, a live SARS-CoV 2 neutralization test (VNT) was also used that analyzes neutralization during multicycle replication of authentic virus (SARS-CoV-2 wild-type strain and VOCs including BA.4, except Omicron BA.2.12.1) with the antibodies present during the entire test period.

In the pVNT, sera from all three cohorts robustly neutralized the wild-type strain, Alpha, Beta, Delta VOCs as well as Omicron BA.1 and BA.2 lineages with neutralization activity being more pronounced in the breakthrough infected individuals, particularly in the BA.1 breakthrough infection cohort (mRNA-Vax3 + BA.1). However, serum neutralizing activity of BNT162b2 triple-vaccinated SARS-CoV-2 naïve (BNT162b2³) and mRNA-Vax³ + BA.1 individuals against BA.2.12.1 was significantly reduced compared to wild-type (p<0.05) and even more so for BA.4/5 (p<0.001; >5-fold compared to the wild-type strain) (Fig. 42(A)). In contrast, sera from triple mRNA-vaccinated individuals with Omicron BA.2 breakthrough infection (mRNA-Vax3 + BA.2) neutralized the BA.2.12.1 pseudovirus as robustly as the wild-type strain. Neutralization of BA.4/5 was broadly similar to that of BA.2.12.1, and the reduction relative to the wild-type strain significant (p<0.05) yet less pronounced (^{~}2.5-fold) as compared to the two other cohorts.

To compare the cohorts with regard to neutralization breadth irrespective of the magnitude of antibody titers, the VOC pVN₅₀ GMTs was normalized against the Wuhan strain. The ratios showed that BA.4/5 cross-neutralization was substantially stronger in mRNA-Vax³ + BA.2 (GMT ratio 0.38) as compared to mRNA-Vax³ + BA.1 and BNT162b2³ sera (GMT ratios 0.18 and 0.17) (Fig. 42(B)). Similarly, cross-neutralization of Omicron BA.2.12.1 by mRNA-Vax³ + BA.2 sera (GMT ratio 0.52) was stronger than by mRNA-Vax³ + BA.1 sera (GMT ratio 0.43), and even more so than by BNT162b2³ sera (GMT ratio 0.26).

A separate analysis including only the BNT162b2 vaccinated individuals within those three cohorts confirmed that BA.2 breakthrough infection is associated with considerable BA.4/5 cross-neutralization (BA.4/5 to wild-type GMT ratio 0.42), whereas after BA.1 breakthrough infection pVN₅₀ GMTs against BA.4/5 were ^{~}6-fold lower than those against wild-type (i.e., GMT ratio 0.17) (Fig. 45 ((A)-(C)). Cross-neutralization of BA.2 and BA.2.12.1 by sera of the BA.1 or BA.2 convalescents was superior to that of BNT162b2 triple-vaccinated SARS-CoV-2 naïve individuals.

The authentic live SARS-CoV-2 virus neutralization assay provided VOC neutralizing titers that strongly correlated with those from the pVNT assay (Fig. 46) and confirmed the major findings in Fig. 42. In this assay, 50% virus neutralization (VN₅₀) GMT against Omicron BA.2 in BNT162b2³ sera was strongly reduced compared to that against wild-type (p<0.0001), whereas sera from both convalescent groups exhibited strong neutralizing activity, with VN₅₀ GMTs comparable to those against the wild-type strain (Fig. 43(A)). Reduction of neutralizing activity against Omicron BA.4 was less pronounced in the BA.2 convalescent cohort as compared to BNT162b2³ and mRNA-Vax³ + BA.1 cohorts (VN₅₀ GMTs ^{~}2.5-fold as compared to ^{~}15-fold and 5-fold lower than against the wild-type strain, respectively).

In line with the pVNT data, magnitude-independent analyses via the calculated ratios of VOC VN₅₀ GMTs against the wild-type strain showed that BA.4 cross-neutralization was stronger in the mRNA-Vax³ + BA.2 cohort (GMT ratio 0.39) as compared to the mRNA-Vax³ + BA.1 (GMT ratio 0.20) and BNT162b2³ (GMT ratio 0.07) cohorts (Fig. 43(B)) and similarly so within the sub-cohort of BNT162b2 triple-vaccinated individuals (Figs. 45(D)-(F)).

In aggregate, these data demonstrate that Omicron BA.2 breakthrough infection of vaccinated individuals was associated with broad neutralizing activity against all tested Omicron-sublineages and previous SARS-CoV-2 VOCs. In particular, these data indicate that breakthrough infection with BA.2 was more effective (^{~}2-fold higher cross neutralization) than that with BA.1 at refocusing neutralizing antibody responses towards the BA.4/BA.5 S glycoprotein.

Neutralization of Omicron BA.2 and BA.4/5 by sera of triple mRNA vaccinated BA.2 convalescent individuals is mediated to a large extent by NTD-targeting antibodies.

To dissect the role of serum antibodies binding either to the RBD or the NTD of the S glycoprotein for neutralization of SARS-CoV-2 wild-type, Omicron BA.1, BA.2, and BA.4/5, these antibody fractions were depleted separately from sera of the three cohorts (n=6 each, Fig. 47(A)). The SARS-CoV-2 wild-type strain S glycoprotein RBD and NTD baits was used for depletion, as VOC breakthrough infections have been demonstrated to predominantly elicit recall responses recognizing epitopes conserved across known VOCs (Refs. 10, 23, and 27). The depletion experiments removed >97% of all RBD-binding antibodies and >74% of all NTD-binding antibodies (Fig. 47(B)). Depleted sera were subsequently tested in pVNT assays. RBD-antibody depletion strongly diminished neutralizing activity against the wild-type strain in sera from all cohorts, whereas neutralizing activity was mostly retained (>80% remaining activity) upon depletion of NTD-binding antibodies (Fig. 44 (A)). Neutralization of Omicron BA.1 was completely abrogated upon depletion of RBD-binding antibodies and largely unaffected by NTD-binding antibody depletion. For neutralization of BA.2, RBD-antibody depletion almost completely abolished neutralizing activity of mRNA-Vax³ + BA.1 sera (about 2% residual neutralization activity). The reduction of neutralizing titers for BNT162b2³ and particularly mRNA-Vax³ + BA.2 sera was less severe with ^{~}12% and ^{~}24% remaining neutralizing activity, respectively. In contrast, depletion of NTD-binding antibodies did not considerably impact the neutralizing activity of BNT162b2³ and mRNA-Vax³ + BA.1 sera (^{~}91 and ^{~}99% of undepleted control, respectively), while neutralizing activity of mRNA-Vax³ + BA.2 sera was reduced to ^{~}50%. A similar pattern was seen following RBD-antibody depletion for neutralization of BA.4/5, with strongly reduced neutralizing activity of mRNA-Vax³ + BA.1 sera (⁻3% residual activity) versus less severe reductions for BNT162b2³ and mRNA-Vax³ + BA.2 sera (^{~}20 and ^{~}26% remaining activity, respectively). Depletion of NTD-binding antibodies had a larger impact for BA.4/5 neutralization compared to BA.2, with remaining neutralizing activity of BNT162b2³ and mRNA-Vax³ + BA.1 sera of ^{~}70 and ^{~}90% respectively, again with the strongest effect (^{~}48% of undepleted control) of mRNA-Vax³ + BA.2 sera.

As an orthogonal approach the neutralizing activity of sera was assessed from those 3 cohorts of vaccinated individuals against a pseudovirus harboring an engineered hybrid S glycoprotein consisting of the Omicron BA.1 N-terminus including the NTD (amino acids 1-338) and the BA.4/5 C terminus including the RBD.

The pVN₅₀ GMT against the Omicron BA.1-BA.4/5 hybrid pseudovirus in sera from BNT162b2³ was moderately below (1.86-fold) the GMT for the BA.4/5 pseudovirus, and in the BA.1 convalescents the GMT was only marginally affected (<1.5-fold reduction) (Fig. 44(B)). In contrast, in BA.2 convalescent sera titers against the hybrid pseudovirus were considerably lower than those against the BA.4/5 pseudovirus (>3-fold reduction of GMT) (Fig. 44(B)), suggesting that substantial neutralizing activity can be attributed to NTD epitopes that are shared between Omicron BA.2 and BA.4/5.

In aggregate the data obtained in both experiments indicate that across all these VOCs RBD-binding antibodies provided a major contribution to neutralization. Additionally, exposure to BA.1 (that differs substantially from previous VOCs in its NTD; Fig. 33) boosted recall responses of vaccine-induced neutralizing antibodies that primarily bind the RBD, whereas exposure to BA.2 S glycoprotein (with an NTD closer related to previous VOCs) can build on existing memory and elicited a considerable recall of NTD-targeting antibodies that in turn contributed substantially to the neutralization of BA.2 and BA.4/5.

### Discussion

Recent studies have demonstrated that Omicron BA.1 breakthrough infection in individuals vaccinated with mRNA vaccines BNT162b2 or mRNA-1273 or an inactivated virus vaccine boosts serum neutralizing titers against VOCs including BA.2 (Refs. 10, 15, 23), but not against BA.2.12.1 or BA.4/BA.5. The immune escape has been attributed to boosting of pre-existing neutralizing antibody responses that recognize epitopes shared between the SARS-CoV-2 wild-type strain and Omicron BA.1 but are in part absent in BA.2.12.1, BA.4, and BA.5 due to alterations at key residues including L452Q/L452R, and F486V (Ref. 15).

In the present Example, BA.2 breakthrough infection was associated with broadly neutralizing activity including BA.2 and its descendants BA.2.12.1, BA.4 and BA.5. These findings suggest that the higher sequence similarity of BA.2 with BA.2.12.1 and BA.4/5 in the S glycoprotein RBD as well as the NTD drives more efficient cross-neutralization as compared to breakthrough infections with the antigenically more distant BA.1 variant. In particular, BA.1 breakthrough infection may not elicit a strong recall of NTD-specific memory B cells owing to the substantial alterations within the BA.1 NTD (Fig. 33) given that breakthrough infection with heterologous SARS-CoV-2 strains primarily expands a memory B cell repertoire against conserved S glycoprotein epitopes (Refs. 10, 23). The data described herein that was obtained in antibody-depletion and hybrid pseudovirus experiments show that NTD-binding antibodies have a substantial contribution to neutralizing activity against Omicron BA.4/5 in triple-vaccinated BA.2 convalescent sera, whereas neutralizing activity in BA.1 convalescent sera largely relies on RBD-binding antibodies. This finding is consistent with the observation that NTD-binding antibodies isolated from BA.2 breakthrough infected individuals do not neutralize BA.1 (Ref. 29). Together these important findings extend our knowledge on how vaccinations and boosters with the current wild-type strain-based vaccines together with breakthrough infections with the various VOCs shape the immunity patterns within the population and are material to inform further vaccine development and adaptation in response to current and emerging VOCs.

Notwithstanding the importance of vaccination with currently approved wild-type-strain based vaccines such as BNT162b2 that offer effective protection from severe disease by current VOCs including Omicron BA.1 and BA.2 (Refs. 30 and 31), the present findings highlight that consideration of rapidly evolving epidemiological landscapes and newly emerging SARS-CoV-2 variants is important for guiding vaccine adaptation programs. For instance, while the efficacy of vaccine adaptation to the BA.1 strain S glycoprotein sequence is currently under investigation in clinical trials, the present data suggest that further benefit may be derived from a vaccine adapted to the sequence of BA.2 or descendants.

### Materials and Methods

### Study design, recruitment of participants and sample collection

The objective of this study was to investigate the effect of Omicron BA.2 breakthrough infection on the cross-variant neutralization capacity of human sera. Immune responses in triple-mRNA (BNT162b2/mRNA-1273)-vaccinated individuals with a confirmed subsequent SARS-CoV-2 breakthrough infection in a period of Omicron BA.2 lineage-dominance in Germany (March to May 2022; mRNA-Vax3 + BA.2), was compared to that of triple-mRNA-vaccinated individuals with a confirmed subsequent SARS-CoV-2 breakthrough infection in a period of Omicron BA.1 lineage-dominance (November 2021 to mid-January 2022; mRNA-Vax³ + BA.1) (Refs. 1 and 2) and triple-BNT162b2-vaccinated individuals that were SARS-CoV-2-naïve (nucleocapsid seronegative) at the time of sample collection (BNT162b2³). Serum neutralizing capability was characterized using pseudovirus and live SARS-CoV-2 neutralization assays. Data for the reference cohorts BNT162b2³ and mRNA-Vax³ + BA.1 were previously published (Ref. 10), except for newly generated BA.2.12.1 neutralization data. Cross-neutralization of variants was further characterized in smaller sub-cohorts after depletion of either wild-type S glycoprotein NTD- or RBD-targeted neutralizing antibodies. Individuals from the BNT162b2³ cohort provided informed consent as part of their participation in the Phase 2 trial BNT162-17 (NCT05004181). Participants from the mRNA-Vax³ + Omi BA.1 and mRNA-Vax³ + BA.2 cohorts were recruited from University Hospital, Goethe University Frankfurt as part of a non-interventional study (protocol approved by the Ethics Board of the University Hospital [No. 2021-560]) researching patients that had experienced Omicron breakthrough infection following vaccination for COVID-19. Omicron BA.1 infections were confirmed with variant-specific PCR. The infections of 4 BA.1 convalescent participants in this study were further characterized by genome sequencing. In all 4 cases, genome sequencing confirmed Omicron BA.1 infection.

Demographic and clinical data for all participants and sampling timepoints are provided (Fig. 41). All participants had no documented history of SARS-CoV-2 infection prior to vaccination. Participants were free of symptoms at the time of blood collection.

Serum was isolated by centrifugation of drawn blood at 2000 x g for 10 minutes and cryopreserved until use.

### VSV-SARS-CoV-2 S variant pseudovirus generation

A recombinant replication-deficient vesicular stomatitis virus (VSV) vector that encodes green fluorescent protein (GFP) and luciferase instead of the VSV-glycoprotein (VSV-G) was pseudotyped with SARS-CoV-1 S glycoprotein (UniProt Ref: P59594) and with SARS-CoV-2 S glycoprotein derived from either the Wuhan-Hu-1 reference strain (NCBI Ref: 43740568), the Alpha variant (alterations: Δ69/70, Δ144, N501Y, A570D, D614G, P681H, T716I, S982A, D1118H), the Beta variant (alterations: L18F, D80A, D215G, Δ242-244, R246I, K417N, E484K, N501Y, D614G, A701V), the Delta variant (alterations: T19R, G142D, E156G, Δ157/158, K417N, L452R, T478K, D614G, P681R, D950N), the Omicron BA.1 variant (alterations: A67V, Δ69/70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F),the Omicron BA.2 variant (alterations: T19I, Δ24-26, A27S, G142D, V213G, G339D, S371F, S373P, S375F, T376A, D405N, R408S, K417N, N440K, S477N, T478K, E484A, Q493R, Q498R, N501Y, Y505H, D614G, H655Y, N679K, P681H, N764K, D796Y, Q954H, N969K), the Omicron BA.2.12.1 variant (alterations: T19I, Δ24-26, A27S, G142D, V213G, G339D, S371F, S373P, S375F, T376A, D405N, R408S, K417N, N440K, L452Q, S477N, T478K, E484A, Q493R, Q498R, N501Y, Y505H, D614G, H655Y, N679K, P681H, S704L, N764K, D796Y, Q954H, N969K), the Omicron BA.4/5 variant (alterations: T19I, Δ24-26, A27S, Δ69/70, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, N969K), or an artificial Omicron BA.1-BA.4/5 hybrid S glycoprotein (alterations: A67V, Δ69/70, T95I, G142D, Δ143-145, Δ211, L212I, ins214EPE, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, N969K) according to published pseudotyping protocols (Ref. 3). A diagram of SARS-CoV-2 S glycoprotein alterations is shown in Fig. 48 and a separate alignment of S glycoprotein alterations in Omicron sub-lineages is displayed in Fig. 33.

In brief, HEK293T/17 monolayers (ATCC^{®} CRL-11268^{™}) cultured in Dulbecco's modified Eagle's medium (DMEM) with GlutaMAX^{™} (Gibco) supplemented with 10% heat-inactivated fetal bovine serum (FBS [Sigma-Aldrich]) (referred to as medium) were transfected with Sanger sequencing-verified SARS-CoV-1 or variant-specific SARS-CoV-2 S expression plasmid with Lipofectamine LTX (Life Technologies) following the manufacturer's instructions. At 24 hours after transfection, the cells were infected at a multiplicity of infection (MOI) of three with VSV-G complemented VSVΔG vector. After incubation for 2 hours at 37 °C with 7.5% CO₂, cells were washed twice with phosphate buffered saline (PBS) before medium supplemented with anti-VSV-G antibody (clone 8G5F11, Kerafast Inc.) was added to neutralize residual VSV-G-complemented input virus. VSV-SARS-CoV-2-S pseudotype-containing medium was harvested 20 hours after inoculation, passed through a 0.2 µm filter (Nalgene) and stored at -80 °C. The pseudovirus batches were titrated on Vero 76 cells (ATCC^{®} CRL-1587^{™}) cultured in medium. The relative luciferase units induced by a defined volume of a SARS-CoV-2 wild-type strain S glycoprotein pseudovirus reference batch previously described in Muik et al., 2021 (Ref. 4), that corresponds to an infectious titer of 200 transducing units (TU) per mL, was used as a comparator. Input volumes for the SARS-CoV-2 variant pseudovirus batches were calculated to normalize the infectious titer based on the relative luciferase units relative to the reference.

### Pseudovirus neutralization assay

Vero 76 cells were seeded in 96-well white, flat-bottom plates (Thermo Scientific) at 40,000 cells/well in medium 4 hours prior to the assay and cultured at 37 °C with 7.5% CO₂. Each individual serum was serially diluted 2-fold in medium with the first dilution being 1:5 (SARS-CoV-2-naïve triple BNT162b2 vaccinated; dilution range of 1:5 to 1:5,120) or 1:30 (triple vaccinated after subsequent Omicron BA.1 or BA.2 breakthrough infection; dilution range of 1:30 to 1:30,720). In the case of the SARS-CoV-1 pseudovirus assay, the serum of all individuals was initially diluted 1:5 (dilution range of 1:5 to 1:5,120). VSV-SARS-CoV-2-S/VSV-SARS-CoV-1-S particles were diluted in medium to obtain 200 TU in the assay. Serum dilutions were mixed 1:1 with pseudovirus (n=2 technical replicates per serum per pseudovirus) for 30 minutes at room temperature before being added to Vero 76 cell monolayers and incubated at 37 °C with 7.5% CO₂ for 24 hours. Supernatants were removed and the cells were lysed with luciferase reagent (Promega). Luminescence was recorded on a CLARIOstar^{®} Plus microplate reader (BMG Labtech), and neutralization titers were calculated as the reciprocal of the highest serum dilution that still resulted in 50% reduction in luminescence. For depletion studies resolution with regards to neutralization titers was increased, in order to discriminate smaller than 2-fold differences on an individual serum level. Neutralization titers were determined by generating a 4-parameter logistical (4PL) fit of the percent neutralization at each serial serum dilution. The 50% pseudovirus neutralization (pVN₅₀) titer was reported as the interpolated reciprocal of the dilution yielding a 50% reduction in luminescence. Results for all pseudovirus neutralization experiments were expressed as geometric mean titers (GMT) of duplicates. If no neutralization was observed, an arbitrary titer value of half of the limit of detection [LOD] was reported. SARS-CoV-2 wild-type strain, and Alpha, Beta, Delta, BA.1, BA.4/5 VOC, as well as SARS-CoV-1 pseudovirus neutralizing GMTs for the SARS-CoV-2 naïve BNT162b2 triple-vaccinated cohort and the triple-vaccinated BA.1 convalescent cohort were previously reported in Quandt. et al. (Ref. 10). Only the BA.2.12.1 neutralization data was newly generated from serum samples for this study.

### Live SARS-CoV-2 neutralization assay

SARS-CoV-2 virus neutralization titers were determined by a microneutralization assay based on cytopathic effect (CPE) at VisMederi S.r.l., Siena, Italy. In brief, heat-inactivated serum samples from individuals were serially diluted 1:2 (starting at 1:10; n=2 technical replicates per serum per virus) and incubated for 1 hour at 37 °C with 100 TCID50 of the live wild-type-like SARS-CoV-2 virus strain 2019-nCOV/ITALY-INMI1 (GenBank: MT066156), Alpha virus strain nCoV19 isolate/England/MIG457/2020 (alterations: Δ69/70, Δ144, N501Y, A570D, D614G, P681H, T716I, S982A, D1118H), Beta virus strain nCoV19 isolate/England ex-SA/HCM002/2021 (alterations: D80A, D215G, Δ242-244, K417N, E484K, N501Y, D614G, A701V), sequence-verified Delta strain isolated from a nasopharyngeal swab (alterations: T19R, G142D, E156G, Δ157/158, L452R, T478K, D614G, P681R, R682Q, D950N), Omicron BA.1 strain hCoV-19/Belgium/rega-20174/2021 (alterations: A67V, Δ69/70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F), sequence-verified Omicron BA.2 strain (alterations:T19I, Δ24-26, A27S, V213G, G339D, S371F, S373P, S375F, T376A, D405N, R408S, K417N, S477N, T478K, E484A, Q493R, Q498R, N501Y, Y505H, D614G, H655Y, N679K, P681H, R682W, N764K, D796Y, Q954H, N969K), or sequence-verified Omicron BA.4 strain (alterations: V3G, T19I, Δ24-26, A27S, Δ69/70, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, N969K) to allow any antigen-specific antibodies to bind to the virus. A diagram of S glycoprotein alterations is shown in Fig. 48. The 2019-nCOV/ITALY-INMI1 strain S glycoprotein is identical in sequence to the wild-type SARS-CoV-2 S (Wuhan-Hu-1 isolate). Vero E6 (ATCC^{®} CRL-1586^{™}) cell monolayers were inoculated with the serum/virus mix in 96-well plates and incubated for 3 days (2019-nCOV/ITALY-INMI1 strain) or 4 days (Alpha, Beta, Delta, Omicron BA.1, BA.2 and BA.4 variant strain) to allow infection by non-neutralized virus. The plates were observed under an inverted light microscope and the wells were scored as positive for SARS-CoV-2 infection (i.e., showing CPE) or negative for SARS-CoV-2 infection (i.e., cells were alive without CPE). The neutralization titer was determined as the reciprocal of the highest serum dilution that protected more than 50% of cells from CPE and reported as GMT of duplicates. If no neutralization was observed, an arbitrary titer value of 5 (half of the LOD) was reported.

### Depletion of RBD- or NTD-binding antibodies from human sera

SARS-CoV-2 wild-type strain S glycoprotein RBD- and NTD-coupled magnetic beads (Acro Biosystems, Cat.no. MBS-K002 and MBS-K019; 40 µg RBD/mg beads and 38 µg NTD/mg beads, respectively) were prepared according to the manufacturer's instructions. Beads were resuspended in ultrapure water at 1 mg beads/mL and a magnet was used to collect and wash the beads with PBS. Beads were resuspended in serum to obtain 20 µg RBD- or NTD-bait per 100 µL serum. A mock depletion (undepleted control) was performed for each serum by adding 0.5 mg Biotin-saturated MyOne^{™} Streptavidin T1 Dynabeads^{™} (ThermoFisher, Cat.no. 65601) per 100 µL serum. Beads were incubated with human sera for 1 hour with gentle rotation. A magnet was used to separate bead-bound antibodies from the depleted supernatant. Depleted and undepleted sera were analyzed for cross-neutralization capacity using pseudovirus neutralization assays. Depletion efficacy for both RBD- and NTD-binding antibodies was determined by a multiplexed electrochemiluminescence immunoassay (Meso Scale Discovery, V-Plex SARS-CoV-2 Panel 1 Kit, Cat. No. K15359U-2).

### Statistical analysis

The statistical method of aggregation used for the analysis of antibody titers is the geometric mean and for the ratio of SARS-CoV-2 VOC titer and wild-type strain titer the geometric mean and the corresponding 95% confidence interval. The use of the geometric mean accounts for the non-normal distribution of antibody titers, which span several orders of magnitude. The Friedman test with Dunn's correction for multiple comparisons was used to conduct pairwise signed-rank tests of group geometric mean neutralizing antibody titers with a common control group. Spearman correlation was used to evaluate the monotonic relationship between non-normally distributed datasets. All statistical analyses were performed using GraphPad Prism software version 9.

### References for Example 17

1. WHO Technical Advisory Group on SARS-CoV-2 Virus Evolution (TAG-VE): Classification of Omicron (B.1.1.259): SARS-CoV-2 Variant of Concern. (2021).
2. WHO Headquarters (HQ), WHO Health Emergencies Programme, Enhancing Response to Omicron SARS-CoV-2 variant: Technical brief and priority actions for Member States. (2022).
3. M. Hoffmann et al., The Omicron variant is highly resistant against antibody-mediated neutralization: Implications for control of the COVID-19 pandemic. Cell 185, 447-456 e411 (2022).
4. W. Dejnirattisai et al., SARS-CoV-2 Omicron-B.1.1.529 leads to widespread escape from neutralizing antibody responses. Cell 185, 467-484 e415 (2022).
5. V. Servellita et al., Neutralizing immunity in vaccine breakthrough infections from the SARS-CoV-2 Omicron and Delta variants. Cell 185, 1539-1548 e1535 (2022).
6. C. Kurhade et al., Neutralization of Omicron BA.1, BA.2, and BA.3 SARS-CoV-2 by 3 doses of BNT162b2 vaccine. Nat Commun 13, 3602 (2022).
7. Y. Cao et al., Omicron escapes the majority of existing SARS-CoV-2 neutralizing antibodies. Nature 602, 657-663 (2022).
8. WHO Technical Advisory Group on COVID-19 Vaccine Composition (TAG-CO-VAC): Interim statement on the composition of current COVID-19 vaccines. (2022).
9. M. E. McMenamin et al., Vaccine effectiveness of one, two, and three doses of BNT162b2 and CoronaVac against COVID-19 in Hong Kong: a population-based observational study. Lancet Infect Dis, (2022).
10. J. Quandt et al., Omicron BA.1 breakthrough infection drives cross-variant neutralization and memory B cell formation against conserved epitopes. Sci Immunol, eabq2427 (2022).
11. E. Callaway, What Omicron's BA.4 and BA.5 variants mean for the pandemic. Nature 606, 848-849 (2022).
12. H. Tegally et al., Emergence of SARS-CoV-2 Omicron lineages BA.4 and BA.5 in South Africa. Nat Med, (2022).
13. S. Xia, L. Wang, Y. Zhu, L. Lu, S. Jiang, Origin, virological features, immune evasion and intervention of SARS-CoV-2 Omicron sublineages. Signal Transduct Target Ther 7, 241 (2022).
14. European Centre for Disease Prevention and Control, Weekly COVID-19 country overview - Country overview report: week 27 2022 (2022).
15. Y. Cao et al., BA.2.12.1, BA.4 and BA.5 escape antibodies elicited by Omicron infection. Nature, (2022).
16. N. P. Hachmann et al., Neutralization Escape by SARS-CoV-2 Omicron Subvariants BA.2.12.1, BA.4, and BA.5. N Engl J Med 387, 86-88 (2022).
17. T. N. Starr, A. J. Greaney, A. S. Dingens, J. D. Bloom, Complete map of SARS-CoV-2 RBD mutations that escape the monoclonal antibody LY-CoV555 and its cocktail with LY-CoV016. Cell Rep Med 2, 100255 (2021).
18. A. Tuekprakhon et al., Antibody escape of SARS-CoV-2 Omicron BA.4 and BA.5 from vaccine and BA.1 serum. Cell 185, 2422-2433 e2413 (2022).
19. Q. Wang et al., Antibody evasion by SARS-CoV-2 Omicron subvariants BA.2.12.1, BA.4, & BA.5. Nature, (2022).
20. Y. Wang et al., Structural basis for SARS-CoV-2 Delta variant recognition of ACE2 receptor and broadly neutralizing antibodies. Nat Commun 13, 871 (2022).
21. P. Wang et al., Antibody resistance of SARS-CoV-2 variants B.1.351 and B.1.1.7. Nature 593, 130-135 (2021).
22. L. Liu et al., Striking antibody evasion manifested by the Omicron variant of SARS-CoV-2. Nature 602, 676-681 (2022).
23. C. I. Kaku et al., Recall of pre-existing cross-reactive B cell memory following Omicron BA.1 breakthrough infection. Sci Immunol, eabq3511 (2022).
24. A. Muik et al., Neutralization of SARS-CoV-2 Omicron by BNT162b2 mRNA vaccine-elicited human sera. Science 375, 678-680 (2022).
25. A. Muik et al., Neutralization of SARS-CoV-2 lineage B.1.1.7 pseudovirus by BNT162b2 vaccine-elicited human sera. Science 371, 1152-1153 (2021).
26. C. W. Tan et al., Pan-Sarbecovirus Neutralizing Antibodies in BNT162b2-Immunized SARS-CoV-1 Survivors. N Engl J Med 385, 1401-1406 (2021).
27. R. Nutalai et al., Potent cross-reactive antibodies following Omicron breakthrough in vaccinees. Cell 185, 2116-2131 e2118 (2022).
28. P. Arora et al., Augmented neutralisation resistance of emerging omicron subvariants BA.2.12.1, BA.4, and BA.5. Lancet Infect Dis, (2022).
29. E. Andreano et al., Anatomy of Omicron BA.1 and BA.2 neutralizing antibodies in COVID-19 mRNA vaccinees. Nat Commun 13, 3375 (2022).
30. N. Andrews et al., Covid-19 Vaccine Effectiveness against the Omicron (B.1.1.529) Variant. N Engl J Med 386, 1532-1546 (2022).
31. S. Y. Tartof et al., Immunocompromise and durability of BNT162b2 vaccine against severe outcomes due to omicron and delta variants. Lancet Respir Med 10, e61-e62 (2022).

### Example 18: Exposure to BA.4/BA.5 Spike glycoprotein drives pan-Omicron neutralization in vaccine-experienced humans and mice

The present Example is an extension of Examples 7, 13, 16, and 17, and describes (i) immune response data from human subjects previously vaccinated against SARS-CoV-2 who have had a BA.4/5-breakthrough infection (in the present example, human subjects who have previously been administered three doses of RNA vaccine(s) encoding the SARS-CoV-2 S protein of a Wuhan variant), and (ii) further mouse data, showing immune responses induced by RNA encoding SARS-CoV-2 S proteins comprising mutations characteristic of certain Omicron variants.

### Abstract

The SARS-CoV-2 Omicron variant and its sublineages show pronounced viral escape from neutralizing antibodies owing to numerous amino acid alterations within the spike (S) glycoprotein. As demonstrated in the previous Examples, breakthrough infection of vaccinated individuals with Omicron sublineages BA.1 and BA.2 has been associated with distinct patterns of cross-neutralizing activity against SARS-CoV-2 variants of concern (VOCs), most notably against the recently emerged BA.2 descendants Omicron BA.4 and BA.5. Here, the effect of Omicron BA.4/BA.5 S glycoprotein exposure on the magnitude and breadth of the neutralizing antibody response is investigated in vaccine-experienced humans and mice. The immune sera from triple mRNA-vaccinated individuals with Omicron BA.4/BA.5 breakthrough infection is shown to display broad and robust neutralizing activity against Omicron BA.1, BA.2, BA.2.12.1, and BA.4/BA.5. Administration of a BA.4/BA.5-adapted mRNA booster vaccine to mice following SARS-CoV-2 wild-type (Wuhan) strain-based primary immunization is associated with similarly broad neutralizing activity. Immunization of vaccine-naïve mice with a bivalent mRNA vaccine encoding BA.4/BA.5-adapted and wild-type-based S glycoprotein immunogens is further shown to induce strong and broad neutralizing activity against Omicron and non-Omicron VOCs. These findings support the conclusion that, when administered as monovalent or bivalent boosters, Omicron BA.4/BA.5-adapted vaccines (e.g., RNA encoding a SARS-CoV-2 S protein comprising mutations characteristic of these variants) can lead to improved neutralization breadth against current and newly emerging VOCs (improved, in particular, as compared to a BA.1-adapted vaccine). These findings also show that a BA.4/BA.5 vaccine, when administered in a bivalent format has the potential to confer protection to subjects with no pre-existing immunity against SARS-CoV-2 (e.g., to exposure-naïve subjects).

### Introduction

The SARS-CoV-2 Omicron variant of concern (VOC) has had a major impact on the epidemiological landscape of the COVID-19 pandemic since its emergence in November 2021 (Refs. 1-2). Significant alterations in the spike (S) glycoprotein of the initial Omicron variant BA.1 led to the loss of many neutralizing antibody epitopes (Ref. 3) and rendered BA.1 capable of partially escaping previously established SARS-CoV-2 wild-type strain (Wuhan-Hu-1)-based immunity (Refs. 4-6). Hence, breakthrough infections of vaccinated individuals with Omicron are more common than with previous VOCs. While Omicron BA.1 was displaced by the BA.2 variant in many countries around the globe, other variants such as BA.1.1 and BA.3 temporarily and/or locally gained momentum but did not become globally dominant (Refs. 7-9). Omicron BA.2.12.1 displaced BA.2 to become dominant in the United States in the interim, whereas BA.4 and BA.5 displaced BA.2 in Europe, parts of Africa, and Asia/Pacific (Refs. 8, 10-12. Currently the VOCs Omicron BA.4 and BA.5 are predominant in large parts of the world, including the United States where they eventually displaced BA.2.12.1 (Ref. 13).

Omicron lineage VOCs have acquired numerous alterations (amino acid exchanges, insertions, or deletions) in the S glycoprotein, among which some are shared between all Omicron VOCs while others are specific to one or more sublineages (see Fig. 33). Antigenically, BA.2.12.1 exhibits high similarity with BA.2 but not BA.1, whereas BA.4 and BA.5 differ considerably from BA.2 and even more so from BA.1, in line with their genealogy (Ref. 14). Major differences of BA.1 from the remaining Omicron VOCs include Δ143-145, L212I, or ins214EPE in the S glycoprotein N-terminal domain and G446S or G496S in the receptor binding domain (RBD). Amino acid changes T376A, D405N, and R408S in the RBD are in turn common to BA.2 and its descendants but not found in BA.1. In addition, some alterations are specific for certain BA.2-descendant VOCs, including L452Q for BA.2.12.1 or L452R and F486V for BA.4 and BA.5. Most of these shared and sublineage-specific alterations were shown to play an important role in immune escape from monoclonal antibodies and polyclonal sera raised against the wild-type S glycoprotein (Ref. 15).

As described in the previous Examples, breakthrough infection with Omicron BA.1 or BA.2 of individuals immunized with mRNA vaccines or an inactivated virus vaccine has been associated with potent neutralizing activity against Omicron BA.1, BA.2 and previous VOCs (see also, Refs. 16-21). A considerable boost of Omicron BA.2.12.1 and BA.4/BA.5 neutralization relative to SARS-CoV-2 naïve vaccinee sera was only evident in BA.2 breakthrough cases, with titers against BA.4/BA.5 being lower than those against the remaining VOCs (see also Refs. 19-21). Administration of an Omicron BA.1-adapted booster to mice after wild-type strain-based primary immunization has previously been shown to enhance neutralizing activity against BA.1 compared to a wild-type booster (see previous Example 16 and Ref. 22). Preliminary analyses of a Phase 2 clinical trial have also shown that sera from SARS-CoV-2 naïve individuals vaccinated with an Omicron BA.1-adapted mRNA vaccine as a 4^{th} dose showed significantly increased neutralizing responses against BA.1 compared to sera from individuals boosted with the prototypic mRNA vaccine (Ref. 23). However, the BA.1-adapted booster was not associated with increased BA.4/BA.5 cross-neutralization, as responses against BA.4/BA.5 were approximately three-fold and five-fold lower than responses against BA.1 and the prototypic strain, respectively, in sera from both BA.1 and prototype-boosted individuals.

Given the current predominance of the highly contagious VOCs Omicron BA.4 and BA.5 in large parts of the globe, the present Example was desiged to investigate whether exposure to an Omicron BA.4/BA.5 S glycoprotein would trigger a broader neutralizing antibody response against relevant Omicron sublineages. The breadth of neutralizing activity was tested against Omicron VOCs in immune sera from vaccinated individuals with Omicron BA.4/BA.5 breakthrough infection and in sera from mice that received Omicron BA.4/BA.5-adapted booster vaccines following primary immunization with BNT162b2. In addition, the breadth of neutralizing activity was evaluated in sera from mice immunized with Omicron BA.4/BA.5-adapted vaccines without prior exposure to the wild-type S glycoprotein. The data described in the present Example provides further insight into Omicron immune escape mechanisms and the effects of immunization on variant cross-neutralization, and thus is useful for guiding selection of vaccination strategies.

### Results

### Study design, cohorts, and sampling

The effect of Omicron BA.4/BA.5 breakthrough infection in individuals vaccinated with three doses of mRNA COVID-19 vaccine (BNT162b2/mRNA-1273 homologous or heterologous regimens) (Fig. 49(A)) and of Omicron BA.4/BA.5-adapted booster vaccination of BNT162b2 pre-immunized mice on the breadth of neutralizing activity in immune sera (Fig. 49(B)) was investigated. In addition, the effect of primary immunization with Omicron BA.4/BA.5-adapted vaccines was investigated in naïve mice (i.e., mice without previous exposure to a SARS-CoV-2 S protein, see Fig. 49(C)).

For the breakthrough infection study, serum samples were collected from triple mRNA vaccinated individuals who subsequently experienced a breakthrough infection with Omicron BA.4 or BA.5 (mRNA-Vax³ + BA.4/BA.5, n=17, Fig. 53). Three cohorts were included for reference: triple mRNA vaccinated individuals with a breakthrough infection with Omicron BA.2 (mRNA-Vax³ + BA.2, n=19) or with BA.1 (mRNA-Vax³ + BA.2, n=14), and BNT162b2 triple-vaccinated individuals who were SARS-CoV-2-naïve at the time of sampling (BNT162b2³, n=18, Fig. 53). Sera were derived the study previously described in Examples 7, 13, and 17.

### Omicron BA.4/BA.5 breakthrough infection of triple mRNA-vaccinated individuals results in pan-Omicron neutralizing activity

Neutralizing activity of immune sera was tested in a well-characterized pseudovirus neutralization test (pVNT) (Refs. 24-25) by determining 50% pseudovirus neutralization (pVN₅₀) geometric mean titers (GMTs) with pseudoviruses bearing the S glycoproteins of the SARS-CoV-2 wild-type strain or Omicron BA.1, BA.2, and the BA.2-derived sublineages BA.2.12.1, BA.4 and BA.5. As BA.4 and BA.5 are identical in their S glycoprotein sequence, in the context of pVNT, BA.4/5 is used. In addition, SARS-CoV was assayed (herein referred to as SARS-CoV-1) to detect potential pan-Sarbecovirus neutralizing activity (Ref. 26). As an orthogonal test system, a live SARS-CoV-2 neutralization test (VNT) was also used that analyzes neutralization during multicycle replication of authentic virus (SARS-CoV-2 wild-type strain and Omicron VOCs BA.1, BA.2, and BA.4) with immune serum present during the entire test period.

In the pVNT assay, sera from the Omicron BA.4/BA.5 breakthrough infection cohort (mRNA-Vax³ + BA.4/BA.5) robustly neutralized the wild-type strain and all tested Omicron VOCs (Fig. 50(A)). The pVN₅₀ GMTs against Omicron BA.2 and BA.2.12.1 pseudoviruses were within a 2-fold range of the GMT against the wild-type strain (GMTs 613 against Omicron vs. GMT 1085 against wild-type). Neutralization of BA.1 and BA.4/5 (GMTs 500-521) was broadly similar to that of BA.2, and the reduction relative to the wild-type strain significant (p<0.05) yet also within a ^{~}2-fold range. The GMT against SARS-CoV-1 was significantly lower (p<0.0001; >50-fold lower than wild-type).

To compare mRNA-Vax³ + BA.4/BA.5 to the reference cohorts with Omicron BA.1 or BA.2 breakthrough infection (mRNA-Vax³ + BA.1 and mRNA-Vax³ + BA.2) and SARS-CoV-2 naïve triple BNT162b2-vaccinated individuals (BNT162b2³), the VOC pVNso GMTs was normalized against the wild-type strain to allow for assessment of neutralization breadth irrespective of the magnitude of antibody titers, which expectedly differs between triple-vaccinated individuals with a breakthrough infection and triple-vaccinated individuals without infection (Refs. 16 and 21). While BNT162b2³ sera mediated considerable cross-neutralization of Omicron BA.1 and BA.2, breakthrough infection with Omicron BA.1 and BA.2 was associated with higher cross-neutralization of the respective homologous strains (Fig 50(B)). Cross-neutralization of BA.2.12.1, and especially BA.4/5, was less effective in the mRNA-Vax³ + BA.1 cohort (GMT ratios 0.43 and 0.18, respectively) compared to mRNA-Vax³ + BA.4/BA.5 (GMT ratios 0.57and 0.48). Cross-neutralization of BA.2.12.1 and BA.4/5 was less reduced in the mRNA-Vax³ + BA.2 cohort (GMT ratios 0.53 and 0.37, respectively). Surprisingly, in the reciprocal case, cross-neutralization of Omicron BA.1 and BA.2 was maintained at comparatively higher levels in the mRNA-Vax³ + BA.4/BA.5 cohort (GMT ratios 0.46 and 0.57, respectively). Hence, BA.4/BA.5 breakthrough infection resulted in the most efficient cross-neutralization across all tested VOCs (GMT ratios ≥0.46) of all cohorts evaluated.

The authentic live SARS-CoV-2 virus neutralization assay largely confirmed the major pVNT assay findings shown in Fig. 50(A)-(B). 50% virus neutralization (VNso) GMTs in Omicron BA.4/BA.5 breakthrough sera against BA.2 and BA.4 were comparable (i.e., within a 1.5-fold range) to that against the wild-type strain (Fig. 50(C)). Reduction of BA.1 neutralization was significant (p<0.01) yet within a 2.5-fold range. The GMTs normalized against the wild-type strain showed robust cross-neutralization of Omicron BA.1, BA.2, and BA.4 by mRNA-Vax³ + BA.4/BA.5 sera (GMT ratio ≥0.40), whereas BA.4 cross-neutralization was considerably less efficient in mRNA-Vax³ + BA.1 (GMT ratio 0.20) and mRNA-Vax³ + BA.2 (GMT ratio 0.39) sera (Fig. 50(D)) (Refs. 16, 21). Hence, the findings in both the pVNT and the VNT assay system showed that Omicron BA.4/BA.5 breakthrough infection was associated with broad neutralizing activity against all Omicron sublineages tested.

### Booster immunization with an Omicron BA.4/BA.5 S glycoprotein adapted mRNA vaccine drives pan-Omicron neutralization in BNT162b2 double-vaccinated mice

The heightened neutralization breadth seen after Omicron BA.4/BA.5 breakthrough infection suggested that variant-adapted vaccines based on the Omicron BA.4/5 S glycoprotein sequence can elicit a recall response with broader cross-neutralization than those based on Omicron BA.1. To test this hypothesis, booster studies were performed in BNT162b2-preimmunized mice (Fig. 49(B)). Mice were administered a primary series of two immunizations with BNT162b2 on days 0 and 21 and a third dose of either BNT162b2 (1 µg), or a BNT162b2-derived variant-adapted vaccine encoding Omicron BA.1 or BA.4/BA.5 S glycoprotein (Fig. 54) on day 104. The adapted vaccines were either administered as monovalent vaccines encoding Omicron BA.1 or BA.4/5 S glycoprotein (1 µg), or bivalent vaccines comprising BNT162b2 and the Omicron BA.1 or BA.4/5 S glycoprotein adapted vaccine (0.5 µg each). Comparable RNA purity and integrity, and expression of antigens in *vitro* were confirmed for BNT162b2 and Omicron-adapted vaccines (Fig. 55(A)-(B)).

Neutralizing titers against pseudoviruses expressing the wild-type strain, Omicron BA.1, BA.2, BA.2.12.1, or BA.4/5 S glycoprotein were determined in pVNT assays using sera drawn before the booster (day 104, pre-D3) and on days 7, 21, and 35 after the booster (d7D3, d21D3 and d35D3). The live SARS-CoV-2 neutralization test was used as an orthogonal test system to confirm the observed pseudovirus neutralizing activity post-boost on d21D3 and d35D3.

Baseline immunization of the mice was assessed by determination of SARS-CoV-2 pseudovirus neutralizing activity in sera drawn on pre-D3. pVNso GMTs of the groups dedicated for the various boosters were comparable, i.e., within a range of 3-fold difference (Fig. 56). pVN₅₀ GMTs against Omicron BA.1 and BA.2 were 3 to 11-fold lower than those against the wild-type strain (GMT ratios ≤ 0.32, Fig. 56(B)). GMTs against BA.2.12.1 and BA.4/5 were 10 to 25-fold lower than those against wild-type (GMT ratios ≤ 0.10).

On d7D3, neutralizing GMTs had increased substantially across groups and against all tested variants (Fig. 57) with peak titers reached on d21D3 (Fig. 51). In sera from BNT162b2 boosted mice, strong neutralizing activity against the wild-type strain was observed, whereas pVN₅₀ GMTs against Omicron variants were substantially lower (Fig. 51(A)). The Omicron BA.1 booster led to comparable neutralization of BA.1 and the wild-type strain, while the pVN₅₀ GMTs against the remaining VOCs were considerably lower. In particular, GMTs against BA.4/5 were reduced 13-fold compared to those against wild-type. In contrast, administration of the BA.4/5 booster resulted in broad neutralizing activity against all Omicron variants, with pVN₅₀ GMTs comparable (within a 1.5-fold range) to that against the wild-type strain. Sera from mice that received the BNT162b2/BA.1 bivalent booster had a high pVN₅₀ GMT against the wild-type strain, and robust neutralization of Omicron BA.1, whereas the GMTs against BA.2 and its descendants were slightly lower. The BNT162b2/Omicron BA.4/5 bivalent booster gave rise to high titers against the wild-type strain, comparable to the BNT162b2 monovalent booster. Omicron neutralization was broadly comparable across all sublineages (within a 2-fold range), with pVN₅₀ GMTs modestly below that against the wild-type strain.

To quantify the booster effect of the third dose of vaccine variants on neutralization of individual VOCs, the fold-changes in pVN₅₀ GMTs detected on d21D3 was evaluated relative to the baseline GMTs determined before administration of the third dose. BNT162b2 comparably increased neutralization of all tested variants (pVN₅₀ GMTs 6 to 10-fold higher than at baseline), whereas the most pronounced effect of the BA.1 booster was detected for the neutralization of the homologous VOC (26-fold increase) (Fig. 51(B)). The BA.4/5 booster strongly potentiated neutralizing activity against BA.2.12.1 and BA.4/5 (>120-fold) and also had a substantial effect on BA.1 and BA.2 neutralization (increases of 34 and 37-fold, respectively). The BNT162b2/BA.1 and BNT162b2/BA.4/5 bivalent vaccines showed patterns of potentiation similar to the BA.1 and BA.4/5 monovalent vaccines, respectively, albeit with less focused increases in neutralization against the homologous VOCs.

To compare the groups with regard to neutralization breadth irrespective of the magnitude, the VOC pVN₅₀ GMTs was normalized against the wild-type strain. The GMT ratios showed that the Omicron BA.4/5 booster vaccine mediated pan-Omicron neutralization (≥0.65 for all tested variants) (Fig. 51(C)). In contrast, the BA.1 booster vaccine was beneficial for neutralization of BA.1 (GMT ratio 0.77), whereas ratios for BA.2 (0.39) and especially for BA.2.12.1 and BA.4/5 (≤0.16) were substantially lower. The bivalent BNT162b2/BA.4/5 vaccine also mediated broad neutralizing activity with enhanced cross-neutralization of BA.2, BA.2.12.1 and BA.4/5 compared to the BNT162b2/BA.1 bivalent vaccine, albeit with lower GMT ratios (between 0.27 and 0.46) than the BA.4/5 monovalent vaccine.

Again, the authentic live SARS-CoV-2 virus neutralization assay largely confirmed the major pVNT assay findings shown in Fig. 51(A)-(C). Sera from mice administered the BA.4/5 booster dose robustly neutralized all the variants tested (Fig. 51(D)), thereby confirming the capacity of this approach to mediate pan-Omicron neutralization (GMT ratios ≥0.39) (Fig. 51(E)). While neutralization of Omicron variants in sera from mice boosted with the BNT162b2/BA.4/5 bivalent vaccine was lower compared to the BA.4/5 monovalent vaccine, both BA.4/5-containing vaccines exhibited stronger cross-neutralization of the Omicron variants than the BNT162b2/BA.1 vaccine, and even more so than the monovalent BA.1 vaccine and BNT162b2.

While absolute titers were slightly lower on d7D3 compared to d21D3, the Omicron BA.4/5 vaccine mediated a similarly broad neutralizing activity against all variants (Fig. 57). BNT162b2/BA.4/5 bivalent booster mediated comparable neutralization breadth (GMT ratios ≥0.35), whereas sera from all other booster groups exhibited considerably lower cross-neutralization of BA.2, BA.2.12.1 (and BA.4/5). Similarly, later analysis of sera on d35D3 showed substantial potentiation of neutralizing activity against Omicron variants by the BA.4/5 vaccine booster compared to baseline, resulting in pan-Omicron neutralization (Fig. 58(A)-(C)). The bivalent BNT162b2/BA.4/5 booster also exhibited broad neutralization across all variants tested, whereas the BA.1 and the BNT162b2/BA.1 vaccines manifested with lower capacity of cross-neutralization, especially against BA.2.12.1 and BA.4/5. Similarly, in the VNT assay, sera from both groups boosted with BA.4/5-containing vaccines exhibited substantially stronger cross-neutralization of all tested Omicron variants compared to the BA.1-containing vaccines and BNT162b2 (Fig. 58(D)-(E)).

These mouse booster results were consistent with those observed in humans with BA.4/5 breakthrough infections, described above, and further suggest that a booster with an Omicron BA.4/5 S glycoprotein adapted vaccine following primary immunization with a wild-type strain-based vaccine can elicit pan-Omicron neutralizing activity superior in breadth to a BA.1 S glycoprotein-based booster.

### Immunization with an Omicron BA.4/BA.5 S glycoprotein adapted mRNA vaccine drives pan-Omicron neutralization in previously unvaccinated mice

Next, experiments were performed to better understand the neutralizing capacity of serum samples after immunization with the Omicron-adapted vaccine in mice with no pre-existing immune response against SARS-CoV-2. Naïve mice were immunized twice at day 0 and day 21 with either BNT162b2, with BA.1 or BA.4/5 S glycoprotein adapted monovalent vaccines, or the bivalent BNT162b2/BA.4/5 vaccine (Fig. 49(C)). Neutralizing titers against pseudoviruses expressing the wild-type strain S glycoprotein, that of the previous VOCs Alpha or Delta, or Omicron BA.1, BA.2, BA.2.12.1, or BA.4/5 were determined in pVNT assays using sera drawn 14 days after the second immunization (d14D2). In sera from BNT162b2 immunized mice, strong neutralizing activity against the wild-type strain was observed. In these sera, robust neutralizing activity against Alpha and Delta was also detected (within a 4-fold range of wild-type), whereas pVN₅₀ GMTs against Omicron variants were substantially (14 to 37-fold) lower than against wild-type (Fig. 52). Immunization with the Omicron BA.1 monovalent vaccine led to high neutralization of BA.1. In these sera, robust neutralization of Omicron BA.2 and BA.2.12.1 was also detected (within a 3-fold range of BA.1), while the pVN₅₀ GMTs against the wild-type strain and remaining VOCs were considerably (7 to 32-fold) lower than BA.1. Immunization with the Omicron BA.4/5 monovalent vaccine led to high neutralization of BA.4/5. In these sera, robust neutralization of Omicron BA.2 and BA.2.12.1 was also detected (within a 2.5-fold range of BA.4/5), while the pVNso GMTs against the wild-type strain and remaining VOCs were considerably (14 to >42-fold lower than BA.4/5. Immunization with the BNT162b2/BA.4/5 bivalent vaccine resulted in high neutralizing activity against BA.4/5. In contrast to the other vaccines, robust neutralizing activity against the wild-type strain and all remaining VOCs (within a 6-fold range of BA.4/5) were also detected in these sera.

These results show that a monovalent vaccine in naïve animals (e.g., animals that have not been previously administered against and/or infected with SARS-CoV-2) can induce a high neutralizing antibody response mostly in a variant-specific manner but can lose potency when testing against more distant variants. In contrast, a bivalent vaccine can induce strong and broad neutralizing antibody responses in naïve animals.

### Discussion

In the present Example, BA.4/BA.5 breakthrough infection of triple-mRNA vaccinated individuals is associated with robust neutralization of all currently or previously predominant Omicron subvariants, i.e., pan-Omicron neutralization is observed in BA.4/5-breakthrough patients. These findings are consistent with a recent report showing strong cross-neutralization of Omicron BA.1, BA.2, as well as Beta and Delta in sera from individuals vaccinated with BNT162b2 or an adenovirus-based vaccine and subsequent BA.4 breakthrough infection (Ref. 27). In line with those observations in humans, pan-Omicron neutralizing activity was also observed in the sera of mice that received an Omicron BA.4/5 booster vaccine following primary immunization with BNT162b2, whereas an Omicron BA.1 boost induced strongly reduced neutralizing antibody titers against BA.4/BA.5. A bivalent BNT162b2/BA.4/5 boost elicited broad Omicron neutralization, albeit less pronounced than the BA.4/5 monovalent booster. Together, these findings provide further understanding on how breakthrough infections or vaccine boosters adapted to Omicron VOCs in a mono- or bivalent format shape immunity and suggest that exposure to Omicron BA.4/5 S glycoprotein may confer heightened protection against the currently circulating and potential future Omicron sublineage VOCs. The finding that immunization of naïve mice with the BNT162b2/BA.4/5 bivalent vaccine elicits strong neutralizing antibody responses against the wild-type strain as well as Omicron and non-Omicron VOCs suggest that this bivalent approach can confer broad protection to unvaccinated individuals not previously infected SARS-CoV-2 (e.g., young pediatric patients), and thus may be particularly suitable for these individuals.

While currently approved SARS-CoV-2 wild-type strain-based vaccines such as BNT162b2 have proven effective at protecting against severe disease (Refs. 28-30), prevention of transmission remains a significant challenge as new variants continue to emerge that are antigenically distant from the wild-type strain (Refs. 16-18, 20). The data described in the present Example suggest that a mono- or bivalent BA.4/BA.5 S glycoprotein adapted booster vaccine (e.g., a mono- or bivalent vaccine described herein), can confer higher benefit against the highly prevalent BA.4 and BA.5 VOCs than a vaccine based on a previously dominant Omicron sublineage such as BA.1. Given their predominance in many regions around the world and their high transmissibility (see, e.g., Refs., 8, 10, 11, and 13), it is possible that new variants with further growth advantage will emerge from Omicron BA.4 or BA.5 that retain partial or full susceptibility to an Omicron BA.4/BA.5-adapted vaccine. Boosting pre-existing immunity with an Omicron BA.4/5 S glycoprotein-based adapted vaccine could therefore represent a suitable strategy to address the current pandemic situation, while close monitoring of virus evolution and epidemiological landscapes remains instrumental for guidance on potential further vaccine adaptations in response to emerging threats.

### Materials and Methods

### Human study design, recruitment of participants and sample collection

The objective of the study described in the present Example was to investigate the effect of Omicron BA.4/BA.5 breakthrough infection on the cross-variant neutralization capacity of human sera. Neutralizing activity was assessed in immune sera from triple-mRNA (BNT162b2/mRNA-1273)-vaccinated individuals with a confirmed subsequent SARS-CoV-2 breakthrough infection, which either occurred in a period of Omicron BA.4/BA.5 lineage-dominance in Germany (mid-June to mid-July 2022;) or was variant-confirmed (BA.4 or BA.5) by genome sequencing (mRNA-Vax³ + BA.4/5) (Fig. 53). The neutralizing activity was compared to that in immune sera from triple mRNA vaccinated individuals with a confirmed subsequent SARS-CoV-2 breakthrough infection in a period of Omicron BA.2 lineage-dominance (March to May 2022; mRNA-Vax³ + BA.2), a period of Omicron BA.1 lineage-dominance in Germany (November 2021 to mid-January 2022; mRNA-Vax³ + BA.1) (Refs. 1, 2), and triple-BNT162b2-vaccinated individuals that were SARS-CoV-2-naïve (nucleocapsid seronegative) at the time of sample collection (BNT162b2³). Serum neutralizing capability was characterized using pseudovirus and live SARS-CoV-2 neutralization assays. Data for the reference cohorts mRNA-Vax³ + BA.2, mRNA-Vax³ + BA.1, and BNT162b2³ were previously published (Refs. 16, 21). Participants from the mRNA-Vax³ + Omi BA.4/5, mRNA-Vax³ + Omi BA.2, and mRNA-Vax³ + BA.1 cohorts were recruited from University Hospital, Goethe University Frankfurt as part of a non-interventional study (protocol approved by the Ethics Board of the University Hospital [No. 2021-560]) researching patients that had experienced Omicron breakthrough infection following vaccination for COVID-19. Individuals from the BNT162b2³ cohort provided informed consent as part of their participation in the Phase 2 trial BNT162-17 (NCT05004181).

The infections of 5 BA.4/5 and 4 BA.1 convalescent participants in this study were confirmed by genome sequencing (Ref. 16).

All participants had no documented history of SARS-CoV-2 infection prior to vaccination. Participants were free of symptoms at the time of blood collection.

Serum was isolated by centrifugation of drawn blood at 2000 x g for 10 minutes and cryopreserved until use.

### In vitro transcription and lipid-nanoparticle (LNP) formulation of the RNA

The BNT162b2 vaccine was designed on a background of S sequences from SARS-CoV-2 isolate Wuhan-Hu-1 (GenBank: MN908947.3) with pre-fusion conformation-stabilizing K986P and V987P mutations. Omicron BA.1 and Omicron BA.4/5 vaccine candidates were designed based on BNT162b2 including sequence changes as shown in Fig. 54. RNA production as well as formulation were performed as described elsewhere (see, e.g., Ref. 32 the contents of which are incorporated herein by reference in their entirety). In brief, DNA templates were cloned into a plasmid vector with backbone sequence elements (T7 promoter, 5' and 3' UTR, 100 nucleotide poly(A) tail) interrupted by a linker (A30LA70, 10 nucleotides) for improved RNA stability and translational efficiency (see, e.g., Refs. 33, 34), amplified via PCR and purified. RNA was *in vitro* transcribed by T7 RNA polymerase in the presence of a trinucleotide cap1 analogue ((m27,3'-O)Gppp(m2'-O)ApG; TriLink) and with N1-methylpseudouridine-5'-triphosphate (m1ΨTP; Thermo Fisher Scientific) replacing uridine-5'-triphosphate (UTP) (Ref. 35). RNA was purified using magnetic particles (Ref. 36) and RNA integrity was assessed by microfluidic capillary electrophoresis (Agilent 2100 Bioanalyzer) and all three RNAs show single sharp peaks resulting in comparable and high purity as well as integrity (Fig. 55(A)). In addition, the concentration, pH, osmolality, endotoxin level and bioburden of the solution were determined.

One ionizable lipid ((4-hydroxybutyl)azanediyl)bis(hexane-6,1-diyl)bis(2-hexyldecanoate)), two structural lipids (1,2-distearoyl-sn-glycero-3-phosphocholine [DSPC] and cholesterol) and one PEGylated lipid (2-[(polyethylene glycol)-2000]-N,N-ditetradecylacetamide) were used for formulation of RNA. After transfer into an aqueous buffer system via diafiltration, the LNP compositions were analyzed ensuring but not limited to high RNA integrity and encapsulation efficacy, as well as a particle size below 100nm. The vaccine candidates were stored at -70 to -80 °C at a concentration of 0.5 mg/mL till time point of usage.

### In vitro expression of RNAs and vaccines

HEK293T cells were transfected with 0.15 µg BNT162b2 or Omicron-adapted vaccines (lipid-nanoparticle-formulated), or with vaccine RNAs using RiboJuice^{™} mRNA Transfection Kit (Merck Millipore) according to the manufacturer's instructions and incubated for 18 h. Transfected HEK293T cells were stained with Fixable Viability Dye (eBioscience) and incubated with mFc-tagged recombinant human ACE-2 (Sino Biological). A secondary donkey anti-mouse antibody conjugated with AF647 was used for detection of surface expression. Cells were fixed (Fixation Buffer, Biolegend) prior to flow cytometry analysis using a FACSCelesta flow cytometer (BD Biosciences, BD FACSDiva software version 8.0.1) and FlowJo software version 10.6.2 (FlowJo, BD Biosciences).

### Mouse studies

All mouse studies were performed at BioNTech SE, and protocols were approved by the local authorities (local welfare committee) and conducted according to Federation of European Laboratory Animal Science Associations recommendations. Study execution and housing were in compliance with the German Animal Welfare Act and Directive 2010/63/EU. Mice were kept in individually ventilated cages with a 12-h light/dark cycle, controlled environmental conditions (22 ± 2 °C, 45% to 65% relative humidity) and under specific-pathogen-free conditions. Food and water were available ad libitum. Only mice with an unobjectionable health status were selected for testing procedures.

For immunization, female BALB/c mice (Janvier) (9-21 weeks old) were randomly allocated to groups. BNT162b2 and Omicron-based vaccines candidates were diluted in 0.9% NaCl and 1 µg of the vaccine candidate was injected into the gastrocnemius muscle at a volume of 20 µl under isoflurane anesthesia. For the mouse booster study, mice were immunized twice (day 0 and 21) with BNT162b2. Third immunization with BNT162b2 and Omicron-based vaccines candidates occurred at day 104 after study start and mice were bled shortly before third immunization and as indicated in Fig. 49. For the naïve moue study, animals were immunized at day 0 and 21 with BNT162b2 and Omicron-based vaccines candidates. 14 days after second immunization, blood was withdrawn. Peripheral blood was collected from the vena facialis without anesthesia. Final bleeding was performed under isoflurane anesthesia from the retro-orbital venous plexus. For serum generation, blood was centrifuged for 5 min at 16,000g and the serum was immediately used for downstream assays or stored at -20 °C till timepoint of usage.

### VSV-SARS-CoV-2 S variant pseudovirus generation

A recombinant replication-deficient vesicular stomatitis virus (VSV) vector that encodes green fluorescent protein (GFP) and luciferase instead of the VSV-glycoprotein (VSV-G) was pseudotyped with SARS-CoV-1 S glycoprotein (UniProt Ref: P59594) and with SARS-CoV-2 S glycoprotein derived from either the wild-type strain (Wuhan-Hu-1, NCBI Ref: 43740568), the Alpha variant (alterations: Δ69/70, Δ144, N501Y, A570D, D614G, P681H, T716I, S982A, D1118H), the Delta variant (alterations: T19R, G142D, E156G, Δ157/158, K417N, L452R, T478K, D614G, P681R, D950N), the Omicron BA.1 variant (alterations: A67V, Δ69/70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F), the Omicron BA.2 variant (alterations: T19I, Δ24-26, A27S, G142D, V213G, G339D, S371F, S373P, S375F, T376A, D405N, R408S, K417N, N440K, S477N, T478K, E484A, Q493R, Q498R, N501Y, Y505H, D614G, H655Y, N679K, P681H, N764K, D796Y, Q954H, N969K), the Omicron BA.2.12.1 variant (alterations: T19I, Δ24-26, A27S, G142D, V213G, G339D, S371F, S373P, S375F, T376A, D405N, R408S, K417N, N440K, L452Q, S477N, T478K, E484A, Q493R, Q498R, N501Y, Y505H, D614G, H655Y, N679K, P681H, S704L, N764K, D796Y, Q954H, N969K), and the Omicron BA.4/5 variant (alterations: T19I, Δ24-26, A27S, Δ69/70, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, N969K) according to published pseudotyping protocols (e.g., as described in Ref. 3, the contents of which are incorporated by reference herein in their entirety). A diagram of SARS-CoV-2 S glycoprotein alterations is shown in Fig. 32 and a separate alignment of S glycoprotein alterations in Omicron sublineages is displayed in Fig. 33.

In brief, HEK293T/17 monolayers (ATCC^{®} CRL-11268^{™}) cultured in Dulbecco's modified Eagle's medium (DMEM) with GlutaMAX^{™} (Gibco) supplemented with 10% heat-inactivated fetal bovine serum (FBS [Sigma-Aldrich]) (referred to as medium) were transfected with Sanger sequencing-verified SARS-CoV-1 or variant-specific SARS-CoV-2 S expression plasmid with Lipofectamine LTX (Life Technologies) following the manufacturer's instructions. At 24 hours after transfection, the cells were infected at a multiplicity of infection (MOI) of three with VSV-G complemented VSVΔG vector. After incubation for 2 hours at 37 °C with 7.5% CO₂, cells were washed twice with phosphate buffered saline (PBS) before medium supplemented with anti-VSV-G antibody (clone 8G5F11, Kerafast Inc.) was added to neutralize residual VSV-G-complemented input virus. VSV-SARS-CoV-2-S pseudotype-containing medium was harvested 20 hours after inoculation, passed through a 0.2 µm filter (Nalgene) and stored at -80 °C. The pseudovirus batches were titrated on Vero 76 cells (ATCC^{®} CRL-1587^{™}) cultured in medium. The relative luciferase units induced by a defined volume of a SARS-CoV-2 wild-type strain S glycoprotein pseudovirus reference batch previously described in Muik et al., 2021 (Ref. 31), that corresponds to an infectious titer of 200 transducing units (TU) per mL, was used as a comparator. Input volumes for the SARS-CoV-2 variant pseudovirus batches were calculated to normalize the infectious titer based on the relative luciferase units relative to the reference.

### Pseudovirus neutralization assay

Vero 76 cells were seeded in 96-well white, flat-bottom plates (Thermo Scientific) at 40,000 cells/well in medium 4 hours prior to the assay and cultured at 37 °C with 7.5% CO₂. Human and mouse serum samples were 2-fold serially diluted in medium with dilutions ranging from 1:5 to 1:30,720 (human sera), from 1:40 to 1:102,400 for mouse booster study (mouse sera; starting dilution was 1:40 [pre-D3], 1:200 [d7D3]) as well as 1:100 [d21D3, d35D3]) and in the naïve setting (mouse sera; monovalent vaccinated groups starting dilution 1:120 to 1:15,360 and bivalent vaccinated groups starting 1:100). VSV-SARS-CoV-2-S/VSV-SARS-CoV-1-S particles were diluted in medium to obtain 200 TU in the assay. Serum dilutions were mixed 1:1 with pseudovirus (n=2 technical replicates per serum per pseudovirus) for 30 minutes at room temperature before being added to Vero 76 cell monolayers and incubated at 37 °C with 7.5% CO₂ for 24 hours. Supernatants were removed and the cells were lysed with luciferase reagent (Promega). Luminescence was recorded on a CLARIOstar^{®} Plus microplate reader (BMG Labtech), and neutralization titers were calculated as the reciprocal of the highest serum dilution that still resulted in 50% reduction in luminescence. Results for all pseudovirus neutralization experiments were expressed as geometric mean titers (GMT) of duplicates. If no neutralization was observed, an arbitrary titer value of half of the limit of detection [LOD] was reported. Tables of the neutralization titers in human sera are provided.

### Live SARS-CoV-2 neutralization assay

SARS-CoV-2 virus neutralization titers were determined by a microneutralization assay based on cytopathic effect (CPE) at VisMederi S.r.l., Siena, Italy. In brief, human and mouse serum samples were serially diluted 1:2 (n=2 technical replicates per serum per virus; starting at 1:10 for human samples and starting at 1:100 [post-boost, day 125] or at 1:50 [post-boost, day 139] for murine samples) and incubated for 1 hour at 37 °C with 100 TCID₅₀ of the live wild-type-like SARS-CoV-2 virus strain 2019-nCOV/ITALY-INMI1 (GenBank: MT066156), Omicron BA.1 strain hCoV-19/Belgium/rega-20174/2021 (alterations: A67V, Δ69/70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F), sequence-verified Omicron BA.2 strain (alterations:T19I, Δ24-26, A27S, V213G, G339D, S371F, S373P, S375F, T376A, D405N, R408S, K417N, S477N, T478K, E484A, Q493R, Q498R, N501Y, Y505H, D614G, H655Y, N679K, P681H, R682W, N764K, D796Y, Q954H, N969K), or sequence-verified Omicron BA.4 strain (alterations: V3G, T19I, Δ24-26, A27S, Δ69/70, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, N969K) to allow any antigen-specific antibodies to bind to the virus. A diagram of S glycoprotein alterations is shown in Fig. 48. The 2019-nCOV/ITALY-INMI1 strain S glycoprotein is identical in sequence to the wild-type SARS-CoV-2 S (Wuhan-Hu-1 isolate). Vero E6 (ATCC^{®} CRL-1586^{™}) cell monolayers were inoculated with the serum/virus mix in 96-well plates and incubated for 3 days (2019-nCOV/ITALY-INMI1 strain) or 4 days (Omicron BA.1, BA.2 and BA.4 variant strain) to allow infection by non-neutralized virus. The plates were observed under an inverted light microscope and the wells were scored as positive for SARS-CoV-2 infection (i.e., showing CPE) or negative for SARS-CoV-2 infection (i.e., cells were alive without CPE). The neutralization titer was determined as the reciprocal of the highest serum dilution that protected more than 50% of cells from CPE and reported as GMT of duplicates. If no neutralization was observed, an arbitrary titer value of 5 (half of the LOD) was reported.

### Statistical analysis

The statistical method of aggregation used for the analysis of antibody titers is the geometric mean and for the ratio of SARS-CoV-2 VOC titer and wild-type strain titer the geometric mean and the corresponding 95% confidence interval. The use of the geometric mean accounts for the non-normal distribution of antibody titers, which span several orders of magnitude. The Friedman test with Dunn's correction for multiple comparisons was used to conduct pairwise signed-rank tests of group geometric mean neutralizing antibody titers with a common control group. Spearman correlation was used to evaluate the monotonic relationship between non-normally distributed datasets. All statistical analyses were performed using GraphPad Prism software version 9.

### References for Example 18

1. WHO Technical Advisory Group on SARS-CoV-2 Virus Evolution (TAG-VE): Classification of Omicron (B.1.1.259): SARS-CoV-2 Variant of Concern. (2021).
2. WHO Headquarters (HQ), WHO Health Emergencies Programme, Enhancing Response to Omicron SARS-CoV-2 variant: Technical brief and priority actions for Member States. (2022).
3. M. Hoffmann et al., The Omicron variant is highly resistant against antibody-mediated neutralization: Implications for control of the COVID-19 pandemic. Cell 185, 447-456 e411 (2022).
4. V. Servellita et al., Neutralizing immunity in vaccine breakthrough infections from the SARS-CoV-2 Omicron and Delta variants. Cell 185, 1539-1548 e1535 (2022).
5. C. Kurhade et al., Neutralization of Omicron BA.1, BA.2, and BA.3 SARS-CoV-2 by 3 doses of BNT162b2 vaccine. Nat Commun 13, 3602 (2022).
6. Y. Cao et al., Omicron escapes the majority of existing SARS-CoV-2 neutralizing antibodies. Nature 602, 657-663 (2022).
7. S. Xia, L. Wang, Y. Zhu, L. Lu, S. Jiang, Origin, virological features, immune evasion and intervention of SARS-CoV-2 Omicron sublineages. Signal Transduct Target Ther 7, 241 (2022).
8. H. Gruell et al., SARS-CoV-2 Omicron sublineages exhibit distinct antibody escape patterns. Cell Host Microbe, (2022).
9. Y. Fan et al., SARS-CoV-2 Omicron variant: recent progress and future perspectives. Signal Transduct Target Ther 7, 141 (2022).
10. H. Tegally et al., Emergence of SARS-CoV-2 Omicron lineages BA.4 and BA.5 in South Africa. Nat Med, (2022).
11. European Centre for Disease Prevention and Control, Weekly COVID-19 country overview - Country overview report: week 31 2022 (2022).
12. J. Hadfield et al., Nextstrain: real-time tracking of pathogen evolution https://nextstrain.org/ncov/gisaid. Bioinformatics 34, 4121-4123 (2018).
13. Centers for Disease Control and Prevention. COVID Data Tracker. Atlanta, GA: US Department of Health and Human Services, CDC; 2022, August 12. https://covid.cdc.gov/covid-data-tracker. (2022).
14. A. Z. Mykytyn et al., Antigenic cartography of SARS-CoV-2 reveals that Omicron BA.1 and BA.2 are antigenically distinct. Sci Immunol, eabq4450 (2022).
15. P. Wang et al., Antibody resistance of SARS-CoV-2 variants B.1.351 and B.1.1.7. Nature 593, 130-135 (2021).
16. J. Quandt et al., Omicron BA.1 breakthrough infection drives cross-variant neutralization and memory B cell formation against conserved epitopes. Sci Immunol, eabq2427 (2022).
17. C. I. Kaku et al., Recall of pre-existing cross-reactive B cell memory following Omicron BA.1 breakthrough infection. Sci Immunol, eabq3511 (2022).
18. Y. Cao et al., BA.2.12.1, BA.4 and BA.5 escape antibodies elicited by Omicron infection. Nature, (2022).
19. P. Arora et al., Augmented neutralisation resistance of emerging omicron subvariants BA.2.12.1, BA.4, and BA.5. Lancet Infect Dis, (2022).
20. Q. Wang et al., Antibody evasion by SARS-CoV-2 Omicron subvariants BA.2.12.1, BA.4, & BA.5. Nature, (2022).
21. A. Muik et al., Omicron BA.2 breakthrough infection enhances cross-neutralization of BA.2.12.1 and BA.4/BA.5. bioRxiv, (2022).
22. P. Du et al., A bivalent vaccine containing D614G and BA.1 spike trimer proteins or a BA.1 spike trimer protein booster shows broad neutralizing immunity. J Med Virol 94, 4287-4293 (2022).
23. A. R. Branche et al., SARS-CoV-2 Variant Vaccine Boosters Trial: Preliminary Analyses. medRxiv, (2022).
24. A. Muik et al., Neutralization of SARS-CoV-2 lineage B.1.1.7 pseudovirus by BNT162b2 vaccine-elicited human sera. Science 371, 1152-1153 (2021).
25. A. Muik et al., Neutralization of SARS-CoV-2 Omicron by BNT162b2 mRNA vaccine-elicited human sera. Science 375, 678-680 (2022).
26. C. W. Tan et al., Pan-Sarbecovirus Neutralizing Antibodies in BNT162b2-Immunized SARS-CoV-1 Survivors. N Engl J Med 385, 1401-1406 (2021).
27. S. I. Richardson et al., SARS-CoV-2 BA.4 infection triggers more cross-reactive neutralizing antibodies than BA.1. bioRxiv, (2022).
28. S. Y. Tartof et al., Immunocompromise and durability of BNT162b2 vaccine against severe outcomes due to omicron and delta variants. Lancet Respir Med 10, e61-e62 (2022).
29. N. Andrews et al., Covid-19 Vaccine Effectiveness against the Omicron (B.1.1.529) Variant. N Engl J Med 386, 1532-1546 (2022).
30. F. P. Polack et al., Safety and Efficacy of the BNT162b2 mRNA Covid-19 Vaccine. N Engl J Med 383, 2603-2615 (2020).
31. W. Dejnirattisai et al., SARS-CoV-2 Omicron-B.1.1.529 leads to widespread escape from neutralizing antibody responses. Cell 185, 467-484 e415 (2022).
32. A. B. Vogel et al., BNT162b vaccines protect rhesus macaques from SARS-CoV-2. Nature 592, 283-289 (2021).
33. A. G. Orlandini von Niessen et al., Improving mRNA-Based Therapeutic Gene Delivery by Expression-Augmenting 3' UTRs Identified by Cellular Library Screening. Mol Ther 27, 824-836 (2019).
34. S. Holtkamp et al., Modification of antigen-encoding RNA increases stability, translational efficacy, and T-cell stimulatory capacity of dendritic cells. Blood 108, 4009-4017 (2006).
35. E. Grudzien-Nogalska et al., Synthetic mRNAs with superior translation and stability properties. Methods Mol Biol 969, 55-72 (2013).
36. S. Berensmeier, Magnetic particles for the separation and purification of nucleic acids. Appl Microbiol Biotechnol 73, 495-504 (2006).

### Example 19: Influence of BA.4/BA.5 Breakthrough Infections on Omicron Immune

### Responses

The present Example 19 is an extension of Examples 14, 17, and 18, and describes experiments in which serum samples collected from BA.1-, BA.2-, and BA.4/5-breakthrough cases were analyzed for neutralization activity against Omicron BA.4, BA.5, BA.4.6, BF.7, and BA.2.75 variants. In addition to confirming the results described in the previous Examples, the present Example 19 also provides further characterization of antibody responses induced by BA.1-, BA.2-, and BA.4/5-breakthrough infections in relation to BA.4.6, BF.7, and BA.2.75 variants. The present Example further demonstrates that a BA.4/5 breakthrough infection can produce a broader neutralization response than BA.1- and BA.2-breakthrough infections, with superior neutralization titers induced against BA.4/5 and BA.4.6/BF.7 variants as compared to BA.1- and BA.2- breakthrough infections, and comparable neutralization titers against a BA.2.75 virus.

Continued evolution of the SARS-CoV-2 Omicron variant has led to the emergence of numerous sublineages with distinct patterns of neutralizing antibody evasion. The recently emerged sublineages BA.4.6, BF.7, and BA.2.75 have raised concerns as their prevalence slowly but steadily increases in many regions. The present Example investigated neutralizing activity against these Omicron sublineages as well as the currently dominant BA.4/BA.5 in immune sera of COVID-19 mRNA triple-vaccinated individuals with subsequent breakthrough infection with BA.1, BA.2, or BA.4/BA.5 SARS-CoV-2 variants. Sera from SARS-CoV-2 naïve triple- or quadruple mRNA-vaccinated individuals were also tested. Sera from BA.1, BA.2, and BA.4/BA.5 convalescents were broadly comparable in their proficiency to cross-neutralize BA.2.75 relative to the SARS-CoV-2 wild-type strain. The strongest cross-neutralization of Omicron BA.4.6/BF.7 was detected in BA.4/BA.5 convalescent sera. These findings indicate that breakthrough infection with recent Omicron sublineages may confer at least partial protection against infection with newly emerging sublineages, and provides further evidence supporting the efficacy of a BA.4/5-specific vaccine (in particular, further evidence that a BA.4/5-specific vaccine can provide a broad immune response against Omciron variants). The SARS-CoV-2 Omicron variant of concern (VOC) contains over 30 amino acid alterations in its spike (S) glycoprotein, which mediate partial escape from previously established immunity. As a result, breakthrough infections with Omicron have been more frequent than with previously circulating VOCs among vaccinated populations. Since Omicron's first emergence in November 2021, sublineages BA.1, BA.2, BA.4, and BA.5 consecutively dominated the pandemic landscape. While BA.5 has been the globally dominant sublineage since mid-2022, virus evolution continues to give rise to new sublineages that harbor additional amino acid alterations in their S glycoprotein. Descendants of previous Omicron sublineages with slow but steady increases in prevalence have been reported in many countries across continents. These descendants include Omicron BA.2.75, BA.4.6, and BF.7, which have emerged from BA.2, BA.4, and BA.5, respectively.

Omicron BA.2.75 contains five amino acid alterations within the N-terminal domain (NTD) of the S protein that distinguishes it from previous Omicron Variants of Concern (VOCs), including its parental sublineage BA.2 (Fig. 60). In addition, BA.2.75 has three alterations in its receptor-binding domain (RBD) that are not found in BA.2, of which G446S is shared with BA.1. The most prevalent strains of Omicron BA.4.6 and BF.7 have identical S glycoprotein sequences, which show high similarity with their respective parental lineages BA.4 and BA.5, which also share their S glycoprotein sequence. A single R346T alteration within the RBD differentiates BA.4.6/BF.7 from BA.4/BA.5, and abrogates neutralization by the therapeutic monoclonal antibody (mAb) cilgavimab (Ref. 1). The combination of cilgavimab and tixagevimab (Evusheld^{™}), which has high clinical relevance for pre-exposure COVID-19 prophylaxis in immunocompromised patients, is expected to be ineffective against Omicron BA.4.6 and BF.7 given that tixagevimab lacks neutralizing activity against Omicron BA.4/BA.5 and their descendants (Refs. 1 and 2).

Primary SARS-CoV-2 immunity in many parts of the world is based on infections with the original wild-type virus or wild-type strain-based vaccines, such as the mRNA vaccines BNT162b2 and mRNA-1273. Immune responses are commonly further shaped through breakthrough infections with antigenically distinct Omicron sublineage viruses or recently authorized Omicron-adapted vaccine boosters. An important question is whether breakthrough infections and Omicron-adapted vaccine boosters elicit substantial neutralizing antibody responses against recently emerged Omicron sublineages currently gaining momentum.

The present Example investigated neutralizing activity against Omicron BA.4.6/BF.7 and BA.2.75 from immune sera isolated from five cohorts of individuals who received three or four doses of mRNA COVID-19 vaccines (BNT162b2/mRNA-1273 homologous or heterologous regimens), with or without subsequent breakthrough infections with Omicron sublineage variants. Individual cohorts were BNT162b2 triple-vaccinated SARS-CoV-2-naïve individuals (BNT162b2³; n=18), BNT162b2 quadruple-vaccinated SARS-CoV-2-naïve elderly individuals (BNT162b2⁴; n=15), and triple mRNA-vaccinated individuals who experienced breakthrough infection with Omicron BA.1 (mRNA-Vax³ + BA.1; n=14), BA.2 (mRNA-Vax³ + BA.2, n=19), or BA.4/BA.5 (mRNA-Vax³ + BA.4/BA.5, n=17) (Fig 61). The study design includes cohorts that are thought to represent a large proportion of the European and North American population, given the local health authorities' recommendation of a fourth vaccine dose for elderly individuals and the high frequency of breakthrough infections with Omicron compared to previous variants. Serum neutralizing activity was tested in a well-characterized pseudovirus neutralization test (pVNT) (Refs. 3-5) by determining 50% pseudovirus neutralization (pVN₅₀) geometric mean titers (GMTs) with pseudoviruses bearing the S glycoproteins of the SARS-CoV-2 wild-type strain or Omicron BA.4/BA.5 (BA.4 and BA.5 are identical in their S glycoprotein sequence), BA.4.6/BF.7 (BA.4.6 and BF.7 are identical in their S glycoprotein sequence), or BA.2.75.

In the BNT162b2³, BNT162b2⁴, and mRNA-Vax³ + BA.1 cohorts, pVNso titers against Omicron BA.4/BA.5 were significantly lower (GMTs reduced 5 to 6-fold) than titers against the wild-type strain (Fig. 59(A)). The reduction of BA.4/BA.5 neutralization in the mRNA-Vax³ + BA.2 and mRNA-Vax³ + BA.4/BA.5 cohorts was also statistically significant yet less pronounced (GMTs 2 to 3-fold lower compared to wild-type) than in the other cohorts. pVN₅₀ titers against Omicron BA.4.6/BF.7 were further significantly reduced compared to BA.4/BA.5 in mRNA-Vax³ + BA.2 (GMT 239 vs. 386; P<0.05). A considerable but statistically non-significant reduction in BA.4.6/BF.7 neutralization relative to BA.4/BA.5 was also seen in BNT162b2⁴ (GMT 55 vs. 121). For all other cohorts, Omicron BA.4.6/BF.7 GMTs were largely comparable to those against Omicron BA.4/BA.5 and titers in the mRNA-Vax³ + BA.4/BA.5 were the most robust (GMT 443). In mRNA-Vax³ + BA.4/BA.5, titers against BA.2.75 were reduced 1.8-fold compared to BA.4/BA.5 (GMT 295 vs. 521), while neutralizing GMTs against BA.2.75 were even increased 2-fold compared to those against BA.4/BA.5 in mRNA-Vax³ + BA.1 (GMT 525 vs. 263). Titers against BA.2.75 were comparable to those against BA.4/5 in the other cohorts.

To allow for assessment of neutralization breadth irrespective of the magnitude of antibody titers, the Omicron sublineage pVN₅₀ GMTs were normalized against the wild-type strain. GMT ratios for all Omicron subvariant pseudoviruses were comparable in the BNT162b2³ and BNT162b2⁴ cohorts (ratios ≤0.22 for all pseudoviruses, Fig. 59(B)), indicating that a fourth dose of BNT162b2 did not improve cross-neutralization of the tested subvariants despite a slight overall increase in antibody titers. Cross-neutralization of BA.4/BA.5 and BA.4.6/BF.7 were significantly (p<0.05) stronger in sera from mRNA-Vax³ + BA.2 compared to BNT162b2³ (GMT ratios 0.37 vs 0.17 for BA.4/BA.5, and 0.23 vs 0.12 for BA.4.6/BF.7). The GMT ratios were even higher in the mRNA-Vax³ + BA.4/BA.5 cohort for both the BA.4/BA.5 pseudovirus (GMT ratio 0.48, p<0.01 versus BNT162b2³) and the BA.4.6/BF.7 pseudovirus (0.41, p<0.0001). Cross-neutralization of the Omicron BA.2.75 pseudovirus was broadly comparable in most cohorts, with a significant (p<0.05) increase seen only in mRNA-Vax³ + BA.1 compared to BNT162b2³.

The finding that neutralizing activity against Omicron BA.4.6/BF.7 is further reduced compared to BA.4/BA.5 in sera of vaccinated individuals and BA.2 convalescents suggests that the R346T alteration mediates further escape from neutralizing antibodies in polyclonal sera. Convergent evolution of the RBD at this site in Omicron BA.4.6, BF.7, and additional currently less prevalent strains (Ref. 1) indicates that the resulting immune escape may confer considerable growth advantage. The findings summarized here indicate that Omicron BA.4/BA.5 breakthrough infection refocuses neutralizing antibody responses to partially restore BA.4.6/BF.7 neutralization. A similar cross-neutralization pattern of the Omicron lineage in BA.4/BA.5 breakthrough infected humans and in mice boosted with an Omicron BA.4/5-adapted vaccine has also been observed, as demonstrated in Example 18. Hence, such findings provide evidence supporting that Omicron BA.4/5-adapted vaccine boosters can also elicit relevant neutralizing antibody responses against BA.4.6/BF.7 in humans.

The observation that cross-neutralization of Omicron BA.2.75 is broadly comparable to that of BA.4/5 is consistent with previous reports (Refs. 2, 6, and 7). Without wishing to be bound by a particular theory, this observation suggests that the growth advantage of BA.2.75 over BA.5 may be related to factors other than immune evasion. For example, in some embodiments, minor differences in the susceptibility of Omicron BA.2.75 and BA.4/BA.5 to neutralization by BA.1 and BA.4/BA.5 convalescent sera may point towards amino acid alterations with a potential context-dependent role in immune evasion.

The investigation summarized in the present Example focused on neutralization of the new Omicron sublineages BA.4.6/BF.7, and BA.2.75, as these sublineages could potentially displace BA.5 in the future according to their current trajectory. The finding that Omicron breakthrough infection is associated with enhanced cross-neutralization of the new sublineages is consistent with the data provided in the previous Examples, showing enhanced neutralization breadth, including against earlier Omicron sublineages and previous SARS-CoV-2 VOCs . Together these findings show that Omicron BA.4/BA.5 breakthrough infection is associated with the broadest neutralization against all variants, including Omicron sublineages, providing evidence supporting that vaccination with RNA encoding a SARS-CoV-2 S protein comprising mutations characteristic of the BA.4/5 variant can produce a broad neutralization response.

### Materials and Methods

### Study design, recruitment of participants and sample collection

The objective of this study was to investigate the cross-neutralizing activity of five different panels of sera against Omicron BA.4.6/BF.7 and BA.2.75 sub-lineages compared to SARS-CoV-2 wild-type and Omicron BA.4/BA.5. Neutralizing activity was assessed in immune sera from (i) SARS-CoV-2-naïve triple-BNT162b2-vaccinated younger adult individuals (BNT162b2³), (ii) SARS-CoV-2-naïve quadruple-BNT162b2-vaccinated older adult individuals (BNT162b2⁴), and triple-mRNA (BNT162b2/mRNA-1273)-vaccinated individuals with a confirmed subsequent SARS-CoV-2 breakthrough infection which either occurred (iii) in a period of Omicron BA.1 lineage-dominance (November 2021 to mid-January 2022; mRNA-Vax³ + BA.1), (iv) in a period of Omicron BA.2 lineage-dominance (March to May 2022; mRNA-Vax³ + BA.2), or (v) in a period of Omicron BA.4/BA.5 lineage-dominance in Germany (mid-June to mid-July 2022; mRNA-Vax³ + BA.4/5). Serum neutralizing capability was characterized using pseudovirus neutralization assays. SARS-CoV-2 wild-type and Omicron BA.4/BA.5 neutralization data for cohorts BNT162b2³, mRNA-Vax³ + BA.1, mRNA-Vax³ + BA.2, mRNA-Vax³ + BA.4/5 were previously published.

Participants from the mRNA-Vax³ + Omi BA.1, mRNA-Vax³ + Omi BA.2, and mRNA-Vax³ + BA.4/5 cohorts were recruited from University Hospital, Goethe University Frankfurt as part of a non-interventional study (protocol approved by the Ethics Board of the University Hospital [No. 2021-560]) researching patients that had experienced Omicron breakthrough infection following vaccination for COVID-19. Individuals from the BNT162b2³ and BNT162b2⁴ cohort provided informed consent as part of their participation in the Phase 2 trial BNT162-17 (NCT05004181) and BNT162-16 Substudy F (NCT04955626), respectively. All participants had no documented history of SARS-CoV-2 infection prior to vaccination. Participants were free of symptoms at the time of blood collection.

Serum was isolated by centrifugation of drawn blood at 2000 x g for 10 minutes and cryopreserved until use.

A recombinant replication-deficient vesicular stomatitis virus (VSV) vector that encodes green fluorescent protein (GFP) and luciferase instead of the VSV-glycoprotein (VSV-G) was pseudotyped with SARS-CoV-1 S glycoprotein (UniProt Ref: P59594) or with SARS-CoV-2 S glycoprotein derived from either the wild-type strain (Wuhan-Hu-1, NCBI Ref: 43740568), the Omicron BA.4/BA.5 variant (alterations: T19I, Δ24-26, A27S, Δ69/70, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, N969K), the Omicron BA.4.6/BF.7 variant (alterations: T19I, Δ24-26, A27S, Δ69/70, G142D, V213G, G339D, R346T, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, N969K), and the Omicron BA.2.75 variant (alterations: T19I, Δ24-26, A27S, G142D, K147E, W152R, F157L, I210V, V213G, G257S, G339H, S371F, S373P, S375F, T376A, D405N, R408S, K417N, N440K, G446S, N460K, S477N, T478K, E484A, Q498R, N501Y, Y505H, D614G, H655Y, N679K, P681H, N764K, D796Y, Q954H, N969K) according to published pseudotyping protocols (Berger Rentsch, Marianne, and Gert Zimmer. "A vesicular stomatitis virus replicon-based bioassay for the rapid and sensitive determination of multi-species type I interferon." PloS one 6.10 (2011): e25858). A diagram of SARS-CoV-2 S glycoprotein alterations is shown in Fig. 62 and a separate alignment of S glycoprotein alterations in Omicron VOCs is displayed in Fig. 60.

In brief, HEK293T/17 monolayers (ATCC^{®} CRL-11268^{™}) cultured in Dulbecco's modified Eagle's medium (DMEM) with GlutaMAX^{™} (Gibco) supplemented with 10% heat-inactivated fetal bovine serum (FBS [Sigma-Aldrich]) (referred to as medium) were transfected with Sanger sequencing-verified variant-specific SARS-CoV-2 S expression plasmid with Lipofectamine LTX (Life Technologies) following the manufacturer's instructions. At 24 hours after transfection, the cells were infected at a multiplicity of infection (MOI) of three with VSV-G complemented VSVΔG vector. After incubation for 2 hours at 37 °C with 7.5% CO₂, cells were washed twice with phosphate buffered saline (PBS) before medium supplemented with anti-VSV-G antibody (clone 8G5F11, Kerafast Inc.) was added to neutralize residual VSV-G-complemented input virus. VSV-SARS-CoV-2-S pseudotype-containing medium was harvested 20 hours after inoculation, passed through a 0.2 µm filter (Nalgene) and stored at -80 °C. The pseudovirus batches were titrated on Vero 76 cells (ATCC^{®} CRL-1587^{™}) cultured in medium. The relative luciferase units induced by a defined volume of a SARS-CoV-2 wild-type strain S glycoprotein pseudovirus reference batch previously described in Muik et al., 2021 (Muik, Alexander, et al. "Neutralization of SARS-CoV-2 lineage B. 1.1. 7 pseudovirus by BNT162b2 vaccine-elicited human sera." Science 371.6534 (2021): 1152-1153), that corresponds to an infectious titer of 200 transducing units (TU) per mL, was used as a comparator. Input volumes for the SARS-CoV-2 variant pseudovirus batches were calculated to normalize the infectious titer based on the relative luciferase units relative to the reference.

### Pseudovirus neutralization assay

Vero 76 cells were seeded in 96-well white, flat-bottom plates (Thermo Scientific) at 40,000 cells/well in medium 4 hours prior to the assay and cultured at 37 °C with 7.5% CO₂. Human serum samples were 2-fold serially diluted in medium with dilutions ranging from 1:10 to 1:10,240. VSV-SARS-CoV-2-S particles were diluted in medium to obtain 200 TU in the assay. Serum dilutions were mixed 1:1 with pseudovirus (n=2 technical replicates per serum per pseudovirus) for 30 minutes at room temperature before being added to Vero 76 cell monolayers and incubated at 37°C with 7.5% CO₂ for 24 hours. Supernatants were removed and the cells were lysed with luciferase reagent (Promega). Luminescence was recorded on a CLARIOstar^{®} Plus microplate reader (BMG Labtech), and neutralization titers were calculated as the reciprocal of the highest serum dilution that still resulted in 50% reduction in luminescence. Results for all pseudovirus neutralization experiments were expressed as geometric mean titers (GMT) of duplicates. If no neutralization was observed, an arbitrary titer value of half of the limit of detection [LOD] was reported.

### Statistical analysis

The statistical method of aggregation used for analysis of antibody titers was the geometric mean and for the ratio of SARS-CoV-2 VOC titer and wild-type strain titer the geometric mean and the corresponding 95% confidence interval. The use of the geometric mean accounts for the non-normal distribution of antibody titers, which span several orders of magnitude. The Friedman test with Dunn's correction for multiple comparisons was used to conduct pairwise signed-rank tests of group geometric mean neutralizing antibody titers with a common control group. The Kruskal-Wallis test with Dunn's correction for multiple comparisons was used to conduct unpaired signed-rank tests of group GMT ratios. All statistical analyses were performed using GraphPad Prism software version 9.

### References Cited in Example 19

1. F. Jian, Y. Yu, W. Song, A. Yisimayi, L. Yu, Y. Gao, N. Zhang, Y. Wang, F. Shao, X. Hao, Y. Xu, R. Jin, Y. Wang, X. S. Xie, Y. Cao, Further humoral immunity evasion of emerging SARS-CoV-2 BA.4 and BA.5 subvariants. Lancet Infect Dis, (2022).
2. Q. Wang, S. Iketani, Z. Li, Y. Guo, A. Y. Yeh, M. Liu, J. Yu, Z. Sheng, Y. Huang, L. Liu, D. D. Ho, Antigenic characterization of the SARS-CoV-2 Omicron subvariant BA.2.75. Cell Host Microbe, (2022).
3. A. Muik, A. K. Wallisch, B. Sanger, K. A. Swanson, J. Muhl, W. Chen, H. Cai, D. Maurus, R. Sarkar, O. Tureci, P. R. Dormitzer, U. Sahin, Neutralization of SARS-CoV-2 lineage B.1.1.7 pseudovirus by BNT162b2 vaccine-elicited human sera. Science 371, 1152-1153 (2021).
4. A. Muik, B. G. Lui, A. K. Wallisch, M. Bacher, J. Muhl, J. Reinholz, O. Ozhelvaci, N. Beckmann, R. C. Guimil Garcia, A. Poran, S. Shpyro, A. Finlayson, H. Cai, Q. Yang, K. A. Swanson, O. Tureci, U. Sahin, Neutralization of SARS-CoV-2 Omicron by BNT162b2 mRNA vaccine-elicited human sera. Science 375, 678-680 (2022).
5. A. Muik, B. G. Lui, M. Bacher, A. K. Wallisch, A. Toker, A. Finlayson, K. Kruger, O. Ozhelvaci, K. Grikscheit, S. Hoehl, S. Ciesek, O. Tureci, U. Sahin, Omicron BA.2 breakthrough infection enhances cross-neutralization of BA.2.12.1 and BA.4/BA.5. bioRxiv, (2022).
6. X. Shen, S. Chalkias, J. Feng, X. Chen, H. Zhou, J. C. Marshall, B. Girard, J. E. Tomassini, A. Aunins, R. Das, D. C. Montefiori, Neutralization of SARS-CoV-2 Omicron BA.2.75 after mRNA-1273 Vaccination. N Engl J Med 387, 1234-1236 (2022).
7. H. Gruell, K. Vanshylla, P. Tober-Lau, D. Hillus, L. E. Sander, F. Kurth, F. Klein, Neutralisation sensitivity of the SARS-CoV-2 omicron BA.2.75 sublineage. Lancet Infect Dis 22, 1422-1423 (2022).
8. WHO Technical Advisory Group on SARS-CoV-2 Virus Evolution (TAG-VE): Classification of Omicron (B.1.1.259): SARS-CoV-2 Variant of Concern. (2021).
9. WHO Headquarters (HQ), WHO Health Emergencies Programme, Enhancing Response to Omicron SARS-CoV-2 variant: Technical brief and priority actions for Member States. (2022).
10. M. Hoffmann et al., The Omicron variant is highly resistant against antibody-mediated neutralization: Implications for control of the COVID-19 pandemic. Cell 185, 447-456 e411 (2022).
11. V. Servellita et al., Neutralizing immunity in vaccine breakthrough infections from the SARS-CoV-2 Omicron and Delta variants. Cell 185, 1539-1548 e1535 (2022).
12. C. Kurhade et al., Neutralization of Omicron BA.1, BA.2, and BA.3 SARS-CoV-2 by 3 doses of BNT162b2 vaccine. Nat Commun 13, 3602 (2022).
13. Y. Cao et al., Omicron escapes the majority of existing SARS-CoV-2 neutralizing antibodies. Nature 602, 657-663 (2022).
14. S. Xia, L. Wang, Y. Zhu, L. Lu, S. Jiang, Origin, virological features, immune evasion and intervention of SARS-CoV-2 Omicron sublineages. Signal Transduct Target Ther 7, 241 (2022).
15. H. Gruell et al., SARS-CoV-2 Omicron sublineages exhibit distinct antibody escape patterns. Cell Host Microbe, (2022).
16. Y. Fan et al., SARS-CoV-2 Omicron variant: recent progress and future perspectives. Signal Transduct Target Ther 7, 141 (2022).
17. H. Tegally et al., Emergence of SARS-CoV-2 Omicron lineages BA.4 and BA.5 in South Africa. Nat Med, (2022).
18. European Centre for Disease Prevention and Control, Weekly COVID-19 country overview - Country overview report: week 31 2022 (2022).
19. J. Hadfield et al., Nextstrain: real-time tracking of pathogen evolution https://nextstrain.org/ncov/gisaid. Bioinformatics 34, 4121-4123 (2018).
20. Centers for Disease Control and Prevention. COVID Data Tracker. Atlanta, GA: US Department of Health and Human Services, CDC; 2022, August 12. https://covid.cdc.gov/covid-data-tracker. (2022).
21. A. Z. Mykytyn et al., Antigenic cartography of SARS-CoV-2 reveals that Omicron BA.1 and BA.2 are antigenically distinct. Sci Immunol, eabq4450 (2022).
22. P. Wang et al., Antibody resistance of SARS-CoV-2 variants B.1.351 and B.1.1.7. Nature 593, 130-135 (2021).
23. J. Quandt et al., Omicron BA.1 breakthrough infection drives cross-variant neutralization and memory B cell formation against conserved epitopes. Sci Immunol, eabq2427 (2022).
24. C. I. Kaku et al., Recall of pre-existing cross-reactive B cell memory following Omicron BA.1 breakthrough infection. Sci Immunol, eabq3511 (2022).
25. Y. Cao et al., BA.2.12.1, BA.4 and BA.5 escape antibodies elicited by Omicron infection. Nature, (2022).
26. P. Arora et al., Augmented neutralisation resistance of emerging omicron subvariants BA.2.12.1, BA.4, and BA.5. Lancet Infect Dis, (2022).
27. Q. Wang et al., Antibody evasion by SARS-CoV-2 Omicron subvariants BA.2.12.1, BA.4, & BA.5. Nature, (2022).
28. A. Muik et al., Omicron BA.2 breakthrough infection enhances cross-neutralization of BA.2.12.1 and BA.4/BA.5. bioRxiv, (2022).
29. P. Du et al., A bivalent vaccine containing D614G and BA.1 spike trimer proteins or a BA.1 spike trimer protein booster shows broad neutralizing immunity. J Med Virol 94, 4287-4293 (2022).
30. A. R. Branche et al., SARS-CoV-2 Variant Vaccine Boosters Trial: Preliminary Analyses. medRxiv, (2022).
31. A. Muik et al., Neutralization of SARS-CoV-2 lineage B.1.1.7 pseudovirus by BNT162b2 vaccine-elicited human sera. Science 371, 1152-1153 (2021).
32. A. Muik et al., Neutralization of SARS-CoV-2 Omicron by BNT162b2 mRNA vaccine-elicited human sera. Science 375, 678-680 (2022).
33. C. W. Tan et al., Pan-Sarbecovirus Neutralizing Antibodies in BNT162b2-Immunized SARS-CoV-1 Survivors. N Engl J Med 385, 1401-1406 (2021).
34. S. I. Richardson et al., SARS-CoV-2 BA.4 infection triggers more cross-reactive neutralizing antibodies than BA.1. bioRxiv, (2022).
35. S. Y. Tartof et al., Immunocompromise and durability of BNT162b2 vaccine against severe outcomes due to omicron and delta variants. Lancet Respir Med 10, e61-e62 (2022).
36. N. Andrews et al., Covid-19 Vaccine Effectiveness against the Omicron (B.1.1.529) Variant. N Engl J Med 386, 1532-1546 (2022).
37. F. P. Polack et al., Safety and Efficacy of the BNT162b2 mRNA Covid-19 Vaccine. N Engl J Med 383, 2603-2615 (2020).
38. W. Dejnirattisai et al., SARS-CoV-2 Omicron-B.1.1.529 leads to widespread escape from neutralizing antibody responses. Cell 185, 467-484 e415 (2022).
39. A. B. Vogel et al., BNT162b vaccines protect rhesus macaques from SARS-CoV-2. Nature 592, 283-289 (2021).
40. A. G. Orlandini von Niessen et al., Improving mRNA-Based Therapeutic Gene Delivery by Expression-Augmenting 3' UTRs Identified by Cellular Library Screening. Mol Ther 27, 824-836 (2019).
41. S. Holtkamp et al., Modification of antigen-encoding RNA increases stability, translational efficacy, and T-cell stimulatory capacity of dendritic cells. Blood 108, 4009-4017 (2006).
42. E. Grudzien-Nogalska et al., Synthetic mRNAs with superior translation and stability properties. Methods Mol Biol 969, 55-72 (2013).
43. S. Berensmeier, Magnetic particles for the separation and purification of nucleic acids. Appl Microbiol Biotechnol 73, 495-504 (2006).

### Example 20: Clinical trial results confirming that a booster dose that includes Omicron BA.4/5-specific vaccine can induce a strong immune response against Omicron BA.4/5 variants

The present Example provides clinical trial data confirming that a BA.4/5-specific vaccine (e.g., a bivalent RNA vaccine comprising a first RNA encoding a SARS-CoV-2 S protein of a Wuhan strain and a second RNA encoding a SARS-CoV-2 S protein comprising one or more mutations characteristic of a BA.4/5 Omicron variant as described herein) can induce a strong immune response in subjects. Specifically, the present Example provides data demonstrating that such an RNA vaccine can induce an immune response (e.g., neutralization antibody titers) that are higher against a BA.4/5 variant than an immune response induced by an RNA vaccine encoding a SARS-CoV-2 S protein of a Wuhan strain. These results confirm the insights provided by the BA.4/5-breakthrough infection data and mouse experiment data described in the previous Examples, with respect to the benefits of administering a BA.4/5-specific vaccine (e.g., ones described herein).

The present Example provides immune response data from a Phase 2/3 clinical trial in which a 30-µg booster dose of an Omicron BA.4/BA.5-adapted bivalent COVID-19 vaccine (comprising 15 µg of RNA encoding a full length SARS-CoV-2 S protein of a Wuhan variant, for example, in some embodiments an RNA comprising a nucleotide sequence that is at least 95% or higher (including and up to 100%) identical to SEQ ID NO: 20 (e.g., in some embodiments BNT 162b2), and 15 µg of RNA encoding a full length SARS-CoV-2 S protein comprising one or more mutations characteristic of an Omicron BA.4/BA.5, for example, in some embodiments, an RNA comprising a nucleotide sequence that is at least 95% or higher (including and up to 100%) identical to SEQ ID NO:72) was administered to individuals 18 to 55 years of age (n=38) and those older than 55 years of age (n=36) who had previously received three doses of an RNA vaccine encoding a SARS-CoV-2 S protein of a Wuhan strain (e.g., BNT162b2). A comparator group of participants older than 55 years of age (n=40) were administered a 30-µg booster dose (as a fourth dose) of an RNA vaccine encoding a SARS-CoV-2 S protein of a Wuhan strain. In the present Example, subjects who were administered a bivalent vaccine as described herein received their last dose of the vaccine that encodes a SARS-CoV-2 S protein of a Wuhan strain on an average of 11 months prior to administeration of a bivalent vaccine, whereas subjects who were administered an RNA vaccine encoding a SARS-CoV-2 protein of a Wuhan strain, e.g., BNT162b2 (as a fourth dose) received their third dose on an average of 6 months previously. Despite this difference, pre-booster antibody titers were similar in both groups. Both groups included subjects with evidence of prior SARS-CoV-2 infection and subjects without prior SARS-CoV-2 infection.

Among subjects administered an Omicron BA.4/BA.5-specific bivalent vaccine, a substantially higher increase in Omicron BA.4/BA.5-neutralizing antibody titers was observed as compared to pre-booster levels. For individuals 18 to 55 years of age administered a bivalent vaccine, the geometric mean titer (GMT) against Omicron BA.4/BA.5 was about 600 (e.g., 606), which was a 9.5-fold rise (95% Cl: 6.7, 13.6) from pre-booster levels. For individuals older than 55 years administered a BA.4/5 bivalent vaccine, the GMT was about 900 (e.g., 896), which was a 13.2-fold rise (95% Cl: 8.0, 21.6) from pre-booster levels. By contrast, participants over 55 years of age who received a 30-µg booster dose of an RNA vaccine encoding a SARS-CoV-2 S protein of a Wuhan strain, a lower neutralizing antibody response was observed against Omicron BA.4/BA.5 measured 1-month post booster. For these participants, the GMT was about 230 (e.g., 236), representing a 2.9-fold rise (95% CI: 2.1, 3.9) from pre-booster levels. The safety profile observed in the clinical trial for the bivalent vaccine was favorable, and consistent with BNT162b2.

In addition, when examining populations of subjects with and without evidence of a prior SARS-CoV-2 infection, an increase in neutralizing antibodies against Omicron BA.4/BA.5 following a booster dose of a bivalent vaccine was observed in both populations, demonstrating that a BA.4/5-bivalent vaccine can improve protection in subjects that receive such a vaccine, regardless of prior infection status.

### Example 21: Improved Neutralization of Omicron BA.4/5, BA.4.6, BA.2.75.2, BQ.1.1, and XBB.1 with a Bivalent BA.4/5 Vaccine

The present Example provides further data demonstrating that a bivalent RNA vaccine comprising RNA encoding a SARS-CoV-2 S protein of a Wuhan strain and RNA encoding a SARS-CoV-2 S protein comprising one or more mutations characteristic of a BA.4/5 Omicron variant (e.g., a bivalent vaccine described herein) can provide an improved immune response as compared to a monovalent RNA vaccine encoding a SARS-CoV-2 S protein of a Wuhan strain. For example, in some embodiments, such an improved immune response includes increased neutralization titers against one or more, including, two or more, three or more, four or more, five or more, six or more, SARS-CoV-2 variants of concern. In some embodiments, such an improved immune response includes increased neutralization titers against one or more, including, two or more, three or more, four or more, five or more, six or more, Omicron variants of concern. In some embodiments, such an improved immune response includes increased neutralization titers against a larger number of Omicron variants of concern, as compared to that observed with a monovalent RNA vaccine encoding a SARS-CoV-2 S protein of a Wuhan strain.

In particular, the present Example provides data demonstrating that such a bivalent vaccine can induce an improved immune response (e.g., in some embodiments higher neutralizing responses) against BA.5-derived sublineages (e.g., BA.4.6, BQ.1.1, and XBB.1) and BA.2-derived sublineages (e.g., BA.2.75.2) as compared to a monovalent RNA vaccine encoding a SARS-CoV-2 S protein of a Wuhan strain when administered as a 4th dose booster. The present Example also provides data demonstrating that subjects with SARS-CoV-2 infection history (e.g., subjects with previous or current SARS-CoV-2 infection) can develop higher immune responses (e.g., higher neutralizing titers) after administration of a 4^{th} booster dose (e.g., of a monovalent or a bivalent vaccine) relative to subjects who have not been infected with SARS-COV-2. The present Example demonstrates that a BA.4/5 bivalent vaccine can induce an improved immune response (e.g., improved neutralizing response) as compared to a monovalent RNA vaccine encoding a SARS-CoV-2 S protein of a Wuhan strain regardless of SARS-CoV-2 infection history. The present Example also provides data demonstrating that improvements in immune response against certain Omicron sublineages (e.g., Omicron sublineages tested herein) provided by a BA.4/5 bivalent vaccine relative to a Wuhan monovalent vaccine are greater for subjects who have not been previously infected with SARS-CoV-2 as compared to subjects who have been previously infected with SARS-CoV-2.

Severe acute respiratory syndrome coronavirus 2 (SARS-CoV-2) Omicron variant has continued to evolve globally into many sublineages since its emergence in November 2021. To mitigate the ongoing Omicron pandemic, the U.S. FDA authorized emergency use of a bivalent BA.4/5-vaccine in September 2022. The U.S. FDA authorized bivalent vaccine contains two mRNAs: one encoding original (Wuhan) SARS-CoV-2 Spike protein (BNT162b2) and another encoding a SARS-CoV-2 S protein comprising mutations characteristic of an Omicron BA.4/5 variant (comprising in some embodiments an amino acid sequence of SEQ ID NO: 69, and in some embodiments SEQ ID NO: 72). Since U.S. FDA approval, new Omicron BA.2- and BA.5-descendent sublineages (e.g., BA.4.6, BA.2.75.2, BQ.1.1, and XBB.1) have emerged and become prevalent (https://covid.cdc.gov/covid-data-tracker/#variant-proportions). Although early epidemiological data suggest no increase in disease severity, these new sublineages have accumulated additional spike mutations that could further evade vaccine- and/or infection-elicited antibody neutralization (Refs. 2-4).

The present Example describes data from a clinical trial in which neutralization titers against Omicron sublineages BA.4/5, BA.4.6, BA.2.75.2, BQ.1.1, and XBB.1 were measured in sera collected from subjects administered as a fourth dose (a) a 30-µg booster dose of an Omicron BA.4/BA.5-adapted bivalent COVID-19 vaccine, which in some embodiments comprises 15 µg of RNA comprising a nucleotide sequence that is at least 95% (including and up to 100%) identical to SEQ ID NO: 20, and 15 µg of RNA encoding a full length SARS-CoV-2 S protein comprising one or more mutations characteristic of an Omicron BA.4/BA.5 variant and comprising a nucleotide sequence that is at least 95% (including and up to 100%) identical to SEQ ID NO:72. In the present Example, an Omicron BA.4/BA.5-adapted bivalent COVID-19 vaccine comprising (i) 15 µg of RNA comprising the sequence of SEQ ID NO: 20 and (ii) 15 µg of RNA comprising the sequence of SEQ ID NO: 72 was administered to the tested subjects.

Participants >55-years-old who had previously received 3 doses of a vaccine that delivered a SARS-CoV-2 S protein of a Wuhan strain (in the present Example, subjects had previously received three 30-µg doses of BNT162b2) were administered a 4th booster dose comprising 30-µg of monovalent BNT162b2 at ^{~}6.6-months-post-dose-3 or 30-µg of a bivalent vaccine (15-µg BNT162b2 plus 15-µg BA.4/5) at ^{~}11-months-post-dose-3. Serum was collected on the day dose 4 was administered (Pre serum samples) and at 1-month-post-dose-4 (1MPD4 serum samples). All participants were screened for evidence of previous and current SARS-CoV-2 infection by viral nucleocapsid antibodies and RT-PCR tests; both vaccine groups in the neutralization analysis were equally distributed among those with or without evidence of infection. For neutralization testing, the complete spike gene from Omicron BA.4/5 (BA.4 and BA.5 encode an identical spike sequence), BA.2.75.2, BQ.1.1, or XBB.1 was cloned into the backbone of fluorescent reporter USA-WA1/2020 SARS-CoV-2 (a strain isolated in January 2020, see Ref. 5). The resulting wild-type- (WT), BA.4/5-, BA.2.75.2-, BQ.1.1-, and XBB.1-spike mNG USA-WA1/2020 were used to measure 50% fluorescent focus reduction neutralization titers (FFRNT₅₀) for each serum sample collected.

For all participants (including subjects with and without evidence of SARS-CoV-2 infection), a 4^{th} dose of monovalent BNT162b2 vaccine was found to induce a 3.0×, 2.9×, 2.3×, 2.1×, 1.9×, and 1.5× geometric mean neutralizing titer fold rise (GMFR) against Wuhan, BA.4/5, BA.4.6, BA.2.75.2, BQ.1.1, and XBB.1, respectively; a bivalent vaccine was found to induce 5.8×, 13.0×, 11.1×, 6.7×, 8.7×, and 4.8× GMFRs (Fig. 63(A)). For individuals without SARS-CoV-2 infection history, BNT162b2 induced 4.4×, 3.0×, 2.6×, 2.1×, 1.5×, and 1.3× GMFRs, respectively; the bivalent vaccine induced 9.9×, 25.9×, 21.2×, 8.6×, 13.0×, and 4.6× GMFRs (Fig. 63(B)). For individuals with previous SARS-CoV-2 infection, BNT162b2 induced 2.0×, 2.8×, 2.1×, 2.1×, 2.2×, and 1.8× GMFRs, respectively; the bivalent vaccine induced 3.5×, 6.7×, 6.2×, 5.3×, 6.3×, and 4.9× GMFRs (Fig. 63C). Despite different intervals between doses 3 and 4, pre-dose-4 neutralizing titers were similar in the monovalent and bivalent vaccine groups in the all participants and the without infection groups.

The present Example provides at least three findings. First, a bivalent BA.4/5 vaccine consistently elicited higher neutralizing responses against BA.5-derived sublineages (BA.4.6, BQ.1.1, and XBB.1) and BA.2-derived sublineage (BA.2.75.2) than a monovalent RNA vaccine encoding a SARS-CoV-2 S protein when administered as a 4th dose booster. Second, individuals with evidence of previous or current SARS-CoV-2 infection developed higher neutralizing titers than those without infection after a 4th dose booster. The improved neutralizing responses induced by a bivalent vaccine was observed regardless of SARS-CoV-2 infection history. Third, for each tested Omicron sublineage, the difference between BNT162b2- and bivalent-GMFR was greater for sera without previous infection than those with previous infection.

Among all Omicron sublineages, BA.2.75.2, BQ.1.1, and XBB.1 exhibited the greatest evasion of vaccine-elicited neutralization; however, neutralizing titers following a bivalent booster were several fold higher than those following a monovalent RNA encoding a SARS-CoV-2 S protein of a Wuhan strain. These data demonstrate that a bivalent vaccine is more immunogenic against circulating Omicron sublineages and underscore the importance of monitoring real-world effectiveness.

### Methods

### Cells

Vero E6 (ATCC^{®} CRL-1586) purchased from the American Type Culture Collection (ATCC, Bethesda, MD) and Vero E6 cells expressing TMPRSS2 purchased from SEKISUI XenoTech, LLC were maintained in a high-glucose Dulbecco's modified Eagle's medium (DMEM) containing 10% fetal bovine serum (FBS; HyClone Laboratories, South Logan, UT) and 1% penicillin/streptomycin at 37°C with 5% CO2. Culture media and antibiotics were purchased from Thermo Fisher Scientific (Waltham, MA). The cell line was tested negative for Mycoplasma.

### Human Serum

In participants >55 years of age who previously received three 30-µg BNT162b2 doses, serum samples were collected just prior to and 1 month post-boost with a 4th dose booster of monovalent original 30-µg BNT162b2 or 30-µg bivalent BA.4/BA.5 vaccine (15 µg original with 15µg BA.4/BA.5). The protocol and informed consent were approved by institutional review boards for each of the investigational centers participating in the study. The study was conducted in compliance with all International Council for Harmonisation Good Clinical Practice guidelines and the ethical principles of the Declaration of Helsinki. The median time between dose-3 and -4 was 6.3 and 11.3 months for BNT162b2 and the bivalent BA.4/5 vaccine, respectively. All participants were screened by SARS-CoV-2 nucleocapsid Ig serological test for preexisting SARS-CoV-2 or RT-PCR for existing infection. The subset of participant's sera (approximately 40 per vaccine group) selected for neutralization testing were equally distributed between those with and without evidence of infection by either test. Human sera were heat-inactivated at 56°C for 30 min before the neutralization test.

### Recombinant Omicron sublineages-FP SARS CoV-2 viruses

Recombinant Omicron sublineage BA.4/5-, BA.4.6-, BA.2.75.2-, BQ.1.1-, and XBB.1-spike fluorescent protein (FP) SARS-CoV-2s was constructed by engineering the complete spike gene from the indicated variants into an infectious cDNA clone of mNG USA-WA1/2020 and reported previously (Refs. 6-10). Viruses were rescued post 2-3 days after electroporation and served as P0 stock. P0 stock was further passaged once on Vero E6 cells to produce P1 stock. The spike gene was sequenced from all P1 stock viruses to ensure no undesired mutation. The infectious titer of the P1 virus was quantified by fluorescent focus assay on Vero E6 cells. The P1 virus was used for the neutralization test.

### Fluorescent focus reduction neutralization test (FFRNT)

Neutralization titers of human sera were measured by FFRNT using the USA-WA1/2020-, BA.4/5, BA.4.6-, BA.2.75.2-, BQ.1.1- and XBB.1-spike FP SARS-CoV-2s. All sera were tested sequentially, USA-WA1/2020 and BA.4/5 followed by the remaining Omicron sublineages. The details of the FFRNT protocol were reported previously (see Refs. 6 and 10-13). Briefly, 2.5 × 10⁴ Vero E6 cells per well were seeded in 96-well plates (Greiner Bio-one^{™}). The cells were incubated overnight. On the next day, each serum was 2-fold serially diluted in the culture medium with the first dilution of 1:20 (final dilution range of 1:20 to 1:20,480). The diluted serum was incubated with 100-150 FFUs of FP SARS-CoV-2 at 37 °C for 1 h, after which the serum virus mixtures were loaded onto the pre-seeded Vero E6 cell monolayer in 96-well plates. After 1 h infection, the inoculum was removed and 100 µl of overlay medium (supplemented with 0.8% methylcellulose) was added to each well. After incubating the plates at 37 °C for 16 h, raw images of FP foci were acquired using CytationTM 7 (BioTek) armed with 2.5× FL Zeiss objective with a wide-field of view and processed using the Gene 5 software settings (GFP [469,525] threshold 4000, object selection size 50-1000 µm). The foci in each well were counted and normalized to the non-serum-treated controls to calculate the relative infectivities. The FFRNT₅₀ value was defined as the minimal serum dilution that suppressed >50% of fluorescent foci. The neutralization titer of each serum was determined in duplicate assays, and the geometric mean was taken. All attempts at replication were successful. Data were initially plotted in GraphPad Prism 9 software and assembled in Adobe Illustrator.

### References cited in Example 21 (each incorporated by reference herein in its entirety)

1. Cele S, Jackson L, Khoury DS, et al. Omicron extensively but incompletely escapes Pfizer BNT162b2 neutralization. Nature 2022; 602(7898): 654-6.
2. Kurhade C, Zou J, Xia H, et al. Neutralization of Omicron sublineages and Deltacron SARS-CoV-2 by 3 doses of BNT162b2 vaccine or BA.1 infection. Emerg Microbes Infect 2022: 1-18.
3. Liu Z, VanBlargan LA, Bloyet LM, et al. Identification of SARS-CoV-2 spike mutations that attenuate monoclonal and serum antibody neutralization. Cell Host Microbe 2021; 29(3): 477-88 e4.
4. Davis-Gardner ME, Lai L, Wali B, et al. mRNA bivalent booster 1 enhances neutralization against BA.2.75.2 and BQ.1.1. bioRxiv 2022: https://doi.org/10.1101/2022.10.31.514636.
5. Muruato AE, Fontes-Garfias CR, Ren P, et al. A high-throughput neutralizing antibody assay for COVID-19 diagnosis and vaccine evaluation. Nat Commun 2020; 11(1): 4059.
6. Kurhade C, Zou J, Xia H, et al. Low neutralization of SARS-CoV-2 Omicron BA.2.75.2, BQ.1.1, and XBB.1 by 4 doses of parental mRNA vaccine or a BA.5-bivalent booster. bioRxiv 2022: 2022.10.31.514580.
7. Xie X, Muruato A, Lokugamage KG, et al. An Infectious cDNA Clone of SARS-CoV-2. Cell host & microbe 2020: S1931-3128(20)30231-6.
8. Xie X, Lokugamage KG, Zhang X, et al. Engineering SARS-CoV-2 using a reverse genetic system. Nature Protocols 2021; 16(3): 1761-84.
9. Muruato AE, Fontes-Garfias CR, Ren P, et al. A high-throughput neutralizing antibody assay for COVID-19 diagnosis and vaccine evaluation. Nat Commun 2020; 11(1): 4059.
10. Xie X, Zou J, Liu M, Ren P, Shi PY. Neutralization of SARS-CoV-2 Omicron sublineages by 4 doses of mRNA vaccine. bioRxiv 2022.
11. Zou J, Xie X, Liu M, Shi PY, Ren P. Neutralization Titers in Vaccinated Patients with SARS-CoV-2 Delta Breakthrough Infections. mBio 2022; 13(4): e0199622.
12. Zou J, Xia H, Xie X, et al. Neutralization against Omicron SARS-CoV-2 from previous non-Omicron infection. Nat Commun 2022; 13(1): 852.
13. Kurhade C, Zou J, Xia H, et al. Neutralization of Omicron sublineages and Deltacron SARS-CoV-2 by 3 doses of BNT162b2 vaccine or BA.1 infection. Emerg Microbes Infect 2022: 1-18.

### Example 22: Distinct cross-neutralization of Omicron sublineages by vaccine-elicited and convalescent immune sera.

Serum samples were drawn from (i) SARS-CoV-2-naïve individuals triple-vaccinated with BNT162b2 or (ii) quadruple-vaccinated with BNT162b2, and individuals with three doses of mRNA COVID-19 vaccine (BNT162b2/mRNA-1273 homologous or heterologous regimens) who subsequently had a breakthrough infection with (iii) Omicron BA.1, (iv) Omicron BA.2 or (v) Omicron BA.4/BA.5. Breakthrough infections occurred at a time of respective variant of concern dominance (BA.1: November 2021 to January 2021, BA.2: March to May 2022, BA.4/5: mid-June to mid-July 2022) and/or were variant confirmed by genome sequencing. Neutralization titers were measured using a pseudovirus neutralization assay, using pseudoviruses bearing the SARS-CoV-2 S protein of a BA.4/5, BA.4.6/BF.7, BQ.1.1, BA.2.75, BA.2.75.2, or XBB Omicron variants on the surface (e.g., using methods described in the previous Examples; Fig. 65). 50% pseudovirus neutralization titers (pVNTso) are shown in Fig. 64(A). Neutralization breadth irrespective of the magnitude of antibody titers was assessed by normalizing the Omicron sublineage pVNTso against those for the wild-type strain (Fig. 64(B)). In the triple-/quadruple-vaccinated individuals without breakthrough infection, pVNso GMTs against Omicron BA.4/5 were 5 to 6-fold lower than GMTs against the wild-type strain (GMTs against BA.4/5 in the range of 69-121) (Fig. 64(A)). GMTs against BA.4/5 were similarly reduced in BA.1 convalescents (GMT 263, 5-fold lower than wild-type), whereas in the BA.2 and BA.4/BA.5 convalescent cohorts titers against BA.4/5 remained higher (GMTs of 386 and 521, respectively; 3 and 2-fold lower than wild-type).

In all three convalescent cohorts, neutralizing titers against Omicron BA.4.6/BF.7 and BA.2.75 were robustly above those of triple-/quadruple-vaccinated SARS-CoV-2 naïve individuals (GMT range 239-525 for convalescents as compared to 55-139 for naïves). In the convalescents, Omicron BA.4.6/BF.7 and BA.2.75 GMTs were largely comparable with no significant differences to those against Omicron BA.4/BA.5. In contrast, pVNso titers against Omicron BQ.1.1, BA.2.75.2, and XBB were lower than those against BA.4/5 across cohorts. Titers against BQ.1.1 were overall low in the SARS-CoV-2 naïve vaccinated cohorts and BA.1 convalescents (GMTs ≤38), and higher in the BA.2 and BA.4/BA.5 convalescent cohorts (GMTs 100 and 154, respectively). Titers against BA.2.75.2 and XBB were relatively low across cohorts (GMTs ≤88 and ≤33, respectively).

To assess neutralization breadth irrespective of the magnitude of antibody titers, Omicron sublineage pVN₅₀ GMTs were normalized against those for the wild-type strain. GMT ratios for all Omicron subvariant pseudoviruses were comparable in the BNT162b2³ and BNT162b2⁴ cohorts (Fig. 64(B)), indicating that a fourth dose did not improve cross-neutralization of the tested sublineages. GMT ratios were in the range of 0.09-0.22 for BA.4/5, BA.4.6/BF.7, and BA.2.75 and ≤0.05 for BQ.1.1, BA.2.75.2, and XBB in both cohorts.

Cross-neutralization of BA.4/BA.5 and BA.4.6/BF.7 was significantly (p<0.05) higher in sera from BA.2.convalescents as compared to triple-vaccinated individuals (GMT ratios 0.37 vs 0.17 for BA.4/BA.5, and 0.23 vs 0.12 for BA.4.6/BF.7) and even more so in BA.4/BA.5-convalescents for both the BA.4/BA.5 pseudovirus (GMT ratio 0.48, p<0.01 versus BNT162b2³) and the BA.4.6/BF.7 pseudovirus (GMT ratio 0.41, p<0.0001). Cross-neutralization of BA.4.6/BF.7 was also significantly (p<0.0001) stronger in BA.4/BA.5 convalescents compared to quadruple-vaccinated individuals. While BQ.1.1 was cross-neutralized less efficiently than BA.4/5 in all cohorts (GMT ratios ≤0.14), cross-neutralization in BA.4/BA.5 and BA.2 convalescents remained significantly stronger compared to SARS-CoV-2 naïve triple or quadruple vaccinated and BA.1 breakthrough infected cohorts. Cross-neutralization of BA.2.75, BA.2.75.2, and XBB pseudoviruses was broadly comparable across cohorts. Together these data show that partial neutralization of some Omicron sublineages is retained especially in BA.4/BA.5 convalescent individuals, and that neutralizating antibody responses observed in vaccinated and breakthrough infected individuals appeared to be less effective against sublineases BA.2.75.2 and XBB. These data also suggest that, in some embodiments, an improved immune response (e.g., increased neutralization titers) may be provided by a vaccine comprising RNA encoding a SARS-CoV-2 S protein comprising one or more mutations characteristic of an XBB variant, a BQ.1.1 variant, or any lineage derived therefrom).

### Example 23: T cell epitope and neutralizing B cell epitope conservedness across SARS-CoV-2 variants of concern.

To estimate the rate of nonsynonymous mutation in T cell epitopes in the S glycoprotein, the Immune Epitope Database (https://www.iedb.org/) was used to obtain epitopes confirmed for T cell reactivity in experimental assays. The database was filtered using the following criteria: Organism: SARS-COV2; Antigen: Spike glycoprotein; Positive Assay; No B cell assays; No MHC assays; MHC Restriction Type: Class I; Host: Homo sapiens (human). The resulting table was filtered by removing epitopes that were "deduced from a reactive overlapping peptide pool", as well as epitopes longer than 14 amino acids in order to restrict the dataset to confirmed minimal epitopes only. The experimental assays confirming the reactivity of these epitopes relied on multimer analysis, ELISpot or ELISpot-like assays, T cell activation assays, etc. The epitopes were reported for at least 27 different HLA-I alleles, including HLA-A, HLA-B, and HLA-C alleles. Of the 251 unique epitope sequences obtained in this approach, 244 were found in the Wuhan strain spike glycoprotein. Of these, 36 epitopes (14.8%) included a position reported to be mutated by sequence analysis described herein.

In addition, conservedness of neutralizing B cell epitopes was calculated by counting the number of antibody neutralising epitopes potentially impacted by mutations. It was calculated by first mapping 719 binding epitopes observed in 332 experimentally resolved structures of nAbs onto the S protein using available protein structures (as described in https://www.biorxiv.org/content/10.1101/2021.12.24.474095v2). For each structure, an epitope per antibody was calculated as the set of positions that were in contact with an antibody, where two residues were considered to be in contact if the smallest Euclidean distance between their atoms was smaller than 4 Angstroms. Each nAb was evaluated and considered to be evaded by a variant if any position of its epitope was mutated.

Given that humoral and cell-mediated immunity together determine susceptibility to severe COVID-19 disease, the degree of conservation among neutralizing B-cell and T-cell epitopes of the S glycoprotein in Omicron sublineages was assessed. The findings presented herein show that over 80% of the BNT162b2 encoded S glycoprotein T-cell epitopes were fully conserved in Omicron sublineages including BA.2.75.2, BQ.1.1, and XBB (Fig. 66). In striking contrast, B-cell epitopes were partially conserved in the earlier variants Alpha, Beta, and Delta (≥50%) but most of these epitopes were altered in the Omicron lineage (≤20% conservation), particularly in BA.2.75.2 and XBB (≤10%). These findings suggest that in the vaccinated population robust T-cell mediated immunity may be maintained against the Omicron variant, including sublineages that evade neutralizing antibodies.

CERTAIN SEQUENCES

<110>  BioNTech SE

<120>  CORONAVIRUS VACCINE

<130>  674-449 PCT

<160>  54

<170>  PatentIn version 3.5

<210>  1
<211>  1273
<212>  PRT
<213>  Artificial Sequence

<220>
<223>  S protein

<400>  1

<210> 2
<211> 3819
<212> RNA
<213> Artificial Sequence

<220>
<223>  Coding Sequence

<400>  2
auguuugugu uucuugugcu gcugccucuu gugucuucuc agugugugaa uuugacaaca      60

agaacacagc ugccaccagc uuauacaaau ucuuuuacca gaggagugua uuauccugau     120

aaaguguuua gaucuucugu gcugcacagc acacaggacc uguuucugcc auuuuuuagc     180

aaugugacau gguuucaugc aauucaugug ucuggaacaa auggaacaaa aagauuugau     240

aauccugugc ugccuuuuaa ugauggagug uauuuugcuu caacagaaaa gucaaauauu     300

auuagaggau ggauuuuugg aacaacacug gauucuaaaa cacagucucu gcugauugug     360

aauaaugcaa caaauguggu gauuaaagug ugugaauuuc aguuuuguaa ugauccuuuu     420

cugggagugu auuaucacaa aaauaauaaa ucuuggaugg aaucugaauu uagaguguau     480

uccucugcaa auaauuguac auuugaauau gugucucagc cuuuucugau ggaucuggaa     540

ggaaaacagg gcaauuuuaa aaaucugaga gaauuugugu uuaaaaauau ugauggauau     600

uuuaaaauuu auucuaaaca cacaccaauu aauuuaguga gagaucugcc ucagggauuu     660

ucugcucugg aaccucuggu ggaucugcca auuggcauua auauuacaag auuucagaca     720

cugcuggcuc ugcacagauc uuaucugaca ccuggagauu cuucuucugg auggacagcc     780

ggagcugcag cuuauuaugu gggcuaucug cagccaagaa cauuucugcu gaaauauaau     840

gaaaauggaa caauuacaga ugcuguggau ugugcucugg auccucuguc ugaaacaaaa     900

uguacauuaa aaucuuuuac aguggaaaaa ggcauuuauc agacaucuaa uuuuagagug     960

cagccaacag aaucuauugu gagauuucca aauauuacaa aucugugucc auuuggagaa    1020

guguuuaaug caacaagauu ugcaucugug uaugcaugga auagaaaaag aauuucuaau    1080

uguguggcug auuauucugu gcuguauaau agugcuucuu uuuccacauu uaaauguuau    1140

ggagugucuc caacaaaauu aaaugauuua uguuuuacaa auguguaugc ugauucuuuu    1200

gugaucagag gugaugaagu gagacagauu gcccccggac agacaggaaa aauugcugau    1260

uacaauuaca aacugccuga ugauuuuaca ggauguguga uugcuuggaa uucuaauaau    1320

uuagauucua aagugggagg aaauuacaau uaucuguaca gacuguuuag aaaaucaaau    1380

cugaaaccuu uugaaagaga uauuucaaca gaaauuuauc aggcuggauc aacaccuugu 1440

aauggagugg aaggauuuaa uuguuauuuu ccauuacaga gcuauggauu ucagccaacc 1500

aauggugugg gauaucagcc auauagagug guggugcugu cuuuugaacu gcugcaugca 1560

ccugcaacag uguguggacc uaaaaaaucu acaaauuuag ugaaaaauaa augugugaau 1620

uuuaauuuua auggauuaac aggaacagga gugcugacag aaucuaauaa aaaauuucug 1680

ccuuuucagc aguuuggcag agauauugca gauaccacag augcagugag agauccucag 1740

acauuagaaa uucuggauau uacaccuugu ucuuuugggg gugugucugu gauuacaccu 1800

ggaacaaaua caucuaauca gguggcugug cuguaucagg augugaauug uacagaagug 1860

ccaguggcaa uucaugcaga ucagcugaca ccaacaugga gaguguauuc uacaggaucu 1920

aauguguuuc agacaagagc aggaugucug auuggagcag aacaugugaa uaauucuuau 1980

gaaugugaua uuccaauugg agcaggcauu ugugcaucuu aucagacaca gacaaauucc 2040

ccaaggagag caagaucugu ggcaucucag ucuauuauug cauacaccau gucucuggga 2100

gcagaaaauu cuguggcaua uucuaauaau ucuauugcua uuccaacaaa uuuuaccauu 2160

ucugugacaa cagaaauuuu accugugucu augacaaaaa caucugugga uuguaccaug 2220

uacauuugug gagauucuac agaauguucu aaucugcugc ugcaguaugg aucuuuuugu 2280

acacagcuga auagagcuuu aacaggaauu gcuguggaac aggauaaaaa uacacaggaa 2340

guguuugcuc aggugaaaca gauuuacaaa acaccaccaa uuaaagauuu uggaggauuu 2400

aauuuuagcc agauucugcc ugauccuucu aaaccuucua aaagaucuuu uauugaagau 2460

cugcuguuua auaaagugac acuggcagau gcaggauuua uuaaacagua uggagauugc 2520

cugggugaua uugcugcaag agaucugauu ugugcucaga aauuuaaugg acugacagug 2580

cugccuccuc ugcugacaga ugaaaugauu gcucaguaca caucugcuuu acuggcugga 2640

acaauuacaa gcggauggac auuuggagcu ggagcugcuc ugcagauucc uuuugcaaug 2700

cagauggcuu acagauuuaa uggaauugga gugacacaga auguguuaua ugaaaaucag 2760

aaacugauug caaaucaguu uaauucugca auuggcaaaa uucaggauuc ucugucuucu 2820

acagcuucug cucugggaaa acugcaggau guggugaauc agaaugcaca ggcacugaau 2880

acucugguga aacagcuguc uagcaauuuu ggggcaauuu cuucugugcu gaaugauauu 2940

cugucuagac uggauaaagu ggaagcugaa gugcagauug auagacugau cacaggaaga 3000

cugcagucuc ugcagacuua ugugacacag cagcugauua gagcugcuga aauuagagcu 3060

ucugcuaauc uggcugcuac aaaaaugucu gaaugugugc ugggacaguc aaaaagagug 3120

gauuuuugug gaaaaggaua ucaucugaug ucuuuuccac agucugcucc acauggagug 3180

guguuuuuac augugacaua ugugccagca caggaaaaga auuuuaccac agcaccagca 3240

auuugucaug auggaaaagc acauuuucca agagaaggag uguuuguguc uaauggaaca 3300

cauugguuug ugacacagag aaauuuuuau gaaccucaga uuauuacaac agauaauaca 3360

uuugugucag gaaauuguga uguggugauu ggaauuguga auaauacagu guaugaucca 3420

cugcagccag aacuggauuc uuuuaaagaa gaacuggaua aauauuuuaa aaaucacaca 3480

ucuccugaug uggauuuagg agauauuucu ggaaucaaug caucuguggu gaauauucag 3540

aaagaaauug auagacugaa ugaaguggcc aaaaaucuga augaaucucu gauugaucug 3600

caggaacuug gaaaauauga acaguacauu aaauggccuu gguacauuug gcuuggauuu 3660

auugcaggau uaauugcaau ugugauggug acaauuaugu uauguuguau gacaucaugu 3720

uguucuuguu uaaaaggaug uuguucuugu ggaagcuguu guaaauuuga ugaagaugau 3780

ucugaaccug uguuaaaagg agugaaauug cauuacaca 3819

<210> 3
<211> 218
<212> PRT
<213> Artificial Sequence

<220>
<223> S Protein RBD Fusion

<400> 3

Met Phe Val Phe Leu Val Leu Leu Pro Leu Val Ser Ser Gln Cys Val
1               5                   10                  15

Val Arg Phe Pro Asn Ile Thr Asn Leu Cys Pro Phe Gly Glu Val Phe
            20                  25                  30

Asn Ala Thr Arg Phe Ala Ser Val Tyr Ala Trp Asn Arg Lys Arg Ile
        35                  40                  45

Ser Asn Cys Val Ala Asp Tyr Ser Val Leu Tyr Asn Ser Ala Ser Phe
    50                  55                  60

Ser Thr Phe Lys Cys Tyr Gly Val Ser Pro Thr Lys Leu Asn Asp Leu
65                  70                  75                  80

Cys Phe Thr Asn Val Tyr Ala Asp Ser Phe Val Ile Arg Gly Asp Glu
                85                  90                  95

Val Arg Gln Ile Ala Pro Gly Gln Thr Gly Lys Ile Ala Asp Tyr Asn
            100                 105                 110

Tyr Lys Leu Pro Asp Asp Phe Thr Gly Cys Val Ile Ala Trp Asn Ser
        115                 120                 125

Asn Asn Leu Asp Ser Lys Val Gly Gly Asn Tyr Asn Tyr Leu Tyr Arg
    130                 135                 140

Leu Phe Arg Lys Ser Asn Leu Lys Pro Phe Glu Arg Asp Ile Ser Thr
145                 150                 155                 160

Glu Ile Tyr Gln Ala Gly Ser Thr Pro Cys Asn Gly Val Glu Gly Phe
                165                 170                 175

Asn Cys Tyr Phe Pro Leu Gln Ser Tyr Gly Phe Gln Pro Thr Asn Gly
            180                 185                 190

Val Gly Tyr Gln Pro Tyr Arg Val Val Val Leu Ser Phe Glu Leu Leu
        195                 200                 205

His Ala Pro Ala Thr Val Cys Gly Pro Lys
    210                 215

<210>  4
<211>  654
<212>  RNA
<213>  Artificial Sequence

<220>
<223>  Coding Sequence

<400>  4
auguuugugu uucuugugcu gcugccucuu gugucuucuc aguguguggu gagauuucca      60

aauauuacaa aucugugucc auuuggagaa guguuuaaug caacaagauu ugcaucugug     120

uaugcaugga auagaaaaag aauuucuaau uguguggcug auuauucugu gcuguauaau     180

agugcuucuu uuuccacauu uaaauguuau ggagugucuc caacaaaauu aaaugauuua     240

uguuuuacaa auguguaugc ugauucuuuu gugaucagag gugaugaagu gagacagauu     300

gcccccggac agacaggaaa aauugcugau uacaauuaca aacugccuga ugauuuuaca     360

ggauguguga uugcuuggaa uucuaauaau uuagauucua aagugggagg aaauuacaau     420

uaucuguaca gacuguuuag aaaaucaaau cugaaaccuu uugaaagaga uauuucaaca     480

gaaauuuauc aggcuggauc aacaccuugu aauggagugg aaggauuuaa uuguuauuuu     540

ccauuacaga gcuauggauu ucagccaacc aauggugugg gauaucagcc auauagagug     600

guggugcugu cuuuugaacu gcugcaugca ccugcaacag uguguggacc uaaa          654


<210>  5
<211>  266
<212>  PRT
<213>  Artificial Sequence

<220>
<223>  S Protein RBD Fusion

<400>  5

Met Phe Val Phe Leu Val Leu Leu Pro Leu Val Ser Ser Gln Cys Val
1               5                   10                  15


Val Arg Phe Pro Asn Ile Thr Asn Leu Cys Pro Phe Gly Glu Val Phe
            20                  25                  30

Asn Ala Thr Arg Phe Ala Ser Val Tyr Ala Trp Asn Arg Lys Arg Ile
        35                  40                  45

Ser Asn Cys Val Ala Asp Tyr Ser Val Leu Tyr Asn Ser Ala Ser Phe
    50                  55                  60

Ser Thr Phe Lys Cys Tyr Gly Val Ser Pro Thr Lys Leu Asn Asp Leu
65                  70                  75                  80

Cys Phe Thr Asn Val Tyr Ala Asp Ser Phe Val Ile Arg Gly Asp Glu
                85                  90                  95

Val Arg Gln Ile Ala Pro Gly Gln Thr Gly Lys Ile Ala Asp Tyr Asn
            100                 105                 110

Tyr Lys Leu Pro Asp Asp Phe Thr Gly Cys Val Ile Ala Trp Asn Ser
        115                 120                 125

Asn Asn Leu Asp Ser Lys Val Gly Gly Asn Tyr Asn Tyr Leu Tyr Arg
    130                 135                 140

Leu Phe Arg Lys Ser Asn Leu Lys Pro Phe Glu Arg Asp Ile Ser Thr
145                 150                 155                 160

Glu Ile Tyr Gln Ala Gly Ser Thr Pro Cys Asn Gly Val Glu Gly Phe
                165                 170                 175

Asn Cys Tyr Phe Pro Leu Gln Ser Tyr Gly Phe Gln Pro Thr Asn Gly
            180                 185                 190

Val Gly Tyr Gln Pro Tyr Arg Val Val Val Leu Ser Phe Glu Leu Leu
        195                 200                 205

His Ala Pro Ala Thr Val Cys Gly Pro Lys Gly Ser Pro Gly Ser Gly
    210                 215                 220

Ser Gly Ser Gly Tyr Ile Pro Glu Ala Pro Arg Asp Gly Gln Ala Tyr
225                 230                 235                 240

Val Arg Lys Asp Gly Glu Trp Val Leu Leu Ser Thr Phe Leu Gly Arg
                245                 250                 255

Ser Leu Glu Val Leu Phe Gln Gly Pro Gly
            260                 265

<210>  6
<211>  798
<212>  RNA
<213>  Artificial Sequence

<220>
<223>  Coding Sequence

<400>  6
auguuugugu uucuugugcu gcugccucuu gugucuucuc aguguguggu gagauuucca      60

aauauuacaa aucugugucc auuuggagaa guguuuaaug caacaagauu ugcaucugug     120

uaugcaugga auagaaaaag aauuucuaau uguguggcug auuauucugu gcuguauaau     180

agugcuucuu uuuccacauu uaaauguuau ggagugucuc caacaaaauu aaaugauuua     240

uguuuuacaa auguguaugc ugauucuuuu gugaucagag gugaugaagu gagacagauu     300

gcccccggac agacaggaaa aauugcugau uacaauuaca aacugccuga ugauuuuaca     360

ggauguguga uugcuuggaa uucuaauaau uuagauucua aagugggagg aaauuacaau     420

uaucuguaca gacuguuuag aaaaucaaau cugaaaccuu uugaaagaga uauuucaaca     480

gaaauuuauc aggcuggauc aacaccuugu aauggagugg aaggauuuaa uuguuauuuu     540

ccauuacaga gcuauggauu ucagccaacc aauggugugg gauaucagcc auauagagug     600

guggugcugu cuuuugaacu gcugcaugca ccugcaacag uguguggacc uaaaggcucc     660

cccggcuccg gcuccggauc ugguuauauu ccugaagcuc caagagaugg gcaagcuuac     720

guucguaaag auggcgaaug gguauuacuu ucuaccuuuu uaggccgguc ccuggaggug     780

cuguuccagg gccccggc                                                   798

<210>  7
<211>  1273

<212>  PRT
<213>  Artificial Sequence

<220>
<223>  S Protein Variant

<400>  7

Met Phe Val Phe Leu Val Leu Leu Pro Leu Val Ser Ser Gln Cys Val
1               5                   10                  15

Asn Leu Thr Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile His Val Ser Gly Thr Asn Gly Thr Lys Arg Phe Asp
65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Thr Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Asp Pro Phe Leu Gly Val Tyr
    130                 135                 140

Tyr His Lys Asn Asn Lys Ser Trp Met Glu Ser Glu Phe Arg Val Tyr
145                 150                 155                 160

Ser Ser Ala Asn Asn Cys Thr Phe Glu Tyr Val Ser Gln Pro Phe Leu
                165                 170                 175

Met Asp Leu Glu Gly Lys Gln Gly Asn Phe Lys Asn Leu Arg Glu Phe
            180                 185                 190

Val Phe Lys Asn Ile Asp Gly Tyr Phe Lys Ile Tyr Ser Lys His Thr
        195                 200                 205

Pro Ile Asn Leu Val Arg Asp Leu Pro Gln Gly Phe Ser Ala Leu Glu
    210                 215                 220

Pro Leu Val Asp Leu Pro Ile Gly Ile Asn Ile Thr Arg Phe Gln Thr
225                 230                 235                 240

Leu Leu Ala Leu His Arg Ser Tyr Leu Thr Pro Gly Asp Ser Ser Ser
                245                 250                 255

Gly Trp Thr Ala Gly Ala Ala Ala Tyr Tyr Val Gly Tyr Leu Gln Pro
            260                 265                 270

Arg Thr Phe Leu Leu Lys Tyr Asn Glu Asn Gly Thr Ile Thr Asp Ala
        275                 280                 285

Val Asp Cys Ala Leu Asp Pro Leu Ser Glu Thr Lys Cys Thr Leu Lys
    290                 295                 300

Ser Phe Thr Val Glu Lys Gly Ile Tyr Gln Thr Ser Asn Phe Arg Val
305                 310                 315                 320

Gln Pro Thr Glu Ser Ile Val Arg Phe Pro Asn Ile Thr Asn Leu Cys
                325                 330                 335

Pro Phe Gly Glu Val Phe Asn Ala Thr Arg Phe Ala Ser Val Tyr Ala
            340                 345                 350

Trp Asn Arg Lys Arg Ile Ser Asn Cys Val Ala Asp Tyr Ser Val Leu
        355                 360                 365

Tyr Asn Ser Ala Ser Phe Ser Thr Phe Lys Cys Tyr Gly Val Ser Pro
    370                 375                 380

Thr Lys Leu Asn Asp Leu Cys Phe Thr Asn Val Tyr Ala Asp Ser Phe
385                 390                 395                 400

Val Ile Arg Gly Asp Glu Val Arg Gln Ile Ala Pro Gly Gln Thr Gly
                405                 410                 415

Lys Ile Ala Asp Tyr Asn Tyr Lys Leu Pro Asp Asp Phe Thr Gly Cys
            420                 425                 430

Val Ile Ala Trp Asn Ser Asn Asn Leu Asp Ser Lys Val Gly Gly Asn
        435                 440                 445

Tyr Asn Tyr Leu Tyr Arg Leu Phe Arg Lys Ser Asn Leu Lys Pro Phe
    450                 455                 460

Glu Arg Asp Ile Ser Thr Glu Ile Tyr Gln Ala Gly Ser Thr Pro Cys
465                 470                 475                 480

Asn Gly Val Glu Gly Phe Asn Cys Tyr Phe Pro Leu Gln Ser Tyr Gly
                485                 490                 495

Phe Gln Pro Thr Asn Gly Val Gly Tyr Gln Pro Tyr Arg Val Val Val
            500                 505                 510

Leu Ser Phe Glu Leu Leu His Ala Pro Ala Thr Val Cys Gly Pro Lys
        515                 520                 525

Lys Ser Thr Asn Leu Val Lys Asn Lys Cys Val Asn Phe Asn Phe Asn
    530                 535                 540

Gly Leu Thr Gly Thr Gly Val Leu Thr Glu Ser Asn Lys Lys Phe Leu
545                 550                 555                 560

Pro Phe Gln Gln Phe Gly Arg Asp Ile Ala Asp Thr Thr Asp Ala Val
                565                 570                 575

Arg Asp Pro Gln Thr Leu Glu Ile Leu Asp Ile Thr Pro Cys Ser Phe
            580                 585                 590

Gly Gly Val Ser Val Ile Thr Pro Gly Thr Asn Thr Ser Asn Gln Val
        595                 600                 605

Ala Val Leu Tyr Gln Asp Val Asn Cys Thr Glu Val Pro Val Ala Ile
    610                 615                 620

His Ala Asp Gln Leu Thr Pro Thr Trp Arg Val Tyr Ser Thr Gly Ser
625                 630                 635                 640

Asn Val Phe Gln Thr Arg Ala Gly Cys Leu Ile Gly Ala Glu His Val
                645                 650                 655

Asn Asn Ser Tyr Glu Cys Asp Ile Pro Ile Gly Ala Gly Ile Cys Ala
            660                 665                 670

Ser Tyr Gln Thr Gln Thr Asn Ser Pro Arg Arg Ala Arg Ser Val Ala
        675                 680                 685

Ser Gln Ser Ile Ile Ala Tyr Thr Met Ser Leu Gly Ala Glu Asn Ser
    690                 695                 700

Val Ala Tyr Ser Asn Asn Ser Ile Ala Ile Pro Thr Asn Phe Thr Ile
705                 710                 715                 720

Ser Val Thr Thr Glu Ile Leu Pro Val Ser Met Thr Lys Thr Ser Val
                725                 730                 735

Asp Cys Thr Met Tyr Ile Cys Gly Asp Ser Thr Glu Cys Ser Asn Leu
            740                 745                 750

Leu Leu Gln Tyr Gly Ser Phe Cys Thr Gln Leu Asn Arg Ala Leu Thr
        755                 760                 765

Gly Ile Ala Val Glu Gln Asp Lys Asn Thr Gln Glu Val Phe Ala Gln
    770                 775                 780

Val Lys Gln Ile Tyr Lys Thr Pro Pro Ile Lys Asp Phe Gly Gly Phe
785                 790                 795                 800

Asn Phe Ser Gln Ile Leu Pro Asp Pro Ser Lys Pro Ser Lys Arg Ser
                805                 810                 815

Phe Ile Glu Asp Leu Leu Phe Asn Lys Val Thr Leu Ala Asp Ala Gly
            820                 825                 830

Phe Ile Lys Gln Tyr Gly Asp Cys Leu Gly Asp Ile Ala Ala Arg Asp
        835                 840                 845

Leu Ile Cys Ala Gln Lys Phe Asn Gly Leu Thr Val Leu Pro Pro Leu
    850                 855                 860

Leu Thr Asp Glu Met Ile Ala Gln Tyr Thr Ser Ala Leu Leu Ala Gly
865                 870                 875                 880

Thr Ile Thr Ser Gly Trp Thr Phe Gly Ala Gly Ala Ala Leu Gln Ile
                885                 890                 895

Pro Phe Ala Met Gln Met Ala Tyr Arg Phe Asn Gly Ile Gly Val Thr
            900                 905                 910

Gln Asn Val Leu Tyr Glu Asn Gln Lys Leu Ile Ala Asn Gln Phe Asn
        915                 920                 925

Ser Ala Ile Gly Lys Ile Gln Asp Ser Leu Ser Ser Thr Ala Ser Ala
    930                 935                 940

Leu Gly Lys Leu Gln Asp Val Val Asn Gln Asn Ala Gln Ala Leu Asn
945                 950                 955                 960

Thr Leu Val Lys Gln Leu Ser Ser Asn Phe Gly Ala Ile Ser Ser Val
                965                 970                 975

Leu Asn Asp Ile Leu Ser Arg Leu Asp Pro Pro Glu Ala Glu Val Gln
            980                 985                 990

Ile Asp Arg Leu Ile Thr Gly Arg Leu Gln Ser Leu Gln Thr Tyr Val
        995                 1000                1005

Thr Gln Gln Leu Ile Arg Ala Ala Glu Ile Arg Ala Ser Ala Asn
    1010                1015                1020

Leu Ala Ala Thr Lys Met Ser Glu Cys Val Leu Gly Gln Ser Lys
    1025                1030                1035

Arg Val Asp Phe Cys Gly Lys Gly Tyr His Leu Met Ser Phe Pro
    1040                1045                1050

Gln Ser Ala Pro His Gly Val Val Phe Leu His Val Thr Tyr Val
    1055                1060                1065

Pro Ala Gln Glu Lys Asn Phe Thr Thr Ala Pro Ala Ile Cys His
    1070                1075                1080

Asp Gly Lys Ala His Phe Pro Arg Glu Gly Val Phe Val Ser Asn
    1085                1090                1095

Gly Thr His Trp Phe Val Thr Gln Arg Asn Phe Tyr Glu Pro Gln
    1100                1105                1110

Ile Ile Thr Thr Asp Asn Thr Phe Val Ser Gly Asn Cys Asp Val
    1115                1120                1125

Val Ile Gly Ile Val Asn Asn Thr Val Tyr Asp Pro Leu Gln Pro
    1130                1135                1140

Glu Leu Asp Ser Phe Lys Glu Glu Leu Asp Lys Tyr Phe Lys Asn
    1145                1150                1155

His Thr Ser Pro Asp Val Asp Leu Gly Asp Ile Ser Gly Ile Asn
    1160                1165                1170

Ala Ser Val Val Asn Ile Gln Lys Glu Ile Asp Arg Leu Asn Glu
    1175                1180                1185

Val Ala Lys Asn Leu Asn Glu Ser Leu Ile Asp Leu Gln Glu Leu
    1190                1195                1200

Gly Lys Tyr Glu Gln Tyr Ile Lys Trp Pro Trp Tyr Ile Trp Leu
    1205                1210                1215

Gly Phe Ile Ala Gly Leu Ile Ala Ile Val Met Val Thr Ile Met
    1220                1225                1230

Leu Cys Cys Met Thr Ser Cys Cys Ser Cys Leu Lys Gly Cys Cys
    1235                1240                1245

Ser Cys Gly Ser Cys Cys Lys Phe Asp Glu Asp Asp Ser Glu Pro
    1250                1255                1260

Val Leu Lys Gly Val Lys Leu His Tyr Thr
    1265                1270

<210> 8
<211> 3819
<212> RNA
<213> Artificial Sequence

<220>
<223> Coding Sequence

<400> 8
auguuugugu uucuugugcu gcugccucuu gugucuucuc agugugugaa uuugacaaca 60

agaacacagc ugccaccagc uuauacaaau ucuuuuacca gaggagugua uuauccugau 120

aaaguguuua gaucuucugu gcugcacagc acacaggacc uguuucugcc auuuuuuagc 180

aaugugacau gguuucaugc aauucaugug ucuggaacaa auggaacaaa aagauuugau 240

aauccugugc ugccuuuuaa ugauggagug uauuuugcuu caacagaaaa gucaaauauu 300

auuagaggau ggauuuuugg aacaacacug gauucuaaaa cacagucucu gcugauugug 360

aauaaugcaa caaauguggu gauuaaagug ugugaauuuc aguuuuguaa ugauccuuuu 420

cugggagugu auuaucacaa aaauaauaaa ucuuggaugg aaucugaauu uagaguguau 480

uccucugcaa auaauuguac auuugaauau gugucucagc cuuuucugau ggaucuggaa 540

ggaaaacagg gcaauuuuaa aaaucugaga gaauuugugu uuaaaaauau ugauggauau 600

uuuaaaauuu auucuaaaca cacaccaauu aauuuaguga gagaucugcc ucagggauuu 660

ucugcucugg aaccucuggu ggaucugcca auuggcauua auauuacaag auuucagaca 720

cugcuggcuc ugcacagauc uuaucugaca ccuggagauu cuucuucugg auggacagcc 780

ggagcugcag cuuauuaugu gggcuaucug cagccaagaa cauuucugcu gaaauauaau 840

gaaaauggaa caauuacaga ugcuguggau ugugcucugg auccucuguc ugaaacaaaa 900

uguacauuaa aaucuuuuac aguggaaaaa ggcauuuauc agacaucuaa uuuuagagug 960

cagccaacag aaucuauugu gagauuucca aauauuacaa aucugugucc auuuggagaa 1020

guguuuaaug caacaagauu ugcaucugug uaugcaugga auagaaaaag aauuucuaau 1080

uguguggcug auuauucugu gcuguauaau agugcuucuu uuuccacauu uaaauguuau 1140

ggagugucuc caacaaaauu aaaugauuua uguuuuacaa auguguaugc ugauucuuuu 1200

gugaucagag gugaugaagu gagacagauu gcccccggac agacaggaaa aauugcugau 1260

uacaauuaca aacugccuga ugauuuuaca ggauguguga uugcuuggaa uucuaauaau 1320

uuagauucua aagugggagg aaauuacaau uaucuguaca gacuguuuag aaaaucaaau 1380

cugaaaccuu uugaaagaga uauuucaaca gaaauuuauc aggcuggauc aacaccuugu 1440

aauggagugg aaggauuuaa uuguuauuuu ccauuacaga gcuauggauu ucagccaacc 1500

aauggugugg gauaucagcc auauagagug guggugcugu cuuuugaacu gcugcaugca 1560

ccugcaacag uguguggacc uaaaaaaucu acaaauuuag ugaaaaauaa augugugaau 1620

uuuaauuuua auggauuaac aggaacagga gugcugacag aaucuaauaa aaaauuucug 1680

ccuuuucagc aguuuggcag agauauugca gauaccacag augcagugag agauccucag 1740

acauuagaaa uucuggauau uacaccuugu ucuuuugggg gugugucugu gauuacaccu 1800

ggaacaaaua caucuaauca gguggcugug cuguaucagg augugaauug uacagaagug 1860

ccaguggcaa uucaugcaga ucagcugaca ccaacaugga gaguguauuc uacaggaucu 1920

aauguguuuc agacaagagc aggaugucug auuggagcag aacaugugaa uaauucuuau 1980

gaaugugaua uuccaauugg agcaggcauu ugugcaucuu aucagacaca gacaaauucc 2040

ccaaggagag caagaucugu ggcaucucag ucuauuauug cauacaccau gucucuggga 2100

gcagaaaauu cuguggcaua uucuaauaau ucuauugcua uuccaacaaa uuuuaccauu 2160

ucugugacaa cagaaauuuu accugugucu augacaaaaa caucugugga uuguaccaug 2220

uacauuugug gagauucuac agaauguucu aaucugcugc ugcaguaugg aucuuuuugu 2280

acacagcuga auagagcuuu aacaggaauu gcuguggaac aggauaaaaa uacacaggaa 2340

guguuugcuc aggugaaaca gauuuacaaa acaccaccaa uuaaagauuu uggaggauuu 2400

aauuuuagcc agauucugcc ugauccuucu aaaccuucua aaagaucuuu uauugaagau 2460

cugcuguuua auaaagugac acuggcagau gcaggauuua uuaaacagua uggagauugc 2520

cugggugaua uugcugcaag agaucugauu ugugcucaga aauuuaaugg acugacagug 2580

cugccuccuc ugcugacaga ugaaaugauu gcucaguaca caucugcuuu acuggcugga 2640

acaauuacaa gcggauggac auuuggagcu ggagcugcuc ugcagauucc uuuugcaaug 2700

cagauggcuu acagauuuaa uggaauugga gugacacaga auguguuaua ugaaaaucag 2760

aaacugauug caaaucaguu uaauucugca auuggcaaaa uucaggauuc ucugucuucu 2820

acagcuucug cucugggaaa acugcaggau guggugaauc agaaugcaca ggcacugaau 2880

acucugguga aacagcuguc uagcaauuuu ggggcaauuu cuucugugcu gaaugauauu 2940

cugucuagac uggauccucc ugaagcugaa gugcagauug auagacugau cacaggaaga 3000

cugcagucuc ugcagacuua ugugacacag cagcugauua gagcugcuga aauuagagcu 3060

ucugcuaauc uggcugcuac aaaaaugucu gaaugugugc ugggacaguc aaaaagagug 3120

gauuuuugug gaaaaggaua ucaucugaug ucuuuuccac agucugcucc acauggagug 3180

guguuuuuac augugacaua ugugccagca caggaaaaga auuuuaccac agcaccagca 3240

auuugucaug auggaaaagc acauuuucca agagaaggag uguuuguguc uaauggaaca 3300

cauugguuug ugacacagag aaauuuuuau gaaccucaga uuauuacaac agauaauaca 3360

uuugugucag gaaauuguga uguggugauu ggaauuguga auaauacagu guaugaucca 3420

cugcagccag aacuggauuc uuuuaaagaa gaacuggaua aauauuuuaa aaaucacaca 3480

ucuccugaug uggauuuagg agauauuucu ggaaucaaug caucuguggu gaauauucag 3540

aaagaaauug auagacugaa ugaaguggcc aaaaaucuga augaaucucu gauugaucug 3600

caggaacuug gaaaauauga acaguacauu aaauggccuu gguacauuug gcuuggauuu 3660

auugcaggau uaauugcaau ugugauggug acaauuaugu uauguuguau gacaucaugu 3720

uguucuuguu uaaaaggaug uuguucuugu ggaagcuguu guaaauuuga ugaagaugau 3780

ucugaaccug uguuaaaagg agugaaauug cauuacaca 3819

<210> 9
<211> 3819
<212> RNA
<213> Artificial Sequence

<220>
<223> Coding Sequence

<400> 9
auguucgugu uccuggugcu gcugccucug guguccagcc agugugugaa ccugaccacc 60

agaacacagc ugccuccagc cuacaccaac agcuuuacca gaggcgugua cuaccccgac 120

aagguguuca gauccagcgu gcugcacucu acccaggacc uguuccugcc uuucuucagc 180

aacgugaccu gguuccacgc cauccacgug uccggcacca auggcaccaa gagauucgac 240

aaccccgugc ugcccuucaa cgacggggug uacuuugcca gcaccgagaa guccaacauc 300

aucagaggcu ggaucuucgg caccacacug gacagcaaga cccagagccu gcugaucgug 360

aacaacgcca ccaacguggu caucaaagug ugcgaguucc aguucugcaa cgaccccuuc 420

cugggcgucu acuaccacaa gaacaacaag agcuggaugg aaagcgaguu ccggguguac 480

agcagcgcca acaacugcac cuucgaguac gugucccagc cuuuccugau ggaccuggaa 540

ggcaagcagg gcaacuucaa gaaccugcgc gaguucgugu uuaagaacau cgacggcuac 600

uucaagaucu acagcaagca caccccuauc aaccucgugc gggaucugcc ucagggcuuc 660

ucugcucugg aaccccuggu ggaucugccc aucggcauca acaucacccg guuucagaca 720

cugcuggccc ugcacagaag cuaccugaca ccuggcgaua gcagcagcgg auggacagcu 780

ggugccgccg cuuacuaugu gggcuaccug cagccuagaa ccuuccugcu gaaguacaac 840

gagaacggca ccaucaccga cgccguggau ugugcucugg auccucugag cgagacaaag 900

ugcacccuga aguccuucac cguggaaaag ggcaucuacc agaccagcaa cuuccgggug 960

cagcccaccg aauccaucgu gcgguucccc aauaucacca aucugugccc cuucggcgag 1020

guguucaaug ccaccagauu cgccucugug uacgccugga accggaagcg gaucagcaau 1080

ugcguggccg acuacuccgu gcuguacaac uccgccagcu ucagcaccuu caagugcuac 1140

ggcguguccc cuaccaagcu gaacgaccug ugcuucacaa acguguacgc cgacagcuuc 1200

gugauccggg gagaugaagu gcggcagauu gccccuggac agacaggcaa gaucgccgac 1260

uacaacuaca agcugcccga cgacuucacc ggcuguguga uugccuggaa cagcaacaac 1320

cuggacucca aagucggcgg caacuacaau uaccuguacc ggcuguuccg gaaguccaau 1380

cugaagcccu ucgagcggga caucuccacc gagaucuauc aggccggcag caccccuugu 1440

aacggcgugg aaggcuucaa cugcuacuuc ccacugcagu ccuacggcuu ucagcccaca 1500

aauggcgugg gcuaucagcc cuacagagug guggugcuga gcuucgaacu gcugcaugcc 1560

ccugccacag ugugcggccc uaagaaaagc accaaucucg ugaagaacaa augcgugaac 1620

uucaacuuca acggccugac cggcaccggc gugcugacag agagcaacaa gaaguuccug 1680

ccauuccagc aguuuggccg ggauaucgcc gauaccacag acgccguuag agauccccag 1740

acacuggaaa uccuggacau caccccuugc agcuucggcg gagugucugu gaucaccccu 1800

ggcaccaaca ccagcaauca gguggcagug cuguaccagg acgugaacug uaccgaagug 1860

cccguggcca uucacgccga ucagcugaca ccuacauggc ggguguacuc caccggcagc 1920

aauguguuuc agaccagagc cggcugucug aucggagccg agcacgugaa caauagcuac 1980

gagugcgaca ucccca ucgg cgcuggaauc ugcgccagcu accagacaca gacaaacagc 2040

ccucggagag ccagaagcgu ggccagccag agcaucauug ccuacacaau gucucugggc 2100

gccgagaaca gcguggccua cuccaacaac ucuaucgcua uccccaccaa cuucaccauc 2160

agcgugacca cagagauccu gccugugucc augaccaaga ccagcgugga cugcaccaug 2220

uacaucugcg gcgauuccac cgagugcucc aaccugcugc ugcaguacgg cagcuucugc 2280

acccagcuga auagagcccu gacagggauc gccguggaac aggacaagaa cacccaagag 2340

guguucgccc aagugaagca gaucuacaag accccuccua ucaaggacuu cggcggcuuc 2400

aauuucagcc agauucugcc cgauccuagc aagcccagca agcggagcuu caucgaggac 2460

cugcuguuca acaaagugac acuggccgac gccggcuuca ucaagcagua uggcgauugu 2520

cugggcgaca uugccgccag ggaucugauu ugcgcccaga aguuuaacgg acugacagug 2580

cugccuccuc ugcugaccga ugagaugauc gcccaguaca caucugcccu gcuggccggc 2640

acaaucacaa gcggcuggac auuuggagca ggcgccgcuc ugcagauccc cuuugcuaug 2700

cagauggccu accgguucaa cggcaucgga gugacccaga augugcugua cgagaaccag 2760

aagcugaucg ccaaccaguu caacagcgcc aucggcaaga uccaggacag ccugagcagc 2820

acagcaagcg cccugggaaa gcugcaggac guggucaacc agaaugccca ggcacugaac 2880

acccugguca agcagcuguc cuccaacuuc ggcgccauca gcucugugcu gaacgauauc 2940

cugagcagac uggacccucc ugaggccgag gugcagaucg acagacugau cacaggcaga 3000

cugcagagcc uccagacaua cgugacccag cagcugauca gagccgccga gauuagagcc 3060

ucugccaauc uggccgccac caagaugucu gagugugugc ugggccagag caagagagug 3120

gacuuuugcg gcaagggcua ccaccugaug agcuucccuc agucugcccc ucacggcgug 3180

guguuucugc acgugacaua ugugcccgcu caagagaaga auuucaccac cgcuccagcc 3240

aucugccacg acggcaaagc ccacuuuccu agagaaggcg uguucguguc caacggcacc 3300

cauugguucg ugacacagcg gaacuucuac gagccccaga ucaucaccac cgacaacacc 3360

uucgugucug gcaacugcga cgucgugauc ggcauuguga acaauaccgu guacgacccu 3420

cugcagcccg agcuggacag cuucaaagag gaacuggaca aguacuuuaa gaaccacaca 3480

agccccgacg uggaccuggg cgauaucagc ggaaucaaug ccagcgucgu gaacauccag 3540

aaagagaucg accggcugaa cgagguggcc aagaaucuga acgagagccu gaucgaccug 3600

caagaacugg ggaaguacga gcaguacauc aaguggcccu gguacaucug gcugggcuuu 3660

aucgccggac ugauugccau cgugaugguc acaaucaugc uguguugcau gaccagcugc 3720

uguagcugcc ugaagggcug uuguagcugu ggcagcugcu gcaaguucga cgaggacgau 3780

ucugagcccg ugcugaaggg cgugaaacug cacuacaca 3819

<210> 10
<211> 41
<212> PRT
<213> Artificial Sequence

<220>
<223> Foldon Sequence

<400> 10

Gly Ser Gly Tyr Ile Pro Glu Ala Pro Arg Asp Gly Gln Ala Tyr Val
1               5                   10                  15

Arg Lys Asp Gly Glu Trp Val Leu Leu Ser Thr Phe Leu Gly Arg Ser
            20                  25                  30

Leu Glu Val Leu Phe Gln Gly Pro Gly
        35                  40

<210> 11
<211> 123
<212> RNA
<213> Artificial Sequence

<220>
<223> Coding Sequence

<400> 11
ggaucugguu auauuccuga agcuccaaga gaugggcaag cuuacguucg uaaagauggc 60

gaauggguau uacuuucuac cuuuuuaggc cggucccugg aggugcuguu ccagggcccc 120

ggc 123

<210> 12
<211> 47
<212> RNA
<213> Artificial Sequence

<220>
<223>  5'-UTR

<400>  12
aacuaguauu cuucuggucc ccacagacuc agagagaacc cgccacc                    47


<210>  13
<211>  278
<212>  RNA
<213>  Artificial Sequence

<220>
<223>  3'-UTR

<400>  13
cugguacugc augcacgcaa ugcuagcugc cccuuucccg uccuggguac cccgagucuc      60

ccccgaccuc gggucccagg uaugcuccca ccuccaccug ccccacucac caccucugcu     120

aguuccagac accucccaag cacgcagcaa ugcagcucaa aacgcuuagc cuagccacac     180

ccccacggga aacagcagug auuaaccuuu agcaauaaac gaaaguuuaa cuaagcuaua     240

cuaaccccag gguuggucaa uuucgugcca gccacacc                             278


<210>  14
<211>  110
<212>  RNA
<213>  Artificial Sequence

<220>
<223>  A30L70

<400>  14
aaaaaaaaaa aaaaaaaaaa aaaaaaaaaa gcauaugacu aaaaaaaaaa aaaaaaaaaa      60

aaaaaaaaaa aaaaaaaaaa aaaaaaaaaa aaaaaaaaaa aaaaaaaaaa                110


<210>  15
<211>  4282
<212>  RNA
<213>  Artificial Sequence

<220>
<223>  RBL063.1

<400>  15
gggcgaacua guauucuucu gguccccaca gacucagaga gaacccgcca ccauguuugu      60

guuucuugug cugcugccuc uugugucuuc ucagugugug aauuugacaa caagaacaca     120

gcugccacca gcuuauacaa auucuuuuac cagaggagug uauuauccug auaaaguguu     180

uagaucuucu gugcugcaca gcacacagga ccuguuucug ccauuuuuua gcaaugugac     240

augguuucau gcaauucaug ugucuggaac aaauggaaca aaaagauuug auaauccugu     300

gcugccuuuu aaugauggag uguauuuugc uucaacagaa aagucaaaua uuauuagagg     360

auggauuuuu ggaacaacac uggauucuaa aacacagucu cugcugauug ugaauaaugc     420

aacaaaugug gugauuaaag ugugugaauu ucaguuuugu aaugauccuu uucugggagu     480

guauuaucac aaaaauaaua aaucuuggau ggaaucugaa uuuagagugu auuccucugc     540

aaauaauugu acauuugaau augugucuca gccuuuucug auggaucugg aaggaaaaca     600

gggcaauuuu aaaaaucuga gagaauuugu guuuaaaaau auugauggau auuuuaaaau     660

uuauucuaaa cacacaccaa uuaauuuagu gagagaucug ccucagggau uuucugcucu     720

ggaaccucug guggaucugc caauuggcau uaauauuaca agauuucaga cacugcuggc     780

ucugcacaga ucuuaucuga caccuggaga uucuucuucu ggauggacag ccggagcugc     840

agcuuauuau gugggcuauc ugcagccaag aacauuucug cugaaauaua augaaaaugg     900

aacaauuaca gaugcugugg auugugcucu ggauccucug ucugaaacaa aauguacauu     960

aaaaucuuuu acaguggaaa aaggcauuua ucagacaucu aauuuuagag ugcagccaac    1020

agaaucuauu gugagauuuc caaauauuac aaaucugugu ccauuuggag aaguguuuaa    1080

ugcaacaaga uuugcaucug uguaugcaug gaauagaaaa agaauuucua auuguguggc    1140

ugauuauucu gugcuguaua auagugcuuc uuuuuccaca uuuaaauguu auggaguguc    1200

uccaacaaaa uuaaaugauu uauguuuuac aaauguguau gcugauucuu uugugaucag    1260

aggugaugaa gugagacaga uugcccccgg acagacagga aaaauugcug auuacaauua    1320

caaacugccu gaugauuuua caggaugugu gauugcuugg aauucuaaua auuuagauuc    1380

uaaaguggga ggaaauuaca auuaucugua cagacuguuu agaaaaucaa aucugaaacc    1440

uuuugaaaga gauauuucaa cagaaauuua ucaggcugga ucaacaccuu guaauggagu    1500

ggaaggauuu aauuguuauu uuccauuaca gagcuaugga uuucagccaa ccaauggugu 1560

gggauaucag ccauauagag ugguggugcu gucuuuugaa cugcugcaug caccugcaac 1620

agugugugga ccuaaaaaau cuacaaauuu agugaaaaau aaauguguga auuuuaauuu 1680

uaauggauua acaggaacag gagugcugac agaaucuaau aaaaaauuuc ugccuuuuca 1740

gcaguuuggc agagauauug cagauaccac agaugcagug agagauccuc agacauuaga 1800

aauucuggau auuacaccuu guucuuuugg gggugugucu gugauuacac cuggaacaaa 1860

uacaucuaau cagguggcug ugcuguauca ggaugugaau uguacagaag ugccaguggc 1920

aauucaugca gaucagcuga caccaacaug gagaguguau ucuacaggau cuaauguguu 1980

ucagacaaga gcaggauguc ugauuggagc agaacaugug aauaauucuu augaauguga 2040

uauuccaauu ggagcaggca uuugugcauc uuaucagaca cagacaaauu ccccaaggag 2100

agcaagaucu guggcaucuc agucuauuau ugcauacacc augucucugg gagcagaaaa 2160

uucuguggca uauucuaaua auucuauugc uauuccaaca aauuuuacca uuucugugac 2220

aacagaaauu uuaccugugu cuaugacaaa aacaucugug gauuguacca uguacauuug 2280

uggagauucu acagaauguu cuaaucugcu gcugcaguau ggaucuuuuu guacacagcu 2340

gaauagagcu uuaacaggaa uugcugugga acaggauaaa aauacacagg aaguguuugc 2400

ucaggugaaa cagauuuaca aaacaccacc aauuaaagau uuuggaggau uuaauuuuag 2460

ccagauucug ccugauccuu cuaaaccuuc uaaaagaucu uuuauugaag aucugcuguu 2520

uaauaaagug acacuggcag augcaggauu uauuaaacag uauggagauu gccuggguga 2580

uauugcugca agagaucuga uuugugcuca gaaauuuaau ggacugacag ugcugccucc 2640

ucugcugaca gaugaaauga uugcucagua cacaucugcu uuacuggcug gaacaauuac 2700

aagcggaugg acauuuggag cuggagcugc ucugcagauu ccuuuugcaa ugcagauggc 2760

uuacagauuu aauggaauug gagugacaca gaauguguua uaugaaaauc agaaacugau 2820

ugcaaaucag uuuaauucug caauuggcaa aauucaggau ucucugucuu cuacagcuuc 2880

ugcucuggga aaacugcagg augguggaa ucagaaugca caggcacuga auacucuggu 2940

gaaacagcug ucuagcaauu uuggggcaau uucuucugug cugaaugaua uucugucuag 3000

acuggauccu ccugaagcug aagugcagau ugauagacug aucacaggaa gacugcaguc 3060

ucugcagacu uaugugacac agcagcugau uagagcugcu gaaauuagag cuucugcuaa 3120

ucuggcugcu acaaaaaugu cugaaugugu gcugggacag ucaaaaagag uggauuuuug 3180

uggaaaagga uaucaucuga ugucuuuucc acagucugcu ccacauggag ugguguuuuu 3240

acaugugaca uaugugccag cacaggaaaa gaauuuuacc acagcaccag caauuuguca 3300

ugauggaaaa gcacauuuuc caagagaagg aguguuugug ucuaauggaa cacauugguu 3360

ugugacacag agaaauuuuu augaaccuca gauuauuaca acagauaaua cauuuguguc 3420

aggaaauugu gaugugguga uuggaauugu gaauaauaca guguaugauc cacugcagcc 3480

agaacuggau ucuuuuaaag aagaacugga uaaauauuuu aaaaaucaca caucuccuga 3540

uguggauuua ggagauauuu cuggaaucaa ugcaucugug gugaauauuc agaaagaaau 3600

ugauagacug aaugaagugg ccaaaaaucu gaaugaaucu cugauugauc ugcaggaacu 3660

uggaaaauau gaacaguaca uuaaauggcc uugguacauu uggcuuggau uuauugcagg 3720

auuaauugca auugugaugg ugacaauuau guuauguugu augacaucau guuguucuug 3780

uuuaaaagga uguuguucuu guggaagcug uuguaaauuu gaugaagaug auucugaacc 3840

uguguuaaaa ggagugaaau ugcauuacac augaugacuc gagcugguac ugcaugcacg 3900

caaugcuagc ugccccuuuc ccguccuggg uaccccgagu cucccccgac cucggguccc 3960

agguaugcuc ccaccuccac cugccccacu caccaccucu gcuaguucca gacaccuccc 4020

aagcacgcag caaugcagcu caaaacgcuu agccuagcca cacccccacg ggaaacagca 4080

gugauuaacc uuuagcaaua aacgaaaguu uaacuaagcu auacuaaccc caggguuggu 4140

caauuucgug ccagccacac ccuggagcua gcaaaaaaaa aaaaaaaaaa aaaaaaaaaa 4200

aagcauauga cuaaaaaaaa aaaaaaaaaa aaaaaaaaaa aaaaaaaaaa aaaaaaaaaa 4260

aaaaaaaaaa aaaaaaaaaa aa 4282

<210> 16
<211> 4282
<212> RNA
<213> Artificial Sequence

<220>
<223>  RBL063.2

<400>  16
gggcgaacua guauucuucu gguccccaca gacucagaga gaacccgcca ccauguucgu      60

guuccuggug cugcugccuc ugguguccag ccagugugug aaccugacca ccagaacaca     120

gcugccucca gccuacacca acagcuuuac cagaggcgug uacuaccccg acaagguguu     180

cagauccagc gugcugcacu cuacccagga ccuguuccug ccuuucuuca gcaacgugac     240

cugguuccac gccauccacg uguccggcac caauggcacc aagagauucg acaaccccgu     300

gcugcccuuc aacgacgggg uguacuuugc cagcaccgag aaguccaaca ucaucagagg     360

cuggaucuuc ggcaccacac uggacagcaa gacccagagc cugcugaucg ugaacaacgc     420

caccaacgug gucaucaaag ugugcgaguu ccaguucugc aacgaccccu uccugggcgu     480

cuacuaccac aagaacaaca agagcuggau ggaaagcgag uuccgggugu acagcagcgc     540

caacaacugc accuucgagu acguguccca gccuuuccug auggaccugg aaggcaagca     600

gggcaacuuc aagaaccugc gcgaguucgu guuuaagaac aucgacggcu acuucaagau     660

cuacagcaag cacaccccua ucaaccucgu gcgggaucug ccucagggcu ucucugcucu     720

ggaaccccug guggaucugc ccaucggcau caacaucacc cgguuucaga cacugcuggc     780

ccugcacaga agcuaccuga caccuggcga uagcagcagc ggauggacag cuggugccgc     840

cgcuuacuau gugggcuacc ugcagccuag aaccuuccug cugaaguaca acgagaacgg     900

caccaucacc gacgccgugg auugugcucu ggauccucug agcgagacaa agugcacccu     960

gaaguccuuc accguggaaa agggcaucua ccagaccagc aacuuccggg ugcagcccac    1020

cgaauccauc gugcgguucc ccaauaucac caaucugugc ccuuucggcg agguguucaa    1080

ugccaccaga uucgccucug uguacgccug gaaccggaag cggaucagca auugcguggc    1140

cgacuacucc gugcuguaca acuccgccag cuucagcacc uucaagugcu acggcguguc    1200

cccuaccaag cugaacgacc ugugcuucac aaacguguac gccgacagcu ucgugauccg    1260

gggagaugaa gugcggcaga uugccccugg acagacaggc aagaucgccg acuacaacua    1320

caagcugccc gacgacuuca ccggcugugu gauugccugg aacagcaaca accuggacuc    1380

caaagucggc ggcaacuaca auuaccugua ccggcuguuc cggaagucca aucugaagcc 1440

cuucgagcgg gacaucucca ccgagaucua ucaggccggc agcaccccuu guaacggcgu 1500

ggaaggcuuc aacugcuacu ucccacugca guccuacggc uuucagccca caaauggcgu 1560

gggcuaucag cccuacagag ugguggugcu gagcuucgaa cugcugcaug ccccugccac 1620

agugugcggc ccuaagaaaa gcaccaaucu cgugaagaac aaaugcguga acuucaacuu 1680

caacggccug accggcaccg gcgugcugac agagagcaac aagaaguucc ugccauucca 1740

gcaguuuggc cgggauaucg ccgauaccac agacgccguu agagaucccc agacacugga 1800

aauccuggac aucaccccuu gcagcuucgg cggagugucu gugaucaccc cuggcaccaa 1860

caccagcaau cagguggcag ugcuguacca ggacgugaac uguaccgaag ugcccguggc 1920

cauucacgcc gaucagcuga caccuacaug gcggguguac uccaccggca gcaauguguu 1980

ucagaccaga gccggcuguc ugaucggagc cgagcacgug aacaauagcu acgagugcga 2040

cauccccauc ggcgcuggaa ucugcgccag cuaccagaca cagacaaaca gcccucggag 2100

agccagaagc guggccagcc agagcaucau ugccuacaca augucucugg gcgccgagaa 2160

cagcguggcc uacuccaaca acucuaucgc uauccccacc aacuucacca ucagcgugac 2220

cacagagauc cugccugugu ccaugaccaa gaccagcgug gacugcacca uguacaucug 2280

cggcgauucc accgagugcu ccaaccugcu gcugcaguac ggcagcuucu gcacccagcu 2340

gaauagagcc cugacaggga ucgccgugga acaggacaag aacacccaag agguguucgc 2400

ccaagugaag cagaucuaca agaccccucc uaucaaggac uucggcggcu ucaauuucag 2460

ccagauucug cccgauccua gcaagcccag caagcggagc uucaucgagg accugcuguu 2520

caacaaagug acacuggccg acgccggcuu caucaagcag uauggcgauu gucugggcga 2580

cauugccgcc agggaucuga uuugcgccca gaaguuuaac ggacugacag ugcugccucc 2640

ucugcugacc gaugagauga ucgcccagua cacaucugcc cugcuggccg gcacaaucac 2700

aagcggcugg acauuuggag caggcgccgc ucugcagauc cccuuugcua ugcagauggc 2760

cuaccgguuc aacggcaucg gagugaccca gaaugugcug uacgagaacc agaagcugau 2820

cgccaaccag uucaacagcg ccaucggcaa gauccaggac agccugagca gcacagcaag 2880

cgcccuggga aagcugcagg acguggucaa ccagaaugcc caggcacuga acacccuggu 2940

caagcagcug uccuccaacu ucggcgccau cagcucugug cugaacgaua uccugagcag 3000

acuggacccu ccugaggccg aggugcagau cgacagacug aucacaggca gacugcagag 3060

ccuccagaca uacgugaccc agcagcugau cagagccgcc gagauuagag ccucugccaa 3120

ucuggccgcc accaagaugu cugagugugu gcugggccag agcaagagag uggacuuuug 3180

cggcaagggc uaccaccuga ugagcuuccc ucagucugcc ccucacggcg ugguguuucu 3240

gcacgugaca uaugugcccg cucaagagaa gaauuucacc accgcuccag ccaucugcca 3300

cgacggcaaa gcccacuuuc cuagagaagg cguguucgug uccaacggca cccauugguu 3360

cgugacacag cggaacuucu acgagcccca gaucaucacc accgacaaca ccuucguguc 3420

uggcaacugc gacgucguga ucggcauugu gaacaauacc guguacgacc cucugcagcc 3480

cgagcuggac agcuucaaag aggaacugga caaguacuuu aagaaccaca caagccccga 3540

cguggaccug ggcgauauca gcggaaucaa ugccagcguc gugaacaucc agaaagagau 3600

cgaccggcug aacgaggugg ccaagaaucu gaacgagagc cugaucgacc ugcaagaacu 3660

ggggaaguac gagcaguaca ucaaguggcc cugguacauc uggcugggcu uuaucgccgg 3720

acugauugcc aucgugaugg ucacaaucau gcuguguugc augaccagcu gcuguagcug 3780

ccugaagggc uguuguagcu guggcagcug cugcaaguuc gacgaggacg auucugagcc 3840

cgugcugaag ggcgugaaac ugcacuacac augaugacuc gagcugguac ugcaugcacg 3900

caaugcuagc ugccccuuuc ccguccuggg uaccccgagu cucccccgac cucggguccc 3960

agguaugcuc ccaccuccac cugccccacu caccaccucu gcuaguucca gacaccuccc 4020

aagcacgcag caaugcagcu caaaacgcuu agccuagcca cacccccacg ggaaacagca 4080

gugauuaacc uuuagcaaua aacgaaaguu uaacuaagcu auacuaaccc caggguuggu 4140

caauuucgug ccagccacac ccuggagcua gcaaaaaaaa aaaaaaaaaa aaaaaaaaaa 4200

aagcauauga cuaaaaaaaa aaaaaaaaaa aaaaaaaaaa aaaaaaaaaa aaaaaaaaaa 4260

aaaaaaaaaa aaaaaaaaaa aa 4282

<210> 17
<211> 1261
<212> RNA
<213> Artificial Sequence

<220>
<223> RBL063.3

<400> 17
gggcgaacua guauucuucu gguccccaca gacucagaga gaacccgcca ccauguuugu 60

guuucuugug cugcugccuc uugugucuuc ucagugugug gugagauuuc caaauauuac 120

aaaucugugu ccauuuggag aaguguuuaa ugcaacaaga uuugcaucug uguaugcaug 180

gaauagaaaa agaauuucua auuguguggc ugauuauucu gugcuguaua auagugcuuc 240

uuuuuccaca uuuaaauguu auggaguguc uccaacaaaa uuaaaugauu uauguuuuac 300

aaauguguau gcugauucuu uugugaucag aggugaugaa gugagacaga uugccccugg 360

acagacagga aaaauugcug auuacaauua caaacugccu gaugauuuua caggaugugu 420

gauugcuugg aauucuaaua auuuagauuc uaaaguggga ggaaauuaca auuaucugua 480

cagacuguuu agaaaaucaa aucugaaacc uuuugaaaga gauauuucaa cagaaauuua 540

ucaggcugga ucaacaccuu guaauggagu ggaaggauuu aauuguuauu uuccauuaca 600

gagcuaugga uuucagccaa ccaauggugu gggauaucag ccauauagag ugguggugcu 660

gucuuuugaa cugcugcaug caccugcaac agugugugga ccuaaaggcu cccccggcuc 720

cggcuccgga ucugguuaua uuccugaagc uccaagagau gggcaagcuu acguucguaa 780

agauggcgaa uggguauuac uuucuaccuu uuuaggccgg ucccuggagg ugcuguucca 840

gggccccggc ugaugacucg agcugguacu gcaugcacgc aaugcuagcu gccccuuucc 900

cguccugggu accccgaguc ucccccgacc ucggguccca gguaugcucc caccuccacc 960

ugccccacuc accaccucug cuaguuccag acaccuccca agcacgcagc aaugcagcuc 1020

aaaacgcuua gccuagccac acccccacgg gaaacagcag ugauuaaccu uuagcaauaa 1080

acgaaaguuu aacuaagcua uacuaacccc aggguugguc aauuucgugc cagccacacc 1140

cuggagcuag caaaaaaaaa aaaaaaaaaa aaaaaaaaaa agcauaugac uaaaaaaaaa 1200

aaaaaaaaaa aaaaaaaaaa aaaaaaaaaa aaaaaaaaaa aaaaaaaaaa aaaaaaaaaa 1260

a                                                                    1261

<210>  18
<211>  250
<212>  PRT
<213>  Artificial Sequence

<220>
<223>  S Protein RBD Fusion

<400>  18

Val Arg Phe Pro Asn Ile Thr Asn Leu Cys Pro Phe Gly Glu Val Phe
1               5                   10                  15

Asn Ala Thr Arg Phe Ala Ser Val Tyr Ala Trp Asn Arg Lys Arg Ile
            20                  25                  30

Ser Asn Cys Val Ala Asp Tyr Ser Val Leu Tyr Asn Ser Ala Ser Phe
        35                  40                  45

Ser Thr Phe Lys Cys Tyr Gly Val Ser Pro Thr Lys Leu Asn Asp Leu
    50                  55                  60

Cys Phe Thr Asn Val Tyr Ala Asp Ser Phe Val Ile Arg Gly Asp Glu
65                  70                  75                  80

Val Arg Gln Ile Ala Pro Gly Gln Thr Gly Lys Ile Ala Asp Tyr Asn
                85                  90                  95

Tyr Lys Leu Pro Asp Asp Phe Thr Gly Cys Val Ile Ala Trp Asn Ser
            100                 105                 110

Asn Asn Leu Asp Ser Lys Val Gly Gly Asn Tyr Asn Tyr Leu Tyr Arg
        115                 120                 125

Leu Phe Arg Lys Ser Asn Leu Lys Pro Phe Glu Arg Asp Ile Ser Thr
    130                 135                 140

Glu Ile Tyr Gln Ala Gly Ser Thr Pro Cys Asn Gly Val Glu Gly Phe
145                 150                 155                 160

Asn Cys Tyr Phe Pro Leu Gln Ser Tyr Gly Phe Gln Pro Thr Asn Gly
                165                 170                 175

Val Gly Tyr Gln Pro Tyr Arg Val Val Val Leu Ser Phe Glu Leu Leu
            180                 185                 190

His Ala Pro Ala Thr Val Cys Gly Pro Lys Gly Ser Pro Gly Ser Gly
        195                 200                 205

Ser Gly Ser Gly Tyr Ile Pro Glu Ala Pro Arg Asp Gly Gln Ala Tyr
    210                 215                 220

Val Arg Lys Asp Gly Glu Trp Val Leu Leu Ser Thr Phe Leu Gly Arg
225                 230                 235                 240

Ser Leu Glu Val Leu Phe Gln Gly Pro Gly
                245                 250

<210>  19
<211>  4283
<212>  RNA
<213>  Artificial Sequence

<220>
<223>  RBP020.1

<400>  19
agaauaaacu aguauucuuc ugguccccac agacucagag agaacccgcc accauguuug      60

uguuucuugu gcugcugccu cuugugucuu cucagugugu gaauuugaca acaagaacac     120

agcugccacc agcuuauaca aauucuuuua ccagaggagu guauuauccu gauaaagugu     180

uuagaucuuc ugugcugcac agcacacagg accuguuucu gccauuuuuu agcaauguga     240

caugguuuca ugcaauucau gugucuggaa caaauggaac aaaaagauuu gauaauccug     300

ugcugccuuu uaaugaugga guguauuuug cuucaacaga aaagucaaau auuauuagag     360

gauggauuuu uggaacaaca cuggauucua aaacacaguc ucugcugauu gugaauaaug     420

caacaaaugu ggugauuaaa gugugugaau uucaguuuug uaaugauccu uuucugggag 480

uguauuauca caaaaauaau aaaucuugga uggaaucuga auuuagagug uauuccucug 540

caaauaauug uacauuugaa uaugugucuc agccuuuucu gauggaucug gaaggaaaac 600

agggcaauuu uaaaaaucug agagaauuug uguuuaaaaa uauugaugga uauuuuaaaa 660

uuuauucuaa acacacacca auuaauuuag ugagagaucu gccucaggga uuuucugcuc 720

uggaaccucu gguggaucug ccaauuggca uuaauauuac aagauuucag acacugcugg 780

cucugcacag aucuuaucug acaccuggag auucuucuuc uggauggaca gccggagcug 840

cagcuuauua ugugggcuau cugcagccaa gaacauuucu gcugaaauau aaugaaaaug 900

gaacaauuac agaugcugug gauugugcuc uggauccucu gucugaaaca aaauguacau 960

uaaaaucuuu uacaguggaa aaaggcauuu aucagacauc uaauuuuaga gugcagccaa 1020

cagaaucuau ugugagauuu ccaaauauua caaaucugug uccauuugga gaaguguuua 1080

augcaacaag auuugcaucu guguaugcau ggaauagaaa aagaauuucu aauugugugg 1140

cugauuauuc ugugcuguau aauagugcuu cuuuuuccac auuuaaaugu uauggagugu 1200

cuccaacaaa auuaaaugau uuauguuuua caaaugugua ugcugauucu uuugugauca 1260

gaggugauga agugagacag auugcccccg gacagacagg aaaaauugcu gauuacaauu 1320

acaaacugcc ugaugauuuu acaggaugug ugauugcuug gaauucuaau aauuuagauu 1380

cuaaaguggg aggaaauuac aauuaucugu acagacuguu uagaaaauca aaucugaaac 1440

cuuuugaaag agauauuuca acagaaauuu aucaggcugg aucaacaccu uguaauggag 1500

uggaaggauu uaauuguuau uuuccauuac agagcuaugg auuucagcca accaauggug 1560

ugggauauca gccauauaga gugguggugc ugucuuuuga acugcugcau gcaccugcaa 1620

cagugugugg accuaaaaaa ucuacaaauu uagugaaaaa uaaaugugug aauuuuaauu 1680

uuaauggauu aacaggaaca ggagugcuga cagaaucuaa uaaaaaauuu cugccuuuuc 1740

agcaguuugg cagagauauu gcagauacca cagaugcagu gagagauccu cagacauuag 1800

aaauucugga uauuacaccu uguucuuuug gggguguguc ugugauuaca ccuggaacaa 1860

auacaucuaa ucagguggcu gugcuguauc aggaugugaa uuguacagaa gugccagugg 1920

caauucaugc agaucagcug acaccaacau ggagagugua uucuacagga ucuaaugugu 1980

uucagacaag agcaggaugu cugauuggag cagaacaugu gaauaauucu uaugaaugug 2040

auauuccaau uggagcaggc auuugugcau cuuaucagac acagacaaau uccccaagga 2100

gagcaagauc uguggcaucu cagucuauua uugcauacac caugucucug ggagcagaaa 2160

auucuguggc auauucuaau aauucuauug cuauuccaac aaauuuuacc auuucuguga 2220

caacagaaau uuuaccugug ucuaugacaa aaacaucugu ggauuguacc auguacauuu 2280

guggagauuc uacagaaugu ucuaaucugc ugcugcagua uggaucuuuu uguacacagc 2340

ugaauagagc uuuaacagga auugcugugg aacaggauaa aaauacacag gaaguguuug 2400

cucaggugaa acagauuuac aaaacaccac caauuaaaga uuuuggagga uuuaauuuua 2460

gccagauucu gccugauccu ucuaaaccuu cuaaaagauc uuuuauugaa gaucugcugu 2520

uuaauaaagu gacacuggca gaugcaggau uuauuaaaca guauggagau ugccugggug 2580

auauugcugc aagagaucug auuugugcuc agaaauuuaa uggacugaca gugcugccuc 2640

cucugcugac agaugaaaug auugcucagu acacaucugc uuuacuggcu ggaacaauua 2700

caagcggaug gacauuugga gcuggagcug cucugcagau uccuuuugca augcagaugg 2760

cuuacagauu uaauggaauu ggagugacac agaauguguu auaugaaaau cagaaacuga 2820

uugcaaauca guuuaauucu gcaauuggca aaauucagga uucucugucu ucuacagcuu 2880

cugcucuggg aaaacugcag gaugugguga aucagaaugc acaggcacug aauacucugg 2940

ugaaacagcu gucuagcaau uuuggggcaa uuucuucugu gcugaaugau auucugucua 3000

gacuggaucc uccugaagcu gaagugcaga uugauagacu gaucacagga agacugcagu 3060

cucugcagac uuaugugaca cagcagcuga uuagagcugc ugaaauuaga gcuucugcua 3120

aucuggcugc uacaaaaaug ucugaaugug ugcugggaca gucaaaaaga guggauuuuu 3180

guggaaaagg auaucaucug augucuuuuc cacagucugc uccacaugga gugguguuuu 3240

uacaugugac auaugugcca gcacaggaaa agaauuuuac cacagcacca gcaauuuguc 3300

augauggaaa agcacauuuu ccaagagaag gaguguuugu gucuaaugga acacauuggu 3360

uugugacaca gagaaauuuu uaugaaccuc agauuauuac aacagauaau acauuugugu 3420

caggaaauug ugauguggug auuggaauug ugaauaauac aguguaugau ccacugcagc 3480

cagaacugga uucuuuuaaa gaagaacugg auaaauauuu uaaaaaucac acaucuccug 3540

auguggauuu aggagauauu ucuggaauca augcaucugu ggugaauauu cagaaagaaa 3600

uugauagacu gaaugaagug gccaaaaauc ugaaugaauc ucugauugau cugcaggaac 3660

uuggaaaaua ugaacaguac auuaaauggc cuugguacau uuggcuugga uuuauugcag 3720

gauuaauugc aauugugaug gugacaauua uguuauguug uaugacauca uguuguucuu 3780

guuuaaaagg auguuguucu uguggaagcu guuguaaauu ugaugaagau gauucugaac 3840

cuguguuaaa aggagugaaa uugcauuaca caugaugacu cgagcuggua cugcaugcac 3900

gcaaugcuag cugccccuuu cccguccugg guaccccgag ucucccccga ccucgggucc 3960

cagguaugcu cccaccucca ccugccccac ucaccaccuc ugcuaguucc agacaccucc 4020

caagcacgca gcaaugcagc ucaaaacgcu uagccuagcc acacccccac gggaaacagc 4080

agugauuaac cuuuagcaau aaacgaaagu uuaacuaagc uauacuaacc ccaggguugg 4140

ucaauuucgu gccagccaca cccuggagcu agcaaaaaaa aaaaaaaaaa aaaaaaaaaa 4200

aaagcauaug acuaaaaaaa aaaaaaaaaa aaaaaaaaaa aaaaaaaaaa aaaaaaaaaa 4260

aaaaaaaaaa aaaaaaaaaa aaa 4283

<210> 20
<211> 4283
<212> RNA
<213> Artificial Sequence

<220>
<223> RBP020.2

<400> 20
agaauaaacu aguauucuuc ugguccccac agacucagag agaacccgcc accauguucg 60

uguuccuggu gcugcugccu cuggugucca gccagugugu gaaccugacc accagaacac 120

agcugccucc agccuacacc aacagcuuua ccagaggcgu guacuacccc gacaaggugu 180

ucagauccag cgugcugcac ucuacccagg accuguuccu gccuuucuuc agcaacguga 240

ccugguucca cgccauccac guguccggca ccaauggcac caagagauuc gacaaccccg 300

ugcugcccuu caacgacggg guguacuuug ccagcaccga gaaguccaac aucaucagag 360

gcuggaucuu cggcaccaca cuggacagca agacccagag ccugcugauc gugaacaacg 420

ccaccaacgu ggucaucaaa gugugcgagu uccaguucug caacgacccc uuccugggcg 480

ucuacuacca caagaacaac aagagcugga uggaaagcga guuccgggug uacagcagcg 540

ccaacaacug caccuucgag uacguguccc agccuuuccu gauggaccug gaaggcaagc 600

agggcaacuu caagaaccug cgcgaguucg uguuuaagaa caucgacggc uacuucaaga 660

ucuacagcaa gcacaccccu aucaaccucg ugcgggaucu gccucagggc uucucugcuc 720

uggaaccccu gguggaucug cccaucggca ucaacaucac ccgguuucag acacugcugg 780

cccugcacag aagcuaccug acaccuggcg auagcagcag cggauggaca gcuggugccg 840

ccgcuuacua ugugggcuac cugcagccua gaaccuuccu gcugaaguac aacgagaacg 900

gcaccaucac cgacgccgug gauugugcuc uggauccucu gagcgagaca aagugcaccc 960

ugaaguccuu caccguggaa aagggcaucu accagaccag caacuuccgg gugcagccca 1020

ccgaauccau cgugcgguuc cccaauauca ccaaucugug ccccuucggc gagguguuca 1080

augccaccag auucgccucu guguacgccu ggaaccggaa gcggaucagc aauugcgugg 1140

ccgacuacuc cgugcuguac aacuccgcca gcuucagcac cuucaagugc uacggcgugu 1200

ccccuaccaa gcugaacgac cugugcuuca caaacgugua cgccgacagc uucgugaucc 1260

ggggagauga agugcggcag auugccccug gacagacagg caagaucgcc gacuacaacu 1320

acaagcugcc cgacgacuuc accggcugug ugauugccug gaacagcaac aaccuggacu 1380

ccaaagucgg cggcaacuac aauuaccugu accggcuguu ccggaagucc aaucugaagc 1440

ccuucgagcg ggacaucucc accgagaucu aucaggccgg cagcaccccu uguaacggcg 1500

uggaaggcuu caacugcuac uucccacugc aguccuacgg cuuucagccc acaaauggcg 1560

ugggcuauca gcccuacaga gugguggugc ugagcuucga acugcugcau gccccugcca 1620

cagugugcgg cccuaagaaa agcaccaauc ucgugaagaa caaaugcgug aacuucaacu 1680

ucaacggccu gaccggcacc ggcgugcuga cagagagcaa caagaaguuc cugccauucc 1740

agcaguuugg ccgggauauc gccgauacca cagacgccgu uagagauccc cagacacugg 1800

aaauccugga caucaccccu ugcagcuucg gcggaguguc ugugaucacc ccuggcacca 1860

acaccagcaa ucagguggca gugcuguacc aggacgugaa cuguaccgaa gugcccgugg 1920

ccauucacgc cgaucagcug acaccuacau ggcgggugua cuccaccggc agcaaugugu 1980

uucagaccag agccggcugu cugaucggag ccgagcacgu gaacaauagc uacgagugcg 2040

acauccccau cggcgcugga aucugcgcca gcuaccagac acagacaaac agcccucgga 2100

gagccagaag cguggccagc cagagcauca uugccuacac aaugucucug ggcgccgaga 2160

acagcguggc cuacuccaac aacucuaucg cuauccccac caacuucacc aucagcguga 2220

ccacagagau ccugccugug uccaugacca agaccagcgu ggacugcacc auguacaucu 2280

gcggcgauuc caccgagugc uccaaccugc ugcugcagua cggcagcuuc ugcacccagc 2340

ugaauagagc ccugacaggg aucgccgugg aacaggacaa gaacacccaa gagguguucg 2400

cccaagugaa gcagaucuac aagaccccuc cuaucaagga cuucggcggc uucaauuuca 2460

gccagauucu gcccgauccu agcaagccca gcaagcggag cuucaucgag gaccugcugu 2520

ucaacaaagu gacacuggcc gacgccggcu ucaucaagca guauggcgau ugucugggcg 2580

acauugccgc cagggaucug auuugcgccc agaaguuuaa cggacugaca gugcugccuc 2640

cucugcugac cgaugagaug aucgcccagu acacaucugc ccugcuggcc ggcacaauca 2700

caagcggcug gacauuugga gcaggcgccg cucugcagau ccccuuugcu augcagaugg 2760

ccuaccgguu caacggcauc ggagugaccc agaaugugcu guacgagaac cagaagcuga 2820

ucgccaacca guucaacagc gccaucggca agauccagga cagccugagc agcacagcaa 2880

gcgcccuggg aaagcugcag gacgugguca accagaaugc ccaggcacug aacacccugg 2940

ucaagcagcu guccuccaac uucggcgcca ucagcucugu gcugaacgau auccugagca 3000

gacuggaccc uccugaggcc gaggugcaga ucgacagacu gaucacaggc agacugcaga 3060

gccuccagac auacgugacc cagcagcuga ucagagccgc cgagauuaga gccucugcca 3120

aucuggccgc caccaagaug ucugagugug ugcugggcca gagcaagaga guggacuuuu 3180

gcggcaaggg cuaccaccug augagcuucc cucagucugc cccucacggc gugguguuuc 3240

ugcacgugac auaugugccc gcucaagaga agaauuucac caccgcucca gccaucugcc 3300

acgacggcaa agcccacuuu ccuagagaag gcguguucgu guccaacggc acccauuggu 3360

ucgugacaca gcggaacuuc uacgagcccc agaucaucac caccgacaac accuucgugu 3420

cuggcaacug cgacgucgug aucggcauug ugaacaauac cguguacgac ccucugcagc 3480

ccgagcugga cagcuucaaa gaggaacugg acaaguacuu uaagaaccac acaagccccg 3540

acguggaccu gggcgauauc agcggaauca augccagcgu cgugaacauc cagaaagaga 3600

ucgaccggcu gaacgaggug gccaagaauc ugaacgagag ccugaucgac cugcaagaac 3660

uggggaagua cgagcaguac aucaaguggc ccugguacau cuggcugggc uuuaucgccg 3720

gacugauugc caucgugaug gucacaauca ugcuguguug caugaccagc ugcuguagcu 3780

gccugaaggg cuguuguagc uguggcagcu gcugcaaguu cgacgaggac gauucugagc 3840

ccgugcugaa gggcgugaaa cugcacuaca caugaugacu cgagcuggua cugcaugcac 3900

gcaaugcuag cugccccuuu cccguccugg guaccccgag ucucccccga ccucgggucc 3960

cagguaugcu cccaccucca ccugccccac ucaccaccuc ugcuaguucc agacaccucc 4020

caagcacgca gcaaugcagc ucaaaacgcu uagccuagcc acacccccac gggaaacagc 4080

agugauuaac cuuuagcaau aaacgaaagu uuaacuaagc uauacuaacc ccaggguugg 4140

ucaauuucgu gccagccaca cccuggagcu agcaaaaaaa aaaaaaaaaa aaaaaaaaaa 4200

aaagcauaug acuaaaaaaa aaaaaaaaaa aaaaaaaaaa aaaaaaaaaa aaaaaaaaaa 4260

aaaaaaaaaa aaaaaaaaaa aaa 4283

<210> 21
<211> 1262
<212> RNA
<213> Artificial Sequence

<220>
<223> RBP020.3

<400> 21
agaauaaacu aguauucuuc ugguccccac agacucagag agaacccgcc accauguuug 60

uguuucuugu gcugcugccu cuugugucuu cucagugugu ggugagauuu ccaaauauua 120

caaaucugug uccauuugga gaaguguuua augcaacaag auuugcaucu guguaugcau 180

ggaauagaaa aagaauuucu aauugugugg cugauuauuc ugugcuguau aauagugcuu 240

cuuuuuccac auuuaaaugu uauggagugu cuccaacaaa auuaaaugau uuauguuuua 300

caaaugugua ugcugauucu uuugugauca gaggugauga agugagacag auugcccccg 360

gacagacagg aaaaauugcu gauuacaauu acaaacugcc ugaugauuuu acaggaugug 420

ugauugcuug gaauucuaau aauuuagauu cuaaaguggg aggaaauuac aauuaucugu 480

acagacuguu uagaaaauca aaucugaaac cuuuugaaag agauauuuca acagaaauuu 540

aucaggcugg aucaacaccu uguaauggag uggaaggauu uaauuguuau uuuccauuac 600

agagcuaugg auuucagcca accaauggug ugggauauca gccauauaga gugguggugc 660

ugucuuuuga acugcugcau gcaccugcaa cagugugugg accuaaaggc ucccccggcu 720

ccggcuccgg aucugguuau auuccugaag cuccaagaga ugggcaagcu uacguucgua 780

aagauggcga auggguauua cuuucuaccu uuuuaggccg gucccuggag gugcuguucc 840

agggccccgg cugaugacuc gagcugguac ugcaugcacg caaugcuagc ugccccuuuc 900

ccguccuggg uaccccgagu cucccccgac cucggguccc agguaugcuc ccaccuccac 960

cugccccacu caccaccucu gcuaguucca gacaccuccc aagcacgcag caaugcagcu 1020

caaaacgcuu agccuagcca cacccccacg ggaaacagca gugauuaacc uuuagcaaua 1080

aacgaaaguu uaacuaagcu auacuaaccc caggguuggu caauuucgug ccagccacac 1140

ccuggagcua gcaaaaaaaa aaaaaaaaaa aaaaaaaaaa aagcauauga cuaaaaaaaa 1200

aaaaaaaaaa aaaaaaaaaa aaaaaaaaaa aaaaaaaaaa aaaaaaaaaa aaaaaaaaaa 1260

aa 1262

<210> 22
<211> 1879
<212> PRT
<213> Artificial Sequence

<220>
<223> Viral Protein

<400> 22

Met Glu Lys Val His Val Asp Ile Glu Glu Asp Ser Pro Phe Leu Arg
1               5                   10                  15

Ala Leu Gln Arg Ser Phe Pro Gln Phe Glu Val Glu Ala Lys Gln Val
            20                  25                  30

Thr Asp Asn Asp His Ala Asn Ala Arg Ala Phe Ser His Leu Ala Ser
        35                  40                  45

Lys Leu Ile Glu Thr Glu Val Asp Pro Ser Asp Thr Ile Leu Asp Ile
    50                  55                  60

Gly Ser Ala Pro Ala Arg Arg Met Tyr Ser Lys His Lys Tyr His Cys
65                  70                  75                  80

Ile Cys Pro Met Arg Cys Ala Glu Asp Pro Asp Arg Leu Tyr Lys Tyr
                85                  90                  95

Ala Thr Lys Leu Lys Lys Asn Cys Lys Glu Ile Thr Asp Lys Glu Leu
            100                 105                 110

Asp Lys Lys Met Lys Glu Leu Ala Ala Val Met Ser Asp Pro Asp Leu
        115                 120                 125

Glu Thr Glu Thr Met Cys Leu His Asp Asp Glu Ser Cys Arg Tyr Glu
    130                 135                 140

Gly Gln Val Ala Val Tyr Gln Asp Val Tyr Ala Val Asp Gly Pro Thr
145                 150                 155                 160

Ser Leu Tyr His Gln Ala Asn Lys Gly Val Arg Val Ala Tyr Trp Ile
                165                 170                 175

Gly Phe Asp Thr Thr Pro Phe Met Phe Lys Asn Leu Ala Gly Ala Tyr
            180                 185                 190

Pro Ser Tyr Ser Thr Asn Trp Ala Asp Glu Thr Val Leu Thr Ala Arg
        195                 200                 205

Asn Ile Gly Leu Cys Ser Ser Asp Val Met Glu Arg Ser Arg Arg Gly
    210                 215                 220

Met Ser Ile Leu Arg Lys Lys Tyr Leu Lys Pro Ser Asn Asn Val Leu
225                 230                 235                 240

Phe Ser Val Gly Ser Thr Ile Tyr His Glu Lys Arg Asp Leu Leu Arg
                245                 250                 255

Ser Trp His Leu Pro Ser Val Phe His Leu Arg Gly Lys Gln Asn Tyr
            260                 265                 270

Thr Cys Arg Cys Glu Thr Ile Val Ser Cys Asp Gly Tyr Val Val Lys
        275                 280                 285

Arg Ile Ala Ile Ser Pro Gly Leu Tyr Gly Lys Pro Ser Gly Tyr Ala
    290                 295                 300

Ala Thr Met His Arg Glu Gly Phe Leu Cys Cys Lys Val Thr Asp Thr
305                 310                 315                 320

Leu Asn Gly Glu Arg Val Ser Phe Pro Val Cys Thr Tyr Val Pro Ala
                325                 330                 335

Thr Leu Cys Asp Gln Met Thr Gly Ile Leu Ala Thr Asp Val Ser Ala
            340                 345                 350

Asp Asp Ala Gln Lys Leu Leu Val Gly Leu Asn Gln Arg Ile Val Val
        355                 360                 365

Asn Gly Arg Thr Gln Arg Asn Thr Asn Thr Met Lys Asn Tyr Leu Leu
    370                 375                 380

Pro Val Val Ala Gln Ala Phe Ala Arg Trp Ala Lys Glu Tyr Lys Glu
385                 390                 395                 400

Asp Gln Glu Asp Glu Arg Pro Leu Gly Leu Arg Asp Arg Gln Leu Val
                405                 410                 415

Met Gly Cys Cys Trp Ala Phe Arg Arg His Lys Ile Thr Ser Ile Tyr
            420                 425                 430

Lys Arg Pro Asp Thr Gln Thr Ile Ile Lys Val Asn Ser Asp Phe His
        435                 440                 445

Ser Phe Val Leu Pro Arg Ile Gly Ser Asn Thr Leu Glu Ile Gly Leu
    450                 455                 460

Arg Thr Arg Ile Arg Lys Met Leu Glu Glu His Lys Glu Pro Ser Pro
465                 470                 475                 480

Leu Ile Thr Ala Glu Asp Val Gln Glu Ala Lys Cys Ala Ala Asp Glu
                485                 490                 495

Ala Lys Glu Val Arg Glu Ala Glu Glu Leu Arg Ala Ala Leu Pro Pro
            500                 505                 510

Leu Ala Ala Asp Val Glu Glu Pro Thr Leu Glu Ala Asp Val Asp Leu
        515                 520                 525

Met Leu Gln Glu Ala Gly Ala Gly Ser Val Glu Thr Pro Arg Gly Leu
    530                 535                 540

Ile Lys Val Thr Ser Tyr Ala Gly Glu Asp Lys Ile Gly Ser Tyr Ala
545                 550                 555                 560

Val Leu Ser Pro Gln Ala Val Leu Lys Ser Glu Lys Leu Ser Cys Ile
                565                 570                 575

His Pro Leu Ala Glu Gln Val Ile Val Ile Thr His Ser Gly Arg Lys
            580                 585                 590

Gly Arg Tyr Ala Val Glu Pro Tyr His Gly Lys Val Val Val Pro Glu
        595                 600                 605

Gly His Ala Ile Pro Val Gln Asp Phe Gln Ala Leu Ser Glu Ser Ala
    610                 615                 620

Thr Ile Val Tyr Asn Glu Arg Glu Phe Val Asn Arg Tyr Leu His His
625                 630                 635                 640

Ile Ala Thr His Gly Gly Ala Leu Asn Thr Asp Glu Glu Tyr Tyr Lys
                645                 650                 655

Thr Val Lys Pro Ser Glu His Asp Gly Glu Tyr Leu Tyr Asp Ile Asp
            660                 665                 670

Arg Lys Gln Cys Val Lys Lys Glu Leu Val Thr Gly Leu Gly Leu Thr
        675                 680                 685

Gly Glu Leu Val Asp Pro Pro Phe His Glu Phe Ala Tyr Glu Ser Leu
    690                 695                 700

Arg Thr Arg Pro Ala Ala Pro Tyr Gln Val Pro Thr Ile Gly Val Tyr
705                 710                 715                 720

Gly Val Pro Gly Ser Gly Lys Ser Gly Ile Ile Lys Ser Ala Val Thr
                725                 730                 735

Lys Lys Asp Leu Val Val Ser Ala Lys Lys Glu Asn Cys Ala Glu Ile
            740                 745                 750

Ile Arg Asp Val Lys Lys Met Lys Gly Leu Asp Val Asn Ala Arg Thr
        755                 760                 765

Val Asp Ser Val Leu Leu Asn Gly Cys Lys His Pro Val Glu Thr Leu
    770                 775                 780

Tyr Ile Asp Glu Ala Phe Ala Cys His Ala Gly Thr Leu Arg Ala Leu
785                 790                 795                 800

Ile Ala Ile Ile Arg Pro Lys Lys Ala Val Leu Cys Gly Asp Pro Lys
                805                 810                 815

Gln Cys Gly Phe Phe Asn Met Met Cys Leu Lys Val His Phe Asn His
            820                 825                 830

Glu Ile Cys Thr Gln Val Phe His Lys Ser Ile Ser Arg Arg Cys Thr
        835                 840                 845

Lys Ser Val Thr Ser Val Val Ser Thr Leu Phe Tyr Asp Lys Lys Met
    850                 855                 860

Arg Thr Thr Asn Pro Lys Glu Thr Lys Ile Val Ile Asp Thr Thr Gly
865                 870                 875                 880

Ser Thr Lys Pro Lys Gln Asp Asp Leu Ile Leu Thr Cys Phe Arg Gly
                885                 890                 895

Trp Val Lys Gln Leu Gln Ile Asp Tyr Lys Gly Asn Glu Ile Met Thr
            900                 905                 910

Ala Ala Ala Ser Gln Gly Leu Thr Arg Lys Gly Val Tyr Ala Val Arg
        915                 920                 925

Tyr Lys Val Asn Glu Asn Pro Leu Tyr Ala Pro Thr Ser Glu His Val
    930                 935                 940

Asn Val Leu Leu Thr Arg Thr Glu Asp Arg Ile Val Trp Lys Thr Leu
945                 950                 955                 960

Ala Gly Asp Pro Trp Ile Lys Thr Leu Thr Ala Lys Tyr Pro Gly Asn
                965                 970                 975

Phe Thr Ala Thr Ile Glu Glu Trp Gln Ala Glu His Asp Ala Ile Met
            980                 985                 990

Arg His Ile Leu Glu Arg Pro Asp  Pro Thr Asp Val Phe  Gln Asn Lys
        995                 1000                1005

Ala Asn Val Cys Trp Ala Lys Ala Leu Val Pro Val Leu Lys Thr
    1010                1015                1020

Ala Gly Ile Asp Met Thr Thr Glu Gln Trp Asn Thr Val Asp Tyr
    1025                1030                1035

Phe Glu Thr Asp Lys Ala His Ser Ala Glu Ile Val Leu Asn Gln
    1040                1045                1050

Leu Cys Val Arg Phe Phe Gly Leu Asp Leu Asp Ser Gly Leu Phe
    1055                1060                1065

Ser Ala Pro Thr Val Pro Leu Ser Ile Arg Asn Asn His Trp Asp
    1070                1075                1080

Asn Ser Pro Ser Pro Asn Met Tyr Gly Leu Asn Lys Glu Val Val
    1085                1090                1095

Arg Gln Leu Ser Arg Arg Tyr Pro Gln Leu Pro Arg Ala Val Ala
    1100                1105                1110

Thr Gly Arg Val Tyr Asp Met Asn Thr Gly Thr Leu Arg Asn Tyr
    1115                1120                1125

Asp Pro Arg Ile Asn Leu Val Pro Val Asn Arg Arg Leu Pro His
    1130                1135                1140

Ala Leu Val Leu His His Asn Glu His Pro Gln Ser Asp Phe Ser
    1145                1150                1155

Ser Phe Val Ser Lys Leu Lys Gly Arg Thr Val Leu Val Val Gly
    1160                1165                1170

Glu Lys Leu Ser Val Pro Gly Lys Met Val Asp Trp Leu Ser Asp
    1175                1180                1185

Arg Pro Glu Ala Thr Phe Arg Ala Arg Leu Asp Leu Gly Ile Pro
    1190                1195                1200

Gly Asp Val Pro Lys Tyr Asp Ile Ile Phe Val Asn Val Arg Thr
    1205                1210                1215

Pro Tyr Lys Tyr His His Tyr Gln Gln Cys Glu Asp His Ala Ile
    1220                1225                1230

Lys Leu Ser Met Leu Thr Lys Lys Ala Cys Leu His Leu Asn Pro
    1235                1240                1245

Gly Gly Thr Cys Val Ser Ile Gly Tyr Gly Tyr Ala Asp Arg Ala
    1250                1255                1260

Ser Glu Ser Ile Ile Gly Ala Ile Ala Arg Gln Phe Lys Phe Ser
    1265                1270                1275

Arg Val Cys Lys Pro Lys Ser Ser Leu Glu Glu Thr Glu Val Leu
    1280                1285                1290

Phe Val Phe Ile Gly Tyr Asp Arg Lys Ala Arg Thr His Asn Pro
    1295                1300                1305

Tyr Lys Leu Ser Ser Thr Leu Thr Asn Ile Tyr Thr Gly Ser Arg
    1310                1315                1320

Leu His Glu Ala Gly Cys Ala Pro Ser Tyr His Val Val Arg Gly
    1325                1330                1335

Asp Ile Ala Thr Ala Thr Glu Gly Val Ile Ile Asn Ala Ala Asn
    1340                1345                1350

Ser Lys Gly Gln Pro Gly Gly Gly Val Cys Gly Ala Leu Tyr Lys
    1355                1360                1365

Lys Phe Pro Glu Ser Phe Asp Leu Gln Pro Ile Glu Val Gly Lys
    1370                1375                1380

Ala Arg Leu Val Lys Gly Ala Ala Lys His Ile Ile His Ala Val
    1385                1390                1395

Gly Pro Asn Phe Asn Lys Val Ser Glu Val Glu Gly Asp Lys Gln
    1400                1405                1410

Leu Ala Glu Ala Tyr Glu Ser Ile Ala Lys Ile Val Asn Asp Asn
    1415                1420                1425

Asn Tyr Lys Ser Val Ala Ile Pro Leu Leu Ser Thr Gly Ile Phe
    1430                1435                1440

Ser Gly Asn Lys Asp Arg Leu Thr Gln Ser Leu Asn His Leu Leu
    1445                1450                1455

Thr Ala Leu Asp Thr Thr Asp Ala Asp Val Ala Ile Tyr Cys Arg
    1460                1465                1470

Asp Lys Lys Trp Glu Met Thr Leu Lys Glu Ala Val Ala Arg Arg
    1475                1480                1485

Glu Ala Val Glu Glu Ile Cys Ile Ser Asp Asp Ser Ser Val Thr
    1490                1495                1500

Glu Pro Asp Ala Glu Leu Val Arg Val His Pro Lys Ser Ser Leu
    1505                1510                1515

Ala Gly Arg Lys Gly Tyr Ser Thr Ser Asp Gly Lys Thr Phe Ser
    1520                1525                1530

Tyr Leu Glu Gly Thr Lys Phe His Gln Ala Ala Lys Asp Ile Ala
    1535                1540                1545

Glu Ile Asn Ala Met Trp Pro Val Ala Thr Glu Ala Asn Glu Gln
    1550                1555                1560

Val Cys Met Tyr Ile Leu Gly Glu Ser Met Ser Ser Ile Arg Ser
    1565                1570                1575

Lys Cys Pro Val Glu Glu Ser Glu Ala Ser Thr Pro Pro Ser Thr
    1580                1585                1590

Leu Pro Cys Leu Cys Ile His Ala Met Thr Pro Glu Arg Val Gln
    1595                1600                1605

Arg Leu Lys Ala Ser Arg Pro Glu Gln Ile Thr Val Cys Ser Ser
    1610                1615                1620

Phe Pro Leu Pro Lys Tyr Arg Ile Thr Gly Val Gln Lys Ile Gln
    1625                1630                1635

Cys Ser Gln Pro Ile Leu Phe Ser Pro Lys Val Pro Ala Tyr Ile
    1640                1645                1650

His Pro Arg Lys Tyr Leu Val Glu Thr Pro Pro Val Asp Glu Thr
    1655                1660                1665

Pro Glu Pro Ser Ala Glu Asn Gln Ser Thr Glu Gly Thr Pro Glu
    1670                1675                1680

Gln Pro Pro Leu Ile Thr Glu Asp Glu Thr Arg Thr Arg Thr Pro
    1685                1690                1695

Glu Pro Ile Ile Ile Glu Glu Glu Glu Glu Asp Ser Ile Ser Leu
    1700                1705                1710

Leu Ser Asp Gly Pro Thr His Gln Val Leu Gln Val Glu Ala Asp
    1715                1720                1725

Ile His Gly Pro Pro Ser Val Ser Ser Ser Ser Trp Ser Ile Pro
    1730                1735                1740

His Ala Ser Asp Phe Asp Val Asp Ser Leu Ser Ile Leu Asp Thr
    1745                1750                1755

Leu Glu Gly Ala Ser Val Thr Ser Gly Ala Thr Ser Ala Glu Thr
    1760                1765                1770

Asn Ser Tyr Phe Ala Lys Ser Met Glu Phe Leu Ala Arg Pro Val
    1775                1780                1785

Pro Ala Pro Arg Thr Val Phe Arg Asn Pro Pro His Pro Ala Pro
    1790                1795                1800

Arg Thr Arg Thr Pro Ser Leu Ala Pro Ser Arg Ala Cys Ser Arg
    1805                1810                1815

Thr Ser Leu Val Ser Thr Pro Pro Gly Val Asn Arg Val Ile Thr
    1820                1825                1830

Arg Glu Glu Leu Glu Ala Leu Thr Pro Ser Arg Thr Pro Ser Arg
    1835                1840                1845

Ser Val Ser Arg Thr Ser Leu Val Ser Asn Pro Pro Gly Val Asn
    1850                1855                1860

Arg Val Ile Thr Arg Glu Glu Phe Glu Ala Phe Val Ala Gln Gln
    1865                1870                1875

Gln

<210>  23
<211>  613
<212>  PRT
<213>  Artificial Sequence

<220>
<223>  Viral Protein

<400>  23

Arg Phe Asp Ala Gly Ala Tyr Ile Phe Ser Ser Asp Thr Gly Gln Gly
1               5                   10                  15

His Leu Gln Gln Lys Ser Val Arg Gln Thr Val Leu Ser Glu Val Val
            20                  25                  30

Leu Glu Arg Thr Glu Leu Glu Ile Ser Tyr Ala Pro Arg Leu Asp Gln
        35                  40                  45

Glu Lys Glu Glu Leu Leu Arg Lys Lys Leu Gln Leu Asn Pro Thr Pro
    50                  55                  60

Ala Asn Arg Ser Arg Tyr Gln Ser Arg Lys Val Glu Asn Met Lys Ala
65                  70                  75                  80

Ile Thr Ala Arg Arg Ile Leu Gln Gly Leu Gly His Tyr Leu Lys Ala
                85                  90                  95

Glu Gly Lys Val Glu Cys Tyr Arg Thr Leu His Pro Val Pro Leu Tyr
            100                 105                 110

Ser Ser Ser Val Asn Arg Ala Phe Ser Ser Pro Lys Val Ala Val Glu
        115                 120                 125

Ala Cys Asn Ala Met Leu Lys Glu Asn Phe Pro Thr Val Ala Ser Tyr
    130                 135                 140

Cys Ile Ile Pro Glu Tyr Asp Ala Tyr Leu Asp Met Val Asp Gly Ala
145                 150                 155                 160

Ser Cys Cys Leu Asp Thr Ala Ser Phe Cys Pro Ala Lys Leu Arg Ser
                165                 170                 175

Phe Pro Lys Lys His Ser Tyr Leu Glu Pro Thr Ile Arg Ser Ala Val
            180                 185                 190

Pro Ser Ala Ile Gln Asn Thr Leu Gln Asn Val Leu Ala Ala Ala Thr
        195                 200                 205

Lys Arg Asn Cys Asn Val Thr Gln Met Arg Glu Leu Pro Val Leu Asp
    210                 215                 220

Ser Ala Ala Phe Asn Val Glu Cys Phe Lys Lys Tyr Ala Cys Asn Asn
225                 230                 235                 240

Glu Tyr Trp Glu Thr Phe Lys Glu Asn Pro Ile Arg Leu Thr Glu Glu
                245                 250                 255

Asn Val Val Asn Tyr Ile Thr Lys Leu Lys Gly Pro Lys Ala Ala Ala
            260                 265                 270

Leu Phe Ala Lys Thr His Asn Leu Asn Met Leu Gln Asp Ile Pro Met
        275                 280                 285

Asp Arg Phe Val Met Asp Leu Lys Arg Asp Val Lys Val Thr Pro Gly
    290                 295                 300

Thr Lys His Thr Glu Glu Arg Pro Lys Val Gln Val Ile Gln Ala Ala
305                 310                 315                 320

Asp Pro Leu Ala Thr Ala Tyr Leu Cys Gly Ile His Arg Glu Leu Val
                325                 330                 335

Arg Arg Leu Asn Ala Val Leu Leu Pro Asn Ile His Thr Leu Phe Asp
            340                 345                 350

Met Ser Ala Glu Asp Phe Asp Ala Ile Ile Ala Glu His Phe Gln Pro
        355                 360                 365

Gly Asp Cys Val Leu Glu Thr Asp Ile Ala Ser Phe Asp Lys Ser Glu
    370                 375                 380

Asp Asp Ala Met Ala Leu Thr Ala Leu Met Ile Leu Glu Asp Leu Gly
385                 390                 395                 400

Val Asp Ala Glu Leu Leu Thr Leu Ile Glu Ala Ala Phe Gly Glu Ile
                405                 410                 415

Ser Ser Ile His Leu Pro Thr Lys Thr Lys Phe Lys Phe Gly Ala Met
                420                 425                 430

Met Lys Ser Gly Met Phe Leu Thr Leu Phe Val Asn Thr Val Ile Asn
            435                 440                 445

Ile Val Ile Ala Ser Arg Val Leu Arg Glu Arg Leu Thr Gly Ser Pro
    450                 455                 460

Cys Ala Ala Phe Ile Gly Asp Asp Asn Ile Val Lys Gly Val Lys Ser
465                 470                 475                 480

Asp Lys Leu Met Ala Asp Arg Cys Ala Thr Trp Leu Asn Met Glu Val
                485                 490                 495

Lys Ile Ile Asp Ala Val Val Gly Glu Lys Ala Pro Tyr Phe Cys Gly
                500                 505                 510

Gly Phe Ile Leu Cys Asp Ser Val Thr Gly Thr Ala Cys Arg Val Ala
            515                 520                 525

Asp Pro Leu Lys Arg Leu Phe Lys Leu Gly Lys Pro Leu Ala Ala Asp
    530                 535                 540

Asp Glu His Asp Asp Asp Arg Arg Arg Ala Leu His Glu Glu Ser Thr
545                 550                 555                 560

Arg Trp Asn Arg Val Gly Ile Leu Ser Glu Leu Cys Lys Ala Val Glu
                565                 570                 575

Ser Arg Tyr Glu Thr Val Gly Thr Ser Ile Ile Val Met Ala Met Thr
                580                 585                 590

Thr Leu Ala Ser Ser Val Lys Ser Phe Ser Tyr Leu Arg Gly Ala Pro
            595                 600                 605

Ile Thr Leu Tyr Gly
    610

<210>  24
<211>  11917
<212>  RNA
<213>  Artificial Sequence

<220>
<223>  RBS004.1

<400>  24
gaugggcggc gcaugagaga agcccagacc aauuaccuac ccaaaaugga gaaaguucac      60

guugacaucg aggaagacag cccauuccuc agagcuuugc agcggagcuu cccgcaguuu     120

gagguagaag ccaagcaggu cacugauaau gaccaugcua augccagagc guuuucgcau     180

cuggcuucaa aacugaucga aacggaggug gacccauccg acacgauccu ugacauugga     240

agugcgcccg cccgcagaau guauucuaag cacaaguauc auuguaucug uccgaugaga     300

ugugcggaag auccggacag auuguauaag uaugcaacua agcugaagaa aaacuguaag     360

gaaauaacug auaaggaauu ggacaagaaa augaaggagc ucgccgccgu caugagcgac     420

ccugaccugg aaacugagac uaugugccuc cacgacgacg agucgugucg cuacgaaggg     480

caagucgcug uuuaccagga uguauacgcg guugacggac cgacaagucu cuaucaccaa     540

gccaauaagg gaguuagagu cgccuacugg auaggcuuug acaccacccc uuuuauguuu     600

aagaacuugg cuggagcaua uccaucauac ucuaccaacu gggccgacga aaccguguua     660

acggcucgua acauaggccu augcagcucu gacguuaugg agcggucacg uagagggaug     720

uccauucuua gaaagaagua uuugaaacca uccaacaaug uucuauucuc uguuggcucg     780

accaucuacc acgaaaagag ggacuuacug aggagcuggc accugccguc uguauuucac     840

uuacguggca agcaaaauua cacaugucgg ugugagacua uaguuaguug cgacggguac     900

gucguuaaaa gaauagcuau caguccaggc cuguauggga agccuucagg cuaugcugcu     960

acgaugcacc gcgagggauu cuugugcugc aaagugacag acacauugaa cggggagagg    1020

gucucuuuuc ccgugugcac guaugugcca gcuacauugu gugaccaaau gacuggcaua    1080

cuggcaacag augucagugc ggacgacgcg caaaaacugc ugguugggcu caaccagcgu    1140

auagucguca acggucgcac ccagagaaac accaauacca ugaaaaauua ccuuuugccc 1200

guaguggccc aggcauuugc uaggugggca aaggaauaua aggaagauca agaagaugaa 1260

aggccacuag gacuacgaga uagacaguua gucauggggu guuguugggc uuuuagaagg 1320

cacaagauaa caucuauuua uaagcgcccg gauacccaaa ccaucaucaa agugaacagc 1380

gauuuccacu cauucgugcu gcccaggaua ggcaguaaca cauuggagau cgggcugaga 1440

acaagaauca ggaaaauguu agaggagcac aaggagccgu caccucucau uaccgccgag 1500

gacguacaag aagcuaagug cgcagccgau gaggcuaagg aggugcguga agccgaggag 1560

uugcgcgcag cucuaccacc uuuggcagcu gauguugagg agcccacucu ggaagccgau 1620

gucgacuuga uguuacaaga ggcuggggcc ggcucagugg agacaccucg uggcuugaua 1680

aagguuacca gcuacgcugg cgaggacaag aucggcucuu acgcugugcu uucuccgcag 1740

gcuguacuca agagugaaaa auuaucuugc auccacccuc ucgcugaaca agucauagug 1800

auaacacacu cuggccgaaa agggcguuau gccguggaac cauaccaugg uaaaguagug 1860

gugccagagg gacaugcaau acccguccag gacuuucaag cucugaguga aagugccacc 1920

auuguguaca acgaacguga guucguaaac agguaccugc accauauugc cacacaugga 1980

ggagcgcuga acacugauga agaauauuac aaaacuguca agcccagcga gcacgacggc 2040

gaauaccugu acgacaucga caggaaacag ugcgucaaga aagagcuagu cacugggcua 2100

gggcucacag gcgagcuggu cgauccuccc uuccaugaau ucgccuacga gagucugaga 2160

acacgaccag ccgcuccuua ccaaguacca accauagggg uguauggcgu gccaggauca 2220

ggcaagucug gcaucauuaa aagcgcaguc accaaaaaag aucuaguggu gagcgccaag 2280

aaagaaaacu gugcagaaau uauaagggac gucaagaaaa ugaaagggcu ggacgucaau 2340

gccagaacug uggacucagu gcucuugaau ggaugcaaac accccguaga gacccuguau 2400

auugacgagg cuuuugcuug ucaugcaggu acucucagag cgcucauagc cauuauaaga 2460

ccuaaaaagg cagugcucug cggagauccc aaacagugcg guuuuuuuaa caugaugugc 2520

cugaaagugc auuuuaacca cgagauuugc acacaagucu uccacaaaag caucucucgc 2580

cguugcacua aaucugugac uucggucguc ucaaccuugu uuuacgacaa aaaaaugaga 2640

acgacgaauc cgaaagagac uaagauugug auugacacua ccggcaguac caaaccuaag 2700

caggacgauc ucauucucac uuguuucaga ggguggguga agcaguugca aauagauuac 2760

aaaggcaacg aaauaaugac ggcagcugcc ucucaagggc ugacccguaa agguguguau 2820

gccguucggu acaaggugaa ugaaaauccu cuguacgcac ccaccucaga acaugugaac 2880

guccuacuga cccgcacgga ggaccgcauc guguggaaaa cacuagccgg cgacccaugg 2940

auaaaaacac ugacugccaa guacccuggg aauuucacug ccacgauaga ggaguggcaa 3000

gcagagcaug augccaucau gaggcacauc uuggagagac cggacccuac cgacgucuuc 3060

cagaauaagg caaacgugug uugggccaag gcuuuagugc cggugcugaa gaccgcuggc 3120

auagacauga ccacugaaca auggaacacu guggauuauu uugaaacgga caaagcucac 3180

ucagcagaga uaguauugaa ccaacuaugc gugagguucu uuggacucga ucuggacucc 3240

ggucuauuuu cugcacccac uguuccguua uccauuagga auaaucacug ggauaacucc 3300

ccgucgccua acauguacgg gcugaauaaa gaaguggucc gucagcucuc ucgcagguac 3360

ccacaacugc cucgggcagu ugccacuggu agagucuaug acaugaacac ugguacacug 3420

cgcaauuaug auccgcgcau aaaccuagua ccuguaaaca gaagacugcc ucaugcuuua 3480

guccuccacc auaaugaaca cccacagagu gacuuuucuu cauucgucag caaauugaag 3540

ggcagaacug uccugguggu cggggaaaag uuguccgucc caggcaaaau gguugacugg 3600

uugucagacc ggccugaggc uaccuucaga gcucggcugg auuuaggcau cccaggugau 3660

gugcccaaau augacauaau auuuguuaau gugaggaccc cauauaaaua ccaucacuau 3720

cagcagugug aagaccaugc cauuaagcua agcauguuga ccaagaaagc augucugcau 3780

cugaaucccg gcggaaccug ugucagcaua gguuaugguu acgcugacag ggccagcgaa 3840

agcaucauug gugcuauagc gcggcaguuc aaguuucccc gaguaugcaa accgaaaucc 3900

ucacuugagg agacggaagu ucuguuugua uucauugggu acgaucgcaa ggcccguacg 3960

cacaauccuu acaagcuauc aucaaccuug accaacauuu auacagguuc cagacuccac 4020

gaagccggau gugcacccuc auaucaugug gugcgagggg auauugccac ggccaccgaa 4080

ggagugauua uaaaugcugc uaacagcaaa ggacaaccug gcggaggggu gugcggagcg 4140

cuguauaaga aauucccgga aaguuucgau uuacagccga ucgaaguagg aaaagcgcga 4200

cuggucaaag gugcagcuaa acauaucauu caugccguag gaccaaacuu caacaaaguu 4260

ucggagguug aaggugacaa acaguuggca gaggcuuaug aguccaucgc uaagauuguc 4320

aacgauaaca auuacaaguc aguagcgauu ccacuguugu ccaccggcau cuuuuccggg 4380

aacaaagauc gacuaaccca aucauugaac cauuugcuga cagcuuuaga caccacugau 4440

gcagauguag ccauauacug cagggacaag aaaugggaaa ugacucucaa ggaagcagug 4500

gcuaggagag aagcagugga ggagauaugc auauccgacg auucuucagu gacagaaccu 4560

gaugcagagc uggugagggu gcaucccaag aguucuuugg cuggaaggaa gggcuacagc 4620

acaagcgaug gcaaaacuuu cucauauuug gaagggacca aguuucacca ggcggccaag 4680

gauauagcag aaauuaaugc cauguggccc guugcaacgg aggccaauga gcagguaugc 4740

auguauaucc ucggagaaag caugagcagu auuaggucga aaugccccgu cgaggagucg 4800

gaagccucca caccaccuag cacgcugccu ugcuugugca uccaugccau gacuccagaa 4860

agaguacagc gccuaaaagc cucacgucca gaacaaauua cugugugcuc auccuuucca 4920

uugccgaagu auagaaucac uggugugcag aagauccaau gcucccagcc uauauuguuc 4980

ucaccgaaag ugccugcgua uauucaucca aggaaguauc ucguggaaac accaccggua 5040

gacgagacuc cggagccauc ggcagagaac caauccacag aggggacacc ugaacaacca 5100

ccacuuauaa ccgaggauga gaccaggacu agaacgccug agccgaucau caucgaagaa 5160

gaagaagaag auagcauaag uuugcuguca gauggcccga cccaccaggu gcugcaaguc 5220

gaggcagaca uucacgggcc gcccucugua ucuagcucau ccugguccau uccucaugca 5280

uccgacuuug auguggacag uuuauccaua cuugacaccc uggagggagc uagcgugacc 5340

agcggggcaa cgucagccga gacuaacucu uacuucgcaa agaguaugga guuucuggcg 5400

cgaccggugc cugcgccucg aacaguauuc aggaacccuc cacaucccgc uccgcgcaca 5460

agaacaccgu cacuugcacc cagcagggcc ugcuccagaa ccagccuagu uuccaccccg 5520

ccaggcguga auagggugau cacuagagag gagcucgaag cgcuuacccc gucacgcacu 5580

ccuagcaggu cggucuccag aaccagccug gucuccaacc cgccaggcgu aaauagggug 5640

auuacaagag aggaguuuga ggcguucgua gcacaacaac aaugacgguu ugaugcgggu 5700

gcauacaucu uuuccuccga caccggucaa gggcauuuac aacaaaaauc aguaaggcaa 5760

acggugcuau ccgaaguggu guuggagagg accgaauugg agauuucgua ugccccgcgc 5820

cucgaccaag aaaaagaaga auuacuacgc aagaaauuac aguuaaaucc cacaccugcu 5880

aacagaagca gauaccaguc caggaaggug gagaacauga aagccauaac agcuagacgu 5940

auucugcaag gccuagggca uuauuugaag gcagaaggaa aaguggagug cuaccgaacc 6000

cugcauccug uuccuuugua uucaucuagu gugaaccgug ccuuuucaag ccccaagguc 6060

gcaguggaag ccuguaacgc cauguugaaa gagaacuuuc cgacuguggc uucuuacugu 6120

auuauuccag aguacgaugc cuauuuggac augguugacg gagcuucaug cugcuuagac 6180

acugccaguu uuugcccugc aaagcugcgc agcuuuccaa agaaacacuc cuauuuggaa 6240

cccacaauac gaucggcagu gccuucagcg auccagaaca cgcuccagaa cguccuggca 6300

gcugccacaa aaagaaauug caaugucacg caaaugagag aauugcccgu auuggauucg 6360

gcggccuuua auguggaaug cuucaagaaa uaugcgugua auaaugaaua uugggaaacg 6420

uuuaaagaaa accccaucag gcuuacugaa gaaaacgugg uaaauuacau uaccaaauua 6480

aaaggaccaa aagcugcugc ucuuuuugcg aagacacaua auuugaauau guugcaggac 6540

auaccaaugg acagguuugu aauggacuua aagagagacg ugaaagugac uccaggaaca 6600

aaacauacug aagaacggcc caagguacag gugauccagg cugccgaucc gcuagcaaca 6660

gcguaucugu gcggaaucca ccgagagcug guuaggagau uaaaugcggu ccugcuuccg 6720

aacauucaua cacuguuuga uaugucggcu gaagacuuug acgcuauuau agccgagcac 6780

uuccagccug gggauugugu ucuggaaacu gacaucgcgu cguuugauaa aagugaggac 6840

gacgccaugg cucugaccgc guuaaugauu cuggaagacu uaggugugga cgcagagcug 6900

uugacgcuga uugaggcggc uuucggcgaa auuucaucaa uacauuugcc cacuaaaacu 6960

aaauuuaaau ucggagccau gaugaaaucu ggaauguucc ucacacuguu ugugaacaca 7020

gucauuaaca uuguaaucgc aagcagagug uugagagaac ggcuaaccgg aucaccaugu 7080

gcagcauuca uuggagauga caauaucgug aaaggaguca aaucggacaa auuaauggca 7140

gacaggugcg ccaccugguu gaauauggaa gucaagauua uagaugcugu ggugggcgag 7200

aaagcgccuu auuucugugg aggguuuauu uugugugacu ccgugaccgg cacagcgugc 7260

cguguggcag acccccuaaa aaggcuguuu aagcuaggca aaccucuggc agcagacgau 7320

gaacaugaug augacaggag aagggcauug caugaggagu caacacgcug gaaccgagug 7380

gguauucuuu cagagcugug caaggcagua gaaucaaggu augaaaccgu aggaacuucc 7440

aucauaguua uggccaugac uacucuagcu agcaguguua aaucauucag cuaccugaga 7500

ggggccccua uaacucucua cggcuaaccu gaauggacua cgacauaguc uaguccgcca 7560

agacuaguau guuuguguuu cuugugcugc ugccucuugu gucuucucag ugugugaauu 7620

ugacaacaag aacacagcug ccaccagcuu auacaaauuc uuuuaccaga ggaguguauu 7680

auccugauaa aguguuuaga ucuucugugc ugcacagcac acaggaccug uuucugccau 7740

uuuuuagcaa ugugacaugg uuucaugcaa uucauguguc uggaacaaau ggaacaaaaa 7800

gauuugauaa uccugugcug ccuuuuaaug auggagugua uuuugcuuca acagaaaagu 7860

caaauauuau uagaggaugg auuuuuggaa caacacugga uucuaaaaca cagucucugc 7920

ugauugugaa uaaugcaaca aaugugguga uuaaagugug ugaauuucag uuuuguaaug 7980

auccuuuucu gggaguguau uaucacaaaa auaauaaauc uuggauggaa ucugaauuua 8040

gaguguauuc cucugcaaau aauuguacau uugaauaugu gucucagccu uuucugaugg 8100

aucuggaagg aaaacagggc aauuuuaaaa aucugagaga auuuguguuu aaaaauauug 8160

auggauauuu uaaaauuuau ucuaaacaca caccaauuaa uuuagugaga gaucugccuc 8220

agggauuuuc ugcucuggaa ccucuggugg aucugccaau uggcauuaau auuacaagau 8280

uucagacacu gcuggcucug cacagaucuu aucugacacc uggagauucu ucuucuggau 8340

ggacagccgg agcugcagcu uauuaugugg gcuaucugca gccaagaaca uuucugcuga 8400

aauauaauga aaauggaaca auuacagaug cuguggauug ugcucuggau ccucugucug 8460

aaacaaaaug uacauuaaaa ucuuuuacag uggaaaaagg cauuuaucag acaucuaauu 8520

uuagagugca gccaacagaa ucuauuguga gauuuccaaa uauuacaaau cuguguccau 8580

uuggagaagu guuuaaugca acaagauuug caucugugua ugcauggaau agaaaaagaa 8640

uuucuaauug uguggcugau uauucugugc uguauaauag ugcuucuuuu uccacauuua 8700

aauguuaugg agugucucca acaaaauuaa augauuuaug uuuuacaaau guguaugcug 8760

auucuuuugu gaucagaggu gaugaaguga gacagauugc ccccggacag acaggaaaaa 8820

uugcugauua caauuacaaa cugccugaug auuuuacagg augugugauu gcuuggaauu 8880

cuaauaauuu agauucuaaa gugggaggaa auuacaauua ucuguacaga cuguuuagaa 8940

aaucaaaucu gaaaccuuuu gaaagagaua uuucaacaga aauuuaucag gcuggaucaa 9000

caccuuguaa uggaguggaa ggauuuaauu guuauuuucc auuacagagc uauggauuuc 9060

agccaaccaa ugguguggga uaucagccau auagaguggu ggugcugucu uuugaacugc 9120

ugcaugcacc ugcaacagug uguggaccua aaaaaucuac aaauuuagug aaaaauaaau 9180

gugugaauuu uaauuuuaau ggauuaacag gaacaggagu gcugacagaa ucuaauaaaa 9240

aauuucugcc uuuucagcag uuuggcagag auauugcaga uaccacagau gcagugagag 9300

auccucagac auuagaaauu cuggauauua caccuuguuc uuuugggggu gugucuguga 9360

uuacaccugg aacaaauaca ucuaaucagg uggcugugcu guaucaggau gugaauugua 9420

cagaagugcc aguggcaauu caugcagauc agcugacacc aacauggaga guguauucua 9480

caggaucuaa uguguuucag acaagagcag gaugucugau uggagcagaa caugugaaua 9540

auucuuauga augugauauu ccaauuggag caggcauuug ugcaucuuau cagacacaga 9600

caaauucccc aaggagagca agaucugugg caucucaguc uauuauugca uacaccaugu 9660

cucugggagc agaaaauucu guggcauauu cuaauaauuc uauugcuauu ccaacaaauu 9720

uuaccauuuc ugugacaaca gaaauuuuac cugugucuau gacaaaaaca ucuguggauu 9780

guaccaugua cauuugugga gauucuacag aauguucuaa ucugcugcug caguauggau 9840

cuuuuuguac acagcugaau agagcuuuaa caggaauugc uguggaacag gauaaaaaua 9900

cacaggaagu guuugcucag gugaaacaga uuuacaaaac accaccaauu aaagauuuug 9960

gaggauuuaa uuuuagccag auucugccug auccuucuaa accuucuaaa agaucuuuua 10020

uugaagaucu gcuguuuaau aaagugacac uggcagaugc aggauuuauu aaacaguaug 10080

gagauugccu gggugauauu gcugcaagag aucugauuug ugcucagaaa uuuaauggac 10140

ugacagugcu gccuccucug cugacagaug aaaugauugc ucaguacaca ucugcuuuac 10200

uggcuggaac aauuacaagc ggauggacau uuggagcugg agcugcucug cagauuccuu 10260

uugcaaugca gauggcuuac agauuuaaug gaauuggagu gacacagaau guguuauaug 10320

aaaaucagaa acugauugca aaucaguuua auucugcaau uggcaaaauu caggauucuc 10380

ugucuucuac agcuucugcu cugggaaaac ugcaggaugu ggugaaucag aaugcacagg 10440

cacugaauac ucuggugaaa cagcugucua gcaauuuugg ggcaauuucu ucugugcuga 10500

augauauucu gucuagacug gauccuccug aagcugaagu gcagauugau agacugauca 10560

caggaagacu gcagucucug cagacuuaug ugacacagca gcugauuaga gcugcugaaa 10620

uuagagcuuc ugcuaaucug gcugcuacaa aaaugucuga augugugcug ggacagucaa 10680

aaagagugga uuuuugugga aaaggauauc aucugauguc uuuuccacag ucugcuccac 10740

auggaguggu guuuuuacau gugacauaug ugccagcaca ggaaaagaau uuuaccacag 10800

caccagcaau uugucaugau ggaaaagcac auuuuccaag agaaggagug uuugugucua 10860

auggaacaca uugguuugug acacagagaa auuuuuauga accucagauu auuacaacag 10920

auaauacauu ugugucagga aauugugaug uggugauugg aauugugaau aauacagugu 10980

augauccacu gcagccagaa cuggauucuu uuaaagaaga acuggauaaa uauuuuaaaa 11040

aucacacauc uccugaugug gauuuaggag auauuucugg aaucaaugca ucugugguga 11100

auauucagaa agaaauugau agacugaaug aaguggccaa aaaucugaau gaaucucuga 11160

uugaucugca ggaacuugga aaauaugaac aguacauuaa auggccuugg uacauuuggc 11220

uuggauuuau ugcaggauua auugcaauug ugauggugac aauuauguua uguuguauga 11280

caucauguug uucuuguuua aaaggauguu guucuugugg aagcuguugu aaauuugaug 11340

aagaugauuc ugaaccugug uuaaaaggag ugaaauugca uuacacauga ugacucgagc 11400

ugguacugca ugcacgcaau gcuagcugcc ccuuucccgu ccuggguacc ccgagucucc 11460

cccgaccucg ggucccaggu augcucccac cuccaccugc cccacucacc accucugcua 11520

guuccagaca ccucccaagc acgcagcaau gcagcucaaa acgcuuagcc uagccacacc 11580

cccacgggaa acagcaguga uuaaccuuua gcaauaaacg aaaguuuaac uaagcuauac 11640

uaaccccagg guuggucaau uucgugccag ccacaccgcg gccgcaugaa uacagcagca 11700

auuggcaagc ugcuuacaua gaacucgcgg cgauuggcau gccgccuuaa aauuuuuauu 11760

uuauuuuuuc uuuucuuuuc cgaaucggau uuuguuuuua auauuucaaa aaaaaaaaaa 11820

aaaaaaaaaa aaaaaaagca uaugacuaaa aaaaaaaaaa aaaaaaaaaa aaaaaaaaaa 11880

aaaaaaaaaa aaaaaaaaaa aaaaaaaaaa aaaaaaa 11917

<210> 25
<211> 11917
<212> RNA
<213> Artificial Sequence

<220>
<223> RBS004.2

<400> 25
gaugggcggc gcaugagaga agcccagacc aauuaccuac ccaaaaugga gaaaguucac 60

guugacaucg aggaagacag cccauuccuc agagcuuugc agcggagcuu cccgcaguuu 120

gagguagaag ccaagcaggu cacugauaau gaccaugcua augccagagc guuucgcauc 180

cuggcuucaa aacugaucga aacggaggug gacccauccg acacgauccu ugacauugga 240

agugcgcccg cccgcagaau guauucuaag cacaaguauc auuguaucug uccgaugaga 300

ugugcggaag auccggacag auuguauaag uaugcaacua agcugaagaa aaacuguaag 360

gaaauaacug auaaggaauu ggacaagaaa augaaggagc ucgccgccgu caugagcgac 420

ccugaccugg aaacugagac uaugugccuc cacgacgacg agucgugucg cuacgaaggg 480

caagucgcug uuuaccagga uguauacgcg guugacggac cgacaagucu cuaucaccaa 540

gccaauaagg gaguuagagu cgccuacugg auaggcuuug acaccacccc uuuuauguuu 600

aagaacuugg cuggagcaua uccaucauac ucuaccaacu gggccgacga aaccguguua 660

acggcucgua acauaggccu augcagcucu gacguuaugg agcggucacg uagagggaug 720

uccauucuua gaaagaagua uuugaaacca uccaacaaug uucuauucuc uguuggcucg 780

accaucuacc acgaaaagag ggacuuacug aggagcuggc accugccguc uguauuucac 840

uuacguggca agcaaaauua cacaugucgg ugugagacua uaguuaguug cgacggguac 900

gucguuaaaa gaauagcuau caguccaggc cuguauggga agccuucagg cuaugcugcu 960

acgaugcacc gcgagggauu cuugugcugc aaagugacag acacauugaa cggggagagg 1020

gucucuuuuc ccgugugcac guaugugcca gcuacauugu gugaccaaau gacuggcaua 1080

cuggcaacag augucagugc ggacgacgcg caaaaacugc ugguugggcu caaccagcgu 1140

auagucguca acggucgcac ccagagaaac accaauacca ugaaaaauua ccuuuugccc 1200

guaguggccc aggcauuugc uaggugggca aaggaauaua aggaagauca agaagaugaa 1260

aggccacuag gacuacgaga uagacaguua gucauggggu guuguugggc uuuuagaagg 1320

cacaagauaa caucuauuua uaagcgcccg gauacccaaa ccaucaucaa agugaacagc 1380

gauuuccacu cauucgugcu gcccaggaua ggcaguaaca cauuggagau cgggcugaga 1440

acaagaauca ggaaaauguu agaggagcac aaggagccgu caccucucau uaccgccgag 1500

gacguacaag aagcuaagug cgcagccgau gaggcuaagg aggugcguga agccgaggag 1560

uugcgcgcag cucuaccacc uuuggcagcu gauguugagg agcccacucu ggaagccgau 1620

gucgacuuga uguuacaaga ggcuggggcc ggcucagugg agacaccucg uggcuugaua 1680

aagguuacca gcuacgcugg cgaggacaag aucggcucuu acgcugugcu uucuccgcag 1740

gcuguacuca agagugaaaa auuaucuugc auccacccuc ucgcugaaca agucauagug 1800

auaacacacu cuggccgaaa agggcguuau gccguggaac cauaccaugg uaaaguagug 1860

gugccagagg gacaugcaau acccguccag gacuuucaag cucugaguga aagugccacc 1920

auuguguaca acgaacguga guucguaaac agguaccugc accauauugc cacacaugga 1980

ggagcgcuga acacugauga agaauauuac aaaacuguca agcccagcga gcacgacggc 2040

gaauaccugu acgacaucga caggaaacag ugcgucaaga aagagcuagu cacugggcua 2100

gggcucacag gcgagcuggu cgauccuccc uuccaugaau ucgccuacga gagucugaga 2160

acacgaccag ccgcuccuua ccaaguacca accauagggg uguauggcgu gccaggauca 2220

ggcaagucug gcaucauuaa aagcgcaguc accaaaaaag aucuaguggu gagcgccaag 2280

aaagaaaacu gugcagaaau uauaagggac gucaagaaaa ugaaagggcu ggacgucaau 2340

gccagaacug uggacucagu gcucuugaau ggaugcaaac accccguaga gacccuguau 2400

auugacgagg cuuuugcuug ucaugcaggu acucucagag cgcucauagc cauuauaaga 2460
ccuaaaaagg cagugcucug cggagauccc aaacagugcg guuuuuuuaa caugaugugc 2520
cugaaagugc auuuuaacca cgagauuugc acacaagucu uccacaaaag caucucucgc 2580
cguugcacua aaucugugac uucggucguc ucaaccuugu uuuacgacaa aaaaaugaga 2640
acgacgaauc cgaaagagac uaagauugug auugacacua ccggcaguac caaaccuaag 2700
caggacgauc ucauucucac uuguuucaga ggguggguga agcaguugca aauagauuac 2760
aaaggcaacg aaauaaugac ggcagcugcc ucucaagggc ugacccguaa agguguguau 2820
gccguucggu acaaggugaa ugaaaauccu cuguacgcac ccaccucaga acaugugaac 2880
guccuacuga cccgcacgga ggaccgcauc guguggaaaa cacuagccgg cgacccaugg 2940
auaaaaacac ugacugccaa guacccuggg aauuucacug ccacgauaga ggaguggcaa 3000
gcagagcaug augccaucau gaggcacauc uuggagagac cggacccuac cgacgucuuc 3060
cagaauaagg caaacgugug uugggccaag gcuuuagugc cggugcugaa gaccgcuggc 3120
auagacauga ccacugaaca auggaacacu guggauuauu uugaaacgga caaagcucac 3180
ucagcagaga uaguauugaa ccaacuaugc gugagguucu uuggacucga ucuggacucc 3240
ggucuauuuu cugcacccac uguuccguua uccauuagga auaaucacug ggauaacucc 3300
ccgucgccua acauguacgg gcugaauaaa gaaguggucc gucagcucuc ucgcagguac 3360
ccacaacugc cucgggcagu ugccacuggu agagucuaug acaugaacac ugguacacug 3420
cgcaauuaug auccgcgcau aaaccuagua ccuguaaaca gaagacugcc ucaugcuuua 3480
guccuccacc auaaugaaca cccacagagu gacuuuucuu cauucgucag caaauugaag 3540
ggcagaacug uccugguggu cggggaaaag uuguccgucc caggcaaaau gguugacugg 3600
uugucagacc ggccugaggc uaccuucaga gcucggcugg auuuaggcau cccaggugau 3660
gugcccaaau augacauaau auuuguuaau gugaggaccc cauauaaaua ccaucacuau 3720
cagcagugug aagaccaugc cauuaagcua agcauguuga ccaagaaagc augucugcau 3780
cugaaucccg gcggaaccug ugucagcaua gguuaugguu acgcugacag ggccagcgaa 3840
agcaucauug gugcuauagc gcggcaguuc aaguuucccc gaguaugcaa accgaaaucc 3900

ucacuugagg agacggaagu ucuguuugua uucauugggu acgaucgcaa ggcccguacg 3960

cacaauccuu acaagcuauc aucaaccuug accaacauuu auacagguuc cagacuccac 4020

gaagccggau gugcacccuc auaucaugug gugcgagggg auauugccac ggccaccgaa 4080

ggagugauua uaaaugcugc uaacagcaaa ggacaaccug gcggaggggu gugcggagcg 4140

cuguauaaga aauucccgga aaguuucgau uuacagccga ucgaaguagg aaaagcgcga 4200

cuggucaaag gugcagcuaa acauaucauu caugccguag gaccaaacuu caacaaaguu 4260

ucggagguug aaggugacaa acaguuggca gaggcuuaug aguccaucgc uaagauuguc 4320

aacgauaaca auuacaaguc aguagcgauu ccacuguugu ccaccggcau cuuuuccggg 4380

aacaaagauc gacuaaccca aucauugaac cauuugcuga cagcuuuaga caccacugau 4440

gcagauguag ccauauacug cagggacaag aaaugggaaa ugacucucaa ggaagcagug 4500

gcuaggagag aagcagugga ggagauaugc auauccgacg auucuucagu gacagaaccu 4560

gaugcagagc uggugagggu gcaucccaag aguucuuugg cuggaaggaa gggcuacagc 4620

acaagcgaug gcaaaacuuu cucauauuug gaagggacca aguuucacca ggcggccaag 4680

gauauagcag aaauuaaugc cauguggccc guugcaacgg aggccaauga gcagguaugc 4740

auguauaucc ucggagaaag caugagcagu auuaggucga aaugccccgu cgaggagucg 4800

gaagccucca caccaccuag cacgcugccu ugcuugugca uccaugccau gacuccagaa 4860

agaguacagc gccuaaaagc cucacgucca gaacaaauua cugugugcuc auccuuucca 4920

uugccgaagu auagaaucac uggugugcag aagauccaau gcucccagcc uauauuguuc 4980

ucaccgaaag ugccugcgua uauucaucca aggaaguauc ucguggaaac accaccggua 5040

gacgagacuc cggagccauc ggcagagaac caauccacag aggggacacc ugaacaacca 5100

ccacuuauaa ccgaggauga gaccaggacu agaacgccug agccgaucau caucgaagaa 5160

gaagaagaag auagcauaag uuugcuguca gauggcccga cccaccaggu gcugcaaguc 5220

gaggcagaca uucacgggcc gcccucugua ucuagcucau ccugguccau uccucaugca 5280

uccgacuuug auguggacag uuuauccaua cuugacaccc uggagggagc uagcgugacc 5340

agcggggcaa cgucagccga gacuaacucu uacuucgcaa agaguaugga guuucuggcg 5400

cgaccggugc cugcgccucg aacaguauuc aggaacccuc cacaucccgc uccgcgcaca 5460

agaacaccgu cacuugcacc cagcagggcc ugcuccagaa ccagccuagu uuccaccccg 5520

ccaggcguga auagggugau cacuagagag gagcucgaag cgcuuacccc gucacgcacu 5580

ccuagcaggu cggucuccag aaccagccug gucuccaacc cgccaggcgu aaauagggug 5640

auuacaagag aggaguuuga ggcguucgua gcacaacaac aaugacgguu ugaugcgggu 5700

gcauacaucu uuuccuccga caccggucaa gggcauuuac aacaaaaauc aguaaggcaa 5760

acggugcuau ccgaaguggu guuggagagg accgaauugg agauuucgua ugccccgcgc 5820

cucgaccaag aaaaagaaga auuacuacgc aagaaauuac aguuaaaucc cacaccugcu 5880

aacagaagca gauaccaguc caggaaggug gagaacauga aagccauaac agcuagacgu 5940

auucugcaag gccuagggca uuauuugaag gcagaaggaa aaguggagug cuaccgaacc 6000

cugcauccug uuccuuugua uucaucuagu gugaaccgug ccuuuucaag ccccaagguc 6060

gcaguggaag ccuguaacgc cauguugaaa gagaacuuuc cgacuguggc uucuuacugu 6120

auuauuccag aguacgaugc cuauuuggac augguugacg gagcuucaug cugcuuagac 6180

acugccaguu uuugcccugc aaagcugcgc agcuuuccaa agaaacacuc cuauuuggaa 6240

cccacaauac gaucggcagu gccuucagcg auccagaaca cgcuccagaa cguccuggca 6300

gcugccacaa aaagaaauug caaugucacg caaaugagag aauugcccgu auuggauucg 6360

gcggccuuua auguggaaug cuucaagaaa uaugcgugua auaaugaaua uugggaaacg 6420

uuuaaagaaa accccaucag gcuuacugaa gaaaacgugg uaaauuacau uaccaaauua 6480

aaaggaccaa aagcugcugc ucuuuuugcg aagacacaua auuugaauau guugcaggac 6540

auaccaaugg acagguuugu aauggacuua aagagagacg ugaaagugac uccaggaaca 6600

aaacauacug aagaacggcc caagguacag gugauccagg cugccgaucc gcuagcaaca 6660

gcguaucugu gcggaaucca ccgagagcug guuaggagau uaaaugcggu ccugcuuccg 6720

aacauucaua cacuguuuga uaugucggcu gaagacuuug acgcuauuau agccgagcac 6780

uuccagccug gggauugugu ucuggaaacu gacaucgcgu cguuugauaa aagugaggac 6840

gacgccaugg cucugaccgc guuaaugauu cuggaagacu uaggugugga cgcagagcug 6900

uugacgcuga uugaggcggc uuucggcgaa auuucaucaa uacauuugcc cacuaaaacu 6960

aaauuuaaau ucggagccau gaugaaaucu ggaauguucc ucacacuguu ugugaacaca 7020

gucauuaaca uuguaaucgc aagcagagug uugagagaac ggcuaaccgg aucaccaugu 7080

gcagcauuca uuggagauga caauaucgug aaaggaguca aaucggacaa auuaauggca 7140

gacaggugcg ccaccugguu gaauauggaa gucaagauua uagaugcugu ggugggcgag 7200

aaagcgccuu auuucugugg aggguuuauu uugugugacu ccgugaccgg cacagcgugc 7260

cguguggcag acccccuaaa aaggcuguuu aagcuaggca aaccucuggc agcagacgau 7320

gaacaugaug augacaggag aagggcauug caugaggagu caacacgcug gaaccgagug 7380

gguauucuuu cagagcugug caaggcagua gaaucaaggu augaaaccgu aggaacuucc 7440

aucauaguua uggccaugac uacucuagcu agcaguguua aaucauucag cuaccugaga 7500

ggggccccua uaacucucua cggcuaaccu gaauggacua cgacauaguc uaguccgcca 7560

agacuaguau guucguguuc cuggugcugc ugccucuggu guccagccag ugugugaacc 7620

ugaccaccag aacacagcug ccuccagccu acaccaacag cuuuaccaga ggcguguacu 7680

accccgacaa gguguucaga uccagcgugc ugcacucuac ccaggaccug uuccugccuu 7740

ucuucagcaa cgugaccugg uuccacgcca uccacguguc cggcaccaau ggcaccaaga 7800

gauucgacaa ccccgugcug cccuucaacg acggggugua cuuugccagc accgagaagu 7860

ccaacaucau cagaggcugg aucuucggca ccacacugga cagcaagacc cagagccugc 7920

ugaucgugaa caacgccacc aacgugguca ucaaagugug cgaguuccag uucugcaacg 7980

accccuuccu gggcgucuac uaccacaaga acaacaagag cuggauggaa agcgaguucc 8040

ggguguacag cagcgccaac aacugcaccu ucgaguacgu gucccagccu uuccugaugg 8100

accuggaagg caagcagggc aacuucaaga accugcgcga guucguguuu aagaacaucg 8160

acggcuacuu caagaucuac agcaagcaca ccccuaucaa ccucgugcgg gaucugccuc 8220

agggcuucuc ugcucuggaa ccccuggugg aucugcccau cggcaucaac aucacccggu 8280

uucagacacu gcuggcccug cacagaagcu accugacacc uggcgauagc agcagcggau 8340

ggacagcugg ugccgccgcu uacuaugugg gcuaccugca gccuagaacc uuccugcuga 8400

aguacaacga gaacggcacc aucaccgacg ccguggauug ugcucuggau ccucugagcg 8460

agacaaagug cacccugaag uccuucaccg uggaaaaggg caucuaccag accagcaacu 8520

uccgggugca gcccaccgaa uccaucgugc gguuccccaa uaucaccaau cugugccccu 8580

ucggcgaggu guucaaugcc accagauucg ccucugugua cgccuggaac cggaagcgga 8640

ucagcaauug cguggccgac uacuccgugc uguacaacuc cgccagcuuc agcaccuuca 8700

agugcuacgg cguguccccu accaagcuga acgaccugug cuucacaaac guguacgccg 8760

acagcuucgu gauccgggga gaugaagugc ggcagauugc cccuggacag acaggcaaga 8820

ucgccgacua caacuacaag cugcccgacg acuucaccgg cugugugauu gccuggaaca 8880

gcaacaaccu ggacuccaaa gucggcggca acuacaauua ccuguaccgg cuguuccgga 8940

aguccaaucu gaagcccuuc gagcgggaca ucuccaccga gaucuaucag gccggcagca 9000

ccccuuguaa cggcguggaa ggcuucaacu gcuacuuccc acugcagucc uacggcuuuc 9060

agcccacaaa uggcgugggc uaucagcccu acagaguggu ggugcugagc uucgaacugc 9120

ugcaugcccc ugccacagug ugcggcccua agaaaagcac caaucucgug aagaacaaau 9180

gcgugaacuu caacuucaac ggccugaccg gcaccggcgu gcugacagag agcaacaaga 9240

aguuccugcc auuccagcag uuuggccggg auaucgccga uaccacagac gccguuagag 9300

auccccagac acuggaaauc cuggacauca ccccuugcag cuucggcgga gugucuguga 9360

ucaccccugg caccaacacc agcaaucagg uggcagugcu guaccaggac gugaacugua 9420

ccgaagugcc cguggccauu cacgccgauc agcugacacc uacauggcgg guguacucca 9480

ccggcagcaa uguguuucag accagagccg gcugucugau cggagccgag cacgugaaca 9540

auagcuacga gugcgacauc cccaucggcg cuggaaucug cgccagcuac cagacacaga 9600

caaacagccc ucggagagcc agaagcgugg ccagccagag caucauugcc uacacaaugu 9660

cucugggcgc cgagaacagc guggccuacu ccaacaacuc uaucgcuauc cccaccaacu 9720

ucaccaucag cgugaccaca gagauccugc cuguguccau gaccaagacc agcguggacu 9780

gcaccaugua caucugcggc gauuccaccg agugcuccaa ccugcugcug caguacggca 9840

gcuucugcac ccagcugaau agagcccuga cagggaucgc cguggaacag gacaagaaca 9900

cccaagaggu guucgcccaa gugaagcaga ucuacaagac cccuccuauc aaggacuucg 9960

gcggcuucaa uuucagccag auucugcccg auccuagcaa gcccagcaag cggagcuuca 10020

ucgaggaccu gcuguucaac aaagugacac uggccgacgc cggcuucauc aagcaguaug 10080

gcgauugucu gggcgacauu gccgccaggg aucugauuug cgcccagaag uuuaacggac 10140

ugacagugcu gccuccucug cugaccgaug agaugaucgc ccaguacaca ucugcccugc 10200

uggccggcac aaucacaagc ggcuggacau uuggagcagg cgccgcucug cagauccccu 10260

uugcuaugca gauggccuac cgguucaacg gcaucggagu gacccagaau gugcuguacg 10320

agaaccagaa gcugaucgcc aaccaguuca acagcgccau cggcaagauc caggacagcc 10380

ugagcagcac agcaagcgcc cugggaaagc ugcaggacgu ggucaaccag aaugcccagg 10440

cacugaacac ccuggucaag cagcuguccu ccaacuucgg cgccaucagc ucugugcuga 10500

acgauauccu gagcagacug gacccuccug aggccgaggu gcagaucgac agacugauca 10560

caggcagacu gcagagccuc cagacauacg ugacccagca gcugaucaga gccgccgaga 10620

uuagagccuc ugccaaucug gccgccacca agaugucuga gugugugcug ggccagagca 10680

agagagugga cuuuugcggc aagggcuacc accugaugag cuucccucag ucugccccuc 10740

acggcguggu guuucugcac gugacauaug ugcccgcuca agagaagaau uucaccaccg 10800

cuccagccau cugccacgac ggcaaagccc acuuuccuag agaaggcgug uucgugucca 10860

acggcaccca uugguucgug acacagcgga acuucuacga gccccagauc aucaccaccg 10920

acaacaccuu cgugucuggc aacugcgacg ucgugaucgg cauugugaac aauaccgugu 10980

acgacccucu gcagcccgag cuggacagcu ucaaagagga acuggacaag uacuuuaaga 11040

accacacaag ccccgacgug gaccugggcg auaucagcgg aaucaaugcc agcgucguga 11100

acauccagaa agagaucgac cggcugaacg agguggccaa gaaucugaac gagagccuga 11160

ucgaccugca agaacugggg aaguacgagc aguacaucaa guggcccugg uacaucuggc 11220

ugggcuuuau cgccggacug auugccaucg ugauggucac aaucaugcug uguugcauga 11280

ccagcugcug uagcugccug aagggcuguu guagcugugg cagcugcugc aaguucgacg 11340

aggacgauuc ugagcccgug cugaagggcg ugaaacugca cuacacauga ugacucgagc 11400

ugguacugca ugcacgcaau gcuagcugcc ccuuucccgu ccuggguacc ccgagucucc 11460

cccgaccucg ggucccaggu augcucccac cuccaccugc cccacucacc accucugcua 11520

guuccagaca ccucccaagc acgcagcaau gcagcucaaa acgcuuagcc uagccacacc 11580

cccacgggaa acagcaguga uuaaccuuua gcaauaaacg aaaguuuaac uaagcuauac 11640

uaaccccagg guuggucaau uucgugccag ccacaccgcg gccgcaugaa uacagcagca 11700

auuggcaagc ugcuuacaua gaacucgcgg cgauuggcau gccgccuuaa aauuuuuauu 11760

uuauuuuuuc uuuucuuuuc cgaaucggau uuuguuuuua auauuucaaa aaaaaaaaaa 11820

aaaaaaaaaa aaaaaaagca uaugacuaaa aaaaaaaaaa aaaaaaaaaa aaaaaaaaaa 11880

aaaaaaaaaa aaaaaaaaaa aaaaaaaaaa aaaaaaa 11917

<210> 26
<211> 8896
<212> RNA
<213> Artificial Sequence

<220>
<223> RBS004.3

<400> 26
gaugggcggc gcaugagaga agcccagacc aauuaccuac ccaaaaugga gaaaguucac 60

guugacaucg aggaagacag cccauuccuc agagcuuugc agcggagcuu cccgcaguuu 120

gagguagaag ccaagcaggu cacugauaau gaccaugcua augccagagc guuucgcauc 180

cuggcuucaa aacugaucga aacggaggug gacccauccg acacgauccu ugacauugga 240

agugcgcccg cccgcagaau guauucuaag cacaaguauc auuguaucug uccgaugaga 300

ugugcggaag auccggacag auuguauaag uaugcaacua agcugaagaa aaacuguaag 360

gaaauaacug auaaggaauu ggacaagaaa augaaggagc ucgccgccgu caugagcgac 420

ccugaccugg aaacugagac uaugugccuc cacgacgacg agucgugucg cuacgaaggg 480

caagucgcug uuuaccagga uguauacgcg guugacggac cgacaagucu cuaucaccaa 540

gccaauaagg gaguuagagu cgccuacugg auaggcuuug acaccacccc uuuuauguuu 600

aagaacuugg cuggagcaua uccaucauac ucuaccaacu gggccgacga aaccguguua 660

acggcucgua acauaggccu augcagcucu gacguuaugg agcggucacg uagagggaug 720

uccauucuua gaaagaagua uuugaaacca uccaacaaug uucuauucuc uguuggcucg 780

accaucuacc acgaaaagag ggacuuacug aggagcuggc accugccguc uguauuucac 840

uuacguggca agcaaaauua cacaugucgg ugugagacua uaguuaguug cgacggguac 900

gucguuaaaa gaauagcuau caguccaggc cuguauggga agccuucagg cuaugcugcu 960

acgaugcacc gcgagggauu cuugugcugc aaagugacag acacauugaa cggggagagg 1020

gucucuuuuc ccgugugcac guaugugcca gcuacauugu gugaccaaau gacuggcaua 1080

cuggcaacag augucagugc ggacgacgcg caaaaacugc ugguugggcu caaccagcgu 1140

auagucguca acggucgcac ccagagaaac accaauacca ugaaaaauua ccuuuugccc 1200

guaguggccc aggcauuugc uaggugggca aaggaauaua aggaagauca agaagaugaa 1260

aggccacuag gacuacgaga uagacaguua gucauggggu guuguugggc uuuuagaagg 1320

cacaagauaa caucuauuua uaagcgcccg gauacccaaa ccaucaucaa agugaacagc 1380

gauuuccacu cauucgugcu gcccaggaua ggcaguaaca cauuggagau cgggcugaga 1440

acaagaauca ggaaaauguu agaggagcac aaggagccgu caccucucau uaccgccgag 1500

gacguacaag aagcuaagug cgcagccgau gaggcuaagg aggugcguga agccgaggag 1560

uugcgcgcag cucuaccacc uuuggcagcu gauguugagg agcccacucu ggaagccgau 1620

gucgacuuga uguuacaaga ggcuggggcc ggcucagugg agacaccucg uggcuugaua 1680

aagguuacca gcuacgcugg cgaggacaag aucggcucuu acgcugugcu uucuccgcag 1740

gcuguacuca agagugaaaa auuaucuugc auccacccuc ucgcugaaca agucauagug 1800

auaacacacu cuggccgaaa agggcguuau gccguggaac cauaccaugg uaaaguagug 1860

gugccagagg gacaugcaau acccguccag gacuuucaag cucugaguga aagugccacc 1920

auuguguaca acgaacguga guucguaaac agguaccugc accauauugc cacacaugga 1980

ggagcgcuga acacugauga agaauauuac aaaacuguca agcccagcga gcacgacggc 2040

gaauaccugu acgacaucga caggaaacag ugcgucaaga aagagcuagu cacugggcua 2100

gggcucacag gcgagcuggu cgauccuccc uuccaugaau ucgccuacga gagucugaga 2160

acacgaccag ccgcuccuua ccaaguacca accauagggg uguauggcgu gccaggauca 2220

ggcaagucug gcaucauuaa aagcgcaguc accaaaaaag aucuaguggu gagcgccaag 2280

aaagaaaacu gugcagaaau uauaagggac gucaagaaaa ugaaagggcu ggacgucaau 2340

gccagaacug uggacucagu gcucuugaau ggaugcaaac accccguaga gacccuguau 2400

auugacgagg cuuuugcuug ucaugcaggu acucucagag cgcucauagc cauuauaaga 2460

ccuaaaaagg cagugcucug cggagauccc aaacagugcg guuuuuuuaa caugaugugc 2520

cugaaagugc auuuuaacca cgagauuugc acacaagucu uccacaaaag caucucucgc 2580

cguugcacua aaucugugac uucggucguc ucaaccuugu uuuacgacaa aaaaaugaga 2640

acgacgaauc cgaaagagac uaagauugug auugacacua ccggcaguac caaaccuaag 2700

caggacgauc ucauucucac uuguuucaga ggguggguga agcaguugca aauagauuac 2760

aaaggcaacg aaauaaugac ggcagcugcc ucucaagggc ugacccguaa aggugugyau 2820

gccguucggu acaaggugaa ugaaaauccu cuguacgcac ccaccucaga acaugugaac 2880

guccuacuga cccgcacgga ggaccgcauc guguggaaaa cacuagccgg cgacccaugg 2940

auaaaaacac ugacugccaa guacccuggg aauuucacug ccacgauaga ggaguggcaa 3000

gcagagcaug augccaucau gaggcacauc uuggagagac cggacccuac cgacgucuuc 3060

cagaauaagg caaacgugug uugggccaag gcuuuagugc cggugcugaa gaccgcuggc 3120

auagacauga ccacugaaca auggaacacu guggauuauu uugaaacgga caaagcucac 3180

ucagcagaga uaguauugaa ccaacuaugc gugagguucu uuggacucga ucuggacucc 3240

ggucuauuuu cugcacccac uguuccguua uccauuagga auaaucacug ggauaacucc 3300

ccgucgccua acauguacgg gcugaauaaa gaaguggucc gucagcucuc ucgcagguac 3360

ccacaacugc cucgggcagu ugccacuggu agagucuaug acaugaacac ugguacacug 3420

cgcaauuaug auccgcgcau aaaccuagua ccuguaaaca gaagacugcc ucaugcuuua 3480

guccuccacc auaaugaaca cccacagagu gacuuuucuu cauucgucag caaauugaag 3540

ggcagaacug uccugguggu cggggaaaag uuguccgucc caggcaaaau gguugacugg 3600

uugucagacc ggccugaggc uaccuucaga gcucggcugg auuuaggcau cccaggugau 3660

gugcccaaau augacauaau auuuguuaau gugaggaccc cauauaaaua ccaucacuau 3720

cagcagugug aagaccaugc cauuaagcua agcauguuga ccaagaaagc augucugcau 3780

cugaaucccg gcggaaccug ugucagcaua gguuaugguu acgcugacag ggccagcgaa 3840

agcaucauug gugcuauagc gcggcaguuc aaguuucccg gaguaugcaa accgaaaucc 3900

ucacuugagg agacggaagu ucuguuugua uucauugggu acgaucgcaa ggcccguacg 3960

cacaauccuu acaagcuauc aucaaccuug accaacauuu auacagguuc cagacuccac 4020

gaagccggau gugcacccuc auaucaugug gugcgagggg auauugccac ggccaccgaa 4080

ggagugauua uaaaugcugc uaacagcaaa ggacaaccug gcggaggggu gugcggagcg 4140

cuguauaaga aauucccgga aaguuucgau uuacagccga ucgaaguagg aaaagcgcga 4200

cuggucaaag gugcagcuaa acauaucauu caugccguag gaccaaacuu caacaaaguu 4260

ucggagguug aaggugacaa acaguuggca gaggcuuaug aguccaucgc uaagauuguc 4320

aacgauaaca auuacaaguc aguagcgauu ccacuguugu ccaccggcau cuuuuccggg 4380

aacaaagauc gacuaaccca aucauugaac cauuugcuga cagcuuuaga caccacugau 4440

gcagauguag ccauauacug cagggacaag aaaugggaaa ugacucucaa ggaagcagug 4500

gcuaggagag aagcagugga ggagauaugc auauccgacg auucuucagu gacagaaccu 4560

gaugcagagc uggugagggu gcaucccaag aguucuuugg cuggaaggaa gggcuacagc 4620

acaagcgaug gcaaaacuuu cucauauuug gaagggacca aguuucacca ggcggccaag 4680

gauauagcag aaauuaaugc cauguggccc guugcaacgg aggccaauga gcagguaugc 4740

auguauaucc ucggagaaag caugagcagu auuaggucga aaugccccgu cgaggagucg 4800

gaagccucca caccaccuag cacgcugccu ugcuugugca uccaugccau gacuccagaa 4860

agaguacagc gccuaaaagc cucacgucca gaacaaauua cugugugcuc auccuuucca 4920

uugccgaagu auagaaucac uggugugcag aagauccaau gcucccagcc uauauuguuc 4980

ucaccgaaag ugccugcgua uauucaucca aggaaguauc ucguggaaac accaccggua 5040

gacgagacuc cggagccauc ggcagagaac caauccacag aggggacacc ugaacaacca 5100

ccacuuauaa ccgaggauga gaccaggacu agaacgccug agccgaucau caucgaagaa 5160

gaagaagaag auagcauaag uuugcuguca gauggcccga cccaccaggu gcugcaaguc 5220

gaggcagaca uucacgggcc gcccucugua ucuagcucau ccugguccau uccucaugca 5280

uccgacuuug auguggacag uuuauccaua cuugacaccc uggagggagc uagcgugacc 5340

agcggggcaa cgucagccga gacuaacucu uacuucgcaa agaguaugga guuucuggcg 5400

cgaccggugc cugcgccucg aacaguauuc aggaacccuc cacaucccgc uccgcgcaca 5460

agaacaccgu cacuugcacc cagcagggcc ugcuccagaa ccagccuagu uuccaccccg 5520

ccaggcguga auagggugau cacuagagag gagcucgaag cgcuuacccc gucacgcacu 5580

ccuagcaggu cggucuccag aaccagccug gucuccaacc cgccaggcgu aaauagggug 5640

auuacaagag aggaguuuga ggcguucgua gcacaacaac aaugacgguu ugaugcgggu 5700

gcauacaucu uuuccuccga caccggucaa gggcauuuac aacaaaaauc aguaaggcaa 5760

acggugcuau ccgaaguggu guuggagagg accgaauugg agauuucgua ugccccgcgc 5820

cucgaccaag aaaaagaaga auuacuacgc aagaaauuac aguuaaaucc cacaccugcu 5880

aacagaagca gauaccaguc caggaaggug gagaacauga aagccauaac agcuagacgu 5940

auucugcaag gccuagggca uuauuugaag gcagaaggaa aaguggagug cuaccgaacc 6000

cugcauccug uuccuuugua uucaucuagu gugaaccgug ccuuuucaag ccccaagguc 6060

gcaguggaag ccuguaacgc cauguugaaa gagaacuuuc cgacuguggc uucuuacugu 6120

auuauuccag aguacgaugc cuauuuggac augguugacg gagcuucaug cugcuuagac 6180

acugccaguu uuugcccugc aaagcugcgc agcuuuccaa agaaacacuc cuauuuggaa 6240

cccacaauac gaucggcagu gccuucagcg auccagaaca cgcuccagaa cguccuggca 6300

gcugccacaa aaagaaauug caaugucacg caaaugagag aauugcccgu auuggauucg 6360

gcggccuuua auguggaaug cuucaagaaa uaugcgugua auaaugaaua uugggaaacg 6420

uuuaaagaaa accccaucag gcuuacugaa gaaaacgugg uaaauuacau uaccaaauua 6480

aaaggaccaa aagcugcugc ucuuuuugcg aagacacaua auuugaauau guugcaggac 6540

auaccaaugg acagguuugu aauggacuua aagagagacg ugaaagugac uccaggaaca 6600

aaacauacug aagaacggcc caagguacag gugauccagg cugccgaucc gcuagcaaca 6660

gcguaucugu gcggaaucca ccgagagcug guuaggagau uaaaugcggu ccugcuuccg 6720

aacauucaua cacuguuuga uaugucggcu gaagacuuug acgcuauuau agccgagcac 6780

uuccagccug gggauugugu ucuggaaacu gacaucgcgu cguuugauaa aagugaggac 6840

gacgccaugg cucugaccgc guuaaugauu cuggaagacu uaggugugga cgcagagcug 6900

uugacgcuga uugaggcggc uuucggcgaa auuucaucaa uacauuugcc cacuaaaacu 6960

aaauuuaaau ucggagccau gaugaaaucu ggaauguucc ucacacuguu ugugaacaca 7020

gucauuaaca uuguaaucgc aagcagagug uugagagaac ggcuaaccgg aucaccaugu 7080

gcagcauuca uuggagauga caauaucgug aaaggaguca aaucggacaa auuaauggca 7140

gacaggugcg ccaccugguu gaauauggaa gucaagauua uagaugcugu ggugggcgag 7200

aaagcgccuu auuucugugg aggguuuauu uugugugacu ccgugaccgg cacagcgugc 7260

cguguggcag acccccuaaa aaggcuguuu aagcuaggca aaccucuggc agcagacgau 7320

gaacaugaug augacaggag aagggcauug caugaggagu caacacgcug gaaccgagug 7380

gguauucuuu cagagcugug caaggcagua gaaucaaggu augaaaccgu aggaacuucc 7440

aucauaguua uggccaugac uacucuagcu agcaguguua aaucauucag cuaccugaga 7500

ggggccccua uaacucucua cggcuaaccu gaauggacua cgacauaguc uaguccgcca 7560

agacuaguau guuuguguuu cuugugcugc ugccucuugu gucuucucag ugugugguga 7620

gauuuccaaa uauuacaaau cuguguccau uuggagaagu guuuaaugca acaagauuug 7680

caucugugua ugcauggaau agaaaaagaa uuucuaauug uguggcugau uauucugugc 7740

uguauaauag ugcuucuuuu uccacauuua aauguuaugg agugucucca acaaaauuaa 7800

augauuuaug uuuuacaaau guguaugcug auucuuuugu gaucagaggu gaugaaguga 7860

gacagauugc ccccggacag acaggaaaaa uugcugauua caauuacaaa cugccugaug 7920

auuuuacagg augugugauu gcuuggaauu cuaauaauuu agauucuaaa gugggaggaa 7980

auuacaauua ucuguacaga cuguuuagaa aaucaaaucu gaaaccuuuu gaaagagaua 8040

uuucaacaga aauuuaucag gcuggaucaa caccuuguaa uggaguggaa ggauuuaauu 8100

guuauuuucc auuacagagc uauggauuuc agccaaccaa ugguguggga uaucagccau 8160

auagaguggu ggugcugucu uuugaacugc ugcaugcacc ugcaacagug uguggaccua 8220

aaggcucccc cggcuccggc uccggaucug guuauauucc ugaagcucca agagaugggc 8280

aagcuuacgu ucguaaagau ggcgaauggg uauuacuuuc uaccuuuuua ggccgguccc 8340

uggaggugcu guuccagggc cccggcugau gacucgagcu gguacugcau gcacgcaaug 8400

cuagcugccc cuuucccguc cuggguaccc cgagucuccc ccgaccucgg gucccaggua 8460

ugcucccacc uccaccugcc ccacucacca ccucugcuag uuccagacac cucccaagca 8520

cgcagcaaug cagcucaaaa cgcuuagccu agccacaccc ccacgggaaa cagcagugau 8580

uaaccuuuag caauaaacga aaguuuaacu aagcuauacu aaccccaggg uuggucaauu 8640

ucgugccagc cacaccgcgg ccgcaugaau acagcagcaa uuggcaagcu gcuuacauag 8700

aacucgcggc gauuggcaug ccgccuuaaa auuuuuauuu uauuuuuucu uuucuuuucc 8760

gaaucggauu uuguuuuuaa uauuucaaaa aaaaaaaaaa aaaaaaaaaa aaaaaagcau 8820

augacuaaaa aaaaaaaaaa aaaaaaaaaa aaaaaaaaaa aaaaaaaaaa aaaaaaaaaa 8880

aaaaaaaaaa aaaaaa 8896

<210> 27
<211> 9079
<212> RNA
<213> Artificial Sequence

<220>
<223> RBS004.4

<400> 27
   gaugggcggc gcaugagaga agcccagacc aauuaccuac ccaaaaugga gaaaguucac 60
   guugacaucg aggaagacag cccauuccuc agagcuuugc agcggagcuu cccgcaguuu 120
   gagguagaag ccaagcaggu cacugauaau gaccaugcua augccagagc guuuucgcau 180
   cuggcuucaa aacugaucga aacggaggug gacccauccg acacgauccu ugacauugga 240
   agugcgcccg cccgcagaau guauucuaag cacaaguauc auuguaucug uccgaugaga 300
   ugugcggaag auccggacag auuguauaag uaugcaacua agcugaagaa aaacuguaag 360
   gaaauaacug auaaggaauu ggacaagaaa augaaggagc ucgccgccgu caugagcgac 420
   ccugaccugg aaacugagac uaugugccuc cacgacgacg agucgugucg cuacgaaggg 480
   caagucgcug uuuaccagga uguauacgcg guugacggac cgacaagucu cuaucaccaa 540
   gccaauaagg gaguuagagu cgccuacugg auaggcuuug acaccacccc uuuuauguuu 600
   aagaacuugg cuggagcaua uccaucauac ucuaccaacu gggccgacga aaccguguua 660
   acggcucgua acauaggccu augcagcucu gacguuaugg agcggucacg uagagggaug 720
   uccauucuua gaaagaagua uuugaaacca uccaacaaug uucuauucuc uguuggcucg 780
   accaucuacc acgaaaagag ggacuuacug aggagcuggc accugccguc uguauuucac 840
   uuacguggca agcaaaauua cacaugucgg ugugagacua uaguuaguug cgacggguac 900
   gucguuaaaa gaauagcuau caguccaggc cuguauggga agccuucagg cuaugcugcu 960
   acgaugcacc gcgagggauu cuugugcugc aaagugacag acacauugaa cggggagagg 1020
   gucucuuuuc ccgugugcac guaugugcca gcuacauugu gugaccaaau gacuggcaua 1080
   cuggcaacag augucagugc ggacgacgcg caaaaacugc ugguugggcu caaccagcgu 1140
   auagucguca acggucgcac ccagagaaac accaauacca ugaaaaauua ccuuuugccc 1200
   guaguggccc aggcauuugc uaggugggca aaggaauaua aggaagauca agaagaugaa 1260
   aggccacuag gacuacgaga uagacaguua gucauggggu guuguugggc uuuuagaagg 1320
   cacaagauaa caucuauuua uaagcgcccg gauacccaaa ccaucaucaa agugaacagc 1380
   gauuuccacu cauucgugcu gcccaggaua ggcaguaaca cauuggagau cgggcugaga 1440
   acaagaauca ggaaaauguu agaggagcac aaggagccgu caccucucau uaccgccgag 1500
   gacguacaag aagcuaagug cgcagccgau gaggcuaagg aggugcguga agccgaggag 1560
   uugcgcgcag cucuaccacc uuuggcagcu gauguugagg agcccacucu ggaagccgau 1620
   gucgacuuga uguuacaaga ggcuggggcc ggcucagugg agacaccucg uggcuugaua 1680
   aagguuacca gcuacgcugg cgaggacaag aucggcucuu acgcugugcu uucuccgcag 1740
   gcuguacuca agagugaaaa auuaucuugc auccacccuc ucgcugaaca agucauagug 1800
   auaacacacu cuggccgaaa agggcguuau gccguggaac cauaccaugg uaaaguagug 1860
   gugccagagg gacaugcaau acccguccag gacuuucaag cucugaguga aagugccacc 1920
   auuguguaca acgaacguga guucguaaac agguaccugc accauauugc cacacaugga 1980
   ggagcgcuga acacugauga agaauauuac aaaacuguca agcccagcga gcacgacggc 2040
   gaauaccugu acgacaucga caggaaacag ugcgucaaga aagagcuagu cacugggcua 2100
   gggcucacag gcgagcuggu cgauccuccc uuccaugaau ucgccuacga gagucugaga 2160
   acacgaccag ccgcuccuua ccaaguacca accauagggg uguauggcgu gccaggauca 2220
   ggcaagucug gcaucauuaa aagcgcaguc accaaaaaag aucuaguggu gagcgccaag 2280
   aaagaaaacu gugcagaaau uauaagggac gucaagaaaa ugaaagggcu ggacgucaau 2340
   gccagaacug uggacucagu gcucuugaau ggaugcaaac accccguaga gacccuguau 2400
   auugacgagg cuuuugcuug ucaugcaggu acucucagag cgcucauagc cauuauaaga 2460
   ccuaaaaagg cagugcucug cggagauccc aaacagugcg guuuuuuuaa caugaugugc 2520
   cugaaagugc auuuuaacca cgagauuugc acacaagucu uccacaaaag caucucucgc 2580
   cguugcacua aaucugugac uucggucguc ucaaccuugu uuuacgacaa aaaaaugaga 2640
   acgacgaauc cgaaagagac uaagauugug auugacacua ccggcaguac caaaccuaag 2700
   caggacgauc ucauucucac uuguuucaga ggguggguga agcaguugca aauagauuac 2760
   aaaggcaacg aaauaaugac ggcagcugcc ucucaagggc ugacccguaa agguguguau 2820
   gccguucggu acaaggugaa ugaaaauccu cuguacgcac ccaccucaga acaugugaac 2880
   guccuacuga cccgcacgga ggaccgcauc guguggaaaa cacuagccgg cgacccaugg 2940
   auaaaaacac ugacugccaa guacccuggg aauuucacug ccacgauaga ggaguggcaa 3000
   gcagagcaug augccaucau gaggcacauc uuggagagac cggacccuac cgacgucuuc 3060
   cagaauaagg caaacgugug uugggccaag gcuuuagugc cggugcugaa gaccgcuggc 3120
   auagacauga ccacugaaca auggaacacu guggauuauu uugaaacgga caaagcucac 3180
   ucagcagaga uaguauugaa ccaacuaugc gugagguucu uuggacucga ucuggacucc 3240
   ggucuauuuu cugcacccac uguuccguua uccauuagga auaaucacug ggauaacucc 3300
   ccgucgccua acauguacgg gcugaauaaa gaaguggucc gucagcucuc ucgcagguac 3360
   ccacaacugc cucgggcagu ugccacuggu agagucuaug acaugaacac ugguacacug 3420
   cgcaauuaug auccgcgcau aaaccuagua ccuguaaaca gaagacugcc ucaugcuuua 3480
   guccuccacc auaaugaaca cccacagagu gacuuuucuu cauucgucag caaauugaag 3540
   ggcagaacug uccugguggu cggggaaaag uuguccgucc caggcaaaau gguugacugg 3600
   uugucagacc ggccugaggc uaccuucaga gcucggcugg auuuaggcau cccaggugau 3660
   gugcccaaau augacauaau auuuguuaau gugaggaccc cauauaaaua ccaucacuau 3720
   cagcagugug aagaccaugc cauuaagcua agcauguuga ccaagaaagc augucugcau 3780
   cugaaucccg gcggaaccug ugucagcaua gguuaugguu acgcugacag ggccagcgaa 3840
   agcaucauug gugcuauagc gcggcaguuc aaguuucccc gaguaugcaa accgaaaucc 3900
   ucacuugagg agacggaagu ucuguuugua uucauugggu acgaucgcaa ggcccguacg 3960
   cacaauccuu acaagcuauc aucaaccuug accaacauuu auacagguuc cagacuccac 4020
   gaagccggau gugcacccuc auaucaugug gugcgagggg auauugccac ggccaccgaa 4080
   ggagugauua uaaaugcugc uaacagcaaa ggacaaccug gcggaggggu gugcggagcg 4140
   cuguauaaga aauucccgga aaguuucgau uuacagccga ucgaaguagg aaaagcgcga 4200
   cuggucaaag gugcagcuaa acauaucauu caugccguag gaccaaacuu caacaaaguu 4260
   ucggagguug aaggugacaa acaguuggca gaggcuuaug aguccaucgc uaagauuguc 4320
   aacgauaaca auuacaaguc aguagcgauu ccacuguugu ccaccggcau cuuuuccggg 4380
   aacaaagauc gacuaaccca aucauugaac cauuugcuga cagcuuuaga caccacugau 4440
   gcagauguag ccauauacug cagggacaag aaaugggaaa ugacucucaa ggaagcagug 4500
   gcuaggagag aagcagugga ggagauaugc auauccgacg auucuucagu gacagaaccu 4560
   gaugcagagc uggugagggu gcaucccaag aguucuuggg cuggaaggaa gggcuacagc 4620
   acaagcgaug gcaaaacuuu cucauauuug gaagggacca aguuucacca ggcggccaag 4680
   gauauagcag aaauuaaugc cauguggccc guugcaacgg aggccaauga gcagguaugc 4740
   auguauaucc ucggagaaag caugagcagu auuaggucga aaugccccgu cgaggagucg 4800
   gaagccucca caccaccuag cacgcugccu ugcuugugca uccaugccau gacuccagaa 4860
   agaguacagc gccuaaaagc cucacgucca gaacaaauua cugugugcuc auccuuucca 4920
   uugccgaagu auagaaucac uggugugcag aagauccaau gcucccagcc uauauuguuc 4980
   ucaccgaaag ugccugcgua uauucaucca aggaaguauc ucguggaaac accaccggua 5040
   gacgagacuc cggagccauc ggcagagaac caauccacag aggggacacc ugaacaacca 5100
   ccacuuauaa ccgaggauga gaccaggacu agaacgccug agccgaucau caucgaagaa 5160
   gaagaagaag auagcauaag uuugcuguca gauggcccga cccaccaggu gcugcaaguc 5220
   gaggcagaca uucacgggcc gcccucugua ucuagcucau ccugguccau uccucaugca 5280
   uccgacuuug auguggacag uuuauccaua cuugacaccc uggagggagc uagcgugacc 5340
   agcggggcaa cgucagccga gacuaacucu uacuucgcaa agaguaugga guuucuggcg 5400
   cgaccggugc cugcgccucg aacaguauuc aggaacccuc cacaucccgc uccgcgcaca 5460
   agaacaccgu cacuugcacc cagcagggcc ugcuccagaa ccagccuagu uuccaccccg 5520
   ccaggcguga auagggugau cacuagagag gagcucgaag cgcuuacccc gucacgcacu 5580
   ccuagcaggu cggucuccag aaccagccug gucuccaacc cgccaggcgu aaauagggug 5640
   auuacaagag aggaguuuga ggcguucgua gcacaacaac aaugacgguu ugaugcgggu 5700
   gcauacaucu uuuccuccga caccggucaa gggcauuuac aacaaaaauc aguaaggcaa 5760
   acggugcuau ccgaaguggu guuggagagg accgaauugg agauuucgua ugccccgcgc 5820
   cucgaccaag aaaaagaaga auuacuacgc aagaaauuac aguuaaaucc cacaccugcu 5880
   aacagaagca gauaccaguc caggaaggug gagaacauga aagccauaac agcuagacgu 5940
   auucugcaag gccuagggca uuauuugaag gcagaaggaa aaguggagug cuaccgaacc 6000
   cugcauccug uuccuuugua uucaucuagu gugaaccgug ccuuuucaag ccccaagguc 6060
   gcaguggaag ccuguaacgc cauguugaaa gagaacuuuc cgacuguggc uucuuacugu 6120
   auuauuccag aguacgaugc cuauuuggac augguugacg gagcuucaug cugcuuagac 6180
   acugccaguu uuugcccugc aaagcugcgc agcuuuccaa agaaacacuc cuauuuggaa 6240
   cccacaauac gaucggcagu gccuucagcg auccagaaca cgcuccagaa cguccuggca 6300
   gcugccacaa aaagaaauug caaugucacg caaaugagag aauugcccgu auuggauucg 6360
   gcggccuuua auguggaaug cuucaagaaa uaugcgugua auaaugaaua uugggaaacg 6420
   uuuaaagaaa accccaucag gcuuacugaa gaaaacgugg uaaauuacau uaccaaauua 6480
   aaaggaccaa aagcugcugc ucuuuuugcg aagacacaua auuugaauau guugcaggac 6540
   auaccaaugg acagguuugu aauggacuua aagagagacg ugaaagugac uccaggaaca 6600
   aaacauacug aagaacggcc caagguacag gugauccagg cugccgaucc gcuagcaaca 6660
   gcguaucugu gcggaaucca ccgagagcug guuaggagau uaaaugcggu ccugcuuccg 6720
   aacauucaua cacuguuuga uaugucggcu gaagacuuug acgcuauuau agccgagcac 6780
   uuccagccug gggauugugu ucuggaaacu gacaucgcgu cguuugauaa aagugaggac 6840
   gacgccaugg cucugaccgc guuaaugauu cuggaagacu uaggugugga cgcagagcug 6900
   uugacgcuga uugaggcggc uuucggcgaa auuucaucaa uacauuugcc cacuaaaacu 6960
   aaauuuaaau ucggagccau gaugaaaucu ggaauguucc ucacacuguu ugugaacaca 7020
   gucauuaaca uuguaaucgc aagcagagug uugagagaac ggcuaaccgg aucaccaugu 7080
   gcagcauuca uuggagauga caauaucgug aaaggaguca aaucggacaa auuaauggca 7140
   gacaggugcg ccaccugguu gaauauggaa gucaagauua uagaugcugu ggugggcgag 7200
   aaagcgccuu auuucugugg aggguuuauu uugugugacu ccgugaccgg cacagcgugc 7260
   cguguggcag acccccuaaa aaggcuguuu aagcuaggca aaccucuggc agcagacgau 7320
   gaacaugaug augacaggag aagggcauug caugaggagu caacacgcug gaaccgagug 7380
   gguauucuuu cagagcugug caaggcagua gaaucaaggu augaaaccgu aggaacuucc 7440
   aucauaguua uggccaugac uacucuagcu agcaguguua aaucauucag cuaccugaga 7500
   ggggccccua uaacucucua cggcuaaccu gaauggacua cgacauaguc uaguccgcca 7560
   agacuaguau guuuguguuu cuugugcugc ugccucuugu gucuucucag ugugugguga 7620
   gauuuccaaa uauuacaaau cuguguccau uuggagaagu guuuaaugca acaagauuug 7680
   caucugugua ugcauggaau agaaaaagaa uuucuaauug uguggcugau uauucugugc 7740
   uguauaauag ugcuucuuuu uccacauuua aauguuaugg agugucucca acaaaauuaa 7800
   augauuuaug uuuuacaaau guguaugcug auucuuuugu gaucagaggu gaugaaguga 7860
   gacagauugc ccccggacag acaggaaaaa uugcugauua caauuacaaa cugccugaug 7920
   auuuuacagg augugugauu gcuuggaauu cuaauaauuu agauucuaaa gugggaggaa 7980
   auuacaauua ucuguacaga cuguuuagaa aaucaaaucu gaaaccuuuu gaaagagaua 8040
   uuucaacaga aauuuaucag gcuggaucaa caccuuguaa uggaguggaa ggauuuaauu 8100
   guuauuuucc auuacagagc uauggauuuc agccaaccaa ugguguggga uaucagccau 8160
   auagaguggu ggugcugucu uuugaacugc ugcaugcacc ugcaacagug uguggaccua 8220
   aaggcucccc cggcuccggc uccggaucug guuauauucc ugaagcucca agagaugggc 8280
   aagcuuacgu ucguaaagau ggcgaauggg uauuacuuuc uaccuuuuua ggaagcggca 8340
   gcggaucuga acaguacauu aaauggccuu gguacauuug gcuuggauuu auugcaggau 8400
   uaauugcaau ugugauggug acaauuaugu uauguuguau gacaucaugu uguucuuguu 8460
   uaaaaggaug uuguucuugu ggaagcuguu guaaauuuga ugaagaugau ucugaaccug 8520
   uguuaaaagg agugaaauug cauuacacau gaugacucga gcugguacug caugcacgca 8580
   augcuagcug ccccuuuccc guccugggua ccccgagucu cccccgaccu cgggucccag 8640
   guaugcuccc accuccaccu gccccacuca ccaccucugc uaguuccaga caccucccaa 8700
   gcacgcagca augcagcuca aaacgcuuag ccuagccaca cccccacggg aaacagcagu 8760
   gauuaaccuu uagcaauaaa cgaaaguuua acuaagcuau acuaacccca ggguugguca 8820
   auuucgugcc agccacaccg cggccgcaug aauacagcag caauuggcaa gcugcuuaca 8880
   uagaacucgc ggcgauuggc augccgccuu aaaauuuuua uuuuauuuuu ucuuuucuuu 8940
   uccgaaucgg auuuuguuuu uaauauuuca aaaaaaaaaa aaaaaaaaaa aaaaaaaaag 9000
   cauaugacua aaaaaaaaaa aaaaaaaaaa aaaaaaaaaa aaaaaaaaaa aaaaaaaaaa 9060
   aaaaaaaaaa aaaaaaaaa 9079

<210> 28
<211> 327
<212> PRT
<213> Artificial Sequence

<220>
<223> Vaccine Antigen

<400> 28

Met Phe Val Phe Leu Val Leu Leu Pro Leu Val Ser Ser Gln Cys Val
1               5                   10                  15

Val Arg Phe Pro Asn Ile Thr Asn Leu Cys Pro Phe Gly Glu Val Phe
            20                  25                  30

Asn Ala Thr Arg Phe Ala Ser Val Tyr Ala Trp Asn Arg Lys Arg Ile
        35                  40                  45

Ser Asn Cys Val Ala Asp Tyr Ser Val Leu Tyr Asn Ser Ala Ser Phe
    50                  55                  60

Ser Thr Phe Lys Cys Tyr Gly Val Ser Pro Thr Lys Leu Asn Asp Leu
65                  70                  75                  80

Cys Phe Thr Asn Val Tyr Ala Asp Ser Phe Val Ile Arg Gly Asp Glu
                85                  90                  95

Val Arg Gln Ile Ala Pro Gly Gln Thr Gly Lys Ile Ala Asp Tyr Asn
            100                 105                 110

Tyr Lys Leu Pro Asp Asp Phe Thr Gly Cys Val Ile Ala Trp Asn Ser
        115                 120                 125

Asn Asn Leu Asp Ser Lys Val Gly Gly Asn Tyr Asn Tyr Leu Tyr Arg
    130                 135                 140

Leu Phe Arg Lys Ser Asn Leu Lys Pro Phe Glu Arg Asp Ile Ser Thr
145                 150                 155                 160

Glu Ile Tyr Gln Ala Gly Ser Thr Pro Cys Asn Gly Val Glu Gly Phe
                165                 170                 175

Asn Cys Tyr Phe Pro Leu Gln Ser Tyr Gly Phe Gln Pro Thr Asn Gly
            180                 185                 190

Val Gly Tyr Gln Pro Tyr Arg Val Val Val Leu Ser Phe Glu Leu Leu
        195                 200                 205

His Ala Pro Ala Thr Val Cys Gly Pro Lys Gly Ser Pro Gly Ser Gly
    210                 215                 220

Ser Gly Ser Gly Tyr Ile Pro Glu Ala Pro Arg Asp Gly Gln Ala Tyr
225                 230                 235                 240

Val Arg Lys Asp Gly Glu Trp Val Leu Leu Ser Thr Phe Leu Gly Ser
                245                 250                 255

Gly Ser Gly Ser Glu Gln Tyr Ile Lys Trp Pro Trp Tyr Ile Trp Leu
            260                 265                 270

Gly Phe Ile Ala Gly Leu Ile Ala Ile Val Met Val Thr Ile Met Leu
        275                 280                 285

Cys Cys Met Thr Ser Cys Cys Ser Cys Leu Lys Gly Cys Cys Ser Cys
    290                 295                 300

Gly Ser Cys Cys Lys Phe Asp Glu Asp Asp Ser Glu Pro Val Leu Lys
305                 310                 315                 320

Gly Val Lys Leu His Tyr Thr
                325

<210>  29
<211>  311
<212>  PRT
<213>  Artificial Sequence

<220>
<223>  BNT162b3c

<400>  29

Met Phe Val Phe Leu Val Leu Leu Pro Leu Val Ser Ser Gln Cys Val
1               5                   10                  15

Asn Leu Thr Val Arg Phe Pro Asn Ile Thr Asn Leu Cys Pro Phe Gly
            20                  25                  30

Glu Val Phe Asn Ala Thr Arg Phe Ala Ser Val Tyr Ala Trp Asn Arg
        35                  40                  45

Lys Arg Ile Ser Asn Cys Val Ala Asp Tyr Ser Val Leu Tyr Asn Ser
    50                  55                  60

Ala Ser Phe Ser Thr Phe Lys Cys Tyr Gly Val Ser Pro Thr Lys Leu
65                  70                  75                  80

Asn Asp Leu Cys Phe Thr Asn Val Tyr Ala Asp Ser Phe Val Ile Arg
                85                  90                  95

Gly Asp Glu Val Arg Gln Ile Ala Pro Gly Gln Thr Gly Lys Ile Ala
            100                 105                 110

Asp Tyr Asn Tyr Lys Leu Pro Asp Asp Phe Thr Gly Cys Val Ile Ala
        115                 120                 125

Trp Asn Ser Asn Asn Leu Asp Ser Lys Val Gly Gly Asn Tyr Asn Tyr
    130                 135                 140

Leu Tyr Arg Leu Phe Arg Lys Ser Asn Leu Lys Pro Phe Glu Arg Asp
145                 150                 155                 160

Ile Ser Thr Glu Ile Tyr Gln Ala Gly Ser Thr Pro Cys Asn Gly Val
                165                 170                 175

Glu Gly Phe Asn Cys Tyr Phe Pro Leu Gln Ser Tyr Gly Phe Gln Pro
            180                 185                 190

Thr Asn Gly Val Gly Tyr Gln Pro Tyr Arg Val Val Val Leu Ser Phe
        195                 200                 205

Glu Leu Leu His Ala Pro Ala Thr Val Cys Gly Pro Lys Gly Ser Pro
    210                 215                 220

Gly Ser Gly Ser Gly Ser Gly Tyr Ile Pro Glu Ala Pro Arg Asp Gly
225                 230                 235                 240

Gln Ala Tyr Val Arg Lys Asp Gly Glu Trp Val Leu Leu Ser Thr Phe
                245                 250                 255

Leu Gly Ser Gly Ser Gly Ser Glu Gln Tyr Ile Lys Trp Pro Trp Tyr
            260                 265                 270

Ile Trp Leu Gly Phe Ile Ala Gly Leu Ile Ala Ile Val Met Val Thr
        275                 280                 285

Ile Met Leu Cys Cys Met Thr Ser Cys Cys Ser Cys Leu Lys Gly Cys
    290                 295                 300

Cys Ser Cys Gly Ser Cys Cys
305                 310

<210>  30
<211>  1397
<212>  RNA
<213>  Artificial Sequence

<220>
<223>  BNT162b3c

<400>  30
agaauaaacu aguauucuuc ugguccccac agacucagag agaacccgcc accauguuug      60

uguuucuugu gcugcugccu cuugugucuu cucagugugu gaauuugaca gugagauuuc     120

caaauauuac aaaucugugu ccauuuggag aaguguuuaa ugcaacaaga uuugcaucug     180

uguaugcaug gaauagaaaa agaauuucua auuguguggc ugauuauucu gugcuguaua     240

auagugcuuc uuuuuccaca uuuaaauguu auggaguguc uccaacaaaa uuaaaugauu     300

uauguuuuac aaauguguau gcugauucuu uugugaucag aggugaugaa gugagacaga     360

uugcccccgg acagacagga aaaauugcug auuacaauua caaacugccu gaugauuuua     420

caggaugugu gauugcuugg aauucuaaua auuuagauuc uaaaguggga ggaaauuaca     480

auuaucugua cagacuguuu agaaaaucaa aucugaaacc uuuugaaaga gauauuucaa 540

cagaaauuua ucaggcugga ucaacaccuu guaauggagu ggaaggauuu aauuguuauu 600

uuccauuaca gagcuaugga uuucagccaa ccaauggugu gggauaucag ccauauagag 660

ugguggugcu gucuuuugaa cugcugcaug caccugcaac agugugugga ccuaaaggcu 720

cccccggcuc cggcuccgga ucugguuaua uuccugaagc uccaagagau gggcaagcuu 780

acguucguaa agauggcgaa uggguauuac uuucuaccuu uuuaggaagc ggcagcggau 840

cugaacagua cauuaaaugg ccuugguaca uuuggcuugg auuuauugca ggauuaauug 900

caauugugau ggugacaauu auguuauguu guaugacauc auguuguucu uguuuaaaag 960

gauguuguuc uuguggaagc uguuguugau gacucgagcu gguacugcau gcacgcaaug 1020

cuagcugccc cuuucccguc cuggguaccc cgagucuccc ccgaccucgg gucccaggua 1080

ugcucccacc uccaccugcc ccacucacca ccucugcuag uuccagacac cucccaagca 1140

cgcagcaaug cagcucaaaa cgcuuagccu agccacaccc ccacgggaaa cagcagugau 1200

uaaccuuuag caauaaacga aaguuuaacu aagcuauacu aaccccaggg uuggucaauu 1260

ucgugccagc cacacccugg agcuagcaaa aaaaaaaaaa aaaaaaaaaa aaaaaaagca 1320

uaugacuaaa aaaaaaaaaa aaaaaaaaaa aaaaaaaaaa aaaaaaaaaa aaaaaaaaaa 1380

aaaaaaaaaa aaaaaaa 1397

<210> 31
<211> 314
<212> PRT
<213> Artificial Sequence

<220>
<223> BNT162b3d

<400> 31

Met Asp Trp Ile Trp Arg Ile Leu Phe Leu Val Gly Ala Ala Thr Gly
1               5                   10                  15

Ala His Ser Gln Met Gln Val Arg Phe Pro Asn Ile Thr Asn Leu Cys
            20                  25                  30

Pro Phe Gly Glu Val Phe Asn Ala Thr Arg Phe Ala Ser Val Tyr Ala
        35                  40                  45

Trp Asn Arg Lys Arg Ile Ser Asn Cys Val Ala Asp Tyr Ser Val Leu
    50                  55                  60

Tyr Asn Ser Ala Ser Phe Ser Thr Phe Lys Cys Tyr Gly Val Ser Pro
65                  70                  75                  80

Thr Lys Leu Asn Asp Leu Cys Phe Thr Asn Val Tyr Ala Asp Ser Phe
                85                  90                  95

Val Ile Arg Gly Asp Glu Val Arg Gln Ile Ala Pro Gly Gln Thr Gly
            100                 105                 110

Lys Ile Ala Asp Tyr Asn Tyr Lys Leu Pro Asp Asp Phe Thr Gly Cys
        115                 120                 125

Val Ile Ala Trp Asn Ser Asn Asn Leu Asp Ser Lys Val Gly Gly Asn
    130                 135                 140

Tyr Asn Tyr Leu Tyr Arg Leu Phe Arg Lys Ser Asn Leu Lys Pro Phe
145                 150                 155                 160

Glu Arg Asp Ile Ser Thr Glu Ile Tyr Gln Ala Gly Ser Thr Pro Cys
                165                 170                 175

Asn Gly Val Glu Gly Phe Asn Cys Tyr Phe Pro Leu Gln Ser Tyr Gly
            180                 185                 190

Phe Gln Pro Thr Asn Gly Val Gly Tyr Gln Pro Tyr Arg Val Val Val
        195                 200                 205

Leu Ser Phe Glu Leu Leu His Ala Pro Ala Thr Val Cys Gly Pro Lys
    210                 215                 220

Gly Ser Pro Gly Ser Gly Ser Gly Ser Gly Tyr Ile Pro Glu Ala Pro
225                 230                 235                 240

Arg Asp Gly Gln Ala Tyr Val Arg Lys Asp Gly Glu Trp Val Leu Leu
                245                 250                 255

Ser Thr Phe Leu Gly Ser Gly Ser Gly Ser Glu Gln Tyr Ile Lys Trp
            260                 265                 270

Pro Trp Tyr Ile Trp Leu Gly Phe Ile Ala Gly Leu Ile Ala Ile Val
        275                 280                 285

Met Val Thr Ile Met Leu Cys Cys Met Thr Ser Cys Cys Ser Cys Leu
    290                 295                 300

Lys Gly Cys Cys Ser Cys Gly Ser Cys Cys
305                 310

<210>  32
<211>  1406
<212>  RNA
<213>  Artificial Sequence

<220>
<223>  BNT162b3d

<400>  32
agaauaaacu aguauucuuc ugguccccac agacucagag agaacccgcc accauggauu      60

ggauuuggag aauccuguuc cucgugggag ccgcuacagg agcccacucc cagaugcagg     120

ugagauuucc aaauauuaca aaucuguguc cauuuggaga aguguuuaau gcaacaagau     180

uugcaucugu guaugcaugg aauagaaaaa gaauuucuaa uuguguggcu gauuauucug     240

ugcuguauaa uagugcuucu uuuuccacau uuaaauguua uggagugucu ccaacaaaau     300

uaaaugauuu auguuuuaca aauguguaug cugauucuuu ugugaucaga ggugaugaag     360

ugagacagau ugcccccgga cagacaggaa aaauugcuga uuacaauuac aaacugccug     420

augauuuuac aggaugugug auugcuugga auucuaauaa uuuagauucu aaagugggag     480

gaaauuacaa uuaucuguac agacuguuua gaaaaucaaa ucugaaaccu uuugaaagag     540

auauuucaac agaaauuuau caggcuggau caacaccuug uaauggagug gaaggauuua 600

auuguuauuu uccauuacag agcuauggau uucagccaac caauggugug ggauaucagc 660

cauauagagu gguggugcug ucuuuugaac ugcugcaugc accugcaaca guguguggac 720

cuaaaggcuc ccccggcucc ggcuccggau cugguuauau uccugaagcu ccaagagaug 780

ggcaagcuua cguucguaaa gauggcgaau ggguauuacu uucuaccuuu uuaggaagcg 840

gcagcggauc ugaacaguac auuaaauggc cuugguacau uuggcuugga uuuauugcag 900

gauuaauugc aauugugaug gugacaauua uguuauguug uaugacauca uguuguucuu 960

guuuaaaagg auguuguucu uguggaagcu guuguugaug acucgagcug guacugcaug 1020

cacgcaaugc uagcugcccc uuucccgucc uggguacccc gagucucccc cgaccucggg 1080

ucccagguau gcucccaccu ccaccugccc cacucaccac cucugcuagu uccagacacc 1140

ucccaagcac gcagcaaugc agcucaaaac gcuuagccua gccacacccc cacgggaaac 1200

agcagugauu aaccuuuagc aauaaacgaa aguuuaacua agcuauacua accccagggu 1260

uggucaauuu cgugccagcc acacccugga gcuagcaaaa aaaaaaaaaa aaaaaaaaaa 1320

aaaaaagcau augacuaaaa aaaaaaaaaa aaaaaaaaaa aaaaaaaaaa aaaaaaaaaa 1380

aaaaaaaaaa aaaaaaaaaa aaaaaa 1406

<210> 33
<211> 9
<212> PRT
<213> Artificial Sequence

<220>
<223> Linker

<400> 33

Gly Ser Pro Gly Ser Gly Ser Gly Ser
1               5

<210> 34
<211> 6
<212> PRT
<213> Artificial Sequence

<220>
<223>  Linker

<400>  34

Gly Ser Gly Ser Gly Ser
1               5



<210>  35
<211>  9
<212>  PRT
<213>  Artificial Sequence

<220>
<223>  Epitope

<400>  35

Tyr Leu Gln Pro Arg Thr Phe Leu Leu
1               5



<210>  36
<211>  9
<212>  PRT
<213>  Artificial Sequence

<220>
<223>  Epitope

<400>  36

Arg Leu Gln Ser Leu Gln Thr Tyr Val
1               5



<210>  37
<211>  9
<212>  PRT
<213>  Artificial Sequence

<220>
<223>  Epitope

<400>  37

Gln Tyr Ile Lys Trp Pro Trp Tyr Ile
1               5

<210>  38
<211>  9
<212>  PRT
<213>  Artificial Sequence

<220>
<223>  Epitope

<400>  38

Asn Tyr Asn Tyr Leu Tyr Arg Leu Phe
1               5



<210>  39
<211>  10
<212>  PRT
<213>  Artificial Sequence

<220>
<223>  Epitope

<400>  39

Lys Trp Pro Trp Tyr Ile Trp Leu Gly Phe
1               5                   10



<210>  40
<211>  9
<212>  PRT
<213>  Artificial Sequence

<220>
<223>  Epitope

<400>  40

Gln Pro Thr Glu Ser Ile Val Arg Phe
1               5



<210>  41
<211>  9
<212>  PRT
<213>  Artificial Sequence

<220>

<223>  Epitope

<400>  41

Ile Pro Phe Ala Met Gln Met Ala Tyr
1               5



<210>  42
<211>  9
<212>  PRT
<213>  Artificial Sequence

<220>
<223>  Epitope

<400>  42

Leu Pro Phe Asn Asp Gly Val Tyr Phe
1               5



<210>  43
<211>  9
<212>  PRT
<213>  Artificial Sequence

<220>
<223>  Epitope

<400>  43

Gly Val Tyr Phe Ala Ser Thr Glu Lys
1               5



<210>  44
<211>  9
<212>  PRT
<213>  Artificial Sequence

<220>
<223>  Epitope

<400>  44

Cys Val Ala Asp Tyr Ser Val Leu Tyr
1               5

<210>  45
<211>  9
<212>  PRT
<213>  Artificial Sequence

<220>
<223>  Epitope

<400>  45

Lys Cys Tyr Gly Val Ser Pro Thr Lys
1               5

<210>  46
<211>  9
<212>  PRT
<213>  Artificial Sequence

<220>
<223>  Epitope

<400>  46

Phe Gln Pro Thr Asn Gly Val Gly Tyr
1               5

<210>  47
<211>  9
<212>  PRT
<213>  Artificial Sequence

<220>
<223>  Epitope

<400>  47

Gly Thr His Trp Phe Val Thr Gln Arg
1               5

<210>  48
<211>  9
<212>  PRT
<213>  Artificial Sequence

<220>
<223>  Epitope

<400>  48

Val Tyr Asp Pro Leu Gln Pro Glu Leu
1               5

<210>  49
<211>  1270
<212>  PRT
<213>  Artificial Sequence

<220>
<223>  Vaccine sequence

<400>  49

Met Phe Val Phe Leu Val Leu Leu Pro Leu Val Ser Ser Gln Cys Val
1               5                   10                  15

Asn Leu Thr Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Val Ile Ser Gly Thr Asn Gly Thr Lys Arg Phe Asp Asn Pro
65                  70                  75                  80

Val Leu Pro Phe Asn Asp Gly Val Tyr Phe Ala Ser Ile Glu Lys Ser
                85                  90                  95

Asn Ile Ile Arg Gly Trp Ile Phe Gly Thr Thr Leu Asp Ser Lys Thr
            100                 105                 110

Gln Ser Leu Leu Ile Val Asn Asn Ala Thr Asn Val Val Ile Lys Val
        115                 120                 125

Cys Glu Phe Gln Phe Cys Asn Asp Pro Phe Leu Asp His Lys Asn Asn
    130                 135                 140

Lys Ser Trp Met Glu Ser Glu Phe Arg Val Tyr Ser Ser Ala Asn Asn
145                 150                 155                 160

Cys Thr Phe Glu Tyr Val Ser Gln Pro Phe Leu Met Asp Leu Glu Gly
                165                 170                 175

Lys Gln Gly Asn Phe Lys Asn Leu Arg Glu Phe Val Phe Lys Asn Ile
            180                 185                 190

Asp Gly Tyr Phe Lys Ile Tyr Ser Lys His Thr Pro Ile Ile Val Arg
        195                 200                 205

Glu Pro Glu Asp Leu Pro Gln Gly Phe Ser Ala Leu Glu Pro Leu Val
    210                 215                 220

Asp Leu Pro Ile Gly Ile Asn Ile Thr Arg Phe Gln Thr Leu Leu Ala
225                 230                 235                 240

Leu His Arg Ser Tyr Leu Thr Pro Gly Asp Ser Ser Ser Gly Trp Thr
                245                 250                 255

Ala Gly Ala Ala Ala Tyr Tyr Val Gly Tyr Leu Gln Pro Arg Thr Phe
            260                 265                 270

Leu Leu Lys Tyr Asn Glu Asn Gly Thr Ile Thr Asp Ala Val Asp Cys
        275                 280                 285

Ala Leu Asp Pro Leu Ser Glu Thr Lys Cys Thr Leu Lys Ser Phe Thr
    290                 295                 300

Val Glu Lys Gly Ile Tyr Gln Thr Ser Asn Phe Arg Val Gln Pro Thr
305                 310                 315                 320

Glu Ser Ile Val Arg Phe Pro Asn Ile Thr Asn Leu Cys Pro Phe Asp
                325                 330                 335

Glu Val Phe Asn Ala Thr Arg Phe Ala Ser Val Tyr Ala Trp Asn Arg
            340                 345                 350

Lys Arg Ile Ser Asn Cys Val Ala Asp Tyr Ser Val Leu Tyr Asn Leu
        355                 360                 365

Ala Pro Phe Phe Thr Phe Lys Cys Tyr Gly Val Ser Pro Thr Lys Leu
    370                 375                 380

Asn Asp Leu Cys Phe Thr Asn Val Tyr Ala Asp Ser Phe Val Ile Arg
385                 390                 395                 400

Gly Asp Glu Val Arg Gln Ile Ala Pro Gly Gln Thr Gly Asn Ile Ala
                405                 410                 415

Asp Tyr Asn Tyr Lys Leu Pro Asp Asp Phe Thr Gly Cys Val Ile Ala
            420                 425                 430

Trp Asn Ser Asn Lys Leu Asp Ser Lys Val Ser Gly Asn Tyr Asn Tyr
        435                 440                 445

Leu Tyr Arg Leu Phe Arg Lys Ser Asn Leu Lys Pro Phe Glu Arg Asp
    450                 455                 460

Ile Ser Thr Glu Ile Tyr Gln Ala Gly Asn Lys Pro Cys Asn Gly Val
465                 470                 475                 480

Ala Gly Phe Asn Cys Tyr Phe Pro Leu Arg Ser Tyr Ser Phe Arg Pro
                485                 490                 495

Thr Tyr Gly Val Gly His Gln Pro Tyr Arg Val Val Val Leu Ser Phe
            500                 505                 510

Glu Leu Leu His Ala Pro Ala Thr Val Cys Gly Pro Lys Lys Ser Thr
        515                 520                 525

Asn Leu Val Lys Asn Lys Cys Val Asn Phe Asn Phe Asn Gly Leu Lys
    530                 535                 540

Gly Thr Gly Val Leu Thr Glu Ser Asn Lys Lys Phe Leu Pro Phe Gln
545                 550                 555                 560

Gln Phe Gly Arg Asp Ile Ala Asp Thr Thr Asp Ala Val Arg Asp Pro
                565                 570                 575

Gln Thr Leu Glu Ile Leu Asp Ile Thr Pro Cys Ser Phe Gly Gly Val
            580                 585                 590

Ser Val Ile Thr Pro Gly Thr Asn Thr Ser Asn Gln Val Ala Val Leu
        595                 600                 605

Tyr Gln Gly Val Asn Cys Thr Glu Val Pro Val Ala Ile His Ala Asp
    610                 615                 620

Gln Leu Thr Pro Thr Trp Arg Val Tyr Ser Thr Gly Ser Asn Val Phe
625                 630                 635                 640

Gln Thr Arg Ala Gly Cys Leu Ile Gly Ala Glu Tyr Val Asn Asn Ser
                645                 650                 655

Tyr Glu Cys Asp Ile Pro Ile Gly Ala Gly Ile Cys Ala Ser Tyr Gln
            660                 665                 670

Thr Gln Thr Lys Ser His Arg Arg Ala Arg Ser Val Ala Ser Gln Ser
        675                 680                 685

Ile Ile Ala Tyr Thr Met Ser Leu Gly Ala Glu Asn Ser Val Ala Tyr
    690                 695                 700

Ser Asn Asn Ser Ile Ala Ile Pro Thr Asn Phe Thr Ile Ser Val Thr
705                 710                 715                 720

Thr Glu Ile Leu Pro Val Ser Met Thr Lys Thr Ser Val Asp Cys Thr
                725                 730                 735

Met Tyr Ile Cys Gly Asp Ser Thr Glu Cys Ser Asn Leu Leu Leu Gln
        740                 745                 750

Tyr Gly Ser Phe Cys Thr Gln Leu Lys Arg Ala Leu Thr Gly Ile Ala
    755                 760                 765

Val Glu Gln Asp Lys Asn Thr Gln Glu Val Phe Ala Gln Val Lys Gln
770                 775                 780

Ile Tyr Lys Thr Pro Pro Ile Lys Tyr Phe Gly Gly Phe Asn Phe Ser
785                 790                 795                 800

Gln Ile Leu Pro Asp Pro Ser Lys Pro Ser Lys Arg Ser Phe Ile Glu
                805                 810                 815

Asp Leu Leu Phe Asn Lys Val Thr Leu Ala Asp Ala Gly Phe Ile Lys
            820                 825                 830

Gln Tyr Gly Asp Cys Leu Gly Asp Ile Ala Ala Arg Asp Leu Ile Cys
        835                 840                 845

Ala Gln Lys Phe Lys Gly Leu Thr Val Leu Pro Pro Leu Leu Thr Asp
    850                 855                 860

Glu Met Ile Ala Gln Tyr Thr Ser Ala Leu Leu Ala Gly Thr Ile Thr
865                 870                 875                 880

Ser Gly Trp Thr Phe Gly Ala Gly Ala Ala Leu Gln Ile Pro Phe Ala
                885                 890                 895

Met Gln Met Ala Tyr Arg Phe Asn Gly Ile Gly Val Thr Gln Asn Val
            900                 905                 910

Leu Tyr Glu Asn Gln Lys Leu Ile Ala Asn Gln Phe Asn Ser Ala Ile
        915                 920                 925

Gly Lys Ile Gln Asp Ser Leu Ser Ser Thr Ala Ser Ala Leu Gly Lys
    930                 935                 940

Leu Gln Asp Val Val Asn His Asn Ala Gln Ala Leu Asn Thr Leu Val
945                 950                 955                 960

Lys Gln Leu Ser Ser Lys Phe Gly Ala Ile Ser Ser Val Leu Asn Asp
                965                 970                 975

Ile Phe Ser Arg Leu Asp Pro Pro Glu Ala Glu Val Gln Ile Asp Arg
            980                 985                 990

Leu Ile Thr Gly Arg Leu Gln Ser  Leu Gln Thr Tyr Val  Thr Gln Gln
        995                 1000                1005

Leu Ile  Arg Ala Ala Glu Ile  Arg Ala Ser Ala Asn  Leu Ala Ala
    1010                1015                1020

Thr Lys  Met Ser Glu Cys Val  Leu Gly Gln Ser Lys  Arg Val Asp
    1025                1030                1035

Phe Cys  Gly Lys Gly Tyr His  Leu Met Ser Phe Pro  Gln Ser Ala
    1040                1045                1050

Pro His  Gly Val Val Phe Leu  His Val Thr Tyr Val  Pro Ala Gln
    1055                1060                1065

Glu Lys  Asn Phe Thr Thr Ala  Pro Ala Ile Cys His  Asp Gly Lys
    1070                1075                1080

Ala His  Phe Pro Arg Glu Gly  Val Phe Val Ser Asn  Gly Thr His
    1085                1090                1095

Trp Phe  Val Thr Gln Arg Asn  Phe Tyr Glu Pro Gln  Ile Ile Thr
    1100                1105                1110

Thr Asp  Asn Thr Phe Val Ser  Gly Asn Cys Asp Val  Val Ile Gly
    1115                1120                1125

Ile Val Asn Asn Thr Val Tyr Asp Pro Leu Gln Pro Glu Leu Asp
    1130                1135                1140

Ser Phe Lys Glu Glu Leu Asp Lys Tyr Phe Lys Asn His Thr Ser
    1145                1150                1155

Pro Asp Val Asp Leu Gly Asp Ile Ser Gly Ile Asn Ala Ser Val
    1160                1165                1170

Val Asn Ile Gln Lys Glu Ile Asp Arg Leu Asn Glu Val Ala Lys
    1175                1180                1185

Asn Leu Asn Glu Ser Leu Ile Asp Leu Gln Glu Leu Gly Lys Tyr
    1190                1195                1200

Glu Gln Tyr Ile Lys Trp Pro Trp Tyr Ile Trp Leu Gly Phe Ile
    1205                1210                1215

Ala Gly Leu Ile Ala Ile Val Met Val Thr Ile Met Leu Cys Cys
    1220                1225                1230

Met Thr Ser Cys Cys Ser Cys Leu Lys Gly Cys Cys Ser Cys Gly
    1235                1240                1245

Ser Cys Cys Lys Phe Asp Glu Asp Asp Ser Glu Pro Val Leu Lys
    1250                1255                1260

Gly Val Lys Leu His Tyr Thr
    1265                1270

<210>  50
<211>  3816
<212>  RNA
<213>  Artificial Sequence

<220>
<223>  Coding sequence

<400>  50
auguucgugu uccuggugcu gcugccucug guguccagcc agugugugaa ccugaccacc        60

agaacacagc ugccuccagc cuacaccaac agcuuuacca gaggcgugua cuaccccgac 120

aagguguuca gauccagcgu gcugcacucu acccaggacc uguuccugcc uuucuucagc 180

aacgugaccu gguuccacgu gaucuccggc accaauggca ccaagagauu cgacaacccc 240

gugcugcccu ucaacgacgg gguguacuuu gccagcaucg agaaguccaa caucaucaga 300

ggcuggaucu ucggcaccac acuggacagc aagacccaga gccugcugau cgugaacaac 360

gccaccaacg uggucaucaa agugugcgag uuccaguucu gcaacgaccc cuuccuggac 420

cacaagaaca acaagagcug gauggaaagc gaguuccggg uguacagcag cgccaacaac 480

ugcaccuucg aguacguguc ccagccuuuc cugauggacc uggaaggcaa gcagggcaac 540

uucaagaacc ugcgcgaguu cguguuuaag aacaucgacg gcuacuucaa gaucuacagc 600

aagcacaccc cuaucaucgu gagagagccc gaggaucugc cucagggcuu cucugcucug 660

gaaccccugg uggaucugcc caucggcauc aacaucaccc gguuucagac acugcuggcc 720

cugcacagaa gcuaccugac accuggcgau agcagcagcg gauggacagc uggugccgcc 780

gcuuacuaug ugggcuaccu gcagccuaga accuuccugc ugaaguacaa cgagaacggc 840

accaucaccg acgccgugga uugugcucug gauccucuga gcgagacaaa gugcacccug 900

aaguccuuca ccguggaaaa gggcaucuac cagaccagca acuuccgggu gcagcccacc 960

gaauccaucg ugcgguuccc caauaucacc aaucugugcc ccuucgacga gguguucaau 1020

gccaccagau ucgccucugu guacgccugg aaccggaagc ggaucagcaa uugcguggcc 1080

gacuacuccg ugcuguacaa ccuggccccc uucuucaccu ucaagugcua cggcgugucc 1140

ccuaccaagc ugaacgaccu gugcuucaca aacguguacg ccgacagcuu cgugauccgg 1200

ggagaugaag ugcggcagau ugccccugga cagacaggca acaucgccga cuacaacuac 1260

aagcugcccg acgacuucac cggcugugug auugccugga acagcaacaa gcuggacucc 1320

aaagucagcg gcaacuacaa uuaccuguac cggcuguucc ggaaguccaa ucugaagccc 1380

uucgagcggg acaucuccac cgagaucuau caggccggca acaagccuug uaacggcgug 1440

gccggcuuca acugcuacuu cccacugcgg uccuacagcu uuaggcccac auacggcgug 1500

ggccaccagc ccuacagagu gguggugcug agcuucgaac ugcugcaugc cccugccaca 1560

gugugcggcc cuaagaaaag caccaaucuc gugaagaaca aaugcgugaa cuucaacuuc 1620

aacggccuga agggcaccgg cgugcugaca gagagcaaca agaaguuccu gccauuccag 1680

caguuuggcc gggauaucgc cgauaccaca gacgccguua gagaucccca gacacuggaa 1740

auccuggaca ucaccccuug cagcuucggc ggagugucug ugaucacccc uggcaccaac 1800

accagcaauc agguggcagu gcuguaccag ggcgugaacu guaccgaagu gcccguggcc 1860

auucacgccg aucagcugac accuacaugg cggguguacu ccaccggcag caauguguuu 1920

cagaccagag ccggcugucu gaucggagcc gaguacguga acaauagcua cgagugcgac 1980

auccccaucg gcgcuggaau cugcgccagc uaccagacac agacaaagag ccaccggaga 2040

gccagaagcg uggccagcca gagcaucauu gccuacacaa ugucucuggg cgccgagaac 2100

agcguggccu acuccaacaa cucuaucgcu auccccacca acuucaccau cagcgugacc 2160

acagagaucc ugccuguguc caugaccaag accagcgugg acugcaccau guacaucugc 2220

ggcgauucca ccgagugcuc caaccugcug cugcaguacg gcagcuucug cacccagcug 2280

aaaagagccc ugacagggau cgccguggaa caggacaaga acacccaaga gguguucgcc 2340

caagugaagc agaucuacaa gaccccuccu aucaaguacu ucggcggcuu caauuucagc 2400

cagauucugc ccgauccuag caagcccagc aagcggagcu ucaucgagga ccugcuguuc 2460

aacaaaguga cacuggccga cgccggcuuc aucaagcagu auggcgauug ucugggcgac 2520

auugccgcca gggaucugau uugcgcccag aaguuuaagg gacugacagu gcugccuccu 2580

cugcugaccg augagaugau cgcccaguac acaucugccc ugcuggccgg cacaaucaca 2640

agcggcugga cauuuggagc aggcgccgcu cugcagaucc ccuuugcuau gcagauggcc 2700

uaccgguuca acggcaucgg agugacccag aaugugcugu acgagaacca gaagcugauc 2760

gccaaccagu ucaacagcgc caucggcaag auccaggaca gccugagcag cacagcaagc 2820

gcccugggaa agcugcagga cguggucaac cacaaugccc aggcacugaa cacccugguc 2880

aagcagcugu ccuccaaguu cggcgccauc agcucugugc ugaacgauau cuucagcaga 2940

cuggacccuc cugaggccga ggugcagauc gacagacuga ucacaggcag acugcagagc 3000

cuccagacau acgugaccca gcagcugauc agagccgccg agauuagagc cucugccaau 3060

cuggccgcca ccaagauguc ugagugugug cugggccaga gcaagagagu ggacuuuugc 3120

ggcaagggcu accaccugau gagcuucccu cagucugccc cucacggcgu gguguuucug 3180

cacgugacau augugcccgc ucaagagaag aauuucacca ccgcuccagc caucugccac 3240

gacggcaaag cccacuuucc uagagaaggc guguucgugu ccaacggcac ccauugguuc 3300

gugacacagc ggaacuucua cgagccccag aucaucacca ccgacaacac cuucgugucu 3360

ggcaacugcg acgucgugau cggcauugug aacaauaccg uguacgaccc ucugcagccc 3420

gagcuggaca gcuucaaaga ggaacuggac aaguacuuua agaaccacac aagccccgac 3480

guggaccugg gcgauaucag cggaaucaau gccagcgucg ugaacaucca gaaagagauc 3540

gaccggcuga acgagguggc caagaaucug aacgagagcc ugaucgaccu gcaagaacug 3600

gggaaguacg agcaguacau caaguggccc ugguacaucu ggcugggcuu uaucgccgga 3660

cugauugcca ucgugauggu cacaaucaug cuguguugca ugaccagcug cuguagcugc 3720

cugaagggcu guuguagcug uggcagcugc ugcaaguucg acgaggacga uucugagccc 3780

gugcugaagg gcgugaaacu gcacuacaca ugauga 3816

<210> 51
<211> 4274
<212> RNA
<213> Artificial Sequence

<220>
<223> Vaccine RNA

<400> 51
agaauaaacu aguauucuuc ugguccccac agacucagag agaacccgcc accauguucg 60

uguuccuggu gcugcugccu cuggugucca gccagugugu gaaccugacc accagaacac 120

agcugccucc agccuacacc aacagcuuua ccagaggcgu guacuacccc gacaaggugu 180

ucagauccag cgugcugcac ucuacccagg accuguuccu gccuuucuuc agcaacguga 240

ccugguucca cgugaucucc ggcaccaaug gcaccaagag auucgacaac cccgugcugc 300

ccuucaacga cgggguguac uuugccagca ucgagaaguc caacaucauc agaggcugga 360

ucuucggcac cacacuggac agcaagaccc agagccugcu gaucgugaac aacgccacca 420

acguggucau caaagugugc gaguuccagu ucugcaacga ccccuuccug gaccacaaga 480

acaacaagag cuggauggaa agcgaguucc ggguguacag cagcgccaac aacugcaccu 540

ucgaguacgu gucccagccu uuccugaugg accuggaagg caagcagggc aacuucaaga 600

accugcgcga guucguguuu aagaacaucg acggcuacuu caagaucuac agcaagcaca 660

ccccuaucau cgugagagag cccgaggauc ugccucaggg cuucucugcu cuggaacccc 720

ugguggaucu gcccaucggc aucaacauca cccgguuuca gacacugcug gcccugcaca 780

gaagcuaccu gacaccuggc gauagcagca gcggauggac agcuggugcc gccgcuuacu 840

augugggcua ccugcagccu agaaccuucc ugcugaagua caacgagaac ggcaccauca 900

ccgacgccgu ggauugugcu cuggauccuc ugagcgagac aaagugcacc cugaaguccu 960

ucaccgugga aaagggcauc uaccagacca gcaacuuccg ggugcagccc accgaaucca 1020

ucgugcgguu ccccaauauc accaaucugu gccccuucga cgagguguuc aaugccacca 1080

gauucgccuc uguguacgcc uggaaccgga agcggaucag caauugcgug gccgacuacu 1140

ccgugcugua caaccuggcc cccuucuuca ccuucaagug cuacggcgug uccccuacca 1200

agcugaacga ccugugcuuc acaaacgugu acgccgacag cuucgugauc cggggagaug 1260

aagugcggca gauugccccu ggacagacag gcaacaucgc cgacuacaac uacaagcugc 1320

ccgacgacuu caccggcugu gugauugccu ggaacagcaa caagcuggac uccaaaguca 1380

gcggcaacua caauuaccug uaccggcugu uccggaaguc caaucugaag cccuucgagc 1440

gggacaucuc caccgagauc uaucaggccg gcaacaagcc uuguaacggc guggccggcu 1500

ucaacugcua cuucccacug cgguccuaca gcuuuaggcc cacauacggc gugggccacc 1560

agcccuacag agugguggug cugagcuucg aacugcugca ugccccugcc acagugugcg 1620

gcccuaagaa aagcaccaau cucgugaaga acaaaugcgu gaacuucaac uucaacggcc 1680

ugaagggcac cggcgugcug acagagagca acaagaaguu ccugccauuc cagcaguuug 1740

gccgggauau cgccgauacc acagacgccg uuagagaucc ccagacacug gaaauccugg 1800

acaucacccc uugcagcuuc ggcggagugu cugugaucac cccuggcacc aacaccagca 1860

aucagguggc agugcuguac cagggcguga acuguaccga agugcccgug gccauucacg 1920

ccgaucagcu gacaccuaca uggcgggugu acuccaccgg cagcaaugug uuucagacca 1980

gagccggcug ucugaucgga gccgaguacg ugaacaauag cuacgagugc gacaucccca 2040

ucggcgcugg aaucugcgcc agcuaccaga cacagacaaa gagccaccgg agagccagaa 2100

gcguggccag ccagagcauc auugccuaca caaugucucu gggcgccgag aacagcgugg 2160

ccuacuccaa caacucuauc gcuaucccca ccaacuucac caucagcgug accacagaga 2220

uccugccugu guccaugacc aagaccagcg uggacugcac cauguacauc ugcggcgauu 2280

ccaccgagug cuccaaccug cugcugcagu acggcagcuu cugcacccag cugaaaagag 2340

cccugacagg gaucgccgug gaacaggaca agaacaccca agagguguuc gcccaaguga 2400

agcagaucua caagaccccu ccuaucaagu acuucggcgg cuucaauuuc agccagauuc 2460

ugcccgaucc uagcaagccc agcaagcgga gcuucaucga ggaccugcug uucaacaaag 2520

ugacacuggc cgacgccggc uucaucaagc aguauggcga uugucugggc gacauugccg 2580

ccagggaucu gauuugcgcc cagaaguuua agggacugac agugcugccu ccucugcuga 2640

ccgaugagau gaucgcccag uacacaucug cccugcuggc cggcacaauc acaagcggcu 2700

ggacauuugg agcaggcgcc gcucugcaga uccccuuugc uaugcagaug gccuaccggu 2760

ucaacggcau cggagugacc cagaaugugc uguacgagaa ccagaagcug aucgccaacc 2820

aguucaacag cgccaucggc aagauccagg acagccugag cagcacagca agcgcccugg 2880

gaaagcugca ggacgugguc aaccacaaug cccaggcacu gaacacccug gucaagcagc 2940

uguccuccaa guucggcgcc aucagcucug ugcugaacga uaucuucagc agacuggacc 3000

cuccugaggc cgaggugcag aucgacagac ugaucacagg cagacugcag agccuccaga 3060

cauacgugac ccagcagcug aucagagccg ccgagauuag agccucugcc aaucuggccg 3120

ccaccaagau gucugagugu gugcugggcc agagcaagag aguggacuuu ugcggcaagg 3180

gcuaccaccu gaugagcuuc ccucagucug ccccucacgg cgugguguuu cugcacguga 3240

cauaugugcc cgcucaagag aagaauuuca ccaccgcucc agccaucugc cacgacggca 3300

aagcccacuu uccuagagaa ggcguguucg uguccaacgg cacccauugg uucgugacac 3360

agcggaacuu cuacgagccc cagaucauca ccaccgacaa caccuucgug ucuggcaacu 3420

gcgacgucgu gaucggcauu gugaacaaua ccguguacga cccucugcag cccgagcugg 3480

acagcuucaa agaggaacug gacaaguacu uuaagaacca cacaagcccc gacguggacc 3540

ugggcgauau cagcggaauc aaugccagcg ucgugaacau ccagaaagag aucgaccggc 3600

ugaacgaggu ggccaagaau cugaacgaga gccugaucga ccugcaagaa cuggggaagu 3660

acgagcagua caucaagugg cccugguaca ucuggcuggg cuuuaucgcc ggacugauug 3720

ccaucgugau ggucacaauc augcuguguu gcaugaccag cugcuguagc ugccugaagg 3780

gcuguuguag cuguggcagc ugcugcaagu ucgacgagga cgauucugag cccgugcuga 3840

agggcgugaa acugcacuac acaugaugac ucgagcuggu acugcaugca cgcaaugcua 3900

gcugccccuu ucccguccug gguaccccga gucucccccg accucggguc ccagguaugc 3960

ucccaccucc accugcccca cucaccaccu cugcuaguuc cagacaccuc ccaagcacgc 4020

agcaaugcag cucaaaacgc uuagccuagc cacaccccca cgggaaacag cagugauuaa 4080

ccuuuagcaa uaaacgaaag uuuaacuaag cuauacuaac cccaggguug gucaauuucg 4140

ugccagccac acccuggagc uagcaaaaaa aaaaaaaaaa aaaaaaaaaa aaaagcauau 4200

gacuaaaaaa aaaaaaaaaa aaaaaaaaaa aaaaaaaaaa aaaaaaaaaa aaaaaaaaaa 4260

aaaaaaaaaa aaaa 4274

<210> 52
<211> 1270
<212> PRT
<213> Artificial Sequence

<220>
<223> Vaccine sequence

<400> 52

Met Phe Val Phe Leu Val Leu Leu Pro Leu Val Ser Ser Gln Cys Val
1               5                   10                  15

Asn Leu Thr Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Val Ile Ser Gly Thr Asn Gly Thr Lys Arg Phe Asp Asn Pro
65                  70                  75                  80

Val Leu Pro Phe Asn Asp Gly Val Tyr Phe Ala Ser Ile Glu Lys Ser
                85                  90                  95

Asn Ile Ile Arg Gly Trp Ile Phe Gly Thr Thr Leu Asp Ser Lys Thr
            100                 105                 110

Gln Ser Leu Leu Ile Val Asn Asn Ala Thr Asn Val Val Ile Lys Val
        115                 120                 125

Cys Glu Phe Gln Phe Cys Asn Asp Pro Phe Leu Asp His Lys Asn Asn
    130                 135                 140

Lys Ser Trp Met Glu Ser Glu Phe Arg Val Tyr Ser Ser Ala Asn Asn
145                 150                 155                 160

Cys Thr Phe Glu Tyr Val Ser Gln Pro Phe Leu Met Asp Leu Glu Gly
                165                 170                 175

Lys Gln Gly Asn Phe Lys Asn Leu Arg Glu Phe Val Phe Lys Asn Ile
            180                 185                 190

Asp Gly Tyr Phe Lys Ile Tyr Ser Lys His Thr Pro Ile Ile Val Arg
        195                 200                 205

Glu Pro Glu Asp Leu Pro Gln Gly Phe Ser Ala Leu Glu Pro Leu Val
    210                 215                 220

Asp Leu Pro Ile Gly Ile Asn Ile Thr Arg Phe Gln Thr Leu Leu Ala
225                 230                 235                 240

Leu His Arg Ser Tyr Leu Thr Pro Gly Asp Ser Ser Ser Gly Trp Thr
                245                 250                 255

Ala Gly Ala Ala Ala Tyr Tyr Val Gly Tyr Leu Gln Pro Arg Thr Phe
            260                 265                 270

Leu Leu Lys Tyr Asn Glu Asn Gly Thr Ile Thr Asp Ala Val Asp Cys
        275                 280                 285

Ala Leu Asp Pro Leu Ser Glu Thr Lys Cys Thr Leu Lys Ser Phe Thr
    290                 295                 300

Val Glu Lys Gly Ile Tyr Gln Thr Ser Asn Phe Arg Val Gln Pro Thr
305                 310                 315                 320

Glu Ser Ile Val Arg Phe Pro Asn Ile Thr Asn Leu Cys Pro Phe Asp
                325                 330                 335

Glu Val Phe Asn Ala Thr Arg Phe Ala Ser Val Tyr Ala Trp Asn Arg
            340                 345                 350

Lys Arg Ile Ser Asn Cys Val Ala Asp Tyr Ser Val Leu Tyr Asn Leu
        355                 360                 365

Ala Pro Phe Phe Thr Phe Lys Cys Tyr Gly Val Ser Pro Thr Lys Leu
    370                 375                 380

Asn Asp Leu Cys Phe Thr Asn Val Tyr Ala Asp Ser Phe Val Ile Arg
385                 390                 395                 400

Gly Asp Glu Val Arg Gln Ile Ala Pro Gly Gln Thr Gly Lys Ile Ala
                405                 410                 415

Asp Tyr Asn Tyr Lys Leu Pro Asp Asp Phe Thr Gly Cys Val Ile Ala
            420                 425                 430

Trp Asn Ser Asn Asn Leu Asp Ser Lys Val Gly Gly Asn Tyr Asn Tyr
        435                 440                 445

Leu Tyr Arg Leu Phe Arg Lys Ser Asn Leu Lys Pro Phe Glu Arg Asp
    450                 455                 460

Ile Ser Thr Glu Ile Tyr Gln Ala Gly Asn Lys Pro Cys Asn Gly Val
465                 470                 475                 480

Ala Gly Phe Asn Cys Tyr Phe Pro Leu Arg Ser Tyr Ser Phe Arg Pro
                485                 490                 495

Thr Tyr Gly Val Gly His Gln Pro Tyr Arg Val Val Val Leu Ser Phe
            500                 505                 510

Glu Leu Leu His Ala Pro Ala Thr Val Cys Gly Pro Lys Lys Ser Thr
        515                 520                 525

Asn Leu Val Lys Asn Lys Cys Val Asn Phe Asn Phe Asn Gly Leu Lys
    530                 535                 540

Gly Thr Gly Val Leu Thr Glu Ser Asn Lys Lys Phe Leu Pro Phe Gln
545                 550                 555                 560

Gln Phe Gly Arg Asp Ile Ala Asp Thr Thr Asp Ala Val Arg Asp Pro
                565                 570                 575

Gln Thr Leu Glu Ile Leu Asp Ile Thr Pro Cys Ser Phe Gly Gly Val
            580                 585                 590

Ser Val Ile Thr Pro Gly Thr Asn Thr Ser Asn Gln Val Ala Val Leu
        595                 600                 605

Tyr Gln Gly Val Asn Cys Thr Glu Val Pro Val Ala Ile His Ala Asp
    610                 615                 620

Gln Leu Thr Pro Thr Trp Arg Val Tyr Ser Thr Gly Ser Asn Val Phe
625                 630                 635                 640

Gln Thr Arg Ala Gly Cys Leu Ile Gly Ala Glu Tyr Val Asn Asn Ser
                645                 650                 655

Tyr Glu Cys Asp Ile Pro Ile Gly Ala Gly Ile Cys Ala Ser Tyr Gln
            660                 665                 670

Thr Gln Thr Lys Ser His Arg Arg Ala Arg Ser Val Ala Ser Gln Ser
        675                 680                 685

Ile Ile Ala Tyr Thr Met Ser Leu Gly Ala Glu Asn Ser Val Ala Tyr
    690                 695                 700

Ser Asn Asn Ser Ile Ala Ile Pro Thr Asn Phe Thr Ile Ser Val Thr
705                 710                 715                 720

Thr Glu Ile Leu Pro Val Ser Met Thr Lys Thr Ser Val Asp Cys Thr
                725                 730                 735

Met Tyr Ile Cys Gly Asp Ser Thr Glu Cys Ser Asn Leu Leu Leu Gln
            740                 745                 750

Tyr Gly Ser Phe Cys Thr Gln Leu Lys Arg Ala Leu Thr Gly Ile Ala
        755                 760                 765

Val Glu Gln Asp Lys Asn Thr Gln Glu Val Phe Ala Gln Val Lys Gln
    770                 775                 780

Ile Tyr Lys Thr Pro Pro Ile Lys Tyr Phe Gly Gly Phe Asn Phe Ser
785                 790                 795                 800

Gln Ile Leu Pro Asp Pro Ser Lys Pro Ser Lys Arg Ser Phe Ile Glu
                805                 810                 815

Asp Leu Leu Phe Asn Lys Val Thr Leu Ala Asp Ala Gly Phe Ile Lys
            820                 825                 830

Gln Tyr Gly Asp Cys Leu Gly Asp Ile Ala Ala Arg Asp Leu Ile Cys
        835                 840                 845

Ala Gln Lys Phe Lys Gly Leu Thr Val Leu Pro Pro Leu Leu Thr Asp
    850                 855                 860

Glu Met Ile Ala Gln Tyr Thr Ser Ala Leu Leu Ala Gly Thr Ile Thr
865                 870                 875                 880

Ser Gly Trp Thr Phe Gly Ala Gly Ala Ala Leu Gln Ile Pro Phe Ala
                885                 890                 895

Met Gln Met Ala Tyr Arg Phe Asn Gly Ile Gly Val Thr Gln Asn Val
            900                 905                 910

Leu Tyr Glu Asn Gln Lys Leu Ile Ala Asn Gln Phe Asn Ser Ala Ile
        915                 920                 925

Gly Lys Ile Gln Asp Ser Leu Ser Ser Thr Ala Ser Ala Leu Gly Lys
    930                 935                 940

Leu Gln Asp Val Val Asn His Asn Ala Gln Ala Leu Asn Thr Leu Val
945                 950                 955                 960

Lys Gln Leu Ser Ser Lys Phe Gly Ala Ile Ser Ser Val Leu Asn Asp
                965                 970                 975

Ile Phe Ser Arg Leu Asp Pro Pro Glu Ala Glu Val Gln Ile Asp Arg
            980                 985                 990

Leu Ile Thr Gly Arg Leu Gln Ser Leu Gln Thr Tyr Val Thr Gln Gln
        995                 1000                1005

Leu Ile Arg Ala Ala Glu Ile Arg Ala Ser Ala Asn Leu Ala Ala
    1010                1015                1020

Thr Lys Met Ser Glu Cys Val Leu Gly Gln Ser Lys Arg Val Asp
    1025                1030                1035

Phe Cys Gly Lys Gly Tyr His Leu Met Ser Phe Pro Gln Ser Ala
    1040                1045                1050

Pro His Gly Val Val Phe Leu His Val Thr Tyr Val Pro Ala Gln
    1055                1060                1065

Glu Lys Asn Phe Thr Thr Ala Pro Ala Ile Cys His Asp Gly Lys
    1070                1075                1080

Ala His Phe Pro Arg Glu Gly Val Phe Val Ser Asn Gly Thr His
    1085                1090                1095

Trp Phe Val Thr Gln Arg Asn Phe Tyr Glu Pro Gln Ile Ile Thr
    1100                1105                1110

Thr Asp Asn Thr Phe Val Ser Gly Asn Cys Asp Val Val Ile Gly
    1115                1120                1125

Ile Val Asn Asn Thr Val Tyr Asp Pro Leu Gln Pro Glu Leu Asp
    1130                1135                1140

Ser Phe Lys Glu Glu Leu Asp Lys Tyr Phe Lys Asn His Thr Ser
    1145                1150                1155

Pro Asp Val Asp Leu Gly Asp Ile Ser Gly Ile Asn Ala Ser Val
    1160                1165                1170

Val Asn Ile Gln Lys Glu Ile Asp Arg Leu Asn Glu Val Ala Lys
    1175                1180                1185

Asn Leu Asn Glu Ser Leu Ile Asp Leu Gln Glu Leu Gly Lys Tyr
    1190                1195                1200

Glu Gln Tyr Ile Lys Trp Pro Trp Tyr Ile Trp Leu Gly Phe Ile
    1205                1210                1215

Ala Gly Leu Ile Ala Ile Val Met Val Thr Ile Met Leu Cys Cys
    1220                1225                1230

Met Thr Ser Cys Cys Ser Cys Leu Lys Gly Cys Cys Ser Cys Gly
    1235                1240                1245

Ser Cys Cys Lys Phe Asp Glu Asp Asp Ser Glu Pro Val Leu Lys
    1250                1255                1260

Gly Val Lys Leu His Tyr Thr
    1265                1270

<210> 53
<211> 3816
<212> RNA
<213> Artificial Sequence

<220>
<223> Coding sequence

<400> 53
auguucgugu uccuggugcu gcugccucug guguccagcc agugugugaa ccugaccacc 60

agaacacagc ugccuccagc cuacaccaac agcuuuacca gaggcgugua cuaccccgac 120

aagguguuca gauccagcgu gcugcacucu acccaggacc uguuccugcc uuucuucagc 180

aacgugaccu gguuccacgu gaucuccggc accaauggca ccaagagauu cgacaacccc 240

gugcugcccu ucaacgacgg gguguacuuu gccagcaucg agaaguccaa caucaucaga 300

ggcuggaucu ucggcaccac acuggacagc aagacccaga gccugcugau cgugaacaac 360

gccaccaacg uggucaucaa agugugcgag uuccaguucu gcaacgaccc cuuccuggac 420

cacaagaaca acaagagcug gauggaaagc gaguuccggg uguacagcag cgccaacaac 480

ugcaccuucg aguacguguc ccagccuuuc cugauggacc uggaaggcaa gcagggcaac 540

uucaagaacc ugcgcgaguu cguguuuaag aacaucgacg gcuacuucaa gaucuacagc 600

aagcacaccc cuaucaucgu gagagagccc gaggaucugc cucagggcuu cucugcucug 660

gaaccccugg uggaucugcc caucggcauc aacaucaccc gguuucagac acugcuggcc 720

cugcacagaa gcuaccugac accuggcgau agcagcagcg gauggacagc uggugccgcc 780

gcuuacuaug ugggcuaccu gcagccuaga accuuccugc ugaaguacaa cgagaacggc 840

accaucaccg acgccgugga uugugcucug gauccucuga gcgagacaaa gugcacccug 900

aaguccuuca ccguggaaaa gggcaucuac cagaccagca acuuccgggu gcagcccacc 960

gaauccaucg ugcgguuccc caauaucacc aaucugugcc ccuucgacga gguguucaau 1020

gccaccagau ucgccucugu guacgccugg aaccggaagc ggaucagcaa uugcguggcc 1080

gacuacuccg ugcuguacaa ccuggccccc uucuucaccu ucaagugcua cggcgugucc 1140

ccuaccaagc ugaacgaccu gugcuucaca aacguguacg ccgacagcuu cgugauccgg 1200

ggagaugaag ugcggcagau ugccccugga cagacaggca agaucgccga cuacaacuac 1260

aagcugcccg acgacuucac cggcugugug auugccugga acagcaacaa ccuggacucc 1320

aaagucggcg gcaacuacaa uuaccuguac cggcuguucc ggaaguccaa ucugaagccc 1380

uucgagcggg acaucuccac cgagaucuau caggccggca acaagccuug uaacggcgug 1440

gccggcuuca acugcuacuu cccacugcgg uccuacagcu uuaggcccac auacggcgug 1500

ggccaccagc ccuacagagu gguggugcug agcuucgaac ugcugcaugc cccugccaca 1560

gugugcggcc cuaagaaaag caccaaucuc gugaagaaca aaugcgugaa cuucaacuuc 1620

aacggccuga agggcaccgg cgugcugaca gagagcaaca agaaguuccu gccauuccag 1680

caguuuggcc gggauaucgc cgauaccaca gacgccguua gagaucccca gacacuggaa 1740

auccuggaca ucaccccuug cagcuucggc ggagugucug ugaucacccc uggcaccaac 1800

accagcaauc agguggcagu gcuguaccag ggcgugaacu guaccgaagu gcccguggcc 1860

auucacgccg aucagcugac accuacaugg cggguguacu ccaccggcag caauguguuu 1920

cagaccagag ccggcugucu gaucggagcc gaguacguga acaauagcua cgagugcgac 1980

auccccaucg gcgcuggaau cugcgccagc uaccagacac agacaaagag ccaccggaga 2040

gccagaagcg uggccagcca gagcaucauu gccuacacaa ugucucuggg cgccgagaac 2100

agcguggccu acuccaacaa cucuaucgcu auccccacca acuucaccau cagcgugacc 2160

acagagaucc ugccuguguc caugaccaag accagcgugg acugcaccau guacaucugc 2220

ggcgauucca ccgagugcuc caaccugcug cugcaguacg gcagcuucug cacccagcug 2280

aaaagagccc ugacagggau cgccguggaa caggacaaga acacccaaga gguguucgcc 2340

caagugaagc agaucuacaa gaccccuccu aucaaguacu ucggcggcuu caauuucagc 2400

cagauucugc ccgauccuag caagcccagc aagcggagcu ucaucgagga ccugcuguuc 2460

aacaaaguga cacuggccga cgccggcuuc aucaagcagu auggcgauug ucugggcgac 2520

auugccgcca gggaucugau uugcgcccag aaguuuaagg gacugacagu gcugccuccu 2580

cugcugaccg augagaugau cgcccaguac acaucugccc ugcuggccgg cacaaucaca 2640

agcggcugga cauuuggagc aggcgccgcu cugcagaucc ccuuugcuau gcagauggcc 2700

uaccgguuca acggcaucgg agugacccag aaugugcugu acgagaacca gaagcugauc 2760

gccaaccagu ucaacagcgc caucggcaag auccaggaca gccugagcag cacagcaagc 2820

gcccugggaa agcugcagga cguggucaac cacaaugccc aggcacugaa cacccugguc 2880

aagcagcugu ccuccaaguu cggcgccauc agcucugugc ugaacgauau cuucagcaga 2940

cuggacccuc cugaggccga ggugcagauc gacagacuga ucacaggcag acugcagagc 3000

cuccagacau acgugaccca gcagcugauc agagccgccg agauuagagc cucugccaau 3060

cuggccgcca ccaagauguc ugagugugug cugggccaga gcaagagagu ggacuuuugc 3120

ggcaagggcu accaccugau gagcuucccu cagucugccc cucacggcgu gguguuucug 3180

cacgugacau augugcccgc ucaagagaag aauuucacca ccgcuccagc caucugccac 3240

gacggcaaag cccacuuucc uagagaaggc guguucgugu ccaacggcac ccauugguuc 3300

gugacacagc ggaacuucua cgagccccag aucaucacca ccgacaacac cuucgugucu 3360

ggcaacugcg acgucgugau cggcauugug aacaauaccg uguacgaccc ucugcagccc 3420

gagcuggaca gcuucaaaga ggaacuggac aaguacuuua agaaccacac aagccccgac 3480

guggaccugg gcgauaucag cggaaucaau gccagcgucg ugaacaucca gaaagagauc 3540

gaccggcuga acgagguggc caagaaucug aacgagagcc ugaucgaccu gcaagaacug 3600

gggaaguacg agcaguacau caaguggccc ugguacaucu ggcugggcuu uaucgccgga 3660

cugauugcca ucgugauggu cacaaucaug cuguguugca ugaccagcug cuguagcugc 3720

cugaagggcu guuguagcug uggcagcugc ugcaaguucg acgaggacga uucugagccc 3780

gugcugaagg gcgugaaacu gcacuacaca ugauga 3816

<210> 54
<211> 4274
<212> RNA
<213> Artificial Sequence

<220>
<223> Vaccine RNA

<400> 54
agaauaaacu aguauucuuc ugguccccac agacucagag agaacccgcc accauguucg 60

uguuccuggu gcugcugccu cuggugucca gccagugugu gaaccugacc accagaacac 120

agcugccucc agccuacacc aacagcuuua ccagaggcgu guacuacccc gacaaggugu 180

ucagauccag cgugcugcac ucuacccagg accuguuccu gccuuucuuc agcaacguga 240

ccugguucca cgugaucucc ggcaccaaug gcaccaagag auucgacaac cccgugcugc 300

ccuucaacga cgggguguac uuugccagca ucgagaaguc caacaucauc agaggcugga 360

ucuucggcac cacacuggac agcaagaccc agagccugcu gaucgugaac aacgccacca 420

acguggucau caaagugugc gaguuccagu ucugcaacga ccccuuccug gaccacaaga 480

acaacaagag cuggauggaa agcgaguucc ggguguacag cagcgccaac aacugcaccu 540

ucgaguacgu gucccagccu uuccugaugg accuggaagg caagcagggc aacuucaaga 600

accugcgcga guucguguuu aagaacaucg acggcuacuu caagaucuac agcaagcaca 660

ccccuaucau cgugagagag cccgaggauc ugccucaggg cuucucugcu cuggaacccc 720

ugguggaucu gcccaucggc aucaacauca cccgguuuca gacacugcug gcccugcaca 780

gaagcuaccu gacaccuggc gauagcagca gcggauggac agcuggugcc gccgcuuacu 840

augugggcua ccugcagccu agaaccuucc ugcugaagua caacgagaac ggcaccauca 900

ccgacgccgu ggauugugcu cuggauccuc ugagcgagac aaagugcacc cugaaguccu 960

ucaccgugga aaagggcauc uaccagacca gcaacuuccg ggugcagccc accgaaucca 1020

ucgugcgguu ccccaauauc accaaucugu gccccuucga cgagguguuc aaugccacca 1080

gauucgccuc uguguacgcc uggaaccgga agcggaucag caauugcgug gccgacuacu 1140

ccgugcugua caaccuggcc cccuucuuca ccuucaagug cuacggcgug uccccuacca 1200

agcugaacga ccugugcuuc acaaacgugu acgccgacag cuucgugauc cggggagaug 1260

aagugcggca gauugccccu ggacagacag gcaagaucgc cgacuacaac uacaagcugc 1320

ccgacgacuu caccggcugu gugauugccu ggaacagcaa caaccuggac uccaaagucg 1380

gcggcaacua caauuaccug uaccggcugu uccggaaguc caaucugaag cccuucgagc 1440

gggacaucuc caccgagauc uaucaggccg gcaacaagcc uuguaacggc guggccggcu 1500

ucaacugcua cuucccacug cgguccuaca gcuuuaggcc cacauacggc gugggccacc 1560

agcccuacag agugguggug cugagcuucg aacugcugca ugccccugcc acagugugcg 1620

gcccuaagaa aagcaccaau cucgugaaga acaaaugcgu gaacuucaac uucaacggcc 1680

ugaagggcac cggcgugcug acagagagca acaagaaguu ccugccauuc cagcaguuug 1740

gccgggauau cgccgauacc acagacgccg uuagagaucc ccagacacug gaaauccugg 1800

acaucacccc uugcagcuuc ggcggagugu cugugaucac cccuggcacc aacaccagca 1860

aucagguggc agugcuguac cagggcguga acuguaccga agugcccgug gccauucacg 1920

ccgaucagcu gacaccuaca uggcgggugu acuccaccgg cagcaaugug uuucagacca 1980

gagccggcug ucugaucgga gccgaguacg ugaacaauag cuacgagugc gacaucccca 2040

ucggcgcugg aaucugcgcc agcuaccaga cacagacaaa gagccaccgg agagccagaa 2100

gcguggccag ccagagcauc auugccuaca caaugucucu gggcgccgag aacagcgugg 2160

ccuacuccaa caacucuauc gcuaucccca ccaacuucac caucagcgug accacagaga 2220

uccugccugu guccaugacc aagaccagcg uggacugcac cauguacauc ugcggcgauu 2280

ccaccgagug cuccaaccug cugcugcagu acggcagcuu cugcacccag cugaaaagag 2340

cccugacagg gaucgccgug gaacaggaca agaacaccca agagguguuc gcccaaguga 2400

agcagaucua caagaccccu ccuaucaagu acuucggcgg cuucaauuuc agccagauuc 2460

ugcccgaucc uagcaagccc agcaagcgga gcuucaucga ggaccugcug uucaacaaag 2520

ugacacuggc cgacgccggc uucaucaagc aguauggcga uugucugggc gacauugccg 2580

ccagggaucu gauuugcgcc cagaaguuua agggacugac agugcugccu ccucugcuga 2640

ccgaugagau gaucgcccag uacacaucug cccugcuggc cggcacaauc acaagcggcu 2700

ggacauuugg agcaggcgcc gcucugcaga uccccuuugc uaugcagaug gccuaccggu 2760

ucaacggcau cggagugacc cagaaugugc uguacgagaa ccagaagcug aucgccaacc 2820

aguucaacag cgccaucggc aagauccagg acagccugag cagcacagca agcgcccugg 2880

gaaagcugca ggacgugguc aaccacaaug cccaggcacu gaacacccug gucaagcagc 2940

uguccuccaa guucggcgcc aucagcucug ugcugaacga uaucuucagc agacuggacc 3000

cuccugaggc cgaggugcag aucgacagac ugaucacagg cagacugcag agccuccaga 3060

cauacgugac ccagcagcug aucagagccg ccgagauuag agccucugcc aaucuggccg 3120

ccaccaagau gucugagugu gugcugggcc agagcaagag aguggacuuu ugcggcaagg 3180

gcuaccaccu gaugagcuuc ccucagucug ccccucacgg cgugguguuu cugcacguga 3240

cauaugugcc cgcucaagag aagaauuuca ccaccgcucc agccaucugc cacgacggca 3300

aagcccacuu uccuagagaa ggcguguucg uguccaacgg cacccauugg uucgugacac 3360

agcggaacuu cuacgagccc cagaucauca ccaccgacaa caccuucgug ucuggcaacu 3420

gcgacgucgu gaucggcauu gugaacaaua ccguguacga cccucugcag cccgagcugg 3480

acagcuucaa agaggaacug gacaaguacu uuaagaacca cacaagcccc gacguggacc 3540

ugggcgauau cagcggaauc aaugccagcg ucgugaacau ccagaaagag aucgaccggc 3600

ugaacgaggu ggccaagaau cugaacgaga gccugaucga ccugcaagaa cuggggaagu 3660

acgagcagua caucaagugg cccugguaca ucuggcuggg cuuuaucgcc ggacugauug 3720

ccaucgugau ggucacaauc augcuguguu gcaugaccag cugcuguagc ugccugaagg 3780

gcuguuguag cuguggcagc ugcugcaagu ucgacgagga cgauucugag cccgugcuga 3840

agggcgugaa acugcacuac acaugaugac ucgagcuggu acugcaugca cgcaaugcua 3900

gcugccccuu ucccguccug gguaccccga gucucccccg accucggguc ccagguaugc 3960

ucccaccucc accugcccca cucaccaccu cugcuaguuc cagacaccuc ccaagcacgc 4020

agcaaugcag cucaaaacgc uuagccuagc cacaccccca cgggaaacag cagugauuaa 4080

ccuuuagcaa uaaacgaaag uuuaacuaag cuauacuaac cccaggguug gucaauuucg 4140

ugccagccac acccuggagc uagcaaaaaa aaaaaaaaaa aaaaaaaaaa aaaagcauau 4200

gacuaaaaaa aaaaaaaaaa aaaaaaaaaa aaaaaaaaaa aaaaaaaaaa aaaaaaaaaa 4260

aaaaaaaaaa aaaa 4274

## Claims

**1.** A composition or medical preparation comprising RNA comprising a nucleotide sequence encoding a SARS-CoV-2 S protein comprising one or more mutations characteristic of an Omicron variant, or an immunogenic fragment thereof.

**2.** The composition or medical preparation of claim 1, wherein the immunogenic fragment of the SARS-CoV-2 S protein comprises the S1 subunit of the SARS-CoV-2 S protein, or the receptor binding domain (RBD) of the S1 subunit of the SARS-CoV-2 S protein.

**3.** The composition or medical preparation of claim 1 or 2, wherein the SARS-CoV-2 S protein or immunogenic fragment thereof comprises one or more mutations characteristic of a BA.4/5, BA.1, BA.2, XBB, XBB.1, or BQ.1.1 Omicron variant or sublineages thereof.

**4.** An immunogenic composition comprising: a lipid nanoparticle (LNP) comprising an RNA, wherein the RNA encodes the polypeptide of SEQ ID NO: 69 and comprises the nucleotide sequence of SEQ ID NO: 70 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 70, and wherein the RNA comprises:
(a) modified uridines; and
(b) a 5' cap; and
wherein the LNP comprises a cationically ionizable lipid, a neutral lipid, a sterol and a polymer-lipid conjugate.

**5.** The immunogenic composition of claim 4, wherein the nucleotide sequence includes modified uridines in place of all uridines.

**6.** The immunogenic composition of claim 4 or 5, wherein the modified uridines are each N1-methyl-pseudou ridine.

**7.** The immunogenic composition of any one of claims 4 to 6, wherein the RNA further comprises at least one, at least two, or all of the following features:
a 5' untranslated region (UTR) comprising SEQ ID NO: 12;
a 3' untranslated region (UTR) comprising SEQ ID NO: 13; and
a poly-A sequence of at least 100 A nucleotides.

**8.** The immunogenic composition of claim 7, wherein the poly-A sequence comprises 30 adenine nucleotides followed by 70 adenine nucleotides, wherein the 30 adenine nucleotides and 70 adenine nucleotides are separated by a linker sequence.

**9.** The immunogenic composition of claim 7, wherein the poly-A sequence comprises SEQ ID NO: 14.

**10.** The immunogenic composition of any one of claims 4 to 9, wherein the RNA comprises SEQ ID NO: 72.

**11.** An immunogenic composition comprising: a lipid nanoparticle (LNP) comprising an RNA, wherein the RNA encodes the polypeptide of SEQ ID NO: 49 and comprises the nucleotide sequence of SEQ ID NO: 50 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 50, and wherein the RNA comprises:
(a) modified uridines; and
(b) a 5' cap; and
wherein the LNP comprises a cationically ionizable lipid, a neutral lipid, a sterol and a polymer-lipid conjugate.

**12.** The immunogenic composition of claim 11, wherein the nucleotide sequence includes modified uridines in place of all uridines.

**13.** The immunogenic composition of claim 11 or 12, wherein the modified uridines are each N1-methyl-pseudouridine.

**14.** The immunogenic composition of any one of claims 11 to 13, wherein the RNA further comprises at least one, at least two, or all of the following features:
a 5' untranslated region (UTR) comprising SEQ ID NO: 12;
a 3' untranslated region (UTR) comprising SEQ ID NO: 13; and
a poly-A sequence of at least 100 A nucleotides.

**15.** The immunogenic composition of claim 14, wherein the poly-A sequence comprises 30 adenine nucleotides followed by 70 adenine nucleotides, wherein the 30 adenine nucleotides and 70 adenine nucleotides are separated by a linker sequence.

**16.** The immunogenic composition of claim 15, wherein the poly-A sequence comprises SEQ ID NO: 14.

**17.** The immunogenic composition of any one of claims 11 to 16, wherein the RNA comprises SEQ ID NO: 51.

**18.** An immunogenic composition comprising a lipid nanoparticle (LNP) comprising an RNA, wherein the RNA encodes the polypeptide of SEQ ID NO: 55 and comprises the nucleotide sequence of SEQ ID NO: 56 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 56, and wherein the RNA comprises:
(a) modified uridines; and
(b) a 5' cap; and
wherein the LNP comprises a cationically ionizable lipid, a neutral lipid, a sterol and a polymer-lipid conjugate.

**19.** The immunogenic composition of claim 18, wherein the nucleotide sequence includes modified uridines in place of all uridines.

**20.** The immunogenic composition of claim 18 or 19, wherein the modified uridines are each N1-methyl-pseudouridine.

**21.** The immunogenic composition of any one of claims 18 to 20, wherein the RNA further comprises at least one, at least two, or all of the following features:
a 5' untranslated region (UTR) comprising SEQ ID NO: 12; and
a 3' untranslated region (UTR) comprising SEQ ID NO: 13; and
a poly-A sequence of at least 100 A nucleotides.

**22.** The immunogenic composition of claim 21, wherein the poly-A sequence comprises 30 adenine nucleotides followed by 70 adenine nucleotides, wherein the 30 adenine nucleotides and 70 adenine nucleotides are separated by a linker sequence.

**23.** The immunogenic composition of claim 22, wherein the poly-A sequence comprises SEQ ID NO: 14.

**24.** The immunogenic composition of any one of claims 18 to 23, wherein the RNA comprises SEQ ID NO: 57.

**25.** An immunogenic composition comprising a a lipid nanoparticle (LNP) comprising an RNA, wherein the RNA encodes the polypeptide of SEQ ID NO: 58 and comprises the nucleotide sequence of SEQ ID NO: 59 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 59, and wherein the RNA comprises:
(a) modified uridines; and
(b) a 5' cap; and
wherein the LNP comprises a cationically ionizable lipid, a neutral lipid, a sterol and a polymer-lipid conjugate.

**26.** The immunogenic composition of claim 25, wherein the nucleotide sequence includes modified uridines in place of all uridines.

**27.** The immunogenic composition of claim 25 or 26, wherein the modified uridines are each N1-methyl-pseudouridine.

**28.** The immunogenic composition of any one of claims 25 to 27, wherein the RNA further comprises at least one, at least two, or all of the following features:
a 5' untranslated region (UTR) comprising SEQ ID NO: 12;
a 3' untranslated region (UTR) comprising SEQ ID NO: 13; and
a poly-A sequence of at least 100 A nucleotides.

**29.** The immunogenic composition of claim 28, wherein the poly-A sequence comprises 30 adenine nucleotides followed by 70 adenine nucleotides, wherein the 30 adenine nucleotides and 70 adenine nucleotides are separated by a linker sequence.

**30.** The immunogenic composition of claim 29, wherein the poly-A sequence comprises SEQ ID NO: 14.

**31.** The immunogenic composition of any one of claims 25 to 30, wherein the RNA comprises SEQ ID NO: 60.

**32.** An immunogenic composition comprising a lipid nanoparticle (LNP) comprising an RNA, wherein the RNA encodes the polypeptide of SEQ ID NO: 61 and comprises the nucleotide sequence of SEQ ID NO: 62a or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 62a, and wherein the RNA comprises:
(a) modified uridines; and
(b) a 5' cap; and
wherein the LNP comprises a cationically ionizable lipid, a neutral lipid, a sterol and a polymer-lipid conjugate .

**33.** The immunogenic composition of claim 32, wherein the nucleotide sequence includes modified uridines in place of all uridines.

**34.** The immunogenic composition of claim 32 or 33, wherein the modified uridines are each N1-methyl-pseudouridine.

**35.** The immunogenic composition of any one of claims 32 to 34, wherein the RNA further comprises at least one, at least two, or all of the following features:
a 5' untranslated region (UTR) comprising SEQ ID NO: 12;
a 3' untranslated region (UTR) comprising SEQ ID NO: 13; and
a poly-A sequence of at least 100 A nucleotides.

**36.** The immunogenic composition of claim 35, wherein the poly-A sequence comprises 30 adenine nucleotides followed by 70 adenine nucleotides, wherein the 30 adenine nucleotides and 70 adenine nucleotides are separated by a linker sequence.

**37.** The immunogenic composition of claim 36, wherein the poly-A sequence comprises SEQ ID NO: 14.

**38.** The immunogenic composition of any one of claims 32 to 37, wherein the RNA comprises SEQ ID NO: 63a.

**39.** An immunogenic composition comprising a first RNA and a second RNA, wherein:
the first RNA encodes the polypeptide of SEQ ID NO: 7 and comprises the nucleotide sequence of SEQ ID NO: 9 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 9, and
the second RNA encodes the polypeptide of SEQ ID NO: 69 and comprises the nucleotide sequence of SEQ ID NO: 70 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 70, and
wherein each of the first RNA and the second RNA comprise:
(a) modified uridines; and
(b) a 5' cap, and
wherein the first RNA and the second RNA are formulated in lipid nanoparticles (LNPs), wherein the LNPs comprise a cationically ionizable lipid, a neutral lipid, a sterol and a polymer-lipid conjugate.

**40.** The immunogenic composition of claim 39, wherein the first RNA and the second RNA are formulated in the same lipid nanoparticles.

**41.** The immunogenic composition of claim 39, wherein the first RNA and the second RNA are formulated in seperate lipid nanoparticles.

**42.** The immunogenic composition of any one of claims 39 to 41, wherein each of the first RNA and the second RNA include modified uridines in place of all uridines.

**43.** The immunogenic composition of any one of claims 39 to 42, wherein the modified uridines are each N1-methyl-pseudouridine.

**44.** The immunogenic composition of any one of claims 39 to 43, wherein the first RNA and the second RNA each indepedently comprise at least one, at least two, or all of the following features:
a 5' untranslated region (UTR) comprising SEQ ID NO: 12;
a 3' untranslated region (UTR) comprising SEQ ID NO: 13; and
a poly-A sequence of at least 100 A nucleotides.

**45.** The immunogenic composition of claim 44, wherein the poly-A sequence comprises 30 adenine nucleotides followed by 70 adenine nucleotides, wherein the 30 adenine nucleotides and 70 adenine nucleotides are separated by a linker sequence.

**46.** The immunogenic composition of claim 44, wherein the poly-A sequence comprises SEQ ID NO: 14.

**47.** An immunogenic composition comprising a first RNA and a second RNA, wherein:
the first RNA encodes the polypeptide of SEQ ID NO: 7 and comprises the nucleotide sequence of SEQ ID NO: 9 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 9, and
the second RNA encodes the polypeptide of SEQ ID NO: 49 and comprises the nucleotide sequence of SEQ ID NO: 50 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 50, and
wherein each of the first RNA and the second RNA comprise:
(a) modified uridines; and
(b) a 5' cap, and
wherein the first RNA and the second RNA are formulated in lipid nanoparticles (LNPs), wherein the LNPs comprise a cationically ionizable lipid, a neutral lipid, a sterol and a polymer-lipid conjugate.

**48.** The immunogenic composition of claim 47, wherein the first RNA and the second RNA are formulated in the same lipid nanoparticles.

**49.** The immunogenic composition of claim 47, wherein the first RNA and the second RNA are formulated in seperate lipid nanoparticles.

**50.** The immunogenic composition of any one of claims 47 to 49, wherein each of the first RNA and the second RNA include modified uridines in place of all uridines.

**51.** The immunogenic composition of any one of claims 47 to 50, wherein the modified uridines are each N1-methyl-pseudouridine.

**52.** The immunogenic composition of any one of claims 47 to 51, wherein the first RNA and the second RNA each indepedently comprise at least one, at least two, or all of the following features:
a 5' untranslated region (UTR) comprising SEQ ID NO: 12;
a 3' untranslated region (UTR) comprising SEQ ID NO: 13; and
a poly-A sequence of at least 100 A nucleotides.

**53.** The immunogenic composition of claim 52, wherein the poly-A sequence comprises 30 adenine nucleotides followed by 70 adenine nucleotides, wherein the 30 adenine nucleotides and 70 adenine nucleotides are separated by a linker sequence.

**54.** The immunogenic composition of claim 52, wherein the poly-A sequence comprises SEQ ID NO: 14.

**55.** The immunogenic composition of any one of claims 47 to 54, wherein the first RNA comprises SEQ ID NO: 20 and the second RNA comprises SEQ ID NO: 51.

**56.** An immunogenic composition comprising a first RNA and a second RNA, wherein:
the first RNA comprises a nucleotide sequence that encodes the polypeptide of SEQ ID NO: 7 and comprises the nucleotide sequence of SEQ ID NO: 9 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 9, and
the second RNA comprises a nucleotide sequence that encodes the polypeptide of SEQ ID NO: 55, 58, or 61 and comprises the nucleotide sequence of SEQ ID NO: 56, 59, or 62a, or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 56, 59, or 62a, and
wherein each of the first RNA and the second RNA comprise:
(a) modified uridines; and
(b) a 5' cap, and
wherein the first RNA and the second RNA are formulated in lipid nanoparticles (LNPs), wherein the LNPs comprise a cationically ionizable lipid, a neutral lipid, a sterol and a polymer-lipid conjugate.

**57.** The immunogenic composition of claim 56, wherein the first RNA and the second RNA are formulated in separate lipid nanoparticles.

**58.** The immunogenic composition of claim 56, wherein the first RNA and the second RNA are formulated in the same lipid nanoparticles.

**59.** The immunogenic composition of any one of claims 56 to 58, wherein the first RNA and the second RNA each include modified uridines in place of all uridines.

**60.** The immunogenic composition of any one of claims 56 to 59, wherein the modified uridines are each N1-methyl-pseudouridine.

**61.** The immunogenic composition of any one of claims 56 to 60, wherein the first RNA and the second RNA each independently further comprise at least one, at least two, or all of the following features:
a 5' untranslated region (UTR) comprising SEQ ID NO: 12;
a 3' untranslated region (UTR) comprising SEQ ID NO: 13; and
a poly-A sequence of at least 100 A nucleotides.

**62.** The immunogenic composition of claim 61, wherein the poly-A sequence comprises 30 adenine nucleotides followed by 70 adenine nucleotides, wherein the 30 adenine nucleotides and 70 adenine nucleotides are separated by a linker sequence.

**63.** The immunogenic composition of claim 62, wherein the poly-A sequence comprises SEQ ID NO: 14.

**64.** The immunogenic composition of any one of claims 56 to 63, wherein the first RNA comprises SEQ ID NO: 9 and the second RNA comprises SEQ ID NO: 56.

**65.** The immunogenic composition of any one of claims 56 to 63, wherein the first RNA comprises SEQ ID NO: 9 and the second RNA comprises SEQ ID NO: 59.

**66.** The immunogenic composition of any one of claims 56 to 63, wherein the first RNA comprises SEQ ID NO: 9 and the second RNA comprises SEQ ID NO: 62a.

**67.** The immunogenic composition of any one of claims 56 to 63, wherein the first RNA comprises SEQ ID NO: 20 and the second RNA comprises SEQ ID NO: 57.

**68.** The immunogenic composition of any one of claims 56 to 63, wherein the first RNA comprises SEQ ID NO: 20 and the second RNA comprises SEQ ID NO: 60.

**69.** The immunogenic composition of any one of claims 56 to 63, wherein the first RNA comprises SEQ ID NO: 20 and the second RNA comprises SEQ ID NO: 63a.

**70.** An immunogenic composition comprising a first RNA and a second RNA, wherein:
the first RNA encodes the polypeptide of SEQ ID NO: 58 and comprises the nucleotide sequence of SEQ ID NO: 59 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 59, and
the second RNA encodes the polypeptide of SEQ ID NO: 49, 55, or 61 and comprises the nucleotide sequence of SEQ ID NO: 50, 56, or 62a, or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 50, 56, or 62a, and
wherein each of the first RNA and the second RNA comprise:
(a) modified uridines; and
(b) a 5' cap, and
wherein the first RNA and the second RNA are formulated in lipid nanoparticles (LNPs), wherein the LNPs comprise a cationically ionizable lipid, a neutral lipid, a sterol and a polymer-lipid conjugate.

**71.** The immunogenic composition of claim 70, wherein the first RNA and the second RNA are formulated in separate lipid nanoparticles.

**72.** The immunogenic composition of claim 70, wherein the first RNA and the second RNA are formulated in the same lipid nanoparticles.

**73.** The immunogenic composition of any one of claims 70 to 72, wherein the first RNA and the second RNA each include modified uridines in place of all uridines.

**74.** The immunogenic composition of any one of claims 70 to 73, wherein the modified uridines are each N1-methyl-pseudouridine.

**75.** The immunogenic composition of any one of claims 70 to 74, wherein the first RNA and the second RNA each independently further comprise at least one, at least two, or all of the following features:
a 5' untranslated region (UTR) comprising SEQ ID NO: 12;
a 3' untranslated region (UTR) comprising SEQ ID NO: 13; and
a poly-A sequence of at least 100 A nucleotides.

**76.** The immunogenic composition of claim 75, wherein the poly-A sequence comprises 30 adenine nucleotides followed by 70 adenine nucleotides, wherein the 30 adenine nucleotides and 70 adenine nucleotides are separated by a linker sequence.

**77.** The immunogenic composition of claim 76, wherein the poly-A sequence comprises SEQ ID NO: 14.

**78.** The immunogenic composition of any one of claims 70 to 77, wherein the first RNA comprises SEQ ID NO: 59 and the second RNA comprises SEQ ID NO: 50.

**79.** The immunogenic composition of any one of claims 70 to 77, wherein the first RNA comprises SEQ ID NO: 59 and the second RNA comprises SEQ ID NO: 56.

**80.** The immunogenic composition of any one of claims 70 to 77, wherein the first RNA comprises SEQ ID NO: 59 and the second RNA comprises SEQ ID NO: 62a.

**81.** The immunogenic composition of any one of claims 70 to 77, wherein the first RNA comprises SEQ ID NO: 60 and the second RNA comprises SEQ ID NO: 51.

**82.** The immunogenic composition of any one of claims 70 to 77, wherein the first RNA comprises SEQ ID NO: 60 and the second RNA comprises SEQ ID NO: 57.

**83.** The immunogenic composition of any one of claims 70 to 77, wherein the first RNA comprises SEQ ID NO: 60 and the second RNA comprises SEQ ID NO: 63a.

**84.** An immunogenic composition comprising a first RNA and a second RNA, wherein:
the first RNA encodes the polypeptide of SEQ ID NO: 49 and comprises the nucleotide sequence of SEQ ID NO: 50 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 50, and
the second RNA encodes the polypeptide of SEQ ID NO: 55 or 61 and comprises the nucleotide sequence of SEQ ID NO: 56 or 62a, or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 56 or 62a, and
wherein each of the first RNA and the second RNA comprise:
(a) modified uridines; and
(b) a 5' cap, and
wherein the first RNA and the second RNA are formulated in lipid nanoparticles (LNPs), wherein the LNPs comprise a cationically ionizable lipid, a neutral lipid, a sterol and a polymer-lipid conjugate.

**85.** The immunogenic composition of claim 84, wherein the first RNA and the second RNA are formulated in separate lipid nanoparticles.

**86.** The immunogenic composition of claim 84, wherein the first RNA and the second RNA are formulated in the same lipid nanoparticles.

**87.** The immunogenic composition of any one of claims 84 to 86, wherein the first RNA and the second RNA each include modified uridines in place of all uridines.

**88.** The immunogenic composition of any one of claims 84 to 87, wherein the modified uridines are each N1-methyl-pseudouridine.

**89.** The immunogenic composition of any one of claims 84 to 88, wherein the first RNA and the second RNA further each independently further comprise at least one, at least two, or all of the following features:
a 5' untranslated region (UTR) comprising SEQ ID NO: 12;
a 3' untranslated region (UTR) comprising SEQ ID NO: 13; and
a poly-A sequence of at least 100 A nucleotides.

**90.** The immunogenic composition of claim 89, wherein the poly-A sequence comprises 30 adenine nucleotides followed by 70 adenine nucleotides, wherein the 30 adenine nucleotides and 70 adenine nucleotides are separated by a linker sequence.

**91.** The immunogenic composition of claim 90, wherein the poly-A sequence comprises SEQ ID NO: 14.

**92.** The immunogenic composition of any one of claims 84 to 91, wherein the first RNA comprises SEQ ID NO: 50 and the second RNA comprises SEQ ID NO: 56.

**93.** The immunogenic composition of any one of claims 84 to 91, wherein the first RNA comprises SEQ ID NO: 50 and the second RNA comprises SEQ ID NO: 62a.

**94.** The immunogenic composition of any one of claims 84 to 91, wherein the first RNA comprises SEQ ID NO: 51 and the second RNA comprises SEQ ID NO: 57.

**95.** The immunogenic composition of any one of claims 84 to 91, wherein the first RNA comprises SEQ ID NO: 51 and the second RNA comprises SEQ ID NO: 63a.

**96.** An immunogenic composition comprising a first RNA and a second RNA, wherein:
the first RNA encodes the polypeptide of SEQ ID NO: 55 and comprises the nucleotide sequence of SEQ ID NO: 56 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 56, and
the second RNA encodes the polypeptide of SEQ ID NO: 61 and comprises the nucleotide sequence of SEQ ID NO: 62a, or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 62a, and
wherein each of the first RNA and the second RNA comprise:
(a) modified uridines; and
(b) a 5' cap, and
wherein the first RNA and the second RNA are formulated in lipid nanoparticles (LNPs), wherein the LNPs comprise a cationically ionizable lipid, a neutral lipid, a sterol and a polymer-lipid conjugate.

**97.** The immunogenic composition of claim 96, wherein the first RNA and the second RNA are formulated in separate lipid nanoparticles.

**98.** The immunogenic composition of claim 96, wherein the first RNA and the second RNA are formulated in the same lipid nanoparticles.

**99.** The immunogenic composition of any one of claims 96 to 98, wherein the first RNA and the second RNA each include modified uridines in place of all uridines.

**100.** The immunogenic composition of any one of claims 96 to 99, wherein the modified uridines are each N1-methyl-pseudouridine.

**101.** The immunogenic composition of any one of claims 96 to 100, wherein the first RNA and the second RNA each independently further comprise at least one, at least two, or all of the following features:
a 5' untranslated region (UTR) comprising SEQ ID NO: 12;
a 3' untranslated region (UTR) comprising SEQ ID NO: 13; and
a poly-A sequence of at least 100 A nucleotides.

**102.** The immunogenic composition of claim 101, wherein the poly-A sequence comprises 30 adenine nucleotides followed by 70 adenine nucleotides, wherein the 30 adenine nucleotides and 70 adenine nucleotides are separated by a linker sequence.

**103.** The immunogenic composition of claim 102, wherein the poly-A sequence comprises SEQ ID NO: 14.

**104.** The immunogenic composition of any one of claims 96 to 103, wherein the first RNA comprises SEQ ID NO: 57 and the second RNA comprises SEQ ID NO: 63a.

**105.** The immunogenic composition of any one of claims 4 to 104, wherein the 5'-cap is or comprises m₂^{7,3'-O}Gppp(m₁^{2'-O})ApG.

**106.** The immunogenic composition of any one of claims 4 to 105, wherein the LNP comprises about 40 to about 50 mole percent of a cationically ionizable lipid, about 35 to about 45 mole percent of a sterol, about 5 to about 15 mole percent of a neutral lipid, and about 1 to about 10 mole percent of a polymer-lipid conjugate.

**107.** The immunogenic composition of any one of claims 4 to 106, wherein the composition comprises a plurality of LNPs, wherein the average diameter of the plurality of LNPs is about 30 nm to about 200 nm or about 60 nm to about 120 nm (e.g., as determined by dynamic light scattering measurements).

**108.** A method of eliciting an immune response against SARS-CoV-2 comprising administering the immunogenic composition of any one of claims 4 to 107.

**109.** The method of claim 108, wherein the immune response is elicited against an Omicron variant of SARS-CoV-2.

**110.** The method of claim 108, wherein the immune response is elicited against a Beta variant of SARS-CoV-2.

**111.** The method of claim 108, wherein the immune response is elicited against an Alpha variant of SARS-CoV-2.

**112.** The method of claim 108, wherein the immune response is elicited against a Delta variant of SARS-CoV-2.

**113.** The method of claim 108, wherein the immune response is elicited against a Wuhan strain, an Omicron variant, a Beta variant, an Alpha variant, and a Delta variant of SARS-CoV-2.

**114.** A method for inducing an immune response in a subject, wherein the method comprises delivering (e.g., as a polypeptide or an RNA encoding such a polypeptide) an antigen of a SARS-CoV-2 virus that is not a BA.1 Omicron variant of SARS-CoV-2.

**115.** The method of claim 114, wherein the subject has previously been infected with or vaccinated against SARS-CoV-2.

**116.** The method of claim 114 or 115, wherein the subject has previously been delivered (e.g., as a polypeptide or an RNA encoding such a polypeptide) an antigen of a Wuhan strain of SARS-CoV-2.

**117.** The method of any one of claims 114 to 116, wherein the subject has previously been administered RNA encoding a SARS-CoV-2 S protein of a Wuhan strain.

**118.** The method of any one of claims 114 to 117, wherein the subject has previously been administered two or more doses of RNA encoding a SARS-CoV-2 S protein of a Wuhan strain.

**119.** The method of any one of claims 114 to 118, wherein the method comprises administering RNA encoding an antigen of a SARS-CoV-2 virus that is not a BA.1 Omicron variant.

**120.** The method of any one of claims 114 to 119, wherein the method comprises administering RNA encoding a SARS-CoV-2 S protein from a SARS-CoV-2 variant that is a BA.1 Omicron variant.

**121.** The method of any one of claims 114 to 120, wherein the method comprises administering RNA encoding an S protein of an Omicron variant of SARS-CoV-2, wherein the Omicron variant is not a BA.1 Omicron variant.

**122.** The method of any one of claims 114 to 121, wherein the method comprises administering RNA encoding an S protein of a BA.2 Omicron variant of SARS-CoV-2.

**123.** The method of any one of claims 114 to 121, wherein the method comprises administering RNA encoding an S protein of a BA.4 or BA.5 Omicron variant of SARS-CoV-2.

**124.** A method for inducing an immune response in a subject, wherein the method comprises administering (a) a first RNA encoding a SARS-CoV-2 S protein of a Wuhan strain, an Alpha variant, a Beta variant, or a Delta variant, and (b) a second RNA encoding a SARS-CoV-2 S protein of an Omicron variant that is not a BA.1 Omicron variant.

**125.** The method of claim 124, where the second RNA encodes an S protein of a BA.2 Omicron variant.

**126.** The method of claim 124, where the second RNA encodes an S protein of a BA.4 or BA.5 Omicron variant.

**127.** A method for inducing an immune response in a subject, wherein the method comprises administering (a) a first RNA encoding a SARS-CoV-2 S protein of a Wuhan strain, an Alpha variant, a Beta variant, a Delta variant, or a BA.1 Omicron variant and (b) a second RNA encoding a SARS-CoV-2 S protein that is antigenically distinct from the S protein encoded by the first RNA.

**128.** The method of claim 127, wherein the second RNA encodes a SARS-CoV-2 S protein of an Omicron variant that is not a BA.1 Omicron variant.

**129.** The method of claim 127, wherein the second RNA encodes a SARS-CoV-2 S protein of a BA.2 Omicron variant.

**130.** The method of claim 127, wherein the second RNA encodes a SARS-CoV-2 S protein of a BA.4 or BA.5 Omicron variant.

**131.** The method of any one of claims 114 to 130, wherein the first RNA and the second RNA are encapsulated in separate LNPs.

**132.** The method of any one of claims 114 to 130, wherein the first RNA and the second RNA are encapsulated in the same LNP.

**133.** The method of any one of claims 114 to 131, wherein the first RNA and the second RNA are administered separately, e.g., at different injection sites.

**134.** A composition comprising (a) a first RNA encoding a SARS-CoV-2 S protein of a Wuhan strain, an Alpha variant, a Beta variant, a Delta variant, or a BA.1 Omicron variant and (b) a second RNA encoding a SARS-CoV-2 S protein that is antigenically distinct from the S protein encoded by the first RNA.

**135.** A composition comprising (a) a first RNA encoding a SARS-CoV-2 S protein of a Wuhan strain, an Alpha variant, a Beta variant, a Delta variant, or a BA.1 Omicron variant and (b) a second RNA encoding a SARS-CoV-2 S protein an Omicron variant that is not a BA.1 Omicron variant.

**136.** The composition of claim 135, wherein the second RNA encodes a SARS-CoV-2 S protein of a BA.2 Omicron variant.

**137.** The composition of claim 135, wherein the second RNA encodes a a SARS-CoV-2 S protein of a BA.4 or BA.5 Omicron variant.

**138.** The composition of claim 135, wherein the first RNA encodes a SARS-CoV-2 S protein of a Wuhan strain and the second RNA encodes a SARS-CoV-2 S protein of a BA.2 Omicron variant.

**139.** The composition of claim 135, wherein the first RNA encodes a SARS-CoV-2 S protein of a Wuhan strain and the second RNA encodes a SARS-CoV-2 S protein of a BA.4 or BA.5 Omicron variant.

**140.** The composition of claim 135, wherein the first RNA encodes a SARS-CoV-2 S protein of a BA.1 Omicron variant and the second RNA encodes a SARS-CoV-2 S protein of a BA.2 Omicron variant.

**141.** The composition of claim 135, wherein the first RNA encodes a SARS-CoV-2 S protein of a BA.1 Omicron variant and the second RNA encodes a SARS-CoV-2 S protein of a BA.4 or BA.5 Omicron variant.

**142.** The composition of any one of claims 134 to 141, wherein the first RNA and the second RNA are administered in encapsulated in the same LNP.

**143.** The composition of any one of claims 134 to 141, wherein the first RNA and the second RNA are encapsulated in separate LNPs.

**144.** A composition or medical preparation comprising RNA encoding an amino acid sequence comprising a SARS-CoV-2 S protein, an immunogenic variant thereof, or an immunogenic fragment of the SARS-CoV-2 S protein or the immunogenic variant thereof.

**145.** The composition or medical preparation of claim 144, wherein an immunogenic fragment of the SARS-CoV-2 S protein comprises the S1 subunit of the SARS-CoV-2 S protein, or the receptor binding domain (RBD) of the S1 subunit of the SARS-CoV-2 S protein.

**146.** The composition or medical preparation of claims 144 or 145, wherein the amino acid sequence comprising a SARS-CoV-2 S protein, an immunogenic variant thereof, or an immunogenic fragment of the SARS-CoV-2 S protein or the immunogenic variant thereof is encoded by a coding sequence which is codon-optimized and/or the G/C content of which is increased compared to wild type coding sequence, wherein the codon-optimization and/or the increase in the G/C content preferably does not change the sequence of the encoded amino acid sequence.

**147.** The composition or medical preparation of any one of claims 144 to 146, wherein
(i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 979 to 1584 of SEQ ID NO: 2, 8 or 9, a nucleotide sequence having at least 99%, 98%, 97%, 96%, 95%, 90%, 85%, or 80% identity to the nucleotide sequence of nucleotides 979 to 1584 of SEQ ID NO: 2, 8 or 9, or a fragment of the nucleotide sequence of nucleotides 979 to 1584 of SEQ ID NO: 2, 8 or 9, or the nucleotide sequence having at least 99%, 98%, 97%, 96%, 95%, 90%, 85%, or 80% identity to the nucleotide sequence of nucleotides 979 to 1584 of SEQ ID NO: 2, 8 or 9; and/or
(ii) a SARS-CoV-2 S protein, an immunogenic variant thereof, or an immunogenic fragment of the SARS-CoV-2 S protein or the immunogenic variant thereof comprises the amino acid sequence of amino acids 327 to 528 of SEQ ID NO: 1, an amino acid sequence having at least 99%, 98%, 97%, 96%, 95%, 90%, 85%, or 80% identity to the amino acid sequence of amino acids 327 to 528 of SEQ ID NO: 1, or an immunogenic fragment of the amino acid sequence of amino acids 327 to 528 of SEQ ID NO: 1, or the amino acid sequence having at least 99%, 98%, 97%, 96%, 95%, 90%, 85%, or 80% identity to the amino acid sequence of amino acids 327 to 528 of SEQ ID NO: 1.

**148.** The composition or medical preparation of any one of claims 144 to 146, wherein
(i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 111 to 986 of SEQ ID NO: 30, a nucleotide sequence having at least 99%, 98%, 97%, 96%, 95%, 90%, 85%, or 80% identity to the nucleotide sequence of nucleotides 111 to 986 of SEQ ID NO: 30, or a fragment of the nucleotide sequence of nucleotides 111 to 986 of SEQ ID NO: 30, or the nucleotide sequence having at least 99%, 98%, 97%, 96%, 95%, 90%, 85%, or 80% identity to the nucleotide sequence of nucleotides 111 to 986 of SEQ ID NO: 30; and/or
(ii) a SARS-CoV-2 S protein, an immunogenic variant thereof, or an immunogenic fragment of the SARS-CoV-2 S protein or the immunogenic variant thereof comprises the amino acid sequence of amino acids 20 to 311 of SEQ ID NO: 29, an amino acid sequence having at least 99%, 98%, 97%, 96%, 95%, 90%, 85%, or 80% identity to the amino acid sequence of amino acids 20 to 311 of SEQ ID NO: 29, or an immunogenic fragment of the amino acid sequence of amino acids 20 to 311 of SEQ ID NO: 29, or the amino acid sequence having at least 99%, 98%, 97%, 96%, 95%, 90%, 85%, or 80% identity to the amino acid sequence of amino acids 20 to 311 of SEQ ID NO: 29..

**149.** The composition or medical preparation of any one of claims 144 to 146, wherein
(i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 49 to 3819 of SEQ ID NO: 2, 8 or 9, a nucleotide sequence having at least 99%, 98%, 97%, 96%, 95%, 90%, 85%, or 80% identity to the nucleotide sequence of nucleotides 49 to 3819 of SEQ ID NO: 2, 8 or 9, or a fragment of the nucleotide sequence of nucleotides 49 to 3819 of SEQ ID NO: 2, 8 or 9, or the nucleotide sequence having at least 99%, 98%, 97%, 96%, 95%, 90%, 85%, or 80% identity to the nucleotide sequence of nucleotides 49 to 3819 of SEQ ID NO: 2, 8 or 9; and/or
(ii) a SARS-CoV-2 S protein, an immunogenic variant thereof, or an immunogenic fragment of the SARS-CoV-2 S protein or the immunogenic variant thereof comprises the amino acid sequence of amino acids 17 to 1273 of SEQ ID NO: 1 or 7, an amino acid sequence having at least 99%, 98%, 97%, 96%, 95%, 90%, 85%, or 80% identity to the amino acid sequence of amino acids 17 to 1273 of SEQ ID NO: 1 or 7, or an immunogenic fragment of the amino acid sequence of amino acids 17 to 1273 of SEQ ID NO: 1 or 7, or the amino acid sequence having at least 99%, 98%, 97%, 96%, 95%, 90%, 85%, or 80% identity to the amino acid sequence of amino acids 17 to 1273 of SEQ ID NO: 1 or 7.

**150.** The composition or medical preparation of any one of claims 144 to 149, wherein the amino acid sequence comprising a SARS-CoV-2 S protein, an immunogenic variant thereof, or an immunogenic fragment of the SARS-CoV-2 S protein or the immunogenic variant thereof comprises a secretory signal peptide.

**151.** The composition or medical preparation of claim 150, wherein the secretory signal peptide is fused, preferably N-terminally, to a SARS-CoV-2 S protein, an immunogenic variant thereof, or an immunogenic fragment of the SARS-CoV-2 S protein or the immunogenic variant thereof.

**152.** The composition or medical preparation of claim 150 or 151, wherein
(i) the RNA encoding the secretory signal peptide comprises the nucleotide sequence of nucleotides 1 to 48 of SEQ ID NO: 2, 8 or 9, a nucleotide sequence having at least 99%, 98%, 97%, 96%, 95%, 90%, 85%, or 80% identity to the nucleotide sequence of nucleotides 1 to 48 of SEQ ID NO: 2, 8 or 9, or a fragment of the nucleotide sequence of nucleotides 1 to 48 of SEQ ID NO: 2, 8 or 9, or the nucleotide sequence having at least 99%, 98%, 97%, 96%, 95%, 90%, 85%, or 80% identity to the nucleotide sequence of nucleotides 1 to 48 of SEQ ID NO: 2, 8 or 9; and/or
(ii) the secretory signal peptide comprises the amino acid sequence of amino acids 1 to 16 of SEQ ID NO: 1, an amino acid sequence having at least 99%, 98%, 97%, 96%, 95%, 90%, 85%, or 80% identity to the amino acid sequence of amino acids 1 to 16 of SEQ ID NO: 1, or a functional fragment of the amino acid sequence of amino acids 1 to 16 of SEQ ID NO: 1, or the amino acid sequence having at least 99%, 98%, 97%, 96%, 95%, 90%, 85%, or 80% identity to the amino acid sequence of amino acids 1 to 16 of SEQ ID NO: 1.

**153.** The composition or medical preparation of any one of claims 144 to 152, wherein
(i) the RNA encoding a SARS-CoV-2 S protein, an immunogenic variant thereof, or an immunogenic fragment of the SARS-CoV-2 S protein or the immunogenic variant thereof comprises the nucleotide sequence of SEQ ID NO: 6, a nucleotide sequence having at least 99%, 98%, 97%, 96%, 95%, 90%, 85%, or 80% identity to the nucleotide sequence of SEQ ID NO: 6, or a fragment of the nucleotide sequence of SEQ ID NO: 6, or the nucleotide sequence having at least 99%, 98%, 97%, 96%, 95%, 90%, 85%, or 80% identity to the nucleotide sequence of SEQ ID NO: 6; and/or
(ii) a SARS-CoV-2 S protein, an immunogenic variant thereof, or an immunogenic fragment of the SARS-CoV-2 S protein or the immunogenic variant thereof comprises the amino acid sequence of SEQ ID NO: 5, an amino acid sequence having at least 99%, 98%, 97%, 96%, 95%, 90%, 85%, or 80% identity to the amino acid sequence of SEQ ID NO: 5, or an immunogenic fragment of the amino acid sequence of SEQ ID NO: 5, or the amino acid sequence having at least 99%, 98%, 97%, 96%, 95%, 90%, 85%, or 80% identity to the amino acid sequence of SEQ ID NO: 5.

**154.** The composition or medical preparation of any one of claims 144 to 153, wherein
(i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 54 to 986 of SEQ ID NO: 30, a nucleotide sequence having at least 99%, 98%, 97%, 96%, 95%, 90%, 85%, or 80% identity to the nucleotide sequence of nucleotides 54 to 986 of SEQ ID NO: 30, or a fragment of the nucleotide sequence of nucleotides 54 to 986 of SEQ ID NO: 30, or the nucleotide sequence having at least 99%, 98%, 97%, 96%, 95%, 90%, 85%, or 80% identity to the nucleotide sequence of nucleotides 54 to 986 of SEQ ID NO: 30; and/or
(ii) a SARS-CoV-2 S protein, an immunogenic variant thereof, or an immunogenic fragment of the SARS-CoV-2 S protein or the immunogenic variant thereof comprises the amino acid sequence of amino acids 1 to 311 of SEQ ID NO: 29, an amino acid sequence having at least 99%, 98%, 97%, 96%, 95%, 90%, 85%, or 80% identity to the amino acid sequence of amino acids 1 to 311 of SEQ ID NO: 29, or an immunogenic fragment of the amino acid sequence of amino acids 1 to 311 of SEQ ID NO: 29, or the amino acid sequence having at least 99%, 98%, 97%, 96%, 95%, 90%, 85%, or 80% identity to the amino acid sequence of amino acids 1 to 311 of SEQ ID NO: 29.

**155.** The composition or medical preparation of any one of claims 144 to 154, wherein the RNA comprises a modified nucleoside in place of uridine, in particular wherein the modified nucleoside is selected from pseudouridine (ψ), N1-methyl-pseudouridine (m1ψ), and 5-methyl-uridine (m5U), in particular wherein the modified nucleoside is N1-methyl-pseudouridine (m1ψ).

**156.** The composition or medical preparation of any one of claims 144 to 155, wherein the RNA comprises a 5' cap.

**157.** The composition or medical preparation of any one of claims 144 to 156, wherein the RNA encoding an amino acid sequence comprising a SARS-CoV-2 S protein, an immunogenic variant thereof, or an immunogenic fragment of the SARS-CoV-2 S protein or the immunogenic variant thereof comprises a 5' UTR comprising the nucleotide sequence of SEQ ID NO: 12, or a nucleotide sequence having at least 99%, 98%, 97%, 96%, 95%, 90%, 85%, or 80% identity to the nucleotide sequence of SEQ ID NO: 12.

**158.** The composition or medical preparation of any one of claims 144 to 157, wherein the RNA encoding an amino acid sequence comprising a SARS-CoV-2 S protein, an immunogenic variant thereof, or an immunogenic fragment of the SARS-CoV-2 S protein or the immunogenic variant thereof comprises a 3' UTR comprising the nucleotide sequence of SEQ ID NO: 13, or a nucleotide sequence having at least 99%, 98%, 97%, 96%, 95%, 90%, 85%, or 80% identity to the nucleotide sequence of SEQ ID NO: 13.

**159.** The composition or medical preparation of any one of claims 144 to 158, wherein the RNA encoding an amino acid sequence comprising a SARS-CoV-2 S protein, an immunogenic variant thereof, or an immunogenic fragment of the SARS-CoV-2 S protein or the immunogenic variant thereof comprises a poly-A sequence.

**160.** The composition or medical preparation of claim 159, wherein the poly-A sequence comprises at least 100 nucleotides.

**161.** The composition or medical preparation of claim 159 or 160, wherein the poly-A sequence comprises or consists of the nucleotide sequence of SEQ ID NO: 14.

**162.** The composition or medical preparation of any one of claims 144 to 161, wherein the RNA is formulated or is to be formulated as a liquid, a solid, or a combination thereof.

**163.** The composition or medical preparation of any one of claims 144 to 162, wherein the RNA is formulated or is to be formulated for injection.

**164.** The composition or medical preparation of any one of claims 144 to 163, wherein the RNA is formulated or is to be formulated for intramuscular administration.

**165.** The composition or medical preparation of any one of claims 144 to 164, wherein the RNA is formulated or is to be formulated as particles.

**166.** The composition or medical preparation of claim 165, wherein the particles are lipid nanoparticles (LNP) or lipoplex (LPX) particles.

**167.** The composition or medical preparation of claim 166, wherein the LNP particles comprise a cationically ionizable lipid, a neutral lipid, a sterol and a polymer-lipid conjugate.

**168.** The composition or medical preparation of claim 167, wherein the RNA lipoplex particles are obtainable by mixing the RNA with liposomes.

**169.** The composition or medical preparation of any one of claims 144 to 168, wherein the RNA is mRNA or saRNA.

**170.** The composition or medical preparation of any one of claims 144 to 169, which is a pharmaceutical composition.

**171.** The composition or medical preparation of any one of claims 144 to 170, which is a vaccine.

**172.** The composition or medical preparation of claim 170 or 171, wherein the pharmaceutical composition further comprises one or more pharmaceutically acceptable carriers, diluents and/or excipients.

**173.** The composition or medical preparation of any one of claims 144 to 172, which is a kit.

**174.** The composition or medical preparation of claim 173, wherein the RNA and optionally the particle forming components are in separate vials.

**175.** The composition or medical preparation of claim 173 or 174, further comprising instructions for use of the composition or medical preparation for inducing an immune response against coronavirus in a subject.

**176.** The composition or medical preparation of any one of claims 144 to 175 for pharmaceutical use.

**176.** The composition or medical preparation of claim 176, wherein the pharmaceutical use comprises inducing an immune response against coronavirus in a subject.

**178.** The composition or medical preparation of claim 176 or 177, wherein the pharmaceutical use comprises a therapeutic or prophylactic treatment of a coronavirus infection.

**179.** The composition or medical preparation of any one of claims 144 to 178, which is for administration to a human.

**180.** The composition or medical preparation of any one of claims 175 to 179, wherein the coronavirus is a betacoronavirus.

**181.** The composition or medical preparation of any one of claims 175 to 180, wherein the coronavirus is a sarbecovirus.

**182.** The composition or medical preparation of any one of claims 175 to 181, wherein the coronavirus is SARS-CoV-2.

**183.** A method of inducing an immune response against coronavirus in a subject comprising administering to the subject a composition comprising RNA encoding an amino acid sequence comprising a SARS-CoV-2 S protein, an immunogenic variant thereof, or an immunogenic fragment of the SARS-CoV-2 S protein or the immunogenic variant thereof.

**184.** The method of claim 183, wherein an immunogenic fragment of the SARS-CoV-2 S protein comprises the S1 subunit of the SARS-CoV-2 S protein, or the receptor binding domain (RBD) of the S1 subunit of the SARS-CoV-2 S protein.

**185.** The method of any one of claims 183 or 184, wherein the amino acid sequence comprising a SARS-CoV-2 S protein, an immunogenic variant thereof, or an immunogenic fragment of the SARS-CoV-2 S protein or the immunogenic variant thereof is encoded by a coding sequence which is codon-optimized and/or the G/C content of which is increased compared to wild type coding sequence, wherein the codon-optimization and/or the increase in the G/C content preferably does not change the sequence of the encoded amino acid sequence.

**186.** The method of any one of claims 183 to 185, wherein
(i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 979 to 1584 of SEQ ID NO: 2, 8 or 9, a nucleotide sequence having at least 99%, 98%, 97%, 96%, 95%, 90%, 85%, or 80% identity to the nucleotide sequence of nucleotides 979 to 1584 of SEQ ID NO: 2, 8 or 9, or a fragment of the nucleotide sequence of nucleotides 979 to 1584 of SEQ ID NO: 2, 8 or 9, or the nucleotide sequence having at least 99%, 98%, 97%, 96%, 95%, 90%, 85%, or 80% identity to the nucleotide sequence of nucleotides 979 to 1584 of SEQ ID NO: 2, 8 or 9; and/or
(ii) a SARS-CoV-2 S protein, an immunogenic variant thereof, or an immunogenic fragment of the SARS-CoV-2 S protein or the immunogenic variant thereof comprises the amino acid sequence of amino acids 327 to 528 of SEQ ID NO: 1, an amino acid sequence having at least 99%, 98%, 97%, 96%, 95%, 90%, 85%, or 80% identity to the amino acid sequence of amino acids 327 to 528 of SEQ ID NO: 1, or an immunogenic fragment of the amino acid sequence of amino acids 327 to 528 of SEQ ID NO: 1, or the amino acid sequence having at least 99%, 98%, 97%, 96%, 95%, 90%, 85%, or 80% identity to the amino acid sequence of amino acids 327 to 528 of SEQ ID NO: 1.

**187.** The method of any one of claims 183 to 186, wherein
(i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 111 to 986 of SEQ ID NO: 30, a nucleotide sequence having at least 99%, 98%, 97%, 96%, 95%, 90%, 85%, or 80% identity to the nucleotide sequence of nucleotides 111 to 986 of SEQ ID NO: 30, or a fragment of the nucleotide sequence of nucleotides 111 to 986 of SEQ ID NO: 30, or the nucleotide sequence having at least 99%, 98%, 97%, 96%, 95%, 90%, 85%, or 80% identity to the nucleotide sequence of nucleotides 111 to 986 of SEQ ID NO: 30; and/or
(ii) a SARS-CoV-2 S protein, an immunogenic variant thereof, or an immunogenic fragment of the SARS-CoV-2 S protein or the immunogenic variant thereof comprises the amino acid sequence of amino acids 20 to 311 of SEQ ID NO: 29, an amino acid sequence having at least 99%, 98%, 97%, 96%, 95%, 90%, 85%, or 80% identity to the amino acid sequence of amino acids 20 to 311 of SEQ ID NO: 29, or an immunogenic fragment of the amino acid sequence of amino acids 20 to 311 of SEQ ID NO: 29, or the amino acid sequence having at least 99%, 98%, 97%, 96%, 95%, 90%, 85%, or 80% identity to the amino acid sequence of amino acids 20 to 311 of SEQ ID NO: 29.

**188.** The method of any one of claims 183 to 187, wherein
(i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 49 to 3819 of SEQ ID NO: 2, 8 or 9, a nucleotide sequence having at least 99%, 98%, 97%, 96%, 95%, 90%, 85%, or 80% identity to the nucleotide sequence of nucleotides 49 to 3819 of SEQ ID NO: 2, 8 or 9, or a fragment of the nucleotide sequence of nucleotides 49 to 3819 of SEQ ID NO: 2, 8 or 9, or the nucleotide sequence having at least 99%, 98%, 97%, 96%, 95%, 90%, 85%, or 80% identity to the nucleotide sequence of nucleotides 49 to 3819 of SEQ ID NO: 2, 8 or 9; and/or
(ii) a SARS-CoV-2 S protein, an immunogenic variant thereof, or an immunogenic fragment of the SARS-CoV-2 S protein or the immunogenic variant thereof comprises the amino acid sequence of amino acids 17 to 1273 of SEQ ID NO: 1 or 7, an amino acid sequence having at least 99%, 98%, 97%, 96%, 95%, 90%, 85%, or 80% identity to the amino acid sequence of amino acids 17 to 1273 of SEQ ID NO: 1 or 7, or an immunogenic fragment of the amino acid sequence of amino acids 17 to 1273 of SEQ ID NO: 1 or 7, or the amino acid sequence having at least 99%, 98%, 97%, 96%, 95%, 90%, 85%, or 80% identity to the amino acid sequence of amino acids 17 to 1273 of SEQ ID NO: 1 or 7.

**189.** The method of any one of claims 183 to 188, wherein the amino acid sequence comprising a SARS-CoV-2 S protein, an immunogenic variant thereof, or an immunogenic fragment of the SARS-CoV-2 S protein or the immunogenic variant thereof comprises a secretory signal peptide.

**190.** The method of claim 189, wherein the secretory signal peptide is fused, preferably N-terminally, to a SARS-CoV-2 S protein, an immunogenic variant thereof, or an immunogenic fragment of the SARS-CoV-2 S protein or the immunogenic variant thereof.

**191.** The method of claim 189 or 190, wherein
(i) the RNA encoding the secretory signal peptide comprises the nucleotide sequence of nucleotides 1 to 48 of SEQ ID NO: 2, 8 or 9 175 a nucleotide sequence having at least 99%, 98%, 97%, 96%, 95%, 90%, 85%, or 80% identity to the nucleotide sequence of nucleotides 1 to 48 of SEQ ID NO: 2, 8 or 9, or a fragment of the nucleotide sequence of nucleotides 1 to 48 of SEQ ID NO: 2, 8 or 9, or the nucleotide sequence having at least 99%, 98%, 97%, 96%, 95%, 90%, 85%, or 80% identity to the nucleotide sequence of nucleotides 1 to 48 of SEQ ID NO: 2, 8 or 9; and/or
(ii) the secretory signal peptide comprises the amino acid sequence of amino acids 1 to 16 of SEQ ID NO: 1, an amino acid sequence having at least 99%, 98%, 97%, 96%, 95%, 90%, 85%, or 80% identity to the amino acid sequence of amino acids 1 to 16 of SEQ ID NO: 1, or a functional fragment of the amino acid sequence of amino acids 1 to 16 of SEQ ID NO: 1, or the amino acid sequence having at least 99%, 98%, 97%, 96%, 95%, 90%, 85%, or 80% identity to the amino acid sequence of amino acids 1 to 16 of SEQ ID NO: 1.

**192.** The method of any one of claims 183 to 191, wherein
(i) the RNA encoding a SARS-CoV-2 S protein, an immunogenic variant thereof, or an immunogenic fragment of the SARS-CoV-2 S protein or the immunogenic variant thereof comprises the nucleotide sequence of SEQ ID NO: 6, a nucleotide sequence having at least 99%, 98%, 97%, 96%, 95%, 90%, 85%, or 80% identity to the nucleotide sequence of SEQ ID NO: 6, or a fragment of the nucleotide sequence of SEQ ID NO: 6, or the nucleotide sequence having at least 99%, 98%, 97%, 96%, 95%, 90%, 85%, or 80% identity to the nucleotide sequence of SEQ ID NO: 6; and/or
(ii) a SARS-CoV-2 S protein, an immunogenic variant thereof, or an immunogenic fragment of the SARS-CoV-2 S protein or the immunogenic variant thereof comprises the amino acid sequence of SEQ ID NO: 5, an amino acid sequence having at least 99%, 98%, 97%, 96%, 95%, 90%, 85%, or 80% identity to the amino acid sequence of SEQ ID NO: 5, or an immunogenic fragment of the amino acid sequence of SEQ ID NO: 5, or the amino acid sequence having at least 99%, 98%, 97%, 96%, 95%, 90%, 85%, or 80% identity to the amino acid sequence of SEQ ID NO: 5.

**193.** The method of any one of claims 183 to 192, wherein
(i) the RNA encoding a SARS-CoV-2 S protein, an immunogenic variant thereof, or an immunogenic fragment of the SARS-CoV-2 S protein or the immunogenic variant thereof comprises the nucleotide sequence of nucleotides 54 to 986 of SEQ ID NO: 30, a nucleotide sequence having at least 99%, 98%, 97%, 96%, 95%, 90%, 85%, or 80% identity to the nucleotide sequence of nucleotides 54 to 986 of SEQ ID NO: 30, or a fragment of the nucleotide sequence of nucleotides 54 to 986 of SEQ ID NO: 30, or the nucleotide sequence having at least 99%, 98%, 97%, 96%, 95%, 90%, 85%, or 80% identity to the nucleotide sequence of nucleotides 54 to 986 of SEQ ID NO: 30; and/or
(ii) a SARS-CoV-2 S protein, an immunogenic variant thereof, or an immunogenic fragment of the SARS-CoV-2 S protein or the immunogenic variant thereof comprises the amino acid sequence of amino acids 1 to 311 of SEQ ID NO: 29, an amino acid sequence having at least 99%, 98%, 97%, 96%, 95%, 90%, 85%, or 80% identity to the amino acid sequence of amino acids 1 to 311 of SEQ ID NO: 29, or an immunogenic fragment of the amino acid sequence of amino acids 1 to 311 of SEQ ID NO: 29, or the amino acid sequence having at least 99%, 98%, 97%, 96%, 95%, 90%, 85%, or 80% identity to the amino acid sequence of amino acids 1 to 311 of SEQ ID NO: 29.

**194.** The method of any one of claims 183 to 192, wherein the RNA comprises a modified nucleoside in place of uridine, in particular wherein the modified nucleoside is selected from pseudouridine (ψ), N1-methyl-pseudouridine (m1ψ), and 5-methyl-uridine (m5U), in particular wherein the modified nucleoside is N1-methyl-pseudouridine (m1ψ).

**195.** The method of any one of claims 183 to 194, wherein the RNA comprises a cap.

**196.** The method of any one of claims 183 to 195, wherein the RNA encoding an amino acid sequence comprising a SARS-CoV-2 S protein, an immunogenic variant thereof, or an immunogenic fragment of the SARS-CoV-2 S protein or the immunogenic variant thereof comprises a 5' UTR comprising the nucleotide sequence of SEQ ID NO: 12, or a nucleotide sequence having at least 99%, 98%, 97%, 96%, 95%, 90%, 85%, or 80% identity to the nucleotide sequence of SEQ ID NO: 12.

**197.** The method of any one of claims 183 to 196, wherein the RNA encoding an amino acid sequence comprising a SARS-CoV-2 S protein, an immunogenic variant thereof, or an immunogenic fragment of the SARS-CoV-2 S protein or the immunogenic variant thereof comprises a 3' UTR comprising the nucleotide sequence of SEQ ID NO: 13, or a nucleotide sequence having at least 99%, 98%, 97%, 96%, 95%, 90%, 85%, or 80% identity to the nucleotide sequence of SEQ ID NO: 13.

**198.** The method of any one of claims 183 to 197, wherein the RNA encoding an amino acid sequence comprising a SARS-CoV-2 S protein, an immunogenic variant thereof, or an immunogenic fragment of the SARS-CoV-2 S protein or the immunogenic variant thereof comprises a poly-A sequence.

**199.** The method of claim 198, wherein the poly-A sequence comprises at least 100 nucleotides.

**200.** The method of claim 198 or 199, wherein the poly-A sequence comprises or consists of the nucleotide sequence of SEQ ID NO: 14.

**201.** The method of any one of claims 183 to 200, wherein the RNA is formulated as a liquid, a solid, or a combination thereof.

**202.** The method of any one of claims 183 to 201, wherein the RNA is administered by injection.

**203.** The method of any one of claims 183 to 202, wherein the RNA is administered by intramuscular administration.

**204.** The method of any one of claims 183 to 203, wherein the RNA is formulated as particles.

**205.** The method of claim 204, wherein the particles are lipid nanoparticles (LNP) or lipoplex (LPX) particles.

**206.** The method of claim 205, wherein the LNP particles comprise a cationically ionizable lipid, a neutral lipid, a sterol and a polymer-lipid conjugate.

**207.** The method of any one of claim 205, wherein the RNA lipoplex particles are obtainable by mixing the RNA with liposomes.

**208.** The composition or medical preparation of any one of claims 183 to 207, wherein the RNA is mRNA or saRNA.

**209.** The method of any one of claims 183 to 208, which is a method for vaccination against coronavirus.

**210.** The method of any one of claims 183 to 209, which is a method for therapeutic or prophylactic treatment of a coronavirus infection.

**211.** The method of any one of claims 183 to 210, wherein the subject is a human.

**212.** The method of any one of claims 183 to 211, wherein the coronavirus is a betacoronavirus.

**213.** The method of any one of claims 183 to 212, wherein the coronavirus is a sarbecovirus.

**214.** The method of any one of claims 183 to 213, wherein the coronavirus is SARS-CoV-2.

**215.** The method of any one of claims 108 to 133 or 183 to 214, wherein the composition is a composition of any one of claims 1 to 107 or 134 to 182.

**216.** A composition or medical preparation of any one of claims 1 to 107 or 134 to 182 for use in a method of any one of claims 108 to 133 or 183 to 215.

**217.** The composition or medical preparation of any one of claims 1 to 107 or 134 to 182, which is a pharmaceutical composition.

**218.** The composition or medical preparation of any one of claims 1 to 107 or 134 to 182, which is a vaccine.

**219.** The composition or medical preparation of claim 217 or 218, wherein the pharmaceutical composition or vaccine further comprises one or more pharmaceutically acceptable carriers, diluents and/or excipients.

**220.** The composition or medical preparation of any one of claim 1 to 107 or 134 to 182, which is a kit.

**221.** The composition or medical preparation of claim 220, wherein the RNA and optionally the particle forming components are in separate vials.

**222.** The composition or medical preparation of claim 220 or 221, further comprising instructions for use of the composition or medical preparation for inducing an immune response against coronavirus in a subject.

**223.** The composition or medical preparation of any one of claims 1 to 107 or 134 to 182 for pharmaceutical use.

**224.** The composition or medical preparation of claim 223, wherein the pharmaceutical use comprises inducing an immune response against coronavirus in a subject.

**225.** The composition or medical preparation of claim 223 or 224, wherein the pharmaceutical use comprises therapeutic or prophylactic treatment of a coronavirus infection.

**226.** The composition or medical preparation of any one of claims 1 to 107 or 134 to 182 for use in the manufacture of a medicament.

**227.** The composition or medical preparation of claim 226, wherein the medicament is for use in inducing an immune response against coronavirus in a subject.

**228.** The composition or medical preparation of claim 226 or 227, wherein the medicament is for use in therapeutic or prophylactic treatment of a coronavirus infection.

**229.** The composition or medical preparation of any one of claims 1 to 107 or 134 to 182, which is for administration to a human.

**230.** The composition or medical preparation of any one of claims 222 to 229, wherein the coronavirus is a betacoronavirus.

**231.** The composition or medical preparation of claim 230, wherein the coronavirus is a sarbecovirus.

**232.** The composition or medical preparation of claim 231, wherein the coronavirus is SARS-CoV-2.
